# EUROPEAN PATENT APPLICATION

(11) **EP 4 223 761 A1**
(43) Date of publication of application: **09.08.2023**
(21) Application number: 21874575.0
(22) Date of filing: 30.09.2021
(51) Int. Cl.: C07D 487/08, C07D 519/00, C07D 487/10, A61P 35/00, A61P 35/02, A61K 31/517, A61K 31/519

(54) **QUINAZOLINE COMPOUND AND APPLICATION THEREOF**

(30) Priority: 30.09.2020 CN 202011065873; 10.02.2021 CN 202110186596; 16.03.2021 CN 202110283127; 23.04.2021 CN 202110442666; 25.05.2021 CN 202110574061; 22.06.2021 CN 202110694128; 15.07.2021 CN 202110801673; 03.09.2021 CN 202111032451
(71) Applicant: Shanghai Pharmaceuticals Holding Co., Ltd., Shanghai 201203 (CN)
(72) Inventor: ZHANG, Chaoxin, Shanghai 201203 (CN); MAO, Haibin, Shanghai 201203 (CN); XIA, Guangxin, Shanghai 201203 (CN); FU, Yuhong, Shanghai 201203 (CN); WANG, Xuesong, Shanghai 201203 (CN); CAI, Yingchun, Shanghai 201203 (CN); MA, Xing, Shanghai 201203 (CN); DENG, Wei, Shanghai 201203 (CN); KE, Ying, Shanghai 201203 (CN); FU, Jianmin, Shanghai 201203 (CN)
(74) Representative: Mewburn Ellis LLP
(86) International application number: PCT/CN2021/122076
(87) International publication number: WO 2022/068921

(57) **Abstract**

A quinazoline compound as shown in formula I, and a pharmaceutically acceptable salt, solvate, prodrug, metabolite or isotopic compound thereof having a good inhibiting effect on a KRAS mutant protein.

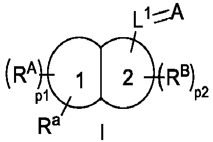

## Description

The present application claims the right of the priorities for the Chinese application number of 2020110658732 filed on Spetember 30, 2020, the Chinese application number of 2021101865969 filed on February 10, 2021, the Chinese application number of 2021102831279 filed on March 16, 2021, the Chinese application number of 2021104426662 filed on April 23, 2021, the Chinese application number of 2021105740619 filed on May 25, 2021, the Chinese application number of 2021106941282 filed on June 22, 2021, the Chinese application number of 2021108016737 filed on July 15, 2021, and the Chinese application number of 202111032451X filed on September 3, 2021, the contents of which are incorporated herein by reference in their entireties.

### TECHNICAL FIELD

The present disclosure related to a quinazoline compound and a use thereof.

### BACKGROUND

RAS represents a group of closely related monomer globular proteins with 189 amino acids (molecular weight of 21 kDa), which are related to plasma membrane and bind to GDP or GTP. RAS acts as a molecular switch. When RAS contains bonded GDP, the RAS is in a stationary or closed position and "inactive". In response to cell exposure to certain growth-promoting stimuli, the RAS is induced to exchange its bonded GDP for GTP. In the case of binding to GTP, the RAS is "turned on" and is able to interact with other proteins (its "downstream targets") and activate the proteins. The RAS protein itself has extremely low inherent ability to hydrolyze GTP back to GDP, thus turning itself into a closed state. Closing RAS requires an exogenous protein called GTPase activated protein (GAP), which interacts with RAS and greatly accelerates the conversion of GTP to GDP. Any mutation in RAS that affects its ability to interact with GAP or convert GTP back to GDP will lead to prolonged activation of protein, and thus prolonging signal to cells to tell them to continue to grow and divide. Since these signals lead to cell growth and division, over-activated RAS signaling can eventually lead to cancer.

Structurally, RAS protein contains G domain which is responsible for the enzymatic activity of RAS-guanine nucleotide binding and hydrolysis (GTPase reaction). It also contains the C-terminal extension region called CAAX box, which can be modified after translation and is responsible for targeting the protein to the membrane. The G domain is about 21-25 kDa in size and contains a phosphate-binding loop (P-loop). The P-loop represents the pocket of bound nucleotides in the protein and this is the rigid part of the structural domain with conserved amino acid residues, and the conserved amino acid residues are necessary for nucleotide binding and hydrolysis (glycine 12, threonine 26 and lysine 16). The G domain also contains the so-called switch I region (residues 30-40) and switch II region (residues 60-76), both of which are dynamic parts of protein, which are often expressed as a "spring-loaded" mechanism because of the ability of the dynamic part to switch between the rest state and the loaded state. The main interaction is the hydrogen bond formed by threonine-35 and glycine-60 with the γ-phosphate of GTP, which maintains the switch 1 and switch 2 regions in their active conformations, respectively. After hydrolysis of GTP and release of phosphate, both of them relax into inactive GDP conformation.

The most notable members of the RAS subfamily are HRAS, KRAS and NRAS, which are mainly implicated in many types of cancers. However, many other members exist, including DIRAS1; DIRAS2; DIRAS3; ERAS; GEM; MRAS; NKIRAS1; NKIRAS2; NRAS; RALA; RALB; RAP1A; RAP1B; RAP2A; RAP2B; RAP2C; RASD1; RASD2; RASL10A; RASL10B; RASL11A; RASL11B; RASL12; REM1; REM2; RERG; RERGL; RRAD; RRAS and RRAS2.

Mutation of any of the three major isoforms of RAS gene (HRAS, NRAS or KRAS) is one of the most common events in human tumor formation. It is found that about 30% of all human tumors carry some mutations in RAS gene. Notably, KRAS mutations are detected in 25% to 30% of tumors. In contrast, the rate of oncogenic mutations in members of the NRAS and HRAS families is much lower (8% and 3%, respectively). The most common KRAS mutations are found in the residues G12 and G13 and residue Q61 in P-loop. Among the tumor-related KRAS G12 mutations, the mutation probability of KRAS G12D is the highest, about 40%.

Based on the importance of abnormal activation of KRAS in cancer progression and the universality of KRAS gene mutation in human cancer, KRAS has always been the target of drug developers. Although progress has been made in this field, there is still a need in this field for improved KRAS G12D mutein inhibitors.

### CONTENT OF THE PRESENT INVENTION

The technical problem to be solved by the present disclosure is to provide a quinazoline compound and a use thereof in order to overcome the lack of KRAS G12D mutant protein inhibitor in the prior art. The quinazoline compound provided by the present disclosure has a good inhibitory effect on KRAS G12D mutant protein.

The present disclosure solves the above technical problems by the following technical scheme.

The present disclosure provides a quinazoline compound represented by formula I, a pharmaceutically acceptable salt thereof, a solvate thereof, a prodrug thereof, a metabolite thereof or an isotopic compound thereof, 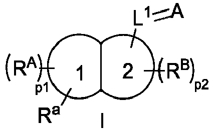
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 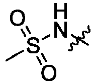 , 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being "halogen, C₁-C₆ alkyl, cyano or alkynyl", C₄-C₈ cycloalkyl-fused C₆-C₁₀ aryl substituted by one or more R^{a-2}, or C₆-C₁₀ aryl substituted by one or more R^{a-4}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 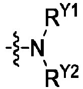 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 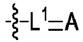
is 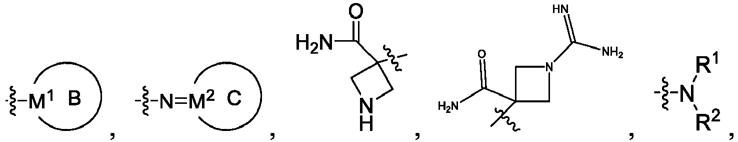 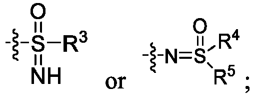
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 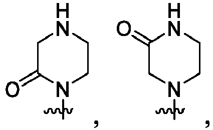 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 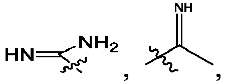 C₁-C₆ alkyl, C₁-C₃ alkyl substituted by CN, or C₄-C₆ alkyl substituted by CN; m is 0, 1 or 2; n is 1, 2 or 3;
R¹⁰ is amino or 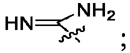
R¹¹ is independently halogen, 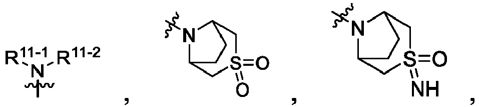 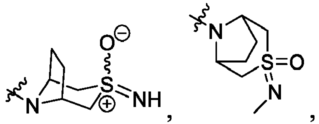 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 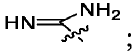
R¹¹⁻³ is H, 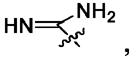 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, C₁-C₆ alkyl substituted by R¹¹⁻³⁻¹, C₁-C₆ alkoxy substituted by R¹¹⁻³⁻², or C₂-C₆ alkenyl;
R¹¹⁻⁴ is C₁-C₆ alkyl;
R¹¹⁻⁶ is unsubstituted C₁-C₆ alkyl, or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 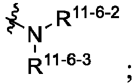
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 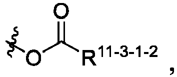 unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R¹¹⁻³⁻¹⁻² is independently 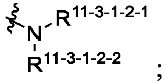
R¹¹⁻³⁻¹⁻²⁻¹ and R¹¹⁻³⁻¹⁻²⁻² are independently H or C₁-C₆ alkyl;
R¹¹⁻³⁻² is C₁-C₁₂ alkenyl;
R^{L} is H or 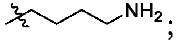
R^{a-1} is independently hydroxyl, halogen, cyano, C₁-C₆ alkyl, C₃-C₁₂ cycloalkyl, unsubstituted C₂-C₆ alkynyl or C₂-C₆ alkynyl substituted by one or more R^{a-1-1}, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R^{a-4} is 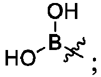
R¹² is C₁-C₆ alkyl;
R¹³ is H, C₁-C₆ alkyl, 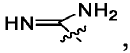 or C₃-C₆ cycloalkyl substituted by R¹³⁻¹;
R¹³⁻¹ is 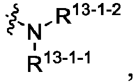 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 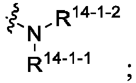
R¹⁴⁻¹⁻¹ and R¹⁴⁻¹⁻² are independently H or C₁-C₆ alkyl;
R¹⁵ is 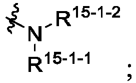
R¹⁵⁻¹⁻¹ and R¹⁵⁻¹⁻² are independently H or C₁-C₆ alkyl;
R¹⁶ is 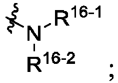
R¹⁶⁻¹ and R¹⁶⁻² are independently H or C₁-C₆ alkyl;
R¹⁷ is 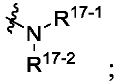
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 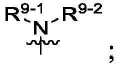
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 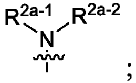
R^{2a-1} and R^{2a-2} are independently H, -C(=O)CH₃, C₁-C₃ alkyl or C₄-C₆ alkyl;
when ring 1 is independently pyridine, R^{B} is

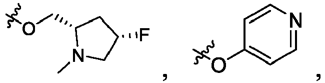

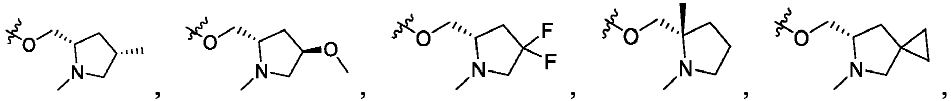

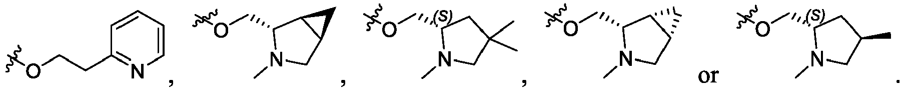

In a certain embodiment, the quinazoline compound represented by formula I, the pharmaceutically acceptable salt thereof, the solvate thereof, the prodrug thereof, the metabolite thereof or the isotopic compound thereof, 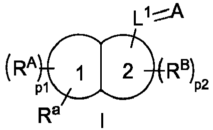
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 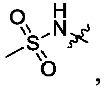 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, C₄-C₈ cycloalkyl-fused C₆-C₁₀ aryl substituted by one or more R^{a-2}, or C₆-C₁₀ aryl substituted by one or more R^{a-4}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 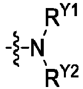 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 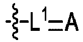
is 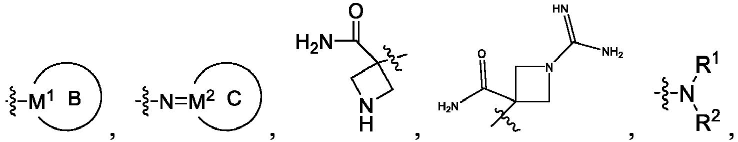 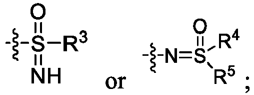
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 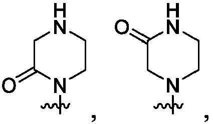 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 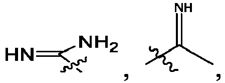 C₁-C₆ alkyl, C₁-C₃ alkyl substituted by CN, or C₄-C₆ alkyl substituted by CN; m is 0, 1 or 2; n is 1, 2 or 3;
R¹⁰ is amino or 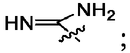
R¹¹ is independently halogen, 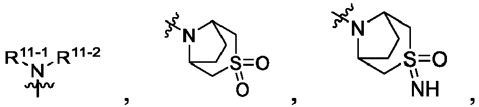 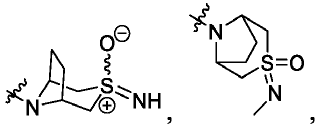 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 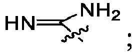
R¹¹⁻³ is H, 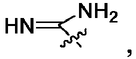 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, C₁-C₆ alkyl substituted by R¹¹⁻³⁻¹, or C₁-C₆ alkoxy substituted by R¹¹⁻³⁻²;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 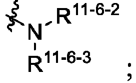
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 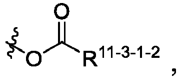 unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R¹¹⁻³⁻¹⁻² is independently 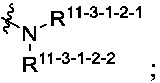
R¹¹⁻³⁻¹⁻²⁻¹ and R¹¹⁻³⁻¹⁻²⁻² are independently H or C₁-C₆ alkyl;
R¹¹⁻³⁻² is C₁-C₁₂ alkenyl;
R^{L} is H or 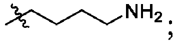
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2}.
R^{a-2} is independently hydroxyl or C₁-C₆ alkyl;
R^{a-1-1} is independently halogen;
R^{a-1-2} is independently halogen;
R^{a-3} is C₁-C₆ alkyl;
R^{a-4} is 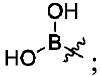
R¹² is C₁-C₆ alkyl;
R¹³ is H, C₁-C₆ alkyl, 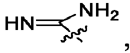 or C₃-C₆ cycloalkyl substituted by R¹³⁻¹;
R¹³⁻¹ is 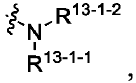 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 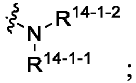
R¹⁴⁻¹⁻¹ and R¹⁴⁻¹⁻² are independently H or C₁-C₆ alkyl;
R¹⁵ is 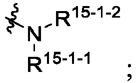
R¹⁵⁻¹⁻¹ and R¹⁵⁻¹⁻² are independently H or C₁-C₆ alkyl;
R¹⁶ is 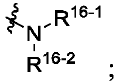
R¹⁶⁻¹ and R¹⁶⁻² are independently H or C₁-C₆ alkyl;
R¹⁷ is 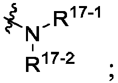
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 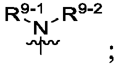
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 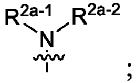
R^{2a-1} and R^{2a-2} are independently H, -C(=O)CH₃, C₁-C₃ alkyl or C₄-C₆ alkyl;
when ring 1 is independently pyridine, R^{B} is

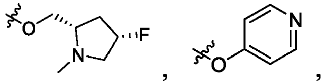

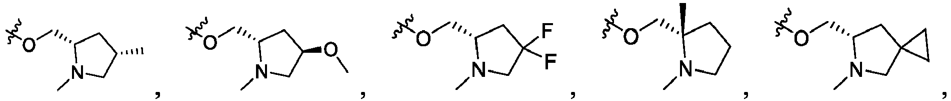

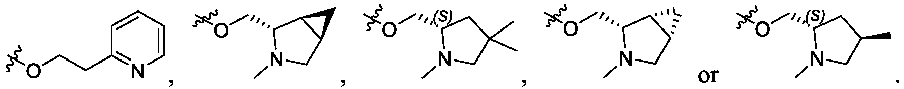

In a certain embodiment, the quinazoline compound represented by formula I, the pharmaceutically acceptable salt thereof, the solvate thereof, the prodrug thereof, the metabolite thereof or the isotopic compound thereof, 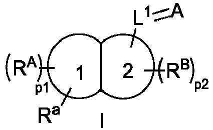
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 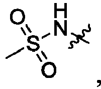 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6-to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 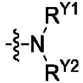 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 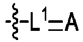
is 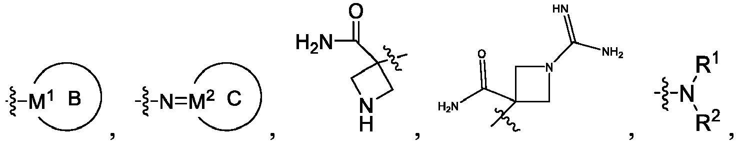 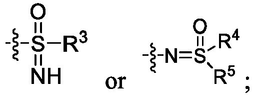
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 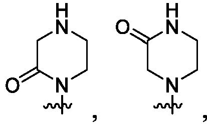 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 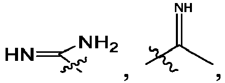 C₁-C₆ alkyl, C₁-C₃ alkyl substituted by CN or C₄-C₆ alkyl substituted by CN; m is 0, 1 or 2; n is 1, 2 or 3;
R¹⁰ is amino or 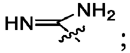
R¹¹ is independently halogen, 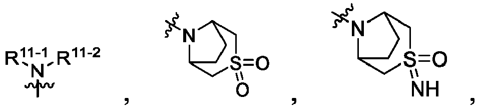 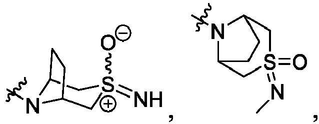 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 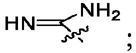
R¹¹⁻³ is H, 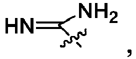 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl, or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 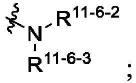
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 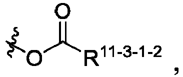 unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R¹¹⁻³⁻¹⁻² is independently 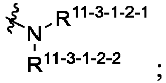
R¹¹⁻³⁻¹⁻²⁻¹ and R¹¹⁻³⁻¹⁻²⁻² are independently H or C₁-C₆ alkyl;
R^{L} is H or 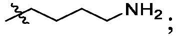
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2}.
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 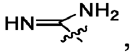 or C₃-C₆ cycloalkyl substituted by R¹³⁻¹;
R¹³⁻¹ is 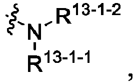 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 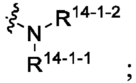
R¹⁴⁻¹⁻¹ and R¹⁴⁻¹⁻² are independently H or C₁-C₆ alkyl;
R¹⁵ is 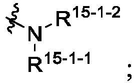
R¹⁵⁻¹⁻¹ and R¹⁵⁻¹⁻² are independently H or C₁-C₆ alkyl;
R¹⁶ is 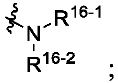
R¹⁶⁻¹ and R¹⁶⁻² are independently H or C₁-C₆ alkyl;
R¹⁷ is 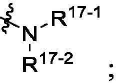
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 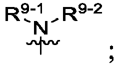
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 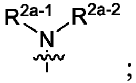
R^{2a-1} and R^{2a-2} are independently H, -C(=O)CH₃, C₁-C₃ alkyl or C₄-C₆ alkyl;
when ring 1 is independently pyridine, R^{B} is

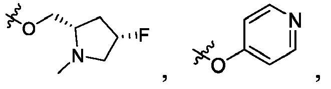

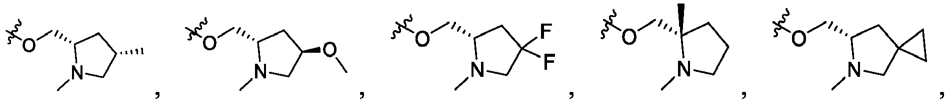

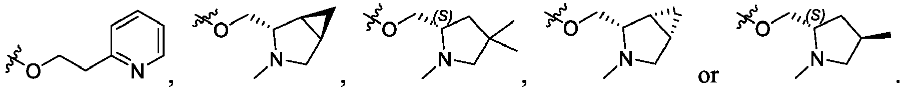

In a certain embodiment, R^{a} is 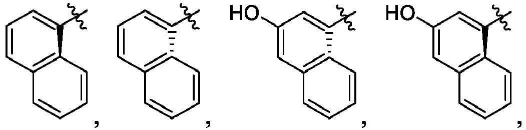 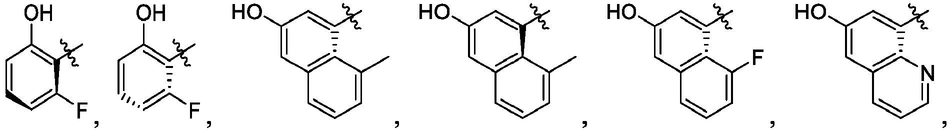 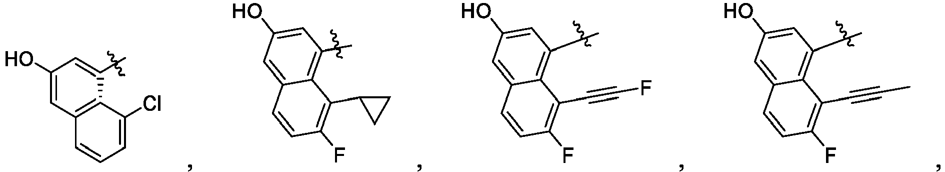 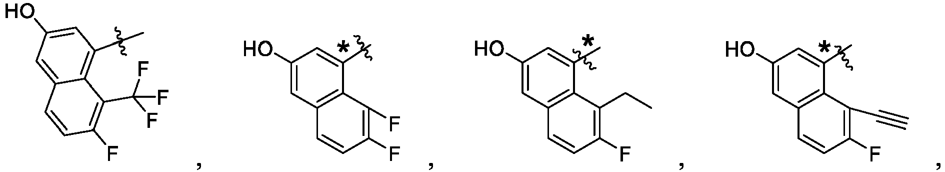 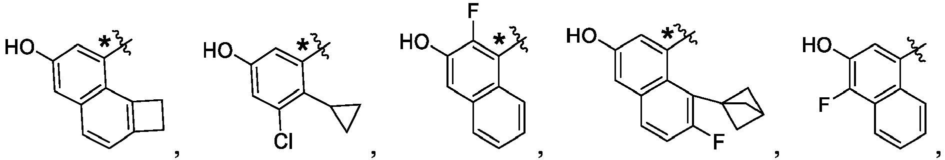 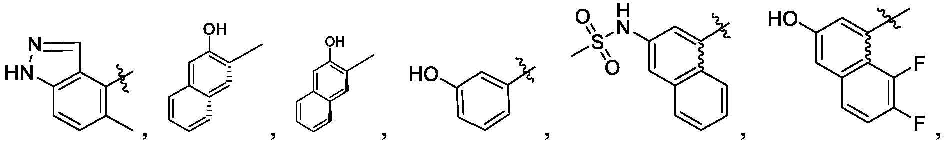 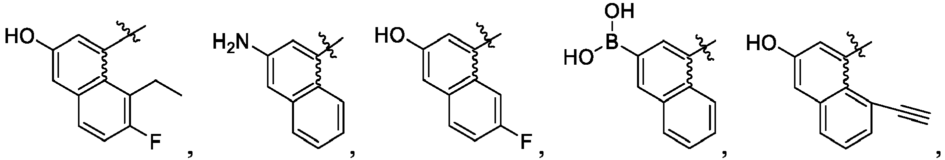 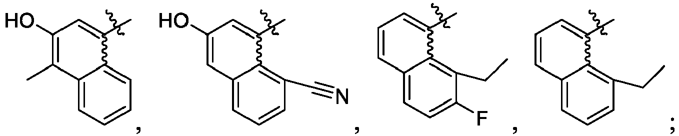 the carbon atom with "*" indicates a carbon atom with a chiral center; the " 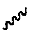 " indicates " 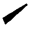 " " 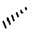 " or a mixture thereof.

In a certain embodiment, R^{a} is

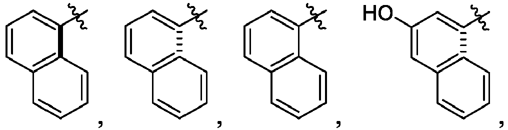

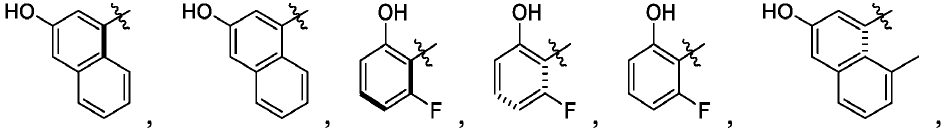

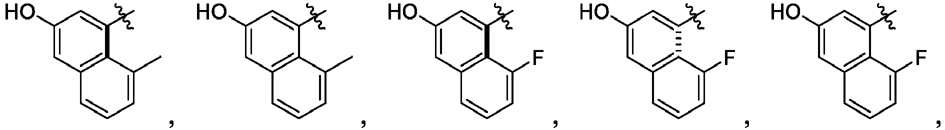

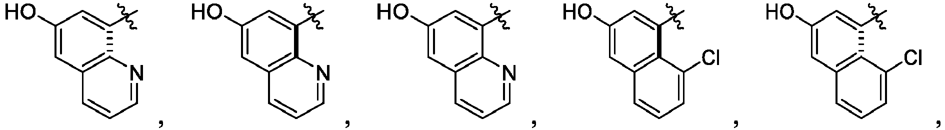

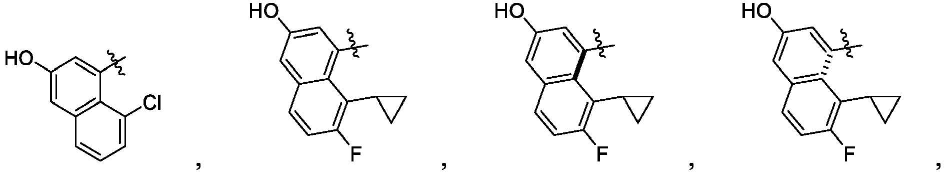

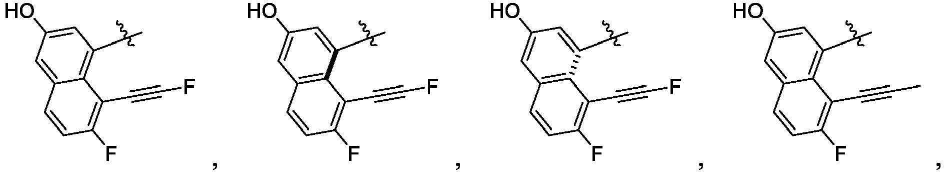

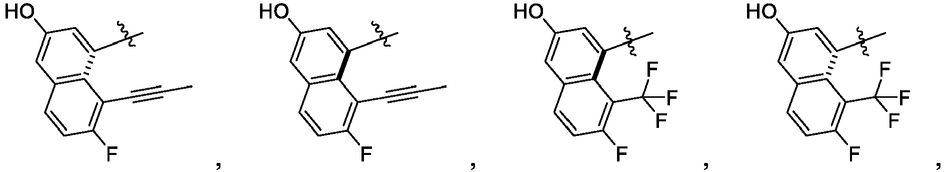

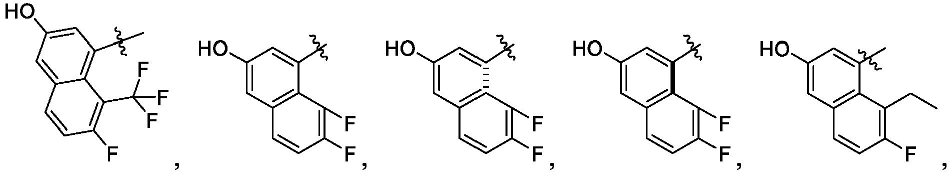

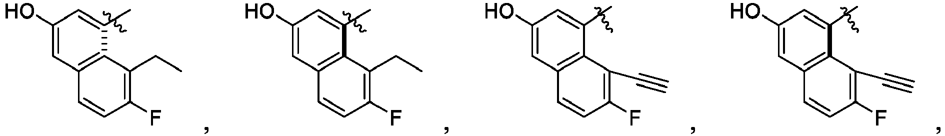

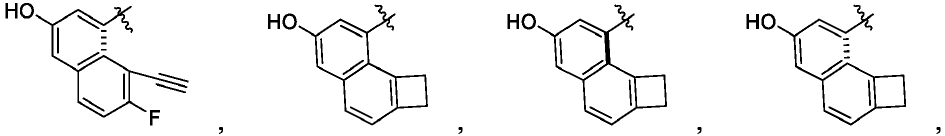

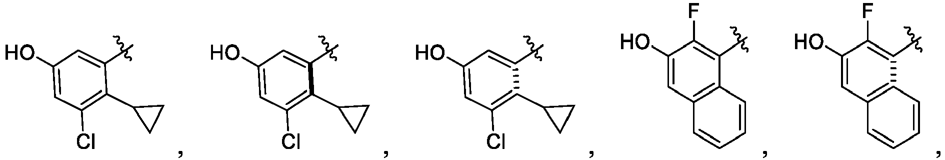

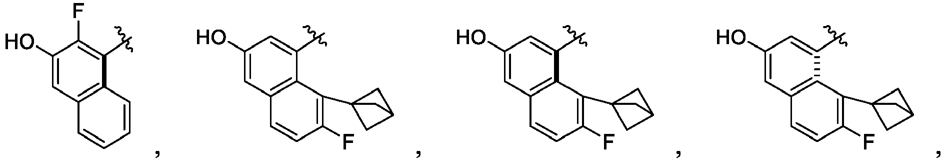

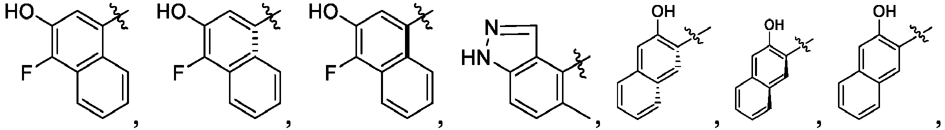

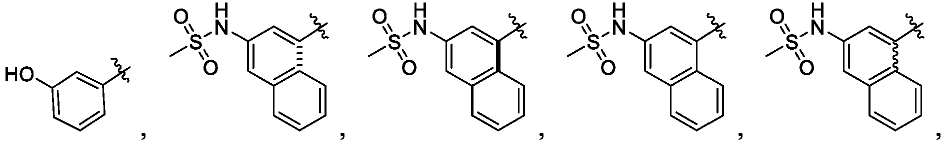

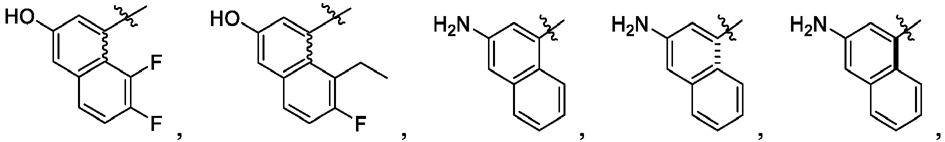

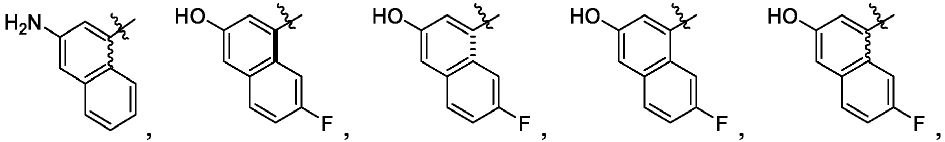

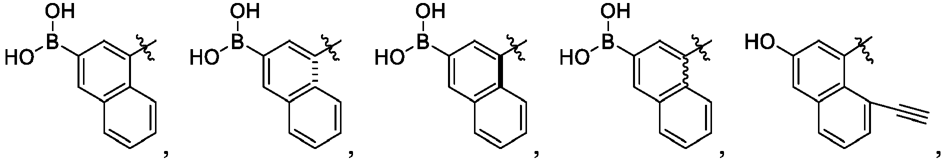

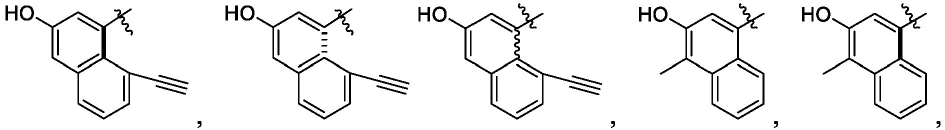

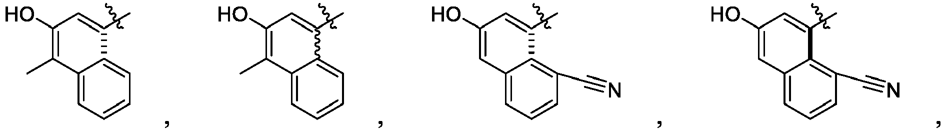

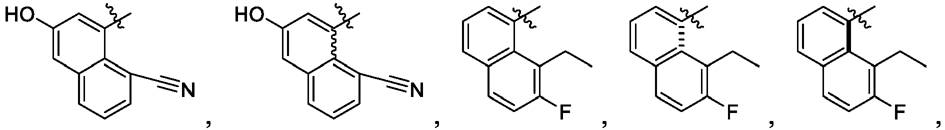

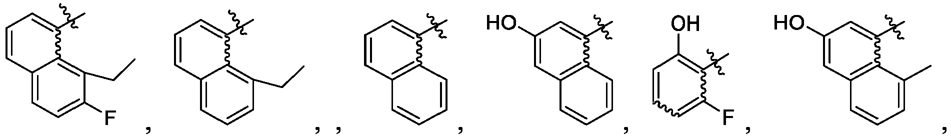

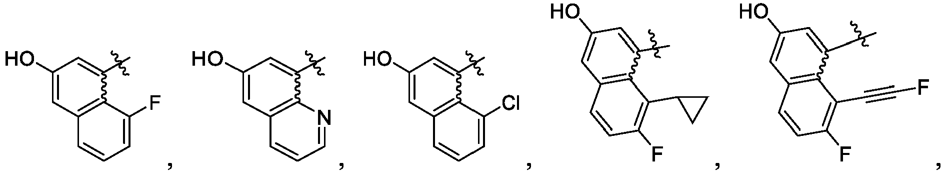

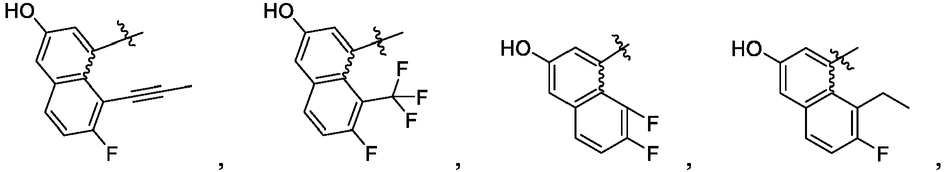

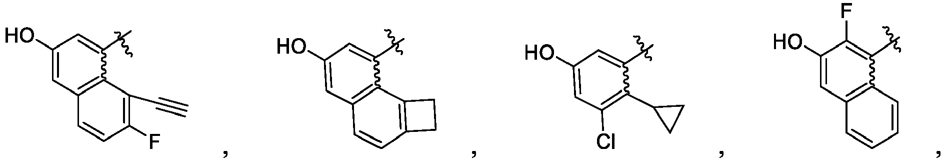

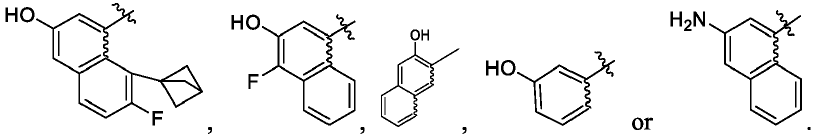

In a certain embodiment, in R^{A}, the halogen is preferably F or Cl.

In a certain embodiment, in R^{A}, the C₁-C₃ alkyl is preferably methyl.

In a certain embodiment, in the C₁-C₆ alkoxy substituted by R¹¹⁻³⁻², the C₁-C₆ alkoxy can be methoxy, ethoxy or propoxy, preferably propoxy.

In a certain embodiment, in R¹¹⁻³⁻², the C₂-C₆ alkenyl is vinyl, propylenyl, n-butylenyl or isobutylenyl, preferably vinyl.

In a certain embodiment, in R¹¹, the 4- to 10-membered heterocycloalkyl with 1 to 3 heteroatoms selected from N and O is preferably 4- to 8-membered monocyclic heterocycloalkyl with 1 to 2 heteroatoms selected from N and O (for example, 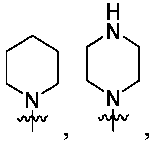 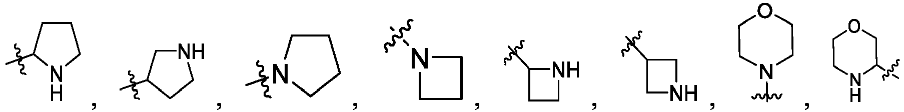 or 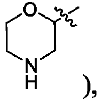 4- to 8-membered bridged heterocycloalkyl with 1 to 2 heteroatoms selected from N and O (for example, 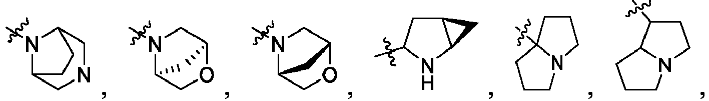 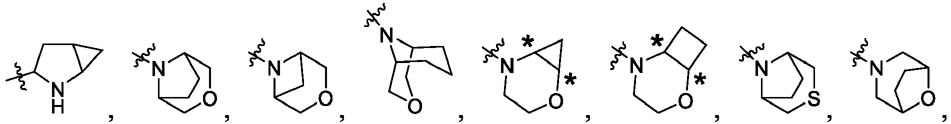 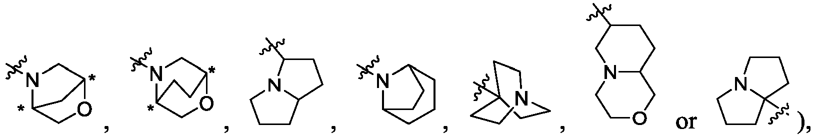 or 4- to 8-membered spiro heterocycloalkyl with 1 to 2 heteroatoms selected from N and O (for example, 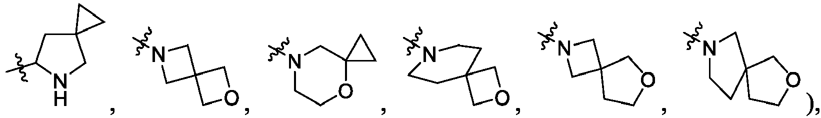 or 9- to 10-membered heterocycloalkyl with 1 to 2 heteroatoms selected from N and O (for example, 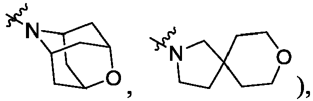 more preferably

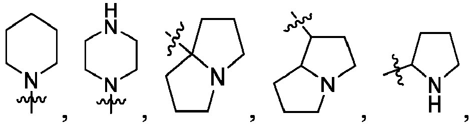

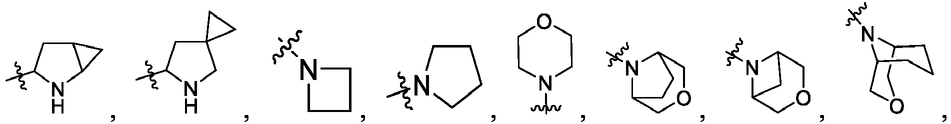

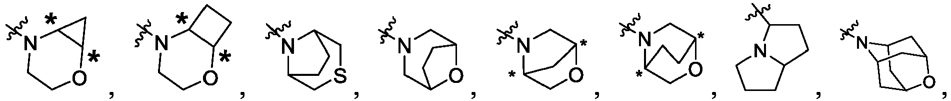

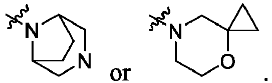

In a certain embodiment, in R¹¹, the 4- to 10-membered heterocycloalkyl with 1 to 3 heteroatoms selected from N and O is preferably 4- to 8-membered monocyclic heterocycloalkyl with 1 to 2 heteroatoms selected from N and O (for example, 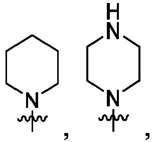 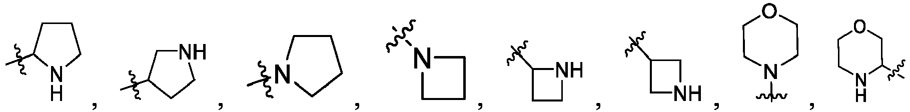 or 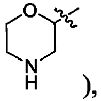 4- to 8-membered bridged heterocycloalkyl with 1 to 2 heteroatoms selected from N and O (for example, 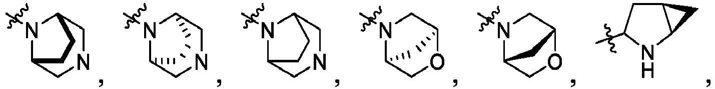 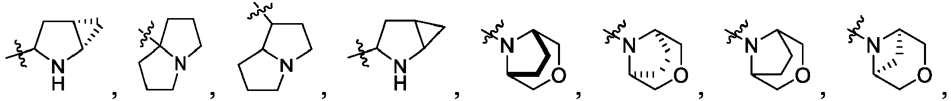 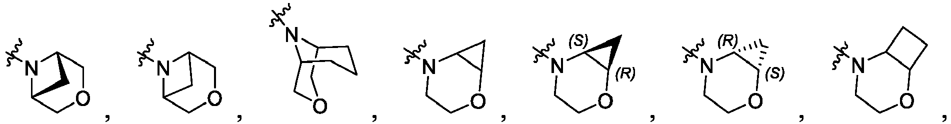 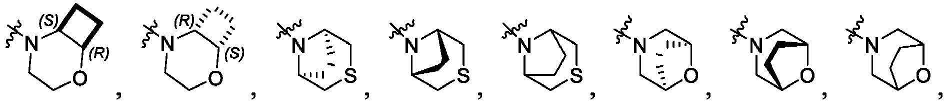 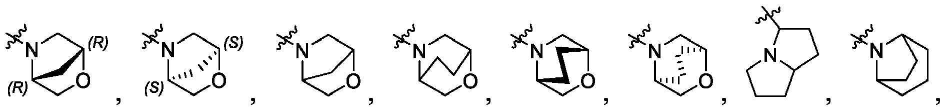 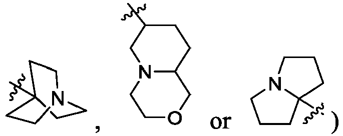 or 4- to 8-membered spiro heterocycloalkyl with 1 to 2 heteroatoms selected from N and O (for example, 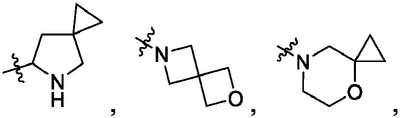 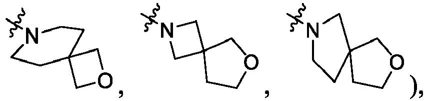 or 9- to 10-membered heterocycloalkyl with 1 to 2 heteroatoms selected from N and O (for example, 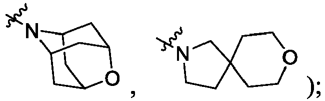 more preferably

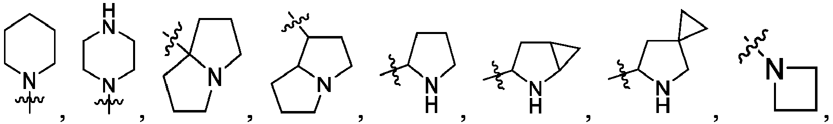

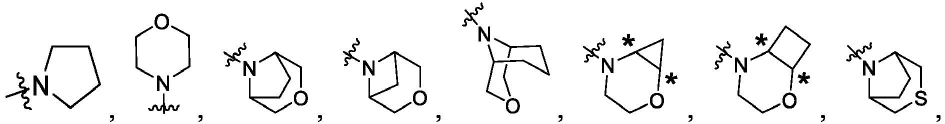

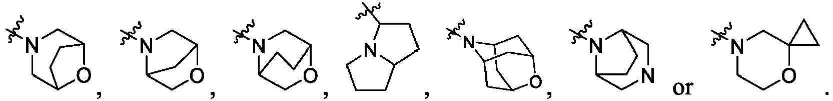

In a certain embodiment, the C₁-C₆ alkoxy substituted by R¹¹ can be

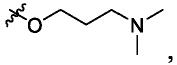

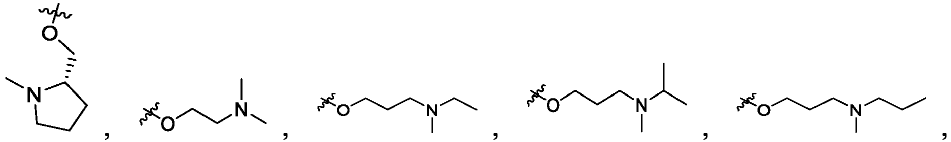

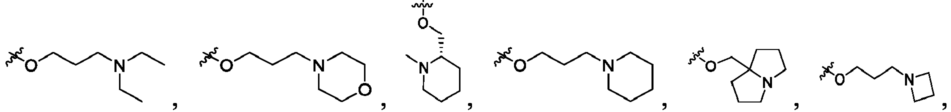

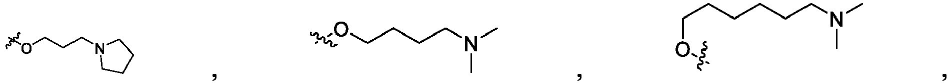

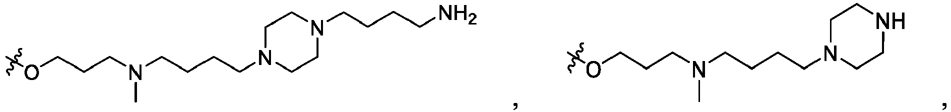

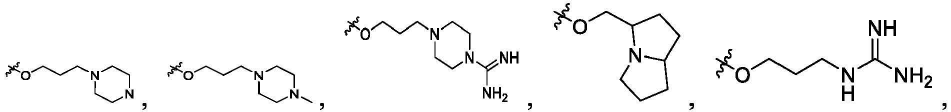

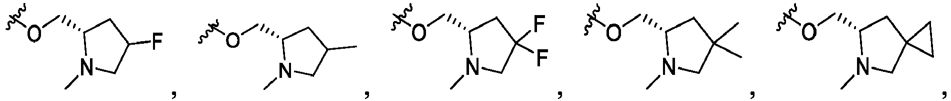

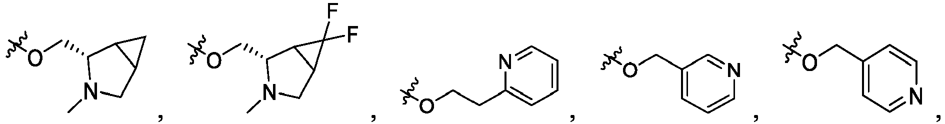

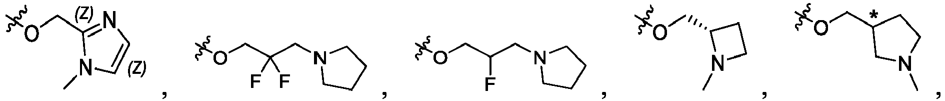

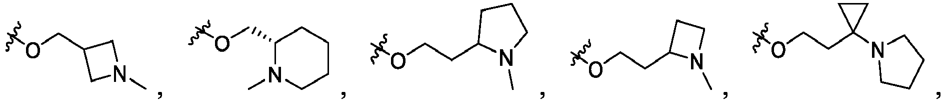

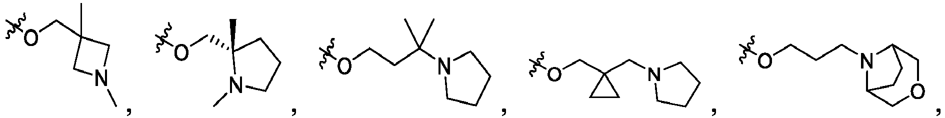

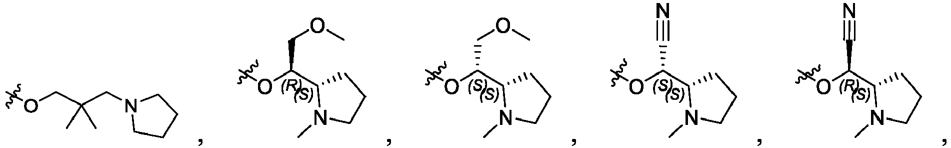

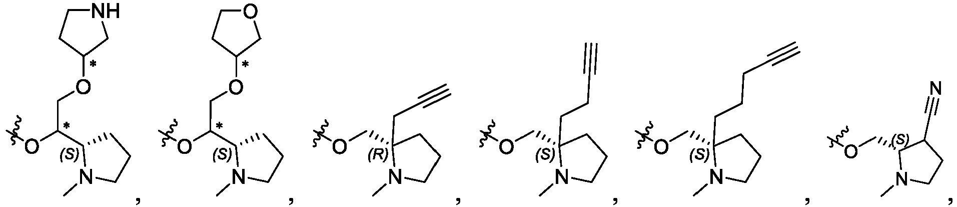

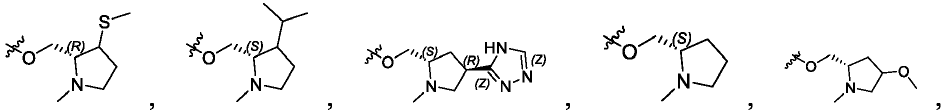

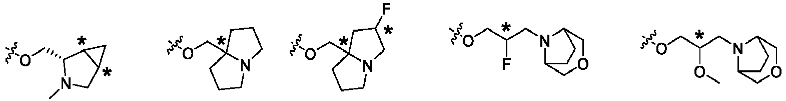

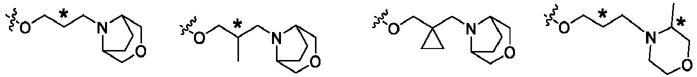

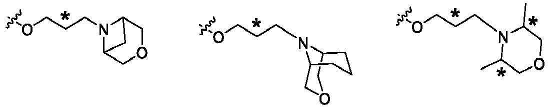

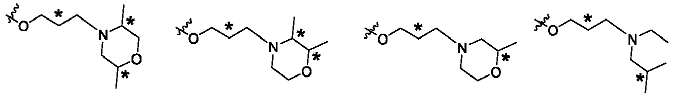

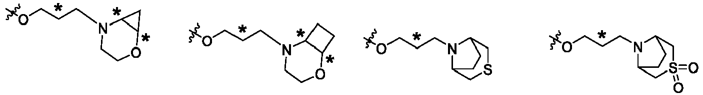

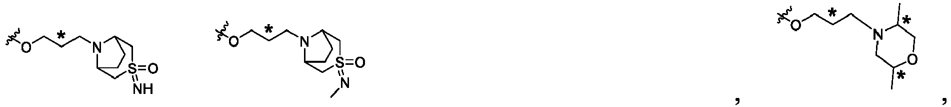

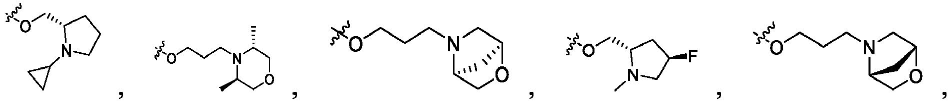

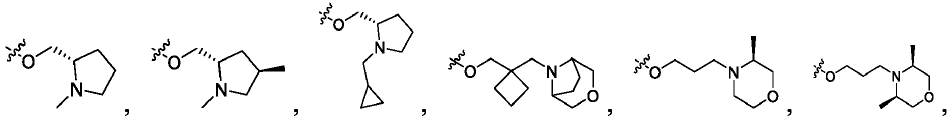

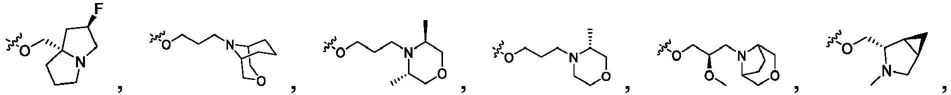

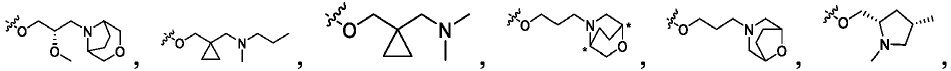

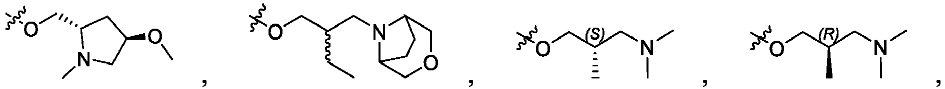

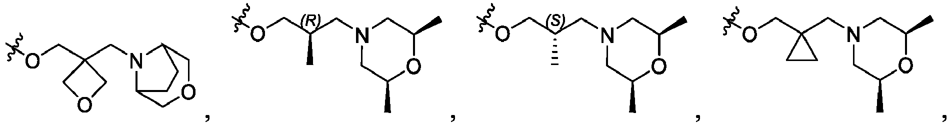

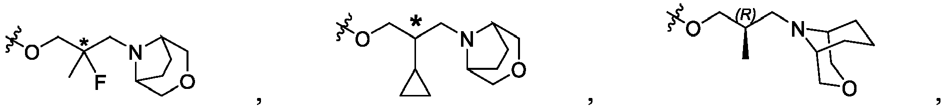

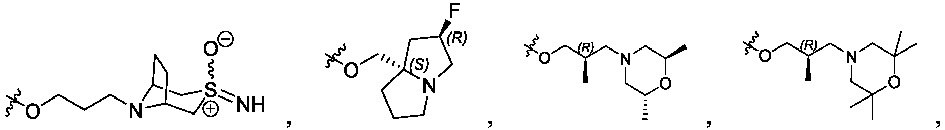

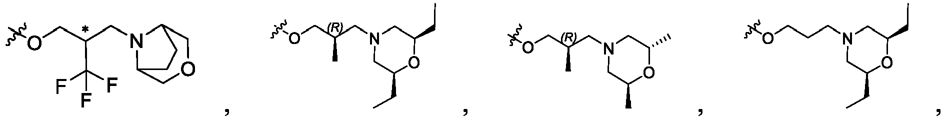

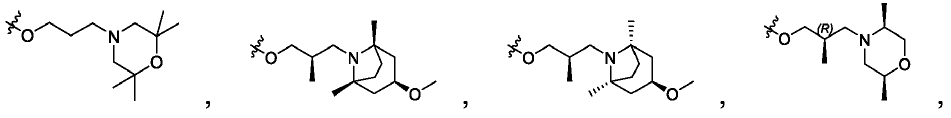

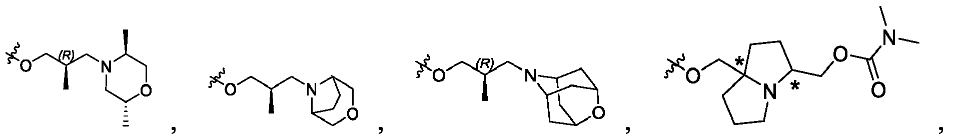

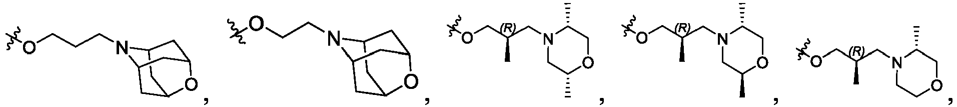

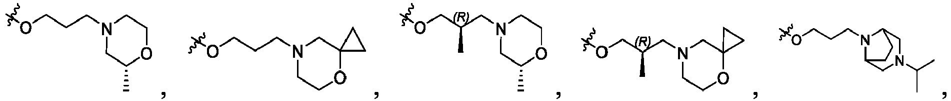

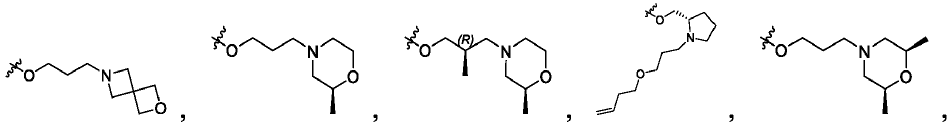

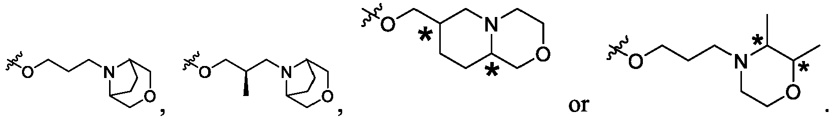

In a certain embodiment, Y¹ is 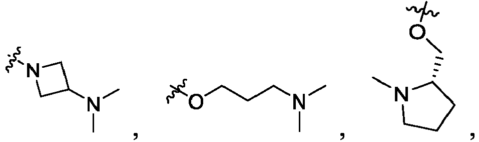 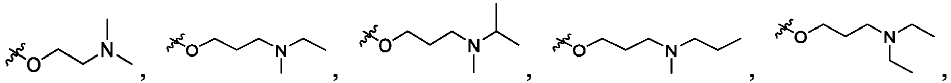 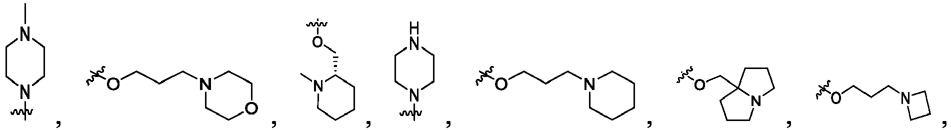 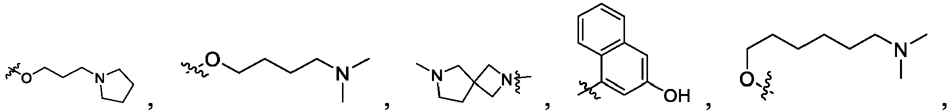 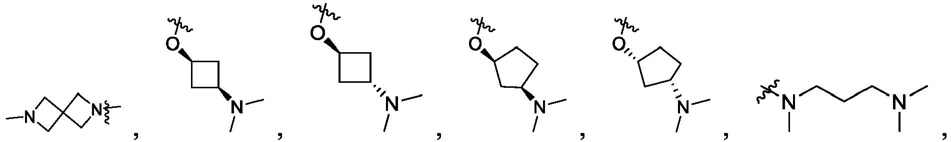 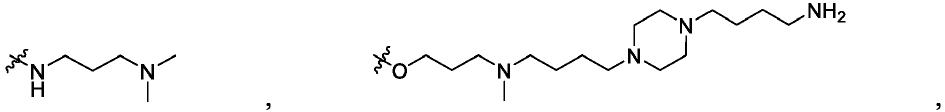 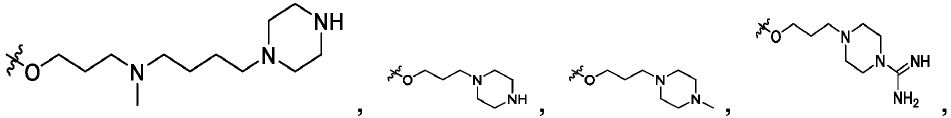 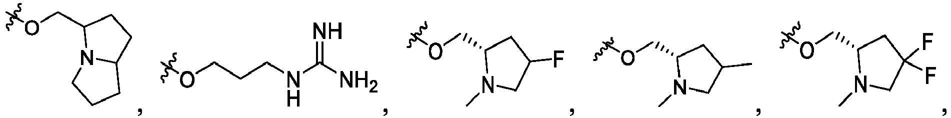 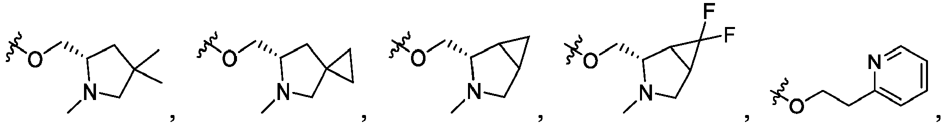 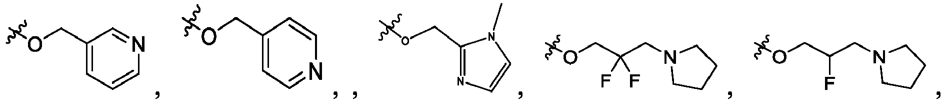 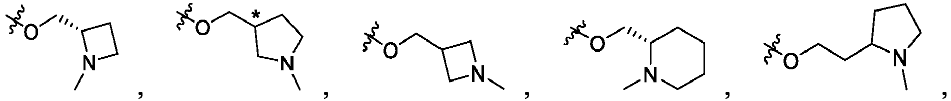 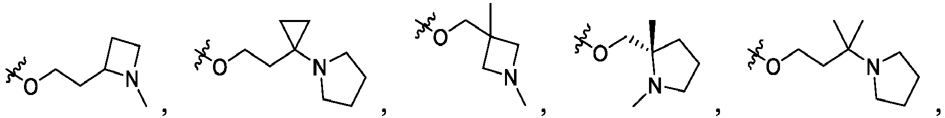 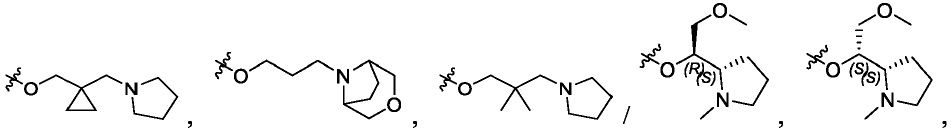 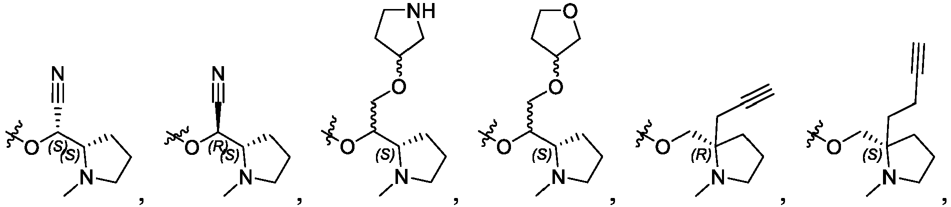 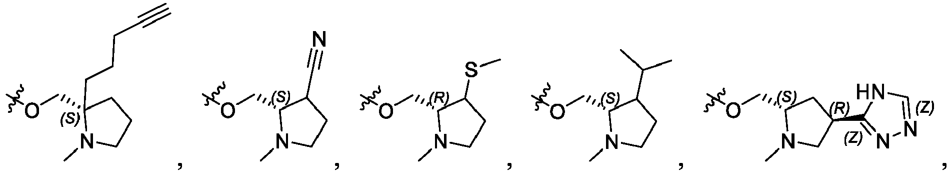 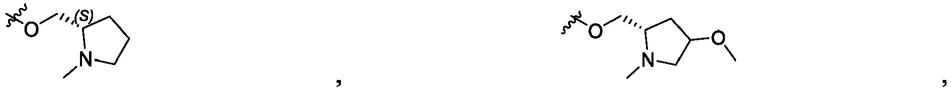 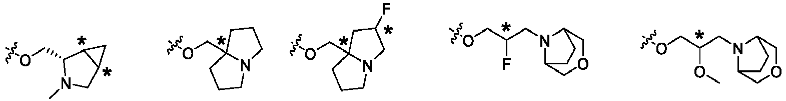 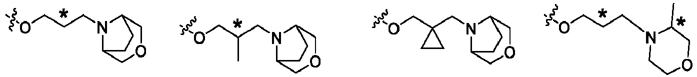 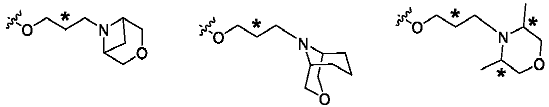 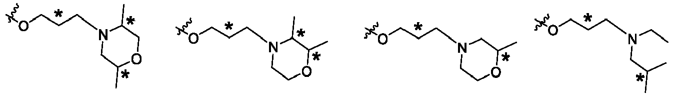 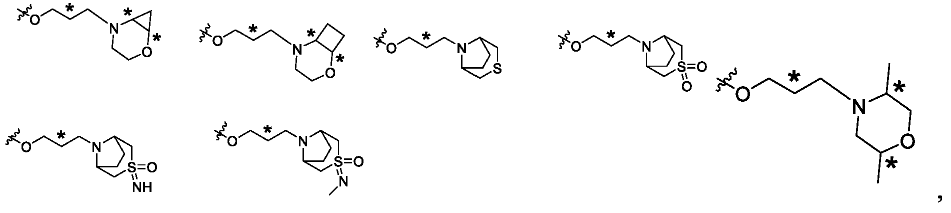 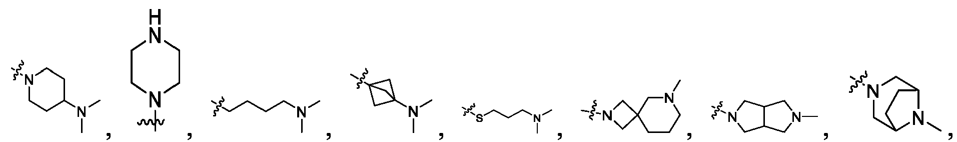 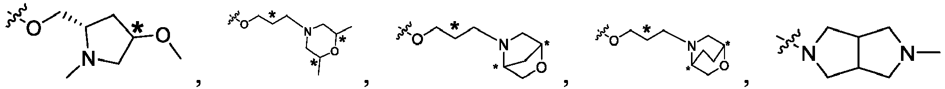 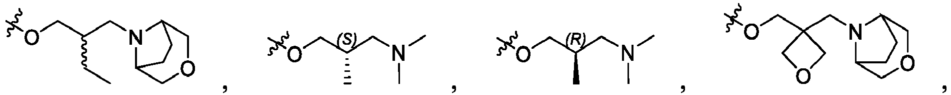 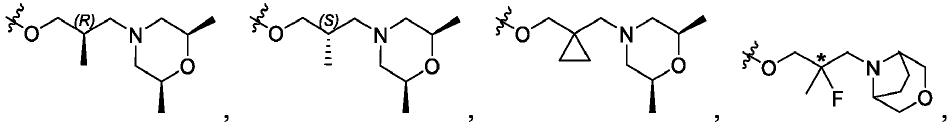 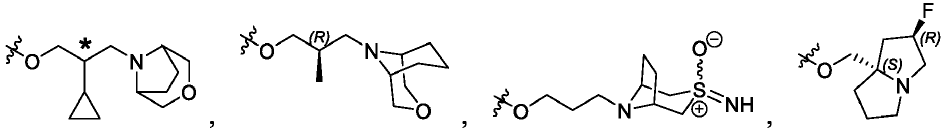 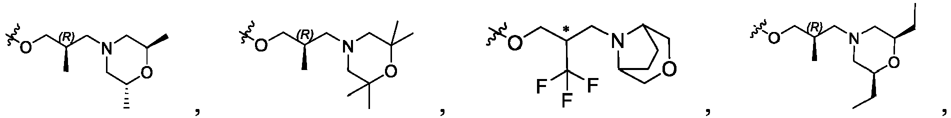 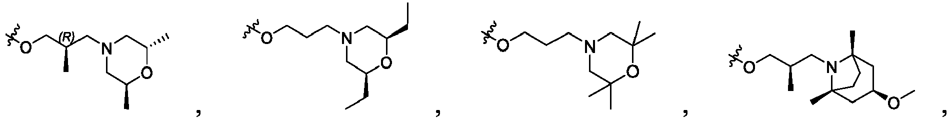 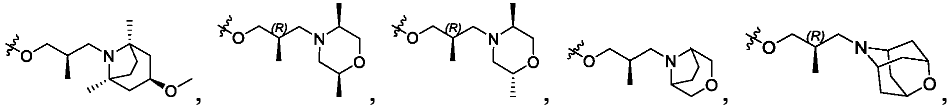 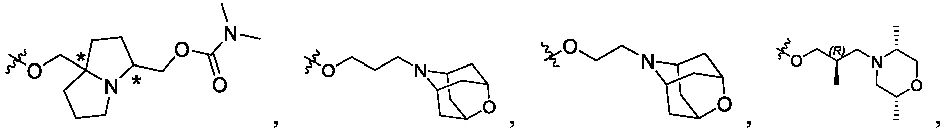 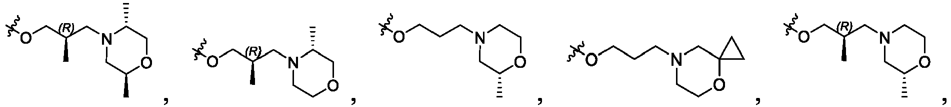 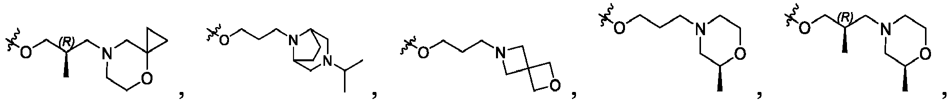 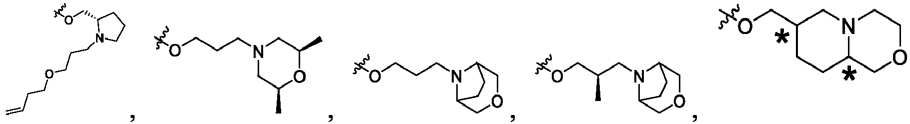 or

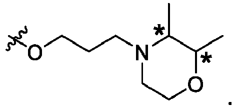

In a certain embodiment, the C₁-C₆ alkoxy substituted by R¹¹ can be 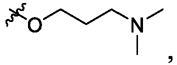 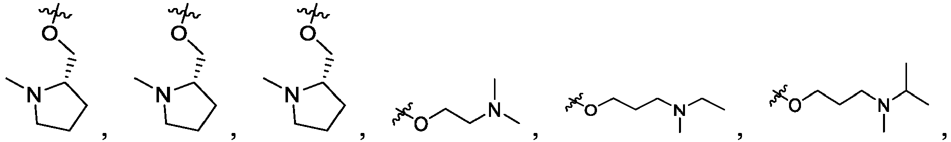 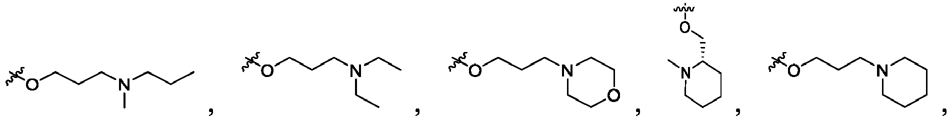 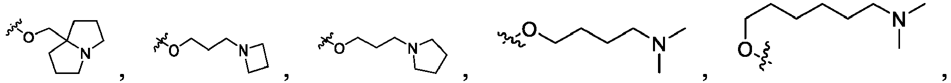 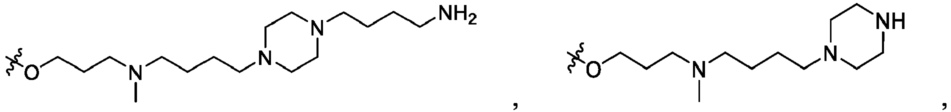 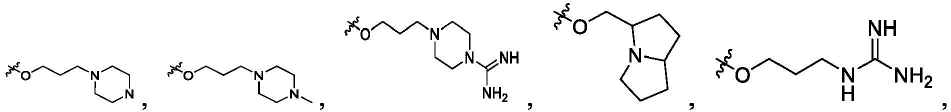 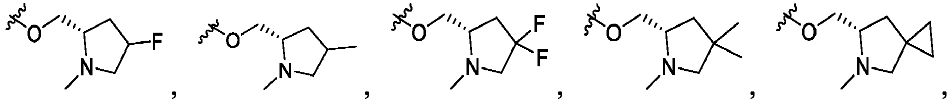 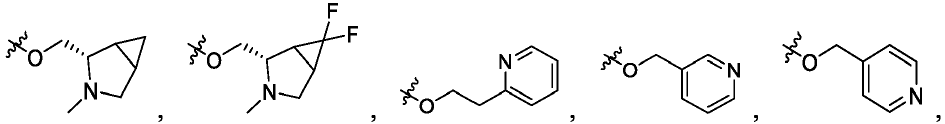 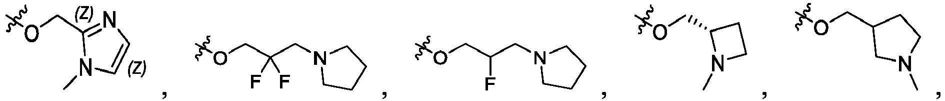 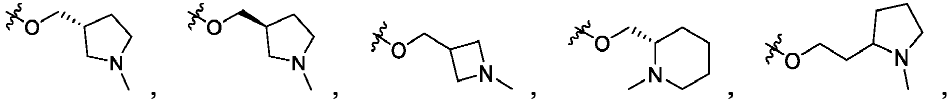 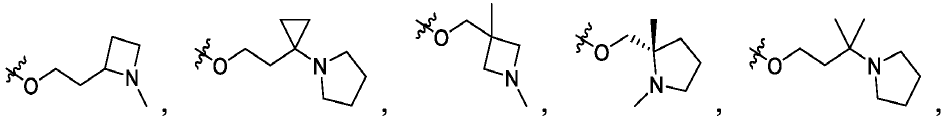 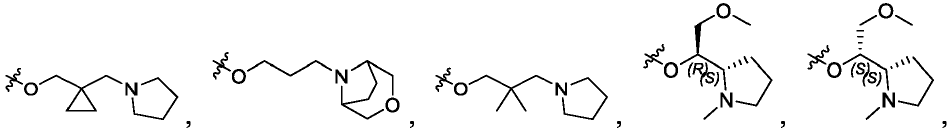 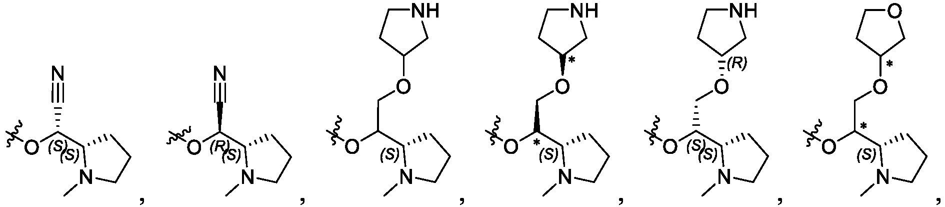 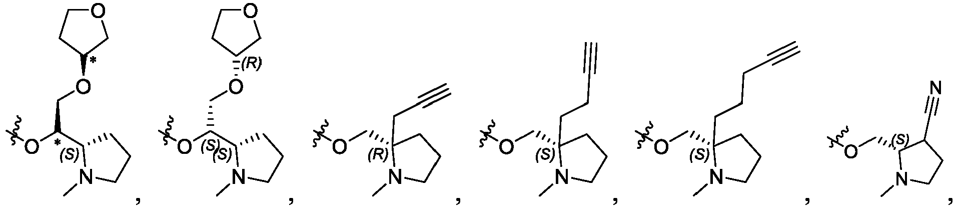 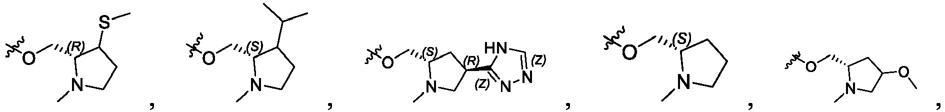 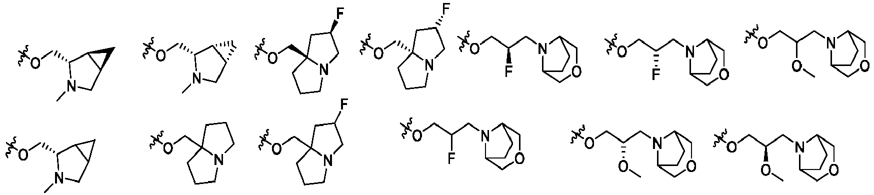 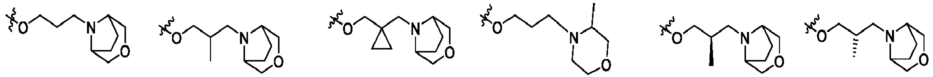 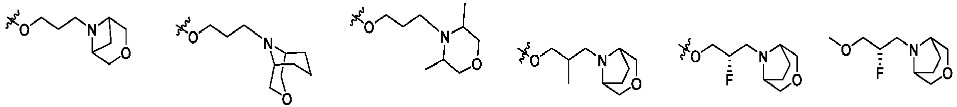 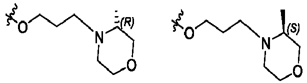 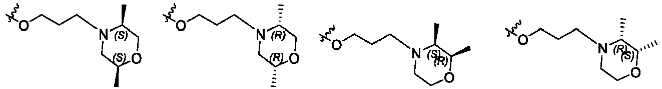 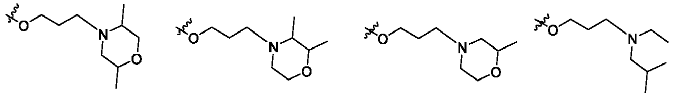 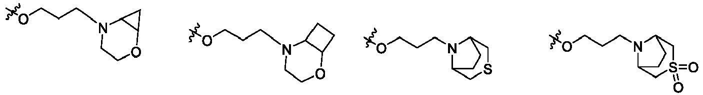 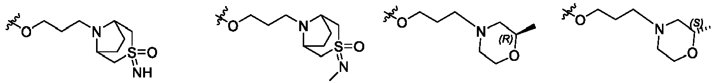 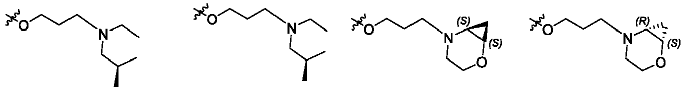 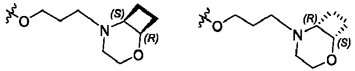 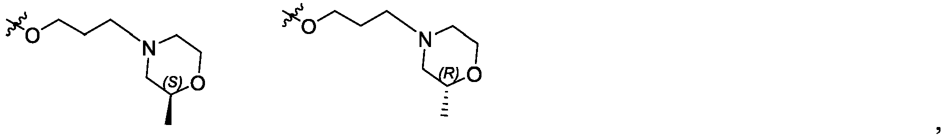 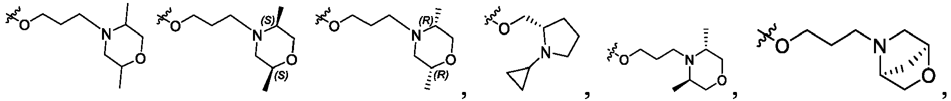 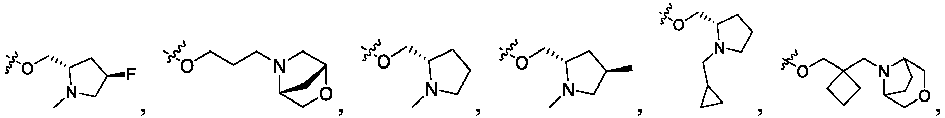 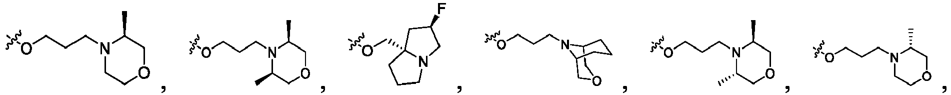 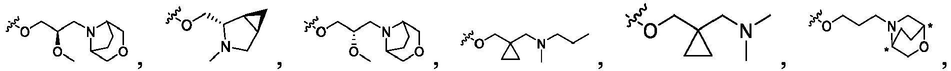 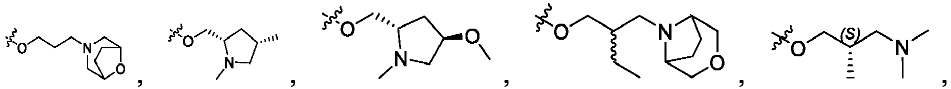 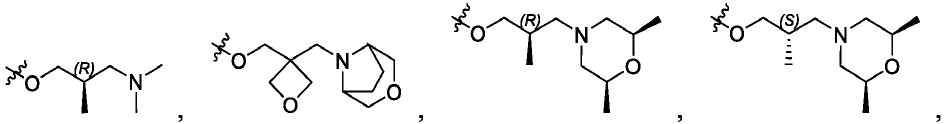 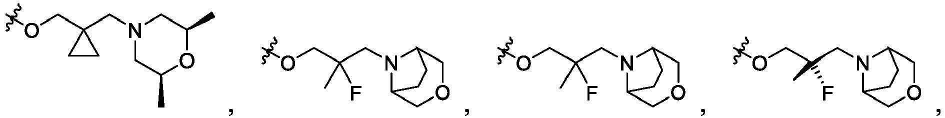 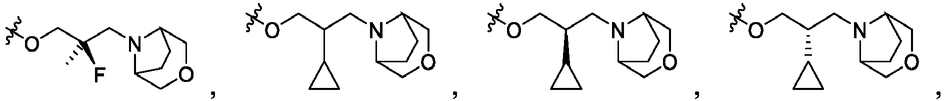 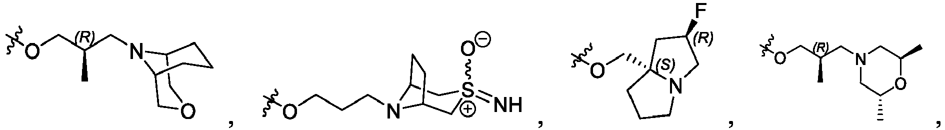 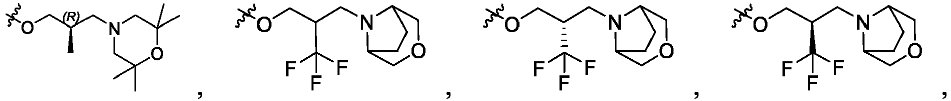 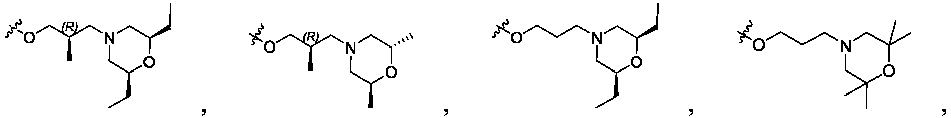 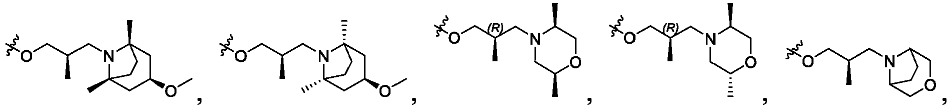 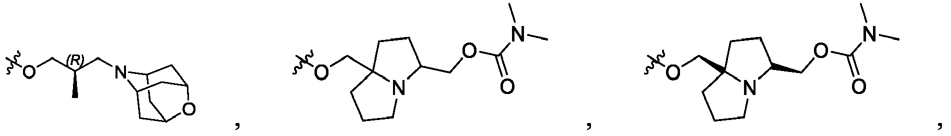 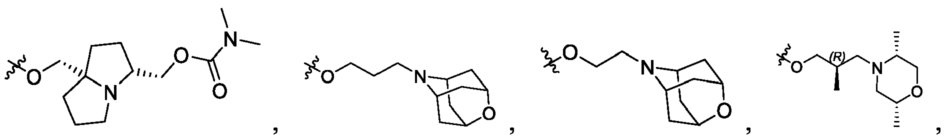 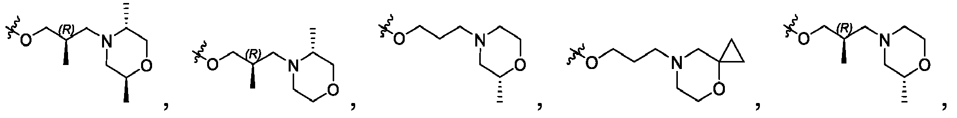 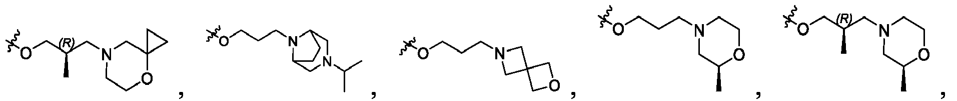 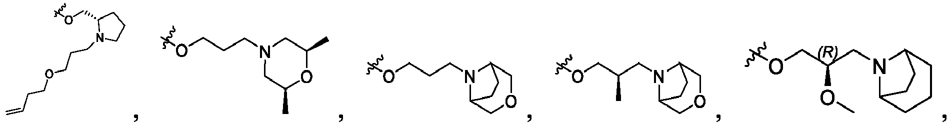 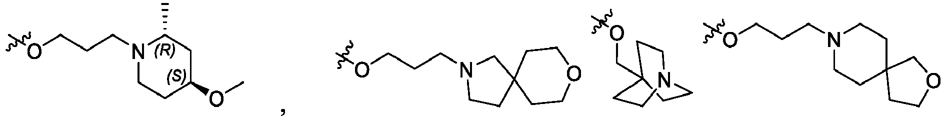 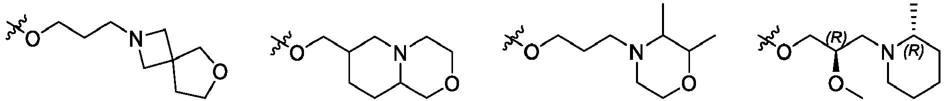 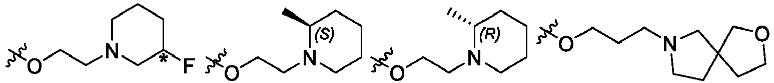 or

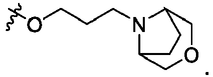

In a certain embodiment, Y¹ is 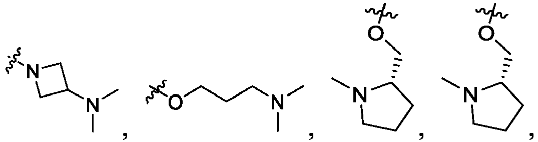 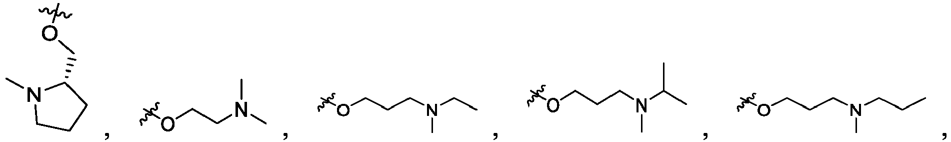 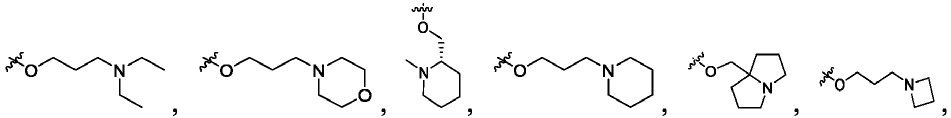 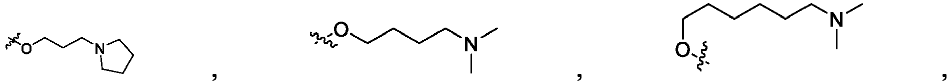 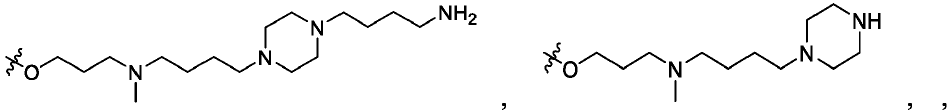 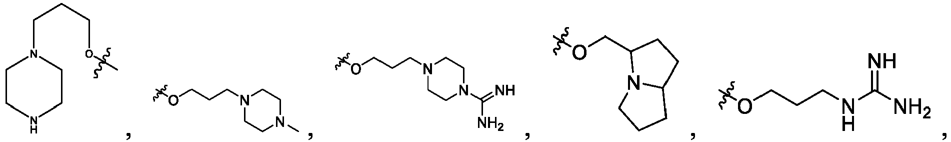 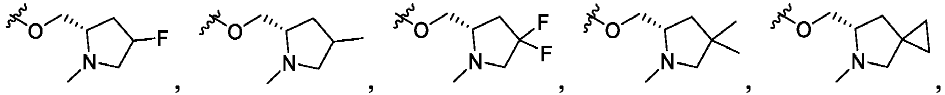 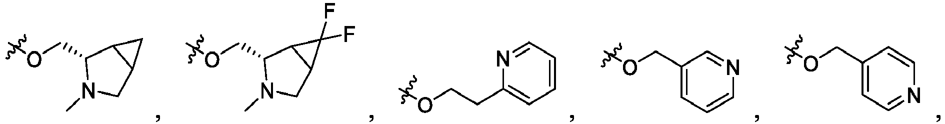 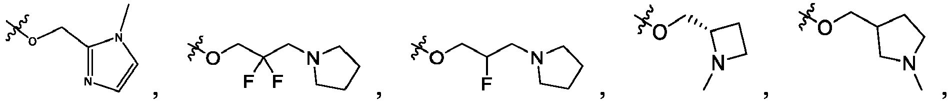 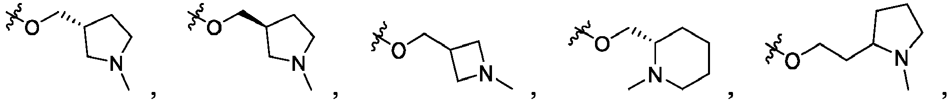 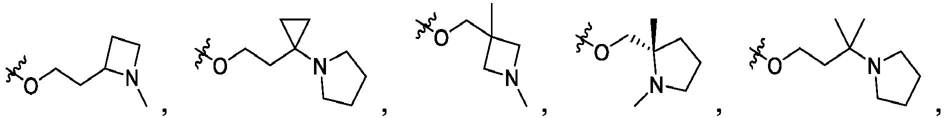 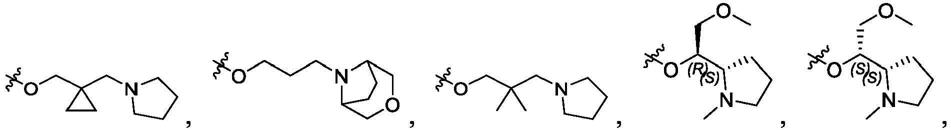 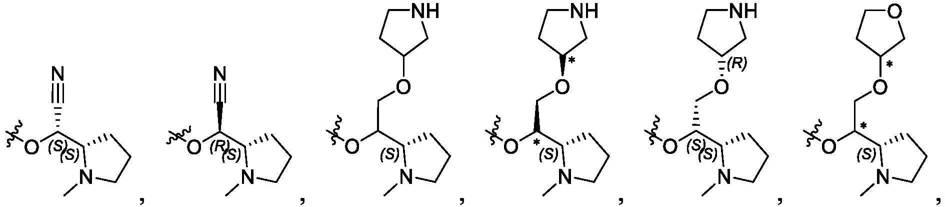 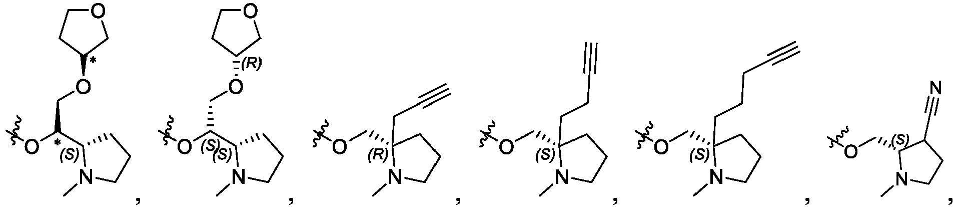 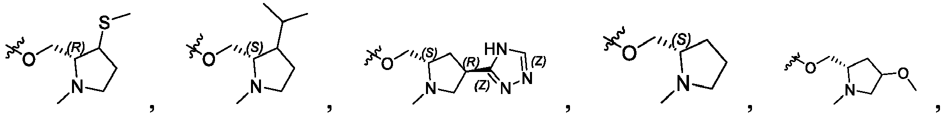 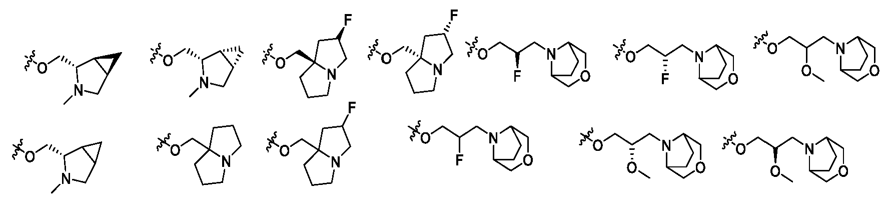 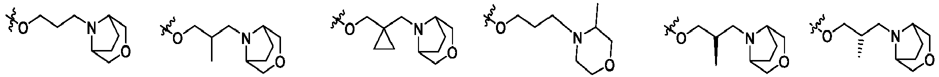 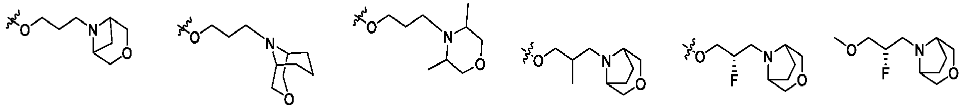 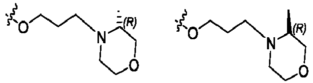 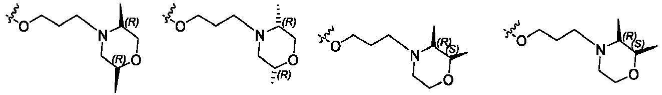 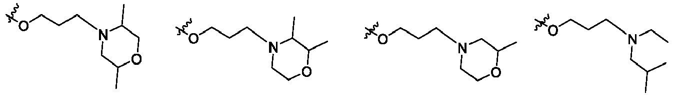 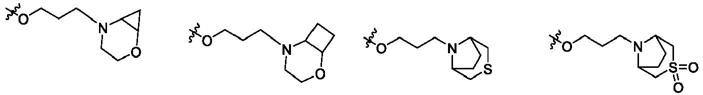 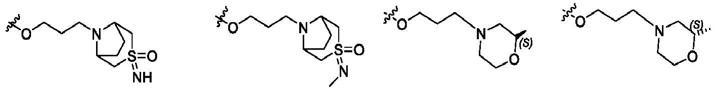 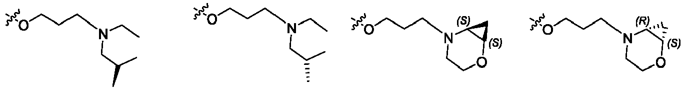 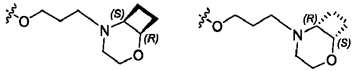 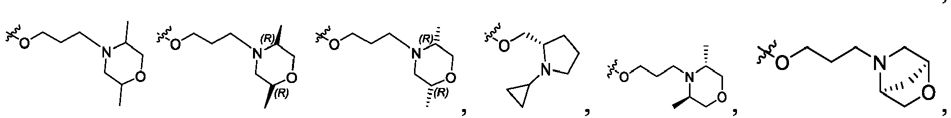 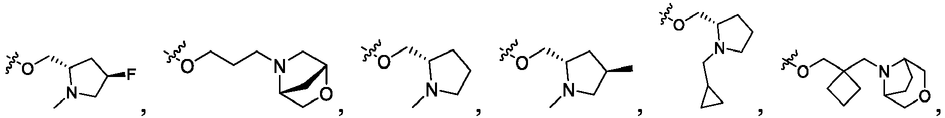 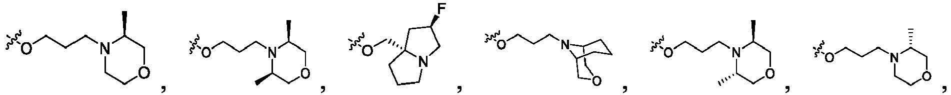 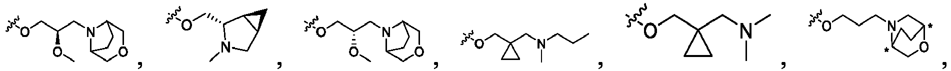 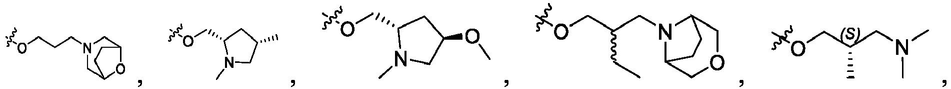 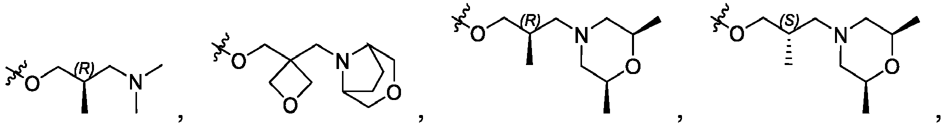 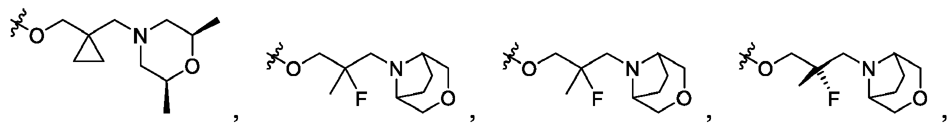 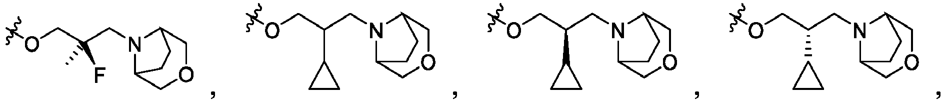 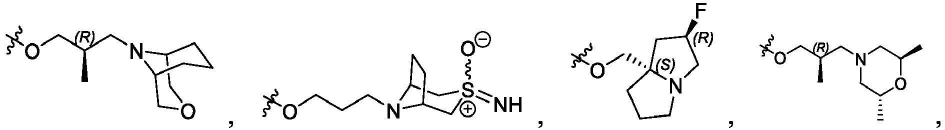 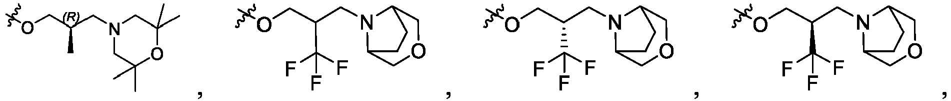 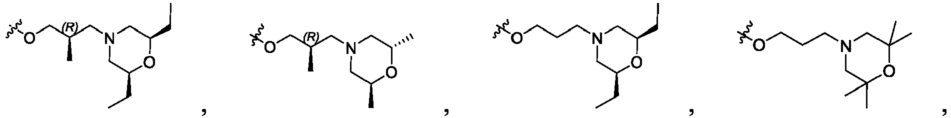 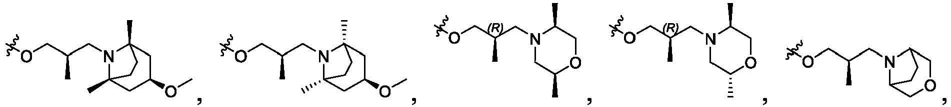 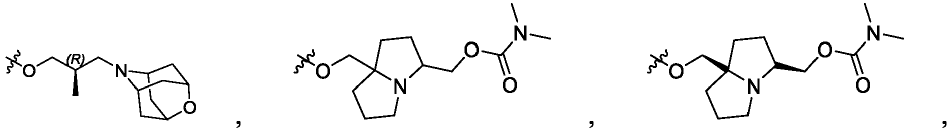 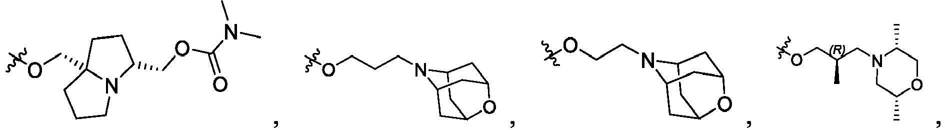 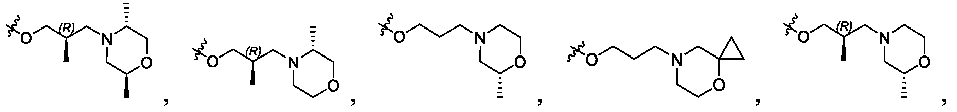 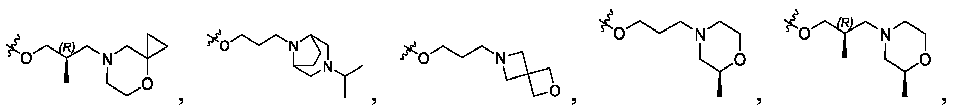 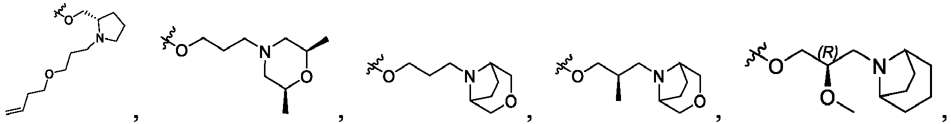 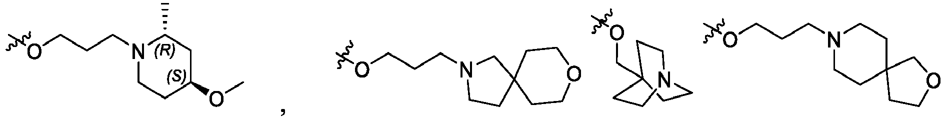 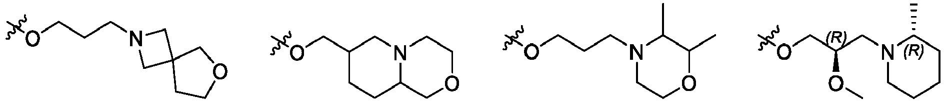 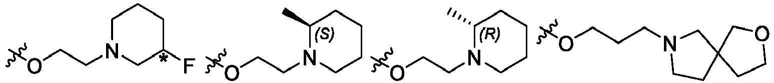 or

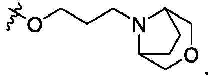

In a certain embodiment, the quinazoline compound represented by formula I, the pharmaceutically acceptable salt thereof, the solvate thereof, the prodrug thereof, the metabolite thereof or the isotopic compound thereof, 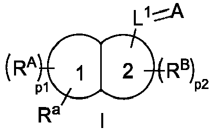
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 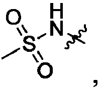 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6-to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 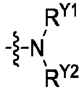 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 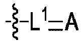
is 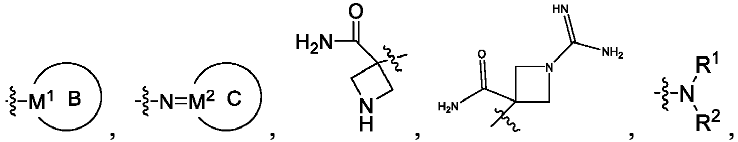 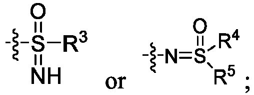
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 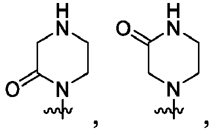 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 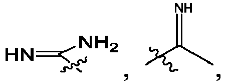 C₁-C₆ alkyl, C₁-C₃ alkyl substituted by CN, or C₄-C₆ alkyl substituted by CN; m is 0, 1 or 2; n is 1, 2 or 3;
R¹⁰ is amino or 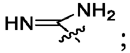
R¹¹ is independently halogen, 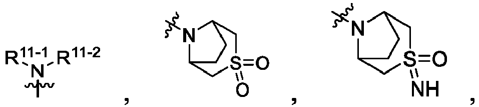 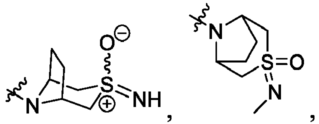 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 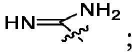
R¹¹⁻³ is H, 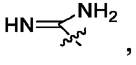 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 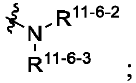
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, unsubstituted 4- to 10-membered heterocycloalkyl or 4-to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R^{L} is H or 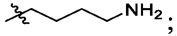
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2}.
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 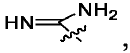 or C₃-C₆ cycloalkyl substituted by R¹³⁻¹;
R¹³⁻¹ is 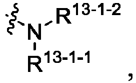 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 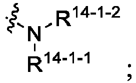
R¹⁴⁻¹⁻¹ and R¹⁴⁻¹⁻² are independently H or C₁-C₆ alkyl;
R¹⁵ is 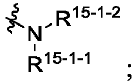
R¹⁵⁻¹⁻¹ and R¹⁵⁻¹⁻² are independently H or C₁-C₆ alkyl;
R¹⁶ is 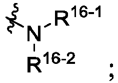
R¹⁶⁻¹ and R¹⁶⁻² are independently H or C₁-C₆ alkyl;
R¹⁷ is 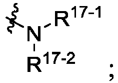
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 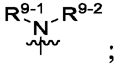
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 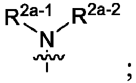
R^{2a-1} and R^{2a-2} are independently H, -C(=O)CH₃, C₁-C₃ alkyl or C₄-C₆ alkyl;
when ring 1 is independently pyridine, R^{B} is

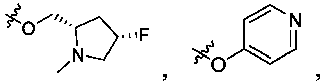

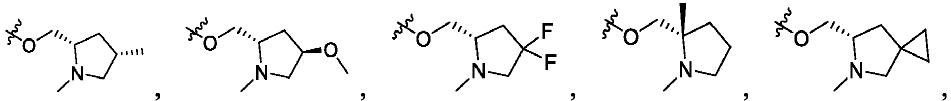

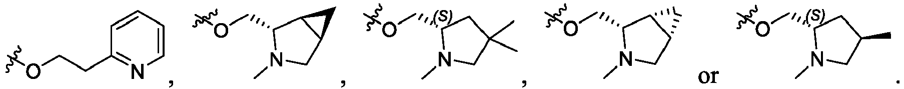

In a certain embodiment, the quinazoline compound represented by formula I is a compound represented by formula I-CI, 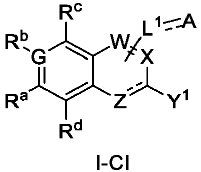
wherein R^{b}, R^{c} and R^{d} are independently H or halogen;
W is C;
Gis C or N;
X and Z are N;
" 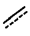 " in 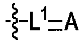 is a double bond;
" 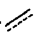 " in 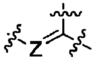 is a double bond;
Y¹ is C₁-C₆ alkoxy substituted by one or more R¹¹;
R¹¹ is independently 4- to 10-membered heterocycloalkyl substituted by one or more R¹¹⁻³ with 1 to 3 heteroatoms selected from N and O;
R¹¹⁻³ is C₁-C₆ alkyl substituted by R¹¹⁻³⁻¹;
R¹¹⁻³⁻¹ is 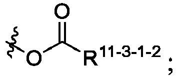
R¹¹⁻³⁻¹⁻² is independently 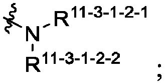
R¹¹⁻³⁻¹⁻²⁻¹ and R¹¹⁻³⁻¹⁻²⁻² are independently H or C₁-C₆ alkyl.

In a certain embodiment, R¹¹⁻³⁻¹⁻²⁻¹ and R¹¹⁻³⁻¹⁻²⁻² are independently H, methyl, ethyl, propyl, n-butyl or *tert*-butyl*.*

In a certain embodiment, when Y¹ is C₁-C₆ alkoxy substituted by R¹¹, the C₁-C₆ alkoxy can be methoxy, ethoxy, n-propoxy, isopropoxy, n-butoxy, isobutoxy, sec-butoxy, n-pentyloxy, isopentyloxy, neopentyloxy, n-hexyloxy, isohexyloxy, neohexyloxy, 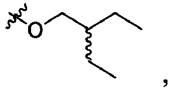 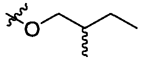 (preferably 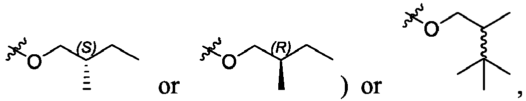 for example, methoxy, n-propoxy, ethoxy, n-butoxy, isobutoxy, isopentyloxy, neopentyloxy, n- hexyloxy,

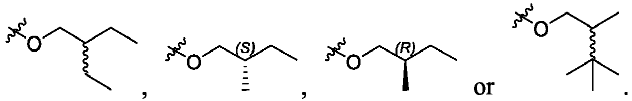

In a certain embodiment of the present disclosure, the quinazoline compound represented by formula I, the pharmaceutically acceptable salt thereof, the solvate thereof, the prodrug thereof, the metabolite thereof or the isotopic compound thereof, 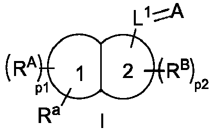
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 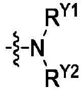 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 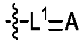
is 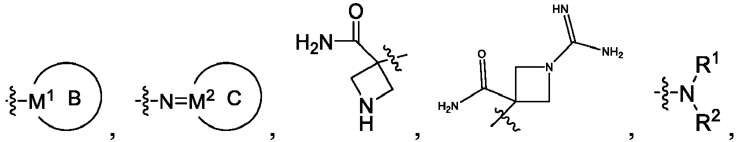 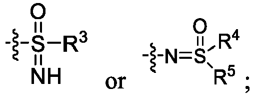
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 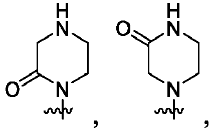 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 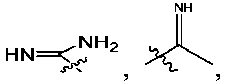 C₁-C₆ alkyl, C₁-C₃ alkyl substituted by CN, or C₄-C₆ alkyl substituted by CN; m is 0, 1 or 2; n is 1, 2 or 3;
R¹⁰ is amino or 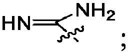
R¹¹ is independently halogen, 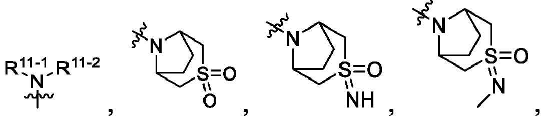 cyano, C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 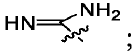
R¹¹⁻³ is H, 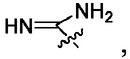 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is 4- to 10-membered heterocycloalkyl with 1 to 3 heteroatoms selected from N and O, or 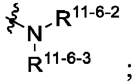
R¹¹⁻⁶⁻² and R¹¹⁻⁶⁻³ are independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl;
R^{L} is H or 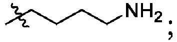
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2}.
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 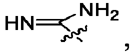 or C₃-C₆ cycloalkyl substituted by R¹³⁻¹;
R¹³⁻¹ is 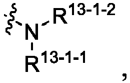 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 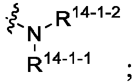
R¹⁴⁻¹⁻¹ and R¹⁴⁻¹⁻² are independently H or C₁-C₆ alkyl;
R¹⁵ is 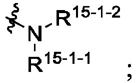
R¹⁵⁻¹⁻¹ and R¹⁵⁻¹⁻² are independently H or C₁-C₆ alkyl;
R¹⁶ is 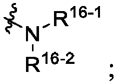
R¹⁶⁻¹ and R¹⁶⁻² are independently H or C₁-C₆ alkyl;
R¹⁷ is 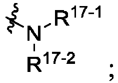
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 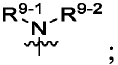
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 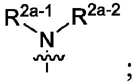
R^{2a-1} and R^{2a-2} are independently H, -C(=O)CH₃, C₁-C₃ alkyl or C₄-C₆ alkyl;
when ring 1 is independently pyridine, R^{B} is

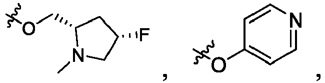

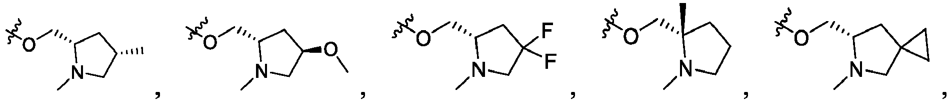

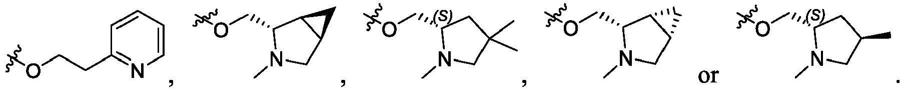

In a certain embodiment of the present disclosure, the quinazoline compound represented by formula I, the pharmaceutically acceptable salt thereof, the solvate thereof, the prodrug thereof, the metabolite thereof or the isotopic compound thereof, 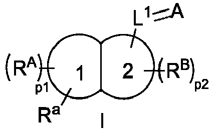
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 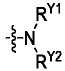 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 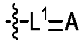
is 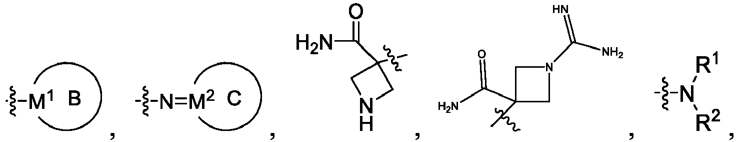 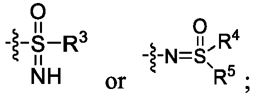
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 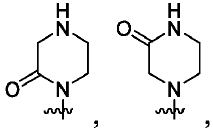 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 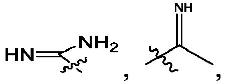 C₁-C₆ alkyl, C₁-C₃ alkyl substituted by CN, or C₄-C₆ alkyl substituted by CN; m is 0, 1 or 2; n is 1, 2 or 3;
R¹⁰ is amino or 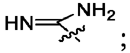
R¹¹ is independently halogen, 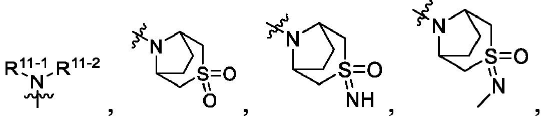 cyano, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4-to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 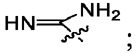
R¹¹⁻³ is H, 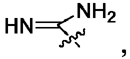 cyano, C₂-C₆ alkynyl, C₁-C₆ alkoxy, halogen, unsubstituted 4- to 10-membered heterocycloalkyl or 4- to 10-membered heterocycloalkyl substituted by C₁-C₆, or C₁-C₆ alkyl;
R¹¹⁻⁴ is C₁-C₆ alkyl;
R¹¹⁻¹⁻¹ is -OH, 4- to 10-membered heterocycloalkyl substituted by R^{L} with 1 to 3 heteroatoms selected from N and O;
R^{L} is H or 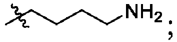
R^{a-1} is independently hydroxyl, halogen, C₁-C₆ alkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 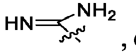 or C₃-C₆ cycloalkyl substituted by R¹³⁻¹;
R¹³⁻¹ is 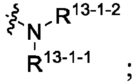
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 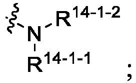
R¹⁴⁻¹⁻¹ and R¹⁴⁻¹⁻² are independently H or C₁-C₆ alkyl;
R¹⁵ is 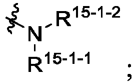
R¹⁵⁻¹⁻¹ and R¹⁵⁻¹⁻² are independently H or C₁-C₆ alkyl;
R¹⁶ is 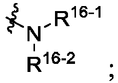
R¹⁶⁻¹ and R¹⁶⁻² are independently H or C₁-C₆ alkyl;
R¹⁷ is 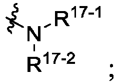
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 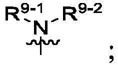
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 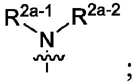
R^{2a-1} and R^{2a-2} are independently H, -C(=O)CH₃, C₁-C₃ alkyl or C₄-C₆ alkyl;
when ring 1 is independently pyridine, R^{B} is

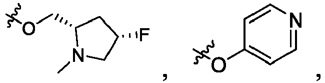

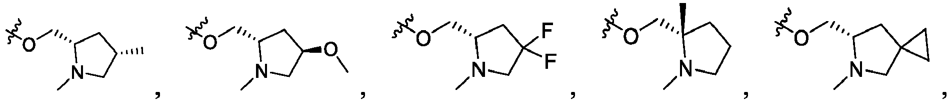

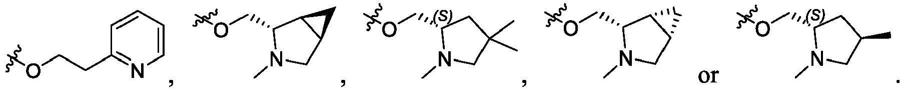

In a certain embodiment, the quinazoline compound represented by formula I is, 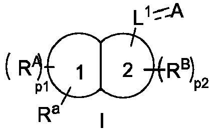
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 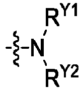 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 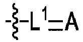
is 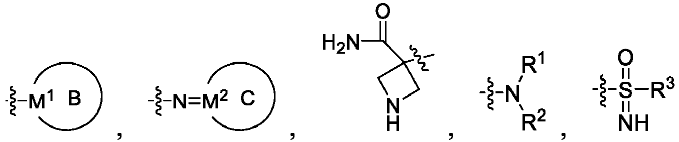 or 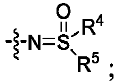
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 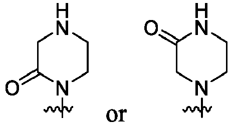 ; one N atom of the 7- to 12-membered heterocycloalkyl is attached to ring 2;
R⁷ is -C(=O)O-(C₁-C₄ alkyl), 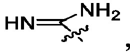 C₁-C₆ alkyl, C₁-C₃ alkyl substituted by CN, or C₄-C₆ alkyl substituted by CN;
R¹⁰ is amino or 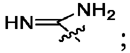
R¹¹ is independently halogen, 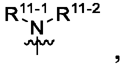 unsubstituted 4- to 10-membered heterocycloalkyl or 4- to 10-membered heterocycloalkyl substituted by one or more R¹¹⁻³ with 1 to 3 heteroatoms selected from N and O, or unsubstituted 5- to 6-membered heteroaryl or 5-to 6-membered heteroaryl substituted by one or more R¹¹⁻⁴ with 1 to 3 heteroatoms selected from N and O;
R¹¹⁻¹ and R¹¹⁻² are independently H, unsubstituted C₁-C₆ alkyl or C₁-C₆ alkyl substituted by R¹¹⁻¹⁻¹, or 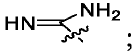
R¹¹⁻³ is H, 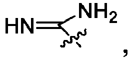 halogen, unsubstituted 4- to 10-membered heterocycloalkyl or 4- to 10-membered heterocycloalkyl substituted by C₁-C₆, or C₁-C₆ alkyl;
R¹¹⁻⁴ is C₁-C₆ alkyl;
R¹¹⁻¹⁻¹ is 4- to 10-membered heterocycloalkyl substituted by R^{L} with 1 to 3 heteroatoms selected from N and O;
R^{L} is H or 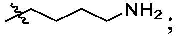
R^{a-1} is independently hydroxyl, halogenor, C₁-C₆ alkyl;
R¹² is C₁-C₆ alkyl;
R¹³ is H, C₁-C₆ alkyl, 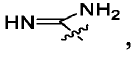 or C₃-C₆ cycloalkyl substituted by R¹³⁻¹;
R¹³⁻¹ is 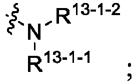
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 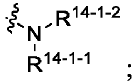
R¹⁴⁻¹⁻¹ and R¹⁴⁻¹⁻² are independently H or C₁-C₆ alkyl;
R¹⁵ is 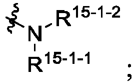
R¹⁵⁻¹⁻¹ and R¹⁵⁻¹⁻² are independently H or C₁-C₆ alkyl;
R¹⁶ is 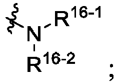
R¹⁶⁻¹ and R¹⁶⁻² are independently H or C₁-C₆ alkyl;
R¹⁷ is 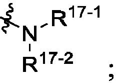
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 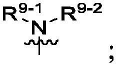
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 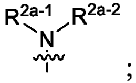
R^{2a-1} and R^{2a-2} are independently H, -C(=O)CH₃, C₁-C₃ alkyl or C₄-C₆ alkyl.

In a certain embodiment, the quinazoline compound represented by formula I is a compound represented by formula I-AI, 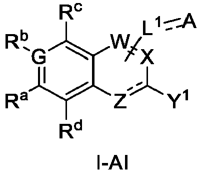
wherein R^{b}, R^{c} and R^{d} are independently H or halogen;
W is C;
G is C or N;
X and Z are N;
" 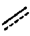 " in 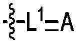 is a single bond or a double bond;
" 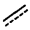 " in 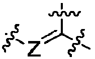 is a single bond or a double bond;

In a certain embodiment, the quinazoline compound represented by formula I is a compound represented by formula I-CI, 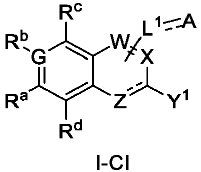
wherein R^{b}, R^{c} and R^{d} are independently H or halogen;
W is C;
G is C or N;
X and Z are N;
" 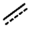 " in 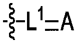 is a double bond;
" 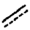 " in 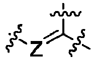 is a double bond;
when ring 1 is independently pyridine, R^{B} is

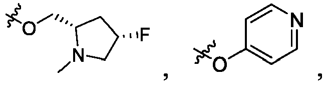

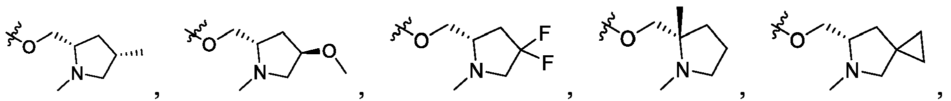

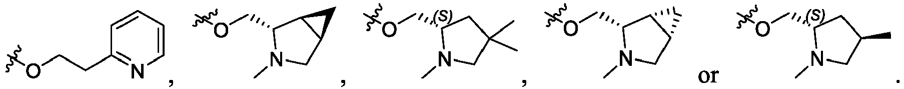

In a certain embodiment, the quinazoline compound represented by formula I is a compound represented by formula I-CI, 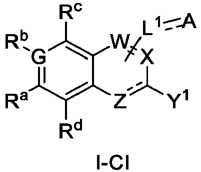
wherein R^{b}, R^{c} and R^{d} are independently H or halogen;
W is C;
Gis C or N;
X and Z are N;
" 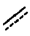 " in 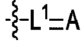 is a double bond;
" 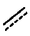 " in 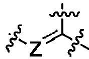 is a double bond;
R^{a} is C₆-C₁₀ alkyl substituted by 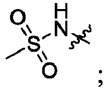
Y¹ is C₁-C₆ alkoxy substituted by one or more R¹¹;
R¹¹ is unsubstituted C₃-C₁₂ cycloalkyl or C₃-C₁₂ cycloalkyl substituted by R¹¹⁻⁶, or unsubstituted 4- to 10-membered heterocycloalkyl or 4- to 10-membered heterocycloalkyl substituted by R¹¹⁻³ with 1 to 3 heteroatoms selected from N and O;
R¹¹⁻⁶⁻¹ is 4- to 10-membered heterocycloalkyl substituted by R¹¹⁻⁶⁻¹⁻¹ with 1 to 3 heteroatoms selected from N and O;
R¹¹⁻⁶⁻¹⁻¹ is C₁-C₆ alkyl;
R¹¹⁻³⁻¹ is C₃-C₁₂ cycloalkyl, unsubstituted 4- to 10-membered heterocycloalkyl or 4-to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl.

In a certain embodiment, the quinazoline compound represented by formula I is a compound represented by formula I-CI, 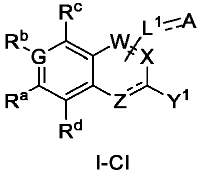
wherein R^{b}, R^{c} and R^{d} are independently H or halogen;
W is C;
G is C or N;
X and Z are N;
" 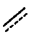 " in 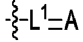 is a double bond;
" 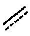 " in 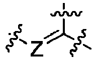 is a double bond;
R^{a} is C₆-C₁₀ aryl substituted by amino, or C₆-C₁₀ aryl substituted by one or more R^{a-1} with at least one substituent being halogen or C₁-C₆ alkyl;
Y¹ is C₁-C₆ alkoxy substituted by one or more R¹¹ or -OR¹³;
R¹¹ is independently C₃-C₁₂ cycloalkyl substituted by R¹¹⁻⁶, or unsubstituted 4- to 10-membered heterocycloalkyl or 4- to 10-membered heterocycloalkyl substituted by R¹¹⁻³ with 1 to 3 heteroatoms selected from N and O;
R¹¹⁻³ is C₃-C₁₂ cycloalkyl, or C₁-C₆ alkyl substituted by R¹¹⁻³⁻¹;
R¹¹⁻⁶ is unsubstituted C₁-C₆ alkyl or C₁-C₆ alkyl substituted by R¹¹⁻⁶⁻¹;
R¹¹⁻⁶⁻¹ is 4- to 10-membered heterocycloalkyl with 1 to 3 heteroatoms selected from N and O, or 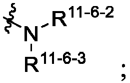
R¹¹⁻⁶⁻² and R¹¹⁻⁶⁻³ are independently H or C₁-C₆ alkyl;
R¹¹⁻³⁻¹ is C₃-C₁₂ cycloalkyl;
R^{a-1} is independently hydroxyl, halogenor, C₃-C₁₂ cycloalkyl;
R¹³ is C₃-C₆ cycloalkyl substituted by R¹³⁻¹;
R¹³⁻¹ is 4- to 10-membered heterocycloalkyl substituted by R¹³⁻¹⁻³ with 1 to 3 heteroatoms selected from N and O;
R¹³⁻¹⁻³ is independently H or C₁-C₆ alkyl.

In a certain embodiment, in R^{a}, the C₆-C₁₀ alkyl substituted by amino can be

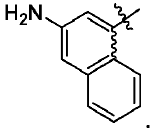

In a certain embodiment, the C₆-C₁₀ aryl in the C₆-C₁₀ aryl substituted by 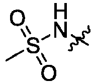 is phenyl or naphthyl.

In a certain embodiment, the unsubstituted C₃-C₁₂ cycloalkyl can be cyclopropyl, cyclobutyl, cyclopentyl or cyclohexyl.

In a certain embodiment, the C₁-C₆ alkyl in the R¹¹⁻⁶⁻¹⁻¹ can be methyl, ethyl, n-propyl, isopropyl, *n*-butyl, isobutyl or *tert*-butyl.

In a certain embodiment, the C₁-C₆ alkyl in R¹¹⁻³⁻¹⁻¹ can be methyl, ethyl, n-propyl, isopropyl, *n-*butyl, isobutyl or *tert*-butyl.

In a certain embodiment, in ring B, the 7- to 12-membered heterocycloalkyl with 2 to 3 heteroatoms selected from one or more of N, O and S is C₇ monocyclic heterocycloalkyl with 2 heteroatoms being N, 7- to 12-membered bridged heterocycloalkyl with 2 to 3 heteroatoms selected from N and O and containing at least 2 N, 7- to 12-membered spiro heterocycloalkyl with 2 heteroatoms being N, or 7- to 12-membered fused heterocycloalkyl with 2 heteroatoms being N, preferably 7- to 12-membered bridged heterocycloalkyl with 2 heteroatoms being N, or 7- to 12-membered spiro heterocycloalkyl with 2 heteroatoms being N; wherein,
the C₇-membered monocyclic heterocycloalkyl with 2 heteroatoms being N is preferably 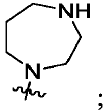
the 7- to 12-membered bridged heterocycloalkyl with 2 to 3 heteroatoms selected from N and O and containing at least 2 N is preferably 7- to 9-membered bridged heterocycloalkyl (for example, 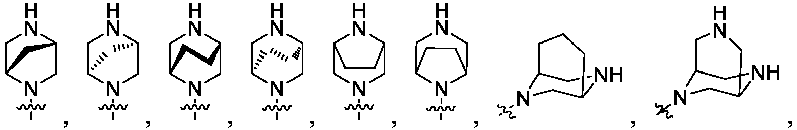 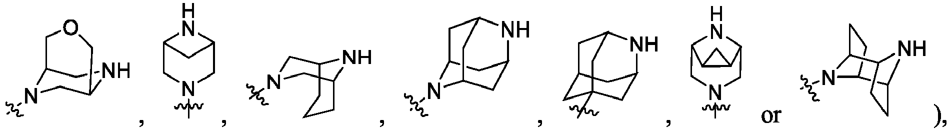 more preferably 7- to 8-membered bridged heterocycloalkyl with 2 heteroatoms being N (for example, 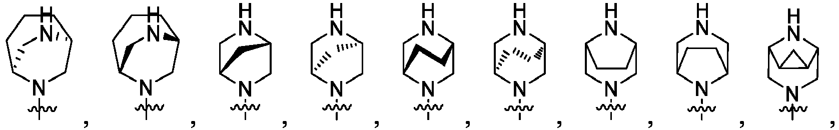 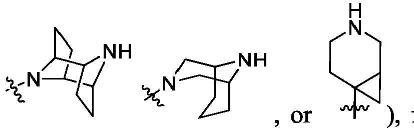 further preferably 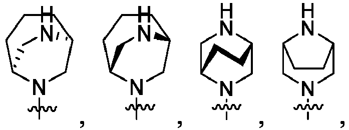 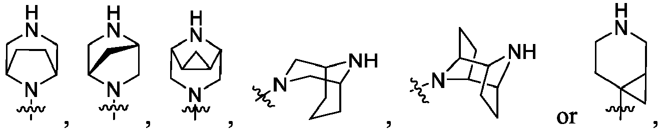 further preferably 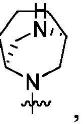 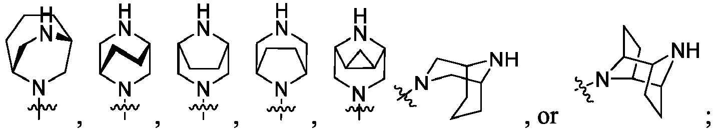
the 7- to 12-membered spiro heterocycloalkyl with 2 heteroatoms being N is preferably 7- to 10-membered spiro heterocycloalkyl with 2 heteroatoms being N (for example, 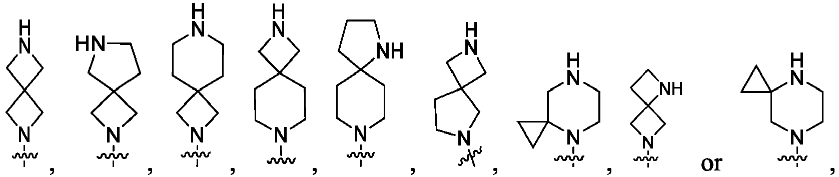 preferably 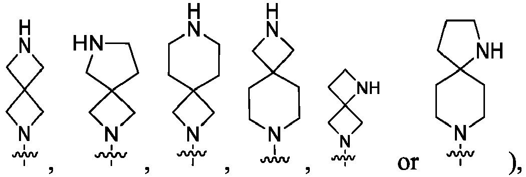 and more preferably 7- to 10-membered spiro heterocycloalkyl with 2 heteroatoms being N, wherein one ring is a 4-membered heterocycloalkyl containing one N atom, for example, 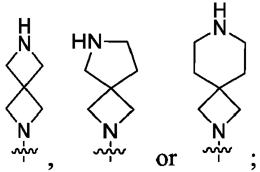
the 7- to 12-membered fused heterocycloalkyl with 2 heteroatoms being N is preferably 7- to 10-membered fused heterocycloalkyl with 2 heteroatoms being N, more preferably 3-membered-fused 6-membered heterocycloalkyl with 2 heteroatoms being N (for example, 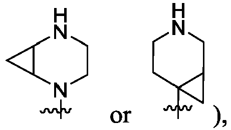 4-membered-fused 6-membered heterocycloalkyl with 2 heteroatoms being N (for example, 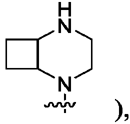 5-membered-fused 6-membered heterocycloalkyl with 2 heteroatoms being N (for example, 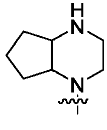 ), 6-membered-fused 6-membered heterocycloalkyl with 2 heteroatoms being N (for example, 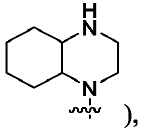 or 5-membered-fused 5-membered heterocycloalkyl with 2 heteroatoms being N (for example,

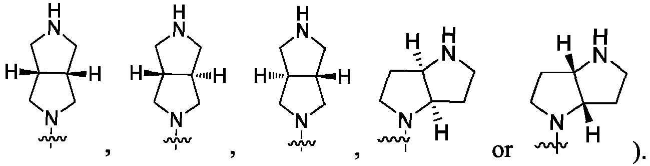

In a certain embodiment, in 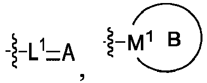 is 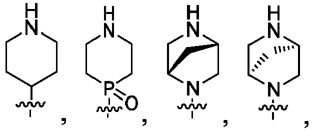 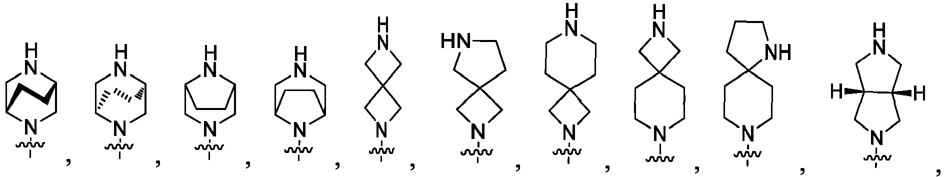 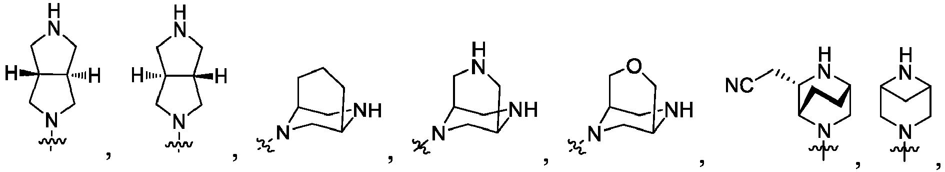 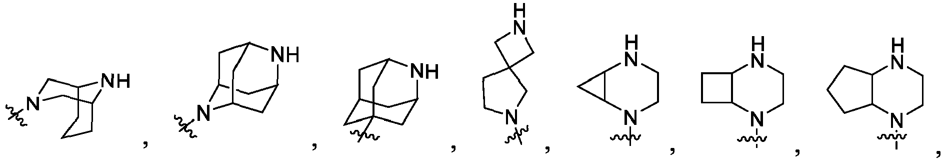 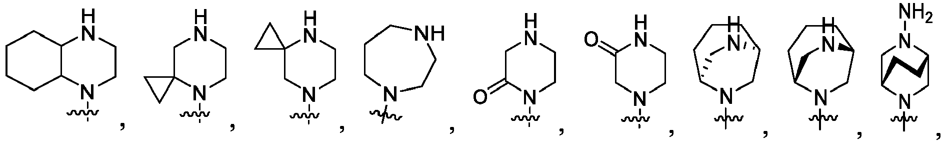 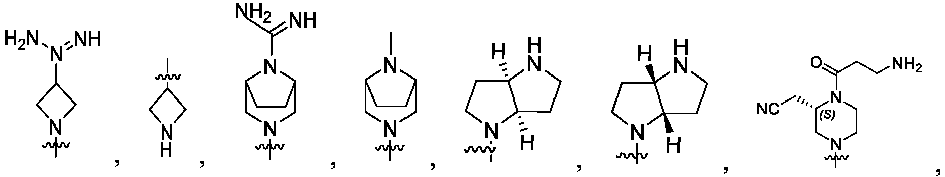 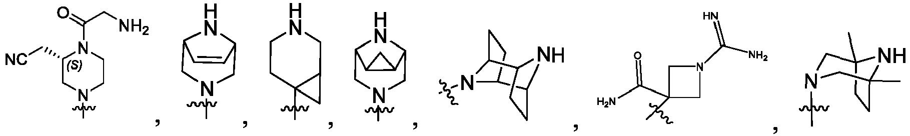 or

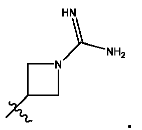

In a certain embodiment, 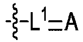 is

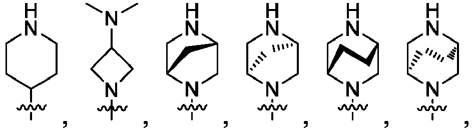

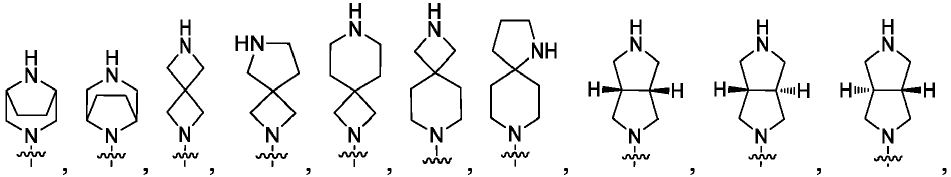

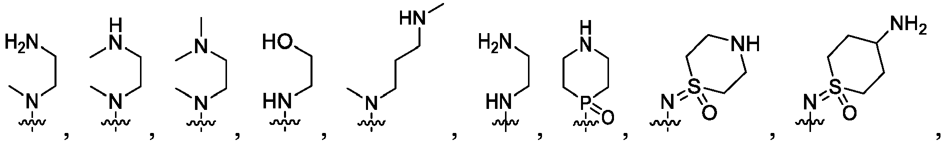

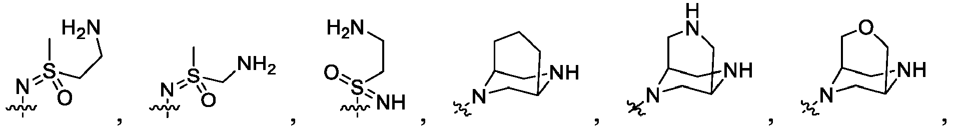

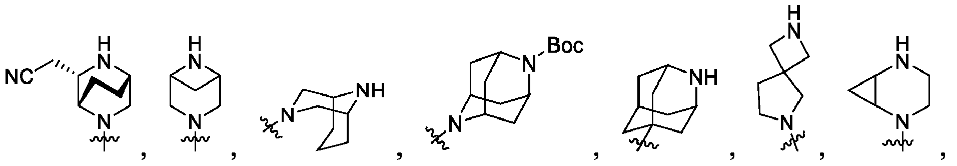

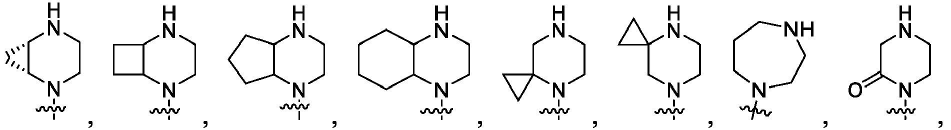

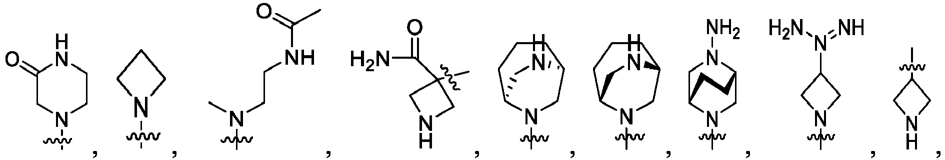

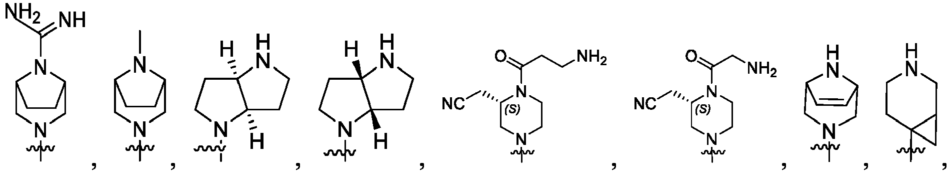

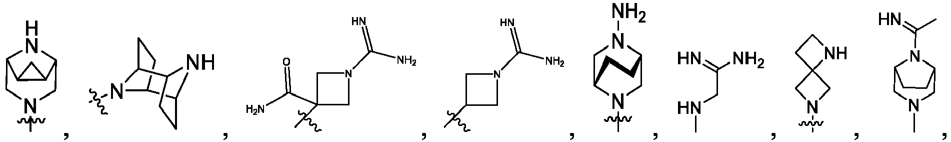

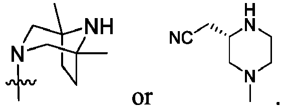

In a certain embodiment, when R¹¹ is the C₃-C₁₂ cycloalkyl substituted by R¹¹⁻⁶, the C₃-C₁₂ cycloalkyl can be cyclopropyl, cyclobutyl, cyclopentyl or cyclohexyl, for example, cyclopropyl.

In a certain embodiment, when R¹¹⁻³ is C₃-C₁₂ cycloalkyl, the C₃-C₁₂ cycloalkyl can be cyclopropyl, cyclobutyl, cyclopentyl or cyclohexyl, for example, cyclopropyl.

In a certain embodiment, when R¹¹⁻⁶ is C₁-C₆ alkyl substituted by R¹¹⁻⁶⁻¹, the C₁-C₆ alkyl can be methyl, ethyl, propyl, *n*-butyl, or *tert*-butyl.

In a certain embodiment, when R¹¹⁻⁶⁻¹ is 4- to 10-membered heterocycloalkyl with 1 to 3 heteroatoms selected from N and O, the 4- to 10-membered heterocycloalkyl with 1 to 3 heteroatoms selected from N and O is 6- to 9-membered monocyclic, fused, bridged or spiro heterocycloalkyl with 1 to 3 heteroatoms selected from N and O.

In a certain embodiment, when R¹¹⁻⁶⁻² and R¹¹⁻⁶⁻³ are C₁-C₆ alkyl, the C₁-C₆ alkyl can be methyl, ethyl, propyl, *n*-butyl, or *tert*-butyl.

In a certain embodiment, R¹¹⁻³⁻¹ can be cyclopropyl, cyclobutyl, cyclopentyl or cyclohexyl, for example, cyclopropyl.

In a certain embodiment, when R¹³⁻¹ is 4- to 10-membered heterocycloalkyl substituted by R¹³⁻¹⁻³ with 1 to 3 heteroatoms selected from N and O, the 4- to 10-membered heterocycloalkyl with 1 to 3 heteroatoms selected from N and O is 6- to 9-membered monocyclic, fused, bridged or spiro heterocycloalkyl with 1 to 3 heteroatoms selected from N and O.

In a certain embodiment, when R¹¹⁻¹⁻³ is C₁-C₆ alkyl, the C₁-C₆ alkyl can be methyl, ethyl, propyl, *n*-butyl, or *tert*-butyl.

In a certain embodiment, R¹³ is

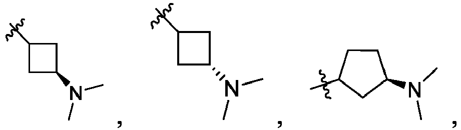

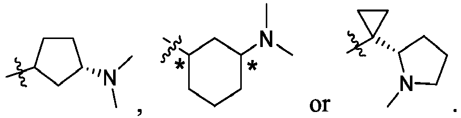

In a certain embodiment, when R^{a} is C₆-C₁₀ aryl substituted by one or more hydroxyl, R^{a} is 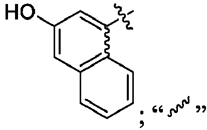 indicates " 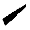 ", " 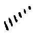 " or a mixture thereof.

In a certain embodiment, when R^{a} is 6- to 10-membered heteroaryl substituted by one or more hydroxyl, the 6- to 10-membered heteroaryl can be 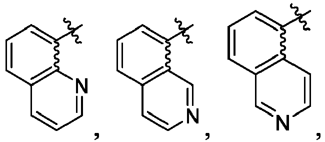 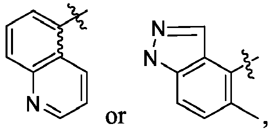 for example, R^{a} is 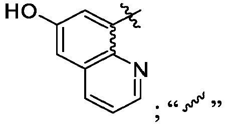 indicates " 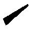 ", " 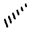 " or a mixture thereof.

In a certain embodiment, when R^{a} is C₆-C₁₀ heteroaryl, the C₆-C₁₀ heteroaryl can be 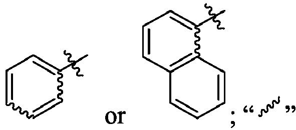 indicates " 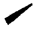 ", " 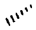 " or a mixture thereof.

In a certain embodiment, when R^{a} is C₆-C₁₀ aryl substituted by one or more R^{a-1} with at least one substituent being halogen or C₁-C₆ alkyl, the C₆-C₁₀ aryl can be 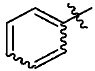 or 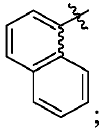 " 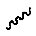 " indicates " 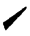 ", " 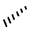 " or a mixture thereof.

In a certain embodiment, when R^{a} is 6- to 10-membered heteroaryl substituted by one or more R^{a-3}, R^{a-3} is 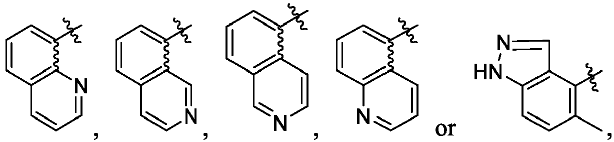 for example,

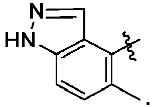

In a certain embodiment, when R^{a-3} is C₁-C₆ alkyl, the C₁-C₆ alkyl can be methyl, ethyl, propyl, *n*-butyl, or *tert*-butyl, for example, methyl.

In a certain embodiment, when R^{a} is C₄-C₈ cycloalkyl-fused C₆-C₁₀ aryl substituted by one or more R^{a-2}, the C₄-C₈ cycloalkyl can be cyclobutyl, cyclopentyl or cyclohexyl, preferably cyclobutyl.

In a certain embodiment, the 3- to 10-membered heterocycloalkyl in the 3- to 10-membered heterocycloalkyl substituted by one or more 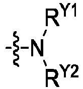 with 1 to 4 heteroatoms selected from one or more of N, O and S is

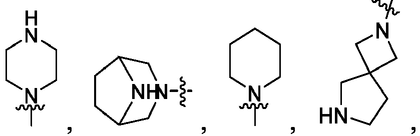

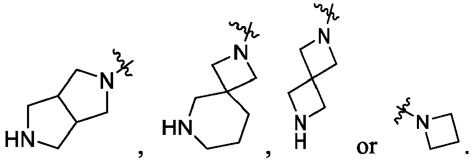

In a certain embodiment, the 3- to 10-membered heterocycloalkyl substituted by one or more 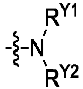 with 1 to 4 heteroatoms selected from one or more of N, O and S is

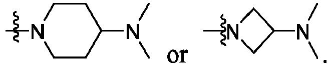

In a certain embodiment, in R^{Y1} and R^{Y2}, the C₁-C₆ alkyl can be methyl, ethyl, n-propyl or isopropyl, preferably methyl.

In a certain embodiment, the unsubstituted 4- to 10-membered heterocycloalkyl or 4-to 10-membered heterocycloalkyl substituted by R¹² with 1 to 3 heteroatoms selected from N and O can be 6- to 9-membered monocyclic, fused, bridged or spiro heterocycloalkyl substituted by R¹² with 1 or 2 heteroatoms being N; the 4- to 10-membered heterocycloalkyl is preferably

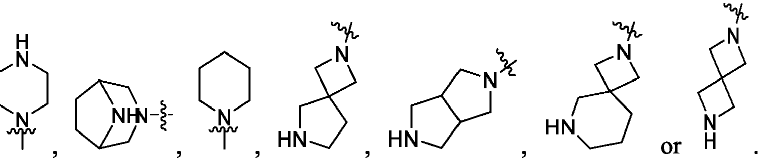

In a certain embodiment, when Y¹ is C₆-C₁₀ aryl substituted by one hydroxyl, the C₆-C₁₀ aryl can be

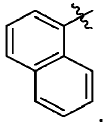

In a certain embodiment, when Y¹ is C₆-C₁₀ aryl substituted by one hydroxyl, Y¹ is

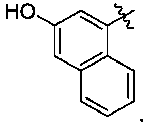

In a certain embodiment, in the C₁-C₆ alkyl substituted by R¹⁵, the C₁-C₆ alkyl can be methyl, ethyl, *n*-propyl and isopropyl.

In a certain embodiment, in the C₁-C₆ alkylthio substituted by R¹⁶, the C₁-C₆ alkylthio is methylthio, ethylthio, *n*-propylthio or isopropylthio.

In a certain embodiment, in the C₃-C₁₀ cycloalkyl substituted by R¹⁷, the C₃-C₁₀ cycloalkyl can be cyclopropyl, cyclobutyl, cyclopentyl or cyclohexyl.

In a certain embodiment, in ring B, the 4- to 10-membered heterocycloalkyl with 1 to 3 heteroatoms or heteroatom groups selected from N and P(=O) and containing only 1 N can be 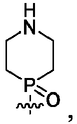 azetidinyl (for example, 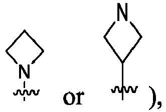 tetrahydropyrrolyl or piperidinyl (for example,

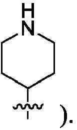

In a certain embodiment, in ring B, the 4- to 10-membered heterocycloalkyl substituted by R¹⁰ with 1 to 3 heteroatoms or heteroatom groups selected from N and P(=O) and containing only 1 N is 4- to 10-membered monocyclic heterocycloalkyl with 1 to 3 heteroatoms or heteroatom groups selected from N and P(=O) and containing only 1 N, 4- to 10-membered bridged heterocycloalkyl with 1 to 3 heteroatoms or heteroatom groups selected from N and P(=O) and containing only 1 N, 4- to 10-membered spiro heterocycloalkyl with 1 to 3 heteroatoms or heteroatom groups selected from N and P(=O) and containing only 1 N, or 4- to 10-membered fused heterocycloalkyl with 1 to 3 heteroatoms or heteroatom groups selected from N and P(=O) and containing only 1 N, preferably 4- to 10-membered monocyclic heterocycloalkyl with 1 to 3 heteroatoms or heteroatom groups selected from N and P(=O) and containing only 1 N, or 4- to 10-membered bridged heterocycloalkyl with 1 to 3 heteroatoms or heteroatom groups selected from N and P(=O) and containing only 1 N;
wherein, the 4- to 10-membered monocyclic heterocycloalkyl with 1 to 3 heteroatoms or heteroatom groups selected from N and P(=O) and containing only 1 N can be 4- to 10-membered monocyclic heterocycloalkyl with 1 heteroatom being N, preferably 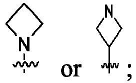
the 4- to 10-membered bridged heterocycloalkyl with 1 to 3 heteroatoms or heteroatom groups selected from N and P(=O) and containing only 1 N can be 7- to 10-membered bridged heterocycloalkyl with 1 heteroatom being N, preferably 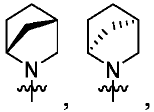 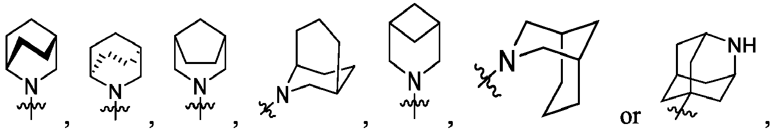 more preferably 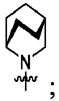
the 4- to 10-membered spiro heterocycloalkyl with 1 to 3 heteroatoms or heteroatom groups selected from N and P(=O) and containing only 1 N is preferably 7- to 10-membered spiro heterocycloalkyl with 1 heteroatom being N, preferably 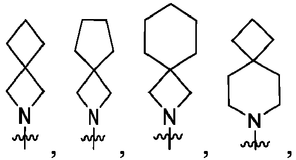 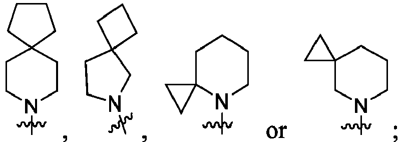
the 4- to 10-membered fused heterocycloalkyl with 1 to 3 heteroatoms or heteroatom groups selected from N and P(=O) and containing only 1 N is preferably 4- to 10-membered fused heterocycloalkyl with 1 heteroatom being N.

In a certain embodiment, the 4- to 10-membered heterocycloalkyl substituted by R¹⁰ with 1 to 3 heteroatoms or heteroatom groups selected from N and P(=O) and containing only one N can be

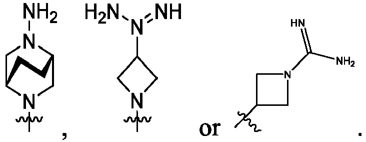

In a certain embodiment, in ring B, the 4- to 10-membered heterocycloalkenyl with 1 to 3 heteroatoms or heteroatom groups selected from N and P(=O) can be

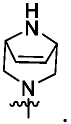

In a certain embodiment, in ring B, the 5- to 6-membered heterocycloalkyl with 2 to 3 heteroatoms selected from one or more of N, O and S can be

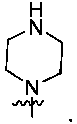

In a certain embodiment, R⁷ can be one or more.

In a certain embodiment, in ring B, the 5- to 6-membered heterocycloalkyl substituted by R⁷ with 2 to 3 heteroatoms selected from one or more of N, O and S is 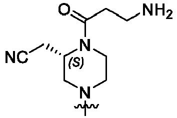 or

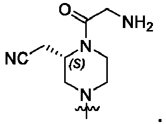

In a certain embodiment, in R⁷, the C₁-C₆ alkyl can be methyl, ethyl, n-propyl or isopropyl, preferably methyl.

In a certain embodiment, the 7- to 12-membered heterocycloalkyl substituted by R⁷ with 2 to 3 heteroatoms selected from one or more of N, O and S can be

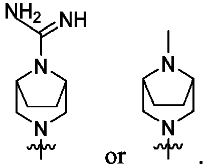

In a certain embodiment, in 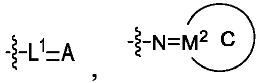 is 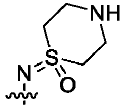 or

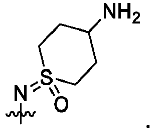

In a certain embodiment, in 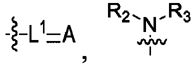 is 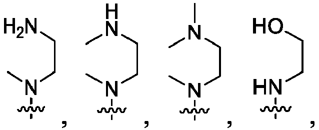 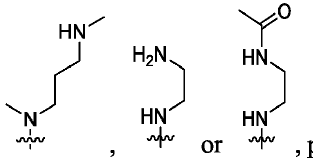 preferably

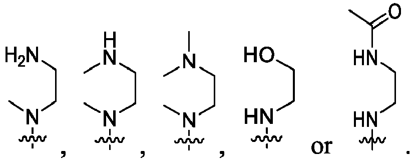

In a certain embodiment, the 4- to 10-membered heterocycloalkyl with 1 to 3 heteroatoms selected from N and S can be

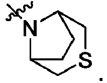

In a certain embodiment, when R¹¹ is unsubstituted 4- to 10-membered heterocycloalkyl or 4- to 10-membered heterocycloalkyl substituted by one or more R¹¹⁻³, the C₁-C₆ alkoxy substituted by R¹¹ is *cis, trans,* or a mixture thereof.

In a certain embodiment, in R¹¹, the unsubstituted 5- to 6-membered heteroaryl or 5-to 6-membered heteroaryl substituted by one or more R¹¹⁻⁴ with 1 to 3 heteroatoms selected from N and O can be

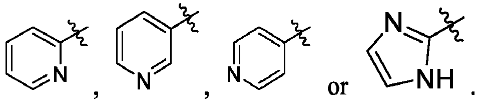

In a certain embodiment, in R¹¹, the C₁-C₆ alkoxy can be methoxy, ethoxy, isopropoxy, n-butyl, isobutyl or *tert*-butyl, preferably methoxy.

In a certain embodiment, in R¹¹⁻⁵, the 4- to 10-membered heterocycloalkyl with 1 to 3 heteroatoms selected from one or more of N and O is preferably pyrrolidinyl or tetrahydrofuranyl.

In a certain embodiment, in the 4- to 10-membered heterocycloalkyl substituted by R^{L} with 1 to 3 heteroatoms selected from N and O, the 4- to 10-membered heterocycloalkyl with 1 to 3 heteroatoms selected from N and O can be

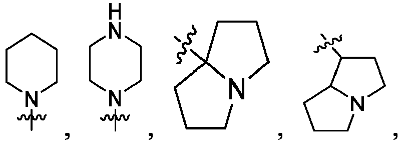

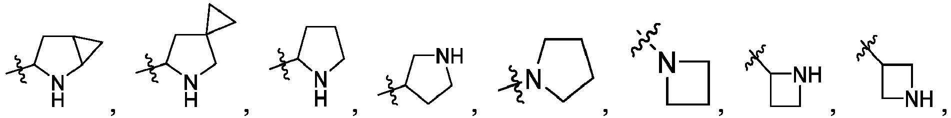

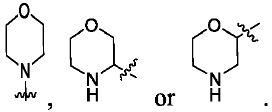

In a certain embodiment, in R¹¹⁻¹ and R¹¹⁻², and in unsubstituted C₁-C₆ alkyl or C₁-C₆ alkyl substituted by R¹¹⁻¹⁻¹, the C₁-C₆ alkyl is methyl, ethyl, *n*-propyl, isopropyl, *n*-butyl, isobutyl or *tert*-butyl, preferably methyl or *n*-butyl.

In a certain embodiment, in R¹¹⁻³, the C₁-C₆ alkyl is methyl, ethyl, n-propyl or isopropyl, preferably methyl.

In a certain embodiment, in R¹¹⁻³, the C₂-C₆ alkynyl can be ethynyl, propynyl, 1-butynyl or 2-butynyl, preferably ethynyl, propynyl or 1-butynyl.

In a certain embodiment, in R¹¹⁻³, the C₁-C₆ alkoxy can be methoxy, ethoxy, n-propoxy, isopropoxy, *n*-butoxy, or *tert*-butoxy, preferably methoxy.

In a certain embodiment, in R¹¹⁻⁴, the C₁-C₆ alkyl is methyl, ethyl, *n*-propyl or isopropyl, preferably methyl or ethyl.

In a certain embodiment, R¹¹⁻¹⁻¹ can be

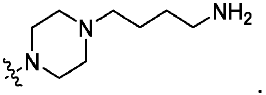

In a certain embodiment, 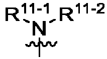 is

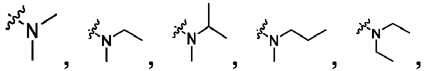

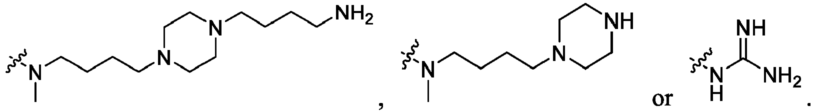

In a certain embodiment, in R^{a-1}, the C₁-C₆ alkyl is methyl, ethyl, *n*-propyl or isopropyl, preferably methyl or ethyl.

In a certain embodiment, in R^{a-1}, the halogen is F, Cl, Br or I, preferably For Cl.

In a certain embodiment, in R^{a-1}, the C₂-C₆ alkynyl can be ethynyl, propynyl, 1-butynyl or 2-butynyl, preferably ethynyl or butynyl.

In a certain embodiment, in R^{a-1}, the C₃-C₁₂ cycloalkyl can be C₃-C₁₂ monocyclic cycloalkyl, C₃-C₁₂ bridged cycloalkyl, or C₃-C₁₂ spiro cycloalkyl, preferably C₃-C₁₂ monocyclic cycloalkyl, or C₃-C₁₂ bridged cycloalkyl, preferably cyclopropyl or bicyclo[1.1.1]pentyl.

In a certain embodiment, the C₁-C₆ alkyl in the C₁-C₆ alkyl substituted by R^{a-1-2} can be methyl, ethyl, *n*-propyl or isopropyl, preferably methyl.

In a certain embodiment, in R^{a-1-1}, the halogen is F, Cl, Br or I, preferably F.

In a certain embodiment, in R^{a-1-2}, the halogen is F, Cl, Br or I, preferably F.

In a certain embodiment, R^{a-1} is hydroxyl, methyl, fluorine, chlorine, ethyl, ethynyl, butynyl, cyclopropyl, bicyclo [1.1.1]pentyl, -CF₃, or fluoroethynyl.

In a certain embodiment, ring 1 and/or 2 can be phenyl, pyridinyl, piperidinyl, pyrrolyl or thiophenyl, preferably, ring 1-fused ring 2 can be 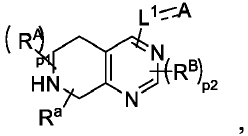 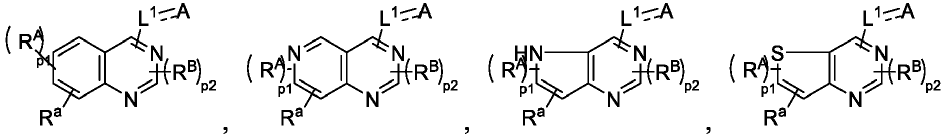 or

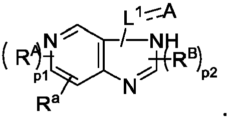

In a certain embodiment, in R¹², the C₁-C₆ alkyl can be methyl, ethyl, *n*-propyl or isopropyl, preferably methyl.

In a certain embodiment, unsubstituted 4- to 10-membered heterocycloalkyl or 4- to 10-membered heterocycloalkyl substituted by R¹² with 1 to 3 heteroatoms or heteroatom groups selected from N and O can be 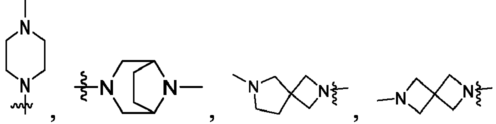 or

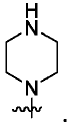

In a certain embodiment, in R¹³, the C₃-C₆ cycloalkyl can be 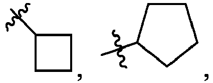 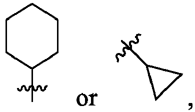 preferably

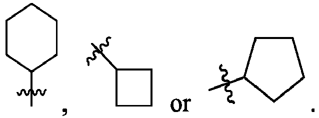

In a certain embodiment, in R¹³⁻¹⁻¹ and R¹³⁻¹⁻², the C₁-C₆ alkyl is methyl, ethyl, n-propyl or isopropyl, for example, methyl.

In a certain embodiment, R¹³ is

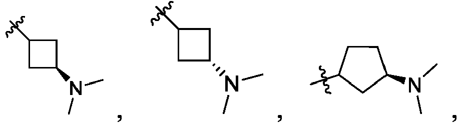

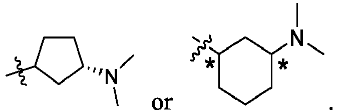

In a certain embodiment, in R¹⁴, the C₁-C₆ alkyl is methyl, ethyl, n-propyl or isopropyl, preferably n-propyl.

In a certain embodiment, in R¹⁴⁻¹⁻¹ and R¹⁴⁻¹⁻², the C₁-C₆ alkyl is methyl, ethyl, *n-*propyl or isopropyl, preferably methyl.

In a certain embodiment, in R¹⁵⁻¹⁻¹ and R¹⁵⁻¹⁻², the C₁-C₆ alkyl is methyl, ethyl, *n-*propyl or isopropyl.

In a certain embodiment, in R¹⁶⁻¹⁻¹ and R¹⁶⁻¹⁻², the C₁-C₆ alkyl is methyl, ethyl, *n-*propyl or isopropyl.

In a certain embodiment, in R¹⁷⁻¹⁻¹ and R¹⁷⁻¹⁻², the C₁-C₆ alkyl is methyl, ethyl, *n-*propyl or isopropyl.

In a certain embodiment, the amino substituted by one or more R¹⁴ is 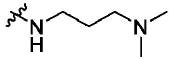 or

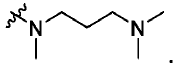

In a certain embodiment, the quinazoline compound represented by formula I is a compound represented by formula I-CI, 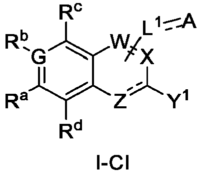
wherein R^{b}, R^{c} and R^{d} are independently H or halogen;
W is C;
GisC;
X and Z are N;
" 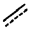 " in 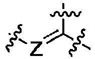 is a double bond.

In a certain embodiment, when R^{a} is C₆-C₁₀ aryl substituted by one or more hydroxyl, R^{a} is 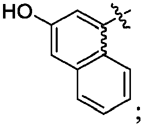 " 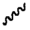 " indicates " 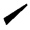 ", " 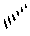 " or a mixture thereof.

In a certain embodiment, when R^{a} is 6- to 10-membered heteroaryl substituted by one or more hydroxyl, the 6- to 10-membered heteroaryl can be 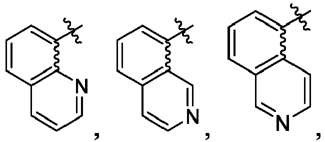 or 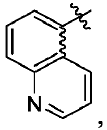 for example, R^{a} is 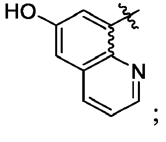 " 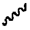 " indicates " 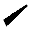 ", " 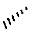 " or a mixture thereof.

In a certain embodiment, when R^{a} is C₆-C₁₀ heteroaryl, the C₆-C₁₀ heteroaryl can be 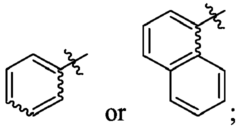 " 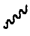 " indicates " 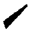 ", " 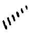 " or a mixture thereof.

In a certain embodiment, when R^{a} is C₆-C₁₀ aryl substituted by one or more R^{a-1} with at least one substituent being halogen or C₁-C₆ alkyl, the C₆-C₁₀ aryl can be 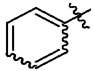 or 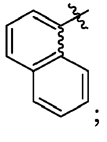 " 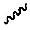 " indicates " 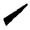 ", " 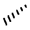 " or a mixture thereof.

In a certain embodiment, the 3- to 10-membered heterocycloalkyl in the 3- to 10-membered heterocycloalkyl substituted by one or more 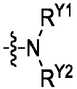 with 1 to 4 heteroatoms selected from one or more of N, O and S is

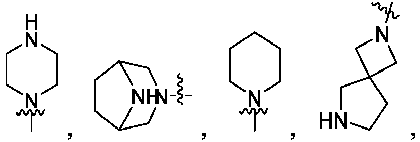

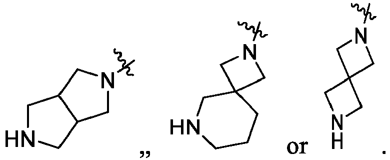

In a certain embodiment, 3- to 10-membered heterocycloalkyl substituted by one or more 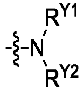 with 1 to 4 heteroatoms selected from one or more of N, O and S is

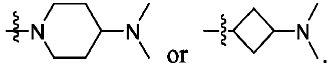

In a certain embodiment, in R^{Y1} and R^{Y2}, the C₁-C₆ alkyl can be methyl, ethyl, *n-*propyl or isopropyl, preferably methyl.

In a certain embodiment, the unsubstituted 4- to 10-membered heterocycloalkyl or 4-to 10-membered heterocycloalkyl substituted by R¹² with 1 to 3 heteroatoms selected from N and O can be 6- to 9-membered monocyclic, fused, bridged or spiro heterocycloalkyl substituted by R¹² with 1 or 2 heteroatoms being N; the 4- to 10-membered heterocycloalkyl is preferably

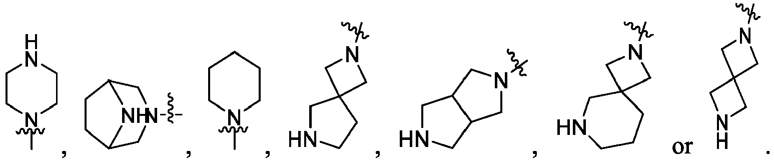

In a certain embodiment, when Y¹ is C₆-C₁₀ aryl substituted by one hydroxyl, the C₆-C₁₀ aryl can be

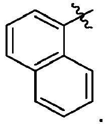

In a certain embodiment, when Y¹ is C₆-C₁₀ aryl substituted by one hydroxyl, Y¹ is

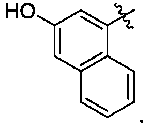

In a certain embodiment, in the C₁-C₆ alkyl substituted by R¹⁵, the C₁-C₆ alkyl can be methyl, ethyl, *n*-propyl and isopropyl.

In a certain embodiment, in the C₁-C₆ alkylthio substituted by R¹⁶, the C₁-C₆ alkylthio is methylthio, ethylthio, n-propylthio or isopropylthio.

In a certain embodiment, in the C₃-C₁₀ cycloalkyl substituted by R¹⁷, the C₃-C₁₀ cycloalkyl can be cyclopropyl, cyclobutyl, cyclopentyl or cyclohexyl.

In a certain embodiment, in ring B, the 4- to 10-membered heterocycloalkyl with 1 to 3 heteroatoms or heteroatom groups selected from N and P(=O) and containing only 1 N can be 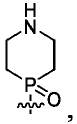 azetidinyl (for example, 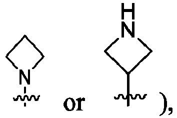 tetrahydropyrrolyl or piperidinyl (for example,

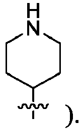

In a certain embodiment, in R⁷, the C₁-C₆ alkyl is methyl, ethyl, n-propyl or isopropyl, preferably methyl.

In a certain embodiment, the 7- to 12-membered heterocycloalkyl substituted by R⁷ with 2 to 3 heteroatoms selected from one or more of N, O and S can be

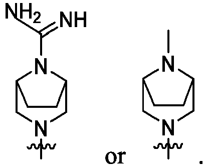

In a certain embodiment, in 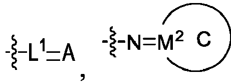 is

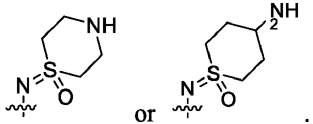

In a certain embodiment, in 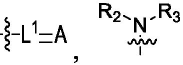 is 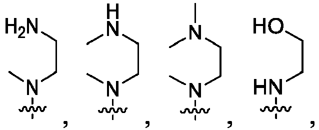 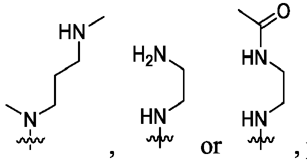 preferably

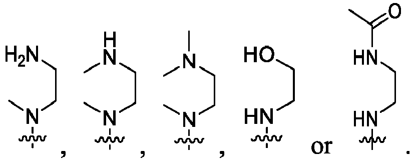

In a certain embodiment, when R¹¹ is 4- to 10-membered heterocycloalkyl, the C₁-C₆ alkoxy substituted by R¹¹ can be *cis, trans,* or a mixture thereof.

In a certain embodiment, in R¹¹, the unsubstituted 5- to 6-membered heteroaryl or 5-to 6-membered heteroaryl substituted by one or more R¹¹⁻⁴ with 1 to 3 heteroatoms selected from N and O can be

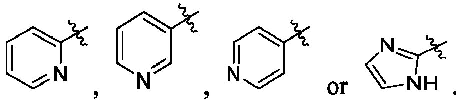

In a certain embodiment, in the 4- to 10-membered heterocycloalkyl substituted by R^{L} with 1 to 3 heteroatoms selected from N and O, the 4- to 10-membered heterocycloalkyl with 1 to 3 heteroatoms selected from N and O can be

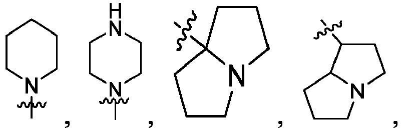

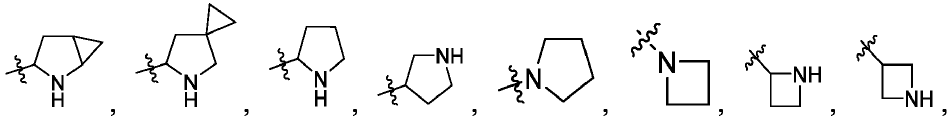

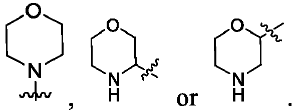

In a certain embodiment, in R¹¹⁻¹ and R¹¹⁻², and in unsubstituted C₁-C₆ alkyl or C₁-C₆ alkyl substituted by R¹¹⁻¹⁻¹, the C₁-C₆ alkyl is methyl, ethyl, *n*-propyl, isopropyl, *n*-butyl, isobutyl or *tert*-butyl, preferably methyl or *n*-butyl.

In a certain embodiment, in R¹¹⁻⁴, the C₁-C₆ alkyl is methyl, ethyl, n-propyl or isopropyl, preferably methyl or ethyl.

In a certain embodiment, R¹¹⁻¹⁻¹ can be

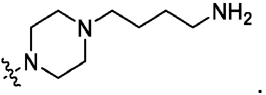

In a certain embodiment, 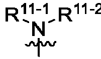 is

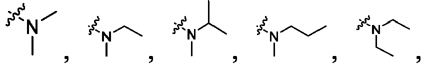

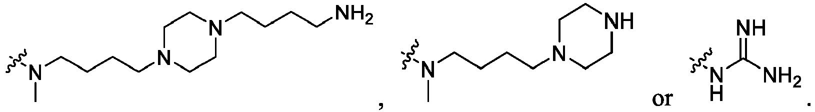

In a certain embodiment, in R¹², the C₁-C₆ alkyl is methyl, ethyl, n-propyl or isopropyl, preferably methyl.

In a certain embodiment, unsubstituted 4- to 10-membered heterocycloalkyl or 4- to 10-membered heterocycloalkyl substituted by R¹² with 1 to 3 heteroatoms or heteroatom groups selected from N and O can be

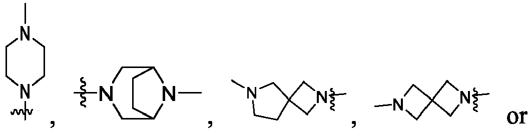

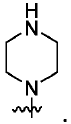

In a certain embodiment, in R¹³, the C₃-C₆ cycloalkyl can be 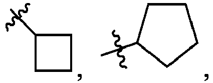 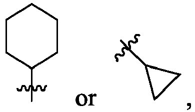 preferably

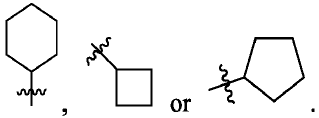

In a certain embodiment, in R¹³⁻¹⁻¹ and R¹³⁻¹⁻², the C₁-C₆ alkyl is methyl, ethyl, *n-*propyl or isopropyl, for example, methyl.

In a certain embodiment, R¹³ is

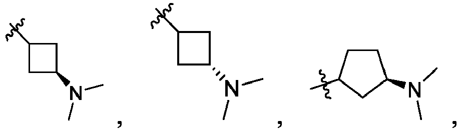

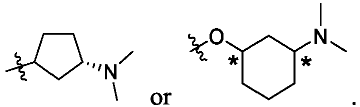

In a certain embodiment, in R¹⁴, the C₁-C₆ alkyl is methyl, ethyl, *n*-propyl or isopropyl, preferably *n*-propyl.

In a certain embodiment, in R¹⁴⁻¹⁻¹ and R¹⁴⁻¹⁻², the C₁-C₆ alkyl is methyl, ethyl, *n-*propyl or isopropyl, preferably methyl.

In a certain embodiment, in R¹⁵⁻¹⁻¹ and R¹⁵⁻¹⁻², the C₁-C₆ alkyl is methyl, ethyl, *n-*propyl or isopropyl.

In a certain embodiment, in R¹⁶⁻¹⁻¹ and R¹⁶⁻¹⁻², the C₁-C₆ alkyl is methyl, ethyl, *n-*propyl or isopropyl.

In a certain embodiment, in R¹⁷⁻¹⁻¹ and R¹⁷⁻¹⁻², the C₁-C₆ alkyl is methyl, ethyl, *n-*propyl or isopropyl.

In a certain embodiment, the amino substituted by one or more R¹⁴ is 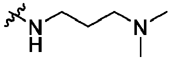 or

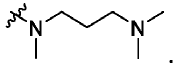

In a certain embodiment, the quinazoline compound represented by formula I is a compound represented by formula I-BI,

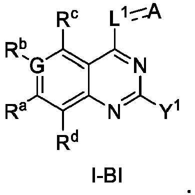

In a certain embodiment, 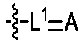 is

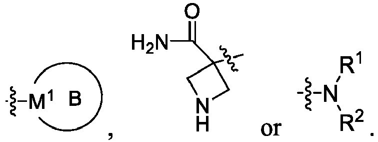

In a certain embodiment, ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 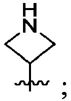
the 4- to 10-membered monocyclic heterocycloalkyl with 1 to 3 heteroatoms or heteroatom groups selected from N and P(=O) and containing only one N can be 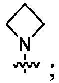
the 4- to 10-membered bridged heterocycloalkyl substituted by R¹⁰ with 1 to 3 heteroatoms or heteroatom groups selected from N and P(=O) and containing only one N can be 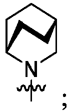
the 7- to 12-membered bridged heterocycloalkyl substituted by R⁷ with 2 to 3 heteroatoms selected from one or more of N, O and S can be

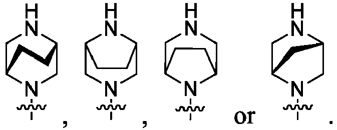

In a certain embodiment, R^{2a-1} and R^{2a-2} are independently H, -C(=O)CH₃, C₁-C₃ alkyl, or C₄-C₆ alkyl, and R^{2a-1} and R^{2a-2} are not C₁-C₃ alkyl or C₄-C₆ alkyl at the same time.

In a certain embodiment, R⁷ is 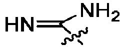 or C₁-C₆ alkyl.

In a certain embodiment, the quinazoline compound represented by formula I is a compound represented by formula I-BI, 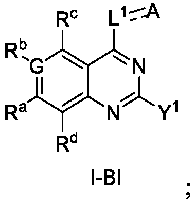
Gis C or N;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl;
R^{b} is halogen;
R^{c} is H;
R^{d} is halogen;
Y¹ is 3- to 10-membered heterocycloalkyl substituted by one or more 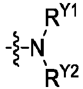 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
" 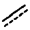 " in 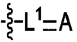 is a single bond; 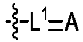
is 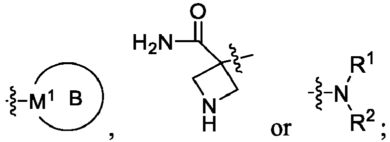
M¹ is Nor CH;
ring B is unsubstituted 4- to 10-membered heterocycloalkyl with 1 heteroatom being N, or unsubstituted 7- to 12-membered bridged heterocycloalkyl with 2 heteroatoms being N, 7- to 12-membered spiro heterocycloalky with 2 heteroatoms being N, 7- to 12-membered fused heterocycloalky with 2 heteroatoms being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S; one N atom in the 7- to 12-membered heterocycloalkyl is attached to ring 2;
wherein, the 4- to 10-membered heterocycloalkyl with 1 heteroatom or heteroatom group being N can be 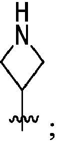
the 4- to 10-membered monocyclic heterocycloalkyl with 1 to 3 heteroatoms or heteroatom groups selected from N and P(=O) and containing only one N can be 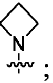
the 4- to 10-membered bridged heterocycloalkyl substituted by R¹⁰ with 1 to 3 heteroatoms or heteroatom groups selected from N and P(=O) and containing only one N can be 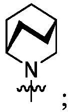
the 7- to 12-membered bridged heterocycloalkyl substituted by R⁷ with 2 to 3 heteroatoms selected from one or more of N, O and S can be 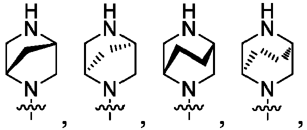 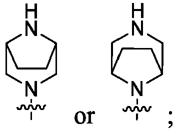
R¹ is H, C₁-C₃ alkyl, or C₄-C₆ alkyl;
R² is -(CH₂)ₙ-R^{2a};
n is 1 or 2;
R^{2a} is 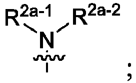
R^{2a-1} and R^{2a-2} are independently H, -C(=O)CH₃, C₁-C₃ alkyl or C₄-C₆ alkyl;
R¹⁰ is amino or 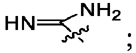
R⁷ is -C(=O)O-(C₁-C₄ alkyl), 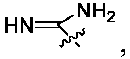 C₁-C₆ alkyl, C₁-C₃ alkyl substituted by CN, or C₄-C₆ alkyl substituted by CN;
R¹¹ is optionally halogen, 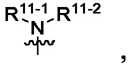 unsubstituted 4- to 10-membered heterocycloalkyl or 4- to 10-membered heterocycloalkyl substituted by one or more R¹¹⁻³ with 1 to 3 heteroatoms selected from N and O, or unsubstituted 5- to 6-membered heteroaryl or 5-to 6-membered heteroaryl substituted by one or more R¹¹⁻⁴ with 1 to 3 heteroatoms selected from N and O;
R¹¹⁻¹ and R¹¹⁻² are independently H, unsubstituted C₁-C₆ alkyl or C₁-C₆ alkyl substituted by R¹¹⁻¹⁻¹, or 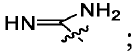
R¹¹⁻³ is H, 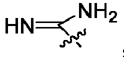 , halogen, unsubstituted 4- to 10-membered heterocycloalkyl or 4- to 10-membered heterocycloalkyl substituted by C₁-C₆, or C₁-C₆ alkyl; the heteroatom is selected from N and O, and the number of heteroatoms is 1 to 3;
R¹¹⁻⁴ is C₁-C₆ alkyl;
R¹¹⁻¹⁻¹ is 4- to 10-membered heterocycloalkyl substituted by R^{L} with 1 to 3 heteroatoms selected from N and O;
R^{L} is H or 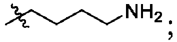
R^{a-1} is optionally hydroxyl, halogen or C₁-C₆ alkyl;
R¹² is C₁-C₆ alkyl;
R¹³ is H, C₁-C₆ alkyl, 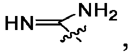 or C₃-C₆ cycloalkyl substituted by R¹³⁻¹;
R¹³⁻¹ is 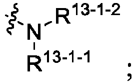
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 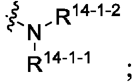
R¹⁴⁻¹⁻¹ and R¹⁴⁻¹⁻² are independently H or C₁-C₆ alkyl;
R¹⁵ is 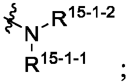
R¹⁵⁻¹⁻¹ and R¹⁵⁻¹⁻² are independently H or C₁-C₆ alkyl;
R¹⁶ is 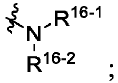
R¹⁶⁻¹ and R¹⁶⁻² are independently H or C₁-C₆ alkyl;
R¹⁷ is 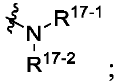
R¹⁷⁻¹ and R¹⁷⁻² are independently H or C₁-C₆ alkyl.

In a certain embodiment, the quinazoline compound represented by formula I is a compound represented by formula I-BI, 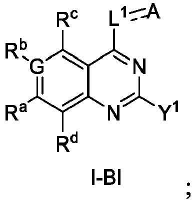
Gis C or N;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl;
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 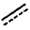 " in 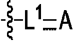 is a single bond; 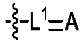
is 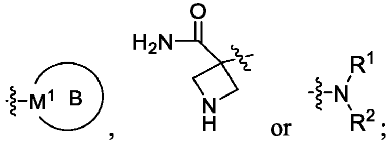
M¹ is Nor CH;
ring B is unsubstituted 4- to 10-membered heterocycloalkyl with 1 heteroatom being N, or unsubstituted 7- to 12-membered bridged heterocycloalkyl with 2 heteroatoms being N, unsubstituted 7- to 12-membered spiro heterocycloalky with 2 heteroatoms being N, unsubstituted 7- to 12-membered fused heterocycloalky with 2 heteroatoms being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 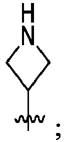
the 4- to 10-membered monocyclic heterocycloalkyl with 1 to 3 heteroatoms or heteroatom groups selected from N and P(=O) and containing only one N can be 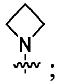
the 4- to 10-membered bridged heterocycloalkyl substituted by R¹⁰ with 1 to 3 heteroatoms or heteroatom groups selected from N and P(=O) and containing only one N can be 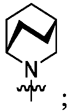
the 7- to 12-membered bridged heterocycloalkyl substituted by R⁷ with 2 to 3 heteroatoms selected from one or more of N, O and S can be 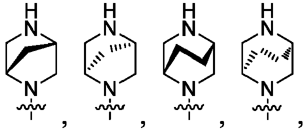 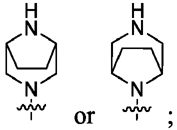
R¹ is H or C₁-C₆ alkyl;
R² is -(CH₂)ₙ-R^{2a};
n is 1 or 2;
R^{2a} is 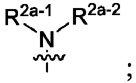
R^{2a-1} and R^{2a-2} are independently H, -C(=O)CH₃ or C₁-C₆ alkyl;
R¹⁰ is amino or 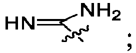
R¹⁰⁻¹ and R¹⁰⁻² are independently H or C₁-C₆ alkyl;
R⁷ is 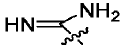 or C₁-C₆ alkyl;
R¹¹ is 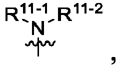 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 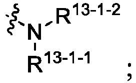
R¹³⁻¹⁻¹ and R¹³⁻¹⁻² are independently H or C₁-C₆ alkyl;
R¹⁴ is C₁-C₆ alkyl substituted by 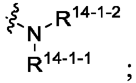
R¹⁴⁻¹⁻¹ and R¹⁴⁻¹⁻² are independently H or C₁-C₆ alkyl.

In a certain embodiment, the quinazoline compound represented by formula I is a compound represented by formula I-BI, 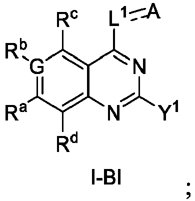
wherein, G is C or N;
R^{a} is 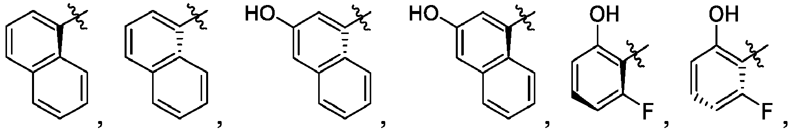 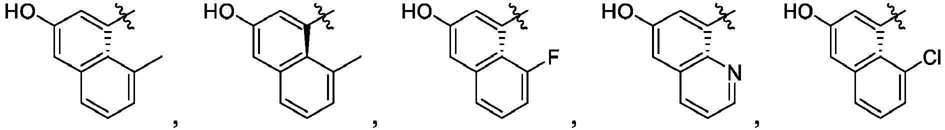 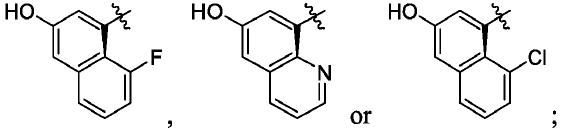
R^{b} is halogen;
R^{c} is H;
R^{d} is halogen;
Y¹ is 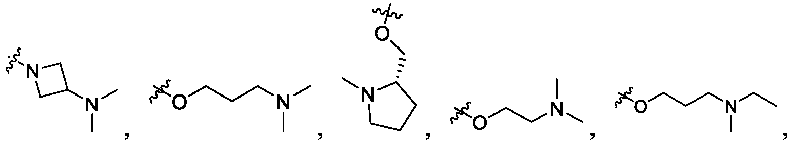 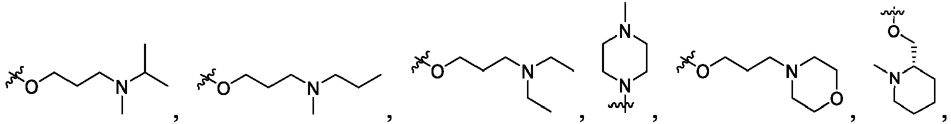 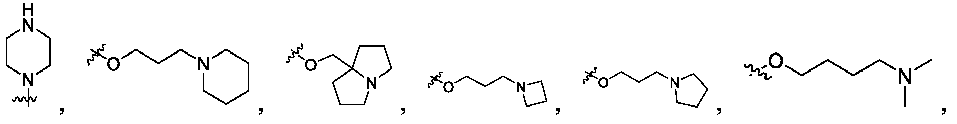 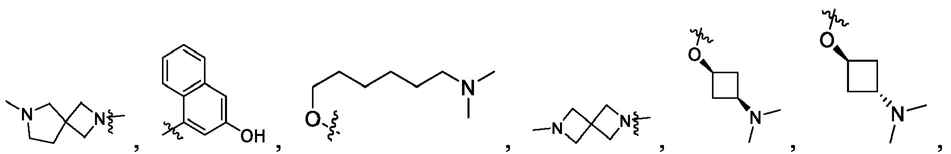 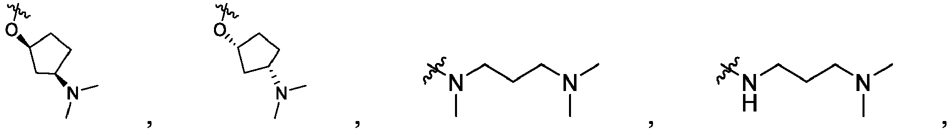 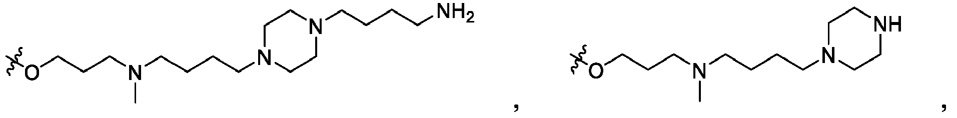 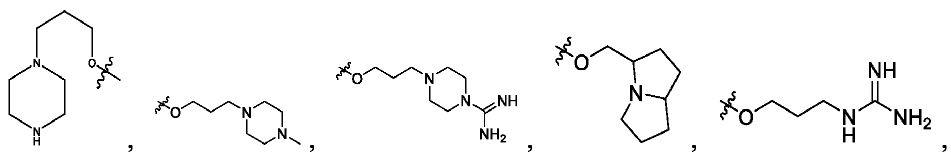 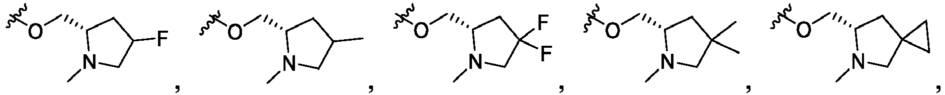 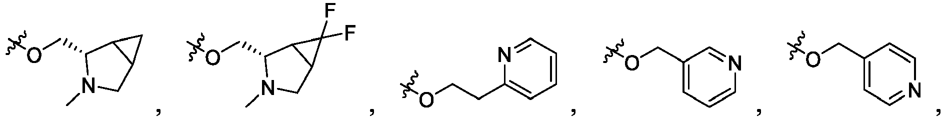 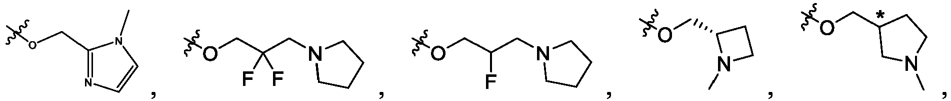 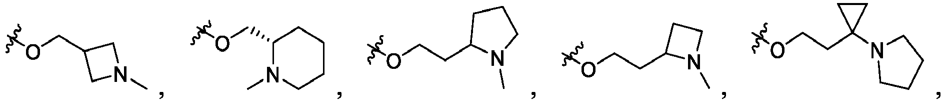 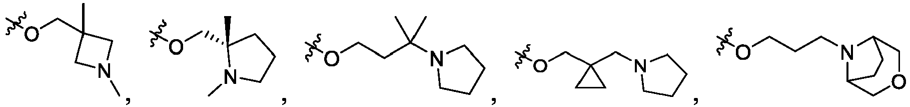 or 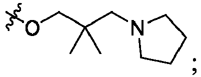
" 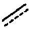 " in 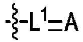 is a single bond; 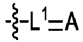
is

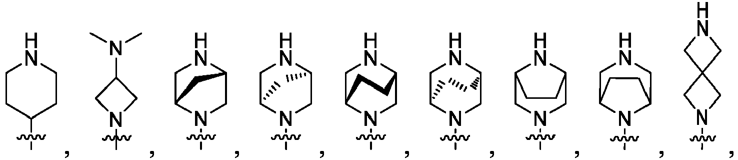

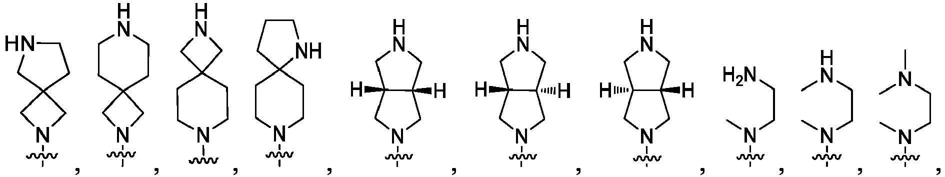

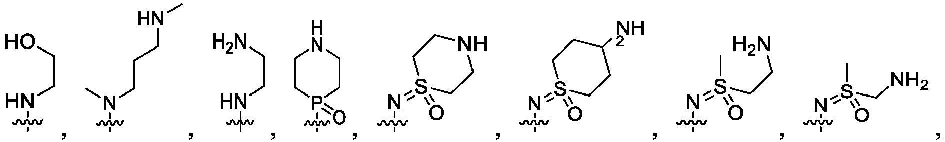

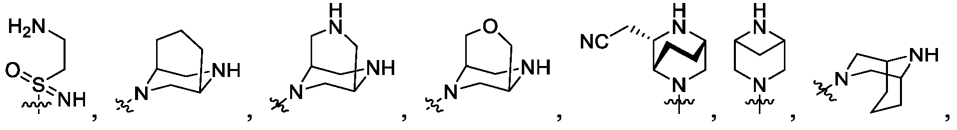

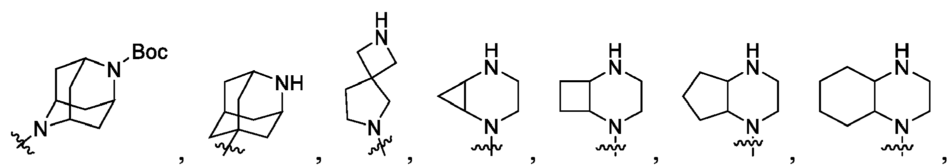

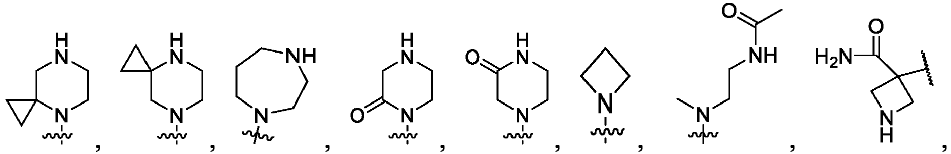

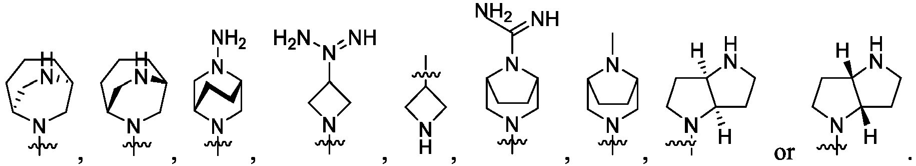

In a certain embodiment, the quinazoline compound represented by formula I is a compound represented by formula I-BI, 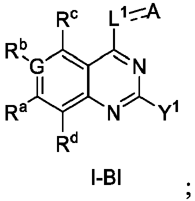
wherein, G is C or N;
R^{a} is 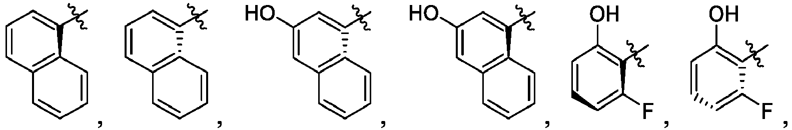 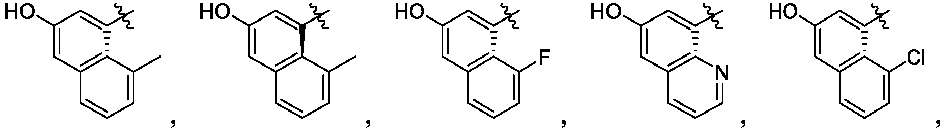 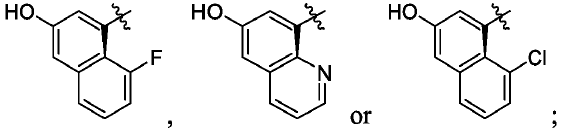
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 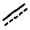 " in 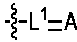 is a single bond; 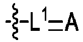
is 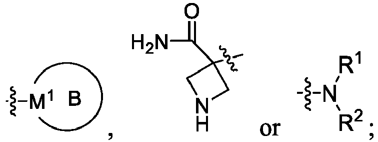
M¹ is Nor CH;
ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 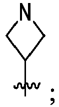
the 4- to 10-membered monocyclic heterocycloalkyl with 1 to 3 heteroatoms or heteroatom groups selected from N and P(=O) and containing only one N can be 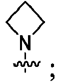
the 4- to 10-membered bridged heterocycloalkyl substituted by R¹⁰ with 1 to 3 heteroatoms or heteroatom groups selected from N and P(=O) and containing only one N can be 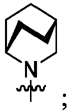
the 7- to 12-membered bridged heterocycloalkyl substituted by R⁷ with 2 to 3 heteroatoms selected from one or more of N, O and S can be 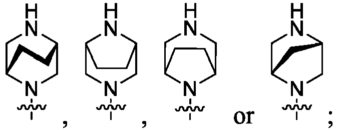
R¹ is H, C₁-C₃ alkyl, or C₄-C₆ alkyl;
R² is -(CH₂)ₙ-R^{2a};
n is 1 or 2;
R^{2a} is 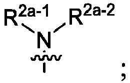
R^{2a-1} and R^{2a-2} are independently H, -NH-C(=O)CH₃ or C₁-C₆ alkyl;
R¹⁰ is amino or 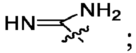
R⁷ is 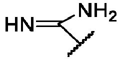 or C₁-C₆ alkyl;
R¹¹ is 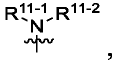 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 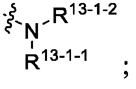
R¹³⁻¹⁻¹ and R¹³⁻¹⁻² are independently H or C₁-C₆ alkyl;
R¹⁴ is C₁-C₆ alkyl substituted by 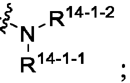
R¹⁴⁻¹⁻¹ and R¹⁴⁻¹⁻² are independently H or C₁-C₆ alkyl.

In a certain embodiment, the quinazoline compound represented by formula I is a compound represented by formula I-BI, 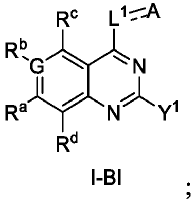
G is C or N;
R^{a} is 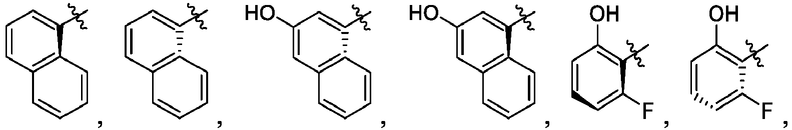 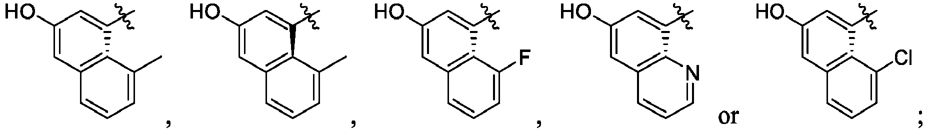
R^{b} is halogen;
R^{c} is H;
R^{d} is halogen;
Y¹ is 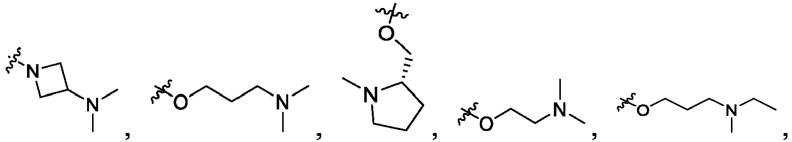 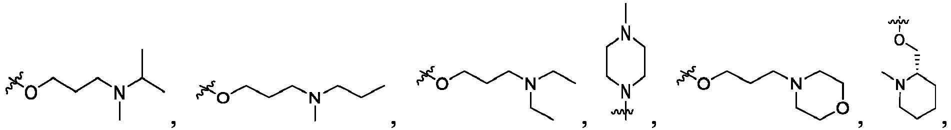 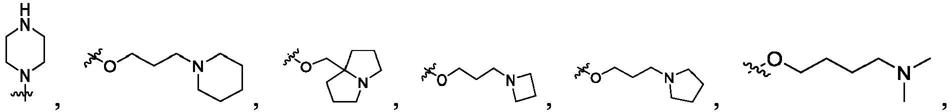 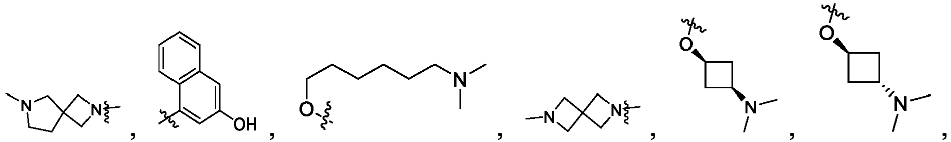 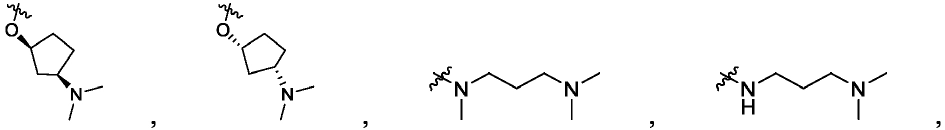 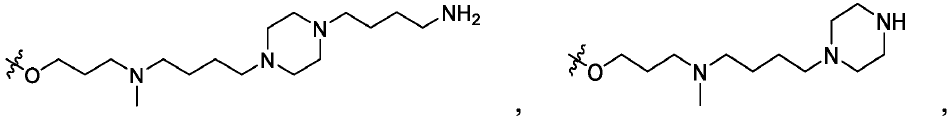 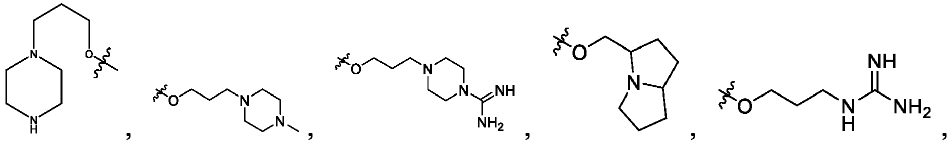 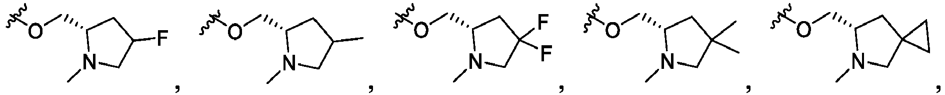 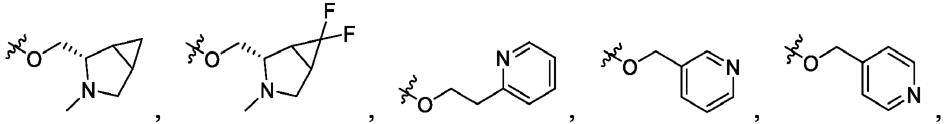 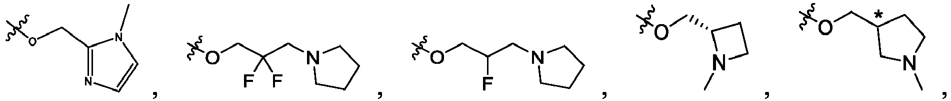 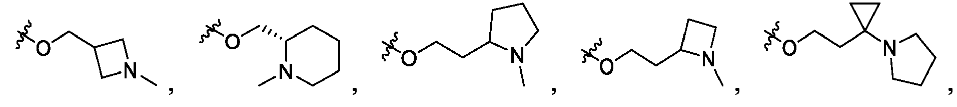 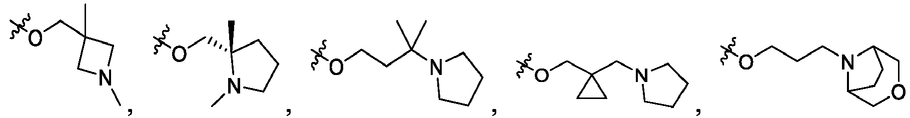 or 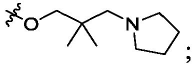
" 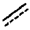 " in 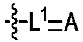 is a single bond; 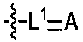
is

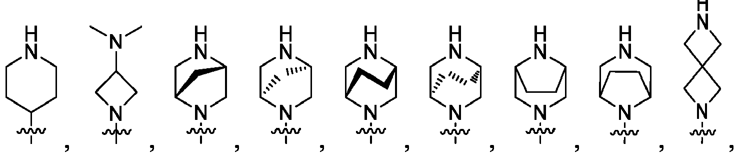

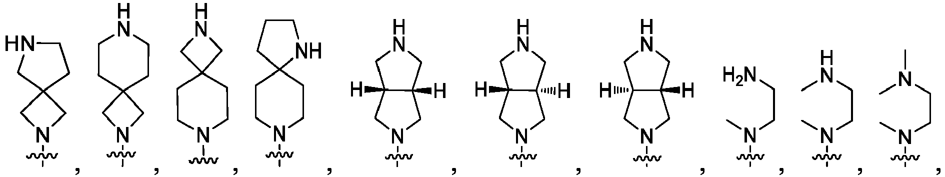

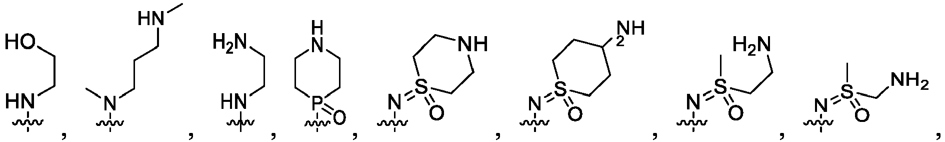

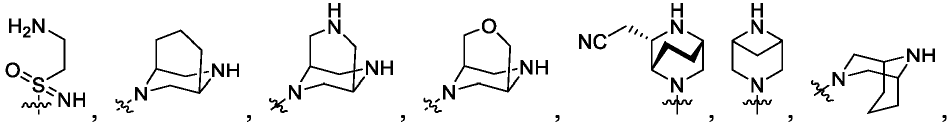

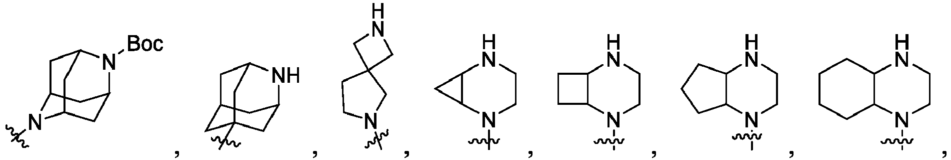

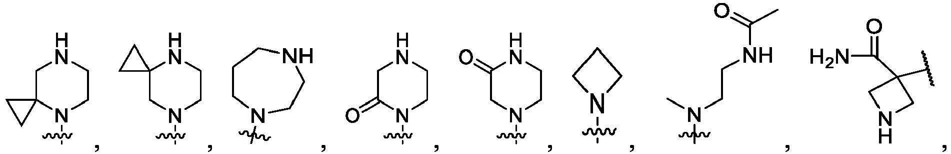

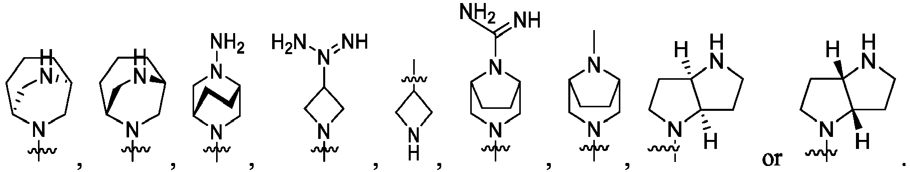

In a certain embodiment, the quinazoline compound represented by formula I is a compound represented by formula I-BI, 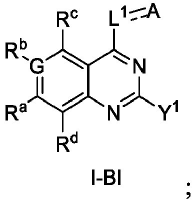
Gis C or N;
R^{a} is 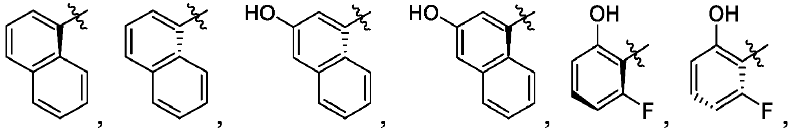 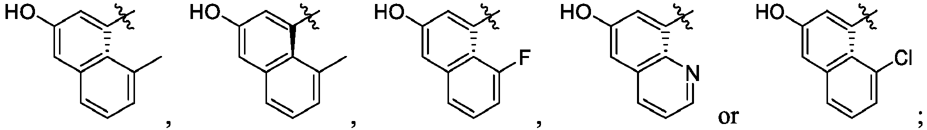
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 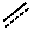 " in 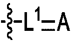 is a single bond; 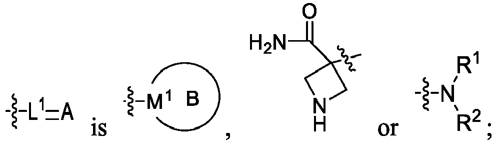
M¹ is Nor CH;
ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 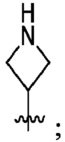
the 4- to 10-membered monocyclic heterocycloalkyl with 1 to 3 heteroatoms or heteroatom groups selected from N and P(=O) and containing only one N can be 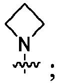
the 4- to 10-membered bridged heterocycloalkyl substituted by R10 with 1 to 3 heteroatoms or heteroatom groups selected from N and P(=O) and containing only one N can be 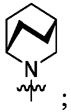
the 7- to 12-membered bridged heterocycloalkyl substituted by R7 with 2 to 3 heteroatoms selected from one or more of N, O and S can be 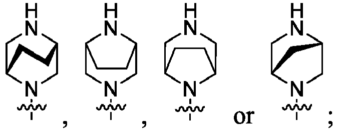
R¹ is H or C₁-C₆ alkyl;
R² is -(CH₂)ₙ-R^{2a};
n is 1 or 2;
R^{2a} is 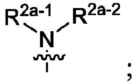
R^{2a-1} and R^{2a-2} are independently H, -NH-C(=O)CH₃ or C₁-C₆ alkyl;
R¹⁰ is amino or 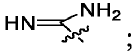
R⁷ is 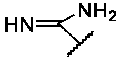 or C₁-C₆ alkyl;
R¹¹ is 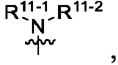 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 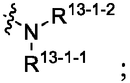
R¹³⁻¹⁻¹ and R¹³⁻¹⁻² are independently H or C₁-C₆ alkyl;
R¹⁴ is C₁-C₆ alkyl substituted by 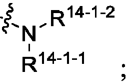
R¹⁴⁻¹⁻¹ and R¹⁴⁻¹⁻² are independently H or C₁-C₆ alkyl.

In a certain embodiment, the quinazoline compound represented by formula I is a compound represented by formula I-BI, 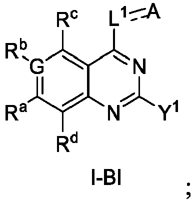
Gis C or N;
R^{a} is 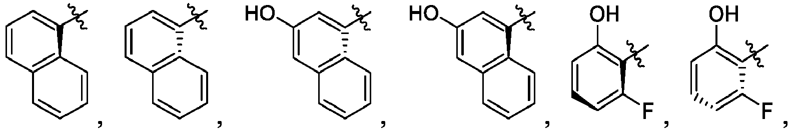 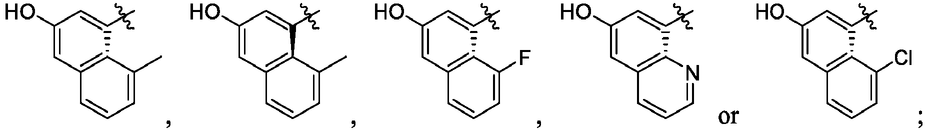
R^{b} is halogen;
R^{c} is H;
R^{d} is halogen;
Y¹ is 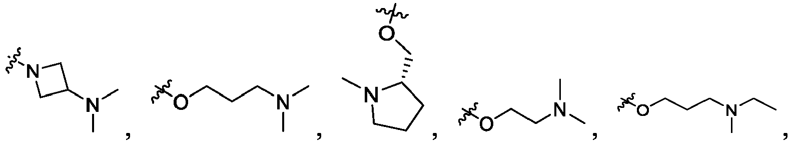 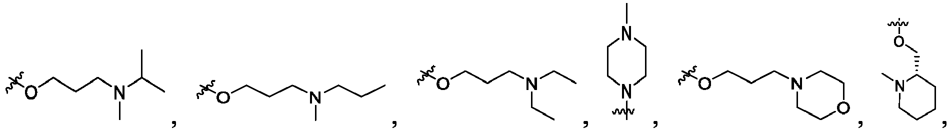 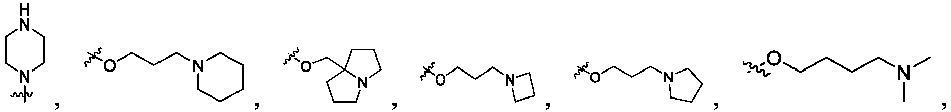 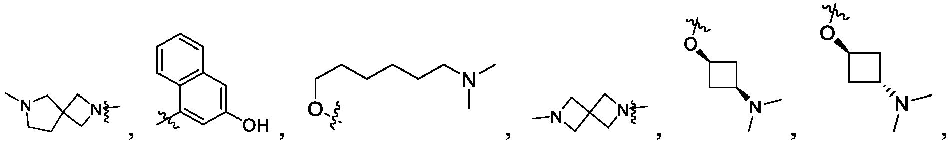 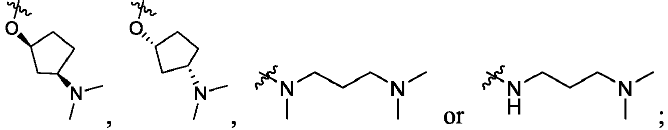 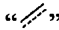
in 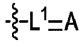 is a single bond; 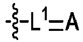
is 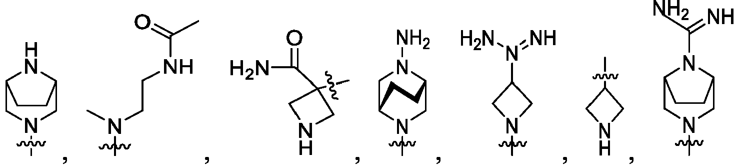 or

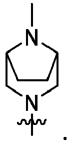

In a certain embodiment, the quinazoline compound represented by formula I, the pharmaceutically acceptable salt thereof, the solvate thereof, the prodrug thereof, the metabolite thereof or the isotopic compound thereof is any of the following structures, 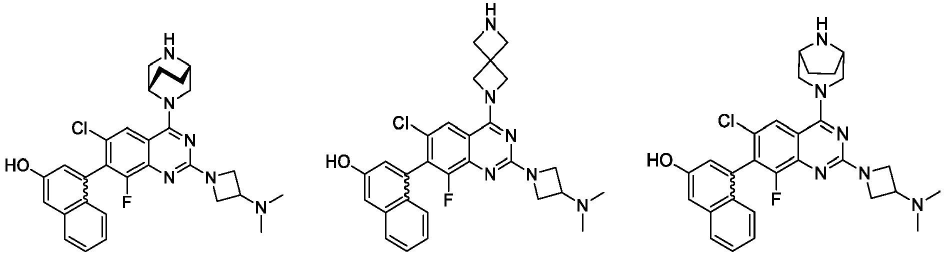 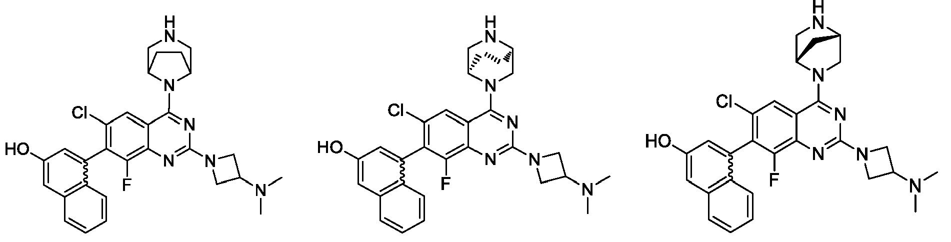 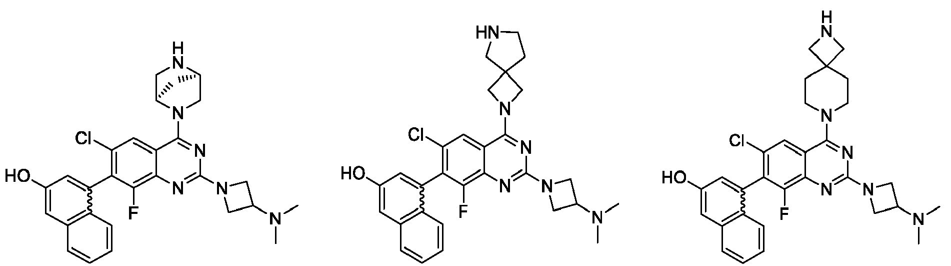 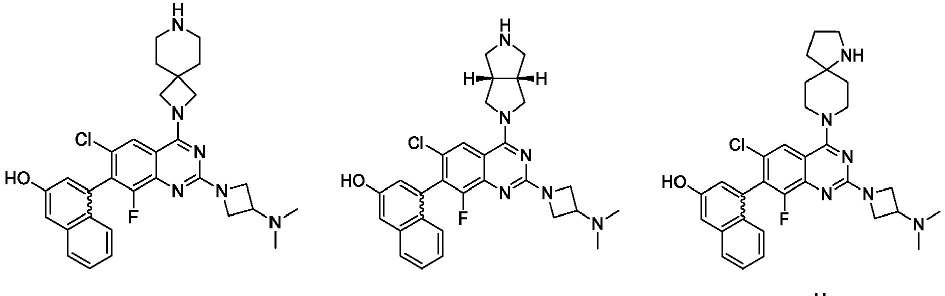 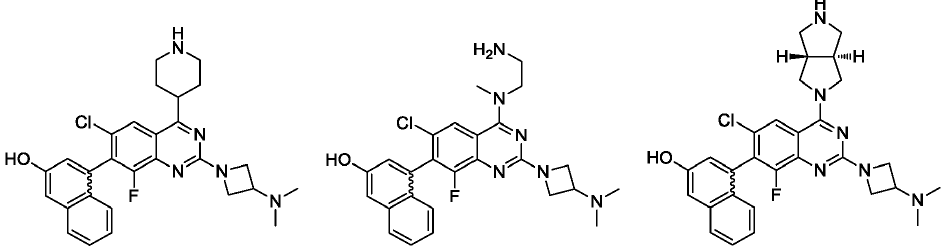 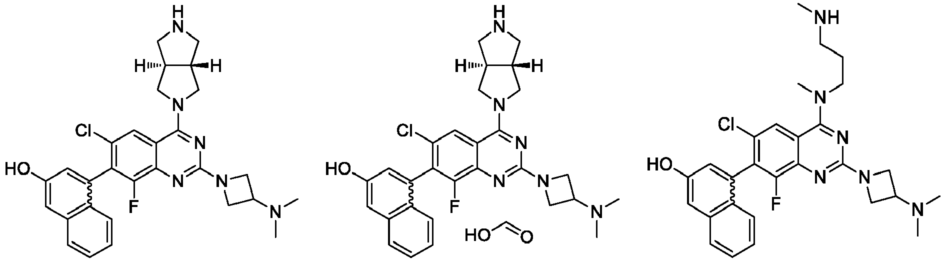 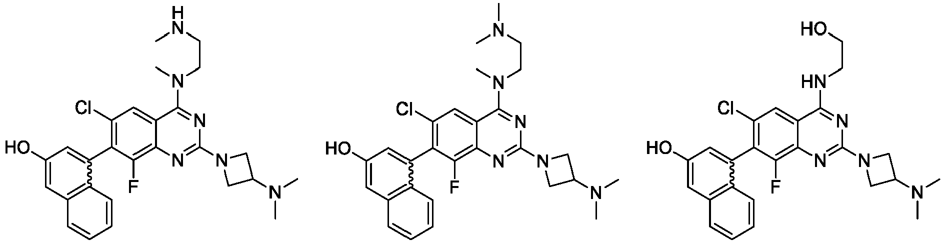 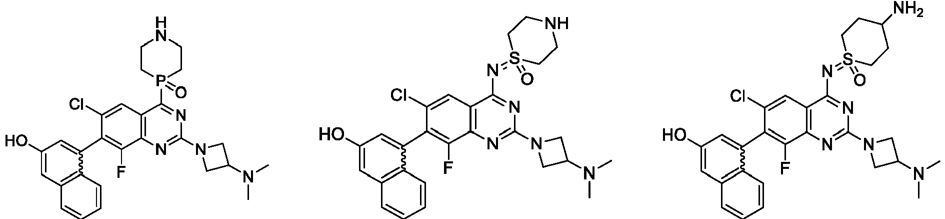 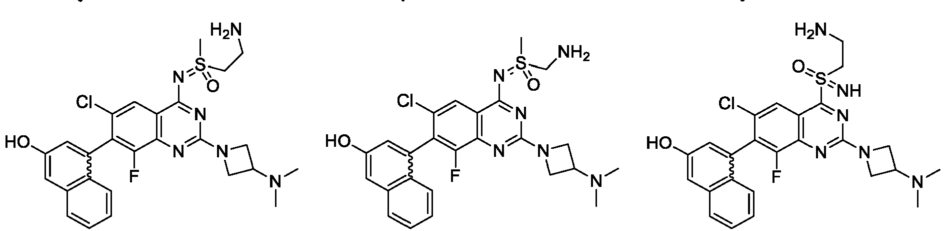 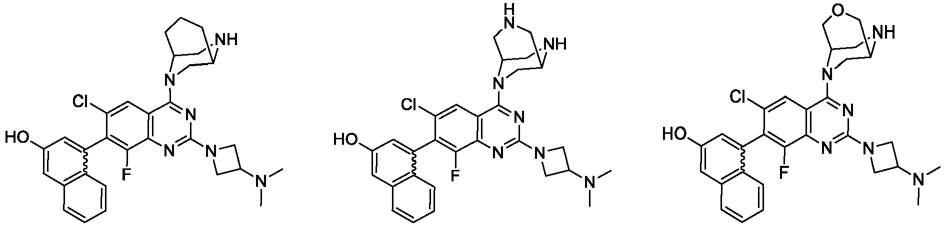 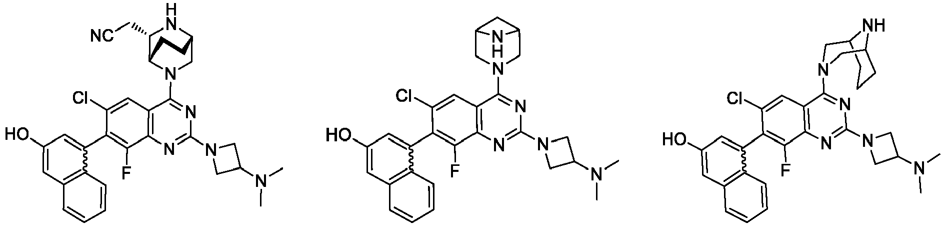 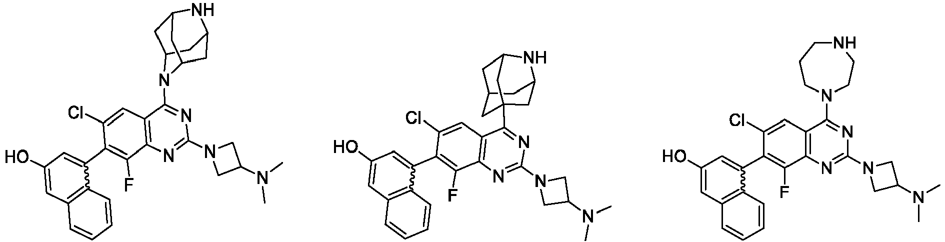 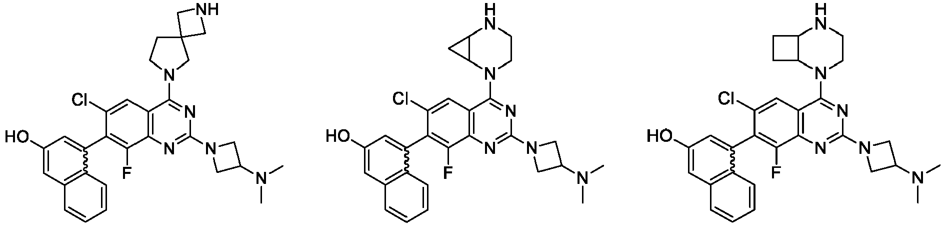 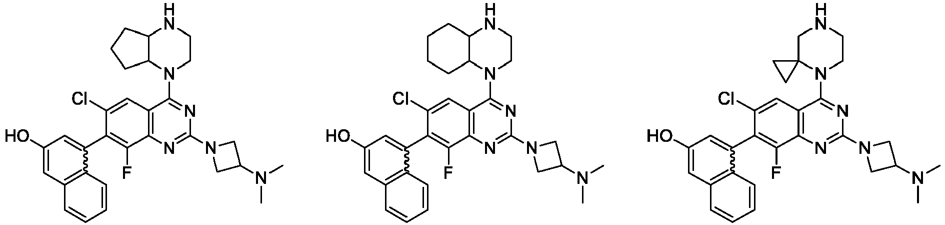 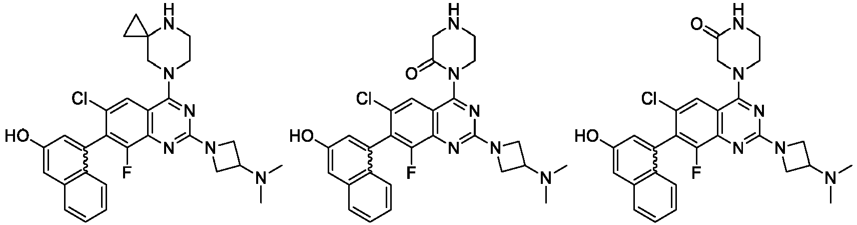 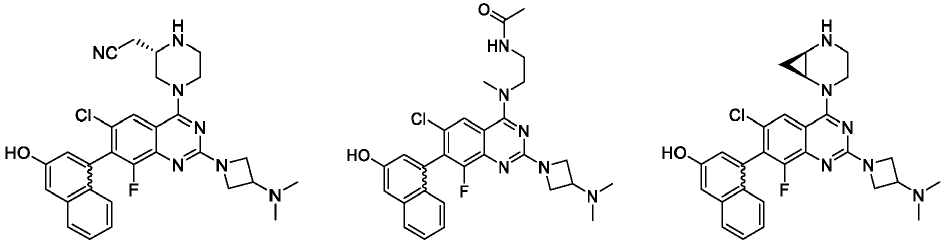 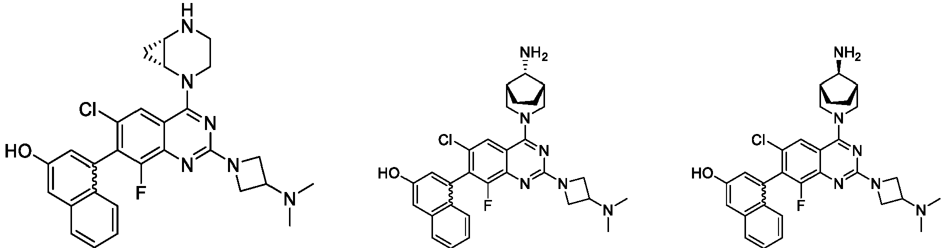 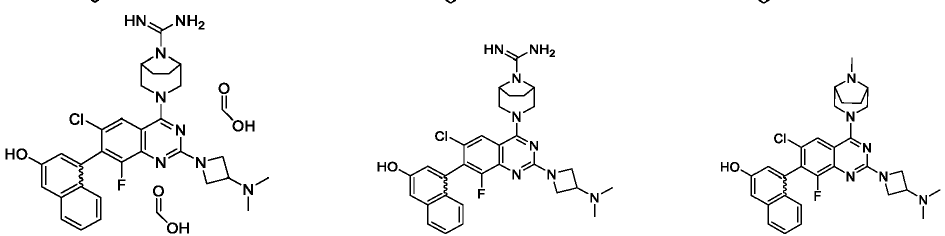 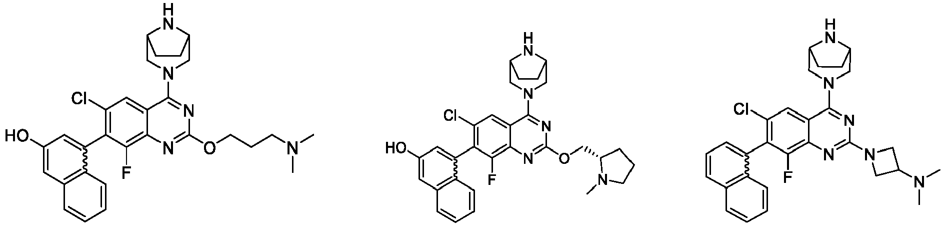 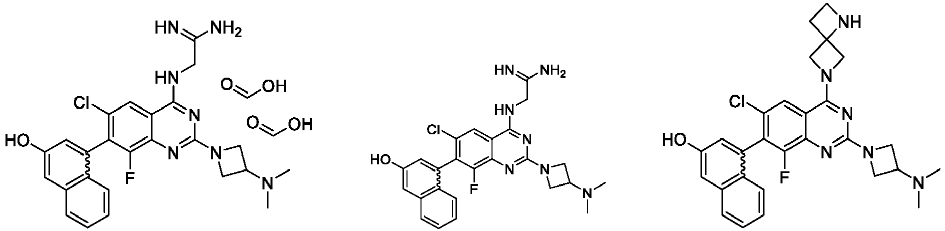 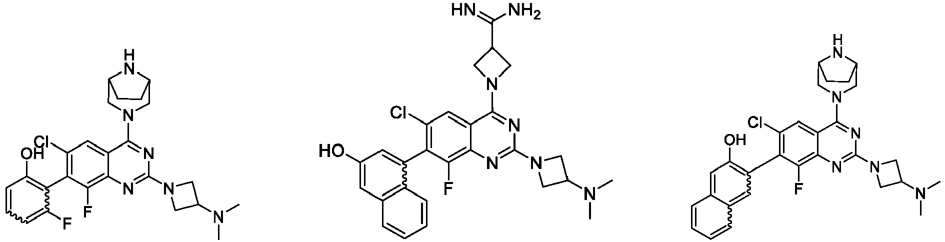 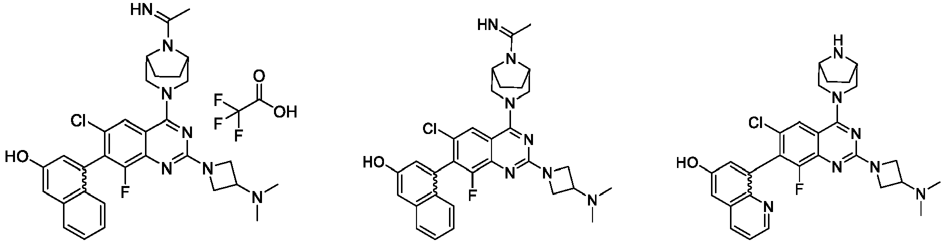 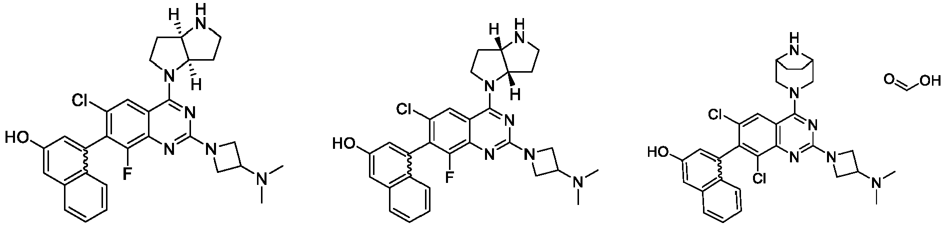 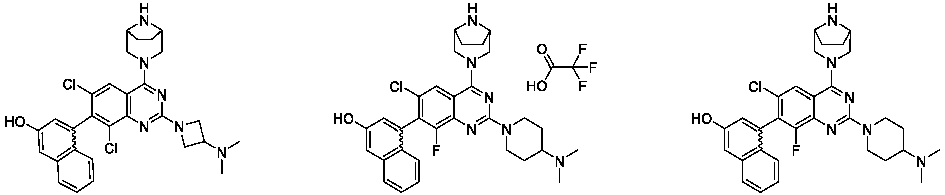 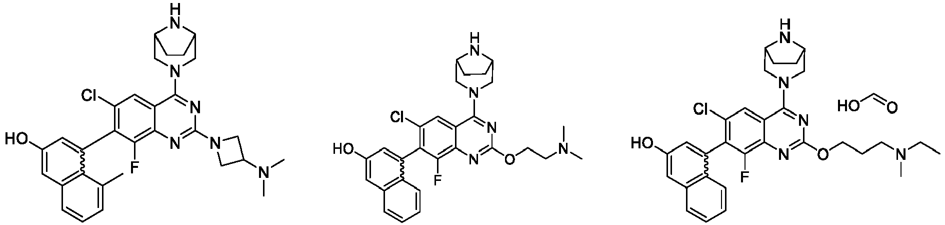 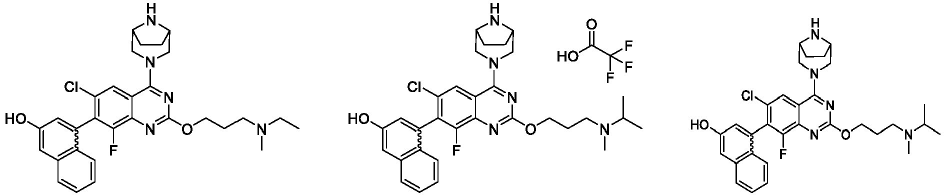 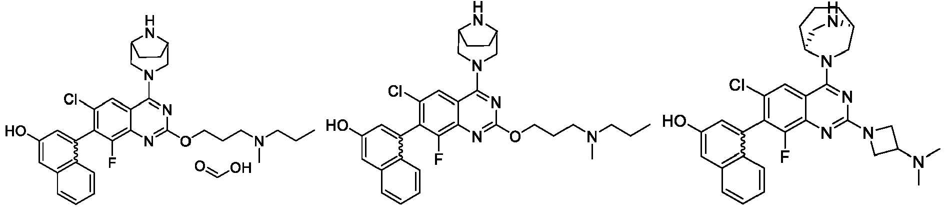 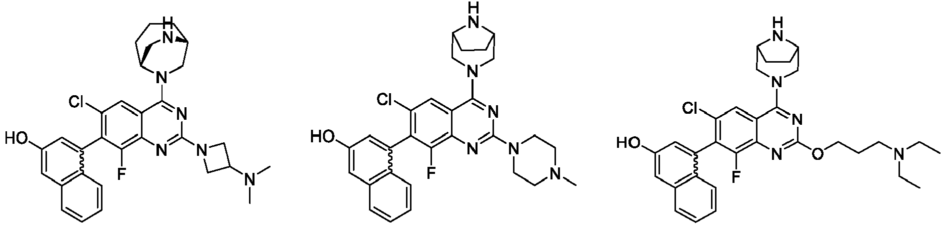 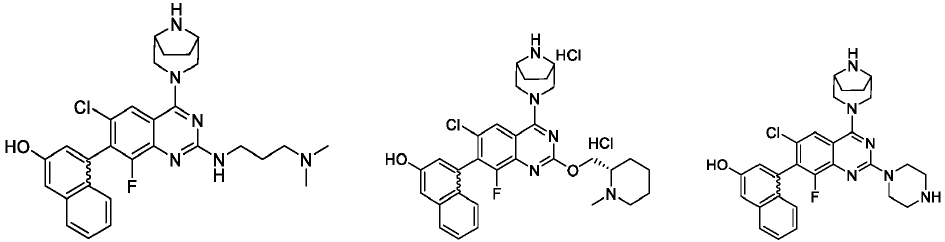 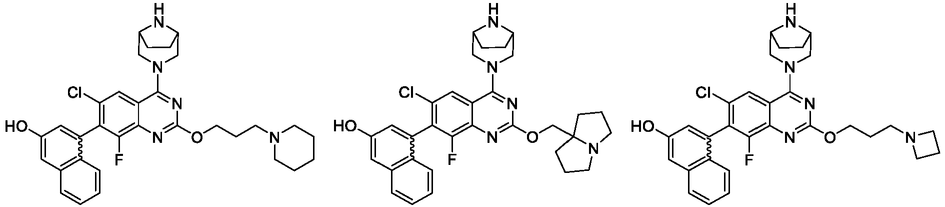 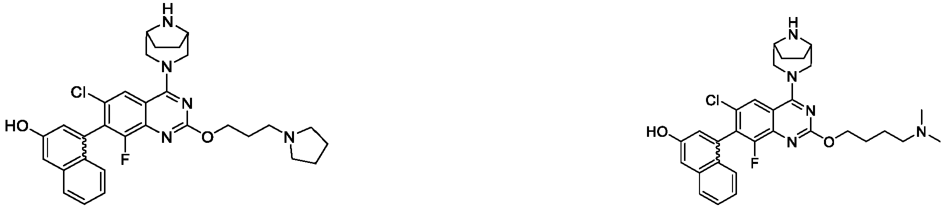 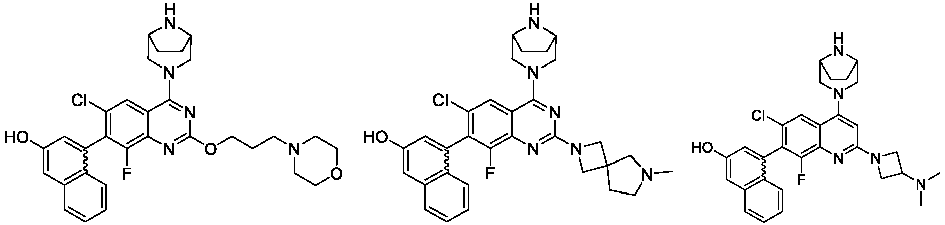 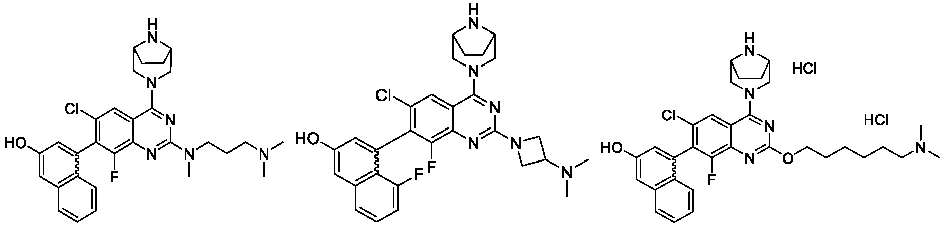 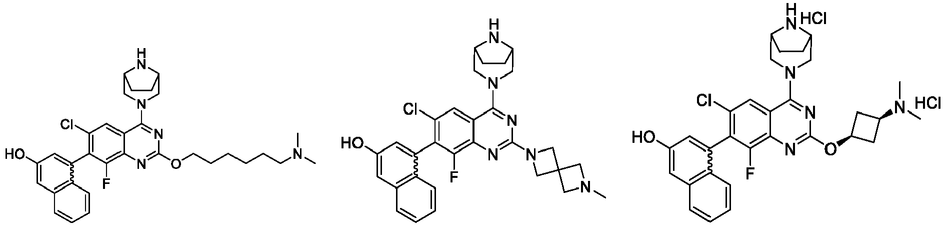 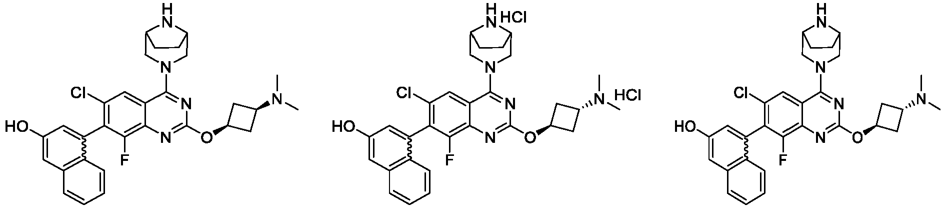 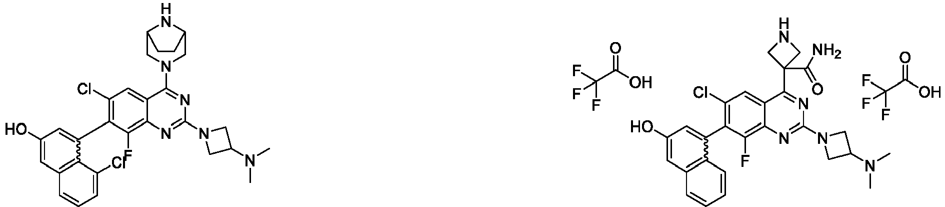 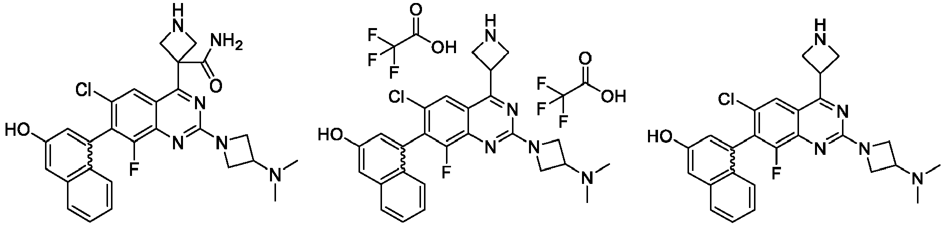 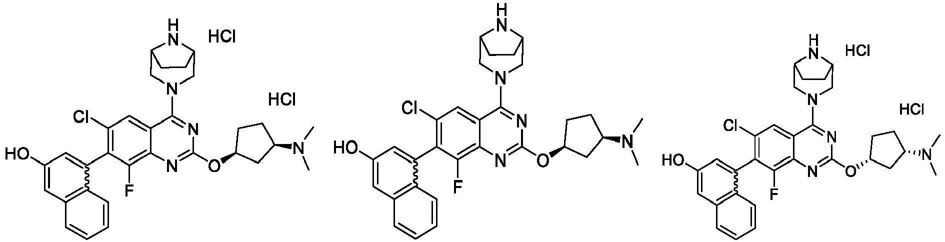 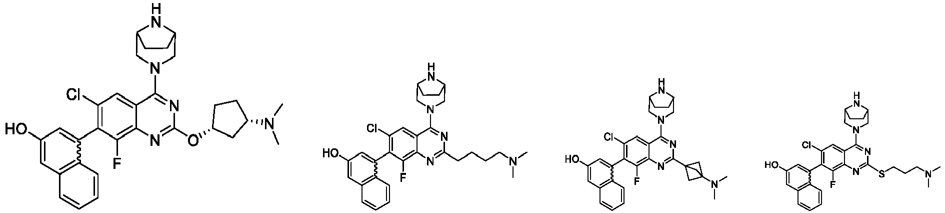 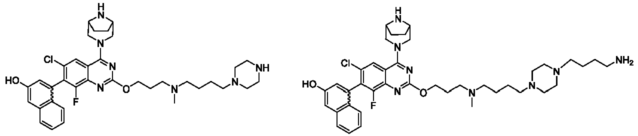 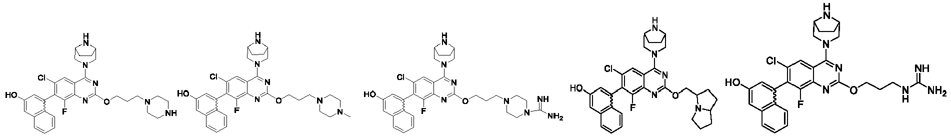 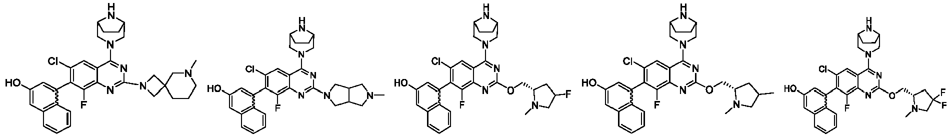 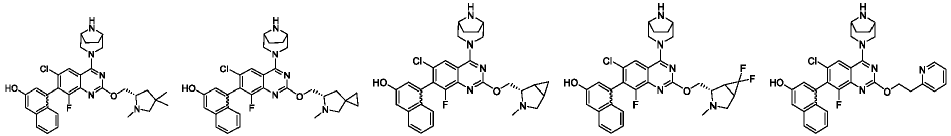 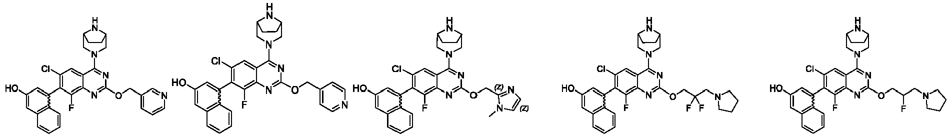 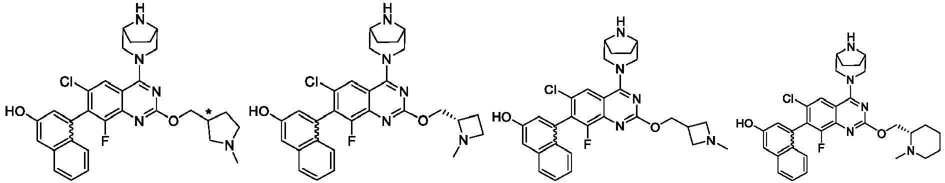 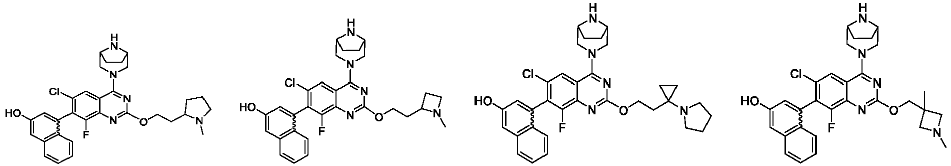 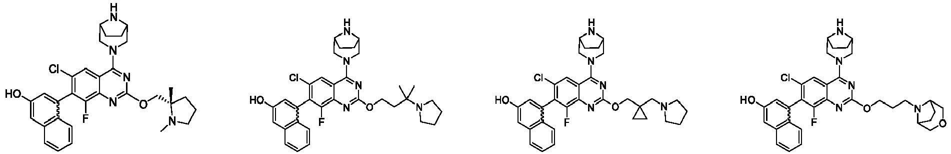 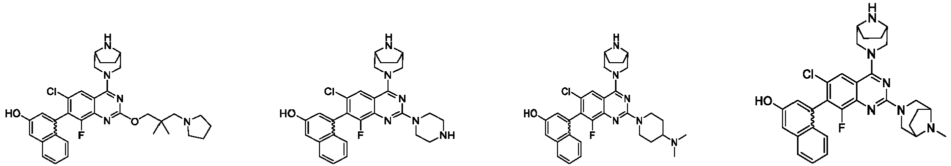 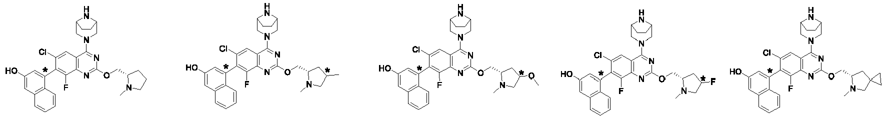 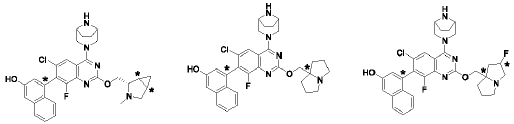 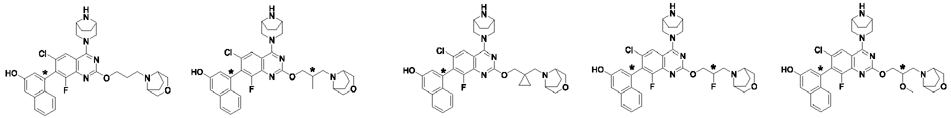 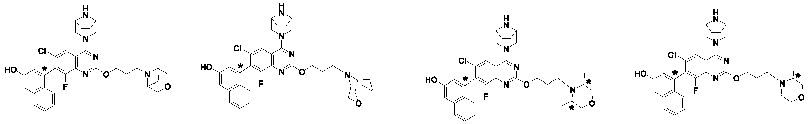 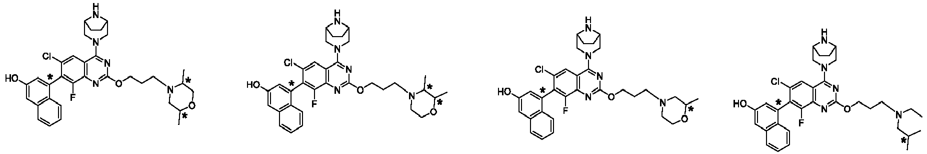 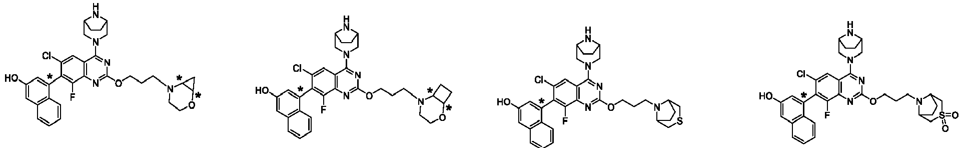 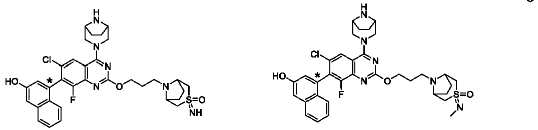 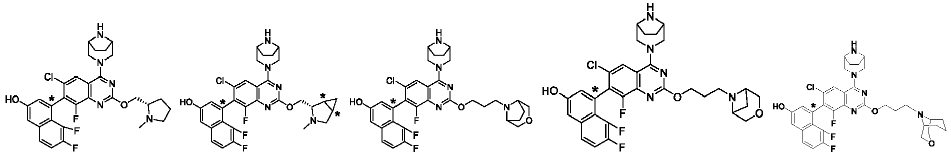 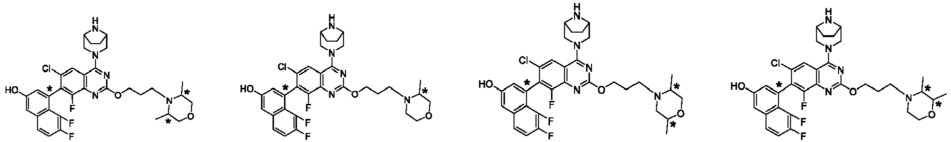 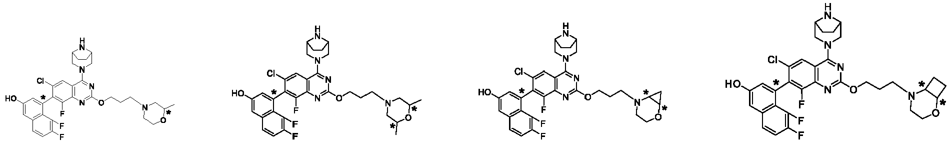 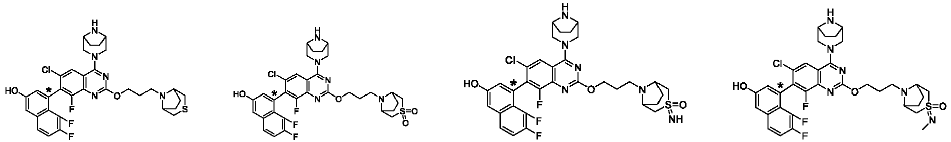 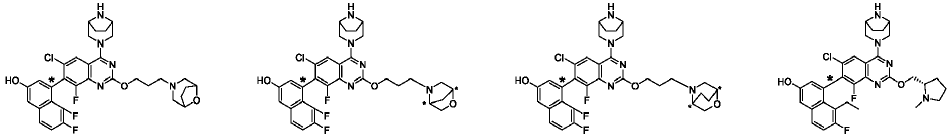 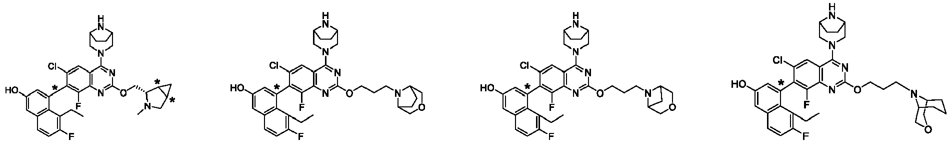 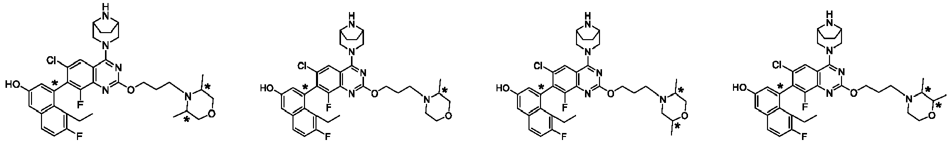 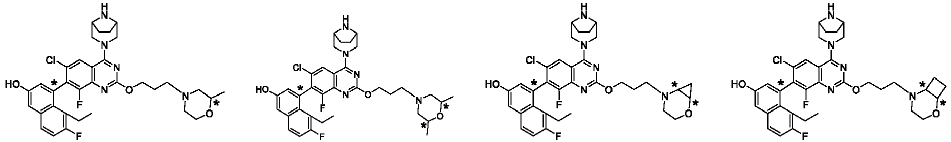 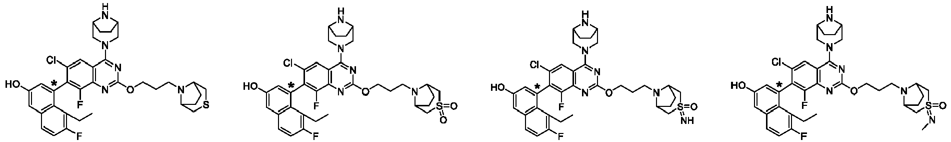 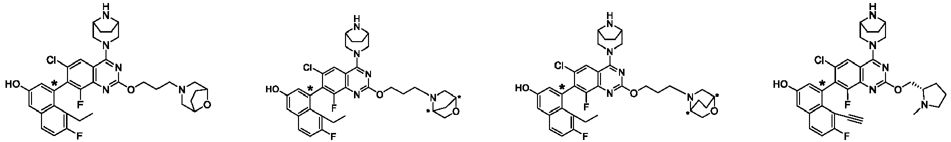 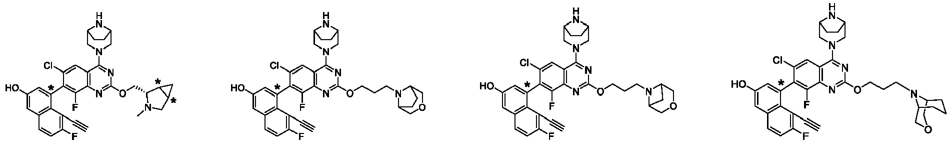 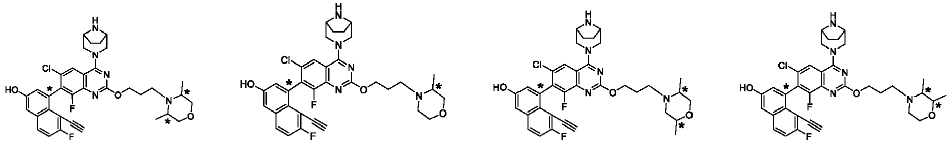 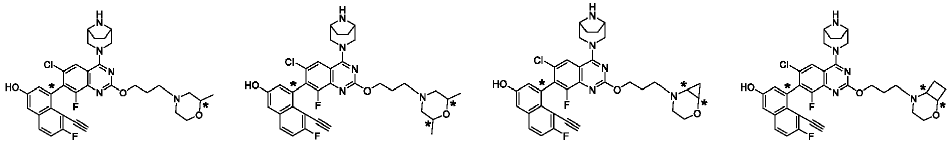 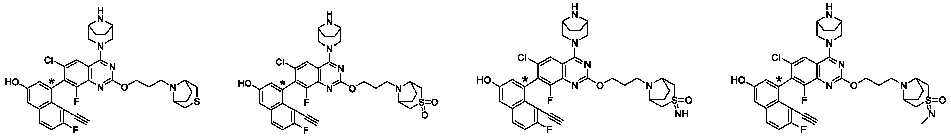 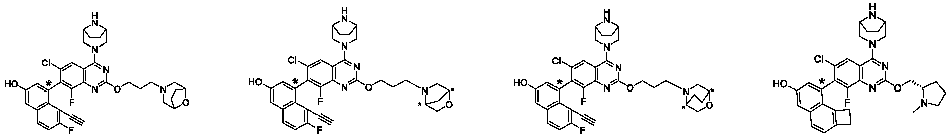 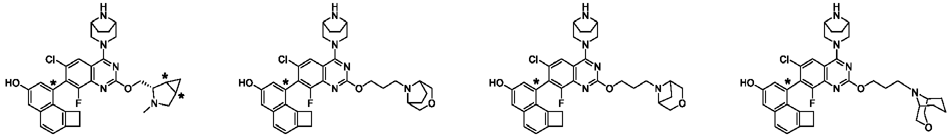 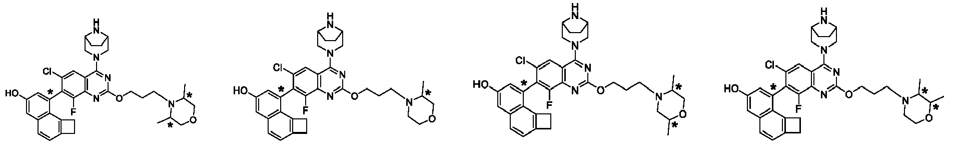 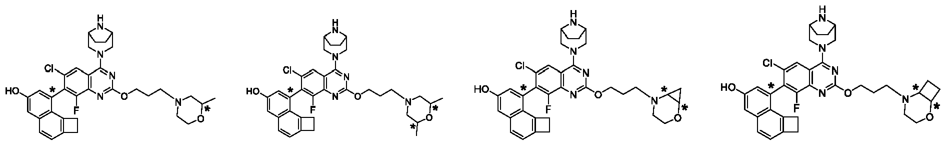 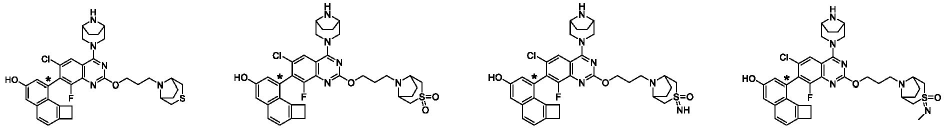 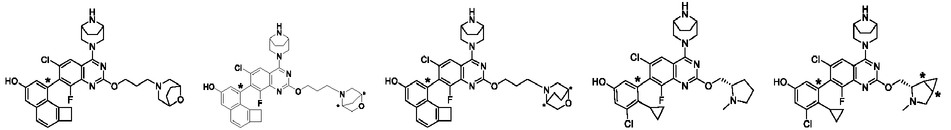 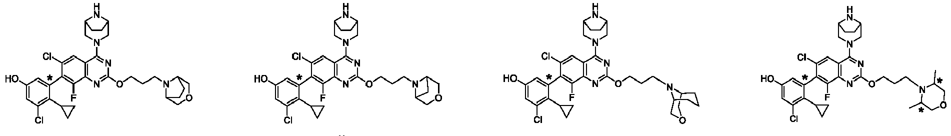 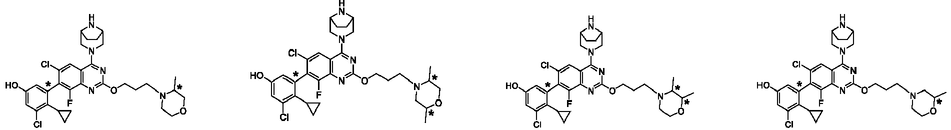 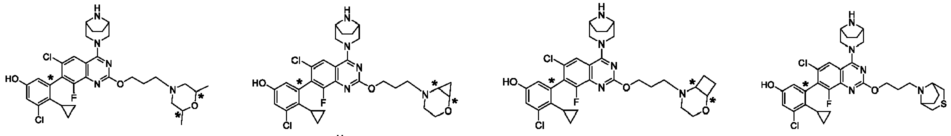 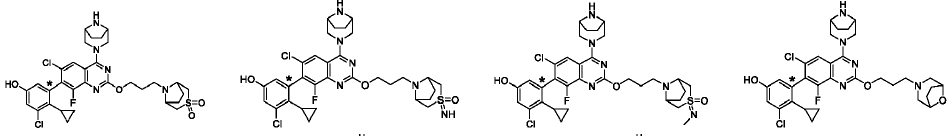 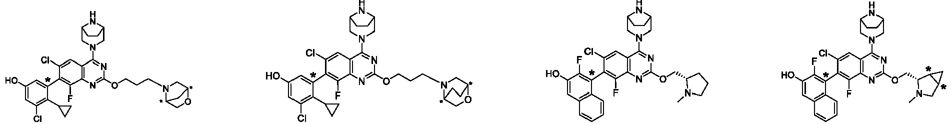 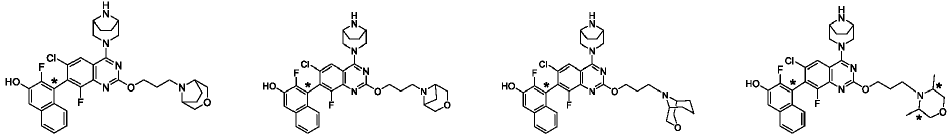 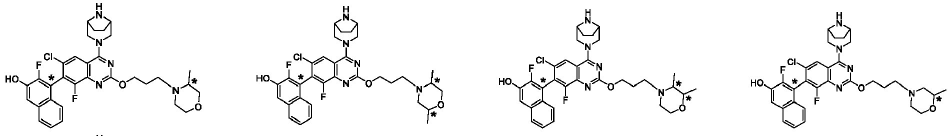 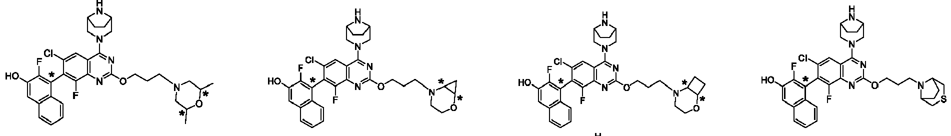 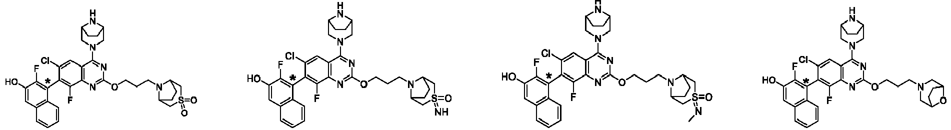 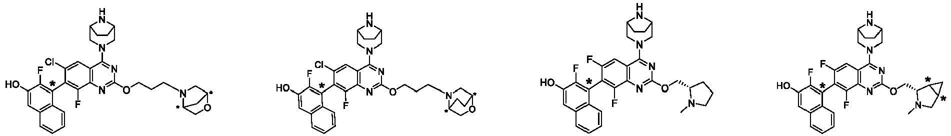 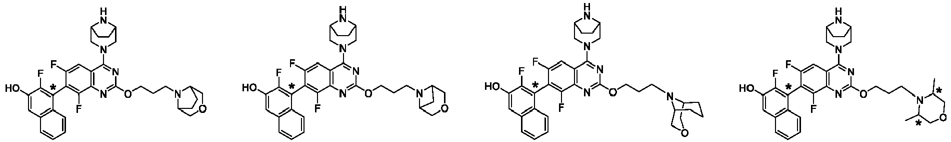 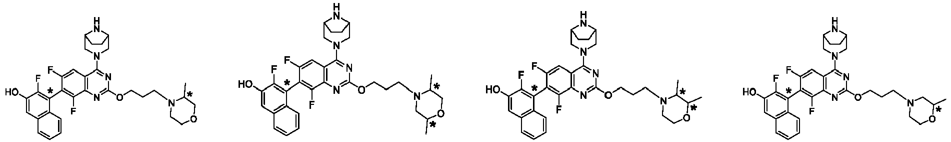 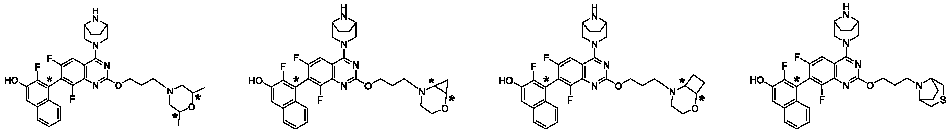 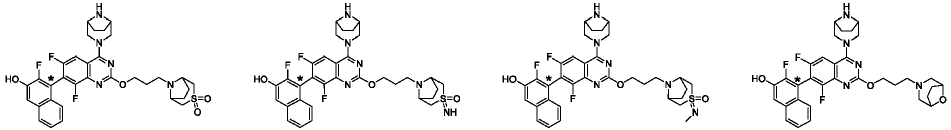 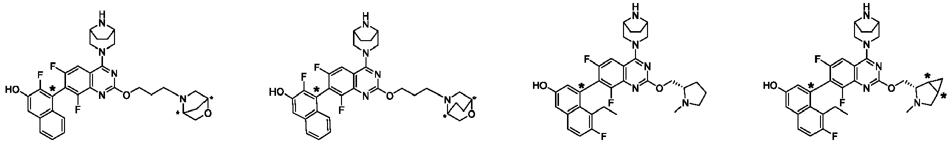 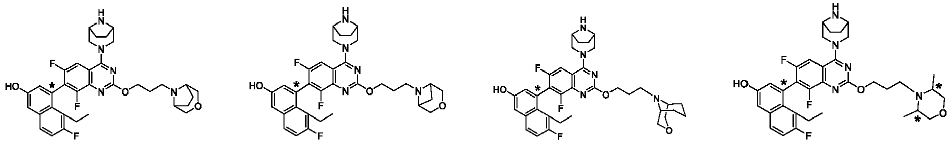 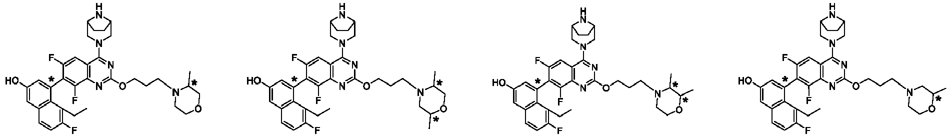 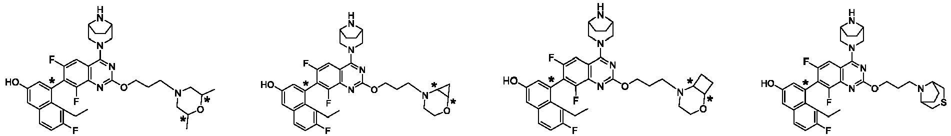 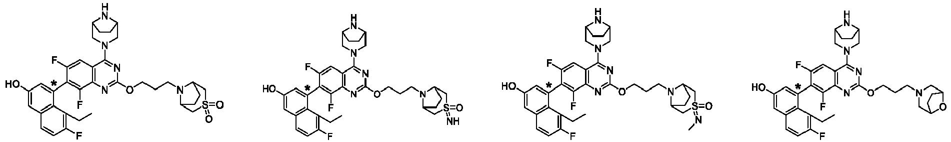 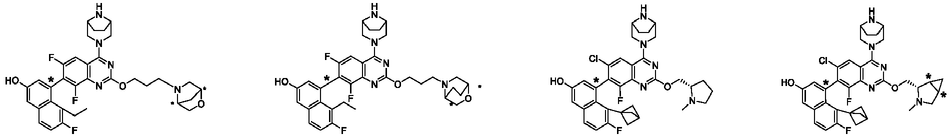 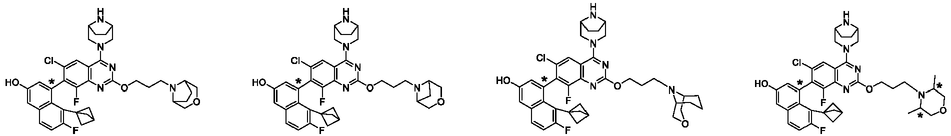 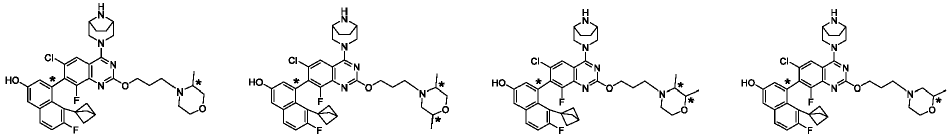 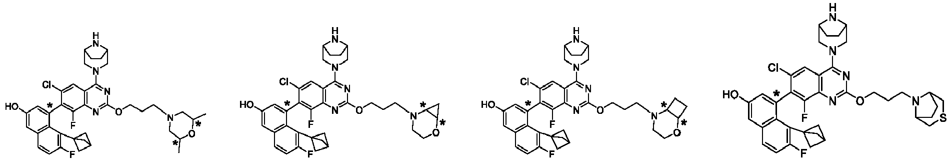 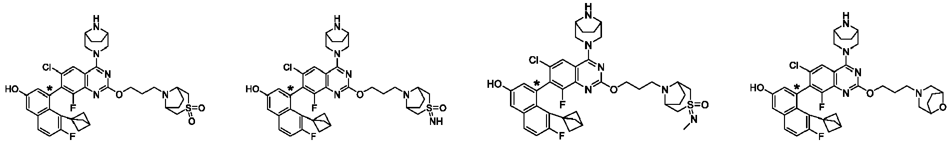 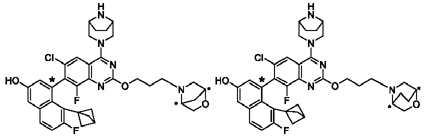 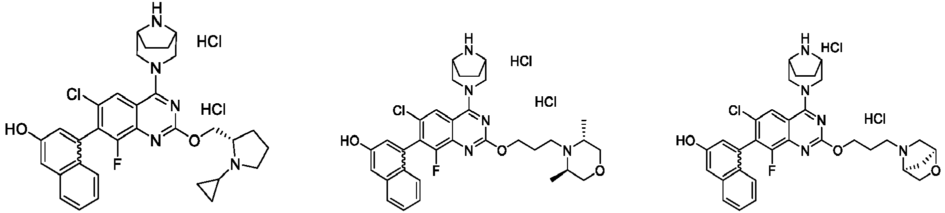 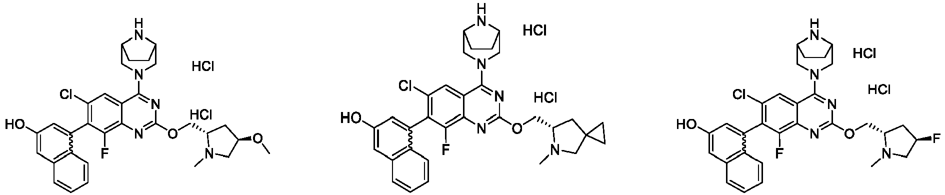 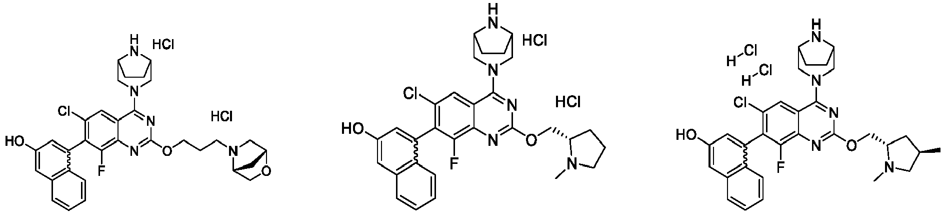 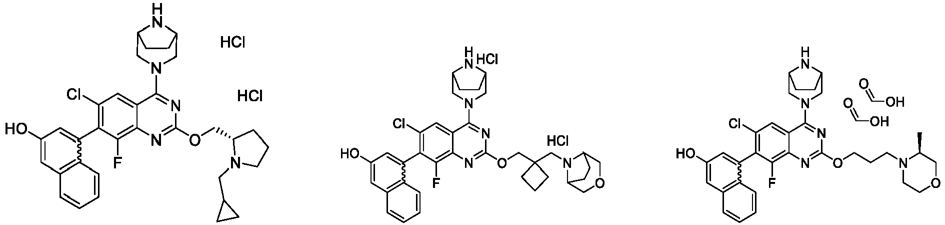 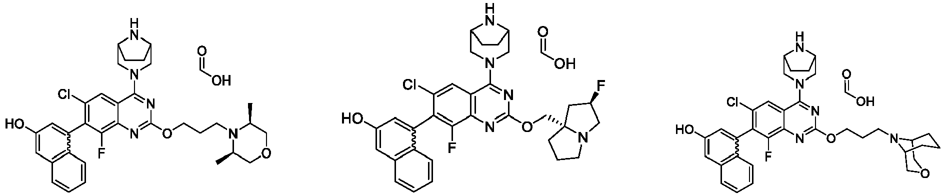 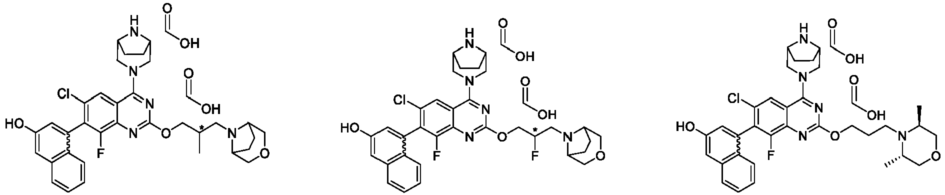 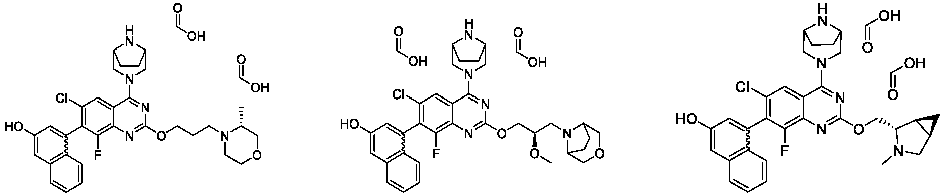 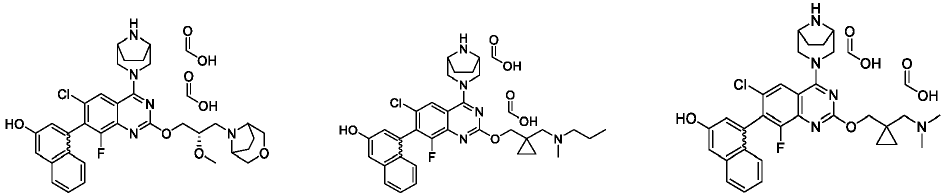 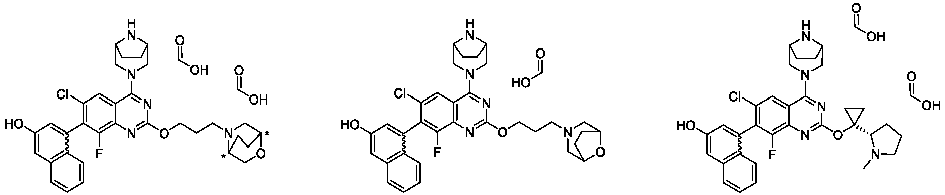 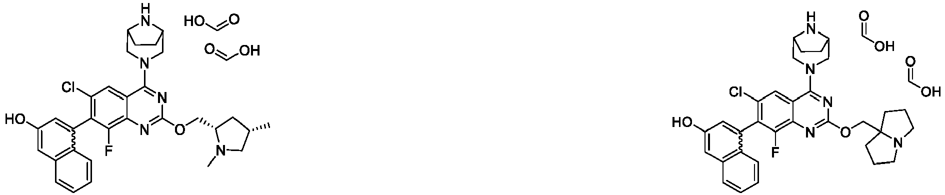 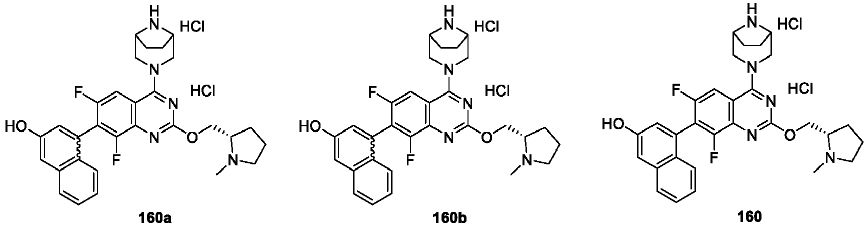 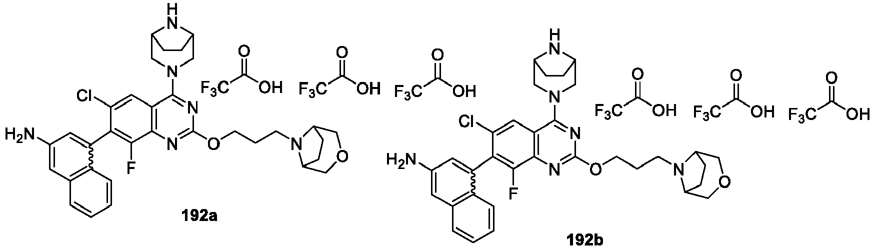 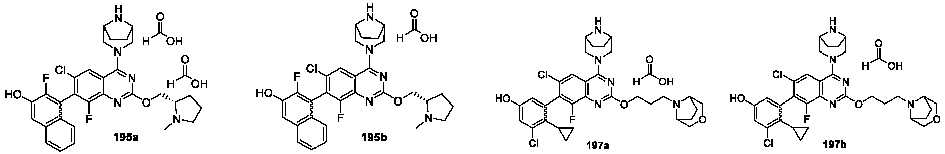 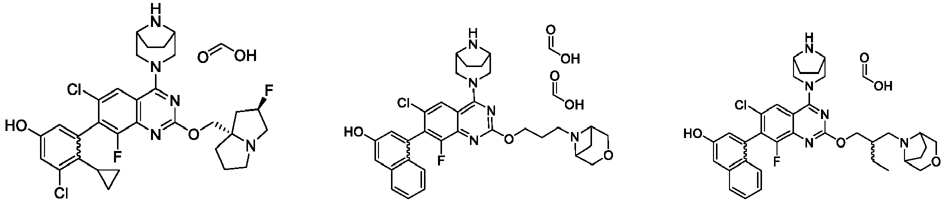 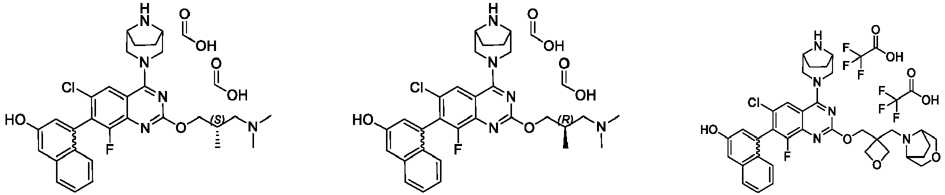 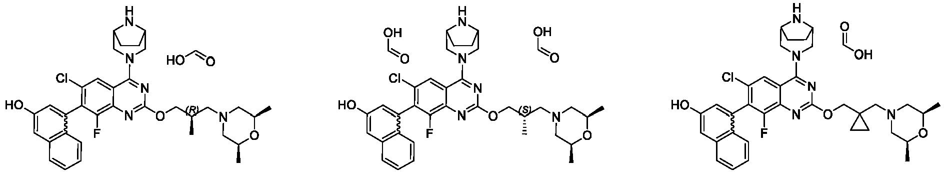 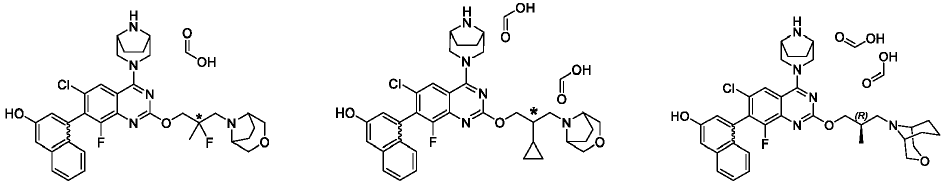 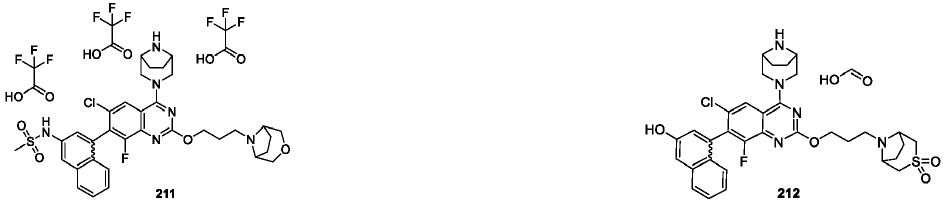 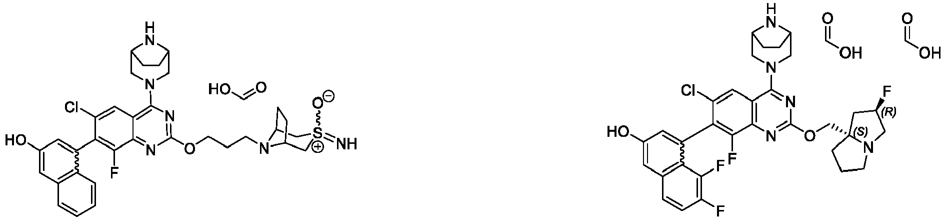 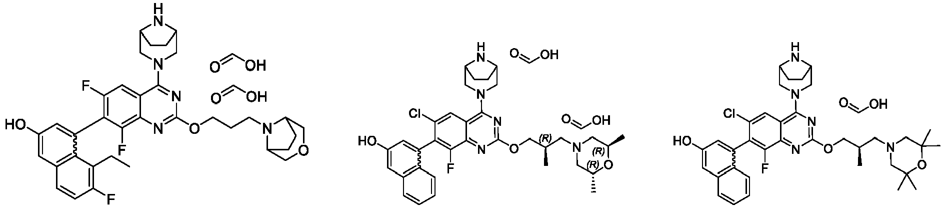 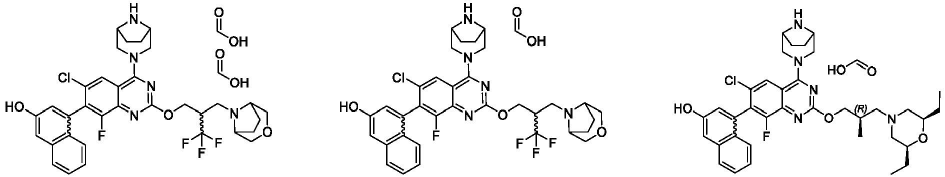 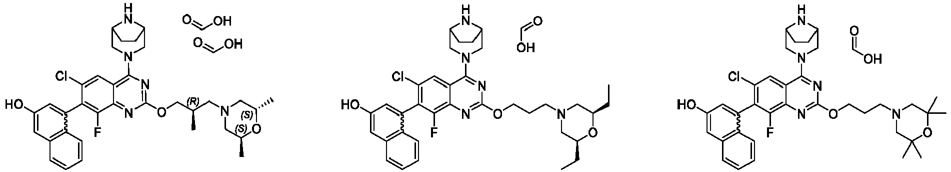 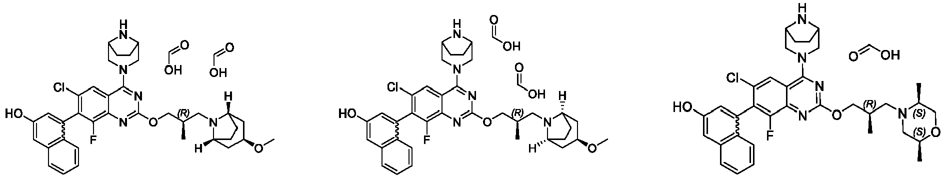 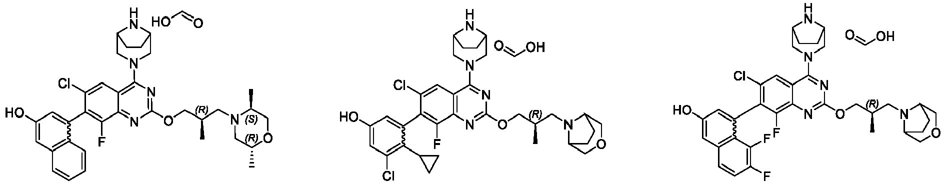 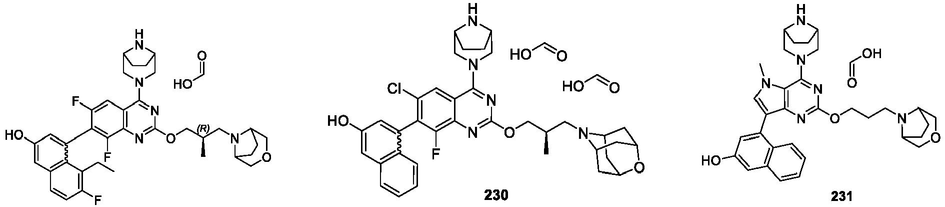 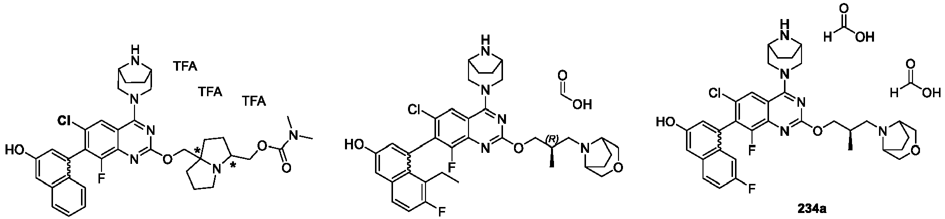 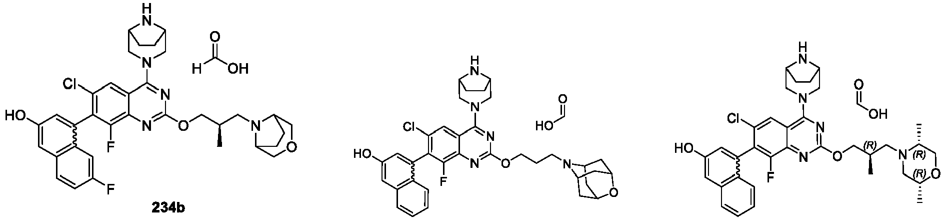 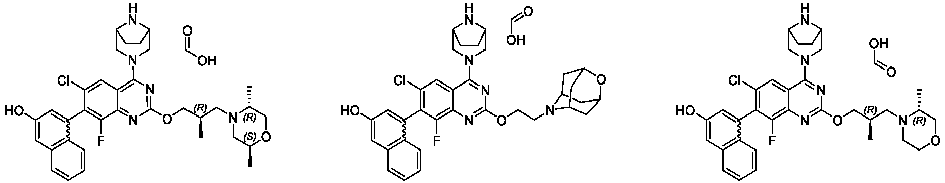 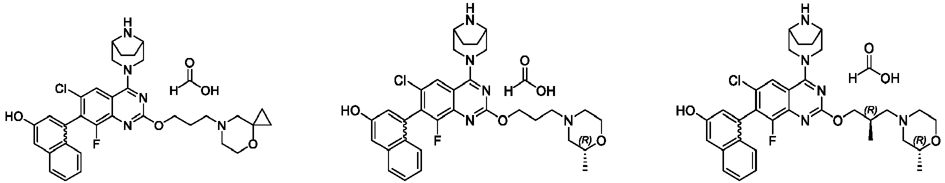 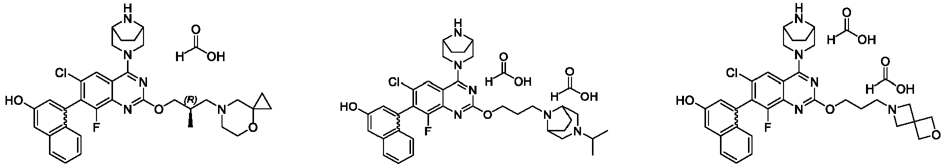 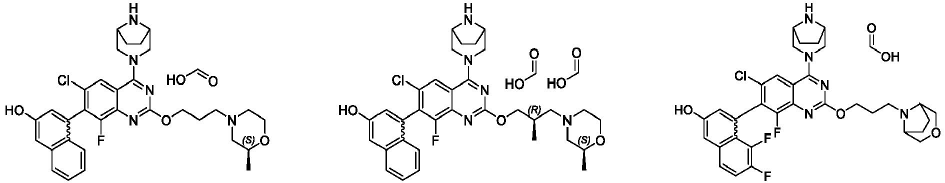 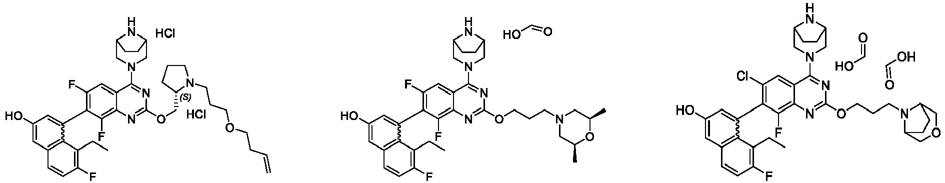 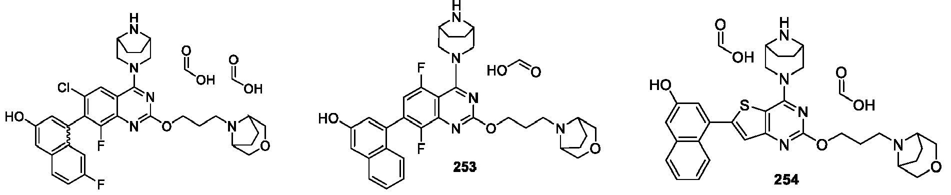 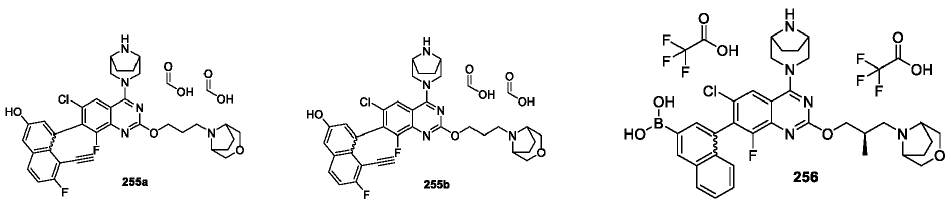 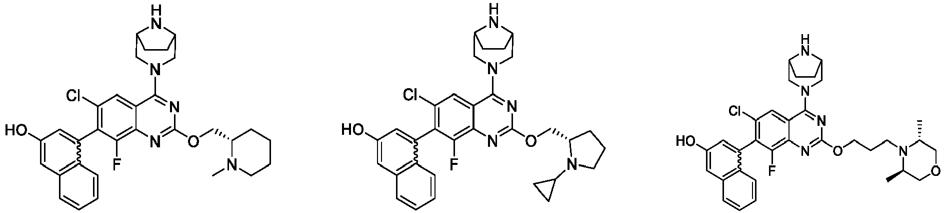 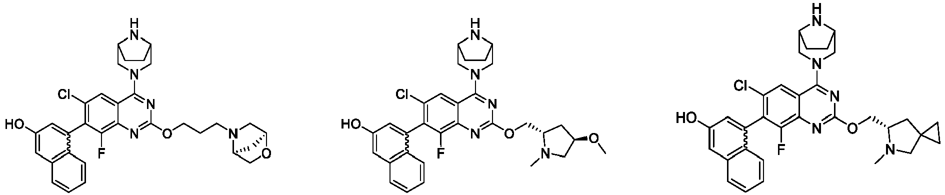 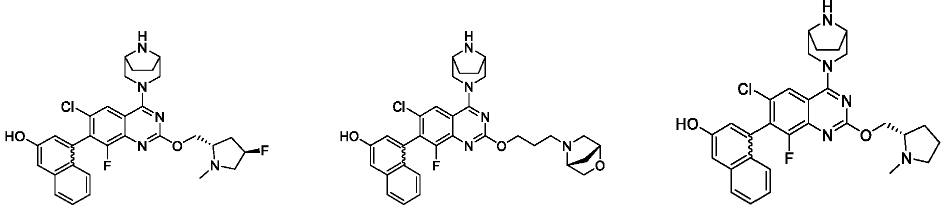 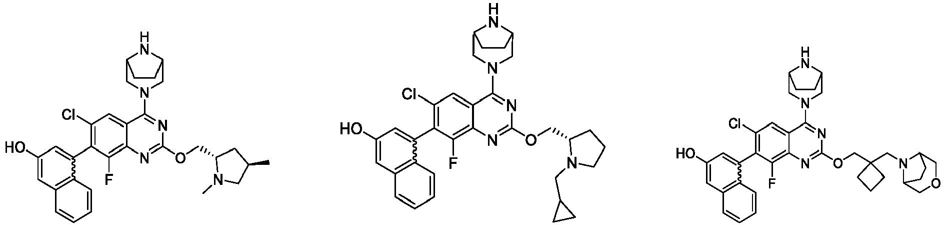 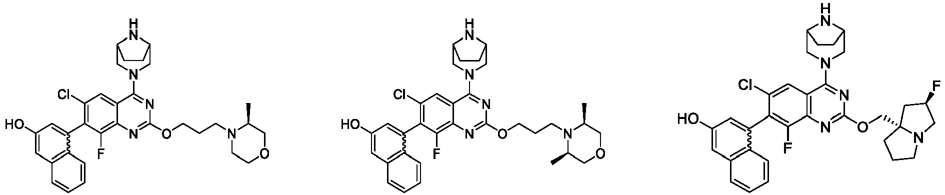 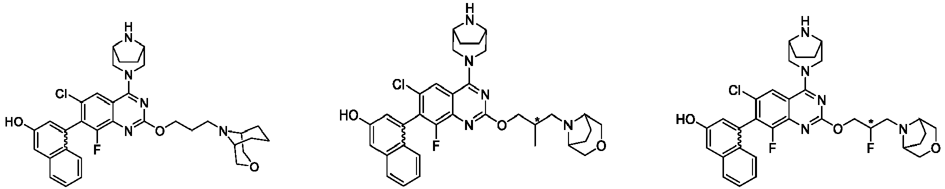 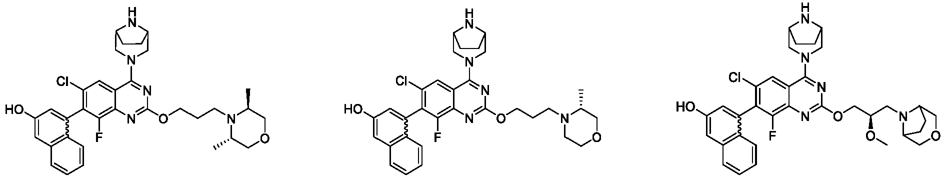 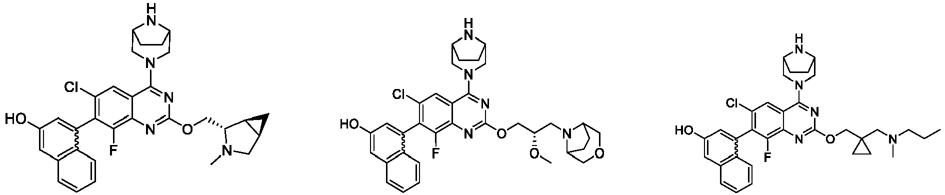 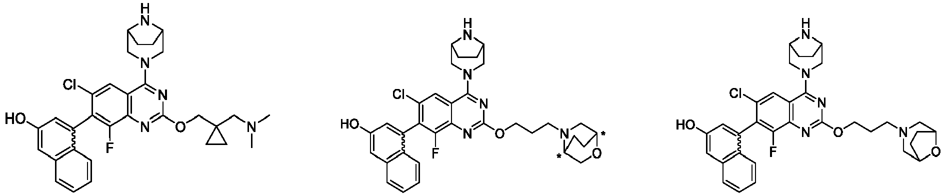 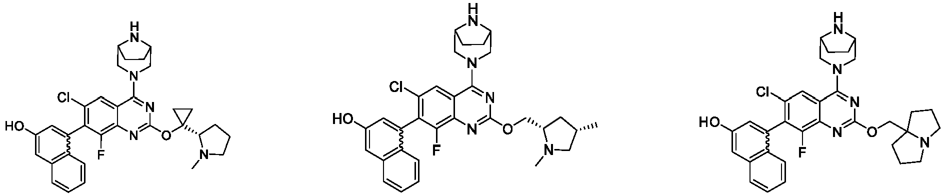 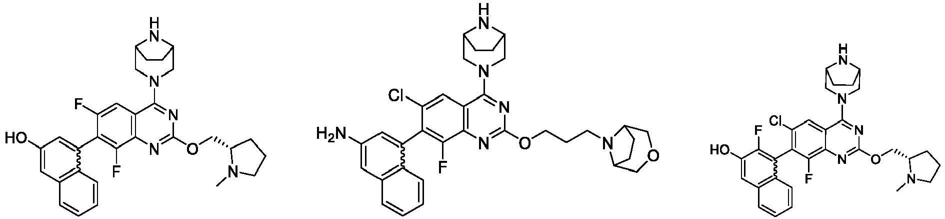 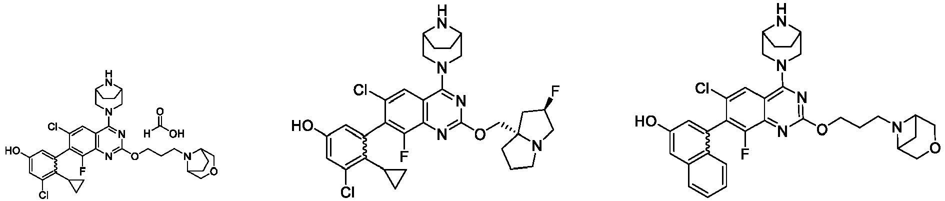 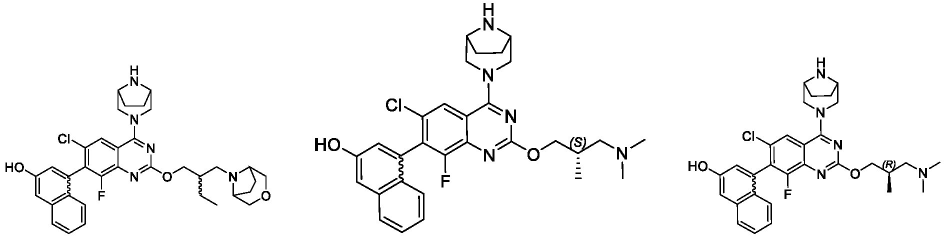 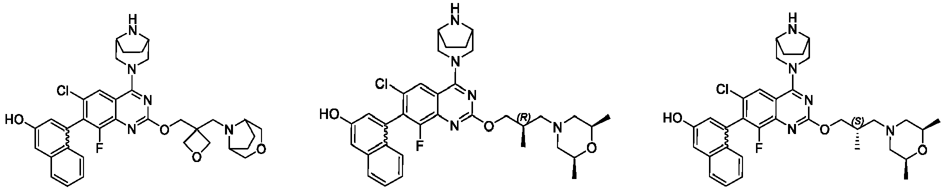 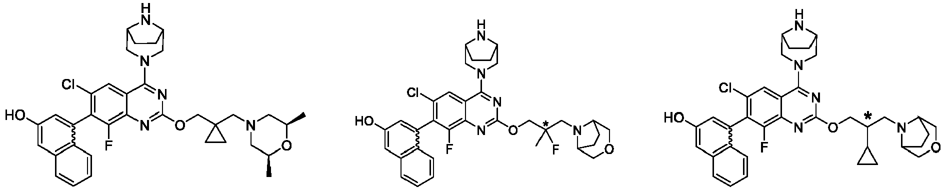 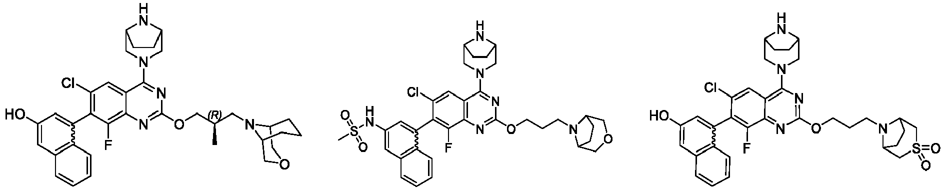 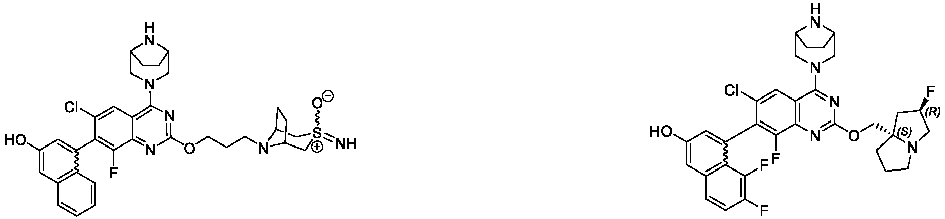 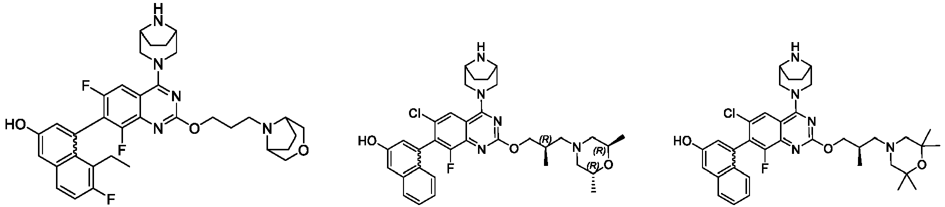 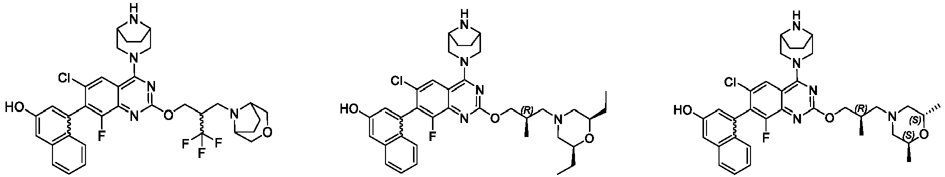 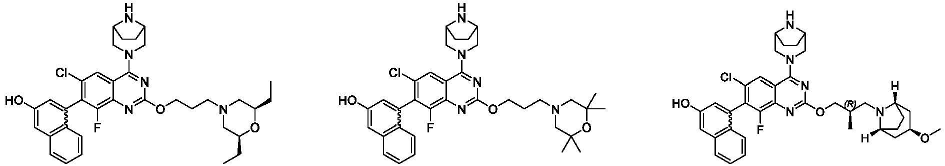 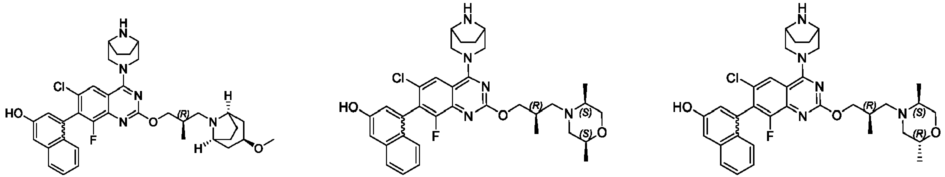 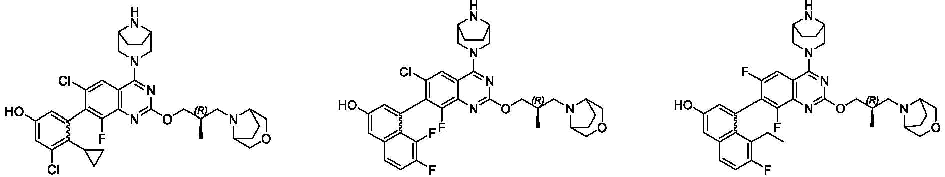 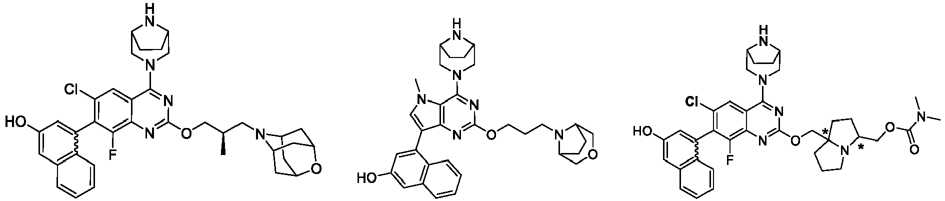 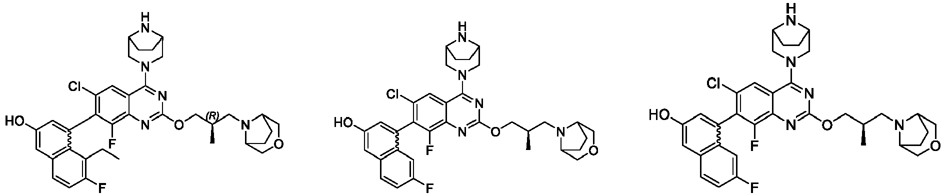 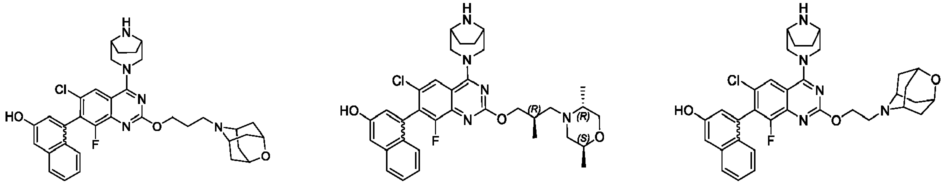 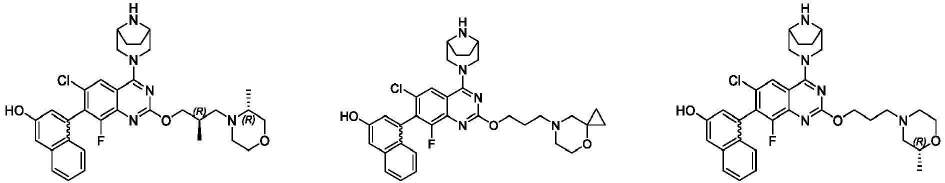 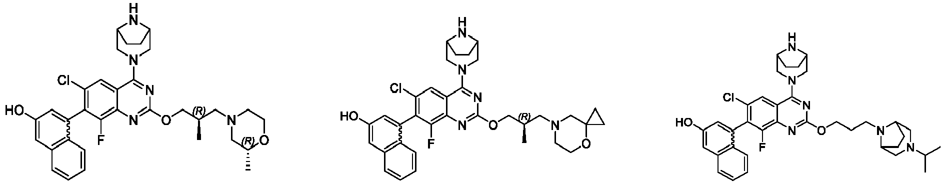 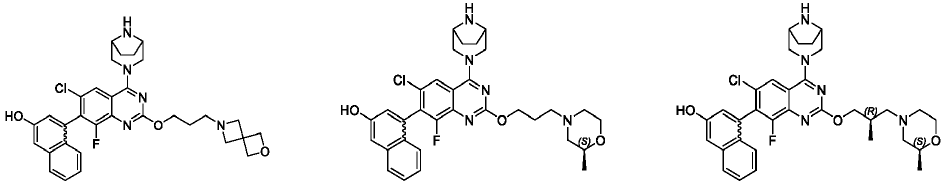 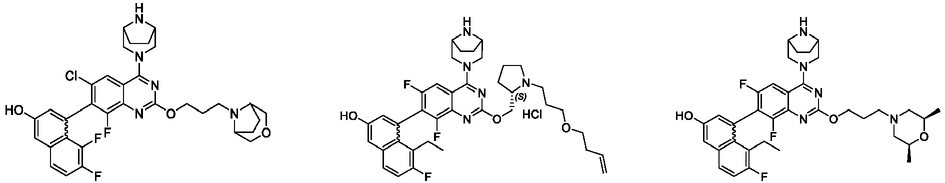 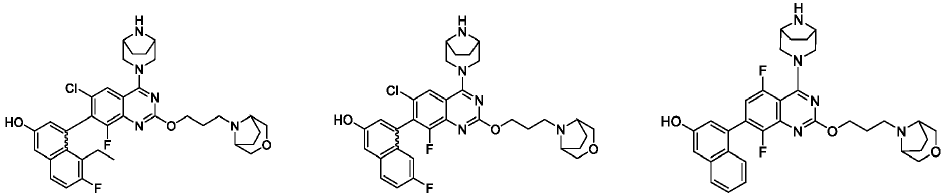 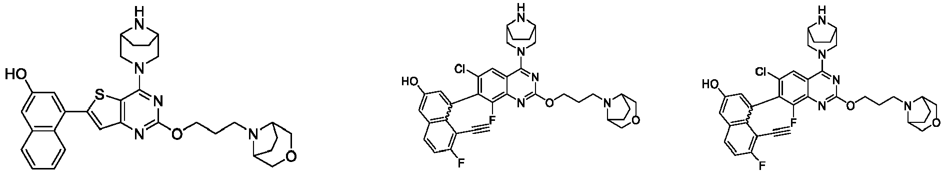 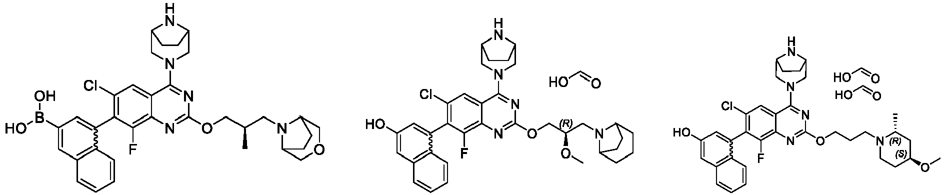 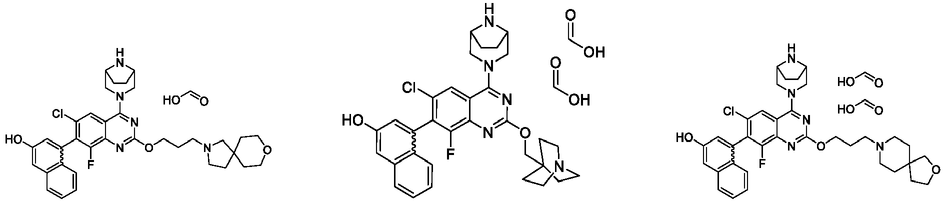 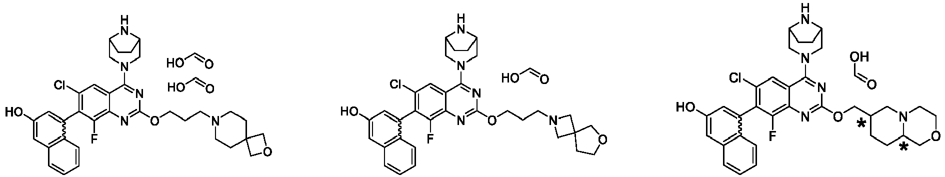 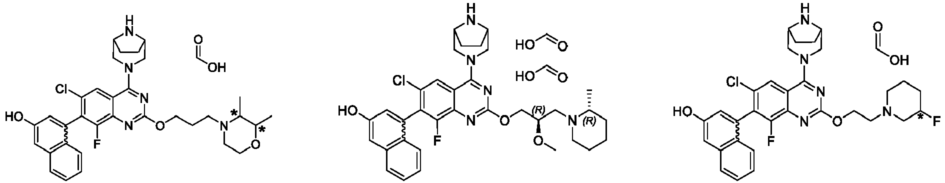 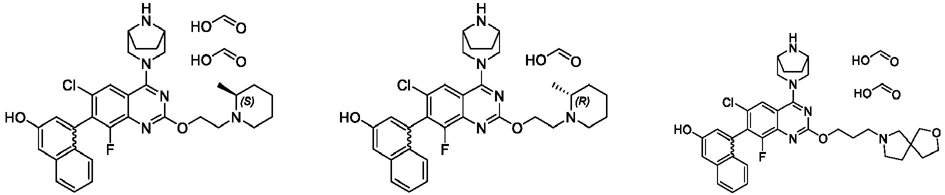 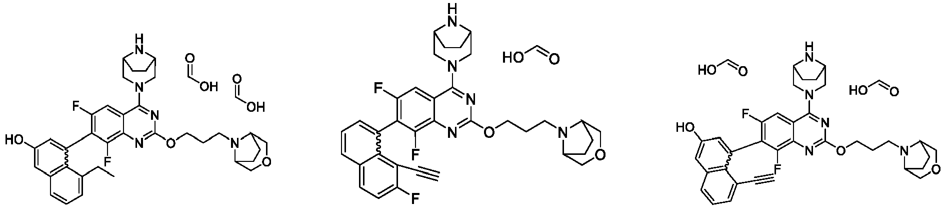 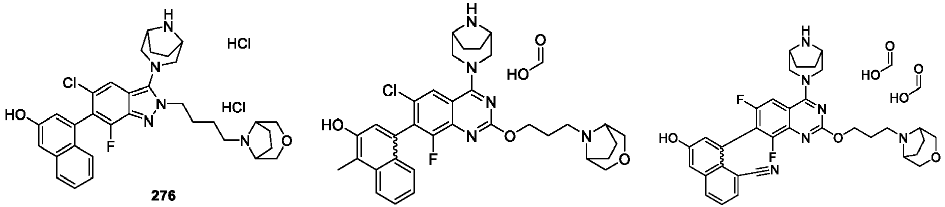 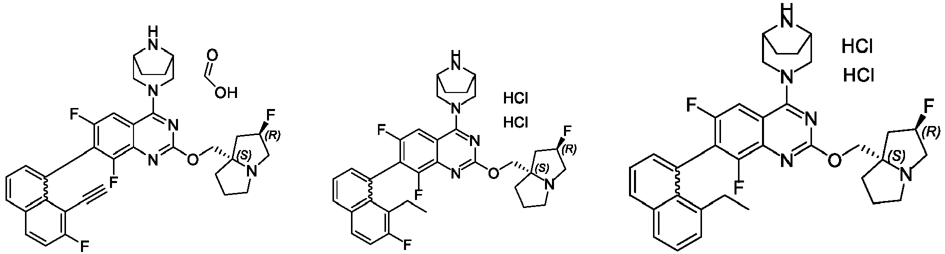 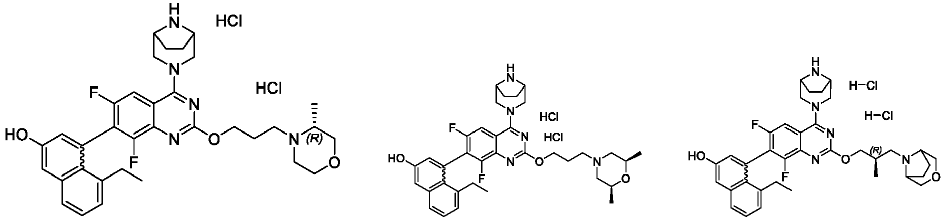 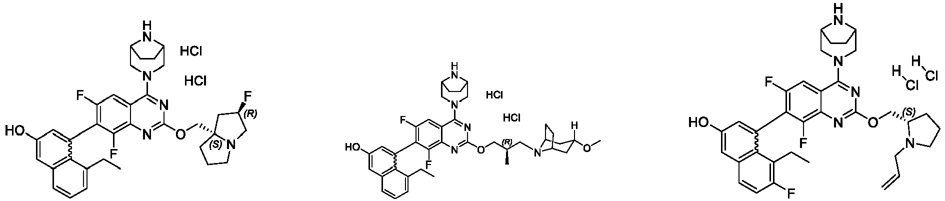 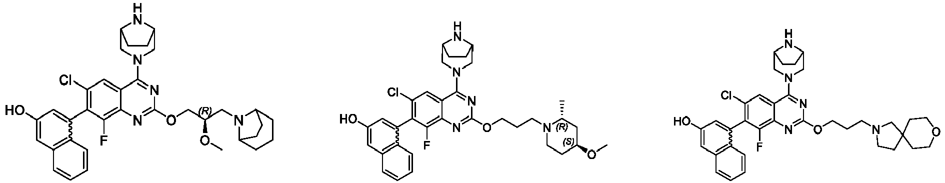 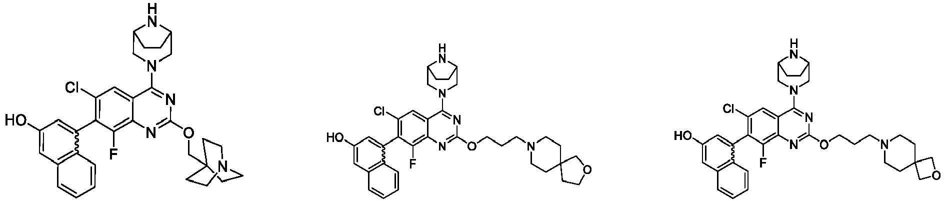 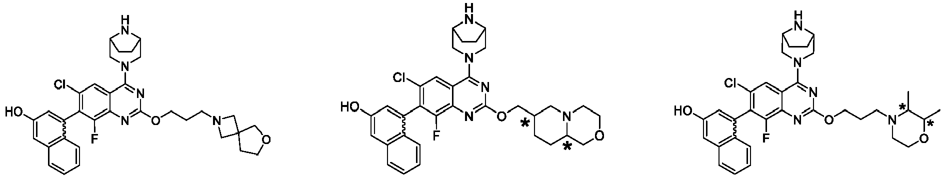 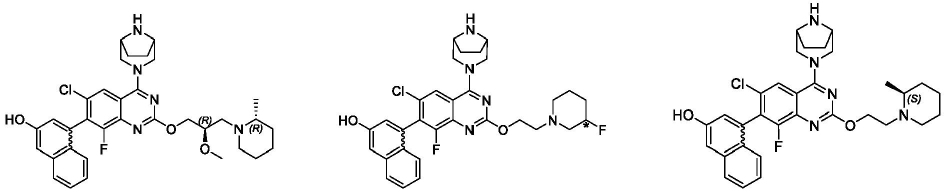 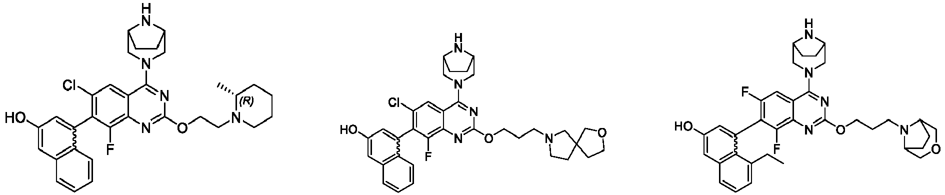 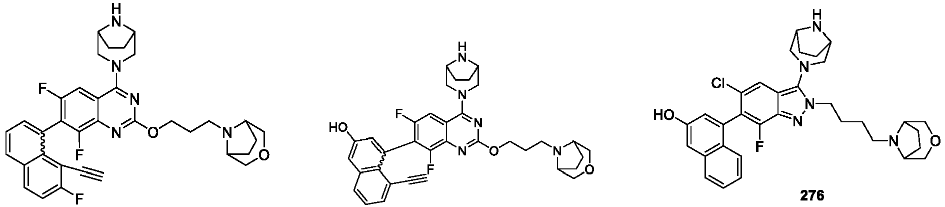 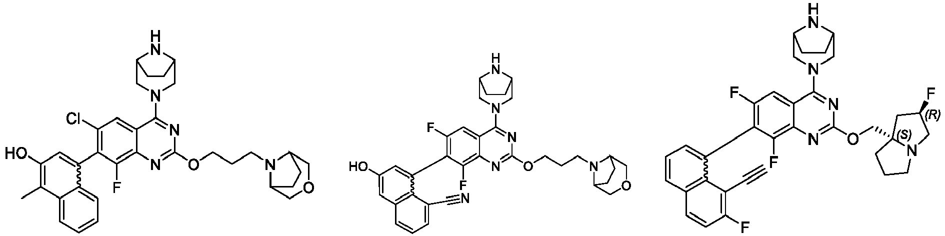 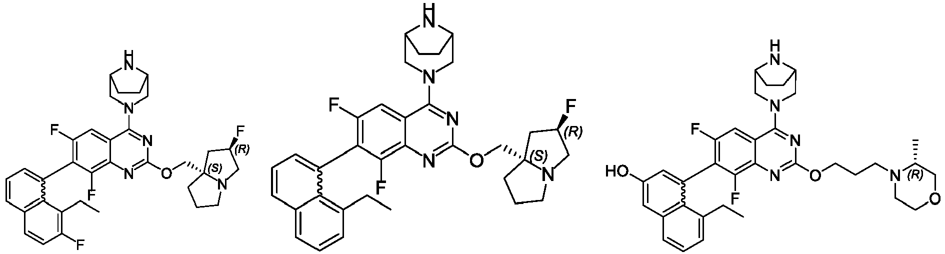 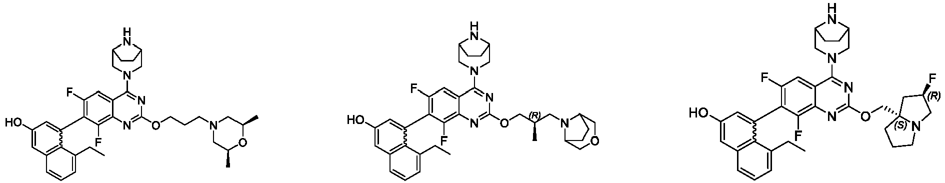 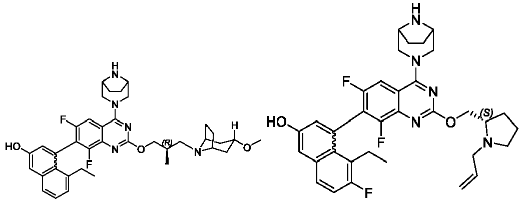 ;the carbon atom with "*" indicates a carbon atom with a chiral center; " 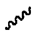 " means " 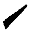 ", " 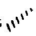 " or a mixture thereof.

In a certain embodiment, the quinazoline compound represented by formula I, the pharmaceutically acceptable salt thereof, the solvate thereof, the prodrug thereof, the metabolite thereof or the isotopic compound thereof is any of the following structures,

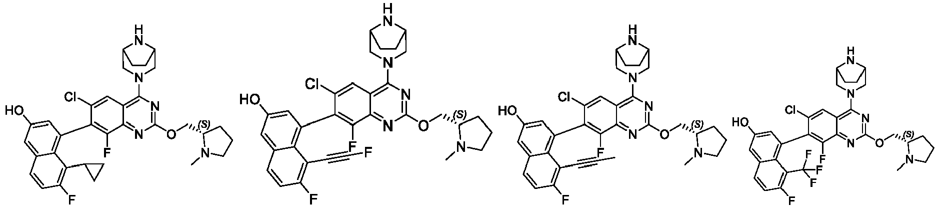

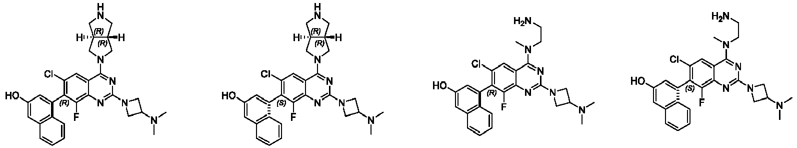

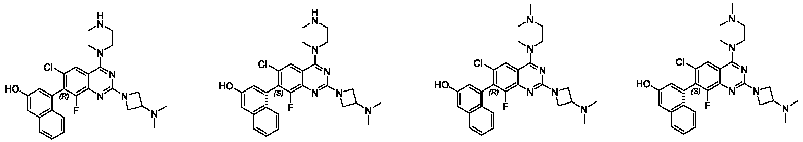

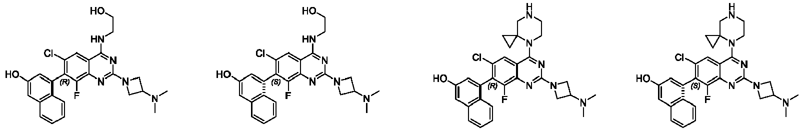

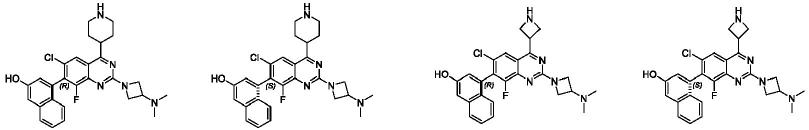

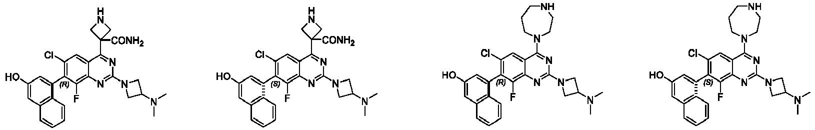

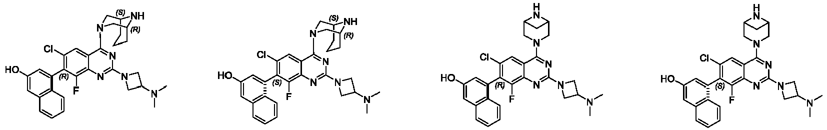

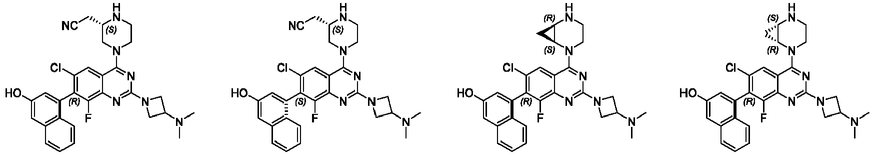

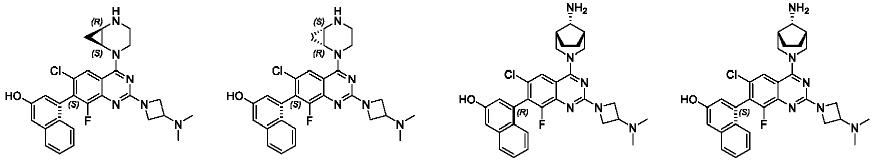

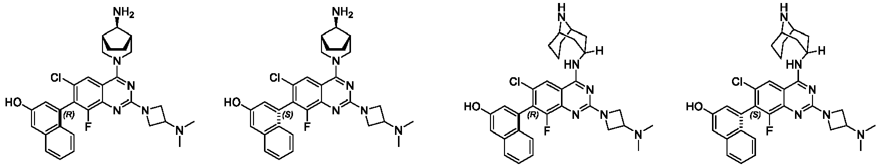

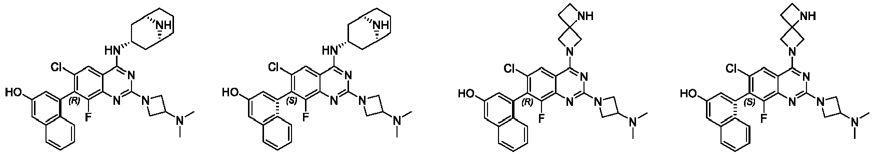

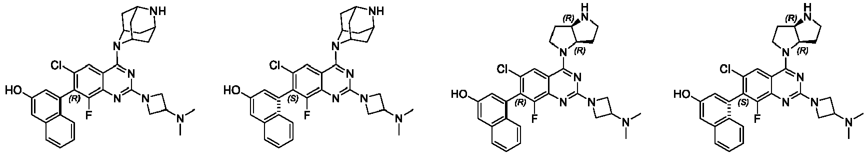

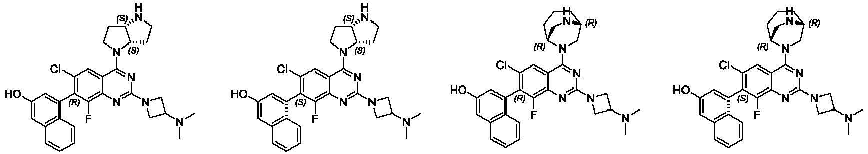

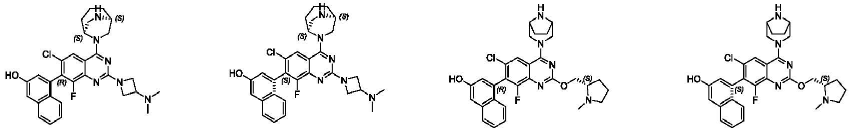

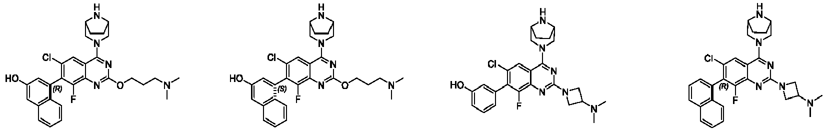

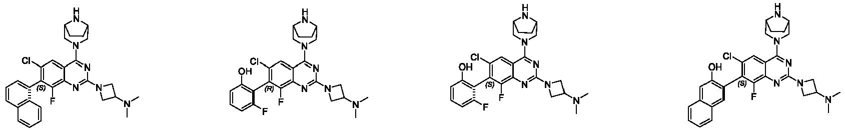

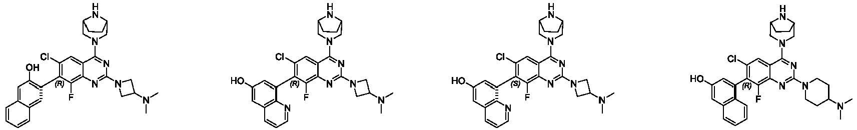

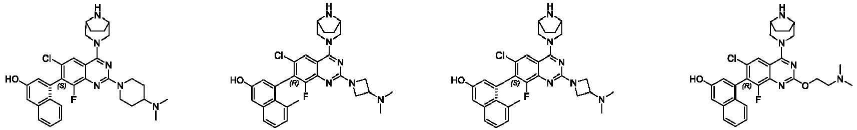

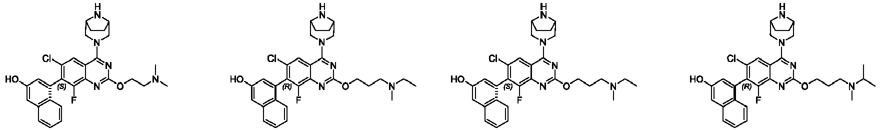

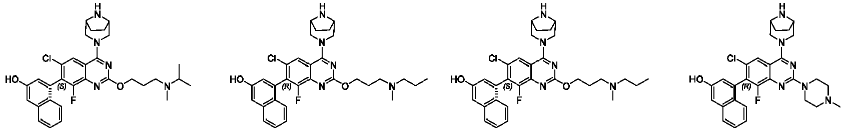

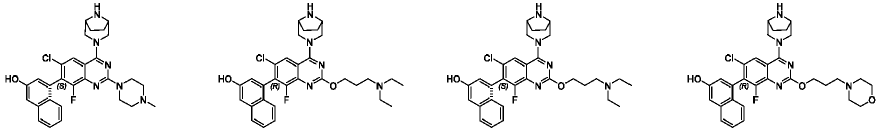

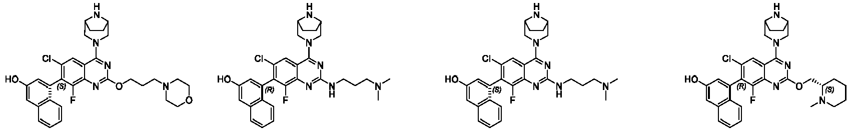

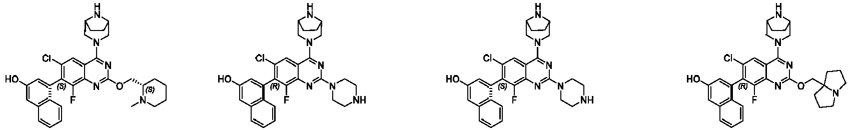

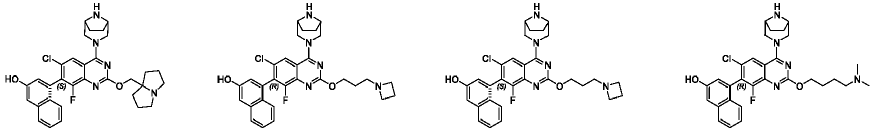

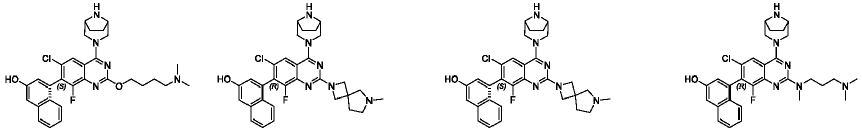

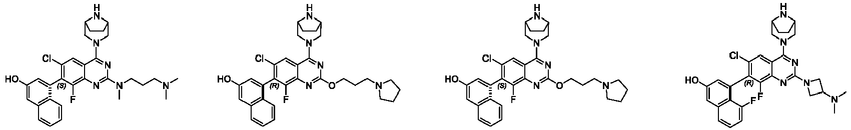

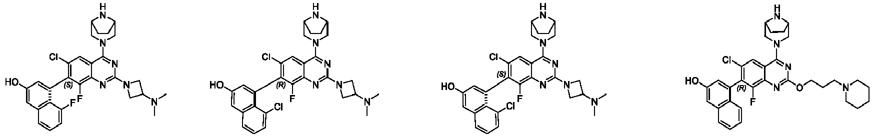

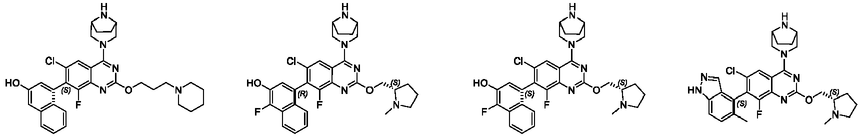

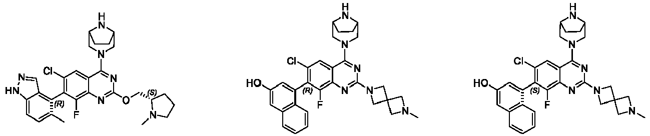

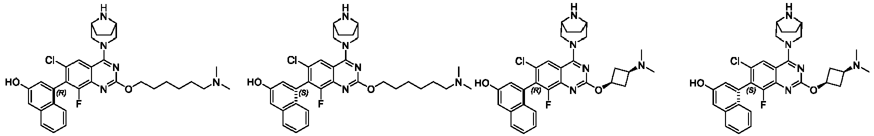

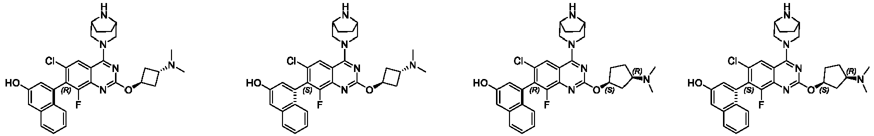

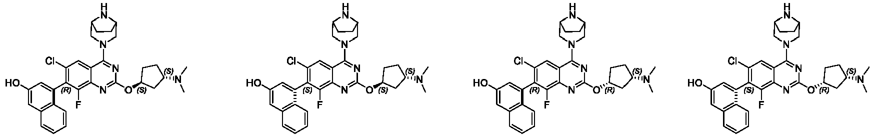

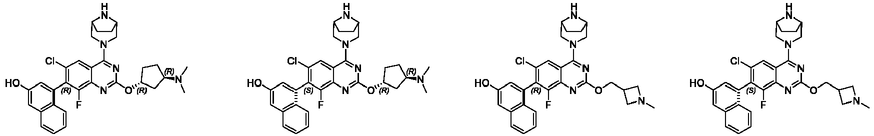

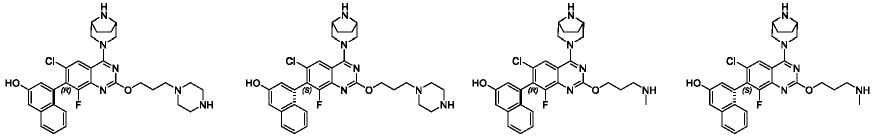

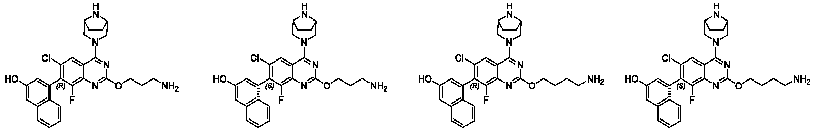

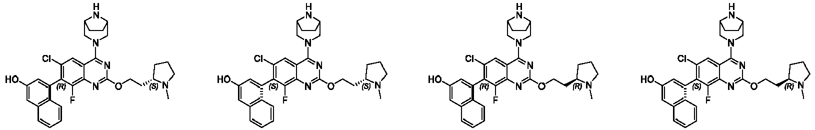

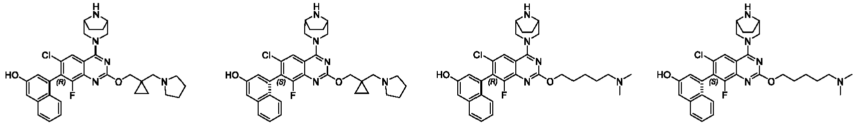

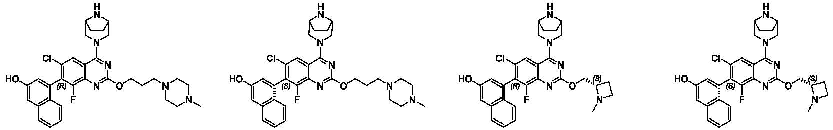

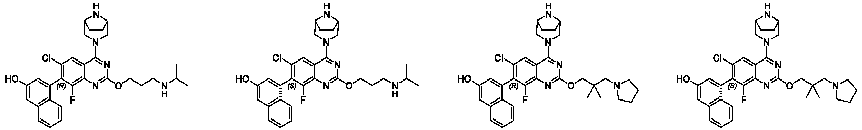

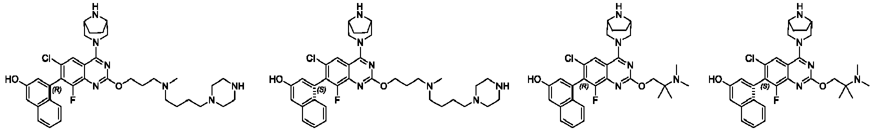

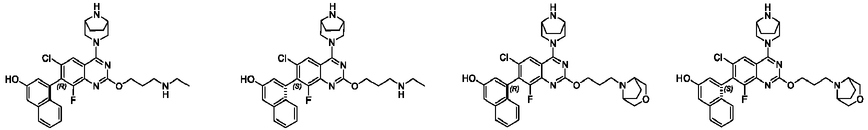

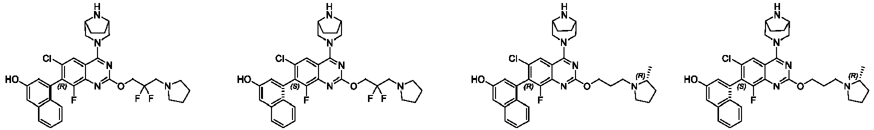

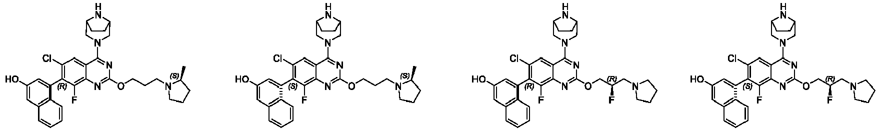

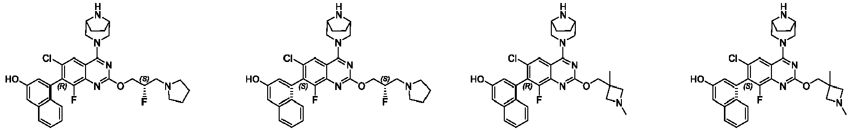

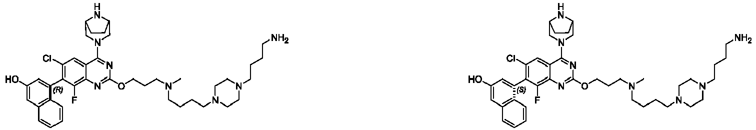

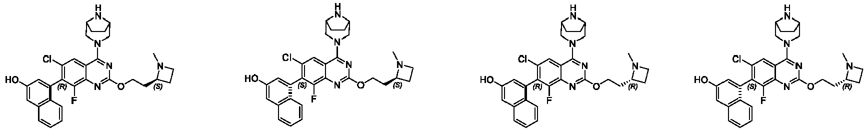

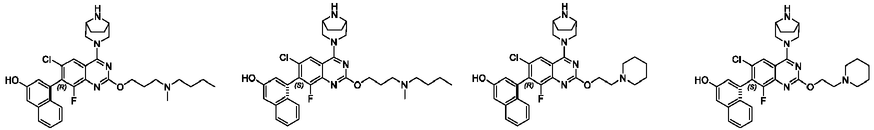

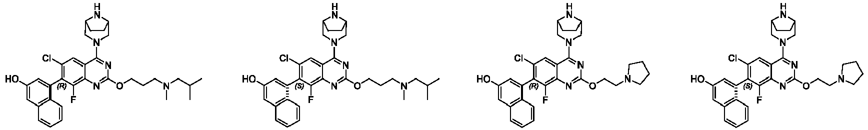

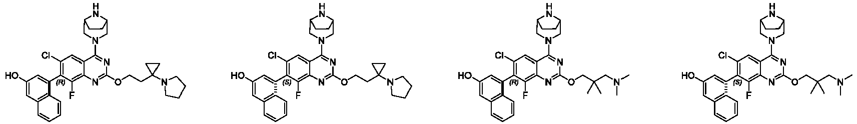

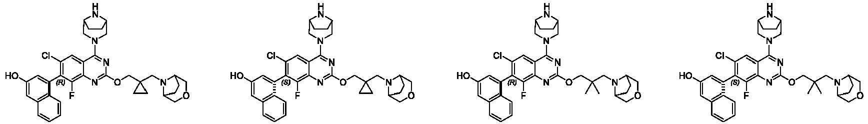

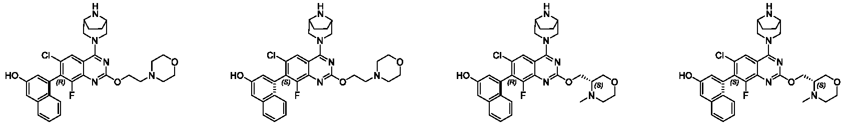

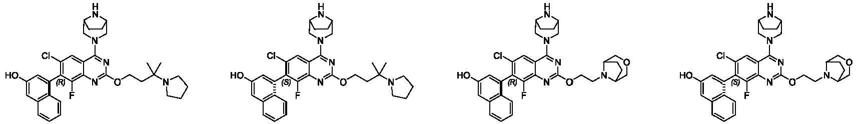

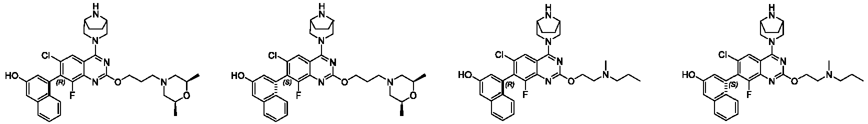

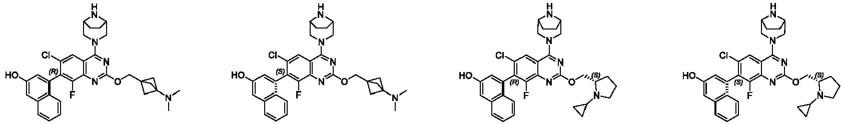

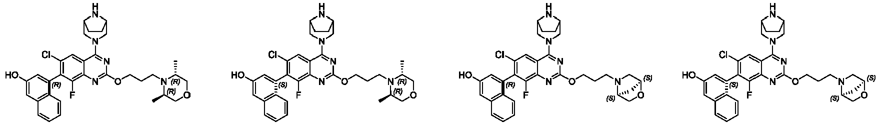

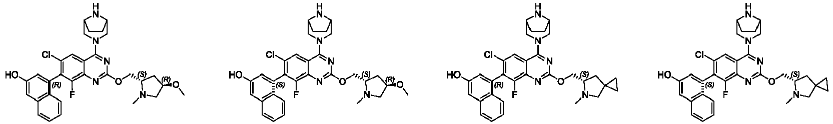

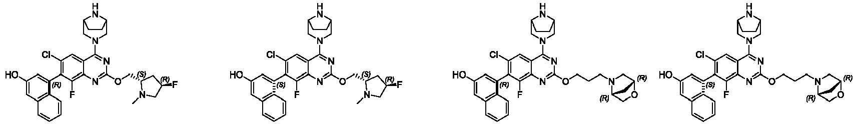

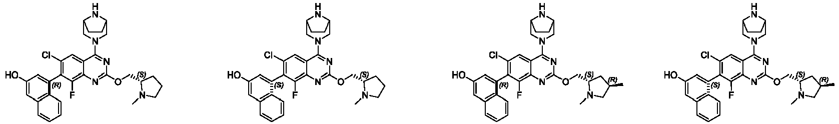

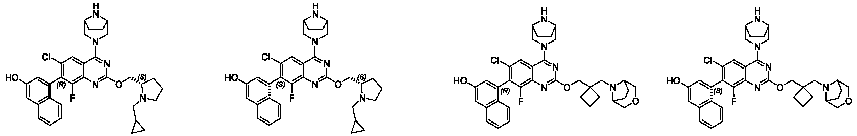

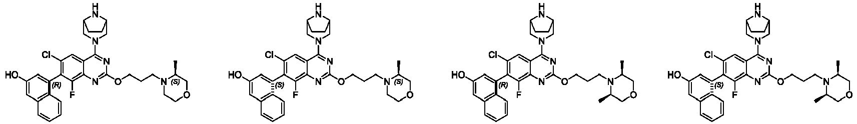

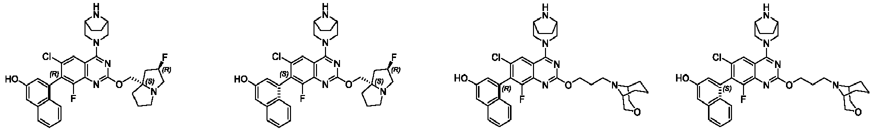

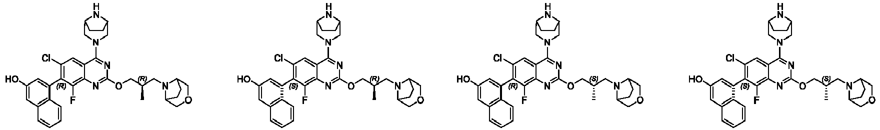

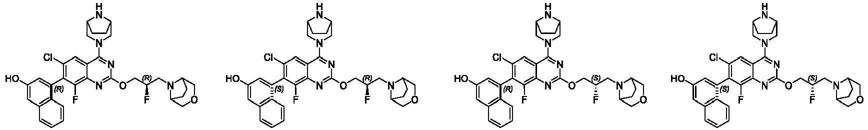

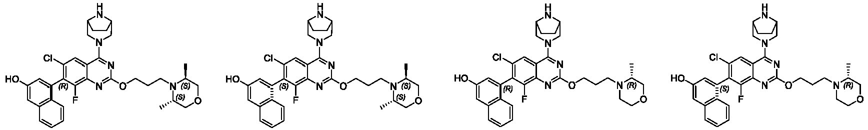

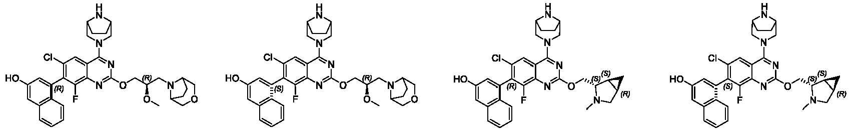

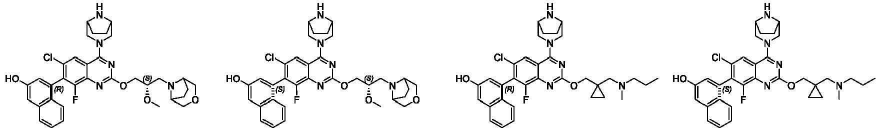

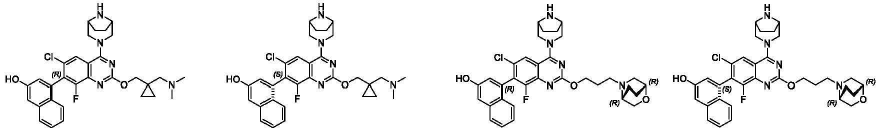

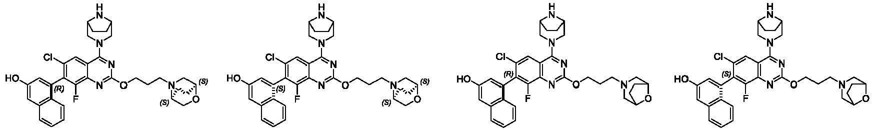

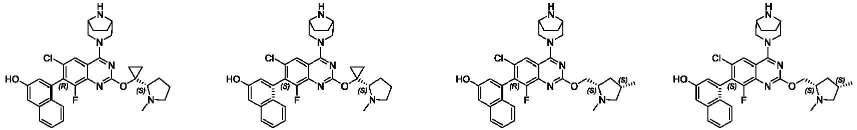

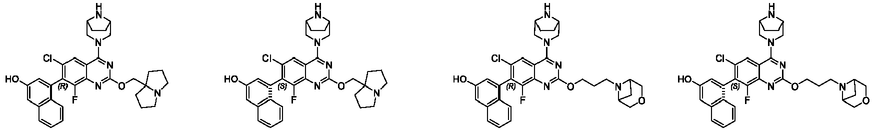

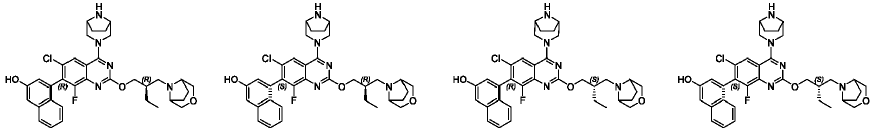

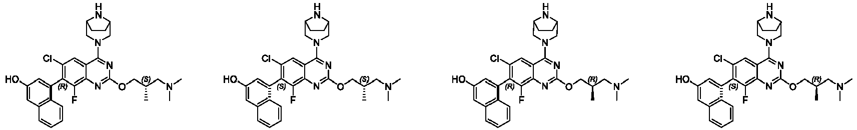

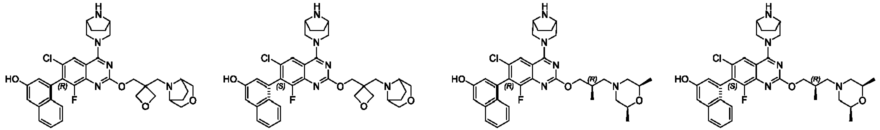

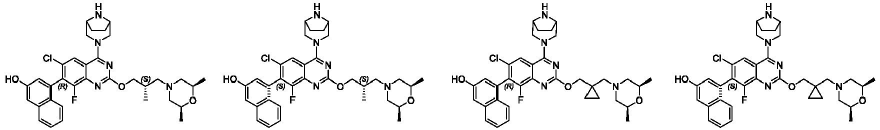

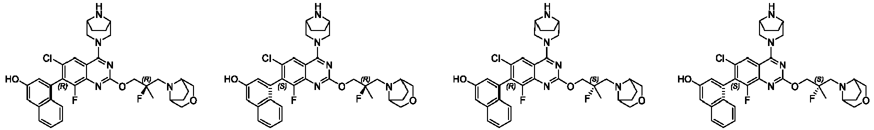

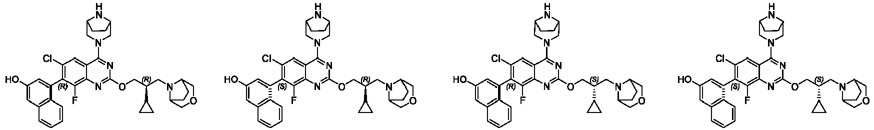

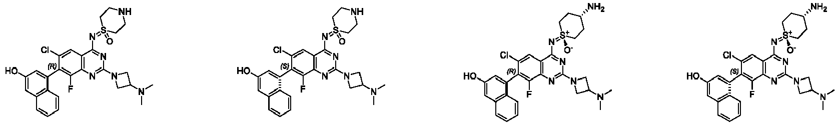

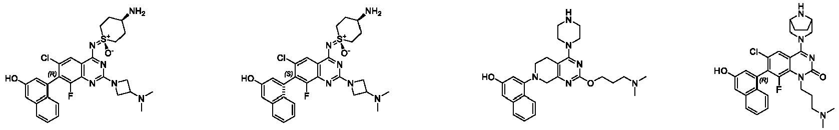

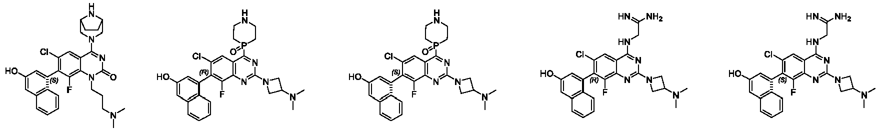

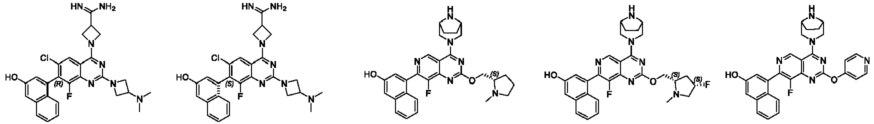

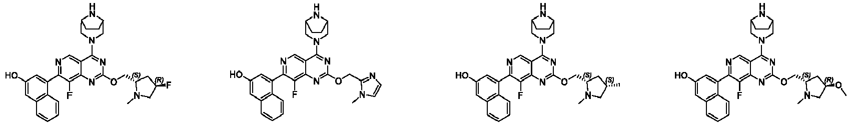

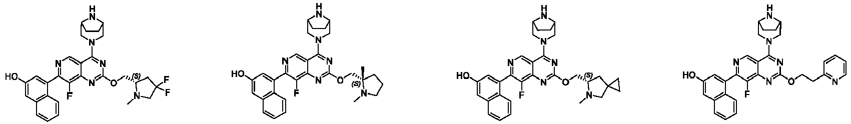

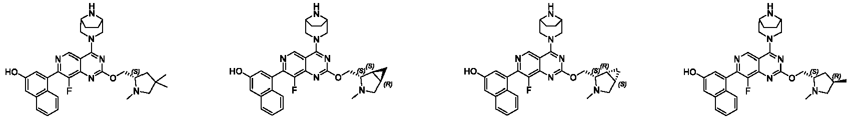

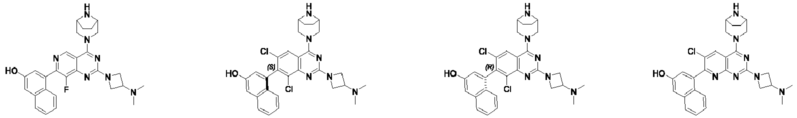

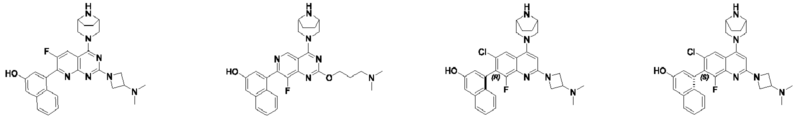

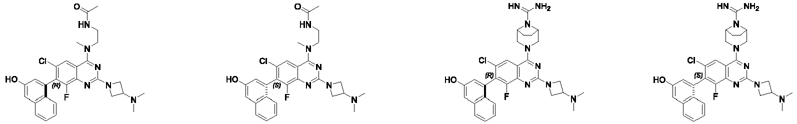

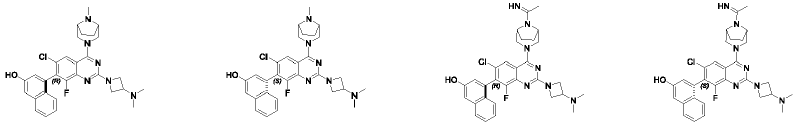

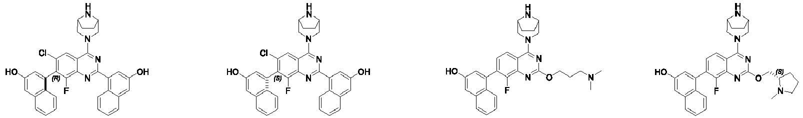

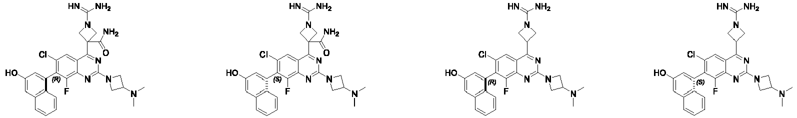

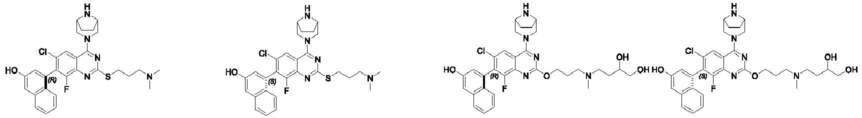

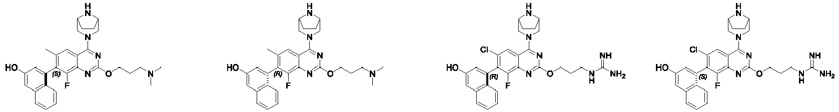

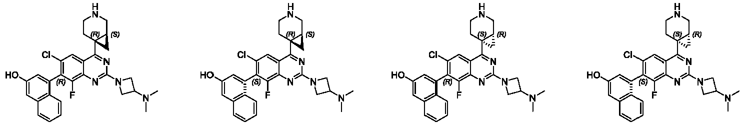

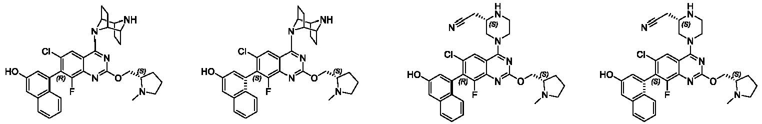

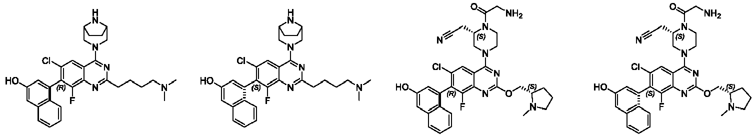

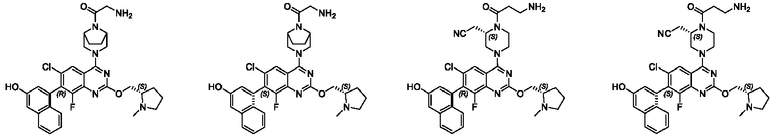

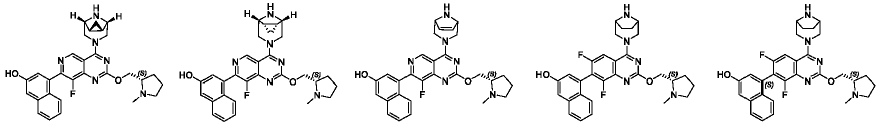

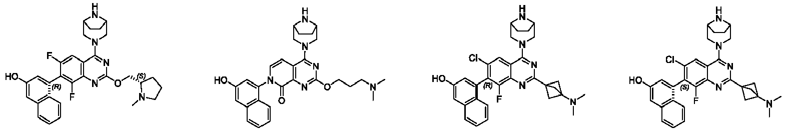

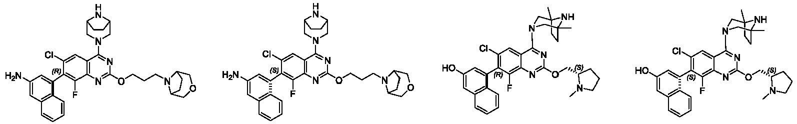

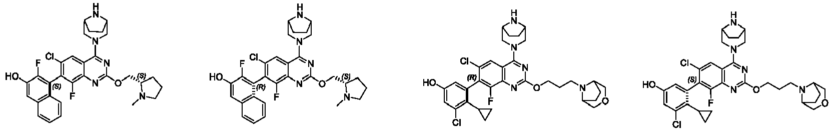

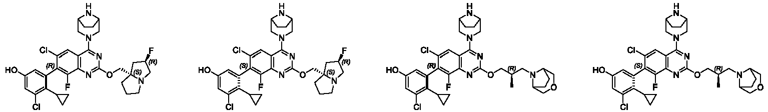

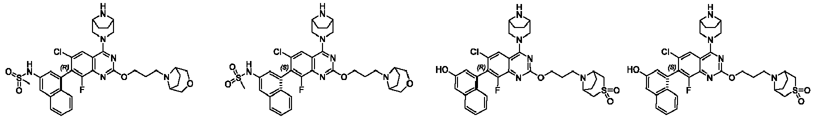

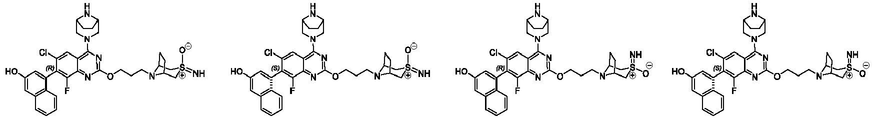

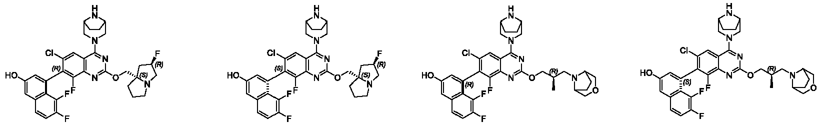

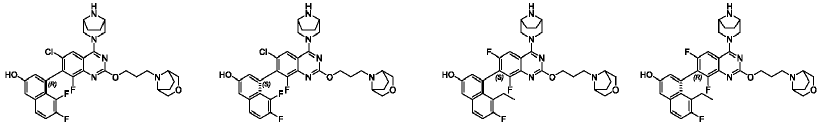

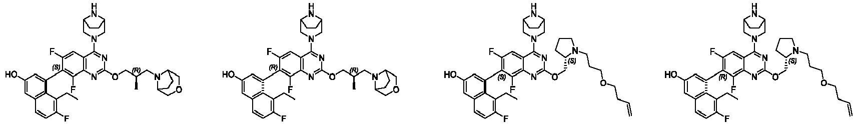

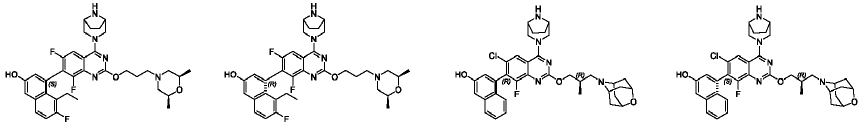

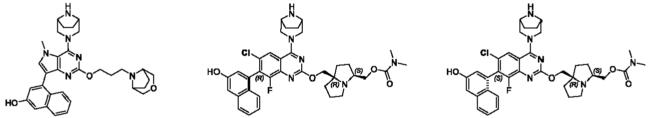

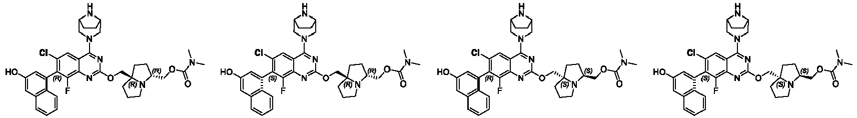

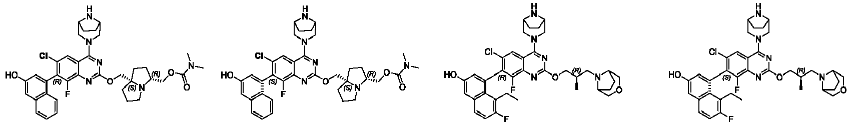

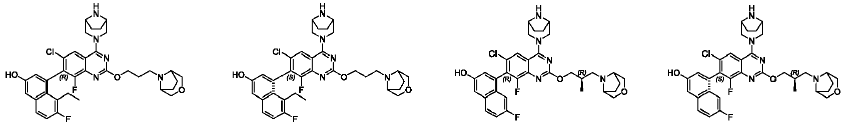

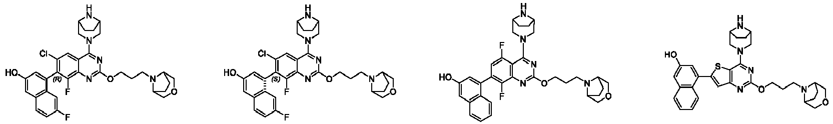

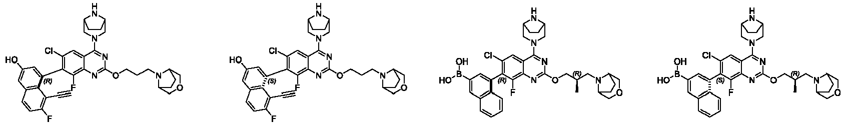

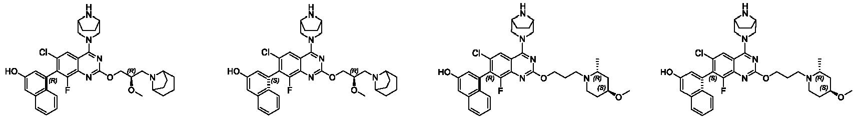

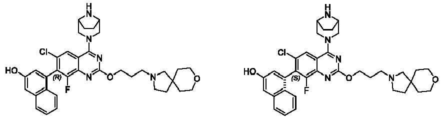

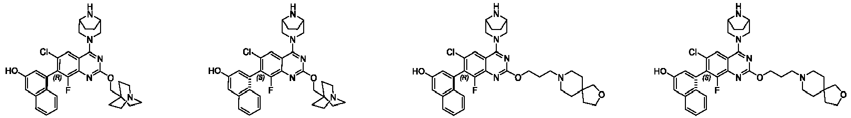

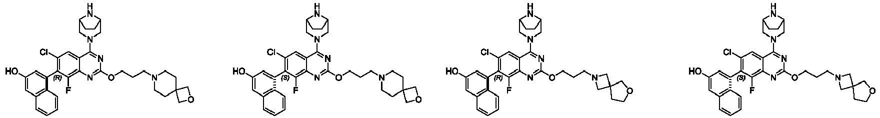

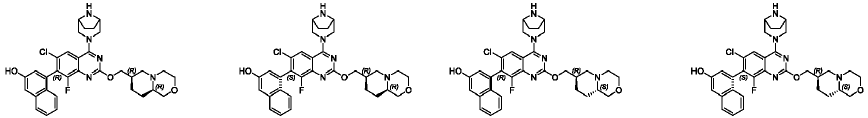

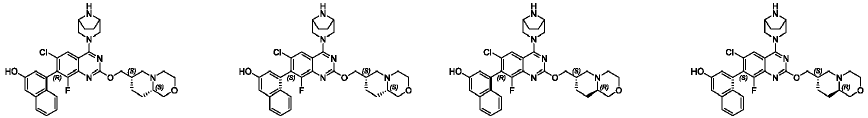

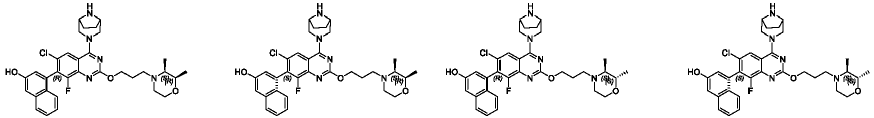

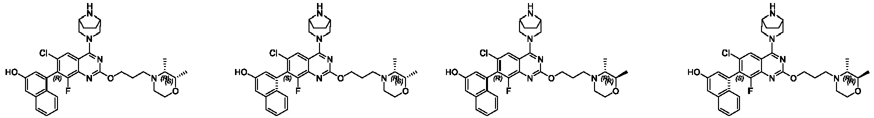

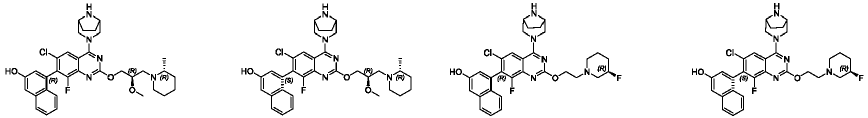

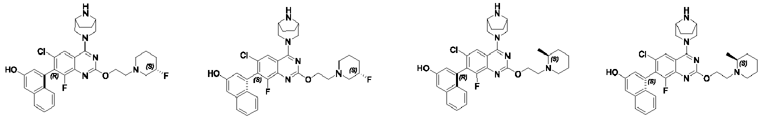

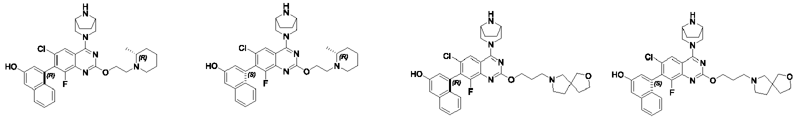

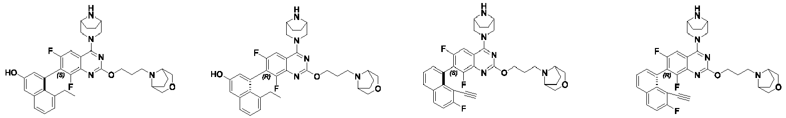

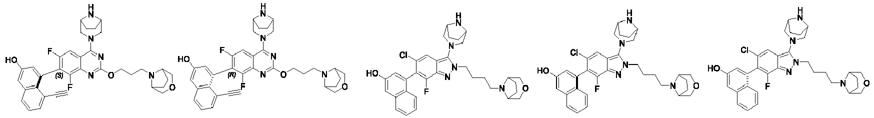

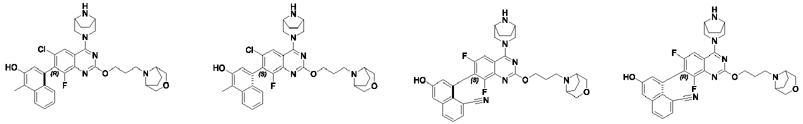

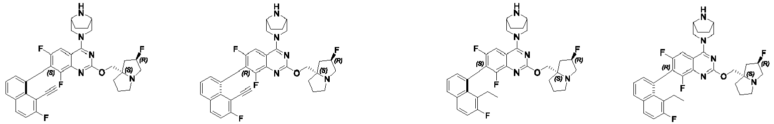

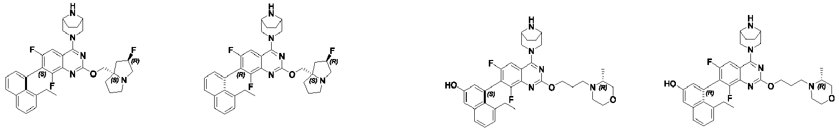

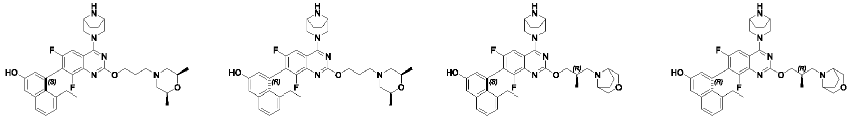

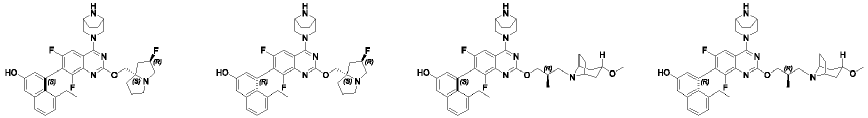

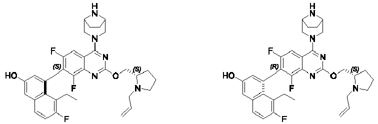

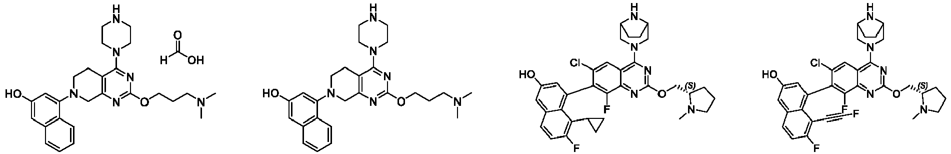

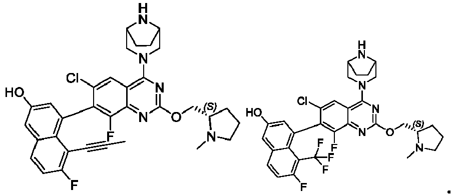

In a certain embodiment, the quinazoline compound represented by formula I, the pharmaceutically acceptable salt thereof, the solvate thereof, the prodrug thereof, the metabolite thereof or the isotopic compound thereof, 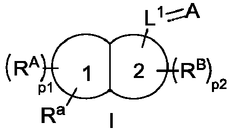
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, or C₁-C₃ alkoxy;
R^{a} is C₆-C₁₀ aryl substituted by one or more hydroxyl;
R^{B} is H, halogen, hydroxyl, cyano, amino, C₁-C₃ alkyl, C₁-C₃ alkoxy or Y¹;
Y¹ is 3- to 10-membered heterocycloalkyl substituted by one or more 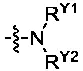 with 1 to 4 heteroatoms selected from one or more of N, O and S;
R^{Y1} and R^{Y2} are independently H or C₁-C₃ alkyl; 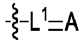
is 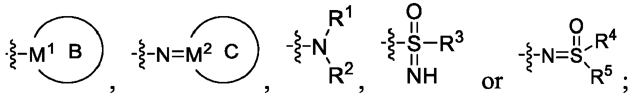
M¹ is N, CH or P(=O);
ring B is unsubstituted 4- to 10-membered heterocycloalkyl with 1 to 3 heteroatoms or heteroatom groups selected from N and P(=O) and containing only one N, unsubstituted 7-to 12-membered heterocycloalkyl or 7- to 12-membered substituted by R⁷ with 2 to 3 heteroatoms selected from one or more of N, O and S, 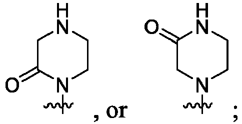 one N atom in the 7- to 12-membered heterocycloalkyl is attached to the quinazoline ring;
R⁷ is -C(=O)O-(C₁-C₄ alkyl) or C₁-C₃ alkyl substituted by CN;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 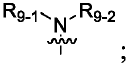
R⁹⁻¹ and R⁹⁻² are independently H, C₁-C₃ alkyl or C₁-C₃ alkyl substituted by CN;
R¹ and R⁴ are independently H or C₁-C₃ alkyl;
R², R³ and R⁵ are independently -(CH₂)ₙ-R^{2a};
n is 1, 2, 3 or 4;
R^{2a} is H, -OH, -O-(C₁-C₃ alkyl) or 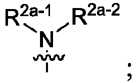
R^{2a-1} and R^{2a-2} are independently H or C₁-C₃ alkyl.

In a certain embodiment, the quinazoline compound represented by formula I is a compound represented by formula I-A, 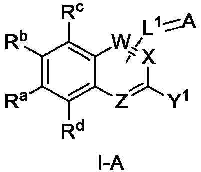
wherein, R^{a} is 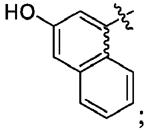
" 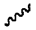 " indicates " 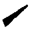 ", " 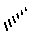 " or a mixture thereof;
R^{b}, R^{c} and R^{d} are independently H, halogen, C₁-C₃ alkyl or C₁-C₃ alkoxy;
W is C;
X and Z are N;
Y¹ is 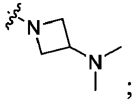
" 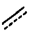 " in 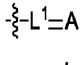 is a single bond or a double bond;
" 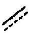 " in 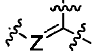 is a single bond or a double bond;

In a certain embodiment, in R^{b}, R^{c} and R^{d}, the halogen is fluorine, chlorine, bromine or iodine, preferably fluorine or chlorine.

In a certain embodiment, in R^{b}, R^{c} and R^{d}, the C₁-C₃ alkyl is methyl, ethyl, propyl or isopropyl.

In a certain embodiment, in R^{b}, R^{c} and R^{d}, the C₁-C₃ alkyl is methoxy, ethoxy, propoxy or isopropoxy.

In a certain embodiment, in R⁷, the C₁-C₄ alkyl in -C(=O)O-(C₁-C₄ alkyl) is methyl, ethyl, n-propyl, isopropyl, n-butyl, secbutyl, isobutyl or tert-butyl, preferably tert-butyl.

In a certain embodiment, in R⁷, the C₁-C₃ alkyl in the C₁-C₃ alkyl substituted by CN is methyl, ethyl, n-propyl or isopropyl, preferably methyl.

In a certain embodiment, in ring C, the 4- to 10-membered heterocycloalkyl with 1 to 3 heteroatoms or heteroatom groups selected from N and S(=O) is 6- to 7-membered heterocycloalkyl with 1 or 2 heteroatoms or heteroatom groups selected from N and S(=O), preferably

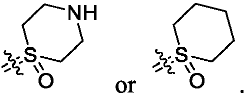

In a certain embodiment, in R⁹⁻¹ and R⁹⁻², the C₁-C₃ alkyl is methyl, ethyl, *n*-propyl or isopropyl.

In a certain embodiment, in R⁹⁻¹ and R⁹⁻², the C₁-C₃ alkyl in the C₁-C₃ alkyl substituted by CN is methyl, ethyl, *n*-propyl or isopropyl.

In a certain embodiment, in R¹ and R⁴, the C₁-C₃ alkyl is methyl, ethyl, *n*-propyl or isopropyl, preferably methyl.

In a certain embodiment, in R^{2a}, the C₁-C₃ alkyl in the -O-(C₁-C₃ alkyl) is methyl, ethyl, *n*-propyl or isopropyl.

In a certain embodiment, in R^{2a-1} and R^{2a-2}, the C₁-C₃ alkyl is methyl, ethyl, n-propyl or isopropyl, preferably methyl.

In a certain embodiment, in 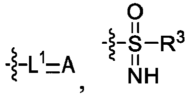 is

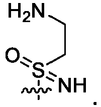

In a certain embodiment, in "" 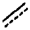 is

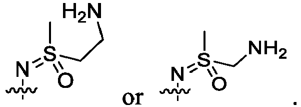

In a certain embodiment, the quinazoline compound represented by formula I is a compound represented by formula I-B,

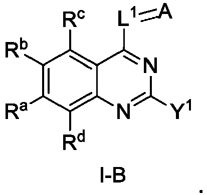

In a certain embodiment, R^{b} is halogen.

In a certain embodiment, R^{c} is H.

In a certain embodiment, R^{d} is halogen.

In a certain embodiment, " 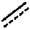 " in 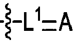 is a single bond.

In a certain embodiment, 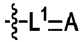 is

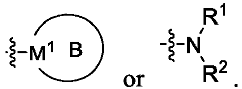

In a certain embodiment, 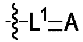 is

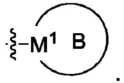

In a certain embodiment, M¹ is N or CH.

In a certain embodiment, ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring.

In a certain embodiment, ring B is unsubstituted 7- to 12-membered bridged heterocycloalkyl with 2 heteroatoms being N with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring.

In a certain embodiment, ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring.

In a certain embodiment, ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
the 7- to 12-membered bridged heterocycloalkyl with 2 heteroatoms being N is 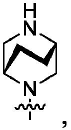 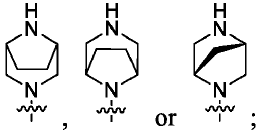
the 7- to 12-membered spiro heterocycloalkyl with 2 heteroatoms being N, wherein one ring is a 4-membered heterocycloalkyl with 1 atom being N, and the N atom in the 4-membered heterocycloalkyl is attached to the quinazoline ring.

In a certain embodiment, R¹ is H or C₁-C₃ alkyl.

In a certain embodiment, R² is -(CH₂)ₙ-R^{2a}.

In a certain embodiment, n is 1 or 2.

In a certain embodiment, R^{2a} is

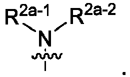

In a certain embodiment, R^{2a-1} and R^{2a-2} are independently H or C₁-C₃ alkyl, and R^{2a-1} and R^{2a-2} are not C₁-C₃ alkyl at the same time.

In a certain embodiment, R^{2a-1} and R^{2a-2} are H.

In a certain embodiment, 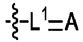
is 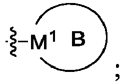
ring B is 7- to 12-membered heterocycloalkyl with 2 to 3 heteroatoms selected from N, S and O.

In a certain embodiment, 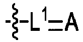
is 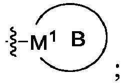
ring B is 7- to 12-membered bridged heterocycloalkyl with 2 heteroatoms being N.

In a certain embodiment,
ring B is 7- to 12-membered spiro heterocycloalkyl with 2 heteroatoms being N.

In a certain embodiment, 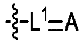 is

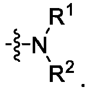

In a certain embodiment, 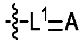
is 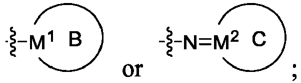
ring B is unsubstituted 4- to 7-membered heterocycloalkyl with 1 to 3 heteroatoms or heteroatom groups selected from N and P(=O) and containing only one N;
ring C is unsubstituted 4- to 7-membered heterocycloalkyl or 4- to 7-membered heterocycloalkyl substituted by R⁹ with 1 to 3 heteroatoms or heteroatom groups selected from N and S(=O) and containing only one N.

In a certain embodiment, 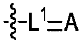 is

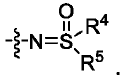

In a certain embodiment, the quinazoline compound represented by formula I is a compound represented by formula I-B, 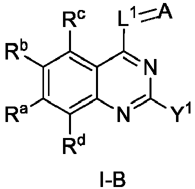
R^{a} is 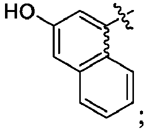
" 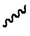 " indicates " 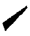 ", " 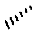 " or a mixture thereof;
R^{b} is halogen;
R^{c} is H;
R^{d} is halogen;
Y¹ is 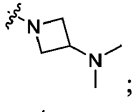
" 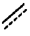 " in 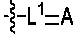 is a single bond; 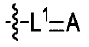
is 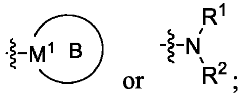
M¹ is N or CH;
ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring;
R¹ is H or C₁-C₃ alkyl;
R² is -(CH₂)ₙ-R₂ₐ;
n is 1, 2, 3 or 4;
R^{2a} is H, -OH or 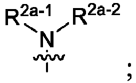
R^{2a-1} and R^{2a-2} are independently H or C₁-C₃ alkyl.

In a certain embodiment, the quinazoline compound represented by formula I is a compound represented by formula I-B, 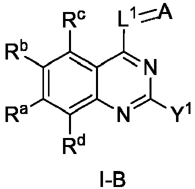
R^{a} is 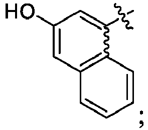
" 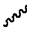 " indicates " 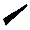 ", " 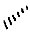 " or a mixture thereof;
R^{b} is halogen;
R^{c} is H;
R^{d} is halogen;
Y¹ is 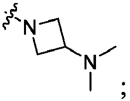
" 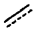 " in 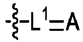 is a single bond; 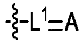
is 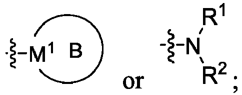
M¹ is Nor CH;
ring B is unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring;
R¹ is H or C₁-C₃ alkyl;
R² is -(CH₂)ₙ-R₂ₐ;
n is 1, 2 or 3;
R^{2a} is H, -OH or 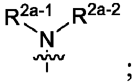
R^{2a-1} and R^{2a-2} are independently H or C₁-C₃ alkyl, and R^{2a-1} and R^{2a-2} are not C₁-C₃ alkyl at the same time.

In a certain embodiment, the quinazoline compound represented by formula I is a compound represented by formula I-B, 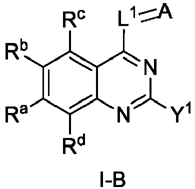
R^{a} is 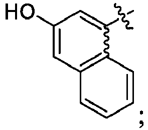
" 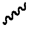 " indicates " 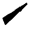 ", " 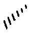 " or a mixture thereof;
R^{b} is halogen;
R^{c} is H;
R^{d} is halogen;
Y¹ is 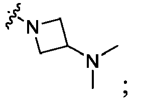
" 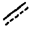 " in 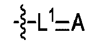 is a single bond; 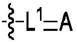
is 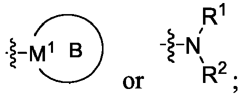
M¹ is N;
ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
R¹ is H or C₁-C₃ alkyl;
R² is -(CH₂)ₙ-R^{2a};
n is 1, 2 or 3;
R^{2a} is 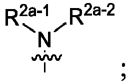
R^{2a-1} and R^{2a-2} are H.

In a certain embodiment, the quinazoline compound represented by formula I is a compound represented by formula I-B, 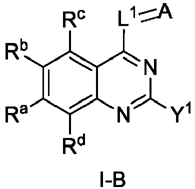
R^{a} is 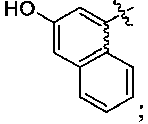
" 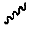 " indicates " 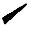 ", " 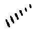 " or a mixture thereof;
R^{b} is halogen;
R^{c} is H;
R^{d} is halogen;
Y¹ is 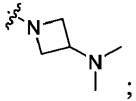
" 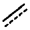 " in 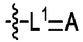 is a single bond; 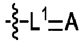
is 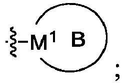
M¹ is N;
ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
the 7- to 12-membered bridged heterocycloalkyl with 2 heteroatoms being N is 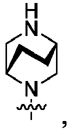 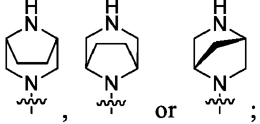
the 7- to 12-membered spiro heterocycloalkyl with 2 heteroatoms being N, wherein one ring is a 4-membered heterocycloalkyl with 1 atom being N, and the N atom in the 4-membered heterocycloalkyl is attached to the quinazoline ring.

In a certain embodiment, the quinazoline compound represented by formula I, the pharmaceutically acceptable salt thereof, the solvate thereof, the prodrug thereof, the metabolite thereof or the isotopic compound thereof is any of the following structures, wherein, the carbon atom with "*" indicates a carbon atom with S configuration or R configuration, 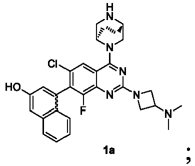
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.946 min; 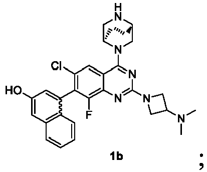
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 5.115 min; 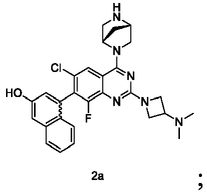
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.104 min; 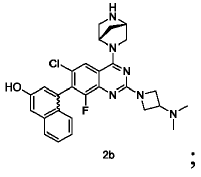
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 3.656 min; 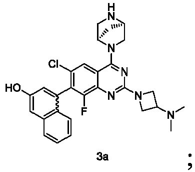
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 10 min; detector UV 220/254 nm; retention time: 2.705 min; 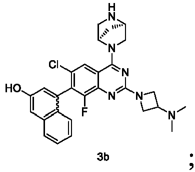
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 10 min; detector UV 220/254 nm; retention time: 6.915 min; 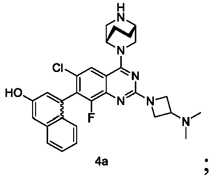
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 15 min; detector UV 220/254 nm; retention time: 5.828 min; 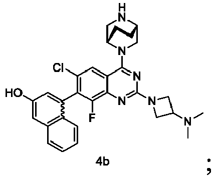
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 15 min; detector UV 220/254 nm; retention time: 9.588 min; 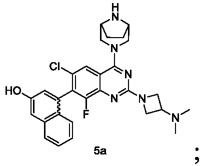
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.549 min; 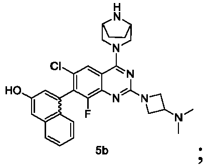
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 3.363 min; 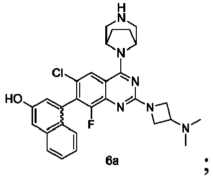
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 9 min; detector UV 220/254 nm; retention time: 6.871 min; 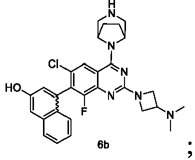
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 9 min; detector UV 220/254 nm; retention time: 4.373 min; 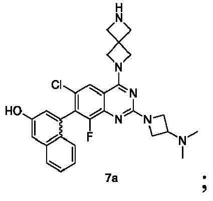
its HPLC conditions are: chiral column: Lux 3 µm Cellulose-2, 4.6 x 50 mm, 3 µm; mobile phase A: *n*-hexane (0.1% diethylamine), mobile phase B: ethanol; flow rate: 1 mL/min; isocratic elution with 30% phase B in 12 min; detector UV 220/254 nm; retention time: 4.914 min; 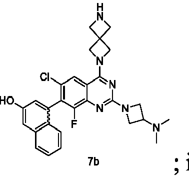
its HPLC conditions are: chiral column: Lux 3 µm Cellulose-2, 4.6 x 50 mm, 3 µm; mobile phase A: *n*-hexane (0.1% diethylamine), mobile phase B: ethanol; flow rate: 1 mL/min; isocratic elution with 30% phase B in 12 min; detector UV 220/254 nm; retention time: 7.935 min; 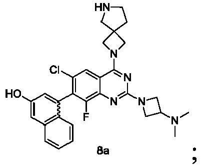
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.844 min; 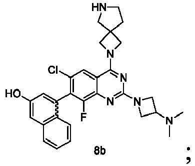
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 4.215 min; 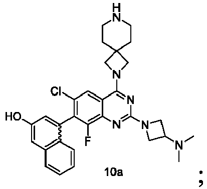
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 4.5 min; detector UV 220/254 nm; retention time: 2.562 min; 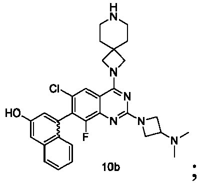
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 4.5 min; detector UV 220/254 nm; retention time: 3.662 min; 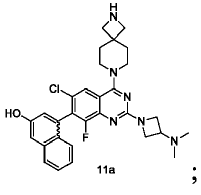
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.856 min; 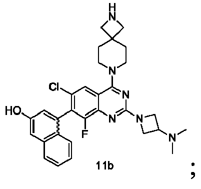
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 4.120 min; 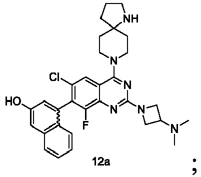
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 4.5 min; detector UV 220/254 nm; retention time: 2.317 min; 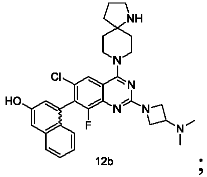
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 4.5 min; detector UV 220/254 nm; retention time: 3.182 min; 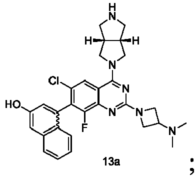
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 10 min; detector UV 220/254 nm; retention time: 4.576 min; 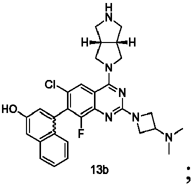
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 10 min; detector UV 220/254 nm; retention time: 8.266 min; 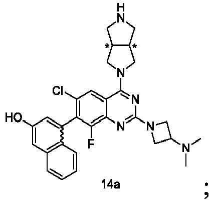
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 3.616 min; 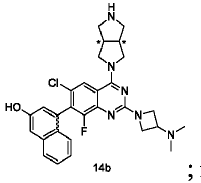
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 5.340 min; 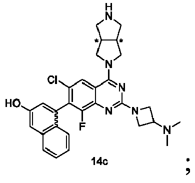
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 10 min; detector UV 220/254 nm; retention time: 5.189 min; 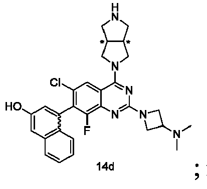
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 10 min; detector UV 220/254 nm; retention time: 7.314 min; 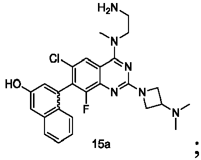
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 3.5 min; detector UV 220/254 nm; retention time: 1.347 min; 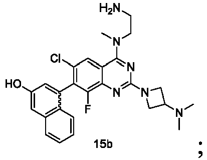
its HPLC conditions are: chiral column: CHIRALPAK IC-3, 4.6 x 50 mm, 3 µm; mobile phase A: *n*-hexane/dichloromethane = 5/1 (0. 1% diethylamine), mobile phase B: isopropanol; flow rate: 1 mL/min; isocratic elution with 30% phase B in 3.5 min; detector UV 220/254 nm; retention time: 2.180 min; 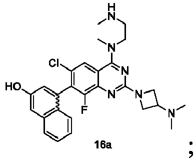
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.690 min; 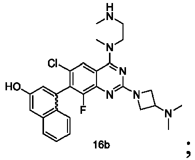
its HPLC conditions are: chiral column: CHIRALPAK IG-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.820 min; 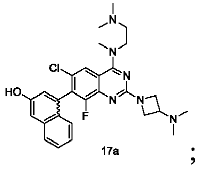
its HPLC conditions are: chiral column: CHIRALPAK IG-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 4.257 min; 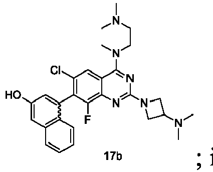
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 2.734 min; 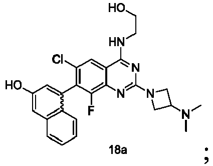
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 5.5 min; detector UV 220/254 nm; retention time: 3.619 min; 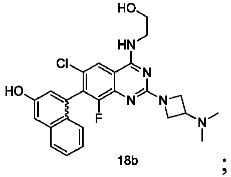
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 5.5 min; detector UV 220/254 nm; retention time: 2.324 min; 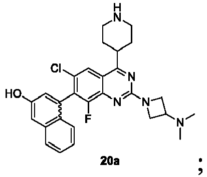
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.433 min; 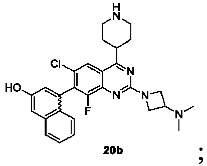
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.905 min; 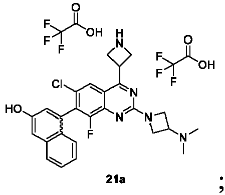
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 10 min; detector UV 220/254 nm; retention time: 3.80 min; 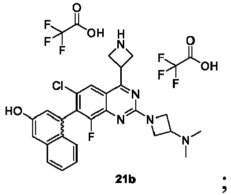
its HPLC conditions are: chiral column: CHIRALPAK IC-3, 4.6 x 100 mm, 3 µm; mobile phase A: n-hexane/dichloromethane = 5/1 (0.1% diethylamine), mobile phase B: ethanol; flow rate: 1 mL/min; isocratic elution with 30% phase B in 6 min; detector UV 220/254 nm; retention time: 5.950 min; 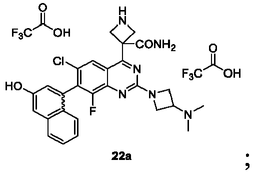
its HPLC conditions are: chiral column: CHIRALPAK ID-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 8 min; detector UV 220/254 nm; retention time: 3.40 min; 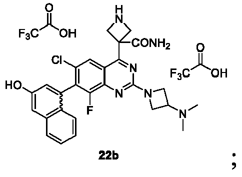
its HPLC conditions are: chiral column: CHIRALPAK ID-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 8 min; detector UV220/254 nm; retention time: 5.31 min; 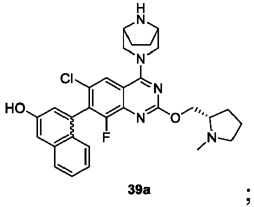
its HPLC conditions are: chiral column: CHIRALPAK IG-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 1.203 min; 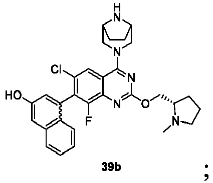
its HPLC conditions are: chiral column: CHIRALPAK IG-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 2.391 min; 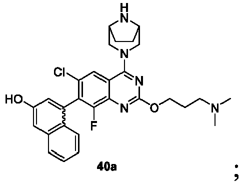
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.960 min; 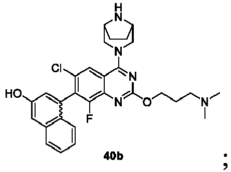
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.715 min; 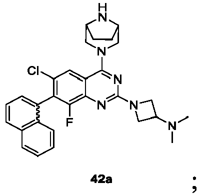
its HPLC conditions are: chiral column: CHIRALPAK ID-3, 4.6 x 50 mm, 3 µm; mobile phase A: *n*-hexane/dichloromethane = 3/1 (0. 1% diethylamine), mobile phase B: isopropanol; flow rate: 1 mL/min; isocratic elution with 10% phase B in 8 min; detector UV 220/254 nm; retention time: 3.759 min; 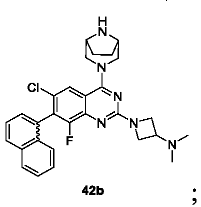
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 10% phase B in 8 min; detector UV 220/254 nm; retention time: 5.749 min; 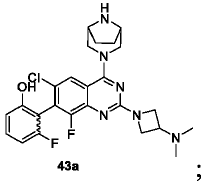
its HPLC conditions are: chiral column: CHIRALPAK IE-3, 3.0 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 3.306 min; 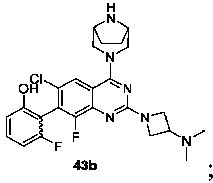
its HPLC conditions are: chiral column: CHIRALPAK IE-3, 3.0 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.803 min; 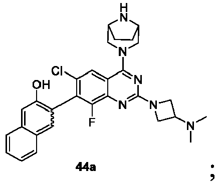
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.737 min; 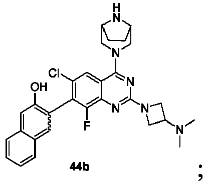
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.713 min; 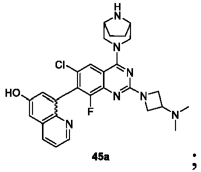
its HPLC conditions are: chiral column: CHIRALPAK IE-3, 4.6 x 50 mm, 3 µm; mobile phase A: methyl *tert*-butyl ether (0.1% diethylamine), mobile phase B: methanol; flow rate: 1 mL/min; isocratic elution with 50% phase B in 6 min; detector UV 220/254 nm; retention time: 1.200 min; 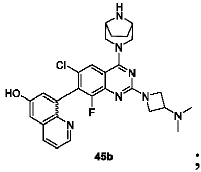
its HPLC conditions are: chiral column: CHIRALPAK IE-3, 4.6 x 50 mm, 3 µm; mobile phase A: methyl *tert*-butyl ether (0.1% diethylamine), mobile phase B: methanol; flow rate: 1 mL/min; isocratic elution with 50% phase B in 6 min; detector UV 220/254 nm; retention time: 3.550 min; 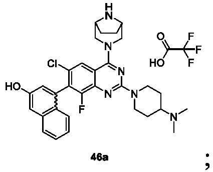
its HPLC conditions are: chiral column: CHIRALPAK IC-3, 4.6 x 50 mm, 3 µm; mobile phase A: *n*-hexane: methyl *tert*-butyl ether = 1:1 (0.1% diethylamine), mobile phase B: ethanol; flow rate: 1 mL/min; isocratic elution with 5% phase B in 10 min; detector UV 220/254 nm; retention time: 5.305 min; 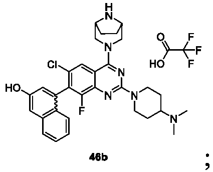
its HPLC conditions are: chiral column: CHIRALPAK IC-3, 4.6 x 50 mm, 3 µm; mobile phase A: *n*-hexane: methyl *tert*-butyl ether = 1:1 (0.1% diethylamine), mobile phase B: ethanol; flow rate: 1 mL/min; isocratic elution with 5% phase B in 10 min; detector UV 220/254 nm; retention time: 7.357 min; 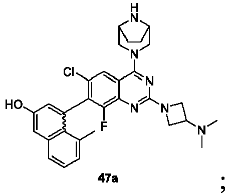
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 220/254 nm; retention time: 3.197 min; 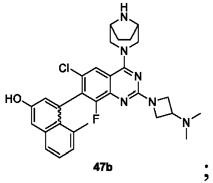
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 220/254 nm; retention time: 4.394 min; 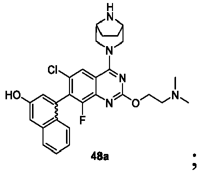
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 7 min; detector UV 220/254 nm; retention time: 4.100 min; 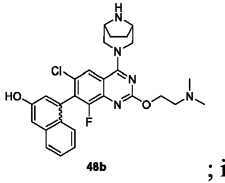
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 7 min; detector UV 220/254 nm; retention time: 5.751 min; 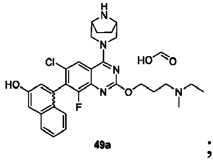
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 4 min; detector UV 220/254 nm; retention time: 1.910 min; 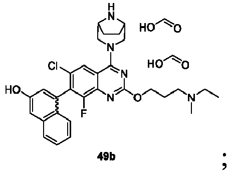
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 4 min; detector UV 220/254 nm; retention time: 2.941 min; 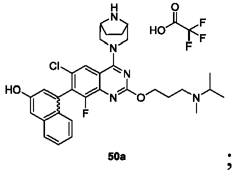
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 3.684 min; 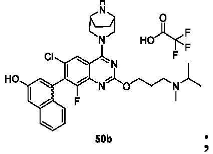
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 6.409 min; 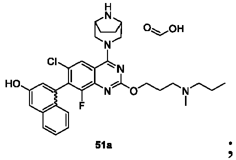
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 3.667 min; 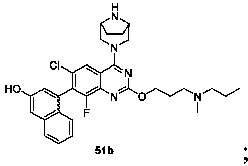
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 6.387 min; 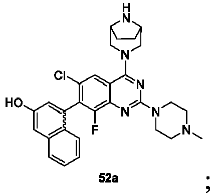
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 5 min; detector UV 220/254 nm; retention time: 1.686 min; 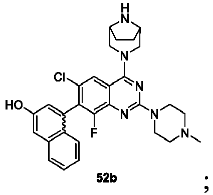
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 5 min; detector UV 220/254 nm; retention time: 2.959 min; 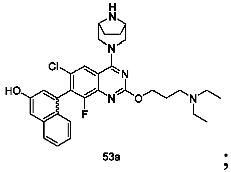
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 20% phase B in 6 min; detector UV 220/254 nm; retention time: 2.759 min; 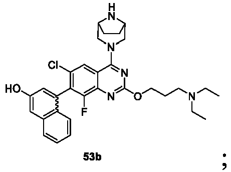
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 20% phase B in 6 min; detector UV 220/254 nm; retention time: 4.652 min; 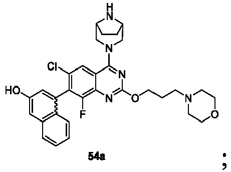
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 5 min; detector UV 220/254 nm; retention time: 1.963 min; 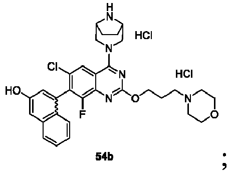
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 5 min; detector UV 220/254 nm; retention time: 3.148 min; 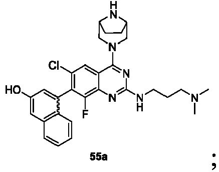
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8 min; detector UV 220 nm; retention time: 4.211 min; 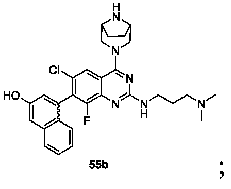
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8 min; detector UV 220 nm; retention time: 6.385 min; 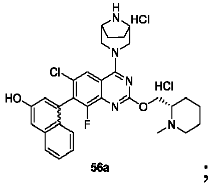
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 3.762 min; 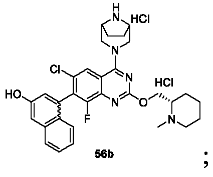
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 254 nm; retention time: 5.467 min; 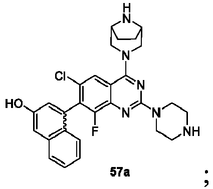
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 5.5 min; detector UV 254 nm; retention time: 2.173 min; 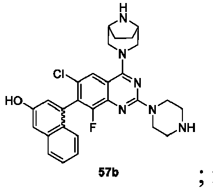
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 5.5 min; detector UV 254 nm; retention time: 3.537 min; 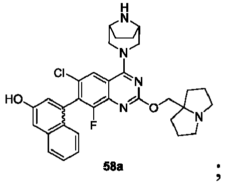
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 2.045 min; 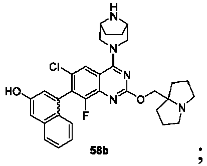
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 3.463 min; 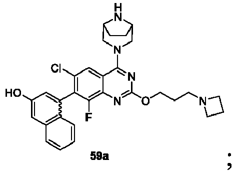
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 5.080 min; 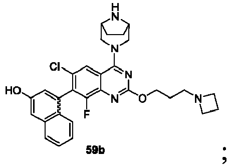
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 3.566 min; 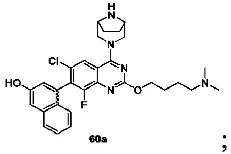
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 15 min; detector UV 254 nm; retention time: 5.980 min; 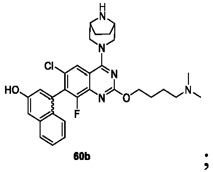
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 15 min; detector UV 254 nm; retention time: 10.313 min; 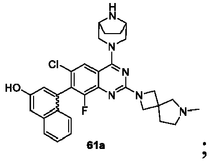
its HPLC conditions are: chiral column: CHIRALPAK ID-3, 4.6 x 50 mm, 3 µm; mobile phase A: *n*-hexane: methyl *tert*-butyl ether = 1/1 (0.1% diethylamine), mobile phase B: ethanol; flow rate: 1 mL/min; isocratic elution with 20% phase B in 6 min; detector UV 254 nm; retention time: 2.202 min; 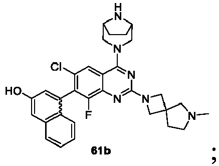
its HPLC conditions are: chiral column: CHIRALPAK ID-3, 4.6 x 50 mm, 3 µm; mobile phase A: *n*-hexane: methyl *tert*-butyl ether = 1/1 (0.1% diethylamine), mobile phase B: ethanol; flow rate: 1 mL/min; isocratic elution with 20% phase B in 6 min; detector UV 254 nm; retention time: 1.694 min; 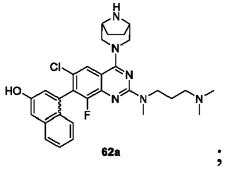
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 254 nm; retention time: 3.894 min; 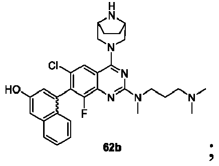
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 254 nm; retention time: 1.814 min; 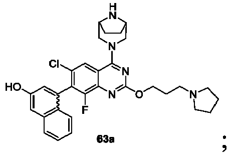
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 2.813 min; 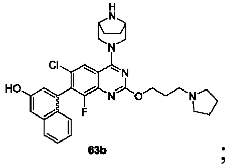
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 1.965 min; 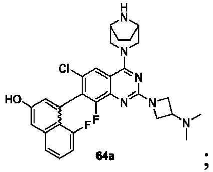
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 10% phase B in 8 min; detector UV 254 nm; retention time: 3.932 min; 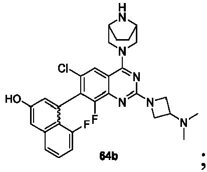
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 10% phase B in 10 min; detector UV 254 nm; retention time: 7.304 min; 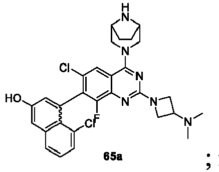
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 10% phase B in 10 min; detector UV 254 nm; retention time: 4.668 min; 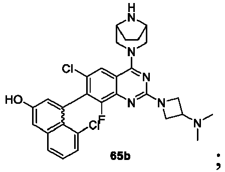
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 10% phase B in 10 min; detector UV 254 nm; retention time: 6.605 min; 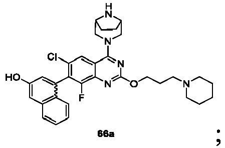
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 30% phase B in 4 min; detector UV 254 nm; retention time: 1.465 min; 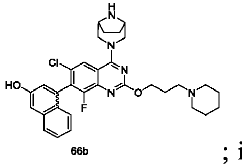
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 30% phase B in 4 min; detector UV 254 nm; retention time: 2.173 min; 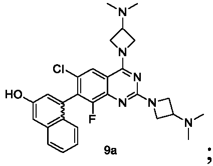
its HPLC conditions are: chiral column: CHIRALPAK IC-3, 4.6 x 50 mm, 3 µm; mobile phase A: *n*-hexane/dichloromethane = (5/1) (0.1% diethylamine), mobile phase B: isopropanol; flow rate: 1 mL/min; isocratic elution with 30% phase B in 6 min; detector UV 220/254 nm; retention time: 2.097 min; 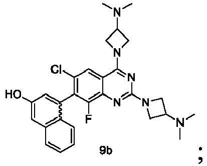
its HPLC conditions are: chiral column: CHIRALPAK IC-3, 4.6 x 50 mm, 3 µm; mobile phase A: *n*-hexane/dichloromethane = (5/1) (0.1% diethylamine), mobile phase B: isopropanol; flow rate: 1 mL/min; isocratic elution with 30% phase B in 6 min; detector UV 220/254 nm; retention time: 3.541 min; 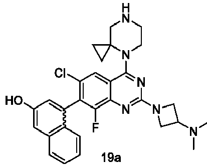
; its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 50% phase B in 6 min; detector UV 220/254 nm; retention time: 1.741 min; 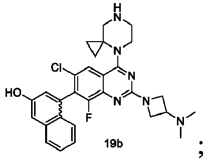
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 50% phase B in 6 min; detector UV 220/254 nm; retention time: 3.291 min; 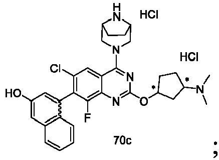
its HPLC conditions are: chiral column: XA-CHIRALPAK AD-3, 4.6 x 250 mm, 3 µm; mobile phase A: *n*-hexane (0.5% diethylamine), mobile phase B: ethanol; flow rate: 1 mL/min; isocratic elution with 50% phase B in 19 min; detector UV 254 nm; retention time: 8.374 min; 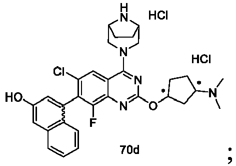
its HPLC conditions are: chiral column: XA-CHIRALPAK AD-3, 4.6 x 250 mm, 3 µm; mobile phase A: *n*-hexane (0.5% diethylamine), mobile phase B: ethanol; flow rate: 1 mL/min; isocratic elution with 50% phase B in 19 min; detector UV 254 nm; retention time: 13.763 min; 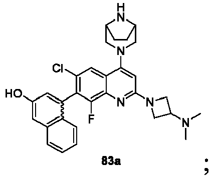
it is prepared from 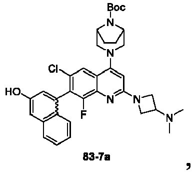 the HPLC conditions of 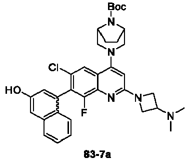 are: chiral column NB-Lux 5 µM i-Cellulose-5, 2.12 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 8% phase B in 40 min; detector: 220 nm; retention time: 16.81 min; 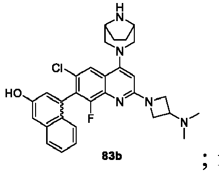
it is prepared from 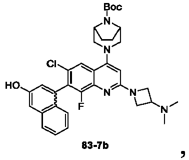 the HPLC conditions of 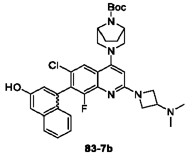 are: chiral column NB-Lux 5 µM i-Cellulose-5, 2.12 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 8% phase B in 40 min; detector: 220 nm; retention time: 25.09 min; 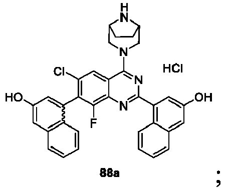
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8.5 min; detector UV 254 nm; retention time: 4.033 min; 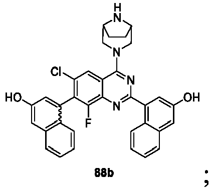
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8.5 min; detector UV 254 nm; retention time: 6.515 min; 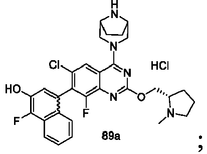
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 3.401 min; 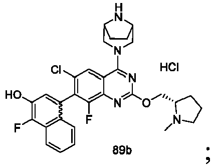
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 4.503 min; 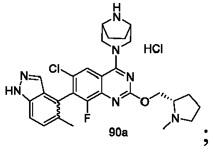
its HPLC conditions are: chiral column: CHIRALPAK IC-3, 3.0 x 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 1.442 min; 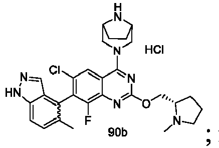
its HPLC conditions are: chiral column: CHIRALPAK IC-3, 3.0 x 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 2.226 min; 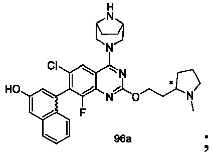
its HPLC conditions are: chiral column: CHIRALART Amylose-C Neo, 50 x 4.6 mm, 3 µm; mobile phase A: *n*-hexane (0.1% diethylamine), mobile phase B: isopropanol/acetonitrile = 2/1; flow rate: 1 mL/min; isocratic elution with 50% phase B in 6.5 min; detector UV 230 nm; retention time: 2.045 min; 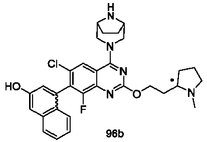
; its HPLC conditions are: chiral column: CHIRAL ART Amylose-C Neo, 50 x 4.6 mm, 3 µm; mobile phase A: *n*-hexane (0.1% diethylamine), mobile phase B: isopropanol/acetonitrile = 2/1; flow rate: 1 mL/min; isocratic elution with 50% phase B in 6.5 min; detector UV 230 nm; retention time: 4.319 min; 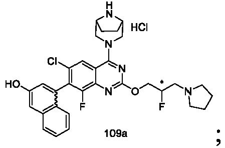
its HPLC conditions are: chiral column: CHIRALPAK ID-3, 4.6 x 50 mm, 3 µm; mobile phase A: *n*-hexane/dichloromethane = 5/1 (0. 1% diethylamine), mobile phase B: ethanol; flow rate: 1 mL/min; isocratic elution with 10% phase B in 12.5 min; detector UV 220 nm; retention time: 7.498 min; 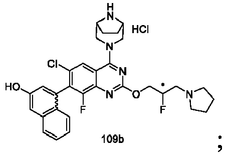
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 12.5 min; detector UV 220 nm; retention time: 9.454 min; 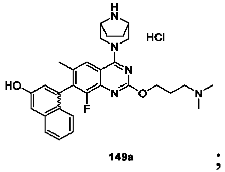
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 6 min; detector UV 220 nm; retention time: 2.457 min; 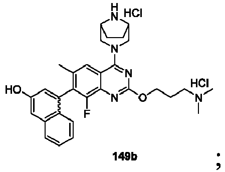
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 6 min; detector UV 220 nm; retention time: 3.982 min; 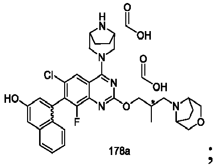
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 6 min; detector UV 220 nm; retention time: 4.024 min; 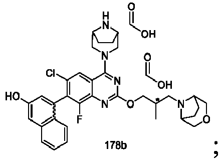
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 6 min; detector UV 220 nm; retention time: 5.203 min; 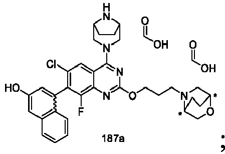
its HPLC conditions are: chiral column: XA-CHIRALPAKAS-3, 4.6 x 50 mm, 3 µm; mobile phase A: *n*-hexane (0.1% diethylamine), mobile phase B: acetonitrile; flow rate: 1.67 mL/min; isocratic elution with 20% phase B in 2 min; detector UV 220 nm; retention time: 0.821 min; 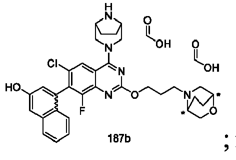
its HPLC conditions are: chiral column: XA-CHIRALPAKAS-3, 4.6 x 50 mm, 3 µm; mobile phase A: *n*-hexane (0.1% diethylamine), mobile phase B: acetonitrile; flow rate: 1.67 mL/min; isocratic elution with 20% phase B in 2 min; detector UV 220 nm; retention time: 1.215 min; 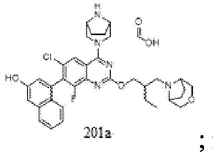
its HPLC conditions are: chiral column: CHIRALPAK IA-3, 4.6 x 100 mm, 3 µm; mobile phase A: *n*-hexane (0.2% n-butylamine), mobile phase B: isopropanol: acetonitrile (2: 1); flow rate: 1 mL/min; isocratic elution with 20% phase B in 8 min; detector UV 254 nm; retention time: 4.688 min; 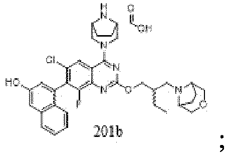
its HPLC conditions are: chiral column: CHIRALPAK IA-3, 4.6 x 100 mm, 3 µm; mobile phase A: *n*-hexane (0.2% n-butylamine), mobile phase B: isopropanol: acetonitrile (2: 1); flow rate: 1 mL/min; isocratic elution with 20% phase B in 8 min; detector UV 254 nm; retention time: 6.033 min; 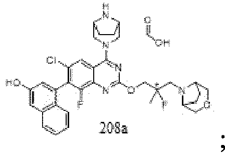
its HPLC conditions are: chiral column: CHIRALPAK IA-3, 4.6 x 100 mm, 3 µm; mobile phase A: *n*-hexane/dichloromethane (5: 1) (0.1% diethylamine), mobile phase B: ethanol; flow rate: 1 mL/min; isocratic elution with 10% phase B in 12.5 min; detector UV 220 nm; retention time: 8.927 min; 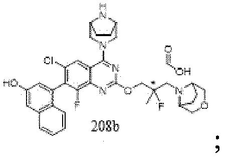
its HPLC conditions are: chiral column: CHIRALPAK IA-3, 4.6 x 100 mm, 3 µm; mobile phase A: *n*-hexane/dichloromethane (5: 1) (0.1% diethylamine), mobile phase B: ethanol; flow rate: 1 mL/min; isocratic elution with 10% phase B in 12.5 min; detector UV 220 nm; retention time: 9.894 min; 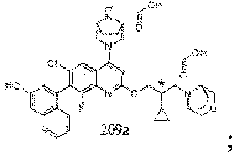
its HPLC conditions are: chiral column: CHIRALPAK ID-3, 3.0 x 100 mm, 3 µm; mobile phase A: supercritical carbon dioxide, mobile phase B: isopropanol (10 mmol ammonia); flow rate: 2 mL/min; gradient elution with 40% to 50% phase B in 8 min; detector UV 220 nm; retention time: 5.095 min; 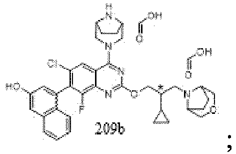
its HPLC conditions are: chiral column: CHIRALPAK ID-3, 3.0 x 100 mm, 3 µm; mobile phase A: supercritical carbon dioxide, mobile phase B: isopropanol (10 mmol ammonia); flow rate: 2 mL/min; gradient elution with 40% to 50% phase B in 8 min; detector UV 220 nm; retention time: 6.135 min; 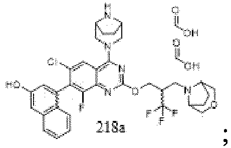
its HPLC conditions are: chiral column: CHIRALPAK IA-3, 3.0 x 100 mm, 3 µm; mobile phase A: supercritical carbon dioxide, mobile phase B: isopropanol (10 mmol ammonia); flow rate: 2 mL/min; isocratic elution with 50% phase B in 4 min; detector UV 220 nm; retention time: 1.742 min; 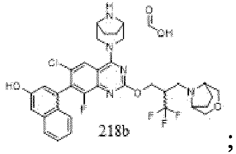
its HPLC conditions are: chiral column: CHIRALPAK IA-3, 3.0 x 100 mm, 3 µm; mobile phase A: supercritical carbon dioxide, mobile phase B: isopropanol (10 mmol ammonia); flow rate: 2 mL/min; isocratic elution with 50% phase B in 4 min; detector UV 220 nm; retention time: 2.904 min; 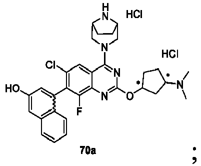
its HPLC conditions are: chiral column: CHIRALPAK IA-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 254 nm; retention time: 4.874 min; 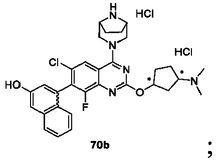
its HPLC conditions are: chiral column: CHIRALPAK IA-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 254 nm; retention time: 2.414 min; 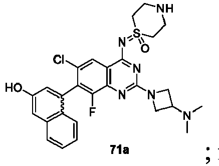
its HPLC conditions are: chiral column: CHIRALPAK IF-3, 4.6 x 50 mm, 3 µm; mobile phase A: *n*-hexane (0.1% diethylamine), mobile phase B: ethanol; flow rate: 1 mL/min; gradient, elution with 20% phase B in 12 min; detector UV 220/254 nm; retention time: 6.743 min; 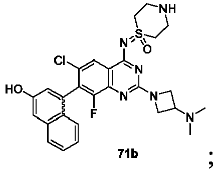
its HPLC conditions are: chiral column: CHIRALPAK IF-3, 4.6 x 50 mm, 3 µm; mobile phase A: *n*-hexane (0.1% diethylamine), mobile phase B: ethanol; flow rate: 1 mL/min; gradient, elution with 20% phase B in 12 min; detector UV 220/254 nm; retention time: 9.968 min; 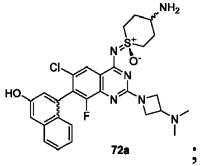
it is prepared from 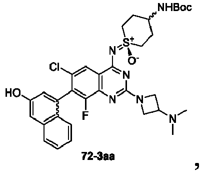 the HPLC conditions of 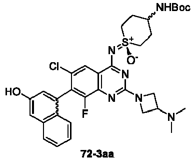 are: chiral column CHIRAL ART Cellulose-SB, 2 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 30% phase B in 10 min; detector UV 250/220 nm; retention time: 3.2 min; 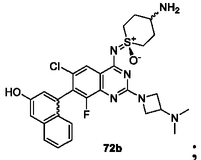
it is prepared from 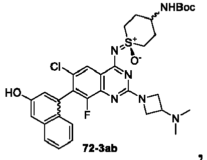 the HPLC conditions of 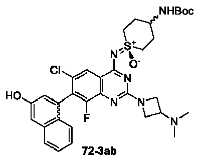 are: chiral column CHIRAL ART Cellulose-SB, 2 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 30% phase B in 10 min; detector UV 250/220 nm; retention time: 5.7 min; 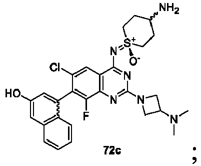
it is prepared from 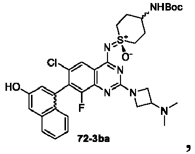 the HPLC conditions of 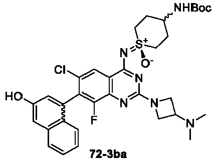 are: chiral column CHIRALART Cellulose-SB, 2 x 25 cm, 5 µm; mobile phase A: *n*-hexane (10 mol/L ammonia methanol solution), mobile phase B: ethanol; flow rate: 20 mL/min; gradient: elution with 30% phase B in 23 min; detector UV 250/220 nm; retention time: 5.8 min; 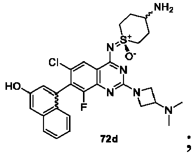
it is prepared from 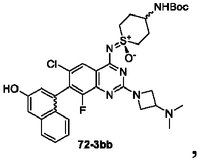 the HPLC conditions of 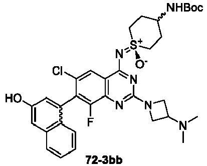 are: chiral column CHIRAL ART Cellulose-SB, 2 x 25 cm, 5 µm; mobile phase A: *n*-hexane (10 mol/L ammonia methanol solution), mobile phase B: ethanol; flow rate: 20 mL/min; gradient: elution with 30% phase B in 23 min; detector UV 250/220 nm; shorter retention time: 15.8 min; 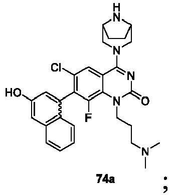
its HPLC conditions are: chiral column: CHIRALPAK ID-3, 4.6 x 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV220/254 nm; retention time: 3.994 min; 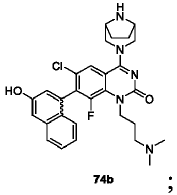
its HPLC conditions are: chiral column: CHIRALPAK ID-3, 4.6 x 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV 220/254 nm; retention time: 4.737 min; 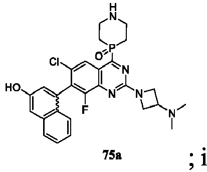
ts HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 17 min; detector UV 254 nm; retention time: 11.543 min; 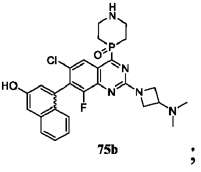
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 17 min; detector UV 254 nm; retention time: 6.706 min; 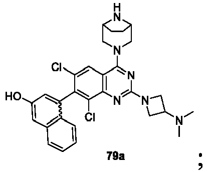
it is prepared from 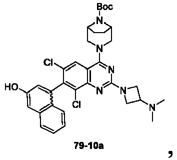 the HPLC conditions of 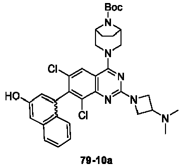 are: chiral column CHIRALPAK IA, 2 x 25 cm, 5 µm; mobile phase A: *n-*hexane (10 mol/L ammonia methanol solution), mobile phase B: isopropanol; flow rate: 20 mL/min; gradient: elution with 30% mobile phase in 11.5 min; detector UV 220/210 nm; retention time: 4.342 min; 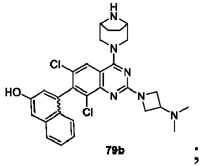
it is prepared from 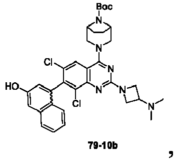 the HPLC conditions of 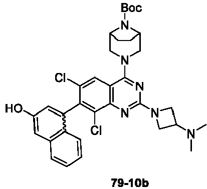 are: chiral column CHIRAL IA, 2 x 25 cm, 5 µm; mobile phase A: *n-*hexane (10 mol/L ammonia methanol solution), mobile phase B: isopropanol; flow rate: 20 mL/min; gradient: elution with 30% mobile phase in 11.5 min; detector UV 220/210 nm; retention time: 7.54 min; 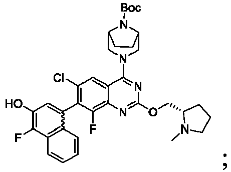
its HPLC conditions are: chiral column CHIRAL ART Cellulose-SC, 2 x 25 cm, 5 µm; mobile phase A: *n*-hexane (0.5% 2 mol/L ammonia methanol solution), mobile phase B: ethanol; flow rate: 20 mL/min; gradient: elution with 20% phase B in 12 min; detector UV 220/254 nm; retention time: 5.875 min; 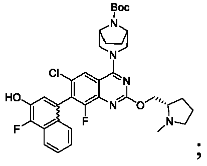
its HPLC conditions are: chiral column: CHIRALART Cellulose-SC, 2 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 20% phase B in 12 min; detector UV 220/254 nm; retention time: 8.193 min; 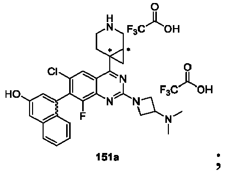
it is prepared from 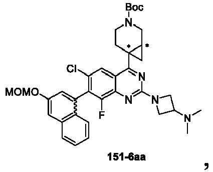 the HPLC conditions of 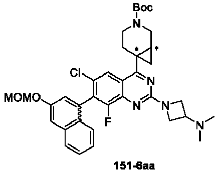 are: chiral column: CHIRALCEL AY-H, 2 x 25 cm, 5 µm; mobile phase A: *n*-hexane (0.5% 2 mol/L ammonia methanol solution), mobile phase B: isopropanol/acetonitrile= 2/1; flow rate: 20 mL/min; gradient: elution with 30% phase B in 13 min; detector UV 226/254 nm; retention time: 3.7 min; 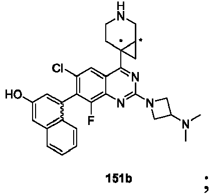
it is prepared from 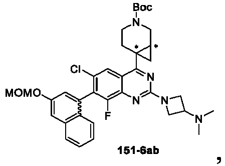 the HPLC conditions of 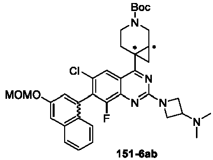 are: chiral column: CHIRALCEL AY-H, 2 x 25 cm, 5 µm; mobile phase A: *n*-hexane (0.5% 2 mol/L ammonia methanol solution), mobile phase B: isopropanol/acetonitrile= 2/1; flow rate: 20 mL/min; gradient: elution with 30% phase B in 13 min; detector UV 226/254 nm; retention time: 6.8 min; 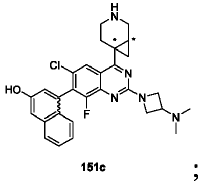
it is prepared from 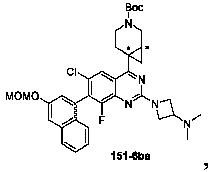 the HPLC conditions of 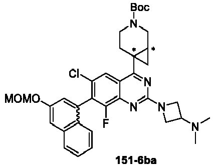 are: chiral column: CHIRALPAK IE, 2 x 25 cm, 5 µm; mobile phase A: *n*-hexane (10 mol/L ammonia methanol solution), mobile phase B: isopropanol/acetonitrile= 2/1; flow rate: 20 mL/min; gradient elution with 10% phase B in 18 min; detector UV 226/254 nm; retention time: 6 min; 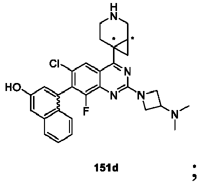
it is prepared from 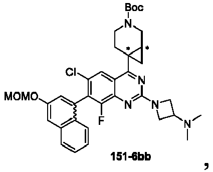 the HPLC conditions of 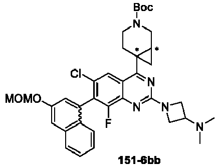 are: chiral column: CHIRALPAK IE, 2 x 25 cm, 5 µm; mobile phase A: n-hexane (10 mol/L ammonia methanol solution), mobile phase B: isopropanol/acetonitrile= 2/1; flow rate: 20 mL/min; gradient elution with 10% phase B in 18 min; detector UV 226/254 nm; retention time: 8.5 min; 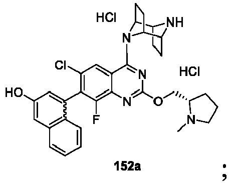
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 6 min; detector UV 220 nm; retention time: 3.123 min; 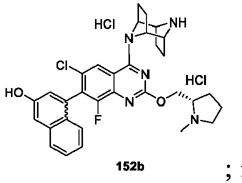
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 6 min; detector UV 220 nm; retention time: 5.171 min; 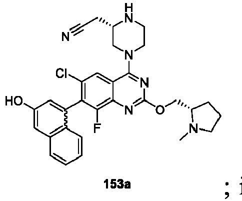
its HPLC conditions are: chiral column: CHIRALPAK ID-3, 4.6 x 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 1.508 min; 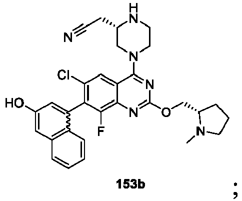
its HPLC conditions are: chiral column: CHIRALPAK ID-3, 4.6 x 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 2.593 min; 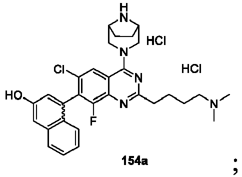
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220 nm; retention time: 2.156 min; 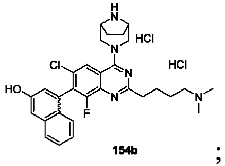
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220 nm; retention time: 4.420 min; 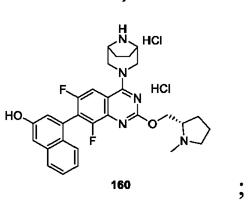
it is prepared from 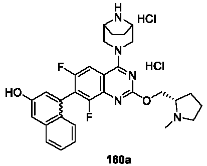 and 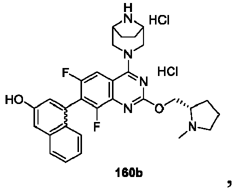 the HPLC conditions of 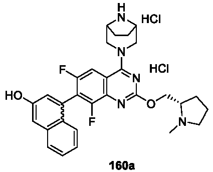 are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector UV 254 nm; retention time: 2.198 min; the HPLC conditions of 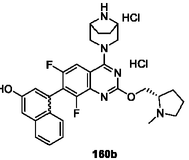 are: chiral column: CHIRALPAK IC-3, 4.6 x 50 mm, 3 µm; mobile phase A: n-hexane (0.1% diethylamine), mobile phase B: isopropanol; flow rate: 1 mL/min; isocratic elution with 30% phase B in 6 min; detector UV 254 nm; retention time: 3.411 min; 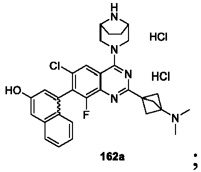
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector: UV 220 nm; retention time: 2.239 min; 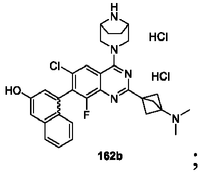
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector UV 220 nm; retention time: 3.881 min; 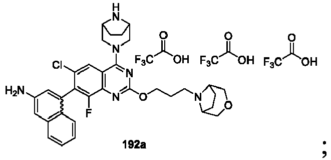
its HPLC conditions are: chiral column: CHIRAL Cellulose-SB, 4.6 x 100 mm, 3 µm; mobile phase A: *n*-hexane: methyl *tert*-butyl ether = 1:1 (0.1% diethylamine), mobile phase B: ethanol; flow rate: 1 mL/min; isocratic elution with 30% phase B in 8.5 min; detector UV 220/254 nm; retention time: 5.962 min; 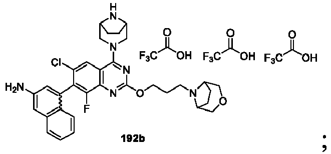
its HPLC conditions are: chiral column: CHIRAL Cellulose-SB, 4.6 x 100 mm, 3 µm; mobile phase A: *n*-hexane: methyl *tert*-butyl ether = 1:1 (0.1% diethylamine), mobile phase B: ethanol; flow rate: 1 mL/min; isocratic elution with 30% phase B in 8.5 min; detector UV 220/254 nm; retention time: 7.373 min; 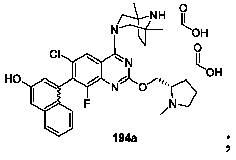
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 8 min; detector UV 220/254 nm; retention time: 1.999 min; 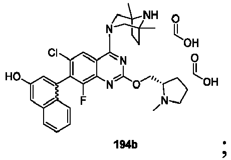
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 8 min; detector UV 220/254 nm; retention time: 3.292 min; 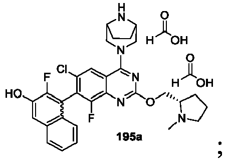
its HPLC conditions are: chiral column: N--Lux 3µm Cellulose-4 (H18-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 3.734 min; 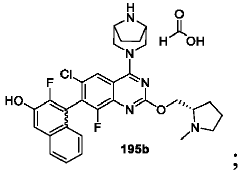
its HPLC conditions are: chiral column: N--Lux 3µm Cellulose-4 (H18-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 2.913 min; 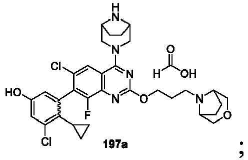
its HPLC conditions are: chiral column: CHIRAL ART Cellulose-SB, 4.6 x 100 mm, 3 µm; mobile phase A: *n*-hexane (0.1% diethylamine), mobile phase B: ethanol; flow rate: 1 mL/min; isocratic elution with 10% phase B in 4.5 min; detector UV 220 nm; retention time: 1.346 min; 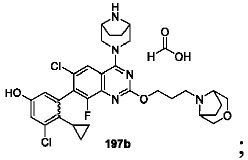
its HPLC conditions are: chiral column: CHIRAL ART Cellulose-SB, 4.6 x 100 mm, 3 µm; mobile phase A: *n*-hexane (0.1% diethylamine), mobile phase B: ethanol; flow rate: 1 mL/min; isocratic elution with 10% phase B in 4.5 min; detector UV 220 nm; retention time: 2.438 min; 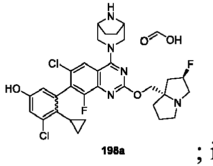
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 3.5 min; detector UV 220/254 nm; retention time: 1.198 min; 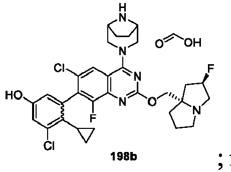
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 4 min; detector UV 220/254 nm; retention time: 1.880 min; 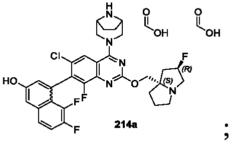
its HPLC conditions are: chiral column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 4.125 min; 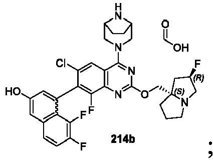
its HPLC conditions are: chiral column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 2.126 min; 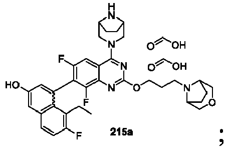
its HPLC conditions are: chiral column: XA-CHIRALPAK IG-3, 4.6 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 4.214 min; 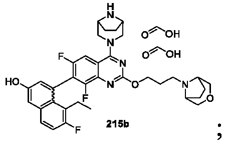
its HPLC conditions are: chiral column: XA-CHIRALPAK IG-3, 4.6 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 2.706 min; 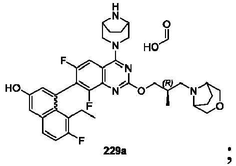
its HPLC conditions are: chiral column: CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 1.083 min; 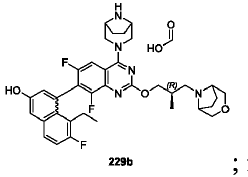
its HPLC conditions are: chiral column: CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 2.010 min; 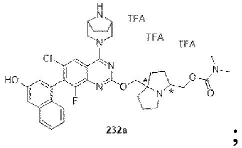
its HPLC conditions are: chiral column: CHIRALPAK IA-3, 4.6 × 50 mm, 3 µm; mobile phase A: *n*-hexane (0.1% diethylamine), mobile phase B: ethanol (10mmol/L ammonia); flow rate: 1 mL/min; isocratic elution with 50% phase B in 4 min; detector UV 220 nm; retention time: 2.761 min; 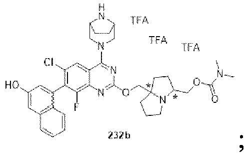
its HPLC conditions are: chiral column: CHIRALPAK IA-3, 4.6 × 50 mm, 3 µm; mobile phase A: *n*-hexane (0.1% diethylamine), mobile phase B: ethanol (10mmol/L ammonia); flow rate: 1 mL/min; isocratic elution with 50% phase B in 4 min; detector UV 220 nm; retention time: 1.705 min; 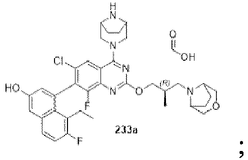
its HPLC conditions are: chiral column: CHIRALPAK IC-3, 4.6 × 50 mm, 3 µm; mobile phase A: n-hexane (0.1% diethylamine), mobile phase B: ethanol; flow rate: 1 mL/min; isocratic elution with 40% phase B in 3.5 min; detector UV 254 nm; retention time: 2.301 min; 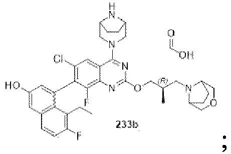
its HPLC conditions are: chiral column: CHIRALPAK IC-3, 4.6 × 50 mm, 3 µm; mobile phase A: *n*-hexane (0.1% diethylamine), mobile phase B: ethanol; flow rate: 1 mL/min; isocratic elution with 40% phase B in 4.5 min; detector UV 254 nm; retention time: 1.465 min; 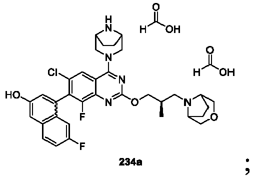
its HPLC conditions are: chiral column: N-Lux 3µm Cellulose-4 (H17-388767); 4.6 × 100 mm, 3 µm; mobile phase A: supercritical carbon dioxide, mobile phase B: methanol (0.1% diethylamine); flow rate: 3.5 mL/min; isocratic elution with 48% phase B in 6 min; detector UV 220 nm; retention time: 2.41 min; 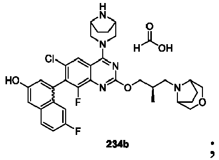
its HPLC conditions are: chiral column: N-Lux 3µm Cellulose-4 (H17-388767); 4.6 × 100 mm, 3 µm; mobile phase A: supercritical carbon dioxide, mobile phase B: methanol (0.1% diethylamine); flow rate: 3.5 mL/min; isocratic elution with 48% phase B in 6 min; detector UV 220 nm; retention time: 3.66 min; 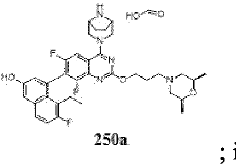
its HPLC conditions are: chiral column: N--CHIRALPAK IC-3 (LotNo.IC3SCK-VK002), 3.0 × 100 mm, 3 µm; mobile phase A: supercritical carbon dioxide, mobile phase B: methanol (20 mmol/L ammonia); flow rate: 2 mL/min; isocratic elution with 10% phase B in 6 min; detector UV 220 nm; retention time: 4.514 min; 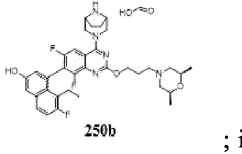
its HPLC conditions are: chiral column: N--CHIRALPAK IC-3 (Lot No.IC3SCK-VK002), 3.0 × 100 mm, 3 µm; mobile phase A: supercritical carbon dioxide, mobile phase B: methanol (20 mmol/L ammonia); flow rate: 2 mL/min; isocratic elution with 10% phase B in 6 min; detector UV 220 nm; retention time: 4.948 min; 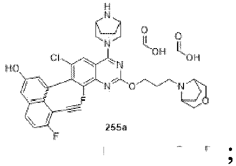
its HPLC conditions are: chiral column: N-CHIRALPAK IG-3, 3.0 × 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 0.845 min; 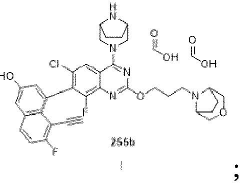
its HPLC conditions are: chiral column: N-CHIRALPAK IG-3, 3.0 × 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 1.905 min; 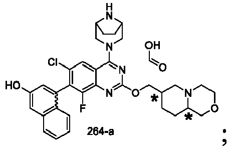
its HPLC conditions are: chiral column: N-CHIRALPAK IE-3 (Lot No.IF3SCK-SD016), 3 × 100 mm, 3 µm; mobile phase A: supercritical carbon dioxide, mobile phase B: methanol (20 mmol/L ammonia); flow rate: 2 mL/min; isocratic elution with 50% phase B in 8 min; detector: UV 220 nm; retention time: 4.857 min; 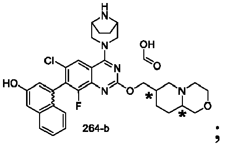
its HPLC conditions are: chiral column: N--CHIRALPAK IE-3 (Lot No.IF3SCK-SD016), 3 × 100 mm, 3 µm; mobile phase A: supercritical carbon dioxide, mobile phase B: methanol (20 mmol/L ammonia); flow rate: 2 mL/min; isocratic elution with 50% phase B in 8 min; detector: UV 220 nm; retention time: 5.877 min; 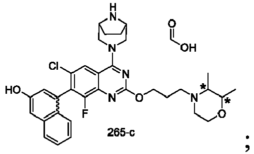
its HPLC conditions are: chiral column: N-Lux 3u i-Cellulose-5; 0.46 × 10 cm, 3 µm; mobile phase A: supercritical carbon dioxide, mobile phase B: methanol: dichloromethane = 1:1 (20 mmol/L ammonia); flow rate: 2 mL/min; isocratic elution with 50% phase B; detector UV 230 nm; retention time: 4.976 min; 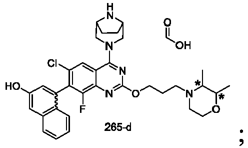
its HPLC conditions are: chiral column: N-Lux 3u i-Cellulose-5, 0.46 × 10 cm, µm; mobile phase A: supercritical carbon dioxide fluid, mobile phase B: methanol: dichloromethane = 1:1 (20 mmol/L ammonia); flow rate: 2 mL/min; isocratic elution with 50% phase B; detector UV 230 nm; retention time: 5.657 min; 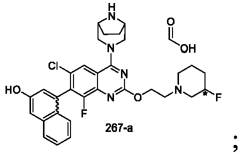
its HPLC conditions are: chiral colunm:N-Lux 3 µm Cellulose-2 (H18-089501), 4.6 × 100 mm, 3.0 µm; mobile phase A: supercritical carbon dioxide, mobile phase B: methanol (0.1% diethylamine); flow rate: 4 mL/min; isocratic elution with 35% phase B; detector: UV 220 nm; retention time: 9.221 min; 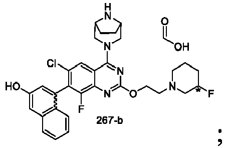
its HPLC conditions are: chiral column: N-Lux 3 µm Cellulose-2 (H18-089501), 4.6 × 100 mm, 3.0 µm; mobile phase A: supercritical carbon dioxide fluid, mobile phase B: methanol (0.1% diethylamine); flow rate: 4 mL/min; isocratic elution with 35% phase B; detector: UV 220 nm; retention time: 7.830 min; 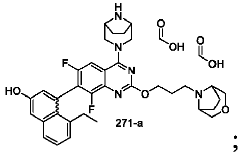
its HPLC conditions are: chiral column: CHIRALPAK ID-3 (Lot No.ID3SCK-TB004), 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.822 min; 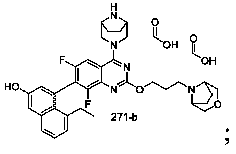
its HPLC conditions are: chiral column: CHIRALPAK ID-3 (Lot No.ID3SCK-TB004), 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.296 min; 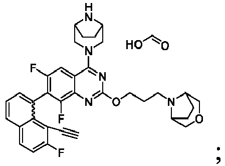
its HPLC conditions are: chiral column: CHIRALART Cellulose-SB (Ser.No. 105CA80166), 4.6 × 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 2.055 min; 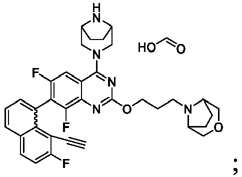
its HPLC conditions are: chiral column: CHIRALART Cellulose-SB (Ser.No. 105CA80166), 4.6 × 100 mm, 3 µm; mobile phase A: supercritical carbon dioxide fluid, mobile phase B: methanol (20 mmol/L ammonia); flow rate: 2 mL/min; isocratic elution with 50% phase B in 4 min; detector UV 230 nm; retention time: 1.632 min; 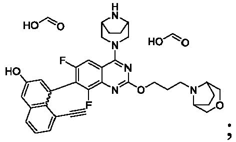
its HPLC conditions are: chiral column: CHIRALPAK ID-3 (Lot No.ID3SCK-TB004), 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.878 min; 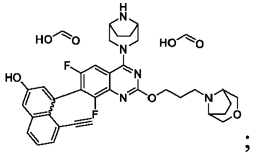
its HPLC conditions are: chiral column: CHIRALPAK ID-3 (Lot No.ID3SCK-TB004), 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.217 min; 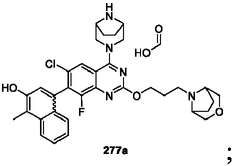
its HPLC conditions are: chiral column: N-CHIRALPAK IG-3, 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 2.601 min; 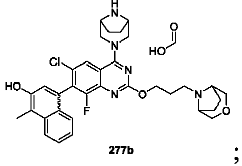
its HPLC conditions are: chiral column: N-CHIRALPAK IG-3, 3.0 × 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 0.815 min; 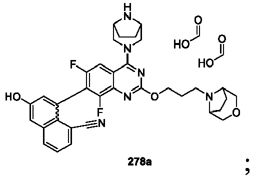
its HPLC conditions are: chiral column: N-CHIRALPAK IG-3, 3.0 × 100 mm, 3 µm; mobile phase A: supercritical carbon dioxide fluid, mobile phase B: isopropanol (20 mmol/L ammonia); flow rate: 2 mL/min; isocratic elution with 10% phase B in 6.5 min; detector UV 230 nm; retention time: 4.553 min; 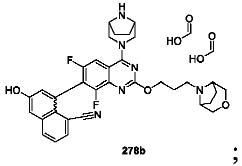
its HPLC conditions are: chiral column: N-CHIRALPAK IG-3, 3.0 × 100 mm, 3 µm; mobile phase A: supercritical carbon dioxide, mobile phase B: isopropanol (20 mmol/L ammonia); flow rate: 2 mL/min; isocratic elution with 10% phase B in 6.5 min; detector UV 230 nm; retention time: 4.074 min; 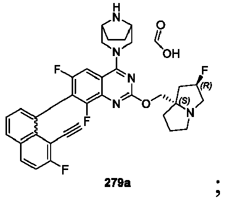
its HPLC conditions are: chiral column: CHIRALPAK IC-3(Lot No.IC3SCK-VK002), 3 × 100 mm, 3 µm; mobile phase A: supercritical carbon dioxide, mobile phase B: methanol (20 mmol/L ammonia); flow rate: 2 mL/min; elution with 50% phase B in 4 min; detector UV 230 nm; retention time: 2.80 min; 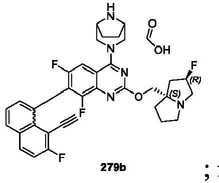
its HPLC conditions are: chiral column: CHIRALPAK IC-3(Lot No.IC3SCK-VK002), 3 × 100 mm, 3 µm; mobile phase A: supercritical carbon dioxide, mobile phase B: methanol (20 mmol/L ammonia); flow rate: 2 mL/min; elution with 50% phase B in 4 min; detector UV 230 nm; retention time: 2.318 min; 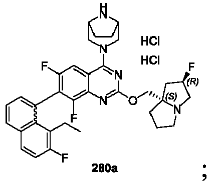
its HPLC conditions are: chiral column: Cellulose-SB, 3 × 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 1.911 min; 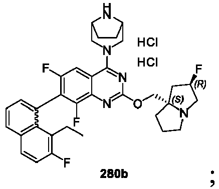
its HPLC conditions are: chiral column: Cellulose-SB, 3 × 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.171 min; 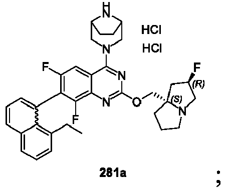
its HPLC conditions are: chiral column: CHIRAL ART Cellulose-SB, 3 × 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.148 min; 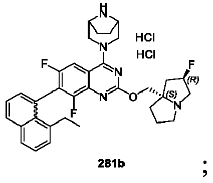
its HPLC conditions are: chiral column CHIRAL ART Cellulose-SB, 3 × 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.443 min; 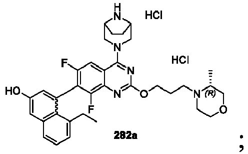
its HPLC conditions are: chiral column: N-Lux 3µm Cellulose-4 (H17-388767); 4.6 × 100 mm, 3 µm; mobile phase A: supercritical carbon dioxide, mobile phase B: ethanol (20 mmol/L ammonia); flow rate: 3.5 mL/min; isocratic elution with 35% phase B in 6.5 min; detector UV 220 nm; retention time: 3.455 min; 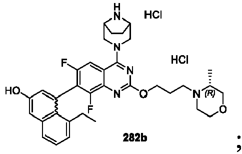
its HPLC conditions are: chiral column: N---Lux 3um Cellulose-4 (H17-388767); 4.6 × 100 mm, 3 µm; mobile phase A: supercritical carbon dioxide, mobile phase B: ethanol (20 mmol/L ammonia); flow rate: 3.5 mL/min; isocratic elution with 35% phase B in 6.5 min; detector UV 220 nm; retention time: 4.723 min; 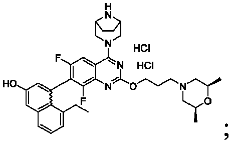
its HPLC conditions are: chiral column: N-Lux 3µm Cellulose-2 (H18-089501), 4.6 × 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 1.349 min; 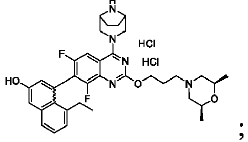
its HPLC conditions are: chiral column: N-Lux 3µm Cellulose-2 (H18-089501), 4.6 × 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 1.903 min; 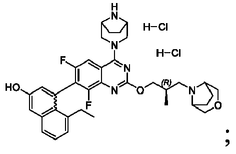
its HPLC conditions are: chiral column: N-Lux 3µm Cellulose-2 (H18-089501), 4.6 × 100 mm, 3 µm; supercritical mobile phase A: carbon dioxide, mobile phase B: methanol (0.1% diethylamine); flow rate: 4 mL/min; gradient, elution with 50% phase B in 6 min; detector UV 254 nm; retention time: 2.715 min; 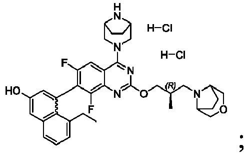
its HPLC conditions are: chiral column: N-Lux 3µm Cellulose-2 (H18-089501), 4.6 × 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 3.767 min; 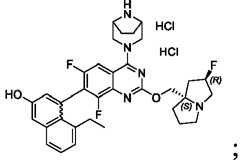
its HPLC conditions are: chiral column: N-Lux 3µm Cellulose-2 (H18-089501), 4.6 × 100 mm, 3 µm; mobile phase A: supercritical carbon dioxide, mobile phase B: methanol (0.1% diethylamine); flow rate: 4 mL/min; gradient, elution with 40% phase B in 7 min; detector UV 220 nm; retention time: 3.698 min; 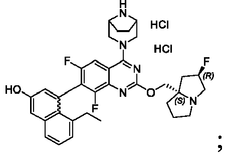
its HPLC conditions are: chiral column: N-Lux 3µm Cellulose-2 (H18-089501), 4.6 × 100 mm, 3 µm; mobile phase A: supercritical carbon dioxide, mobile phase B: methanol (0.1% diethylamine); flow rate: 4 mL/min; gradient, elution with 40% phase B in 7 min; detector UV 220 nm; retention time: 5.109 min; 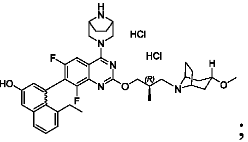
its HPLC conditions are: chiral column: CHIRALPAK IC-3, 4.6 × 50 mm, 3 µm; mobile phase A: *n*-hexane (0.1% diethylamine), mobile phase B: ethanol; flow rate: 1 mL/min; gradient: elution with 50% phase B in 4 min; detector UV 254 nm; retention time: 0.617 min; 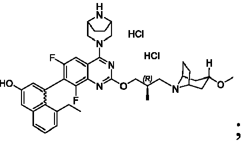
its HPLC conditions are: chiral column: CHIRALPAK IC-3, 4.6 × 50 mm, 3 µm; mobile phase A: *n*-hexane (0.1% diethylamine), mobile phase B: ethanol; flow rate: 1 mL/min; gradient: elution with 50% phase B in 4 min; detector UV 254 nm; retention time: 1.334 min.

In the present disclosure, the quinazoline compound represented by formula I, the pharmaceutically acceptable salt thereof, the solvate thereof, the prodrug thereof, the metabolite thereof or the isotopic compound thereof can have one or more chiral carbon atoms, so that optically pure isomers, such as pure enantiomers, or racemates, or mixed isomers can be isolated. Pure single isomers can be obtained by separation methods in the art, such as chiral crystallization into salts, or chiral preparative column separations.

In the present disclosure, if a stereoisomer of the quinazoline compound represented by formula I, the pharmaceutically acceptable salt thereof, the solvate thereof, the prodrug thereof, the metabolite thereof or the isotopic compound thereof exists, then it can exist in the form of a single tautomer or a mixture thereof (for example, racemate). The term "stereoisomer" refers to *cis-trans* isomer or optical isomer. These stereoisomers can be separated, purified and enriched by asymmetric synthesis or chiral separation methods (including but not limited to thin layer chromatography, rotary chromatography, column chromatography, gas chromatography, high pressure liquid chromatography, etc.), and can also be obtained by chiral resolution by bonding (chemical bonding, etc.) or salting (physical bonding, etc.) with other chiral compounds. The term "single stereoisomer" means that one stereoisomer of a compound of the present disclosure is not less than 95% by mass relative to all stereoisomers of the compound.

In the present disclosure, if a stereoisomer of the quinazoline compound represented by formula I, the pharmaceutically acceptable salt thereof, the solvate thereof, the prodrug thereof, the metabolite thereof or the isotopic compound thereof exists, then it can exist in the form of a single tautomer or a mixture thereof, preferably in the form of a more stable tautomer. For example, when the following structural fragments are included:

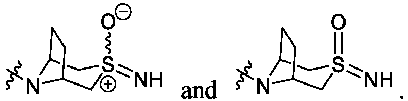

The compounds of the present disclosure also include crystalline forms and amorphous forms of those compounds with the same type of activity, pharmaceutically acceptable salts and active metabolites, including, for example, polymorphs, pseudopolymorphs, solvates, hydrates, unsolvated polymorphs (including anhydrous substances), conformational polymorphs and amorphous forms of compounds, and mixtures thereof.

The compounds described herein can exhibit their natural isotopic abundance, or one or more atoms can be artificially enriched in one or more atoms having the same atomic number, but an atomic mass or mass number different from that found in nature. All isotopic variants of the compounds of the present disclosure, whether radioactive or not, are within the scope of the present disclosure. For example, hydrogen has three naturally occurring isotopes, namely 1H (protium), 2 H (deuterium) and 3 H (tritium). Protium is the most abundant hydrogen isotope in nature. Deuterium enrichment can provide some therapeutic advantages, such as increasing half-life and/or exposure *in vivo*, or can provide compounds that can be used to study drug elimination and metabolic pathways *in vivo.* Isotope-enriched compounds can be prepared by conventional techniques well known to those skilled in the art.

The quinazoline compound represented by formula I, the pharmaceutically acceptable salt thereof, the solvate thereof, the prodrug thereof, the metabolite thereof or the isotopic compound thereof of the present disclosure can be synthesized by methods similar to those known in the chemical field, and the steps and conditions can be referred to the steps and conditions of similar reactions in the field, especially according to the description herein. Starting materials are typically from commercial sources such as Aldrich or can be readily prepared using methods known to those skilled in the art (obtained through SciFinder, Reaxys online database).

The present disclosure provides a pharmaceutical composition comprising the quinazoline compound represented by formula I, the pharmaceutically acceptable salt thereof, the solvate thereof, the prodrug thereof, the metabolite thereof or the isotopic compound thereof, and one or more pharmaceutical excipients. In the pharmaceutical composition, the amount of the quinazoline compound represented by formula I, the pharmaceutically acceptable salt thereof, the solvate thereof, the prodrug thereof, the metabolite thereof or the isotopic compound thereof can be in a therapeutically effective amount.

The present disclosure also provides a use of the quinazoline compound represented by formula I, the pharmaceutically acceptable salt thereof, the solvate thereof, the prodrug thereof, the metabolite thereof or the isotopic compound thereof, or the pharmaceutical composition in the manufacture of a KRAS mutant protein inhibitor. In the use, the KRAS mutant protein can be KRAS G12D mutant protein; the KRAS mutant protein inhibitor is used *in vitro*, mainly for experimental purposes, for example, the KRAS mutant protein can be used as a standard sample or a control sample to provide comparison, or can be made into a kit according to the conventional method in the art to provide rapid detection for the effect of KRAS G12D mutant protein inhibitor.

The present disclosure also provides a use of the quinazoline compound represented by formula I, the pharmaceutically acceptable salt thereof, the solvate thereof, the prodrug thereof, the metabolite thereof or the isotopic compound thereof, or the pharmaceutical composition in the manufacture of a medicament, the medicament is preferably used for the prevention and/or treatment of cancer mediated by KRAS mutation; and the KRAS mutation protein can be KRAS G12D mutant protein; the cancer can be hematological cancer, pancreatic cancer, MYH-associated polyposis, colorectal cancer or lung cancer and the like.

The present disclosure provides a use of the quinazoline compound represented by formula I, the pharmaceutically acceptable salt thereof, the solvate thereof, the prodrug thereof, the metabolite thereof or the isotopic compound thereof, or the pharmaceutical composition in the manufacture of a medicament, the medicament is preferably used for the prevention and/or treatment of cancer. The cancer is, for example, hematological cancer, pancreatic cancer, MYH-associated polyposis, colorectal cancer or lung cancer and the like.

The present disclosure also provides a use of the quinazoline compound represented by formula I, the pharmaceutically acceptable salt thereof, the solvate thereof, the prodrug thereof, the metabolite thereof or the isotopic compound thereof, or the pharmaceutical composition in the manufacture of a medicament. The medicament can be used for the prevention and/or treatment of cancer mediated by KRAS mutation; and the KRAS mutation protein can be KRAS G12D mutant protein; the cancer can be hematological cancer, pancreatic cancer, MYH-associated polyposis, colorectal cancer or lung cancer and the like.

The present disclosure also provides a use of the quinazoline compound represented by formula I, the pharmaceutically acceptable salt thereof, the solvate thereof, the prodrug thereof, the metabolite thereof or the isotopic compound thereof, or the pharmaceutical composition in the manufacture of a medicament. The medicament can be used for the prevention and/or treatment of cancer. The cancer is, for example, hematological cancer, pancreatic cancer, MYH-associated polyposis, colorectal cancer or lung cancer and the like.

The present disclosure also provides a method for preventing and/or treating cancer mediated by KRAS mutation, comprising administering a therapeutically effective amount of the quinazoline compound represented by formula I, the pharmaceutically acceptable salt thereof, the solvate thereof, the prodrug thereof, the metabolite thereof or the isotopic compound thereof, or the pharmaceutical composition to a subject. The cancer is, for example, hematological cancer, pancreatic cancer, MYH-associated polyposis, colorectal cancer or lung cancer and the like. The KRAS mutant protein can be KRAS G12D mutant protein.

The present disclosure provides a method for preventing and/or treating cancer, comprising administering a therapeutically effective amount of the quinazoline compound represented by formula I, the pharmaceutically acceptable salt thereof, the solvate thereof, the prodrug thereof, the metabolite thereof or the isotopic compound thereof, or the pharmaceutical composition to a subject. The cancer is, for example, hematological cancer, pancreatic cancer, MYH-associated polyposis, colorectal cancer or lung cancer and the like.

The present disclosure also relates to a method for treating hyperproliferative diseases in mammals, comprising administering a therapeutically effective amount of the compound or the salt thereof, the ester thereof, the prodrug thereof, the solvate thereof, the hydrate thereof or the derivative thereof of the present disclosure to a mammal.

Ras mutations include, but are not limited to, Ras mutations of K-Ras, H-Ras or N-Ras mutations that have been identified in hematological cancers or malignancies (for example, cancers affecting blood, bone marrow and/or lymph nodes). Therefore, certain embodiments relate to administering the disclosed compounds (for example, in the form of pharmaceutical compositions) to patients in need of treatment of hematological cancers or malignancies.

In certain embodiments, the present disclosure relates to a method for treating lung cancer, comprising administering an effective amount of any of the above compounds (or pharmaceutical compositions containing the compounds) to a subject in need thereof.

In the present disclosure, the cancer or malignancy includes, but is not limited to, leukemia and lymphoma. In certain embodiments, the hematological diseases are, for example, acute lymphoblastic leukemia (ALL), acute myeloid leukemia (AML), chronic lymphocytic leukemia (CLL), small lymphocytic lymphoma (SLL), chronic myeloid leukemia (CML), acute monocytic leukemia (AMoL) and/or other leukemias. In certain embodiments, the lymphoma, such as all subtypes of Hodgkin's lymphoma or non-Hodgkin's lymphoma.

In certain embodiments of the present disclosure, the lung cancer is non-small cell lung cancer (NSCLC), such as adenocarcinoma, squamous cell lung cancer or large cell lung cancer. In other embodiments, the lung cancer is small cell lung cancer. Other lung cancers include but are not limited to adenoma, carcinoid and undifferentiated cancer.

In some embodiments of the present disclosure, the cancer, such as acute myeloid leukemia, juvenile cancer, pediatric adrenocortical cancer, AIDS-related cancer (for example, lymphoma and Kaposi's sarcoma), anal cancer, appendiceal cancer, astrocytoma, atypical malformation, basal cell carcinoma, bile duct cancer, bladder cancer, bone cancer, brain stem glioma, brain tumor, breast cancer, bronchial tumor, Burkitt's lymphoma, carcinoid tumor, atypical malformation, embryonal tumor, germ cell tumor, primary lymphoma, cervical cancer, childhood cancer, chordoma, cardiac tumor, chronic lymphocytic leukemia (CLL), chronic myeloid leukemia (CML), chronic myeloproliferative disease, colon cancer, colorectal cancer, craniopharyngioma, cutaneous T-cell lymphoma, extrahepatic ductal carcinoma in situ (DCIS), embryonal tumor, central nervous system cancer, endometrial cancer, ependymoma, esophageal cancer , granulomatous neuroblastoma, Ewing's sarcoma, extracranial germ cell tumor, extragonadal germ cell tumor, eye cancer, bone fibrous histiocytoma, gallbladder cancer, gastric cancer, gastrointestinal carcinoid tumor, gastrointestinal stromal tumor (GIST), germ cell tumors, gestational trophoblastic tumors, hairy cell leukemia, head and neck cancer, heart disease, hepatoma, Hodgkin's lymphoma, hypopharyngeal carcinoma, intraocular melanoma, islet cell tumor, pancreatic neuroendocrine tumor, renal carcinoma, laryngeal carcinoma, lip and oral cancer, hepatocellular carcinoma, lobular carcinoma in situ (LCIS), lung cancer, lymphoma, metastatic squamous carcinoma, occult primary, midline carcinoma, oral cancer, multiple endocrine tumor syndrome, Multiple myeloma/plasmacytoma, fungal disease, mycosis fungoides sarcoidosis, myelodysplastic syndrome, myelodysplastic/myeloproliferative neoplasm, multiple myeloma, Merkel cell carcinoma, malignant mesothelioma, malignant fibrous histiocytoma of bone and osteosarcoma, nasal and paranasal sinus, nasal and sinus neuroblastoma, non-Hodgkin's lymphoma, non-small cell lung cancer (NSCLC), oral cancer, lip and oral cancer, oropharyngeal cancer, ovarian cancer, pancreatic cancer, papillomatosis, paraganglioma, paranasal sinuses and nasal cancer, parathyroid gland cancer, penile cancer, throat cancer, pleuropulmonary blastoma, primary central nervous system (CNS) lymphoma, prostate cancer, rectal cancer, transitional cell carcinoma, retinoblastoma, rhabdomyosarcoma, salivary gland carcinoma, skin carcinoma, stomach carcinoma, small cell lung cancer, small bowel carcinoma, soft tissue sarcoma, T cell lymphoma, testicular carcinoma, laryngeal carcinoma, thymoma and thymic carcinoma, thyroid carcinoma, transitional cell carcinoma of the renal pelvis and ureter, trophoblastic tumor, uncommon childhood cancer, urethral carcinoma, uterine sarcoma, vaginal carcinoma, vulvar carcinoma or viral carcinoma. In some embodiments, the non-cancerous hyperproliferative disease, such as benign hyperplasia of the skin (for example, psoriasis), restenosis or prostate (for example, benign prostatic hypertrophy (BPH).

### Definition of terms

The term "pharmaceutically acceptable" means that the salts, solvents, excipients and the like are generally nontoxic, safe and suitable for patient use. The "patient" is preferably a mammal, more preferably a human.

The term "pharmaceutically acceptable salt" refers to a pharmaceutically acceptable salt as defined herein, and has all the effects of the parent compound. Pharmaceutically acceptable salts can be prepared by adding corresponding acids into suitable organic solvents of organic bases and treating according to conventional methods.

Examples of salt formation include: for alkali addition salts, it is possible to prepare salts of alkali metals (for example, sodium, potassium or lithium) or alkaline earth metals (for example, aluminum, magnesium, calcium, zinc or bismuth) by treating compounds of the present disclosure with appropriate acidic protons in an aqueous medium using alkali metal hydroxides, alkaline earth metal hydroxides, alcohol salts (for example, ethanol salts or methanol salts) or appropriate alkaline organic amines (for example, diethanolamine, choline or glucosamine).

Or, for acid addition salts, salt formed with inorganic acids, such as hydrochloric acid, hydrobromic acid, sulfuric acid, nitric acid, phosphoric acid; salts formed with organic acids, such as acetic acid, benzene sulfonic acid, benzoic acid, camphor sulfonic acid, citric acid, ethyl sulfonic acid, fumaric acid, glucoheptonic acid, glutamic acid, glycolic acid, hydroxynaphthoic acid, 2-hydroxyethylsulfonic acid, lactic acid, maleic acid, malic acid, oxalic acid, pyruvic acid, malonic acid, mandelic acid, methanesulfonic acid, 2-naphthalene sulfonic acid, propionic acid, salicylic acid, succinic acid, tartaric acid, citric acid, cinnamic acid, *p*-toluene sulfonic acid or trimethylacetic acid.

In the present disclosure, the quinazoline compound represented by formula I, the pharmaceutically acceptable salt thereof, the solvate thereof, the prodrug thereof, the metabolite thereof or the isotopic compound thereof, can also be obtained by peripheral modification of the quinazoline compound represented by formula I, the pharmaceutically acceptable salt thereof, the solvate thereof, the prodrug thereof, the metabolite thereof or the isotopic compound thereof, which has already been prepared, using conventional methods in the art.

The term "solvate" refers to a substance formed by combining a compound of the present disclosure with a stoichiometric or non-stoichiometric solvent. Solvent molecules in solvates can exist in the form of ordered or unordered arrangement. The solvents include but are not limited to: water, methanol, ethanol, etc.

The term "prodrug" refers to the compounds obtained by chemical structure modification that are inactive or less active *in vitro* and release active drugs through enzymatic or non-enzymatic transformation *in vivo* to exert efficacy.

The term "metabolite" refers to the intermediate metabolite and the final metabolite in metabolism.

The term " isotopic compound" means that one or more atoms in the compound can exist in its unnatural abundance. Taking hydrogen atom as an example, the form of its unnatural abundance means that about 95% of it is deuterium.

The term "pharmaceutical excipients" can be those widely used in the field of pharmaceutical production. Excipients are mainly used to provide a safe, stable and functional pharmaceutical composition and can also provide methods to allow the active ingredient to dissolve at a desired rate after the subject accepts the administration of the composition or to facilitate effective absorption of the active ingredient after the subjects accept the administration of the composition. The pharmaceutical excipients can be inert fillers or provide certain functions, such as stabilizing the overall pH value of the composition or preventing the degradation of active ingredients of the composition. The pharmaceutical excipients may include one or more of the following excipients: adhesive, suspending agents, emulsifier, diluent, filler, granulating agent, adhesive, disintegrant, lubricant, anti-adhesion agent, glidant, wetting agent, gelling agent, absorption delaying agent, dissolution inhibitor, enhancer, adsorbent, buffer, chelating agent, preservative, coloring agent, flavoring agent and sweetener.

The pharmaceutical compositions of the present disclosure may be prepared according to the disclosure using any method known to those skilled in the art. For example, conventional mixing, dissolving, granulating, emulsifying, grinding, encapsulating, embedding or freeze-drying processes.

The pharmaceutical compositions of the present disclosure can be administered in any form, including injection (intravenous), mucous membrane, oral (solid and liquid preparations), inhalation, eye, rectum, local or extra-gastrointestinal (infusion, injection, implantation, subcutaneous, intravenous, intra-arterial, intramuscular) administration. The pharmaceutical composition of the present disclosure can also be a controlled release or delayed release dosage form (for example, liposome or microsphere). Examples of solid oral preparations include but are not limited to powders, capsules, caplets, soft capsules and tablets. Examples of liquid preparations for oral or mucosal administration include, but are not limited to, suspensions, emulsions, elixirs and solutions. Examples of topical preparations include but are not limited to emulsions, gels, ointments, creams, patches, pastes, foams, lotions, drops or serum preparations. Examples of preparations for parenteral administration include, but are not limited to, solutions for injection, dry preparations that can be dissolved or suspended in pharmaceutically acceptable carriers, suspensions for injection and emulsions for injection. Examples of other suitable preparations of the pharmaceutical composition include, but are not limited to, eye drops and other ophthalmic preparations; aerosol: such as nasal spray or inhalant; liquid dosage forms suitable for parenteral administration; and suppositories and lozenges.

"Treatment" means any treatment of diseases in mammals, including: (1) preventing diseases, that is, causing clinical disease symptoms not to develop; (2) inhibiting diseases, that is, preventing the development of clinical symptoms; (3) relieve the disease, that is, causing the clinical symptoms to subside.

An "effective amount" means an amount of a compound, when administered to a patient in need of treatment, that is sufficient to (i) treat the disease in question, (ii) attenuate, ameliorate or eliminate one or more symptoms of a particular disease or condition, or (iii) delay the onset of one or more symptoms of a particular disease or condition as described herein. The amount of the carbonyl heterocyclic compound represented by formula II or pharmaceutically acceptable salt thereof, or pharmaceutical composition, corresponding to such amount will vary depending on, for example, the particular compound, the disease condition and its severity, the characteristics of the patient to be treated (for example, body weight) and the like, but nevertheless can be routinely determined by those of skill in the art.

The term "prevention" in the present disclosure refers to the reduction of the risk of acquiring or developing diseases or disorders.

The term "alkyl" refers to a linear or branched alkyl with a specified number of carbon atoms. Examples of alkyl include methyl, ethyl, *n*-propyl, isopropyl, *n*-butyl, *tert*-butyl, isobutyl, *sec*-butyl, *n*-pentyl, *n*-hexyl, *n*-heptyl, *n*-octyl and similar alkyl. Unless a substituent is specified, the alkyl group is unsubstituted.

The term "cycloalkyl" means a stable 3- to 16-membered saturated cyclic group consisting of 2 to 11 carbon atoms. Unless otherwise specified in this specification, cycloalkyl groups can be monocyclic ("monocyclic heterocycloalkyl") or bicyclic, tricyclic or more cyclic ring systems, which can include fused, bridged or spiro ring systems (such as bicyclic systems ("bicyclic heterocycloalkyl")). The ring system of bicyclic cycloalkyl rings may include one or more heteroatoms in one or both rings; and the ring system is saturated. Unless a substituent is specified, the cycloalkyl is unsubstituted.

The term "heterocycloalkyl" means a stable 3- to 16-membered saturated cyclic group consisting of 2 to 11 carbon atoms with 1 to 5 heteroatoms selected from nitrogen, oxygen and sulfur. Unless otherwise specified in this specification, heterocycloalkyl groups can be monocyclic ("monocyclic heterocycloalkyl") or bicyclic, tricyclic or more cyclic ring systems, which can include fused, bridged or spiro ring systems (such as bicyclic systems ("bicyclic heterocycloalkyl")). The ring system of bicyclic heterocycloalkyl rings may include one or more heteroatoms in one or both rings; and the ring system is saturated. Unless a substituent is specified, the heterocycloalkyl is unsubstituted.

The term "aryl" refers to phenyl or naphthyl.

The term "heteroaryl" refers to an aromatic group containing heteroatoms, preferably containing 1, 2 or 3 aromatic 5- to 6-membered monocyclic or 9- to 10-membered bicyclic rings independently selected from nitrogen, oxygen and sulfur, such as furyl, pyridyl, pyridazinyl, pyrimidinyl, pyrazinyl, thienyl, isoxazole, oxazolyl, diazolyl, imidazolyl, pyrrolyl, triazolyl, tetrazolyl, thiazolyl, isothiazolyl, thiadiazolyl, benzimidazolyl, indole, indazole, benzothiazolyl, benzoisothiazolyl, benzoxazolyl, benzoisoazolyl, quinolyl, isoquinolyl and the like.

On the basis of conforming to the common sense in the field, the above preferred conditions can be arbitrarily combined to obtain the preferred embodiments of the present disclosure.

The reagents and raw materials used in the present disclosure are commercially available.

The positive progressive effect of the present disclosure is that the quinazoline compound provided by the present disclosure has a good inhibitory effect on KRAS G12D mutant protein.

### DETAILED DESCRIPTION OF THE PREFERRED EMBODIMENT

The present disclosure is further described below by way of embodiments, but the present disclosure is not limited to the scope of the described embodiments. Experimental methods for which specific conditions are not indicated in the following embodiment are selected according to conventional methods and conditions, or according to the product instructions.

### Embodiment 1

### 1.1 (Synthesis method I)

### (R or S)-4-(4-((1R,4R)-2,5-diazabicyclo[2.2.2]octan-2-yl)-6-chloro-2-(3-(dimethylamino)azetidin-1-yl)-8-fluoroquinazolin-7-yl)naphthalen-2-ol 1a; (S or R)-4-(4-((1R,4R) -2,5-diazabicyclo[2.2.2]octan-2-yl)-6-chloro-2-(3-(dimethylamino)azetidin-1-yl)-8-fluoroquinazolin-7-yl)naphthalen-2-ol 1b

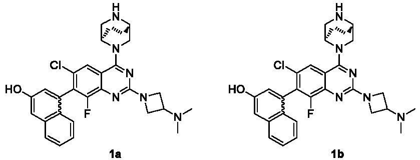

The synthetic route was as follows:

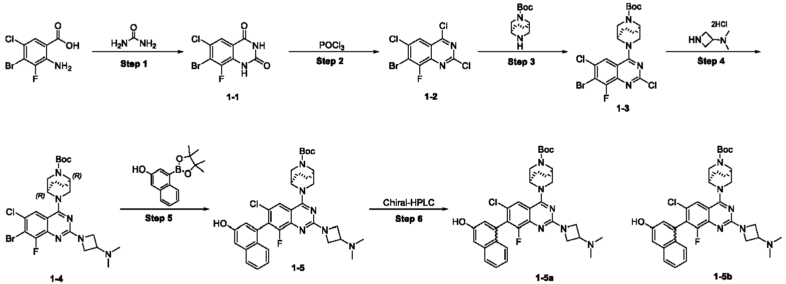

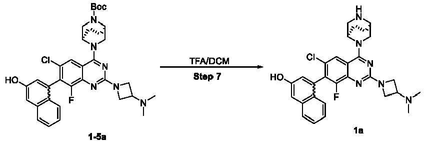

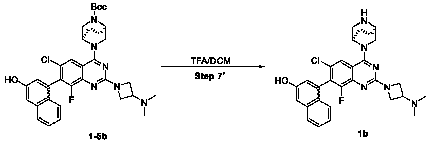

### Step 1

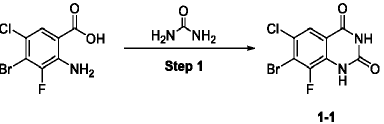

2-Amino-4-bromo-5-chloro-3-fluorobenzoic acid (15.0 g, 55.9 mmol, 1.0 eq) and urea (33.6 g, 558.7 mmol, 10.0 eq) were added to a 500 mL round bottom flask with stirring at 25 °C. The mixture was heated to 150 °C and stirred at this temperature for 6 hours. After the reaction was completed, the temperature was lowered to 25 °C, the mixture was diluted with 750 mL of water, stirred for 30 min, filtered, and the filter cake was washed with water (50 mL × 3), then the solid was collected and dried under reduced pressure to obtain a crude product of compound **1-1** (yellow solid, 15.5 g, purity 56%), this compound was directly used in the next synthesis without further purification. MS (ESI, *m*/*z*): 290.9/292.9/294.8 [M - H]⁻; ¹H NMR (400 MHz, DMSO-*d₆*) δ 7.79 (d, *J* = 1.8 Hz, 1H), 6.86 (s, 1H), 5.41 (s, 1H).

### Step 2

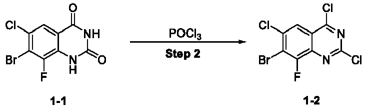

Compound **1-1** (5.0 g, purity 56 %, 9.5 mmol, 1.0 eq), *N*,*N-*diisopropylethylamine (7.5 mL, 40.9 mmol, 4.3 eq) and phosphorus oxychloride (75.0 mL) were successively added to a reaction flask with stirring and under the protection of nitrogen at 25 °C. The obtained mixture was stirred and the reaction was carried out at 90 °C for 5 hours. The reaction process was monitored by liquid chromatography-mass spectrometry and thin layer chromatography. After the reaction was completed, the reaction mixture was cooled to room temperature, and concentrated under reduced pressure to remove the excess reagent. Then 200 mL of water was added to the obtained crude product and the mixture was extracted with ethyl acetate (200 mL × 3). The organic phases were combined and washed with 300 mL of saturated brine, dried over anhydrous sodium sulfate, filtered to remove the drying agent, and concentrated under reduced pressure to obtain a crude product. The crude product was purified by silica gel column chromatography, eluted with a gradient of 0% → 12% ethyl acetate/petroleum ether mobile phase, and the obtained fractions were evaporated under reduced pressure to obtain compound 1-2 (yellow solid, 2.2 g, yield: 70%). MS (ESI, m/z): 328.8/330.8/332.8 [M + H]⁺; ¹H NMR (400 MHz, CDCl₃) *δ* 8.21 (d, *J* = 2.0 Hz, 1H).

### Step 3

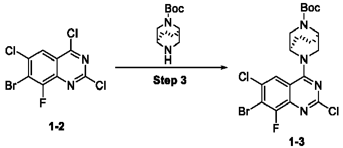

A solution of *tert*-butyl (*1R*,*4R*)-2,5-diazabicyclo[2.2.2]octane-2-carboxylate (257.0 mg, 1.2 mmol, 1.0 eq) in super-dry 1,4-dioxane (1.0 mL) was added dropwise to a mixture of compound **1-2** (400.0 mg, 1.2 mmol, 1.0 eq), super-dry 1,4-dioxane (4.0 mL) and triethylamine (367.6 mg, 3.6 mmol, 3 eq) at 0 °C with stirring and under the protection of nitrogen. The reaction was carried out at 25 °C for 16 hours, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 10% → 36% ethyl acetate/petroleum ether mobile phase, the obtained fraction was evaporated under reduced pressure to obtain compound **1-3** (white solid, 550 mg, yield: 90%). MS (ESI, *m*/*z*): 505.2/507.2/509.2 [M + H]⁺; ¹H NMR (400 MHz, CDCl₃) *δ* 7.94 (s, 1H), 5.22 - 5.15 (m, 1H), 4.54 - 4.50 (m, 1H), 4.33 - 4.26 (m, 1H), 4.06 - 3.97 (m, 1H), 3.83 - 3.78 (m, 1H), 3.68 - 3.57 (m, 1H), 2.36 - 2.10 (m, 2H), 2.02 - 1.82 (m, 2H), 1.50 (s, 9H).

### Step 4

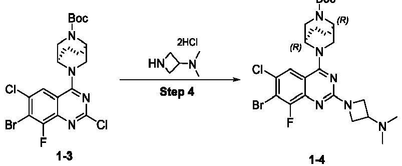

*N,N*-diisopropylethylamine (1.9 mL, 10.9 mmol, 10 eq) and 3-(dimethylamino)azetidine dihydrochloride (282.1 mg, 1.6 mmol, 1.5 eq) were added to a solution of compound **1-3** (550 mg, 1.1 mmol, 1 eq) in N-methylpyrrolidone (5.0 mL) with stirring at room temperature. The obtained mixture was stirred for 2 hours at 60 °C, and the reaction process was monitored by liquid chromatography-mass spectrometry and thin layer chromatography. After the reaction was completed, the reaction mixture was cooled to 25 °C. The reaction mixture was directly purified by reversed-phase rapid chromatographic column (C18 column), and eluted with 50% → 95% acetonitrile/water mobile phase (0.1% ammonium bicarbonate) in 20 min; detector, UV254 nm; to obtain compound **1-4** (light yellow solid, 480 mg, yield: 78%). MS (ESI, m/z): 569.2/571.2/573.2 [M + H]⁺; ¹H NMR (300 MHz, DMSO-*d₆*) *δ* 7.92 (s, 1H), 4.84 (s, 1H), 4.21 - 4.16 (m, 2H), 4.10 - 4.05 (m, 2H), 3.98 - 3.95 (m, 1H), 3.86 - 3.82 (m, 2H), 3.70 - 3.46 (m, 2H), 3.15 - 3.07 (m, 1H), 2.19 - 2.12 (m, 7H), 1.88 -1.75 (m, 3H), 1.42 (s, 9H).

### Step 5

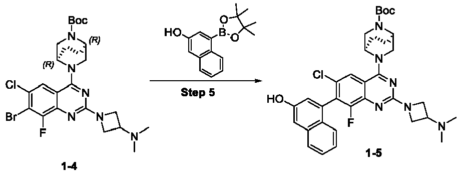

4-(4,4,5,5-Tetramethyl-1,3,2-dioxaborolan-2-yl)naphthalen-2-ol (213.3 mg, 0.8 mmol, 1.5 eq), potassium phosphate (223.4 mg, 1.0 mmol, 2 eq) and chloro(2-dicyclohexylphosphino-2',4',6'-triisopropyl-1,1'-biphenyl)[2-(2'-amino-1,1'-biphenyl)]palladium(II) (41.4 mg, 0.05 mmol, 0.1 eq) were added to a solution of compound **1-4** (300.0 mg, 0.5 mmol, 1 eq) in tetrahydrofuran/water (10/1, 4 mL) at 25 °C with stirring and under the protection of nitrogen. The reaction solution was stirred at 60 °C for 1.5 hours, and the reaction process was monitored by liquid chromatography-mass spectrometry and thin layer chromatography. After the reaction was completed, the reaction mixture was cooled to 25 °C. The reaction solution was concentrated and purified by reversed-phase rapid chromatographic column (C18 column), and eluted with 50% → 95% acetonitrile/water mobile phase (0.1% ammonium bicarbonate) in 20 min; detector, UV254 nm; to obtain compound **1-5** (a mixture of two stereoisomers, white solid, 240 mg, yield: 72%). MS (ESI, m/z): 633.4/635.4 [M + H]⁺.

### Step 6

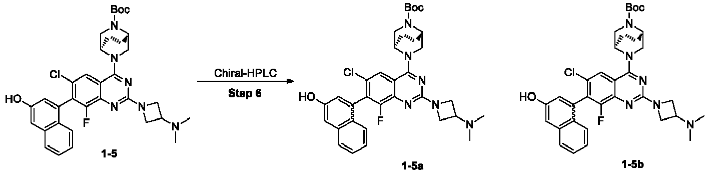

Compound **1-5** (240 mg) obtained in step 5 was subjected to chiral resolution by preparative chiral high-pressure liquid chromatography, chiral column CHIRALPAK IC, 2 × 25 cm, 5 µm; mobile phase A: *n*-hexane (10 mmol/L ammonia), mobile phase B: isopropanol; flow rate: 20 mL/min; elution with 50% phase B in 23.25 min, detector UV 220/210 nm. Two products were obtained, the product with shorter retention time (8.87 min) was compound **1-5a,** *tert*-butyl (1*R*,4*R*)-5-((*R* or *S*)-6-chloro-2-(3-(dimethylamino)azelaic acid-1-yl)-8-fluoro-7-(3-hydroxynaphthalen-1-yl)quinazolin-4-yl)-2,5-diazabicyclo[2.2.2]octane-2-carboxylate (off-white solid, 90 mg, recovery rate: 38%); the product with longer retention time (13.91 min) was compound **1-5b**, *tert*-butyl (1*R*,4*R*)-5-((*S* or *R*)-6-chloro-2-(3-(dimethylamino)azelaic acid-1-yl)-8-fluoro-7-(3-hydroxynaphthalen-1-yl)quinazolin-4-yl)-2,5-diazabicyclo[2.2.2]octane-2-carboxylate (off-white solid, 80 mg, recovery rate: 33%).

Compound **1-5a**: MS (ESI, *m*/*z*): 633.4/635.4 [M + H]⁺; ¹H NMR (300 MHz, DMSO-*d₆*) *δ* 9.99 (s, 1H), 7.96 (s, 1H), 7.80 (d, *J* = 8.3 Hz, 1H), 7.46 - 7.41 (m, 1H), 7.27 (d, *J* = 2.4 Hz, 1H), 7.22 - 7.20 (m, 2H), 7.11 (d, *J* = 2.4 Hz, 1H), 4.91 (s, 1H), 4.28 - 4.19 (m, 2H), 4.15 - 4.12 (m, 3H), 3.90 - 3.83 (m, 2H), 3.72 - 3.63 (m, 1H), 3.56 - 3.49 (m, 1H), 3.32 - 3.30 (m, 1H), 2.24 - 2.10 (s, 7H), 1.91 - 1.80 (m, 3H), 1.43 (s, 9H).

Compound **1-5b**: MS (ESI, m/z): 633.4/635.4 [M + H]⁺; ¹H NMR (300 MHz, DMSO-*d₆*) *δ* 10.06 (s, 1H), 8.04 (s, 1H), 7.87 (d, *J* = 8.3 Hz, 1H), 7.54 - 7.48 (m, 1H), 7.34 - 7.28 (m, 3H), 7.11 (d, *J* = 2.4 Hz, 1H), 4.99 (s, 1H), 4.36 - 4.27 (m, 2H), 4.21 - 4.13 (m, 3H), 4.03 - 3.93 (m, 2H), 3.81 - 3.72 (m, 1H), 3.64 - 3.58 (m, 1H), 3.39 - 3.37 (m, 1H), 2.35 - 2.23 (s, 7H), 1.98 - 1.84 (m, 3H), 1.50 (s, 9H).

The chiral resolution methods of some chiral compounds, the retention times thereof and the ee values thereof in the present disclosure are shown in the following table 1 respectively.

**Table 1**

| Nu mbe r of the com pou nd | Compound structure | Compound name | Mas s spec trum [M+ H]⁺ | Chiral resolution conditions/retention time/ee value |
|---|---|---|---|---|
| **2-5a** | 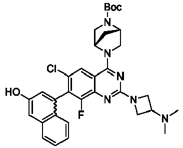 | *tert*-Butyl (1*S*,4*S*)-5-((*R* or *S*)-6-chloro-2-(3-(dimethylamino)azetidi n-1-yl)-8-fluoro-7-(3-hydroxynaphthalen-1- yl)quinazolin-4-yl)-2,5-diazabicyclo[2.2.1]hept ane-2-carboxylate | 619. 3/62 1.3 | Chiral column: NB-Lux 5 µm i-Cellulose-5, 2.12 x 25 cm, 5 µm; mobile phase A: n-hexane/dichloromethane = 5/1 (0.1% diethylamine), mobile phase B: isopropanol; flow rate: 20 mL/min; gradient: elution with 30% phase B in 16 min, detector UV 220/254 nm, retention time: 9.093 min; ee > 99%. |
| **2-5b** | 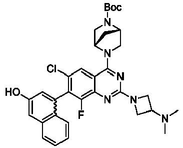 | *tert-Butyl* (1*S*,4*S*)-5-((*S* or *R*)-6-chloro-2-(3-(dimethylamino)azetidin-1-yl)-8-fluoro-7-(3-hydroxynaphthalen-1- yl)quinazolin-4-yl)-2,5-diazabicyclo[2.2.1]hept ane-2-carboxylate | 619. 3/62 1.3 | Chiral column: NB-Lux 5 µm i-Cellulose-5, 2.12 x 25 cm, 5 µm; mobile phase A: *n*-hexane/dichloromethane = 5/1 (0.1% diethylamine), mobile phase B: isopropanol; flow rate: 20 mL/min; gradient: elution with 30% phase B in 16 min, detector UV 220/254 nm, retention time: 11.9225 min; ee: 98%. |
| **3-5a** | 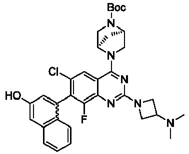 | *tert*-Butyl (1*R*,4*R*)-5-((*R* or *S*)-6-chloro-2-(3-(dimethylamino)azetidi n-1-yl)-8-fluoro-7-(3-hydroxynaphthalen-1- yl)quinazolin-4-yl)-2,5-diazabicyclo[2.2.1]hept ane-2-carboxylate | 619. 2/62 1.2 | Chiral column: NB-Lux 5 µm i-Cellulose-5, 2.12 x 25 cm, 5 µm; mobile phase A: *n*-hexane/dichloromethane = 1/1 (0.5% 2 mol/L ammonia methanol solution), mobile phase B: isopropanol; flow rate: 20 mL/min; gradient: elution with 5% phase B in 23 min, detector UV 220/254 nm; retention time: 12.286 min; ee > 99%. |
| **3-5b** | 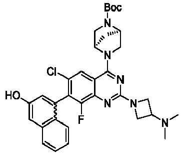 | *tert*-Butyl (1*R*,4*R*)-5-((*S* or *R*)-6-chloro-2-(3-(dimethylamino)azetidi n-1-yl)-8-fluoro-7-(3-hydroxynaphthalen-1- yl)quinazolin-4-yl)-2,5-diazabicyclo[2.2.1]hept ane-2-carboxylate | 619. 2/62 1.2 | Chiral column: NB-Lux 5µm i-Cellulose-5, 2.12 x 25 cm, 5 µm; mobile phase A: *n*-hexane/dichloromethane = 1/1 (0.5% 2 mol/L ammonia methanol solution), mobile phase B: isopropanol; flow rate: 20 mL/min; gradient: elution with 5% phase B in 23 min, detector UV 220/254 nm; retention time: 16.05 min; ee > 99%. |
| **4-5a** | 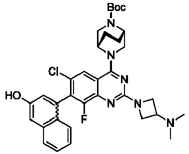 | *tert*-Butyl (1*S*,4*S*)-5-((*R* or *S*)-6-chloro-2-(3-(dimethylamino)azelaic acid-1-yl)-8-fluoro-7-(3-hydroxynaphthalen-1-yl) quinazolin-4-yl)-2,5-diazabicyclo[2.2.2]octa ne-2-carboxylate | 633. 3/63 5.3 | Chiral column: NB-Lux 5 µm i-Cellulose-5, 2.12 x 25 cm, 5 µm; mobile phase A: n-hexane (10 mmol/L ammonia methanol solution), mobile phase B: isopropanol; flow rate: 20 mL/min; gradient: elution with 50% phase B in 38 min; detector UV 220/210 nm; retention time: 21.325 min; ee > 99%. |
| **4-5b** | 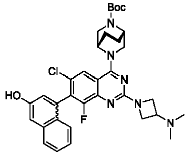 | *tert*-Butyl (1*S*,4*S*)-5-((*S* or *R*)-6-chloro-2-(3-(dimethylamino)azelaic acid-1-yl)-8-fluoro-7-(3-hydroxynaphthalen-1-yl)quinazolin-4-yl)-2,5-diazabicyclo[2.2.2]octa ne-2-carboxylate | 633. 3/63 5.3 | Chiral column: NB-Lux 5 µm i-Cellulose-5, 2.12 x 25 cm, 5 µm; mobile phase A: *n*-hexane (10 mmol/L ammonia methanol solution), mobile phase B: isopropanol; flow rate: 20 mL/min; gradient: elution with 50% phase B in 38 min; detector UV 220/210 nm; retention time: 30.240 min; ee > 99%. |
| **5-5a** | 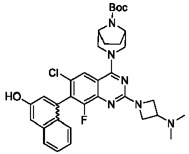 | *tert*-Butyl (1*R*,5*S*)-3-((*R* or S)-6-chloro-2-(3-(dimethylamino)azelaic acid-1-yl)-8-fluoro-7-(3-hydroxynaphthalen-1-yl)quinazolin-4-yl)-3,8-diazabicyclo[3.2.1]octa ne-8-carboxylate | 633. 4/63 5.4 | Chiral column: Lux 5 µm Cellulose-2, 2.12 x 25 cm, 5 µm; mobile phase A: *n-*hexane (10 mmol/L ammonia methanol solution), mobile phase B: isopropanol; flow rate: 15 mL/min; gradient: elution with 50% phase B in 18 min; detector UV 220/254 nm; retention time: 6.586 min; ee > 99%. |
| **5-5b** | 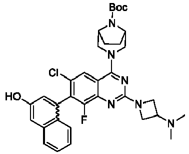 | *tert*-Butyl (1*R*,5*S*)-3-((*S* or *R*)-6-chloro-2-(3-(dimethylamino)azelaic acid-1-yl)-8-fluoro-7-(3-hydroxynaphthalen-1-yl)quinazolin-4-yl)-3,8-diazabicyclo[3.2.1]octa ne-8-carboxylate | 633. 4/63 5.4 | Chiral column: Lux 5 µm Cellulose-2, 2.12 x 25 cm, 5 µm; mobile phase A: n-hexane (10 mmol/L ammonia methanol solution), mobile phase B: isopropanol; flow rate: 15 mL/min; gradient: elution with 50% phase B in 18 min; detector UV 220/254 nm; retention time: 12.225 min; ee > 99%. |
| **6-5a** | 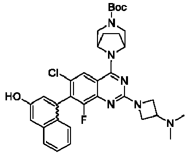 | *tert-*Butyl (1*R*,5*S*)-8-((*R* or *S*)-6-chloro-2-(3-(dimethylamino)azelaic acid-1-yl)-8-fluoro-7-(3-hydroxynaphthalen-1-yl)quinazolin-4-yl)-3,8-diazabicyclo[3.2.1]octa ne-3-carboxylate | 633. 3/63 5.2 | Chiral column: CHIRAL ART Cellulose-SB, 2 x 25 cm, 5 µm; mobile phase A: *n*-hexane/dichloromethane = 5/1 (0.1% diethylamine), mobile phase B: isopropanol; flow rate: 20 mL/min; gradient: elution with 30% phase B in 12 min; detector UV 220/254 nm; retention time: 3.356 min; ee > 99%. |
| **6-5b** | 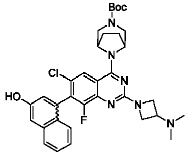 | *tert*-Butyl (1*R*,5*S*)-8-((*S* or *R*)-6-chloro-2-(3-(dimethylamino)azelaic acid-1-yl)-8-fluoro-7-(3-hydroxynaphthalen-1-yl)quinazolin-4-yl)-3,8-diazabicyclo[3.2.1]octa ne-3-carboxylate | 633. 3/63 5.2 | Chiral column: CHIRAL ART Cellulose-SB, 2 x 25 cm, 5 µm; mobile phase A: *n*-hexane/dichloromethane = 5/1 (0.1% diethylamine), mobile phase B: isopropanol; flow rate: 20 mL/min; gradient: elution with 30% phase B in 12 min; detector UV 220/254 nm; retention time: 7.99 min; ee > 99%. |
| **7-5a** | 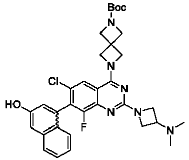 | *tert*-Butyl (*R* or *S*)-6-(6-chloro-2-(3-(dimethylamino)azetidi n-1-yl)-8-fluoro-7-(3-hydroxynaphthalen-1- yl)quinazolin-4-yl)-2,6-diazaspiro[3.3]heptane-2-carboxylate | 619. 3/62 1.3 | Chiral column: Lux 5 µm Amylose-1, 2.12 x 25 cm, 5 µm; mobile phase A: *n-*hexane (10 mmol/L ammonia methanol solution), mobile phase B: ethanol; flow rate: 15 mL/min: gradient: elution with 50% phase B in 16 min; detector UV 227/254 nm; retention time: 8.1325 min; ee > 99%. |
| **7-5b** | 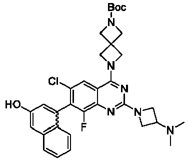 | *tert*-Butyl (*S* or *R*)-6-(6-chloro-2-(3-(dimethylamino)azetidi n-1-yl)-8-fluoro-7-(3-hydroxynaphthalen-1 - yl)quinazolin-4-yl)-2,6-diazaspiro[3.3]heptane-2-carboxylate | 619. 3/62 1.3 | Chiral column: Lux 5 µm Amylose-1, 2.12 x 25 cm, 5 µm; mobile phase A: *n-*hexane (10 mmol/L ammonia methanol solution), mobile phase B: ethanol; flow rate: 15 mL/min: gradient: elution with 50% phase B in 16 min; detector UV 227/254 nm; retention time: 12.166 min; ee > 99%. |
| **8-5a** | 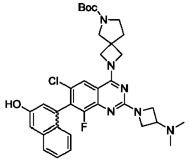 | *tert*-Butyl (*R* or *S*)-2-(6-chloro-2-(3-(dimethylamino)azelaic acid-1-yl)-8-fluoro-7-(3-hydroxynaphthalen-1-yl)quinazolin-4-yl)-2,6-diazaspiro[3.4]octane-6-carboxylate | 633. 3/63 5.3 | Chiral column: CHIRALPAK IC, 2 x 25 cm, 5 µm; mobile phase A: *n-*hexane (10 mmol/L ammonia methanol solution), mobile phase B: ethanol; flow rate: 20 mL/min; gradient: elution with 30% phase B in 28 min; detector UV 220/210 nm; retention time: 15.965 min; ee > 99%. |
| **8-5b** | 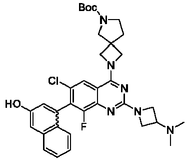 | *tert*-Butyl (*S* or *R*)-2-(6-chloro-2-(3-(dimethylamino)azelaic acid-1-yl)-8-fluoro-7-(3-hydroxynaphthalen-1-yl)quinazolin-4-yl)-2,6-diazaspiro[3.4]octane-6-carboxylate | 633. 3/63 5.3 | Chiral column: CHIRALPAK IC, 2 x 25 cm, 5 µm; mobile phase A: *n-*hexane (10 mmol/L ammonia methanol solution), mobile phase B: ethanol; flow rate: 20 mL/min; gradient: elution with 30% phase B in 28 min; detector UV 220/210 nm; retention time: 21.8 min; ee > 99%. |
| **9a** | 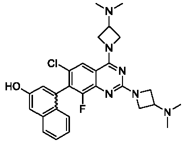 | (*R* or *S*)-4-(6-chloro-2,4-bis(3-(dimethylamino)azetidi n-1-yl)-8-fluoro-7-yl)naphthalen-2-ol | 521. 2/52 3.2 | Chiral column: NB-Lux 5 µm i-Cellulose-5, 2.12 x 25 cm, 5 µm; mobile phase A: *n*-hexane/dichloromethane = 5/1 (0.1% diethylamine), mobile phase B: isopropanol; flow rate: 20 mL/min; gradient: elution with 30% phase B in 17 min; detector UV 226/295 nm; retention time: 7.115 min; ee > 99%. |
| **9b** | 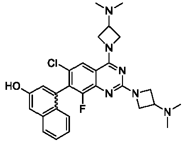 | (*S* or *R*)-4-(6-chloro-2,4-bis(3-(dimethylamino)azetidi n-1-yl)-8-fluoro-7-yl)naphthalen-2-ol | 521. 2/52 3.2 | Chiral column: NB-Lux 5 µm i-Cellulose-5, 2.12 x 25 cm, 5 µm; mobile phase A: *n*-hexane/dichloromethane = 5/1 (0.1% diethylamine), mobile phase B: isopropanol; flow rate: 20 mL/min; gradient: elution with 30% phase B in 17 min; detector UV 226/295 nm; retention time: 11.15 min; ee > 99%. |
| **10-5a** | 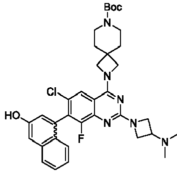 | *tert*-Butyl (*R* or *S*)-2-(6-chloro-2-(3-(dimethylamino)azelaic acid-1-yl)-8-fluoro-7-(3-hydroxynaphthalen-1-yl)quinazolin-4-yl)-2,7-diazaspiro[3.5]nonane-7-carboxylate | 647. 4/64 9.4 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 22 min; detector UV 227/220 nm; retention time: 14.005 min; ee value: 95.03%. |
| **10-5b** | 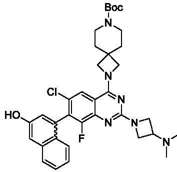 | *tert*-Butyl (*S* or *R*)-2-(6-chloro-2-(3-(dimethylamino)azelaic acid-1-yl)-8-fluoro-7-(3-hydroxynaphthalen-1-yl)quinazolin-4-yl)-2,7-diazaspiro[3.5]nonane-7-carboxylate | 647. 4/64 9.4 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 22 min; detector UV 227/220 nm; retention time: 18.9 min. ee > 99%. |
| **11-5a** | 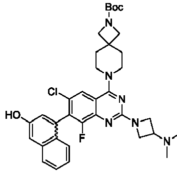 | *tert*-Butyl (*R* or *S*)-7-(6-chloro-2-(3-(dimethylamino)azelaic acid-1-yl)-8-fluoro-7-(3-hydroxynaphthalen-1-yl)quinazolin-4-yl)-2,7-diazaspiro[3.5]nonane-2-carboxylate | 647. 4/64 9.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 21 min; detector UV 227/210 nm; retention time: 11.3 min; ee > 99%. |
| **11-5b** | 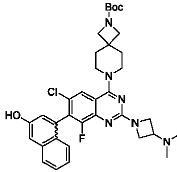 | *tert*-Butyl (*S* or *R*)-7-(6-chloro-2-(3-(dimethylamino)azelaic acid-1-yl)-8-fluoro-7-(3-hydroxynaphthalen-1-yl)quinazolin-4-yl)-2,7-diazaspiro[3.5]nonane-2-carboxylate | 647. 4/64 9.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 21 min; detector UV 227/210 nm; retention time: 15.6 min; ee > 99%. |
| **12-5a** | 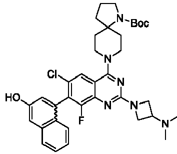 | *tert*-Butyl (*R* or *S*)-8-(6-chloro-2-(3-(dimethylamino)azelaic acid-1-yl)-8-fluoro-7-(3-hydroxynaphthalen-1-yl)quinazolin-4-yl)-1,8-diazaspiro[4.5]decane-1-carboxylate | 661. 2/66 3.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 20 min; detector UV 254/220 nm; retention time: 12.675 min; ee > 99%. |
| **12-5b** | 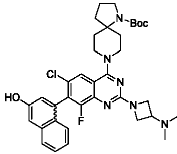 | *tert*-Butyl (*S* or *R*)-8-(6-chloro-2-(3-(dimethylamino)azelaic acid-1-yl)-8-fluoro-7-(3-hydroxynaphthalen-1-yl)quinazolin-4-yl)-1,8-diazaspiro[4.5]decane-1-carboxylate | 661. 2/66 3.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 20 min; detector UV 254/220 nm; retention time: 16.23 min; ee value: 99%. |
| **13-5a** | 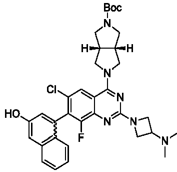 | *tert*-Butyl (3*aR,*6a*S*)-5-((*R* or *S*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-*c*]pyrrole-2(1*H*)-carboxylate | 633. 2/63 5.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 31 min; detector UV 254/227 nm; retention time: 17.8 min; ee > 99%. |
| **13-5b** | 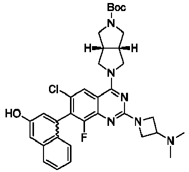 | *tert*-Butyl (3*aR,*6*aS*)-5-((*S* or *R*)-6-chloro-2-(3-(dimethylamino)azelaic acid -1-yl)-8-fluoro-7-(3-hydroxynaphthalen-1-yl)quinazolin-4-yl)hexahydropyrrolo[3, 4-*c*]pyrrole-2(1*H*)-carboxylate | 633. 2/63 5.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 31 min; detector UV 254/227 nm; retention time: 25.5 min; ee > 99%. |
| **14-5Q** | 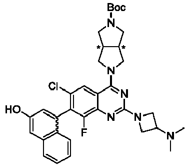 generated by **14-**5 separation | *tert-Butyl* (3*aS,*6*aS* or 3*aR,*6*aR*)-5-((*R* or *S*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-*c*]pyrrole-2(1*H*)-carboxylate | 633. 2/63 5.2 | Preparative column: XBridge Prep C18 OBD column, 19 x 150 mm, 5 µm; mobile phase A: water (10 mmol/L, ammonium bicarbonate), mobile phase B: acetonitrile; flow rate: 25 mL/min; elution gradient: 44% phase B to 63% phase B in 12 min, maintaining 63% phase B for 1 minute; detector: UV 220/254 nm; retention time: 11.37 min. |
| **14-5H** | 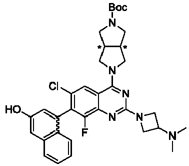 generated by **14-5** separation | *tert*-Butyl (3*aR,*6*aR* or 3*aS,*6*aS*)-5-((*S* or *R*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-*c*]pyrrole-2(1*H*)-carboxylate | 633. 2/63 5.2 | Preparative column: XBridge Prep C18 OBD column, 19 x 150 mm, 5 µm; mobile phase A: water (10 mmol/L, ammonium bicarbonate), mobile phase B: acetonitrile; flow rate: 25 mL/min; elution gradient: 44% phase B to 63% phase B in 12 min, maintaining 63% phase B for 1 minute; detector: UV 220/254 nm; retention time: 12.40 min. |
| **14-5a** | 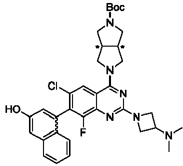 generated by **14-5Q** separation | tert-Butyl (3*aS,*6*aS* or 3*aR,*6*aR*)-5-((*R* or *S*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-*c*]pyrrole-2(1*H*)-carboxylate | 633. 2/63 5.2 | Chiral column: Lux 5 µm Cellulose-2, 2.12 x 25 cm, 5 µm; mobile phase A: n-hexane (10 mmol/L ammonia methanol solution), mobile phase B: ethanol; flow rate: 20 mL/min; gradient: elution with 30% phase B in 30 min; detector UV 230/254 nm; retention time: 16.76 min; ee > 99%. |
| **14-5b** | 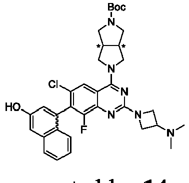 generated by **14-5Q** separation | *tert*-Butyl (3*aS,*6*aS* or 3*aR,*6*aR*)-5-((*S* or *R*)-6-chloro-2-(3-(dimethylamino)azelaic acid-1 -yl)-8-fluoro-7-(3-hydroxynaphthalen-1-yl)quinazolin-4-yl)hexahydropyrrolo[3, 4-*c*]pyrrole-2(1*H*)-carboxylate | 633. 3/63 5.2 | Chiral column: Lux 5 µm Cellulose-2, 2.12 x 25 cm, 5 µm; mobile phase A: *n-*hexane (10 mmol/L ammonia methanol solution), mobile phase B: ethanol; flow rate: 20 mL/min; gradient: elution with 30% phase B in 30 min; detector UV 230/254 nm; retention time: 23.5325 min; ee > 99%. |
| **14-5c** | 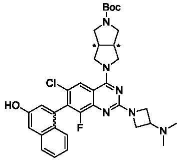 generated by **14-5H** separation | *tert*-Butyl (3*aR,*6*aR* or 3*aS,*6*aS*)-5-((*R* or *S*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-*c*]pyrrole-2(1*H*)-carboxylate | 633. 3/63 5.2 | Chiral column: Lux 5 µm Cellulose-4, 2.12 x 25 cm, 5 µm; mobile phase A: n-hexane (10 mmol/L ammonia methanol solution), mobile phase B: ethanol; flow rate: 20 mL/min; gradient: elution with 30% phase B in 32 min, detector UV 230/254 nm; retention time: 15.915 min; ee > 99%. |
| **14-5d** | 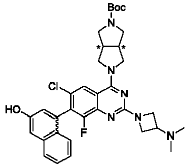 generated by **14-5H** separation | *tert*-Butyl (3*aR,*6*aR* or 3*aS,*6*aS*)-5-((*S* or *R*)-6-chloro-2-(3-(dimethylamino)azelaic acid-1-yl)-8-fluoro-7-(3-hydroxynaphthalen-1-yl)quinazolin-4-yl)hexahydropyrrolo[3, 4-c]pyrrole-2(1*H*)-carboxylate | 633. 3/63 5.2 | Chiral column: Lux 5 µm Cellulose-4, 2.12 x 25 cm, 5 µm; mobile phase A: *n-*hexane (10 mmol/L ammonia methanol solution), mobile phase B: ethanol; flow rate: 20 mL/min; gradient: elution with 30% phase B in 32 min, detector UV 230/254 nm; retention time: 24.7175 min; ee > 99%. |
| **15-5a** | 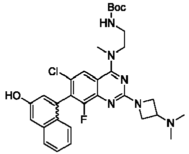 | *tert*-Butyl (*R* or *S*)-(2-((6-chloro-2-(3-(dimethylamino)azelaic acid-1-yl)-8-fluoro-7-(3-hydroxynaphthalen-1-yl)quinazolin-4-yl)(methyl)amino)ethyl) carbamate | 595. 3/59 7.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 16 min; detector UV 220/210 nm; retention time: 10.2725 min; ee > 99%. |
| 15-5b | 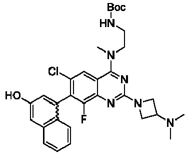 | *tert-*Butyl (*S* or R)-(2-((6-chloro-2-(3-(dimethylamino)azelaic acid-1-yl)-8-fluoro-7-(3-hydroxynaphthalen-1-yl)quinazolin-4-yl)(methyl)amino)ethyl) carbamate | 595. 3/59 7.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 16 min; detector UV 220/210 nm; retention time: 13.235 min; ee: 99%. |
| **16-5a** | 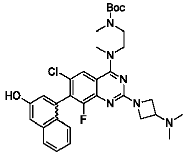 | *tert*-Butyl (*R* or *S*)-(2-((6-chloro-2-(3-(dimethylamino)azetidi n-1-yl)-8-fluoro-7-(3-hydroxynaphthalen-1- yl)quinazolin-4-yl)(methyl)amino)ethyl) (methyl)carbamate | 609. 3/61 1.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 30 min; detector UV 220/210 nm; retention time: 15 min; ee > 99%. |
| **16-5b** | 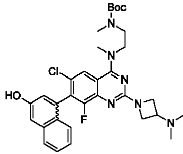 | *tert*-Butyl (*S* or *R*)-(2-((6-chloro-2-(3-(dimethylamino)azetidi n-1-yl)-8-fluoro-7-(3-hydroxynaphthalen-1- yl)quinazolin-4-yl)(methyl)amino)ethyl) (methyl)carbamate | 609. 3/61 1.3 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 20% phase B in 30 min; detector UV 220/210 nm; retention time: 19.3 min; ee > 99%. |
| **18-5a** | 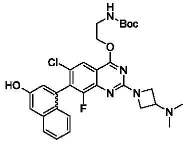 | *tert*-Butyl (*R* or *S*)-(2-((6-chloro-2-(3-(dimethylamino)azetidi n-1-yl)-8-fluoro-7-(3-hydroxynaphthalen-1-yl)quinazolin-4-yl)oxy)ethyl)carbamate | 582. 2/58 4.2 | Chiral column: CHIRALPAK ID, 2 x 25 cm, 5 µm; mobile phase A: *n-*hexane (10 mmol/L ammonia methanol solution), mobile phase B: isopropanol; flow rate: 20 mL/min; gradient: elution with 30% phase B in 26.5 min; detector UV 220/210 nm; retention time: 7.65 min; ee > 99%. |
| **18-5b** | 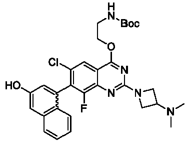 | *tert*-Butyl (*S* or *R*)-(2-((6-chloro-2-(3-(dimethylamino)azetidi n-1-yl)-8-fluoro-7-(3-hydroxynaphthalen-1- yl)quinazolin-4-yl)oxy)ethyl)carbamate | 582. 2/58 4.2 | Chiral column: CHIRALPAK ID, 2 x 25 cm, 5 µm; mobile phase A: *n-*hexane (10 mmol/L ammonia methanol solution), mobile phase B: isopropanol; flow rate: 20 mL/min; gradient: elution with 30% phase B in 26.5 min; detector UV 220/210 nm; retention time: 19.36 min; ee > 99%. |
| **19-5a** | 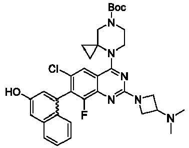 | *tert*-Butyl (*R* or S) 4-(6-chloro-2-(3-(dimethylamino)azetidi n-1-yl)-8-fluoro-7-(3-hydroxynaphthalen-1- yl)quinazolin-4-yl)-4,7-diazaspiro [2.5] octane-7-carboxylate | 633. 3/63 5.3 | Chiral column: NB-Lux 5 µm i-Cellulose-5, 2.12 x 25 cm, 5 µm; mobile phase A: *n*-hexane (10 mmol/L ammonia methanol solution), mobile phase B: isopropanol; flow rate: 17 mL/min; gradient: elution with 50% phase B in 25 min; detector UV 210/254 nm; retention time: 6.0 min; ee > 99%. |
| **19-5b** | 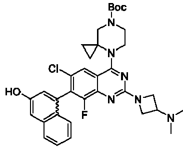 | *tert*-Butyl (*S* or *R*) 4-(6-chloro-2-(3-(dimethylamino)azetidi n-1-yl)-8-fluoro-7-(3-hydroxynaphthalen-1- yl)quinazolin-4-yl)-4,7-diazaspiro [2.5] octane-7-carboxylate | 633. 3/63 5.3 | Chiral column: NB-Lux 5 µm i-Cellulose-5, 2.12 x 25 cm, 5 µm; mobile phase A: *n*-hexane (10 mmol/L ammonia methanol solution), mobile phase B: isopropanol; flow rate: 17 mL/min; gradient: elution with 50% phase B in 25 min; detector UV 210/254 nm; retention time: 17.5 min; ee > 99%. |

### Step 7

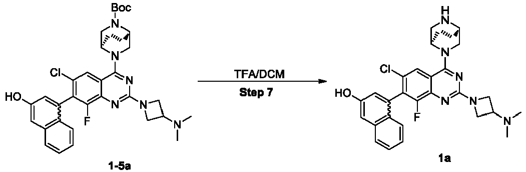

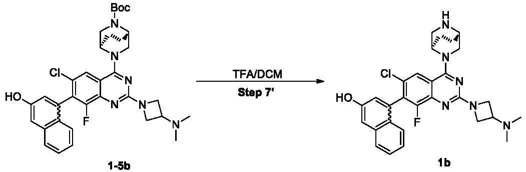

Trifluoroacetic acid (1 mL) was added dropwise to a solution of compound **1-5a** (90 mg, 0.1 mmol, 1.0 eq) in dichloromethane (5 mL) at 25 °C; after the addition, the reaction mixture was stirred at this temperature for 1 hour, and the reaction process was monitored by liquid chromatography-mass spectrometry. After the reaction was completed, the reaction solution was concentrated under reduced pressure to obtain a crude product. The crude product was purified by reversed-phase flash chromatography (C18 column) and eluted with 50% → 95% acetonitrile/water mobile phase (0.1% ammonium bicarbonate) in 15 min; detector, UV254 nm; then **1a** was obtained (white solid, 50 mg, yield: 65%). Compound **1b** (white solid, 45 mg, yield: 67%) can be obtained by the same method as above.

Compound **1a**: MS (ESI, m/z): 533.2/535.2 [M + H]⁺; ¹H NMR (300 MHz, DMSO-*d₆*) *δ* 9.99 (s, 1H), 7.94 (d, *J* = 1.7 Hz, 1H), 7.80 (d, *J* = 8.3 Hz, 1H), 7.46 - 7.41 (m, 1H), 7.27 (d, *J* = 2.4 Hz, 1H), 7.24 - 7.18 (m, 2H), 7.03 (d, *J* = 2.4 Hz, 1H), 4.64 (s, 1H), 4.16 - 4.00 (m, 4H), 3.86 - 3.81 (m, 2H), 3.37 - 3.35 (m, 1H), 3.12 - 3.02 (m, 3H), 2.19 - 2.11 (s, 7H), 1.89 - 1.70 (m, 3H); ¹⁹F NMR (282 MHz, DMSO-*d₆*) *δ* -123.51. The chiral analysis conditions of compound **1a** were: CHIRALPAK IC-3, 4.6 × 50 mm, 3 µm; mobile phase A: *n-*hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.946 min; ee > 99%.

Compound **1b**: MS (ESI, m/z): 533.2/535.2 [M + H]⁺; ¹H NMR (300 MHz, DMSO-*d₆*) *δ* 9.98 (s, 1H), 7.94 (d, *J* = 1.7 Hz, 1H), 7.80 (d, *J* = 8.3 Hz, 1H), 7.46 - 7.41 (m, 1H), 7.27 (d, *J* = 2.4 Hz, 1H), 7.24 - 7.22 (m, 2H), 7.03 (d, *J* = 2.4 Hz, 1H), 4.64 (s, 1H), 4.16 - 4.00 (m, 4H), 3.86 - 3.81 (m, 2H), 3.37 - 3.35 (m, 1H), 3.12 - 3.02 (m, 3H), 2.19 - 2.11 (s, 7H), 1.89 - 1.70 (m, 3H); ¹⁹F NMR (282 MHz, DMSO-*d₆*) *δ* -123.50. The chiral analysis conditions of compound **1b** were: CHIRALPAK IC-3, 4.6 × 50 mm, 3 µm; mobile phase A: *n-*hexane/dichloromethane = 5/1 (0.1% diethylamine), mobile phase B: isopropanol; flow rate: 1 mL/min; isocratic elution with 30% phase B in 6 min; detector UV 220/254 nm; retention time: 5.115 min; ee > 99%.

Other similar compounds of the present disclosure can be prepared by the synthetic method shown in Embodiment 1 above. Some compounds prepared with reference to the above synthetic method and their characterization data are shown in table 2.

**Table 2**

| Number of the compound | Compound structure | Compound name | Chiral analysis conditions/retentio n time/ee value/specific rotation | Mass spectrum [M+ H]+ | ¹H & ¹⁹F NMR |
|---|---|---|---|---|---|
| 2a | 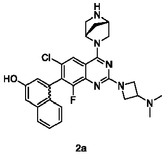 | (*R* or *S*)-4-(4-((1*S*,4*S*)-2,5-diazabicycl o[2.2.1]hept an-2-yl)-6-chloro-2-(3-(dimethyla mino)azetid in-1-yl)-8-fluoroquina zolin-7-yl)naphthale n-2-ol | CHIRALPAK IC-3, 4.6 x 50 mm, 3 µm; mobile phase A: n-hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 2.104 min; ee > 99%. Specific rotation [*a*]_{D}²⁵ = - 132.5 (c = 0.200, methanol) | 519. 2/52 1.2 | ¹H NMR (400 MHz, DMSO-*d₆*) *δ* 9.99 (s, 1H), 7.90 (d, *J* = 1.5 Hz, 1H), 7.79 (d, *J* = 8.3 Hz, 1H), 7.45 - 7.41 (m, 1H), 7.26 (d, *J* = 2.4 Hz, 1H), 7.24 - 7.17 (m, 2H), 7.00 (d, *J* = 2.4 Hz, 1H), 5.07 (s, 1H), 4.18 - 4.15 (m, 1H), 4.12 - 4.00 (m, 2H), 3.86 - 3.80 (m, 2H), 3.74 - 3.66 (m, 2H), 3.13 - 3.06 (m, 2H), 2.98 - 2.95 (m, 1H), 2.11 (s, 6H), 1.84 (d, *J* = 9.5 Hz, 1H), 1.72 (d, *J* = 9.5 Hz, 1H); ¹⁹F NMR (282 MHz, DMSO-*d₆*) *δ* - 123.40. |
| **2b** | 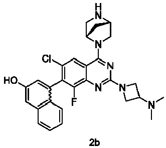 | (*S* or *R*)-4-(4-((1*S*,4*S*)-2,5-diazabicycl o[2.2.1]heptan-2-yl)-6-chloro-2-(3-(dimethyla mino)azetid in-1-yl)-8-fluoroquina zolin-7-yl)naphthale n-2-ol | CHIRALPAK IC-3, 4.6 x 50 mm, 3 µm; mobile phase A: n-hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 3.656 min; ee value: 99%. Specific rotation [a]_{D}²⁵ = -36.297 (c = 0.045, methanol) | 519. 2/52 1.2 | ¹H NMR (400 MHz, DMSO-*d₆*) *δ* 10.00 (s, 1H), 7.91 (d, *J* = 1.6 Hz, 1H), 7.80 (d, *J* = 8.3 Hz, 1H), 7.46 - 7.41 (m, 1H), 7.26 (d, *J* = 2.4 Hz, 1H), 7.23 - 7.18 (m, 2H), 7.04 (d, *J* = 2.4 Hz, 1H), 5.06 (s, 1H), 4.19 - 4.16 (m, 1H), 4.10 - 4.03 (m, 2H), 3.87 - 3.80 (m, 2H), 3.74 - 3.70 (m, 2H), 3.14 - 3.06 (m, 2H), 2.98 - 2.95 (m, 1H), 2.11 (s, 6H), 1.86 (d, *J* = 9.6 Hz, 1H), 1.72 (d, *J* = 9.6 Hz, 1H); ¹⁹F NMR (282 MHz, DMSO-*d₆*) *δ* - 123.50. |
| **3a** | 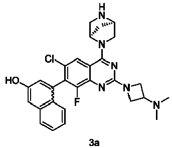 | (*R* or *S*)-4-(4-((1*R*,4*R*)-2,5-diazabicycl o[2.2.1]hept an-2-yl)-6-chloro-2-(3-(dimethyla mino)azetid in-1-yl)-8-fluoroquina zolin-7-yl)naphthalen-2-ol | Chiral column CHIRALPAK IC-3, 4.6 x 50 mm, 3 µm; mobile phase A: n-hexane/dichloromet hane = 5/1 (0.1% diethylamine), mobile phase B: isopropanol; flow rate: 1 mL/min; isocratic elution with 30% phase B in 10 min; detector UV 220/254 nm; retention time: 2.705 min; ee > 99%. | 519. 2/52 1.2 | ¹H NMR (300 MHz, DMSO-*d₆*) *δ* 9.99 (s, 1H), 7.91 (d, *J* = 1.6 Hz, 1H), 7.80 (d, *J* = 8.3 Hz, 1H), 7.46 - 7.41 (m, 1H), 7.26 (d, *J* = 2.5 Hz, 1H), 7.24 - 7.17 (m, 2H), 7.04 (d, *J* = 2.4 Hz, 1H), 5.06 (s, 1H), 4.20 - 4.16 (m, 1H), 4.10 - 4.03 (m, 2H), 3.87 - 3.80 (m, 2H), 3.75 - 3.70 (m, 2H), 3.13 - 3.06 (m, 2H), 2.99 - 2.95 (m, 1H), 2.11 (s, 6H), 1.86 (d, *J* = 9.6 Hz, 1H), 1.73 (d, *J* = 9.6 Hz, 1H); ¹⁹F NMR (282 MHz, DMSO-*d₆*) *δ* - 123.48. |
| **3b** | 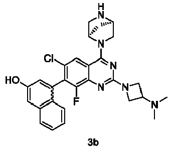 | (*S* or *R*)-4-(4-((1*R*,4*R*)-2,5-diazabicycl o[2.2.1]hept an-2-yl)-6-chloro-2-(3-(dimethyla mino)azetid in-1-yl)-8-fluoroquina zolin-7-yl)naphthale n-2-ol | Chiral column CHIRALPAK IC-3, 4.6 x 50 mm, 3 µm; mobile phase A: n-hexane/dichloromet hane = 5/1 (0.1% diethylamine), mobile phase B: isopropanol; flow rate: 1 mL /min; isocratic elution with 30% phase B in 10 min; detector UV 220/254 nm; retention time: 6.915 min; ee value: 99%. | 519. 2/52 1.2 | ¹H NMR (300 MHz, DMSO-*d₆*) *δ* 9.99 (s, 1H), 7.91 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.25 - 7.16 (m, 2H), 7.01 (d, *J* = 2.4 Hz, 1H), 5.07 (s, 1H), 4.25 - 4.13 (m, 1H), 4.10 - 4.03 (m, 2H), 3.88 - 3.78 (m, 2H), 3.77 - 3.67 (m, 2H), 3.14 - 3.06 (m, 2H), 3.02 - 2.95 (m, 1H), 2.11 (s, 6H), 1.86 (d, *J* = 9.7 Hz, 1H), 1.73 (d, *J* = 9.7 Hz, 1H); ¹⁹F NMR (282 MHz, DMSO-*d₆*) *δ* - 123.39. |
| 4a | 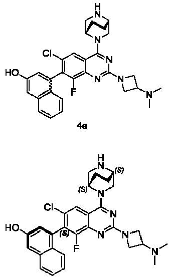 | (*R* or *S*)-4-(4-((1*S*,4*S*)-2,5-diazabicycl o[2.2.2]octa n-2-yl)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: n-hexane (0.1% diethylamine), mobile phase B: isopropanol; flow rate: 1 mL/min; isocratic elution with 30 % phase B in 15 min; detector UV 220/254 nm; retention time: 5.828 min; ee > 99%. Specific rotation [a]_{D}²⁵ = - 59.535 (c = 0.215, methanol) | 533. 2/53 5.2 | ¹H NMR (300 MHz, DMSO-*d₆*) *δ* 10.00 (s, 1H), 7.93 (d, *J* = 1.6 Hz, 1H), 7.79 (d, *J* = 8.3 Hz, 1H), 7.46 - 7.39 (m, 1H), 7.26 (d, *J* = 2.4 Hz, 1H), 7.23 - 7.20 (m, 2H), 7.03 (d, *J* = 2.4 Hz, 1H), 4.63 (s, 1H), 4.18 - 3.96 (m, 4H), 3.85 - 3.80 (m, 2H), 3.37 - 3.35 (m, 1H), 3.16 - 2.99 (m, 3H), 2.20 - 2.07 (m, 7H), 1.95 - 1.66 (m, 3H); ¹⁹F NMR (282 MHz, DMSO-*d₆*) *δ* - 123.57. |
| 4b | 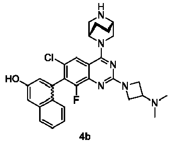 | (*S* or *R*)-4-(4-((1*S*,4*S*)-2,5-diazabicycl o[2.2.2]octa n-2-yl)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: n-hexane (0.1% diethylamine), mobile phase B: isopropanol; flow rate: 1 mL/min; isocratic elution with 30 % phase B in 15 min; detector UV 220/254 nm; retention time: 9.588 min; ee > 99%. Specific rotation [a]_{D}²⁵ = +46.667 (c = 0.057, methanol) | 533. 2/53 5.2 | ¹H NMR (300 MHz, DMSO-*d₆*) *δ* 10.00 (s, 1H), 7.94 (d, *J* = 1.6 Hz, 1H), 7.80 (d, *J* = 8.3 Hz, 1H), 7.46 - 7.41 (m, 1H), 7.26 (d, *J* = 2.4 Hz, 1H), 7.25 - 7.17 (m, 2H), 7.03 (d, *J* = 2.4 Hz, 1H), 4.64 (s, 1H), 4.19 - 3.97 (m, 4H), 3.85 - 3.80 (m, 2H), 3.37 - 3.35 (m, 1H), 3.13 - 3.01 (m, 3H), 2.19 - 2.08 (m, 7H), 1.98 - 1.66 (m, 3H); ¹⁹F NMR (282 MHz, DMSO-*d₆*) *δ* - 123.53. |
| **5a** | 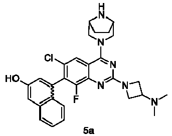 | (*R* or *S*)-4-(4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 2.549 min; ee > 99%. Specific rotation [a]_{D}²⁵ = - 58.865 (c = 0.235, methanol) | 533. 2/53 5.2 | ¹H NMR (300 MHz, DMSO-*d₆*) *δ* 9.99 (s, 1H), 7.80 (d, *J* = 8.3 Hz, 1H), 7.75 (d, *J* = 1.3 Hz, 1H), 7.46 - 7.41 (m, 1H), 7.26 (d, *J* = 2.4 Hz, 1H), 7.24 - 7.21 (m, 2H), 7.03 (d, *J* = 2.4 Hz, 1H), 4.27 - 4.20 (m, 2H), 4.12 - 4.05 (m, 2H), 3.88 - 3.82 (m, 2H), 3.52 - 3.40 (m, 5H), 3.15 - 3.07 (m, 1H), 2.12 (s, 6H), 1.76 - 1.59 (m, 4H); ¹⁹F NMR (282 MHz, DMSO-*d₆*) *δ* - 123.55. |
| **5b** | 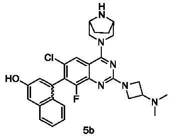 | (*S* or *R*)-4-(4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 3.363 min; ee > 99%. Specific rotation [a]_{D}²⁵ = +58.095 (c = 0.210, methanol) | 533. 2/53 5.2 | ¹H NMR (300 MHz, DMSO-*d₆*) *δ* 9.99 (s, 1H), 7.80 (d, *J* = 8.3 Hz, 1H), 7.75 (d, *J* = 1.6 Hz, 1H), 7.46 - 7.41 (m, 1H), 7.27 (d, *J* = 2.4 Hz, 1H), 7.24 - 7.21 (m, 2H), 7.03 (d, *J* = 2.4 Hz, 1H), 4.29 - 4.20 (m, 2H), 4.12 - 4.06 (m, 2H), 3.88 - 3.82 (m, 2H), 3.57 - 3.40 (m, 5H), 3.18 - 3.05 (m, 1H), 2.12 (s, 6H), 1.74 - 1.65 (m, 4H); ¹⁹F NMR (282 MHz, DMSO-*d₆*) *δ* - 123.54. |
| **6a** | 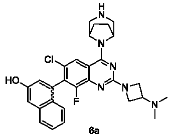 | (*R* or *S*)-4-(4-((1*R*,5*S*)-3,8-diazabicycl o[3.2.1]octa n-8-yl)-6-chloro-2-(3-(dimethyla mino)azetid in-1-yl)-8-fluoroquina zolin-7-yl)naphthale n-2-ol | Chiral column: CHIRALPAK IA-3, 4.6 x 50 mm, 3 µm; mobile phase A: n-hexane (0.1% diethylamine), mobile phase B: isopropanol; flow rate: 1 mL/min; isocratic elution with 20% phase B in 9 min; detector UV 220/254 nm; retention time: 6.871 min; ee value: 99%. Specific rotation [a]_{D}²⁵ = -77.204 (c = 0.155, methanol) | 533. 2/53 5.2 | ¹H NMR (400 MHz, DMSO-*d₆*) *δ* 9.99 (s, 1H), 7.81 - 7.78 (m, 2H), 7.45 - 7.41 (m, 1H), 7.30 - 7.25 (m, 1H), 7.23 - 7.21 (m, 2H), 7.05 - 7.03 (m, 1H), 4.72 - 4.65 (m, 2H), 4.12 - 4.05 (m, 2H), 3.87 - 3.83 (m, 2H), 3.13 - 3.04 (m, 3H), 2.78 - 2.73 (m, 2H), 2.11 (s, 6H), 1.95 - 1.84 (m, 4H); ¹⁹F NMR (282 MHz, DMSO-*d₆*) *δ* -123.55. |
| **6b** | 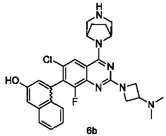 | (S or R)-4-(4-((1*R,*5*S*)-3,8-diazabicycl o[3.2.1]octa n-8-yl)-6-chloro-2-(3-(dimethyla mino)azetid in-1-yl)-8-fluoroquina zolin-7-yl)naphthale n-2-ol | Chiral column: CHIRALPAK IA-3, 4.6 x 50 mm, 3 µm; mobile phase A: n-hexane (0.1% diethylamine), mobile phase B: isopropanol; flow rate: 1 mL/min; isocratic elution with 20% phase B in 9 min; detector UV 220/254 nm; retention time: 4.373 min; ee > 99%. Specific rotation [a]_{D}²⁵ = +57.879 (c = 0.220, methanol) | 533. 2/53 5.2 | ¹H NMR (400 MHz, DMSO-*d₆*) *δ* 9.99 (s, 1H), 7.83 - 7.75 (m, 2H), 7.47 - 7.41 (m, 1H), 7.27 (d, *J* = 2.4 Hz, 1H), 7.23 - 7.20 (m, 2H), 7.04 (d, *J* = 2.4 Hz, 1H), 4.72 - 4.66 (m, 2H), 4.12 - 4.06 (m, 2H), 3.88 - 3.83 (m, 2H), 3.15 - 3.04 (m, 3H), 2.78 - 2.72 (m, 2H), 2.11 (s, 6H), 1.96 - 1.80 (m, 4H); ¹⁹F NMR (282 MHz, DMSO-*d₆*) *δ* 123.54. |
| **7a** | 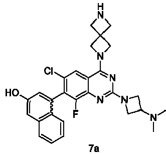 | (*R* or *S*)-4-(6-chloro-2-(3-(dimethyla mino)azetid in-1-yl)-8-fluoro-4-(2,6-diazaspiro[3 .3]heptan-2-yl)quinazoli n-7-yl)naphthale n-2-ol | Chiral column: Lux 3 µm Cellulose-2, 4.6 x 50 mm, 3 µm; mobile phase A: n-hexane (0.1% diethylamine), mobile phase B: ethanol; flow rate: 1 mL/min; isocratic elution with 30% phase B in 12 min; detector UV 220/254 nm; retention time: 4.914 min; ee > 99%. Specific rotation [a]_{D}²⁵ = - 51.556 (c = 0.225, methanol) | 519. 2/52 1.2 | ¹H NMR (300 MHz, DMSO-*d₆*) *δ* 7.80 (d, *J* = 8.3 Hz, 1H), 7.71 (d, *J* = 1.6 Hz, 1H), 7.46 - 7.41 (m, 1H), 7.26 (d, *J* = 2.4 Hz, 1H), 7.25 - 7.15 (m, 2H), 7.02 (d, *J* = 2.4 Hz, 1H), 4.60 (s, 4H), 4.10 - 4.03 (m, 2H), 3.86 - 3.80 (m, 2H), 3.72 - 3.69 (s, 4H), 3.15 - 3.05 (m, 1H), 2.11 (s, 6H); ¹⁹F NMR (282 MHz, *DMSO-d₆*) *δ* -123.48. |
| **7b** | 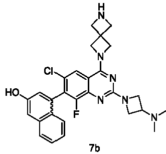 | (*S* or *R*)-4-(6-chloro-2-(3-(dimethyla mino)azetid in-1-yl)-8-fluoro-4-(2,6-diazaspiro[3 .3]heptan-2-yl)quinazoli n-7-yl)naphthale n-2-ol | Chiral column: Lux 3µm Cellulose-2, 4.6 x 50 mm, 3 µm; mobile phase A: n-hexane (0.1% diethylamine), mobile phase B: ethanol; flow rate: 1 mL/min; isocratic elution with 30% phase B in 12 min; detector UV 220/254 nm; retention time: 7.935 min; ee > 99%. Specific rotation [a]_{D}²⁵ = +53.000 (c = 0.200, methanol) | 519. 2/52 1.2 | ¹H NMR (300 MHz, DMSO-*d₆*) *δ* 7.80 (d, *J* = 8.3 Hz, 1H), 7.71 (d, *J* = 1.6 Hz, 1H), 7.46 - 7.41 (m, 1H), 7.26 (d, *J* = 2.4 Hz, 1H), 7.25 - 7.14 (m, 2H), 7.02 (d, *J* = 2.4 Hz, 1H), 4.60 (s, 4H), 4.09 - 4.03 (m, 2H), 3.86 - 3.80 (m, 2H), 3.68 (s, 4H), 3.17 - 3.03 (m, 1H), 2.11 (s, 6H); ¹⁹F NMR (282 MHz, DMSO-*d₆*) *δ* - 123.48. |
| **8a** | 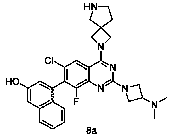 | (*R* or *S*)-4-(6-chloro-2-(3-(dimethyla mino)azetid in-1-yl)-8-fluoro-4-(2,6-diazaspiro[3 .4]octan-2-yl)quinazoli n-7-yl)naphthale n-2-ol | Chiral column: CHIRALPAK IC-3, 4.6 x 50 mm, 3 µm; mobile phase A: n-hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 2.844 min; ee > 99%. | 533. 2/53 5.2 | ¹H NMR (400 MHz, CD₃OD) *δ* 7.78 - 7.70 (m, 2H), 7.41 - 7.37 (m, 1H), 7.25 - 7.14 (m, 3H), 7.01 - 6.99 (m, 1H), 4.51 (s, 4H), 4.24 - 4.19 (m, 2H), 4.01 - 3.96 (m, 2H), 3.24 - 3.18 (m, 3H), 3.09 - 3.04 (m, 2H), 2.25 - 2.18 (m, 8H); ¹⁹F NMR (282 MHz, DMSO-*d₆*) *δ* -123.49. |
| **8b** | 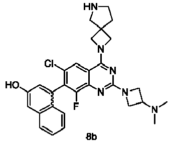 | (*S* or *R*)-4-(6-chloro-2-(3-(dimethyla mino)azetid in-1-yl)-8-fluoro-4-(2,6-diaza spiro[3.4]oc tan-2-yl) quinazolin-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 4.215 min; ee > 99%. | 533. 2/53 5.2 | ¹H NMR (400 MHz, CD₃OD) *δ* 7.78 - 7.70 (m, 2H), 7.41 - 7.37 (m, 1H), 7.25 - 7.14 (m, 3H), 7.01 - 6.99 (m, 1H), 4.51 (s, 4H), 4.24 - 4.19 (m, 2H), 4.01 - 3.96 (m, 2H), 3.24 - 3.16 (m, 3H), 3.04 - 2.99 (m, 2H), 2.24 - 2.15 (m, 8H); ¹⁹F NMR (282 MHz, DMSO-*d₆*) *δ* -123.48. |
| **9a** | 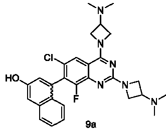 | (*R* or *S*)-4-(6-chloro-2,4-bis(3-(dimethyla mino)azetid in-1-yl)-8-fluoro-7-yl)naphthale n-2-ol | Chiral column: CHIRALPAK IC-3, 4.6 x 50 mm, 3 µm; mobile phase A: *n-*hexane/dichloromet hane = (5/1) (0.1% diethylamine), mobile phase B: isopropanol; flow rate: 1 mL/min; isocratic elution with 30% phase B in 6 min; detector UV 220/254 nm; retention time: 2.097 min; ee > 99%. | 521. 2/52 3.2 | ¹H NMR (300 MHz, DMSO-*d₆*) *δ* 9.99 (s, 1H), 7.85 - 7.71 (m, 2H), 7.46 - 7.41 (m, 1H), 7.26 (d, *J* = 2.4 Hz, 1H), 7.25 - 7.16 (m, 2H), 7.03 (d, *J* = 2.4 Hz, 1H), 4.53 (s, 2H), 4.30 (s, 2H), 4.10 - 4.03 (m, 2H), 3.87 - 3.80 (m, 2H), 3.28 - 3.19 (m, 1H), 3.13 - 3.05 (m, 1H), 2.17 (s, 6H), 2.11 (s, 6H); ¹⁹F NMR (282 MHz, DMSO-*d₆*) *δ* -123.51. |
| **9b** | 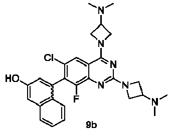 | (*S* or *R*)-4-(6-chloro-2,4-bis(3-(dimethyla mino)azetid in-1-yl)-8-fluoro-7-yl)naphthale n-2-ol | Chiral column: CHIRALPAK IC-3, 4.6 x 50 mm, 3 µm; mobile phase A: *n-*hexane/dichloromet hane = (5/1) (0.1% diethylamine), mobile phase B: isopropanol; flow rate: 1 mL/min; isocratic elution with 30% phase B in 6 min; detector UV 220/254 nm; retention time: 3.541 min; ee > 99%. | 521. 2/52 3.2 | ¹H NMR (300 MHz, DMSO-*d₆*) *δ* 9.99 (s, 1H), 7.85 - 7.71 (m, 2H), 7.46 - 7.41 (m, 1H), 7.26 (d, *J* = 2.4 Hz, 1H), 7.25 - 7.16 (m, 2H), 7.03 (d, *J* = 2.4 Hz, 1H), 4.53 (s, 2H), 4.30 (s, 2H), 4.10 - 4.03 (m, 2H), 3.87 - 3.80 (m, 2H), 3.28 - 3.19 (m, 1H), 3.13 - 3.05 (m, 1H), 2.17 (s, 6H), 2.11 (s, 6H); ¹⁹F NMR (282 MHz, DMSO-*d₆*) *δ* -123.51. |
| **10a** | 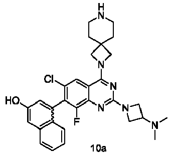 | (*R* or *S*)-4-(6-chloro-2-(3-(dimethyla mino)azetid in-1-yl)-8-fluoro-4-(2,7-diazaspiro[3 .5]nonan-2-yl)quinazoli n-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 4.5 min; detector UV 220/254 nm; retention time: 2.562 min; ee > 99%. | 547. 2/54 9.2 | ¹H NMR (300 MHz, DMSO-*d₆*) *δ* 9.99 (s, 1H),7.83 - 7.74 (m, 2H), 7.47 - 7.40 (m, 1H), 7.26 (d, *J* = 2.4 Hz, 1H), 7.25 - 7.15 (m, 2H), 7.02 (d, *J* = 2.4 Hz, 1H), 4.29 - 4.03 (m, 5H), 3.86 - 3.80 (m, 2H), 3.13 - 3.06 (m, 1H), 2.75 - 2.67 (m, 4H), 2.29 - 2.26 (m, 1H), 2.11 (s, 6H), 1.76 - 1.69 (m, 4H), 1.24 (s, 1H); ¹⁹F NMR (282 MHz, DMSO-*d₆*) *δ* -123.49. |
| **10b** | 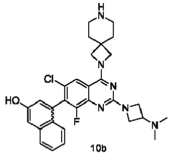 | (*S* or *R*)-4-(6-chloro-2-(3-(dimethyla mino)azetid in-1-yl)-8-fluoro-4-(2,7-diazaspiro[3 .5]nonan-2-yl)quinazoli n-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 4.5 min; detector UV 220/254 nm; retention time: 3.662 min; ee > 99%. | 547. 2/54 9.2 | ¹H NMR (300 MHz, DMSO-*d₆*) *δ* 9.99 (s, 1H), 7.83 - 7.74 (m, 2H), 7.47 - 7.40 (m, 1H), 7.26 (d, *J* = 2.4 Hz, 1H), 7.25 - 7.15 (m, 2H), 7.02 (d, *J* = 2.4 Hz, 1H), 4.29 - 4.03 (m, 5H), 3.86 - 3.80 (m, 2H), 3.13 - 3.06 (m, 1H), 2.75 - 2.67 (m, 4H), 2.29 - 2.26 (m, 1H), 2.11 (s, 6H), 1.76 - 1.69 (m, 4H), 1.24 (s, 1H); ¹⁹F NMR (282 MHz, DMSO-*d₆*) *δ* -123.49. |
| **11a** | 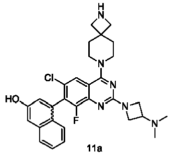 | (*R* or *S*)-4-(6-chloro-2-(3-(dimethyla mino)azetidin-1-yl)-8-fluoro-4-(2,7-diazaspiro[3 .5]nonan-7-yl)quinazoli n-7-yl)naphthale n-2-ol | Chiral column: CHIRALPAK IC-3, 4.6 x 50 mm, 3 µm; mobile phase A: *n-*hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.856 min; ee > 99%. | 547. 2/54 9.2 | ¹H NMR (300 MHz, DMSO-*d₆*) *δ* 7.79 (d, *J* = 8.3 Hz, 1H), 7.71 (d, *J* = 1.6 Hz, 1H), 7.46 - 7.39 (m, 1H), 7.26 (d, *J* = 2.4 Hz, 1H), 7.23 - 7.20 (m, 2H), 7.04 (d, *J* = 2.4 Hz, 1H), 4.13 - 4.06 (m, 2H), 3.90 - 3.83 (m, 2H), 3.66 - 3.51 (m, 7H), 3.18 - 2.99 (m, 2H), 2.11 (s, 6H), 1.93 - 1.87 (m, 4H); ¹⁹F NMR (282 MHz, DMSO-*d₆*) *δ* - 123.52. |
| **11b** | 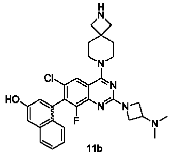 | (*S* or *R*)-4-(6-chloro-2-(3-(dimethyla mino)azetid in-1-yl)-8-fluoro-4-(2,7-diazaspiro[3 .5]nonan-7-yl)quinazoli n-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 4.120 min; ee value: 99%. | 547. 2/54 9.2 | ¹H NMR (300 MHz, DMSO-*d₆*) *δ* 7.80 (d, *J* = 8.3 Hz, 1H), 7.72 (d, *J* = 1.6 Hz, 1H), 7.46 - 7.41 (m, 1H), 7.27 (d, *J* = 2.4 Hz, 1H), 7.23 - 7.20 (m, 2H), 7.04 (d, *J* = 2.4 Hz, 1H), 4.14 - 4.06 (m, 2H), 3.90 - 3.84 (m, 2H), 3.66 - 3.57 (m, 7H), 3.16 - 3.08 (m, 2H), 2.12 (s, 6H), 1.93 - 1.87 (m, 4H); ¹⁹F NMR (282 MHz, DMSO-*d₆*) *δ* - 123.53. |
| **12a** | 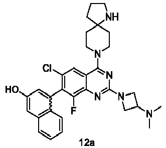 | (*R* or *S*)-4-(6-chloro-2-(3-(dimethyla mino)azetid in-1-yl)-8-fluoro-4-(1,8-diazaspiro[4 .5]decan-8-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 4.5 min; detector UV 220/254 nm; retention time: 2.317 min; ee > 99% | 561. 2/56 3.2 | ¹H NMR (400 MHz, DMSO-*d₆*) *δ* 10.07 (s, 1H), 7.78 (d, *J* = 8.3 Hz, 1H), 7.72 (d, *J* = 1.5 Hz, 1H), 7.44 - 7.40 (m, 1H), 7.25 (d, *J* = 2.4 Hz, 1H), 7.22 - 7.18 (m, 2H), 7.03 (dd, *J* = 2.4, 1.5 Hz, 1H), 4.12 - 4.07 (m, 2H), 3.89 - 3.83 (m, 2H), 3.78 - 3.68 (m, 4H), 3.16 - 3.06 (m, 1H), 2.86 (t, *J* = 6.8 Hz, 2H), 2.11 (s, 6H), 1.78 - 1.66 (m, 4H), 1.66 - 1.52 (m, 4H); ¹⁹F NMR (377 MHz, DMSO-*d₆*) *δ* -123.55. |
| **12b** | 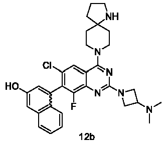 | (*S* or *R*)-4-(6-chloro-2-(3-(dimethyla mino)azetid in-1-yl)-8-fluoro-4-(1,8-diazaspiro[4 .5]decan-8-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 4.5 min; detector UV 220/254 nm; retention time: 3.182 min; ee > 99%. | 561. 2/56 3.2 | ¹H NMR (400 MHz, DMSO-*d₆*) *δ* 9.98 (s, 1H), 7.79 (d, *J* = 8.3 Hz, 1H), 7.73 (d, *J* = 1.5 Hz, 1H), 7.46 - 7.40 (m, 1H), 7.26 (d, *J* = 2.4 Hz, 1H), 7.23 - 7.20 (m, 2H), 7.04 (d, *J* = 2.4 Hz, 1H), 4.12 - 4.07 (m, 2H), 3.88 - 3.84 (m, 2H), 3.76 - 3.71 (m, 4H), 3.14 - 3.08 (m, 1H), 2.88 (t, *J* = 6.8 Hz, 2H), 2.11 (s, 6H), 1.78 - 1.57 (m, 8H); ¹⁹F NMR (377 MHz, DMSO-*d₆*) *δ* - 123.54. |
| **13a** | 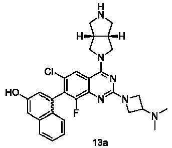 | (*R* or *S*)-4-(6-chloro-2-(3-(dimethyla mino)azetid in-1-yl)-8-fluoro-4-((3*aR*,6*aS*)-hexahydrop yrrolo[3,4-c]pyrrol-2 (1*H*)-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 10 min; detector UV 220/254 nm; retention time: 4.576 min; ee > 99%. | 533. 2/53 5.2 | ¹H NMR (300 MHz, DMSO-*d₆*) *δ* 8.08 (d, *J* = 1.7 Hz, 1H), 7.80 (d, *J* = 8.2 Hz, 1H), 7.46 - 7.41 (m, 1H), 7.27 (d, *J* = 2.4 Hz, 1H), 7.25 - 7.18 (m, 2H), 7.03 (d, *J* = 2.4 Hz, 1H), 4.20 - 4.04 (m, 4H), 3.86 - 3.77 (m, 4H), 3.58 - 3.50 (m, 1H), 3.14 - 3.05 (m, 1H), 3.01 - 2.86 (m, 4H), 2.84 - 2.74 (m, 2H), 2.11 (s, 6H); ¹⁹F NMR (282 MHz, DMSO-*d₆*) *δ* - 123.48. |
| **13b** | 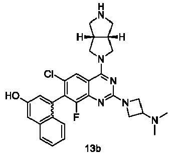 | (*S* or *R*)-4-(6-chloro-2-(3-(dimethylamino)azetid in-1-yl)-8-fluoro-4-((3*aR*,6*aS*)-hexahydrop yrrolo[3,4-c]pyrrol-2 (1*H*)-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 10 min; detector UV 220/254 nm; retention time: 8.266 min; ee > 98%. | 533. 2/53 5.2 | ¹H NMR (300 MHz, DMSO-*d₆*) *δ* 8.08 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.25 - 7.18 (m, 2H), 7.03 (d, *J* = 2.4 Hz, 1H), 4.20 - 4.05 (m, 4H), 3.86 - 3.78 (m, 4H), 3.60 - 3.50 (m, 1H), 3.14 - 3.06 (m, 1H), 3.00 - 2.86 (m, 4H), 2.82 - 2.74 (m, 2H), 2.11 (s, 6H); ¹⁹F NMR (282 MHz, DMSO-*d₆*) *δ* - 123.53. |
| **14a** | 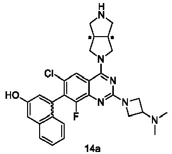 | (*R* or *S*)-4-(6-chloro-2-(3-(dimethyla mino)azetid in-1-yl)-8-fluoro-4-((3*aS*,6*aS* or 3*aR*,6*aR*)-hexahydrop yrrolo[3,4-c]pyrrol-2 (1*H*)-yl)quinazoli n-7-yl)naphthalen-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 3.616 min; ee > 99%. | 533. 2/53 5.2 | ¹H NMR (300 MHz, DMSO-*d₆*) *δ* 8.02 (d, *J* = 1.7 Hz, 1H), 7.80 (d, *J* = 8.3 Hz, 1H), 7.47 - 7.41 (m, 1H), 7.27 (d, *J* = 2.4 Hz, 1H), 7.24 - 7.21 (m, 2H), 7.05 (d, *J* = 2.4 Hz, 1H), 4.11 - 3.95 (m, 4H), 3.87 - 3.81 (m, 2H), 3.74 - 3.62 (m, 2H), 3.14 - 2.94 (m, 3H), 2.64 (t, *J* = 9.3 Hz, 2H), 2.33 - 2.24 (m, 2H), 2.11 (s, 6H); ¹⁹F NMR (282 MHz, DMSO-*d₆*) *δ* - 123.67. |
| **14b** | 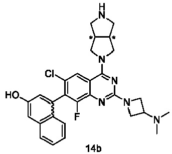 | (*S* or *R*)-4-(6-chloro-2-(3-(dimethyla mino)azetid in-1-yl)-8-fluoro-4-((3*aS*,6*aS* or 3*aR*,6*aR*)-hexahydrop yrrolo[3,4-c]pyrrol-2(1*H*)-yl)quinazoli n-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 5.340 min; ee > 99%. | 533. 2/53 5.2 | ¹H NMR (300 MHz, DMSO-*d₆*) *δ* 8.01 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.24 - 7.21 (m, 2H), 7.05 (d, *J* = 2.4 Hz, 1H), 4.10 - 3.94 (m, 4H), 3.87 - 3.57 (m, 4H), 3.14 - 3.03 (m, 3H), 2.68 (d, *J* = 9.6 Hz, 2H), 2.36 - 2.27 (m, 2H), 2.11 (s, 6H); ¹⁹F NMR (282 MHz, DMSO-*d₆*) *δ* - 123.64. |
| **14c** | 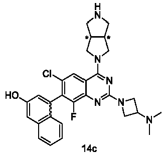 | (*R* or *S*)-4-(6-chloro-2-(3-(dimethyla mino)azetid in-1-yl)-8-fluoro-4-((3*aR*,6*aR* or 3*aS*,6*aS*)-hexahydrop yrrolo[3,4-c]pyrrol-2(1*H*)-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 10 min; detector UV 220/254 nm; retention time: 5.189 min; ee value: 98%. | 533. 2/53 5.2 | ¹H NMR (300 MHz, DMSO-*d₆*) *δ* 7.99 (s, 1H), 7.79 (d, *J* = 8.3 Hz, 1H), 7.46 - 7.41 (m, 1H), 7.27 (d, *J* = 2.4 Hz, 1H), 7.25 - 7.12 (m, 2H), 7.03 (d, *J* = 2.4 Hz, 1H), 4.10 - 3.98 (m, 4H), 3.87 - 3.74 (m, 4H), 3.39 - 3.29 (s, 3H), 3.15 - 3.05 (m, 2H), 2.99 - 2.89 (m, 2H), 2.11 (s, 6H); ¹⁹F NMR (282 MHz, DMSO-*d₆*) *δ* - 123.31. |
| **14d** | 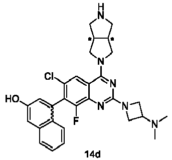 | (*S* or *R*)-4-(6-chloro-2-(3-(dimethyla mino)azetid in-1-yl)-8-fluoro-4-((3*aR*,6*aR* or 3*aS*,6*aS*)-hexahydrop yrrolo[3,4-*c*]pyrrol-2(1*H*)-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 30% phase B in 10 min; detector UV 220/254 nm; retention time: 7.314 min; ee > 99%. | 533. 2/53 5.2 | ¹H NMR (300 MHz, DMSO-*d₆*) *δ* 8.02 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.25 - 7.14 (m, 2H), 7.03 (d, *J* = 2.4 Hz, 1H), 4.09 - 3.94 (m, 4H), 3.87 - 3.65 (m, 4H), 3.15 - 2.95 (m, 3H), 2.64 (t, *J* = 9.6 Hz, 2H), 2.33 - 2.24 (m, 2H), 2.11 (s, 6H); ¹⁹F NMR (282 MHz, DMSO-*d₆*) *δ* - 123.44. |
| **15a** | 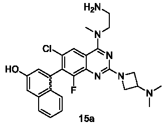 | (*R* or *S*)-4-(4((2-aminoethyl) (methyl)-amino)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 3.5 min; detector UV 220/254 nm; retention time: 1.347 min; ee value: 98%. | 495. 2/49 7.2 | ¹H NMR (300 MHz, DMSO-*d₆*) *δ* 8.22 (d, *J* = 1.5 Hz, 1H), 7.79 (d, *J* = 8.3 Hz, 1H), 7.46 - 7.40 (m, 1H), 7.26 (d, *J* = 2.4 Hz, 1H), 7.25 - 7.16 (m, 2H), 7.03 (d, *J* = 2.4 Hz, 1H), 4.10 - 4.04 (m, 2H), 3.87 - 3.81 (m, 2H), 3.66 - 3.59 (m, 2H), 3.14 - 3.06 (m, 1H), 2.81 (t, *J* = 6.3 Hz, 2H), 2.37 (s, 3H), 2.11 (s, 6H); ¹⁹F NMR (282 MHz, DMSO-*d₆*) *δ* -124.16. |
| **15b** | 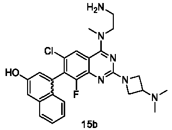 | (*S* or *R*)-4-(4((2-aminoethyl) (methyl)-amino)-6-chloro-2-(3-(dimethyla mino)azetid in-1-yl)-8-fluoroquina zolin-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 3.5 min; detector UV 220/254 nm; retention time: 2.180 min; ee value: 98%. | 495. 2/49 7.2 | ¹H NMR (300 MHz, DMSO-*d₆*) *δ* 8.23 - 8.18 (m, 1H), 7.79 (d, *J* = 8.3 Hz, 1H), 7.46 - 7.40 (m, 1H), 7.26 (d, *J* = 2.4 Hz, 1H), 7.24 - 7.16 (m, 2H), 7.09 - 7.00 (m, 1H), 4.11 - 4.03 (m, 2H), 3.87 - 3.81 (m, 2H), 3.66 - 3.58 (m, 2H), 3.14 - 3.04 (m, 1H), 2.80 (t, *J* = 6.3 Hz, 2H), 2.36 (s, 3H), 2.11 (s, 6H); ¹⁹F NMR (282 MHz, DMSO-*d₆*) *δ* -124.22. |
| **16a** | 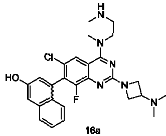 | (*R* or *S*)-4-(6-chloro-2-(3-(dimethyla mino)azetid in-1-yl)-8-fluoro-4-(methyl(2-(methylami no)ethyl)am ino)quinazo lin-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 1.690 min; ee > 99%. | 509. 2/51 1.2 | ¹H NMR (300 MHz, DMSO-*d₆*) *δ* 9.95 (s, 1H), 8.16 (d, *J* = 1.7 Hz, 1H), 7.80 (d, *J* = 8.3 Hz, 1H), 7.46 - 7.41 (m, 1H), 7.26 (d, *J* = 2.4 Hz, 1H), 7.24 - 7.20 (m, 2H), 7.04 (d, *J* = 2.4 Hz, 1H), 4.11 - 4.05 (m, 2H), 3.87 - 3.76 (m, 4H), 3.36 (s, 3H), 3.16 - 3.06 (m, 1H), 2.89 (t, *J* = 6.5 Hz, 2H), 2.36 (s, 3H), 2.11 (s, 6H); ¹⁹F NMR (282 MHz, DMSO-*d₆*) *δ* - 123.61. |
| **16b** | 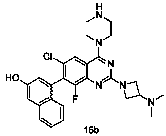 | (*S* or *R*)-4-(6-chloro-2-(3-(dimethyla mino)azetid in-1-yl)-8-fluoro-4-(methyl(2-(methylami no)ethyl)am ino)quinazo lin-7-yl)naphthale n-2-ol | Chiral column: CHIRALPAK IC-3, 4.6 x 50 mm, 3 µm; mobile phase A: *n-*hexane/dichloromet hane = 5/1 (0.1% diethylamine), mobile phase B: isopropanol; flow rate: 1 mL/min; isocratic elution with 30% phase B in 6 min; detector UV 220/254 nm; retention time: 2.820 min; ee value: 98%. | 509. 2/51 1.2 | ¹H NMR (300 MHz, DMSO-*d₆*) *δ* 9.95 (s, 1H), 8.16 (d, *J* = 1.7 Hz, 1H), 7.80 (d, *J* = 8.3 Hz, 1H), 7.46 - 7.41 (m, 1H), 7.26 (d, *J* = 2.4 Hz, 1H), 7.24 - 7.20 (m, 2H), 7.04 (d, *J* = 2.4 Hz, 1H), 4.11 - 4.05 (m, 2H), 3.87 - 3.76 (m, 4H), 3.36 (s, 3H), 3.16 - 3.06 (m, 1H), 2.91 (t, *J* = 6.5 Hz, 2H), 2.37 (s, 3H), 2.11 (s, 6H); ¹⁹F NMR (282 MHz, DMSO-*d₆*) *δ* - 123.61. |
| **17a** | 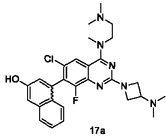 | (*R* or *S*)-4-(6-chloro-2-(3-(dimethyla mino)azetid in-1-yl)-4-((2-(dimethyla mino)ethyl) (methyl)ami no)-8-fluoroquinazolin-7-yl)naphthale n-2-ol | Chiral column: CHIRALPAK IG-3, 4.6 x 50 mm, 3 µm; mobile phase A: *n-*hexane/dichloromet hane = 3/1 (0.1% diethylamine), mobile phase B: ethanol; flow rate: 1 mL/min; isocratic elution with 50% phase B in 6 min; detector UV 220/254 nm; retention time: 4.257 min; ee value: 99%. | 523. 2/52 5.2 | ¹H NMR (300 MHz, DMSO-*d₆*) *δ* 9.99 (s, 1H), 8.08 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.24 - 7.20 (m, 2H), 7.04 (d, *J* = 2.4 Hz, 1H), 4.14 - 4.02 (m, 2H), 3.90 - 3.75 (m, 4H), 3.38 (s, 3H), 3.16 - 3.06 (m, 1H), 2.67 (t, *J* = 6.9 Hz, 2H), 2.26 (s, 6H), 2.11 (s, 6H); ¹⁹F NMR (282 MHz, DMSO-*d₆*) *δ* - 123.46. |
| **17b** | 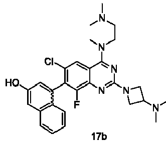 | (*S* or *R*)-4-(6-chloro-2-(3-(dimethyla mino)azetid in-1-yl)-4-((2-(dimethyla mino)ethyl) (methyl)ami no)-8-fluoroquinazolin-7-yl)naphthale n-2-ol | Chiral column: CHIRALPAK IG-3, 4.6 x 50 mm, 3 µm; mobile phase A: *n-*hexane/dichloromet hane = 3/1 (0.1% diethylamine), mobile phase B: ethanol; flow rate: 1 mL/min; isocratic elution with 50% phase B in 6 min; detector UV 220/254 nm; retention time: 2.734 min; ee > 99%. | 523. 2/52 5.2 | ¹H NMR (300 MHz, DMSO-*d₆*) *δ* 9.99 (s, 1H), 8.08 (d, *J* = 1.6 Hz, 1H), 7.80 (d, *J* = 8.3 Hz, 1H), 7.46 - 7.41 (m, 1H), 7.27 (d, *J* = 2.4 Hz, 1H), 7.24 - 7.20 (m, 2H), 7.04 (d, *J* = 2.4 Hz, 1H), 4.14 - 4.02 (m, 2H), 3.90 - 3.75 (m, 4H), 3.38 (s, 3H), 3.16 - 3.06 (m, 1H), 2.67 (t, *J* = 6.9 Hz, 2H), 2.26 (s, 6H), 2.11 (s, 6H); ¹⁹F NMR (282 MHz, DMSO-*d₆*) *δ* - 123.46. |
| **18a** | 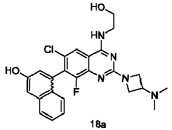 | (*R* or *S*)-4-(6-chloro-2-(3-(dimethyla mino)azetid in-1-yl)-8-fluoro-4-((2-hydroxyeth yl)amino)-quinazolin-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 20% phase B in 5.5 min; detector UV 220/254 nm; retention time: 3.619 min; ee value: 97%. | 482. 1/48 4.1 | ¹H NMR (300 MHz, DMSO-*d₆*) *δ* 9.97 (s, 1H), 8.34 - 8.29 (m, 1H), 8.26 (d, *J* = 1.5 Hz, 1H), 7.80 (d, *J* = 8.3 Hz, 1H), 7.46 - 7.41 (m, 1H), 7.26 (d, *J* = 2.4 Hz, 1H), 7.25 - 7.17 (m, 2H), 7.03 (d, *J* = 2.4 Hz, 1H), 4.81 (t, *J* = 5.4 Hz, 1H), 4.14 - 4.07 (m, 2H), 3.93 - 3.84 (m, 2H), 3.70 - 3.56 (m, 4H), 3.25 - 3.16 (m, 1H), 2.20 (s, 6H); ¹⁹F NMR (282 MHz, DMSO-*d₆*) *δ* - 124.20. |
| **18b** | 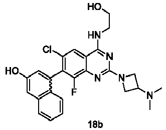 | (*S* or *R*)-4-(6-chloro-2-(3-(dimethyla mino)azetid in-1-yl)-8-fluoro-4-((2-hydroxyeth yl)amino)-quinazolin-7-yl)naphthale n-2-ol | Chiral column: CHIRALPAK IC-3, 4.6 x 50 mm, 3 µm; mobile phase A: *n-*hexane (0.1% diethylamine), mobile phase B: ethanol; flow rate: 1 mL/min; isocratic elution with 20% phase B in 5.5 min; detector UV 220/254 nm; retention time: 2.324 min; ee > 99%. | 482. 1/48 4.1 | ¹H NMR (300 MHz, DMSO-*d₆*) *δ* 9.97 (s, 1H), 8.29 (t, *J* = 5.3 Hz, 1H), 8.25 (d, *J* = 1.6 Hz, 1H), 7.79 (d, *J* = 8.3 Hz, 1H), 7.46 - 7.41 (m, 1H), 7.26 (d, *J* = 2.4 Hz, 1H), 7.24 - 7.17 (m, 2H), 7.03 (d, *J* = 2.4 Hz, 1H), 4.81 (t, *J* = 5.3 Hz, 1H), 4.11 - 4.04 (m, 2H), 3.87 - 3.80 (m, 2H), 3.70 - 3.56 (m, 4H), 3.14 - 3.05 (m, 1H), 2.11 (s, 6H); ¹⁹F NMR (282 MHz, DMSO-*d₆*) *δ* - 124.20. |
| **19a** | 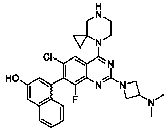 | (*R* or *S*)-4-(6-chloro-2-(3-(dimethyla mino)azetid in-1-yl)-8-fluoro-4-(4,7-diazaspiro[2 .5]octan-4-yl)quinazolin-7-yl)naphthale n-2-ol | Chiral column: CHIRALPAK IC-3, 4.6 x 50 mm, 3 µm; mobile phase A: *n-*hexane (0.1% diethylamine), mobile phase B: isopropanol; flow rate: 1 mL/min; isocratic elution with 50% phase B in 6 min; detector UV 220/254 nm; retention time: 1.741 min; ee > 99%. | 533. 3/53 5.3 | ¹H NMR (400 MHz, CD₃OD) *δ* 7.76 (d, *J* = 1.6 Hz, 1H), 7.73 (d, *J* = 7.8 Hz, 1H), 7.41 - 7.37 (m, 1H), 7.24 - 7.16 (m, 3H), 7.00 (d, *J* = 2.4 Hz, 1H), 4.28 - 4.24 (m, 2H), 4.08 - 3.93 (m, 4H), 3.34 - 3.32 (m, 2H), 3.28 - 3.22 (m, 1H), 2.90 - 2.84 (m, 2H), 2.24 (s, 6H), 1.02 - 0.97 (m, 2H), 0.90 - 0.85 (m, 2H); ¹⁹F NMR (377 MHz, CD₃OD) *δ* - 125.21. |
| **19b** | 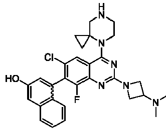 | (*S* or *R*)-4-(6-chloro-2-(3-(dimethyla mino)azetid in-1-yl)-8-fluoro-4-(4,7-diazaspiro[2 .5]octan-4-yl)quinazoli n-7-yl)naphthale n-2-ol | Chiral column: CHIRALPAK IC-3, 4.6 x 50 mm, 3 µm; mobile phase A: *n-*hexane (0.1% diethylamine), mobile phase B: isopropanol; flow rate: 1 mL/min; isocratic elution with 50% phase B in 6 min; detector UV 220/254 nm; retention time: 3.291 min; ee > 99%. | 533. 3/53 5.3 | ¹H NMR (400 MHz, CD₃OD) *δ* 7.76 (d, *J* = 1.6 Hz, 1H), 7.73 (d, *J* = 7.8 Hz, 1H), 7.41 - 7.37 (m, 1H), 7.24 - 7.16 (m, 3H), 7.00 (d, *J* = 2.4 Hz, 1H), 4.28 - 4.24 (m, 2H), 4.08 - 3.93 (m, 4H), 3.29 - 3.22 (m, 3H), 2.90 - 2.84 (m, 2H), 2.24 (s, 6H), 1.02 - 0.97 (m, 2H), 0.90 - 0.85 (m, 2H); ¹⁹F NMR (377 MHz, CD₃OD) *δ* - 125.21. |

### 1.2 Confirmation of the configuration of compound 4a

### Single crystal culture and data collection

### 1) Single crystal culture

First, 5 mg of compound **4a** (Lot number #EB2106527-197C1) was dissolved in 0.5 mL of MeOH, the mixture was filtered into a clean vial, and a little polymer HPMCP was added to the filtrate as template. The vial was covered with perforated sealing membrane and placed in a fume hood to slowly evaporate at room temperature. One day later, long sheet-like single crystals were obtained. This single crystal sample was used for single crystal X-ray diffraction analysis.

### 2) Instruments and parameters

Single crystal X-ray data of compound **4a** were collected on a Bruker D8 Venture diffractometer using a light source of Ga target Kα rays (λ = 1.34139 Å). During data collection, the crystal was kept at 296 K. The single crystal structure was analyzed in Olex2 software, the initial structure was calculated by the Intrinsic Phasing method of the SHELXT program, and the structure was refined by the least squares method of the SHELXL program.

### Single crystal X-ray diffraction analysis

The single crystal structure of compound **4a** belonged to the space group *P*2₁2₁2₁ of the orthorhombic system, and the molecular formula was C₂₉H₃₀ClFN₆O·CH₄O. There were one compound **4a** molecule and one MeOH molecule in each asymmetric unit, and there were 4 asymmetric units in each unit cell. The refined crystal structure parameters are shown in table 17.

**Table 17. Crystallographic data and structural refinement parameters of compound 4a**

| | |
|---|---|
| Empirical formula | C₂₉H₃₀ClFN₆O·CH₄O |
| Molecular weight | 565.08 |
| Temperature/K | 296 |
| Crystalline system | Orthorhombic system |
| Space group | *P*2₁2₁2₁ |
| a/Å | 7.65810(10) |
| b/Å | 17.5196(3) |
| c/Å | 20.1408(4) |
| α/° | 90 |
| β/° | 90 |
| γ/° | 90 |
| Unit cell volume/Å3 | 2702.23(8) |
| Z | 4 |
| ρ_{calc}g/cm³ | 1.389 |
| µ/mm⁻¹ | 1.067 |
| F(000) | 1192.0 |
| Crystal size/mm³ | 0.05 × 0.01 × 0.01 |
| Diffractive light source | Ga Kα (λ = 1.34139 Å) |
| Data collection 2θ range/° | 7.638 to 109.976 |
| Diffraction index range | -9 ≤ h ≤ 7, -21 ≤ k ≤ 21, -24 ≤ 1 ≤ 24 |
| collected diffraction points | 32187 |
| independent diffraction points | 5152 [Rᵢₙₜ = 0.0688, R_{sigma} = 0.0489] |
| Data/Limitations/Parameters | 5152/0/370 |
| Goodness of fit based on F² | 1.049 |
| Final R factor [I>=2σ (I)] | R₁ = 0.0465, wR₂ = 0.1071 |
| R factor [all data] | R₁ = 0.0700, wR₂ = 0.1202 |
| Maximum residual electron density peak/valley e Å⁻³ | 0.28/-0.22 |
| Flack parameters | 0.012(12) |

From the above, it can be seen that the configuration of compound **4a** was

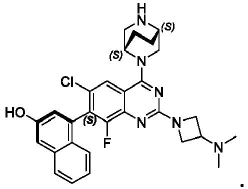

### Embodiment 2 (Synthesis method II)

### (R or S)-4-(6-chloro-2-(3-(dimethylamino)azetidin-1-yl)-8-fluoro-4-(piperidin-4-yl)quninazolin-7-yl)naphthalen-2-ol 20a; (S or R)-4-(6-chloro-2-(3-(dimethylamino)azetidin-1-yl)-8-fluoro-4-(piperidin-4-yl)quinazolin-7-yl)naphthalen-2-ol 20b

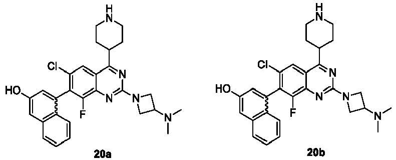

The synthetic route was as follows:

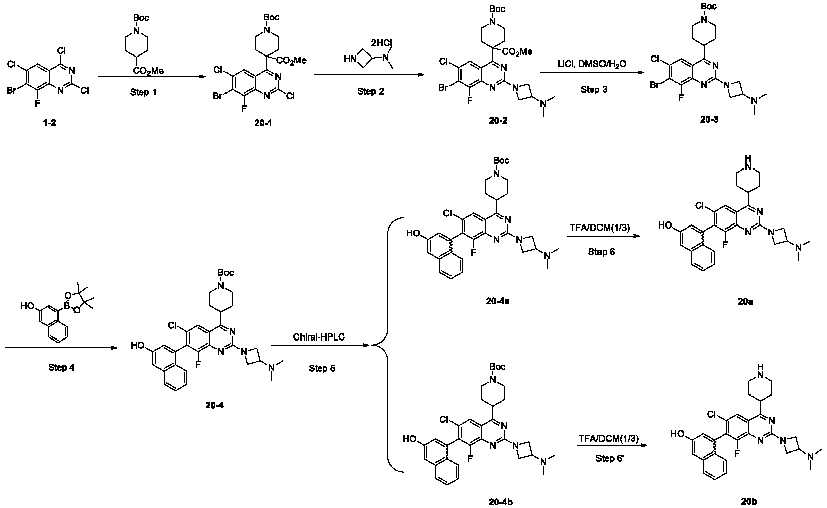

### Step 1:

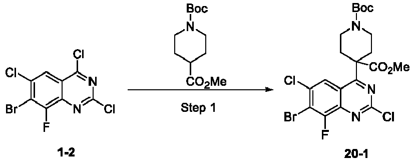

Compound **1-2** (1.5 g, 4.5 mmol, 1.0 eq), 1-(*tert*-butyl)4-methylpiperidine-1,4-dicarboxylate (1.1 g, 4.5 mmol, 1.0 eq) were added successively to a 50 mL Schlenk tube at room temperature, the mixture was replaced with nitrogen three times, and anhydrous tetrahydrofuran (15 mL) was added under a nitrogen atmosphere, then the mixture was cooled to -78 °C, and lithium bis(trimethylsilyl)amide (6.8 mL, 6.8 mmol, 1.5 eq) was added dropwise, after the dropwise addition, the mixture was recovered to room temperature and the reaction was carried out under stirring for 2 hours. The reaction process was monitored by liquid chromatography-mass spectrometry and thin layer chromatography. After the reaction was completed, the reaction was quenched with 0.5 M sodium dihydrogen phosphate (20 mL) and extracted with ethyl acetate (150 mL × 3). The organic phases were combined and washed with 200 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to obtain compound **20-1** (yellow solid, 0.79 g, yield: 33%). MS (ESI, *m*/*z*): 480.0/482.0/484.0 [M-*^{t}*Bu + H]⁺; ¹H NMR (300 MHz, CDCl₃) *δ* 7.98 (d, *J* = 2.0 Hz, 1H), 3.75 - 3.69 (m, 2H), 3.65 (s, 3H), 3.61 - 3.47 (m, 2H), 2.48-2.32 (s, 4H), 1.45 (s, 9H).

### Step 2:

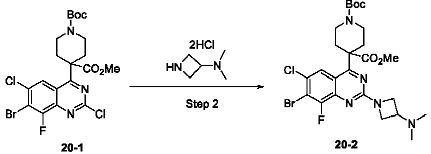

*N*,*N-*diisopropylethylamine (2.5 mL, 14.7 mmol, 10 eq) and 3-(dimethylamino)azetidine dihydrochloride (381.8 mg, 2.2 mmol, 1.5 eq) were added to a solution of compound **20-1** (790 mg, 1.5 mmol, 1 eq) in N-methylpyrrolidone (8.0 mL) with stirring at room temperature. The obtained mixture was stirred for 2 hours at 60 °C, and the reaction process was monitored by liquid chromatography-mass spectrometry and thin layer chromatography. After the reaction was completed, the reaction mixture was cooled to 25 °C. The reaction solution was directly purified by reversed-phase flash chromatography (C18 column), eluted with 50% → 95% acetonitrile/water mobile phase (0.1% ammonium bicarbonate) in 20 min; detector, UV254 nm; compound **20-2** (light yellow oily liquid, 450 mg, yield: 51%) was obtained. MS (ESI, m/z): 600.2/602.2/604.2 [M + H]⁺.

### Step 3:

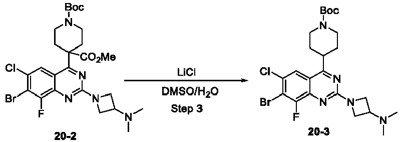

At room temperature, compound **20-2** (450 mg, 0.75 mmol, 1 eq) was added to a reaction flask, then dimethyl sulfoxide (5.0 mL) and water (0.5 mL) were added thereto, and finally lithium chloride (159 mg, 3.7 mmol, 5 eq) was added thereto. The obtained mixture was stirred for 3 hours at 150 °C, and the reaction process was monitored by liquid chromatography-mass spectrometry. After the reaction was completed, the reaction mixture was cooled to 25 °C. The reaction mixture was directly purified by reversed-phase rapid chromatographic column (C18 column), and eluted with 50% → 95% acetonitrile/water mobile phase (0.1% ammonium bicarbonate) in 20 min; detector, UV254 nm; compound **20-3** was obtained (light yellow solid, 200 mg, yield: 49%). MS (ESI, m/z): 542.2/544.2/546.2 [M + H]⁺; ¹H NMR (300 MHz, CDCl₃) *δ* 7.76 (d, *J* = 2.0 Hz, 1H), 4.28 (m, 4H), 4.09 (m, 2H), 3.42 - 3.30 (m, 1H), 3.28 - 3.17 (m, 1H), 2.94 (m, 2H), 2.24 (s, 6H), 1.87 (m, 4H), 1.49 (s, 9H).

### Step 4:

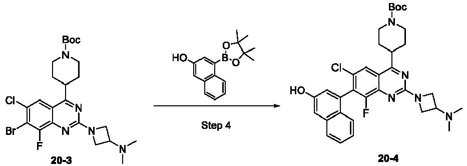

4-(4,4,5,5-Tetramethyl-1,3,2-dioxaborolan-2-yl)naphthalen-2-ol (149 mg, 0.6 mmol, 1.5 eq), potassium phosphate (170 mg, 0.8 mmol, 2 eq) and chloro(2-dicyclohexylphosphino-2',4',6'-triisoporpyl-1,1'-biphenyl)[2-(2'-amino-1,1'-biphenyl)] palladium (II) (31 mg, 0.04 mmol, 0.1 eq) were added to a solution of compound **20-3** (200 mg, 0.4 mmol, 1 eq) in tetrahydrofuran/water (10/1, 4 mL) with stirring under the protection of nitrogen at 25 °C. The obtained mixture was stirred for 1.5 hours at 60 °C, and the reaction process was monitored by liquid chromatography-mass spectrometry and thin layer chromatography. After the reaction was completed, the reaction mixture was cooled to 25 °C. The reaction mixture was concentrated and purified by reversed-phase rapid chromatographic column (C18 column), and eluted with 50% → 95% acetonitrile/water mobile phase (0.1% ammonium bicarbonate) in 20 min; detector, UV254 nm; compound **20-4** (a mixture of two stereoisomers, light yellow solid, 200 mg, yield: 89%) was obtained. MS (ESI, *m*/*z*): 606.3/608.3 [M + H]⁺; ¹H NMR (300 MHz, CDCl₃) *δ* 7.83 (d, *J* = 1.6 Hz, 1H), 7.71 (d, *J* = 8.3 Hz, 1H), 7.44 - 7.36 (m, 1H), 7.28 - 7.25 (m, 2H), 7.23 - 7.16 (m, 1H), 7.05 (d, *J* = 2.4 Hz, 1H), 4.34 (m, 4H), 4.16 (m, 2H), 3.45 (s, 1H), 3.32 - 3.19 (m, 1H), 2.98 (s, 2H), 2.27 (s, 6H), 1.91 (m, 4H), 1.49 (s, 9H).

### Step 5:

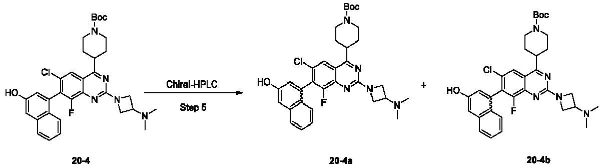

The compound **20-4** (240 mg) obtained in step 4 was subjected to chiral resolution by preparative chiral high pressure liquid chromatography: chiral column Lux 5µm Cellulose-4, 2.12 × 25 cm, 5 µm; mobile phase A: *n*-hexane (10 mmol/L ammonia-methanol), mobile phase B: ethanol; flow rate: 20 mL/min; elution with 30% phase B in 15 min, detector UV 220/210 nm. Two products were obtained, the product with shorter retention time (7.5 min) was compound **20-4a,** *tert*-butyl (*R* or *S*)-4-(6-chloro-2-(3-(dimethylamino)azetidin-1-yl)-8-fluoro-7-(3-hydroxynaphthalen-1-yl)quinazolin-4-yl)piperidin-1-carboxylate (light yellow solid, 85 mg, recovery rate: 42%), MS (ESI, m/z): 606.3/608.3 [M + H]⁺; the product with longer retention time (11.8 min) was compound **20-4b,** *tert*-butyl (*S* or *R*)-4-(6-chloro-2-(3-(dimethylamino)azetidin-1-yl)-8-fluoro-7-(3-hydroxynaphthalen-1-yl)quinazolin-4-yl) piperidin-1-carboxylate (light yellow solid, 75 mg, recovery rate: 37%), MS (ESI, m/z): 606.3/608.3 [M + H]⁺.

### Step 6:

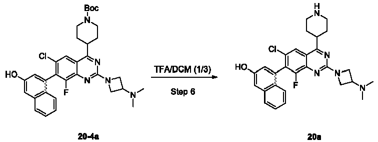

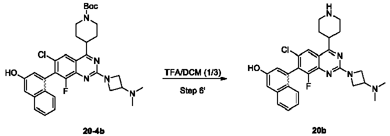

Trifluoroacetic acid (1 mL) was added dropwise to a solution of compound **20-4a** (85 mg, 0.1 mmol, 1.0 eq) in dichloromethane (3 mL) with stirring at 25 °C, and the reaction solution was stirred at this temperature for 1 hour, the reaction process was monitored by liquid chromatography-mass spectrometry. After the reaction was completed, the reaction solution was concentrated under reduced pressure to obtain a crude product. The crude product was directly purified by reversed-phase rapid chromatographic column (C18 column), and eluted with 50% → 95% acetonitrile/water mobile phase (0.1% ammonium bicarbonate) in 15 min; detector, UV254 nm; **20a** (light yellow solid, 22 mg, yield: 31%) was obtained. Compound **20b** (light yellow solid, 33 mg, yield: 52%) can be obtained by the same method as above.

Compound **20a:** MS (ESI, m/z): 506.2/508.2 [M + H]⁺; ¹H NMR (300 MHz, DMSO-*d*₆) *δ* 8.25 (s, 1H), 7.81 (d, *J* = 8.3 Hz, 1H), 7.44 (m, 1H), 7.31 - 7.14 (m, 3H), 7.06 (d, *J* = 2.4 Hz, 1H), 4.18 (m, 2H), 3.95 (m, 2H), 3.70 (s, 1H), 3.17 (m, 1H), 3.07 (m, 2H), 2.79 (m, 2H), 2.14 (s, 6H), 1.79 (s, 4H). The chiral analysis conditions of compound **20a** were: CHIRALPAK IC-3, 4.6 × 50 mm, 3 µm; mobile phase A: n-hexane/dichloromethane=5/1 (0.1% diethylamine), mobile phase B: isopropanol; flow rate: 1 mL/min; isocratic elution with 30% phase B in 6 min; detector UV 220/254 nm; retention time: 1.433 min; ee > 99%.

Compound **20b:** MS (ESI, m/z): 506.2/508.2 [M + H]⁺; ¹H NMR (300 MHz, DMSO-*d*₆) *δ* 8.25 (s, 1H), 7.81 (d, *J* = 8.3 Hz, 1H), 7.44 (m, 1H), 7.31 - 7.14 (m, 3H), 7.06 (d, *J* = 2.4 Hz, 1H), 4.18 (m, 2H), 3.95 (m, 2H), 3.70 (s, 1H), 3.17 (m, 1H), 3.07 (m, 2H), 2.79 (m, 2H), 2.14 (s, 6H), 1.79 (s, 4H). The chiral analysis conditions of compound **20b** were: CHIRALPAK IC-3, 4.6 × 50 mm, 3 µm; mobile phase A: *n*-hexane/dichloromethane=5/1 (0.1% diethylamine), mobile phase B: isopropanol; flow rate: 1 mL/min; isocratic elution with 30% phase B in 6 min; detector UV 220/254 nm; retention time: 1.905min; ee > 98%.

### Embodiment 3

### (S or R)-4-(4-(azetidin-3-yl)-6-chloro-2-(3-(dimethylamino)azetidin-1-yl)-8-fluoroquinazolin-7-yl)naphthalen-2-ol bistrifluoroacetate 21a; (R or S)-4-(4-(azetidin-3-yl)-6-chloro-2-(3-(dimethylamino)azetidin-1-yl)-8-fluoroquinazolin-7-yl)naphthalen-2-ol bistrifluoroacetate 21b

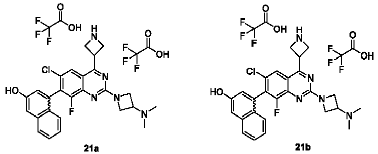

### Step 1:

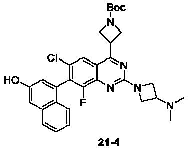

Compound **21-4** was synthesized according to Embodiment 2 (synthesis method II). Compound **21-4** (light yellow solid): MS (ESI, m/z): 578.3/580.3 [M+H]⁺.

### Step 2:

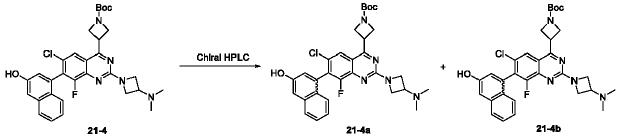

The compound **21-4** (210 mg) obtained in step 1 was subjected to chiral resolution by preparative chiral high performance liquid chromatography: chiral column NB_Lux 5µm i-Cellulose-5, 2.12 × 25 cm, 5 µm; mobile phase A: *n*-hexane/dichloromethane=5/1 (0.5% 2 mol/L ammonia-methanol), mobile phase B: isopropanol; flow rate: 18 mL/min; elution with 30% phase B in 30 min, detector UV 220 nm. Two products were obtained, the product with shorter retention time (5.8 min) was compound **21-4a,** *tert*-butyl (*S* or *R*)-3-(6-chloro-2-(3-(dimethylamino)azetidin-1-yl)-8-fluoro-7-(3-hydroxynaphthalen-1-yl)quinazolin-4-yl)azetidine-1-carboxylate (light yellow solid, 97 mg, recovery rate: 46%); the product with longer retention time (13.1 min) was compound **21-4b,** *tert*-butyl (*R* or *S*)-3-(6-chloro-2-(3-(dimethylamino)azetidin-1-yl)-8-fluoro-7-(3-hydroxynaphthalen-1-yl)quinazolin-4-yl)azetidine-1-carboxylate (light yellow solid, 100 mg, recovery rate: 47%).

### Step 3:

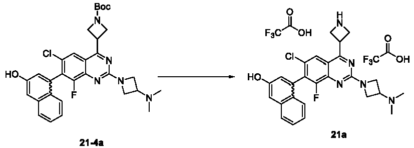

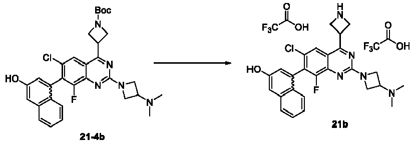

Trifluoroacetic acid (1.5 mL) was added dropwise to a solution of compound **21-4a** (97 mg, 0.16 mmol, 1.0 eq) in dichloromethane (4.5 mL) with stirring at 25 °C, and after the addition, the reaction was carried out at this temperature for 1 hour, and the reaction process was monitored by liquid chromatography-mass spectrometry. After the reaction was completed, the reaction solution was concentrated under reduced pressure to obtain a crude product. The crude product was purified by preparative high performance liquid chromatography under the following purification conditions: XSelect CSH Prep C18 OBD Column, 19 × 150 mm, 5 µm; mobile phase A: water (0.05% trifluoroacetic acid), mobile phase B: acetonitrile; flow rate: 25 mL/min; gradient: elution with 2% phase B in 2 min, gradient elution with 2% → 9% phase B in 2.5 min, and gradient elution with 9% → 30% phase B in 9.5 min; detector: UV 254/220 nm; **21a** (yellow solid, 72 mg, yield: 63%) was obtained. Compound **21b** (light yellow solid, 76 mg, yield: 65%) can be obtained by the same method as above.

Compound **21a:** MS (ESI, m/z): 478.2/480.2 [M + H]⁺; ¹H NMR (300 MHz, CD₃OD) *δ* 7.96 (d, *J* = 1.6 Hz, 1H), 7.78 (d, *J* = 8.3 Hz, 1H), 7.46 - 7.41 (m, 1H), 7.29 (d, *J* = 2.4 Hz, 1H), 7.23 - 7.16 (m, 2H), 7.03 (d, *J* = 2.4 Hz, 1H), 5.09 - 4.98 (m, 1H), 4.72 - 4.51 (m, 8H), 4.40-4.26 (m, 1H), 3.00 (s, 6H); ¹⁹F NMR (282 MHz, CD₃OD) *δ* -77.76, -125.02. The chiral analysis conditions of compound **21a** were: CHIRALPAK IC-3, 4.6 × 100 mm, 3 µm; mobile phase A: *n*-hexane/dichloromethane=5/1 (0.1% diethylamine), mobile phase B: ethanol; flow rate: 1 mL/min; isocratic elution with 30% phase B in 10 min; detector UV 220/254 nm; retention time: 3.80 min; ee > 99%.

Compound **21b:** MS (ESI, m/z): 478.2/480.2 [M + H]⁺; ¹H NMR (300 MHz, CD₃OD) *δ* 7.96 (d, *J* = 1.6 Hz, 1H), 7.78 (d, *J* = 8.3 Hz, 1H), 7.46 - 7.41 (m, 1H), 7.29 (d, *J* = 2.4 Hz, 1H), 7.23 - 7.16 (m, 2H), 7.03 (d, *J* = 2.4 Hz, 1H), 5.09 - 4.98 (m, 1H), 4.72 - 4.51 (m, 8H), 4.40-4.26 (m, 1H), 3.00 (s, 6H); ¹⁹F NMR (282 MHz, CD₃OD) δ -77.93, -125.02. The chiral analysis conditions of compound **21b** were: CHIRALPAK IC-3, 4.6 × 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 6 min; detector UV 220/254 nm; retention time: 5.950 min; ee > 99%.

### Embodiment 4 (Synthesis method III)

### (S or R)-3-(6-chloro-2-(3-(dimethylamino)azetidin-1-yl)-8-fluoro-7-(3-hydroxynaphthalen-1-yl)quinazolin-4-yl)azetidin-3-carboxamide bistrifluoroacetate 22a; (R or S)-3-(6-chloro-2-(3-(dimethylamino)azetidin-1-yl)-8-fluoro-7-(3-hydroxynaphthalen-1-yl)quinazolin-4-yl)azetidin-3-carboxamide bistrifluoroacetate 22b

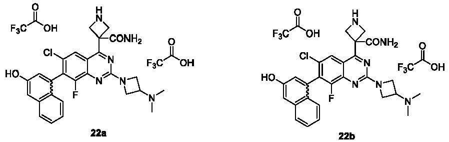

The synthetic route was as follows:

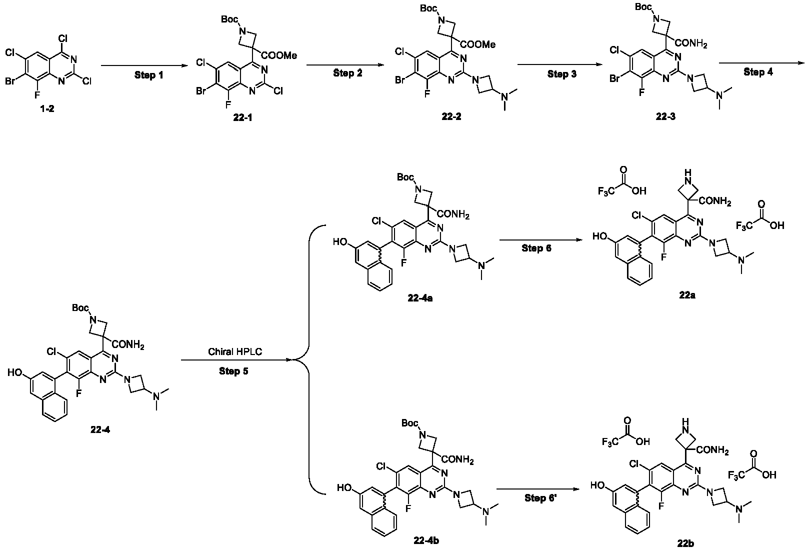

### Step 1:

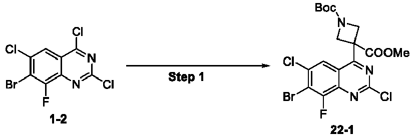

Compound **1-2** (2.0 g, 5.75 mmol, 1.0 eq), methyl 1-(*tert*-butoxycarbonyl)azetidin-3-carboxylate (1.3 g, 5.75 mmol, 1.0 eq) were successively added to a 50 mL Schlenk tube with stirring at 25 °C, and the mixture was replaced with nitrogen three times, then anhydrous tetrahydrofuran (20 mL) was added thereto under nitrogen atmosphere, and the mixture was cooled to -78 °C. Lithium bis(trimethylsilyl)amide (1.2 mol/L tetrahydrofuran solution, 5.6 mL, 6.90 mmol, 1.2 eq) was added dropwise to the mixture, after the dropwise addition, the temperature was slowly raised to room temperature and the reaction was carried out for 2 hours. The reaction process was monitored by liquid chromatography-mass spectrometry. After the reaction was completed, the reaction was quenched with 0.5 mol/L sodium dihydrogen phosphate solution (20 mL) and extracted with ethyl acetate (150 mL × 3). The organic phases were combined, washed with 200 mL saturated brine, dried over anhydrous sodium sulfate, filtered, and concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to obtain compound **22-1** (yellow solid, 1.9 g, yield: 64%). MS (ESI, *m*/*z*): 508.1/510.1/512.1 [M + H]⁺; ¹H NMR (300 MHz, CDCl₃) *δ* 7.53 (d, *J* = 2.0 Hz, 1H), 4.73 - 4.67 (m, 4H), 3.76 (s, 3H), 1.47 (s, 9H).

### Step 2:

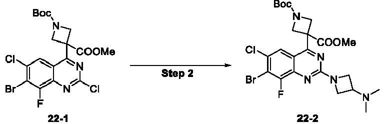

*N*,*N*-diisopropylethylamine (4.7 g, 36.36 mmol, 10.0 eq) and 3-(dimethylamino) azetidine dihydrochloride (940 mg, 5.43 mmol, 1.5 eq) were added to a solution of compound **22-1** (1.85 g, 3.63 mmol, 1.0 eq) in *N*-methylpyrrolidone (18.0 mL) with stirring at 25 °C. The reaction was carried out for 2 hours at 60 °C, and the reaction process was monitored by liquid chromatography-mass spectrometry. After the reaction was completed, the reaction mixture was cooled to 25 °C. The reaction mixture was directly purified by reversed-phase rapid chromatographic column (C18 column), and eluted with 50% → 95% acetonitrile/water (0.1% ammonium bicarbonate) in 20 min; detector, UV254 nm; compound **22-2** (yellow solid, 450 mg, yield: 51%) was obtained. MS (ESI, m/z): 572.1/574.1 [M + H]⁺; ¹H NMR (300 MHz, CDCl₃) *δ* 7.23 (d, *J* = 1.9 Hz, 1H), 4.63 - 4.59 (m, 4H), 4.42 - 4.18 (m, 4H), 3.73 (s, 3H), 3.39 - 3.32 (s, 1H), 2.34 (s, 6H), 1.47 (s, 9H).

### Step 3:

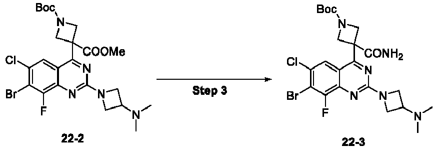

Compound **22-2** (380 mg, 0.65 mmol, 1.0 eq) and 7 mol/L ammonia methanol solution (20 mL) were added to a 100 mL sealed jar at 25 °C. The reaction was carried out for 16 hours in a sealed condition at 50 °C. After the reaction was completed, the reaction solution was cooled to room temperature and concentrated to obtain a crude product of compound **22-3** (yellow solid, 370 mg). The crude product was used directly in the next step without further purification. MS (ESI, m/z): 557.2/559.2 [M + H]⁺.

### Step 4:

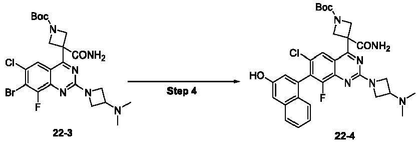

4-(4,4,5,5-Tetramethyl-1,3,2-dioxaborolan-2-yl)-naphthalen-2-ol (349 mg, 1.50 mmol, 1.5 eq), sodium carbonate (182 mg, 3.00 mmol, 2.0 eq) and [1,1'-bis(diphenylphosphino)ferrocene]dichloropalladium (70 mg, 0.15 mmol, 0.1 eq) were added to a solution of compound **22-3** (370 mg) in 1,4-dioxane/water (5/1, 5.0 mL). The reaction was carried out for 1.5 hours at 80 °C under nitrogen atmosphere, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **22-4** (a mixture of two stereoisomers, brown solid, 385 mg, yield: 93%). MS (ESI, m/z): 621.2/623.2 [M + H]⁺.

### Step 5:

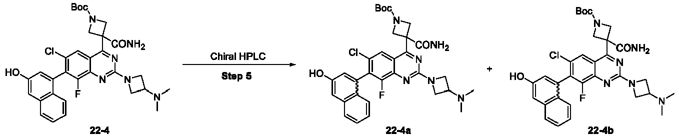

The compound **22-4** (385 mg) obtained in step 4 was subjected to chiral resolution, and the resolution conditions were: chiral column: CHIRALPAK ID, 2 × 25 cm, 5 µm; mobile phase A: *n*-hexane/dichloromethane = 5/1 (0.5%, 2 mol/L ammonia methanol solution), mobile phase B: isopropanol; flow rate: 20 mL/min; gradient: elution with 30% phase B in 13 min; detector: UV 220 nm; two products were obtained, the compound with shorter retention time (4.02 min) was **22-4a,** *tert*-butyl (*S* or *R*)-3-carbamoyl-3-(6-chloro-2-(3-(dimethylamino)azetidin-1-yl)-8-fluoro-7-(3-hydroxynaphthalen-1-yl)quinazolin-4-yl)azetidin-1-carboxylate (yellow solid, 166 mg, recovery rate: 43%); the product with longer retention time (7.55 min) was **22-4b,** *tert*-butyl (*R* or *S*)-3-carbamoyl-3-(6-chloro-2-(3-(dimethylamino)azetidin-1-yl)-8-fluoro-7-(3-hydroxynaphthalen-1-yl)quinazolin-4-yl)azetidin-1-carboxylate (yellow solid, 157 mg, recovery rate: 40% ).

### Step 6:

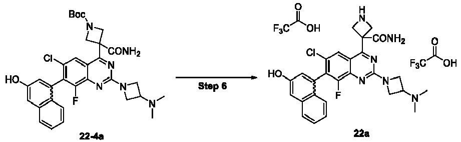

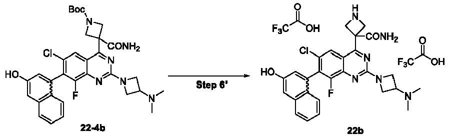

Trifluoroacetic acid (1.5 mL) was added dropwise to a solution of compound **22-4a** (160 mg, 0.26 mmol, 1.0 eq) in dichloromethane (4.5 mL) with stirring at 25 °C. The reaction was carried out for 1 hour at that temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), eluted with 2%→32% acetonitrile/water (0.05% TFA) mobile phase in 15 min; detector: UV254/220 nm; the compound **22a** (yellow solid, 139.2 mg, yield: 72%) was obtained. Compound **22b** (yellow solid, 128.8 mg, yield: 71%) can be obtained by the same method as above.

Compound **22a:** MS (ESI, m/z): 521.3/523.2 [M + H]⁺; ¹H NMR (300 MHz, DMSO-*d*₆) *δ* 10.91 (s, 1H), 10.11 (s, 1H), 9.37 (s, 1H), 9.19 (s, 1H), 7.84 - 7.81 (m, 2H), 7.52 (d, *J* = 1.5 Hz, 1H), 7.48 - 7.40 (m, 2H), 7.31 (d, *J* = 2.4 Hz, 1H), 7.26 - 7.21 (m, 1H), 7.13 (d, *J* = 8.4 Hz, 1H), 7.06 (d, *J* = 2.4 Hz, 1H), 4.76 - 4.70 (m, 4H), 4.53 - 4.42 (m, 4H), 4.33 - 4.26 (m, 1H), 2.86 (s, 6H); ¹⁹F NMR (282 MHz, DMSO-*d*₆) *δ* -73.69, -122.74. The chiral analysis conditions of compound **22a** were: CHIRALPAK ID-3, 4.6 × 100 mm, 3 µm; mobile phase A: n-hexane/dichloromethane=5/1 (0.1% diethylamine), mobile phase B: ethanol; flow rate: 1 mL/min; isocratic elution with 30% phase B in 8 min; detector UV 220/254 nm; retention time: 3.40 min; ee > 99%.

Compound **22b:** MS (ESI, m/z): 521.15/523.15 [M + H]⁺; ¹H NMR (300 MHz, DMSO-*d*₆) *δ* 10.91 (s, 1H), 10.11 (s, 1H), 9.37 (s, 1H), 9.19 (s, 1H), 7.84 - 7.81 (m, 2H), 7.52 (d, *J* = 1.5 Hz, 1H), 7.48 - 7.40 (m, 2H), 7.31 (d, *J* = 2.4 Hz, 1H), 7.26 - 7.21 (m, 1H), 7.13 (d, *J* = 8.4 Hz, 1H), 7.06 (d, *J* = 2.4 Hz, 1H), 4.76 - 4.70 (m, 4H), 4.53 - 4.42 (m, 4H), 4.33 - 4.26 (m, 1H), 2.86 (s, 6H). ¹⁹F NMR (282 MHz, DMSO-*d*₆) *δ* -73.80, -122.74. The chiral analysis conditions of compound **22b** were: CHIRALPAK ID-3, 4.6 × 100 mm, 3 µm; mobile phase A: n-hexane/dichloromethane=5/1 (0.1% diethylamine), mobile phase B: ethanol; flow rate: 1 mL/min; isocratic elution with 30% phase B in 8 min; detector UV220/254 nm; retention time: 5.31 min; ee > 99%.

### Embodiment 5 (Synthesis method IV)

### (S or R)-4-(6-chloro-4-(1,4-diaza-1-yl)-2-(3-(dimethylamino)azetidin-1-yl)-8-fluoroquinazolin-7-yl)naphthalen-2-ol 23

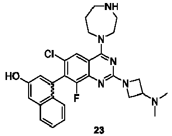

The synthetic route was as follows:

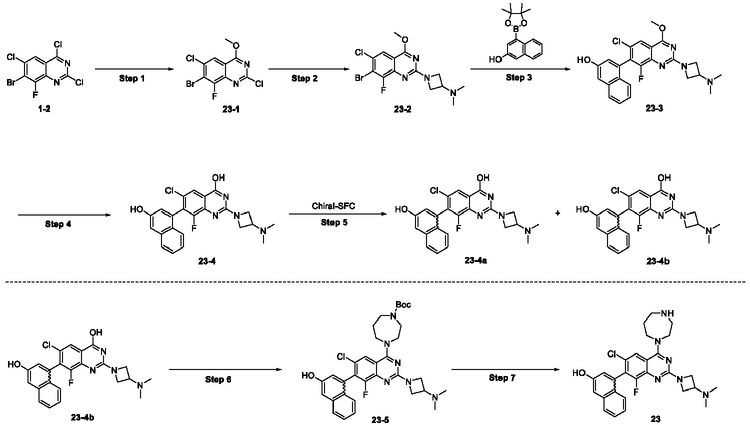

### Step 1:

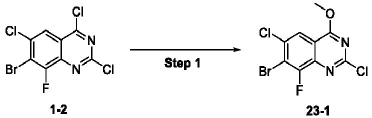

Sodium methoxide (0.98 g, 17.23 mmol, 1.2 eq) was added to a solution of compound **1-2** (5 g, 14.38 mmol, 1.00 eq) in methanol (5 mL) with stirring under the protection of nitrogen at 0 °C. The reaction was carried out for 4 hours at 25 °C under nitrogen atmosphere, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 10% ethyl acetate/petroleum ether mobile phase, and the obtained fractions were evaporated under reduced pressure to obtain compound **23-1** (light yellow solid, 4.3 g, yield: 87 %). MS (ESI, *m*/*z*): 324.9/326.9/328.9 [M + H]⁺; ¹H NMR (400 MHz, CDCl₃) *δ* 8.06 (d, *J* = 2.0 Hz, 1H), 4.25 (s, 3H).

### Step 2:

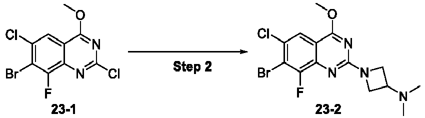

3-(Dimethylamino) azetidine dihydrochloride (3.42 g, 19.76 mmol, 1.5 eq) and *N,N-*diisopropylethylamine (8.53 g, 66.00 mmol, 5.0 eq) were added to a solution of compound **23-1** (4.3 g, 13.19 mmol, 1.00 eq) in *N*-methylpyrrolidone (43.0 mL)with stirring under the protection of nitrogen at 25 °C. The reaction was carried out for 2 hours at 60 °C under nitrogen atmosphere. The reaction process was monitored by liquid chromatography-mass spectrometry. After the reaction was completed, the reaction mixture was cooled to 25 °C. The reaction mixture was directly purified by reversed-phase chromatographic column (C18 column), and eluted with 5% → 95% acetonitrile/water mobile phase (0.1% ammonia water) in 25 min; detector, UV254/220 nm; compound **23-2** (white solid, 4.9 g, yield: 95%) was obtained. MS (ESI, *m*/*z*): 389.0/391.0/393.0 [M + H]⁺; ¹H NMR (300 MHz, CDCl₃) *δ* 7.83 (d, *J* = 1.9 Hz, 1H), 4.31 -4.25 (m, 2H), 4.12 - 4.07 (m, 5H), 3.28 - 3.20 (m, 1H), 2.26 (s, 6H).

### Step 3:

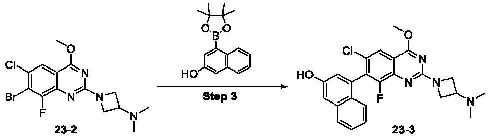

Water (5.0 mL), potassium phosphate (4.36 g, 19.50 mmol, 2.0 eq) and 4-(4,4,5,5-tetramethyl-1,3,2-dioxaborolan-2-yl)naphthalen-2-ol (3.61 g, 12.68 mmol, 1.3 eq) and chloro(2-dicyclohexylphosphino-2',4',6'-triisoporpyl-1,1 '-biphenyl) [2-(2'-amino-1,1'-biphenyl)] palladium (II) (810 mg, 0.98 mmol, 0.10 eq) were successively added to a solution of compound **23-2** (4 g, 9.75 mmol, 1.00 eq) in tetrahydrofuran (50.0 mL) with stirring under the protection of nitrogen at 25 °C. The reaction was carried out for 2 hours at 65 °C under nitrogen atmosphere. The reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **23-3** (off-white solid, 4 g, yield: 90%). MS (ESI, *m*/*z*): 453.2/455.2 [M + H]⁺; ¹H NMR (300 MHz, CDCl₃) *δ* 7.83 (d, *J* = 1.5 Hz, 1H), 7.68 (d, *J* = 8.2 Hz, 1H), 7.38 - 7.34 (m, 1H), 7.25 - 7.23 (m, 2H), 7.17 - 7.13 (m, 1H), 7.06 - 7.04 (m, 1H), 4.35 - 4.26 (m, 2H), 4.17 - 4.07 (s, 5H), 3.23 - 3.17 (m, 1H), 2.23 (s, 6H).

### Step 4:

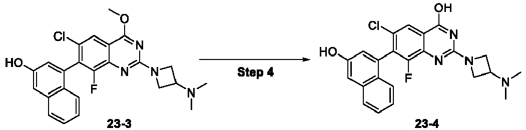

An aqueous solution of hydrochloric acid (4 mol/L, 10.0 mL) was added to a solution of compound **23-3** (4 g, 8.39 mmol, 1.00 eq) in tetrahydrofuran (50.0 mL) with stirring at 25 °C. The reaction was carried out for 2 hours at 80 °C under nitrogen atmosphere. The reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→20% dichloromethane/methanol mobile phase, and the obtained fractions were evaporated under reduced pressure to remove the solvent to obtain compound **23-4** (a mixture of two stereoisomers, light yellow solid, 2.6 g, yield: 70%). MS (ESI, *m*/*z*): 439.1/441.1 [M + H]⁺; ¹H NMR (300 MHz, DMSO-*d₆*) *δ* 11.85 (s, 1H), 9.99 (s, 1H), 7.89 (d, *J* = 1.5 Hz, 1H), 7.80 (d, *J* = 8.3 Hz, 1H), 7.46 - 7.41 (m, 1H), 7.27 - 7.17 (m, 3H), 7.03 (d, *J* = 2.4 Hz, 1H), 4.18 - 4.08 (m, 2H), 3.94 - 3.89 (m, 2H), 3.17 - 3.11 (m, 1H), 2.11 (s, 6H).

### Step 5:

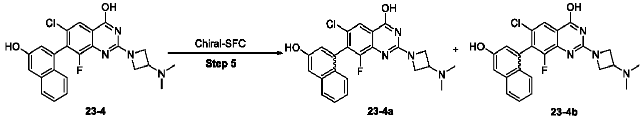

The compound **23-4** (2.6 g) obtained in step 4 was subjected to chiral resolution, the resolution conditions were: chiral column NBCHIRALPAK AD-H, 3x25 cm, 5 µm; mobile phase A: supercritical carbon dioxide fluid, mobile phase B: methanol; flow rate: 60 mL/min; column temperature: 35 °C; elution with 55% mobile phase B in15 min; detector UV 215 nm; two products were obtained. The product with shorter retention time (5.46 min) was **23-4a,** (*S* or *R*)-6-chloro-2-(3-(dimethylamino)azetidin-1-yl)-8-fluoro-7-(3-hydroxynaphthalen-1-yl)quinazolin-4-ol (light yellow solid, 1.23 g, recovery rate: 47%); the product with longer retention time (9.15 min) was **23-4b,** (*R* or *S*)-6-chloro-2-(3-(dimethylamino)azetidin-1-yl)-8-fluoro-7-(3-hydroxynaphthalen-1-yl)quinazolin-4-ol (light yellow solid, 1.25 g, recovery rate: 48%).

### Step 6:

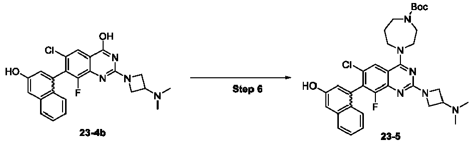

Bromo-tris-pyrrolidino-phosphonium hexafluorophosphate (318 mg, 0.68 mmol, 3.0 eq) and triethylamine (138 mg, 1.37 mmol, 6.00 eq) were added to a solution of compound **23-4b** (100 mg, 0.23 mmol, 1.00 eq) in *N*-methylpyrrolidone (2.0 mL) with stirring under the protection of nitrogen at 25 °C. The reaction was carried out for 0.5 hours at 25 °C under nitrogen atmosphere. *tert*-Butyl 1,4-diazacycloheptane-1-carboxylate (68 mg, 0.34 mmol, 1.5 eq) was added to the reaction solution, and the reaction was continued for 1.5 hours at 25 °C, the reaction process was monitored by liquid chromatography-mass spectrometry. After the reaction was completed, the reaction mixture was directly purified by reversed-phase chromatographic column (C18 column), and eluted with 5% → 95% (acetonitrile/methanol 1/ 1)/water mobile phase (0.1% ammonium bicarbonate) in 25 min; detector, UV254/220 nm; compound **23-5** (light yellow solid, 75 mg, yield: 52%) was obtained. MS (ESI, *m*/*z*): 621.2/623.2 [M + H]⁺; ¹HNMR (300 MHz, CDCl₃) *δ* 7.72 - 7.68 (m, 2H), 7.39 - 7.31 (m, 2H), 7.26 (d, *J* = 2.4 Hz, 1H), 7.21 - 7.15 (m, 2H), 4.35 - 4.25 (m, 2H), 4.15 - 3.90 (m, 4H), 3.78 - 3.56 (m, 4H), 3.53 - 3.40 (m, 1H), 3.22 - 3.16 (m, 2H), 2.24 - 2.09 (m, 7H), 1.85 - 1.81 (m, 1H), 1.47 (s, 5H), 1.40 (s, 4H).

### Step 7:

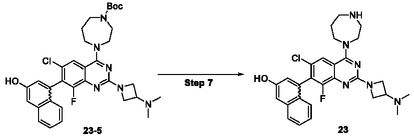

Trifluoroacetic acid (1 mL) was added dropwise to a solution of compound **23-5** (75 mg, 0.12 mmol, 1.00 eq) in dichloromethane (3 mL) with stirring at room temperature. The reaction was carried out for 1 hour at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% (acetonitrile/methanol 1/ 1)/water mobile phase (0.1% ammonium bicarbonate) in 25 min; detector, UV254/220 nm; compound 23 (white solid, 21.0 mg, yield: 35%) was obtained. MS (ESI, m/z): 521.2/523.2 [M + H]⁺; ¹H NMR (300 MHz, DMSO-*d₆*) *δ* 9.99 (s, 1H), 7.90 (d, *J* = 1.7 Hz, 1H), 7.80 (d, *J* = 8.3 Hz, 1H), 7.46 - 7.41 (m, 1H), 7.26 (d, *J* = 2.4 Hz, 1H), 7.23 - 7.21 (m, 2H), 7.04 (d, *J* = 2.4 Hz, 1H), 4.10 - 4.05 (m, 2H), 3.98 - 3.90 (m, 4H), 3.87 - 3.82 (m, 2H), 3.15 - 3.08 (m, 1H), 3.07 - 3.01 (m, 2H), 2.83 - 2.78 (m, 2H), 2.11 (s, 6H), 1.97 - 1.90 (m, 2H); ¹⁹F NMR (282 MHz, DMSO-*d₆*) *δ* -123.51.

The chiral separation conditions of some chiral compounds prepared with reference to the synthetic method of Embodiment 5 (synthesis method IV) were as follows:

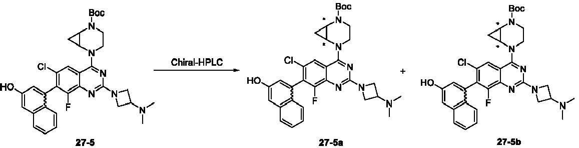

Chiral column: CHIRALPAK ID, 2 × 25 cm, 5 µm; mobile phase A: *n*-hexane/methyl tert-butyl ether = 1/1 (0.5% 2 mol/L ammonia methanol solution), mobile phase B: ethanol; flow rate: 20 mL/min; gradient: elution with 30% phase B in 12 min, detector UV 220 nm. Two products were obtained, the product with shorter retention time (9.14 min) was **27-5a,** *tert*-butyl (1*R*,6*S* or 1*S*,6*R*)-5-((*S* or *R*)-6-chloro-2-(3-(dimethylamino)azetidin-1-yl)-8-fluoro-7-(3-hydroxynaphthalen-1-yl)quinazolin-4-yl)-2,5-diazabicyclo[4.1.0]heptane-2-carboxylate (white solid); the product with longer retention time (11.442 min) was **27-5b,** *tert*-butyl (1*S*,6*R* or 1*R*,6*S*)-5-((*S* or *R*)-6-chloro-2-(3-(dimethylamino)azetidin-1-yl)-8-fluoro-7-(3-hydroxynaphthalen-1-yl)quinazolin-4-yl)-2,5-diazabicyclo[4.1.0]heptan-2-carboxylate (white solid). Compound **27** was obtained from compound **27-5a** after removing the protective groups; compound **28** was obtained from compound **27-5b** after removing protective groups.

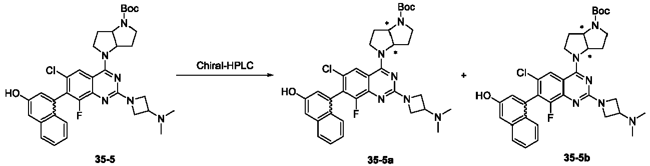

Chiral column: CHIRAL ART Amylose-SA, 2 × 25 cm, 5 µm; mobile phase A: *n-*hexane (10 mmol/L ammonia methanol solution), mobile phase B: isopropanol; flow rate: 20 mL/min; gradient: elution with 40% phase B in 16 min, detector UV 220 nm. Two products were obtained, the product with shorter retention time (6.5 min) was **35-5a,** *tert*-butyl (3a*R*,6a*R* or 3a*S*,6a*S*)-4-((*S* or *R*)-6-chloro-2-(3-(dimethylamino)azetidin-1-yl)-8-fluoro-7-(3-hydroxynaphthalen-1-yl) quinazolin-4-yl)hexahydropyrrolo[3,2-*b*]pyrrole-1(2*H*)-carboxylate (white solid); the product with longer retention time (13.5 min) was **35-5b,** *tert*-butyl (3a*S*,6a*S* or 3a*R*,6a*R*)-4-((*S* or *R*)-6-chloro-2-(3-(dimethylamino)azetidin-1-yl)-8-fluoro-7-(3-hydroxynaphthalen-1-yl)quinazolin-4-yl)hexahydropyrrolo[3,2-*b*]pyrrole-1(2*H*)-carboxylate (white solid). Compound **35** was obtained from compound **35-5a** after removing the protective groups; compound **36** was obtained from compound **35-5b** after removing protective groups.

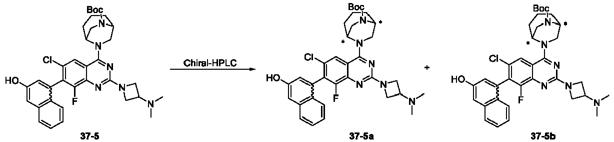

Chiral column: CHIRALPAK ID, 2 × 25 cm, 5 µm; mobile phase A: *n*-hexane (10 mmol/L ammonia methanol solution), mobile phase B: ethanol; flow rate: 20 mL/min; gradient: elution with 30% phase B in 25 min, detector UV 220/254 nm. Two products were obtained. The product with shorter retention time (11.46 min) was **37-5a,** *tert*-butyl (1*S*,5*S* or 1*R*,5*R*)-8-((*S* or *R*)-6-chloro-2-(3-(dimethylamino)azetidin-1-yl)-8-fluoro-7-(3-hydroxynaphthalen-1-yl)quinazolin-4-yl)-6,8-diazabicyclo[3.2.2]nonane-6-carboxylate (white solid); the product with longer retention time (18.52 min) was **37-5b,** *tert*-butyl (1*R*,5*R* or 1*R*,5*R*)*-*8-((*S* or *R*)-6-chloro-2-(3-(dimethylamino)azetidin-1-yl)-8-fluoro-7-(3-hydroxynaphthalen-1-yl)quinazolin-4-yl)-6,8-diazabicyclo[3.2.2]nonane-6-carboxylate (white solid). Compound **37** was obtained from compound **37-5a** after removing the protective groups; compound **38** was obtained from compound **37-5b** after removing protective groups.

Other similar compounds of the present disclosure can be prepared by the synthesis method of Embodiment **5** (synthesis method IV) above. Some compounds prepared with reference to the above synthetic method and their characterization data are shown in table 3.

**Table 3**

| Num ber of the com poun d | Compound structure | Compound name | Mas s spec trum [M+ H]⁺ | ¹H & ¹⁹F NMR |
|---|---|---|---|---|
| **24** | 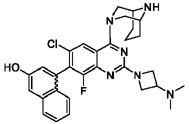 | 4-((*S* or *R*)-4-((1*R*,5*S*)-3,9-diazabicyclo[3.3 .1]nonan-3-yl)-6-chloro-2-(3-(dimethylamino) azetidin-1-yl)-8-fluoroquinazolin -7-yl)naphthalen-2-ol | 547. 2/54 9.2 | ¹H NMR (300 MHz, DMSO-*d₆*) *δ* 10.00 (s, 1H), 7.81 - 7.78 (m, 2H), 7.47 - 7.41 (m, 1H), 7.27 (d, *J* = 2.4 Hz, 1H), 7.25 - 7.22 (m, 2H), 7.04 (d, *J* = 2.4 Hz, 1H), 4.39 - 4.34 (m, 2H), 4.12 - 4.07 (m, 2H), 3.88 - 3.84 (m, 2H), 3.61 - 3.54 (m, 2H), 3.15 - 3.08 (m, 3H), 2.14 - 2.02 (s, 7H), 1.91 - 1.75 (m, 4H), 1.58 - 1.48 (m, 1H); ¹⁹F NMR (282 MHz, DMSO-*d₆*) -123.44. |
| **25** | 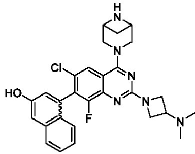 | 4-((7*S* or 7*R*)-4-(3,6-diazabicyclo[3.1 .1]heptan-3-yl)-6-chloro-2-(3-(dimethylamino) azetidin-1-yl)-8-fluoroquinazolin -7-yl)naphthalen-2-ol | 519. 2/52 1.2 | ¹H NMR (400 MHz, DMSO-*d₆*) *δ* 9.99 (s, 1H), 8.28 (d, *J* = 1.6 Hz, 1H), 7.80 (d, *J* = 8.3 Hz, 1H), 7.45 - 7.41 (m, 1H), 7.26 (d, *J* = 2.4 Hz, 1H), 7.25 - 7.18 (m, 2H), 7.04 (d, *J* = 2.4 Hz, 1H), 4.25 - 4.14 (m, 4H), 4.10 - 4.06 (m, 2H), 3.87 - 3.83 (m, 2H), 3.73 - 3.70 (m, 2H), 3.14 - 3.07 (m, 1H), 2.11 (s, 6H), 2.03 - 1.93 (m, 1H), 1.52 - 1.49 (m, 1H); ¹⁹F NMR (377 MHz, DMSO-*d₆*) *δ* - 122.90 |
| **26** | 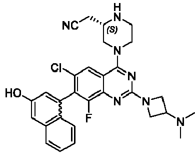 | 2-((*S*)-4-((*S* or *R*)-6-chloro-2-(3-(dimethylamino) azetidin-1-yl)-8-fluoro-7-(3-hydroxynaphtha len-1-yl)quinazolin-4-yl)piperazin-2-yl)acetonitrile | 546. 2/54 8.2 | ¹H NMR (400 MHz, DMSO-*d₆*) *δ* 9.99 (s, 1H), 7.81 - 7.78 (m, 2H), 7.45 - 7.41 (m, 1H), 7.27 (d, *J* = 2.4 Hz, 1H), 7.24 - 7.21 (m, 2H), 7.03 (d, *J* = 2.4 Hz, 1H), 4.20 - 4.03 (m, 4H), 3.90 - 3.86 (m, 2H), 3.31 - 3.25 (m, 1H), 3.18 - 3.10 (m, 2H), 3.05 - 2.98 (m, 2H), 2.90 - 2.83 (m, 1H), 2.72 (d, *J* = 6.0 Hz, 2H), 2.12 (s, 6H); ¹⁹F NMR (377 MHz, DMSO-*d₆*) *δ* -124.45. |
| **27** | 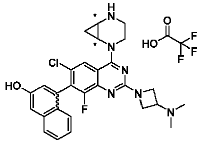 | 4-((*S* or *R*)-4-((1*S*,6*R* or 1*R*,6*S*)-2,5-diazabicyclo[4.1 .0]heptan-2-yl)-6-chloro-2-(3-(dimethylamino) azetidin-1-yl)-8-fluoroquinazolin -7-yl)naphthalen-2-ol trifluoroacetate | 519. 2/52 1.2 | ¹H NMR (300 MHz, DMSO-*d₆*) *δ* 10.02 (s, 1H), 8.63 (d, *J* = 1.7 Hz, 1H), 7.81 (d, *J* = 8.3 Hz, 1H), 7.47 - 7.42 (m, 1H), 7.28 (d, *J* = 2.4 Hz, 1H), 7.26 - 7.16 (m, 2H), 7.04 (d, *J* = 2.4 Hz, 1H), 4.35 - 4.29 (m, 1H), 4.19 - 4.13 (m, 2H), 4.00 - 3.94 (m, 2H), 3.46 - 3.38 (m, 2H), 3.17 - 2.94 (m, 4H), 2.31 (s, 6H), 1.25 -1.16 (m, 2H); ¹⁹F NMR (282 MHz, DMSO-*d₆*) *δ* -73.45, -123.09. |
| **28** | 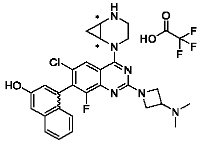 | 4-((*S* or *R*)-4-((1*R*,6*S* or 1*S*, 6*R*)-2,5-diazabicyclo[4.1 .0]heptan-2-yl)-6-chloro-2-(3-(dimethylamino) azetidin-1-yl)-8-fluoroquinazolin -7-yl)naphthalen-2-ol trifluoroacetate | 519. 2/52 1.2 | ¹H NMR (300 MHz, DMSO-*d₆*) *δ* 10.01 (s, 1H), 8.61 (d, *J* = 1.7 Hz, 1H), 7.81 (d, *J* = 8.3 Hz, 1H), 7.47 - 7.41 (m, 1H), 7.28 (d, *J* = 2.4 Hz, 1H), 7.26 - 7.19 (m, 2H), 7.06 (d, *J* = 2.4 Hz, 1H), 4.35 - 4.29 (m, 1H), 4.18 - 4.13 (m, 2H), 4.00 - 3.94 (m, 2H), 3.49 - 3.37 (m, 2H), 3.17 - 2.95 (m, 4H), 2.31 (s, 6H), 1.24 - 1.18 (m, 2H); ¹⁹F NMR (282 MHz, DMSO-*d₆*) *δ* -73.45, -123.25. |
| **29** | 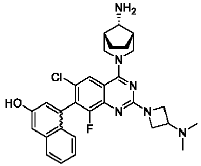 | (*S* or *R*)-4-(4-((1*R*,5*S*,8*R*)-8-amino-3-azabicyclo[3.2.1 ]octan-3-yl)-6-chloro-2-(3-(dimethylamino) azetidin-1-yl)-8-fluoroquinazolin -7-yl)naphthalen-2-ol | 547. 2/54 9.2 | ¹H NMR (300 MHz, DMSO-*d₆*) *δ* 10.01 (s, 1H), 7.83 (d, *J* = 1.6 Hz, 1H), 7.80 (d, *J* = 8.5 Hz, 1H), 7.46 - 7.41 (m, 1H), 7.26 (d, *J* = 2.4 Hz, 1H), 7.24 - 7.19 (m, 2H), 7.04 (d, *J* = 2.4 Hz, 1H), 4.14 - 4.06 (m, 4H), 3.92 - 3.82 (m, 4H), 3.15 - 3.07 (m, 2H), 2.12 (s, 6H), 2.00 - 1.96 (m, 2H), 1.72 - 1.57 (m, 4H); ¹⁹F NMR (282 MHz, DMSO-*d₆*) *δ* -123.64. |
| **30** | 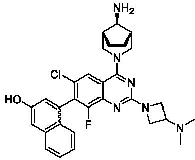 | (*S* or *R*)-4-(4-((1*R*,5*S*,8*S*)-8-amino-3-azabicyclo[3.2.1 ]octan-3-yl)-6-chloro-2-(3-(dimethylamino) azetidin-1-yl)-8-fluoroquinazolin -7-yl)naphthalen-2-ol | 547. 2/54 9.2 | ¹H NMR (400 MHz, CD₃OD) *δ* 7.79 (d, *J* = 1.7 Hz, 1H), 7.73 (d, *J* = 8.3 Hz, 1H), 7.42 - 7.37 (m, 1H), 7.26 - 7.16 (m, 3H), 7.01 (d, *J* = 2.4 Hz, 1H), 4.53 - 4.47 (m, 2H), 4.27 - 4.22 (m, 2H), 4.03 - 3.99 (m, 2H), 3.47 - 3.42 (m, 2H), 3.27 - 3.22 (m, 2H), 2.25 - 2.21 (m, 8H), 1.92 - 1.89 (m, 2H), 1.71 - 1.66 (m, 2H); ¹⁹F NMR (377 MHz, CD₃OD) *δ* - 125.07. |
| **31** | 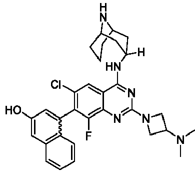 | 4-((*S* or *R*)-4-(((1*R*,3*R*,5*S*)-9-azabicyclo[3.3.1 ]nonan-3-yl)amino)-6-chloro-2-(3-(dimethylamino) azetidin-1-yl)-8-fluoroquinazolin -7-yl)naphthalen-2-ol | 561. 2/56 3.2 | ¹H NMR (300 MHz, DMSO-*d₆*) *δ* 9.98 (s, 1H), 8.31 (s, 1H), 7.82 - 7.77 (m, 2H), 7.46 - 7.40 (m, 1H), 7.26 (d, *J* = 2.4 Hz, 1H), 7.24 - 7.18 (m, 2H), 7.03 (d, *J* = 2.4 Hz, 1H), 4.60 - 4.47 (m, 1H), 4.11 - 4.05 (m, 2H), 3.87 - 3.81 (m, 2H), 3.31 - 3.25 (m, 2H), 3.13 - 3.05 (m, 1H), 2.28 - 2.02 (m, 9H), 1.64 - 1.24 (m, 7H); ¹⁹F NMR (377 MHz, CD₃OD) *δ* - 125.78. |
| **32** | 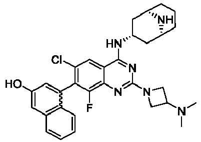 | 4-((*S* or *R*)-4-(((1*R*,3*S*,5*S*)-9-azabicyclo[3.3.1 ]nonan-3-yl)amino)-6-chloro-2-(3-(dimethylamino) azetidin-1-yl)-8-fluoroquinazolin -7-yl)naphthalen-2-ol | 561. 2/56 3.2 | ¹H NMR (300 MHz, DMSO-*d₆*) *δ* 8.35 (d, *J* = 1.5 Hz, 1H), 7.96 (d, *J* = 7.3 Hz, 1H), 7.79 (d, *J* = 8.3 Hz, 1H), 7.45 - 7.40 (m, 1H), 7.26 (d, *J* = 2.4 Hz, 1H), 7.24 - 7.17 (m, 2H), 7.03 (d, *J* = 2.4 Hz, 1H), 5.27 - 5.14 (m, 1H), 4.09 - 4.04 (m, 2H), 3.86 - 3.81 (m, 2H), 3.24 - 3.08 (m, 3H), 2.13 - 1.96 (m, 9H), 1.91 - 1.65 (m, 7H); ¹⁹F NMR (282 MHz, DMSO-*d₆*) *δ* -124.17. |
| **33** | 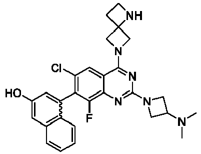 | (*S* or *R*)-4-(6-chloro-2-(3-(dimethylamino) azetidin-1-yl)-8-fluoro-4-(1,6-diazaspiro[3.3]h eptan-6-yl)quinazolin-7-yl)naphthalen-2-ol | 519. 2/52 1.2 | ¹H NMR (300 MHz, DMSO-*d₆*) *δ* 10.01 (s, 1H), 7.79 (d, *J* = 8.3 Hz, 1H), 7.70 (d, *J* = 1.6 Hz, 1H), 7.45 - 7.40 (m, 1H), 7.26 (d, *J* = 2.4 Hz, 1H), 7.25 - 7.15 (m, 2H), 7.03 (d, *J* = 2.4 Hz, 1H), 4.68 - 4.62 (m, 2H), 4.52 - 4.46 (m, 2H), 4.09 - 4.03 (m, 2H), 3.85 - 3.80 (m, 2H), 3.32 - 3.27 (m, 2H), 3.13 - 3.05 (m, 1H), 2.49 - 2.47 (m, 2H), 2.10 (s, 6H); ¹⁹F NMR (282 MHz, DMSO-*d₆*) *δ* - 123.45. |
| **34** | 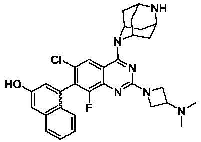 | 4-((*S* or *R*)-4-(2,6-diazaadamantan-2-yl)-6-chloro-2-(3-(dimethylamino) azetidin-1-yl)-8-fluoroquinazolin -7-yl)naphthalen-2-ol | 559. 3/56 1.2 | ¹H NMR (300 MHz, DMSO-*d₆*) *δ* 10.05 (s, 1H), 7.80 (d, *J* = 8.2 Hz, 1H), 7.56 (d, *J* = 1.5 Hz, 1H), 7.46 - 7.41 (m, 1H), 7.27 (d, *J* = 2.4 Hz, 1H), 7.23 - 7.21 (m, 2H), 7.04 (d, *J* = 2.4 Hz, 1H), 4.66 - 4.62 (m, 2H), 4.13 - 4.07 (m, 2H), 3.89 - 3.84 (m, 2H), 3.42 - 3.36 (m, 2H), 3.16 - 3.08 (m, 1H), 2.16 - 1.97 (m, 14H); ¹⁹F NMR (282 MHz, DMSO-*d₆*) *δ* - 123.58. |
| **35** | 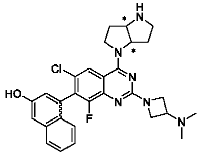 | 4-((*S* or *R*)-6-chloro-2-(3-(dimethylamino) azetidin-1-yl)-8-fluoro-4-((3a*R*,6a*R* or 3a*S*,6a*S*)-hexahydropyrrol o[3,2-*b*]pyrrole-1(2*H*)-yl)quinazolin-7-yl)naphthalen-2-ol | 533. 2/53 5.2 | ¹H NMR (400 MHz, DMSO-*d₆*) *δ* 9.98 (s, 1H), 8.06 (d, *J* = 1.7 Hz, 1H), 7.79 (d, *J* = 8.3 Hz, 1H), 7.45 - 7.41 (m, 1H), 7.26 (d, *J* = 2.4 Hz, 1H), 7.23 -7.16 (m, 2H), 7.03 (d, *J* = 2.4 Hz, 1H), 4.92 - 4.88 (m, 1H), 4.12 - 4.00 (m, 4H), 3.87 - 3.81 (m, 2H), 3.75 - 3.72 (m, 1H), 3.13 - 3.07 (m, 1H), 2.92 - 2.86 (m, 1H), 2.84 - 2.77 (m, 1H), 2.21 - 2.12 (m, 1H), 2.11 (s, 6H), 1.99 - 1.94 (m, 2H), 1.77 - 1.69 (m, 1H); ¹⁹F NMR (377 MHz, DMSO-*d₆*) *δ* - 123.43. |
| **36** | 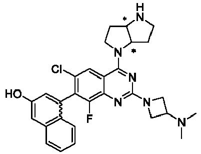 | 4-((*S* or *R*)-6-chloro-2-(3-(dimethylamino) azetidin-1-yl)-8-fluoro-4-((3a*S*,6a*S* or 3a*R*,6a*R*)-hexahydropyrrol o[3,2-*b*]pyrrole-1(2*H*)-yl)quinazolin-7-yl)naphthalen-2-ol | 533. 2/53 5.2 | ¹H NMR (400 MHz, DMSO-*d₆*) *δ* 9.97 (s, 1H), 8.06 (d, *J* = 1.6 Hz, 1H), 7.79 (d, *J* = 8.3 Hz, 1H), 7.45 - 7.41 (m, 1H), 7.26 (d, *J* = 2.4 Hz, 1H), 7.24 - 7.20 (m, 2H), 7.03 (d, *J* = 2.4 Hz, 1H), 4.92 - 4.88 (m, 1H), 4.09 - 4.02 (m, 4H), 3.87 - 3.81 (m, 2H), 3.76 - 3.72 (m, 1H), 3.13 - 3.07 (m, 1H), 2.93 - 2.87 (m, 1H), 2.84 - 2.77 (m, 1H), 2.22 - 2.13 (m, 1H), 2.11 (s, 6H), 2.00 - 1.95 (m, 2H), 1.77 - 1.70 (m, 1H); ¹⁹F NMR (377 MHz, DMSO-*d₆*) *δ* - 123.59. |
| **37** | 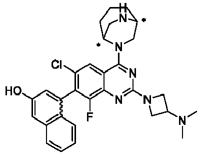 | 4-((*S* or *R*)-4-((1*S*,5*S* or 1*R*,5*R*)-6,8-diazabicyclo[3.2 .2]nonan-6-yl)-6-chloro-2-(3-(dimethylamino) azetidin-1-yl)-8-fluoroquinazolin -7-yl)naphthalen-2-ol | 547. 3/54 9.2 | ¹H NMR (300 MHz, DMSO-*d*₆) *δ* 9.98 (s, 1H), 7.99 (s, 1H), 7.80 (d, *J* = 8.3 Hz, 1H), 7.49 - 7.40 (m, 1H), 7.33 - 7.18 (m, 3H), 7.04 (d, *J* = 2.4 Hz, 1H), 4.66 - 4.56 (m, 1H), 4.40 - 4.28 (m, 1H), 4.16 - 3.99 (m, 2H), 3.96 - 3.76 (m, 3H), 3.50 - 3.38 (m, 2H), 3.17 - 3.02 (m, 2H), 2.11 (s, 6H), 1.93 - 0.82 (m, 6H); ¹⁹F NMR (282 MHz, DMSO-*d*₆) *δ* - 123.63. |
| **38** | 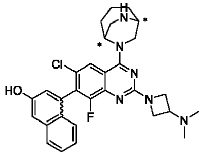 | 4-((*S* or *R*)-4-((1*R*,5*R* or 1*S*,5*S*)-6,8-diazabicyclo[3.2 .2]nonan-6-yl)-6-chloro-2-(3-(dimethylamino) azetidin-1-yl)-8-fluoroquinazolin -7-yl)naphthalen-2-ol | 547. 3/54 9.2 | ¹H NMR (300 MHz, DMSO-*d*₆) *δ* 10.01 (s, 1H), 8.06 - 7.97 (m, 1H), 7.80 (d, *J* = 8.3 Hz, 1H), 7.49 - 7.39 (m, 1H), 7.30 - 7.12 (m, 3H), 7.02 (d, *J* = 2.4 Hz, 1H), 4.59 (d, *J* = 6.7 Hz, 1H), 4.39 - 4.29 (m, 1H), 4.16 - 3.98 (m, 2H), 3.93 - 3.76 (m, 3H), 3.42 - 3.36 (m, 1H), 3.29 - 3.35 (m, 1H), 3.16 - 3.06 (m, 1H), 3.04 - 2.94 (m, 1H), 2.11 (s, 6H), 1.93 - 1.04 (m, 6H); ¹⁹F NMR (282 MHz, DMSO-*d*₆) *δ* -123.35. |

### Embodiment 6 (Synthesis method V)

### (S or R)-4-(4-((1R,5S)-3,8-diazabicyclo[3.2.1]octan-3-yl)-6-chloro-8-fluoro-2-((S)-1-methylpyrrolidin-2-yl)methoxy)quinazolin-7-yl)naphthalen-2-ol 39a; (R or S)-4-(4-((1R,5S)-3,8-diazabicyclo[3.2.1]octan-3-yl)-6-chloro-8-fluoro-2-((S)-1-methylpyrrolidin-2-yl)methoxy)quinazolin-7-yl)naphthalen-2-ol 39b

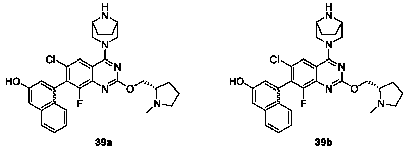

The synthetic route was as follows:

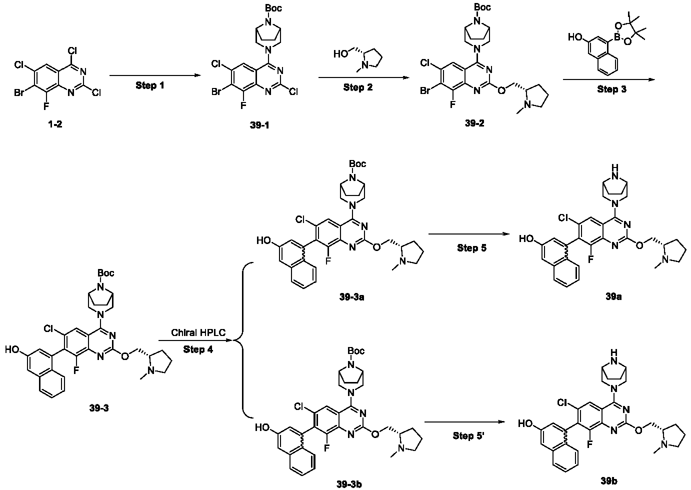

### Step 1:

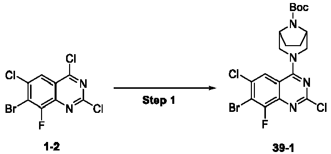

Compound **1-2** (8 g, 23.00 mmol, 1.00 eq) was dissolved in 80 mL of dichloromethane with stirring under the protection of nitrogen at 25 °C. Triethylamine (7.35 g, 69.00 mmol, 3.0 eq) and *tert*-butyl (1*R*,5*S*)-3,8-diazabicyclo[3.2.1]octane-8-carboxylate (5.14 g, 23.00 mmol, 1.00 eq) were successively added to the solution, and then the reaction was carried out at 25 °C for 1 hour, the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% ethyl acetate/dichloromethane mobile phase, and the obtained fractions were evaporated under reduced pressure to remove the solvent to obtain compound **39-1** (white solid, 10.00 g, yield: 81%). MS (ESI, *m*/*z*): 505.0/507.0/509.0 [M + H]⁺; ¹H NMR (400 MHz, CDCl₃) *δ* 7.75 (d, *J* = 2.0 Hz, 1H), 4.45 - 4.33 (m, 4H), 3.72 - 3.56 (m, 2H), 1.98 - 1.94 (m, 2H), 1.75 - 1.68 (m, 2H), 1.52 (s, 9H).

### Step 2:

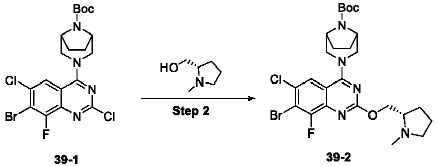

*N*-methyl-*L*-proline (238 mg, 1.96 mmol, 1.5 eq) and potassium carbonate (497 mg, 3.42 mmol, 2.6 eq) were added to a solution of compound **39-1** (700 mg, 1.31 mmol, 1.00 eq) in acetonitrile (5.0 mL) under the protection of nitrogen at 25 °C. The reaction was carried out for 16 hours at 80 °C under nitrogen atmosphere, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **39-2** (white solid, 400 mg, yield: 49%). MS (ESI, m/z): 584.3/586.3/588.3 [M + H]⁺; ¹H NMR (400 MHz, CDCl₃) *δ* 7.69 (d, *J* = 2.0 Hz, 1H), 4.55 - 4.51 (m, 1H), 4.37 - 4.27 (m, 5H), 3.61 - 3.52 (m, 2H), 3.15 - 3.10 (m, 1H), 2.78 - 2.72 (m, 1H), 2.52 (s, 3H), 2.35 - 2.27 (m, 1H), 2.11 - 2.02 (m, 1H), 1.96 - 1.75 (m, 7H), 1.52 (s, 9H).

### Step 3:

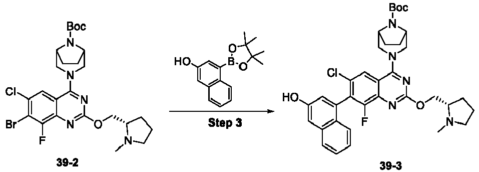

4-(4,4,5,5-Tetramethyl-1,3,2-dioxaborolan-2-yl)naphthalen-2-ol (277 mg, 1.03 mmol, 1.5 eq), potassium phosphate (276 mg, 1.30 mmol, 2.0 eq) and chloro(2-dicyclohexylphosphino-2',4',6'-triisoporpyl-1,1'-biphenyl) [2-(2'-amino-1,1'-biphenyl)] palladium (II) (47 mg, 0.06 mmol, 0.10 eq) were added to a solution of compound **39-2** (400 mg, 0.65mmol, 1.0 eq) in tetrahydrofuran/water (10/1, 4 mL) with stirring under the protection of nitrogen at 25 °C. The reaction was carried out with stirring for 2 hours at 60 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **39-3** (a mixture of two stereoisomers, yellow solid, 180 mg, yield: 41%). MS (ESI, m/z): 648.3/650.2 [M + H]⁺; ¹H NMR (400 MHz, DMSO-*d*₆) *δ* 10.10 (s, 1H), 8.00 (s, 1H), 7.80 (d, *J* = 8.3 Hz, 1H), 7.46 - 7.42 (m, 1H), 7.29 (d, *J* = 2.4 Hz, 1H), 7.27 - 7.18 (m, 2H), 7.07 (d, *J* = 2.4 Hz, 1H), 4.48 - 4.33 (m, 3H), 4.29 - 4.25 (m, 2H), 4.20 - 4.14 (m, 1H), 3.63 - 3.54 (m, 2H), 2.96 - 2.92 (m, 1H), 2.61 - 2.54 (m, 1H), 2.35 (s, 3H), 2.20 - 2.13 (m, 1H), 1.97 - 1.90 (m, 1H), 1.85 - 1.70 (m, 4H), 1.70 - 1.60 (m, 3H), 1.47 (s, 9H).

### Step 4:

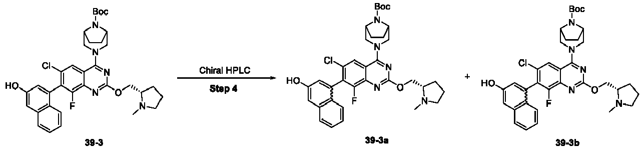

The compound **39-3** (180 mg) obtained in step 3 was subjected to chiral resolution, and the resolution conditions were: chiral column NB-Lux 5 µm i-Cellulose-5, 2.12 × 25 cm, 5 µm; mobile phase A: n-hexane (10 mmol/L ammonia methanol solution), mobile phase B: ethanol; flow rate: 20 mL/min; elution with 20% mobile phase B in 25 min; detector UV 220/254 nm; two products were obtained. The product with shorter retention time (11.7 min) was **39-3a,** *tert*-butyl (1*R*,5*S*)-3-(6-chloro-8-fluoro-7-((*S* or *R*)-3-hydroxynaphthalen-1-yl)-2-((*S*)-1-methylpyrrolidin-2-yl)methoxy)quinazolin-4-yl)-3,8-diazabicyclo[3.2.1]octane-8-carboxylate (white solid, 80 mg, recovery rate: 42%), the product with longer retention time (21.18 min) was compound **39-3b,** *tert*-butyl (1*R*,5*S*)-3-(6-chloro-8-fluoro-7-((*R* or *S*)-3-hydroxynaphthalen-1-yl)-2-((*S*)-1-methylpyrrolidin-2-yl)methoxy)quinazoline-4-yl)-3,8-diazabicyclo[3.2.1]octane-8-carboxylate (white solid, 70 mg, recovery rate: 37% ).

The chiral resolution methods of some similar chiral compounds, the retention times thereof and the ee values thereof in the present disclosure are shown in the following table 4 respectively.

**Table 4**

| Nu mbe r of the com pou nd | Compound structure | Compound name | Mas s spec trum [M+ H]+ | Chiral resolution conditions/retention time/ee value |
|---|---|---|---|---|
| **40-3a** | 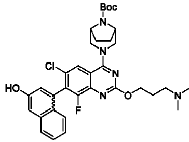 | *tert-*Butyl (1*R*,5*S*)-3-(6-chloro-2-(3-(dimethylamino)pro poxy)-8-fluoro-7-((*S* or *R*)-3-hydroxynaphthalen-1-yl)quinazolin-4-yl)-3,8-diazabicyclo[3.2.1]o ctane-8-carboxylate | 636. 2/63 8.2 | Chiral column: CHIRAL ART Cellulose-SC, 2 x 25 cm, 5 µm; mobile phase A: *n*-hexane (10 mmol/L ammonia methanol solution), mobile phase B: ethanol; flow rate: 20 mL/min; gradient: elution with 30% phase B in 15 min, detector UV 220/254 nm, retention time: 6.780 min; ee > 99%. |
| **40-3b** | 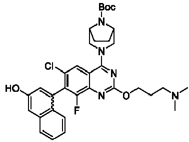 | *tert*-Butyl (1*R*,5*S*)-3-(6-chloro-2-(3-(dimethylamino)pro poxy)-8-fluoro-7-((*R* or *S*)-3-hydroxynaphthalen-1-yl)quinazolin-4-yl)-3,8-diazabicyclo[3.2.1]o ctane-8-carboxylate | 636. 2/63 8.2 | Chiral column: CHIRAL ART Cellulose-SC, 2 x 25 cm, 5 µm; mobile phase A: *n*-hexane (10 mmol/L ammonia methanol solution), mobile phase B: ethanol; flow rate: 20 mL/min; gradient: elution with 30% phase B in 15 min, detector UV 220/254 nm, retention time: 10.525 min; ee > 99%. |
| **42-3a** | 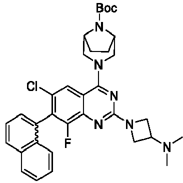 | *tert*-Butyl (1*R*,5*S*)-3-((*S* or *R*)-6-chloro-2-(3-(dimethylamino)azet idin-1-yl)-8-fluoro-7-(naphthalen-1-yl)quinazolin-4-yl)-3,8-diazabicyclo[3.2.1]o ctane-8-carboxylate | 617. 3/61 9.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: isopropanol; flow rate: 20 mL/min; gradient: elution with 10% phase B in 11.5 min, detector UV 220/254 nm, retention time: 7.655 min; ee > 99%. |
| **42-3b** | 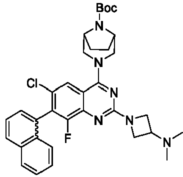 | *tert*-Butyl (1*R*,5*S*)-3-((*R* or *S*)-6-chloro-2-(3-(dimethylamino)azet idin-1-yl)-8-fluoro-7-(naphthalen-1-yl)quinazolin-4-yl)-3,8-diazabicyclo[3.2.1]o ctane-8-carboxylate | 617. 3/61 9.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: isopropanol; flow rate: 20 mL/min; gradient: elution with 10% phase B in 11.5 min, detector UV 220/254 nm, retention time: 9.235 min; ee > 99%. |
| **43-3a** | 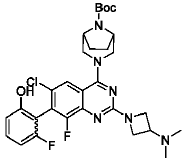 | *tert*-Butyl (1*R*,5*S*)-3-((*S* or *R*)-6-chloro-2-(3-(dimethylamino)azet idin-1-yl)-8-fluoro-7-(2-fluoro-6-hydroxyphenyl)quin azolin-4-yl)-3,8-diazabicyclo[3.2.1]o ctane-8-carboxylate | 601. 3/60 3.3 | Chiral column: CHIRALPAK IE, 2 x 25 cm, 5 µm; mobile phase A: *n*-hexane ( 10 mmol/L ammonia methanol solution), mobile phase B: ethanol; flow rate: 20 mL/min; gradient: elution with 30% phase B in 31 min, detector UV 210/265 nm, retention time: 8.25 min; ee > 99%. |
| **43-3b** | 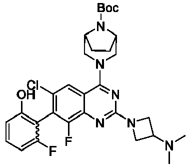 | *tert*-Butyl (1*R*,5*S*)-3-((*R* or *S*)-6-chloro-2-(3-(dimethylamino)azet idin-1-yl)-8-fluoro-7-(2-fluoro-6-hydroxyphenyl)quin azolin-4-yl)-3,8-diazabicyclo[3.2.1]o ctane-8-carboxylate | 601. 3/60 3.3 | Chiral column: CHIR ALPAK IE, 2 x 25 cm, 5 µm; mobile phase A: *n*-hexane (10 mmol/L ammonia methanol solution), mobile phase B: ethanol; flow rate: 20 mL/min; gradient: elution with 30% phase B in 31 min, detector UV 210/265 nm, retention time: 12.09 min; ee > 99%. |
| **44-3a** | 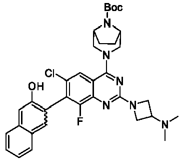 | *tert*-Butyl (1*R*,5*S*)-3-((*S* or *R*)-6-chloro-2-(3-(dimethylamino)azet idin-1-yl)-8-fluoro-7-(3-hydroxynaphthalen-2-yl)quinazolin-4-yl)-3,8-diazabicyclo[3.2.1]o ctane-8-carboxylate | 633. 3/63 5.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL /min; gradient: elution with 30% phase B in 21min; detector UV 210/265 nm; retention time:10.085 min; ee > 97%. |
| **44-3b** | 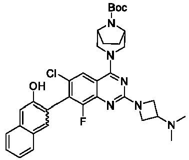 | *tert*-Butyl (1*R*,5*S*)-3-((*R* or *S*)-6-chloro-2-(3-(dimethylamino)azet idin-1-yl)-8-fluoro-7-(3-hydroxynaphthalen-2-yl)quinazolin-4-yl)-3,8-diazabicyclo[3.2.1]o ctane-8-carboxylate | 633. 3/63 5.2 | Chiral column: NB-Lux 5 µm i-Cellulose-5, 2.12 x 25 cm, 5 µm; mobile phase A: *n*-hexane (10 mmol/L ammonia methanol solution), mobile phase B: ethanol; flow rate: 20 mL/min; gradient: elution with 30% phase B in 21min; detector UV 210/265 nm; retention time:17.370 min; ee > 94%. |
| **45-3a** | 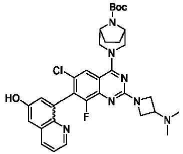 | *tert*-Butyl (1*R*,5*S*)-3-((*S* or *R*)-6-chloro-2-(3-(dimethylamino)azet idin-1-yl)-8-fluoro-7-(6-hydroxyquinolin-8-yl)quinazolin-4-yl)-3,8-diazabicyclo[3.2.1]o ctane-8-carboxylate | 634. 4/63 6.3 | Chiral column: CHIR ALPAK IE, 2 x 25 cm, 5 µm; mobile phase A: methyl *tert*-butyl ether (10 mmol/L ammonia methanol solution), mobile phase B: methanol; flow rate: 20 mL/min; gradient: elution with 50% phase B in 15 min, detector UV 240/210 nm, retention time: 3.930 min; ee > 99%. |
| **45-3b** | 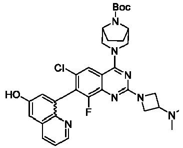 | *tert*-Butyl (1*R*,5*S*)-3-((*R* or *S*)-6-chloro-2-(3-(dimethylamino)azet idin-1-yl)-8-fluoro-7-(6-hydroxyquinolin-8-yl)quinazolin-4-yl)-3,8-diazabicyclo[3.2.1]octane-8-carboxylate | 634. 3/63 6.3 | Chiral column: CHIRALPAK IE, 2 x 25 cm, 5 µm; mobile phase A: methyl *tert*-butyl ether (10 mmol/L ammonia methanol solution), mobile phase B: methanol; flow rate: 20 mL/min; gradient: elution with 50% phase B in 15 min, detector UV 240/210 nm, retention time: 11.471 min; ee > 99%. |
| **46-3a** | 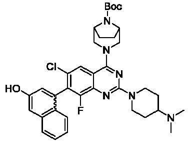 | *tert*-Butyl (1*R*,5*S*)-3-((*S* or *R*)-6-chloro-2-(4-(dimethylamino)pipe ridin-1-yl)-8-fluoro-7-(3-hydroxynaphthalen-1-yl)quinazolin-4-yl)-3,8-diazabicyclo[3.2.1]o ctane-8-carboxylate | 661. 4/66 3.4 | Chiral column: NB_Lux 5 µm i-Cellulose-5, 2.12 x 25 cm, 5 µm; mobile phase A: *n-*hexane/dichloromethane = 5/1 (0.1% diethylamine), mobile phase B: isopropanol; flow rate: 20 mL/min; gradient: elution with 15% phase B in 21 min, detector UV 254 nm, retention time: 10.635 min; ee > 99%. |
| **46-3b** | 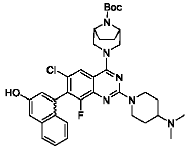 | *tert*-Butyl (1*R*,5*S*)-3-((*R* or *S*)-6-chloro-2-(4-(dimethylamino)pipe ridin-1-yl)-8-fluoro-7-(3-hydroxynaphthalen-1-yl)quinazolin-4-yl)-3,8-diazabicyclo[3.2.1]o ctane-8-carboxylate | 661. 4/66 3.4 | Chiral column: NB_Lux 5 µm i-Cellulose-5, 2.12 x 25 cm, 5 µm; mobile phase A: *n-*hexane/dichloromethane = 5/1 (0.1% diethylamine), mobile phase B: isopropanol; flow rate: 20 mL/min; gradient: elution with 15% phase B in 21 min, detector UV 254 nm, retention time: 16.912 min; ee > 99%. |
| **47-3a** | 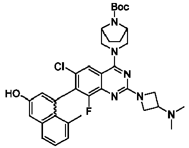 | *tert*-Butyl (1*R*,5*S*)-3-((*S* or *R*)-6-chloro-2-(3-(dimethylamino)azet idin-1-yl)-8-fluoro-7-(3-hydroxy-8-methylnaphthalen-1-yl)quinazolin-4-yl)-3,8-diazabicyclo[3.2.1]o ctane-8-carboxylate | 647. 3/64 9.3 | Chiral column: NB-Lux 5µm i-Cellulose-5, 2.12 x 25cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 22 min, detector UV 254/220 nm, retention time: 9.675 min; ee > 99%. |
| **47-3b** | 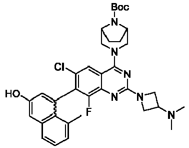 | *tert*-Butyl (1*R*,5*S*)-3-((*R* or *S*)-6-chloro-2-(3-(dimethylamino)azet idin-1-yl)-8-fluoro-7-(3-hydroxy-8-methylnaphthalen-1-yl)quinazolin-4-yl)-3,8-diazabicyclo[3.2.1]o ctane-8-carboxylate | 647. 3/64 9.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 22 min, detector UV 254/220 nm, retention time: 17.035 min; ee > 99%. |
| **48-3a** | 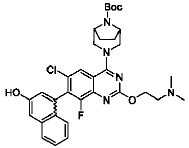 | *tert*-Butyl (1*R*,5*S*)-3-((*S* or *R*)-6-chloro-2-(2-(dimethylamino)etho xy)-8-fluoro-7-(3-hydroxynaphthalen-1-yl)quinazolin-4-yl)-3,8-diazabicyclo[3.2.1]o ctane-8-carboxylate | 622. 3/62 4.2 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 25 min, detector UV 220 nm, retention time: 12.215 min; ee > 99%. |
| **48-3b** | 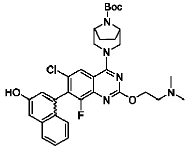 | *tert-*Butyl (1*R*,5*S*)-3-((*R* or *S*)-6-chloro-2-(2-(dimethylamino)etho xy)-8-fluoro-7-(3-hydroxynaphthalen-1-yl)quinazolin-4-yl)-3,8-diazabicyclo[3.2.1]o ctane-8-carboxylate | 622. 3/62 4.2 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 25 min, detector UV 220 nm, retention time: 18.367 min; ee > 99%. |
| **49-3a** | 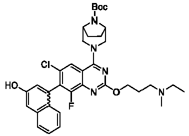 | *tert-*Butyl (1*R,*5*S*)-3-((S or *R*)-6-chloro-2-(3-(ethyl(methyl)amino )propoxy)-8-fluoro-7-(3-hydroxynaphthalen-1-yl)quinazolin-4-yl)-3,8-diazabicyclo[3.2.1]o ctane-8-carboxylate | 650. 3/65 2.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 30% phase B in 11 min, detector UV 220 nm, retention time: 5.595 min; ee > 99%. |
| **49-3b** | 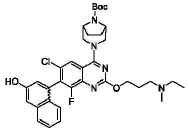 | *tert-*Butyl (1*R,*5*S*)*-3-*((*R* or *S*)-6-chloro-2-(3-(ethyl(methyl)amino )propoxy)-8-fluoro-7-(3-hydroxynaphthalen-1-yl)quinazolin-4-yl)-3,8-diazabicyclo[3.2.1]o ctane-8-carboxylate | 650. 3/65 2.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 30% phase B in 11 min, detector UV 220 nm, retention time: 8.273 min; ee > 99%. |
| **50-3a** | 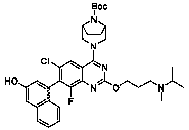 | *tert-*Butyl (1*R,*5*S*)*-3-*((*S* or *R*)-6-chloro-8-fluoro-7-(3-hydroxynaphthalen-1-yl)-2-(3-(isopropyl(methyl)a mino)propoxy)quina zolin-4-yl)-3,8-diazabicyclo[3.2.1]o ctane-8-carboxylate | 664. 4/66 6.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 17 min, detector UV 220 nm, retention time: 6.202 min; ee > 99%. |
| **50-3b** | 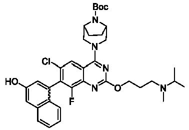 | *tert-*Butyl (1*R,*5*S*)-3-((*R* or *S*)-6-chloro-8-fluoro-7-(3-hydroxynaphthalen-1-yl)-2-(3-(isopropyl(methyl)a mino)propoxy)quina zolin-4-yl)-3,8-diazabicyclo[3.2.1]o ctane-8-carboxylate | 664. 4/66 6.3 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 17 min, detector UV 220 nm, retention time: 13.190 min; ee > 99% |
| **51-3a** | 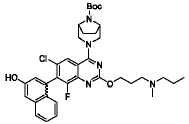 | *tert-*Butyl (1*R,*5*S*)*-*3-((*S* or *R*)-6-chloro-8-fluoro-7-(3-hydroxynaphthalen-1-yl)-2-(3-(methyl(propyl))ami no)propoxy)quinazol in-4-yl)-3,8-diazabicyclo[3.2.1]o ctane-8-carboxylate | 664. 3/66 6.3 | Chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 18 mL/min; gradient: elution with 10% phase B in 12 min, detector UV 220 nm, retention time: 4.856 min; ee > 99% |
| **51-3b** | 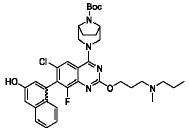 | *tert-*Butyl (1*R,*5*S*)-3-((*R* or *S*)-6-chloro-8-fluoro-7-(3-hydroxynaphthalen-1-yl)-2-(3-(methyl(propyl))ami no)propoxy)quinazol in-4-yl)-3,8-diazabicyclo[3.2.1]o ctane-8-carboxylate | 664. 3/66 6.3 | Chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 18 mL/min; gradient: elution with 10% phase B in 12 min, detector UV 220 nm, retention time: 7.205 min; ee > 99% |
| **52-3a** | 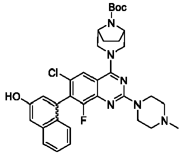 | *tert-*Butyl (1*R,*5*S*)-3-((*S* or *R*)-6-chloro-8-fluoro-7-(3-hydroxynaphthalen-1-yl)-2-(4-methylpiperazin-1-yl)quinazolin-4-yl)-3,8-diazabicyclo[3.2.1]o ctane-8-carboxylate | 633. 4/63 5.4 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 17 min, detector UV 220 nm, retention time: 9.275 min; ee > 99% |
| **52-3b** | 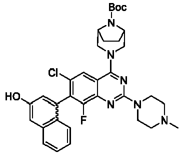 | *tert-*Butyl (1*R,*5*S*)-3-((*R* or *S*)-6-chloro-8-fluoro-7-(3-hydroxynaphthalen-1-yl)-2-(4-methylpiperazin-1-yl)quinazolin-4-yl)-3,8-diazabicyclo[3.2.1]o ctane-8-carboxylate | 633. 4/63 5.4 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 17 min, detector UV 220 nm, retention time: 12.827 min; ee > 99% |
| **53-3a** | 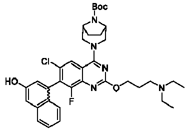 | *tert-*Butyl (1*R*,5*S*)-3-((*S* or *R*)-6-chloro-2-(3-(diethylamino)propo xy)-8-fluoro-7-(3-hydroxynaphthalen-1-yl)quinazolin-4-yl)-3,8-diazabicyclo[3.2.1]o ctane-8-carboxylate | 664. 4/66 6.4 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 20% phase B in 18 min, detector UV 220 nm, retention time: 5.910 min; ee > 99% |
| **53-3b** | 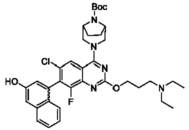 | *tert-*Butyl (1*R,*5*S*)*-*3-((*R* or *S*)-6-chloro-2-(3-(diethylamino)propo xy)-8-fluoro-7-(3-hydroxynaphthalen-1-yl)quinazolin-4-yl)-3,8-diazabicyclo[3.2.1]o ctane-8-carboxylate | 664. 4/66 6.4 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 20% phase B in 18 min, detector UV 220 nm, retention time: 12.225 min; ee > 99% |
| **54-3a** | 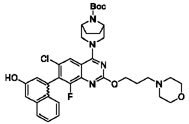 | *tert-*Butyl (1*R*,5*S*)-3-((*S* or *R*)-6-chloro-8-fluoro-7-(3-hydroxynaphthalen-1-yl)-2-(3-morpholinopropoxy) quinazolin-4-yl)-3,8-diazabicyclo[3.2.1]o ctane-8-carboxylate | 678. 3/68 0.3 | Chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 18 mL/min; gradient: elution with 30% phase B in 11 min, detector UV 220 nm, retention time: 5.015 min; ee > 99% |
| **54-3b** | 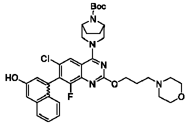 | *tert-*Butyl (1*R*,5*S*)-3-((*R* or S)-6-chloro-8-fluoro-7-(3-hydroxynaphthalen-1-yl)-2-(3-morpholinopropoxy) quinazolin-4-yl)-3,8-diazabicyclo[3.2.1]o ctane-8-carboxylate | 678. 3/68 0.3 | Chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 18 mL/min; gradient: elution with 30% phase B in 11 min, detector UV 220 nm, retention time: 7.837 min; ee > 99% |
| **55-3a** | 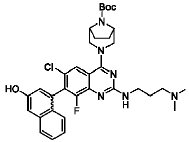 | *tert-*Butyl (1*R*,5*S*)-3-(6-chloro-2-((3-(dimethylamino)pro pyl)amino)-8-fluoro-7-((S or *R*)*-3-*hydroxynaphthalen-1-yl)quinazolin-4-yl)-3,8-diazabicyclo[3.2.1]o ctane-8-carboxylate | 635. 2/63 7.2 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 26 min, detector UV 220/254 nm, retention time: 11.8565 min; ee > 99% |
| **55-3b** | 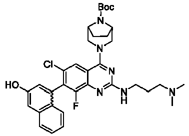 | *tert-*Butyl (1*R,*5*S*)-3-(6-chloro-2-((3-(dimethylamino)pro pyl)amino)-8-fluoro-7-((*R* or *S*)-3-hydroxynaphthalen-1-yl)quinazolin-4-yl)-3,8-diazabicyclo[3.2.1]o ctane-8-carboxylate | 635. 2/63 7.2 | Chiral column: NB-Lux 5µm i-Cellulose-5, 2.12 x 25cm, 5µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 26 min, detector UV 220/254 nm, retention time: 20.375 min; ee > 99% |
| **56-3a** | 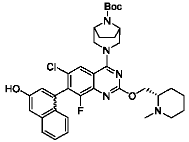 | *tert-*Butyl (1*R,*5*S*)-3-((*S* or *R*)-6-chloro-8-fluoro-7-(3-hydroxynaphthalen-1-yl)-2-((*S*)-1-methylpiperidin-2-yl)methoxy)quinazolin-4-yl)-3,8-diazabicyclo[3.2.1]o ctane-8-carboxylate | 662. 3/66 4.4 | chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 12 min, detector UV 220 nm, retention time: 4.7625 min; ee > 99% |
| **56-3b** | 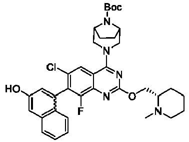 | *tert-*Butyl (1*R*,5*S*)-3-((*R* or *S*)-6-chloro-8-fluoro-7-(3-hydroxynaphthalen-1-yl)-2-((*S*)-1-methylpiperidin-2-yl)methoxy)quinazol in-4-yl)-3,8-diazabicyclo[3.2.1]o ctane-8-carboxylate | 662. 3/66 4.4 | chiral column: NB-Lux 5µm i-Cellulose-5, 2.12 x 25cm, 5µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 12 min, detector UV 220 nm, retention time: 7.7925 min; ee > 99% |
| **57-3a** | 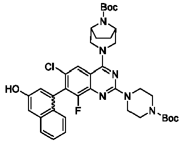 | *tert-*Butyl (1*R*,5*S*)-3-((*S* or *R*)*-*2-(4-(*tert-*butoxycarbonyl)pipe razin-1 -yl)-6-chloro-8-fluoro-7-(3-hydroxynaphthalen-1-yl)quinazolin-4-yl)-3,8-diazabicyclo[3.2.1]o ctane-8-carboxylate | 719. 4/72 1.4 | Chiral column: CHIRALPAK ID, 2x25 cm, 5 µm; mobile phase A: n-hexane/dichloromethane = 5/1 (0.5% 2 mol/L ammonia methanol solution), mobile phase B: isopropanol; flow rate: 20 mL/min; gradient: elution with 10% phase B in 35 min, detector UV 220 nm, retention time: 15.82 min; ee > 99% |
| **57-3b** | 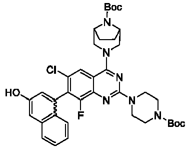 | *tert-*Butyl (1*R*,5*S*)-3-((*R* or *S*)-2-(4-(*tert-*butoxycarbonyl)pipe razin-1 -yl)-6-chloro-8-fluoro-7-(3-hydroxynaphthalen-1-yl)quinazolin-4-yl)-3,8-diazabicyclo[3.2.1]o ctane-8-carboxylate | 719. 4/72 1.3 | Chiral column: CHIRALPAK ID, 2 x 25 cm, 5 µm; mobile phase A: n-hexane/dichloromethane = 5/1 (0.5% 2 mol/L ammonia methanol solution), mobile phase B: isopropanol; flow rate: 20 mL/min; gradient: elution with 10% phase B in 35 min, detector UV 220 nm, retention time: 23.44 min; ee > 99% |
| **58-3a** | 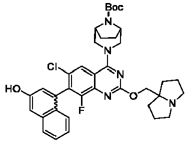 | *tert*-Butyl (1*R,*5*S*)-3-((*S* or *R*)-6-chloro-8-fluoro-7-(3-hydroxynaphthalen-1-yl)-2-((tetrahydro-1*H*-pyrrolin-7*α*(5*H*)-yl)methoxy)quinazol in-4-yl)-3,8-diazabicyclo[3.2.1]o ctane-8-carboxylate | 674. 3/67 6.3 | Chiral column: NB-Lux 5 µm i-Cellulose-5, 2.12 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 17 min, detector UV 220 nm, retention time: 6.3925 min; ee > 99% |
| **58-3b** | 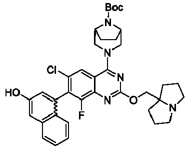 | *tert-*Butyl (1*R*,5*S*)-3-((*R* or *S*)-6-chloro-8-fluoro-7-(3-hydroxynaphthalen-1-yl)-2-((tetrahydro-1*H*-pyrrolin-7*α*(5*H*)-yl)methoxy)quinazol in-4-yl)-3,8-diazabicyclo[3.2.1]o ctane-8-carboxylate | 674. 3/67 6.3 | Chiral column: NB-Lux 5 µm i-Cellulose-5, 2.12 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 17 min, detector UV 220 nm, retention time: 11.442 min; ee > 99% |
| **59-3a** | 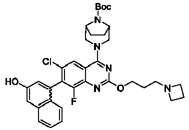 | *tert-*Butyl (1*R*,5*S*)-3-((*S* or *R*)-2-(3-(azetidin-1-yl)propoxy)-6-chloro-8-fluoro-7-(3-hydroxynaphthalen-1-yl)quinazolin-4-yl)-3,8-diazabicyclo[3.2.1]o ctane-8-carboxylate | 648. 3/65 0.3 | Chiral column: NB-Lux 5 µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 22 min, detector UV 225/ 220 nm, retention time: 8.47 min; ee > 99% |
| **59-3b** | 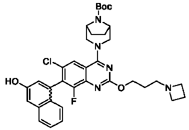 | *tert-*Butyl (1*R,*5*S*)-*3-*((*R* or *S*)-2-(3-(azetidin-1-yl)propoxy)-6-chloro-8-fluoro-7-(3-hydroxynaphthalen-1-yl)quinazolin-4-yl)-3,8-diazabicyclo[3.2.1]o ctane-8-carboxylate | 648. 3/65 0.3 | Chiral column: NB-Lux 5 µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 22 min, detector UV 225/220 nm, retention time: 15.82 min; ee > 98% |
| **60-3a** | 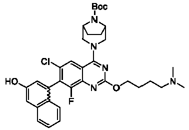 | *tert-*Butyl (1*R,*5*S*)-3-((*S* or *R*)-6-chloro-2-(4-(dimethylamino)buto xy)-8-fluoro-7-(3-hydroxynaphthalen-1-yl)quinazolin-4-yl)-3,8-diazabicyclo[3.2.1]o ctane-8-carboxylate | 650. 4/65 2.3 | Chiral column: NB-Lux 5 µm i-Cellulose-5, 2.12 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 22 min, detector UV 220 nm, retention time: 5.815 min; ee > 92% |
| **60-3b** | 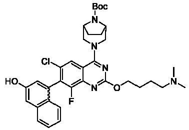 | *tert*-Butyl (1*R,*5*S*)-3-((*R* or *S*)-6-chloro-2-(4-(dimethylamino)buto xy)-8-fluoro-7-(3-hydroxynaphthalen-1-yl)quinazolin-4-yl)-3,8-diazabicyclo[3.2.1]o ctane-8-carboxylate | 650. 4/65 2.3 | Chiral column: NB-Lux 5 µm i-Cellulose-5, 2.12 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 22 min, detector UV 220 nm, retention time: 11.63 min; ee > 99% |
| **61-3a** | 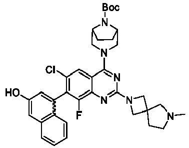 | *tert-*Butyl (1*R,*5*S*)-3-((*S* or *R*)-6-chloro-8-fluoro-7-(3-hydroxynaphthalen-1-yl)-2-(6-methyl-2,6-diazaspiro[3.4]octan-2-yl)quinazolin-4-yl)-3,8-diazabicyclo[3.2.1]o ctane-8-carboxylate | 659. 3/66 1.3 | Chiral column: NB-Lux 5 µm i-Cellulose-5, 2.12 x 25 cm, 5 µm; mobile phase A: *n-*hexane/methyl tert-butyl ether = 1/1 (0.5% 2 mol/L ammonia methanol solution), mobile phase B: ethanol; flow rate: 20 mL/min; gradient: elution with 10% phase B in 14 min, detector UV 220/300 nm, retention time: 6.17 min; ee > 99% |
| **61-3b** | 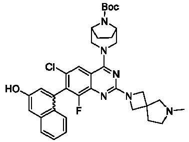 | *tert-*Butyl (1*S*,5*R*)-3-((*R* or *S*)-6-chloro-8-fluoro-7-(3-hydroxynaphthalen-1-yl)-2-(6-methyl-2,6-diazaspiro[3.4]octan-2-yl)quinazolin-4-yl)-3,8-diazabicyclo[3.2.1]o ctane-8-carboxylate | 659. 3/66 1.3 | Chiral column: NB-Lux 5 µm i-Cellulose-5, 2.12 x 25 cm, 5 µm; mobile phase A: *n-*hexane/methyl tert-butyl ether = 1/1 (0.5% 2 mol/L ammonia methanol solution), mobile phase B: ethanol; flow rate: 20 mL/min; gradient: elution with 10% phase B in 14 min, detector UV 220/300 nm, retention time: 7.84 min; ee > 98% |
| **62-3a** | 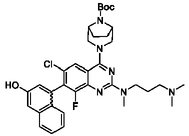 | *tert-*Butyl (1*R,*5*S*)*-*3-((*S* or *R*)-6-chloro-2-(3-(dimethylamino)pro pyl)(methyl)amino)-8-fluoro-7-(3-hydroxynaphthalen-1-yl)quinazolin-4-yl)-3,8-diazabicyclo[3.2.1]o ctane-8-carboxylate | 649. 5/65 1.3 | Chiral column: NB-Lux 5 µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 5% phase B in 26 min, detector UV 220 nm, retention time: 13.879 min; ee > 99% |
| **62-3b** | 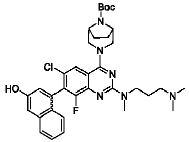 | *tert-Butyl* (1*R,*5S)-3-((*R* or S)-6-chloro-2-(3-(dimethylamino)pro pyl)(methyl)amino)-8-fluoro-7-(3-hydroxynaphthalen-1-yl)quinazolin-4-yl)-3,8-diazabicyclo[3.2.1]octane-8-carboxylate | 649. 3/65 1.3 | Chiral column: NB-Lux 5 µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 5% phase B in 26 min, detector UV 220 nm, retention time: 17.6775 min; ee > 99% |
| **63-3a** | 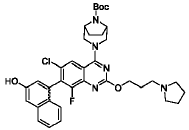 | *tert-*Butyl (1*R*,5*S*)-3-((*S* or *R*)-6-chloro-8-fluoro-7-(3-hydroxynaphthalen-1-yl)-2-(3-(pyrrolidin-1-yl)propoxy)quinazoli n-4-yl)-3,8-diazabicyclo[3.2.1]o ctane-8-carboxylate | 662. 4/66 4.3 | Chiral column: CHIRAL ART Cellulose-SC, 2 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 15 min, detector UV 220 nm, retention time: 4.745 min; ee > 99% |
| **63-3b** | 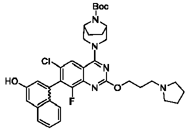 | *tert-*Butyl (1*R*,5*S*)-3-((*R* or *S*)-6-chloro-8-fluoro-7-(3-hydroxynaphthalen-1-yl)-2-(3-(pyrrolidin-1-yl)propoxy)quinazoli n-4-yl)-3,8-diazabicyclo[3.2.1]o ctane-8-carboxylate | 662. 4/66 4.3 | Chiral column: CHIRAL ART Cellulose-SC, 2 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 15 min, detector UV 220 nm, retention time: 9.2375 min; ee > 99% |
| **64-3a** | 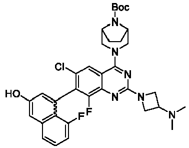 | *tert-*Butyl (1*R*,5*S*)-3-((*S* or *R*)-6-chloro-2-(3-(dimethylamino)azet idin-1-yl)-8-fluoro-7-(8-fluoro-3-hydroxynaphthalen-1-yl)quinazolin-4-yl)-3,8-diazabicyclo[3.2.1]o ctane-8-carboxylate | 651. 4/65 3.3 | Chiral column: NB-Lux 5µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 15 min, detector UV 254/ 220 nm, retention time: 7.435 min; ee > 99% |
| **64-3b** | 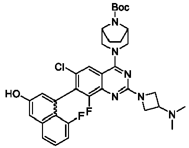 | *tert-*Butyl (1*R,*5*S*)-3-((*R* or *S*)-6-chloro-2-(3-(dimethylamino)azet idin-1-yl)-8-fluoro-7-(8-fluoro-3-hydroxynaphthalen-1-yl)quinazolin-4-yl)-3,8-diazabicyclo[3.2.1]o ctane-8-carboxylate | 651. 4/65 3.3 | Chiral column: NB-Lux 5 i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 15 min, detector UV 254/ 220 nm, retention time: 11.1545 min; ee > 99% |
| **65-3a** | 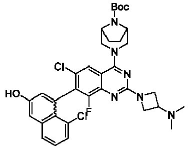 | *tert*-Butyl (1*R,*5*S*)-3-((*S* or *R*)-6-chloro-7-(8-chloro-3-hydroxynaphthalen-1-yl)-2-(3-(dimethylamino)azet idin-1-yl)-8-fluoroquinazolin-4-yl)-3,8-diazabicyclo[3.2.1]o ctane-8-carboxylate | 667. 3/66 9.3 | Chiral column: NB-Lux 5 i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 18 min, detector UV 220 nm, retention time: 7.05 min; ee > 99% |
| **65-3b** | 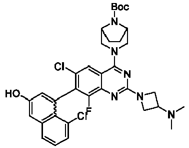 | *tert-*Butyl (1*R*,5*S*)-3-((*R* or *S*)-6-chloro-7-(8-chloro-3-hydroxynaphthalen-1-yl)-2-(3-(dimethylamino)azet idin-1-yl)-8-fluoroquinazolin-4-yl)-3,8-diazabicyclo[3.2.1]o ctane-8-carboxylate | 667. 3/66 9.3 | Chiral column: NB-Lux 5 µm i-Cellulose-5, 2.12 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 10% phase B in 18 min, detector UV 220 nm, retention time: 11.425 min; ee > 99% |
| **66-3a** | 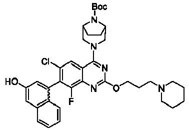 | *tert*-Butyl (1*R*,5*S*)-3-((*S* or *R*)-6-chloro-8-fluoro-7-(3-hydroxynaphthalen-1-yl)-2-(3-(piperidin-1-yl)propoxy)quinazoli n-4-yl)-3,8-diazabicyclo[3.2.1]o ctane-8-carboxylate | 676. 3/67 8.3 | Chiral column: CHIRAL ART Cellulose-SC, 2 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 13 min, detector UV 220 nm, retention time: 4.425 min; ee > 99% |
| **66-3b** | 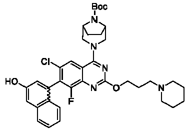 | *tert-*Butyl (1*R*,5*S*)-3-((*R* or S)-6-chloro-8-fluoro-7-(3-hydroxynaphthalen-1-yl)-2-(3-(piperidin-1-yl)propoxy)quinazolin-4-yl)-3,8-diazabicyclo[3.2.1]o ctane-8-carboxylate | 676. 3/67 8.3 | Chiral column: CHIRAL ART Cellulose-SC, 2 x 25 cm, 5 µm; mobile phase A: *n-*hexane/dichloromethane = 3/1 (0.5% 2 mol/L ammonia methanol solution), mobile phase B: ethanol; flow rate: 20 mL/min; gradient: elution with 10% phase B in 13 min, detector UV 220 nm, retention time: 8.87 min; ee > 99% |
| **89-3a** | 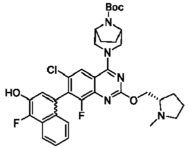 | *tert-*Butyl (1*R*,5*S*)-3-(6-chloro-8-fluoro-7-((*S* or *R*)-4-fluoro-3-hydroxynaphthalen-1-yl)-2-((S)-1-methylpyrrolidin-2-yl)methoxy)quinazol in-4-yl)-3,8-diazabicyclo[3.2.1]o ctane-8-carboxylate | 666. 3/66 8.2 | Chiral column: CHIRAL ART Cellulose-SC, 2 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 8% phase B in 12 min, detector UV 220/206 nm, retention time: 5.875 min; ee > 95%. |
| **89-3b** | 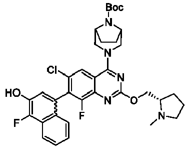 | *tert*-Butyl (1*R*,5*S*)-3-(6-chloro-8-fluoro-7-((*R* or *S*)-4-fluoro-3-hydroxynaphthalen-1-yl)-2-((S)-1-methylpyrrolidin-2-yl)methoxy)quinazol in-4-yl)-3,8-diazabicyclo[3.2.1]o ctane-8-carboxylate | 666. 3/66 8.2 | Chiral column: CHIRAL ART Cellulose-SC, 2 x 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: elution with 8% phase B in 12 min, detector UV 220/206 nm, retention time: 8.193 min; ee> 99%. |
| **90-3a** | 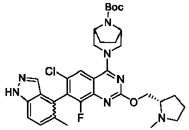 | *tert-*Butyl (1*R*,5*S*)-3-(6-chloro-8-fluoro-7-((*R* or *S*)-5-methyl-1*H*-indazol-4-yl)-2-((*S*)-1-methylpyrrolidin-2-yl)methoxy)quinazol in-4-yl)-3,8-diazabicyclo[3.2.1]o ctane-8-carboxylate | 636. 3/63 8.3 | Chiral column: CHIRAL ART Cellulose-SC, 2 x 25 cm, 5 µm; mobile phase A: *n*-hexane (10 mmol/L ammonia methanol solution), mobile phase B: ethanol; flow rate: 20 mL/min; gradient: elution with 50% phase B in 10 min, detector UV 210/240 nm, retention time: 4.332 min; ee > 99%. |
| **90-3b** | 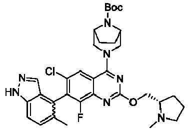 | *tert*-Butyl (1*R*,5*S*)-3-(6-chloro-8-fluoro-7-((*S* or *R*)-5-methyl-1*H*-indazol-4-yl)-2-((*S*)-1-methylpyrrolidin-2-yl)methoxy)quinazol in-4-yl)-3,8-diazabicyclo[3.2.1]o ctane-8-carboxylate | 636. 3/63 8.3 | Chiral column: CHIRAL ART Cellulose-SC, 2 x 25 cm, 5 µm; mobile phase A: *n*-hexane (10 mmol/L ammonia methanol solution), mobile phase B: ethanol; flow rate: 20 mL/min; gradient: elution with 50% phase B in 10 min, detector UV 210/240 nm, retention time: 6.27 min; ee > 95%. |

### Step 5:

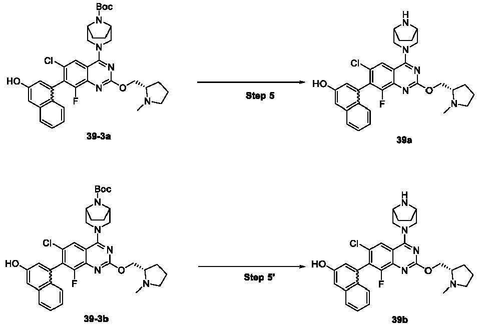

Trifluoroacetic acid (1 mL) was added dropwise to a solution of compound **39-3a** (80 mg, 0.14 mmol, 1.0 eq) in dichloromethane (3 mL) with stirring at 25 °C, after the dropwise addition, the reaction solution was stirred at this temperature for 1 hour, and the reaction process was monitored by liquid chromatography-mass spectrometry. After the reaction was completed, the reaction solution was concentrated under reduced pressure to obtain a crude product. The obtained crude product was purified by reversed-phase chromatographic column (C18 column), and eluted with 5% → 95% (acetonitrile/methanol (1:1))/water mobile phase (0.1% ammonium bicarbonate) in 40 min; detector, UV254 nm; compound **39a** (white solid, 31 mg, yield: 45%) was obtained. Compound **39b** (white solid, 30.3 mg, yield: 54%) can be obtained by the same method as above.

Compound **39a:** MS (ESI, m/z): 548.3/550.3 [M + H]⁺; ¹H NMR (300 MHz, CD₃OD): δ 7.97 - 7.96 (m, 1H), 7.76 (d, *J=* 8.3 Hz, 1H), 7.45 - 7.39 (m, 1H), 7.27 (d, *J=* 2.4 Hz, 1H), 7.25 - 7.18 (m, 2H), 7.04 (d, *J=* 2.4 Hz, 1H), 4.55 - 4.40 (m, 4H), 3.68 - 3.64 (m, 4H), 3.15 - 3.08 (m, 1H), 2.85 - 2.80 (m, 1H), 2.55 (d, *J=* 1.3 Hz, 3H), 2.43 - 2.34(m, 1H), 2.19 - 2.07 (m, 1H), 1.97 - 1.67 (m, 7H); ¹⁹F NMR (282 MHz, CD₃OD) δ -123.19. The chiral analysis conditions of compound **39a** were: CHIRALPAK IG-3, 4.6 x 50 mm, 3 µm; mobile phase A: n-hexane/dichloromethane=3/1 (0.1% diethylamine), mobile phase B: ethanol; flow rate: 1 mL/min; isocratic elution with 50% phase B in 6 min; detector UV 220/254 nm; retention time: 1.203 min; ee > 99%.

Compound **39b:** MS (ESI, m/z): 548.3/550.3 [M + H]⁺; ¹H NMR (300 MHz, CD₃OD): δ 7.95 - 7.94 (m, 1H), 7.74 (d, *J=* 8.3 Hz, 1H), 7.43 - 7.37 (m, 1H), 7.25 (d, *J=* 2.4 Hz, 1H), 7.23 - 7.16 (m, 2H), 7.02 (d, *J=* 2.4 Hz, 1H), 4.53 - 4.40 (m, 4H), 3.67 - 3.61 (m, 4H), 3.15 - 3.07 (m, 1H), 2.89 - 2.82 (m, 1H), 2.55 (d, *J=* 2.1 Hz, 3H), 2.45 - 2.36(m, 1H), 2.16 - 2.07 (m, 1H), 1.89 - 1.70 (m, 7H); ¹⁹F NMR (282 MHz, CD₃OD) δ -123.23. The chiral analysis conditions of compound **39b** were: CHIRALPAK IG-3, 4.6 x 50 mm, 3 µm; mobile phase A: n-hexane/dichloromethane=3/1 (0.1% diethylamine), mobile phase B: ethanol; flow rate: 1 mL/min; isocratic elution with 50% phase B in 6 min; detector UV 220/254 nm; retention time: 2.391 min; ee > 99%.

Other similar compounds of the present disclosure can be prepared by the synthetic method shown in Embodiment **6** above. Some compounds prepared with reference to the above synthetic method and their characterization data are shown in table 5.

**Table 5**

| Number of the compound | Compound structure | Compound name | Chiral analysis conditions/retenti on time/ee value/specific rotation | Mass spec trum [M+ H]⁺ | ¹H & ¹⁹F NMR |
|---|---|---|---|---|---|
| **40a** | 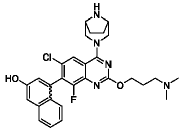 | (*S* or *R*)-4-(4-((1*R*,5*S*)-3,8-diazabicycl o[3.2.1]octa n-3-yl)-6-chloro-2-(3-(dimethyla mino)propo xy)-8-fluoroquina zolin-7-yl)naphthale n-2-ol | CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.960 min; ee > 99%. | 536. 2/53 8.2 | ¹H NMR (400 MHz, DMSO-*d*₆) δ 10.04 (s, 1H), 7.93 (d, *J* = 1.5 Hz, 1H), 7.81 (d, *J* = 8.3 Hz, 1H), 7.46 - 7.42 (m, 1H), 7.28 (d, *J* = 2.4 Hz, 1H), 7.25 - 7.21 (m, 2H), 7.06 (d, *J* = 2.4 Hz, 1H), 4.36 - 4.29 (m, 4H), 3.55 - 3.49 (m, 4H), 2.34 (t, *J* = 7.1 Hz, 2H), 2.13 (s, 6H), 1.90 - 1.83 (m, 2H), 1.67 - 1.62 (m, 4H); ¹⁹F NMR (377 MHz, DMSO-*d*₆) δ - 122.49. |
| **40b** | 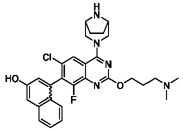 | (*R* or *S*)-4-(4-((1*R*,5*S*)-3,8-diazabicycl o[3.2.1]octa n-3-yl)-6-chloro-2-(3-(dimethyla mino)propo xy)-8-fluoroquina zolin-7-yl)naphthale n-2-ol | CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.715 min; ee > 99%. | 536. 2/53 8.2 | ¹H NMR (400 MHz, DMSO-*d*₆) δ 10.02 (s, 1H), 7.93 (d, *J* = 1.6 Hz, 1H), 7.80 (d, *J =* 8.3 Hz, 1H), 7.46 - 7.42 (m, 1H), 7.28 (d, *J* = 2.4 Hz, 1H), 7.23 - 7.21 (m, 2H), 7.06 (d, *J* = 2.4 Hz, 1H), 4.35 - 4.29 (m, 4H), 3.55 - 3.49 (m, 4H), 2.34 (t, *J* = 7.0 Hz, 2H), 2.13 (s, 6H), 1.90 - 1.83 (m, 2H), 1.67 - 1.62 (m, 4H); ¹⁹F NMR (377 MHz, DMSO-*d*₆) δ - 122.48. |
| **41** | 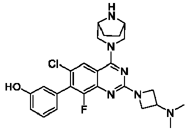 | 3-(4-((1R,5S)-3,8-diazabicycl o[3.2.1]octa n-3-yl)-6-chloro-2-(3-(dimethyla mino)azetid in-1-yl)-8-fluoroquina zolin-7-yl)phenol | | 483. 2/48 5.1 | ¹H NMR (300 MHz, CD₃OD) δ 7.70 (d, *J=* 1.8 Hz, 1H), 7.31 (dd, *J* = 8.2, 7.4 Hz, 1H), 6.90 - 6.86 (m, 1H), 6.83 - 6.78 (m, 2H), 4.38 - 4.32 (m, 2H), 4.29 - 4.23 (m, 2H), 4.05 - 3.99 (m, 2H), 3.61 - 3.58 (m, 2H), 3.55 - 3.49 (m, 2H), 3.30 - 3.21 (m, 1H), 2.26 (s, 6H), 1.88 - 1.81 (m, 4H); ¹⁹F NMR (282 MHz, CD₃OD) δ - 126.91. |
| **42a** | 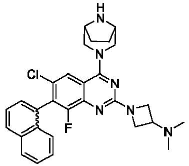 | 1-((S or *R)-*4-((1*R*,5*S*)-3,8-diazabicycl o[3.2.1]octa n-3-yl)-6-chloro-8-fluoro-7-(naphthalen -1-yl)quinazoli n-2-yl)-*N,N-*dimethylaze tidin-3-amine | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 10% phase B in 8 min; detector UV 220/254 nm; retention time: 3.759 min; ee > 99%. | 517. 2/51 9.2 | ¹H NMR (300 MHz, CD₃OD) δ 8.03 - 7.97 (m, 2H), 7.81 (d, *J* = 1.8 Hz, 1H), 7.62 (dd, *J =* 8.3, 7.1 Hz, 1H), 7.57 - 7.51 (m, 1H), 7.48 - 7.40 (m, 3H), 4.44 - 4.37 (m, 2H), 4.30 - 4.24 (m, 2H), 4.06 - 4.01 (m, 2H), 3.64 - 3.53 (m, 4H), 3.30 - 3.21 (m, 1H), 2.26 (s, 6H), 1.91 - 1.83 (m, 4H); ¹⁹F NMR (282 MHz, CD₃OD) δ - 124.81. |
| **42b** | 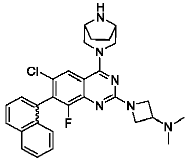 | -((*R* or *S*)-4-((1*R*,5*S*)-3,8-diazabicycl o[3.2.1]octa n-3-yl)-6-chloro-8-fluoro-7-(naphthalen -1-yl)quinazoli n-2-yl)-*N,N-*dimethylaze tidin-3-amine | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 10% phase B in 8 min; detector UV 220/254 nm; retention time: 5.749 min; ee > 99%. | 517. 2/51 9.2 | ¹H NMR (300 MHz, CD₃OD) δ 8.03 - 7.97 (m, 2H), 7.81 (d, *J* = 1.8 Hz, 1H), 7.62 (dd, *J =* 8.3, 7.1 Hz, 1H), 7.57 - 7.51 (m, 1H), 7.48 - 7.40 (m, 3H), 4.44 - 4.37 (m, 2H), 4.30 - 4.24 (m, 2H), 4.06 - 4.01 (m, 2H), 3.62 - 3.53 (m, 4H), 3.30 - 3.21 (m, 1H), 2.26 (s, 6H), 1.91 - 1.84 (m, 4H); ¹⁹F NMR (282 MHz, CD₃OD) *δ* - 124.82. |
| **43a** | 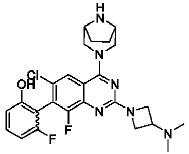 | 2-((*S* or *R*)-4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)-3-fluoropheno 1 | CHIRALPAK IE-3, 3.0 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 3.306 min; ee > 99%. | 501. 3/50 3.2 | ¹H NMR (300 MHz, DMSO-*d*₆) δ 7.64 (d, *J* = 1.6 Hz, 1H), 7.21 - 7.13 (m, 1H), 6.69 (d, *J* = 8.3 Hz, 1H), 6.57 - 6.51 (m, 1H), 4.22 - 4.05 (m, 4H), 3.87 - 3.81 (m, 2H), 3.47 - 3.35 (m, 4H), 3.15 - 3.06 (m, 1H), 2.12 (s, 6H), 1.70 - 1.63 (m, 4H); ¹⁹F NMR (282 MHz, DMSO-*d*₆) δ - 114.13, -122.06. |
| **43b** | 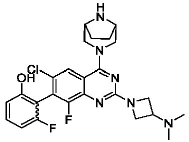 | 2-((*R* or *S*)-4-((1*R,*5*S*)-3,8-diazabicycl o[3.2.1]octa n-3-yl)-6-chloro-2-(3-(dimethylamino)azetid in-1-yl)-8-fluoroquina zolin-7-yl)-3-fluorophenol | CHIRALPAK IE-3, 3.0 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 4.803 min; ee > 99%. | 501. 3/50 3.2 | ¹H NMR (300 MHz, DMSO-*d*₆) *δ* 10.22 (s, 1H), 7.68 (d, *J* = 1.6 Hz, 1H), 7.36 - 7.28 (m, 1H), 6.83 (d, *J* = 8.2 Hz, 1H), 6.81 - 6.74 (m, 1H), 4.22 - 4.15 (m, 2H), 4.12 - 4.05 (m, 2H), 3.87 - 3.81 (m, 2H), 3.49 - 3.37 (m, 4H), 3.15 - 3.07 (m, 1H), 2.12 (s, 6H), 1.70 - 1.61 (m, 4H); ¹⁹F NMR (282 MHz, DMSO-*d*₆) δ -113.65, - 122.17. |
| **44a** | 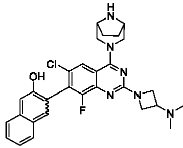 | 3-((*S* or *R)-*4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)naphthale n-2-ol | CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.737 min; ee > 78%. | 533. 2/53 5.2 | ¹H NMR (300 MHz, DMSO-*d*₆) δ 10.04 (s, 1H), 7.83 (d, *J* = 8.2 Hz, 1H), 7.77 - 7.74 (m, 2H), 7.68 (d, *J* = 1.6 Hz, 1H), 7.49 - 7.43 (m, 1H), 7.35 - 7.29 (m, 2H), 4.23 - 4.17 (m, 2H), 4.12 - 4.06 (m, 2H), 3.87 - 3.82 (m, 2H), 3.52 - 3.40 (m, 4H), 3.15 - 3.07 (m, 1H), 2.12 (s, 6H), 1.71-1.63 (m, 4H); ¹⁹F NMR (282 MHz, DMSO-*d*₆) δ -123.31. |
| **44b** | 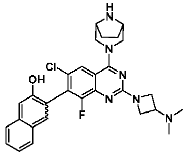 | 3-((*R* or *S*)-4-((1*R,*5*S*)-3,8-diazabicycl o[3.2.1]octan-3-yl)-6-chloro-2-(3-(dimethyla mino)azetid in-1-yl)-8-fluoroquina zolin-7-yl)naphthale n-2-ol | CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.713 min; ee > 70%. | 533. 2/53 5.2 | ¹H NMR (300 MHz, DMSO-*d*₆) δ 10.04 (s, 1H), 7.83 (d, *J* = 8.2 Hz, 1H), 7.78 - 7.74 (m, 2H), 7.68 (d, *J*= 1.6 Hz, 1H), 7.49 - 7.43 (m, 1H), 7.35 - 7.29 (m, 2H), 4.23 - 4.17 (m, 2H), 4.12 - 4.06 (m, 2H), 3.87 - 3.82 (m, 2H), 3.52 - 3.40 (m, 4H), 3.15 - 3.07 (m, 1H), 2.12 (s, 6H), 1.72 -1.63 (m, 4H); ¹⁹F NMR (282 MHz, DMSO-*d*₆) δ -123.32. |
| **45a** | 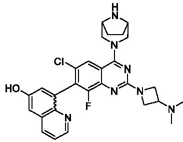 | 8-((*S* or *R)-*4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)quinolin-6-ol | CHIRALPAK IE-3, 4.6 x 50 mm, 3 µm; mobile phase A: methyl *tert-*butyl ether (0.1% diethylamine), mobile phase B: methanol; flow rate: 1 mL/min; isocratic elution with 50% phase B in 6 min; detector UV 220/254 nm; retention time: 1.200 min; ee > 99%. | 534. 2/53 6.2 | ¹H NMR (400 MHz, CD₃OD) δ 8.51 (dd, *J=* 4.3, 1.7 Hz, 1H), 8.22 - 8.19 (m, 1H), 7.73 - 7.72 (m, 1H), 7.45 - 7.41 (m, 1H), 7.29 - 7.26 (m, 2H), 4.50 - 4.46 (m, 1H), 4.35 - 4.29 (m, 1H), 4.27 - 4.22 (m, 2H), 4.03 - 3.99 (m, 2H), 3.62 - 3.57 (m, 3H), 3.50 - 3.46 (m, 1H), 3.27 - 3.20 (m, 1H), 2.24 (s, 6H), 1.96 - 1.80 (m, 4H); ¹⁹F NMR (377 MHz, CD₃OD) *δ* - 125.35. |
| **45b** | 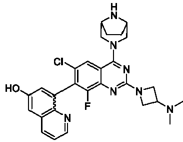 | 8-((*R* or *S*)-4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)quinolin-6-ol | CHIRALPAK IE-3, 4.6 x 50 mm, 3 µm; mobile phase A: methyl *tert-*butyl ether (0.1% diethylamine), mobile phase B: methanol; flow rate: 1 mL/min; isocratic elution with 50% phase B in 6 min; detector UV 220/254 nm; retention time: 3.550 min; ee > 99%. | 534. 2/53 6.2 | ¹H NMR (300 MHz, CD₃OD) δ 8.55 - 8.52 (m, 1H), 8.25 - 8.21 (m, 1H), 7.75 (d, *J*= 1.8 Hz, 1H), 7.48 - 7.43 (m, 1H), 7.32 - 7.29 (m, 2H), 4.54 - 4.47 (m, 1H), 4.39 - 4.24 (m, 3H), 4.06 - 4.00 (m, 2H), 3.66 - 3.59 (m, 3H), 3.53 - 3.47 (m, 1H), 3.30 - 3.22 (m, 1H), 2.26 (s, 6H), 1.99 -1.83 (m, 4H); ¹⁹F NMR (282 MHz, CD₃OD) δ -125.36. |
| **46a** | 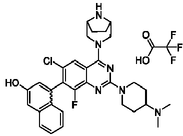 | 4-((*S* or *R*)-4-((1*R*,5*S*)-3,8-diazabicycl o[3.2.1]octa n-3-yl)-6-chloro-2-(4-(dimethyla mino)piperi din-1-yl)-8-fluoroquina zolin-7-yl)naphthalen-2-ol trifluoroacetate | CHIRALPAK IC-3, 4.6 x 50 mm, 3 µm; mobile phase A: n-hexane: methyl *tert*-butyl ether 1:1 (0.1% diethylamine), mobile phase B: ethanol; flow rate: 1 mL/min; isocratic elution with 5% phase B in 10 min; detector UV 220/254 nm; retention time: 5.305 min; ee > 99%. | 561. 3/56 3.2 | ¹H NMR (300 MHz, DMSO-*d*₆) *δ* 7.82 - 7.75 (m, 2H), 7.47 - 7.41 (m, 1H), 7.28 (d, *J* = 2.4 Hz, 1H), 7.24 - 7.21 (m, 2H), 7.04 (d, *J* = 2.4 Hz, 1H), 4.87 - 4.77 (m, 2H), 4.35 - 4.26 (m, 2H), 3.96 - 3.89 (m, 2H), 3.65 - 3.56 (m, 2H), 2.99 - 2.89 (m, 2H), 2.82 - 2.72 (m, 1H), 2.40 (s, 6H), 1.97 - 1.85 (m, 6H), 1.48 - 1.35 (m, 2H); ¹⁹F NMR (282 MHz, DMSO-*d*₆) δ - 73.50, -123.61. |
| **46b** | 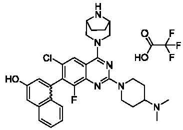 | 4-((*R* or *S*)-4-((1*R,*5*S*)-3,8-diazabicycl o[3.2.1]octa n-3-yl)-6-chloro-2-(4-(dimethyla mino)piperi din-1-yl)-8-fluoroquina zolin-7-yl)naphthale n-2-ol trifluoroacet ate | CHIRALPAK IC-3, 4.6 x 50 mm, 3 µm; mobile phase A: *n*-hexane: methyl tert-butyl ether (0.1% diethylamine), mobile phase B: ethanol; flow rate: 1 mL/min; isocratic elution with 5% phase B in 10 min; detector UV 220/254 nm; retention time: 7.357 min; ee > 99%. | 561. 3/56 3.2 | ¹H NMR (300 MHz, DMSO-*d*₆) *δ* 7.82 - 7.75 (m, 2H), 7.47 - 7.41 (m, 1H), 7.27 (d, *J* = 2.4 Hz, 1H), 7.24 - 7.21 (m, 2H), 7.04 (d, *J* = 2.4 Hz, 1H), 4.86 - 4.77 (m, 2H), 4.35 - 4.26 (m, 2H), 3.97 - 3.90 (m, 2H), 3.64 - 3.56 (m, 2H), 2.99 - 2.89 (m, 2H), 2.83 - 2.71 (m, 1H), 2.39 (s, 6H), 1.97 - 1.84 (m, 6H), 1.48 - 1.35 (m, 2H); ¹⁹F NMR (282 MHz, DMSO-*d*₆) *δ* - 73.50, -123.62. |
| **47a** | 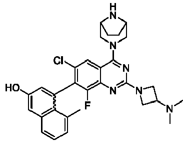 | 4-((*S* or *R)-*4-((1*R*,5*S*)-3,8-diazabicycl o[3.2.1]octan-3-yl)-6-chloro-2-(3-(dimethyla mino)azetid in-1-yl)-8-fluoroquina zolin-7-yl)-5-methylnapht halen-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 6 min; detector UV 220/254 nm; retention time: 3.197 min; ee > 99%. | 547. 2/54 9.2 | ¹H NMR (400 MHz, CD₃OD) δ 7.71 (d, *J=* 1.7 Hz, 1H), 7.60 (d, *J* = 8.0 Hz, 1H), 7.28 - 7.22 (m, 2H), 7.04 - 7.01 (m, 1H), 6.84 (d, *J* = 2.6 Hz, 1H), 4.43 - 4.38 (m, 1H), 4.32 - 4.22 (m, 3H), 4.03 - 3.99 (m, 2H), 3.63 - 3.46 (m, 4H), 3.27 - 3.21 (m, 1H), 2.24 (s, 6H), 2.03 (s, 3H), 1.93 - 1.82 (m, 4H); ¹⁹F NMR (377 MHz, CD₃OD) *δ* -124.81. |
| **47b** | 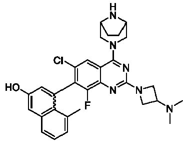 | 4-((*R* or *S*)-4-((1*R,*5*S*)-3,8-diazabicycl o[3.2.1]octa n-3-yl)-6-chloro-2-(3-(dimethyla mino)azetid in-1-yl)-8-fluoroquina zolin-7-yl)-5-methylnapht halen-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 6 min; detector UV 220/254 nm; retention time: 4.394 min; ee > 99%. | 547. 2/54 9.2 | ¹H NMR (400 MHz, CD₃OD) *δ* 7.71 (d, *J =* 1.7 Hz, 1H), 7.60 (d, *J* = 8.2 Hz, 1H), 7.28 - 7.22 (m, 2H), 7.04 - 7.01 (m, 1H), 6.84 (d, *J* = 2.6 Hz, 1H), 4.42 - 4.38 (m, 1H), 4.31 - 4.22 (m, 3H), 4.03 - 3.99 (m, 2H), 3.63 - 3.54 (m, 3H), 3.50 - 3.46 (m, 1H), 3.27 - 3.21 (m, 1H), 2.24 (s, 6H), 2.03 (s, 3H), 1.93 - 1.82 (m, 4H); ¹⁹F NMR (377 MHz, CD₃OD) *δ* -124.81. |
| **48a** | 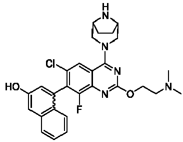 | 4-((*S* or *R*)-4-((1*R*,5*S*)-3,8-diazabicycl o[3.2.1]octa n-3-yl)-6-chloro-2-(2-(dimethyla mino)ethox y)-8-fluoroquina zolin-7-yl)naphthale n-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 7 min; detector UV 220/254 nm; retention time: 4.100 min; ee > 99%. | 522. 3/52 4.3 | ¹H NMR (300 MHz, DMSO-*d*₆) δ 10.03 (s, 1H), 7.94 (d, *J* = 1.6 Hz, 1H), 7.81 (d, *J* = 8.3 Hz, 1H), 7.47 - 7.42 (m, 1H), 7.29 (d, *J* = 2.4 Hz, 1H), 7.24 - 7.21 (m, 2H), 7.07 (d, *J* = 2.4 Hz, 1H), 4.43 - 4.30 (m, 4H), 3.59 - 3.50 (m, 4H), 2.63 (t, *J* = 5.9 Hz, 2H), 2.21 (s, 6H), 1.67 (s, 4H); ¹⁹F NMR (282 MHz, DMSO-*d*₆) δ -122.47. |
| **48b** | 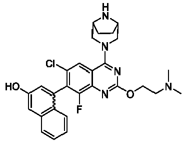 | 4-((*R* or *S*)-4-((1*R,*5*S*)-3,8-diazabicycl o[3.2.1]octa n-3-yl)-6-chloro-2-(2-(dimethyla mino)ethox y)-8-fluoroquina zolin-7-yl)naphthale n-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 7 min; detector UV 220/254 nm; retention time: 5.751 min; ee > 99%. | 522. 3/52 4.3 | ¹H NMR (300 MHz, DMSO-*d*₆) δ 10.02 (s, 1H), 7.94 (d, *J* = 1.6 Hz, 1H), 7.81 (d, *J* = 8.3 Hz, 1H), 7.47 - 7.42 (m, 1H), 7.29 (d, *J* = 2.4 Hz, 1H), 7.24 - 7.22 (m, 2H), 7.07 (d, *J* = 2.4 Hz, 1H), 4.43 - 4.30 (m, 4H), 3.58 - 3.50 (m, 4H), 2.63 (t, *J* = 5.9 Hz, 2H), 2.21 (s, 6H), 1.67 (s, 4H); ¹⁹F NMR (282 MHz, DMSO-*d*₆) δ -122.48. |
| **49a** | 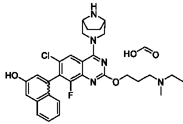 | 4-((*S* or *R)-*4-((1*R*,5*S*)-3,8-diazabicycl o[3.2.1]octa n-3-yl)-6-chloro-2-(3-(ethyl(meth yl)amino)pr opoxy)-8-fluoroquina zolin-7-yl)naphthale n-2-ol carboxylate | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 30% phase B in 4 min; detector UV 220/254 nm; retention time: 1.910 min; ee > 99%. | 550. 3/55 2.3 | ¹H NMR (400 MHz, CD₃OD) δ 8.49 (s, 1H), 7.98 (d, *J* = 1.7 Hz, 1H), 7.76 (d, *J* = 8.3 Hz, 1H), 7.44 - 7.39 (m, 1H), 7.27 (d, *J* = 2.4 Hz, 1H), 7.23 - 7.17 (m, 2H), 7.02 (d, *J* = 2.4 Hz, 1H), 4.65 - 4.60 (m, 2H), 4.56 (t, *J*= 5.9 Hz, 2H), 4.03 - 4.01 (m, 2H), 3.83 - 3.78 (m, 2H), 3.36 - 3.32 (m, 2H), 3.22 (q, *J* = 7.3 Hz, 2H), 2.86 (s, 3H), 2.29 - 2.22 (m, 2H), 2.05 - 2.03 (m, 4H), 1.33 (t, *J* = 7.3 Hz, 3H); ¹⁹F NMR (377 MHz, CD₃OD) δ -123.25. |
| **49b** | 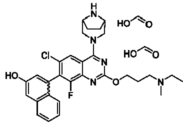 | 4-((*R* or *S*)-4-((1*R*,5*S*)-3,8-diazabicyclo[3.2.1]octa n-3-yl)-6-chloro-2-(3-(ethyl(meth yl)amino)pr opoxy)-8-fluoroquina zolin-7-yl)naphthale n-2-ol dicarboxylate | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 30% phase B in 4 min; detector UV 220/254 nm; retention time: 2.941 min; ee > 99%. | 550. 3/55 2.3 | ¹H NMR (400 MHz, CD₃OD) *δ* 8.51 (s, 2H), 7.98 (d, *J* = 1.7 Hz, 1H), 7.76 (d, *J* = 8.3 Hz, 1H), 7.44 - 7.39 (m, 1H), 7.27 (d, *J* = 2.4 Hz, 1H), 7.24 - 7.16 (m, 2H), 7.02 (d, *J = 2.4* Hz, 1H), 4.65 - 4.59 (m, 2H), 4.56 (t*, J* = 5.9 Hz, 2H), 4.03 - 4.00 (m, 2H), 3.83 - 3.78 (m, 2H), 3.34 - 3.31 (m, 2H), 3.21 (q, *J* = 7.3 Hz, 2H), 2.85 (s, 3H), 2.28 - 2.22 (m, 2H), 2.05 - 2.01 (s, 4H), 1.33 (t, *J* = 7.3 Hz, 3H); ¹⁹F NMR (377 MHz, CD₃OD) δ -123.21. |
| **50a** | 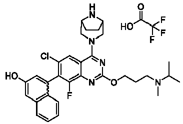 | 4-((*S* or *R*)-4-((1*R*,5*S*)-3,8-diazabicycl o[3.2.1]octa n-3-yl)-6-chloro-8-fluoro-2-(3-(isopropyl( methyl)ami no)propoxy) quinazolin-7-yl)naphthale n-2-ol trifluoroacet ate | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 8 min; detector UV 220/254 nm; retention time: 3.684 min; ee > 99% | 564. 4/56 6.4 | ¹H NMR (300 MHz, CD₃OD) δ 8.01 (d*, J* = 1.8 Hz, 1H), 7.78 (d, *J* = 8.3 Hz, 1H), 7.47 - 7.41 (m, 1H), 7.29 (d, *J* = 2.4 Hz, 1H), 7.26 - 7.18 (m, 2H), 7.04 (d, *J* = 2.4 Hz, 1H), 4.71 - 4.64 (m, 2H), 4.59 (t, *J* = 5.8 Hz, 2H), 4.18 - 4.11 (m, 2H), 3.91 - 3.82 (m, 2H), 3.74 - 3.65 (m, 1H), 3.40 - 3.34 (m, 2H), 2.85 - 2.83 (m, 3H), 2.34 - 2.24 (m, 2H), 2.13 - 2.10 (m, 4H), 1.37 (d, *J* = 6.6 Hz, 6H); ¹⁹F NMR (282 MHz, CD₃OD) δ -76.91, - 123.08. |
| **50b** | 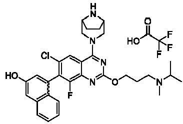 | 4-((*R* or *S*)-4-((1*R*,5*S*)-3,8-diazabicycl o[3.2.1]octa n-3-yl)-6-chloro-8-fluoro-2-(3-(isopropyl( methyl)ami no)propoxy) quinazolin-7-yl)naphthale n-2-ol trifluoroacet ate | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 8 min; detector UV 220/254 nm; retention time: 6.409 min; ee > 99% | 564. 4/56 6.4 | ¹H NMR (300 MHz, CD₃OD) δ 8.01 (d, *J =* 1.8 Hz, 1H), 7.78 (d, *J* = 8.3 Hz, 1H), 7.47 - 7.42 (m, 1H), 7.29 (d, *J* = 2.4 Hz, 1H), 7.26 - 7.18 (m, 2H), 7.04 (d, *J* = 2.4 Hz, 1H), 4.71 - 4.64 (m, 2H), 4.59 (t, *J* = 5.8 Hz, 2H), 4.19 - 4.10 (m, 2H), 3.92 - 3.82 (m, 2H), 3.74 - 3.64 (m, 1H), 3.40 - 3.35 (m, 2H), 2.85 - 2.83 (m, 3H), 2.34 - 2.24 (m, 2H), 2.14 - 2.09 (m, 4H), 1.37 (d, *J* = 6.6 Hz, 6H); ¹⁹F NMR (282 MHz, CD₃OD) δ -76.90, - 123.07. |
| **51a** | 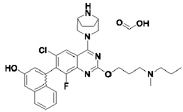 | 4-((S or *R*)-4-((1*R*,5*S*)-3,8-diazabicycl o[3.2.1]octa n-3-yl)-6-chloro-8-fluoro-2-(3-(methyl(pro pyl)amino)p ropoxy)quin azolin-7-yl)naphthale n-2-ol carboxylate | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 8 min; detector UV 220/254 nm; retention time: 3.667 min; ee > 99% | 564. 2/56 6.2 | ¹H NMR (300 MHz, DMSO-*d*₆) *δ* 8.25 (s, 1H), 7.94 (d, *J* = 1.6 Hz, 1H), 7.81 (d, *J* = 8.3 Hz, 1H), 7.47 - 7.42 (m, 1H), 7.29 (d, *J* = 2.4 Hz, 1H), 7.26 - 7.21 (m, 2H), 7.06 (d, *J* = 2.4 Hz, 1H), 4.40 - 4.32 (m, 4H), 3.67 - 3.55 (m, 4H), 2.50 - 2.44 (m, 2H), 2.33 - 2.27 (m, 2H), 2.19 (s, 3H), 1.94 - 1.85 (m, 2H), 1.74 - 1.70 (m, 4H), 1.48 - 1.36 (m, 2H), 0.82 (t, *J* = 7.4 Hz, 3H); ¹⁹F NMR (282 MHz, DMSO-*d*₆) δ - 122.40. |
| **51b** | 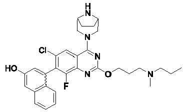 | 4-((*R* or *S*)-4-((1*R,*5*S*)-3,8-diazabicycl o[3.2.1]octa n-3-yl)-6-chloro-8-fluoro-2-(3-(methyl(propyl)amino)p ropoxy)quin azolin-7-yl)naphthale n-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 8 min; detector UV 220/254 nm; retention time: 6.387 min; ee > 99% | 564. 2/56 6.2 | ¹H NMR (300 MHz, DMSO-*d*₆) δ 10.03 (s, 1H), 7.93 (d, *J* = 1.7 Hz, 1H), 7.81 (d, *J =* 8.3 Hz, 1H), 7.47 - 7.42 (m, 1H), 7.29 (d, *J* = 2.4 Hz, 1H), 7.26 - 7.21 (m, 2H), 7.06 (d, *J* = 2.4 Hz, 1H), 4.38 - 4.30 (m, 4H), 3.57 - 3.50 (m, 4H), 2.42 (t, *J* = 7.0 Hz, 2H), 2.27 - 2.22 (m, 2H), 2.14 (s, 3H), 1.92 - 1.82 (m, 2H), 1.69 - 1.63 (m, 4H), 1.46 - 1.33 (m, 2H), 0.82 (t*, J* = 7.3 Hz, 3H); ¹⁹F NMR (282 MHz, DMSO-*d*₆) δ - 122.47. |
| **52a** | 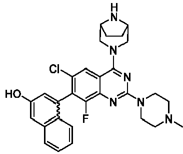 | 4-((*S* or *R)-*4-((1*R*,5*S*)-3,8-diazabicycl o[3.2.1]octa n-3-yl)-6-chloro-8-fluoro-2-(4-methylpiper azin-1-yl)quinazoli n-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 5 min; detector UV 220/254 nm; retention time: 1.686 min; ee > 99% | 533. 3/53 5.3 | ¹H NMR (400 MHz, DMSO-*d*₆) δ 9.98 (s, 1H), 7.80 - 7.78 (m, 1H), 7.74 (d, *J* = 1.6 Hz, 1H), 7.45 - 7.41 (m, 1H), 7.26 (d,J= 2.4 Hz, 1H), 7.24 - 7.19 (m, 2H), 7.03 (d, *J =* 2.4 Hz, 1H), 4.22 - 4.17 (m, 2H), 3.80 - 3.77 (m, 4H), 3.51 - 3.49 (m, 2H), 3.46 - 3.41 (m, 2H), 2.38 - 2.32 (m, 4H), 2.21 (s, 3H), 1.72 - 1.63 (m, 4H); ¹⁹F NMR (377 MHz, DMSO-*d*₆) δ -123.85. |
| **52b** | 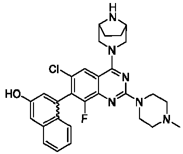 | 4-((*R* or *S*)-4-((1*R*,5*S*)-3,8-diazabicycl o[3.2.1]octa n-3-yl)-6-chloro-8-fluoro-2-(4-methylpiper azin-1-yl)quinazoli n-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 5 min; detector UV 220/254 nm; retention time: 2.959 min; ee > 99% | 533. 3/53 5.3 | ¹H NMR (400 MHz, DMSO-*d*₆) δ 9.98 (s, 1H), 7.79 (d, *J* = 8.3 Hz, 1H), 7.74 (d, *J* = 1.5 Hz, 1H), 7.45 - 7.41 (m, 1H), 7.26 (d, *J* = 2.4 Hz, 1H), 7.24 - 7.19 (m, 2H), 7.03 (d, *J* = 2.4 Hz, 1H), 4.22 - 4.18 (m, 2H), 3.80 - 3.77 (m, 4H), 3.51 - 3.49 (m, 2H), 3.45 - 3.41 (m, 2H), 2.37 - 2.32 (m, 4H), 2.21 (s, 3H), 1.72 - 1.63 (m, 4H); ¹⁹F NMR (377 MHz, DMSO-*d*₆) *δ* - 123.85. |
| **53a** | 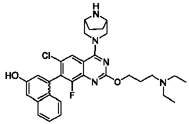 | 4-((*S* or *R*)-4-((1*R*,5*S*)-3,8-diazabicycl o[3.2.1]octa n-3-yl)-6-chloro-2-(3-(diethylami no)propoxy) -8-fluoroquinazolin-7-yl)naphthalen-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 20% phase B in 6 min; detector UV 220/254 nm; retention time: 2.759 min; ee > 99% | 564. 4/56 6.4 | ¹H NMR (300 MHz, DMSO-*d*₆) δ 10.02 (s, 1H), 7.94 (d, *J* = 1.7 Hz, 1H), 7.81 (d, *J* = 8.3 Hz, 1H), 7.47 - 7.42 (m, 1H), 7.29 (d, *J* = 2.4 Hz, 1H), 7.26 - 7.21 (m, 2H), 7.06 (d, *J* = 2.4 Hz, 1H), 4.38 - 4.31 (m, 4H), 3.58 - 3.51 (m, 4H), 2.57 - 2.46 (m, 6H), 1.90 - 1.81 (m, 2H), 1.71 - 1.63 (m, 4H), 0.95 (t, *J* = 7.1 Hz, 6H); ¹⁹F NMR (282 MHz, DMSO-*d*₆) δ -122.47. |
| **53b** | 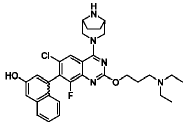 | 4-((*R* or *S*)-4-((1*R*,5*S*)-3,8-diazabicycl o[3.2.1]octa n-3-yl)-6-chloro-2-(3-(diethylami no)propoxy) -8-fluoroquina zolin-7-yl)naphthalen-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 20% phase B in 6 min; detector UV 220/254 nm; retention time: 4.652 min; ee > 99% | 564. 4/56 6.4 | ¹H NMR (300 MHz, DMSO-*d*₆) δ 10.03 (s, 1H), 7.95 (d, *J* = 1.6 Hz, 1H), 7.82 (d, *J* = 8.3 Hz, 1H), 7.48 - 7.42 (m, 1H), 7.29 (d, *J* = 2.4 Hz, 1H), 7.26 - 7.20 (m, 2H), 7.06 (d, *J* = 2.4 Hz, 1H), 4.42 - 4.34 (m, 4H), 3.72 - 3.68 (m, 2H), 3.64 - 3.55 (m, 2H), 2.71 - 2.59 (m, 6H), 1.96 - 1.88 (m, 2H), 1.77 - 1.71 (m, 4H), 1.00 (t, *J* = 7.0 Hz, 6H); ¹⁹F NMR (282 MHz, DMSO-*d*₆) δ -122.38. |
| **54a** | 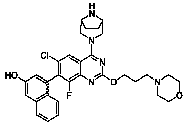 | 4-((*S* or *R*)-4-((1*R*,5*S*)-3,8-diazabicycl o[3.2.1]octan-3-yl)-6-chloro-8-fluoro-2-(3-morpholino propoxy)qui nazolin-7-yl)naphthale n-2-ol | CHIRALPAK IC-3, 4.6 x 50 mm, 3 µm; mobile phase A: *n-*hexane/dichloromethane=5/1 (0.1% diethylamine), mobile phase B: ethanol; flow rate: 1 mL/min; isocratic elution with 30% phase B in 5 min; detector UV 220/254 nm; retention time: 1.963 min; ee > 99% | 578. 2/58 0.2 | ¹H NMR (400 MHz, DMSO-*d*₆) δ 10.03 (s, 1H), 7.93 (d, *J* = 1.6 Hz, 1H), 7.81 (d, *J =* 8.3 Hz, 1H), 7.46 - 7.42 (m, 1H), 7.28 (d, *J* = 2.4 Hz, 1H), 7.25 - 7.21 (m, 2H), 7.06 (d, *J* = 2.4 Hz, 1H), 4.38 - 4.30 (m, 4H), 3.56 - 3.49 (m, 8H), 2.41 (t, *J* = 7.1 Hz, 2H), 2.38 - 2.33 (m, 4H), 1.93 - 1.86 (m, 2H), 1.68 - 1.62 (s, 4H); ¹⁹F NMR (377 MHz, DMSO-*d*₆) δ -122.53. |
| **54b** | 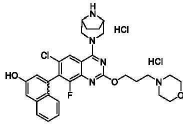 | 4-((*R* or *S*)-4-((1*R*,5*S*)-3,8-diazabicycl o[3.2.1]octa n-3-yl)-6-chloro-8-fluoro-2-(3-morpholino propoxy)qui nazolin-7-yl)naphthale n-2-ol dihydrochlo ride | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 30% phase B in 5 min; detector UV 220/254 nm; retention time: 3.148 min; ee > 99% | 578. 2/58 0.2 | ¹H NMR (400 MHz, DMSO-*d*₆) *δ* 11.24 (s, 1H), 10.20 - 9.94 (m, 2H), 9.73 (s, 1H), 7.99 (d, *J* = 1.6 Hz, 1H), 7.82 (d, *J* = 8.3 Hz, 1H), 7.47 - 7.43 (m, 1H), 7.31 (d, *J* = 2.4 Hz, 1H), 7.25 - 7.19 (m, 2H), 7.10 (d, *J* = 2.4 Hz, 1H), 4.56 - 4.44 (m, 4H), 4.27 - 4.18 (m, 2H), 3.97 - 3.90 (m, 4H), 3.86 - 3.79 (m, 2H), 3.48 - 3.43 (m, 2H), 3.29 - 3.23 (m, 2H), 3.12 - 3.03 (m, 2H), 2.28 - 2.21 (m, 2H), 2.02 - 1.90 (m, 4H); ¹⁹F NMR (377 MHz, DMSO-*d*₆) δ - 122.03. |
| **55a** | 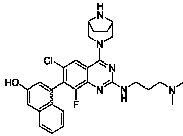 | (*S* or *R*) 4-(4-((1*R,*5*S*)-3,8-diazabicycl o[3.2.1]octa n-3-yl)-6-chloro-2-((3-(dimethyla mino)propyl )amino)-8-fluoroquina zolin-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 8 min; detector UV 220 nm; retention time: 4.211 min; ee > 99% | 535. 2/53 7.2 | ¹H NMR (300 MHz, CD₃OD) δ 7.80 (d, *J =* 1.7 Hz, 1H), 7.76 (d, *J* = 8.5 Hz, 1H), 7.45 - 7.39 (m, 1H), 7.29 - 7.18 (m, 3H), 7.04 (d, *J* = 2.4 Hz, 1H), 4.43 - 4.36 (m, 2H), 3.63 - 3.50 (m, 6H), 2.66 - 2.58 (m, 2H), 2.39 (s, 6H), 1.96 - 1.85 (m, 6H); ¹⁹F NMR (282 MHz, CD₃OD) δ - 126.21. |
| **55b** | 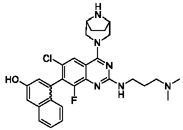 | (*R* or *S*) 4-(4-((1*R*,5*S*)-3,8-diazabicycl o[3.2.1]octa n-3-yl)-6-chloro-2-((3-(dimethyla mino)propyl )amino)-8-fluoroquina zolin-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 8 min; detector UV 220 nm; retention time: 6.385 min; ee > 99% | 535. 2/53 7.2 | ¹H NMR (300 MHz, CD₃OD) δ 7.80 (d, *J =* 1.7 Hz, 1H), 7.75 (d, *J* = 8.3 Hz, 1H), 7.44 - 7.39 (m, 1H), 7.29 - 7.18 (m, 3H), 7.03 (d, *J* = 2.4 Hz, 1H), 4.42 - 4.35 (m, 2H), 3.63 - 3.49 (m, 6H), 2.62 - 2.55 (m, 2H), 2.37 (s, 6H), 1.94 - 1.85 (m, 6H); ¹⁹F NMR (282 MHz, CD₃OD) *δ* - 126.29. |
| **56a** | 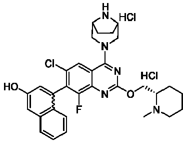 | 4-((*S* or *R*)-4-((1*R*,5*S*)-3,8-diazabicycl o[3.2.1]octa n-3-yl)-6-chloro-8-fluoro-2-((-*S*)-1-methylpiper idin-2-yl)methoxy) quinazolin-7-yl)naphthale n-2-ol dihydrochlo ride | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 6 min; detector UV 254 nm; retention time: 3.762 min; ee > 99% | 562. 3/56 4.2 | ¹H NMR (300 MHz, DMSO-*d*₆) *δ* 10.68 - 9.89 (m, 3H), 9.77 - 9.62 (m, 1H), 8.07 - 7.96 (m, 1H), 7.82 (d, *J* = 8.3 Hz, 1H), 7.50 - 7.40 (m, 1H), 7.31 (d, *J* = 2.4 Hz, 1H), 7.27 - 7.15 (m, 2H), 7.10 (d, *J* = 2.3 Hz, 1H), 4.75 - 4.58 (m, 2H), 4.60 - 4.42 (m, 2H), 4.22 - 4.14 (m, 2H), 3.97 - 3.88 (m, 2H), 3.57 - 3.44 (m, 1H), 3.43 - 3.29 (m, 1H), 3.12 - 2.94 (m, 1H), 2.90 - 2.75 (m, 3H), 2.14 - 1.88 (m, 5H), 1.87 - 1.62 (m, 4H), 1.58 - 1.38 (m, 1H); ¹⁹F NMR (282 MHz, DMSO-*d*₆) δ -121.92. |
| **56b** | 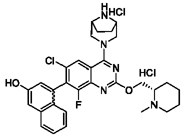 | 4-((*R* or *S*)-4-((1*R,*5*S*)-3,8-diazabicycl o[3.2.1]octa n-3-yl)-6-chloro-8-fluoro-2-((-S)-1-methylpiper idin-2-yl)methoxy) quinazolin-7-yl)naphthale n-2-ol dihydrochlo ride | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 8 min; detector UV 254 nm; retention time: 5.467 min; ee > 99% | 562. 3/56 4.3 | ¹H NMR (300 MHz, DMSO-*d*₆) *δ* 10.60 - 10.04 (m, 2H), 10.00 - 9.89 (m, 1H), 9.74 - 9.59 (m, 1H), 8.02 - 8.00 (m, 1H), 7.82 *(d, J* = 8.3 Hz, 1H), 7.50 - 7.40 (m, 1H), 7.31 (d, *J* = 2.4 Hz, 1H), 7.28 - 7.15 (m, 2H), 7.09 (d, *J* = 2.3 Hz, 1H), 4.73 - 4.44 (m, 4H), 4.23 - 4.14 (m, 2H), 3.89 - 3.78 (m, 2H), 3.59 - 3.44 (m, 1H), 3.43 - 3.30 (m, 1H), 3.10 - 2.95 (m, 1H), 2.90 - 2.75 (m, 3H), 2.09 - 1.88 (m, 5H), 1.87 - 1.66 (m, 4H), 1.57 - 1.38 (m, 1H); ¹⁹F NMR (282 MHz, DMSO-*d*₆) *δ* -121.92. |
| **57a** | 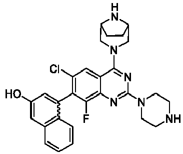 | 4-((*S* or *R*)-4-((1*R*,5*S*)-3,8-diazabicycl o[3.2.1]octa n-3-yl)-6-chloro-8-fluoro-2-(piperazin-1-yl)quinazoli n-7-yl)naphthale n-2-ol | CHIRALPAK ID-3, 4.6 x 50 mm, 3 µm; mobile phase A: *n-*hexane/dichlorom ethane=5/1 (0.1% diethylamine), mobile phase B: isopropanol; flow rate: 1 mL/min; isocratic elution with 30% phase B in 5.5 min; detector UV 254 nm; retention time: 2.173 min; ee > 99% | 519. 3/52 1.2 | ¹H NMR (300 MHz, DMSO-*d*₆) δ 9.97 (s, 1H), 7.79 (d, *J* = 8.3 Hz, 1H), 7.72 (d, *J* = 1.6 Hz, 1H), 7.47 - 7.38 (m, 1H), 7.29 - 7.19 (m, 3H), 7.03 (d, *J* = 2.4 Hz, 1H), 4.18 (d, *J =* 11.9 Hz, 2H), 3.81 - 3.66 (m, 4H), 3.54 - 3.47 (m, 2H), 3.46 - 3.40 (m, 2H), 2.79 - 2.69 (m, 4H), 1.76 - 1.61 (m, 4H); ¹⁹F NMR (282 MHz, DMSO-*d*₆) δ -123.90. |
| **57b** | 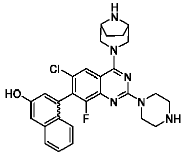 | 4-((*R* or *S*)-4-((1*R*,5*S*)-3,8-diazabicycl o[3.2.1]octa n-3-yl)-6-chloro-8-fluoro-2-(piperazin-1-yl)quinazoli n-7-yl)naphthale n-2-ol | CHIRALPAK ID-3, 4.6 x 50 mm, 3 µm; mobile phase A: *n-*hexane/dichlorom ethane=5/1 (0.1% diethylamine), mobile phase B: isopropanol; flow rate: 1 mL/min; isocratic elution with 30% phase B in 5.5 min; detector UV 254 nm; retention time: 3.537 min; ee > 99% | 519. 3/52 1.1 | ¹H NMR (300 MHz, DMSO-*d*₆) δ 9.98 (s, 1H), 7.79 (d, *J* = 8.3 Hz, 1H), 7.72 (d, *J* = 1.6 Hz, 1H), 7.48 - 7.39 (m, 1H), 7.29 - 7.19 (m, 3H), 7.03 (d, *J* = 2.4 Hz, 1H), 4.18 (d, *J* = 11.9 Hz, 2H), 3.78 - 3.65 (m, 4H), 3.54 - 3.47 (m, 2H), 3.46 - 3.39 (m, 2H), 2.78 - 2.70 (m, 4H), 1.75 - 1.60 (m, 4H); ¹⁹F NMR (282 MHz, DMSO-*d*₆) δ -123.92. |
| **58a** | 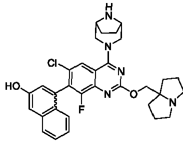 | 4-((*S* or *R)-*4-((1*R*,5*S*)-3,8-diazabicycl o[3.2.1]octa n-3-yl)-6-chloro-8-fluoro-2-((tetrahydro -1*H-*pyrrolin-7*α*(5*H*)-yl)methoxy) quinazolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 220 nm; retention time: 2.045 min; ee > 99% | 574. 3/57 6.2 | ¹H NMR (300 MHz, DMSO-*d*₆) δ 10.03 (s, 1H), 7.93 (d, *J* = 1.6 Hz, 1H), 7.81 (d, *J* = 8.2 Hz, 1H), 7.52 - 7.40 (m, 1H), 7.32 - 7.18 (m, 3H), 7.06 (d, *J* = 2.4 Hz, 1H), 4.42 - 4.25 (m, 2H), 4.02 (s, 2H), 3.60 - 3.46 (m, 4H), 3.01 - 2.85 (m, 2H), 2.62 - 2.52 (m, 2H), 1.96 - 1.84 (m, 2H), 1.84 - 1.70 (m, 4H), 1.70 - 1.50 (m, 6H); ¹⁹F NMR (282 MHz, DMSO-*d*₆) δ - 122.50. |
| **58b** | 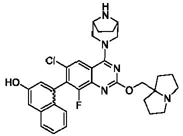 | 4-((*R* or *S*)-4-((1*R,*5*S*)-3,8-diazabicycl o[3.2.1]octa n-3-yl)-6-chloro-8-fluoro-2-((tetrahydro *-*1*H-*pyrrolin-7*α*(5*H*)-yl)methoxy) quinazolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 220 nm; retention time: 3.463 min; ee > 99% | 574. 5/57 6.3 | ¹H NMR (300 MHz, DMSO-*d*₆) δ 10.03 (s, 1H), 7.93 (d, *J* = 1.7 Hz, 1H), 7.80 (d, *J =* 8.3 Hz, 1H), 7.48 - 7.37 (m, 1H), 7.28 (d, *J* = 2.4 Hz, 1H), 7.24 - 7.18 (m, 2H), 7.06 (d, *J* = 2.4 Hz, 1H), 4.40 - 4.28 (m, 2H), 4.02 (s, 2H), 3.61 - 3.47 (m, 4H), 2.99 - 2.87 (m, 2H), 2.63 - 2.53 (m, 2H), 1.96 - 1.84 (m, 2H), 1.83 - 1.70 (m, 4H), 1.69 - 1.51 (m, 6H); ¹⁹F NMR (282 MHz, DMSO-*d*₆) δ - 122.49. |
| **59a** | 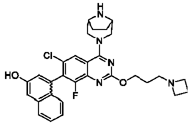 | 4-((*S* or *R*)-2-(3-(azetidin-1-yl)propoxy) -4-((1*R*,5*S*)-3,8-diazabicycl o[3.2.1]octa n-3-yl)-6-chloro-8-fluoroquina zolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 6 min; detector UV 254 nm; retention time: 5.080 min; ee > 99% | 548. 4/55 0.3 | ¹H NMR (400 MHz, CD₃OD) δ 7.97 - 7.90 (m, 1H), 7.74 (d, *J* = 8.3 Hz, 1H), 7.46 - 7.37 (m, 1H), 7.27 - 7.15 (m, 3H), 7.02 (d, *J* = 2.4 Hz, 1H), 4.55 - 4.47 (m, 2H), 4.47 - 4.41 (m, 2H), 3.67 - 3.60 (m, 4H), 3.41 - 3.34 (m, 4H), 2.78 - 2.69 (m, 2H), 2.19 - 2.10 (m, 2H), 1.91 - 1.78 (m, 6H); ¹⁹F NMR (377 MHz, CD₃OD) δ - 123.31. |
| **59b** | 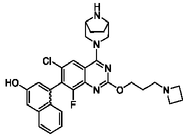 | 4-((*R* or *S*)-2-(3-(azetidin-1-yl)propoxy) -4-((1*R*,5*S*)-3,8-diazabicycl o[3.2.1]octa n-3-yl)-6-chloro-8-fluoroquina zolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 10% phase B in 6 min; detector UV 254 nm; retention time: 3.566 min; ee > 99% | 548. 3/55 0.4 | ¹H NMR (400 MHz, CD₃OD) δ 7.96 - 7.91 (m, 1H), 7.74 (d, *J*= 8.3 Hz, 1H), 7.44 - 7.37 (m, 1H), 7.27 - 7.16 (m, 3H), 7.02 (d, *J*= 2.4 Hz, 1H), 4.54 - 4.48 (m, 2H), 4.47 - 4.41 (m, 2H), 3.68 - 3.57 (m, 4H), 3.40 - 3.33 (m, 4H), 2.76 - 2.63 (m, 2H), 2.18 - 2.08 (m, 2H), 1.90 - 1.79 (m, 6H); ¹⁹F NMR (377 MHz, CD₃OD) δ - 123.27. |
| **60a** | 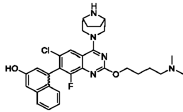 | 4-((*S* or *R*)-4-((1*R*,5*S*)-3,8-diazabicycl o[3.2.1]octa n-3-yl)-6-chloro-2-(4-(dimethyla mino)butox y)-8-fluoroquinazolin-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 15 min; detector UV 254 nm; retention time: 5.980 min; ee > 99% | 550. 4/55 2.3 | ¹H NMR (300 MHz, CD₃OD) δ 7.94 (d, *J=* 1.8 Hz, 1H), 7.74 (d, *J* = 8.3 Hz, 1H), 7.45 - 7.35 (m, 1H), 7.28 - 7.15 (m, 3H), 7.03 (d, *J* = 2.5 Hz, 1H), 4.56 - 4.41 (m, 4H), 3.69 - 3.58 (m, 4H), 2.59 - 2.45 (m, 2H), 2.35 (s, 6H), 1.92 - 1.80 (m, 6H), 1.78 - 1.65 (m, 2H); ¹⁹F NMR (282 MHz, CD₃OD) δ - 123.26. |
| **60b** | 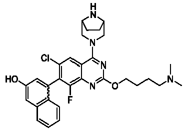 | 4-((*R* or S)-4-((1*R,*5*S*)-3,8-diazabicycl o[3.2.1]octa n-3-yl)-6-chloro-2-(4-(dimethyla mino)butox y)-8-fluoroquina zolin-7-yl)naphthale n-2-ol | CHIRALPAK IC-3, 4.6 x 50 mm, 3 µm; mobile phase A: *n-*hexane/dichlorom ethane=5/1 (0.1% diethylamine), mobile phase B: ethanol; flow rate: 1 mL/min; isocratic elution with 10% phase B in 15 min; detector UV 254 nm; retention time: 10.313 min; ee > 99% | 550. 4/55 2.3 | ¹H NMR (300 MHz, CD₃OD) δ 7.94 (d, *J =* 1.8 Hz, 1H), 7.74 (d, *J* = 8.3 Hz, 1H), 7.45 - 7.36 (m, 1H), 7.27 - 7.15 (m, 3H), 7.03 (d, *J* = 2.4 Hz, 1H), 4.55 - 4.42 (m, 4H), 3.69 - 3.58 (m, 4H), 2.51 - 2.42 (m, 2H), 2.30 (s, 6H), 1.92 - 1.78 (m, 6H), 1.78 - 1.65 (m, 2H); ¹⁹F NMR (282 MHz, CD₃OD) *δ* - 123.25. |
| **61a** | 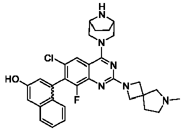 | 4-((*S* or *R*)-4-((1*R*,5*S*)-3,8-diazabicycl o[3.2.1]octa n-3-yl)-6-chloro-8-fluoro-2-(6-methyl-2,6-diazaspiro[3 .4]octan-2-yl)quinazoli n-7-yl)naphthale n-2-ol | CHIRALPAK ID-3, 4.6 x 50 mm, 3 µm; mobile phase A: *n-*hexane/methyl tert-butyl ether=1/1 (0.1% diethylamine), mobile phase B: ethanol; flow rate: 1 mL/min; isocratic elution with 20% phase B in 6 min; detector UV 254 nm; retention time: 2.202 min; ee > 99% | 559. 5/56 1.3 | ¹H NMR (400 MHz, DMSO-*d*₆) δ 10.01 (s, 1H), 7.79 (d, *J* = 8.3 Hz, 1H), 7.76 - 7.72 (m, 1H), 7.46 - 7.39 (m, 1H), 7.26 (d, *J =* 2.4 Hz, 1H), 7.21 (d, *J* = 4.0 Hz, 2H), 7.04 - 7.01 (m, 1H), 4.26 - 4.15 (m, 2H), 4.06 - 3.92 (m, 4H), 3.53 - 3.38 (m, 4H), 2.65 (s, 2H), 2.48 - 2.42 (m, 2H), 2.23 (s, 3H), 2.10 - 2.01 (m, 2H), 1.74 - 1.57 (m, 4H); ¹⁹F NMR (377 MHz, DMSO-*d*₆) δ -123.46. |
| **61b** | 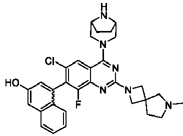 | 4-((*R* or *S*)-4-((1*R*,5*S*)-3,8-diazabicycl o[3.2.1]octa n-3-yl)-6-chloro-8-fluoro-2-(6-methyl-2,6-diazaspiro[3 .4]octan-2-yl)quinazoli n-7-yl)naphthale n-2-ol | CHIRALPAK ID-3, 4.6 x 50 mm, 3 µm; mobile phase A: *n-*hexane/methyl *tert*-butyl ether=1/1 (0.1% diethylamine), mobile phase B: ethanol; flow rate: 1 mL/min; isocratic elution with 20% phase B in 6 min; detector UV 254 nm; retention time: 1.694 min; ee > 99% | 559. 5/56 1.3 | ¹H NMR (400 MHz, DMSO-*d*₆) *δ* 9.99 (s, 1H), 7.79 (d, *J* = 8.3 Hz, 1H), 7.76 - 7.72 (m, 1H), 7.47 - 7.39 (m, 1H), 7.26 (d, *J =* 2.4 Hz, 1H), 7.23 - 7.19 (m, 2H), 7.02 (d, *J* = 2.3 Hz, 1H), 4.27 - 4.16 (m, 2H), 4.04 - 3.94 (m, 4H), 3.52 - 3.38 (m, 4H), 2.66 (s, 2H), 2.48 - 2.42 (m, 2H), 2.23 (s, 3H), 2.10 - 2.03 (m, 2H), 1.76 - 1.60 (m, 4H); ¹⁹F NMR (377 MHz, DMSO-*d*₆) δ -123.46. |
| **62a** | 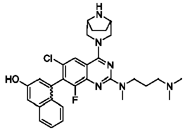 | 4-((*S* or *R*)-4-((1*R*,5*S*)-3,8-diazabicycl o[3.2.1]octa n-3-yl)-6-chloro-2-((3-(dimethyla mino)propyl )(methyl)am ino)-8-fluoroquina zolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 254 nm; retention time: 3.894 min; ee > 99% | 549. 3/55 1.2 | ¹H NMR (400 MHz, CD₃OD) δ 7.80 - 7.70 (m, 2H), 7.43 - 7.35 (m, 1H), 7.29 - 7.22 (m, 2H), 7.21 - 7.14 (m, 1H), 7.01 (d, *J* = 2.3 Hz, 1H), 4.37 - 4.27 (m, 2H), 3.78 - 3.71 (m, 2H), 3.64 - 3.57 (m, 2H), 3.55 - 3.47 (m, 2H), 3.23 (s, 3H), 2.54 - 2.41 (m, 2H), 2.28 (s, 6H), 1.97 - 1.82 (m, 6H); ¹⁹F NMR (282 MHz, CD₃OD) *δ* - 125.43. |
| **62b** | 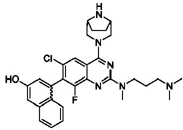 | 4-((*R* or *S*)-4-((1*R,*5*S*)-3,8-diazabicycl o[3.2.1]octa n-3-yl)-6-chloro-2-((3-(dimethyla mino)propyl )(methyl)amino)-8-fluoroquina zolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 254 nm; retention time: 1.814 min; ee > 98% | 549. 3/55 1.3 | ¹H NMR (400 MHz, CD₃OD) δ 7.77 - 7.70 (m, 2H), 7.42 - 7.36 (m, 1H), 7.28 - 7.15 (m, 3H), 7.01 (d, *J* = 2.4 Hz, 1H), 4.37 - 4.25 (m, 2H), 3.78 - 3.70 (m, 2H), 3.62 - 3.58 (m, 2H), 3.55 - 3.47 (m, 2H), 2H), 3.23 (s, 3H), 2.50 - 2.38 (m, 2H), 2.28 (s, 6H), 1.98 - 1.81 (m, 6H); ¹⁹F NMR (282 MHz, CD₃OD) *δ* -125.36. |
| **63a** | 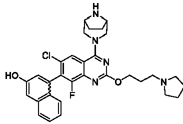 | 4-((*S* or *R*)-4-((1*R*,5*S*)-3,8-diazabicycl o[3.2.1]octa n-3-yl)-6-chloro-8-fluoro-2-(3-(pyrrolidin-1-yl)propoxy) quinazolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 220 nm; retention time: 2.813 min; ee > 99% | 562. 4/56 4.4 | ¹H NMR (300 MHz, DMSO-*d*₆) δ 10.02 (s, 1H), 7.93 (d, *J* = 1.7 Hz, 1H), 7.80 (d, *J* = 8.3 Hz, 1H), 7.48 - 7.38 (m, 1H), 7.31 - 7.17 (m, 3H), 7.09 - 7.03 (m, 1H), 4.41 - 4.25 (m, 4H), 3.62 - 3.44 (m, 4H), 2.55 - 2.52 (m, 2H), 2.45 - 2.36 (m, 4H), 1.96 - 1.80 (m, 2H), 1.75 - 1.56 (m, 8H); ¹⁹F NMR (282 MHz, DMSO-*d*₆) δ -122.48. |
| **63b** | 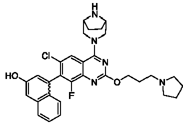 | 4-((*R* or *S*)-4-((1*R*,5*S*)-3,8-diazabicycl o[3.2.1]octa n-3-yl)-6-chloro-8-fluoro-2-(3-(pyrrolidin-1-yl)propoxy) quinazolin-7-yl)naphthale n-2-ol | CHIRALPAK ID-3, 4.6 x 50 mm, 3 µm; mobile phase A: *n-*hexane/dichlorom ethane=3/1 (0.1% diethylamine), mobile phase B: isopropanol; flow rate: 1 mL/min; isocratic elution with 30% phase B in 6 min; detector UV 220 nm; retention time: 1.965 min; ee > 99% | 562. 3/56 4.2 | ¹H NMR (300 MHz, DMSO-*d*₆) δ 10.03 (s, 1H), 7.93 (d, *J* = 1.6 Hz, 1H), 7.81 (d, *J =* 8.3 Hz, 1H), 7.48 - 7.40 (m, 1H), 7.29 (d, *J* = 2.4 Hz, 1H), 7.22 (d, *J* = 3.9 Hz, 2H), 7.06 (d, *J* = 2.4 Hz, 1H), 4.42 - 4.26 (m, 4H), 3.60 - 3.46 (m, 4H), 2.56 - 2.52 (m, 2H), 2.46 - 2.39 (m, 4H), 1.96 - 1.84 (m, 2H), 1.71-1.60 (m, 8H); ¹⁹F NMR (282 MHz, DMSO-*d*₆) δ -122.49. |
| **64a** | 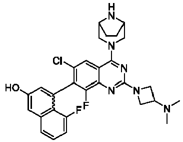 | 4-((*S* or *R)-*4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)-5-fluoronapht halen-2-ol | CHIRALPAK IC-3, 4.6 x 50 mm, 3 µm; mobile phase A: *n-*hexane/dichlorom ethane=5/1 (0.1% diethylamine), mobile phase B: isopropanol; flow rate: 1 mL/min; isocratic elution with 10% phase B in 8 min; detector UV 254 nm; retention time: 3.932 min; ee > 99% | 551. 2/55 3.2 | ¹H NMR (300 MHz, DMSO-*d*₆) δ 10.29 (s, 1H), 7.72 - 7.61 (m, 2H), 7.46 - 7.36 (m, 1H), 7.36 - 7.31 (m, 1H), 7.04 - 6.93 (m, 2H), 4.28 - 4.22 (m, 1H), 4.19 - 4.03 (m, 3H), 3.89 - 3.77 (m, 2H), 3.56 - 3.40 (m, 4H), 3.17 - 3.06 (m, 1H), 2.12 (s, 6H), 1.80 - 1.59 (m, 4H); ¹⁹F NMR (282 MHz, DMSO-*d*₆) δ -115.92, - 124.95. |
| **64b** | 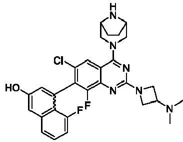 | 4-((*R* or *S*)-4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)-5-fluoronapht halen-2-ol | CHIRALPAK IC-3, 4.6 x 50 mm, 3 µm; mobile phase A: *n-*hexane/dichlorom ethane=5/1 (0.1% diethylamine), mobile phase B: isopropanol; flow rate: 1 mL/min; isocratic elution with 10% phase B in 10 min; detector UV 254 nm; retention time: 7.304 min; ee > 99% | 551. 3/55 3.2 | ¹H NMR (300 MHz, DMSO-*d*₆) *δ* 10.25 (s, 1H), 7.71 - 7.61 (m, 2H), 7.45 - 7.36 (m, 1H), 7.36 - 7.29 (m, 1H), 7.05 - 6.91 (m, 2H), 4.26 - 4.22 (m, 1H), 4.18 - 4.02 (m, 3H), 3.91 - 3.79 (m, 2H), 3.54 - 3.39 (m, 4H), 3.17 - 3.07 (m, 1H), 2.12 (s, 6H), 1.76 - 1.60 (m, 4H); ¹⁹F NMR (282 MHz, DMSO-*d*₆) δ -115.92, - 124.97. |
| **65a** | 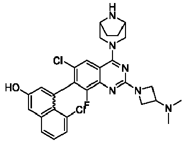 | 4-((*S* or *R)-*4-((1*R*,5*S*)-3,8-diazabicycl o[3.2.1]octa n-3-yl)-6-chloro-2-(3-(dimethyla mino)azetid in-1-yl)-8-fluoroquina zolin-7-yl)-5-chloronapht halen-2-ol | CHIRALPAK IC-3,4.6 x 100 mm, 3 µm; mobile phase A: *n-*hexane/dichlorom ethane=5/1 (0.1% diethylamine), mobile phase B: ethanol; flow rate: 1 mL/min; isocratic elution with 10% phase B in 10 min; detector UV 254 nm; retention time: 4.668 min; ee > 99% | 567. 2/56 9.2 | ¹H NMR (300 MHz, DMSO-*d*₆) *δ* 10.34 (s, 1H), 7.96 - 7.83 (m, 1H), 7.73 (s, 1H), 7.52 - 7.31 (m, 3H), 7.05 (d, *J* = 2.5 Hz, 1H), 4.44 - 4.29 (m, 1H), 4.28 - 4.06 (m, 3H), 4.00 - 3.85 (m, 2H), 3.84 - 3.72 (m, 2H), 3.69 - 3.56 (m, 2H), 3.22 - 3.12 (m, 1H), 2.19 (s, 6H), 1.97 - 1.70 (m, 4H); ¹⁹F NMR (282 MHz, DMSO-*d*₆) δ - 123.63. |
| **65b** | 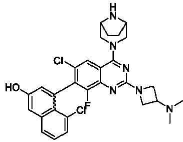 | 4-((*R* or *S*)-4-((1*R,*5*S*)-3,8-diazabicycl o[3.2.1]octa n-3-yl)-6-chloro-2-(3-(dimethyla mino)azetid in-1-yl)-8-fluoroquina zolin-7-yl)-5-chloronapht halen-2-ol | CHIRALPAK IC-3,4.6 x 100 mm, 3 µm; mobile phase A: *n-*hexane/dichlorom ethane=5/1 (0.1% diethylamine), mobile phase B: ethanol; flow rate: 1 mL/min; isocratic elution with 10% phase B in 10 min; detector UV 254 nm; retention time: 6.605 min; ee > 99% | 567. 2/56 9.1 | ¹H NMR (300 MHz, DMSO-*d*₆) *δ* 10.30 (s, 1H), 7.93 - 7.78 (m, 1H), 7.67 (s, 1H), 7.51 - 7.27 (m, 3H), 6.99 (d, *J* = 2.5 Hz, 1H), 4.41 - 4.25 (m, 1H), 4.23 - 4.03 (m, 3H), 3.93 - 3.76 (m, 4H), 3.67 - 3.59 (m, 1H), 3.50 (d, *J* = 12.6 Hz, 1H), 3.17 - 3.04 (m, 1H), 2.12 (s, 6H), 1.93 - 1.74 (m, 4H); ¹⁹F NMR (282 MHz, DMSO-*d*₆) δ - 123.55. |
| **66a** | 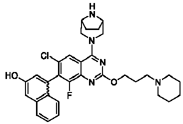 | 4-((*S* or *R)-*4-((1*R*,5*S*)-3,8-diazabicycl o[3.2.1]octa n-3-yl)-6-chloro-8-fluoro-2-(3-(piperidin-1-yl)propoxy) quinazolin-7-yl)naphthale n-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 30% phase B in 4 min; detector UV 254 nm; retention time: 1.465 min; ee > 99% | 576. 3/57 8.3 | ¹H NMR (400 MHz, DMSO-*d*₆) δ 10.03 (s, 1H), 7.93 (d, *J =* 1.6 Hz, 1H), 7.81 (d, *J* = 8.3 Hz, 1H), 7.49 - 7.39 (m, 1H), 7.28 *(d, J* = 2.4 Hz, 1H), 7.22 (d, *J* = 3.9 Hz, 2H), 7.06 (d, *J* = 2.4 Hz, 1H), 4.39 - 4.27 (m, 4H), 3.58 - 3.45 (m, 4H), 2.42 - 2.23 (m, 6H), 1.93 - 1.82 (m, 2H), 1.72 - 1.59 (m, 4H), 1.52 - 1.41 (m, 4H), 1.40 - 1.28 (m, 2H); ¹⁹F NMR (377 MHz, DMSO-*d*₆) *δ* -122.50. |
| **66b** | 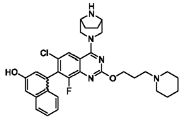 | 4-((*R* or *S*)-4-((1*R*,5*S*)-3,8-diazabicycl o[3.2.1]octa n-3-yl)-6-chloro-8-fluoro-2-(3-(piperidin-1-yl)propoxy) quinazolin-7-yl)naphthale n-2-ol | CHIRALPAK IC-3, 4.6 x 50 mm, 3 µm; mobile phase A: *n-*hexane/dichlorom ethane=3/1 (0.1% diethylamine), mobile phase B: ethanol; flow rate: 1 mL/min; isocratic elution with 30% phase B in 4 min; detector UV 254 nm; retention time: 2.173 min; ee > 99% | 576. 3/57 8.3 | ¹H NMR (400 MHz, DMSO-*d*₆) δ 10.03 (s, 1H), 7.93 (d, *J* = 1.6 Hz, 1H), 7.81 (d, *J* = 8.3 Hz, 1H), 7.48 - 7.40 (m, 1H), 7.28 (d, *J* = 2.4 Hz, 1H), 7.24 - 7.20 (m, 2H), 7.06 (d, *J* = 2.4 Hz, 1H), 4.39 - 4.27 (m, 4H), 3.59 - 3.44 (m, 4H), 2.43 - 2.25 (m, 6H), 1.93 - 1.82 (m, 2H), 1.72 - 1.59 (m, 4H), 1.52 - 1.41 (m, 4H), 1.40 - 1.29 (m, 2H); ¹⁹F NMR (377 MHz, DMSO-*d*₆) δ -122.50. |
| **89a** | 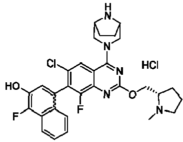 | (*S* or *R*)-4-(4-((1*R,*5*S*)-3,8-diazabicycl o[3.2.1]octa n-3-yl)-6-chloro-8-fluoro-2-(*S*)-1-methylpyrro lidin-2-yl)methoxy) quinazolin-7-yl)-1-fluoronapht halen-2-ol hydrochlori de | CHIR ALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 3.401 min; ee > 95%. | 566. 2/56 8.2 | ¹H NMR (300 MHz, DMSO-*d*₆) *δ* 10.96 (s, 1H), 10.08 (s, 1H), 9.82 (s, 1H), 8.03 - 8.00 (m, 2H), 7.62 - 7.57 (m, 1H), 7.39 - 7.33 (m, 2H), 7.27 (d, *J =* 8.6 Hz, 1H), 4.81 - 4.68 (m, 2H), 4.59 - 4.50 (m, 2H), 4.19 - 4.16 (m, 2H), 4.02 - 3.95 (m, 2H), 3.87 - 3.79 (m, 1H), 3.61 - 3.52 (m, 1H), 3.16 - 3.05 (m, 1H), 2.93 (d, *J =* 4.7 Hz, 3H), 2.31 - 2.20 (m, 1H), 2.06 - 1.84 (m, 7H); ¹⁹F NMR (282 MHz, DMSO-*d*₆) *δ* -121.70, - 148.53. |
| **89b** | 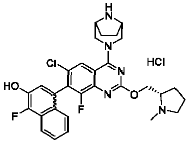 | (*R* or *S*)-4-(4-((1*R*,5*S*)-3,8-diazabicycl o[3.2.1]octa n-3-yl)-6-chloro-8-fluoro-2-(*S*)-1-methylpyrro lidin-2-yl)methoxy) quinazolin-7-yl)-1-fluoronapht halen-2-ol hydrochlori de | CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 4.503 min; ee > 99%. | 566. 2/56 8.2 | ¹H NMR (300 MHz, DMSO-*d*₆) *δ* 10.85 (s, 1H), 10.01 (s, 1H), 9.75 (s, 1H), 8.03 - 8.00 (m, 2H), 7.63 - 7.57 (m, 1H), 7.39 - 7.34 (m, 2H), 7.27 (d, *J =* 8.6 Hz, 1H), 4.76-4.73 (m, 2H), 4.59 - 4.51 (m, 2H), 4.19 - 4.16 (m, 2H), 4.00 - 3.92 (m, 2H), 3.87 - 3.79 (m, 1H), 3.62 - 3.54 (m, 1H), 3.17 - 3.06 (m, 1H), 2.93 (d, *J* = 4.8 Hz, 3H), 2.31 - 2.21 (m, 1H), 2.06 - 1.87 (m, 7H); ¹⁹F NMR (282 MHz, DMSO-*d₆*) *δ* -121.71, - 148.51. |
| **90a** | 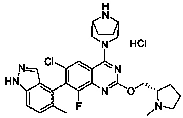 | 4-((1*R*,5*S*)-3,8-diazabicycl o[3.2.1]octa n-3-yl)-6-chloro-8-fluoro-7-((*R* or *S*)-5-methyl-1*H-*indazol-4-yl)-2-((*S*)-1-methylpyrro lidin-2-yl)methoxy) quinazoline hydrochloride | CHIR ALPAK IC-3, 3.0 × 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 1.442 min; ee > 99%. | 536. 3/53 8.2 | ¹H NMR (300 MHz, DMSO-*d₆*) *δ* 10.91 (s, 1H), 10.03 (s, 1H), 9.79 (s, 1H), 8.03 (d, *J* = 1.6 Hz, 1H), 7.60 (d, *J* = 8.6 Hz, 1H), 7.54 (s, 1H), 7.40 (d, *J* = 8.6 Hz, 1H), 4.77 - 4.73 (m, 2H), 4.55 - 4.49 (m, 2H), 4.19 - 4.16 (m, 2H), 3.98 - 3.94 (m, 2H), 3.86 - 3.79 (m, 1H), 3.61 - 3.54 (m, 1H), 3.14 - 3.04 (m, 1H), 2.93 (d, *J* = 4.8 Hz, 3H), 2.31 - 2.22 (m, 1H), 2.17 (s, 3H), 2.06 - 1.85 (m, 7H); ¹⁹F NMR (282 MHz, DMSO-*d₆*) *δ* - 122.23. |
| **90b** | 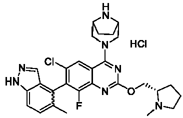 | 4-((1*R*,5*S*)-3,8-diazabicycl o[3.2.1]octa n-3-yl)-6-chloro-8-fluoro-7-((*S* or *R*)-5-methyl-177-indazol-4-yl)-2-((*S*)-1-methylpyrro lidin-2-yl)methoxy) quinazoline hydrochlori de | CHIR ALPAK IC-3, 3.0 x 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 2.226 min; ee > 99%. | 536. 2/53 8.2 | ¹H NMR (300 MHz, DMSO-*d₆*) *δ* 11.04 (s, 1H), 10.08 (s, 1H), 9.85 (s, 1H), 8.03 (d, *J* = 1.6 Hz, 1H), 7.60 (d, *J* = 8.6 Hz, 1H), 7.54 (s, 1H), 7.39 (d, *J* = 8.6 Hz, 1H), 4.78 - 4.74 (m, 2H), 4.55 - 4.49 (m, 2H), 4.18 - 4.16 (m, 2H), 4.00 - 3.93 (m, 2H), 3.87 - 3.79 (m, 1H), 3.62 - 3.53 (m, 1H), 3.15 - 3.04 (m, 1H), 2.93 (d, *J* = 4.8 Hz, 3H), 2.30 - 2.23 (m, 1H), 2.17 (s, 3H), 2.06 - 1.86 (m, 7H); ¹⁹F NMR (282 MHz, DMSO-*d₆*) *δ* - 122.22. |

### Embodiment 7 (Synthesis method VI)

### (S or R)-4-(4-((1R,5S)-3,8-diazabicyclo[3.2.1]octan-3-yl)-6-chloro-2-((6-(dimethylamino)hexyl)oxy)-8-fluoroquinazolin-7-yl)naphthalen-2-ol 67

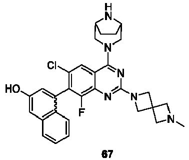

The synthetic route was as follows:

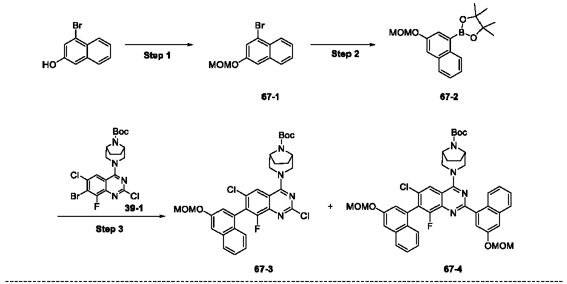

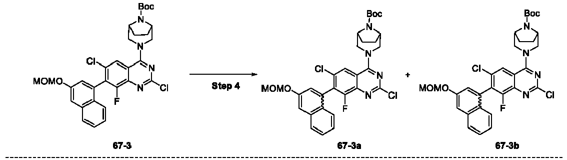

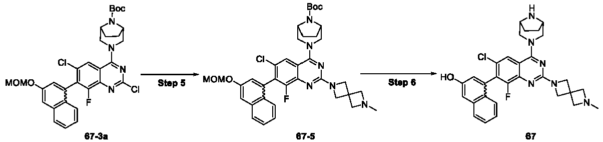

### Step 1:

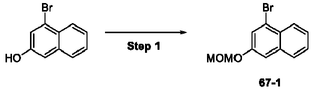

*N,N*-diisopropylethylamine (8.69 g, 66.57 mmol, 1.5 eq) and chloromethyl methyl ether (4.69 g, 57.69 mmol, 1.3 eq) were added to a solution of 1-bromo-3-hydroxynaphthalene (10 g, 44.38 mmol, 1.0 eq) in dichloromethane (100 mL) with stirring under the protection of nitrogen at 25 °C. The reaction was carried out for 3 hours at 25 °C, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 10% ethyl acetate/petroleum ether mobile phase, and the obtained fractions were evaporated under reduced pressure to obtain compound **67-1** (white solid, 10.5 g, yield: 87%). MS (ESI, *m*/*z*): 267.1/269.1 [M+H]⁺; ¹H NMR (300 MHz, CDCl₃) *δ* 8.16 - 8.11 (m, 1H), 7.75 - 7.71 (m, 1H), 7.57 (d, *J* = 2.4 Hz, 1H), 7.50 - 7.43 (m, 2H), 7.39 (d, *J* = 2.4 Hz, 1H), 5.28 (s, 2H), 3.52 (s, 3H).

### Step 2:

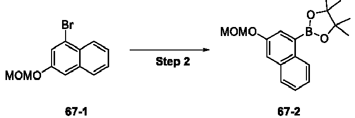

Potassium acetate (14.70 g, 142.26 mmol, 4.0 eq), bis(pinacolato)diboron (12.36 g, 46.23 mmol, 1.3 eq) and [1,1'-bis(diphenylphosphino)ferrocene]dichloropalladium (3.05 g, 3.55 mmol, 0.1 eq) were successively added to a solution of **67-1** (10 g, 35.56 mmol, 1.0 eq) in 1,4-dioxane (100 mL)with stirring under the protection of nitrogen at 25 °C. The reaction was carried out for 1 hour at 100 °C under nitrogen atmosphere, and the reaction process was monitored by liquid chromatography-mass spectrometry. After the reaction was completed, the reaction mixture was cooled to 25 °C, and concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to obtain compound **67-2** (white solid, 10 g, yield: 85%). MS (ESI, *m*/*z*): 315.2 [M + H]⁺; ¹H NMR (300 MHz, CDCl₃) *δ* 8.72 - 8.66 (m, 1H), 7.82 (d, *J* = 2.7 Hz, 1H), 7.79 - 7.73 (m, 1H), 7.51 (d, *J* = 2.7 Hz, 1H), 7.49 - 7.40 (m, 2H), 5.33 (s, 2H), 3.54 (s, 3H), 1.44 (s, 12H).

### Step 3:

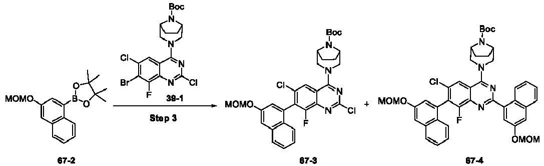

Compound **39-1** (9 g, 16.89 mmol, 1.0 eq), **67-2** (5.59 g, 16.89 mmol, 1.0 eq), 1,4-dioxane (80 mL), water (20 mL), sodium carbonate (3.77 g, 33.78 mmol, 2.0 eq) and tridibenzylidene acetone dipalladium (0) (775 mg, 0.85 mmol, 0.05 eq), 3-(*tert*-butyl)-4-(2,6-dimethoxyphenyl)-2,3-dihydrobenzo[*d*][1,3]oxaphosphole (558 mg, 1.69 mmol, 0.1 eq) were successively added to a 250 mL three-neck flask under the protection of nitrogen at 25 °C. The reaction was carried out for 2 hours at 60 °C under nitrogen atmosphere. The reaction process was monitored by liquid chromatography-mass spectrometry. After the reaction was completed, the reaction mixture was concentrated to obtain a crude product. The crude product was purified by silica gel column chromatography, eluted with a gradient of 0% → 20% ethyl acetate/petroleum ether mobile phase, and the obtained fractions were evaporated under reduced pressure to obtain a mixture of **67-3** and **67-4.** The mixture was purified by reversed-phase chromatographic column (C18 column), eluted with 40% → 90% acetonitrile/water (0.1% ammonium bicarbonate) mobile phase in 20 min; detector: UV254/220 nm; compound **67-3** (a racemic mixture of two stereoisomers, yellow solid, 6 g, yield: 55%) and **67-4** (a racemic mixture of two stereoisomers, yellow solid, 5 g, yield: 37%) were obtained.

Compound **67-3:** MS (ESI, m/z): 613.2/615.2 [M + H]⁺; ¹H NMR (400 MHz, CDCl₃) δ 7.88 - 7.85 (m, 2H), 7.58 (d, *J* = 2.5 Hz, 1H), 7.53 - 7.47 (m, 1H), 7.32 - 7.30 (m, 2H), 7.21 (d, *J* = 2.4 Hz, 1H), 5.36 (s, 2H), 4.56 - 4.41 (m, 4H), 3.77 - 3.65 (m, 2H), 3.58 (s, 3H), 2.06 - 2.00 (m, 2H), 1.87 - 1.79 (m, 2H), 1.56 (s, 9H).

Compound **67-4:** MS (ESI, m/z): 765.2/767.2 [M + H]⁺; ¹H NMR (300 MHz, CDCl₃) δ 8.86 (d, *J* = 8.4 Hz, 1H), 8.03 (d, *J* = 2.5 Hz, 1H), 7.96 (s, 1H), 7.89 - 7.81 (m, 2H), 7.59 (d, *J* = 2.1 Hz, 1H), 7.56 (d, *J* = 2.1 Hz, 1H), 7.53 - 7.32 (m, 5H), 7.29 - 7.27 (m, 1H), 5.40 - 5.35 (m, 4H), 4.64 - 4.42 (m, 4H), 3.80 - 3.68 (m, 2H), 3.59 (s, 3H), 3.57 (s, 3H), 2.07 - 1.93 (m, 4H), 1.56 (s, 9H).

### Step 4:

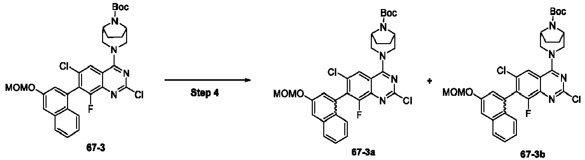

Compound **67-3** (6 g) obtained in step 3 was subjected to chiral resolution, and the resolution conditions were: chiral column NBCHIRALPAK IC, 5 × 25 cm, 5 µm; mobile phase A: supercritical carbon dioxide fluid, mobile phase B: ethanol; flow rate: 160 mL/min; column temperature: 35 °C; elution with 45% mobile phase B in 15 min; detector UV225 nm. Two products were obtained, the product with shorter retention time (9.03 min) was **67-3a,** *tert*-butyl (1*R*,5*S*)-3-(2,6-dichloro-8-fluoro-7-((*S* or *R*)-3-(methoxymethoxy)naphthalen-1-yl)quinazolin-4-yl)-3,8-diazabicyclo[3.2.1]octane-8-carboxylate (yellow solid, 2.5 g, recovery rate: 41%), specific rotation of compound **68-3a:** [α]D²⁵ = 15.4 (*c* = 0.100 g/100 mL, methanol, ee > 99%); the product with longer retention time (10.78 min) was **67-3b,** *tert*-butyl (1*R*,5*S*)-3-(2,6-dichloro-8-fluoro-7-((*R* or *S*)-3-(methoxymethoxy)naphthalen-1-yl)quinazolin-4-yl)-3,8-diazabicyclo[3.2.1]octane-8-carboxylate (yellow solid, 2.9 g, recovery rate: 48%).

### Step 5:

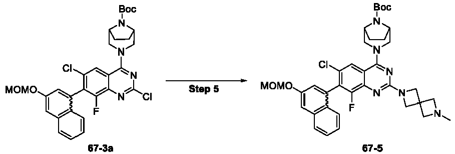

Potassium tert-butoxide (1 mol/mL tetrahydrofuran solution, 0.65 mL, 0.65 mmol, 4.0 eq) was added dropwise to a solution of **67-3a** (100 mg, 0.16 mmol, 1.0 eq) and 2-methyl-2,6-diazaspiro[3.3]heptane ditrifluoroacetate (80 mg, 0.23 mmol, 1.5 eq) in tetrahydrofuran (1 mL) with stirring at 20 °C. After the dropwise addition, the reaction was carried out at 20 °C for 1 hour, and the reaction process was monitored by liquid chromatography-mass spectrometry. After the reaction was completed, 5 mL of water was added to the reaction solution, and the mixture was extracted with ethyl acetate (5 mL × 3), then the organic phases were combined, dried over anhydrous sodium sulfate, filtered, and the filtrate was concentrated to obtain a crude product. The obtained crude product was purified by silica gel column chromatography, eluted with a gradient of 0%—>10% methanol/dichloromethane mobile phase, and the obtained fractions were evaporated under reduced pressure to remove the solvent to obtain compound **67-5** (light yellow solid, 45 mg, yield: 42%). MS (ESI, m/z): 689.3/691.4 [M + H]⁺; ¹H NMR (300 MHz, DMSO-*d₆*) δ 7.93 (d, *J* = 8.3 Hz, 1H), 7.82 (d, *J* = 1.7 Hz, 1H), 7.61 (d, *J* = 2.5 Hz, 1H), 7.55 - 7.50 (m, 1H), 7.37 - 7.26 (m, 2H), 7.23 (d, *J* = 2.5 Hz, 1H), 5.38 (s, 2H), 4.34 - 4.24 (m, 4H), 4.18 - 4.16 (m, 4H), 3.59 - 3.46 (m, 9H), 2.37 (s, 3H), 1.86 - 1.74 (m, 4H), 1.47 (s, 9H).

### Step 6:

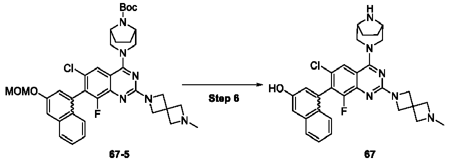

Trifluoroacetic acid (0.3 mL) and triethylsilane (30 mg, 0.26 mmol, 4.0 eq) were added to a solution of **67-5** (45 mg, 0.06 mmol, 1.0 eq) in dichloromethane (0.8 mL) with stirring at 0 °C. The reaction was carried out for 1 hour at 25 °C, and the reaction process was monitored by liquid chromatography-mass spectrometry. After the reaction was completed, the reaction mixture was concentrated to obtain a crude product. The crude product was prepared and purified by high performance liquid chromatography under the following conditions: chromatographic column: XBridge Prep C18 OBD, 19 × 150 mm, 5 µm; mobile phase A: water (10 mmol/L ammonium bicarbonate solution), mobile phase B: acetonitrile; flow rate: 25 mL/min; gradient: elution with 10% B in 2 min, then elution with a gradient of 10% B to 21% B in 2.5 min, and finally elution with a gradient of 21% B to 45% B in 10.5 min; detector UV220 nm; retention time: 9.62 min. The obtained fraction was concentrated under reduced pressure to obtain **67** (white solid, 11.3 mg, yield: 31%). MS (ESI, m/z): 545.3/547.4 [M + H]⁺; ¹H NMR (300 MHz, DMSO-*d₆*) *δ* 9.98 (s, 1H), 7.79 (d, *J* = 8.3 Hz, 1H), 7.75 (d, *J* = 1.6 Hz, 1H), 7.46 - 7.41 (m, 1H), 7.26 (d, *J* = 2.4 Hz, 1H), 7.25 - 7.19 (m, 2H), 7.03 (d, *J* = 2.4 Hz, 1H), 4.25 - 4.19 (m, 2H), 4.11 (s, 4H), 3.51 - 3.40 (m, 4H), 3.26 (s, 4H), 2.18 (s, 3H), 1.72 - 1.61 (m, 4H); ¹⁹F NMR (282 MHz, DMSO-*d₆*) *δ* -123.43.

Other similar compounds of the present disclosure can be prepared by the synthetic method shown in Embodiment 7 above. Some compounds prepared with reference to the above synthetic method and their characterization data are shown in table 6.

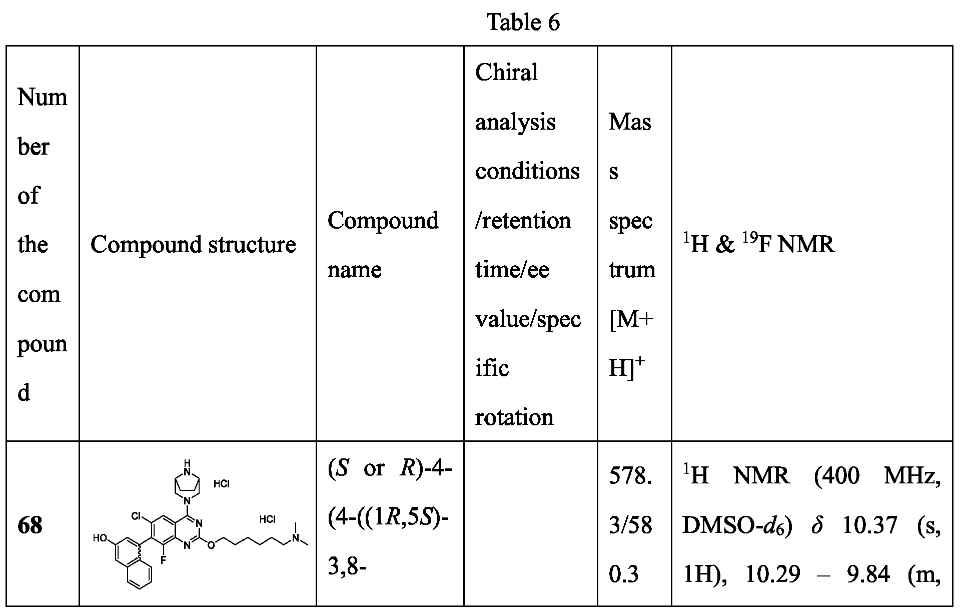

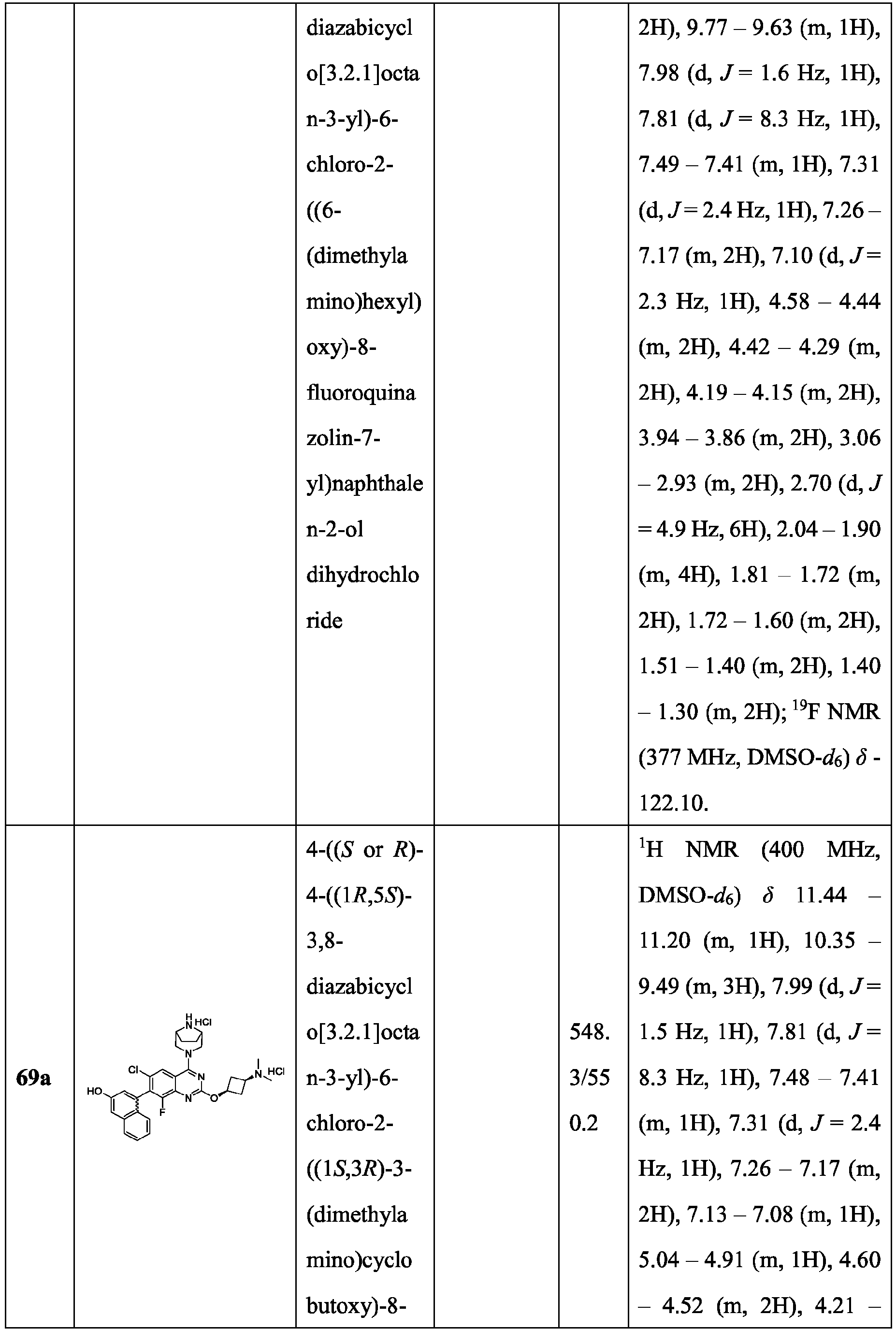

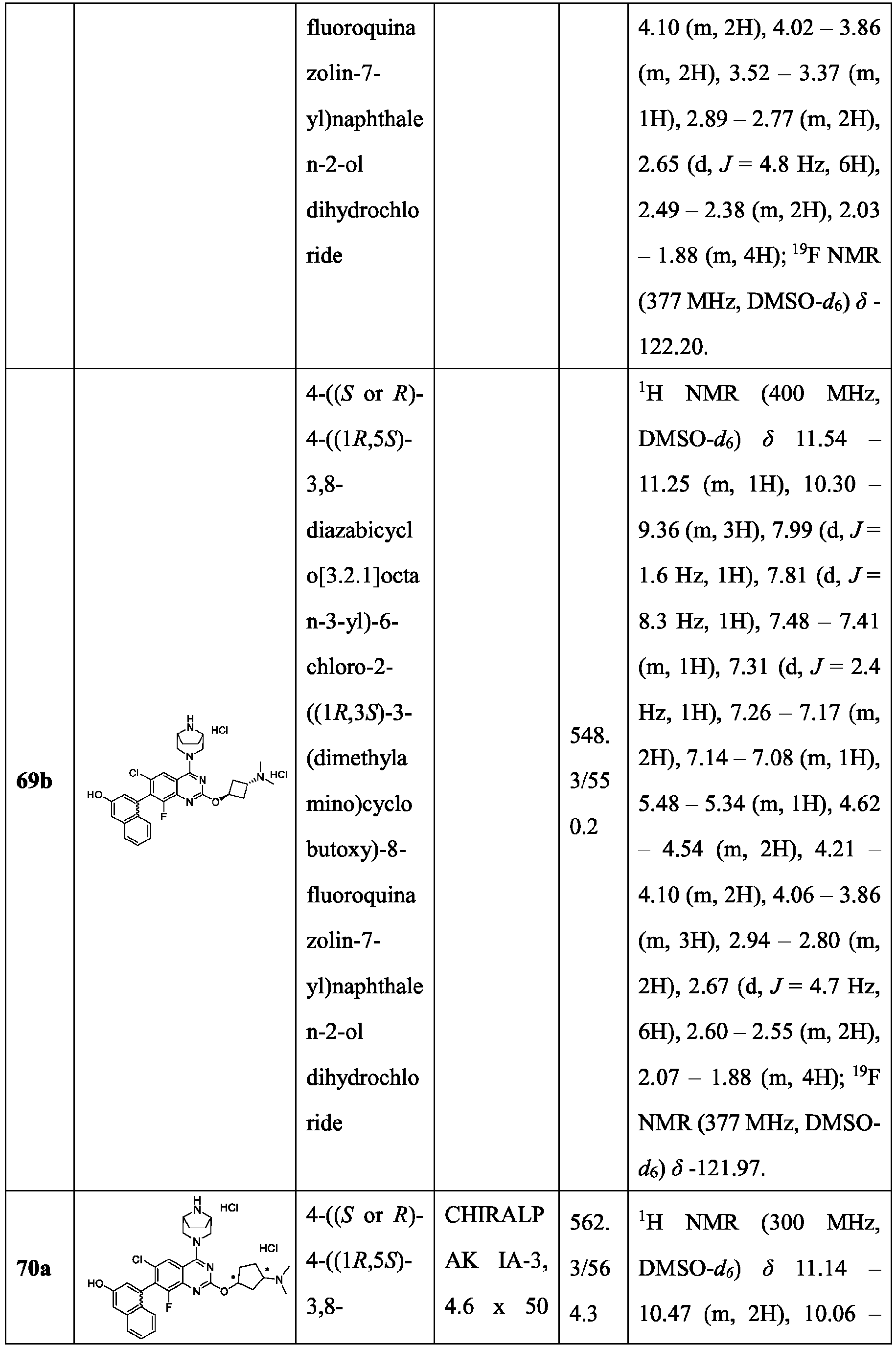

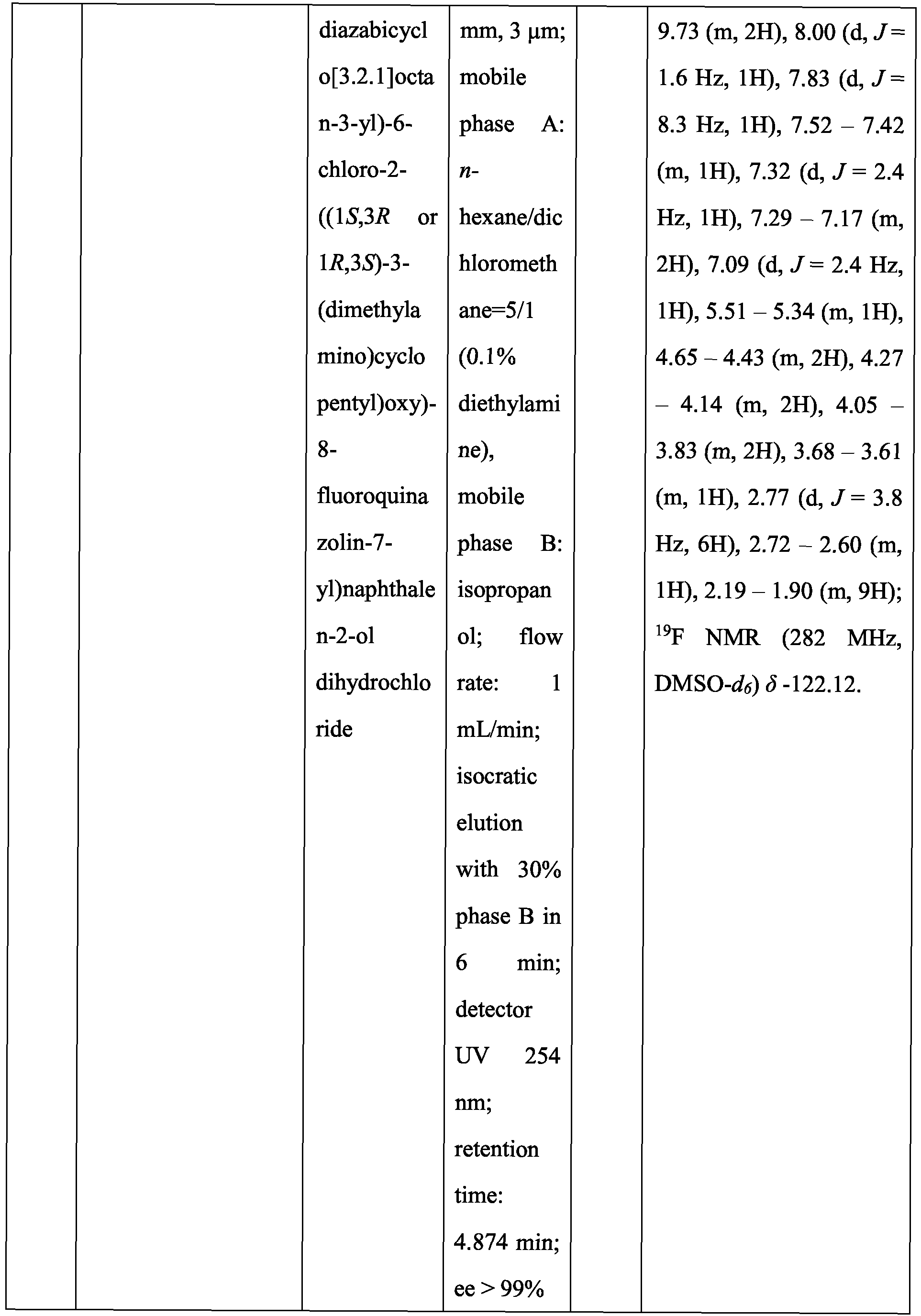

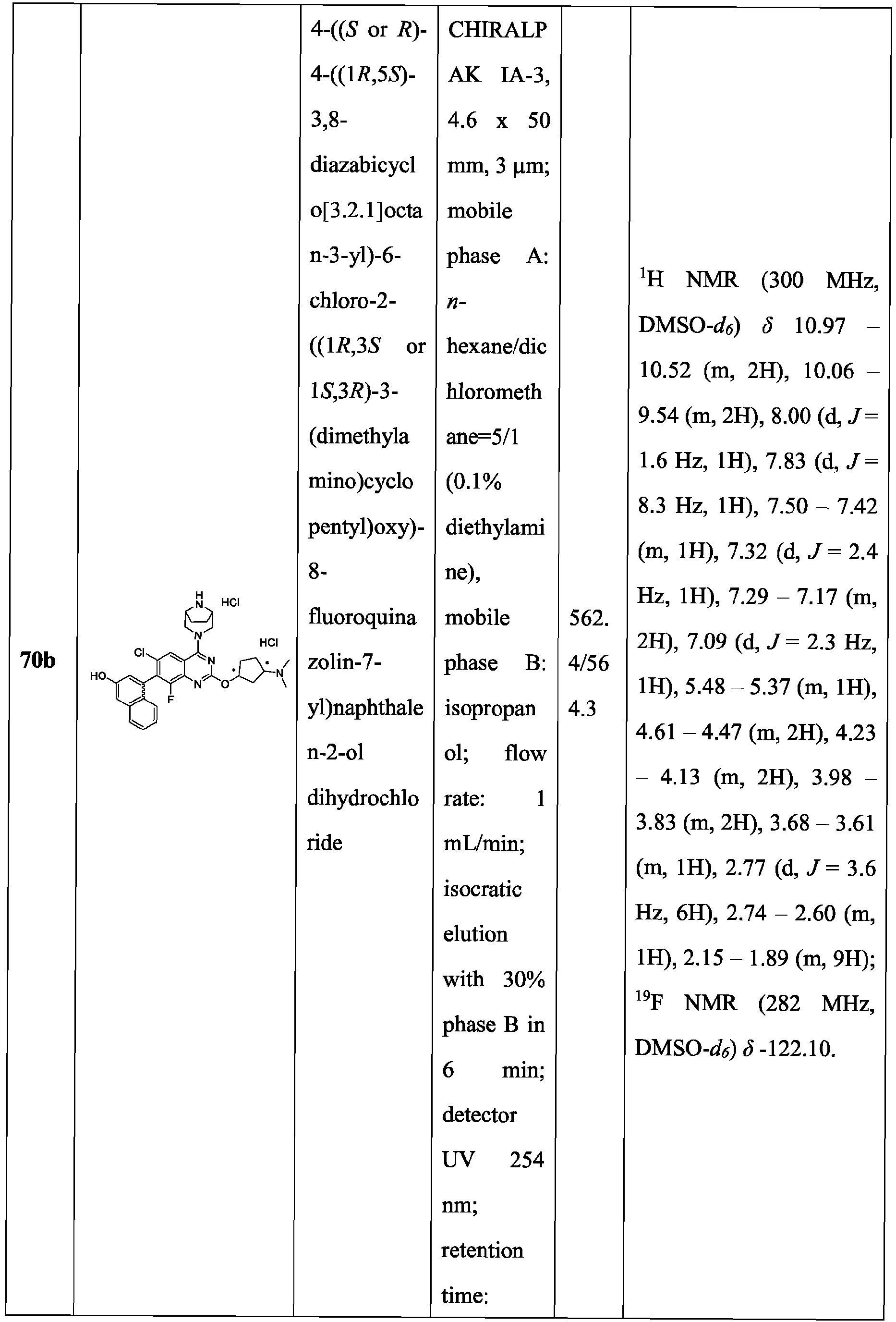

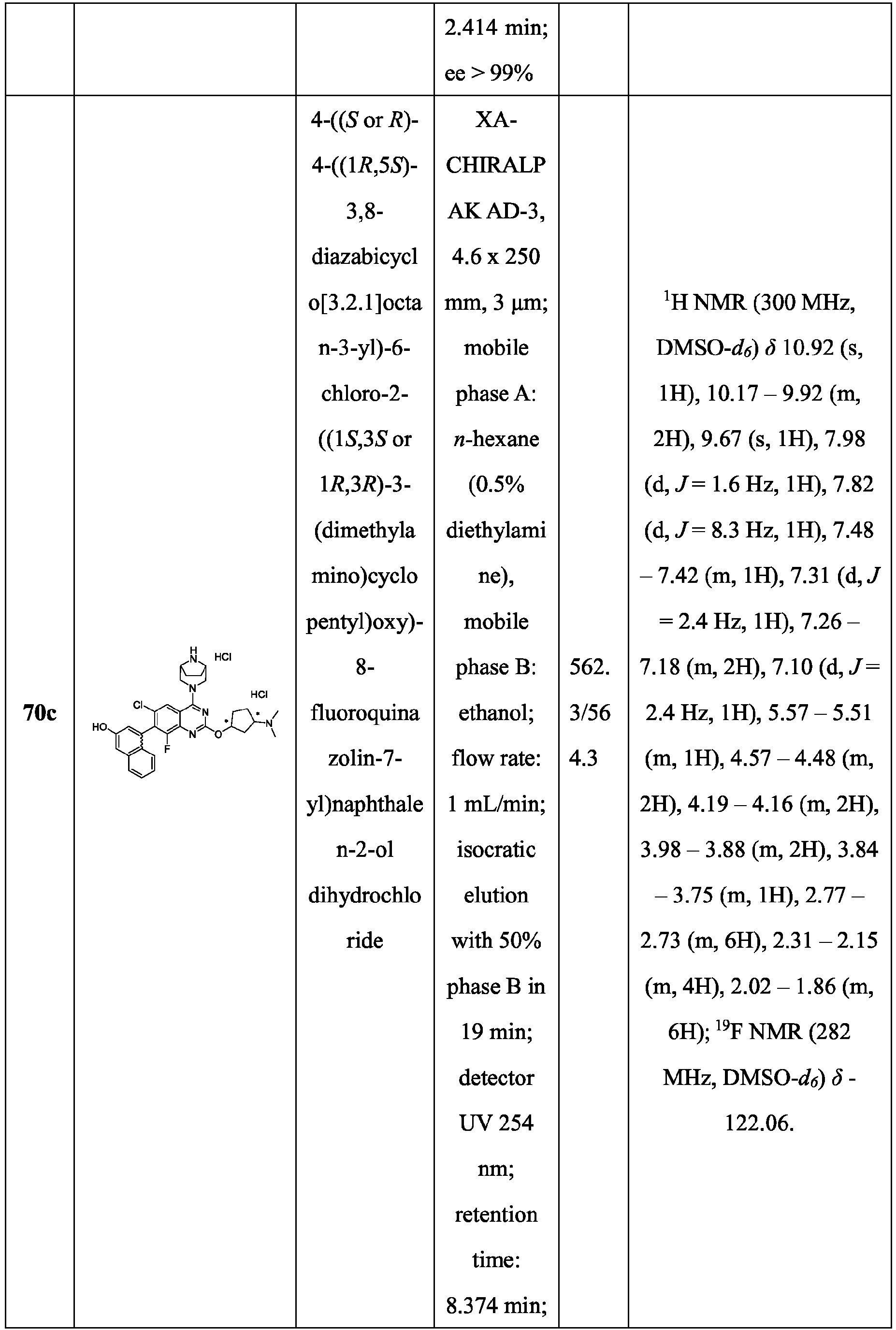

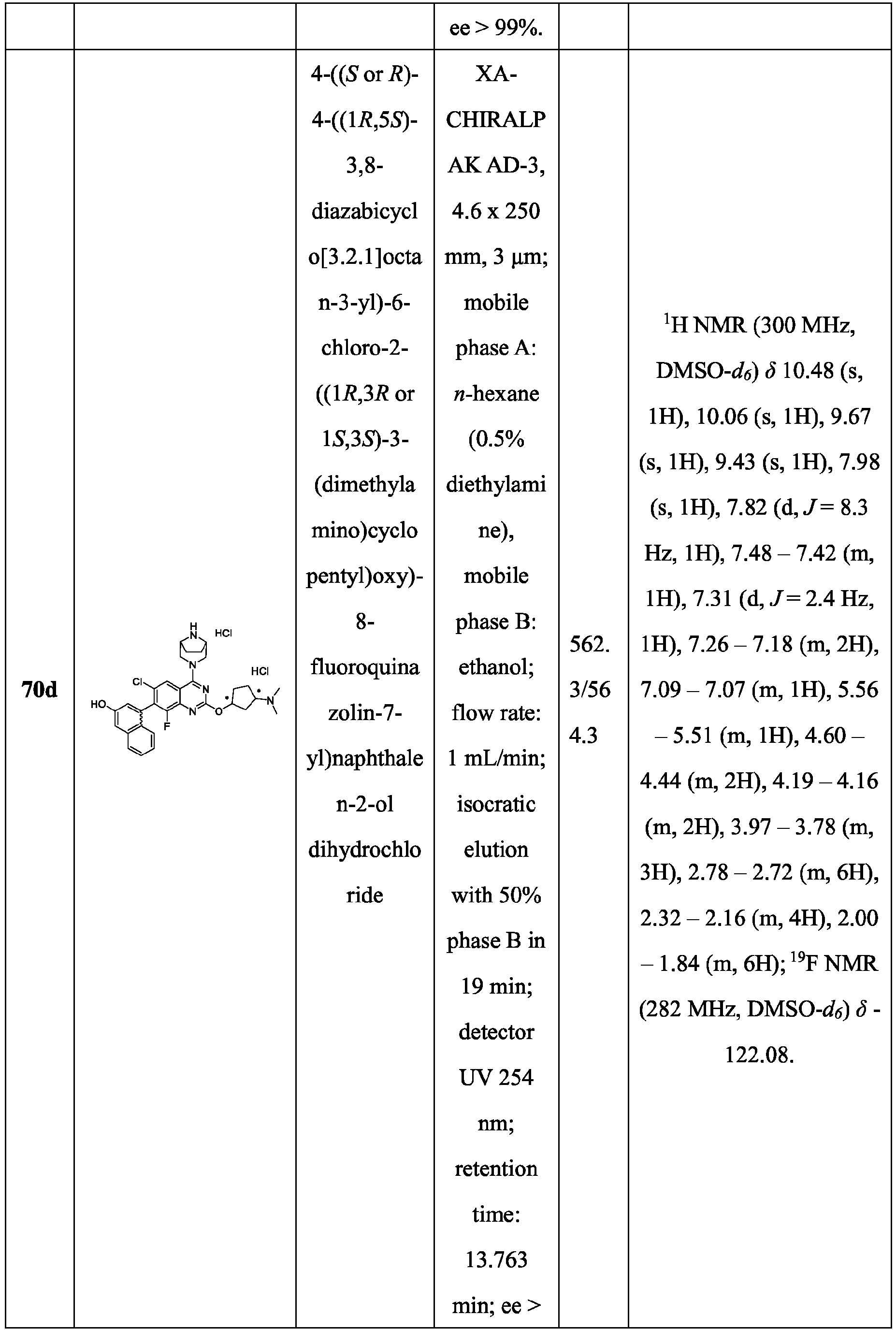

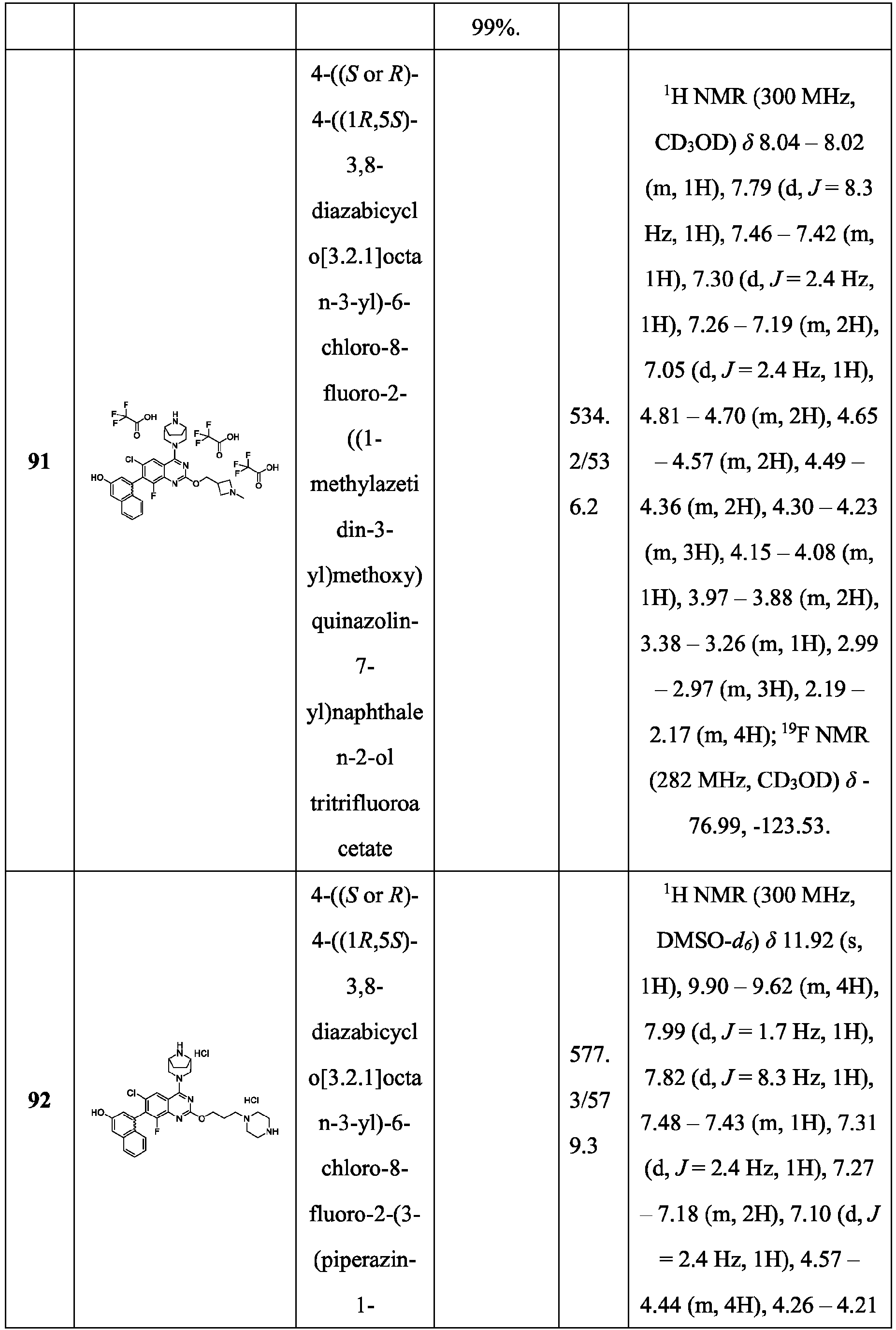

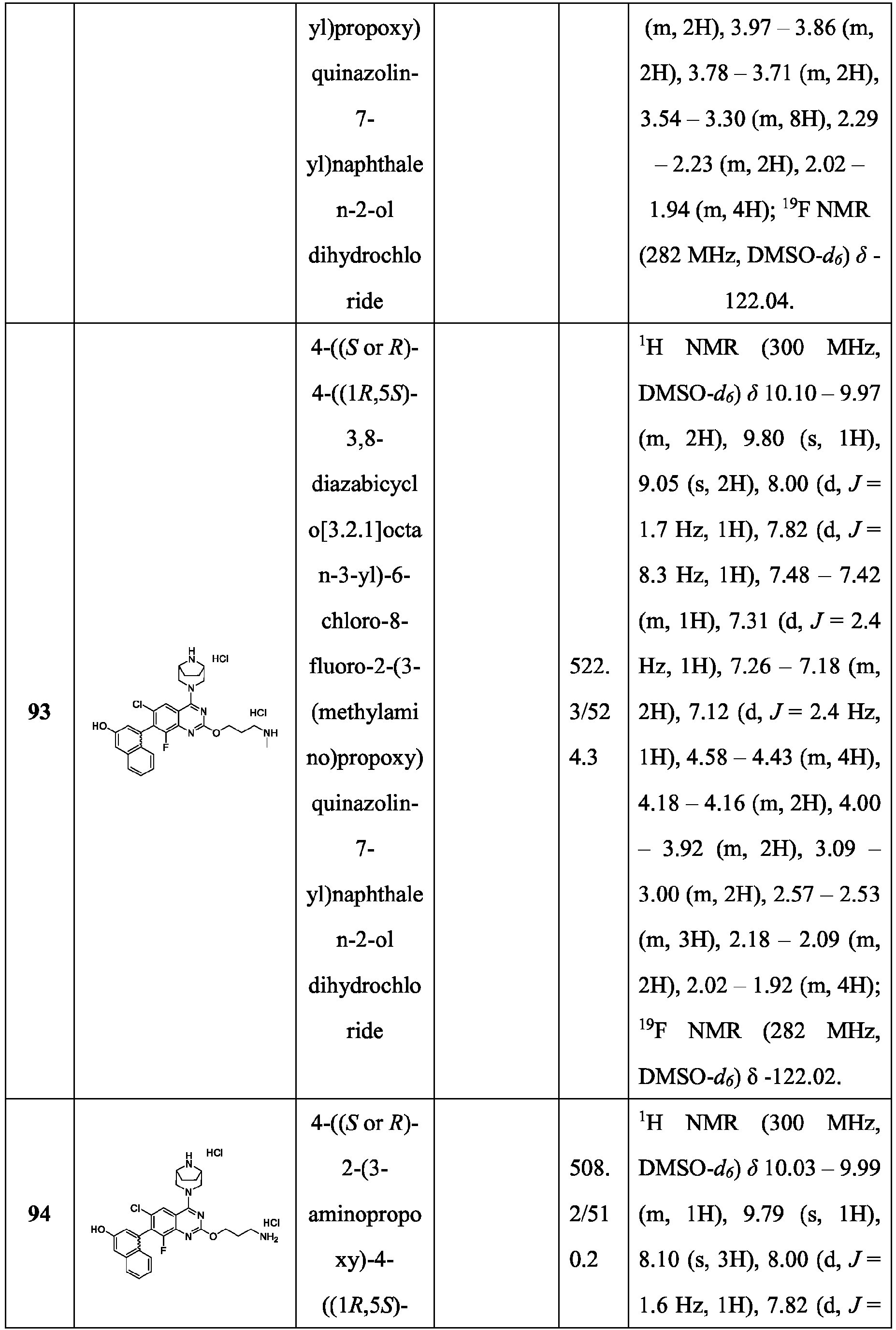

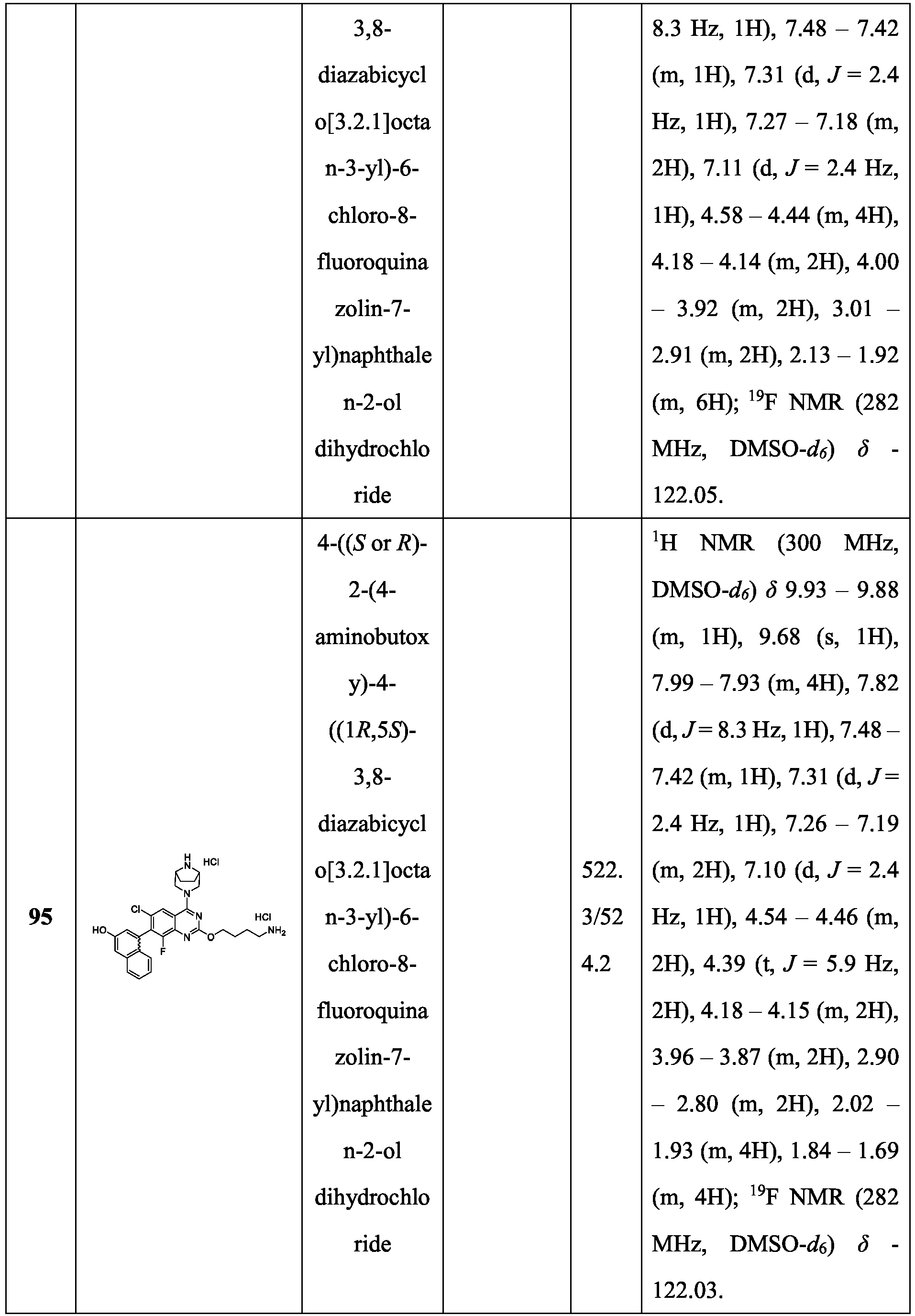

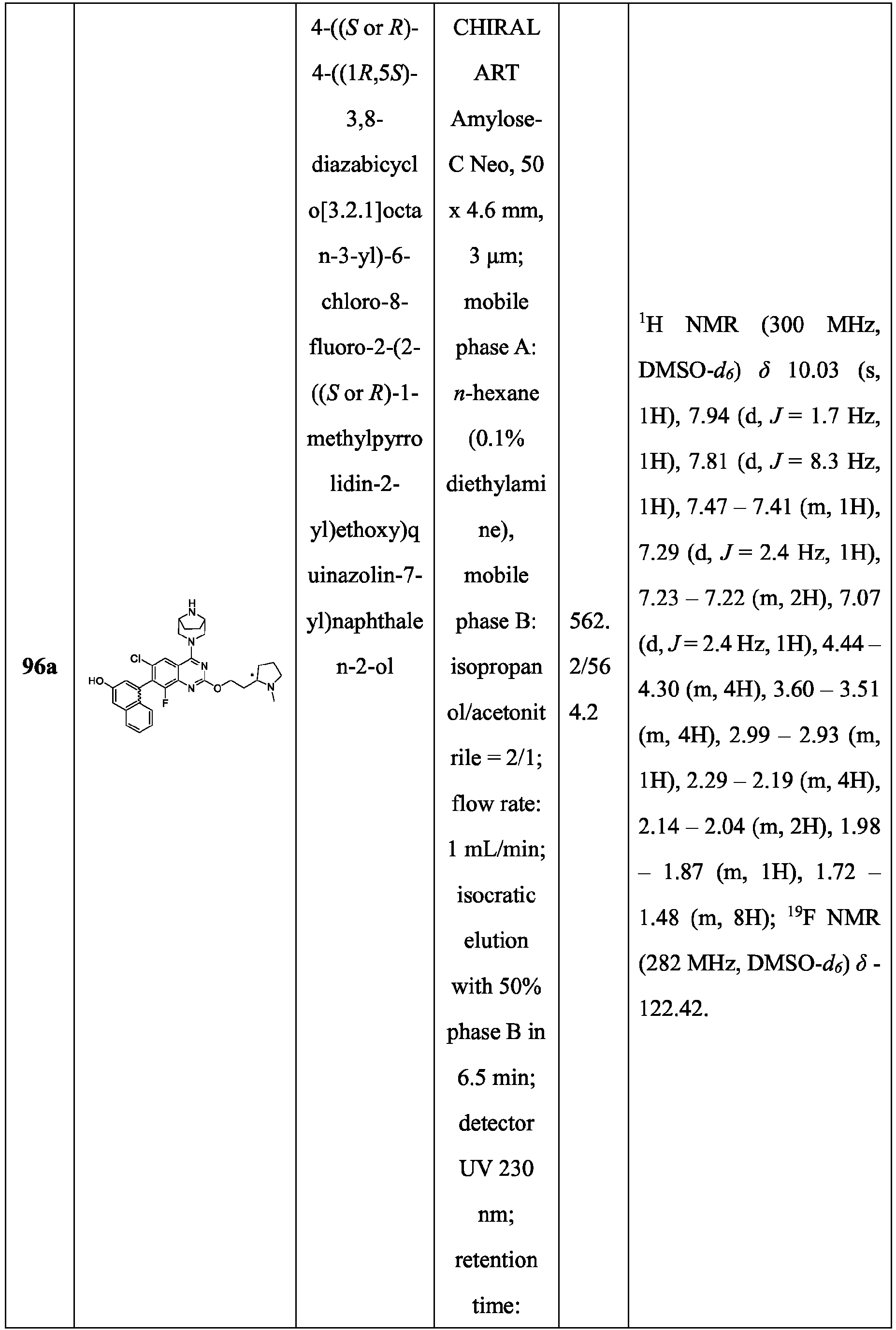

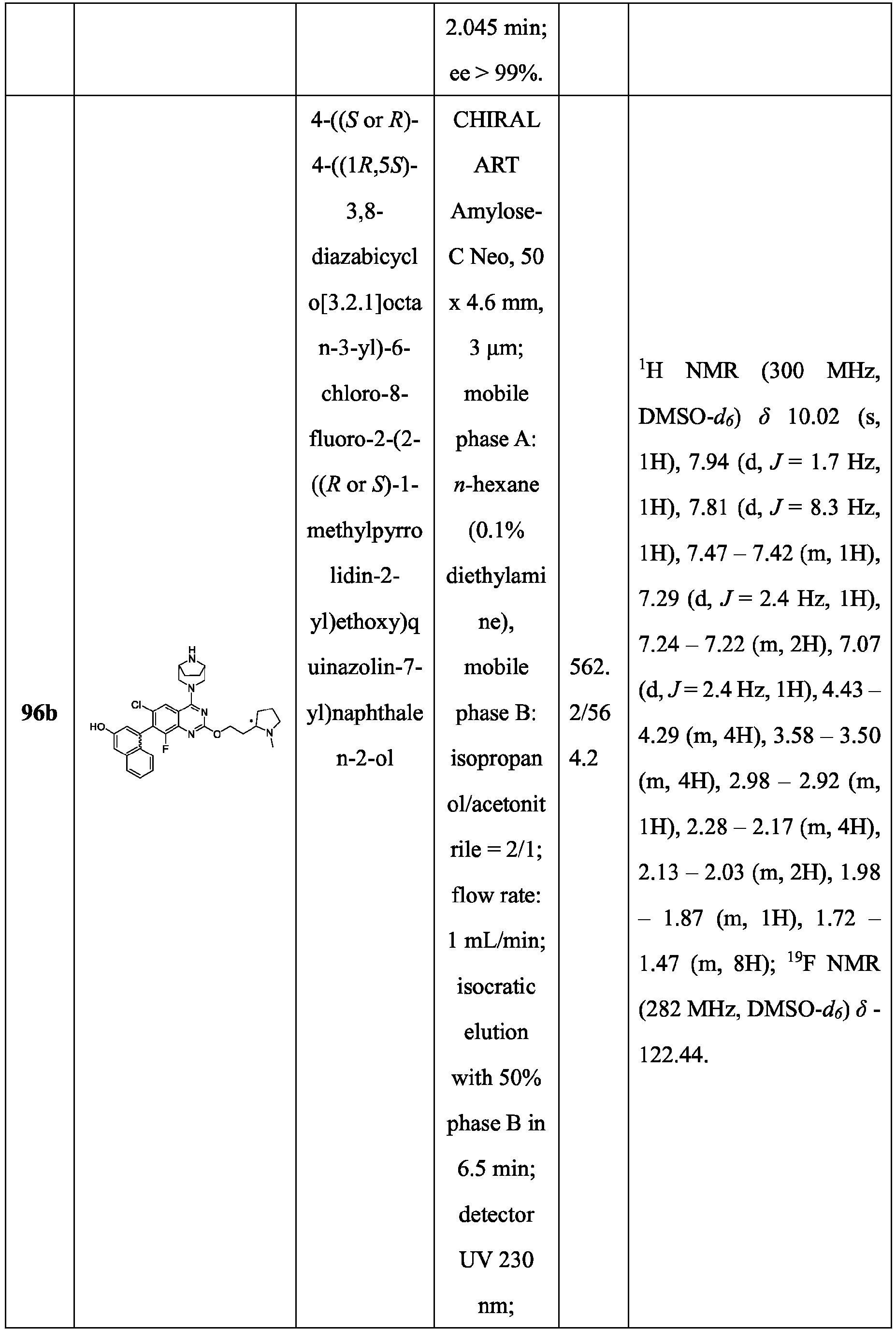

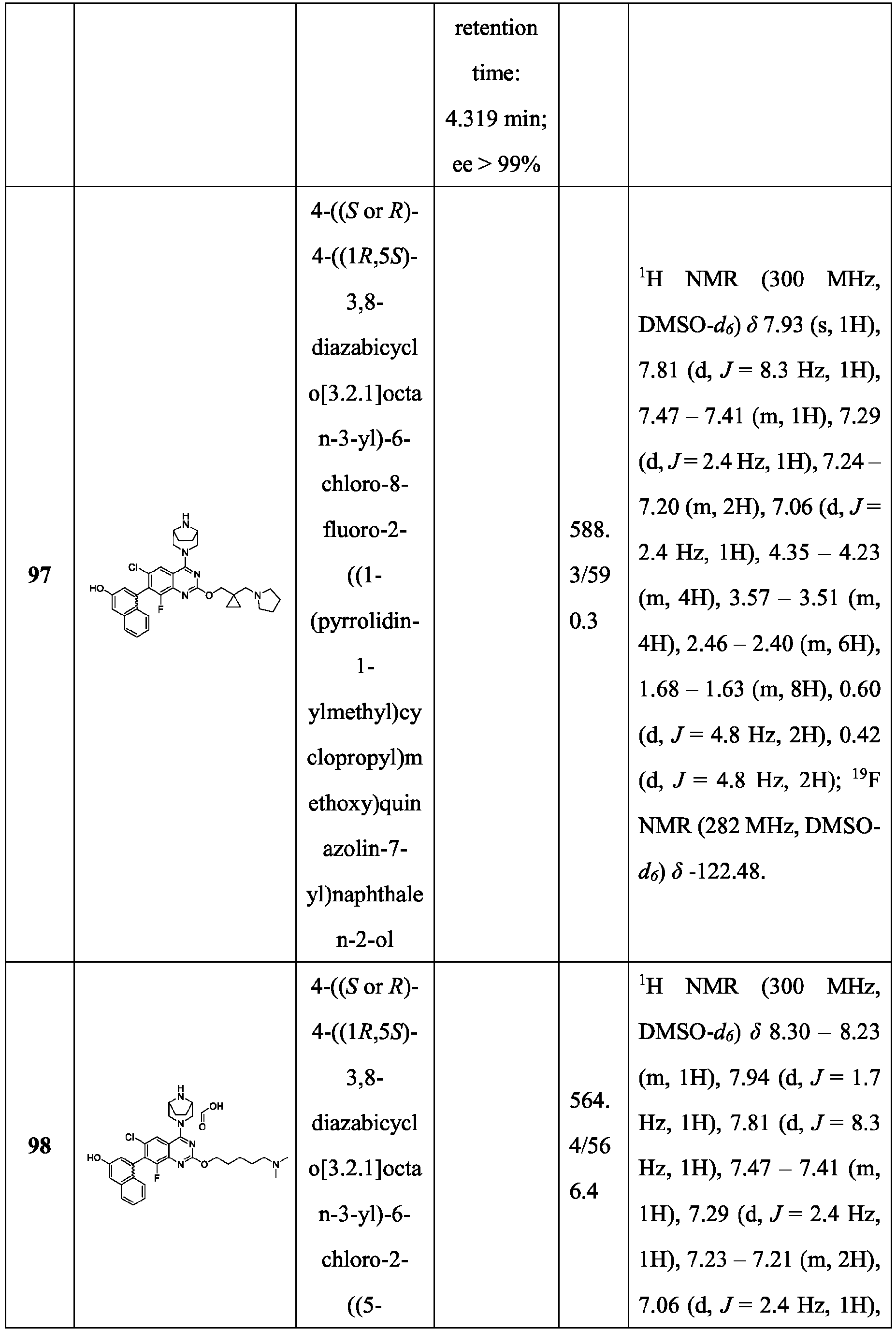

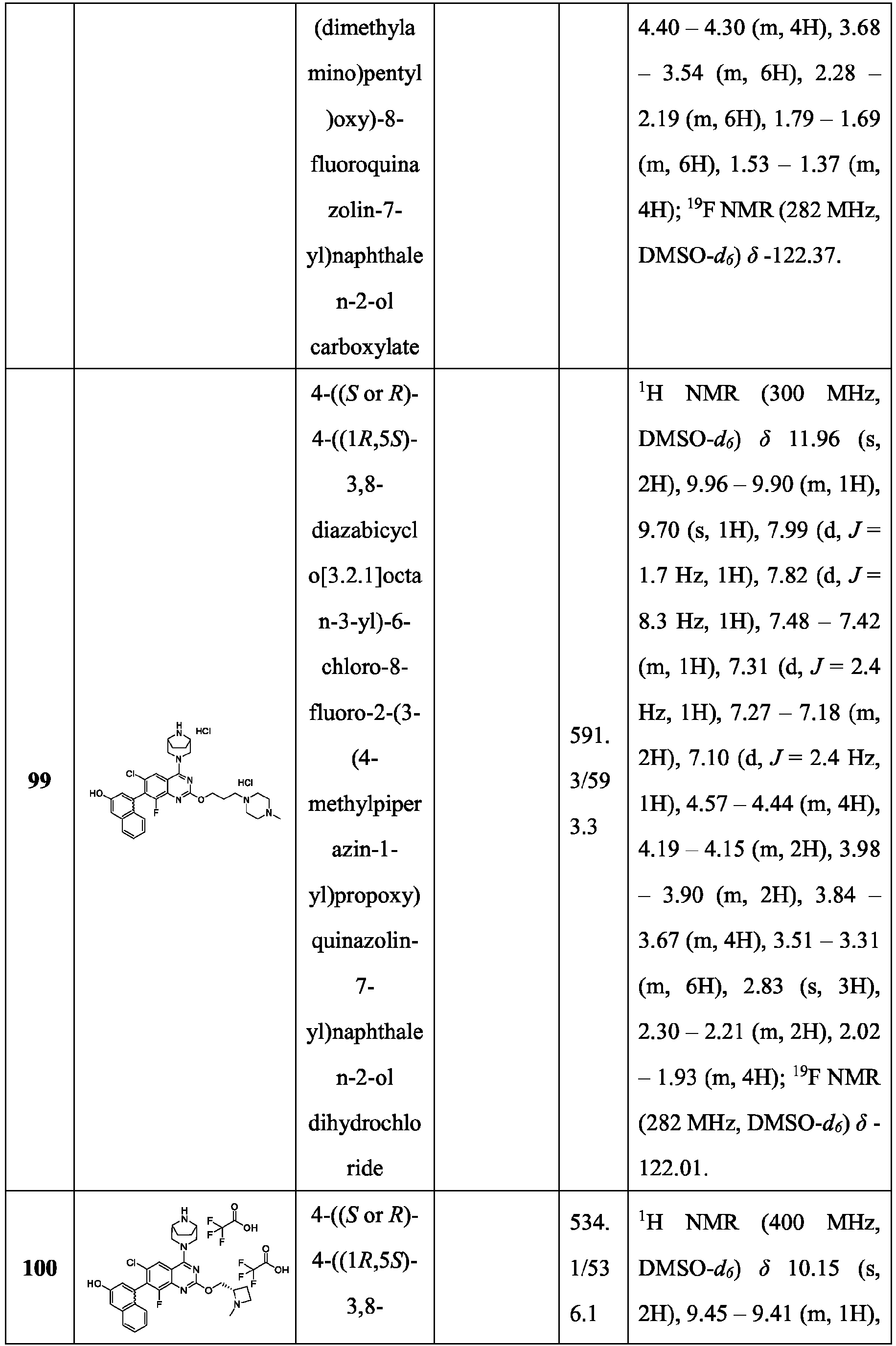

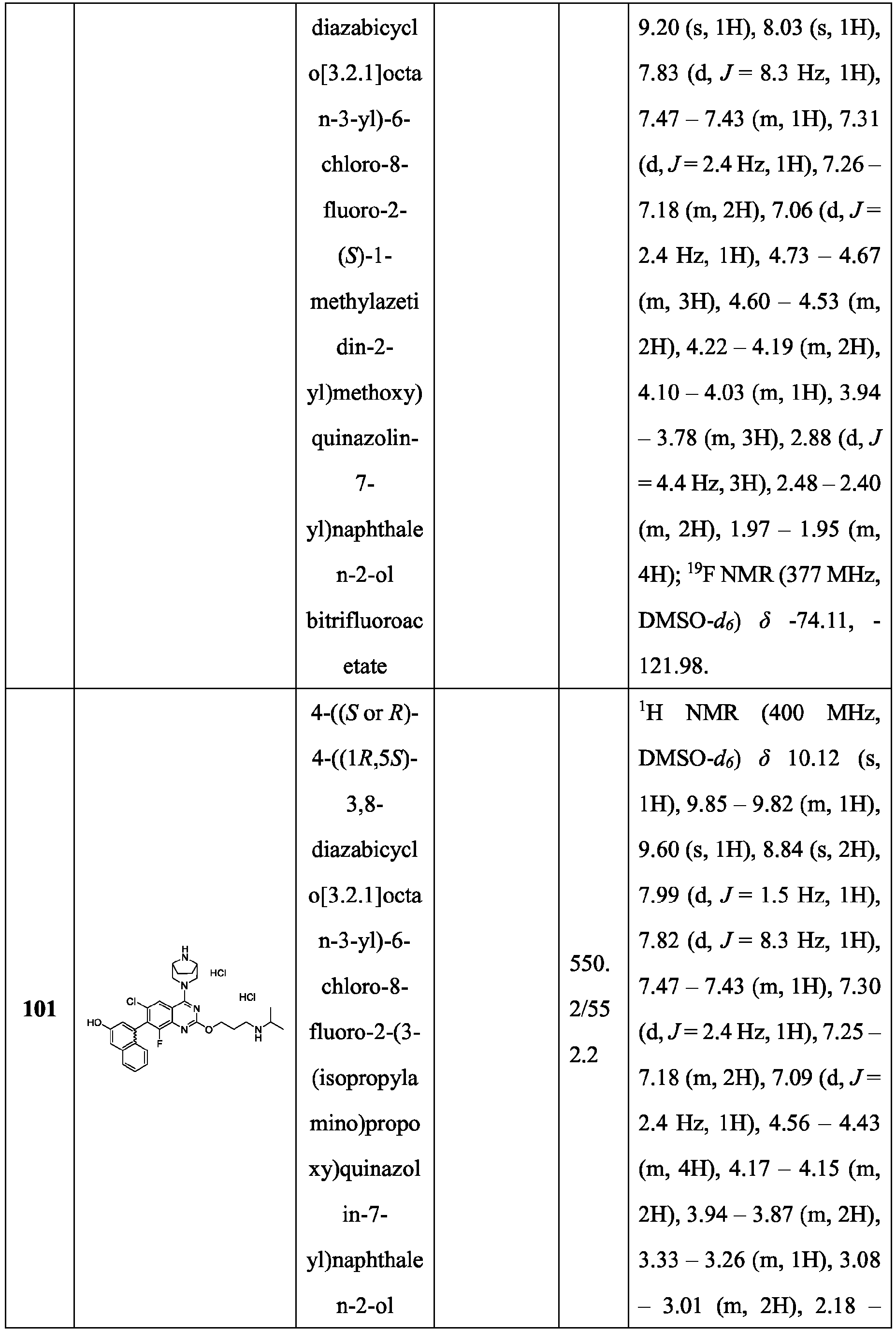

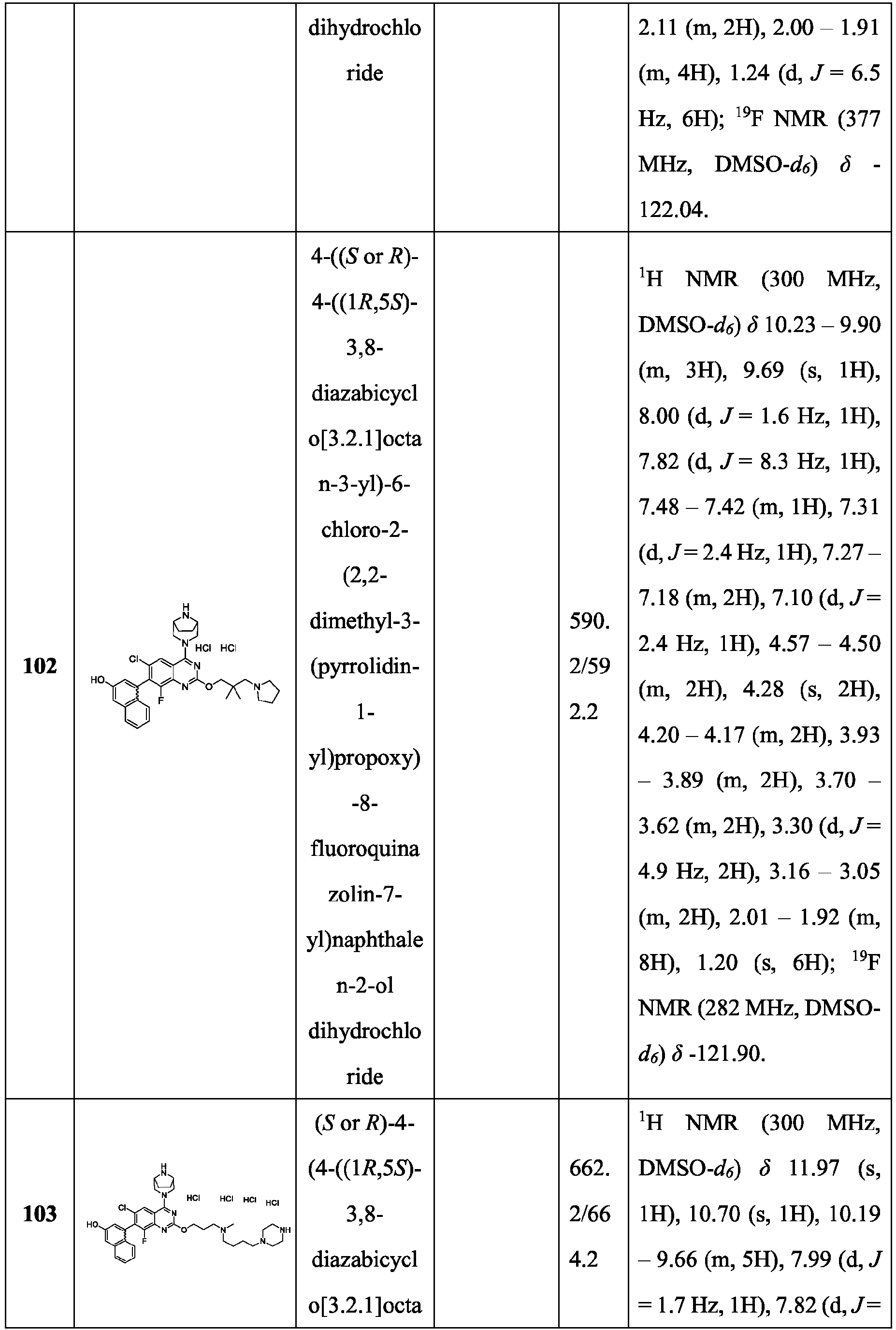

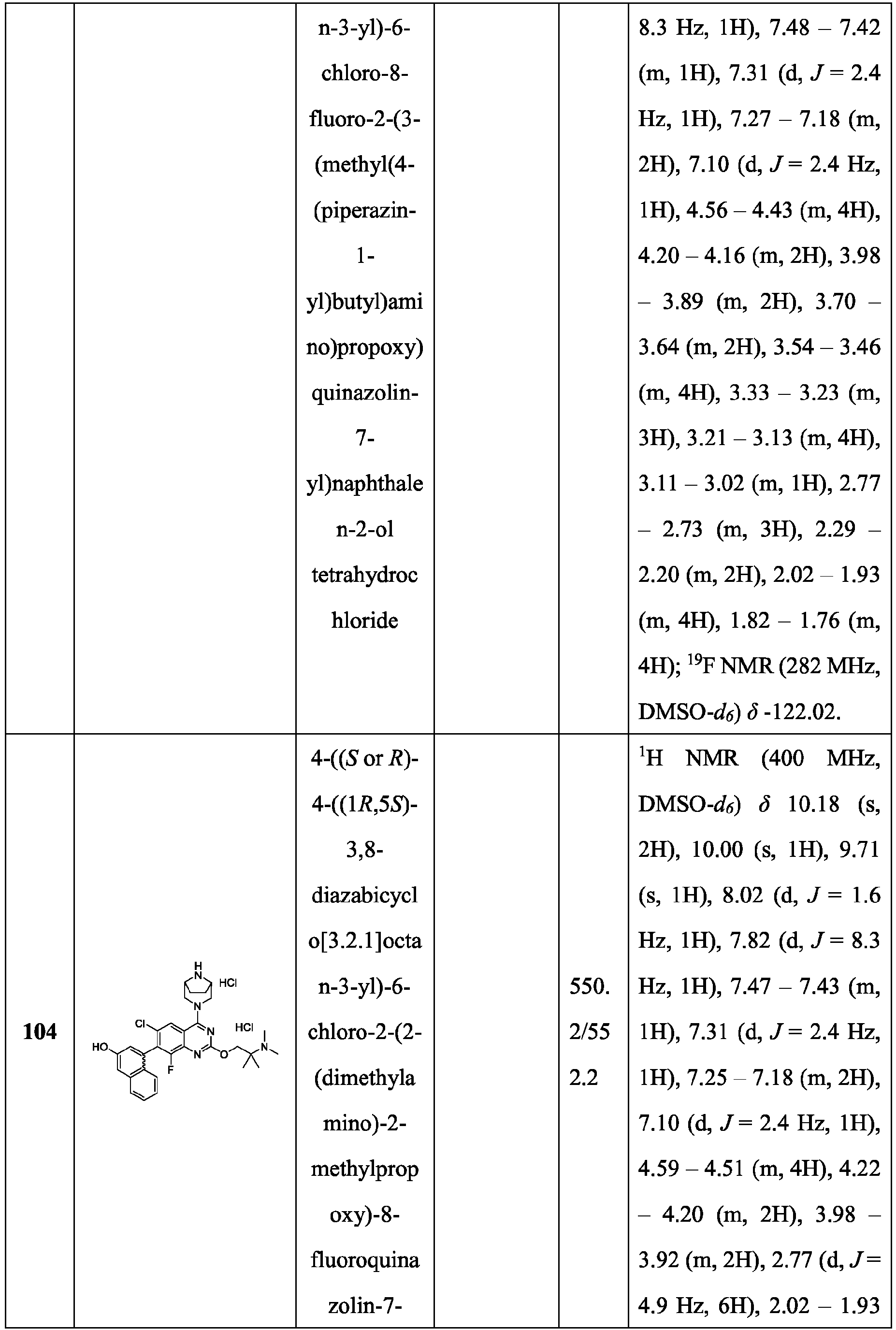

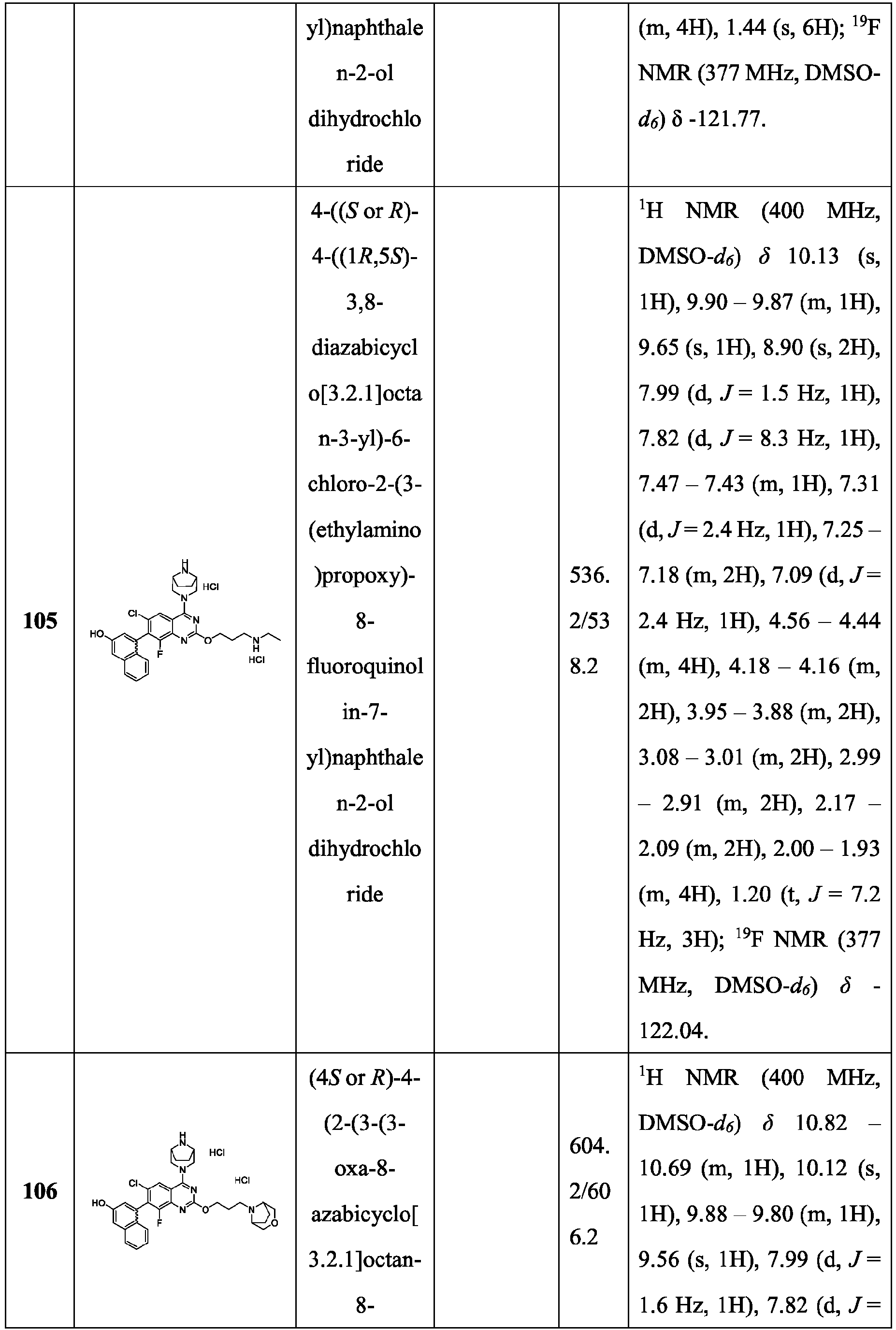

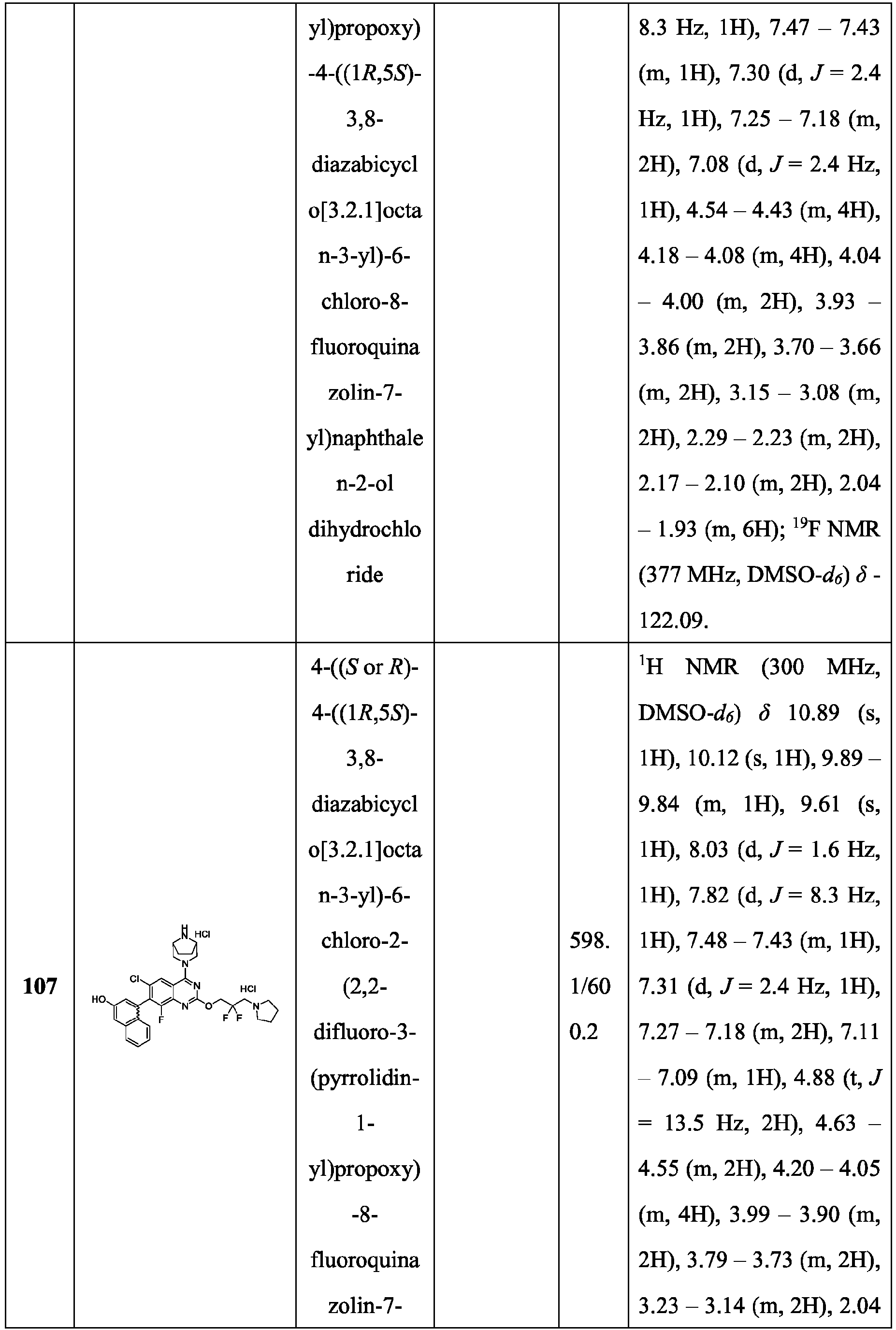

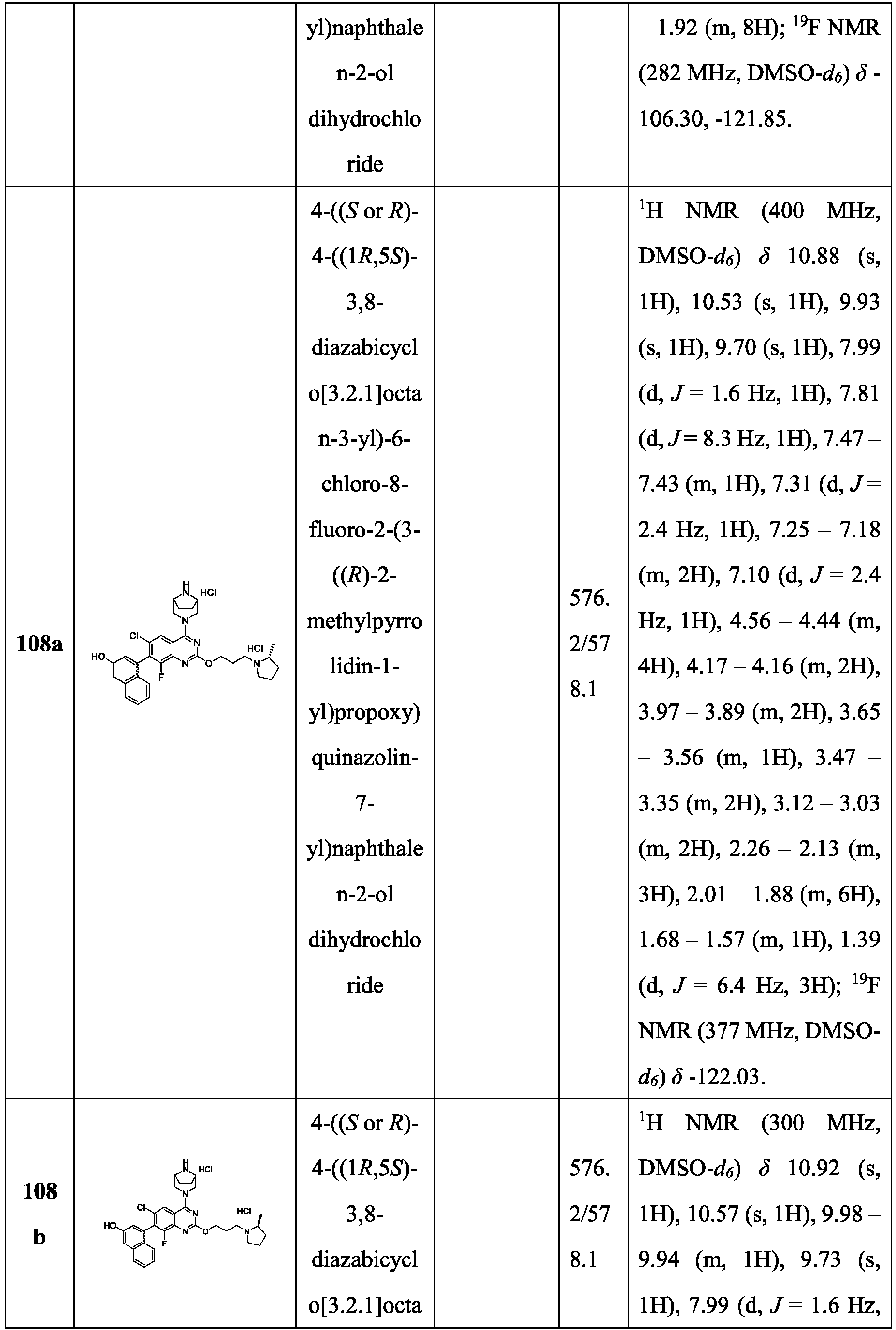

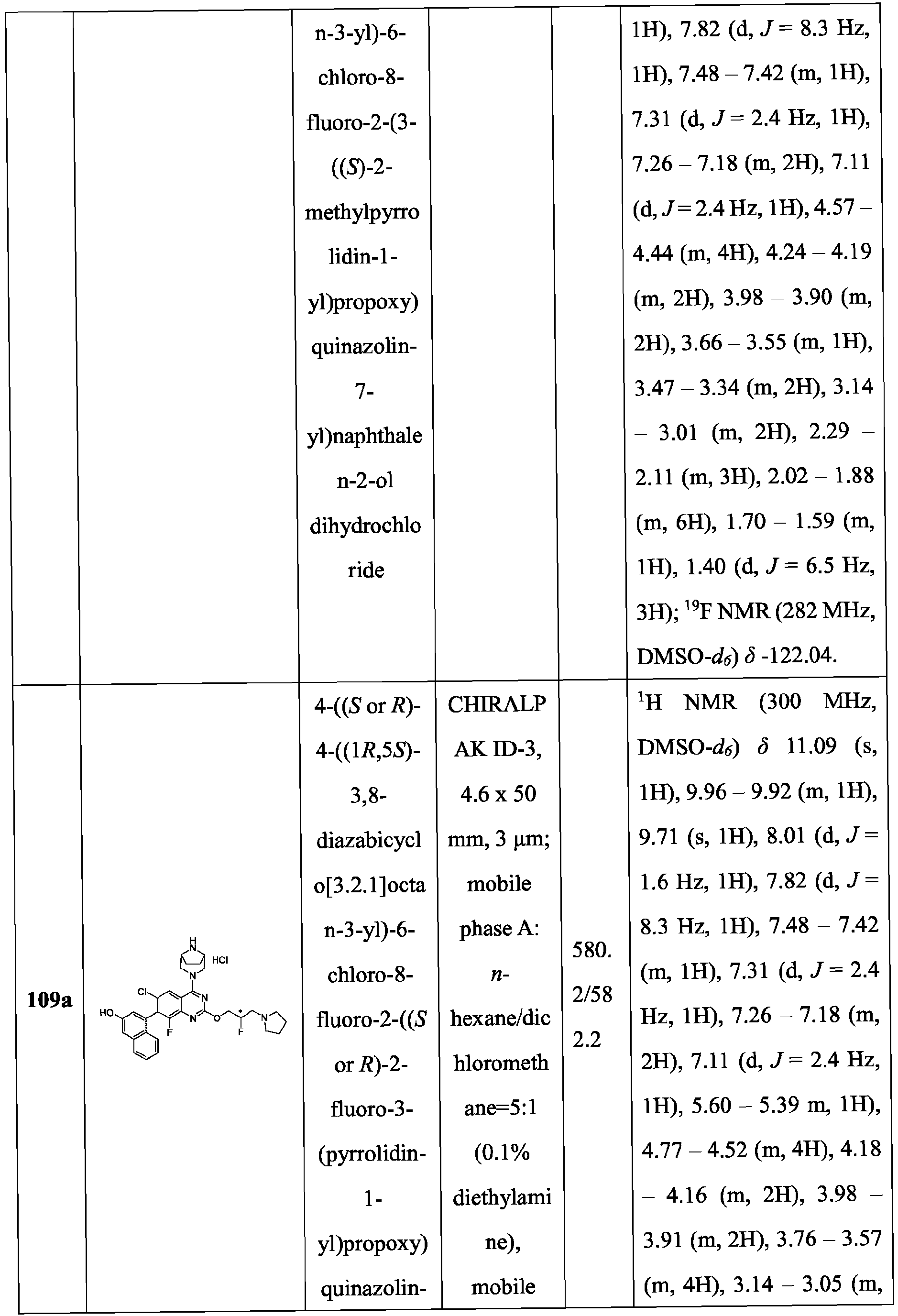

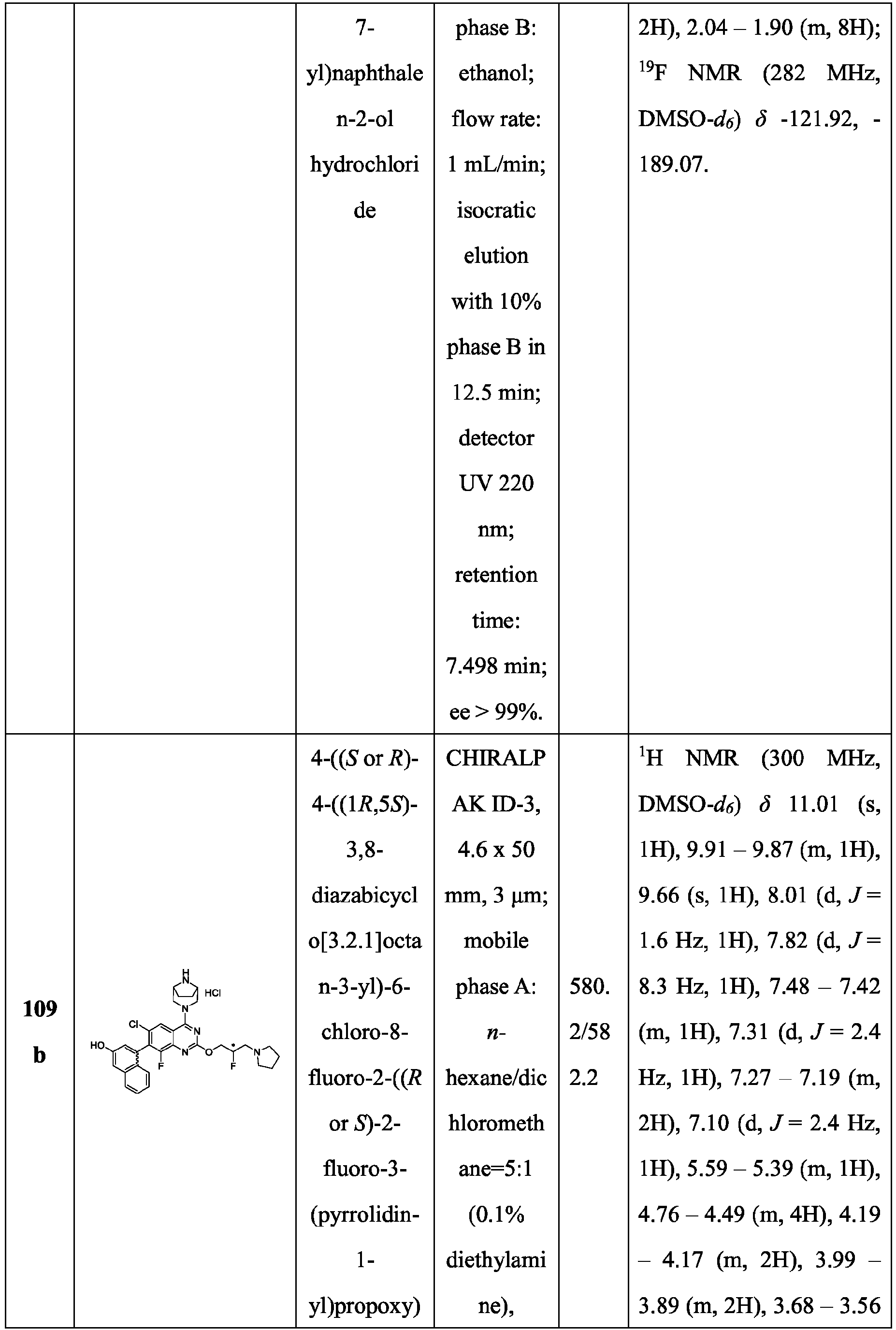

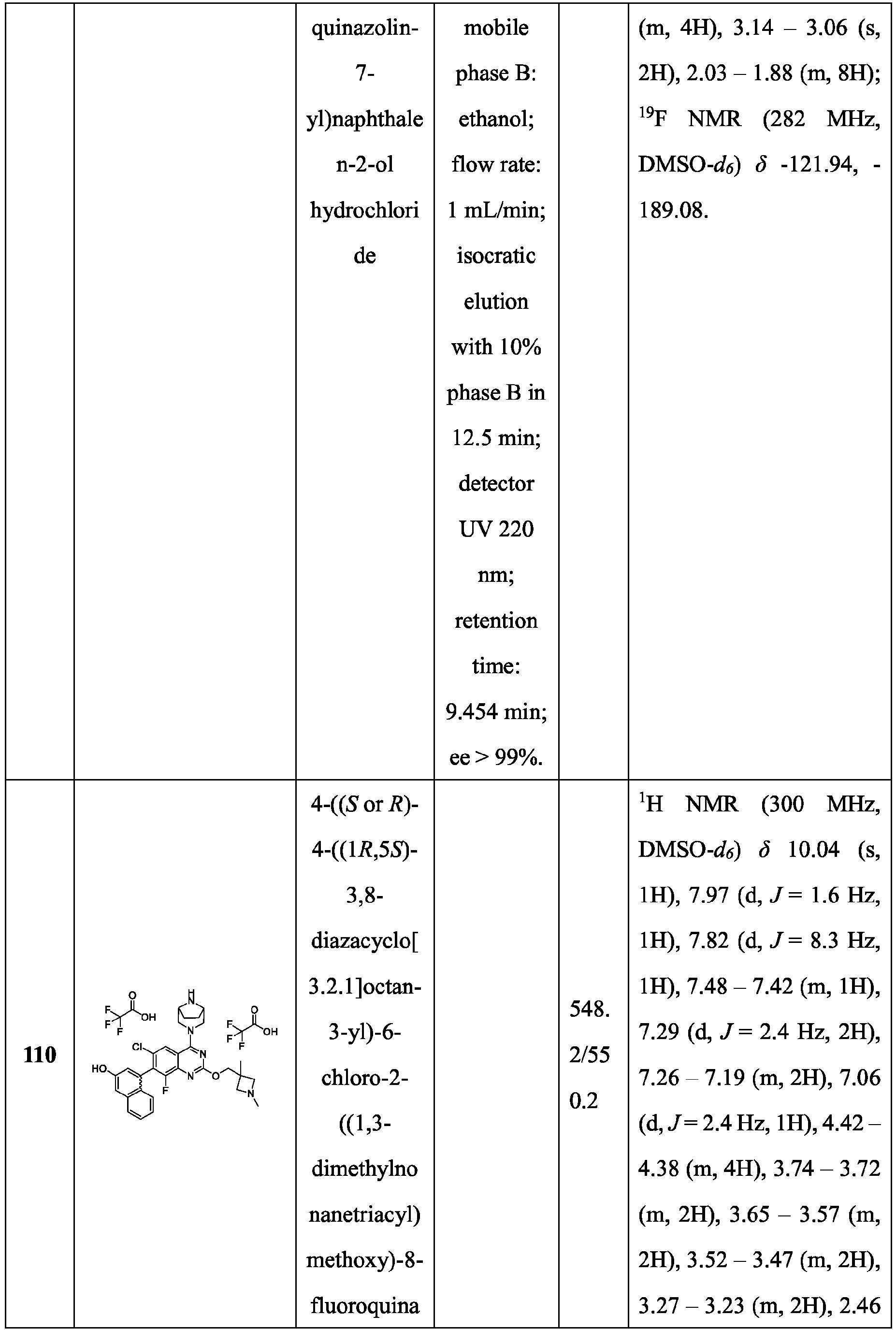

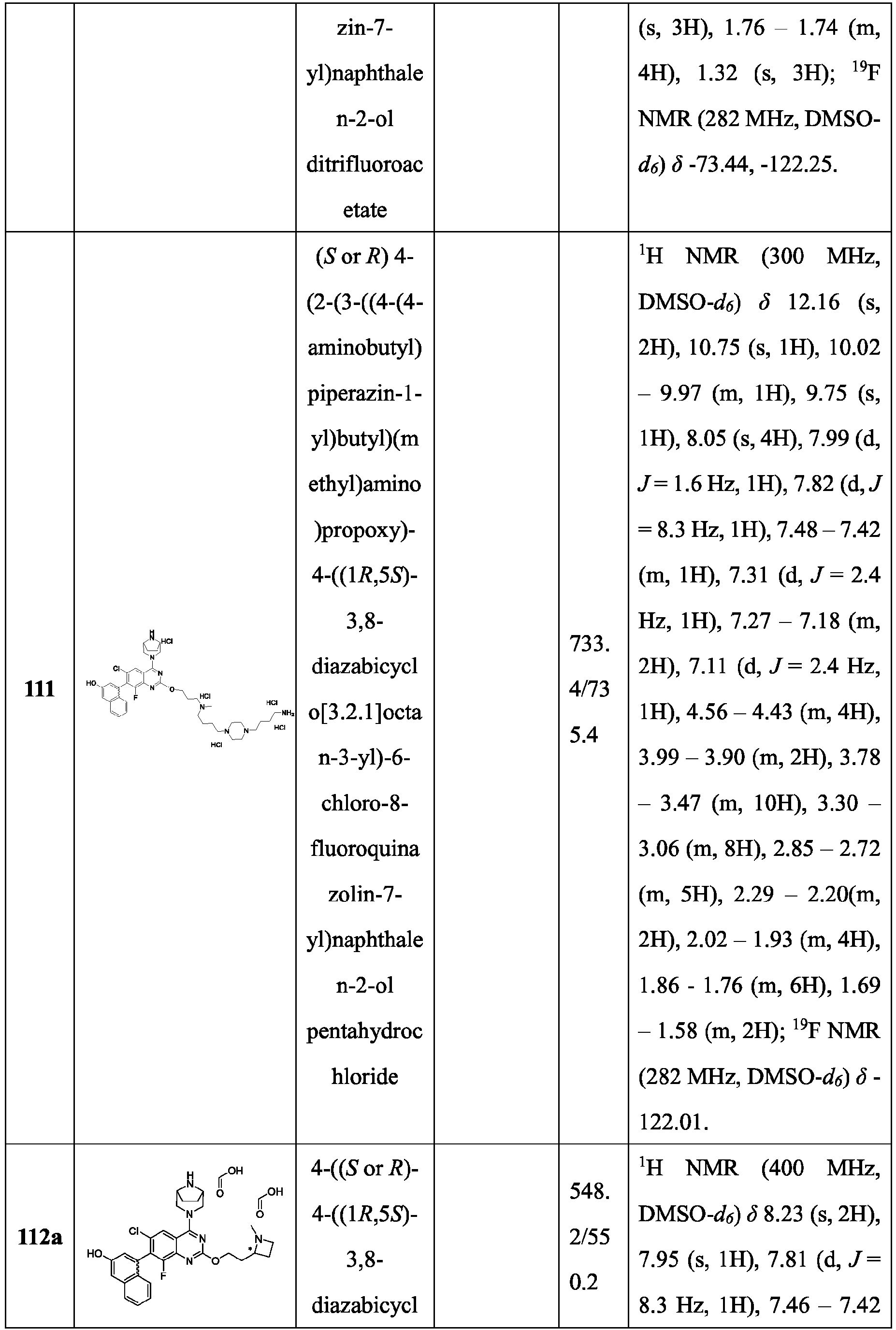

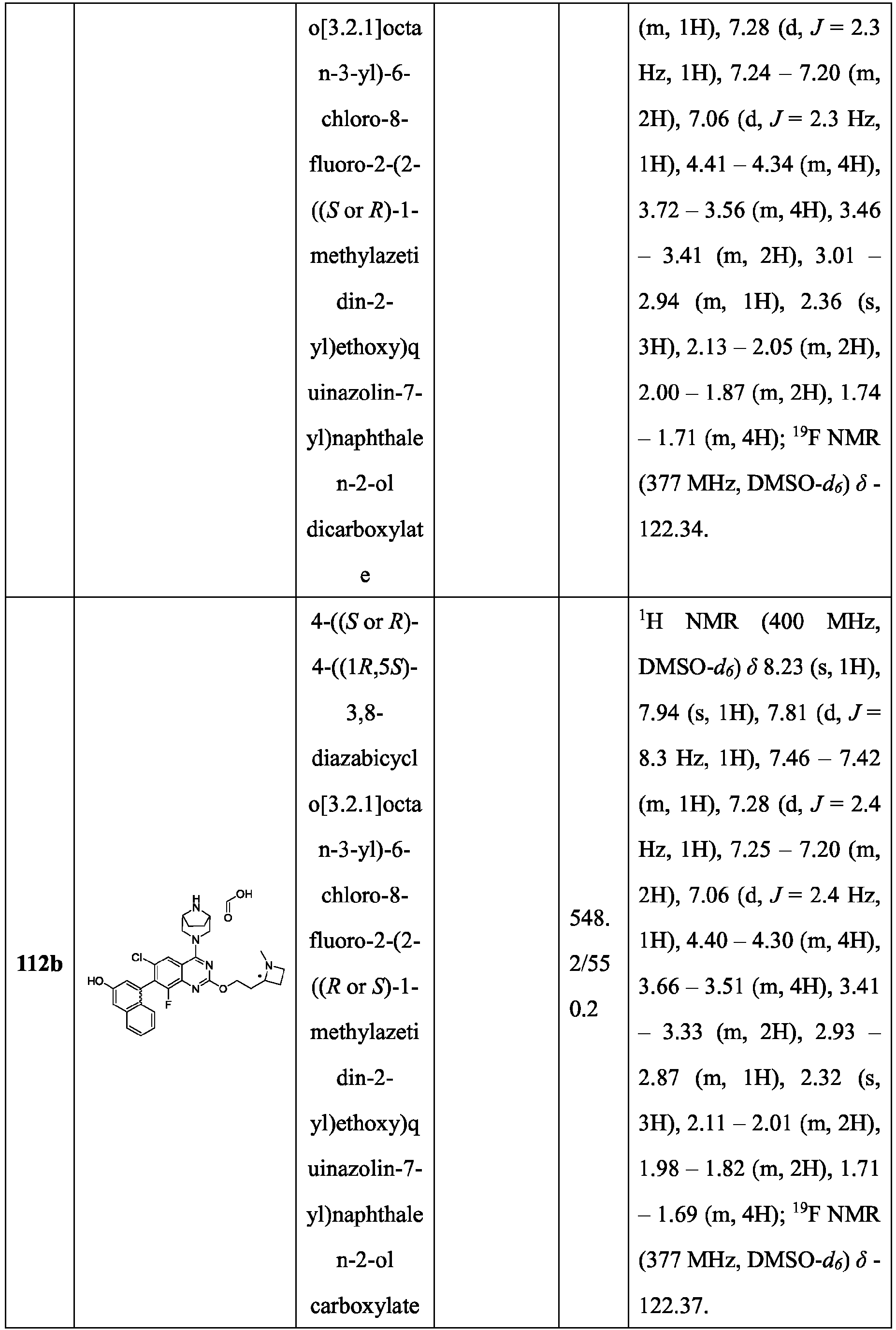

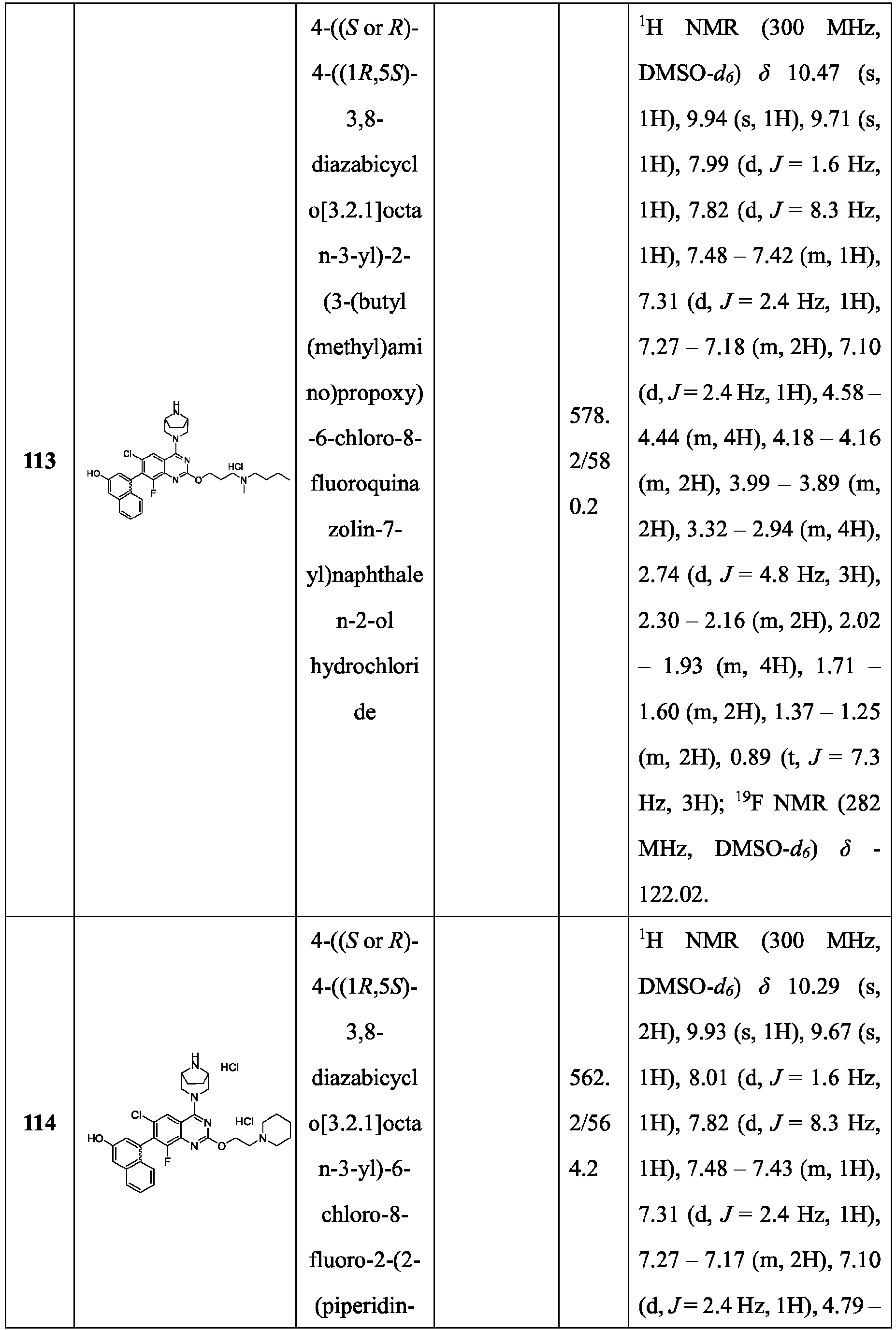

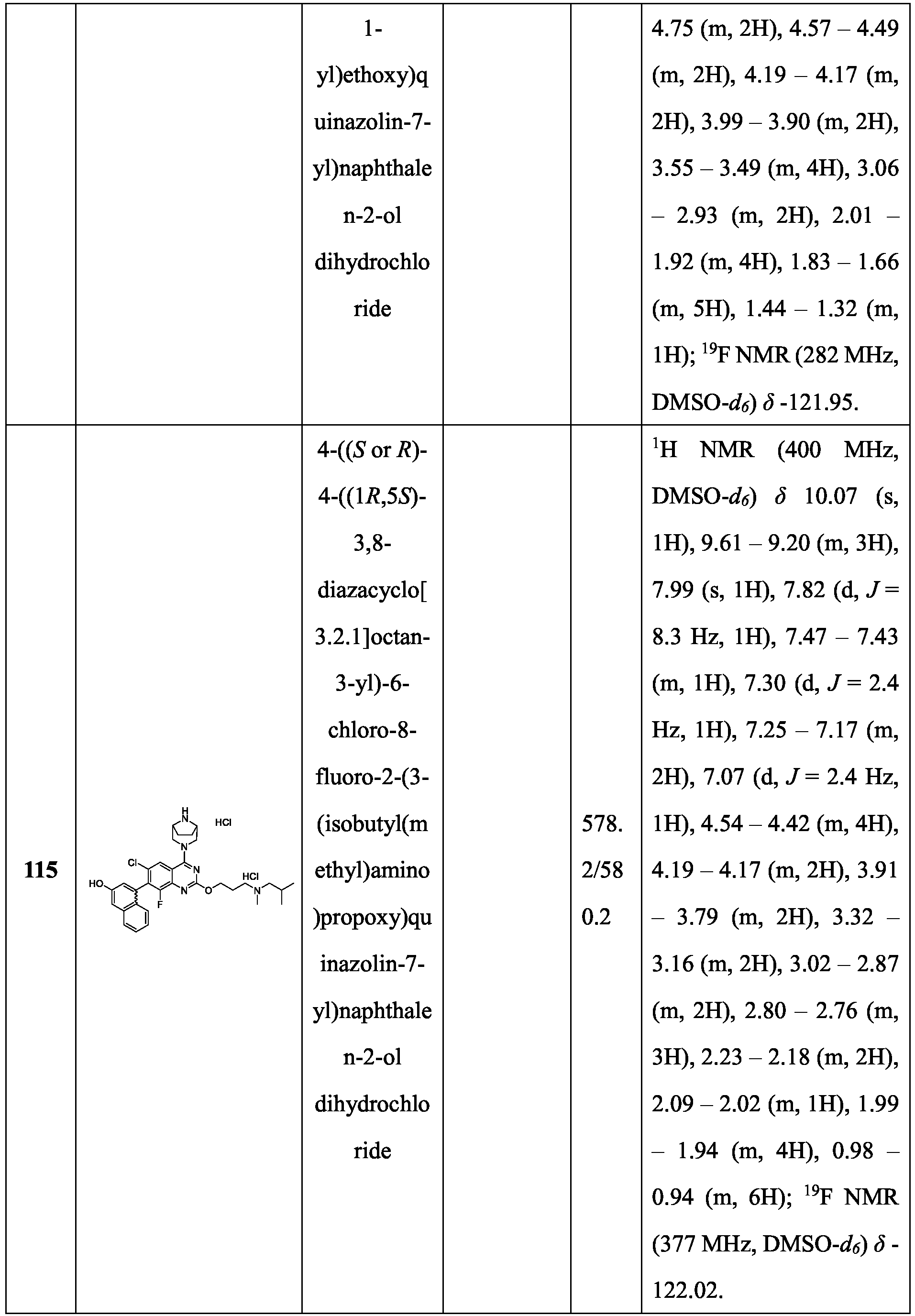

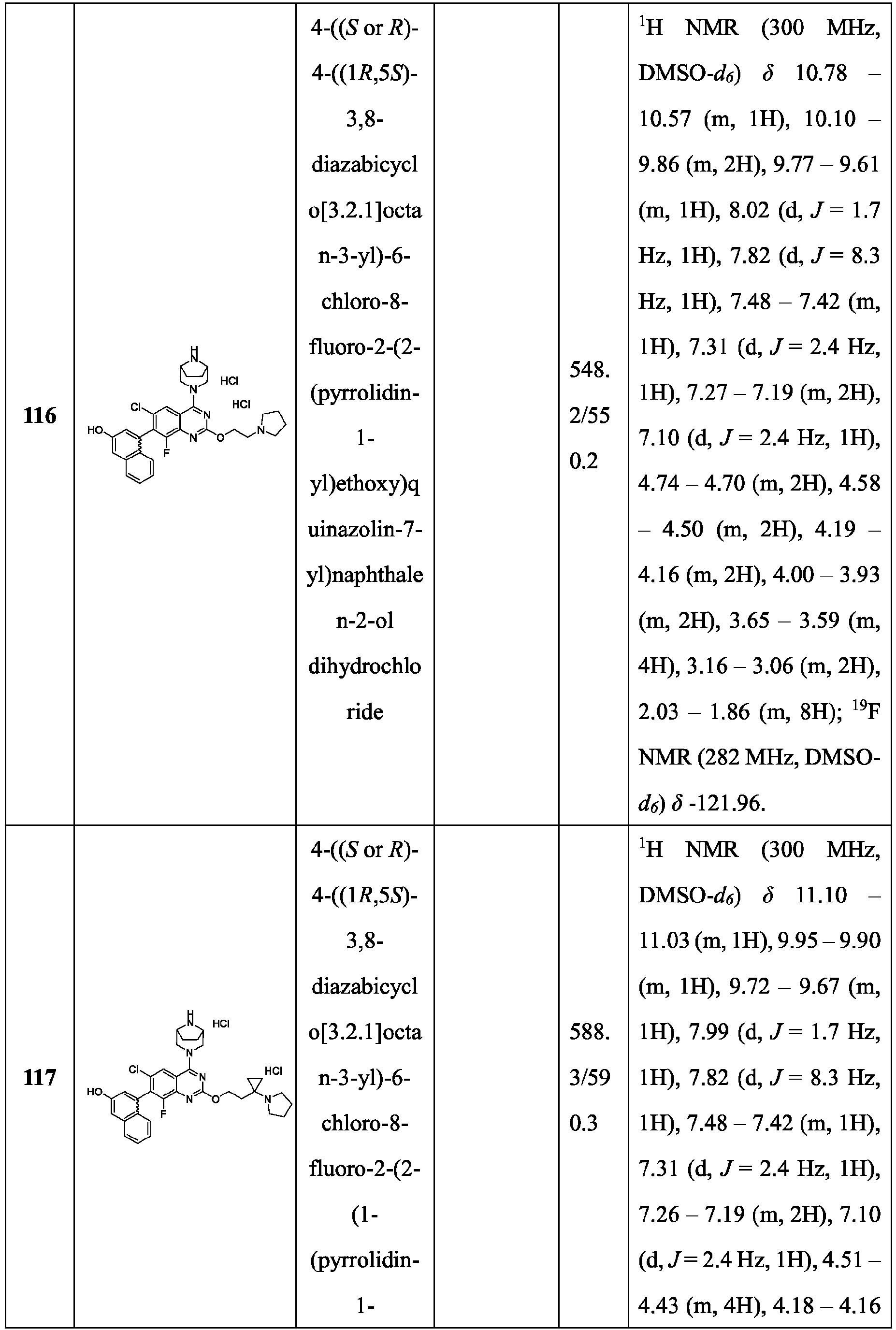

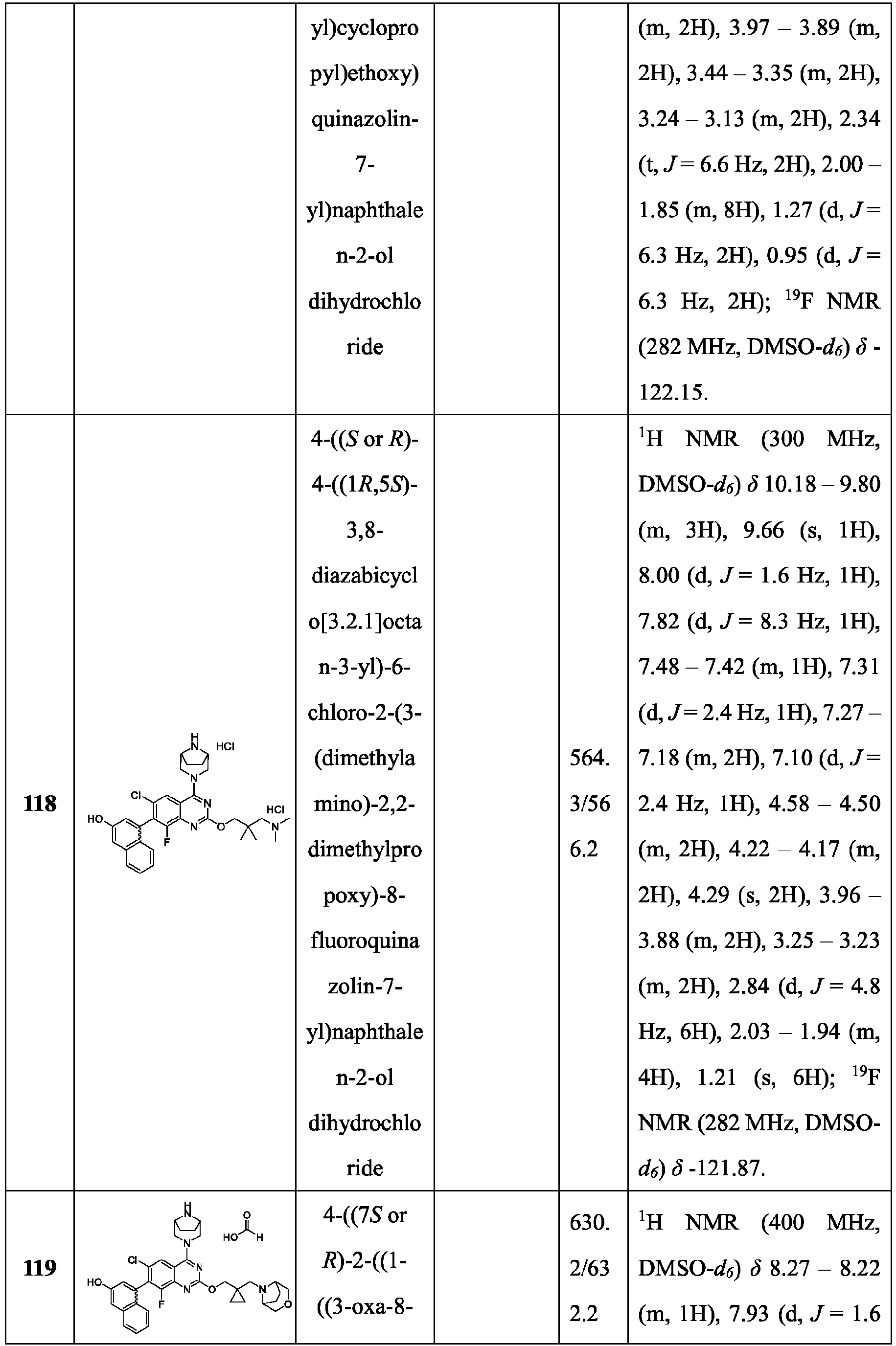

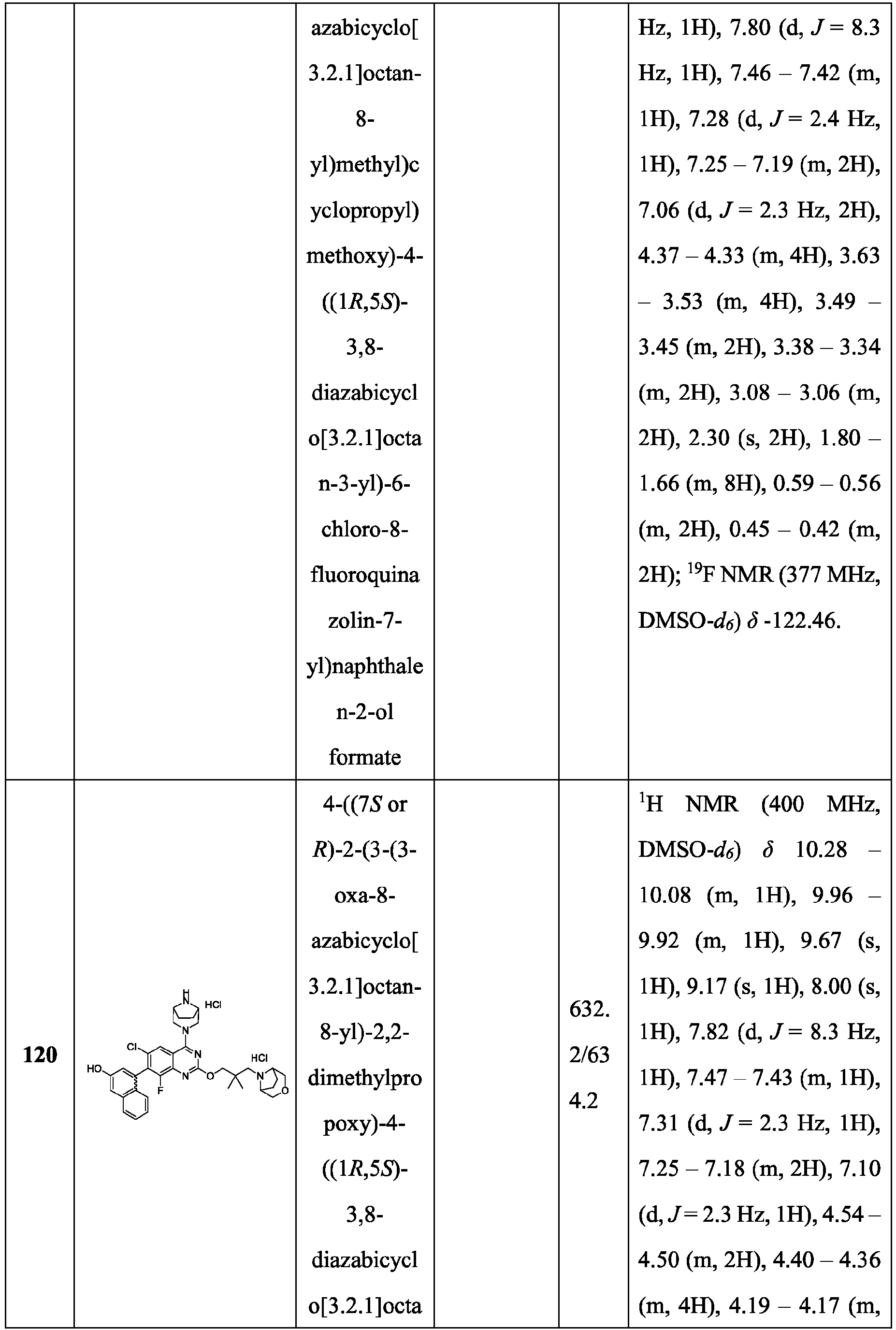

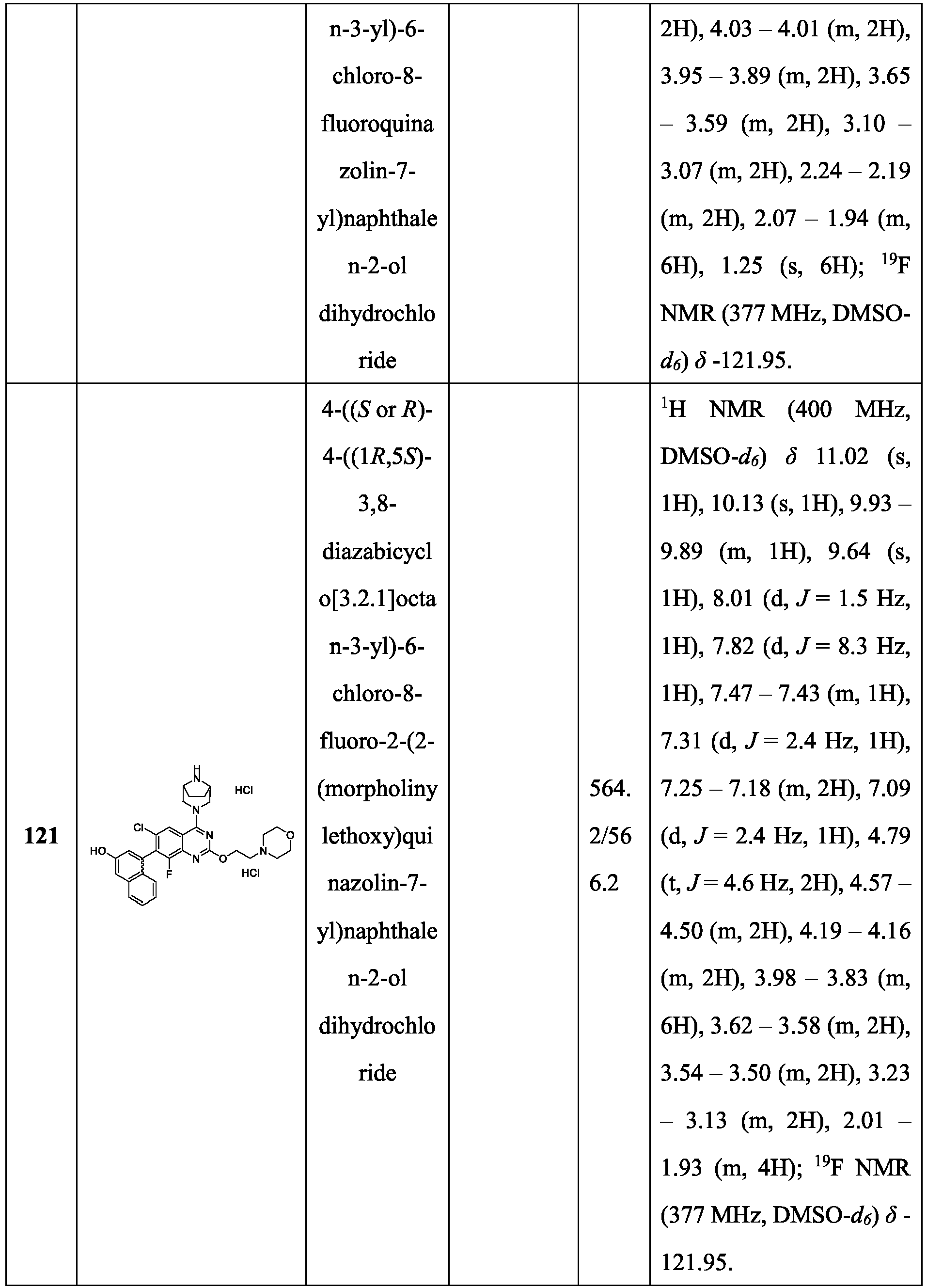

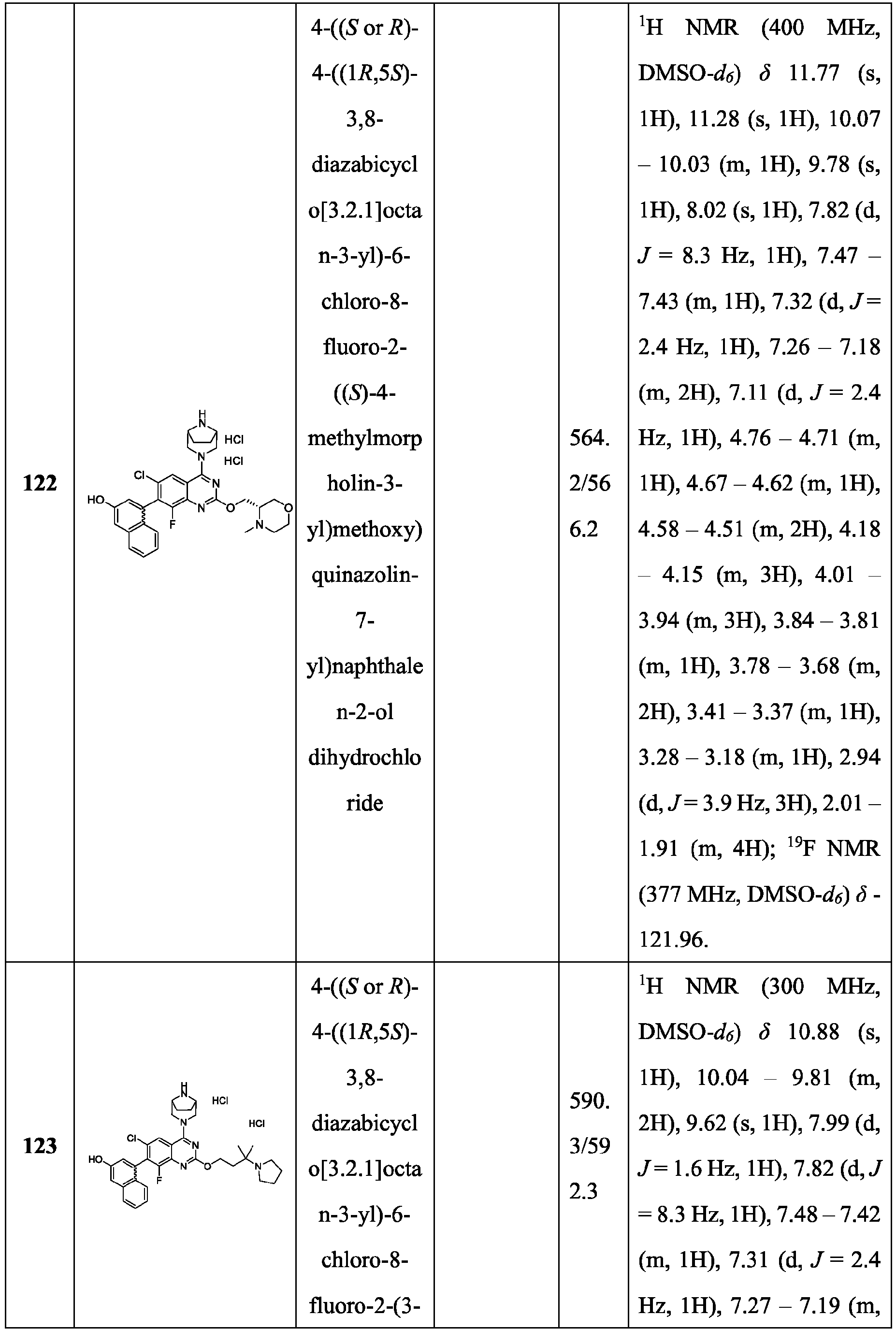

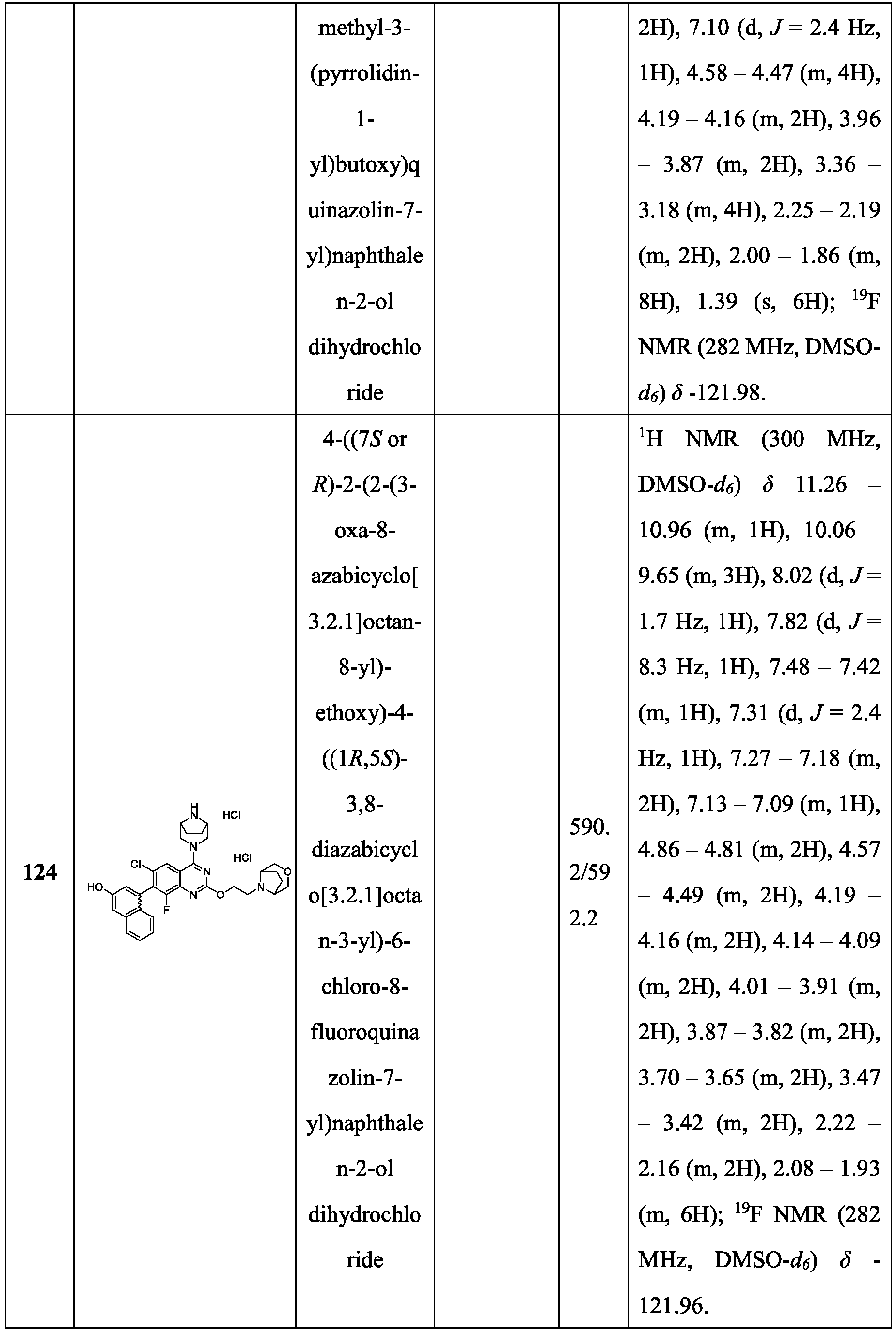

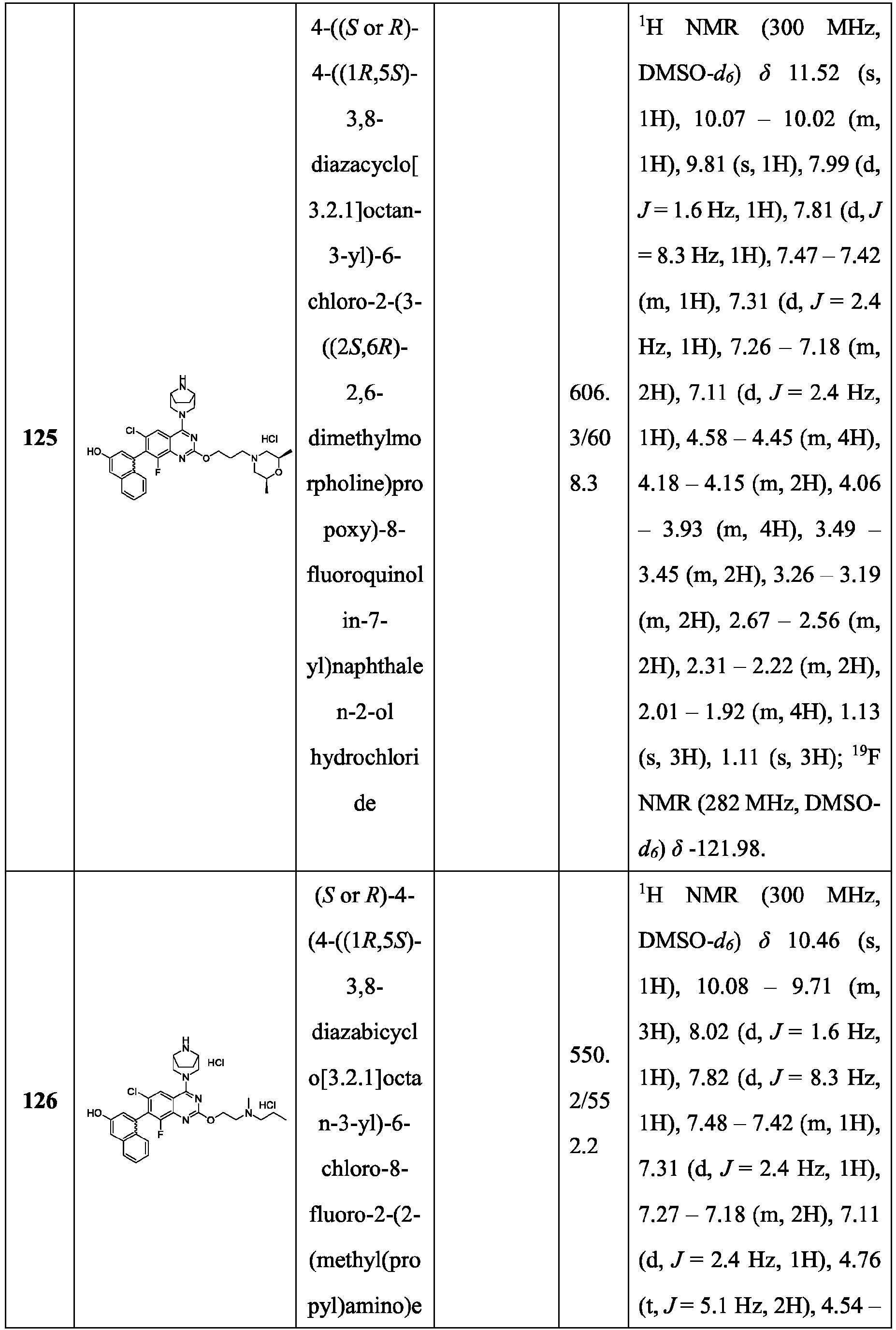

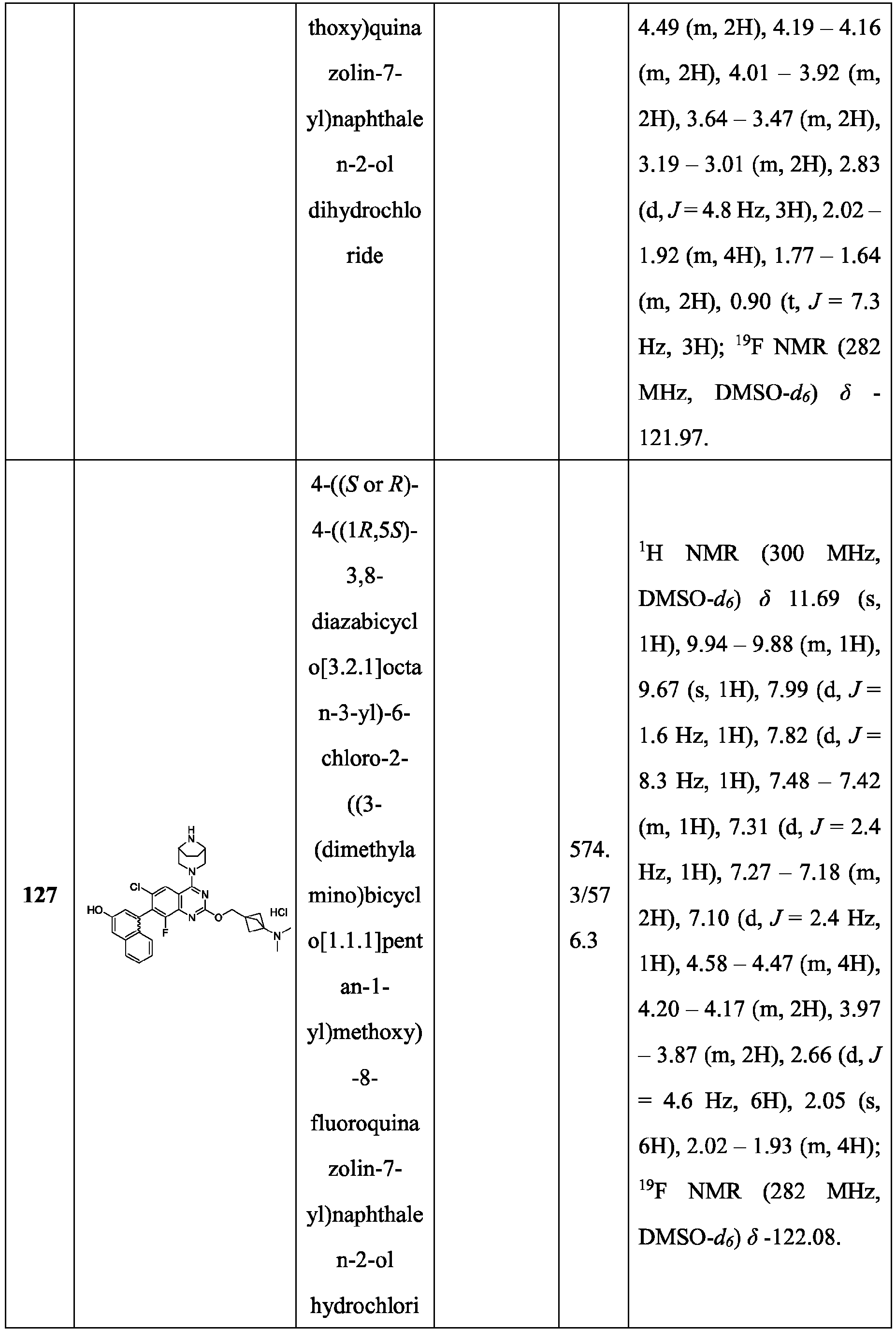

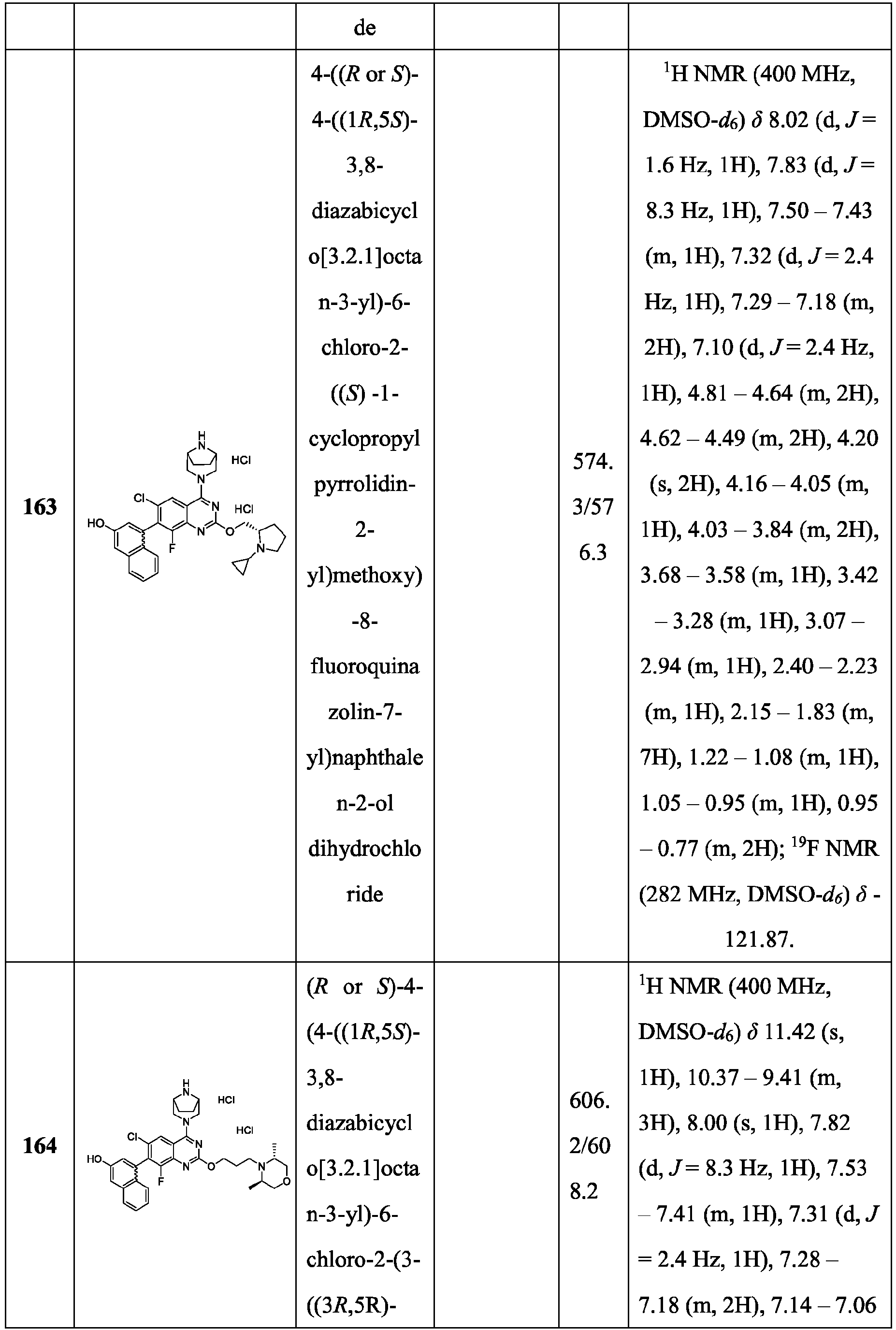

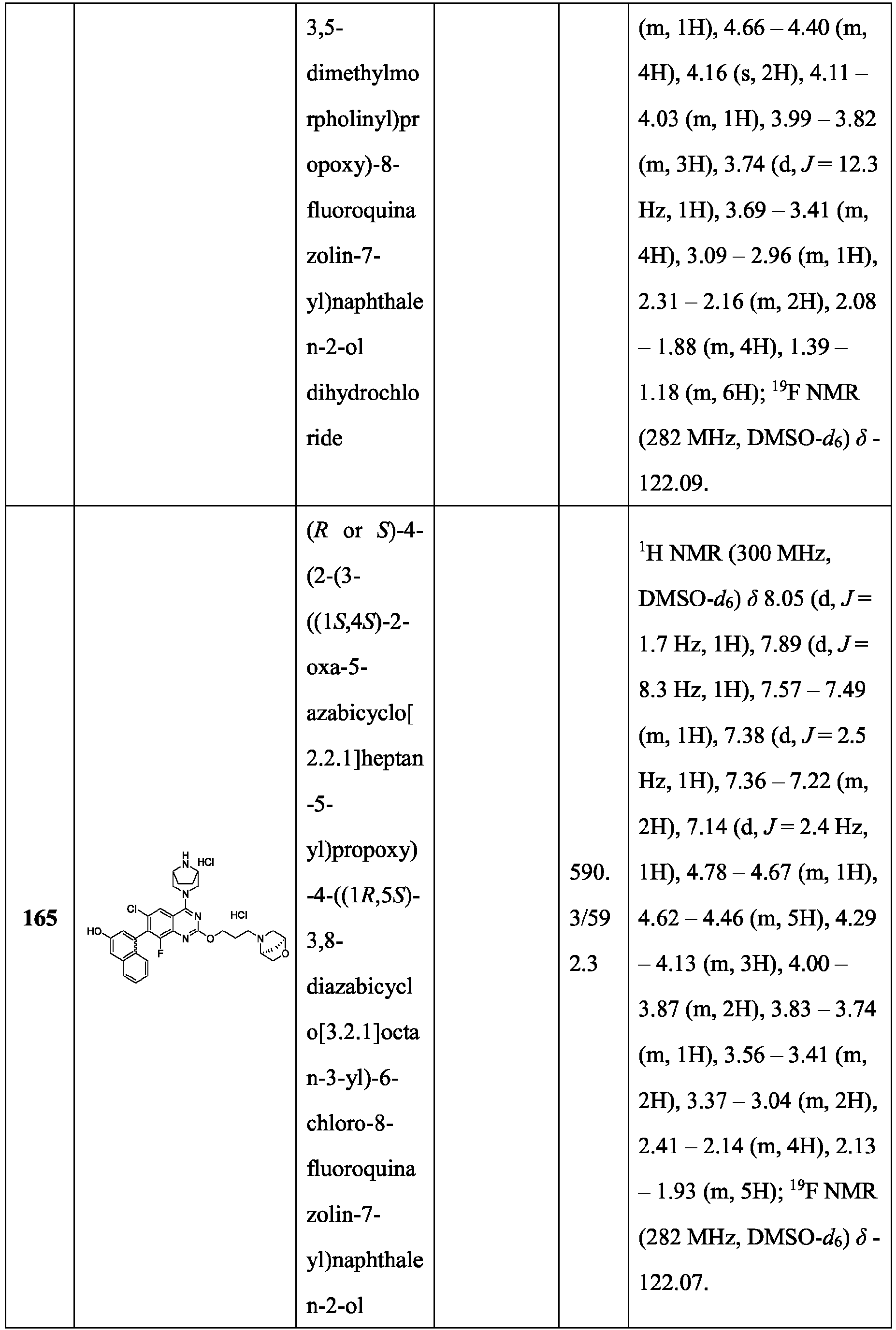

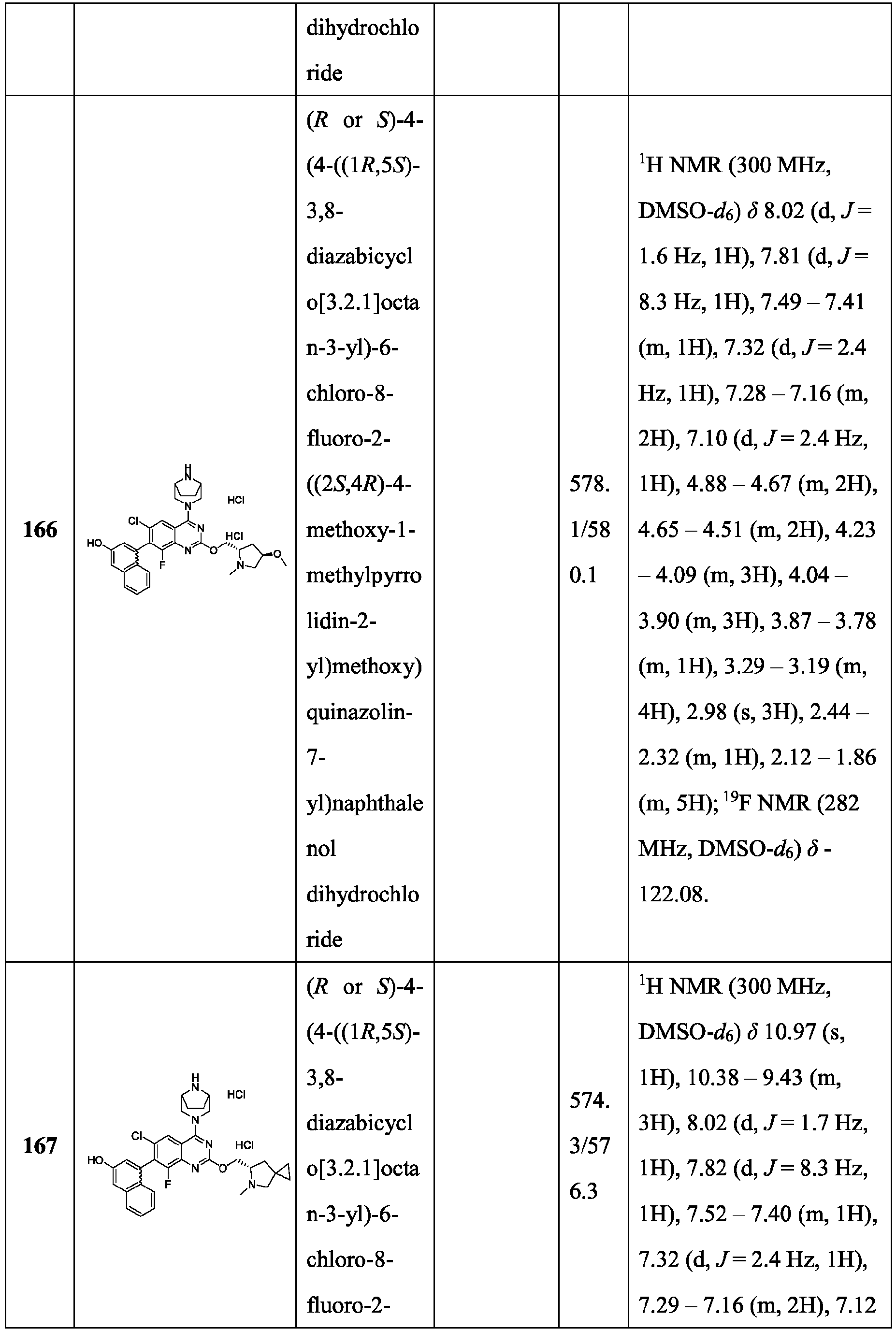

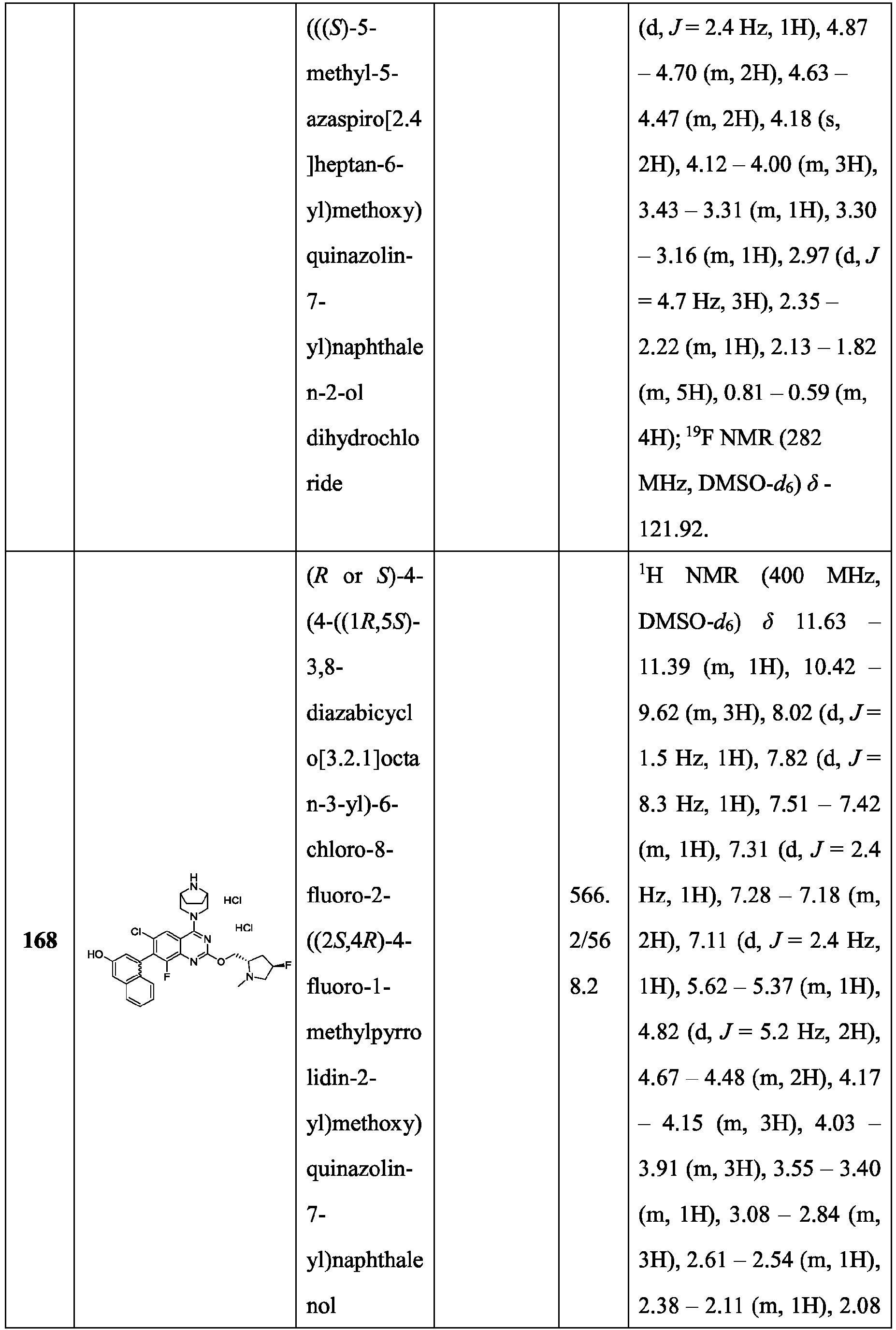

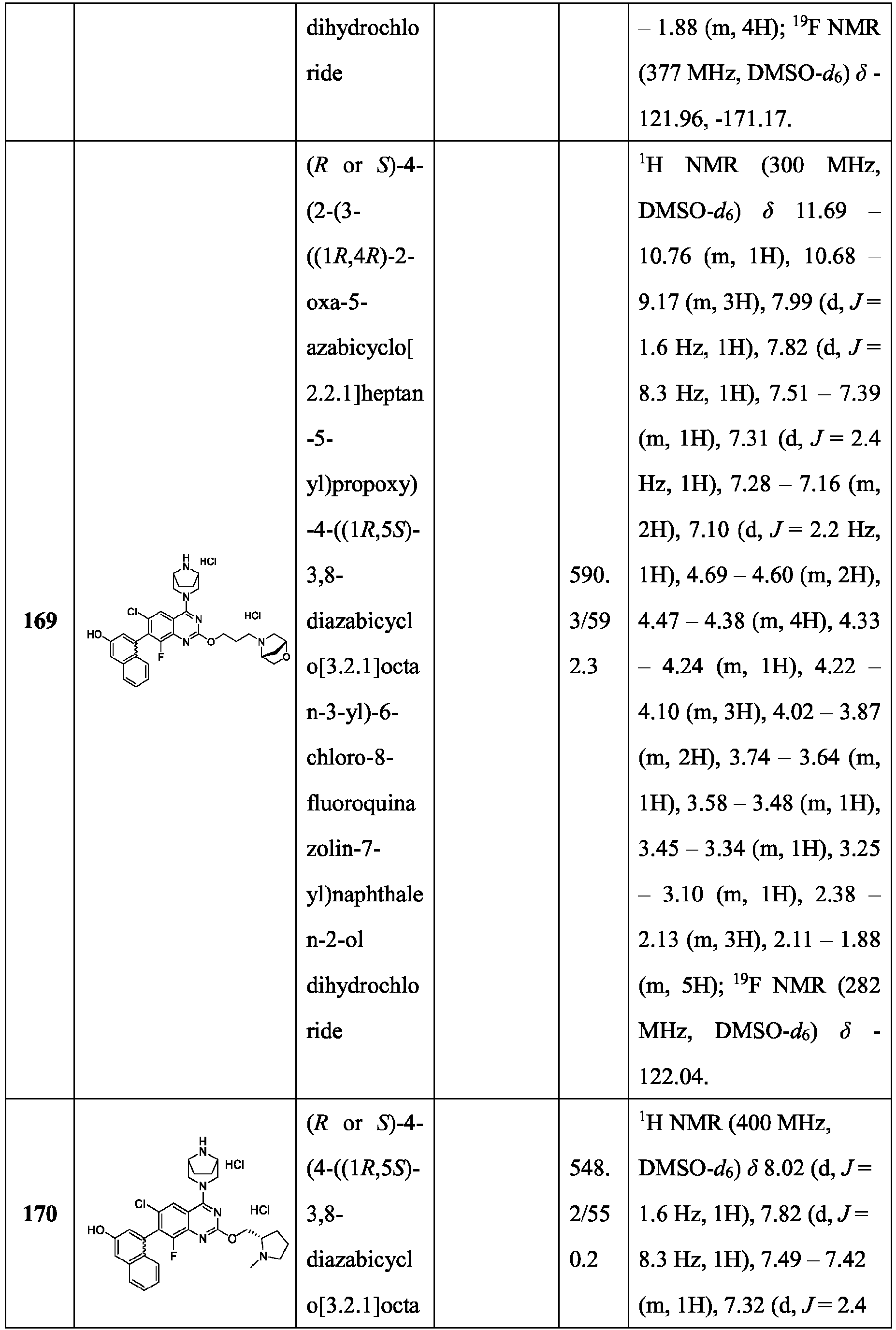

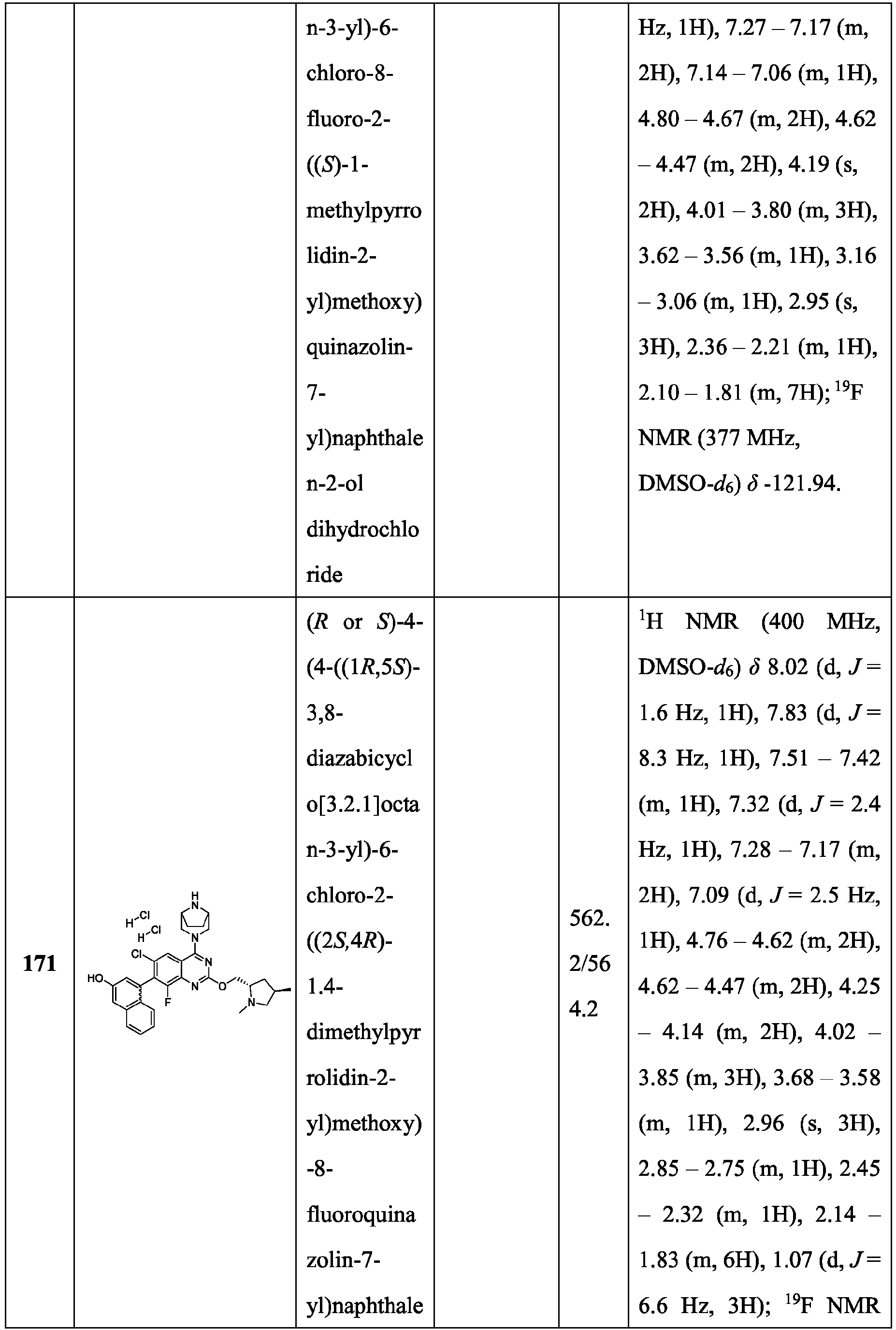

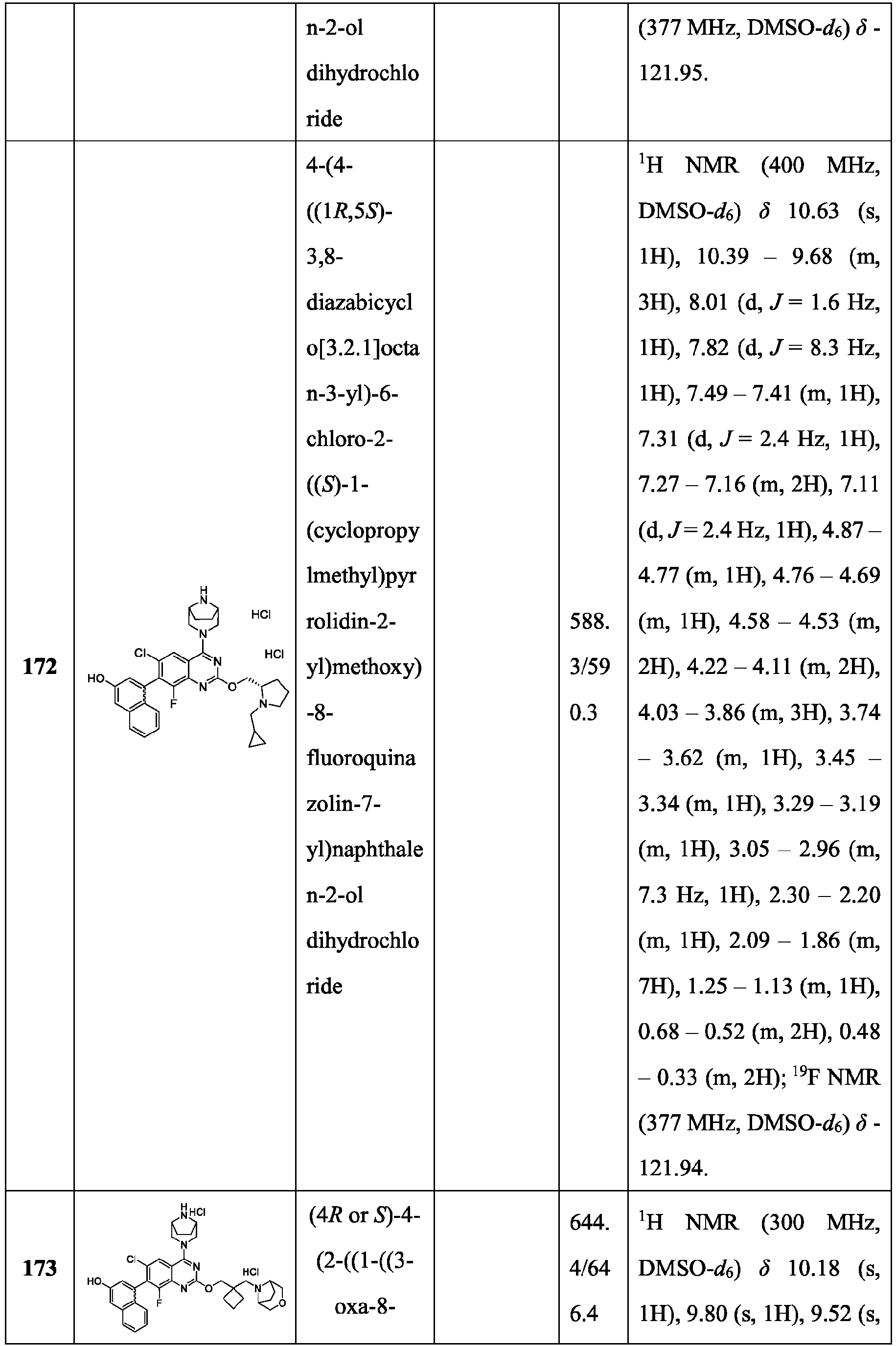

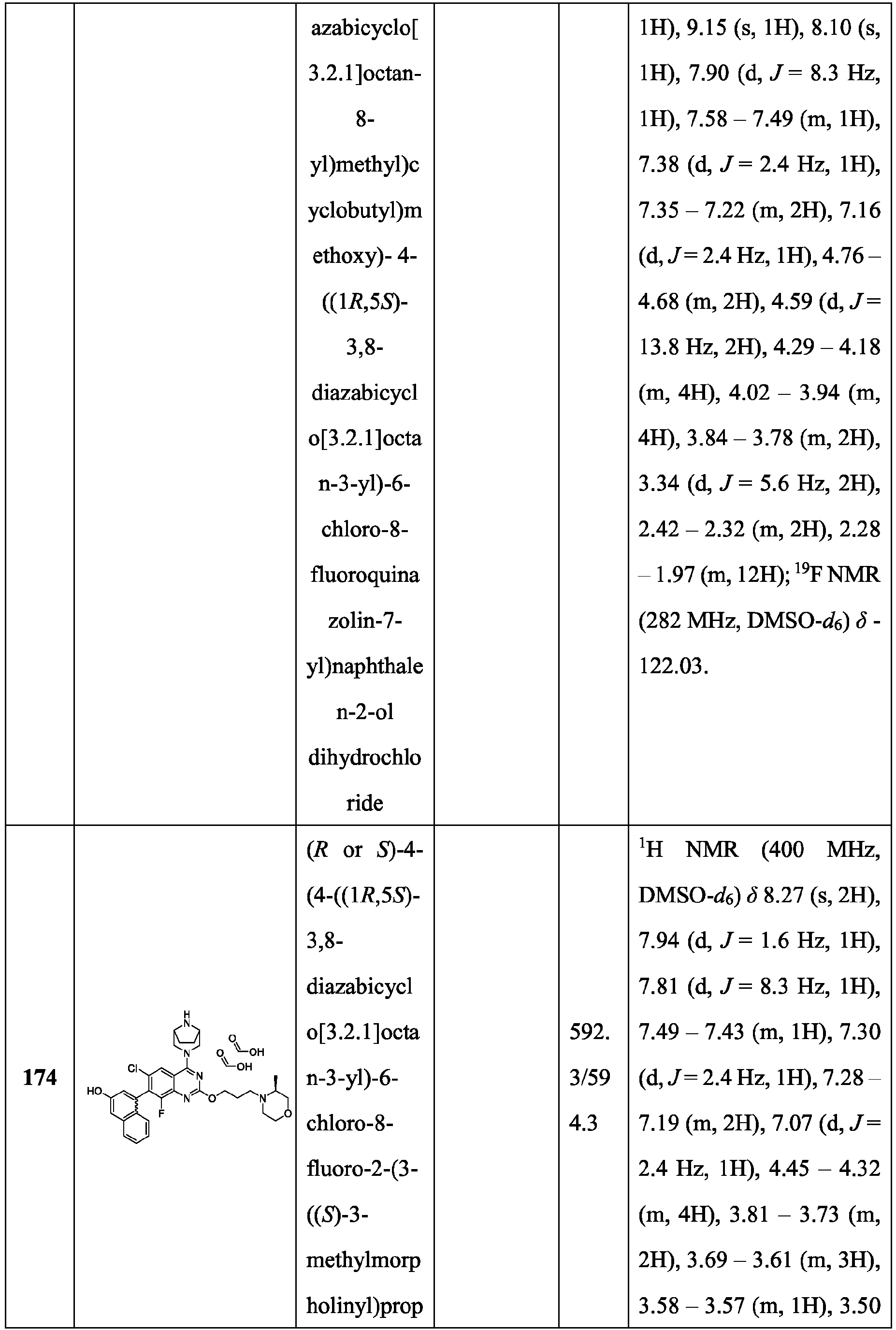

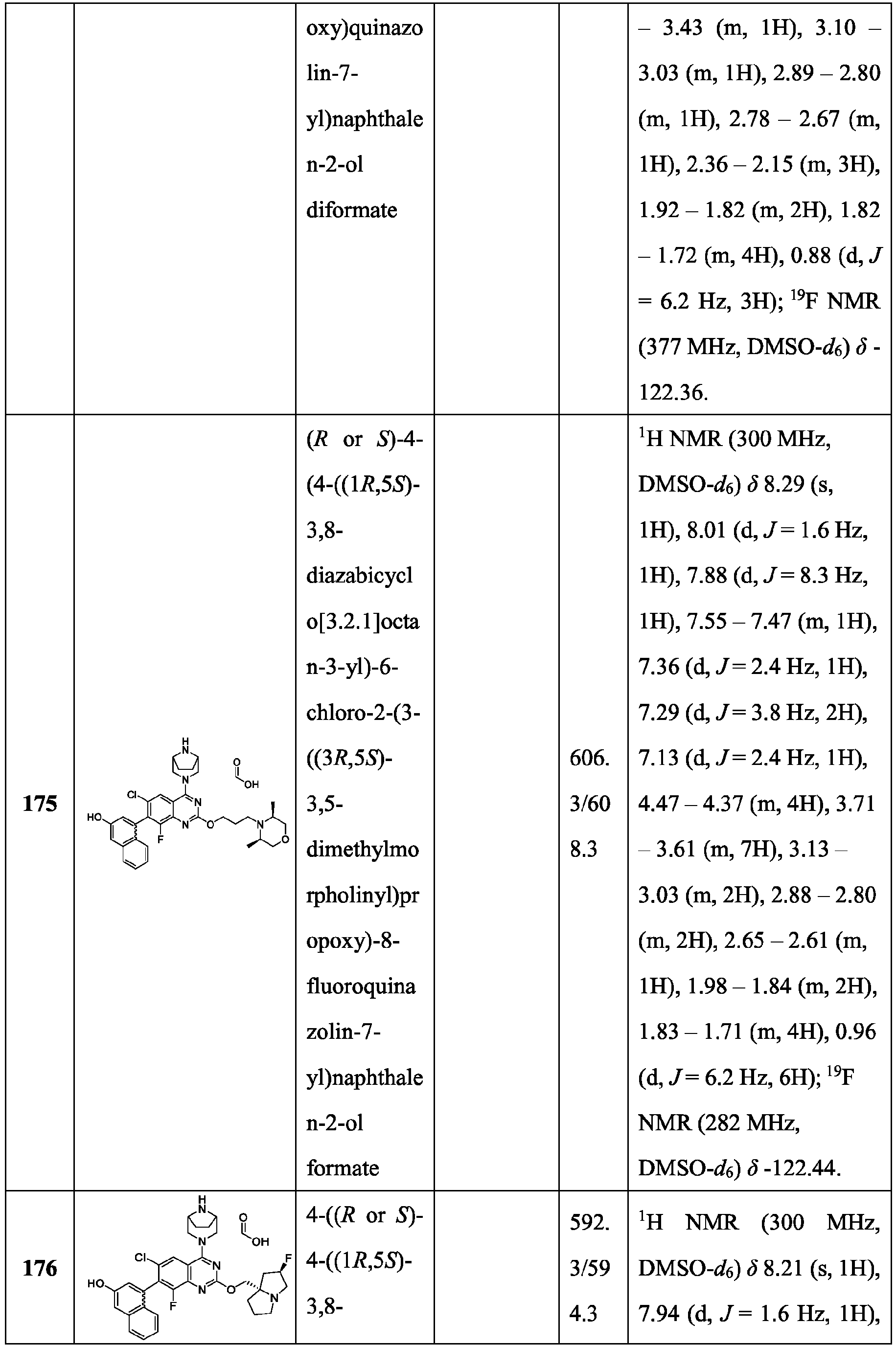

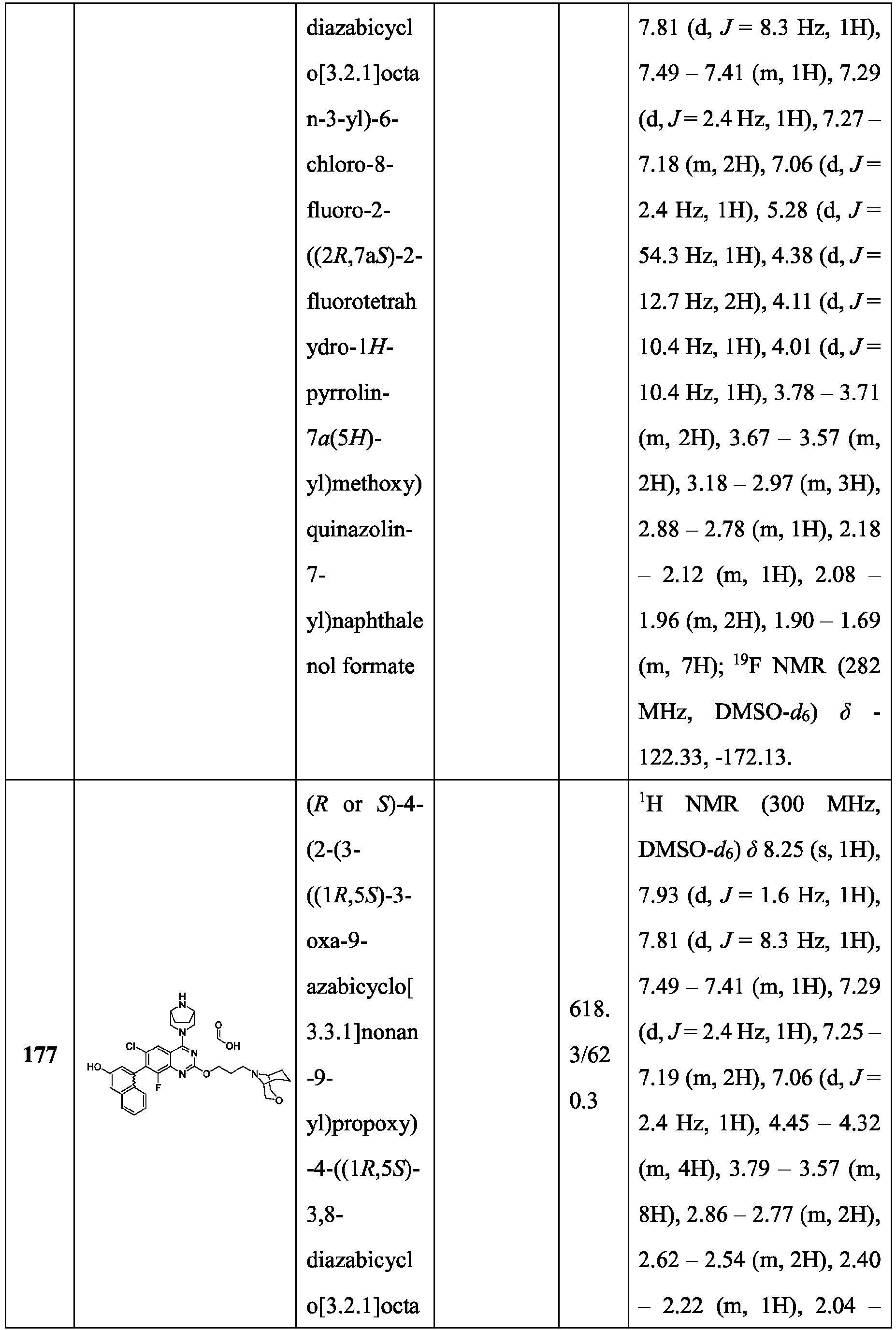

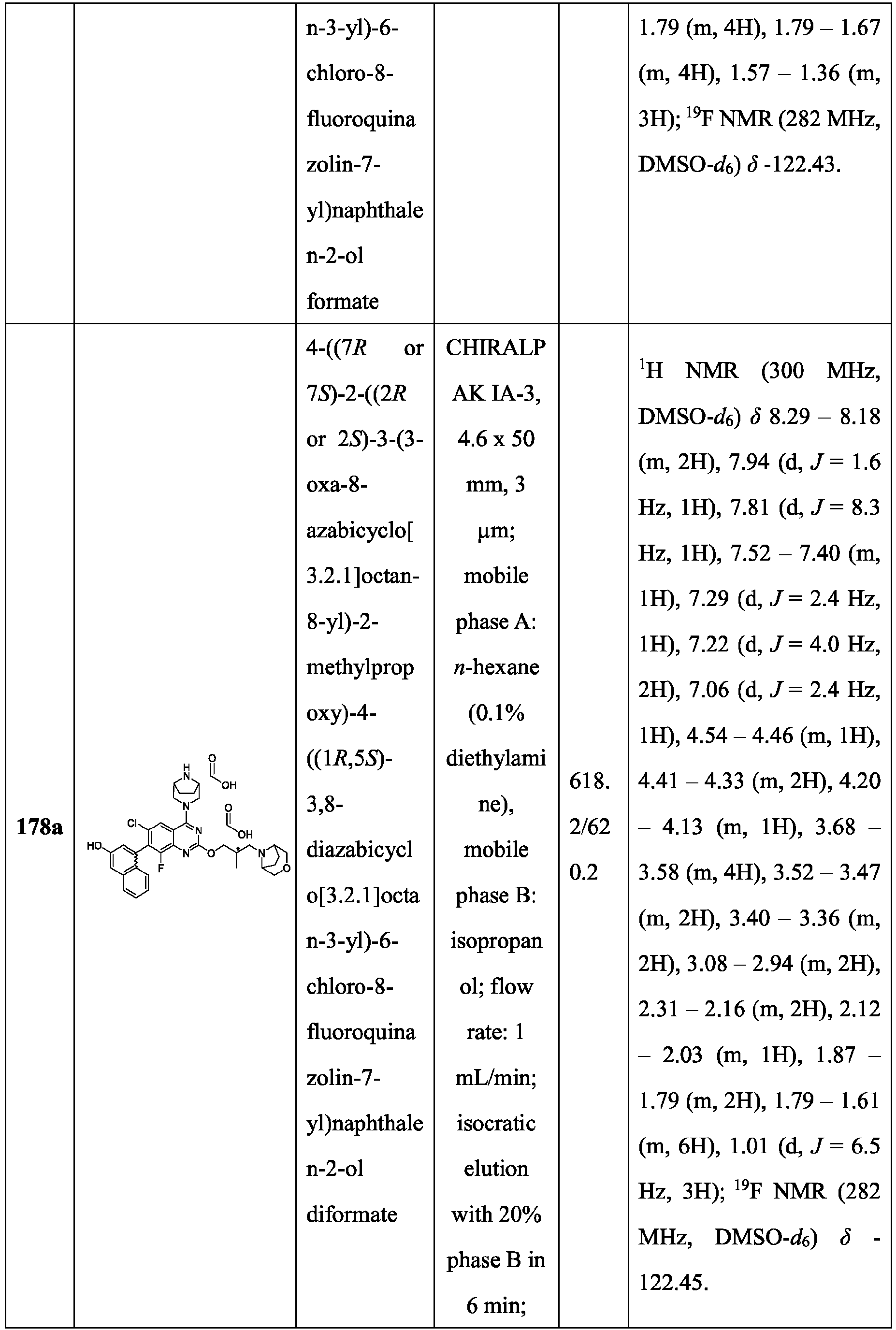

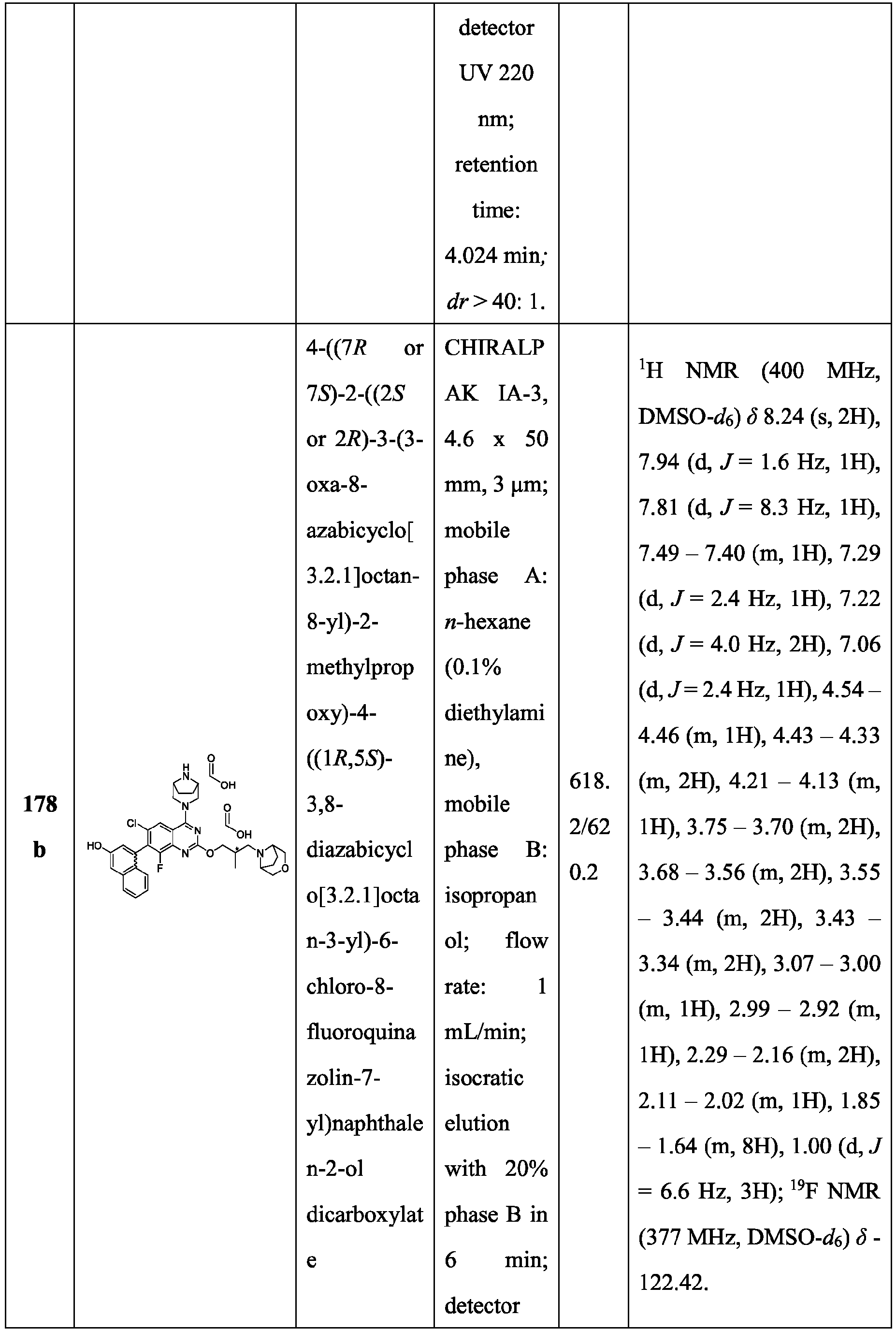

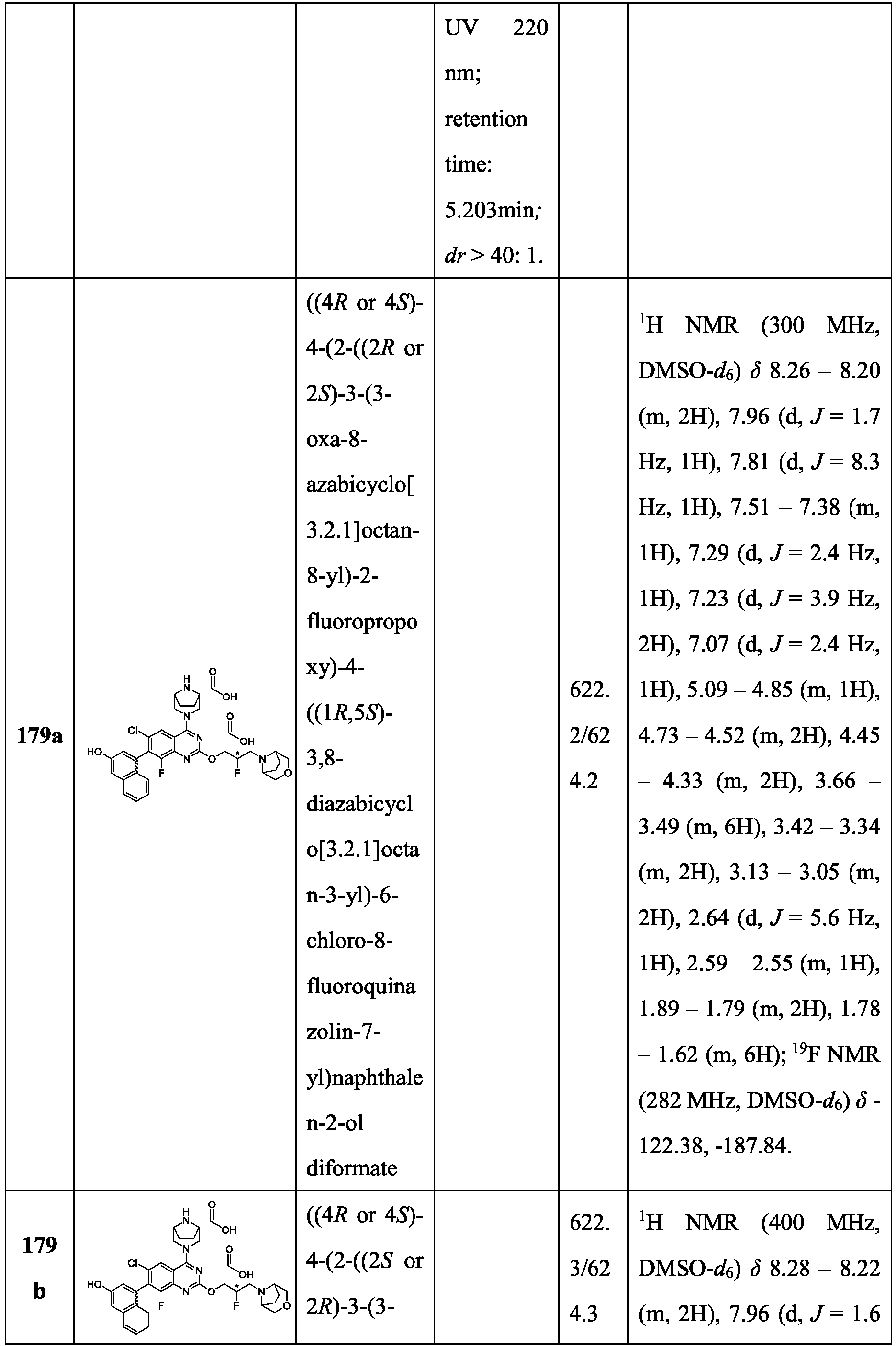

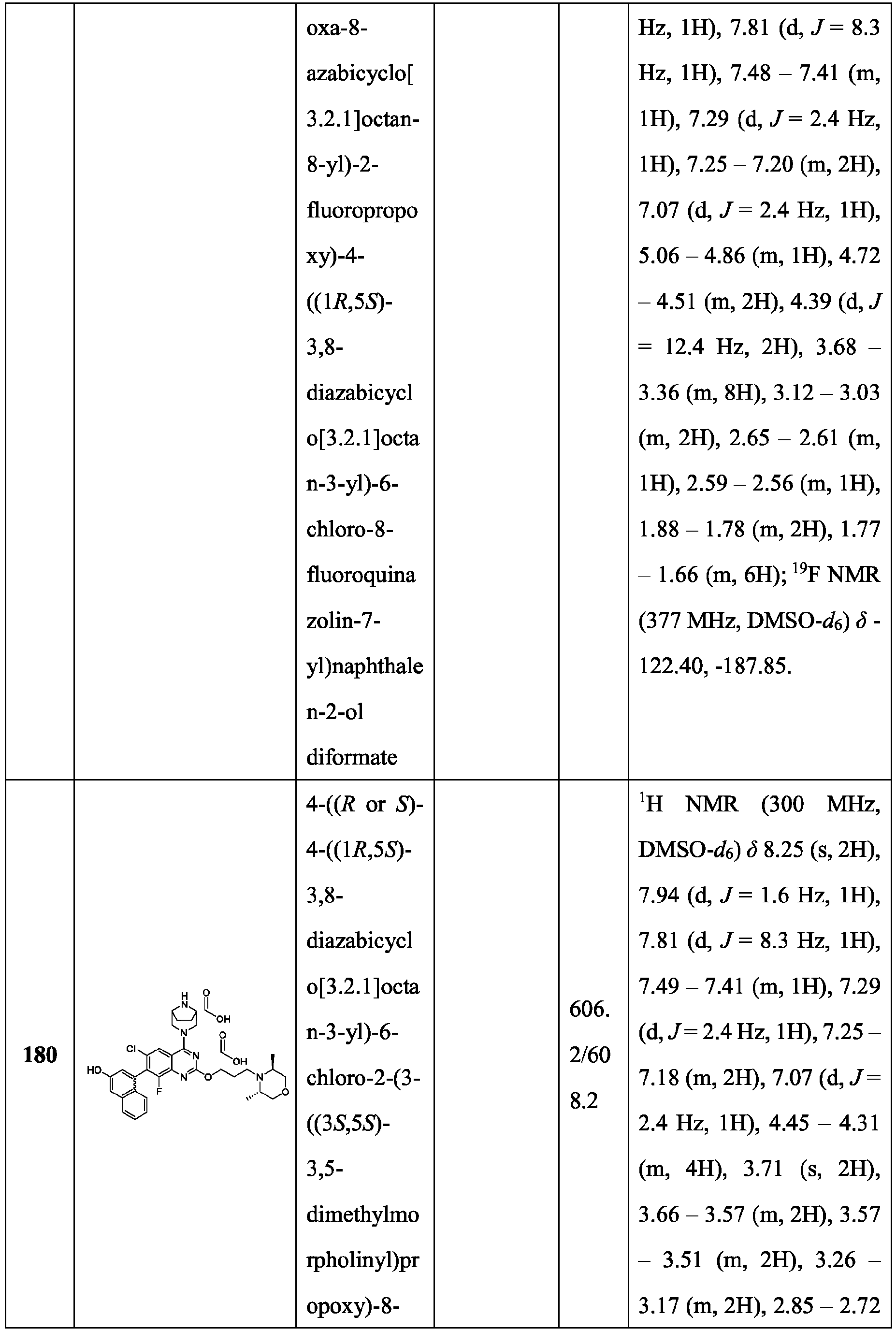

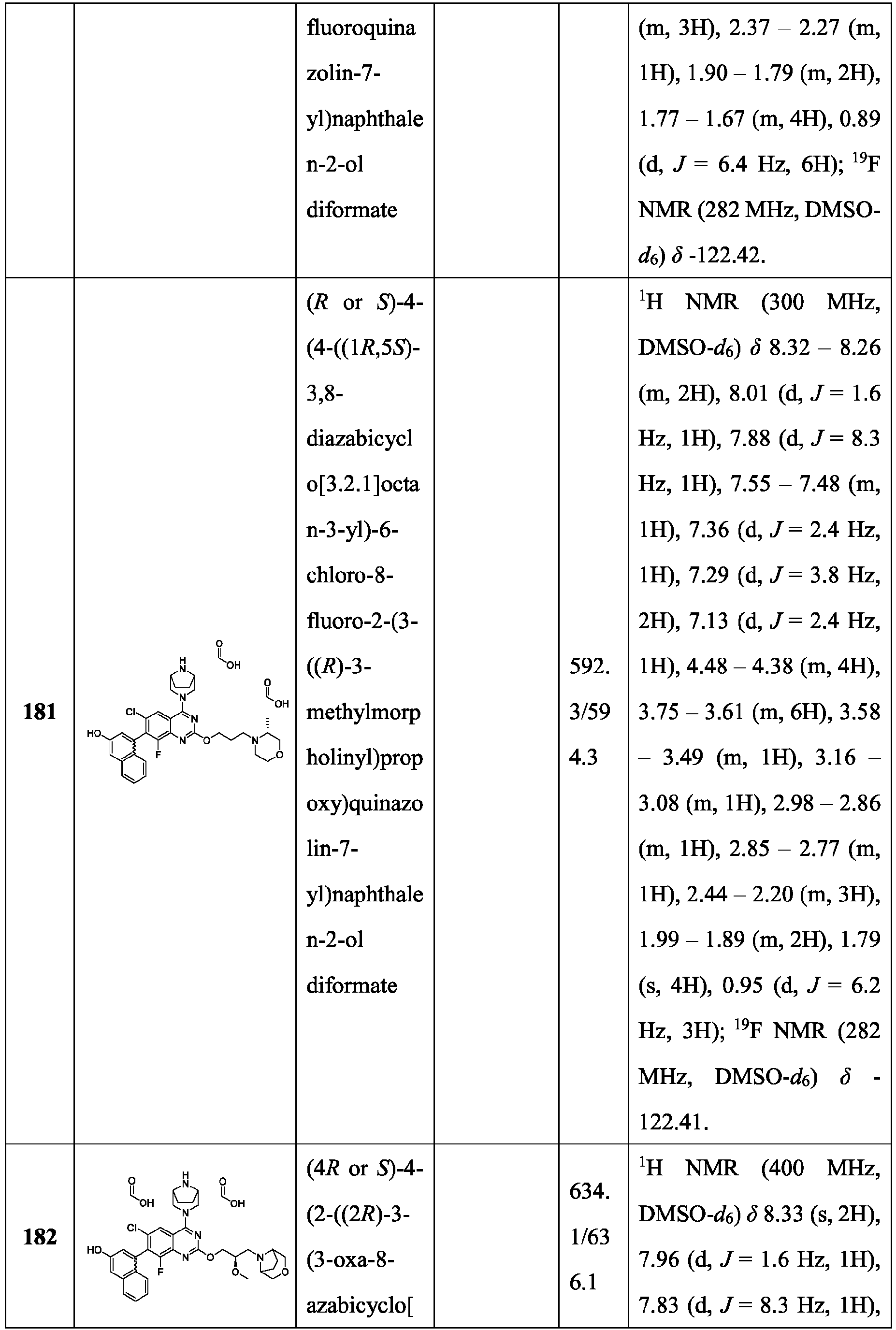

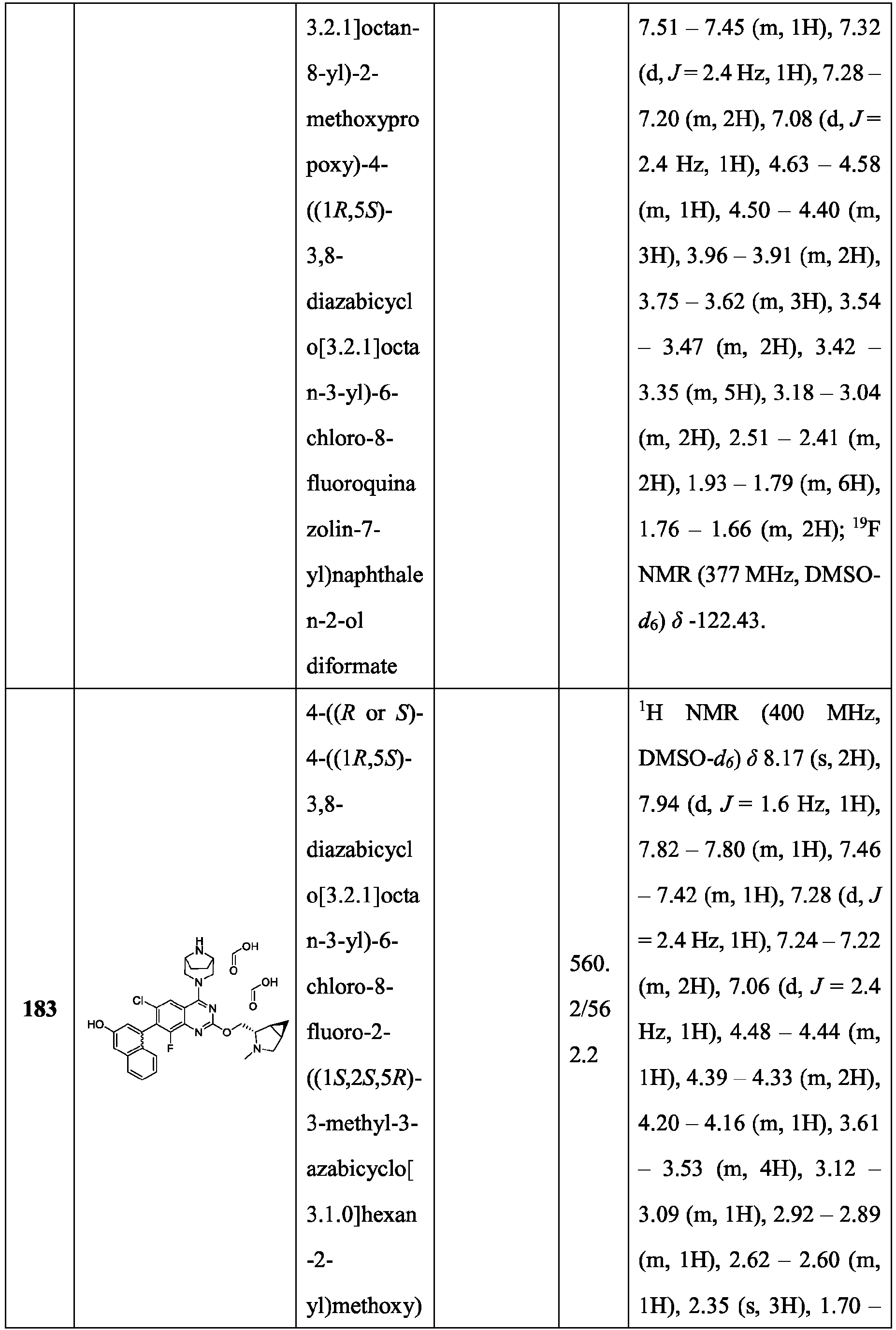

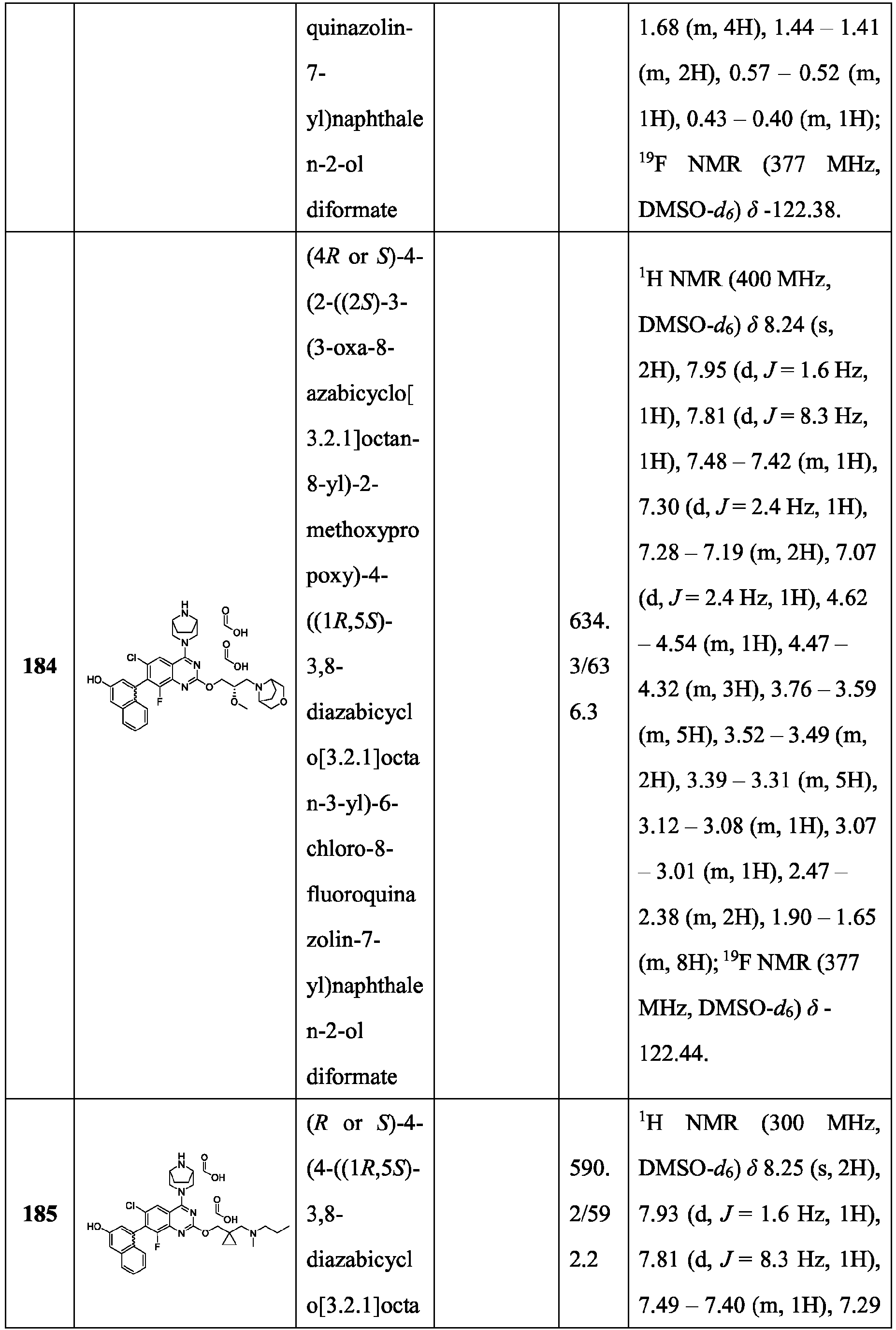

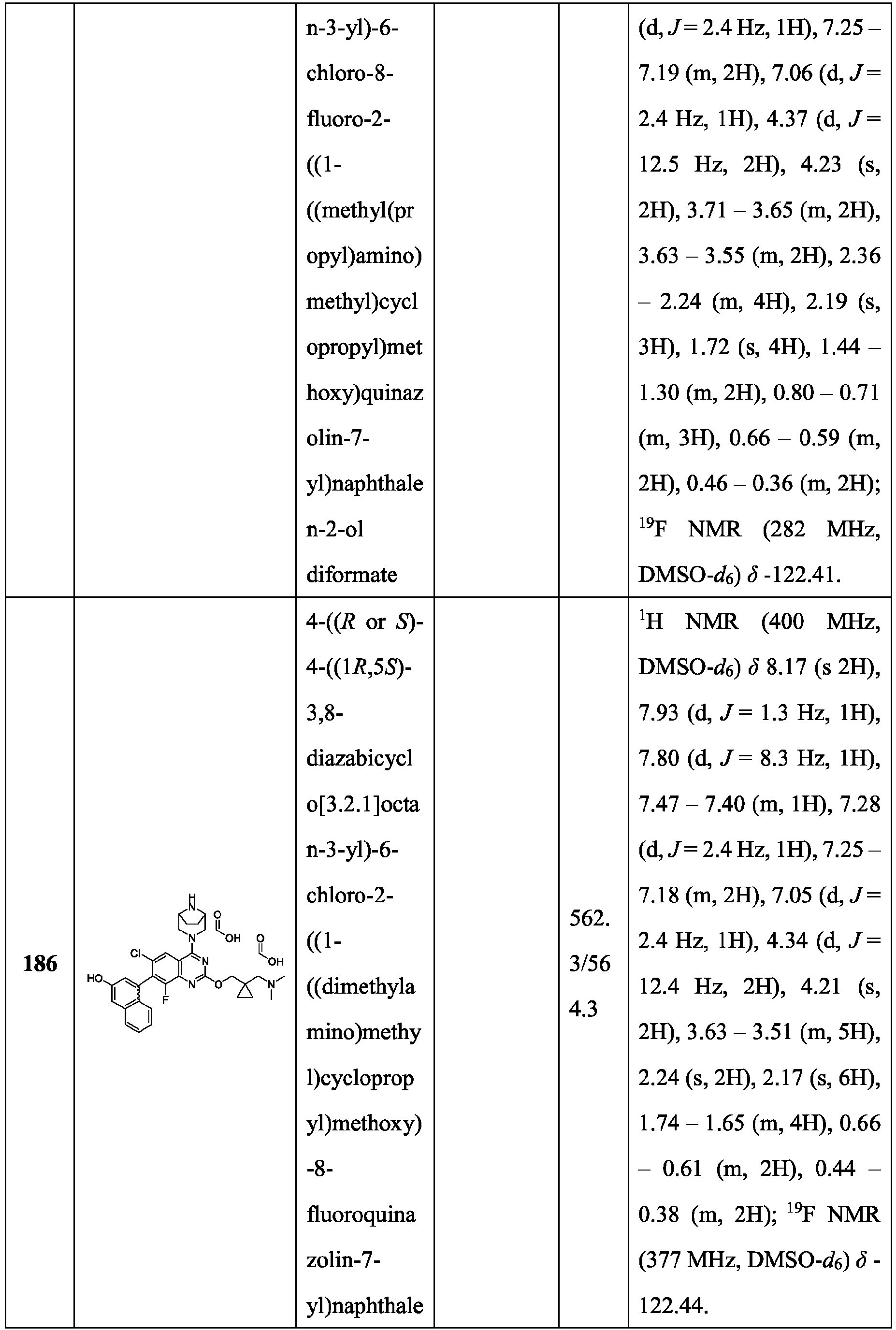

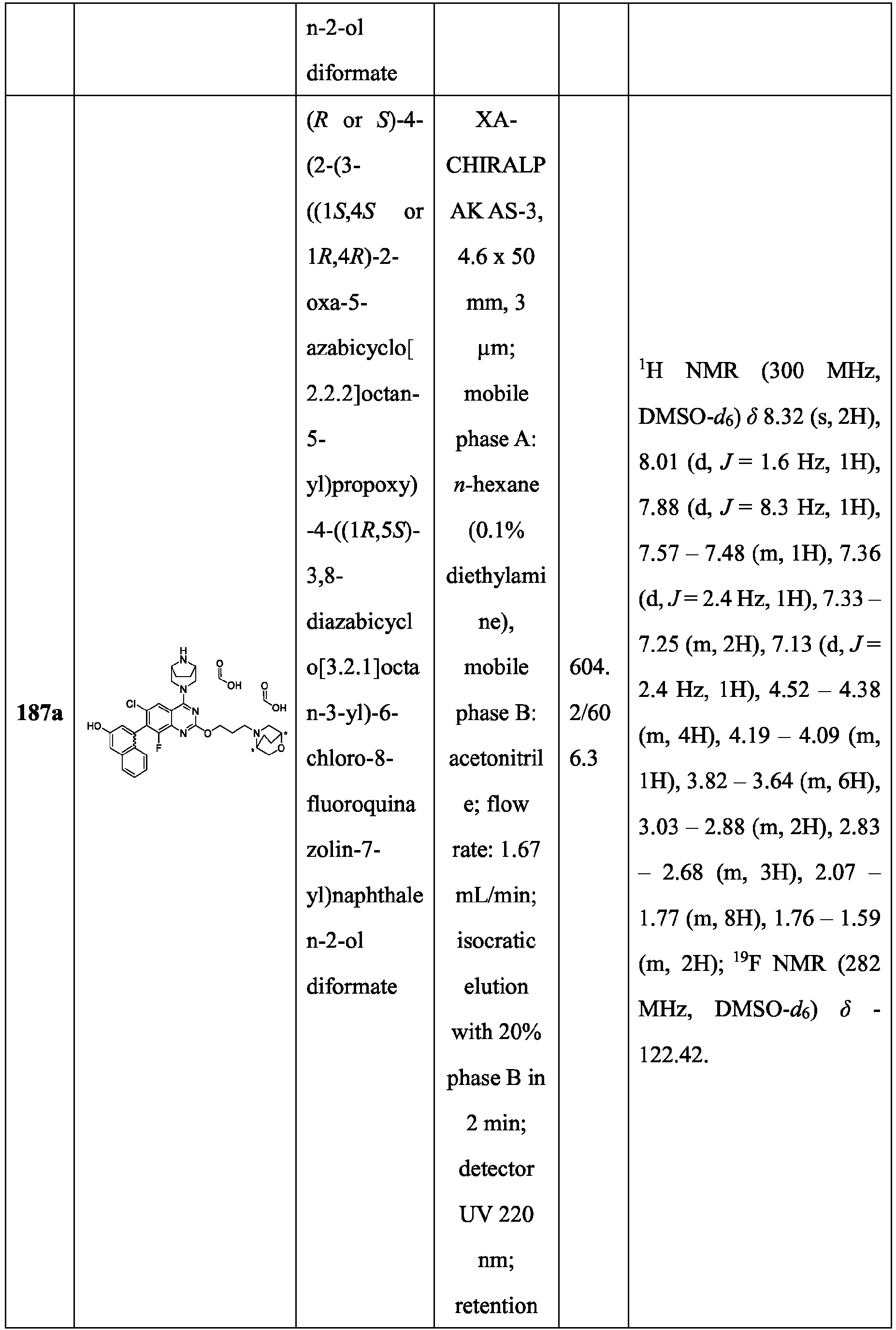

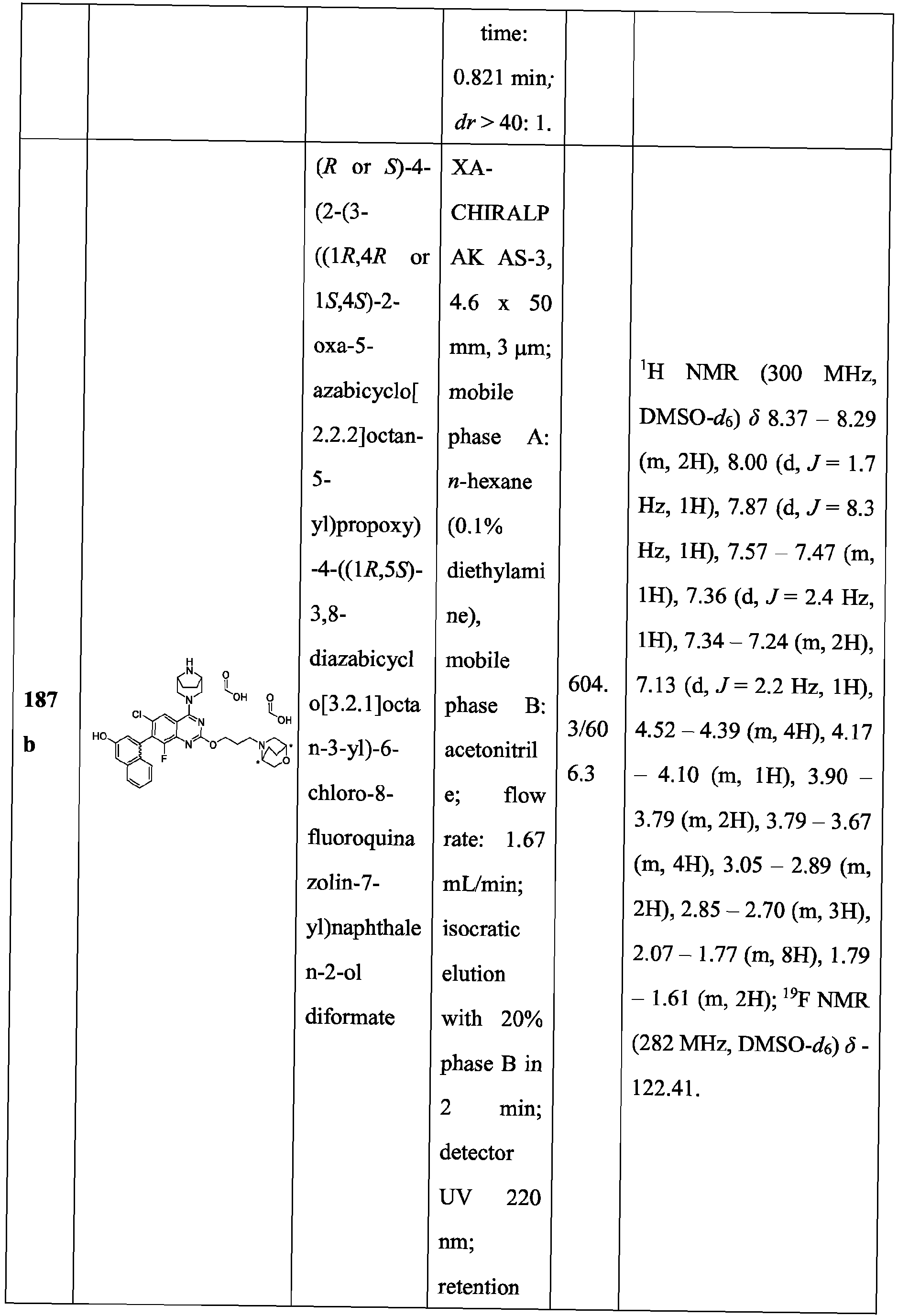

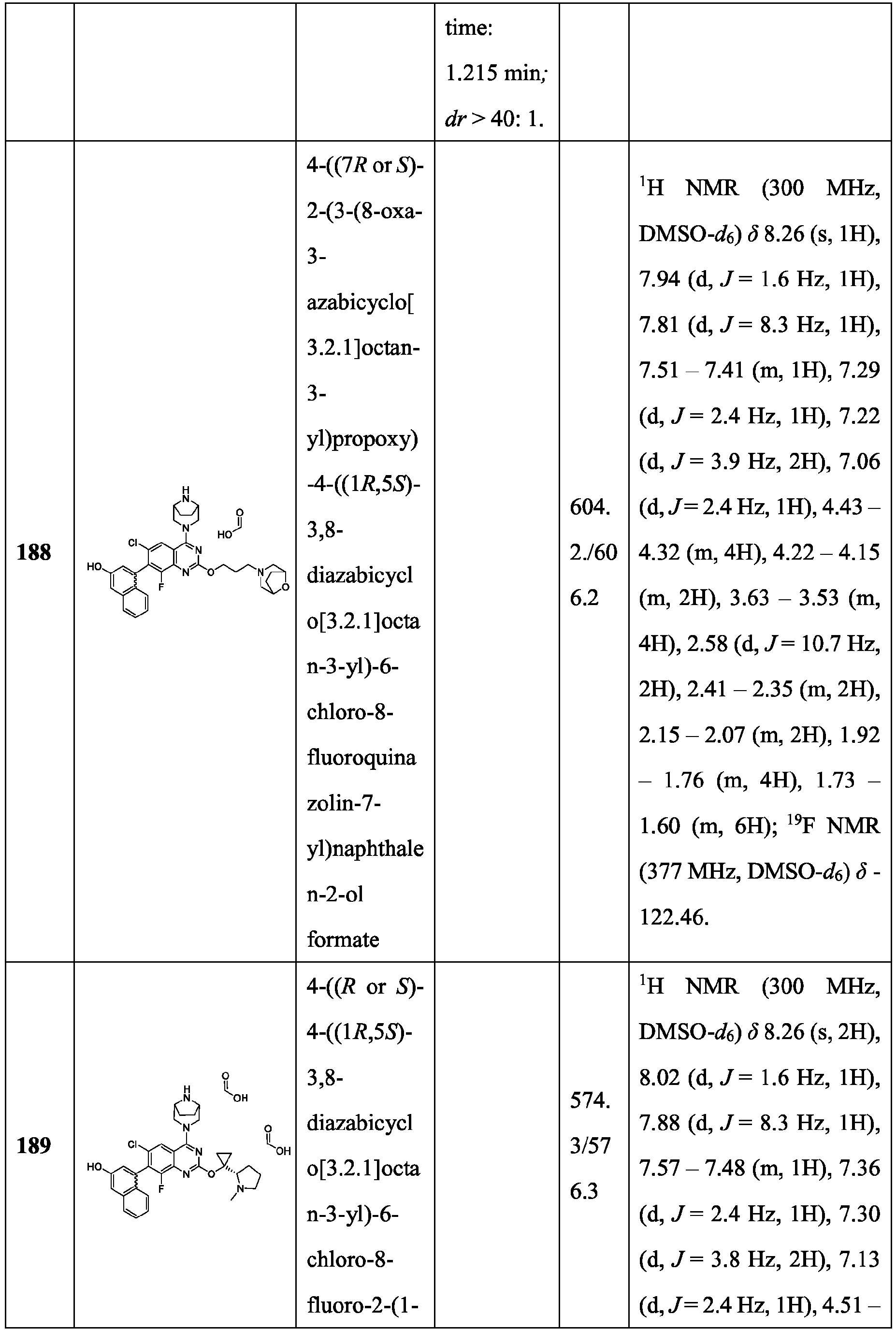

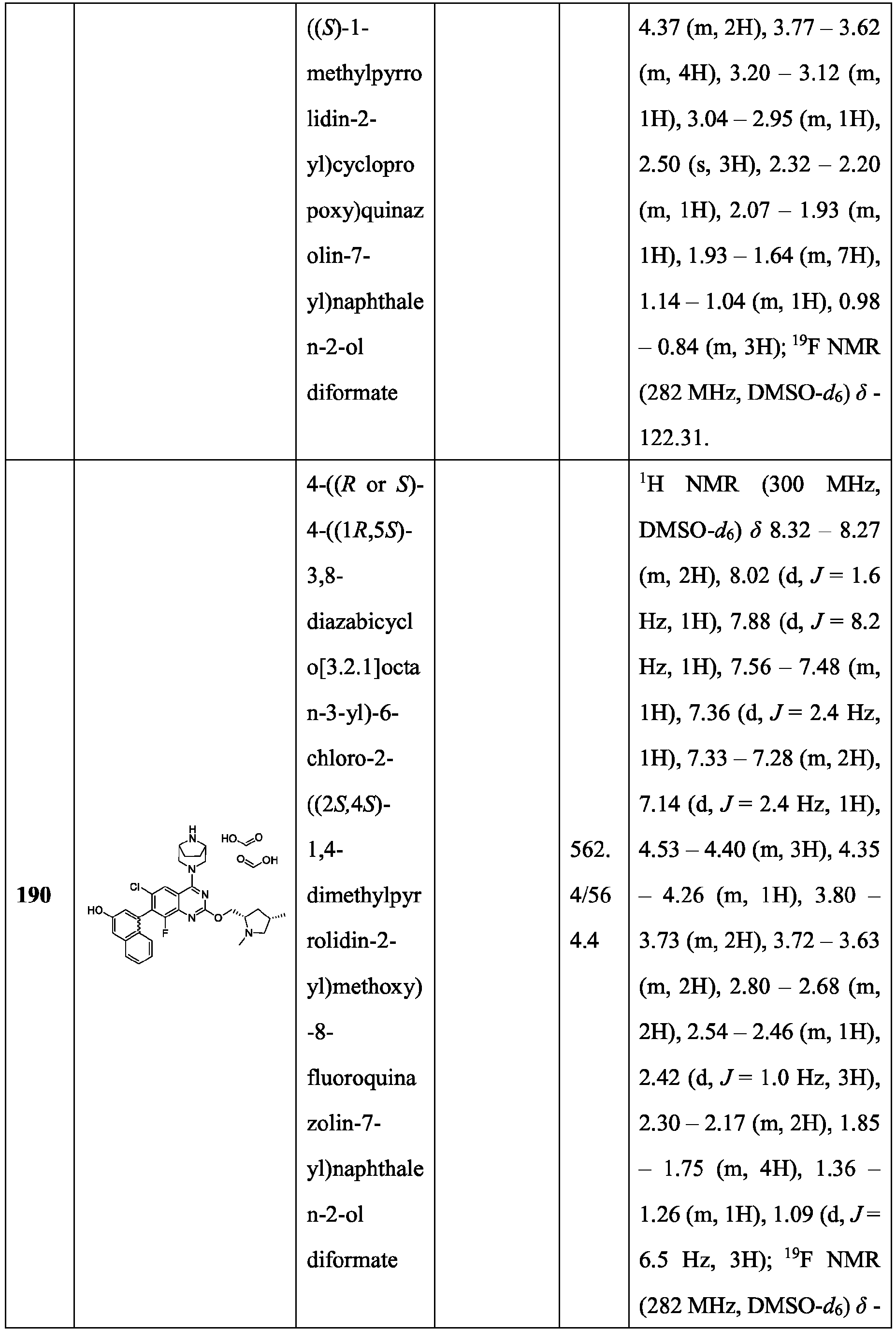

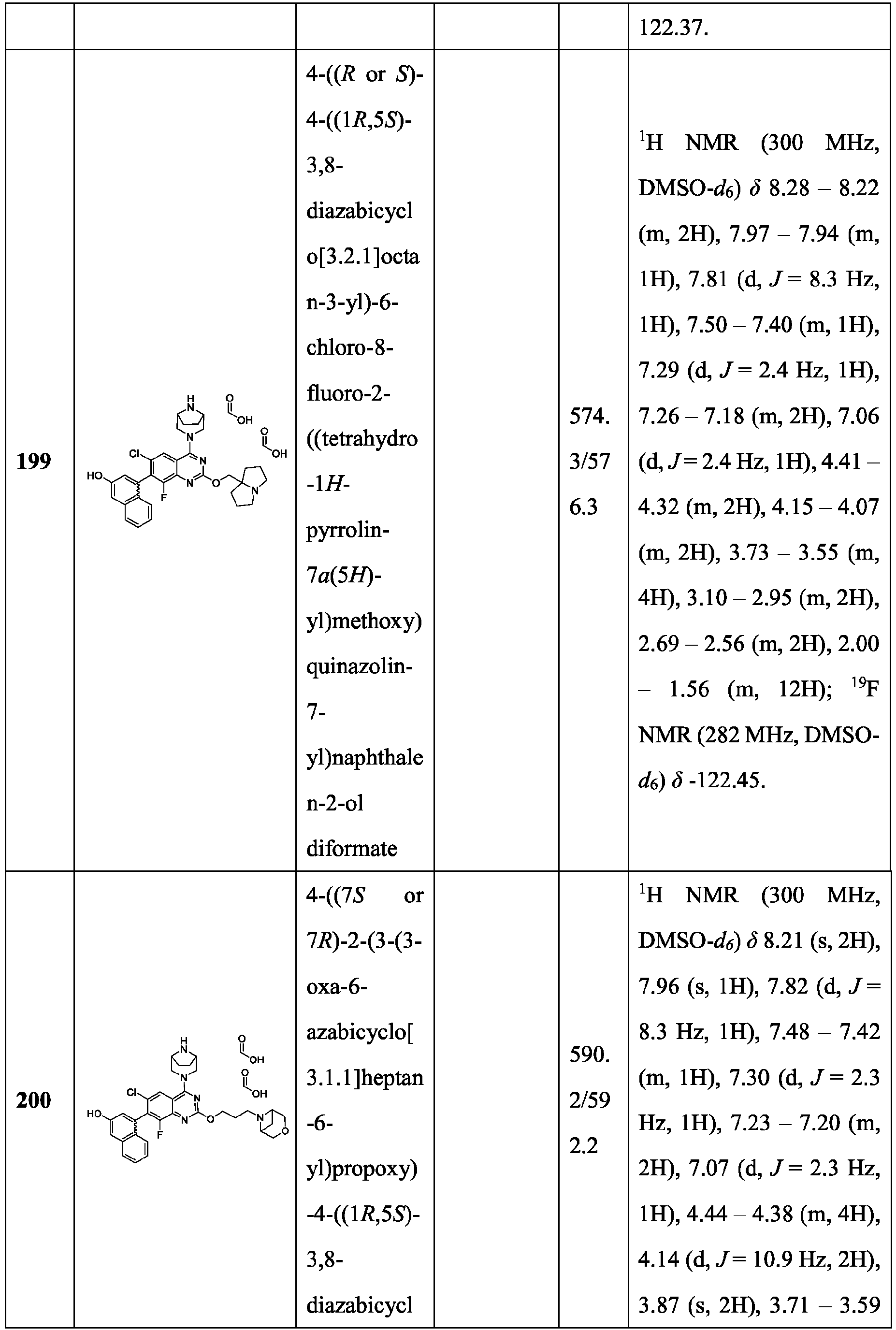

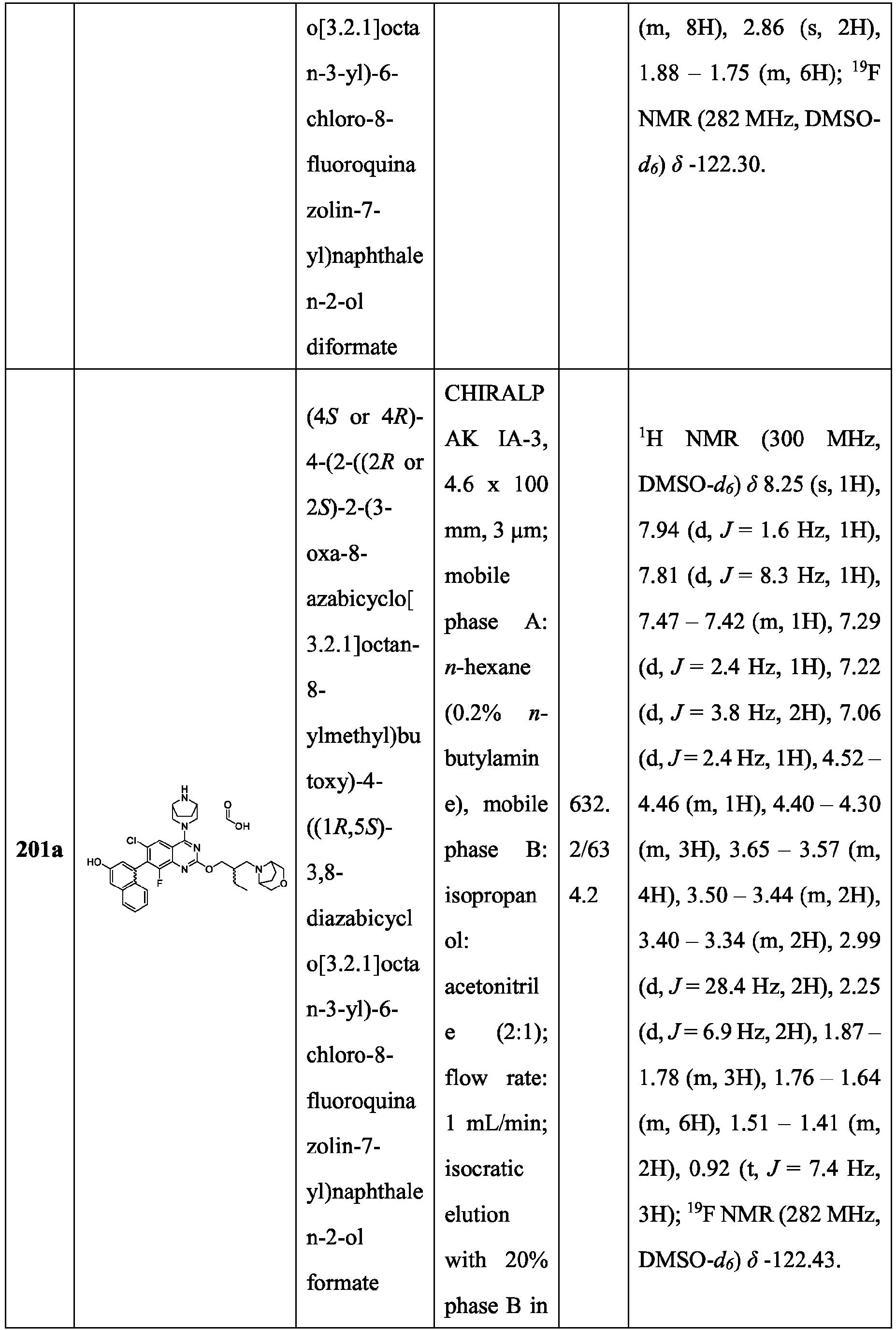

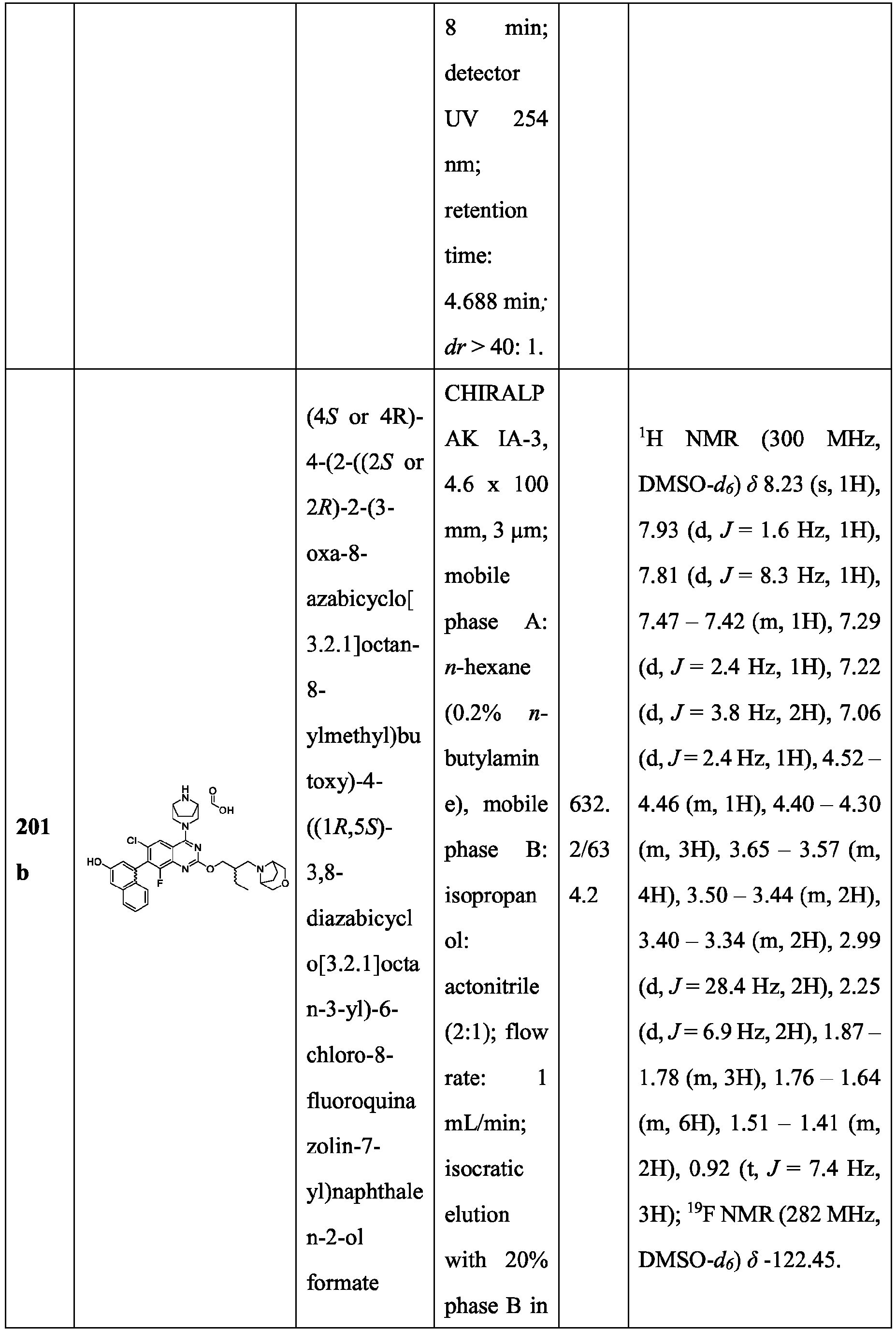

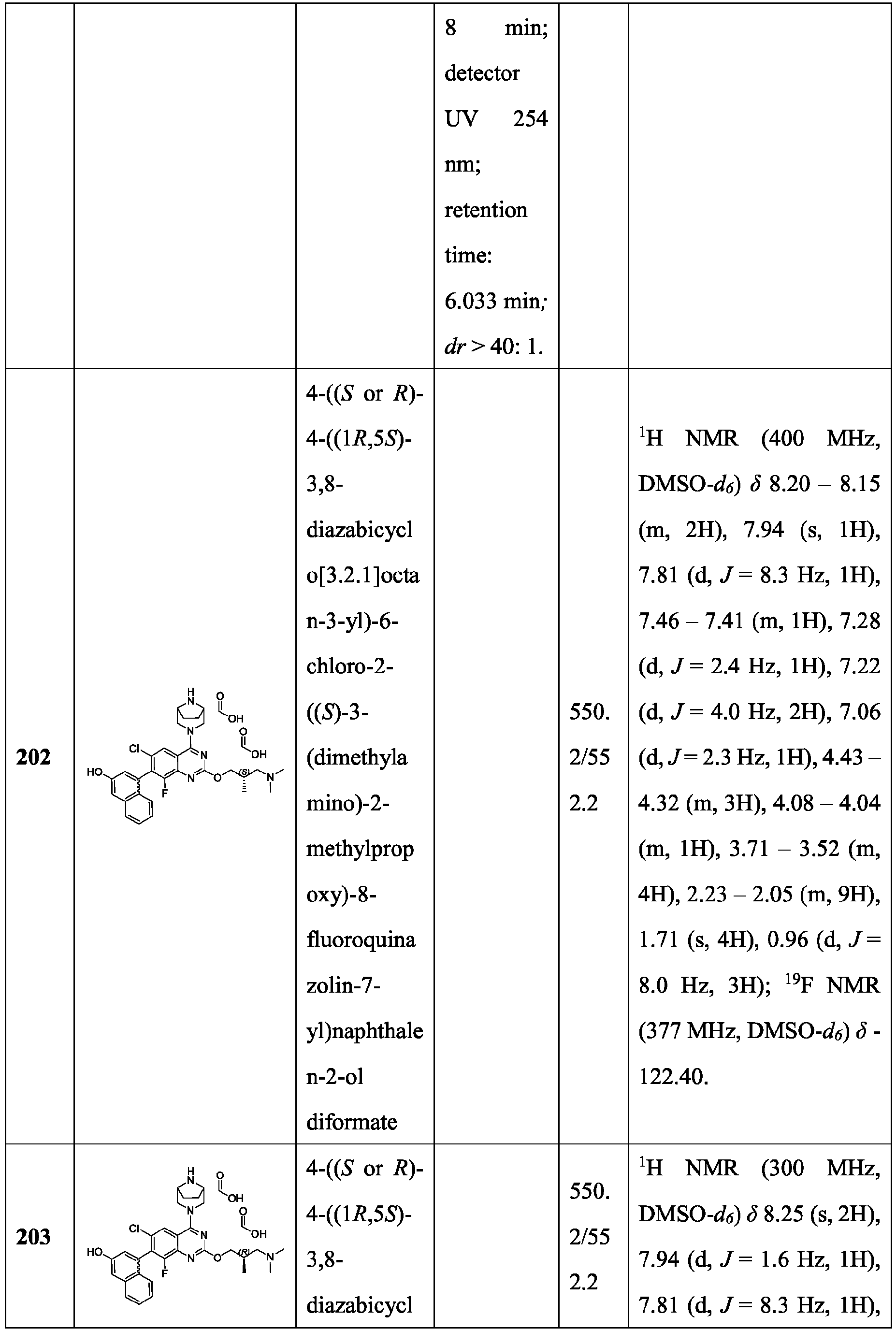

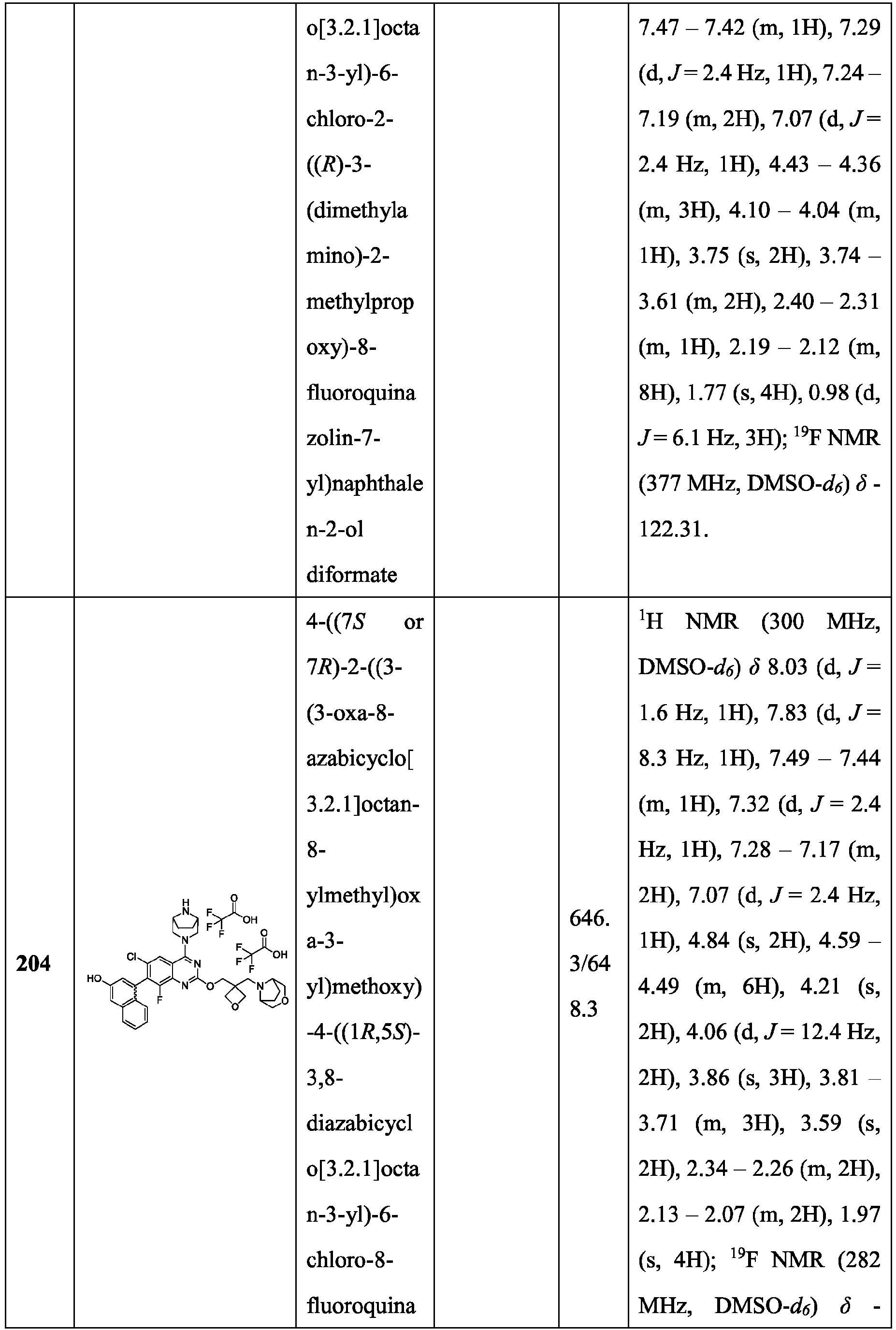

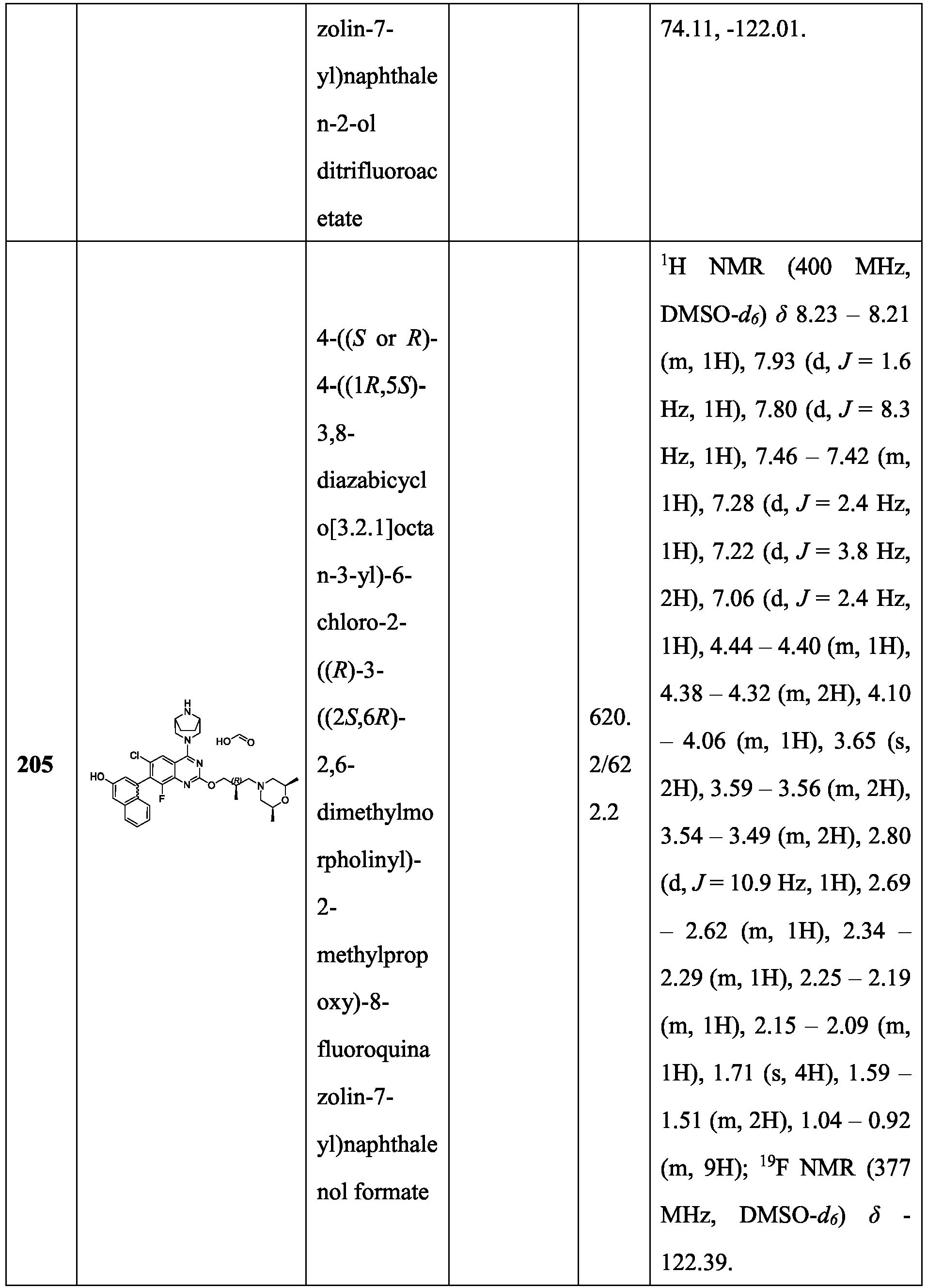

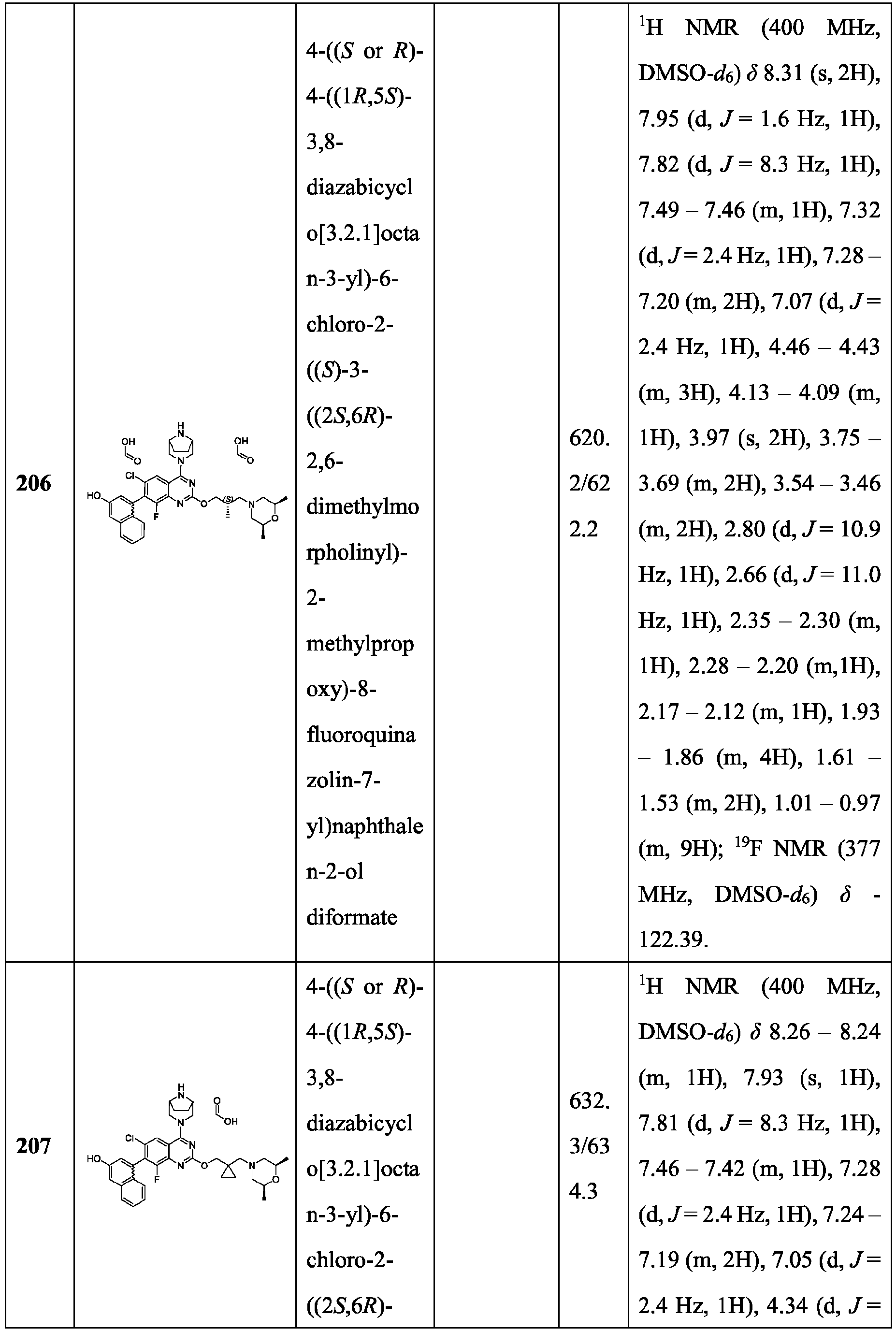

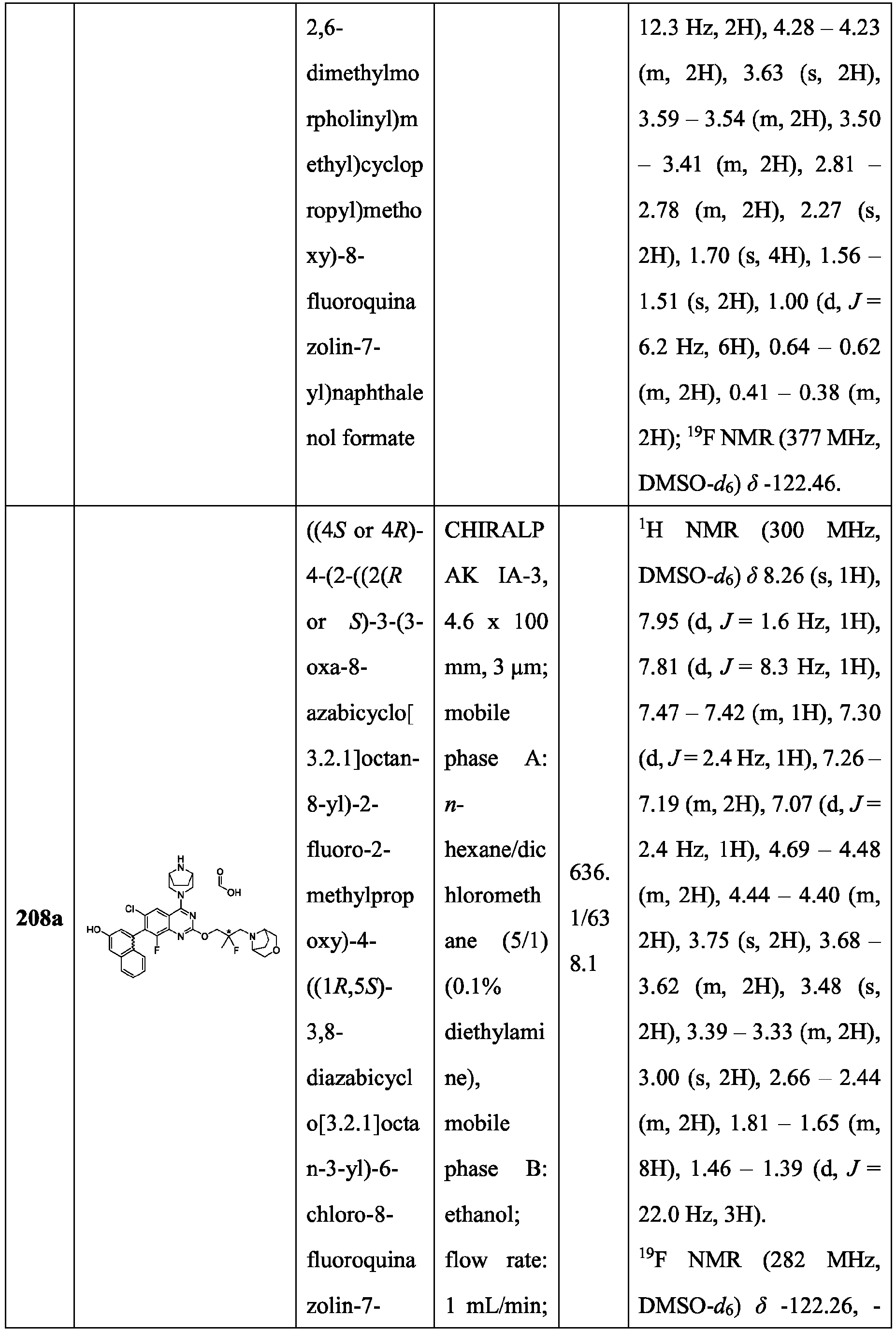

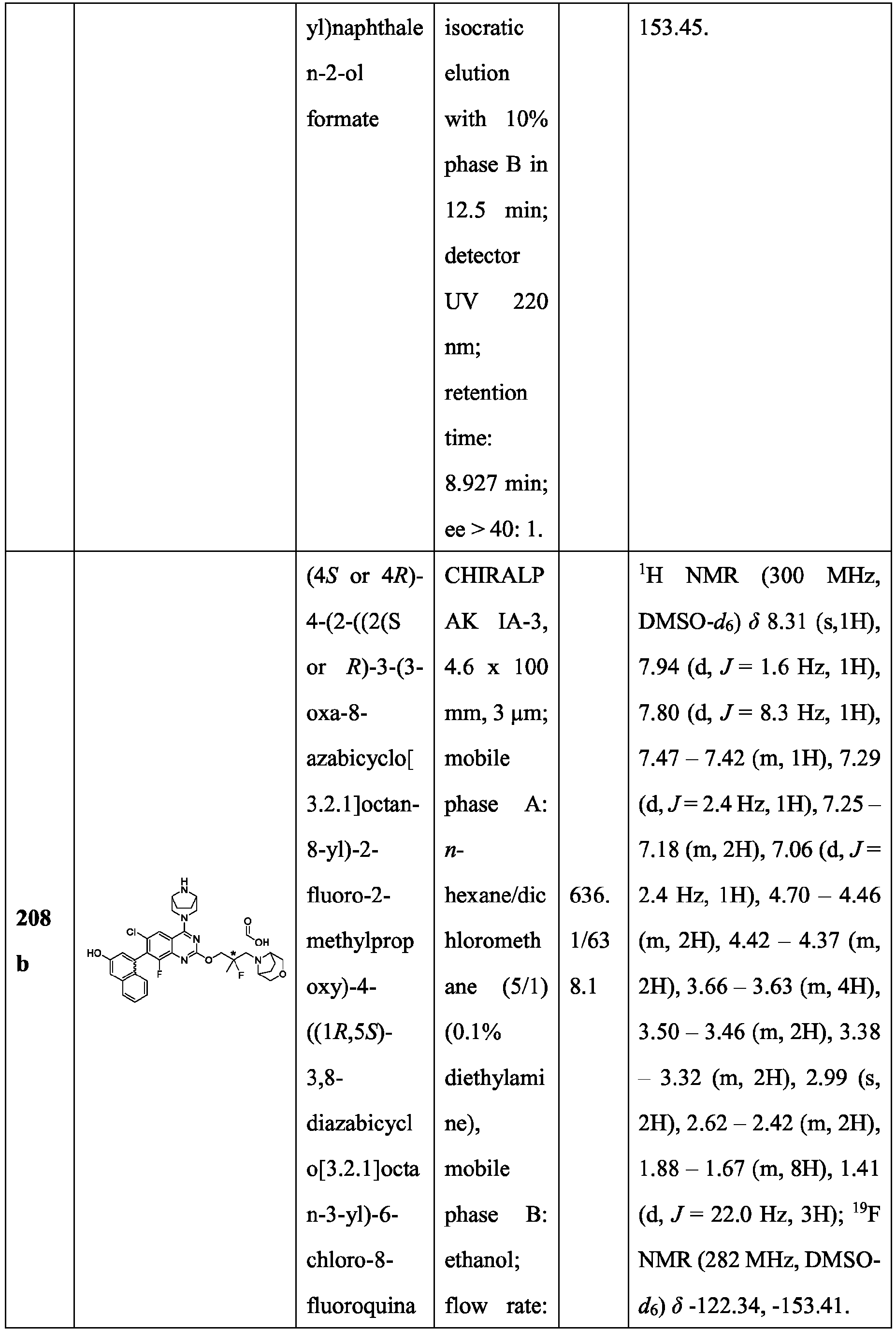

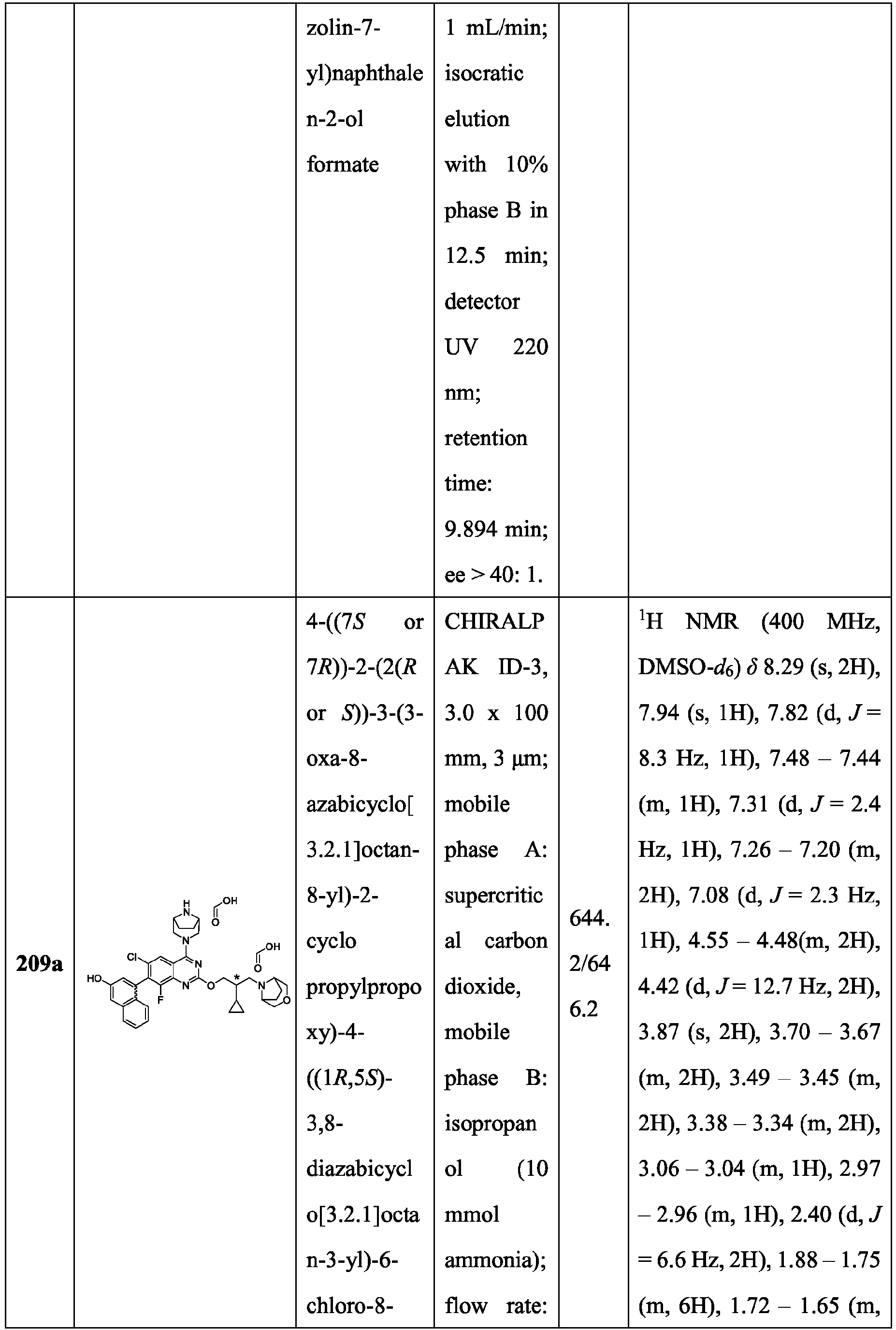

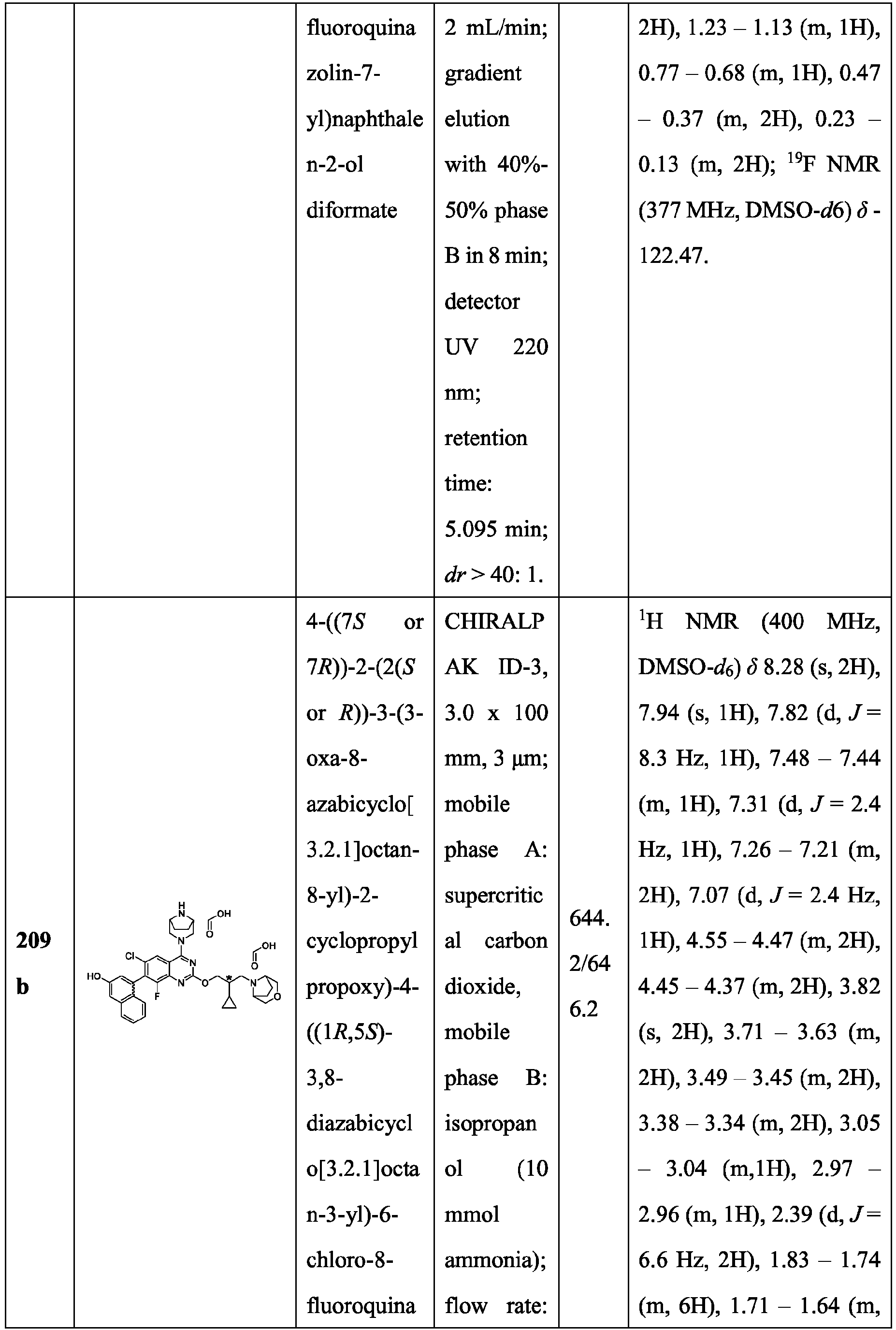

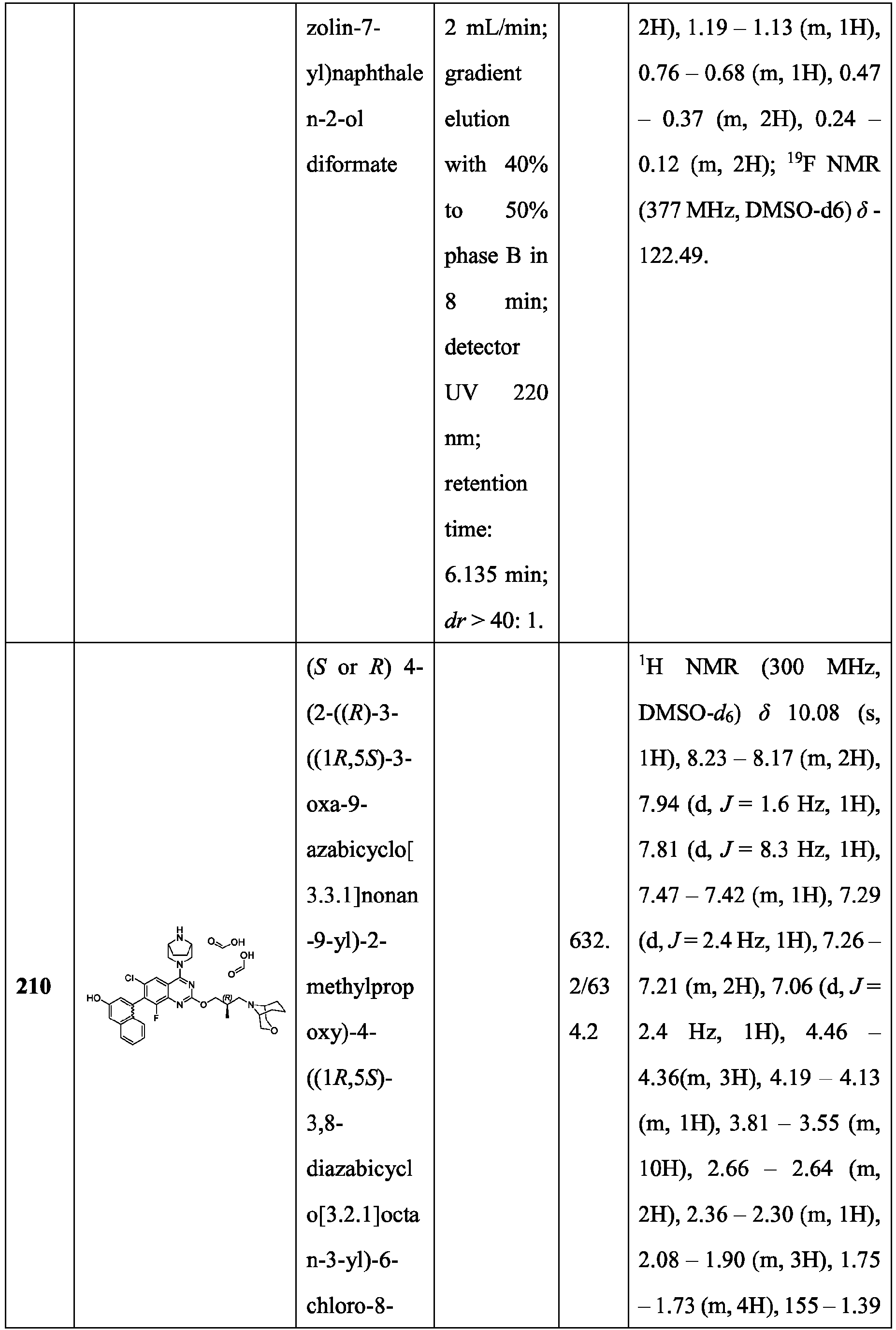

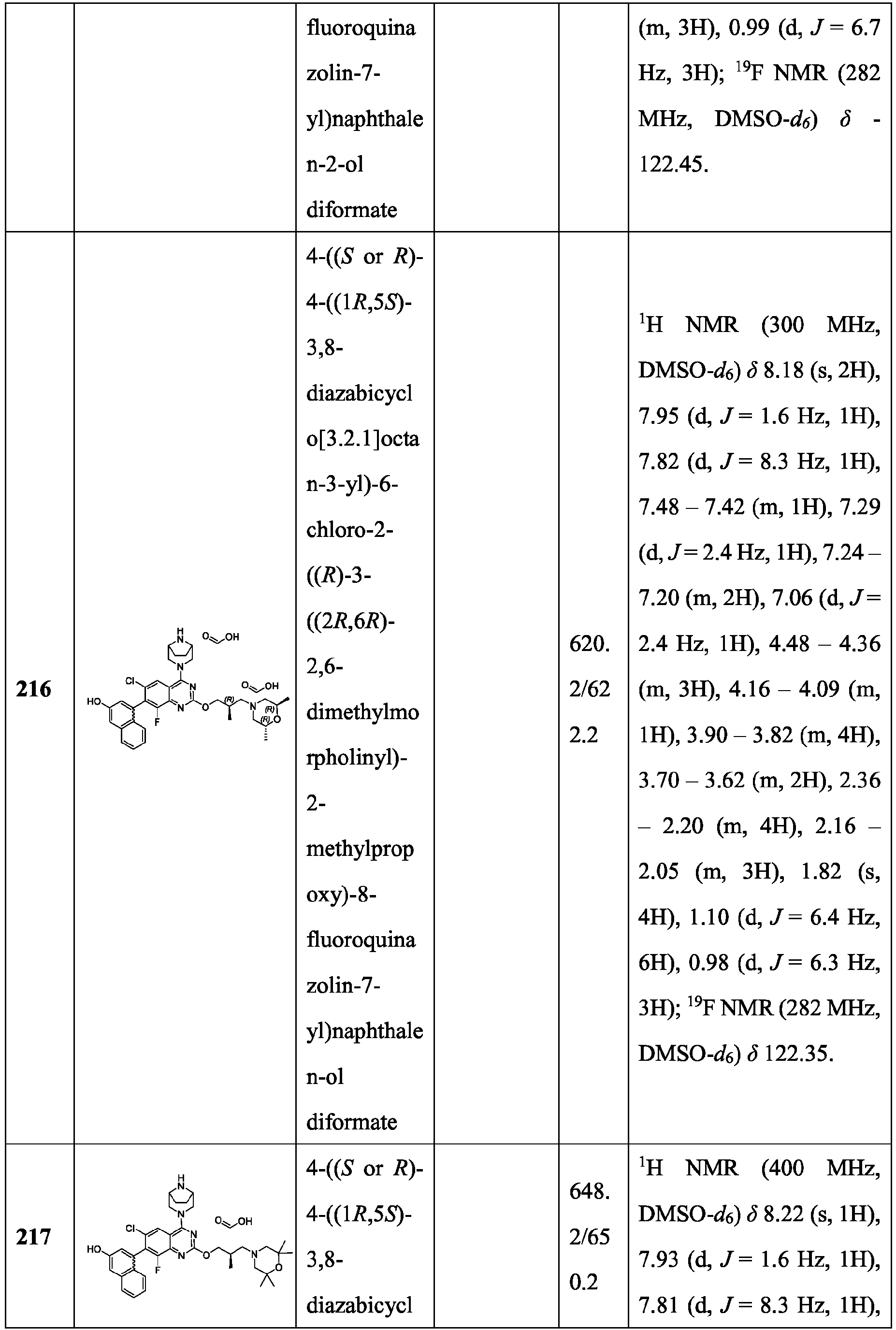

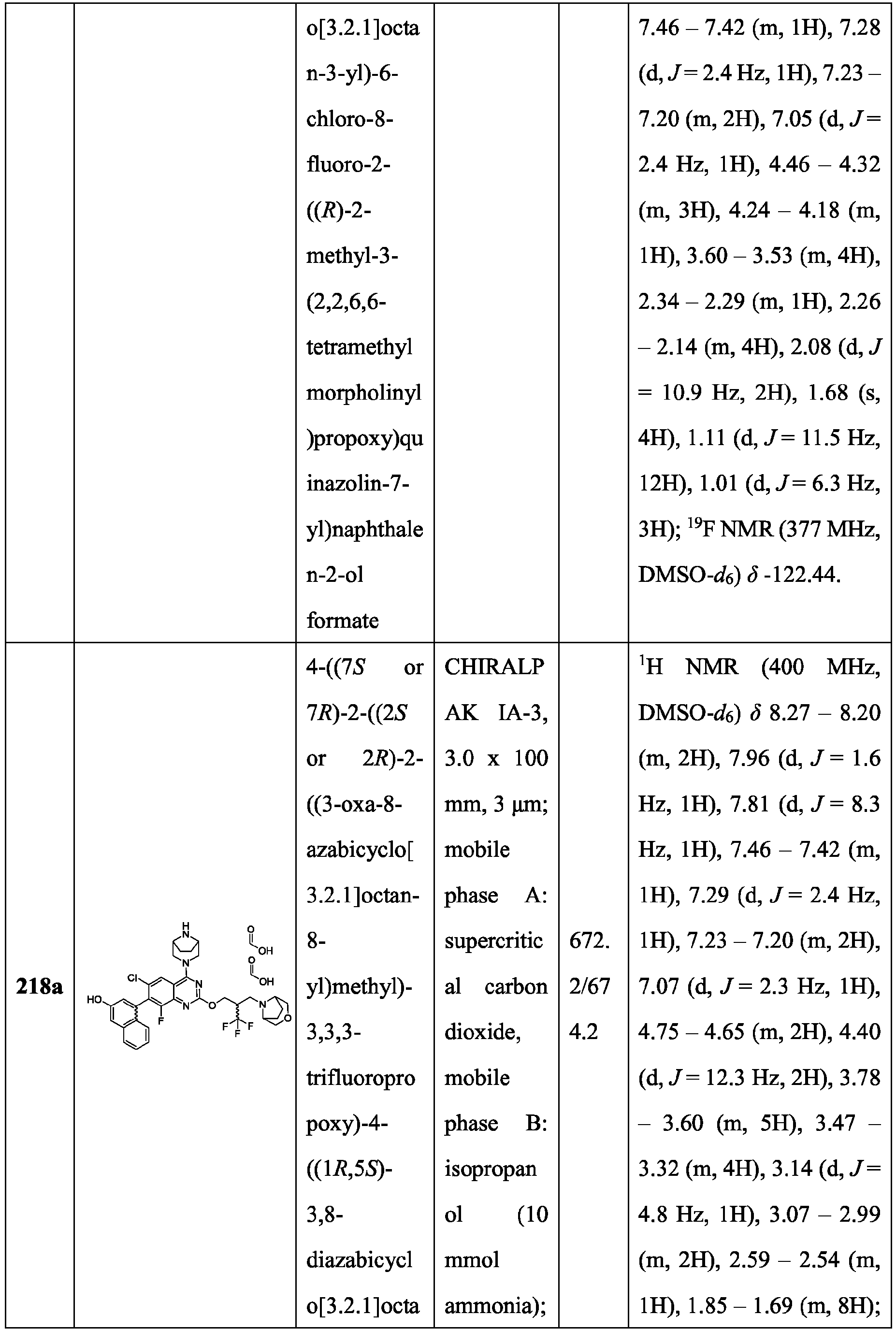

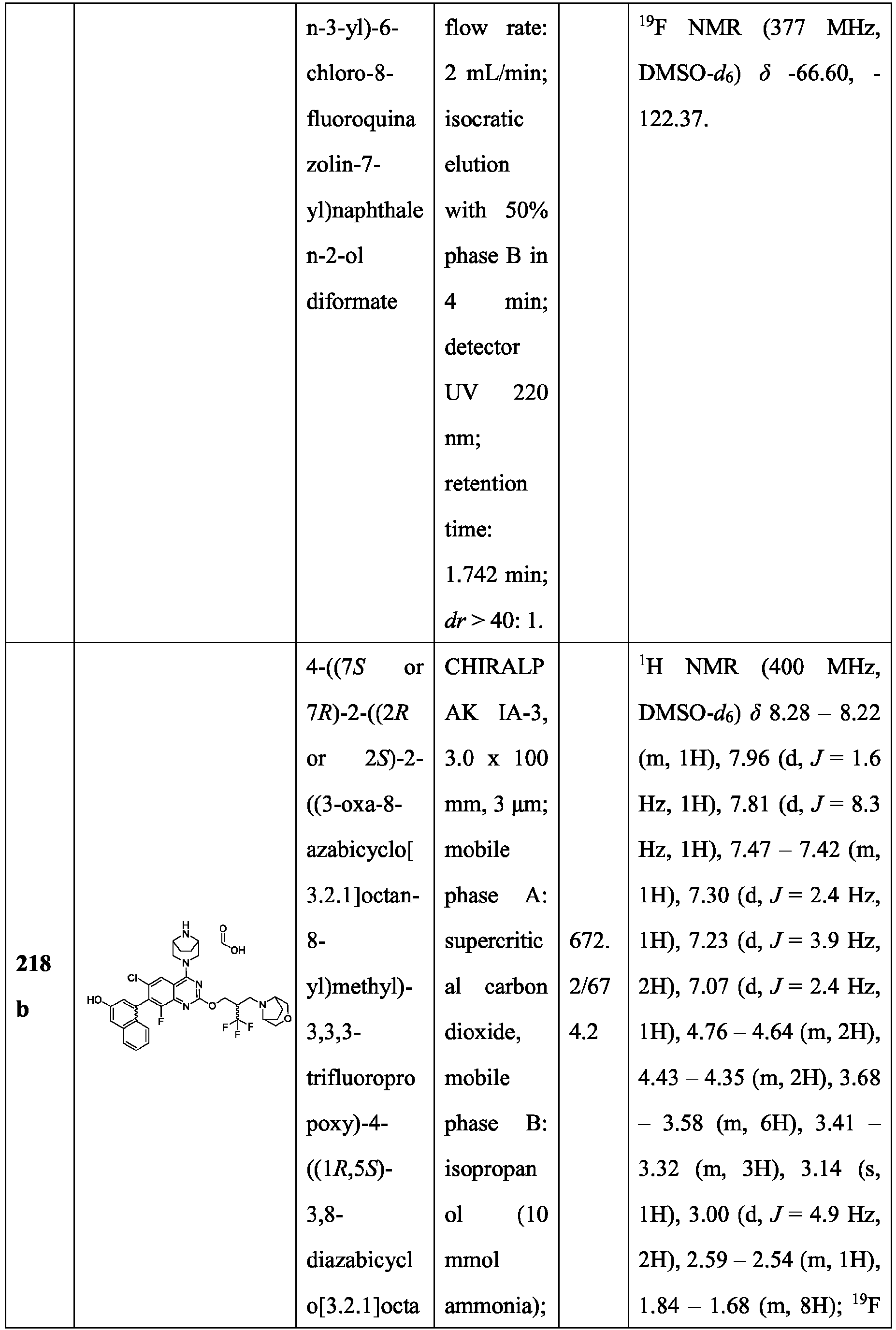

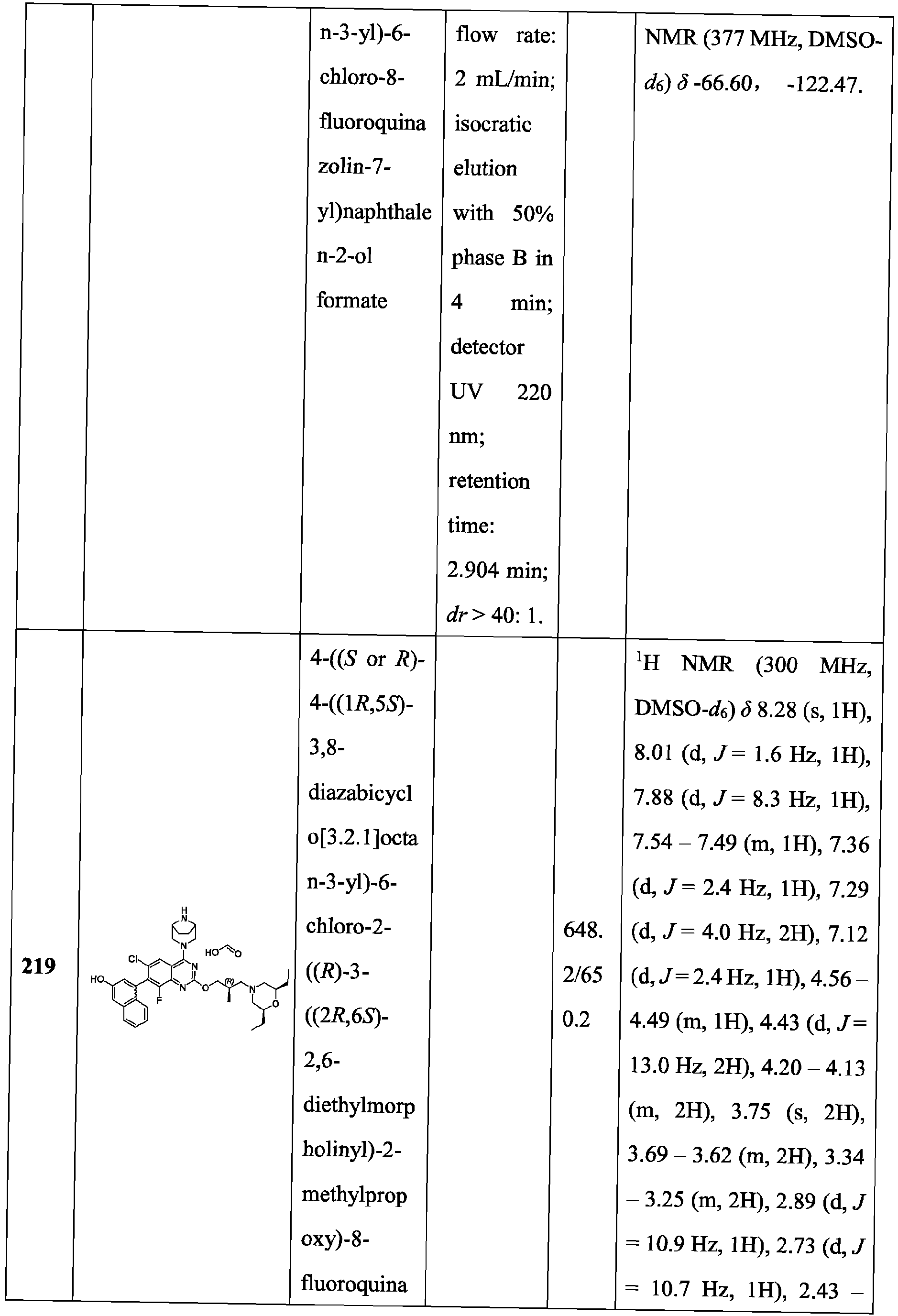

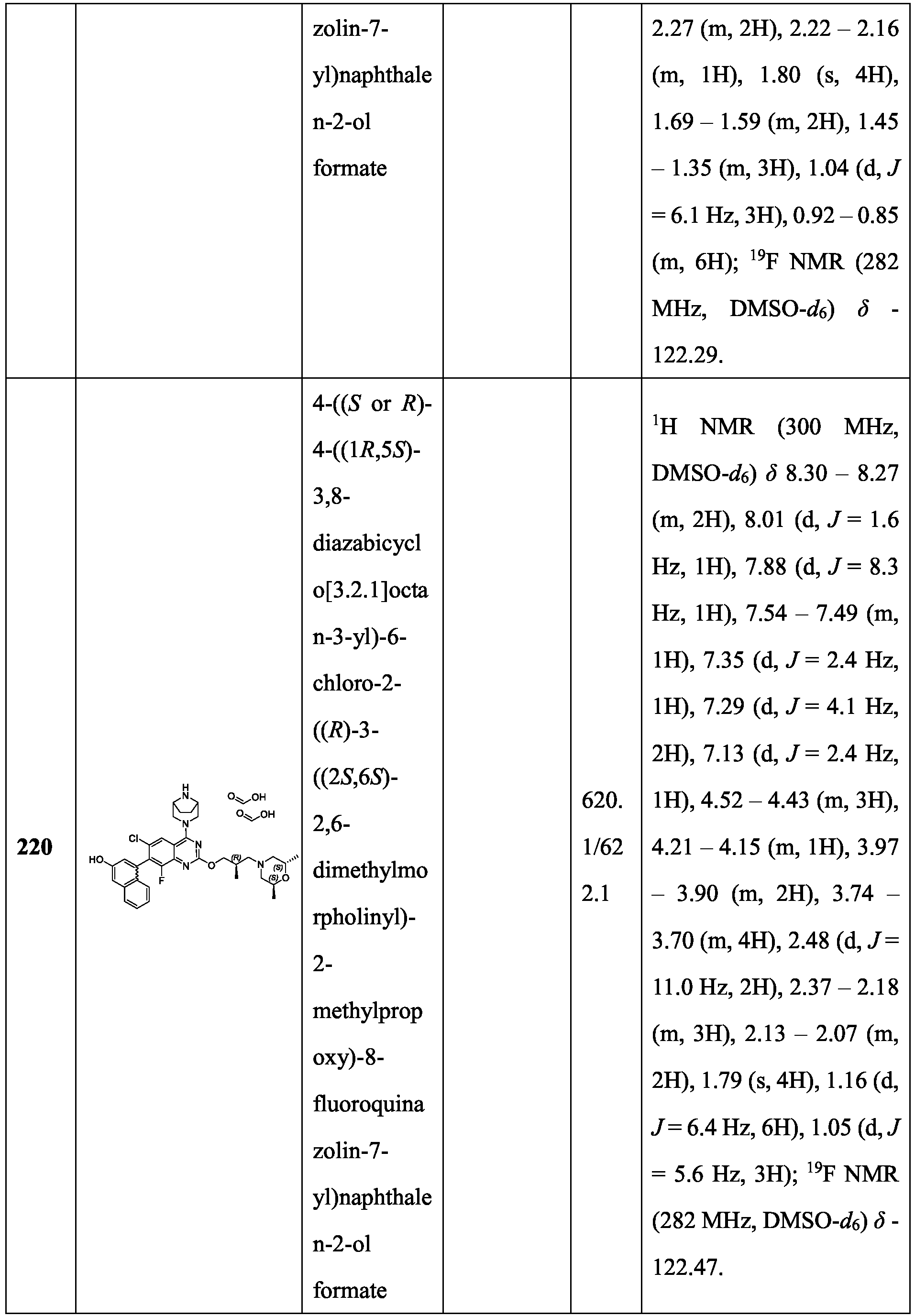

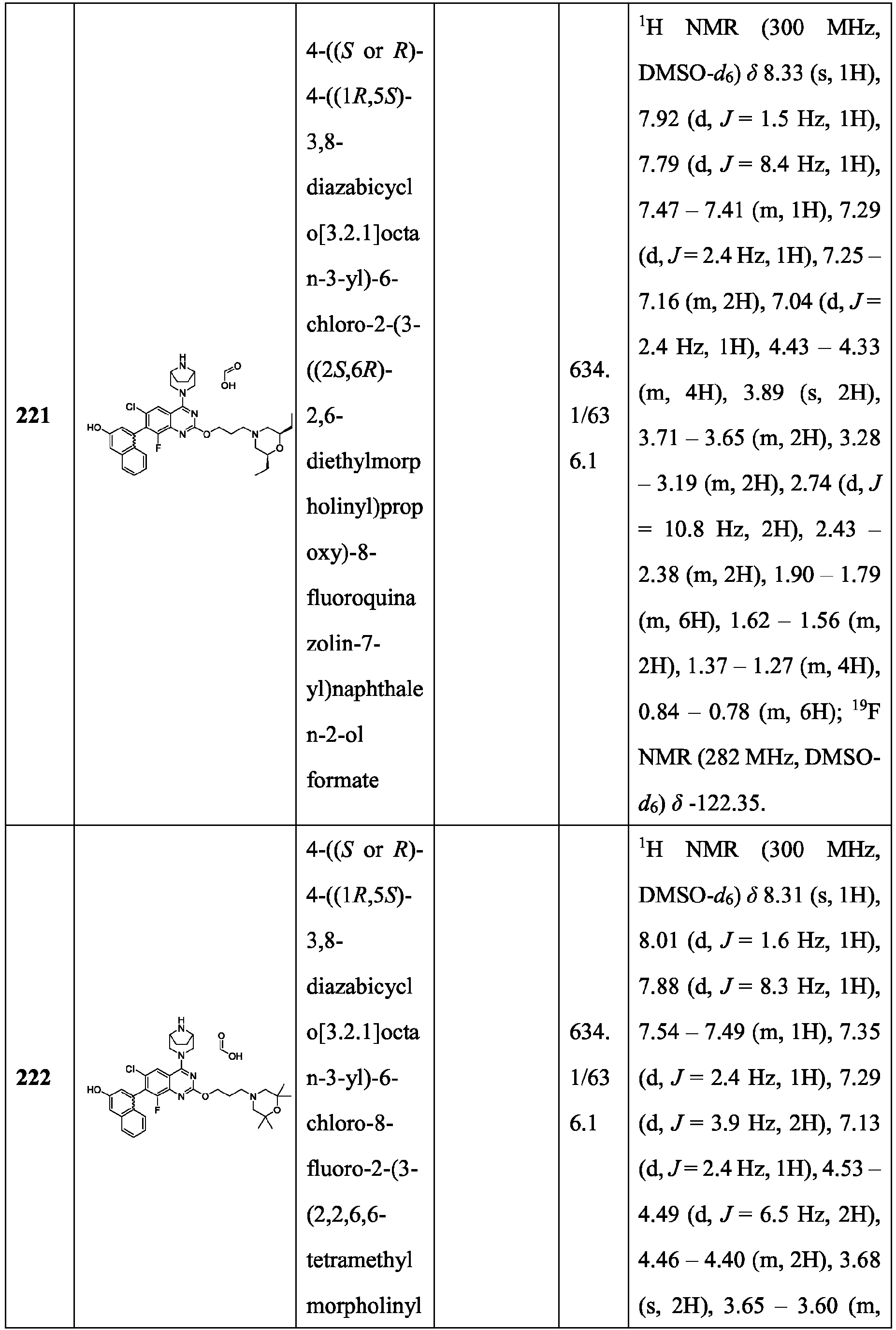

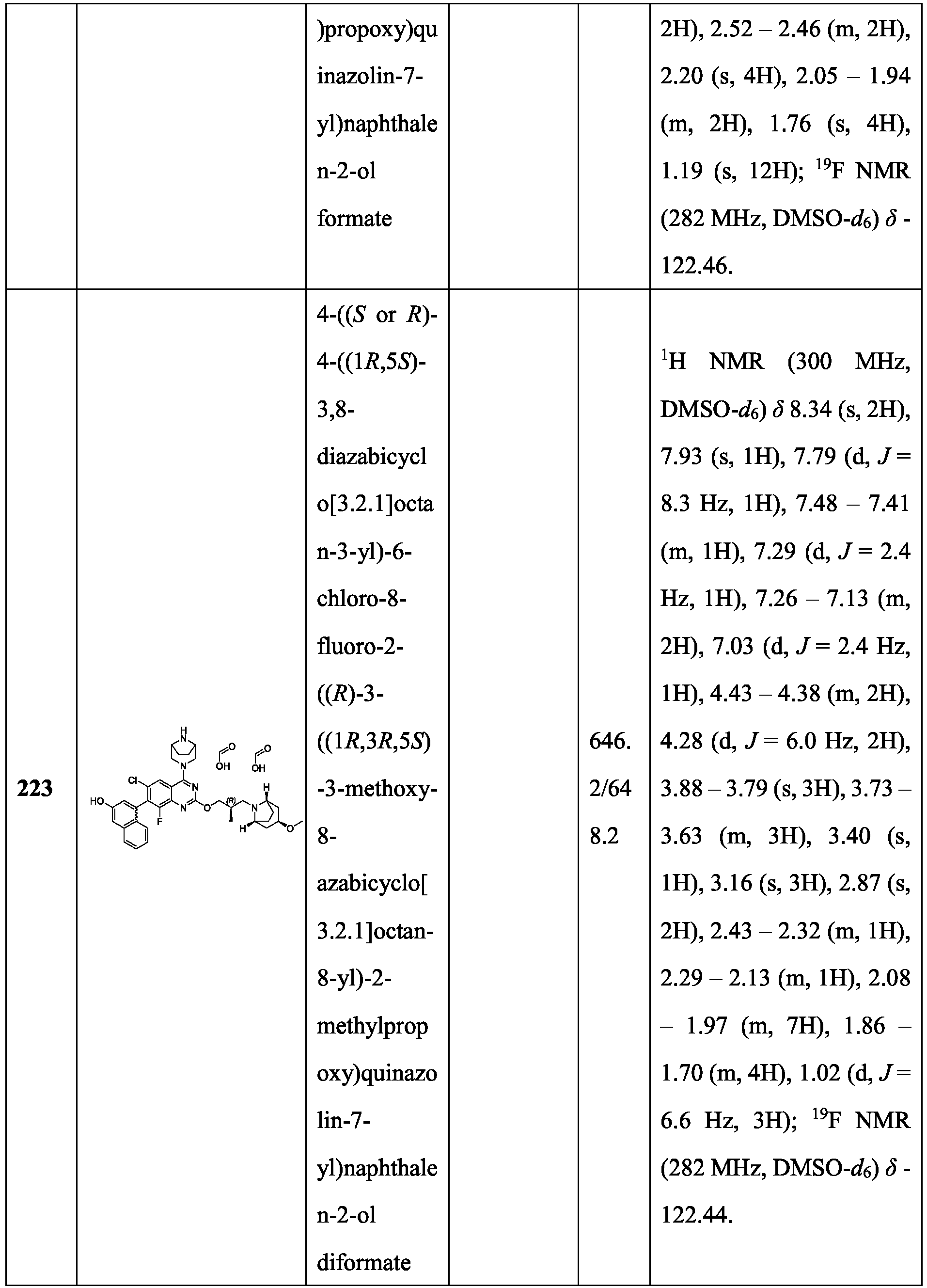

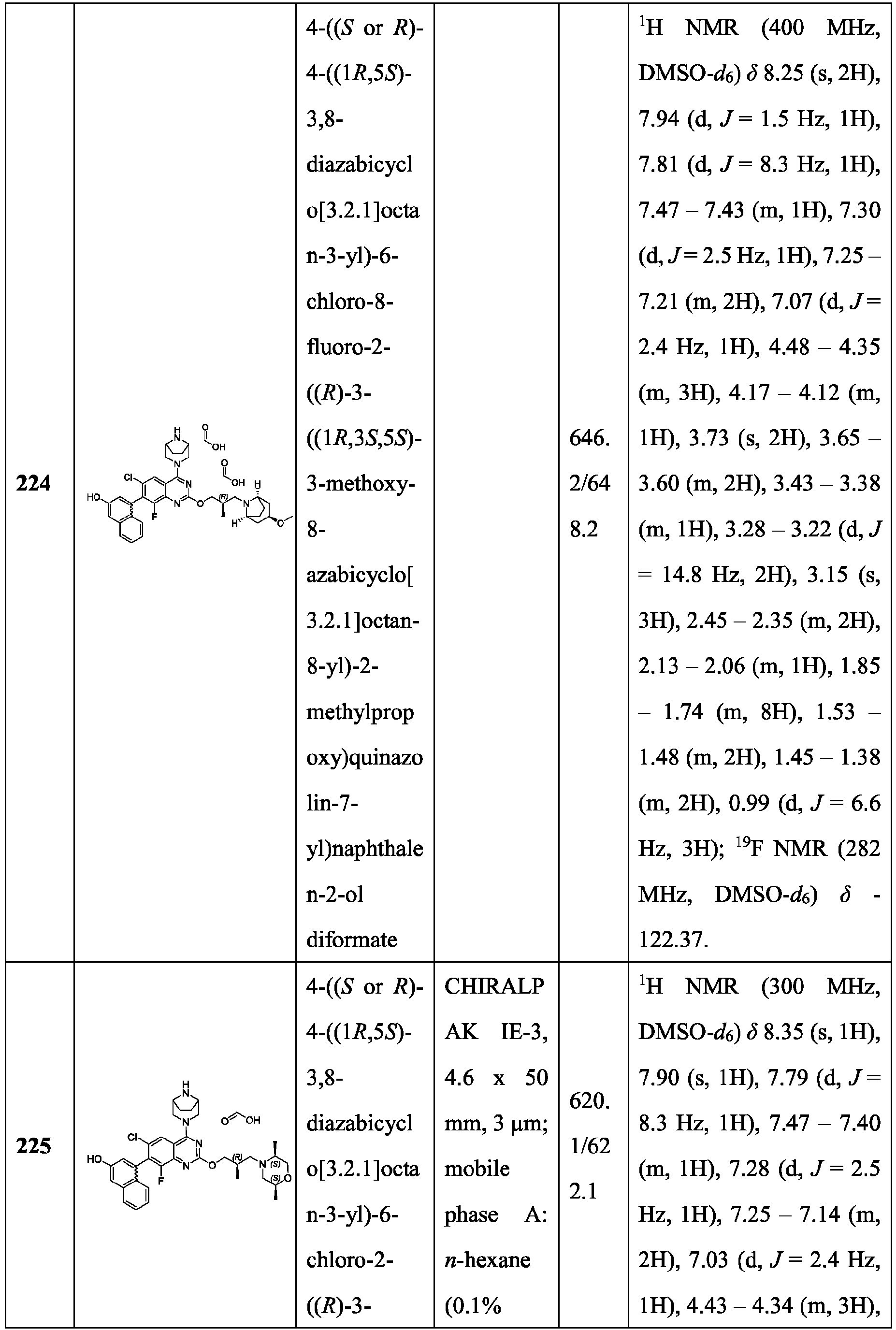

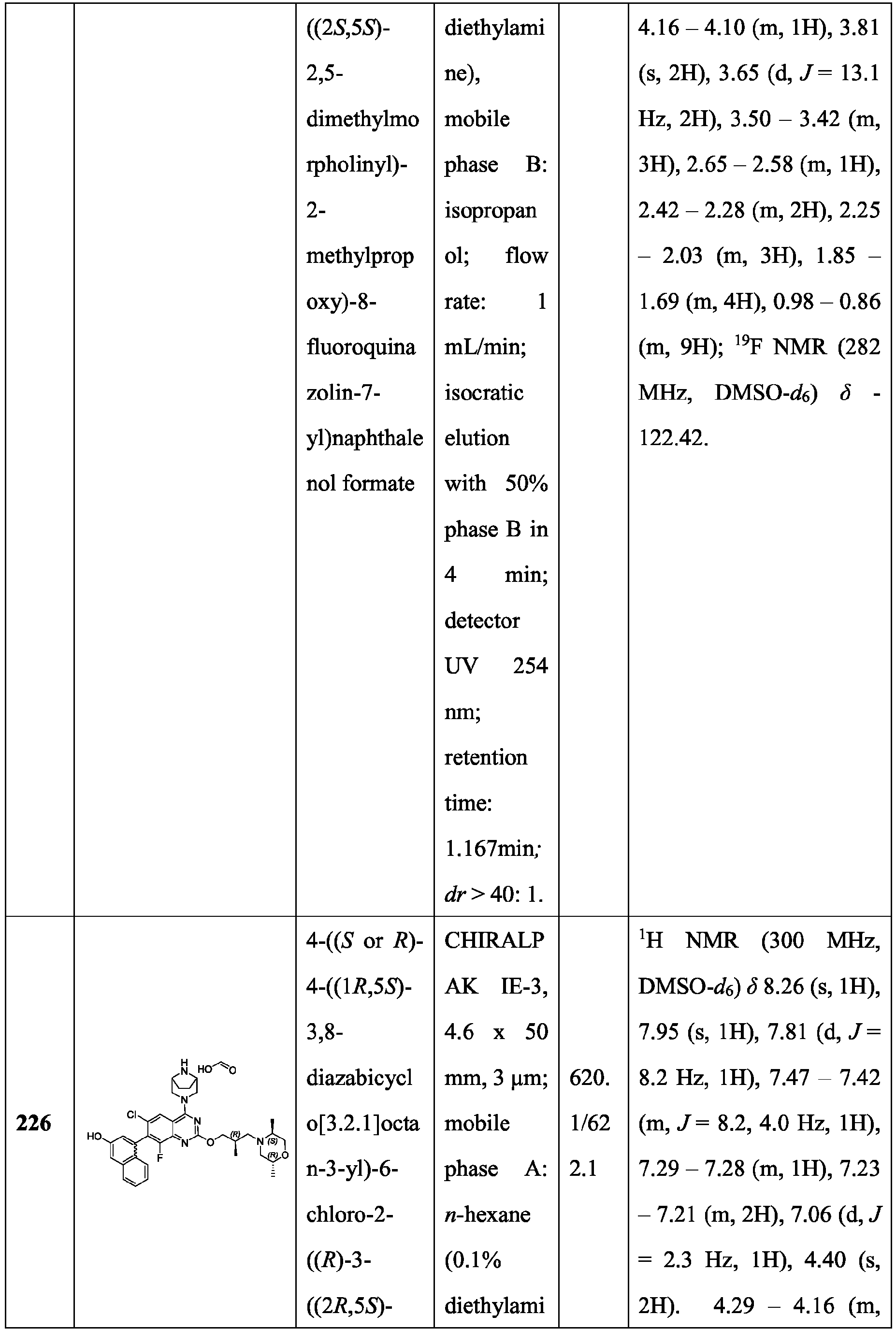

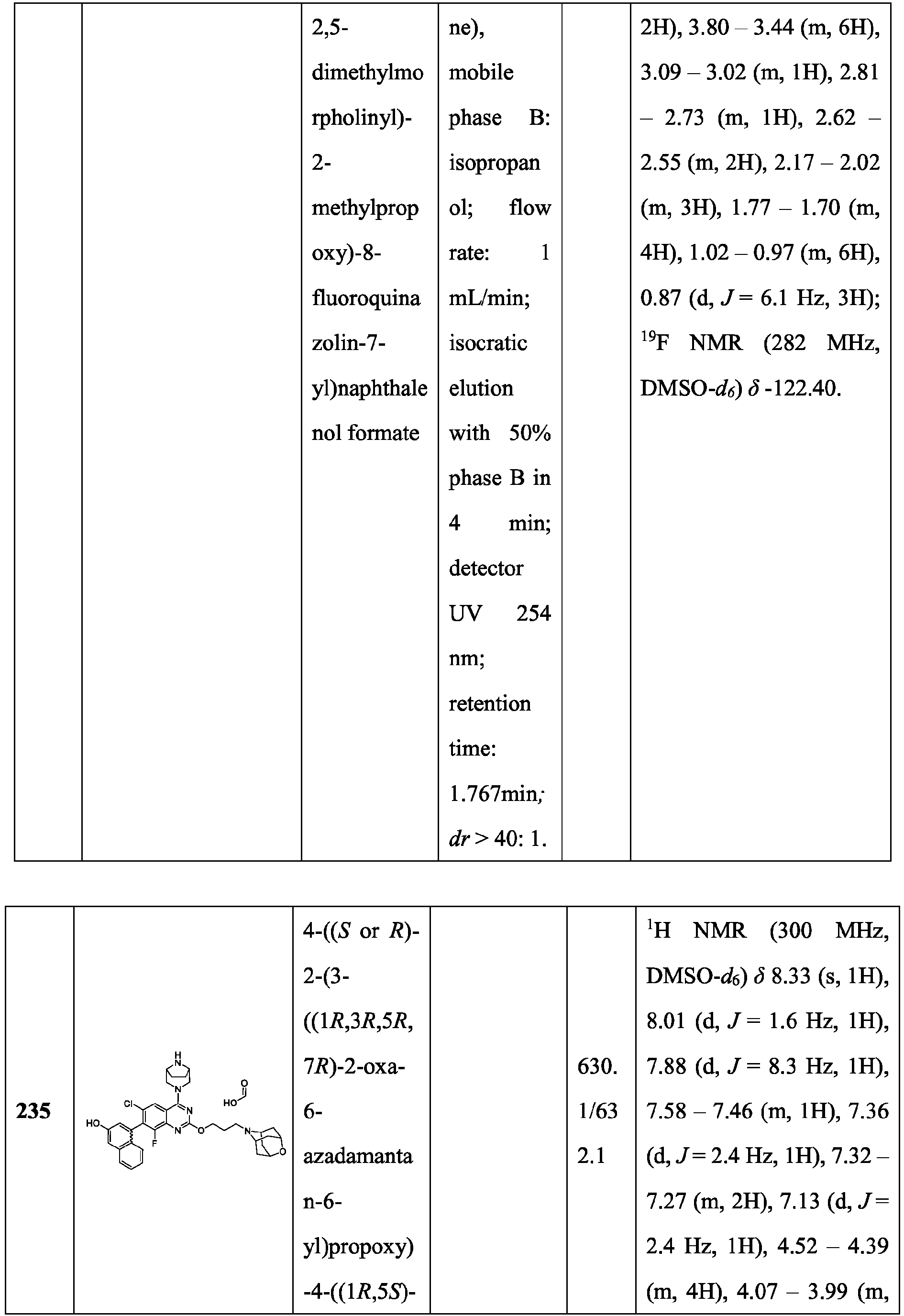

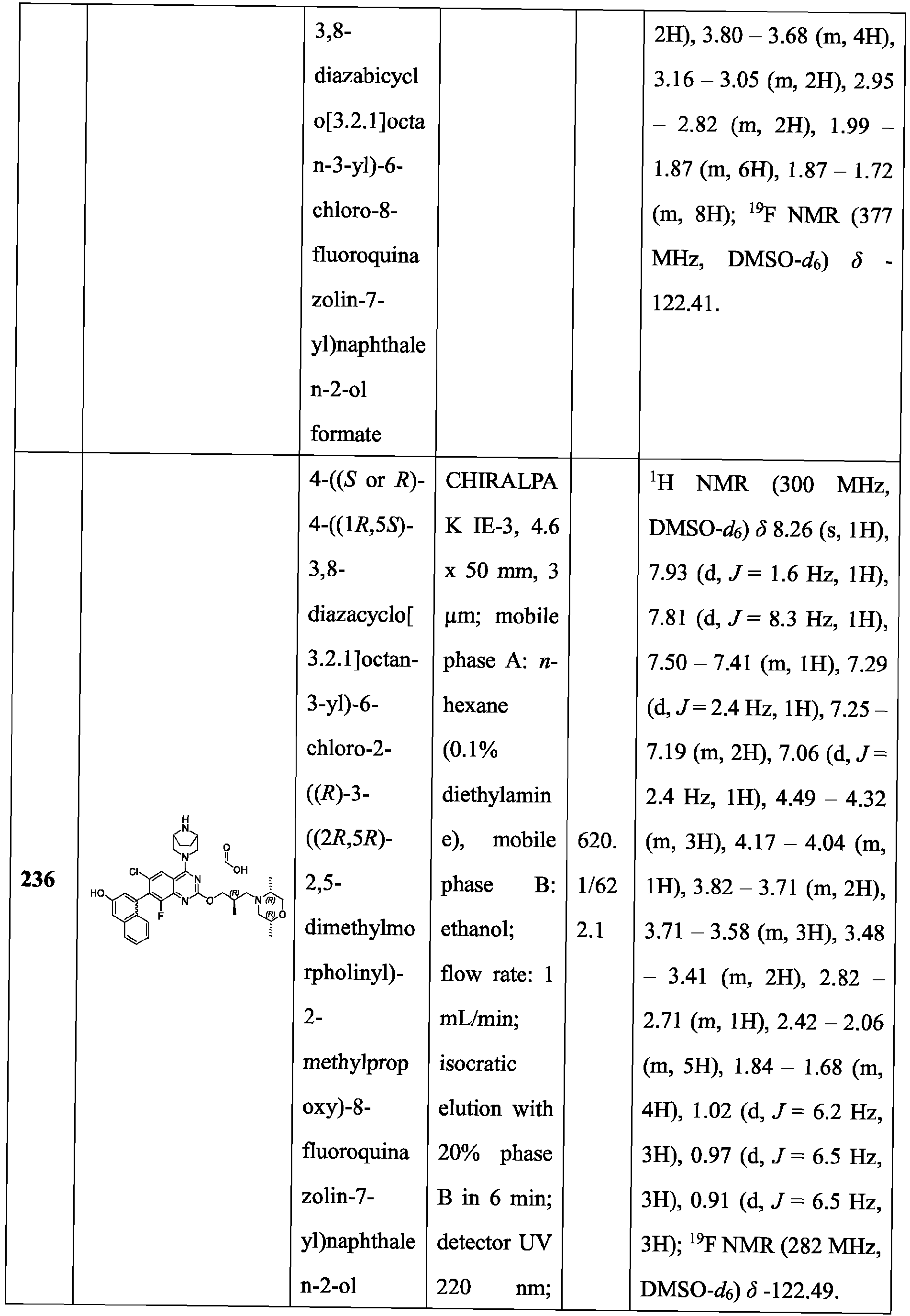

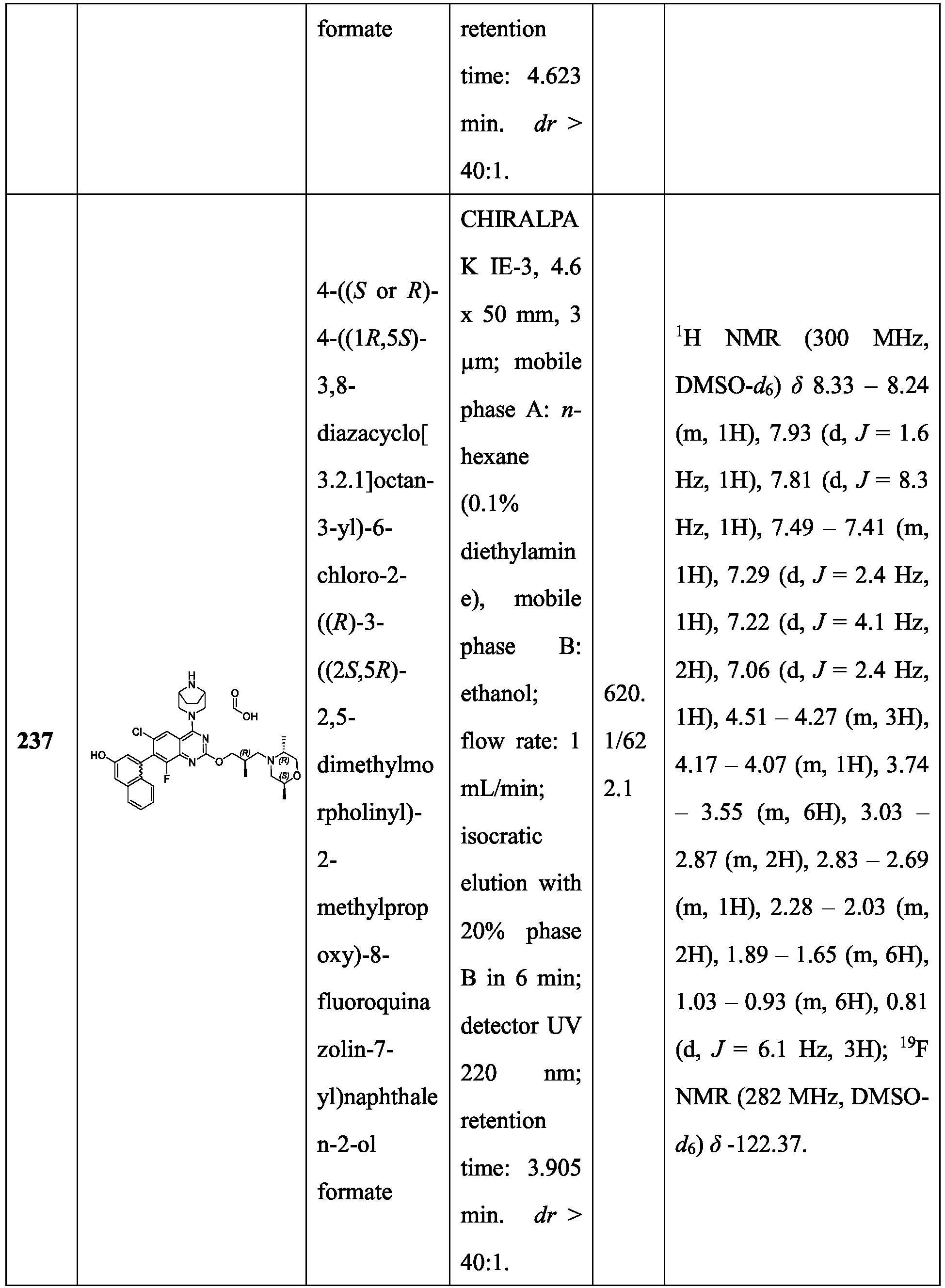

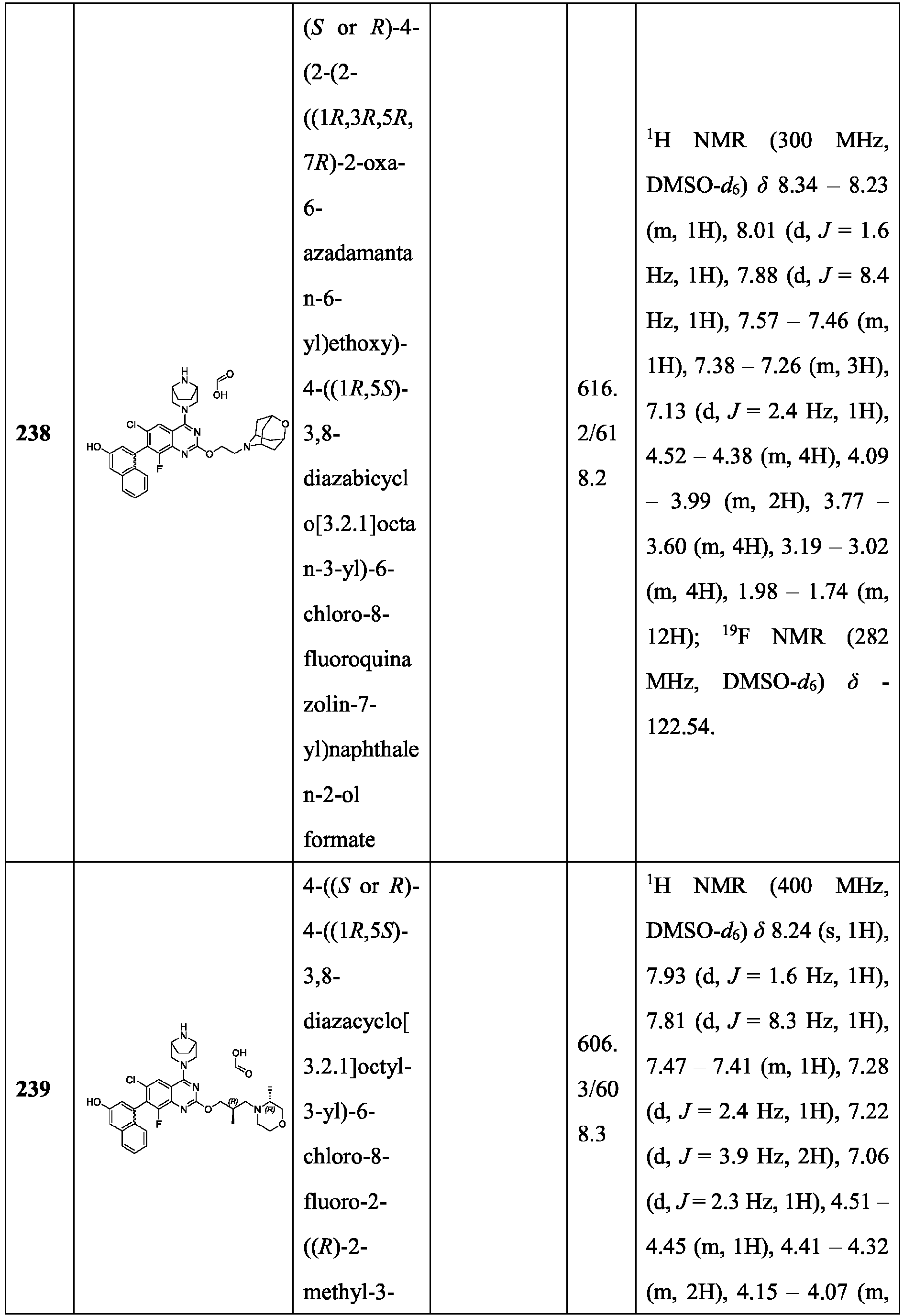

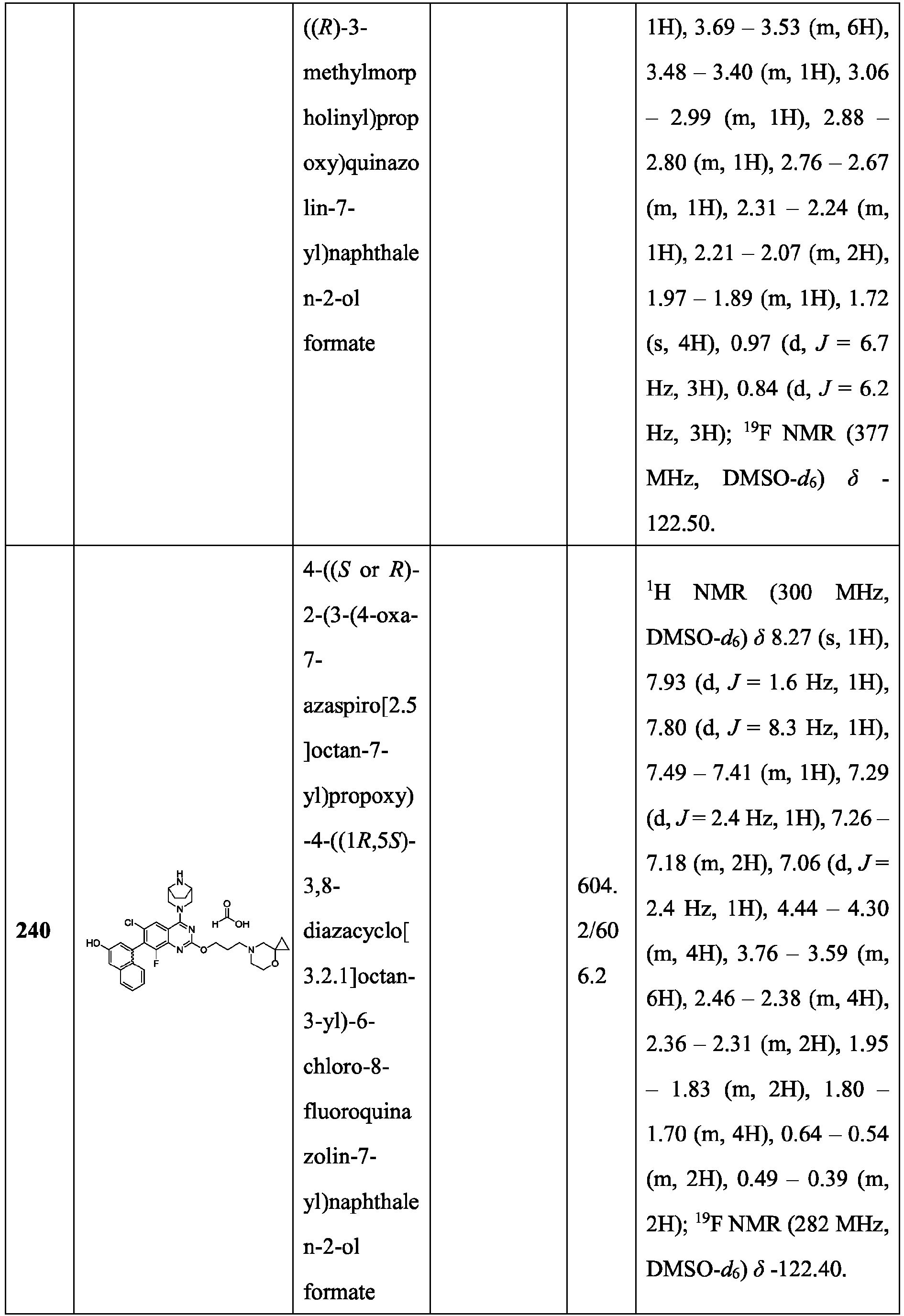

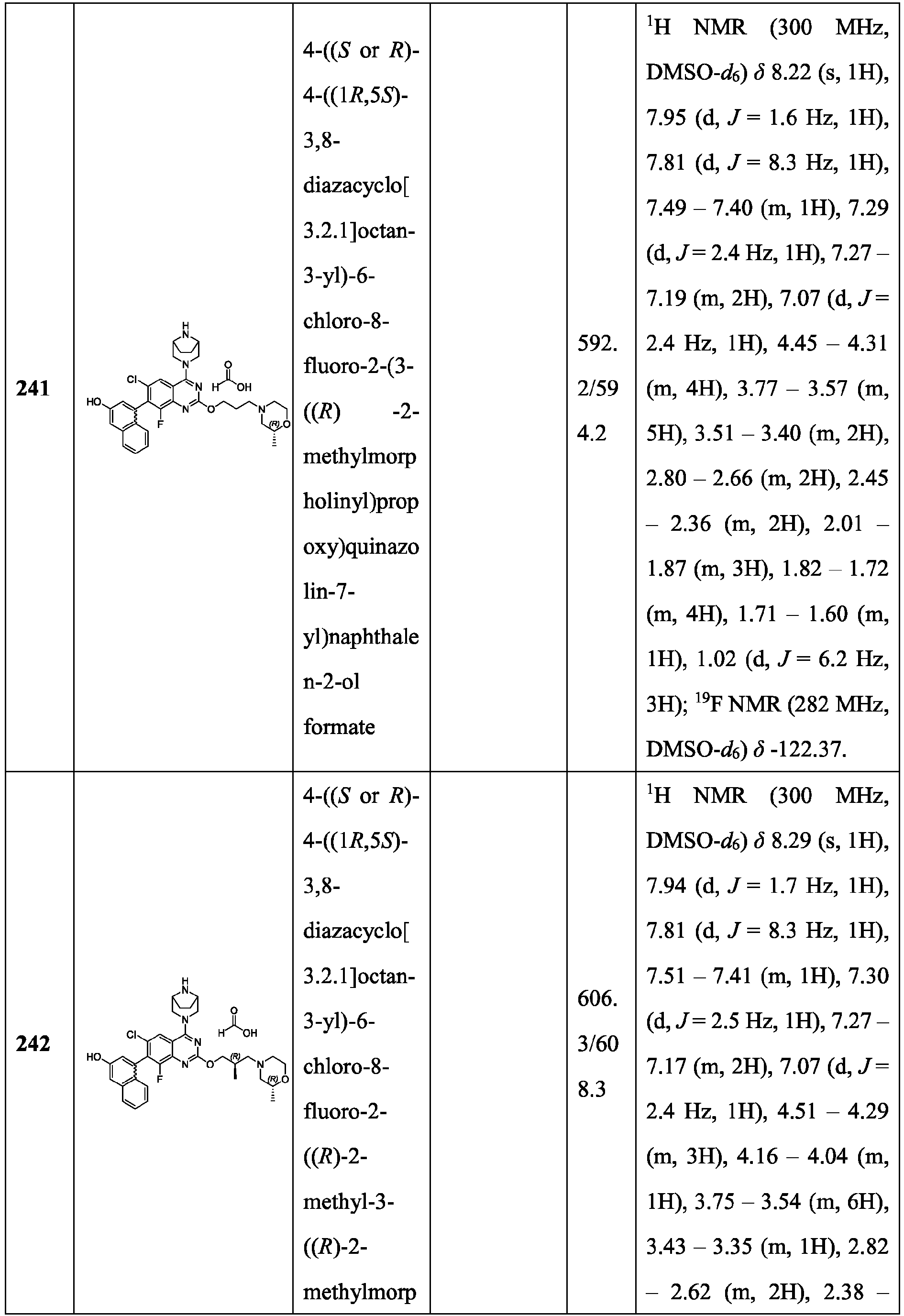

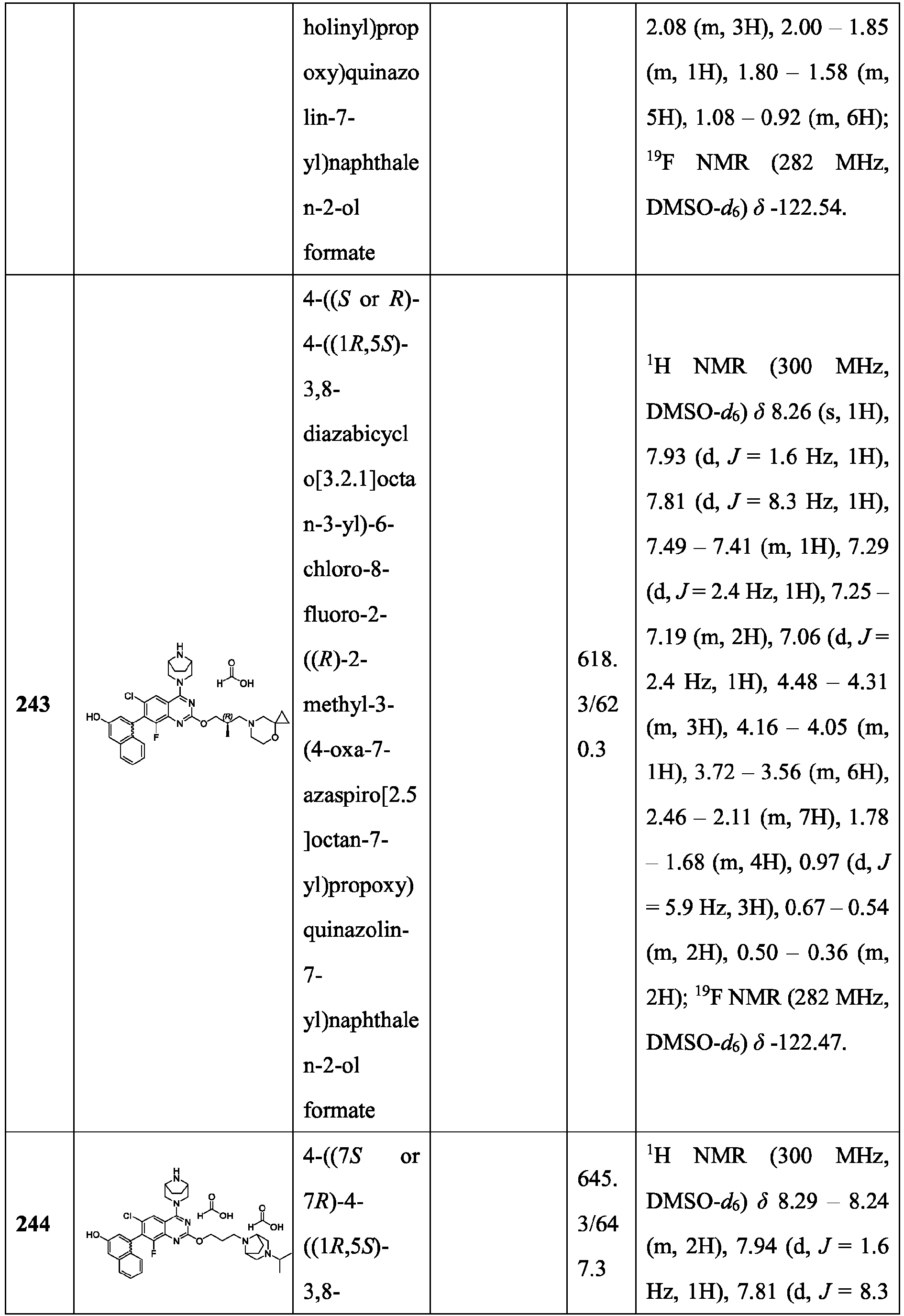

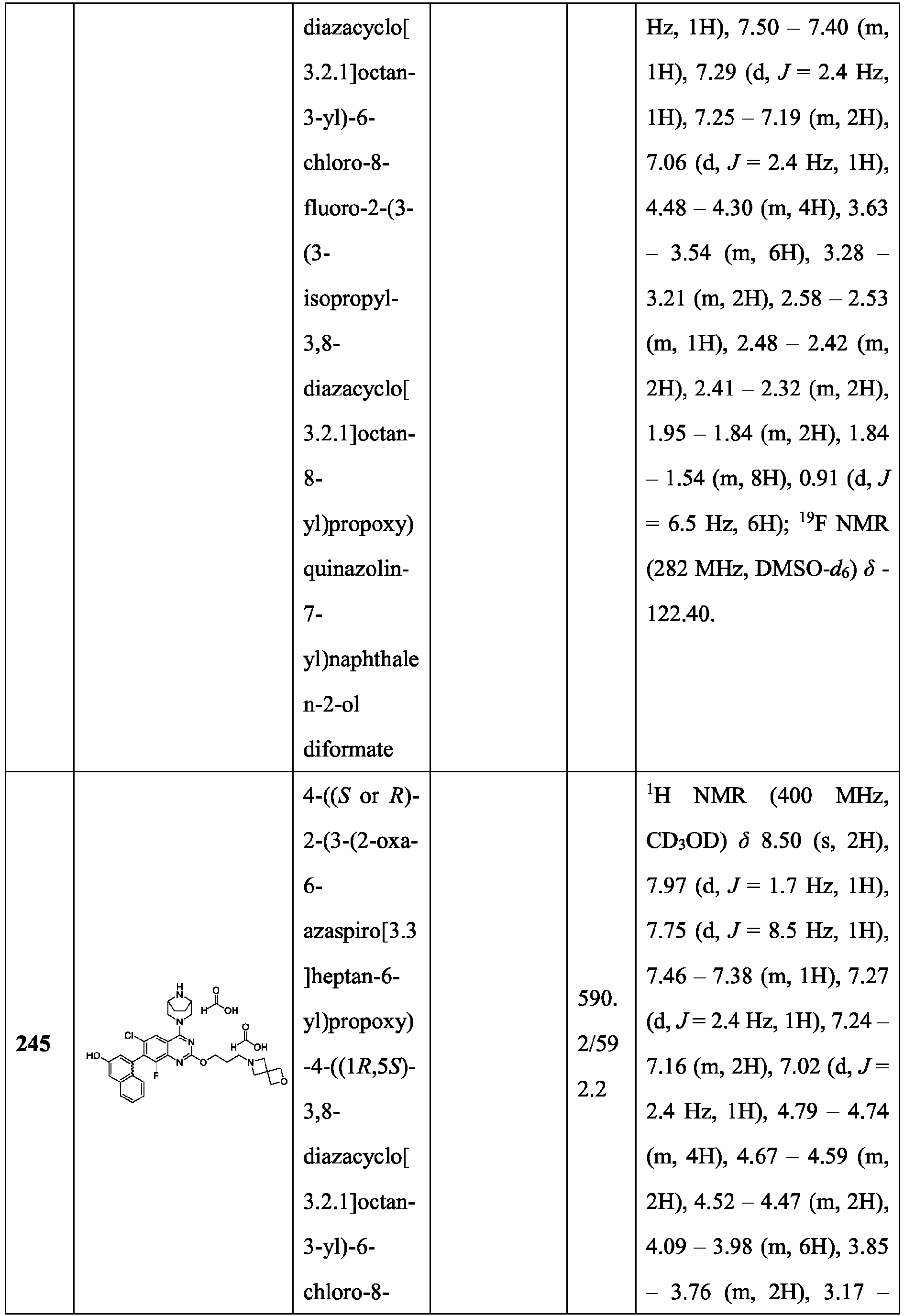

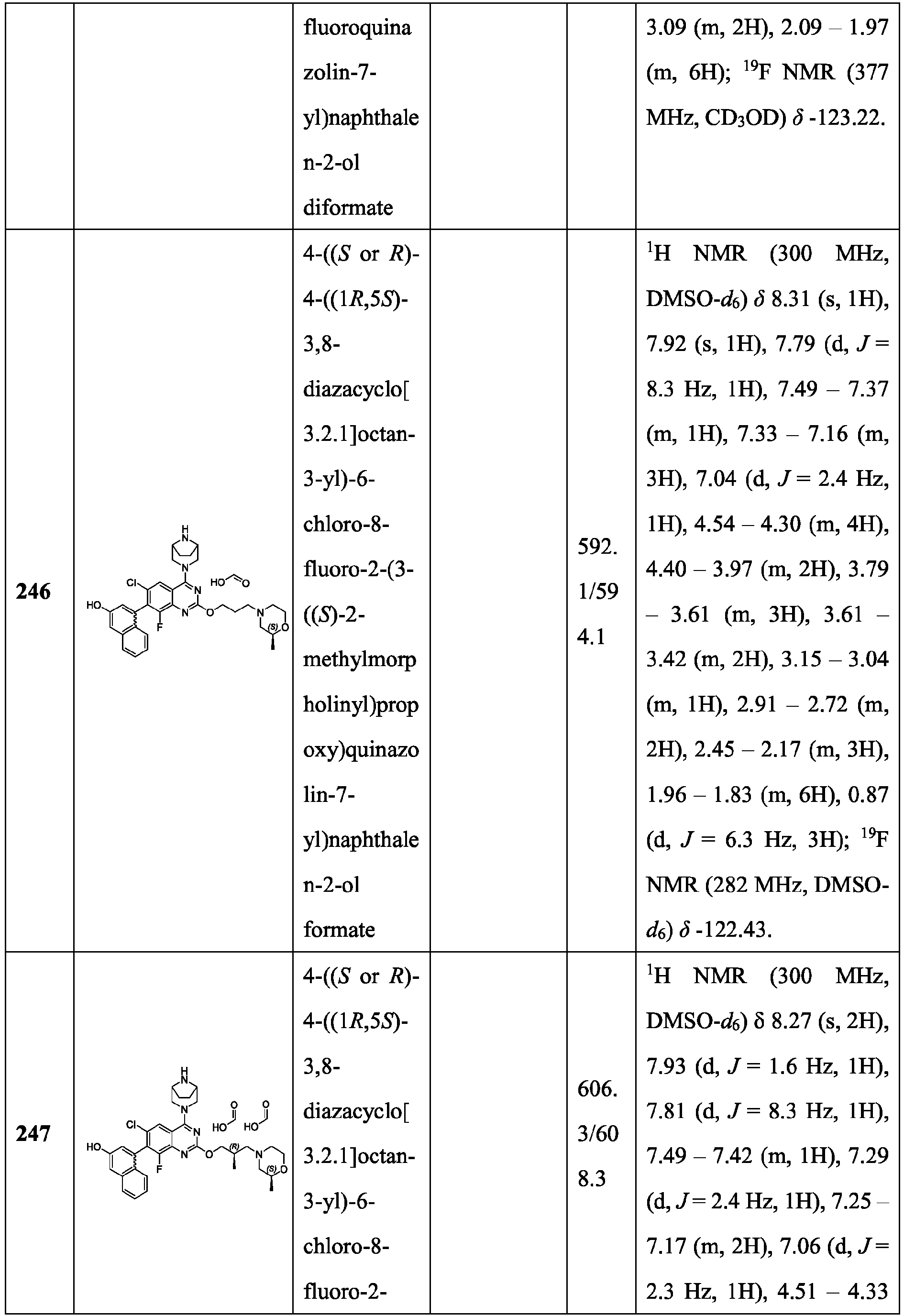

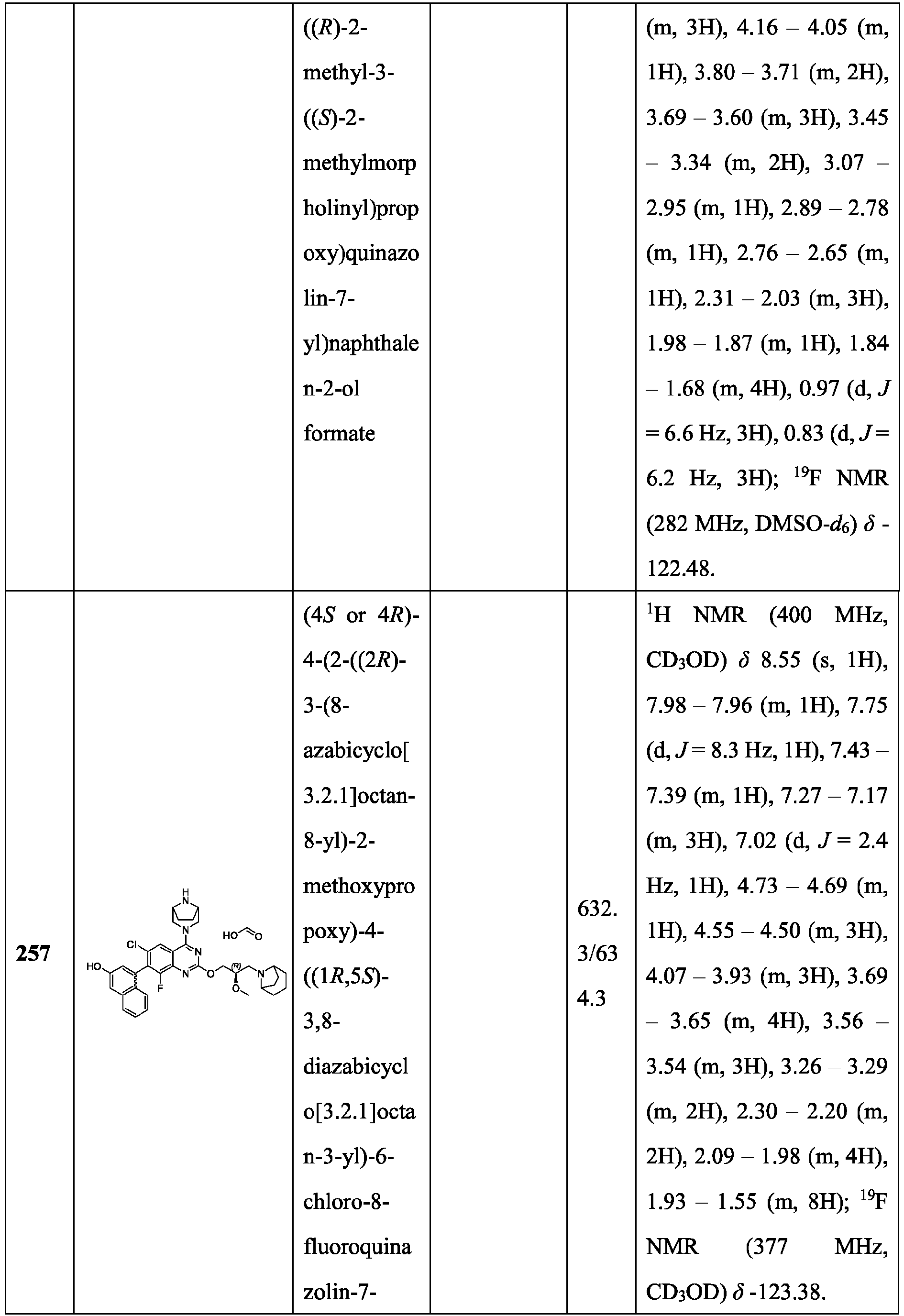

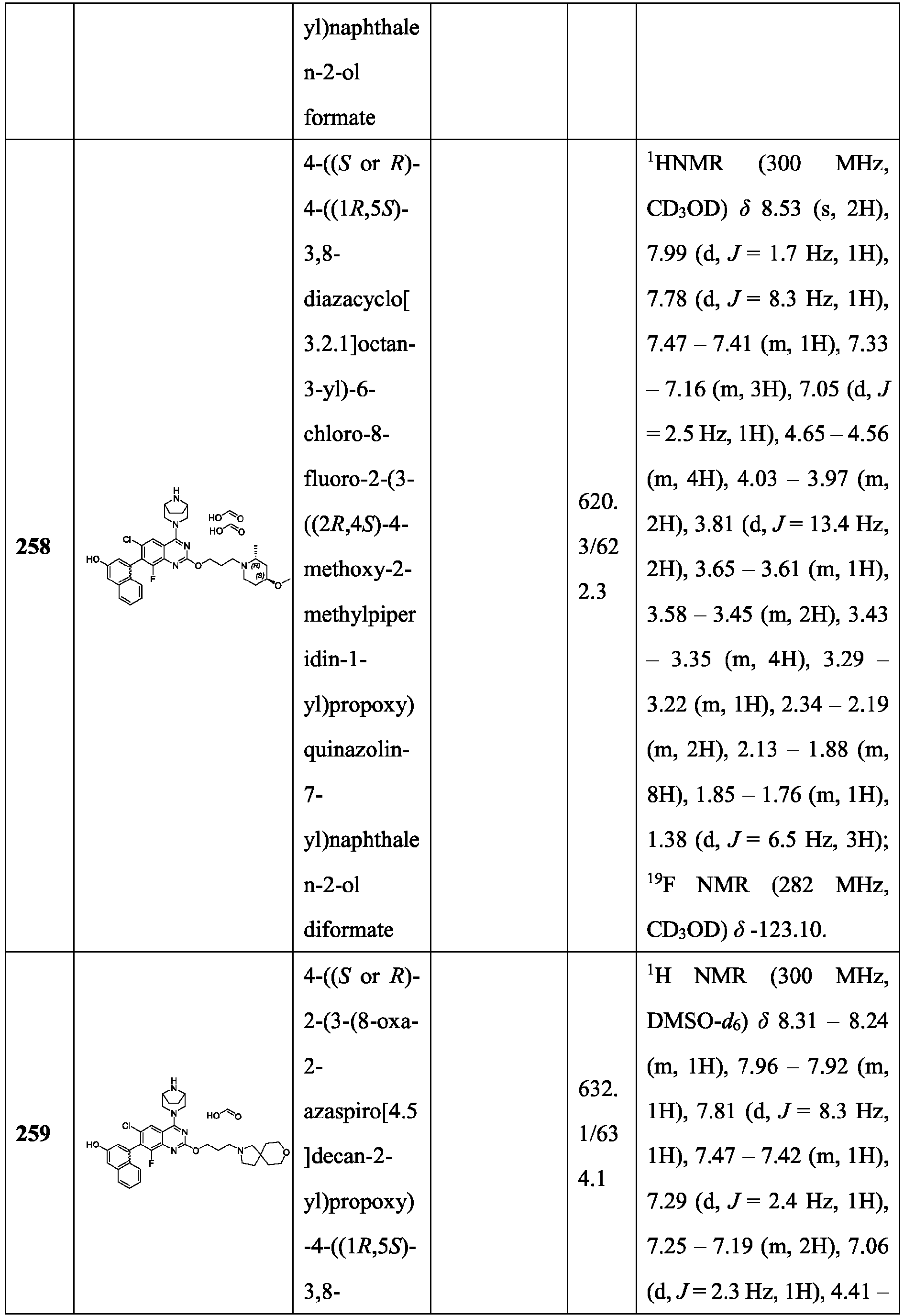

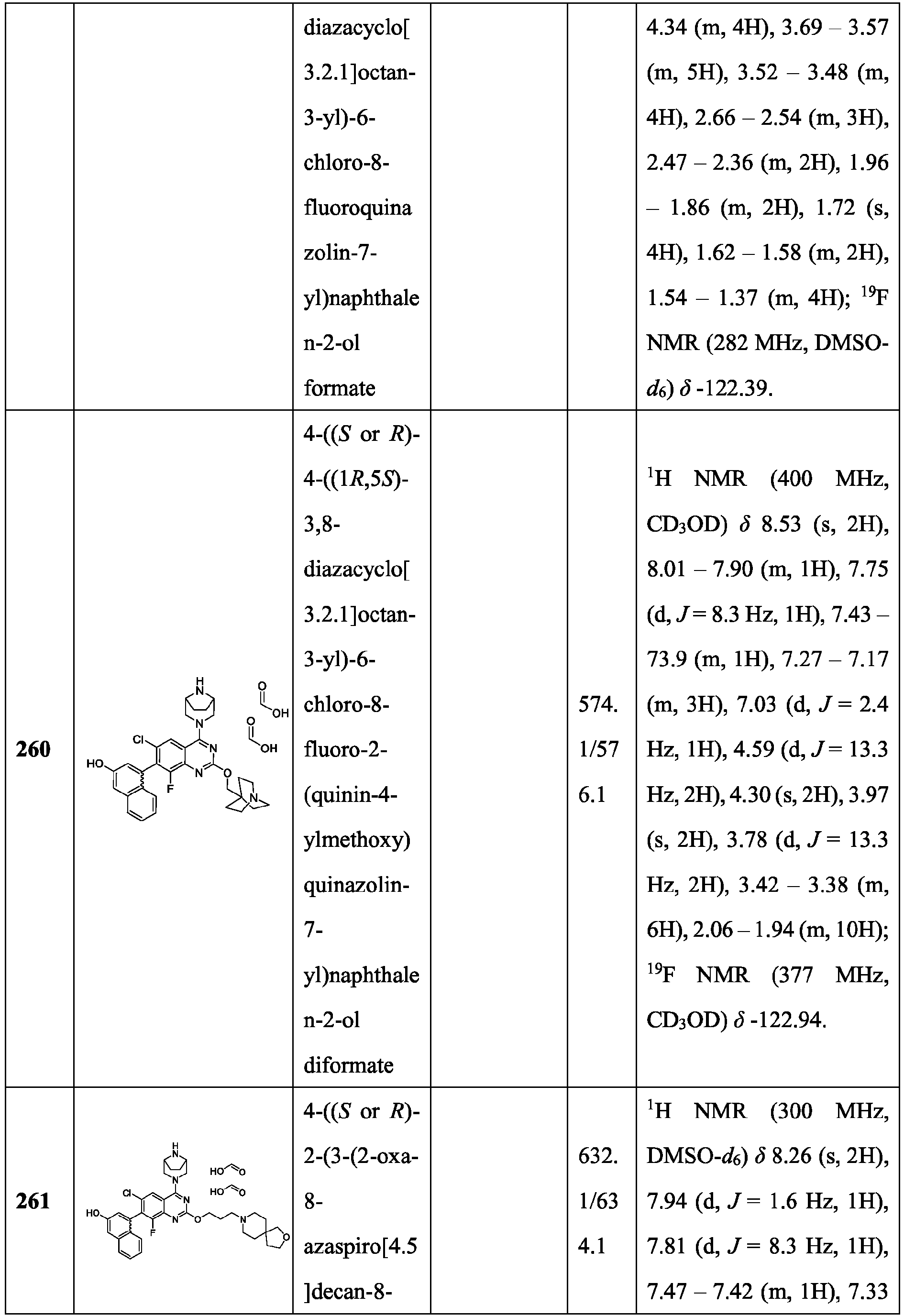

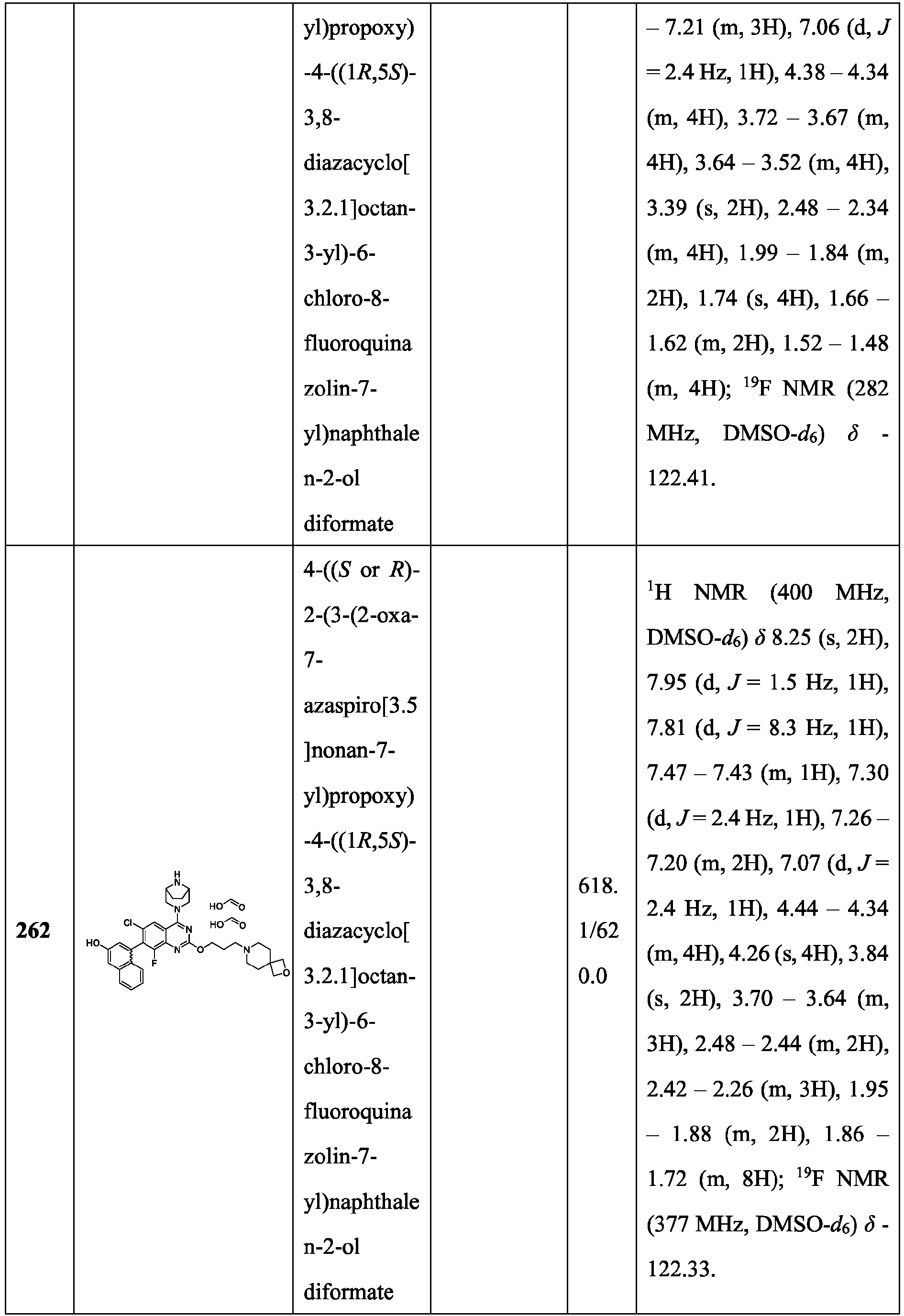

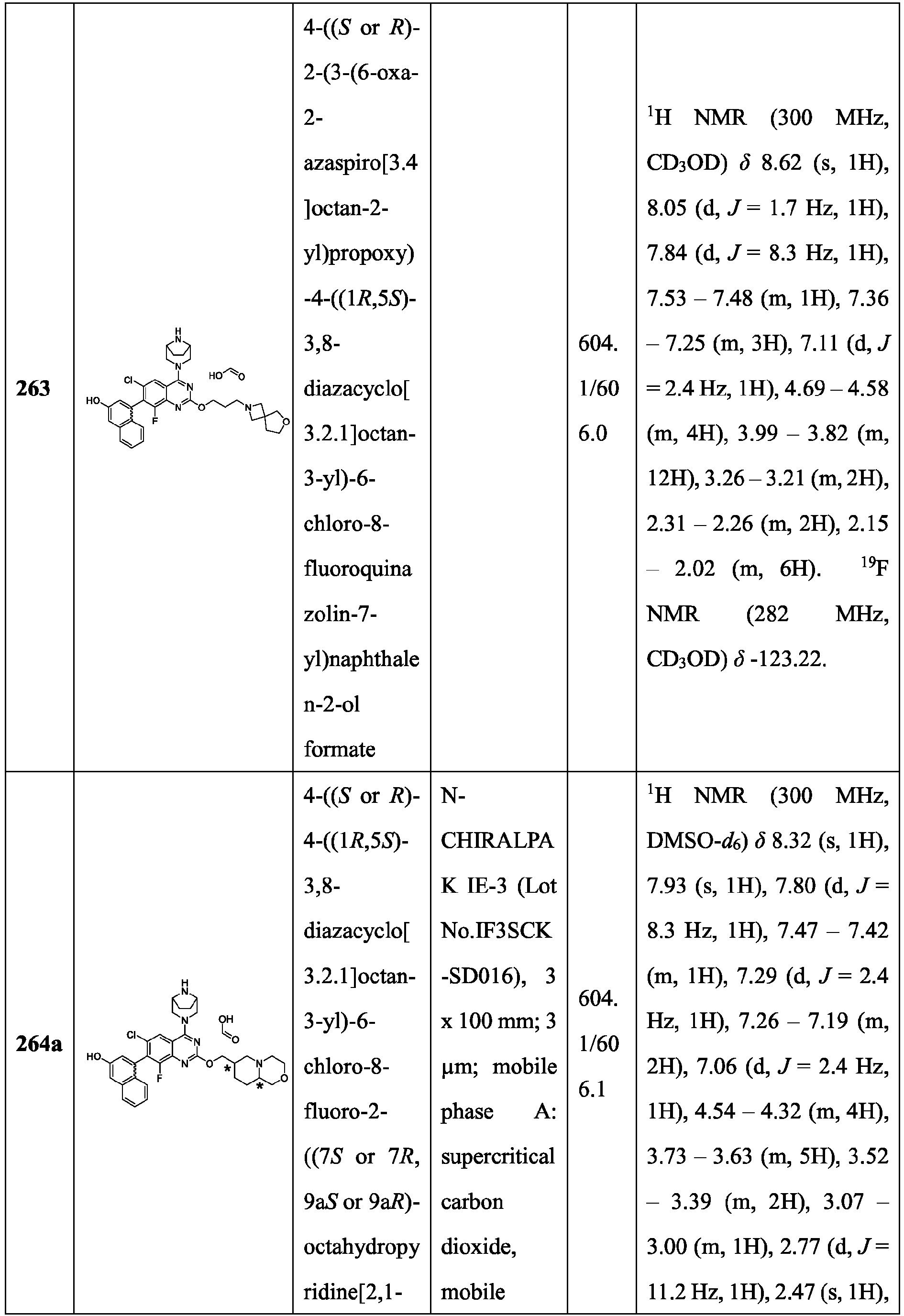

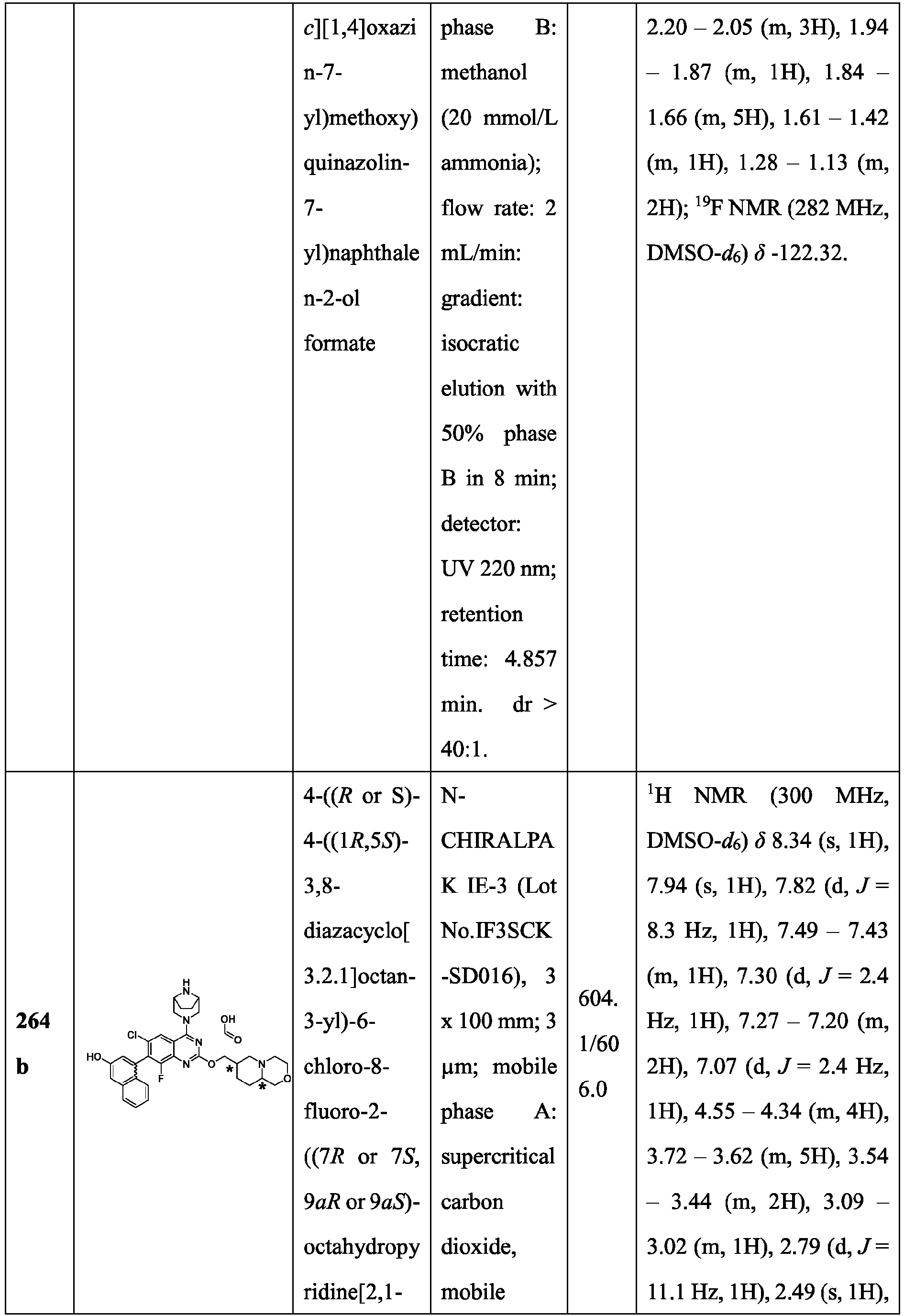

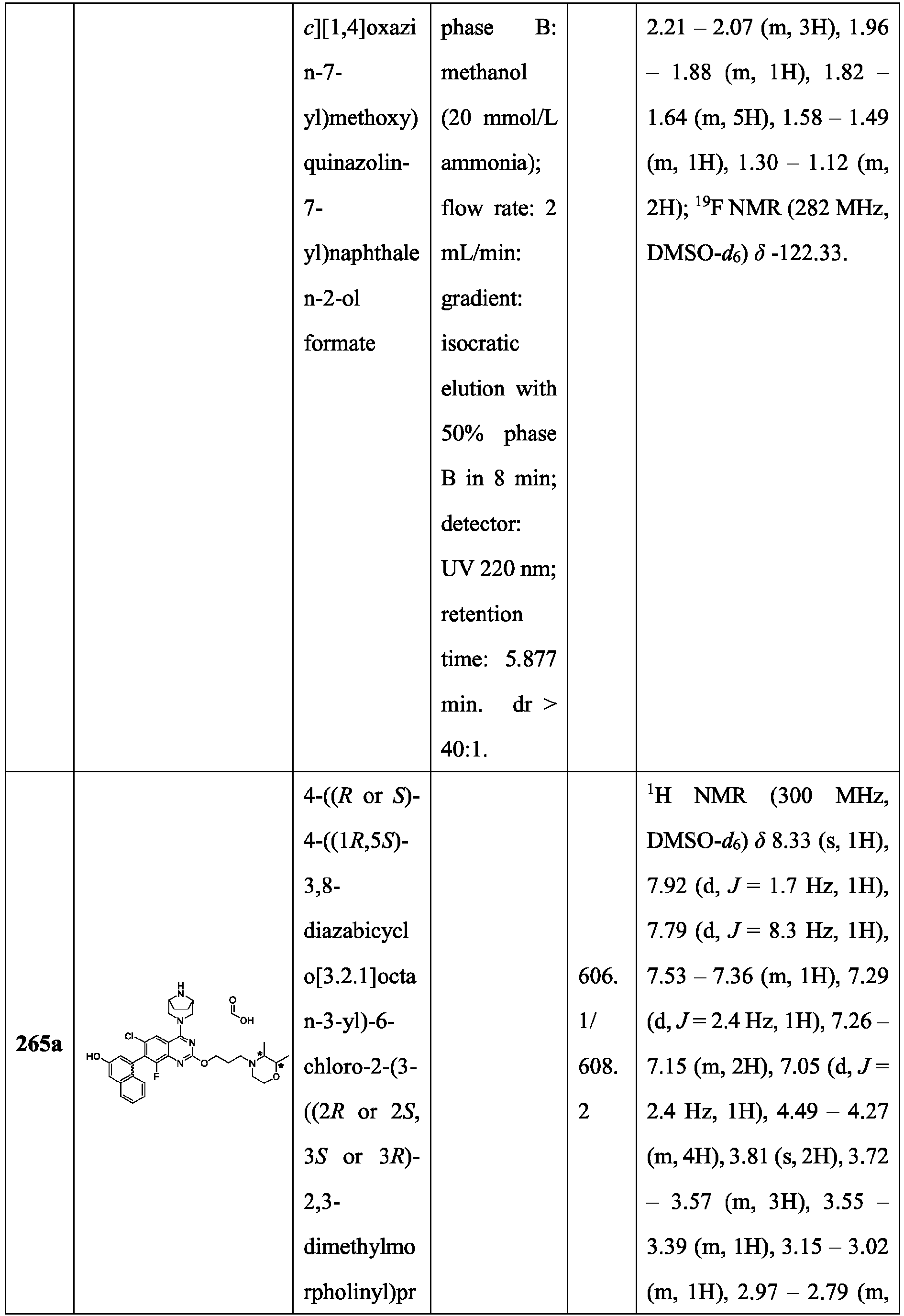

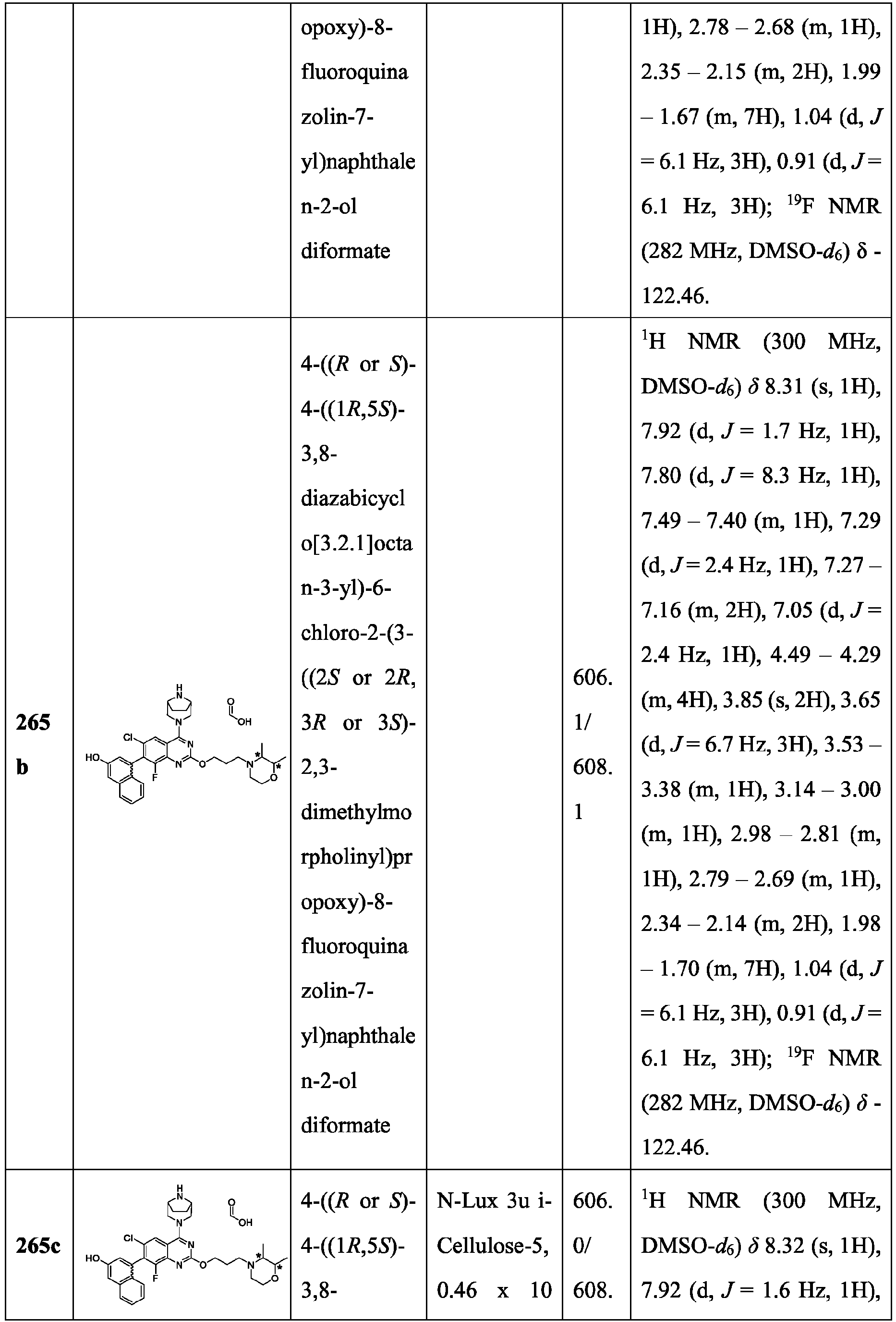

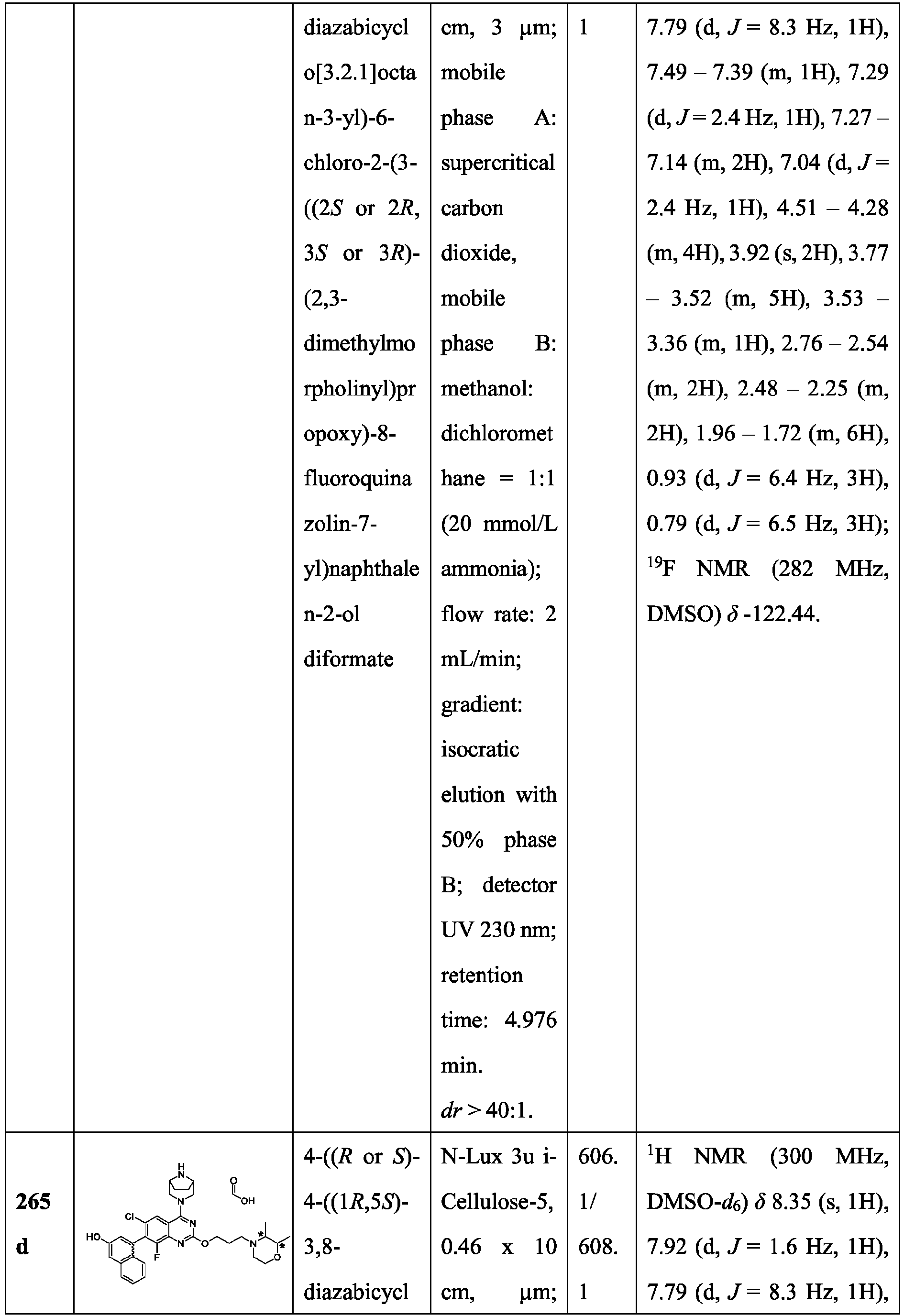

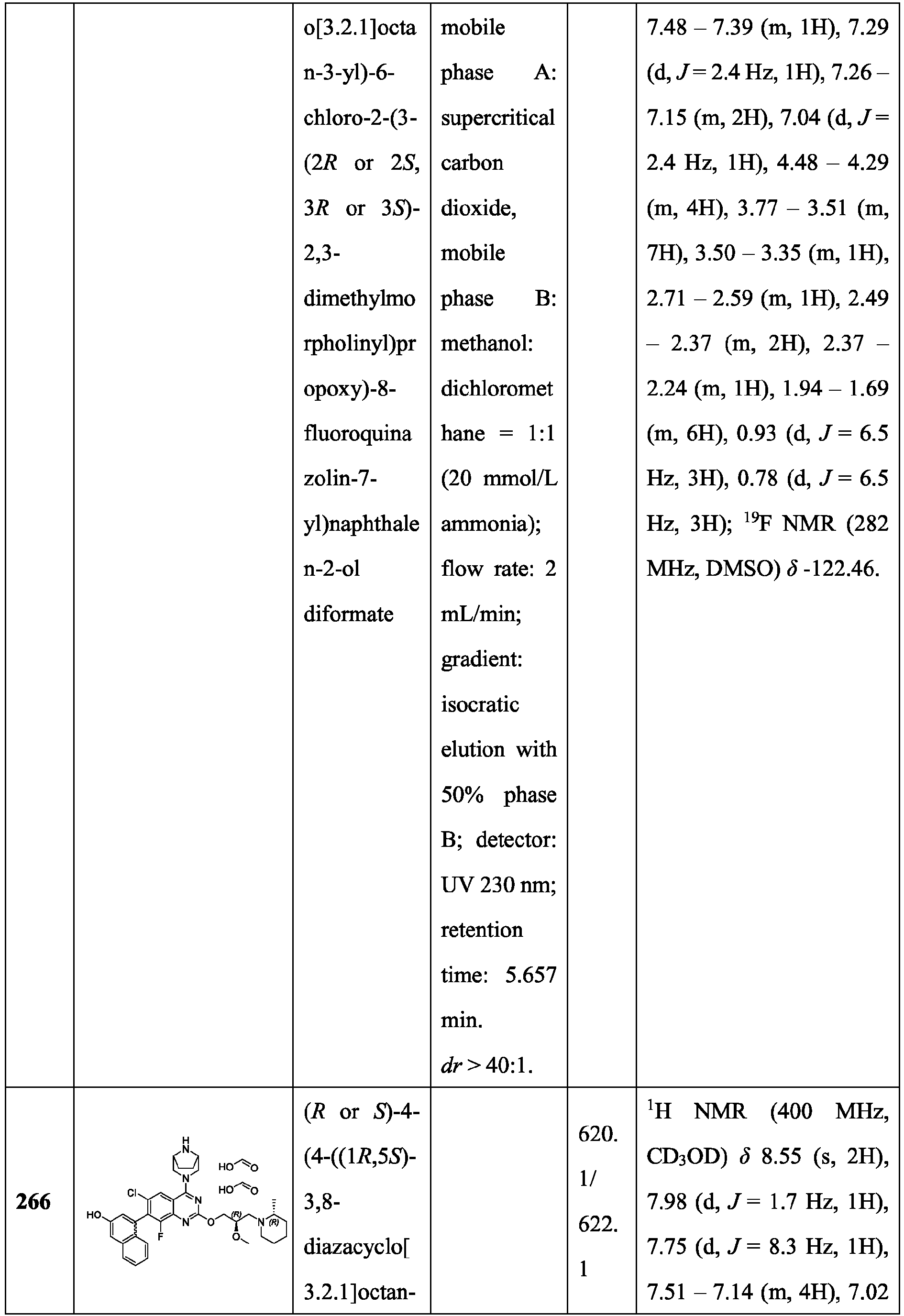

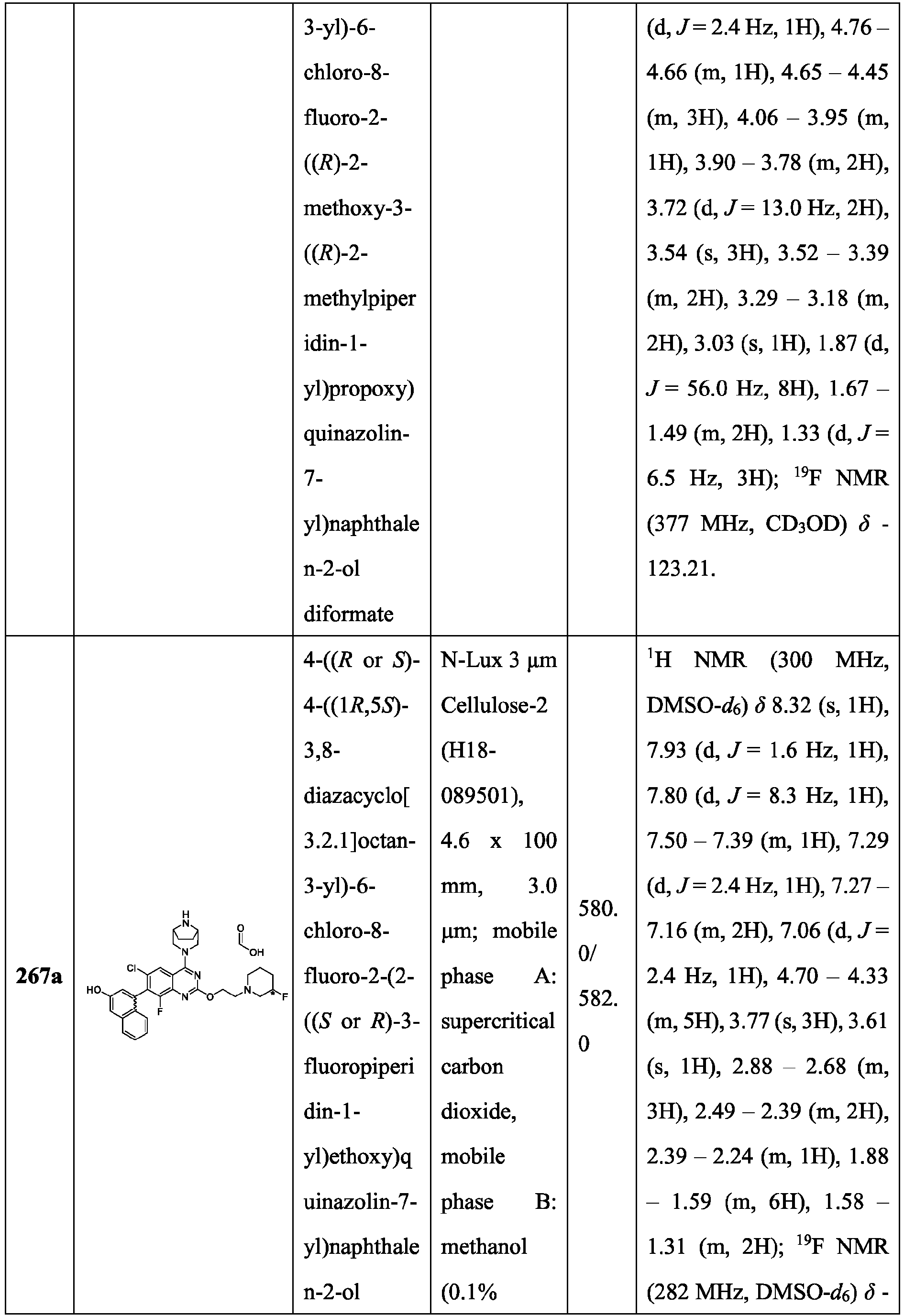

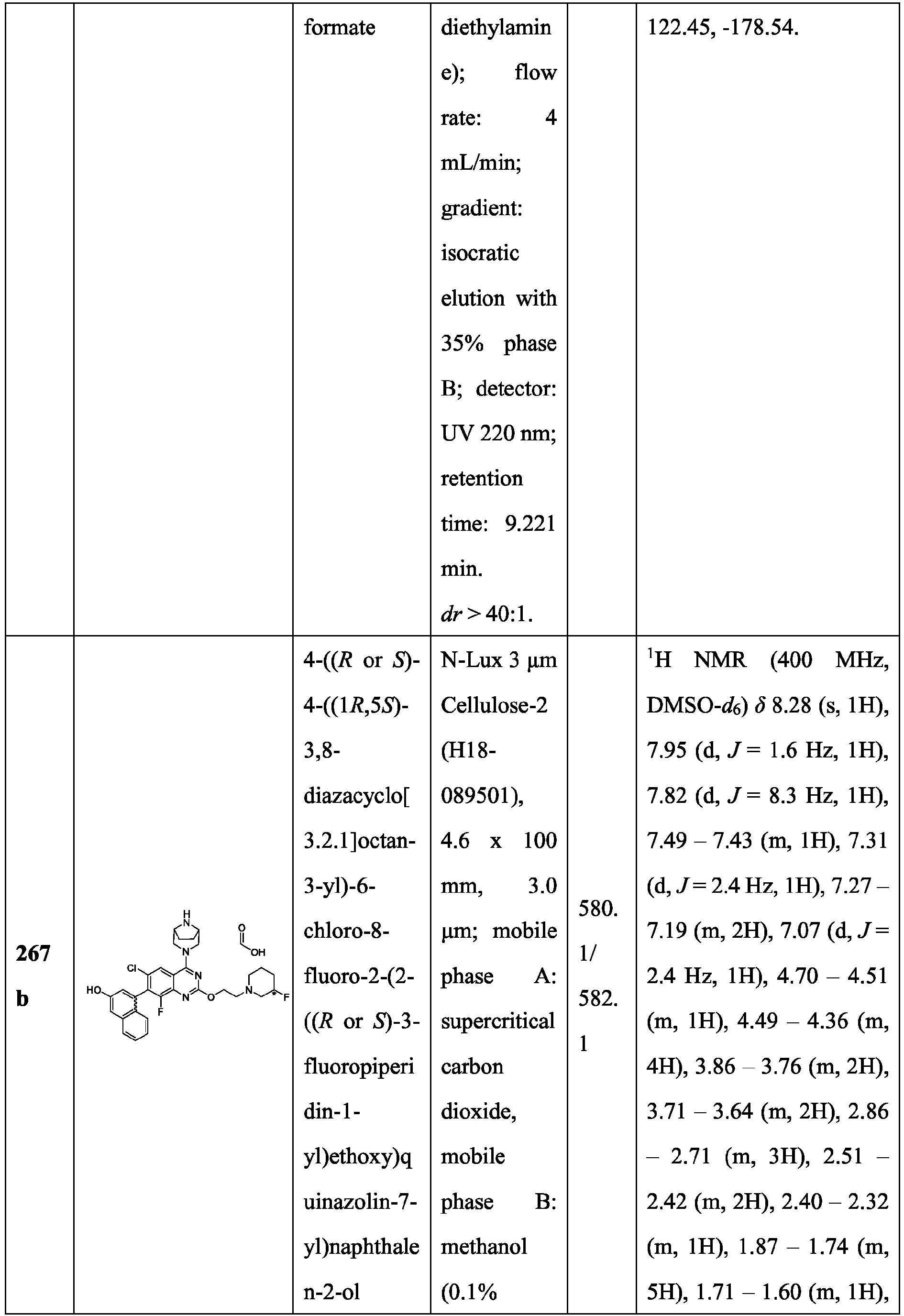

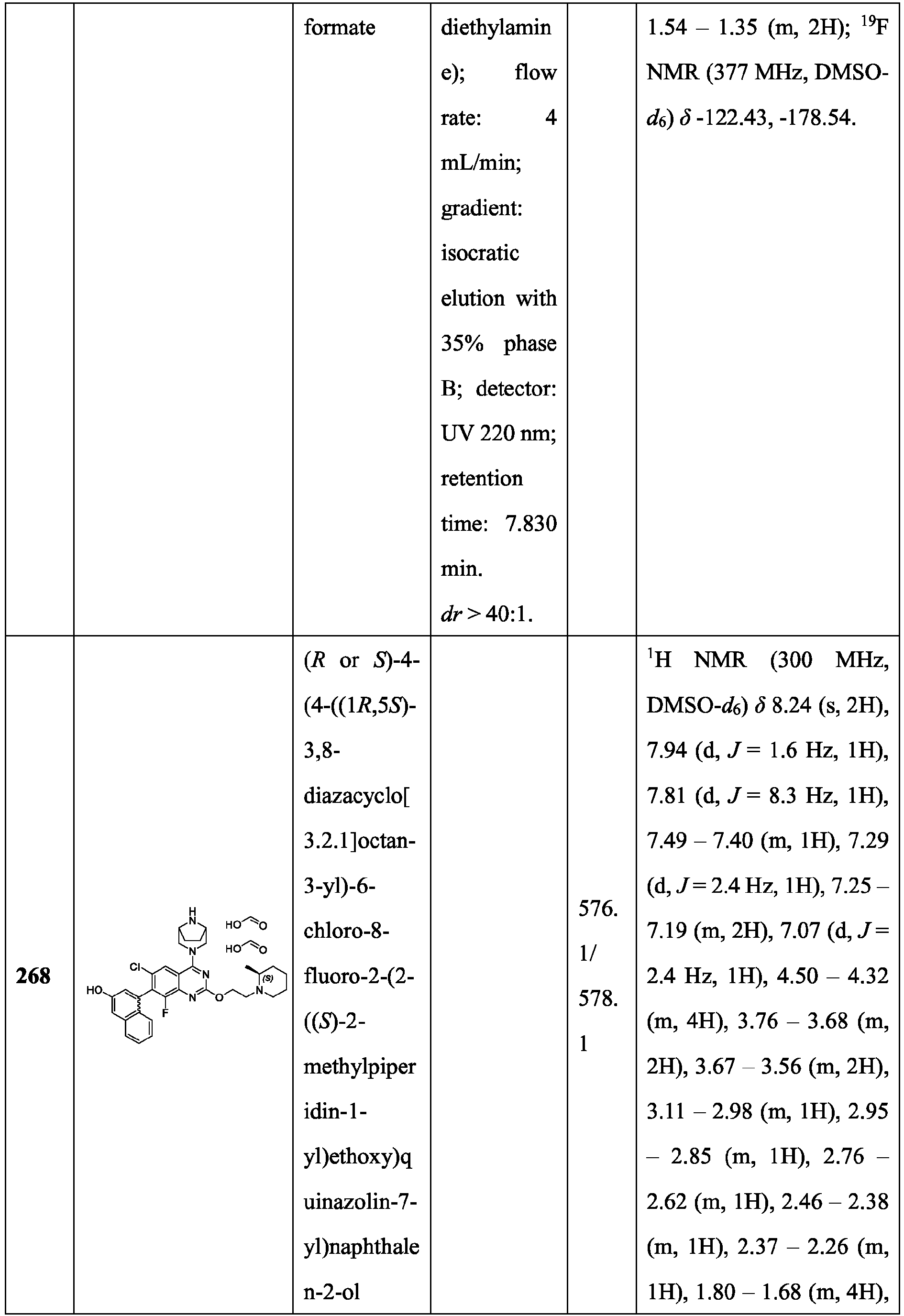

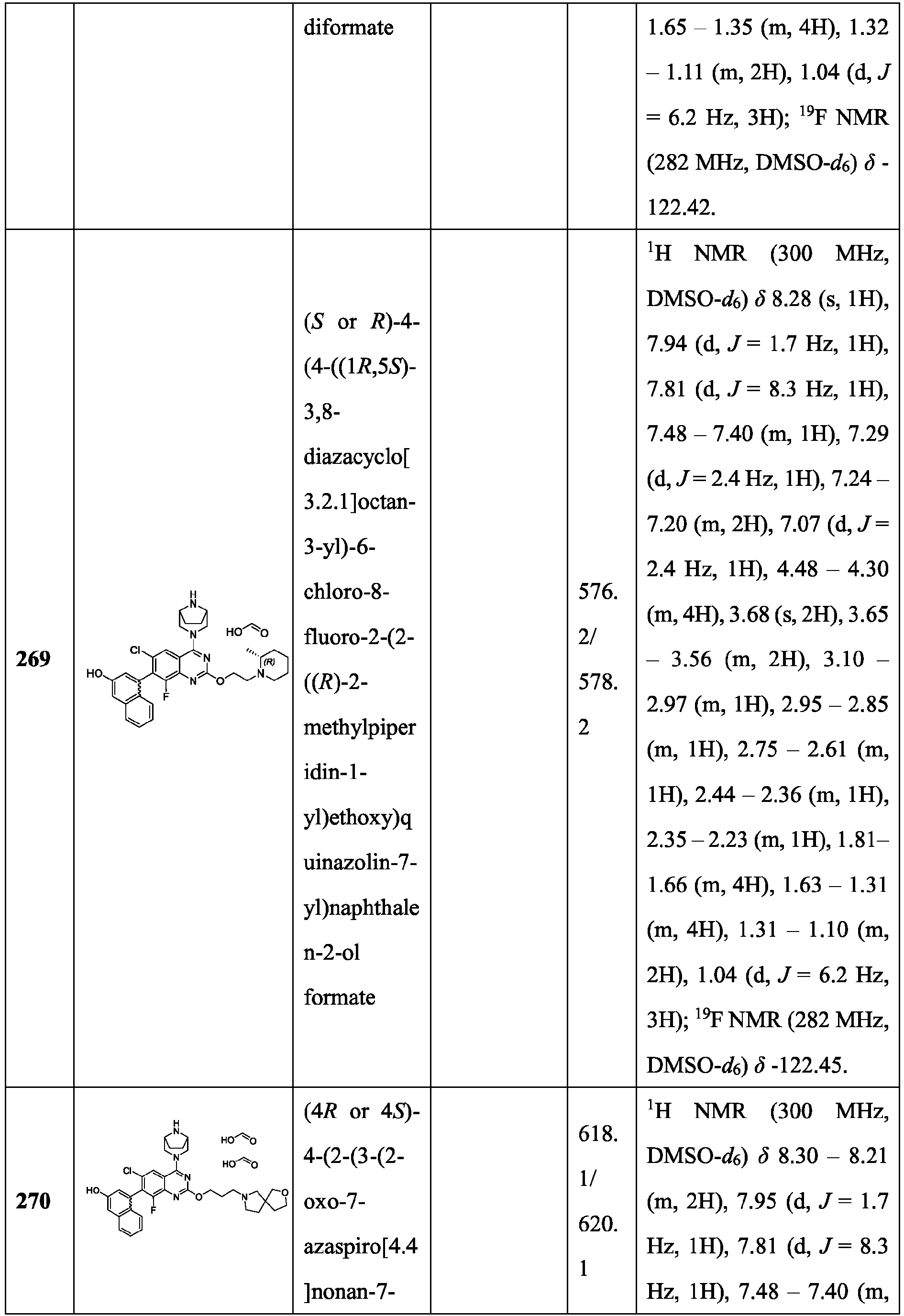

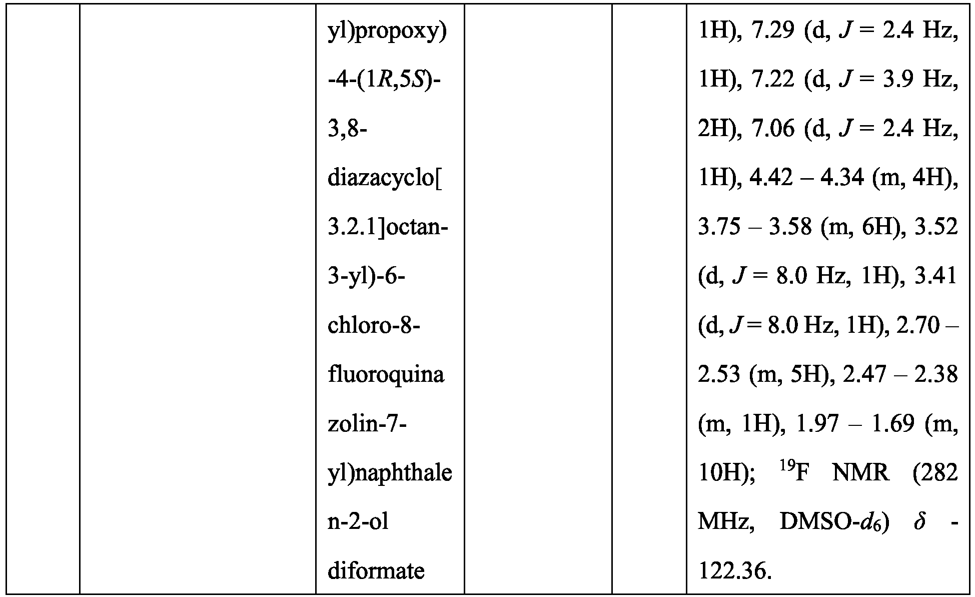

### Embodiment 8 (Synthesis method VII)

### (S or R)-1-((6-chloro-2-(3-(dimethylamino)azetidin-1-yl)-8-fluoro-7-(3-hydroxynaphthalen-1-yl)quinazolin-4-yl)imino)-1-thiomorpholine-1-oxide 71a; (R or S)-1-((6-chloro-2-(3-(dimethylamino)azetidin-1-yl)-8-fluoro-7-(3-hydroxynaphthalen-1-yl)quinazolin-4-yl)imino)-1-thiomorpholine-1-oxide 71b

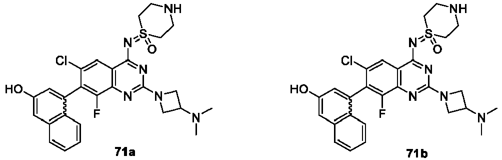

The synthetic route was as follows:

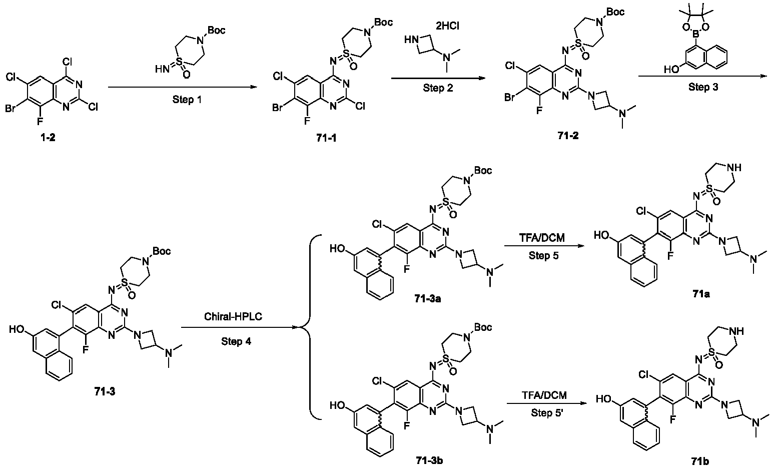

### Step 1:

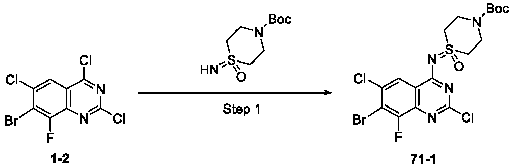

Compound **1-2** (700 mg, 2.01 mmol, 1.00 eq), 1,4-dioxane (8.0 mL), *tert*-butyl *1-*imino-1-oxothiomorpholine-4-carboxylate (496 mg, 2.01 mmol, 1.0 eq), 4,5-bis(diphenylphosphino)-9,9-dimethylxanthene (245 mg, 0.40 mmol, 0.2 eq), potassium *tert-*butoxide (262 mg, 2.21 mmol, 1.1 eq) and tris(dibenzylideneacetone)dipalladium (0) (194 mg, 0.20 mmol, 0.1 eq) were successively added to a 25 mL Schlenk tube with stirring under the protection of nitrogen at 25 °C. The reaction was carried out for 1 hour at 80 °C under nitrogen atmosphere. The reaction process was monitored by liquid chromatography-mass spectrometry. After the reaction was completed, the reaction mixture was cooled to 25 °C, and concentrated to obtain a crude product. The crude product was purified by silica gel column chromatography, eluted with a gradient of 0% → 30% ethyl acetate/petroleum ether mobile phase, and the obtained fractions were evaporated under reduced pressure to obtain compound **71-1** (yellow solid, 460 mg, yield: 41%). MS (ESI, *m*/*z*): 527.1/529.1/531.1 [M + H]⁺; ¹H NMR (300 MHz, DMSO-*d₆*) *δ* 8.24 (d, *J* = 1.9 Hz, 1H), 4.13 - 3.97 (m, 4H), 3.77 - 3.59 (m, 4H), 1.43 (s, 9H).

### Step 2:

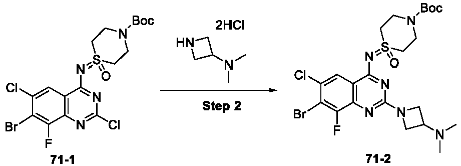

Compound **71-1** (460 mg, 0.83 mmol, 1.0 eq), *N*-methylpyrrolidone (12.0 mL), 3-(dimethylamino) azetidine dihydrochloride (131 mg, 1.24 mmol, 1.5 eq) and *N,N-*diisopropylethylamine (1.12 g, 8.27 mmol, 10.0 eq) were successively added to a 25 mL Schlenk tube with stirring under the protection of nitrogen at 25 °C. The reaction was carried out for 2 hours at 60 °C under nitrogen atmosphere. The reaction process was monitored by liquid chromatography-mass spectrometry. After the reaction was completed, the reaction mixture was cooled to 25 °C. The reaction mixture was directly purified by reversed-phase chromatographic column (C18 column), and eluted with 10% → 95% acetonitrile/water (0.1% ammonium bicarbonate) mobile phase in 35 min; detector, UV254/220 nm; to obtain compound **71-2** (yellow solid, 350 mg, yield: 67%). MS (ESI, *m*/*z*): 591.2/593.2/595.2 [M + H]⁺; ¹H NMR (400 MHz, CDCl₃) δ 7.98 (d, *J=* 1.9 Hz, 1H), 4.25 - 4.17 (m, 4H), 4.10 - 4.04 (m, 2H), 3.95 - 3.87 (m, 2H), 3.81 - 3.75 (m, 2H), 3.40 - 3.34 (m, 2H), 3.27 - 3.21 (m, 1H), 2.26 (s, 6H), 1.49 (s, 9H).

### Step 3:

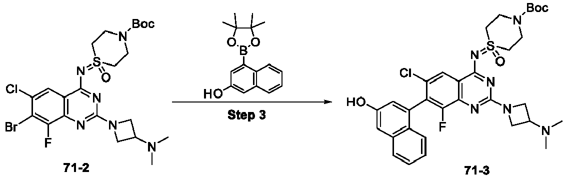

Water (0.8 mL), potassium phosphate (237 mg, 1.06 mmol, 2.0 eq) and 4-(4,4,5,5-tetramethyl-1,3,2-dioxaborolan-2-yl)naphthalen-2-ol (150.6 mg, 0.53 mmol, 1.00 eq) and chloro(2-dicyclohexylphosphino-2',4',6'-triisoporpyl-1,1 '-biphenyl) [2-(2'-amino-1,1'-biphenyl)] palladium (II) (43.8 mg, 0.05 mmol, 0.1 eq) were successively added to a solution of compound **71-2** (330 mg, 0.53 mmol, 1.0 eq) in tetrahydrofuran (8 mL) with stirring under the protection of nitrogen at 25 °C. The reaction was carried out for 1 hour at 60 °C under nitrogen atmosphere. The reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%--+ 15% methanol/dichloromethane mobile phase, and the obtained fractions were evaporated under reduced pressure to remove the solvent to obtain compound **71-3** (a mixture of two stereoisomers, yellow solid, 320 mg, yield: 87%). MS (ESI, *m*/*z*): 655.4/657.4 [M + H]⁺; ¹HNMR (300 MHz, CDCl₃) *δ* 8.05 (d, *J* = 1.5 Hz, 1H), 7.70 (d, *J* = 8.2 Hz, 1H), 7.41 - 7.35 (m, 1H), 7.32 - 7.29 (m, 1H), 7.26 (d, *J* = 2.4 Hz, 1H), 7.21 - 7.15 (m, 1H), 7.05 (d, *J* = 2.4 Hz, 1H), 4.33 - 4.20 (m, 4H), 4.14 - 4.06 (m, 2H), 4.01 - 3.92 (m, 2H), 3.83 - 3.74 (m, 2H), 3.42 - 3.32 (m, 2H), 3.25 - 3.16 (m, 1H), 2.24 (s, 6H), 1.52 (s, 9H).

### Step 4:

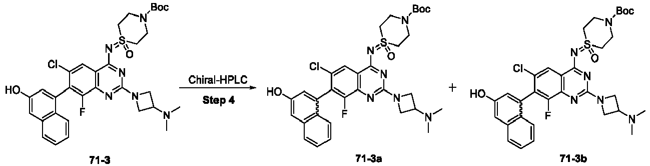

The compound **71-3** (320 mg) obtained in step 3 was subjected to chiral resolution, and the resolution conditions were: chiral column: CHIRALPAK ID, 2 x 25 cm, 5 µm; mobile phase A: n-hexane/dichloromethane = 5/1 (0.5% 2 mol/L ammonia methanol solution), mobile phase B: isopropanol; flow rate: 20 mL/min; gradient: elution with 10% phase B in 60 min; detector UV 220/210 nm; two products were obtained. The product with shorter retention time (28.92 min) was **71-3a,** (*S* or *R*)*tert*-butyl 1-((6-chloro-2-(3-(dimethylamino)azetidin-1-yl)-8-fluoro-7-(3-hydroxynaphthalen-1-yl)quinazolin-4-yl)imino)-1-thiomorpholine-4-carboxylate 1-oxide (yellow solid, 111 mg, recovery rate: 35%); the product with longer retention time (43.175 min) was **71-3b,** (*R* or *S*) *tert*-butyl 1-((6-chloro-2-(3-(dimethylamino)azetidin-1-yl)-8-fluoro-7-(3-hydroxynaphthalen-1-yl)quinazolin-4-yl)imino)-1-thiomorpholine-4-carboxylate 1-oxide (yellow solid, 116 mg, recovery rate: 37% ).

### Step 5:

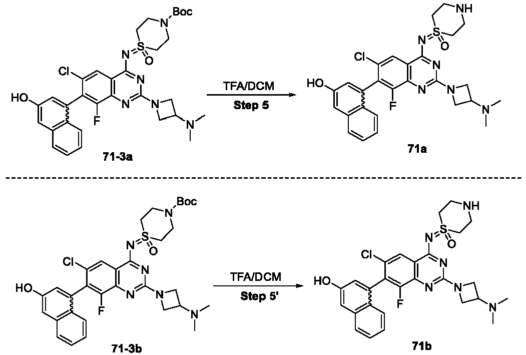

Trifluoroacetic acid (1.00 mL) was added dropwise to a solution of **71-3a** (100 mg, 0.145 mmol, 1.00 eq) in dichloromethane (4.00 mL) with stirring at 25 °C. After the dropwise addition, the reaction was carried out at room temperature for 1 hour, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 40% acetonitrile/water mobile phase (0.5% ammonium bicarbonate) in 20 min; detector, UV254/220 nm; to obtain compound **71a** (white solid, 40 mg, yield: 49%). Compound **71b** (white solid, 48 mg, yield: 53%) can be obtained by the same method as above.

Compound **71a:** MS (ESI, m/z): 555.2/557.2 [M + H]⁺; ¹H NMR (300 MHz, DMSO-*d₆*) *δ* 9.98 (s, 1H), 8.05 (d, *J* = 1.5 Hz, 1H), 7.80 (d, *J* = 8.3 Hz, 1H), 7.46 - 7.41 (m, 1H), 7.27 (d, *J* = 2.4 Hz, 1H), 7.25 - 7.18 (m, 2H), 7.04 (d, *J* = 2.4 Hz, 1H), 4.13 - 4.07 (m, 2H), 3.99 - 3.83 (m, 4H), 3.48 - 3.40 (m, 2H), 3.30 - 3.27 (m, 2H), 3.17 - 3.04 (m, 3H), 2.13 (s, 6H); ¹⁹F NMR (282 MHz, DMSO-*d₆*) *δ* -124.87. The chiral analysis conditions of compound **71a** were: CHIRALPAK IF-3, 4.6 × 50 mm, 3 µm; mobile phase A: *n*-hexane (0.1% diethylamine), mobile phase B: ethanol; flow rate: 1 mL/min; gradient: elution with 20% phase B in 12 min; detector UV 220/254 nm; retention time: 6.743 min; ee > 99%.

Compound **71b:** MS (ESI, m/z): 555.2/557.2 [M + H]⁺; ¹H NMR (300 MHz, DMSO-*d*₆) *δ* 9.98 (s, 1H), 8.05 (d, *J* = 1.5 Hz, 1H), 7.80 (d, *J* = 8.3 Hz, 1H), 7.46 - 7.41 (m, 1H), 7.27 (d, *J* = 2.4 Hz, 1H), 7.25 - 7.18 (m, 2H), 7.04 (d, *J* = 2.4 Hz, 1H), 4.13 - 4.07 (m, 2H), 3.99 - 3.83 (m, 4H), 3.48 - 3.40 (m, 2H), 3.30 - 3.27 (m, 2H), 3.17 - 3.04 (m, 3H), 2.13 (s, 6H); ¹⁹F NMR (282 MHz, DMSO-*d₆*) *δ* -124.87. The chiral analysis conditions of compound **71b** were: CHIRALPAK IF-3, 4.6 × 50 mm, 3 µm; mobile phase A: n-hexane (0.1% diethylamine), mobile phase B: ethanol; flow rate: 1 mL/min; gradient: elution with 20% phase B in 12 min; detector UV 220/254 nm; retention time: 9.968 min; ee > 99%.

### Embodiment 9

### (4R or S)-4-amino-1-(((R or S)-6-chloro-2-(3-(dimethylamino)azetidin-1-yl)-8-fluoro-7-(3 -hydroxynaphthalen-1 -yl)quinazolin-4-yl)imino)hexahydro-1 -thiopyran 1-oxide 72a; (4R or S)-4-amino-1-(((S or R)-6-chloro-2-(3-(dimethylamino)azetidin-1-yl)-8-fluoro-7-(3-hydroxynaphthalen-1-yl)quinazolin-4-yl)imino)hexahydro-1-thiopyran 1-oxide 72b; (4S or R)-4-amino-1-(((R or S)-6-chloro-2-(3-(dimethylamino)azetidin-1-yl)-8-fluoro-7-(3-hydroxynaphthalen-1-yl)quinazolin-4-yl)imino)hexahydro-1-thiopyran 1-oxide 72c; (4S or R)-4-amino-1-(((S or R)-6-chloro-2-(3-(dimethylamino)azetidin-1-yl)-8-fluoro-7-(3-hydroxynaphthalen-1-yl)quinazolin-4-yl)imino)hexahydro-1-thiopyran 1-oxide 72d

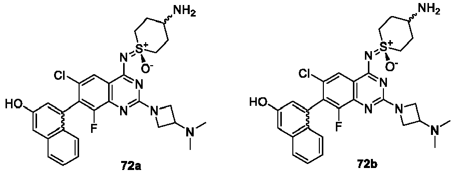

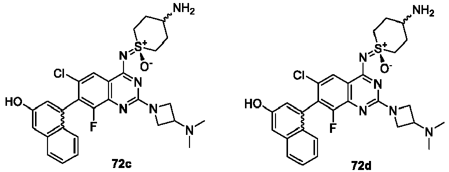

### Step 1:

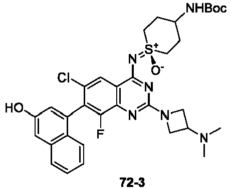

Compound **72-3** was synthesized according to Embodiment 8 (synthesis method VII). Compound **72-3** (a mixture of four stereoisomers, yellow solid, 320 mg): MS (ESI, m/z): 669.2/671.2 [M + H]⁺.

### Step 2:

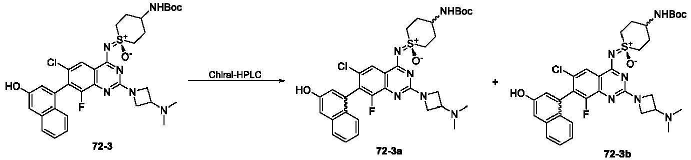

The compound **72-3** (310 mg) obtained in step 1 was subjected to chiral resolution by preparative chiral high performance liquid chromatography, and the resolution conditions were: chiral column CHIRAL ART Amylose-SA, 3 × 25 cm, 5 µm; mobile phase A: n-hexane (10 mmol/L ammonia methanol solution), mobile phase B: isopropanol; flow rate: 40 mL/min; gradient: gradient elution with 20% mobile phase B in 30 min; detector UV 250/220 nm, two products were obtained. The product with shorter retention time (15 min) was **72-3a** (a mixture of two stereoisomers, yellow solid, 133 mg, recovery rate: 43%); the product with longer retention time (20 min) was **72-3b** (a mixture of two stereoisomers, yellow solid, 140 mg, recovery rate: 45%).

### Step 3:

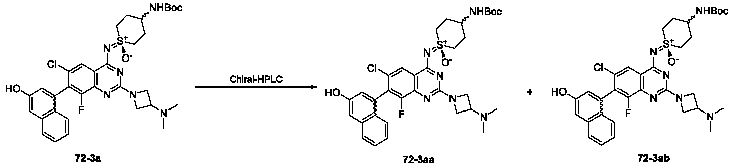

The compound **72-3a** (133 mg) obtained in step 2 was subjected to chiral resolution by preparative chiral high performance liquid chromatography, and the resolution conditions were: chiral column CHIRAL ART Cellulose-SB, 2 × 25 cm, 5 µm; mobile phase A: *n-*hexane/dichloromethane = 5/1 (0.5% 2 mol/L ammonia methanol solution), mobile phase B: ethanol; flow rate: 20 mL/min; gradient: gradient elution with 30% mobile phase B in 10 min; detector UV 250/220 nm; two products were obtained. The product with shorter retention time (3.2 min) was **72-3aa,** *tert*-butyl((1S,4R or 1*R*,4*S*)-1-(((*S* or *R*)-6-chloro-2-(3-(dimethylamino)azetidin-1-yl)-8-fluoro-7-(3-hydroxynaphthalen-1-yl)quinazolin-4-yl)imino)-1-oxyhexahydro-1-thiopyran-4-yl)carbamate (yellow solid, 117 mg, recovery rate: 87%), the product with longer retention time (5.7 min) was **72-3ab,** *tert*-butyl ((1*R*, 4*S* or 1*S*,4*R*)-1-(((*S* or *R*)-6-chloro-2-(3 -(dimethylamino)azetidin-1 -yl)-8-fluoro-7-(3 -hydroxylnaphthalen-1 - yl)quinazolin-4-yl)imino)-1-oxyhexahydro-1-thiopyran-4-yl)carbamate (yellow solid, 11 mg, recovery rate: 8% ).

### Step 4:

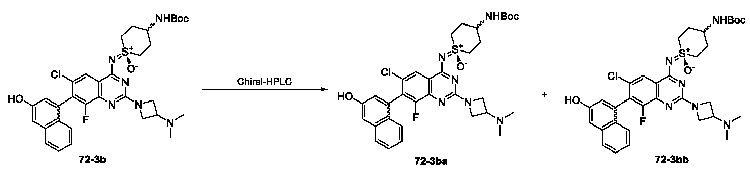

The compound **72-3b** (140 mg) obtained in step 2 was subjected to chiral resolution by preparative chiral high performance liquid chromatography, and the resolution conditions were: chiral column CHIRAL ART Cellulose-SB, 2 × 25 cm, 5 µm; mobile phase A: n-hexane (10 mmol/L ammonia methanol solution), mobile phase B: ethanol; flow rate: 20 mL/min; gradient: gradient elution with 30% mobile phase B in 23 min; detector UV 250/220 nm; two products were obtained. The product with shorter retention time (5.8 min) was **72-3ba,** *tert-*butyl((1*S*,4*R* or 1*R*,4*S*)-1-(((*R* or *S*)-6-chloro-2-(3-(dimethylamino)azetidin-1-yl)-8-fluoro-7-(3-hydroxynaphthalen-1-yl)quinazolin-4-yl)imino)-1-oxyhexahydro-1 -thiopyran-4-yl)carbamate (yellow solid, 110 mg, recovery rate: 78%), the product with longer retention time (15.8 min) was **72-3bb,** *tert*-butyl ((1*R*,4*S* or 1*S*,4*R*)-1-(((*R* or *S*)-6-chloro-2-(3-(dimethylamino)azetidin-1-yl)-8-fluoro-7-(3-hydroxylnaphthalen-1-yl)quinazolin-4-yl)imino)-1-oxyhexahydro-1-thiopyran-4-yl)carbamate (yellow solid, 15 mg, recovery rate: 10%).

### Step 5:

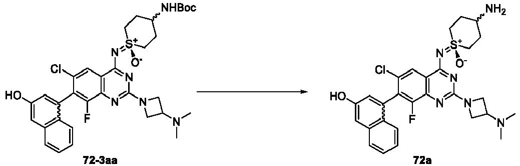

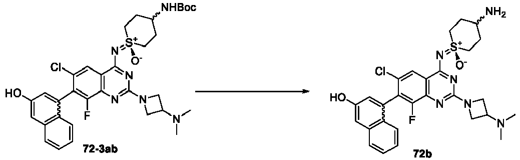

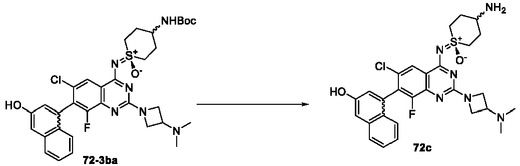

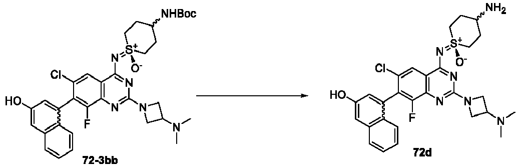

Trifluoroacetic acid (1.0 mL) was added dropwise to a solution of **72-3aa** (100 mg, 0.14 mmol, 1.0 eq) in dichloromethane (4.0 mL) with stirring at 25 °C. After the dropwise addition, the reaction was carried out at that temperature for 0.5 hours, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 35% → 65% acetonitrile/water mobile phase (0.5% ammonium bicarbonate) in 20 min; detector, UV254/220 nm; to obtain compound **72a** (white solid, 56 mg, yield: 67%). Compound **72b** (white solid, 5 mg, yield: 49%), **72c** (white solid, 50 mg, yield: 60%) and **72d** (white solid, 5.8 mg, yield: 46%) can also be obtained by the same method.

Compound **72a:** MS (ESI, m/z): 569.3/571.3 [M + H]⁺; ¹H NMR (300 MHz, CD₃OD) *δ* 8.10 (d, *J* = 1.4 Hz, 1H), 7.75 (d, *J* = 8.3 Hz, 1H), 7.44 - 7.39 (m, 1H), 7.26 - 7.17 (m, 3H), 7.04 - 7.02 (m, 1H), 4.31 -4.26 (m, 2H), 4.07 - 4.02 (m, 2H), 3.95 - 3.78 (m, 4H), 3.31- 3.25 (m, 1H), 3.22 - 3.15 (m, 1H), 2.37 - 2.25 (m, 8H), 2.20 - 2.09 (m, 2H); ¹⁹F NMR (282 MHz, CD₃OD) *δ* -126.72.

Compound **72b:** MS (ESI, m/z): 569.3/571.3 [M + H]⁺; ¹H NMR (300 MHz, CD₃OD) *δ* 8.18 (d, *J* = 1.7 Hz, 1H), 7.76 (d, *J* = 8.2 Hz, 1H), 7.45 - 7.39 (m, 1H), 7.27 - 7.17 (m, 3H), 7.03 (d, *J* = 2.4 Hz, 1H), 4.31 - 4.13 (m, 4H), 4.07 - 4.02 (m, 2H), 3.66 - 3.52 (m, 2H), 3.31 - 3.14 (m, 2H), 2.34 - 2.24 (m, 8H), 2.11 - 1.98 (m, 2H); ¹⁹F NMR (282 MHz, CD₃OD) *δ* - 126.71.

Compound **72c:** MS (ESI, m/z): 569.2/571.2 [M + H]⁺; ¹H NMR (300 MHz, CD₃OD) *δ* 8.11 (d, *J* = 1.7 Hz, 1H), 7.75 (d, *J* = 8.3 Hz, 1H), 7.44 - 7.39 (m, 1H), 7.26 - 7.17 (m, 3H), 7.03 (d, *J* = 2.4 Hz, 1H), 4.31 - 4.25 (m, 2H), 4.07 - 4.02 (m, 2H), 3.95 - 3.76 (m, 4H), 3.30 - 3.15 (m, 2H), 2.36 - 2.25 (m, 8H), 2.20 - 2.08 (m, 2H); ¹⁹F NMR (282 MHz, CD₃OD) δ - 126.67.

Compound **72d:** MS (ESI, m/z): 569.2/571.2 [M + H]⁺; ¹H NMR (300 MHz, CD₃OD) *δ* 8.18 (d, *J* = 1.6 Hz, 1H), 7.76 (d, *J* = 8.4 Hz, 1H), 7.45 - 7.39 (m, 1H), 7.27 - 7.18 (m, 3H), 7.03 (d, *J* = 2.4 Hz, 1H), 4.31 - 4.14 (m, 4H), 4.07 - 4.02 (m, 2H), 3.66 - 3.52 (m, 2H), 3.30 - 3.14 (m, 2H), 2.33 - 2.25 (m, 8H), 2.11 - 1.98 (m, 2H); ¹⁹F NMR (282 MHz, CD₃OD) *δ* - 126.70.

### Embodiment 10 (Synthesis method IX)

### 4-(2-(3-(Dimethylamino)propoxy)-4-(piperazin-1-yl)-5,8-dihydropyrido[3,4-d]pyrimidin-7(6H)-yl)naphthalen-2-ol 73

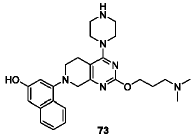

The synthetic route was as follows:

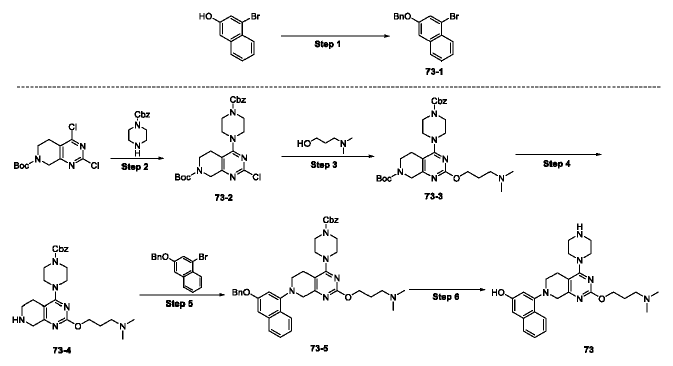

### Step 1:

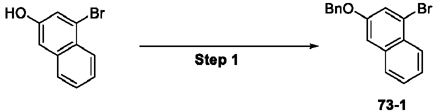

Potassium carbonate (620 mg, 4.48 mmol, 2.0 eq) and benzyl bromide (460 mg, 2.69 mmol, 1.2 eq) were added to a solution of 1-bromo-3-hydroxynaphthalene (500 mg, 2.24 mmol, 1.0 eq) in *N, N*-dimethylformamide (5.0 mL) with stirring under the protection of nitrogen at 25 °C. The reaction was carried out for 2 hours at 25 °C under nitrogen atmosphere, and the reaction process was monitored by thin layer chromatography. After the reaction was completed, the mixture was extracted with ethyl acetate (20 mL × 3), and the organic phases were combined, dried over anhydrous sodium sulfate, filtered, and the filtrate was concentrated to obtain a crude product. The crude product was purified by silica gel column chromatography, eluted with a gradient of 0% → 10% ethyl acetate/petroleum ether mobile phase, and the obtained fractions were evaporated under reduced pressure to obtain compound **73-1** (colorless oil, 500 mg, yield: 70%). ¹H NMR (400 MHz, CDCl₃) *δ* 8.22 - 8.08 (m, 1H), 7.74 - 7.67 (m, 1H), 7.59 (d, *J* = 2.4, 1H), 7.52 - 7.39 (m, 6H), 7.39 - 7.31 (m, 1H), 7.20 (d, *J* = 2.4, 1H), 5.17 (s, 2H).

### Step 2:

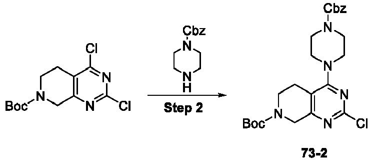

*N,N*-diisopropylethylamine (8.5 g, 64.43 mmol, 4.0 eq) and benzyl-1-piperazine carbonate (3.6 g, 16.11 mmol, 1.0 eq) were successively added to a solution of *tert*-butyl 2,4-dichloro-5,6-dihydropyridino[3, 4-*d*]pyrimidine-7(8*H*)-carboxylate (5.0 g, 16.10 mmol, 1.0 eq) in *N, N*-dimethylacetamide (30 mL) with stirring at 25 °C. The reaction was carried out for 3 hours at 50 °C, and the reaction process was monitored by liquid chromatography-mass spectrometry. After the reaction was completed, the mixture was extracted with ethyl acetate (100 mL × 3), and the organic phases were combined and washed with 100mL of saturated brine, dried over anhydrous sodium sulfate, filtered, and the filtrate was concentrated to obtain a crude product. The obtained crude product was purified by silica gel column chromatography, eluted with a gradient of 0%—>10% methanol/dichloromethane mobile phase, and the obtained fractions were evaporated under reduced pressure to remove the solvent to obtain compound **73-2** (white solid, 6.3 g, yield: 76%). MS (ESI, m/z): 488.2/490.2 [M + H]⁺; ¹H NMR (300 MHz, CDCl₃) *δ* 7.42 - 7.29 (m, 5H), 5.17 (s, 2H), 4.53 (s, 2H), 3.65 - 3.57 (m, 4H), 3.59 - 3.56 (m, 2H), 3.50 - 3.48 (m, 4H), 2.64 - 2.60 (m, 2H), 1.48 (s, 9H).

### Step 3:

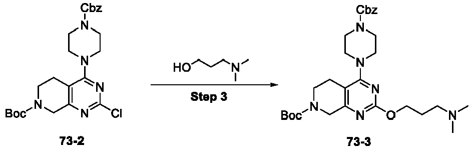

3-Dimethylamino-1-propanol (603 mg, 5.84 mmol, 1.2 eq), cesium carbonate (4.8 g, 14.60 mmol, 3.0 eq) and mesylate (2-dicyclohexylphosphono-2',6'-diisopropoxy-1,1'-biphenyl)(2-amino-1,1'-biphen-2-yl) palladium (II) (407 mg, 0.49 mmol, 0.1 eq) were successively added to a solution of **73-2** (2.5 g, 4.87 mmol, 1.0 eq) in 1,4-dioxane (10.0 mL) with stirring under the protection of nitrogen at 25 °C. The reaction was carried out for 2 hours at 90 °C under nitrogen atmosphere, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **73-3** (brown yellow oil, 1.4 g, yield: 51%). MS (ESI, m/z): 555.5 [M + H]⁺; ¹H NMR (300 MHz, CDCl₃) *δ* 7.41 - 7.35 (m, 5H), 5.19 (s, 2H), 4.48 (s, 2H), 4.34 (t, *J* = 6.5 Hz, 2H), 3.65 - 3.56 (m, 6H), 3.46 - 3.41 (m, 4H), 2.62 - 2.58 (m, 2H), 2.50 (t, *J* = 7.5 Hz, 2H), 2.28 (s, 6H), 2.03 - 1.94 (m, 2H), 1.51 (s, 9H).

### Step 4:

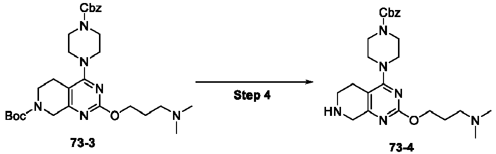

Trifluoroacetic acid (5 mL) was added dropwise to a solution of **73- 3** (1.4 g, 2.50 mmol, 1.0 eq) in dichloromethane (12.0 mL) with stirring at 25 °C. The reaction was carried out for 1 hour at 25 °C, and the reaction process was monitored by liquid chromatography-mass spectrometry. After the reaction was completed, the reaction solution was concentrated to obtain a crude product of **73-4** (brown yellow oil, 1.1 g, yield: 95%). The crude product was used directly in the next step without further purification. MS (ESI, m/z): 455.3 [M + H]⁺.

### Step 5:

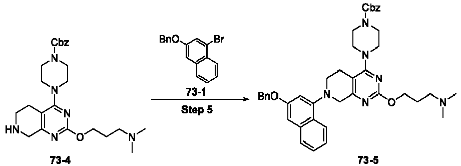

**73-1** (530 mg, 1.68 mL, 1.1 eq), cesium carbonate (1.5 g, 4.57 mmol, 3.0 eq), 2-dicyclohexylphosphorus-2',6'-diisopropoxy-1,1'-biphenyl (108 mg, 0.23 mmol, 0.15 eq) and tris(dibenzylideneacetone)dipalladium (0) (141 mg, 0.15 mmol, 0.1 eq) were added successively to a solution of **73-4** (700 mg, 1.52 mmol, 1.0 eq) in 1,4-dioxane (8.0 mL) with stirring at 25°C under the protection of nitrogen. The reaction was carried out for 5 hours at 85 °C under nitrogen atmosphere, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **73-5** (white solid, 500 mg, yield: 46%). MS (ESI, m/z): 687.4 [M + H]⁺.

### Step 6:

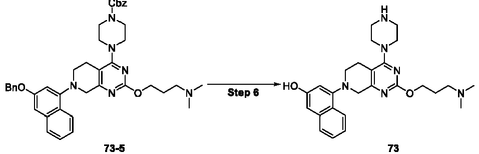

Palladium hydroxide carbon (20 mg) was added to a solution of **73-5** (120 mg, 0.18 mmol, 1.0 eq) in ethyl acetate (20.0 mL) with stirring under the protection of nitrogen at 25 °C. The reaction was carried out at 70 °C under hydrogen (10 atmospheric pressure) atmosphere for 5 hours, and the reaction process was monitored by liquid chromatography-mass spectrometry. After the reaction was completed, the reaction solution was filtered with diatomite, and the filter cake was washed with ethyl acetate (50 mL × 3), and the filtrate was concentrated to obtain a crude product. The obtained crude product was purified by reversed-phase chromatographic column (C18 column), and eluted with 5% → 95% acetonitrile/water mobile phase (0.1% formic acid) in 20 min; detector, UV254/220 nm; to obtain compound 73 (white solid, 3.1 mg, yield: 3.5%). MS (ESI, m/z): 463.2 [M + H]⁺; ¹H NMR (300 MHz, DMSO-*d₆*) *δ* 9.68 (s, 1H), 8.01 (d, *J* = 8.4 Hz, 1H), 7.67 (d, *J* = 7.8 Hz, 1H), 7.42 - 7.36 (m, 1H), 7.30 - 7.24 (m, 1H), 6.85 (d, *J* = 2.2 Hz, 1H), 6.76 (d, *J* = 2.2 Hz, 1H), 4.23 (t, *J* = 6.6 Hz, 2H), 4.06 (s, 2H), 3.42 - 3.22 (m, 6H), 2.86 - 2.78 (m, 6H), 2.33 (t, *J* = 7.1 Hz, 2H), 2.14 (s, 6H), 1.87 - 1.77 (m, 2H).

### Embodiment 11 (Synthesis method X)

### 4-((1R,5S)-3,8-diazabicyclo[3.2.1]octan-3-yl)-6-chloro-1-(3-(dimethylamino)propyl)-8-fluoro-7-((S or R)-3-hydroxynaphthalen-1-yl)quinazolin-2(1H)-one 74a; 4-((1R,5S)-3,8-diazabicyclo[3.2.1]octan-3-yl)-6-chloro-1-(3-(dimethylamino)propyl)-8-fluoro-7-((R or S)-3-hydroxynaphthalen-1-yl)quinazolin-2(1H)-one 74b

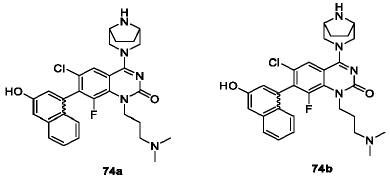

The synthetic route was as follows:

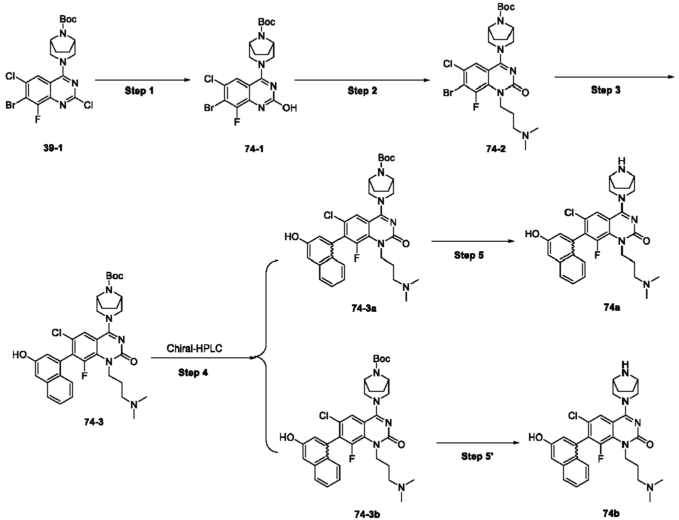

### Step 1:

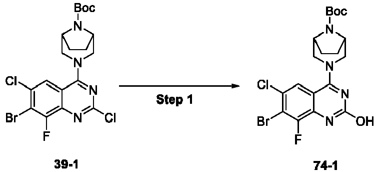

**39-1** (1.7 g, 3.19 mmol, 1.0 eq) and glacial acetic acid (20.0 mL) were added to a 50 mL round bottom flask at 25 °C. The reaction was carried out for 3 hours at 80 °C, and the reaction process was monitored by liquid chromatography-mass spectrometry. After the reaction was completed, the reaction mixture was cooled to 25 °C, and concentrated to obtain a intermediate. The intermediate was dissolved in 50 mL of dichloromethane, and di*-tert-*butyl dicarbonate (870 mg, 3.82 mmol, 1.2 eq) and triethylamine (1.4 mL) were added thereto at 25 °C. The reaction was carried out for 1 hour at that temperature, and the reaction process was monitored by liquid chromatography-mass spectrometry. After the reaction was completed, the reaction mixture was concentrated to obtain a crude product. The crude product was purified by reversed-phase chromatographic column (C18 column), and eluted with 50% → 95% methanol/water mobile phase (0.1% formic acid) in 15 min; detector, UV254/220 nm; to obtain compound **74-1** (white solid, 1.2 g, yield: 72%). MS (ESI, *m*/*z*): 487.1/489.1/491.1 [M + H]⁺; ¹H NMR (300 MHz, DMSO-*d₆*) *δ* 11.24 (s, 1H), 7.81 (d, *J* = 2.0 Hz, 1H), 4.32 - 4.18 (m, 4H), 3.51 - 3.45 (m, 2H), 1.79 - 1.64 (m, 4H), 1.46 (s, 9H).

### Step 2:

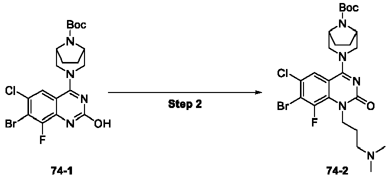

(3-Bromopropyl) dimethylamine hydrobromide (0.56 g, 2.14 mmol, 1.0 eq) and potassium carbonate (1.25 g, 8.57 mmol, 4.0 eq) were added to a solution of **74-1** (1.1 g, 2.14 mmol, 1.0 eq) in acetonitrile (15 mL) at 25 °C. The reaction was carried out for 2 hours at 80 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%—>10% methanol/dichloromethane mobile phase, and the obtained fractions were evaporated under reduced pressure to remove the solvent to obtain compound **74-2** (light yellow solid, 310 mg, yield: 25%). MS (ESI, *m*/*z*): 572.2/574.2 [M + H]⁺; ¹HNMR (300 MHz, CD₃OD) *δ* 7.85 (d, *J* = 2.0 Hz, 1H), 4.45 - 4.39 (m, 2H), 4.34 - 4.27 (m, 4H), 3.65 - 3.59 (m, 2H), 3.17 (t, *J* = 7.4 Hz, 2H), 2.85 (s, 6H), 2.30 - 2.20 (m, 2H), 1.93 - 1.74 (m, 4H), 1.54 (s, 9H).

### Step 3:

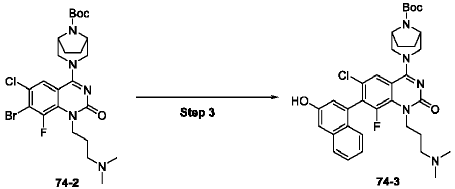

4-(4,4,5,5-Tetramethyl-1,3,2-dioxaborolan-2-yl)naphthalen-2-ol (177 mg, 0.62 mmol, 1.5 eq), potassium phosphate (185 mg, 0.82 mmol, 2.0 eq) and chloro(2-dicyclohexylphosphino-2',4',6'-triisoporpyl-1,1'-biphenyl)[2-(2'-amino-1,1'-biphenyl)] palladium (II) (34 mg, 0.04 mmol, 0.10 eq) were added to a solution of compound **74-2** (250 mg, 0.41 mmol, 1.0 eq) in tetrahydrofuran/water (10/1, 4 mL) with stirring under the protection of nitrogen at 25 °C. The reaction was carried out with stirring for 2 hours at 60 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%—>10% methanol/dichloromethane mobile phase, and the obtained fractions were evaporated under reduced pressure to remove the solvent to obtain compound **74-3** (a mixture of two stereoisomers, yellow solid, 250 mg, yield: 94%). MS (ESI, m/z): 636.3/638.3 [M + H]⁺.

### Step 4:

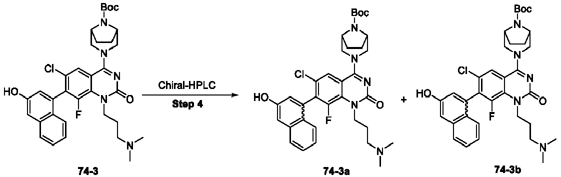

The compound **74-3** (250 mg) obtained in step 3 was subjected to chiral resolution, and the resolution conditions were: chiral column CHIRALPAK IG, 2 × 25 cm, 5 µm; mobile phase A: *n*-hexane (10 mmol/L ammonia methanol solution), mobile phase B: ethanol; flow rate: 20 mL/min; elution with 30% mobile phase B in 25 min; detector UV 227/254 nm; two products were obtained. The product with shorter retention time (7.01 min) was **74-3a,** *tert-*butyl (1*R*,5*S*)-3-(6-chloro-1-(3-(dimethylamino)propyl)-8-fluoro-7-((*S* or *R)*-3-hydroxynaphthalen-1-yl)-2-oxo-1,2-dihydroquinazolin-4-yl)-3,8-diazabicyclo[3.2.1]octane-8-carboxylate (light yellow solid, 100 mg, recovery rate: 40% ); the product with longer retention time (13.14 min) was **74-3b,** *tert*-butyl (1*R*,5*S*)-3-(6-chloro-1-(3-(dimethylamino)propyl)-8-fluoro-7-((*R* or *S*)-3-hydroxynaphthalen-1-yl)-2-oxo-1,2-dihydroquinazolin-4-yl)-3,8-diazabicyclo[3.2.1]octane-8-carboxylate (light yellow solid, 100 mg, recovery rate: 40%).

### Step 5:

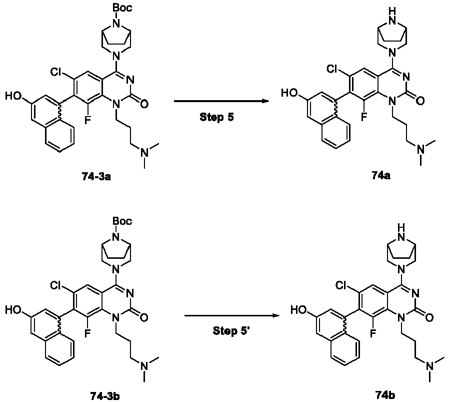

Trifluoroacetic acid (1.0 mL) was added dropwise to a solution of **74-3a** (100 mg, 0.14 mmol, 1.0 eq) in dichloromethane (4.0 mL) with stirring at 25 °C. After the dropwise addition, the reaction was carried out at 25 °C for 1 hour, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 50% → 95% methanol/water mobile phase (0.5% ammonium bicarbonate) in 20 min; detector, UV254/220 nm; to obtain compound **74a** (light yellow solid, 35 mg, yield: 42%). Compound **74b** (white solid, 50 mg, yield: 61%) can be obtained by the same method as above.

Compound **74a:** MS (ESI, m/z): 536.2/538.2 [M + H]⁺; ¹H NMR (300 MHz, DMSO-*d₆*) *δ* 10.05 (s, 1H), 7.81 (d, *J* = 8.3 Hz, 1H), 7.77 (d, *J* = 1.5 Hz, 1H), 7.47 - 7.42(m, 1H), 7.30 - 7.20 (m, 3H), 7.08 (d, *J* = 2.4 Hz, 1H), 4.27 - 4.20 (m, 2H), 4.11 - 4.04 (m, 2H), 3.52 - 3.41 (m, 4H), 2.15 (t, *J* = 7.0 Hz, 2H), 2.03 (s, 6H), 1.78 - 1.67(m, 2H), 1.65 - 1.62 (m, 4H); ¹⁹F NMR (282 MHz, DMSO-*d₆*) *δ* -119.44. The chiral analysis conditions of compound **74a** were: CHIRALPAK ID-3, 4.6 × 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV220/254 nm; retention time: 3.994 min; ee > 99%.

Compound **74b:** MS (ESI, m/z):536.2/538.2 [M + H]⁺; ¹H NMR (300 MHz, DMSO-*d₆) δ* 10.05 (s, 1H), 7.81 (d, *J* = 8.3 Hz, 1H), 7.77 (d, *J* = 1.5 Hz, 1H), 7.47 - 7.42(m, 1H), 7.30 - 7.20 (m, 3H), 7.08 (d, *J* = 2.4 Hz, 1H), 4.27 - 4.20 (m, 2H), 4.11 - 4.04 (m, 2H), 3.52 - 3.41 (m, 4H), 2.15 (t, *J* = 7.0 Hz, 2H), 2.03 (s, 6H), 1.78 - 1.67(m, 2H), 1.65 - 1.62 (m, 4H); ¹⁹F NMR (282 MHz, DMSO-*d*₆) *δ* -119.44. The chiral analysis conditions of compound **74b** were: CHIRALPAK ID-3, 4.6 × 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV 220/254 nm; retention time: 4.737 min; ee > 99%.

### Embodiment 12 (Synthesis method XI)

### (R or S) 4-(6-chloro-2-(3-(dimethylamino)azetidin-1-yl)-8-fluoro-7-(3-hydroxynaphthalen-1-yl)quinazolin-4-yl)-1,4-azaphosphinane-4-oxide 75a; (S or R) 4-(6-chloro-2-(3-(dimethylamino)azetidin-1-yl)-8-fluoro-7-(3-hydroxynaphthalen-1-yl)quinazolin-4-yl)-1,4-azaphosphinane-4-oxide 75b

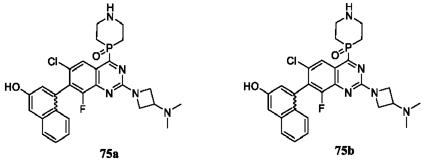

The synthetic route was as follows:

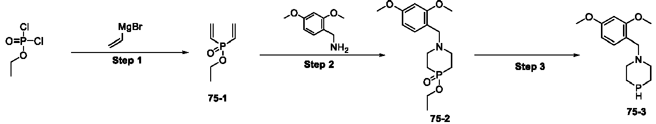

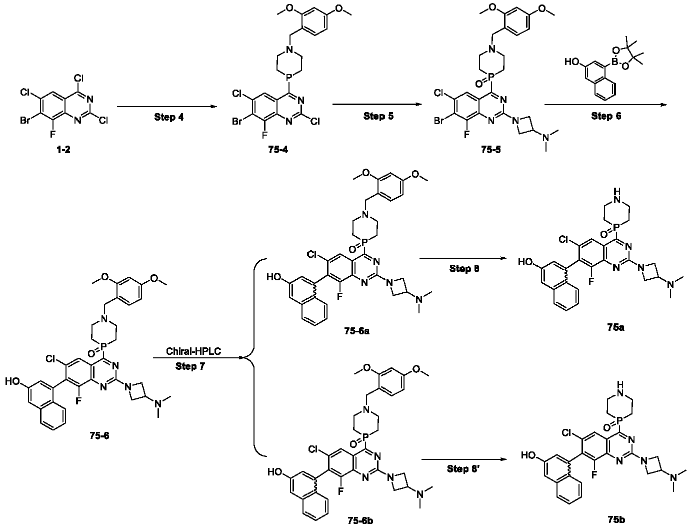

### Step 1:

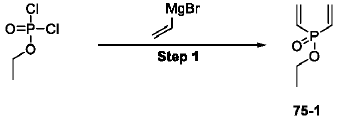

Vinyl magnesium bromide (1 mol/L tetrahydrofuran solution, 118 mL, 118.00 mmol, 2.0 eq) was added slowly dropwise to a solution of ethyl dichlorophosphate (10 g, 58.31 mmol, 1.0 eq) in dichloromethane (200 mL) with stirring under the protection of nitrogen at -78°C for not less than 30 min. After the dropwise addition, the reaction was carried out at this temperature for 3 hours, and the reaction process was monitored by silica gel thin layer chromatography (dichloromethane/methanol 10/1, R*_{f}* = 0.3). After the reaction was completed, the reaction solution was slowly raised to 25 °C, and the reaction was quenched with a 1 mol/L hydrochloric acid solution (50 mL), then the mixture after quenching was directly concentrated to obtain a crude product of **75-1.** The obtained crude product was purified by silica gel column chromatography, eluted with a gradient of 0%→10% methanol/dichloromethane mobile phase, and the obtained fractions were evaporated under reduced pressure to remove the solvent to obtain compound **75-1** (yellow oil, 7.2 g, yield: 80%). ¹H NMR (400 MHz, CDCl₃) *δ* 6.37 - 6.32 (m, 1H), 6.32 - 6.26 (m, 1H), 6.26 - 6.22 (m, 2H), 6.19 - 6.11 (m, 2H), 4.10 - 4.02 (m, 2H), 1.34 (t, *J* = 7.1 Hz, 3H).

### Step 2:

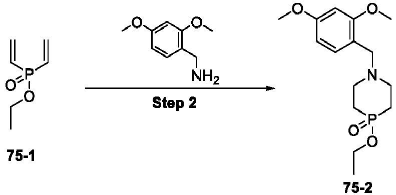

2,4-Dimethoxybenzylamine (8.24 g, 46.81 mmol, 1.0 eq) was added to a solution of compound **75-1** (7.2 g, 46.81 mmol, 1.0 eq) in ethanol (200 mL) with stirring at 25 °C. The reaction was carried out at 80 °C for 2 hours. The reaction process was monitored by liquid chromatography-mass spectrometry. After the reaction was completed, the reaction mixture was cooled to room temperature, and concentrated under reduced pressure to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **75-2** (yellow oil, 11 g, yield: 79%). MS (ESI, *m*/*z*): 314.3 [M + H]⁺; ¹H NMR (400 MHz, CDCl₃) *δ* 7.23 (d, *J* = 8.1 Hz, 1H), 6.53 - 6.45 (m, 2H), 4.15 - 4.03 (m, 2H), 3.83 (s, 3H), 3.81 (s, 3H), 3.61 (s, 2H), 3.15 - 2.95 (m, 2H), 2.76 - 2.64 (m, 2H), 2.08 - 1.79 (m, 4H), 1.38 - 1.34 (m, 3H).

### Step 3:

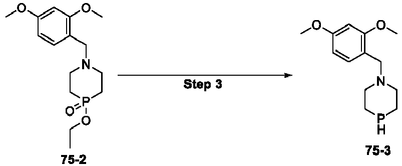

Lithium aluminum hydride (1 mol/L tetrahydrofuran solution, 64.0 mL, 64.00 mmol, 4.0 eq) was added to a solution of **75-2** (6 g, 18.19 mmol, 1.0 eq) in anhydrous tetrahydrofuran (100 mL) with stirring under the protection of nitrogen at 0 °C. The reaction was carried out for 48 hours at 25 °C, and the reaction process was monitored by liquid chromatography-mass spectrometry. After the reaction was completed, 2 mL of water, 2 mL of 15% sodium hydroxide solution and 6 mL of water were added successively, and the mixture was stirred for 15 min, filtered, and concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **75-3** (colorless oil, 2.4 g, yield: 49%). MS (ESI, m/z): 254.1 [M + H]⁺.

### Step 4:

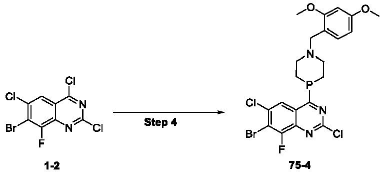

**75-3** (2.4 g, 9.06 mmol, 0.9 eq) and triethylamine (3.22 g, 30.20 mmol, 3.0 eq) were added to a solution of compound **1-2** (3.5 g, 10.06 mmol, 1.0 eq) in 1,4-dioxane (50 mL) with stirring at 25 °C. The reaction was carried out for 16 hours at that temperature, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 30% ethyl acetate/petroleum ether mobile phase, and the obtained fractions were evaporated under reduced pressure to remove the solvent to obtain compound **75-4** (white solid, 4 g, yield: 69%). MS (ESI, m/z): 546.0/548.0/550.0 [M + H]⁺; ¹H NMR (400 MHz, CDCl₃) *δ* 8.17 (d, *J* = 2.8, 1.9 Hz, 1H), 7.20 (d, *J* = 8.2 Hz, 1H), 6.47 - 6.43 (m, 2H), 3.80 (s, 3H), 3.78 (s, 3H), 3.48 (s, 2H), 2.82 - 2.71 (m, 4H), 2.40 - 2.22 (m, 4H); ³¹P NMR (162 MHz, CDCl₃) *δ* -35.26.

### Step 5:

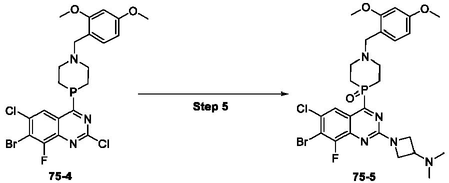

*N,N*-diisopropylethylamine (4.72 g, 34.72 mmol, 5.0 eq) and 3-(dimethylamino) azetidine dihydrochloride (1.9 g, 10.42 mmol, 1.5 eq) were added to a solution of compound **75-4** (4 g, 6.94 mmol, 1.0 eq) in *N*-methylpyrrolidone (40 mL) in an air atmosphere with stirring at 25 °C. The mixture was stirred for 2 hours at 60 °C, and the reaction process was monitored by liquid chromatography-mass spectrometry. After the reaction was completed, the reaction mixture was cooled to 25 °C. The reaction mixture was directly purified by reversed-phase rapid chromatographic column (C18 column), and eluted with 35% → 85% acetonitrile/water mobile phase (0.1% ammonium bicarbonate) in 25 min; detector, UV254 nm; to obtain compound **75-5** (yellow solid, 2.4 g, yield: 55%). MS (ESI, m/z): 626.2/628.2/630.2 [M + H]⁺; ¹H NMR (400 MHz, CDCl₃) *δ* 8.88 (d, *J* = 2.1 Hz, 1H), 7.23 (d, *J* = 7.9 Hz, 1H), 6.50 - 6.46 (m, 2H), 4.31 - 4.25 (m, 2H), 4.14 - 4.08 (m, 2H), 3.82 (s, 3H), 3.80 (s, 3H), 3.68 (s, 2H), 3.30 - 3.23 (m, 1H), 3.14 - 2.99 (m, 4H), 2.54 - 2.44 (m, 2H), 2.26 (s, 6H), 2.24 - 2.15 (m, 2H); ³¹P NMR (162 MHz, CDCl₃) *δ* 34.58.

### Step 6:

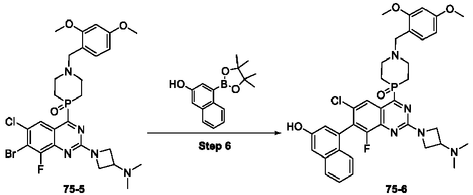

4-(4,4,5,5-Tetramethyl-1,3,2-dioxaborolan-2-yl)naphthalen-2-ol (1.03 g, 3.64 mmol, 1.0 eq), potassium phosphate (2.44 g, 10.91 mmol, 3.0 eq) and chloro(2-dicyclohexylphosphino-2',4',6'-triisoporpyl-1,1'-biphenyl)[2-(2'-amino-1,1'-biphenyl)] palladium (II) (904 mg, 1.09 mmol, 0.3 eq) were added to a solution of compound **75-2** (2.4 g, 3.64 mmol, 1.0 eq) in tetrahydrofuran/water (10/1, 4 mL) with stirring under the protection of nitrogen at 25 °C. The reaction was carried out with stirring for 2 hours at 60 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%—>10% methanol/dichloromethane mobile phase, and the obtained fractions were evaporated under reduced pressure to remove the solvent to obtain compound **75-6** (a racemic mixture of two stereoisomers, yellow solid, 800 mg, yield: 30%). MS (ESI, m/z): 690.3/692.3 [M + H]⁺; ¹H NMR (300 MHz, CDCl₃) *δ* 8.84 (d, *J* = 1.6 Hz, 1H), 7.72 (d, *J* = 8.3 Hz, 1H), 7.43 - 7.37 (m, 1H), 7.27 - 7.16 (m, 4H), 7.06 (d, *J* = 2.4 Hz, 1H), 6.54 - 6.49 (m, 2H), 4.31 - 4.25 (m, 2H), 4.16 - 4.11 (m, 2H), 3.84 (s, 3H), 3.82 (s, 3H), 3.75 (s, 2H), 3.34 - 3.25 (m, 1H), 3.19 - 3.09 (m, 4H), 2.65 - 2.53 (m, 2H), 2.34 - 2.23 (s, 8H); ³¹P NMR (121 MHz, CDCl₃) δ 35.60.

### Step 7:

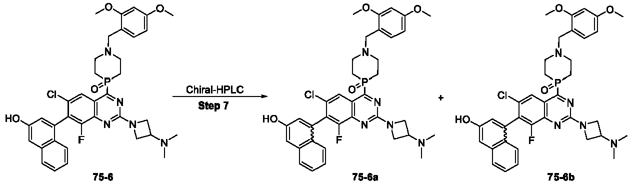

The compound **75-6** (800 mg) obtained in step 6 was subjected to chiral resolution, and the resolution conditions were: chiral column CHIRALPAK IA, 2.12 × 25 cm, 5 µm; mobile phase A: *n*-hexane (10 mmol/L ammonia methanol solution), mobile phase B: ethanol; flow rate: 20 mL/min; elution with 30% mobile phase B in 24 min; detector UV 220/254 nm. Two products were obtained, the product with shorter retention time (7.79 min) was **75-6a,** (*S* or *R*)4-(6-chloro-2-(3-(dimethylamino)azetidin-1-)yl)-8-fluoro-7-(3-hydroxynaphthalen-1-yl)quinazolin-4-yl)-1-(2,4-dimethoxybenzyl)-1,4-azaphosphinane-4-oxide (white solid, 310 mg, recovery rate: 38%); the product with longer retention time (15.77 min) was **75-6b,** (*R* or *S*) 4-(6-chloro-2-(3- (dimethylamino)azetidin-1 -yl)-8-fluoro-7-(3 -hydroxynaphthalen-1 - yl)quinazolin-4-yl)-1-(2,4- dimethoxybenzyl)-1,4-azaphosphinane-4-oxide (white solid, 299 mg, recovery rate: 37%).

### Step 8:

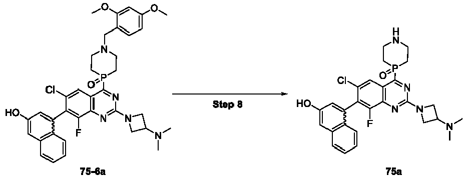

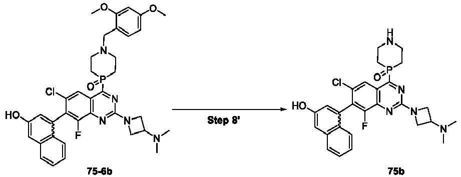

**75-6a** (150 mg, 0.21 mmol, 1.0 eq) was dissolved in 2 mL of trifluoroacetic acid with stirring at room temperature. The reaction was carried out for 1 hour at 80 °C, and the reaction process was monitored by liquid chromatography-mass spectrometry. After the reaction was completed, the reaction mixture was cooled to 25 °C, and concentrated to obtain a crude product. The obtained crude product was purified by reversed-phase chromatographic column (C18 column), and eluted with 40% → 95% acetonitrile/water mobile phase (0.1% ammonium bicarbonate) in 20 min; detector, UV254/220 nm; to obtain compound **75a** (yellow solid, 50 mg, yield: 43%). **75b** (yellow solid, 50 mg, yield: 42%) was obtained by the same method as above.

Compound **75a:** MS (ESI, m/z): 540.2/542.2 [M + H]⁺; ¹H NMR (400 MHz, CD₃OD) *δ* 8.83 (d, *J* = 1.6 Hz, 1H), 8.42 - 8.37 (m, 1H), 7.75 (d, *J* = 8.3 Hz, 1H), 7.43 - 7.39 (m, 1H), 7.27 (d, *J* = 2.4 Hz, 1H), 7.22 - 7.17 (m, 2H), 7.03 (d, *J* = 2.4 Hz, 1H), 4.42 - 4.37 (m, 2H), 4.17 - 4.13 (m, 2H), 3.54 - 3.37 (m, 5H), 2.80 - 2.69 (m, 2H), 2.37 - 2.27 (m, 8H); ¹⁹F NMR (377 MHz, CD₃OD) *δ* -124.36; ³¹P NMR (162 MHz, CD₃OD) *δ* 35.25. The chiral analysis conditions of compound **75a** were: CHIRALPAK IA-3, 4.6 × 50 mm, 3 µm; mobile phase A: n-hexane (0.1% diethylamine), mobile phase B: isopropanol; flow rate: 1 mL/min; isocratic elution with 20% phase B in 17 min; detector UV 254 nm; retention time: 11.543 min; ee > 99%.

Compound **75b:** MS (ESI, m/z): 540.2/542.1 [M + H]⁺; ¹H NMR (400 MHz, CD₃OD) *δ* 8.83 (d, *J* = 1.6 Hz, 1H), 8.41 (s, 1H), 7.75 (d, *J* = 8.3 Hz, 1H), 7.43 - 7.39 (m, 1H), 7.27 (d, *J* = 2.4 Hz, 1H), 7.22 - 7.17 (m, 2H), 7.03 (d, *J* = 2.4 Hz, 1H), 4.42 - 4.37 (m, 2H), 4.17 - 4.14 (m, 2H), 3.55 - 3.39 (m, 5H), 2.80 - 2.70 (m, 2H), 2.39 - 2.27 (m, 8H); ¹⁹F NMR (377 MHz, CD₃OD) *δ* -124.33; ³¹P NMR (162 MHz, CD₃OD) *δ* 35.01. The chiral analysis conditions of compound **75b** were: CHIRALPAK IA-3, 4.6 × 50 mm, 3 µm; mobile phase A: *n*-hexane (0.1% diethylamine), mobile phase B: isopropanol; flow rate: 1 mL/min; isocratic elution with 20% phase B in 17 min; detector UV 254 nm; retention time: 6.706 min; ee > 99%.

### Embodiment 13 (Synthesis method XII)

### (S or R)-2-((6-chloro-2-(3-(dimethylamino)azetidin-1-yl)-8-fluoro-7-(3-hydroxynaphthalen-1-yl)quinazolin-4-yl)amino)acetimidamide diformate 76

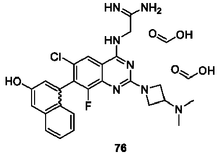

The synthetic route was as follows:

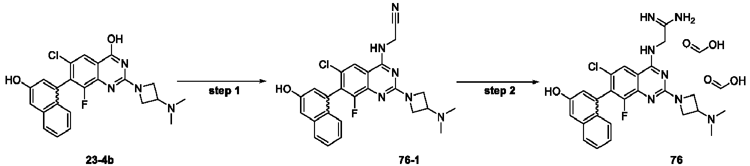

### Step 1:

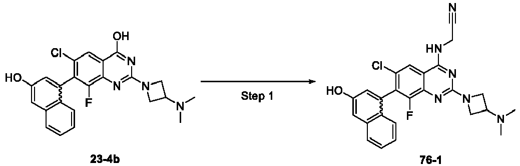

Bromo-tris-pyrrolidino-phosphonium hexafluorophosphate (1.06 g, 2.16 mmol, 2.0 eq) and triethylamine (922 mg, 8.66 mmol, 8.0 eq) were added to a solution of compound **23-4b** (500 mg, 1.08 mmol, 1.0 eq) in *N*-methylpyrrolidone (2.0 mL) with stirring under the protection of nitrogen at 25 °C. The reaction was carried out for 0.5 hours at 25 °C under nitrogen atmosphere. Aminoacetonitrile hydrochloride (158 mg, 1.62 mmol, 1.5 eq) was added to the reaction solution, the reaction was continued for 1.5 hours, and the reaction process was monitored by liquid chromatography-mass spectrometry. After the reaction was completed, the reaction mixture was directly purified by reversed-phase chromatographic column (C18 column), and eluted with a gradient of 5% → 95% (acetonitrile/methanol = 1/ 1)/water (0.1% ammonium bicarbonate) in 25 min; detector, UV254/220 nm; to obtain compound **76-1** (white solid, 90.1 mg, yield: 16%). (ESI, *m*/*z*): 475.1/477.1 [M - H]⁻.

### Step 2:

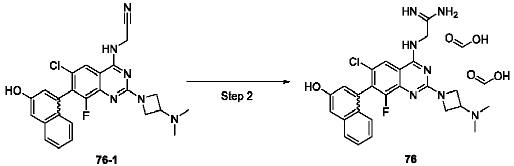

Sodium methoxide (50 mg, 0.28 mmol, 4.0 eq) was added to a solution of compound **76-1** (35 mg, 0.07 mmol, 1.0 eq) in ultra-dry methanol (3 mL) with stirring at 25 °C. The reaction was carried out at this temperature for 8 hours. After the reaction was completed, ammonium chloride (23 mg, 0.41 mmol, 6.0 eq) was added to the reaction solution, and the reaction was continued at 25 °C for 12 hours, then the reaction process was monitored by liquid chromatography-mass spectrometry. After the reaction was completed, the reaction mixture was concentrated to obtain a crude product. The crude product was prepared and purified by high performance liquid chromatography, and the preparation conditions were: (XBridge Prep C18 OBD, 19 × 150 mm, 5 µm; mobile phase A: water (0.1% formic acid), mobile phase B: acetonitrile; flow rate: 25 mL/min; gradient: elution with 1% phase B in 2 min, then eluted with 1% → 9% phase B in 2.5 min, finally eluted with 9% → 34% phase B in 9.5 min, detector: 220 nm; retention time: 7.77 min) and the fraction was concentrated under reduced pressure to obtain compound **76** (white solid, 7.2 mg, yield: 17%). MS (ESI, *m*/*z*): 494.8/496.7 [M + H]⁺; ¹H NMR (400 MHz, CD₃OD) *δ* 8.45 (s, 2H), 8.05 (s, 1H), 7.74 (d, *J* = 8.3 Hz, 1H), 7.42 - 7.38 (m, 1H), 7.25 (d, *J* = 2.4 Hz, 1H), 7.23 - 7.16 (m, 2H), 7.00 (d, *J* = 2.4 Hz, 1H), 4.87 - 4.85 (m, 1H), 4.47 - 4.45 (m, 1H), 4.27 - 4.23 (m, 2H), 4.06 - 3.99 (m, 2H), 3.34 - 3.32 (m, 1H), 2.33 - 2.28 (m, 6H); ¹⁹F NMR (377 MHz, CD₃OD) *δ* -125.21.

### Embodiment 14

### (S)-1-(6-chloro-2-(3-(dimethylamino)azetidin-1-yl)-8-fluoro-7-(3-hydroxynaphthalen-1-yl)quinazolin-4-yl)azetidine-3-carboximidamide 77

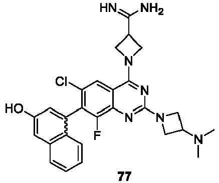

Compound **77** was synthesized according to Embodiment 13 (synthesis method XII). Compound **77** (white solid). MS (ESI, m/z): 520.3/522.3 [M + H]⁺; ¹HNMR (300 MHz, DMSO-*d₆*) *δ* 8.41 (s, 1H), 7.80 (d, *J* = 8.1 Hz, 1H), 7.70 (d, *J* = 1.6 Hz, 1H), 7.46 - 7.41 (m, 1H), 7.27 (d, *J* = 2.4 Hz, 1H), 7.26 - 7.15 (m, 2H), 7.03 (d, *J* = 2.4 Hz, 1H), 4.80 - 4.72 (m, 2H), 4.67 - 4.60 (m, 2H), 4.12 - 4.06 (m, 2H), 3.95 - 3.83 (m, 3H), 3.16 - 3.07 (m, 1H), 2.12 (s, 6H); ¹⁹FNMR (282 MHz, DMSO-*d₆*) *δ* -123.30.

### Embodiment 15 (Synthesis method XIII)

### 4-(4-((1R,5S)-3,8-diazabicyclo[3.2.1]octan-3-yl)-2-(3-(dimethylamino)azetidin-1-yl)-8-fluoropyridine[4,3-d]pyrimidin-7-yl)naphthalen-2-ol 78

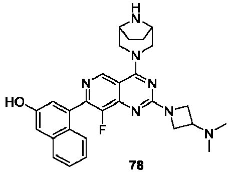

The synthetic route was as follows:

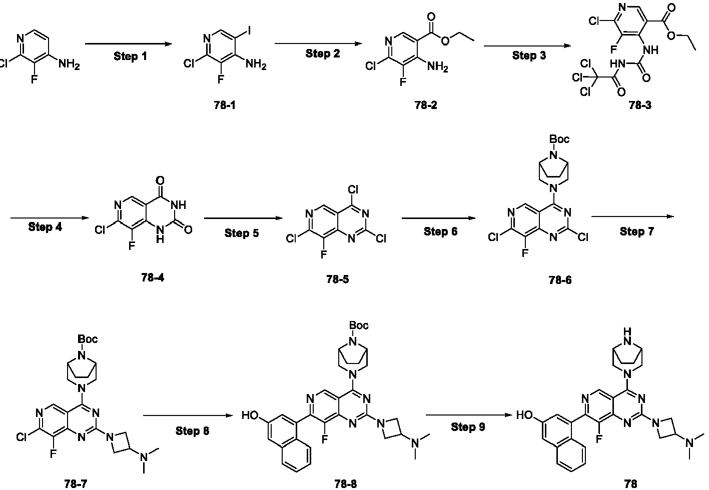

### Step 1:

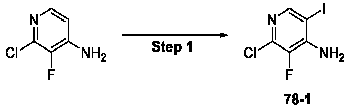

N-iodosuccinimide (1.84 g, 7.77 mmol, 1.2 eq) and p-toluenesulfonic acid (130 mg, 0.65 mmol, 0.1 eq) were added to a solution of 2-chloro-3-fluoro-4-aminopyridine (1 g, 6.82 mmol, 1.0 eq) in acetonitrile (10 mL) with stirring at 25 °C. The reaction was carried out for 16 hours at 70 °C. The reaction process was monitored by liquid chromatography-mass spectrometry. After the reaction was completed, the mixture was cooled to 25 °C, diluted with 30 mL of water, extracted with ethyl acetate (50 mL × 3), and the organic phases were combined. The organic phase was washed successively with 50 mL of saturated sodium carbonate solution, 50 mL of saturated sodium sulfite solution and 50 mL of saturated brine, after washing, the organic phase was dried over anhydrous sodium sulfate, filtered and concentrated to obtain a crude product. The crude product was purified by silica gel column chromatography, eluted with a gradient of 0% → 45% ethyl acetate/petroleum ether mobile phase, and the obtained fractions were evaporated under reduced pressure to obtain compound **78-1** (yellow solid, 1.72 g, yield: 95%). MS (ESI, *m*/*z*): 272.9/274.9 [M + H]⁺; ¹H NMR (400 MHz, CDCl₃) *δ* 8.17 (s, 1H), 4.84 (s, 2H).

### Step 2:

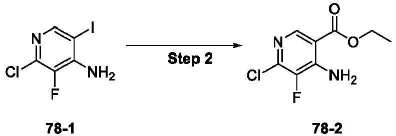

Triethylamine (1.38 g, 12.96 mmol, 3.6 eq) and bis(triphenylphosphine)palladium(II) chloride (266 mg, 0.36 mmol, 0.1 eq) were added to a solution of compound **78-1** (1 g, 3.67 mmol, 1.0 eq) in ethanol (10 mL) with stirring under the protection of nitrogen at 25 °C. The mixture was reacted at 80 °C in carbon monoxide atmosphere (3 atmospheric pressures) for 15 hours, and the reaction process was monitored by liquid chromatography-mass spectrometry. After the reaction was completed, the reaction liquid was cooled to 25 °C, filtered with diatomite, and the filtrate was concentrated under reduced pressure to obtain a crude product. The crude product was purified by silica gel column chromatography, eluted with a gradient of 0% → 31% ethyl acetate/petroleum ether mobile phase, and the obtained fractions were evaporated under reduced pressure to obtain compound **78-2** (yellow solid, 660 mg, yield: 74%). MS (ESI, *m*/*z*): 219.3/221.3 [M + H]⁺; ¹H NMR (300 MHz, CDCl₃) *δ* 8.56 (s, 1H), 4.41 (q, *J* = 7.1 Hz, 2H), 1.43 (t, *J* = 7.1, 3H).

### Step 3:

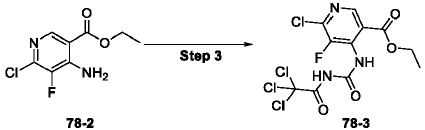

Trichloroacetyl isocyanate (853 mg, 4.30 mmol, 1.5 eq) was added dropwise to a solution of compound **78-2** (660 mg, 2.87 mmol, 1.0 eq) in tetrahydrofuran (6 mL) with stirring under the protection of nitrogen at 25 °C. The reaction was carried out for 20 min at 25 °C, and the reaction process was monitored by liquid chromatography-mass spectrometry. After the reaction was completed, the reaction mixture was concentrated to obtain a crude product. The obtained crude product was slurried with 10 mL of MTBE, filtered, and the filter cake was washed with MTBE (2 mL × 3), and the filter cake was dried to obtain compound **78-3** (white solid, 1.0 g, yield: 77%). MS (ESI, *m*/*z*): 406.0/408.0/410.0 [M + H]⁺; ¹H NMR (400 MHz, CDCl₃) *δ* 11.19 (s, 1H), 8.91 (s, 1H), 8.78 (d, *J* = 0.8 Hz, 1H), 4.48 (q, *J* = 7.1 Hz, 2H), 1.43 (t, *J* = 7.1, 3H).

### Step 4:

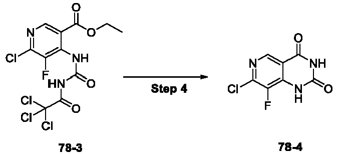

A solution of 7 mol/L ammonia methanol solution (1 mL) was added dropwise to a solution of compound **78-3** (1 g, 2.334 mmol, 1.00 eq) in methanol (10 mL) with stirring at 25 °C. The reaction was carried out at 25 °C for 1 hour. The reaction process was monitored by liquid chromatography-mass spectrometry. After the reaction was completed, the reaction mixture was concentrated to obtain a crude product. The obtained crude product was slurried with 10 mL of MTBE, filtered, and the filter cake was washed with MTBE (2 mL × 3), and the filter cake was dried to obtain compound **78-4** (white solid, 594 mg, yield: 94%). MS (ESI, m/z): 216.1/218.1 [M + H]⁺; ¹H NMR (400 MHz, DMSO-*d*₆) *δ* 8.34 (d, *J* = 1.2 Hz, 1H).

### Step 5:

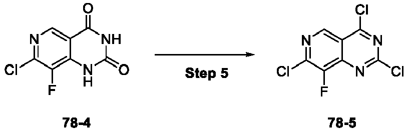

Compound **78-4** (500 mg, 2.20 mmol, 1.0 eq), phosphorus oxychloride (9 mL) and *N,N*-diisopropylethylamine (0.9 mL) were successively added to a dry 100 mL single-neck flask under the protection of nitrogen at 0 °C. The mixture was stirred at 0 °C for 10 min, and then transferred to an oil bath at 90 °C to react under reflux for 12 hours, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 30% ethyl acetate/petroleum ether mobile phase, and the obtained fractions were evaporated under reduced pressure to obtain compound **78-5** (yellow solid, 425 mg, yield: 72%).

### Step 6:

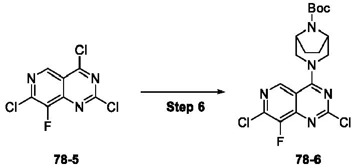

Compound **78-5** (425 mg, 1.68 mmol, 1.0 eq) was dissolved in 5 mL of dichloromethane with stirring under the protection of nitrogen at 25 °C. *N,N-*diisopropylethylamine (652 mg, 4.80 mmol, 3.0 eq) and *tert*-butyl (1*R*, 5*S*)-3,8-diazabicyclo[3.2.1]octane-8-carboxylate (357 mg, 1.60 mmol, 1.0 eq) were successively added to the solution, and then the reaction was carried out at 25 °C for 1 hour, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→30% ethyl acetate/dichloromethane mobile phase, and the obtained fractions were evaporated under reduced pressure to remove the solvent to obtain compound **78-6** (yellow solid, 700 mg, yield: 97%). MS (ESI, *m*/*z*): 428.2/430.2 [M + H]⁺; ¹H NMR (300 MHz, CDCl₃) *δ* 8.86 (d, *J* = 0.6 Hz, 1H), 4.59 - 4.41 (m, 4H), 3.78 - 3.71 (m, 2H), 2.04 - 1.96 (m, 2H), 1.75 - 1.65 (m, 2H), 1.54 (s, 9H).

### Step 7:

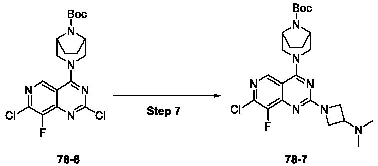

3-(Dimethylamino)azetidine dihydrochloride (311 mg, 1.71 mmol, 1.1 eq) and *N,N-*diisopropylethylamine (1.06 g, 7.76 mmol, 5.0 eq) were added to a solution of compound **78-6** (700 mg, 1.55 mmol, 1.0 eq) in *N*-methylpyrrolidone (7 mL) with stirring under the protection of nitrogen at 25 °C. The reaction was carried out for 3 hours at 60 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), eluted with 5%→90% methanol/water (0.1%) mobile phase in 20 min; detector: UV254/220 nm; the compound **78-7** (yellow solid, 754 mg, yield: 93%) was obtained. MS (ESI, m/z): 492.3/494.3 [M + H]⁺; ¹H NMR (400 MHz, CDCl₃) *δ* 8.51 (s, 1H), 4.38 - 4.06 (m, 8H), 3.60 - 3.51 (m, 2H), 3.29 - 3.22 (m, 1H), 2.28 (s, 6H), 1.96 - 1.89 (m, 2H), 1.76 - 1.71 (m, 2H), 1.51 (s, 9H).

### Step 8:

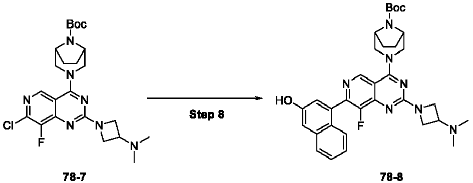

4-(4,4,5,5-Tetramethyl-1,3,2-dioxaborolan-2-yl)naphthalen-2-ol (329 mg, 1.16 mmol, 1.5 eq), cesium carbonate (530 mg, 1.55 mmol, 2.0 eq) and tetrakis(triphenylphosphine)palladium (94 mg, 0.08 mmol, 0.1 eq) were added to a solution of compound **78-7** (400 mg, 0.77 mmol, 1.0 eq) in 1,4-dioxane/water (5/1, 12 mL). The reaction was carried out for 2 hours at 100 °C under nitrogen atmosphere, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **78-8** (red solid, 480 mg, yield: 98%). MS (ESI, m/z): 600.3/602.3 [M + H]⁺; ¹H NMR (400 MHz, CDCl₃) *δ* 8.84 (s, 1H), 7.68 - 7.64 (m, 2H), 7.40 - 7.34 (m, 2H), 7.31 (d, *J* = 2.5 Hz, 1H), 7.24 - 7.20 (m, 1H), 7.18 (d*, J* = 2.5 Hz, 1H), 4.46 - 4.09 (m, 8H), 3.60 - 3.55 (m, 2H), 3.26 - 3.21 (m, 1H), 2.26 (s, 6H), 1.94 - 1.91 (m, 2H), 1.81 - 1.76 (m, 2H), 1.51 (s, 9H).

### Step 9:

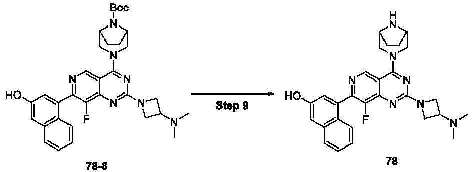

Trifluoroacetic acid (1 mL) was added dropwise to a solution of compound **78-** 8 (100 mg, 0.17 mmol, 1.0 eq) in dichloromethane (3 mL) with stirring at 25 °C. The reaction was carried out for 1 hour at this temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% acetonitrile/water mobile phase (0.1% ammonium bicarbonate) in 30 min; detector, UV254/220 nm; to obtain compound **78** (white solid, 40.5 mg, yield: 50%). MS (ESI, m/z): 500.3/502.2 [M + H]⁺; ¹H NMR (300 MHz, DMSO-*d₆*) *δ* 9.95 (s, 1H), 8.93 (s, 1H), 7.79 (d, *J* = 8.2 Hz, 1H), 7.59 - 7.51 (m, 1H), 7.43 (m, 1H), 7.30 - 7.15 (m, 3H), 4.42 - 4.36 (m, 2H), 4.17 - 4.11 (m, 2H), 3.93 - 3.88 (m, 2H), 3.56 - 3.50 (m, 4H), 3.18 - 3.10 (m, 1H), 2.13 (s, 6H), 1.70 - 1.64 (m, 4H); ¹⁹F NMR (282 MHz, DMSO-*d₆*) *δ* -141.10.

Other similar compounds of the present disclosure can be prepared by the synthetic method shown in Embodiment 15 above. Some compounds prepared with reference to the above synthetic method and their characterization data are shown in table 7.

**Table 7**

| Num ber of the com poun d | Compound structure | Compound name | Chiral analysis conditions /retention time/ee value/spec ific rotation | Mas s spec trum [M+ H]⁺ | ¹H & ¹⁹F NMR |
|---|---|---|---|---|---|
| **128** | 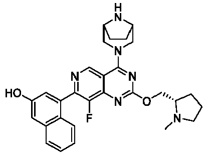 | 4-(4-((1*R*,5*S*)-3,8-diazabicyclo[ 3.2.1]octan-3-yl)-8-fluoro-2-((*S*)-1-methylpyrroli din-2-yl)methoxy)p yridinyl[4,3-*d*]pyrimidin-7-yl)naphthalen -2-ol | | 515. 3 | ¹H NMR (300 MHz, DMSO-*d₆*) *δ* 10.03 (s, 1H), 9.17 (s, 1H), 7.80 (d, *J* = 8.2 Hz, 1H), 7.57 (d, *J* = 8.7 Hz, 1H), 7.47 - 7.41 (m, 1H), 7.30 - 7.22 (m, 3H), 4.49 - 4.38 (m, 3H), 4.24 - 4.18 (m, 1H), 3.64 - 3.59 (m, 2H), 3.56 - 3.54 (m, 2H), 2.99 - 2.93 (m, 1H), 2.64 - 2.56 (m, 1H), 2.37 (s, 3H), 2.23 - 2.14(m, 1H), 1.99 - 1.90 (m, 1H), 1.72 - 1.63(m, 7H); ¹⁹F NMR (282 MHz, DMSO-*d₆*) *δ* -139.79. |
| **129** | 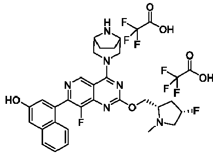 | 4-(4-((1*R*, 5*S*)-3,8-diazabicyclo[ 3.2.1]octan-3-yl)-8-fluoro-2-((2*S*, 4S)-4-fluoro-1-methylpyrroli din-2-yl) methoxy) pyridinyl[4, 3-d]pyrimidin-7-yl) naphthalen-2-ol ditrifluoroace tate | | 533. 2 | ¹H NMR (400 MHz, CD₃OD) *δ* 9.21 (s, 1H), 7.77 (d, *J* = 8.3 Hz, 1H), 7.53 (d, *J* = 8.5 Hz, 1H), 7.46 - 7.42 (m, 1H), 7.31 (d, *J* = 2.6 Hz, 1H), 7.26 - 7.22 (m, 2H), 5.55 - 5.40 (m, 1H), 5.02 -4.98 (dd, *J* = 12.8, 3.2 Hz, 1H), 4.93 - 4.89 (m, 2H), 4.74 (dd, *J* = 12.8, 8.2 Hz, 1H), 4.28 - 4.26 (m, 2H), 4.16 - 4.10 (m, 1H), 4.04 - 3.96 (m, 3H), 3.58 - 3.44 (m, 1H), 3.20 (s, 3H), 2.96 - 2.78 (m, 1H), 2.43 - 2.31 (m, 1H), 2.17 - 2.09 (m, 4H); ¹⁹F NMR (377 MHz, CD₃OD) *δ* - 77.14, -139.38, -173.67. |
| **130** | 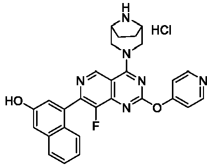 | 4-(4-((1*R*,5*S*)-3,8-diazabicyclo[ 3.2.1]octan-3-yl)-8-fluoro-2-(pyridinyl-4-oxy)pyridyl[4 ,3-d]pyrimidin-7-yl)naphthalen -2-ol hydrochlorid e | | 495. 0 | ¹H NMR (400 MHz, DMSO-*d₆*) *δ* 10.00 - 9.94 (m, 1H), 9.68 (s, 1H), 9.39 (s, 1H), 9.25 - 9.19 (m, 2H), 7.83 (d, *J* = 8.3 Hz, 1H), 7.57 (d, *J* = 8.4 Hz, 1H), 7.48 - 7.44 (m, 1H), 7.34 (d, *J* = 2.4 Hz, 1H), 7.32 - 7.25 (m, 2H), 6.70 - 6.63 (m, 2H), 4.93 - 4.89 (m, 2H), 4.22 - 4.10 (m, 4H), 2.00 - 1.90 (m, 4H); ¹⁹F NMR (377 MHz, DMSO-*d₆*) *δ* - 138.28. |
| **131** | 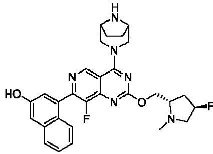 | 4-(4-((1*R*, 5*S*)-3,8-diazabicyclo[ 3.2.1]octan-3-yl)-8-fluoro-2-((2*S*,4*R*)-4-fluoro-1-methylpyrroli din-2-yl)methoxy)p yridinyl[4,3-d]pyrimidin-7-yl)naphthalen -2-ol | | 533. 2 | ¹H NMR (300 MHz, CD₃OD) *δ* 9.22 (s, 1H), 7.79 (d, *J* = 8.3 Hz, 1H), 7.57 - 7.54 (m, 1H), 7.52 - 7.40 (m, 1H), 7.33 (d, *J* = 2.5 Hz, 1H), 7.32 - 7.20 (m, 2H), 5.61 - 5.41 (m, 1H), 5.04 (dd, *J* = 13.1, 2.7 Hz, 1H), 4.95 - 4.92 (m, 2H), 4.80 (dd, *J* = 13.0, 6.2 Hz, 1H), 4.33 - 4.28 (m, 3H), 4.21-4.15 (m, 1H), 4.04 - 3.98 (m, 2H), 3.75 - 3.62 (m, 1H), 3.22 (s, 3H), 2.78 - 2.65 (m, 1H), 2.56 - 2.34 (m, 1H), 2.20 - 2.10 (m, 4H); ¹⁹F NMR (282 MHz, CD₃OD) *δ* -77.17, - 139.37, -174.03. |
| **132** | 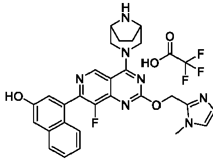 | 4-(4-((1*R*,5*S*)-3,8-diazabicyclo[ 3.2.1]octan-3-yl)-8-fluoro-2-((1-methyl-1*H-*imidazol-2-yl)methoxy)p yridinyl[4,3-d]pyrimidin-7-yl)naphthalen -2-ol trifluoroaceta te | | 512. 2 | ¹H NMR (300 MHz, DMSO-*d₆*) *δ* 10.06 (s, 1H), 9.27 (s, 1H), 7.82 (d, *J* = 8.3 Hz, 1H), 7.55 (d, *J* = 8.4 Hz, 1H), 7.49 - 7.43 (m, 1H), 7.35 - 7.31 (m, 2H), 7.29 - 7.23 (m, 2H), 7.12 (s, 1H), 5.57 (s, 2H), 4.76 - 4.71 (m, 2H), 4.22 - 4.19 (m, 2H), 3.92 - 3.86 (m, 2H), 3.81 (s, 3H), 1.95 - 1.93 (m, 4H); ¹⁹F NMR (282 MHz, DMSO-*d₆*) *δ* - 73.52, -139.10. |
| **133** | 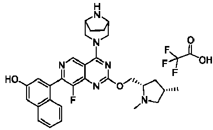 | 4-(4-((1*R*, 5*S*)-3, 8-diazabicyclo[ 3.2.1]octan-3-yl)-2-((2*S*, 4*S*)-1,4-dimethylpyrrolidin-2-yl)methoxy)-8-fluoropyridin yl[4,3-*d*] pyrimidin-7-yl)naphthalen -2-ol trifluoroaceta te | | 529. 2 | ¹H NMR (400 MHz, DMSO-*d₆*) *δ* 10.04 (s, 1H), 9.49 (s, 2H), 9.25 (s, 1H), 7.82 (d, *J* = 8.3 Hz, 1H), 7.55 (d, *J* = 8.4 Hz, 1H), 7.47 - 7.43 (m, 1H), 7.31 (d, *J* = 2.4 Hz, 1H), 7.27 - 7.23 (m, 2H), 4.69 -4.64 (m, 2H), 4.60-4.53 (m, 2H), 4.13 - 4.11 (m, 2H), 3.86 - 3.82 (m, 2H), 2.92 - 2.86 (m, 2H), 2.68 -2.63 (m, 3H), 2.39-2.26 (m, 3H), 1.94 - 1.88 (m, 4H), 1.42 - 1.34 (m, 1H), 1.05 (d, *J* = 6.5 Hz, 3H); ¹⁹F NMR (377 MHz, DMSO-*d₆*) *δ* -73.47, - 139.38. |
| **134** | 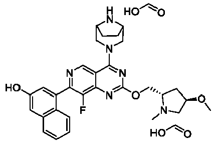 | 4-(4-((1*R*,5*S*)-3,8-diazabicyclo[ 3.2.1]octan-3-yl)-8-fluoro-2-((2*S*, 4*R*)-4-methxoy-1-methylpyrroli din-2-yl)methoxy)p yridinyl[4,3-*d*]pyrimidin-7-yl)naphthalen -2-ol diformate | | 545. 3 | ¹H NMR (300 MHz, DMSO-*d₆*) *δ* 9.19 (s, 1H), 8.23 (s, 2H), 7.81 (d, *J* = 8.2 Hz, 1H), 7.56 (d, *J* = 8.4 Hz, 1H), 7.47 - 7.42 (m, 1H), 7.30 (d, *J* = 2.4 Hz, 1H), 7.28 - 7.22 (m, 2H), 4.55 - 4.50 (m, 2H), 4.42 (dd, *J* = 11.0, 4.7 Hz, 1H), 4.29 (dd, *J* = 11.0, 5.7 Hz, 1H), 3.91 - 3.84 (m, 1H), 3.74 - 3.66 (m, 4H), 3.34 - 3.28 (m, 1H), 3.20 (s, 3H), 2.83 - 1.73 (m, 1H), 2.36 (s, 3H), 2.22 - 2.17 (m, 1H), 1.97 - 1.82 (m, 2H), 1.77 - 1.70 (m, 4H); ¹⁹F NMR (282 MHz, DMSO-*d₆*) *δ* -139.69. |

Other similar compounds of the present disclosure was obtained by the synthesis method according to Embodiment **15,** wherein 5 eq of *N,N*-diisopropylethylamine in step 7 were replaced with 1.2 eq of potassium *tert*-butoxide and the reaction was carried out at 0 °C for 1 hour, then purified to obtain parts of the compounds, and their characterization data are shown in table 8

**Table 8**

| Num ber of the com poun d | Compound structure | Compound name | Chiral analysis condition s/retentio n time/ee value/spe cific rotation | Mas s spec trum [M+ H]⁺ | ¹H & ¹⁹F NMR |
|---|---|---|---|---|---|
| **135** | 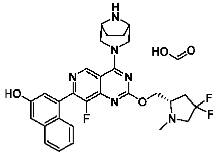 | 4-(4-((1*R*,5*S*)-3,8-diazabicycl o[3.2.1]octa n-3-yl)-2-((*S*)-4,4-difluoro-1-methylpyrro lidin-2-yl)methoxy) -8-fluoropyridinyl[4,3-*d*]pyrimidin -7-yl)naphthale n-2-ol formate | | 551. 2 | ¹H NMR (300 MHz, DMSO-*d₆*) *δ* 9.99 (s, 1H), 9.21 (s, 1H), 8.17 (s, 1H), 7.81 (d, *J* = 8.3 Hz, 1H), 7.56 (d, *J* = 8.4 Hz, 1H), 7.48 - 7.42 (m, 1H), 7.30 (d, *J* = 2.4 Hz, 1H), 7.28 - 7.23 (m, 2H), 4.59 - 4.40 (m, 4H), 3.82 - 3.80 (m, 2H), 3.74 - 3.69 (m, 2H), 3.42 - 3.32 (m, 2H), 3.02 - 2.93 (m, 1H), 2.75 - 2.63 (m, 1H), 2.38 (s, 3H), 2.33 - 2.13 (m, 1H), 1.78 - 1.75 (m, 4H); ¹⁹F NMR (282 MHz, DMSO-*d₆*) *δ* -89.40, -90.20, -95.01, - 95.81, -139.60. |
| **136** | 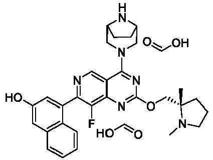 | 4-(4-((1*R*,5*S*)-3,8-diazabicycl o[3.2.1]octa n-3-yl)-2-((*S*)-1,2-methylpyrro lidin-2-yl)methoxy) -8-fluoropyridi nyl[4,3-*d*] pyrimidin-7-yl)naphthale n-2-ol diformate | | 529. 2 | ¹H NMR (300 MHz, CD₃OD) *δ* 9.19 (s, 1H), 8.52 (s, 2H), 7.79 (d, *J* = 8.2 Hz, 1H), 7.55 (d, *J* = 8.5 Hz, 1H), 7.48 - 7.43 (m, 1H), 7.32 (d, *J* = 2.5 Hz, 1H), 7.29 - 7.23 (m, 2H), 4.82 - 4.63 (m, 4H), 4.00 - 3.95 (m, 2H), 3.91 - 3.85 (m, 2H), 3.67 - 3.57 (m, 1H), 3.40 - 3.34 (m, 1H), 2.91 (s, 3H), 2.39 - 2.29 (m, 1H), 2.19 - 2.10 (m, 3H), 2.08 - 1.94 (m, 4H), 1.52 (s, 3H); ¹⁹F NMR (282 MHz, CD₃OD) *δ* -139.52. |
| **137** | 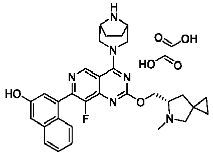 | 4-(4-((1*R*,5*S*)-3,8-diazabicycl o[3.2.1]octan-3-yl)-8-fluoro-2-((*S*)-5-methyl-5-azaspiro[2.4 ]heptan-6-yl)methoxy) pyridin[4,3-*d*]pyrimidin -7-yl)naphthale n-2-ol diformate | | 541. 2 | ¹H NMR (400 MHz, DMSO-*d₆*) *δ* 9.19 (s, 1H), 8.22 (s, 2H), 7.80 (d, *J* = 8.3 Hz, 1H), 7.56 (d, *J* = 8.4 Hz, 1H), 7.46 - 7.42 (m, 1H), 7.29 (d, *J* = 2.4 Hz, 1H), 7.27 - 7.22 (m, 2H), 4.54 - 4.47 (m, 3H), 4.32 (dd, *J* = 10.9, 6.2 Hz, 1H), 3.75 - 3.68 (m, 4H), 2.93 - 2.86 (m, 1H), 2.68 - 2.65 (m, 1H), 2.54 - 2.52 (m, 1H), 2.39 (s, 3H), 2.02 (dd, *J* = 12.6, 7.9 Hz, 1H), 1.76 - 1.71 (m, 4H), 1.64 (dd, *J* = 12.6, 7.9 Hz, 1H), 0.59 - 0.45 (m, 4H); ¹⁹F NMR (377 MHz, DMSO-d₆) *δ* -139.68. |
| **138** | 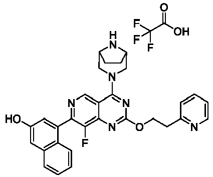 | 4-(4-((1*R*,5*S*)-3,8-diazabicycl o[3.2.1]octa n-3-yl)-8-fluoro-2-(2-(pyridin-2-yl) ethoxy)pyri dyl[4,3-*d*]pyrimidin -7-yl)naphthale n-2-ol trifluoroacetate | | 523. 2 | ¹H NMR (300 MHz, DMSO-*d₆*) *δ* 10.04 (s, 1H), 9.34 (s, 1H), 9.24 (s, 1H), 9.14 (s, 1H), 8.65 (d, J = 4.9 Hz, 1H), 8.06 - 8.00 (m, 1H), 7.82 (d, *J* = 8.3 Hz, 1H), 7.65 (d, *J* = 7.7 Hz, 1H), 7.57 - 7.42 (m, 3H), 7.31 (d, *J* = 2.5 Hz, 1H), 7.29 - 7.22 (m, 2H), 4.83 (t, *J* = 6.4 Hz, 2H), 4.70 - 4.65 (m, 2H), 4.21 - 4.18 (m, 2H), 3.89 - 3.83 (m, 2H), 3.36 (t, *J* = 6.4 Hz, 2H), 1.97 - 1.92 (m, 4H); ¹⁹F NMR (282 MHz, DMSO-*d₆*) *δ* -74.24, -139.34. |
| **139** | 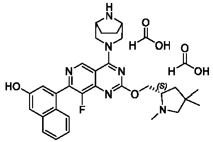 | 4-(4-((1*R*,5*S*)-3,8-diazabicycl o[3.2.1]octa n-3-yl)-8-fluoro-2-((*S*)-1,4,4-trimethylpyr rolidin-2-yl)methoxy) pyridinyl[4, 3-*d*]pyrimidin -7-yl)naphthale n-2-ol diformate | | 543. 2 | ¹H NMR (400 MHz, DMSO-*d₆*) *δ* 9.18 (s, 1H), 8.22 (s, 2H), 7.80 (d, *J* = 8.2 Hz, 1H), 7.56 (d, *J* = 8.5 Hz, 1H), 7.46 - 7.42 (m, 1H), 7.29 (d, *J* = 2.4 Hz, 1H), 7.27 - 7.22 (m, 2H), 4.54 - 4.50 (m, 2H), 4.44 (dd, *J* = 10.9, 5.0 Hz, 1H), 4.29 (dd, *J* = 10.9, 6.1 Hz, 1H), 3.74 - 3.67 (m, 4H), 2.76 - 2.69 (m, 2H), 2.35 (s, 3H), 2.12 - 2.10 (m, 1H), 1.84 - 1.78 (m, 1H), 1.75 - 1.70 (m, 4H), 1.51 - 1.45 (m, 1H), 1.10 (s, 3H), 1.03 (s, 3H); ¹⁹F NMR (377 MHz, DMSO-*d₆*) *δ* -139.69. |
| **140** | 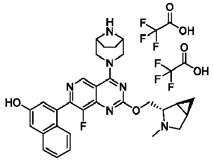 | 4-(4-((1*R*,5*S*)-3,8-diazabicycl o[3.2.1]octa n-3-yl)-8-fluoro-2-((1*S,*2*S,*5*R*)-3-methyl-3-azabicyclo[3.1.0]hexan -2-yl)methoxy) pyridin[4,3-*d*]pyrimidin -7-yl)naphthale n-2-ol dihydrochlo ride | | 527. 3 | ¹H NMR (400 MHz, DMSO-*d₆*) *δ* 10.47 (s, 1H), 10.06 - 9.93 (m, 1H), 9.39 (s, 1H), 9.29 (d, *J* = 2.7 Hz, 1H), 9.16 (s, 1H), 7.82 (d, *J* = 8.3 Hz, 1H), 7.55 - 7.53 (m, 1H), 7.48 - 7.44 (m, 1H), 7.32 (d, *J* = 2.4 Hz, 1H), 7.28 - 7.23 (m, 2H), 4.92 - 4.70 (m, 4H), 4.23 - 4.21 (m, 2H), 4.09 - 3.87 (m, 3H), 3.51 - 3.40 (m, 1H), 2.91 - 2.83 (m, 4H), 1.98 - 1.91 (m, 5H), 1.86 - 1.82 (m, 1H), 1.20 - 1.05 (m, 1H), 0.82 - 0.75 (m, 1H); ¹⁹F NMR (377 MHz, DMSO-*d₆*) *δ* -73.94, -139.14. |
| **141** | 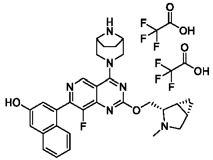 | 4-(4-((1*R*,5*S*)-3,8-diazabicycl o[3.2.1]octa n-3-yl)-8-fluoro-2-((1*R*,2*S*,5*R*) -3-methyl-3-azabicyclo[ 3.1.0]hexan -2-yl)methoxy) pyridin[4,3-*d*]pyrimidin -7-yl)naphthale n-2-oldihydrochlo ride | | 527. 2 | ¹H NMR (400 MHz, DMSO-*d₆*) *δ* 9.97 (s, 2H), 9.55 - 9.48 (m, 1H), 9.34 - 9.27 (m, 2H), 7.82 (d, *J* = 8.2 Hz, 1H), 7.55 (d, *J* = 8.4 Hz, 1H), 7.48 - 7.44 (m, 1H), 7.32 (d, *J* = 2.4 Hz, 1H), 7.29 - 7.21 (m, 2H), 4.83 (dd, *J* = 12.2, 4.6 Hz, 1H), 4.76 - 4.70 (m, 3H), 4.23 - 4.16 (m, 3H), 3.94 - 3.88 (m, 2H), 3.68 - 3.65 (m, 1H), 3.46 - 3.41 (m, 1H), 2.99 (s, 3H), 1.98 - 1.90 (m, 5H), 1.82 - 1.76 (m, 1H), 0.81 - 0.77 (m, 1H), 0.74 - 0.67 (m, 1H); ¹⁹F NMR (377 MHz, DMSO-*d₆*) *δ* -74.12, - 139.19. |
| **142** | 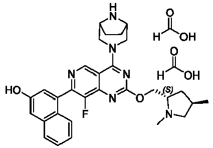 | 4-(4-((1*R*, 5S)-3,8-diazabicycl o[3.2.1]octa n-3-yl)-2-((2*S*,4*R*)-1,4-methylpyrro lidin-2-yl)methoxy) -8-fluoropyridi nyl[4,3-*d*]pyrimidin -7-yl)naphthale n-2-ol diformate | | 529. 3 | ¹H NMR (400 MHz, DMSO-*d₆*) *δ* 9.19 (s, 1H), 8.18 - 8.16 (m, 2H), 7.80 (d, *J* = 8.3 Hz, 1H), 7.56 (d, *J* = 8.4 Hz, 1H), 7.46 - 7.42 (m, 1H), 7.29 (d, *J* = 2.4 Hz, 1H), 7.27 - 7.22 (m, 2H), 4.56 - 4.51 (m, 2H), 4.39 (dd, *J* = 10.8, 4.9 Hz, 1H), 4.23 (dd, *J* = 10.8, 6.3 Hz, 1H), 3.79 - 3.76 (m, 2H), 3.73 - 3.68 (m, 2H), 3.08 - 3.04 (m, 1H), 2.82 - 2.77 (m, 1H), 2.38 (s, 3H), 2.21 - 2.14 (m, 1H), 1.96 - 1.82 (m, 2H), 1.76 - 1.74 (m, 4H), 1.59 - 1.52 (m, 1H), 0.97 (d, *J* = 6.6 Hz, 3H); ¹⁹F NMR (377 MHz, DMSO-*d₆*) *δ* -139.67. |

### Embodiment 16 (Synthesis method XIV)

### 4-((R or S)-4-((1R,5S)-3,8-diazabicyclo[3.2.1]octan-3-yl)-6,8-dichloro-2-(3-(dimethylamino)azetidin-1-yl)quinazolin-7-yl)naphthalen-2-ol 79a; 4-((S or R)-4-((1R,5S)-3,8-diazabicyclo [3.2.1] octan-3 -yl)-6,8-dichloro-2 -(3 -(dimethylamino)azetidin-1 - yl)quinazolin-7-yl)naphthalen-2-ol 79b

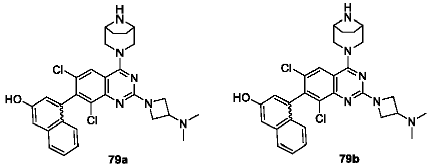

The synthetic route was as follows:

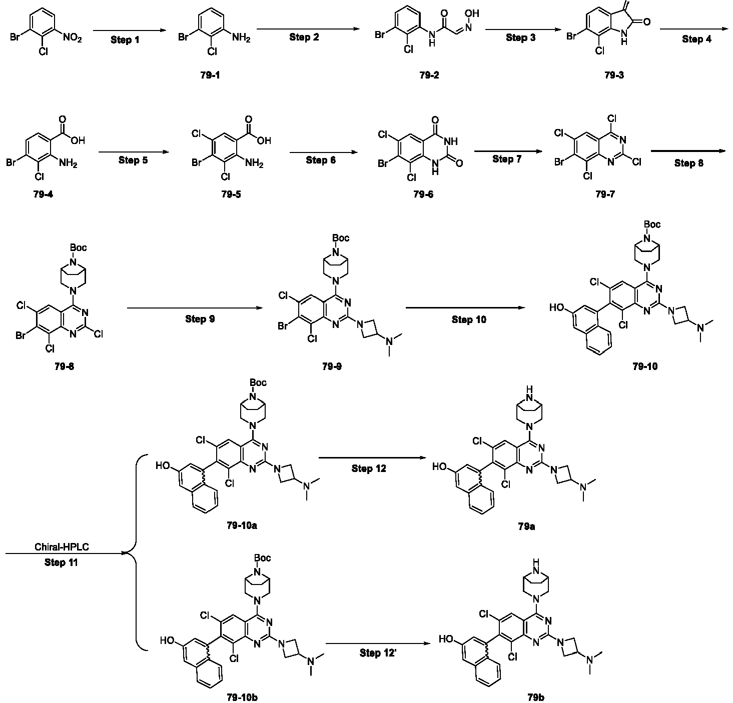

### Step 1:

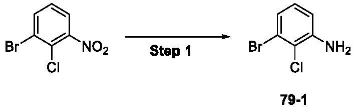

1- Bromo-2-chloro-3-nitrobenzene (25 g, 100.00 mmol, 1.0 eq), ethanol (160 mL), water (40.00 mL), iron powder (29.52 g, 502.20 mmol, 5.0 eq) and ammonium chloride (28 g, 518.30 mmol, 5.1 eq) were added successively to a 250 mL round bottom flask at 25 °C. The reaction was carried out with stirring for 17 hours at 25 °C, and the reaction process was monitored by liquid chromatography-mass spectrometry. After the reaction was completed, the reaction solution was filtered with a Buchner funnel, and the filter cake was washed with ethyl acetate (100 mL × 3), and the filtrate was concentrated to obtain a crude product. The crude product was purified by silica gel column chromatography, and the mobile phase was eluted with a gradient of 0% → 10% ethyl acetate/petroleum ether mobile phase, and the obtained fractions were evaporated under reduced pressure to remove the solvent to obtain compound **79-1** (orange oil, 12.57 g, yield: 60%). MS (ESI, *m*/*z*): 206.1/208.0 [M + H]⁺; ¹H NMR (300 MHz, CDCl₃) *δ* 7.03 (dd, *J=* 8.0, 1.5 Hz, 1H), 6.93 (t, *J=* 8.0 Hz, 1H), 6.72 (dd, *J=* 8.0, 1.5 Hz, 1H), 4.19 (s, 2H).

### Step 2:

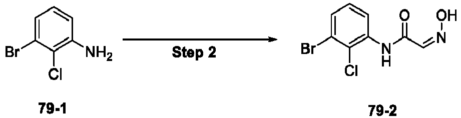

Chloral hydrate (10.57 g, 60.70 mmol, 1.1 eq), sodium sulfate (107.32 g, 717.80 mmol, 13.0 eq), sulfuric acid (0.1 mol/L, 0.10 mL) and hydroxylamine hydrochloride (12.12 g, 165.60 mmol, 3.0 eq) were added to a solution of compound **79-1** (12.0 g, 55.20 mmol, 1.0 eq) in water (252 mL) with stirring at 25 °C. The reaction was carried out for 16 hours at 70 °C, and the reaction process was monitored by liquid chromatography-mass spectrometry. After the reaction was completed, the reaction solution was cooled to 25 °C, filtered, and the filter cake was washed with water (100 mL x 3), and the filtrate was concentrated to obtain a crude product of **79-2** (25 g), which was directly used in the next step without further purification. MS (ESI, m/z): 274.8/276.8/278.7 [M - H]⁻.

### Step 3:

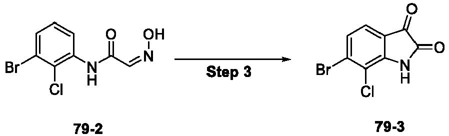

**79-2** (25g) and concentrated sulfuric acid (230 mL) were added to a 500 mL round bottom flask at 25 °C. The reaction was carried out for 16 hours at 70 °C. After the reaction was completed, the reaction solution was cooled to 25 °C, filtered, and the filter cake was washed with water (1000 mL x 3), and the filter cake was concentrated to obtain a crude product of compound **79-3** (10 g), which was directly used in the next step without further purification. MS (ESI, *m*/*z*): 257.8/259.8/261.6 [M - H]⁻.

### Step 4:

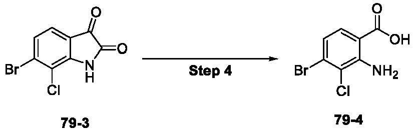

**79-3** (10 g), sodium hydroxide aqueous solution (2 mol/L, 110 mL) and 30% hydrogen peroxide (20 mL) were added to a 250 mL round bottom flask at 25 °C. The reaction was carried out for 16 hours at 25 °C. After the reaction was completed, the reaction solution was poured into 100 mL of water, and the pH value was adjusted to 1 with hydrochloric acid (6 mol/L), and after no significant solids were precipitated, the mixture was filtered, and the filter cake was washed with water (100 mL x 3), and dried to obtain a crude product of compound **79-4** (9.5 g), which was directly used in the next step without further purification. MS (ESI, *m*/*z*): 250.1/252.1/254.1 [M + H]⁺.

### Step 5:

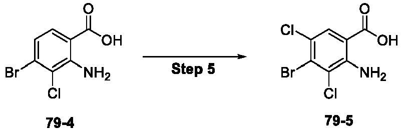

*N*-chlorosuccinimide (2.9 g) was added to a solution of compound **79-4** (9 g) in *N,N-*dimethylformamide (200.00 mL) with stirring at 25 °C. The mixture was stirred at 25°C for 16 hours. After the reaction was completed, the reaction solution was poured into 100 mL water, filtered, and the filter cake was washed with water (100 mL x 3), and the filter cake was concentrated to obtain a crude product of compound **79-5** (6.8 g), which was directly used in the next step without further purification. MS (ESI, m/z): 283.9/285.9/287.9 [M + H]⁺.

### Step 6:

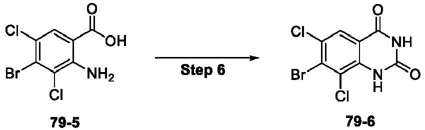

**79-5** (3g) and urea (4g) were added to a 25 mL round bottom flask at 25 °C. The reaction was carried out at 150 °C for 8 hours. After the reaction was completed, the mixture was cooled to 25°C and diluted with 100 mL of water, filtered, and the filter cake was washed with water (3 x 50 mL), then dried to obtain a crude compound of **79-6** (2 g, crude product). The crude product was used directly in the next step without further purification. MS (ESI, *m*/*z*): 306.9/308.9/310.9 [M - H]⁻.

### Step 7:

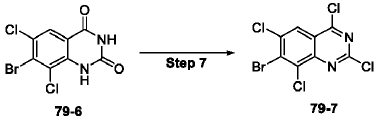

*N*,*N*-diisopropylethylamine (2.6 mL) was slowly added dropwise to a solution of compound **79-6** (1.8 g) in phosphorus oxychloride (26.0 mL) with stirring under the protection of nitrogen at 0 °C. The reaction was carried out for 12 hours at 90 °C. After the reaction was completed, the reaction mixture was cooled to 25 °C, and concentrated under reduced pressure. Dichloromethane (50 mL) was added and the residual phosphorus oxychloride was removed by concentration, this operation was repeated three times to obtain a crude product. The crude product was purified by silica gel column chromatography, eluted with a gradient of 0% → 10% ethyl acetate/petroleum ether mobile phase, and the obtained fractions were evaporated under reduced pressure to obtain compound **79-7** (900 mg, light yellow solid).

### Step 8:

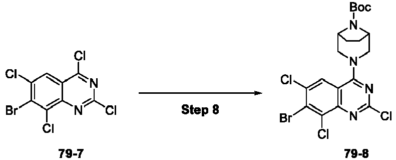

**79-7** (450 mg, 2.00 mmol, 1.0 eq), 1,4-dioxane (4.0 mL), *tert*-butyl 3,8-diazabicyclo[3.2.1]octane-8-carboxylate (276 mg, 2.00 mmol, 1.0 eq) and triethylamine (394 mg, 6.00 mmol, 3.0 eq) were added to a 25 mL Schlenk tube under the protection of nitrogen at 25 °C. The reaction was carried out for 2 hours at 25 °C under nitrogen atmosphere, and the reaction process was monitored by liquid chromatography-mass spectrometry. After the reaction was completed, the reaction mixture was concentrated under reduced pressure to obtain a crude product. The crude product was purified by silica gel column chromatography, eluted with a gradient of 5% → 30% ethyl acetate/petroleum ether mobile phase to obtain compound **79-8** (yellow solid, 640 mg, yield: 94%). MS (ESI, *m*/*z):* 521.0/523.0/525.0 [M + H]⁺; ¹HNMR (300 MHz, CDCl₃) *δ*7.86 (s, 1H), 4.46 - 4.33 (m, 4H), 3.70 - 3.64 (m, 2H), 1.99 - 1.95 (m, 2H), 1.76 - 1.70 (m, 2H), 1.54 (s, 9H).

### Step 9:

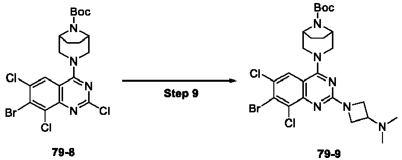

Compound **79-8** (350 mg, 0.83 mmol, 1.0 eq), *N*-methylpyrrolidone (4 mL), 3-(dimethylamino)azetidine dihydrochloride (96 mg, 0.96 mmol, 1.5 eq) and *N,N-*diisopropylethylamine (822 mg, 6.36 mmol, 10.0 eq) were added to a 25 mL Schlenk tube under the protection of nitrogen at 25 °C. The reaction was carried out for 2 hours at 60 °C under nitrogen atmosphere, and the reaction process was monitored by liquid chromatography-mass spectrometry. After the reaction was completed, the reaction mixture was cooled to 25 °C, and concentrated to obtain a crude product. The obtained crude product was purified by reversed-phase chromatographic column (C18 column), and eluted with 10% → 95% acetonitrile/water mobile phase (0.5% ammonium bicarbonate) in 20 min; detector, UV254/220 nm; to obtain compound **79-9** (yellow solid, 320 mg, yield: 82%). MS (ESI, *m*/*z*): 585.3/587.3 [M + H]⁺; ¹HNMR (300 MHz, CDCl₃) *δ* 7.67 (s, 1H), 4.34 - 4.17 (m, 6H), 4.09 - 4.04 (m, 2H), 3.56 - 3.45 (m, 2H), 3.29 - 3.20 (m, 1H), 2.28 (s, 6H), 1.95 - 1.77 (m, 4H), 1.53 (s, 9H).

### Step 10:

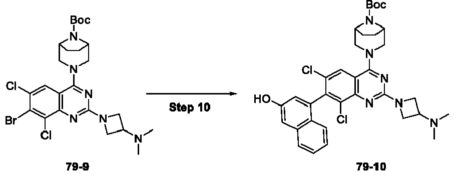

Potassium phosphate (217 mg, 0.98 mmol, 2.0 eq) and 4-(4,4,5,5-tetramethyl-1,3,2-dioxaborolan-2-yl)naphthalen-2-ol (207 mg, 0.73 mmol, 1.5 eq) and chloro(2-dicyclohexylphosphino-2',4',6'-triisoporpyl-1,1'-biphenyl) [2-(2'-amino-1,1'-biphenyl)] palladium (II) (40 mg, 0.05 mmol, 0.1 eq) were successively added to a solution of compound **79-9** (300 mg, 0.49 mmol, 1.0 eq) in tetrahydrofuran/water (10/1, 4 mL) at 25 °C with stirring under the protection of nitrogen. The reaction was carried out for 1 hour at 60 °C under nitrogen atmosphere, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%--+ 15% methanol/dichloromethane mobile phase, and the obtained fractions were evaporated under reduced pressure to remove the solvent to obtain compound **79-10** (a racemic mixture of two stereoisomers, yellow solid, 310 mg, yield: 93%). MS (ESI, *m*/*z*): 649.3/651.4 [M + H]⁺; ¹HNMR (300 MHz, CDCl₃) *δ* 7.76 - 7.74 (m, 2H), 7.46 - 7.40 (m, 1H), 7.28 - 7.18 (m, 3H), 6.96 (d, *J=* 2.5 Hz, 1H), 4.38 - 4.11 (m, 8H), 3.59 - 3.47 (m, 2H), 3.38 - 3.31(m, 1H), 2.34 (s, 6H), 1.98 - 1.84 (m, 4H), 1.54 (s, 9H).

### Step 11:

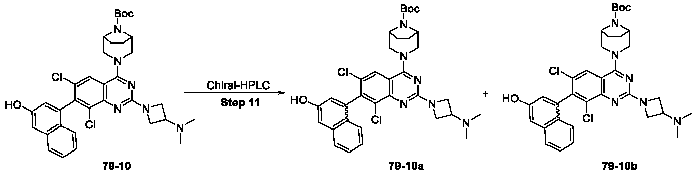

The compound **79-10** (310 mg) obtained in step 10 was subjected to chiral resolution, and the resolution conditions were: chiral column CHIRALPAK IA, 2 x 25 cm, 5 µm; mobile phase A: *n*-hexane (10 mmol/L ammonia methanol solution), mobile phase B: isopropanol; flow rate: 20 mL/min; gradient: elution with 30% mobile phase B in 11.5 min; detector UV 220/210 nm; two products were obtained. The product with shorter retention time (4.342 min) was **79-10a,** *tert*-butyl(1*R*,5*S*)-3-((*R* or *S*)-6,8-dichloro-2-(3-(dimethylamino)azetidin-1-yl)-7-(3-hydroxynaphthalen-1-yl)quinazolin-4-yl)-3,8-diazabicyclo[3.2.1]octane-8-carboxylate (yellow solid, 100 mg, recovery rate: 33%); the product with longer retention time (7.54 min) was **79-10b,** *tert*-butyl(1*R*,5*S*)-3-((*S* or *R*)-6,8-dichloro-2-(3-(dimethylamino)azetidin-1-yl)-7-(3-hydroxynaphthalen-1-yl)quinazolin-4-yl)-3,8-diazabicyclo[3.2.1]octane-8-carboxylate (yellow solid, 105 mg, recovery rate: 35%).

### Step 12:

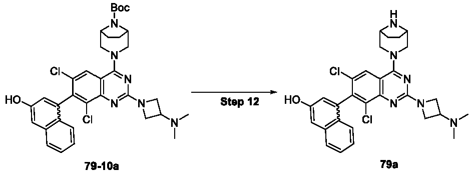

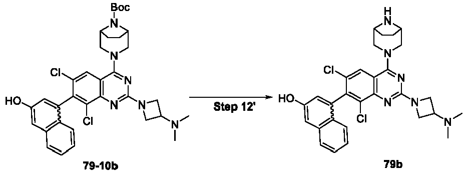

Trifluoroacetic acid (1.00 mL) was added dropwise to a solution of compound **79-10a** (100 mg) in dichloromethane (4.0 mL) with stirring at 25 °C. The reaction was carried out for 0.5 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 35% acetonitrile/water (0.5% formic acid) mobile phase in 20 min; detector, 254/220 nm; to obtain compound **79a** (white solid, 30 mg, yield: 52%). Compound **79b** (white solid, 20 mg, yield: 33%) can be obtained by the same method as above.

Compound **79a:** MS (ESI, m/z): 549.20/551.20 [M + H]⁺; ¹HNMR (300 MHz, DMSO-*d₆*) *δ* 9.95 (s, 1H), 7.86 (s, 1H), 7.78 (d, *J=* 8.3 Hz, 1H), 7.44 - 7.38 (m, 1H), 7.23 (d, *J=* 2.4 Hz, 1H), 7.22 - 7.10 (m, 2H), 6.94 (d, *J=* 2.4 Hz, 1H), 4.26 - 4.19 (m, 2H), 4.14 - 4.08 (m, 2H), 3.89 - 3.84 (m, 2H), 3.50 - 3.43 (m, 4H), 3.16 - 3.09 (m, 1H), 2.12 (s, 6H), 1.74 - 1.62 (m, 4H).

Compound **79b:** MS (ESI, m/z): 549.20/551.20 [M + H]⁺; ¹HNMR (300 MHz, DMSO-*d₆*) *δ* 9.95 (s, 1H), 7.86 (s, 1H), 7.78 (d, *J=* 8.3 Hz, 1H), 7.44 - 7.38 (m, 1H), 7.23 (d, *J=* 2.4 Hz, 1H), 7.22 - 7.10 (m, 2H), 6.94 (d, *J=* 2.4 Hz, 1H), 4.26 - 4.19 (m, 2H), 4.14 - 4.08 (m, 2H), 3.89 - 3.84(m, 2H), 3.50 - 3.43 (m, 4H), 3.16 - 3.09 (m, 1H), 2.12 (s, 6H), 1.74 - 1.62 (m, 4H).

### Embodiment 17 (Synthesis method XV)

### 4-(4-((1R,5S)-3,8-diazabicyclo[3.2.1]octan-3-yl)-6-chloro-2-(3-(dimethylamino)azetidin-1-yl)pyrido[2,3-d]pyrimidin-7-yl)naphthalen-2-ol 80

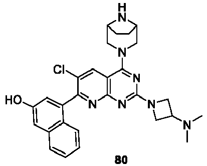

The synthetic route was as follows:

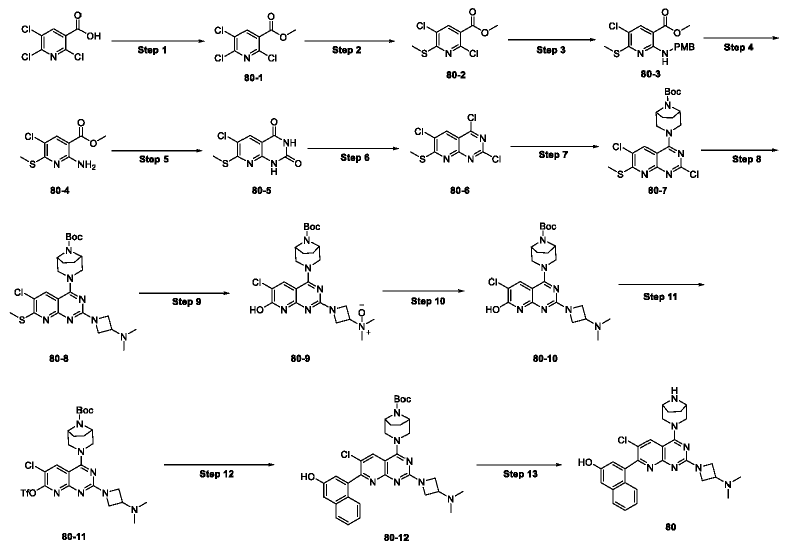

### Step 1:

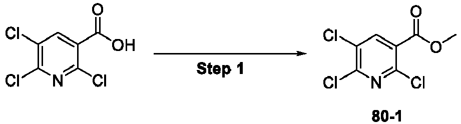

Thionyl chloride (19.47 g, 163.62 mmol, 2.0 eq) was added dropwise to a solution of 2,5,6-trichloronicotinic acid (19.5 g, 81.81 mmol, 1.0 eq) in methanol (190.0 mL) with stirring under the protection of nitrogen at 0 °C. After the dropwise addition, the mixture was heated to 60 °C and the reaction was carried out at this temperature for 16 hours, and the reaction process was monitored by liquid chromatography-mass spectrometry. After the reaction was completed, the mixture was cooled to room temperature, then evaporated to dryness to obtain a crude product of compound **80-1** (white solid, 20.5 g, yield: 99%). The crude product was used directly in the next step without further purification. MS (ESI, m/z): 240.0 /242.0 [M + H]⁺; ¹H NMR (300 MHz, CDCl₃) *δ* 8.28 (s, 1H), 3.98 (s, 3H).

### Step 2:

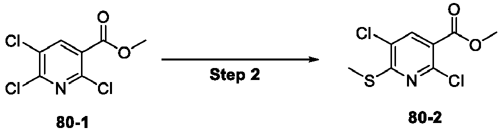

Water (120 mL) and sodium methyl mercaptan (3.32 g, 47.41 mmol, 1.00 eq) were added to a solution of **80-1** (12 g, 47.41 mmol, 1.0 eq) in tetrahydrofuran (120 mL) under the protection of nitrogen with stirring at 0 °C. The reaction was carried out for 2 hours at 25 °C, and the reaction process was monitored by liquid chromatography-mass spectrometry. After the reaction was completed, 500 mL of water was added to the reaction solution for dilution, and the mixture was extracted with ethyl acetate (500 mL x 3), then the organic phases were combined, dried over anhydrous sodium sulfate, filtered, and the filtrate was concentrated to obtain a crude product. The crude product was purified by silica gel column chromatography, eluted with a gradient of 0% → 60% ethyl acetate/petroleum ether mobile phase, and the obtained fractions were evaporated under reduced pressure to remove the solvent to obtain compound **80-2** (white solid, 12 g, yield: 91%). MS (ESI, m/z): 252.0/254.0 [M + H]⁺; ¹H NMR (300 MHz, CDCl₃) *δ* 8.06 (s, 1H), 3.95 (s, 3H), 2.62 (s, 3H).

### Step 3:

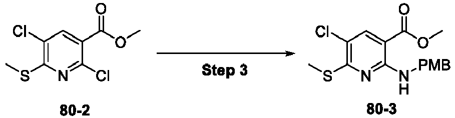

4-Methoxybenzylamine (9.19 g, 67.82 mmol, 1.5 eq) and *N*,*N*-diisopropylethylamine (12.30 g, 90.44 mmol, 2.0 eq) were added to a solution of **80-2** (12 g, 45.22 mmol, 1.0 eq) in *N*-methylpyrrolidone (120 mL) with stirring at 25 °C. The reaction was carried out for 16 hours at 90 °C, and the reaction process was monitored by liquid chromatography-mass spectrometry. After the reaction was completed, the reaction mixture was cooled to room temperature. The reaction mixture was purified by reversed-phase chromatographic column (C18 column), and eluted with 5% → 95% acetonitrile/water (10 mmol/L ammonium bicarbonate) in 50 min; detector, UV254/220 nm; to obtain compound **80-3** (white solid, 11.1 g, yield: 70%). MS (ESI, *m*/*z):* 353.1/355.1 [M + H]⁺; ¹H NMR (300 MHz, CDCl₃) *δ* 8.44 - 8.26 (m, 1H), 7.93 (s, 1H), 7.30 - 7.25 (m, 2H), 6.90 - 6.85 (m, 2H), 4.72 (d, *J=* 5.6 Hz, 2H), 3.86 (s, 3H), 3.82 (s, 3H), 2.49 (s, 3H).

### Step 4:

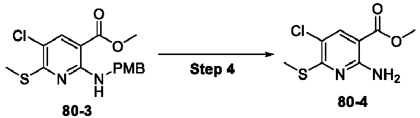

Anisole (32.03 g, 296.62 mmol, 10.0 eq) was added to a solution of **80-3** (11 g, 29.62 mmol, 1.0 eq) in trifluoroacetic acid (110 mL) with stirring under the protection of nitrogen at 25 °C. The reaction was carried out for 1 hour at 80 °C, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **80-4** (white solid, 6.5 g, yield: 94%). MS (ESI, *m*/*z):* 233.1/235.1 [M + H]⁺.

### Step 5:

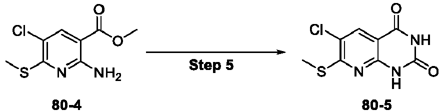

Trichloroacetyl isocyanate (2.31 g, 12.25 mmol, 1.0 eq) was added dropwise to asolution **80-4** (3.0 g, 12.25 mmol, 1.0 eq) in tetrahydrofuran (30 mL) with stirring under the protection of nitrogen at 25 °C. The reaction was carried out for 1 hour at this temperature, and the reaction process was monitored by liquid chromatography-mass spectrometry. After the reaction was completed, the reaction mixture was concentrated to obtain a crude product. An ammonia-methanol solution (7 mol/L, 50 mL) was added to the crude product with stirring at 25 °C. The reaction was carried out at 25 °C for 1 hour. The reaction process was monitored by liquid chromatography-mass spectrometry. After the reaction was completed, 500mL of water was added to the reaction solution for dilution, and the mixture was extracted with ethyl acetate (500 mL x 3), then the organic phases were combined, dried over anhydrous sodium sulfate, filtered, and the filtrate was concentrated to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **80-5** (white solid, 3 g, yield: 95%). MS (ESI, *m*/*z*): 244.1/246.1 [M + H]⁺; ¹H NMR (300 MHz, DMSO-*d₆*) *δ* 8.04 (s, 1H), 2.56 (s, 3H).

### Step 6:

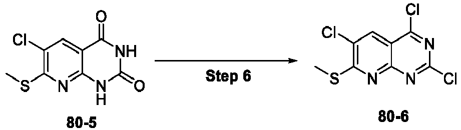

*N*,*N*-diisopropylethylamine (7.5 mL) was added dropwise to a solution of compound **80-5** (3g, 11.70 mmol, 1.0 eq) in phosphorus oxychloride (300 mL) with stirring under the protection of nitrogen at 0 °C, and the dropwise addition time was not less than 10 min. After the dropwise addition, the reaction solution was heated to 90 °C and the reaction was carried out at this temperature for 16 hours, and the reaction process was monitored by liquid chromatography-mass spectrometry. After the reaction was completed, the reaction mixture was cooled to 25 °C then concentrated under reduced pressure. Dichloromethane (300 mL) was added and the residual phosphorus oxychloride was removed by concentration, this operation was repeated three times to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **80-6** (white solid, 3.1 g, yield: 94%). MS (ESI, *m*/*z*): 280.0/282.0 [M + H]⁺; ¹H NMR (300 MHz, CDCl₃) *δ* 8.30 (s, 1H), 2.77 (s, 3H).

### Step 7:

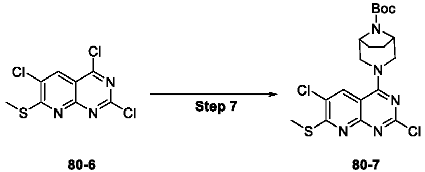

Triethylamine (3.35 g, 11.05 mmol, 3.0 eq) and *tert*-butyl(1*R*, 5*S*)-3,8-diazabicyclo[3.2.1]octane-8-carboxylate (2.35 g, 11.05 mmol, 1.0 eq) were added to a solution of the compound **80-6** (3.1 g, 11.05 mmol, 1.0 eq) in 1,4-dioxane (30 mL) with stirring under the protection of nitrogen at 25 °C. The reaction was carried out for 16 hours at 25 °C, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 20% ethyl acetate/petroleum ether mobile phase, and the obtained fractions were evaporated under reduced pressure to obtain compound **80-7** (white solid, 4.0 g, yield: 78%). MS (ESI, *m*/*z*): 456.2/458.2 [M + H]⁺.

### Step 8:

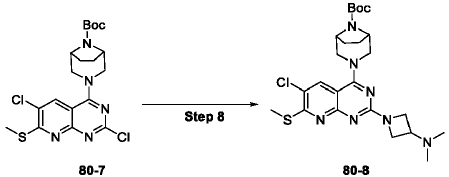

3-(Dimethylamino)azetidine dihydrochloride (660 mg, 6.57 mmol, 1.5 eq) and *N,N-*diisopropylethylamine (2.27 g, 17.56 mmol, 4.0 eq) were added to a solution of compound **80-7** (2.0 g, 4.16 mmol, 1.0 eq) in *N*-methylpyrrolidone (20 mL) with stirring under the protection of nitrogen at 25 °C. The reaction was carried out for 16 hours at 60 °C, and the reaction process was monitored by liquid chromatography-mass spectrometry. After the reaction was completed, the reaction mixture was cooled to 25 °C, then purified by reversed-phase chromatographic column (C18 column), and eluted with 5% → 95% acetonitrile/water (10 mmol/L ammonium bicarbonate) mobile phase in 50 min; detector, UV254/220 nm; to obtain compound **80-8** (white solid, 2.20 g, yield: 98%). MS (ESI, *m*/*z*): 520.2/522.2 [M + H]⁺; ¹H NMR (300 MHz, DMSO-*d₆*) *δ* 8.03 (s, 1H), 4.26 - 4.15 (m, 4H), 4.12 - 4.06(m, 2H), 3.88 - 3.83 (m, 2H), 3.50 - 3.44 (m, 2H), 3.16 - 3.08 (m, 1H), 2.56 (s, 3H), 2.12 (s, 6H), 1.81 - 1.75 (m, 2H), 1.71 - 1.65 (m, 2H), 1.46 (s, 9H).

### Step 9:

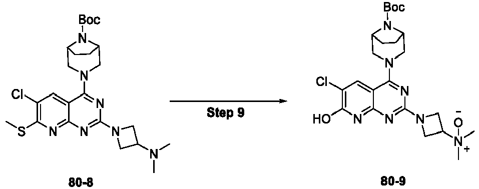

*m*-Chloroperoxybenzoic acid (1.32 g, 7.66 mmol, 3.0 eq) was added to a solution of compound **80-8** (1.40 g, 2.56 mmol, 1.0 eq) in dichloromethane (15 mL) with stirring under the protection of nitrogen at 25 °C. The reaction was carried out for 1 hour at that temperature, and the reaction process was monitored by liquid chromatography-mass spectrometry. After the reaction was completed, the reaction mixture was concentrated to obtain a crude product. The above crude product was dissolved in tetrahydrofuran (10 mL); and water (10 mL) and sodium hydroxide (0.51 g, 12.84 mmol, 5.2 eq) were added thereto with stirring at 0°C under the protection of nitrogen. The reaction was carried out for 1 hour at 60 °C, and the reaction process was monitored by liquid chromatography-mass spectrometry. After the reaction was completed, the reaction mixture was cooled to 25 °C. The reaction solution was concentrated to obtain a crude product. The obtained crude product was purified by reversed-phase chromatographic column (C18 column), and eluted with 5% → 95% acetonitrile/water mobile phase (10 mmol/L ammonium bicarbonate) in 30 min; detector, UV254/220 nm; to obtain compound **80-9** (white solid, 1.10 g, yield 85%). MS (ESI, *m*/*z*): 506.2/508.2 [M + H]⁺; ¹H NMR (300 MHz, DMSO-*d₆*) *δ* 13.43 (s, 1H), 7.79 (s, 1H), 4.78 - 4.74 (m, 1H), 4.47 - 4.40 (m, 2H), 4.17 - 4.12 (m, 2H), 4.09 - 4.03 (m, 3H), 3.94 - 3.86 (m, 1H), 3.32 - 3.24 (m, 2H), 3.02 (s, 6H), 1.80 - 1.55 (m, 4H), 1.45 (s, 9H).

### Step 10:

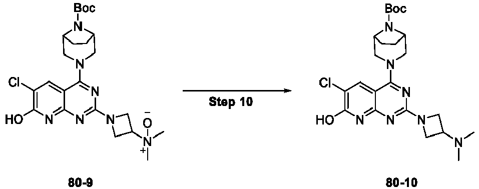

Iron powder (0.36 g, 6.54 mmol, 3.0 eq), water (1.6 mL) and ammonium chloride (0.58 g, 10.90 mmol, 5.0 eq) were added to a solution of compound **80-9** (1.10 g, 2.18 mmol, 1.0 eq) in ethanol (8 mL) with stirring under the protection of nitrogen at 25 °C. The reaction was carried out for 1 hour at that temperature, and the reaction process was monitored by liquid chromatography-mass spectrometry. After the reaction was completed, the reaction mixture was filtered and concentrated to obtain a crude product. The obtained crude product was purified by silica gel column chromatography, eluted with a gradient of 0%→12% methanol/dichloromethane mobile phase, and the obtained fractions were evaporated under reduced pressure to remove the solvent to obtain compound **80-10** (white solid, 700 mg, yield: 69%). MS (ESI, *m*/*z*): 490.3/492.3 [M + H]⁺; ¹H NMR (400 MHz, DMSO-*d₆*) *δ* 12.09 (s, 1H), 7.89 (s, 1H), 4.16 - 4.03 (m, 6H), 3.82 - 3.79 (m, 2H), 3.41 - 3.36 (m, 2H), 3.15 - 3.07 (m, 1H), 2.11 (s, 6H), 1.80 - 1.76 (m, 2H), 1.70 - 1.66 (m, 2H), 1.44 (s, 9H).

### Step 11:

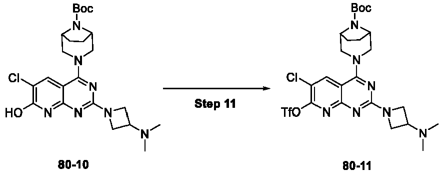

Trifluoromethylsulfonic anhydride (302.28 mg, 1.07 mmol, 1.5 eq) and *N,N-*diisopropylethylamine (277 mg, 2.14 mmol, 3.0 eq) were added to a solution of compound **80-10** (350 mg, 0.71 mmol, 1.0 eq) in dichloromethane (4.00 mL) under the protection of nitrogen with stirring at 0 °C. The reaction was carried out for 0.5 hours at that temperature, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **80-11** (white solid, 170mg, yield: 36%). MS (ESI, *m*/*z*): 622.1/624.1 [M + H]⁺; ¹H NMR (400 MHz, DMSO-*d₆*) *δ* 8.53 (s, 1H), 4.34 - 4.30 (m, 2H), 4.19 - 4.11 (m, 4H), 3.92 - 3.88 (m, 2H), 3.56 - 3.52 (m, 2H), 3.21 - 3.16 (m, 1H), 2.16 (s, 6H), 1.81 - 1.77 (m, 2H), 1.68 - 1.65 (m, 2H), 1.46 (s, 9H).

### Step 12:

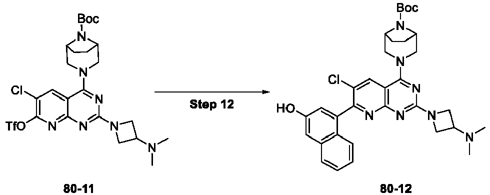

4-(4,4,5,5-Tetramethyl-1,3,2-dioxaborolan-2-yl)naphthalen-2-ol (105 mg, 0.39 mmol, 1.5 eq), water (0.30 mL), sodium carbonate (55.03 mg, 0.519 mmol, 2.00 eq) and tetrakis(triphenylphosphine)palladium (30 mg, 0.03 mmol, 0.1 eq) were added to a solution of compound **80-11** (170 mg, 0.26 mmol, 1.0 eq) in ethylene glycol dimethyl ether (1.50 mL) under the protection of nitrogen with stirring at 25 °C. The reaction was carried out for 2 hours at 85 °C under nitrogen atmosphere, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→12% methanol/dichloromethane mobile phase, and the obtained fractions were evaporated under reduced pressure to remove the solvent to obtain compound **80-12** (yellow solid, 40 mg, yield: 24%). MS (ESI, *m*/*z):* 616.3/618.3 [M + H]⁺; ¹H NMR (300 MHz, CDCl₃) *δ* 8.04 (s, 1H), 7.65 (d, *J=* 8.2 Hz, 1H), 7.40 - 7.33 (m, 3H), 7.22 - 7.17 (m, 2H), 4.46 -4.05 (m, 8H), 3.67 - 3.56 (m, 2H), 3.30 - 3.21 (m, 1H), 2.26 (s, 6H), 2.00 - 1.93 (m, 2H), 1.86 - 1.79 (m, 2H), 1.55 (s, 9H).

### Step 13:

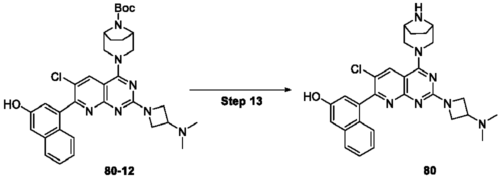

Trifluoroacetic acid (1.00 mL) was added dropwise to a solution of compound **80-12** (40 mg, 0.06 mmol, 1.0 eq) in dichloromethane (3.00 mL) with stirring at 25 °C. The reaction was carried out for 0.5 hours at this temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% acetonitrile/water mobile phase (10 mmol/L ammonium bicarbonate) in 20 min; detector, UV254/220 nm; to obtain compound **80** (yellow solid, 10 mg, yield: 31%). MS (ESI, m/z): 516.2/518.2 [M + H]⁺; ¹H NMR (300 MHz, CD₃OD) *δ* 8.39 (s, 1H), 7.81 (d, *J=* 8.2 Hz, 1H), 7.51 - 7.45 (m, 2H), 7.33 - 7.26 (m, 2H), 7.20 (d, *J=* 2.4 Hz, 1H), 4.57 - 4.51 (m, 2H), 4.36 - 4.29 (m, 2H), 4.12 - 4.07 (m, 2H), 3.71 - 3.66 (m, 4H), 3.35 - 3.29 (m, 1H), 2.33 (s, 6H), 1.94 - 1.92 (m, 4H).

### Embodiment 18

### 4-(4-((1R,5S)-3,8-diazabicyclo[3.2.1]octan-3-yl)-6-fluoro-2-(3-(dimethylamino)azetidin-1-yl)pyrido[2,3-d]pyrimidin-7-yl)naphthalen-2-ol 81

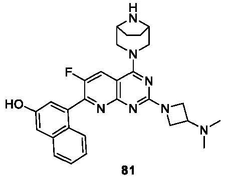

Compound **81** was synthesized according to Embodiment 17 (synthesis method XV). Compound **81** (yellow solid). MS (ESI, m/z): 500.2 [M + H]⁺; ¹H NMR (300 MHz, CDCl₃) *δ* 7.68 - 7.56 (m, 4H), 7.38 - 7.33(m, 1H), 7.26 - 7.19 (m, 2H), 4.27 - 4.17 (m, 4H), 4.05 - 3.99 (m, 2H), 3.67 - 3.64 (m, 2H), 3.59 - 3.54 (m, 2H), 3.20 - 3.12 (m, 1H), 2.21 (s, 6H), 1.82 - 1.81 (m, 4H); ¹⁹F NMR (282 MHz, CDCl₃) *δ* -127.79.

### Embodiment 19 (Synthesis method XVI)

### 4-(4-((1R,5S)-3,8-diazabicyclo[3.2.1]octan-3-yl)-2-(3-(dimethylamino)propoxy)-8-fluoropyrido[4,3-d]pyrimidin-7-yl)naphthalen-2-ol 82

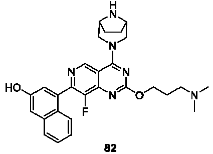

The synthetic route was as follows:

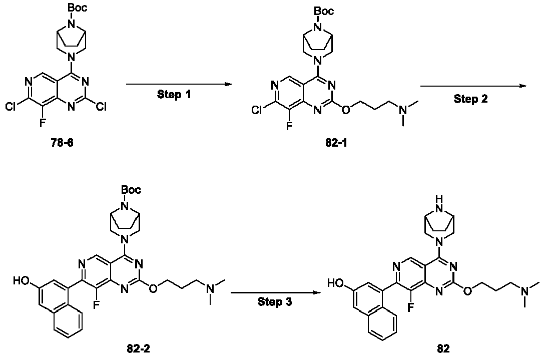

### Step 1:

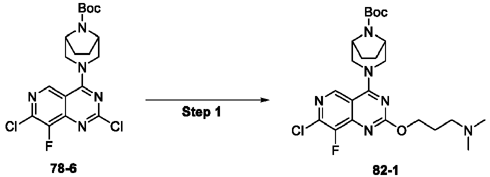

Sodium hydride (60% mineral oil mixture, 106 mg, 2.66 mmol, 3.0 eq) was added to a solution of 3-dimethylamino-1-propanol (193 mg, 1.77 mmol, 2.0 eq) in tetrahydrofuran (8 mL) with stirring at 0 °C. The reaction was carried out at this temperature for 30 mins. **78-6** (400 mg, 0.89 mmol, 1.0 eq) was added to the mixture, and the reaction was continued for 1 hour, and the reaction process was monitored by liquid chromatography-mass spectrometry. After the reaction was completed, the reaction solution was concentrated, diluted with 50 mL ice water, extracted with ethyl acetate (40 mL x 3), and the combined organic phases were washed with 100 mL of saturated brine, dried over anhydrous sodium sulfate, filtered, and the filtrate was concentrated to obtain a crude product. The crude product was purified by reversed-phase chromatographic column (C18 column), eluted with 5%→95% methanol/water (0.1%) mobile phase in 20 min; detector: UV254/220 nm; concentrated to obtain the compound **82-1.** MS (ESI, *m*/*z*): 495.2/497.2 [M + H]⁺; ¹H NMR (300 MHz, CDCl₃) *δ* 8.75 (s, 1H), 4.56 -4.38 (m, 6H), 3.70 - 3.64 (m, 2H), 2.56 (t, *J* = 7.4 Hz, 2H), 2.33 (s, 6H), 2.12 -1.96 (m, 4H), 1.77 - 1.72 (m, 2H), 1.54 (s, 9H).

### Step 2:

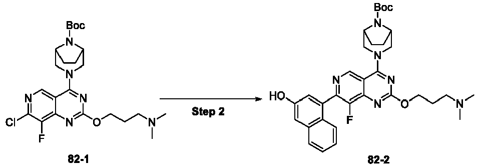

4-(4,4,5,5-Tetramethyl-1,3,2-dioxaborolan-2-yl)naphthalen-2-ol (118 mg, 0.43 mmol, 1.5 eq), cesium carbonate (197 mg, 0.58 mmol, 2.0 eq) and tetrakis(triphenylphosphine)palladium (35 mg, 0.03 mmol, 0.1 eq) were added to a solution of compound **82-1** (150 mg, 0.29 mmol, 1.0 eq) in 1,4-dioxane/water (5/1, 4 mL) with stirring under the protection of nitrogen at 25 °C. The reaction was carried out for 4 hours at 100 °C under nitrogen atmosphere, and the reaction process was monitored by liquid chromatography-mass spectrometry. After the reaction was completed, the reaction mixture was cooled to 25 °C, and concentrated under reduced pressure to obtain a crude product. The crude product was purified by reversed-phase chromatographic column (C18 column), eluted with 40%→90% methanol/water (0.1% ammonium bicarbonate) mobile phase in 20 min; detector: UV254/220 nm; the compound **82-2** (yellow solid, 150 mg, yield: 82%) was obtained. MS (ESI, *m*/*z):* 603.4 [M + H]⁺; ¹H NMR (400 MHz, CDCl₃) *δ* 8.96 (s, 1H), 7.67 - 7.62 (m, 2H), 7.39 - 7.35 (m, 1H), 7.25 - 7.19 (m, 3H), 4.54 - 4.46 (m, 4H), 4.39 - 4.30 (m, 4H), 3.66 - 3.57 (m, 2H), 2.68 - 2.64 (m, 2H), 2.37 (s, 6H), 2.14 - 2.08 (m, 2H), 1.93 - 1.89 (m, 2H), 1.73 - 1.68 (m, 2H), 1.52 (s, 9H).

### Step 3:

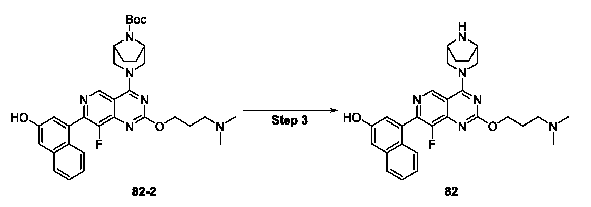

Trifluoroacetic acid (1 mL) was added dropwise to a solution of compound **82-2** (97 mg, 0.15 mmol, 1.0 eq) in dichloromethane (3 mL) with stirring at 0 °C. The reaction was carried out for 1 hour at 25 °C, and the reaction process was monitored by liquid chromatography-mass spectrometry. After the reaction was completed, the reaction mixture was concentrated to obtain a crude product. The crude product was purified by high performance liquid chromatography, and the purification conditions were: XBridge Prep C18 OBD Column, 19 x 150 mm, 5 µm; mobile phase A: water (10 mmol/L ammonium bicarbonate), mobile phase B: acetonitrile; flow rate: 25 mL/min; gradient: elution with 5% phase B in 2 min, then eluted with 5% → 21% phase B in 2.5 min, and finally eluted with 21 % → 38% phase B in 8.5 min; detection wavelength: UV220 nm; retention time: 8.03 min, to obtain compound **82** (yellow solid, 27 mg, yield: 35%). MS (ESI, *m*/*z):* 503.2 [M + H]⁺; ¹H NMR (400 MHz, DMSO-*d₆*) *δ* 10.00 (s, 1H), 9.17 (s, 1H), 7.80 (d, *J=* 8.4 Hz, 1H), 7.56 (d, *J* = 8.3 Hz, 1H), 7.46 - 7.42 (m, 1H), 7.29 (d, *J* = 2.4 Hz, 1H), 7.27 - 7.22 (m, 2H), 4.49 - 4.45 (m, 2H), 4.39 (t, *J=* 6.6 Hz, 2H), 3.65 - 3.59 (m, 4H), 2.41 (t, *J* = 7.1 Hz, 2H), 2.19 (s, 6H), 1.93 - 1.86 (m, 2H), 1.68 - 1.64 (m, 4H); ¹⁹F NMR (377 MHz, DMSO-*d₆*) *δ* -139.75.

### Embodiment 20 (Synthesis method XVII)

### 4-((S or R)-4-((1R,5S)-3,8-diazabicyclo[3.2.1]octan-3-yl)-6-chloro-2-(3-(dimethylamino)azetidin-1-yl)-8-fluoroquinolin-7-yl)naphthalen-2-ol 83a; 4-((R or S)-4-((1R,5S)-3,8-diazabicyclo[3.2.1]octan-3-yl)-6-chloro-2-(3-(dimethylamino)azetidin-1-yl)-8-fluoroquinolin-7-yl)naphthalen-2-ol 83b

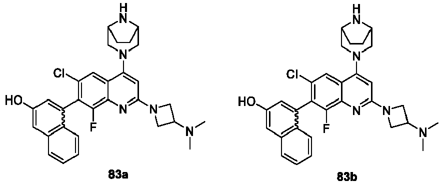

The synthetic route was as follows:

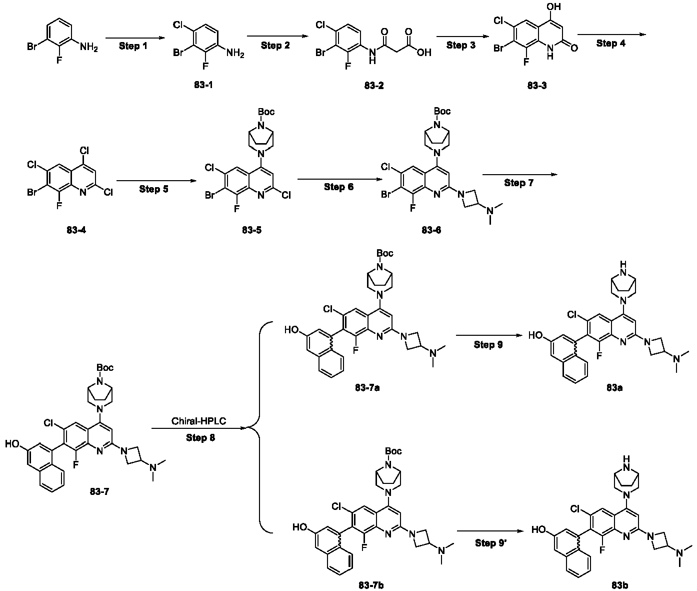

### Step 1:

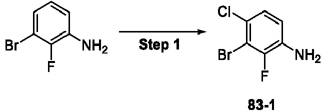

*N*-chlorosuccinimide (7.03 g, 50.00 mmol, 1.0 eq) was added to a solution of 3-bromo-2-fluoroaniline (10.0 g, 50.00 mmol, 1.0 eq) in *N*,*N*-dimethylformamide (100 mL) with stirring at 25 °C. The reaction was carried out for 16 hours at that temperature, and the reaction process was monitored by liquid chromatography-mass spectrometry. After the reaction was completed, the mixture was diluted with 200 mL of water, extracted with ethyl acetate (100 mL x 3), and the organic phases were combined and washed with 100 mL of saturated brine, dried over anhydrous sodium sulfate, and concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **83-1** (red solid, 6.18 g, yield: 52%). MS (ESI, *m*/*z*): 223.9/225.9 [M + H]⁺; ¹H NMR (400 MHz, CDCl₃) *δ* 7.06 - 7.03 (m, 1H), 6.69 - 6.65 (m, 1H).

### Step 2:

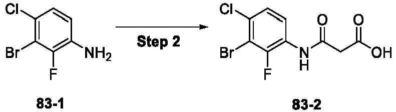

2,2-Dimethyl-1,3-dioxane-4,6-dione (2.57 g, 16.94 mmol, 2.0 eq) was added to a solution of compound **83-1** (2 g, 8.47 mmol, 1.0 eq) in toluene (20 mL) with stirring at 25 °C. The reaction was carried out for 3 hours at 90 °C, and the reaction process was monitored by liquid chromatography-mass spectrometry. After the reaction was completed, the reaction solution was cooled to 25 °C, diluted with 30 mL of toluene, filtered, the filter cake was washed with toluene (10 mL x 3) and dried to obtain compound **83-2** (white solid, 2.1 g, yield: 75%). MS (ESI, *m*/*z*): 309.9/312.0 [M + H]⁺; ¹H NMR (400 MHz, DMSO-*d₆*) *δ* 12.72 (s, 1H), 10.21 (s, 1H), 8.04 - 8.00 (m, 1H), 7.50 - 7.47 (m, 1H), 3.48 (s, 2H).

### Step 3:

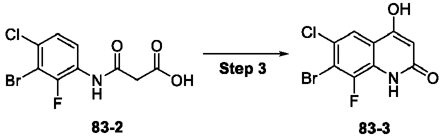

**83-2** (2.5 g, 7.65 mmol, 1.0 eq) and polyphosphoric acid (2.40 g, 20.66 mmol, 2.7 eq) were added to a 100 mL single-neck flask with stirring at 25°C. The reaction was carried out for 6 hours at 150 °C, and the reaction process was monitored by liquid chromatography-mass spectrometry. After the reaction was completed, the mixture was cooled to 25°C and diluted with 100 mL of water, filtered, and the filter cake was washed with water (50 mL x 3), then dried to obtain a crude compound of **83-3** (red solid, 2.4g, yield: 96%). The crude product was used directly in the next step without further purification. MS (ESI, *m*/*z*): 291.9/293.9 [M + H]⁺.

### Step 4:

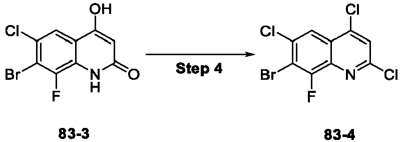

*N*,*N*-diisopropylethylamine (4.2 mL) was added dropwise to a solution of compound **83-3** (2.8g, 9.09 mmol, 1.0 eq) in phosphorus oxychloride (42.0 mL) with stirring under the protection of nitrogen at 0 °C, the dropwise addition time was not less than 5 mins. After the dropwise addition, the reaction solution was heated to 90 °C and the reaction was carried out at this temperature for 5 hours, and the reaction process was monitored by liquid chromatography-mass spectrometry. After the reaction was completed, the reaction mixture was cooled to 25 °C then concentrated under reduced pressure. Dichloromethane (30 mL) was added and the residual phosphorus oxychloride was removed by concentration, this operation was repeated three times to obtain a crude product. The crude product was purified by silica gel column chromatography, eluted with a gradient of 0% → 30% ethyl acetate/petroleum ether mobile phase, and the obtained fractions were evaporated under reduced pressure to obtain compound **83-4** (yellow solid, 1.3 g, yield: 41%). MS (ESI, *m*/*z*): 327.9/329.9 [M + H]⁺; ¹H NMR (400 MHz, CDCl₃) *δ* 8.15 (d, *J* = 1.9 Hz, 1H), 7.62 (s, 1H).

### Step 5:

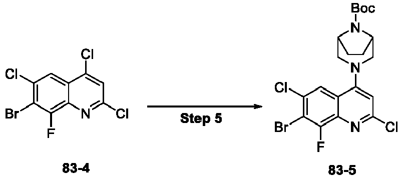

*N*,*N*-diisopropylethylamine (1.18 g, 8.67 mmol, 3.0 eq) and *tert*-butyl (1*R*, 5*S*)-3,8-diazabicyclo[3.2.1]octane-8-carboxylate (709 mg, 3.17 mmol, 1.1 eq) were added to a solution of compound **83-4** (1 g, 2.88 mmol, 1.0 eq) in *N*-methylpyrrolidone (8 mL) with stirring under the protection of nitrogen at 25 °C. The reaction was carried out for 12 hours at 25 °C, and the reaction process was monitored by liquid chromatography-mass spectrometry. After the reaction was completed, the obtained crude product was purified by reversed-phase chromatographic column (C18 column), eluted with 5%→95% methanol/water (0.1% ammonia water) mobile phase in 30 min; detector: UV254/220 nm; the compound **83-5** (white solid, 341.6 mg, yield: 22%) was obtained. MS (ESI, *m*/*z):* 504.0/506.0 [M + H]⁺; ¹H NMR (400 MHz, CDCl₃) *δ* 7.91 (d, *J=* 2.0 Hz, 1H), 6.90 (s, 1H), 4.48 - 4.38 (m, 2H), 3.37 - 3.32 (m, 2H), 3.18 - 3.10 (m, 2H), 2.13 - 2.12 (m, 4H), 1.50 (s, 9H).

### Step 6:

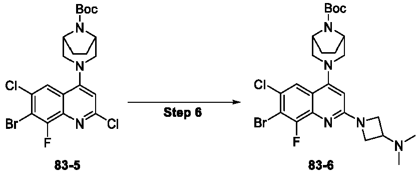

3-(Dimethylamino)azetidine dihydrochloride (175 mg, 0.96 mmol, 1.5 eq) and *N,N-*diisopropylethylamine (435 mg, 3.20 mmol, 5.0 eq) were added to a solution of compound **83-5** (340 mg, 0.64 mmol, 1.0 eq) in *N*-methylpyrrolidone (3 mL) with stirring under the protection of nitrogen at 25 °C. The reaction was carried out for 12 hours at 60 °C, and the reaction process was monitored by liquid chromatography-mass spectrometry. After the reaction was completed, the reaction mixture was cooled to 25 °C, and purified directly by a reversed-phase chromatographic column (C18 column), eluted with 5% → 95% methanol/water (0.1% ammonium bicarbonate) in 30 min; detector: UV254/220 nm; to obtain a crude product of compound **83-6**, and then the crude product was purified by preparative silica gel thin layer chromatography (elution solvent system: dichloromethane/methanol = 19/1) to obtain compound **83-6** (white solid, 230 mg, yield: 60%). MS (ESI, m/z): 568.2/570.2 [M + H]⁺; ¹H NMR (400 MHz, CDCl₃) *δ* 7.74 (d, *J=* 1.9 Hz, 1H), 6.03 (s, 1H), 4.43-4.32 (m, 2H), 4.24-4.19 (m, 2H), 4.05-4.01 (m, 2H), 3.33-3.26 (m, 2H), 3.17-3.09 (m, 2H), 2.92-2.86 (m, 1H), 2.27 (s, 6H), 2.18-2.07(s, 4H), 1.49 (s, 9H).

### Step 7:

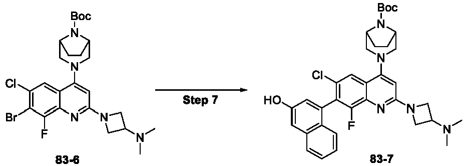

Compound **83-6** (247 mg, 0.41 mmol, 1.0 eq), 4-(4,4,5,5-tetramethyl-1,3,2-dioxaborolan-2-yl) naphthalen-2-ol (176 mg, 0.62 mmol, 1.5 eq), 1,4-dioxane (5 mL), water (1 mL), potassium carbonate (120.01 mg, 0.825 mmol, 2.00 eq) and [1,1'-bis(diphenylphosphino)ferrocene]dichlopalladium (35 mg, 0.04 mmol, 0.1 eq) were successively added to a 50 mL Schlenk tube under the protection of nitrogen at 25 °C. The reaction was carried out for 1 hour at 80 °C under nitrogen atmosphere, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5%→95% methanol/water (0.1% ammonium bicarbonate) mobile phase in 30 min; detector: UV254/220 nm; compound **83-7** was obtained (a mixture of two stereoisomers, white solid, 130 mg, yield: 47%). MS (ESI, m/z): 632.2/634.3 [M + H]⁺; ¹H NMR (300 MHz, CDCl₃) *δ* 7.83 (d, *J=* 1.6 Hz, 1H), 7.74 (d, *J=* 8.3 Hz, 1H), 7.45-7.36 (m, 2H), 7.28-7.27 (m, 1H), 7.24-7.19 (m, 1H), 7.10 (d, *J=* 2.5 Hz, 1H), 6.10 (s, 1H), 4.48-4.38 (m, 2H), 4.32-4.20 (m, 2H), 4.07-4.02 (m, 2H), 3.39-3.18 (m, 5H), 2.24 (s, 6H), 2.22-2.10 (m, 4H), 1.53 (s, 9H).

### Step 8:

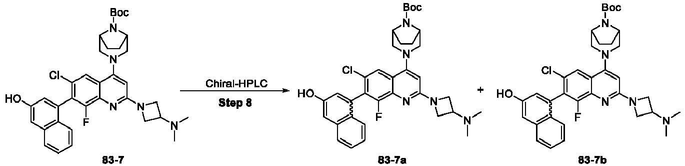

The compound **83-7** (130 mg) obtained in step 7 was subjected to chiral resolution, and the resolution conditions were: chiral column NB-Lux 5µm i-Cellulose-5, 2.12 x 25 cm, 5 µm; mobile phase A: *n*-hexane/dichloromethane = 5/1 (0.5%, 2 mol/L ammonia methanol solution), mobile phase B: ethanol; flow rate: 20 mL/min; gradient: elution with 8% phase B in 40 min; detector: 220 nm; two products were obtained. The product with shorter retention time (16.81 min) was **83-7a,** *tert*-butyl(1*R*,5*S*)-3-((*S* or *R*)-6-chloro-2-(3-(dimethylamino)azetidin-1-yl)-8-fluoro-7-(3-hydroxynaphthalen-1-yl)quinazolin-4-yl)-3,8-diazabicyclo[3.2.1]octane-8-carboxylate (white solid, 50 mg, recovery rate: 39%); the product with longer retention time (25.09 min) was **83-7b,** *tert*-butyl(1*R*,5*S*)-3-((*R* or *S*)-6-chloro-2-(3-(dimethylamino)azetidin-1-yl)-8-fluoro-7-(3-hydroxynaphthalen-1-yl)quinazolin-4-yl)-3,8-diazabicyclo[3.2.1]octane-8-carboxylate (white solid, 46 mg, recovery rate: 35%).

### Step 9:

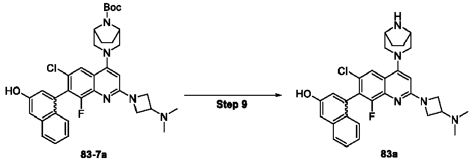

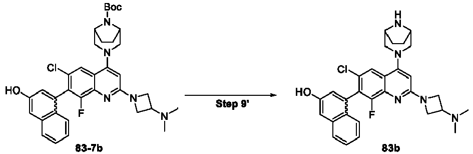

Trifluoroacetic acid (1 mL) was added dropwise to a solution of compound **83-7a** (51 mg, 0.08 mmol, 1.0 eq) in dichloromethane (3 mL) with stirring at 25 °C. The reaction was carried out for 1 hour at this temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% methanol/water mobile phase (0.1% ammonium bicarbonate) in 20 min; detector, UV254/220 nm; to obtain compound **83a** (white solid, 13.6 mg, yield: 33%). Compound **83b** (white solid, 15.6 mg, yield: 41%) can be obtained by the same method as above.

Compound **83a:** MS (ESI, m/z): 532.2/534.2 [M + H]⁺; ¹H NMR (400 MHz, DMSO-*d₆*) *δ* 9.97 (s, 1H), 7.80-7.78(m, 2H), 7.45-7.40 (m, 1H), 7.26 (d, *J=* 2.4 Hz, 1H), 7.22-7.18 (m, 2H), 7.04 (d, *J=* 2.4 Hz, 1H), 6.22 (s, 1H), 4.14-4.10 (m, 2H), 3.89-3.84(m, 2H), 3.54-3.51 (m, 2H), 3.26-3.22 (m, 2H), 3.21-3.15 (m, 1H), 2.99-2.94 (m, 2H), 2.12 (s, 6H), 2.03-1.99 (m, 2H), 1.82-1.78 (m, 2H); ¹⁹F NMR (377 MHz, DMSO-*d₆*) *δ*-122.02. The chiral analysis conditions of compound **83a** were: CHIRALPAK IC-3, 4.6 x 50 mm, 3 µm; mobile phase A: *n-*hexane/dichloromethane=5/1 (0.1% diethylamine), mobile phase B: ethanol; flow rate: 1 mL/min; isocratic elution with 10% phase B in 6 min; detector UV 254 nm; retention time: 4.198 min; ee > 99%.

Compound **83b:** MS (ESI, m/z): 532.3/534.2 [M + H]⁺; ¹H NMR (400 MHz, DMSO-*d₆*) *δ* 9.97 (s, 1H), 7.80-7.78(m, 2H), 7.45-7.40 (m, 1H), 7.26 (d, *J=* 2.4 Hz, 1H), 7.22-7.18 (m, 2H), 7.04 (d, *J=* 2.4 Hz, 1H), 6.22 (s, 1H), 4.14-4.10 (m, 2H), 3.89-3.84(m, 2H), 3.54-3.51 (m, 2H), 3.26-3.22 (m, 2H), 3.21-3.15 (m, 1H), 2.99-2.94 (m, 2H), 2.12 (s, 6H), 2.03-1.99 (m, 2H), 1.82-1.78 (m, 2H); ¹⁹F NMR (377 MHz, DMSO-*d₆*) *δ*-122.02. The chiral analysis conditions of compound **83b** were:: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 10% phase B in 6 min; detector UV 254 nm; retention time: 5.084 min; ee > 99%.

### Embodiment 21 (Synthesis method XVIII)

### (S or R)-N-(2-((6-chloro-2-(3-(dimethylamino)azetidin-1-yl)-8-fluoro-7-(3-hydroxynaphthalen-1-yl)quinazolin-4-yl)(methyl)amino)ethyl)acetamide 84

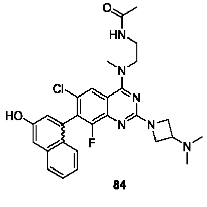

### Step 1:

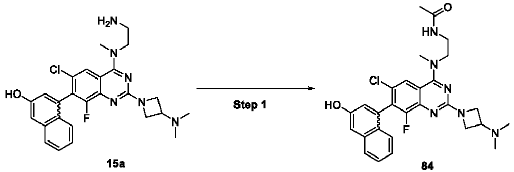

Acetic acid (8 mg, 0.14 mmol, 1.2 eq), *O*-(7-azabenzotriazol-1-yl)-*N*,*N*,*N'*,*N'-*tetramethylurea hexafluorophosphate (53 mg, 0.14 mmol, 1.2 eq) and *N*,*N*-dimethylformamide (3 mL) were added to a 50 mL single-neck flask with stirring at 25 °C. The reaction was carried out at this temperature for 0.5 hours, then **15a** (60 mg, 0.12 mmol, 1.0 eq) and *N,N-*diisopropylpropanamide (45 mg, 0.34 mmol, 3.0 eq) were added to the mixture. The reaction was carried out for 2 hours at 25 °C, and the reaction process was monitored by liquid chromatography-mass spectrometry. After the reaction was completed, the obtained crude product was purified by reversed-phase chromatographic column (C18 column), eluted with 5%→95% methanol/water (0.1% ammonia water) mobile phase in 20 min; detector: UV254/220 nm. Compound **84** (white solid, 7.6 mg, yield: 12%) was obtained. MS (ESI, m/z): 537.2/539.2 [M + H]⁺; ¹H NMR (400 MHz, DMSO-*d₆*) *δ* 9.97 (s, 1H), 8.47-8.39 (m, 1H), 8.15-8.14 (m, 1H), 7.79 (d, *J=* 8.2 Hz, 1H), 7.45-7.41 (m, 1H), 7.25 (d, *J=* 2.4 Hz, 1H), 7.24-7.17 (m, 2H), 7.03-7.02 (m, 1H), 4.11-4.06 (m, 2H), 3.87-3.83 (m, 2H), 3.73-3.61 (m, 2H), 3.59-3.53 (m, 2H), 3.14-3.07 (m, 1H), 3.04 (s, 1.5H), 2.89 (s, 1.5H), 2.11 (s, 6H), 1.99 (d, *J=* 6.9 Hz, 3H); ¹⁹F NMR (377 MHz, DMSO-*d₆*) *δ*-124.11.

### Embodiment 22 (Synthesis method XIX)

### (1R,5S)-3-((S or R)-6-chloro-2-(3-(dimethylamino)azetidin-1-yl)-8-fluoro-7-(3-hydroxynaphthalen-1-yl)quinazolin-4-yl)-3,8-diazabicyclo[3.2.1]octane-8-carboximidamide diformate 85

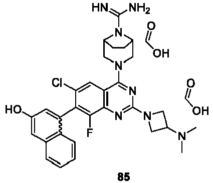

### Step 1:

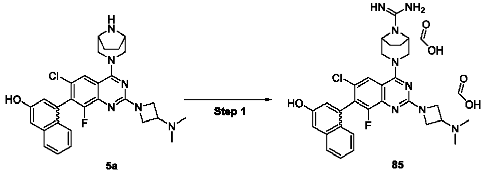

**5a** (50 mg, 0.09 mmol, 1.0 eq), 1,2,4-triazol-1-carboxyimide (104 mg, 0.89 mmol, 10.0 eq), *N*,*N*-diisopropylethylamine (175 mg, 1.34 mmol, 10.0 eq) and *N*-methylpyrrolidone (2.0 mL) were added to a 25 mL Schlenk tube with stirring under the protection of nitrogen at 25 °C. The reaction was carried out for 24 hours at 70 °C under nitrogen atmosphere, and the reaction process was monitored by liquid chromatography-mass spectrometry. After the reaction was completed, the reaction mixture was cooled down to 25 °C, then directly purified by reversed-phase chromatographic column (C18 column), and eluted with 20% → 70% acetonitrile/water (0.1% formic acid) mobile phase in 20 min; detector, UV254/220 nm; compound **85** was obtained (white solid, 25 mg, yield: 39%). MS (ESI, *m*/*z*): 575.2/577.2 [M + H]⁺; ¹H NMR (300 MHz, DMSO-*d₆*) *δ* 8.46-8.32 (m, 5H), 7.81-7.77 (m, 2H), 7.45-7.41 (m, 1H), 7.26 (d, *J=* 2.4 Hz, 1H), 7.23-7.20 (m, 2H), 7.03 (d, *J=* 2.4 Hz, 1H), 4.54-4.51 (m, 2H), 4.33-4.28 (m, 2H), 4.13-4.09 (m, 2H), 3.89-3.85 (m, 2H), 3.62-3.57 (m, 2H), 3.16-3.10 (m, 1H), 2.12 (s, 6H), 1.95-1.89 (m, 4H); ¹⁹F NMR (377 MHz, DMSO-*d₆*) *δ*-123.46.

### Embodiment 23 (Synthesis method XX)

### 4-((S or R)-6-chloro-2-(3-(dimethylamino)azetidin-1-yl)-8-fluoro-4-((1R,5S)-8-methyl)-3,8-diazabicyclo[3.2.1]octan-3-yl)quinazolin-7-yl)naphthalen-2-ol 86

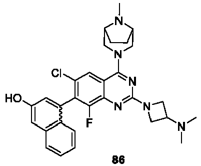

### Step 1:

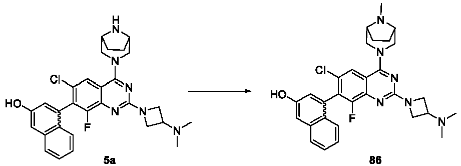

Acetic acid (3 mg, 0.06 mmol, 1.0 eq), sodium cyanoborohydride (5 mg, 0.08 mmol, 1.5 eq) and formaldehyde aqueous solution (37%, 5 mg, 0.06 mmol, 1.2 eq) were successively added to a solution of compound **5a** (30 mg, 0.05 mmol, 1.0 eq) in methanol (1.5 mL) with stirring at 25 °C. The reaction was carried out for 1.5 hours at 25 °C, and the reaction process was monitored by liquid chromatography-mass spectrometry. After the reaction was completed, the reaction mixture was purified by reversed-phase chromatographic column (C18 column), and eluted with 5%→95% (methanol:acetonitrile=1:1)/water (0.1% ammonium bicarbonate) mobile phase in 20 min; detector: UV254/ 220 nm; compound **86** (white solid, 15 mg, yield: 50%) was obtained. MS (ESI, m/z): 547.2/549.2 [M + H]⁺; ¹H NMR (300 MHz, CD₃OD) *δ* 7.79 (d, *J* = 1.7 Hz, 1H), 7.75 (d, *J=* 8.2 Hz, 1H), 7.44-7.39 (m, 1H), 7.28-7.17 (m, 3H), 7.03 (d, *J* = 2.4 Hz, 1H), 4.43-4.37 (m, 2H), 4.30-4.24 (m, 2H), 4.06-4.01 (m, 2H), 3.64-3.8 (m, 2H), 3.35-3.34 (m, 2H), 3.30-3.22 (m, 1H), 2.40 (s, 3H), 2.26 (s, 6H), 2.12-2.05 (m, 2H), 1.84-1.78 (m, 2H); ¹⁹F NMR (282 MHz, CD₃OD) *δ*-124.91.

### Embodiment 24 (Synthesis method XXI)

### 4-((S or R)-6-chloro-2-(3-(dimethylamino)azetidin-1-yl)-8-fluoro-4-((1R,5S)-8-(1-iminoethyl)-3,8-diazabicyclo[3.2.1]octan-3-yl)quinazolin-7-yl)naphthalen-2-ol trifluoroacetate 87

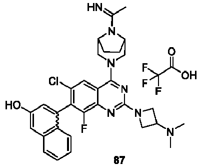

The synthetic route was as follows:

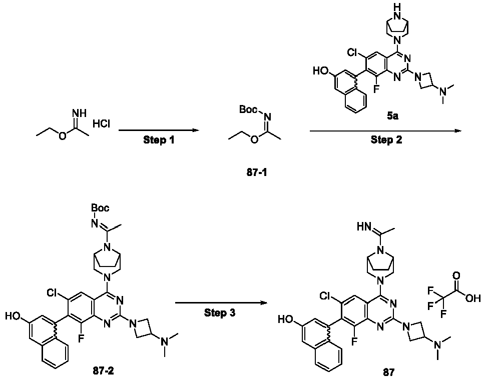

### Step 1:

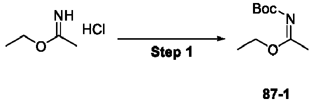

4-Dimethylaminopyridine (188 mg, 1.54 mmol, 0.1 eq) and pyridine (5.47 g, 69.19 mmol, 4.5 eq) were added to a solution of ethylacetylimide hydrochloride (2 g, 15.38 mmol, 1.0 eq) in dichloromethane (25 mL) with stirring at 25 °C. Then, a solution of di-*tert*-butyl dicarbonate (11.74 g, 53.81 mmol, 3.5 eq) in dichloromethane (20 mL) was slowly added dropwise to the reaction mixture for not less than 20 min. After the dropwise addition, the reaction was carried out at 25 °C for 16 hours, and the reaction process was monitored by liquid chromatography-mass spectrometry. After the reaction was completed, the reaction mixture was concentrated to obtain a crude product. The crude product was dissolved in 20 mL of dichloromethane, filtered, and concentrated to obtain a crude product of compound **87-1** (brown oil, 1.96 g, yield: 45%). MS (ESI, m/z): 188.1 [M + H]⁺.

### Step 2:

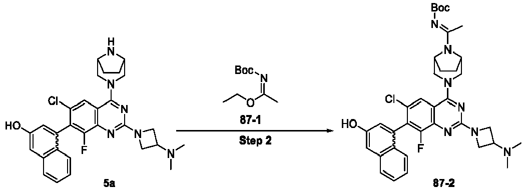

**87-1** (169 mg, 0.90 mmol, 10.0 eq) and *N*,*N*-diisopropylethylamine (58 mg, 0.45 mmol, 5.0 eq) were added to a solution of compound **5a** (48 mg, 0.09 mmol, 1.0 eq) in methanol (1.5 mL) with stirring at 25 °C. The reaction was carried out for 16 hours at 60 °C, and the reaction process was monitored by liquid chromatography-mass spectrometry. After the reaction was completed, the obtained crude product was purified by reversed-phase chromatographic column (C18 column), eluted with 5%→95% (methanol: acetonitrile=1:1)/water (0.1% ammonium bicarbonate) mobile phase in 20 min; detector: UV254/220 nm; compound **87-2** (white solid, 40 mg, yield: 65%) was obtained. MS (ESI, *m*/*z*): 674.3/676.2 [M + H]⁺.

### Step 3:

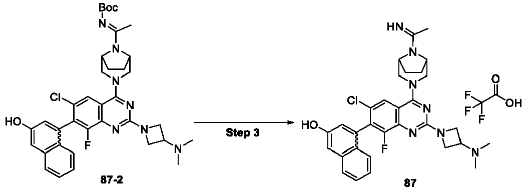

Trifluoroacetic acid (1 mL) was slowly added dropwise to a solution of **87-2** (40 mg, 0.06 mmol, 1.0 eq) in dichloromethane (3 mL) with stirring at 0 °C. The reaction was carried out for 1 hour at 25 °C, and the reaction process was monitored by liquid chromatography-mass spectrometry. After the reaction was completed, the reaction mixture was concentrated under reduced pressure to obtain a crude product. The crude product was purified by preparative high performance liquid chromatography. Preparative conditions: XSelect CSH Prep C18 OBD Column, 19 x 250 mm, 5 µm; mobile phase A: water (0.1% formic acid); mobile phase B: acetonitrile; flow rate: 25 mL/min; gradient: elution with a gradient of 5%→24% mobile phase B in 7 min; detector UV 225 nm; product **87** was obtained (white solid, 26 mg, yield: 67%). MS (ESI, *m*/*z*): 574.2/576.2 [M + H]⁺; ¹H NMR (300 MHz, CD₃OD) *δ* 7.82 (d, *J* = 1.7 Hz, 1H), 7.77 (d, *J* = 8.2 Hz, 1H), 7.46-7.40 (m, 1H), 7.27 (d, *J=* 2.4 Hz, 1H), 7.25-7.18 (m, 2H), 7.03 (d, *J* = 2.4 Hz, 1H), 4.72-4.52 (m, 4H), 4.40-4.33 (m, 2H), 4.18-4.12 (m, 2H), 3.80-3.68 (m, 2H), 3.60-3.52 (m, 1H), 2.48-2.44 (m, 9H), 2.17-2.11 (m, 4H); ¹⁹F NMR (282 MHz, CD₃OD) *δ*-76.90, -124.43.

### Embodiment 25 (Synthesis method XXII)

### 4,4'-((S or R)-4-((1R,5S)-3,8-diazabicyclo[3.2.1]octan-3-yl)-6-chloro-8-fluoroquinazolin-2,7-diyl)bis(naphthalen-2-ol) hydrochloride 88a; 4,4'-((R or S)-4-((1R,5S)-3,8-diazabicyclo[3.2.1]octan-3-yl)-6-chloro-8-fluoroquinazolin-2,7-diyl)bis(naphthalen-2-ol) 88b

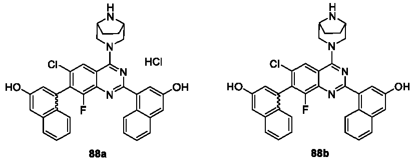

The synthetic route was as follows:

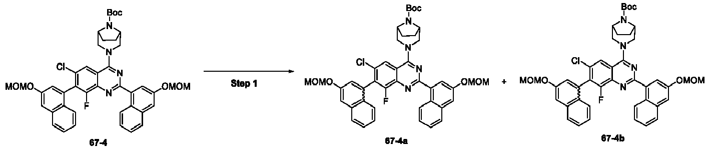

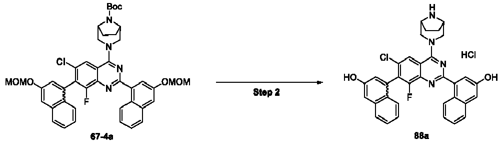

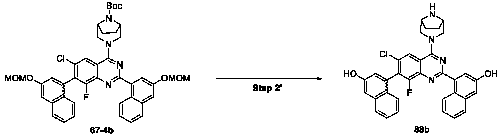

### Step 1:

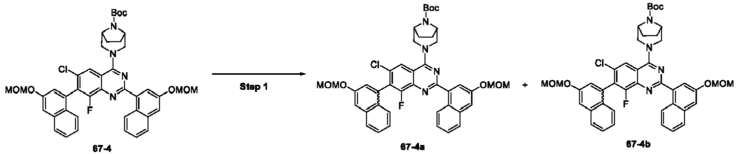

The compound **67-4** (200 mg) obtained in embodiment 7 was subjected to chiral resolution under the following conditions: chiral column NB_Lux 5µm i-Cellulose-5, 2.12 x 25 cm, 5 µm; mobile phase A: *n*-hexane/dichloromethane = 5/1 (0.5% 2 mol/L ammonia methanol solution), mobile phase B: ethanol; flow rate: 20 mL/min; eluted with 10% mobile phase B in 12 min; detector UV225 nm; two compounds were obtained. The product with shorter retention time (6.86 min) was **67-4a,** *tert*-butyl(1*R*,5*S*)-3-((*S* or *R*)-6-chloro-8-fluoro-2,7-bis(3-(methoxymethoxy)naphthalen-1-yl)quinazolin-4-yl)-3,8-diazabicyclo[3.2.1]octan-8-carboxylate (yellow solid, 80 mg, recovery rate: 40%); the product with longer retention time (9.107 min) was **67-4b**, *tert*-butyl (1*R*,5*S*)-3-((*R* or *S*)-6-chloro-8-fluoro-2,7-bis(3-(methoxymethoxy)naphthalen-1-yl)quinazolin-4-yl)-3,8-diazabicyclo[3.2.1]octan-8-carboxylate (yellow solid, 88 mg, recovery rate: 44%).

### Step 2:

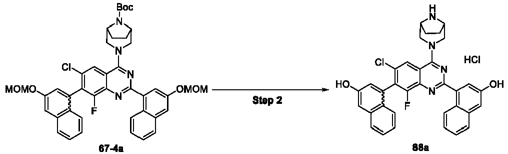

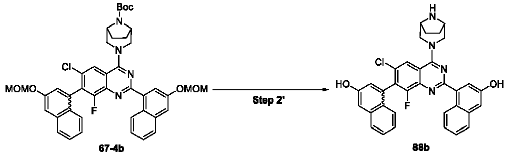

A solution of hydrochloric acid (4 mol/L, 1 mL) in 1,4-dioxane was added dropwise to a solution of compound **67-4a** (30 mg, 0.04 mmol, 1.0 eq) in methanol (1 mL) with stirring at 25 °C. The reaction was carried out for 1 hour at room temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 40% acetonitrile/water mobile phase (0.1% hydrochloric acid) in 20 min; detector, UV254/220 nm; to obtain compound **88a** (yellow solid, 10 mg, yield: 41%). Compound **88b** (yellow solid, 10 mg, yield: 41%) can be obtained by the same method as above.
compound **88a:** MS (ESI, m/z): 577.2/579.2 [M + H]⁺; ¹H NMR (300 MHz, DMSO-*d₆*) *δ* 10.21-9.83 (m, 2H), 8.70 (d, *J=* 8.6 Hz, 1H), 8.27 (s, 1H), 8.10 (d, *J=* 1.7 Hz, 1H), 7.85-7.76 (m, 3H), 7.49-7.40 (m, 2H), 7.33-7.22 (m, 5H), 7.14 (d, *J=* 2.4 Hz, 1H), 4.55-4.42 (m, 2H), 3.72-3.51 (m, 4H), 1.78-1.68 (m, 4H); ¹⁹F NMR (282 MHz, DMSO-*d₆*) *δ*-120.77. The chiral analysis conditions of compound **88a** were: CHIRALPAK ID-3, 4.6 x 50 mm, 3 µm; mobile phase A: *n*-hexane/dichloromethane=5/1 (0.1% diethylamine), mobile phase B: ethanol; flow rate: 1 mL/min; isocratic elution with 10% phase B in 8.5 min; detector UV 254 nm; retention time: 4.033 min; ee > 99%.
compound **88b:** MS (ESI, m/z): 577.2/579.2 [M + H]⁺; ¹H NMR (300 MHz, DMSO-*d₆*) *δ* 10.06 (s, 1H), 9.93 (s, 1H), 8.70 (d, *J=* 8.6 Hz, 1H), 8.10 (d, *J=* 1.6 Hz, 1H), 7.85-7.76 (m, 3H), 7.49-7.40 (m, 2H), 7.33-7.22 (m, 5H), 7.15 (d, *J=* 2.3 Hz, 1H), 4.56-4.43 (m, 2H), 3.73-3.58 (m, 4H), 1.80-1.67 (m, 4H); ¹⁹F NMR (282 MHz, DMSO-*d₆*) *δ*-120.76. The chiral analysis conditions of compound **88b** were: CHIRALPAK ID-3, 4.6 x 50 mm, 3 µm; mobile phase A: n-hexane/dichloromethane =3/1 (0.1% diethylamine), mobile phase B: ethanol; flow rate: 1 mL/min; isocratic elution with 10% phase B in 8.5 min; detector UV 254 nm; retention time: 6.515 min; ee > 99%.

### Embodiment 26 (Synthesis method XXIII)

### 4-(4-((1R,5S)-3,8-diazabicyclo[3.2.1]octan-3-yl)-2-(3-(dimethylamino)propoxy)-8-fluoroquinazolin-7-yl)naphthalen-2-ol 143

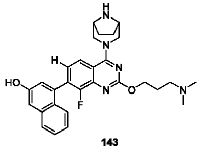

The synthetic route was as follows:

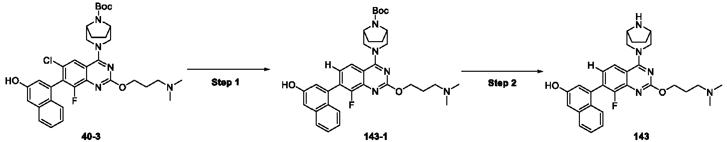

### Step 1

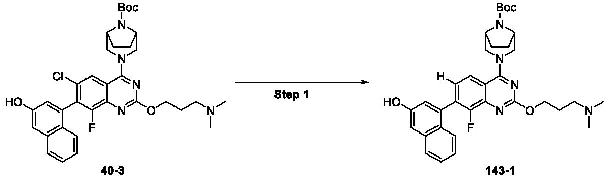

Anhydrous palladium carbon (10% palladium content, 124 mg) was added to a solution of compound **40-3** (124 mg, 0.19 mmol, 1.0 eq) in ethanol (10 mL) with stirring under the protection of nitrogen at 20 °C. The reaction was carried out for 1 hour at 80 °C in hydrogen atmosphere, and the reaction process was monitored by liquid chromatography-mass spectrometry. After the reaction was completed, the reaction mixture was cooled to 20 °C, filtered with diatomite, and the filter cake was washed with ethanol (10 mL x 3), and concentrated to obtain a crude product. The obtained crude product was purified by preparative silica gel thin layer chromatography (elution solvent system: dichloromethane/methanol = 10/1) to obtain compound **143-1** (yellow solid, 68 mg, yield: 55%). MS (ESI, m/z): 602.4 [M + H]⁺; ¹H NMR (400 MHz, CDCl₃) *δ* 7.73 (d, *J* = 8.3 Hz, 1H), 7.60 (d, *J=* 8.7 Hz, 1H), 7.53 (d, *J=* 8.3 Hz, 1H), 7.44-7.39 (m, 1H), 7.26-7.13 (m, 4H), 4.53 (d, *J=* 6.4 Hz, 2H), 4.45-4.31 (m, 4H), 3.61-3.51 (m, 2H), 2.67 (t, *J=* 7.8 Hz, 2H), 2.37 (s, 6H), 2.17-2.07 (m, 2H), 1.98-1.80 (m, 4H), 1.54 (s, 9H).

### Step 2

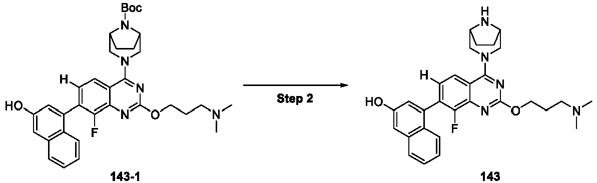

Trifluoroacetic acid (1 mL) was added dropwise to a solution of compound **143-1** (18 mg, 0.03 mmol, 1.0 eq) in dichloromethane (3 mL) with stirring at 20 °C. The reaction was carried out for 1 hour at this temperature, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% acetonitrile/water mobile phase (0.1% ammonium bicarbonate) in 30 min; detector, UV254/220 nm; to obtain compound **143** (white solid, 3.5 mg, yield: 22%). MS (ESI, m/z): 502.4 [M + H]⁺; ¹H NMR (400 MHz, DMSO-*d₆*) δ 9.98 (s, 1H), 7.87 (d, *J=* 8.7 Hz, 1H), 7.81 (d, *J=* 8.2 Hz, 1H), 7.47-7.39 (m, 2H), 7.28-7.21 (m, 3H), 7.10 (d, *J=* 2.4 Hz, 1H), 4.39-4.26 (m, 4H), 3.56-3.43 (m, 4H), 2.38 (t, *J=* 7.1 Hz, 2H), 2.16 (s, 6H), 1.93-1.84 (m, 2H), 1.74-1.66 (m, 4H); ¹⁹F NMR (282 MHz, DMSO-*d₆*) *δ*-128.02.

### Embodiment 27

### 4-(4-((1R,5S)-3,8-diazabicyclo[3.2.1]octan-3-yl)-8-fluoro-2-((S)-1-methylpyrrolidin-2-yl)methoxy)quinazolin-7-yl)naphthalen-2-ol tritrifluoroacetate

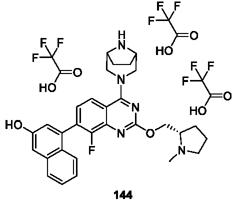

Compound **144** was synthesized according to Embodiment 26 (synthesis method XXIII). compound **144** (yellow solid): MS (ESI, m/z): 514.3 [M + H]⁺; ¹H NMR (300 MHz, CD₃OD) *δ* 7.97 (d, *J=* 8.7 Hz, 1H), 7.80-7.76 (m, 1H), 7.49-7.42 (m, 3H), 7.28 (d, *J=* 2.5 Hz, 1H), 7.27-7.21 (m, 1H), 7.13 (d, *J=* 2.5 Hz, 1H), 4.98-4.91 (m, 1H), 4.82-4.67 (m, 3H), 4.29-4.27 (m, 2H), 3.93-3.84 (m, 3H), 3.78-3.71 (m, 1H), 3.30-3.23 (m, 1H), 3.12 (s, 3H), 2.48-2.39 (m, 1H), 2.26-2.08 (m, 7H); ¹⁹F NMR (282 MHz, CD₃OD) *δ*-77.21, -129.15.

### Embodiment 28

### (R or S)-1-Carbamimidoyl-3-(6-chloro-2-(3-(dimethylamino)azetidin-1-yl)-8-fluoro-7-(3-hydroxynaphthalen-1-yl)quinazolin-4-yl)azetidin-3-carboxamide ditrifluoroacetate 145

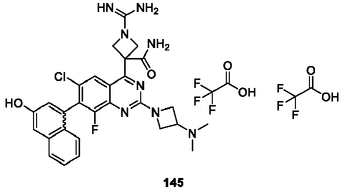

Compound **145** was synthesized according to Embodiment 22 (synthesis method XIX). compound **145** (yellow solid): MS (ESI, m/z): 563.2/565.2 [M + H]⁺; ¹H NMR (300 MHz, DMSO-*d₆*) *δ* 10.96 (s, 1H), 10.11 (s, 1H), 7.82 (d, *J=* 8.3 Hz, 1H), 7.76 (s, 1H), 7.59-7.54 (m, 4H), 7.48-7.38 (m, 3H), 7.30 (d, *J=* 2.4 Hz, 1H), 7.26-7.20 (m, 1H), 7.11 (d, *J=* 8.4 Hz, 1H), 7.07 (d, *J=* 2.4 Hz, 1H), 4.85-4.72 (m, 4H), 4.52-4.43 (m, 4H), 4.33-4.26 (m, 1H), 2.86 (s, 6H); ¹⁹F NMR (282 MHz, DMSO-*d₆*) *δ*-73.87, -122.62.

### Embodiment 29

### (S or R)-3-(6-chloro-2-(3-(dimethylamino) azetidan-1-yl)-8-fluoro-7-(3-hydroxynaphthalen-1-yl)quinazolin-4-yl)azetidine-1-carboxamide ditrifluoroacetate 146

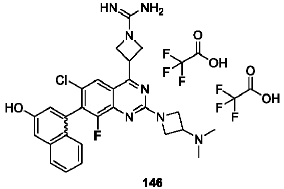

Compound **146** was synthesized according to Embodiment 22 (synthesis method XIX). compound **146** (yellow solid): MS (ESI, m/z): 520.2/522.2 [M + H]⁺; ¹H NMR (300 MHz, DMSO-*d₆*) *δ* 10.82 (s, 1H), 10.10 (s, 1H), 8.03 (d, *J=* 1.5 Hz, 1H), 7.82 (d, *J=* 8.3 Hz, 1H), 7.52-7.50 (m, 4H), 7.48-7.42 (m, 1H), 7.30 (d, *J=* 2.4 Hz, 1H), 7.26-7.20 (m, 1H), 7.14 (d, *J* = 8.4 Hz, 1H), 7.07 (d, *J=* 2.4 Hz, 1H), 4.95-4.84 (m, 1H), 4.66-4.59 (m, 2H), 4.51-4.38 (m, 6H), 4.31-4.22 (m, 1H), 2.86 (s, 6H); ¹⁹F NMR (282 MHz, DMSO-*d₆*) *δ*-73.83, -123.48.

### Embodiment 30 (Synthesis method XXIV)

### (S or R) 4-(4-((1R,5S)-3,8-diazabicyclo[3.2.1]octan-3-yl)-6-chloro-2-((3-(dimethylamino)propyl)thio)-8-fluoroquinazolin-7-yl)naphthalen-2-ol dihydrochloride 147

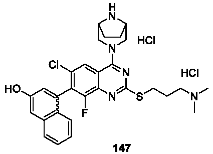

The synthetic route was as follows:

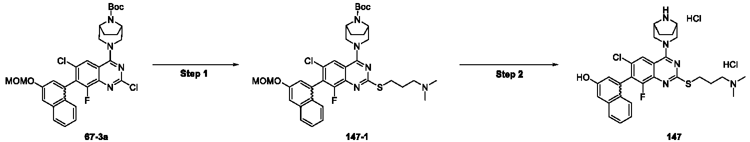

### Step 1

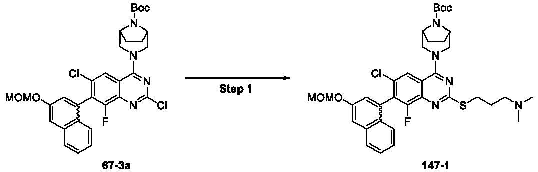

3-(Dimethylamino)propan-1-thiol (33 mg, 0.27 mmol, 1.2 eq) and potassium carbonate (64 mg, 0.46 mmol, 2.0 eq) were added to a solution of compound **67-3a** (150 mg, 0.23 mmol, 1.0 eq) in *N*,*N*-dimethylformamide (2 mL) with stirring at 25 °C. The reaction was carried out for 2 hours at 25 °C, and the reaction process was monitored by liquid chromatography-mass spectrometry. After the reaction was completed, 20 mL of water was added to the reaction mixture, the mixture was extracted with ethyl acetate (20 mL x 3), and the organic phases were combined, then the organic phases were washed with 20 mL of saturated brine, dried over anhydrous sodium sulfate, filtered, and the filtrate was concentrated to obtain a crude product. The obtained crude product was purified by preparative silica gel thin layer chromatography (elution solvent system: dichloromethane/methanol = 10/1) to obtain compound **147-1** (white solid, 97 mg, yield: 56%). MS (ESI, m/z): 696.2/698.2 [M + H]⁺; ¹H NMR (300 MHz, CDCl₃) *δ* 7.86 (d, *J=* 8.2 Hz, 1H), 7.79 (d, *J=* 1.7 Hz, 1H), 7.57 (d, *J=* 2.4 Hz, 1H), 7.53-7.47 (m, 1H), 7.37-7.29 (m, 2H), 7.22 (d, *J=* 2.4 Hz, 1H), 5.37 (s, 2H), 4.44-4.39 (m, 4H), 3.67-3.55 (m, 5H), 3.25 (t, *J=* 7.1 Hz, 2H), 2.91-2.85 (m, 2H), 2.55 (s, 6H), 2.28-2.22 (m, 2H), 2.04-1.99 (m, 2H), 1.90-1.83 (m, 2H), 1.55 (s, 9H).

### Step 2

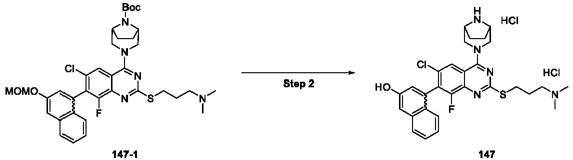

A solution of hydrochloric acid (4 mol/L, 2 mL) in 1,4-dioxane was added dropwise to a solution of compound **147-1** (80 mg, 0.11 mmol, 1.0 eq) in methanol (2 mL) with stirring at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% acetonitrile/water mobile phase (0.1% hydrochloric acid) in 20 min; detector, UV254/220 nm; to obtain compound **147** (light yellow solid, 33 mg, yield: 45%). MS (ESI, m/z): 552.2/554.2 [M + H]⁺; ¹H NMR (400 MHz, DMSO-*d₆*) *δ* 10.61 (s, 1H), 10.22-9.98 (m, 2H), 9.76 (s, 1H), 7.97 (d, *J=* 1.6 Hz, 1H), 7.82 (d, *J=* 8.3 Hz, 1H), 7.47-7.43 (m, 1H), 7.31 (d, *J=* 2.4 Hz, 1H), 7.25-7.20 (m, 2H), 7.12 (d, *J=* 2.4 Hz, 1H), 4.53-4.45 (m, 2H), 4.19-4.17 (m, 2H), 3.94 (t, *J=* 13.8 Hz, 2H), 3.22 (t, *J=* 7.3 Hz, 2H), 3.18-3.11 (m, 2H), 2.71 (d, *J=* 4.8 Hz, 6H), 2.18-2.10 (m, 2H), 2.02-1.91 (m, 4H); ¹⁹F NMR (377 MHz, DMSO-*d₆*) *δ*-121.69.

### Embodiment 31 (Synthesis method XXV)

### 4-((3-((S or R)-4-((1R,5S)-3,8-diazabicyclo[3.2.1]octan-3-yl)-6-chloro-8-fluoro-7-(3-hydroxynaphthalen-1 -yl)quinazolin-2-yl)oxy)propyl)(methyl)amino)butan-1,2-diol ditrifluoroacetate 148

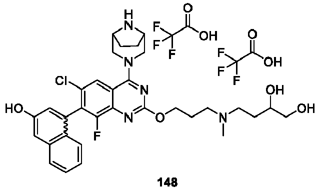

The synthetic route was as follows:

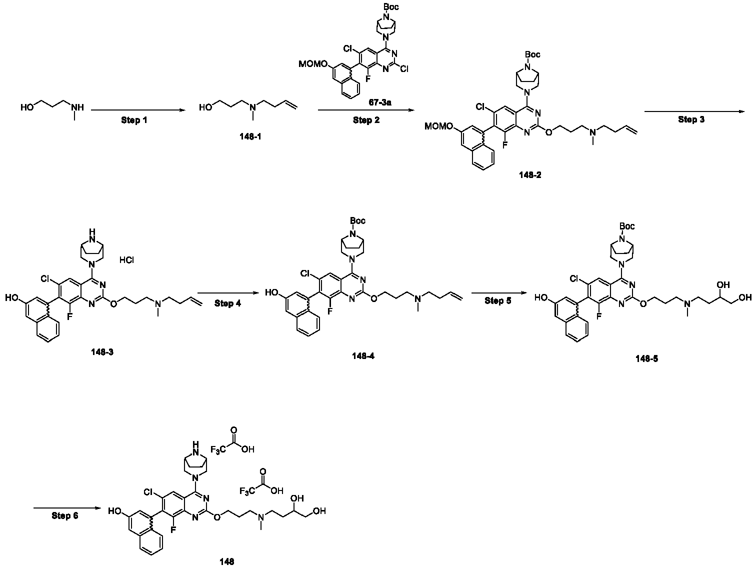

### Step 1

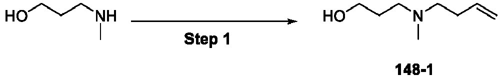

4-Chloro-1-butene (3.66 g, 38.37 mmol, 1.2 eq) and potassium carbonate (9.3 g, 63.95 mmol, 2.0 eq) were successively added to a solution of compound 3-(methylamino) propanol (3 g, 31.97 mmol, 1.0 eq) in acetonitrile (40 mL) with stirring at 25 °C. The reaction was carried out for 4 hours at 80 °C, and the reaction process was monitored by liquid chromatography-mass spectrometry. After the reaction was completed, the reaction mixture was cooled to 25°C, then filtered with a Buchner funnel, and the filter cake was washed with dichloromethane (50 mL x 3), and the filtrate was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **148-1** (light yellow oil, 1.2 g, yield: 26%). MS (ESI, m/z): 144.2 [M + H]⁺; ¹H NMR (300 MHz, CDCl₃) *δ* 5.86-5.72 (m, 1H), 5.11-5.01 (m, 2H), 3.82-3.78 (m, 2H), 2.64-2.60 (m, 2H), 2.50-2.44 (m, 2H), 2.31-2.22 (m, 5H), 1.74-1.67 (m, 2H).

### Step 2

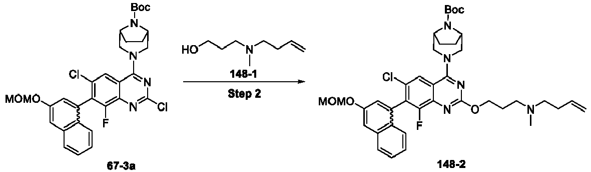

Potassium *tert*-butoxide (1 mol/L of tetrahydrofuran solution, 0.56 mL, 0.56 mmol, 1.5 eq) was added dropwise to a solution of **67-3a** (240 mg, 0.36 mmol, 1.0 eq) and **148-1** (84 mg, 0.56 mmol, 1.5 eq) in anhydrous tetrahydrofuran (5 mL) with stirring under the protection of nitrogen at 0 °C. After the dropwise addition, the reaction was carried out at 0 °C under the protection of nitrogen for 1 hour, and the reaction process was monitored by liquid chromatography-mass spectrometry. After the reaction was completed, 10 mL of water was added to the reaction mixture, and the mixture was extracted with ethyl acetate (10 mL x 3), then the organic phases were combined, dried over anhydrous sodium sulfate, filtered, and the filtrate was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **148-2** (off-white solid, 220 mg, yield: 82%). MS (ESI, m/z): 720.4/722.4 [M + H]⁺; ¹H NMR (300 MHz, CDCl₃) *δ* 7.86 (d, *J* = 8.2 Hz, 1H), 7.81 (d, *J=* 1.7 Hz, 1H), 7.56 (d, *J=* 2.4 Hz, 1H), 7.52-7.46 (m, 1H), 7.38-7.30 (m, 2H), 7.22 (d, *J=* 2.4 Hz, 1H), 5.86-5.72 (m, 1H), 5.37-5.36 (m, 2H), 5.13-5.02 (m, 2H), 4.53 (t, *J=* 6.4 Hz, 2H), 4.47-4.39 (m, 4H), 3.68-3.56 (m, 5H), 2.83-2.64 (m, 4H), 2.48-2.35 (m, 5H), 2.22-2.14 (m, 2H), 2.05-1.87 (m, 4H), 1.55 (s, 9H).

### Step 3

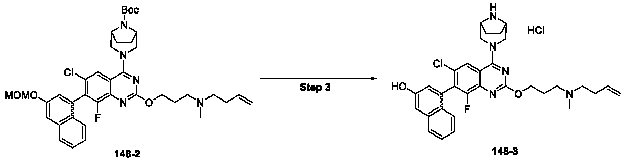

A solution of hydrochloric acid (4 mol/L, 3 mL) in 1,4-dioxane was added dropwise to a solution of compound **148-2** (210 mg, 0.27 mmol, 1.0 eq) in methanol (3 mL) with stirring at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 95% acetonitrile/water mobile phase (0.1% hydrochloric acid) in 20 min; detector, UV254/220 nm; to obtain compound **148-3** (yellow solid, 150 mg, yield: 83%). MS (ESI, m/z): 576.4/578.4 [M + H]⁺; ¹H NMR (300 MHz, DMSO-*d₆*) *δ* 10.46 (s, 1H), 9.91-9.86 (m, 1H), 9.64 (s, 1H), 7.99 (d, *J=* 1.6 Hz, 1H), 7.82 (d, *J=* 8.3 Hz, 1H), 7.50-7.41 (m, 1H), 7.31 (d, *J=* 2.4 Hz, 1H), 7.27-7.18 (m, 2H), 7.10 (d, *J=* 2.4 Hz, 1H), 5.85-5.71 (m, 1H), 5.21-5.09 (m, 2H), 4.56-4.43 (m, 4H), 4.19-4.16 (m, 2H), 3.99-3.87 (m, 4H), 3.32-3.07 (m, 4H), 2.78 (d, *J=* 4.8 Hz, 3H), 2.29-2.18 (m, 2H), 2.01-1.93 (m, 4H).

### Step 4

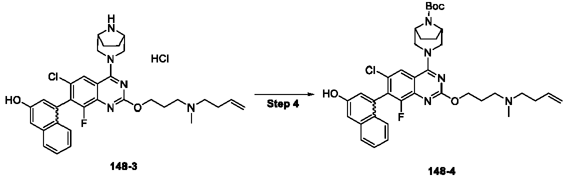

Triethylamine (110 mg, 1.00 mmol, 4.0 eq) and di-tert-butyl dicarbonate (71 mg, 0.30 mmol, 1.2 eq) were successively added to a solution of compound **148-3** (150 mg, 0.25 mmol, 1.0 eq) in dichloromethane (4 mL) with stirring at 25 °C. The reaction was carried out for 1 hour at 25 °C, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **148-4** (off-white solid, 100 mg, yield: 60%). MS (ESI, m/z): 676.3/678.3 [M + H]⁺.

### Step 5

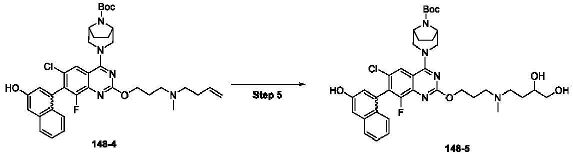

Water (1 mL), potassium osmium dihydrate (11 mg, 0.03 mmol, 0.1 eq) and *N-*methylmorpholine oxide (52 mg, 0.42 mmol, 1.5 eq) were successively added to a solution of **148-4** (200 mg, 0.28 mmol, 1.0 eq) in acetone (4 mL) with stirring at 0 °C. The reaction was carried out for 2 hours at 25 °C, and the reaction process was monitored by liquid chromatography-mass spectrometry. Ther reaction mixture was concentrated to obtain a crude product. The obtained crude product was purified by reversed-phase chromatographic column (C18 column), and eluted with 50% → 95% methanol/water mobile phase (0.1% formic acid) in 15 min; detector, UV254/220 nm; to obtain compound **148-5** (off white solid, 140 mg, yield: 63%). MS (ESI, m/z): 710.3/712.3 [M + H]⁺; ¹H NMR (300 MHz, DMSO-*d₆*) *δ* 10.02 (s, 1H), 8.00 (d, *J=* 1.7 Hz, 1H), 7.81 (d, *J=* 8.3 Hz, 1H), 7.48-7.42 (m, 1H), 7.29 (d, *J*= 2.4 Hz, 1H), 7.25-7.22 (m, 2H), 7.07 (d, *J=* 2.4 Hz, 1H), 4.84 (s, 2H), 4.47-4.34 (m, 4H), 4.29-4.26 (m, 2H), 3.59 (t, *J=* 11.8 Hz, 2H), 3.49-3.43 (m, 1H), 3.31-3.18 (m, 3H), 2.48-2.34 (m, 3H), 2.16 (s, 3H), 1.93-1.72 (m, 6H), 1.64-1.55 (m, 1H), 1.48 (s, 9H), 1.42-1.31 (m, 1H).

### Step 6

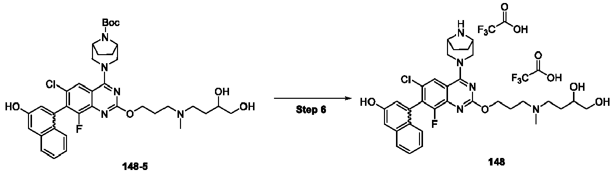

Trifluoroacetic acid (0.5 mL) was added dropwise to a solution of compound **148-5** (20 mg, 0.02 mmol, 1.0 eq) in dichloromethane (1.5 mL) with stirring at 25 °C. After the dropwise addition, the reaction was carried out at 25 °C for 1 hour, and the reaction process was monitored by liquid chromatography-mass spectrometry. After the reaction was completed, the reaction mixture was concentrated to obtain a crude product. The obtained crude product was prepared and purified by high performance liquid chromatography under the following conditions: chromatographic column: Xselect CSH Prep C18 OBD, 19 x 150 mm, 5 µm; mobile phase A: water (0.05% trifluoroacetic acid), mobile phase B: acetonitrile; flow rate: 25 mL/min; gradient: elution with 1% B in 2 min, then eluted with a gradient of 1% B to 6% B in 2.5 min, and finally eluted with a gradient of 6% B to 32% B in 9.5 min; detector UV 254/220 nm; retention time: 9.03 min. The obtained fractions were concentrated under reduced pressure to obtain compound **148** (yellow solid, 14.6 mg, yield: 53%). MS (ESI, m/z): 610.2/612.2 [M + H]⁺; ¹H NMR (300 MHz, DMSO-*d₆*) *δ* 10.10 (s, 1H), 9.43 (d, J = 9.6 Hz, 2H), 9.22 (s, 1H), 7.97 (d, *J=* 1.7 Hz, 1H), 7.81 (d, *J=* 8.3 Hz, 1H), 7.48-7.42 (m, 1H), 7.30 (d, *J=* 2.4 Hz, 1H), 7.27-7.16 (m, 2H), 7.05 (d, *J=* 2.4 Hz, 1H), 4.55-4.48 (m, 2H), 4.45-4.40 (m, 2H), 4.18-4.16 (m, 2H), 3.82-3.76 (m, 2H), 3.55-3.47 (m, 1H), 3.38-3.10 (m, 6H), 2.79 (d, *J=* 1.7 Hz, 3H), 2.19-2.08 (m, 2H), 2.01-1.93 (m, 4H), 1.89-1.79 (m, 1H), 1.69-1.58 (m, 1H); ¹⁹F NMR (282 MHz, DMSO-*d₆*) *δ*-74.01, 122.03.

### Embodiment 32 (Synthesis method XXVI)

### 4-((R or S)-4-((1R,5S)-3,8-diazabicyclo[3.2.1]octan-3-yl)-2-(3-(dimethylamino)propoxy)-8-fluoro-6-methylquinazolin-7-yl)naphthalen-2-ol hydrochloride 149a; 4-((S or R)-4-((1R,5S)-3,8-diazabicyclo[3.2.1]octan-3-yl)-2-(3-(dimethylamino)propoxy)-8-fluoro-6-methylquinazolin-7-yl) naphthalen-2-ol dihydrochloride 149b

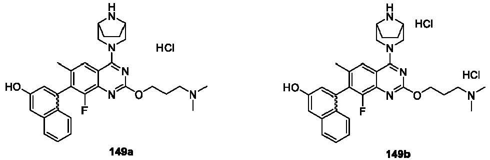

The synthetic route was as follows:

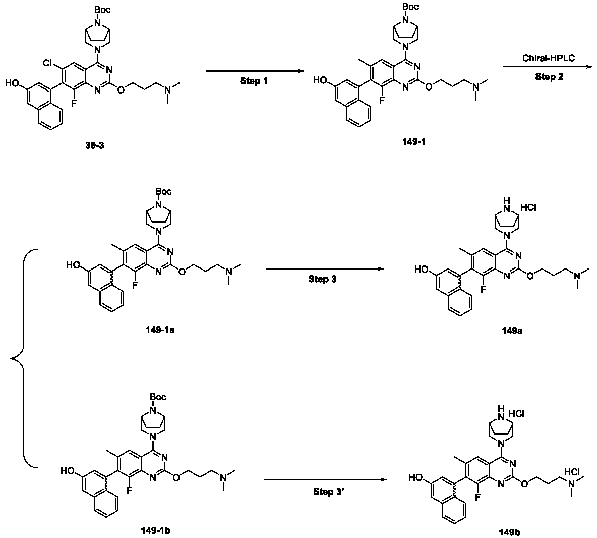

### Step 1

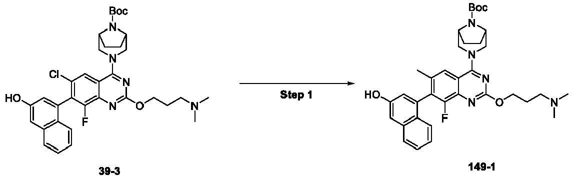

Under nitrogen protection at 25 °C, compound **39-3** (240 mg, 0.37 mmol, 1.0 eq), potassium carbonate (108 mg, 0.75 mmol, 2.0 eq), 2-dicyclohexylphosphine-2',4',6'-triisopropylbiphenyl (18 mg, 0.03 mmol, 0.1 eq), methanesulfonic acid (2-dicyclohexylphosphine-2',4',6'-triisopropyl-1,1'-biphenyl) (2'-amino-1,1'-biphenyl-2-yl) palladium (II) (33 mg, 0.03 mmol, 0.1 eq), 1,4 dioxane (5 mL) and trimethyl-1,3,5,2,4, 6-trioxyboron (186 mg, 0.75 mmol, 2.0 eq) were successively added to a 250 mL three-neck flask. The reaction was carried out for 2 hours at 100 °C under nitrogen atmosphere. The reaction process was monitored by liquid chromatography-mass spectrometry. After the reaction was completed, the reaction mixture was cooled to 25 °C, and concentrated to obtain a crude product. The obtained crude product was purified by preparative silica gel thin layer chromatography (elution solvent system: ethyl acetate/ammonia methanol solution (7 mol/L) = 15/1) to obtain compound **149-1** (yellow solid, 180 mg, yield: 77%). MS (ESI, m/z): 616.4 [M + H]⁺.

### Step 2

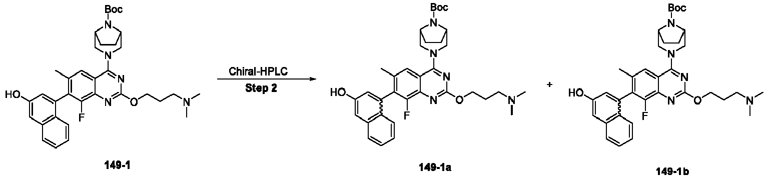

The compound **149-1** (180 mg) obtained in step 1 was subjected to chiral resolution by preparative chiral high pressure liquid chromatography under the following conditions: chiral column NB-Lux 5 µm i-Cellulose-5, 2.12 x 25 cm, 5 µm; mobile phase A: *n*-hexane (10 mmol/L ammonia methanol solution), mobile phase B: ethanol; flow rate: 18 mL/min; eluted with 50% mobile phase B in 20 min; detector UV 220 nm; two products were obtained. The compound with a shorter retention time (7.86 min) was **149-1a,** *tert*-butyl(1*R,*5*S*)-3-((*R* or *S*)-2-(3-(dimethylamino)propoxy)-8-fluoro-7-(3-hydroxynaphthalen-1-yl)-6-methylquinazolin-4-yl)-3,8-diazabicyclo[3.2.1]octan-8-carboxylate (white solid, 72 mg, recovery rate: 40%); the product with longer retention time (14.037 min) was **149-1b,** *tert*-butyl(1*R*,5*S*)-3-((*S* or R)-2-(3-(dimethylamino)propoxy)-8-fluoro-7-(3-hydroxynaphthalen-1-yl)-6-methylquinazolin-4-yl)-3,8-diazabicyclo[3.2.1]octane-8-carboxylate (white solid, 70 mg, recovery rate: 40%).

### Step 3

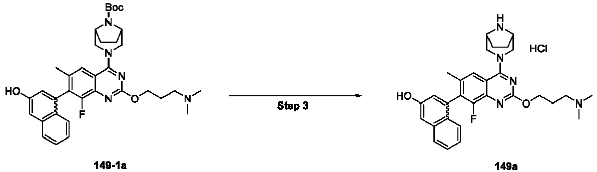

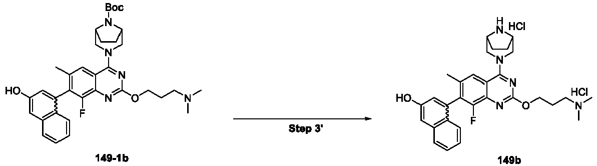

A solution of hydrochloric acid (4 mol/L, 3 mL) in 1,4-dioxane was added dropwise to a solution of compound **149-1a** (70 mg, 0.11 mmol, 1.0 eq) in methanol (3 mL) at 25 °C, after dropwise addition, the reaction mixture was stirred at this temperature for 1 hour, and the reaction process was monitored by liquid chromatography-mass spectrometry. After the reaction was completed, the reaction mixture was concentrated under reduced pressure to obtain a crude product. The crude product was purified by reversed-phase flash chromatography (C18 column), eluted with 5%→95% acetonitrile/water mobile phase (0.1% hydrochloric acid) in 30 min; detector, UV 254 nm; to obtain compound **149a** (white solid, 40.4 mg, yield: 62%). Compound **149b** (white solid, 38.5 mg, yield: 56%) can be obtained by the same method as above.
compound **149a:** MS (ESI, m/z): 516.4 [M + H]⁺; ¹H NMR (300 MHz, DMSO-*d₆*) *δ* 10.71 (s, 1H), 10.14 (s, 1H), 9.77 (s, 1H), 7.83-7.80 (m, 2H), 7.47-7.41 (m, 1H), 7.29 (d, *J=* 2.3 Hz, 1H), 7.24-7.18 (m, 1H), 7.13 (d, *J=* 8.3 Hz, 1H), 7.07-7.04 (m, 1H), 4.58-4.46 (m, 4H), 4.19-4.16 (m, 2H), 4.02-3.92 (m, 2H), 3.26-3.18 (m, 2H), 2.77 (d, *J=* 4.9 Hz, 6H), 2.25-2.17 (m, 2H), 2.06 (s, 3H), 2.03-1.96 (m, 4H); ¹⁹F NMR (282 MHz, DMSO-*d₆*) *δ*-126.80. The chiral analysis conditions of compound **149a** were: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 6 min; detector UV 220 nm; retention time: 2.457 min; ee > 99%.
compound **149b:** MS (ESI, m/z): 516.3 [M + H]⁺; ¹H NMR (300 MHz, DMSO-*d₆*) *δ* 10.55 (s, 1H), 10.22-9.99 (m, 2H), 9.69 (s, 1H), 7.83-7.80 (m, 2H), 7.47-7.41 (m, 1H), 7.29 (d, *J=* 2.4 Hz, 1H), 7.24-7.18 (m, 1H), 7.13 (d, *J=* 8.3 Hz, 1H), 7.06-7.04 (m, 1H), 4.56-4.45 (m, 4H), 4.18-4.16 (m, 2H), 3.98-3.90 (m, 2H), 3.26-3.18 (m, 2H), 2.77 (d, *J=* 4.8 Hz, 6H), 2.24-2.15 (m, 2H), 2.06 (s, 3H), 2.03-1.96 (m, 4H); ¹⁹F NMR (282 MHz, DMSO-*d₆*) *δ*-126.80. The chiral analysis conditions of compound **149b** were: CHIRALPAK IC-3, 4.6 x 50 mm, 3 µm; mobile phase A: n-hexane (0.1% diethylamine), mobile phase B: ethanol; flow rate: 1 mL/min; isocratic elution with 40% phase B in 6 min; detector UV 220 nm; retention time: 3.982 min; ee > 99%.

### Embodiment 33 (Synthesis method XXVII)

### 1-(3-((4-((1R,5S)-3,8-diazabicyclo[3.2.1]octan-3-yl)-6-chloro-8-fluoro-7-((S or R)-3-hydroxynaphthalen-1-yl)quinazolin-2-yl)oxy)propyl)guanidine diformate 150

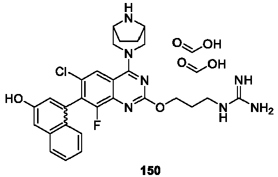

The synthetic route was as follows:

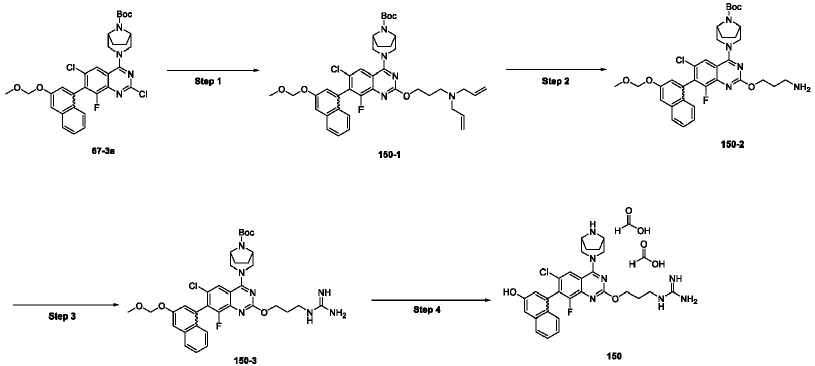

### Step 1

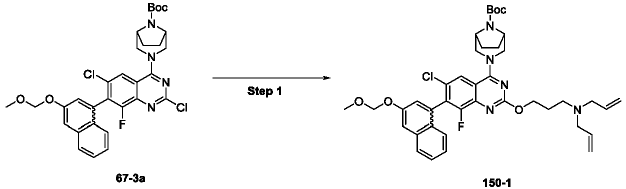

Potassium *tert*-butoxide (1 mol/L of tetrahydrofuran solution, 0.46 mL, 0.46 mmol, 1.3 eq) was added dropwise to a solution of **67-3a** (220 mg, 0.34 mmol, 1.0 eq) and 3-(diallylamino)propanol (72 mg, 0.46 mmol, 1.3 eq) in anhydrous tetrahydrofuran (2 mL) with stirring at 0 °C. After the dropwise addition, the reaction was carried out at 0 °C for 1 hour, and the reaction process was monitored by liquid chromatography-mass spectrometry. After the reaction was completed, 10 mL of water was added to the reaction mixture, and the mixture was extracted with ethyl acetate (10 mL x 3), then the organic phases were combined, dried over anhydrous sodium sulfate, filtered, and the filtrate was concentrated to obtain a crude product. The obtained crude product was purified by silica gel column chromatography, eluted with a gradient of 0%→5% methanol/dichloromethane mobile phase, and the obtained fractions were evaporated under reduced pressure to remove the solvent to obtain compound **150-1** (light yellow solid, 45 mg, yield: 42%). MS (ESI, m/z): 732.4/734.4 [M + H]⁺; ¹H NMR (400 MHz, CDCl₃) *δ* 7.83 (d, *J=* 8.2 Hz, 1H), 7.78 (d, *J=* 1.7 Hz, 1H), 7.54 (d, *J=* 2.4 Hz, 1H), 7.49-7.44 (m, 1H), 7.35 (d, *J=* 8.3 Hz, 1H), 7.30-7.28 (m, 1H), 7.20 (d, *J=* 2.4 Hz, 1H), 5.92-5.82 (m, 2H), 5.35-5.34 (m, 2H), 5.21-5.12 (m, 4H), 4.48 (t, *J=* 6.7 Hz, 2H), 4.43-4.36 (m, 4H), 3.66-3.54 (m, 5H), 3.18-3.12 (m, 4H), 2.70-2.65 (m, 2H), 2.06-1.98 (m, 4H), 1.90-1.86 (m, 2H), 1.53 (s, 9H).

### Step 2

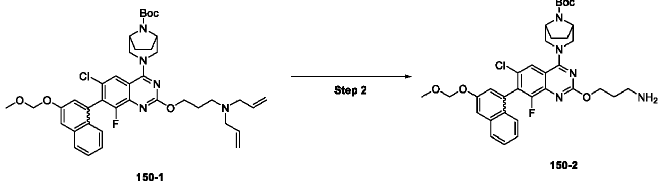

1, 3-Dimethyl-1,3-diazin-2,4,6-trione (115 mg, 0.73 mmol, 3.0 eq) and tetrakis(triphenylphosphine)palladium (28 mg, 0.02 mmol, 0.1 eq) were added to a solution of compound **150-1** (180 mg, 0.24 mmol, 1.0 eq) in dichloromethane (5 mL) with stirring under the protection of nitrogen at 25 °C. The reaction was carried out for 2 hours at 25 °C under nitrogen atmosphere, and the reaction process was monitored by liquid chromatography-mass spectrometry. After the reaction was completed, the reaction mixture was concentrated to obtain a crude product. The crude product was purified by reversed-phase flash chromatography (C18 column), eluted with 10%→95% acetonitrile/water mobile phase (0.1% formic acid) in 25 min; detector, UV 254 nm; to obtain compound **150-2** (orange solid, 82 mg, yield: 53%). MS (ESI, m/z): 652.4/654.4 [M + H]⁺; ¹H NMR (400 MHz, DMSO-*d₆*) *δ* 8.02 (d, *J=* 1.6 Hz, 1H), 7.94 (d, *J=* 8.3 Hz, 1H), 7.62 (d, *J*= 2.5 Hz, 1H), 7.55-7.51 (m, 1H), 7.37-7.33 (m, 1H), 7.31-7.26 (m, 2H), 5.38 (s, 2H), 4.45-4.38 (m, 4H), 4.28-4.26 (m, 2H), 3.65-3.53(m, 2H), 3.46 (s, 3H), 2.84 (t, *J* = 7.1 Hz, 2H), 1.98-1.91 (m, 2H), 1.85-1.81 (m, 2H), 1.74-1.71 (m, 2H), 1.47 (s, 9H).

### Step 3

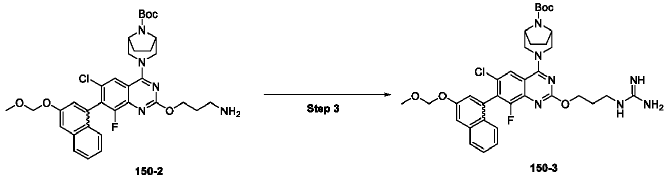

**150-2** (57 mg, 0.08 mmol, 1.0 eq), 1,2,4-triazol-1-carboxγimide (64 mg, 0.43 mmol, 5.0 eq), *N*,*N*-diisopropylethylamine (135 mg, 1.04 mmol, 12.0 eq) and *N*-methylpyrrolidone (1.0 mL) were added to a 25 mL Schlenk tube with stirring under the protection of nitrogen at 25 °C. The reaction was carried out for 2 hours at 25 °C under nitrogen atmosphere, and the reaction process was monitored by liquid chromatography-mass spectrometry. After the reaction was completed, the obtained crude product was directly purified by reversed-phase chromatographic column (C18 column), eluted with 30%→95% (methanol/acetonitrile=9:1 )/water (0.2% formic acid) mobile phase in 20 min; detector: UV254 nm; compound **150-3** (orange solid, 50 mg, yield: 86%) was obtained. MS (ESI, m/z): 694.3/696.3 [M + H]⁺.

### Step 4

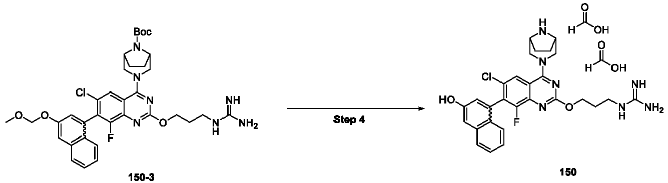

A solution of hydrochloric acid (4 mol/L, 2 mL) in 1,4-dioxane was added dropwise to a solution of compound **150-3** (50 mg, 0.07 mmol, 1.0 eq) in methanol (2 mL) with stirring at 0 °C. The reaction was carried out for 1 hour at 25 °C, and the reaction process was monitored by liquid chromatography-mass spectrometry. After the reaction was completed, the reaction mixture was concentrated to obtain a crude product. The obtained crude product was prepared and purified by high performance liquid chromatography under the following conditions: chromatographic column: Xselect CSH Prep C18 OBD, 19 x 150 mm, 5 µm; mobile phase A: water (0.1% formic acid), mobile phase B: acetonitrile; flow rate: 25 mL/min; gradient: elution with a gradient of 5% B to 26% B in 7 min; detector UV 254/220 nm; retention time: 5.85 min. The obtained fractions were concentrated under reduced pressure to obtain compound **150** (white solid, 32 mg, yield: 72%). MS (ESI, m/z): 550.3/552.3 [M + H]⁺; ¹H NMR (300 MHz, DMSO-*d₆*) *δ* 8.58 (s, 1H), 8.35 (s, 2H), 7.95 (d, *J=* 1.7 Hz, 1H), 7.81 (d, *J=* 8.3 Hz, 1H), 7.65-7.58 (m, 4H), 7.47-7.41 (m, 1H), 7.29 (d, *J=* 2.4 Hz, 1H), 7.23-7.21 (m, 2H), 7.07 (d, *J=* 2.4 Hz, 1H), 4.40-4.32 (m, 4H), 3.60-3.53 (m, 4H), 3.28-3.21 (m, 2H), 2.01-1.92 (m, 2H), 1.69-1.67 (m, 4H); ¹⁹F NMR (282 MHz, DMSO-*d₆*) *δ*-122.39.

### Embodiment 34 (Synthesis method XXVIII)

### 4-((S or R)-4-((1R,6S or 1S,6R)-3-azabicyclo[4.1.0]heptan-6-yl)-6-chloro-2-(3-(dimethylamino)azetidin-1-yl)-8-fluoroquinazolin-7-yl)naphthalen-2-ol ditrifluoroacetate 151a; 4-((S or R)-4-((1S,6R or 1R,6S)-3-azabicyclo[4.1.0]heptan-6-yl)-6-chloro-2-(3-(dimethylamino)azetidin-1-yl)-8-fluoroquinazolin-7-yl)naphthalen-2-ol 151b; 4-((R or S)-4-((1R,6S or 1S,6R)-3-azabicyclo[4.1.0]heptan-6-yl)-6-chloro-2-(3-(dimethylamino)azetidin-1-yl)-8-fluoroquinazolin-7-yl)naphthalen-2-ol 151c; 4-((R or S)-4-((1S,6R or 1R,6S)-3-azabicyclo[4.1.0]heptan-6-yl)-6-chloro-2-(3 -(dimethylamino)azetidin-1 -yl)-8-fluoroquinazolin-7-yl)naphthalen-2-ol 151d

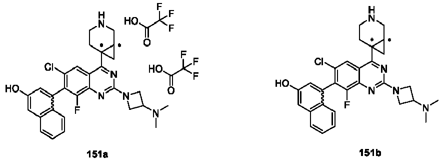

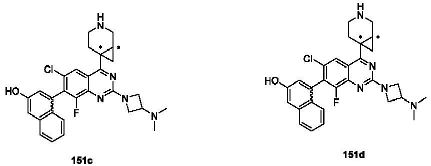

The synthetic route was as follows:

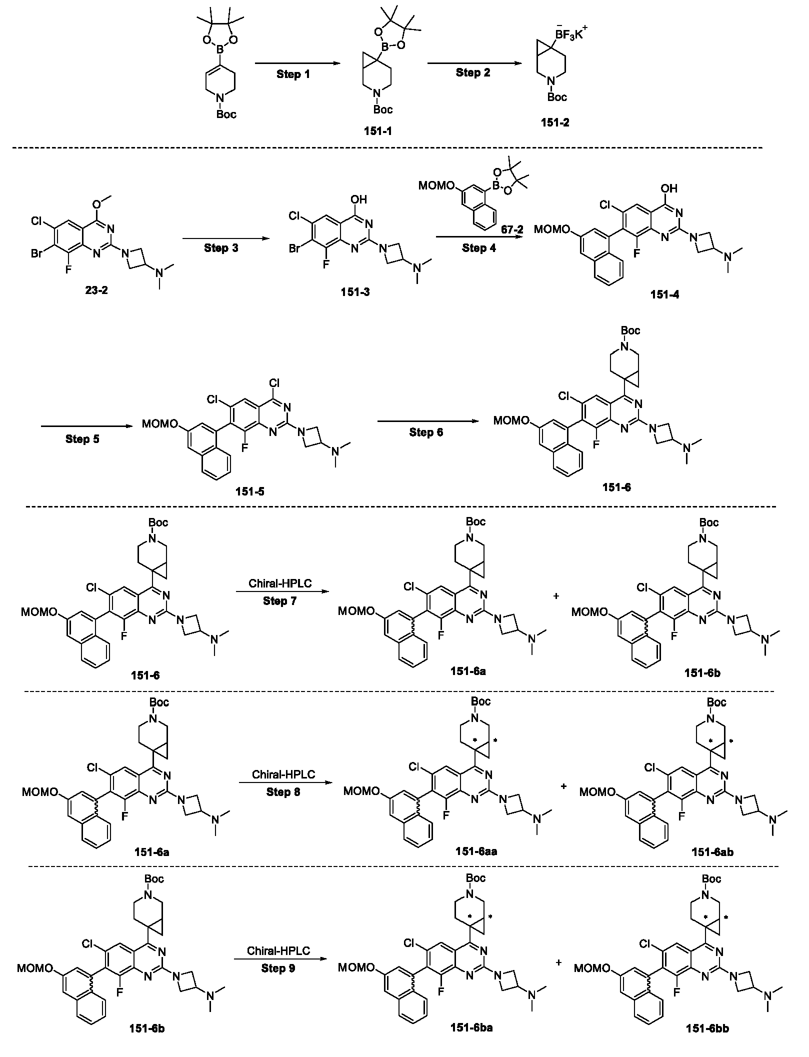

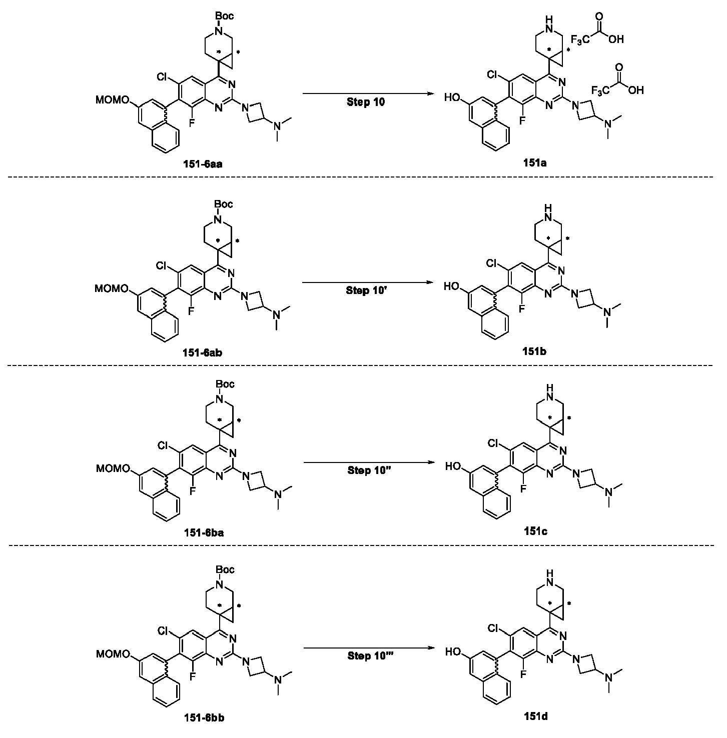

### Step 1

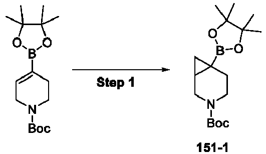

A solution of diiodomethane (69.3 g, 245.79 mmol, 16.0 eq) in dichloromethane (20 mL) was slowly dropwise added to a solution of diethylzinc (16 g, 122.89 mmol, 8.0 eq) in dichloromethane (250 mL) under the protection of nitrogen at -40 °C. The dropwise addition time was not less than 30 min. After the dropwise addition, the reaction mixture was carried out for 1 hour under nitrogen atmosphere at -40 °C. A solution of trifluoroacetic acid (14.8 g, 122.89 mmol, 8.0 eq) in dichloromethane (20 mL) was added dropwise to the reaction mixture while keeping the temperature constant over a period of not less than 20 min. After the dropwise addition, the reaction mixture was heated to-15 °C and the reaction was carried out at this temperature for 1 hour, then the mixture was kept at this temperature, and a solution of *N*-*tert*-butoxycarbonyl-1,2,5,6-tetrahydropyridin-4-boronic acid pinacol ester (5 g, 15.36 mmol, 1.0 eq) in dichloromethane (20 mL) was added dropwise to the reaction mixture. After the dropwise addition, the reaction mixture was carried out for 16 hours under nitrogen atmosphere at 25 °C. The volume of the reaction mixture was concentrated to a quarter of the original volume under reduced pressure, and tetrahydrofuran (70 mL), triethylamine (16 g, 153.62 mmol, 10.0 eq), 4-dimethylaminopyridine (493 mg, 3.84 mmol, 0.3 eq) and di-*tert-*butyl dicarbonate (17.6 g, 76.81 mmol, 5.0 eq) were added. The reaction was carried out for 3 hours at 25 °C, and the reaction process was monitored by thin layer chromatography. After the reaction was completed, 100 mL of water was added to the reaction mixture, the mixture was extracted with ethyl acetate (200 mL x 3), the organic phases were combined, and the organic phases were sequentially washed with saturated sodium bicarbonate (100 mL), water (100 mL) and saturated brine (100 mL), dried over anhydrous sodium sulfate, filtered, and the filtrate was concentrated to obtain a crude product. The crude product was purified by silica gel column chromatography, eluted with a gradient of 0% → 40% ethyl acetate/petroleum ether mobile phase, and the obtained fractions were evaporated under reduced pressure to remove the solvent to obtain compound **151-1** (yellow solid, 2.1 g, yield: 40%). MS (ESI, m/z): 268.1 [M + H-*^{t}*Bu]⁺; ¹H NMR (300 MHz, CDCl₃) *δ* 3.86 (dd, *J=* 13.6, 2.3 Hz, 1H), 3.52-3.41 (m, 2H), 2.92-2.82 (m, 1H), 2.13-2.04 (m, 1H), 1.63-1.57 (m, 1H), 1.46 (s, 9H), 1.22-1.16 (m, 13H), 0.93-0.88 (m, 1H), 0.45-0.41 (m, 1H).

### Step 2

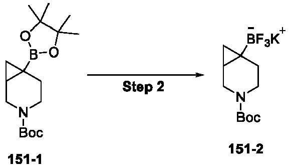

Potassium hydrofluoride (1.6 g, 20.57 mmol, 7.0 eq) was added to a solution of compound **151-1** (1 g, 2.94 mmol, 1.0 eq) in methanol (5 mL) with stirring at 25 °C. The reaction was carried out for 20 hours at 60 °C, and the reaction process was monitored by liquid chromatography-mass spectrometry. After the reaction was completed, the reaction mixture was cooled to 25 °C, and concentrated under reduced pressure to obtain a crude product. The crude product was dissolved with 20 mL of acetonitrile, filtered, and the filter cake was washed with acetonitrile (5 mL x 3), then the solid obtained by concentrating the filtrate was slurried with a mixed solution of n-hexane/acetone=10/1 (20 mL), and filtered to obtain compound **151-2** (white solid, 500 mg, 56%). ¹H NMR (400 MHz, DMSO-*d₆*) *δ* 3.48-3.41 (m, 2H), 3.12-3.04 (m, 1H), 2.91-2.85 (m, 1H), 1.80-1.71 (m, 1H), 1.38-1.26 (m, 10H), 0.62-0.55 (m, 1H), 0.23 (dd, *J* = 7.5, 2.8 Hz, 1H), -0.28 (s, 1H); ¹⁹F NMR (377 MHz, DMSO-*d₆*) *δ*-146.64; ¹¹B NMR (128 MHz, DMSO-*d₆*) *δ* 3.95.

### Step 3

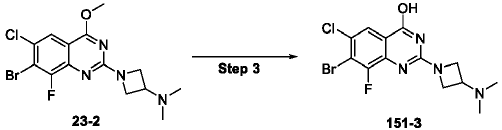

An aqueous solution of hydrochloric acid (4 mol/L, 3 mL) was added to a solution of compound **23-2** (1.2 g, 2.93 mmol, 1.0 eq) in tetrahydrofuran (15 mL) at 25 °C. The reaction was carried out for 1 hour at 80 °C, and the reaction process was monitored by liquid chromatography-mass spectrometry. After the reaction was completed, the reaction mixture was cooled to 25 °C, and concentrated under reduced pressure to obtain a crude product. The crude product was purified by reversed-phase flash chromatography (C18 column), eluted with 30%→70% acetonitrile/water mobile phase (0.1% ammonium bicarbonate) in 20 min; detector, UV 254/220 nm; to obtain compound **151-3** (yellow solid, 1 g, yield: 85%). MS (ESI, m/z): 375.0/377.0/379.0 [M + H]⁺; ¹H NMR (300 MHz, DMSO-*d₆*) *δ* 11.80 (s, 1H), 7.82 (d, *J* = 1.8 Hz, 1H), 4.18-4.15 (m, 2H), 3.94-3.89 (m, 2H), 3.20-3.12 (m, 1H), 2.12 (s, 6H).

### Step 4

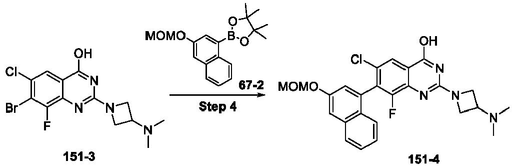

**67-2** (1.0 g, 3.03 mmol, 1.2 eq), potassium phosphate (1.1 g, 5.06 mmol, 2.0 eq) and chloro(2-dicyclohexylphosphino-2',4',6'-triisopropyl-1,1'-biphenyl)[2-(2'-amino-1,1'-biphenyl)]palladium(II) (209 mg, 0.25 mmol, 0.1 eq) were added to a solution of compound **151-3** (1.0 g, 2.53 mmol, 1.0 eq) in tetrahydrofuran/water (5/1, 20 mL) with stirring at 25 °C. The obtained reaction was carried out for 2 hours at 60 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **151-4** (yellow solid, 1 g, yield: 77%). MS (ESI, m/z): 483.2/485.2 [M + H]⁺; ¹H NMR (300 MHz, CDCl₃) *δ* 11.18 (s, 1H), 8.04 (d, *J* = 1.6 Hz, 1H), 7.85 (d, *J* = 8.2 Hz, 1H), 7.55 (d, *J* = 2.4 Hz, 1H), 7.51-7.46 (m, 1H), 7.43-7.28 (m, 2H), 7.22 (d, *J* = 2.4 Hz, 1H), 5.36 (s, 2H), 4.44-4.35 (m, 2H), 4.30-4.25 (m, 2H), 3.57 (s, 3H), 3.44-3.32 (m, 1H), 2.31 (s, 6H).

### Step 5

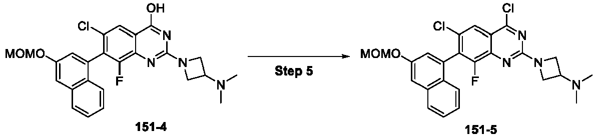

Trichloroacetonitrile (269 mg, 1.77 mmol, 1.5 eq) was added dropwise to a solution of compound **151-4** (600 mg, 1.18 mmol, 1.0 eq) and triphenylphosphine (977 mg, 3.54 mmol, 3.0 eq) in toluene (60 mL) under the protection of nitrogen at 100 °C. After the dropwise addition, the reaction was carried out at that temperature for 20 min and the reaction process was monitored by liquid chromatography-mass spectrometry. After the reaction was completed, the reaction mixture was cooled to 25 °C, and concentrated under reduced pressure to obtain a crude product. The crude product was purified by reversed-phase flash chromatography (C18 column), eluted with 10%→50% acetonitrile/water mobile phase (0.1% formic acid) in 15 min; detector, UV 254/220 nm; to obtain compound **151-5** (yellow solid, 600 mg, yield: 96%). MS (ESI, m/z): 501.1/503.1 [M + H]⁺; ¹H NMR (300 MHz, CDCl₃) *δ* 8.03 (d, *J* = 1.7 Hz, 1H), 7.85 (d, *J* = 8.3 Hz, 1H), 7.63-7.57 (m, 2H), 7.37-7.29 (m, 2H), 7.20 (d, *J* = 2.5 Hz, 1H), 5.36 (s, 2H), 4.46-4.29 (m, 2H), 4.25-4.15 (m, 2H), 3.57 (s, 3H), 3.40-3.28 (m, 1H), 2.30 (s, 6H).

### Step 6

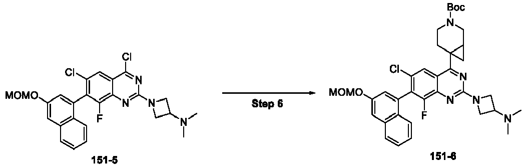

Compound **151-2** (136 mg, 0.42 mmol, 1.2 eq), cesium carbonate (244 mg, 0.71 mmol, 2.0 eq) and [1,1'-bis (diphenylphosphino)ferrocene]dichloropalladium (27 mg, 0.04 mmol, 0.1 eq) were added to a solution of compound **151-5** (600 mg, 0.35 mmol, 1.0 eq) in toluene/water (10/1, 10.0 mL) with stirring under the protection of nitrogen at 25 °C. The reaction was carried out for 3 hours at 110 °C under nitrogen atmosphere, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **151-6** (a mixture of four stereoisomers, yellow solid, 310 mg, yield: 39%). MS (ESI, m/z): 662.4/664.3 [M + H]⁺ ; ¹H NMR (300 MHz, CDCl₃) *δ* 7.98 (d, *J* = 1.6 Hz, 1H), 7.84 (d, *J* = 8.2 Hz, 1H), 7.55 (d, *J* = 2.4 Hz, 1H), 7.50-7.45 (m, 1H), 7.38 (d, *J* = 8.5 Hz, 1H), 7.32-7.27 (m, 1H), 7.22-7.20 (m, 1H), 5.35 (s, 2H), 4.35-4.29 (m, 2H), 4.18-4.07(m, 3H), 3.82-3.67 (m, 2H), 3.57 (s, 3H), 3.33-3.19 (m, 2H), 2.31-2.14 (m, 7H), 1.82-1.73 (m, 1H), 1.52 (s, 9H), 1.36-1.27 (m, 2H), 1.03-0.99 (m, 1H).

### Step 7

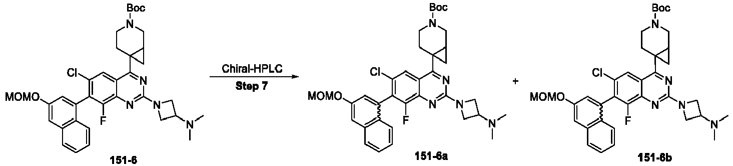

The compound **151-6** (300 mg) obtained in step 6 was subjected to chiral resolution by preparative chiral high performance liquid chromatography, and the resolution conditions were: chiral column CHIRAL ART Cellulose-SB, 2 × 25 cm, 5 µm; mobile phase A: *n*-hexane (10 mmol/L ammonia methanol solution), mobile phase B: isopropanol; flow rate: 20 mL/min; eluted with a gradient of 50% mobile phase B in 11 min; detector UV 225/254 nm, and two products were obtained. The product with shorter retention time (5.37 min) was **151-6a** (a mixture of two stereoisomers, yellow solid, 145 mg, recovery rate: 48%); the product with longer retention time (8.33 min) was **151-6b** (a mixture of two stereoisomers, yellow solid, 95 mg, recovery rate: 31%).

### Step 8

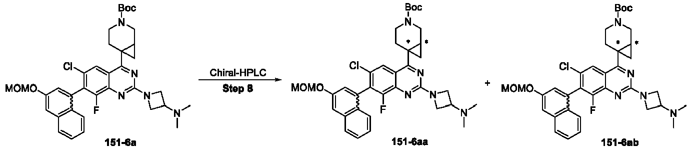

The compound **151-6a** (140 mg) obtained in step 7 was subjected to chiral resolution by preparative chiral high performance liquid chromatography, and the resolution conditions were: chiral column CHIRALCEL AY-H, 2 × 25 cm, 5 µm; mobile phase A: *n*-hexane (0.5% 2mol/L ammonia methanol solution), mobile phase B: isopropanol/acetonitrile = 2/1; flow rate: 20 mL/min; gradient: elution with a gradient of 30% mobile phase B in 13 min; detector UV 226/254 nm; two products were obtained. The product with shorter retention time (3.7 min) was **151-6aa,** *tert*-butyl(1*R*,6*S* or *1S,6R*)*-6-*((*S* or *R*)-6-chloro-2-(3-(dimethylamino))azetidin-1-yl)-8-fluoro-7-(3-(methoxymethoxy)naphthalen-1-yl)quinazolin-4-yl)-3- azabicyclo[ 4.1.0]heptan-3-carboxylate (yellow solid, 65 mg, recovery rate: 46%); the product with longer retention time (6.8 min) was **151-6ab,** *tert*-butyl (1*S,* 6*R* or 1*R*, 6*S*)-6-((*S* or *R*)-6-chloro-2-(3-(dimethylamino)azetidin-1-yl)-8-fluoro-7-(3-(methoxymethoxy)naphthalen-1-yl)quinazolin-4-yl)-3-azabicyclo[4.1.0]heptan-3-carboxylate (yellow solid, 45 mg, recovery rate: 32%).

### Step 9

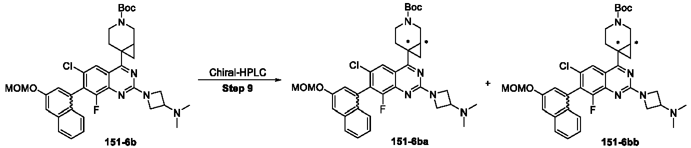

The compound **151-6b** (95 mg) obtained in step 7 was subjected to chiral resolution by preparative chiral high performance liquid chromatography, and the resolution conditions were: chiral column CHIRALPAK IE, 2 × 25 cm, 5 µm; mobile phase A: *n*-hexane (10mmol/L ammonia methanol solution), mobile phase B: isopropanol/acetonitrile = 2/1; flow rate: 20 mL/min; eluted with a gradient of 10% mobile phase B in 18 min; detector UV 226/254 nm; two products were obtained. The product with shorter retention time (6 min) was **151-6ba,** *tert*-butyl(1*R*,6*S* or 1*S,*6*R*)-6-((*R* or *S*)-6-chloro-2-(3-(dimethylamino))azetidin-1-yl)-8-fluoro-7-(3-(methoxymethoxy)naphthalen-1-yl)quinazolin-4-yl)-3-azabicyclo[ 4.1.0]heptan-3-carboxylate (yellow solid, 43 mg, recovery rate: 45%); the product with longer retention time (8.5 min) was **151-6bb,** *tert*-butyl (1*S,* 6*R* or 1*R*, 6*S*)-6-((*R* or *S*)-6-chloro-2-(3-(dimethylamino)azetidin-1-yl)-8-fluoro-7-(3-(methoxymethoxy)naphthalen-1-yl)quinazolin-4-yl)-3-azabicyclo[4.1.0]heptan-3-carboxylate (yellow solid, 30 mg, recovery rate: 31%).

### Step 10

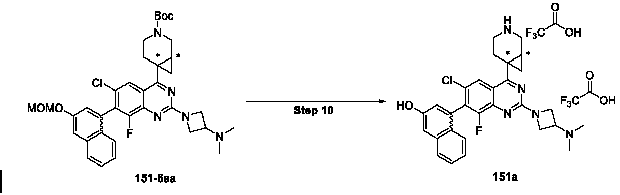

Trifluoroacetic acid (1.0 mL) and triethylsilylhydrogen (50 mg, 0.43 mmol, 5.0 eq) were added to a solution of compound **151-6aa** (60 mg, 0.09 mmol, 1.0 eq) in dichloromethane (4.0 mL) with stirring at 25 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 10% → 50% acetonitrile/water mobile phase (0.5% trifluoroacetic acid) in 15 min; detector, UV254/220 nm; to obtain compound **151a** (yellow solid, 22 mg, yield: 33%). Compound **151b** (yellow solid, 9.8 mg, yield: 27%), **151c** (yellow solid, 11.4 mg, yield: 29%) and **151d** (yellow solid, 12.6 mg, yield: 38%) were also obtained by the same method.
compound **151a:** MS (ESI, m/z): 518.2/520.1 [M + H]⁺; ¹H NMR (300 MHz, DMSO-*d₆*) *δ* 10.72 (s, 1H), 10.09 (s, 1H), 8.96 (s, 1H), 8.65 (s, 1H), 8.17 (d, *J* = 1.5 Hz, 1H), 7.83 (d, *J* = 8.3 Hz, 1H), 7.48-7.43 (m, 1H), 7.31 (d, *J* = 2.4 Hz, 1H), 7.26-7.15 (m, 2H), 7.07 (d, *J* = 2.4 Hz, 1H), 4.48-4.33 (m, 4H), 4.27-4.21 (m, 1H), 3.78-3.70 (m, 1H), 3.42-3.34 (m, 1H), 3.11-3.06 (m, 2H), 2.85 (s, 6H), 2.49-2.42 (m, 1H), 2.38-2.26 (m, 1H), 1.99-1.91 (m, 1H), 1.52-1.47 (m, 1H), 1.35-1.29 (m, 1H); ¹⁹F NMR (282 MHz, DMSO-*d₆*) *δ*-74.20, -123.24.
compound **151b:** MS (ESI, m/z): 518.2/520.1 [M + H]⁺; ¹H NMR (300 MHz, DMSO-*d₆*) *δ* 10.03 (s, 1H), 8.22 (d, *J* = 1.5 Hz, 1H), 7.81 (d, *J* = 8.3 Hz, 1H), 7.47-7.42 (m, 1H), 7.28 (d, *J* = 2.4 Hz, 1H), 7.23-7.21 (m, 2H), 7.06 (d, *J* = 2.4 Hz, 1H), 4.22-4.15 (m, 2H), 3.97-3.92 (m, 2H), 3.27-3.15 (m, 2H), 3.10-3.05 (m, 1H), 2.76-2.70 (m, 1H), 2.64-2.56 (m, 1H), 2.13 (s, 6H), 2.10-1.91 (m, 2H), 1.53-1.46 (m, 1H), 1.32-1.28 (m, 1H), 1.16-1.12 (m, 1H); ¹⁹F NMR (282 MHz, DMSO-*d₆*) *δ*-123.70.
compound **151c:** MS (ESI, m/z): 518.2/520.1 [M + H]⁺; ¹H NMR (300 MHz, DMSO-*d₆*) *δ* 10.02 (s, 1H), 8.22 (d, *J* = 1.5 Hz, 1H), 7.81 (d, *J* = 8.3 Hz, 1H), 7.47-7.41 (m, 1H), 7.29 (d, *J* = 2.4 Hz, 1H), 7.26-7.18 (m, 2H), 7.07 (d, *J* = 2.4 Hz, 1H), 4.22-4.16 (m, 2H), 3.97-3.91 (m, 2H), 3.29-3.15 (m, 2H), 3.10-3.05 (m, 1H), 2.76-2.70 (m, 1H), 2.64-2.56 (m, 1H), 2.13 (s, 6H), 2.12-2.01 (m, 1H), 1.99-1.90 (m, 1H), 1.55-1.49 (m, 1H), 1.31-1.24 (m, 1H), 1.17-1.12 (m, 1H); ¹⁹F NMR (282 MHz, DMSO-*d₆*) *δ*-123.67.
compound **151d:** MS (ESI, m/z): 518.2/520.1 [M + H]⁺; ¹HNMR (300 MHz, DMSO-*d₆*) *δ* 10.04 (s, 1H), 8.22 (d, *J* = 1.5 Hz, 1H), 7.81 (d, *J* = 8.3 Hz, 1H), 7.47-7.41 (m, 1H), 7.29 (d, *J* = 2.4 Hz, 1H), 7.23-7.21 (m, 2H), 7.06 (d, *J* = 2.4 Hz, 1H), 4.21-4.16 (m, 2H), 3.97-3.92 (m, 2H), 3.27-3.14 (m, 2H), 3.10-3.05 (m, 1H), 2.76-2.70 (m, 1H), 2.64-2.55 (m, 1H), 2.13 (s, 6H), 2.11-2.05 (m, 1H), 2.01-1.93 (m, 1H), 1.53-1.46 (m, 1H), 1.32-1.27 (m, 1H), 1.16-1.12 (m, 1H); ¹⁹F NMR (282 MHz, DMSO-*d₆*) *δ*-123.69.

### Embodiment 35

### 4-((S or R)-6-chloro-8-fluoro-2-((S)-1-methylpyrrolidin-2-yl)methoxy)-4-((1R,2R,5S,6S)-9,10-diazatricyclo[4.2.1.1^{2,5}]decan-9-yl)quinazolin-7-yl)naphthalen-2-ol dihydrochloride 152a; 4-((R or S )-6-chloro-8-fluoro-2-((S)-1-methylpyrrolidin-2-yl)methoxy)-4-((1R,2R,5S,6S)-9,10-diazatricyclo[4.2.1.1^{2,5}]decan-9-yl)quinazolin-7-yl)naphthalen-2-ol dihydrochloride 152b

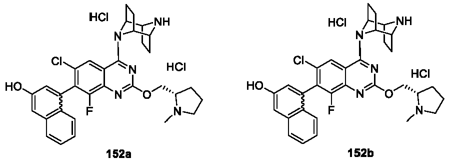

The synthetic route was as follows:

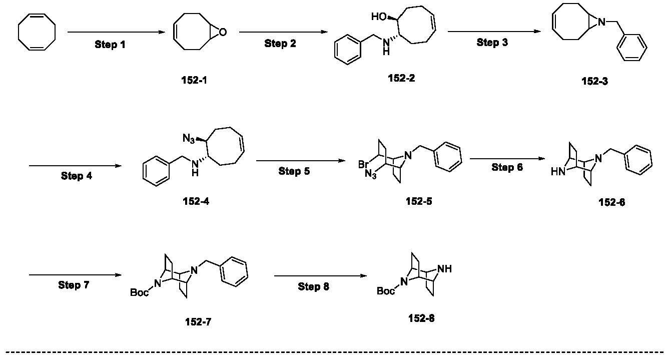

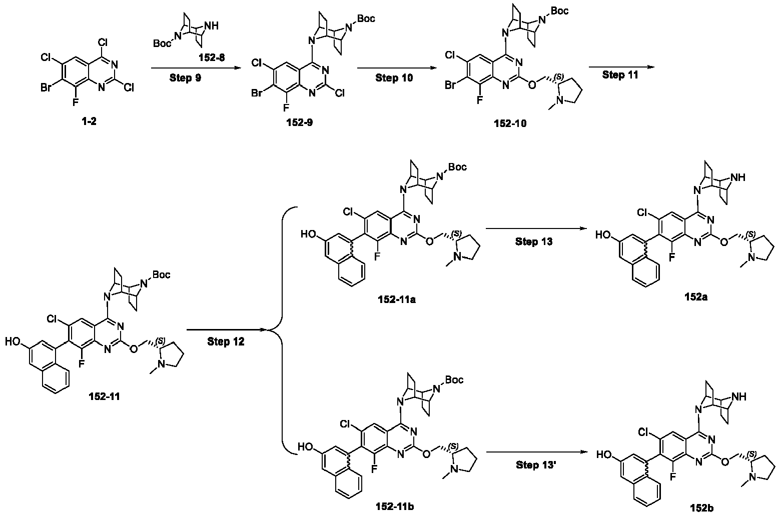

### Step 1

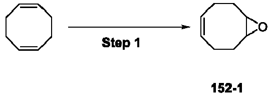

3-Chloroperoxybenzoic acid (89 g, 439.07 mmol, 1.0 eq) was added in batches to a solution of 1,5-cyclooctadiene (50 g, 439.07 mmol, 1.0 eq) in dichloromethane (1 L) with stirring at 0 °C. After the addition, the reaction was carried out for 16 hours at 25 °C, and the reaction process was monitored by liquid chromatography-mass spectrometry. After the reaction was completed, 500 mL of water was added, the mixture was extracted with ethyl acetate (500 mL × 3), and the organic phases were combined, washed sequentially with 500 mL of saturated sodium carbonate solution and 500 mL of saturated brine, then the organic phases were dried over anhydrous sodium sulfate, filtered and concentrated to obtain a crude product. The crude product was purified by silica gel column chromatography, eluted with a gradient of 0% → 10% ethyl acetate/petroleum ether mobile phase, and the obtained fractions were evaporated under reduced pressure to remove the solvent to obtain compound **152-1** (white solid, 18.8 g, yield: 32%). ¹H NMR (400 MHz, CDCl₃) *δ* 5.61-5.53 (m, 2H), 3.06-3.02 (m, 2H), 2.48-2.41 (m, 2H), 2.18-2.10 (m, 2H), 2.08-1.99 (m, 4H).

### Step 2

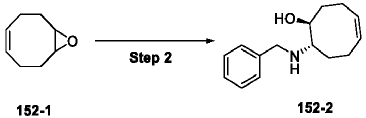

Ytterbium trifluoromethesulfonate (5.4 g, 8.45 mmol, 5%) and benzylamine (27 g, 253.65 mmol, 1.5 eq) were added to a solution of compound **152-1** (21 g, 160.65 mmol, 1.0 eq) in tetrahydrofuran (210 mL) with stirring under the protection of nitrogen at 25 °C. The reaction was carried out for 24 hours at 65 °C under nitrogen atmosphere, and the reaction process was monitored by liquid chromatography-mass spectrometry. After the reaction was completed, the reaction mixture was cooled to 25 °C, and concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **152-2** (white solid, 25 g, yield: 63%). MS (ESI, *m*/*z):* 232.1 [M + H]⁺; ¹H NMR (400 MHz, CDCl₃) *δ* 7.36-7.28 (m, 4H), 7.28-7.22 (m, 1H), 5.69-5.63 (m, 1H), 5.56-5.49 (m, 1H), 3.88 (d, *J* = 12.7 Hz, 1H), 3.72 (d, *J* = 12.7 Hz, 1H), 3.41-3.36 (m, 1H), 2.63-2.57 m, 1H), 2.44-2.33 (m, 1H), 2.28-1.99 (m, 5H), 1.46-1.33 (m, 2H).

### Step 3

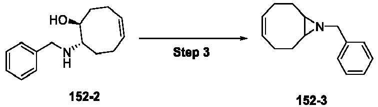

4-Dimethylaminopyridine (900 mg, 6.99 mmol, 0.1 eq), methylsulfonyl chloride (11 g, 90.73 mmol, 1.3 eq) and triethylamine (22 g, 209.45 mmol, 3.0 eq) were added to a solution of compound **152-2** (17 g, 69.81 mmol, 1.0 eq) in dichloromethane (340 mL) with stirring under the protection of nitrogen at 0 °C. After the addition, the reaction was carried out for 16 hours at 25 °C, and the reaction process was monitored by liquid chromatography-mass spectrometry. After the reaction was completed, 100 mL of water was added to the reaction mixture, the mixture was extracted with ethyl acetate (100 mL × 3), and the organic phases were combined, washed with 100 mL of saturated brine, dried over anhydrous sodium sulfate, filtered, and the filtrate was concentrated to obtain a crude product. The crude product was purified by silica gel column chromatography, eluted with a gradient of 0% → 60% ethyl acetate/petroleum ether mobile phase, and the obtained fractions were evaporated under reduced pressure to remove the solvent to obtain compound **152-3** (colorless oil, 11.4 g, yield: 72%). MS (ESI, *m*/*z):* 214.1 [M + H]⁺; ¹H NMR (400 MHz, CDCl₃) *δ* 7.36-7.30 (m, 4H), 7.25-7.22 (m, 1H), 5.60-5.52 (m, 2H), 3.54 (s, 2H), 2.45-2.36 (m, 2H), 2.15-2.07 (m, 2H), 2.05-1.96 (m, 2H), 1.94-1.86 (m, 2H), 1.62-1.56 (m, 2H).

### Step 4

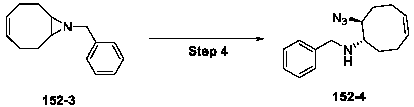

Water (100 mL), sodium azide (12.7 g, 185.26 mmol, 4.0 eq) and ammonium chloride (10.4 g, 185.26 mmol, 4.0 eq) were added to a solution of compound **152-3** (10.4 g, 46.32 mmol, 1.0 eq) in ethanol (150 mL) under the protection of nitrogen at 25 °C. The reaction was carried out for 6 hours at 80 °C under nitrogen atmosphere, and the reaction process was monitored by liquid chromatography-mass spectrometry. After the reaction was completed, the reaction mixture was cooled to 25 °C, and concentrated to obtain a crude product. The crude product was purified by silica gel column chromatography, eluted with a gradient of 0 → 60% ethyl acetate/petroleum ether mobile phase, and the obtained fractions were evaporated under reduced pressure to obtain compound **152-4.** (Bright yellow oil, 12.4 g, yield: 99%). MS (ESI, *m*/*z):* 257.2 [M + H]⁺; ¹H NMR (300 MHz, CDCl₃) *δ* 7.39-7.32 (m, 4H), 7.30-7.25 (m, 1H), 5.70-5.56 (m, 2H), 3.88 (d, *J* = 12.9 Hz, 1H), 3.74 (d, *J* = 12.9 Hz, 1H), 3.71-3.64 (m, 1H), 2.89-2.82 (m, 1H), 2.58-2.41 (m, 2H), 2.25-2.07 (m, 4H), 1.87-1.64 (m, 2H).

### Step 5

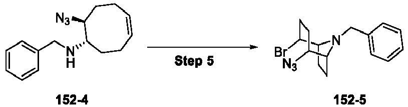

Liquid bromine (7.3 g, 45.53 mmol, 1.0 eq) was added dropwise to a solution of compound **152-4** (11.7 g, 43.36 mmol, 1.0 eq) in dichloromethane (100 mL) under the protection of nitrogen at -78 °C. After the dropwise addition, the reaction was carried out at that temperature for 2 hours. Then sodium bicarbonate (7.3 g, 86.72 mmol, 2.0 eq) was added in batches and the temperature was slowly raised to room temperature, the reaction was continued for 1 hour, and the reaction process was monitored by liquid chromatography-mass spectrometry. After the reaction was completed, 100 mL of water was added to the reaction mixture, and the mixture was extracted with dichloromethane (100 mL × 3), then the organic phases were combined, washed with 100 mL of saturated brine, dried over anhydrous sodium sulfate, filtered, and the filtrate was concentrated to obtain a crude product. The crude product was purified by silica gel column chromatography, eluted with a gradient of 0% → 40% ethyl acetate/petroleum ether mobile phase, and the obtained fractions were evaporated under reduced pressure to remove the solvent to obtain compound **152-5.** (Bright yellow oil, 4.3 g, yield: 14%). MS (ESI, *m*/*z):* 335.1/337.1 [M + H]⁺.

### Step 6

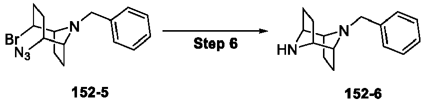

Triphenylphosphine (4.1 g, 14.96 mmol, 1.2 eq) was added in batches to a solution of compound **152-5** (4.3 g, 12.47 mmol, 1.0 eq) in anhydrous tetrahydrofuran (50 mL) with stirring under the protection of nitrogen at 0 °C. The reaction was carried out for 4 hours at 65 °C under nitrogen atmosphere, then cooled to 0 °C, 5 mL of water was added to the mixture. After the addition, the reaction was continued for 16 hours at 65 °C, and the reaction process was monitored by liquid chromatography-mass spectrometry. After the reaction was completed, the reaction mixture was cooled to room temperature, and concentrated to obtain a crude product. The obtained crude product was purified by silica gel column chromatography, eluted with a gradient of 0%→12% methanol/dichloromethane mobile phase, and the obtained fractions were evaporated under reduced pressure to remove the solvent to obtain compound **152-6.** (White solid, 3.2 g, yield: 56%). MS (ESI, m/z): 229.2 [M + H]⁺.

### Step 7

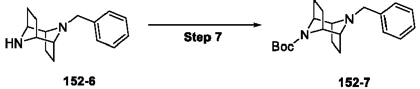

Triethylamine (1.5 g, 14.01 mmol, 2.0 eq) and di-*tert*-butyl dicarbonate (2.4 g, 10.51 mmol, 1.5 eq) were successively added to a solution of compound **152-6** (3.2 g, 7.01 mmol, 1.0 eq) in dichloromethane (40 mL) with stirring at 25 °C. The reaction was carried out for 2 hours at 25 °C, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 40% ethyl acetate/petroleum ether mobile phase, and the obtained fractions were evaporated under reduced pressure to obtain compound **152-7** (white solid, 1.6 g, yield: 66%). MS (ESI, m/z): 329.2 [M + H]⁺; ¹H NMR (400 MHz, CDCl₃) *δ* 7.39-7.37 (m, 2H), 7.34-7.30 (m, 2H), 7.27-7.23 (m, 1H), 4.04-4.02 (m, 1H), 3.94-3.91 (m, 1H), 2.94-2.89 (m, 2H), 2.04-2.01 (m, 2H), 1.88-1.78 (m, 4H), 1.61-1.55 (m, 4H), 1.46 (s, 9H).

### Step 8

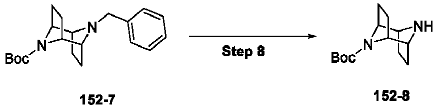

Anhydrous palladium carbon (185 mg, 0.87 mmol, 0.5 eq) and acetic acid (329 mg, 5.20 mmol, 3.0 eq) were added to a solution of compound **152-7** (600 mg, 1.74 mmol, 1.0 eq) in anhydrous ethanol (12 mL) under the protection of nitrogen at 25 °C. The reaction was carried out at 60 °C under hydrogen (10 atmospheric pressures) atmosphere for 4 hours, and the reaction process was monitored by liquid chromatography-mass spectrometry. After the reaction was completed, the mixture was filtered with diatomite, and the diatomite was washed with dichloromethane (20 mL × 3), then the filtrate was concentrated to obtain a crude product. The obtained crude product was purified by silica gel column chromatography, eluted with a gradient of 0%→12% methanol/dichloromethane mobile phase, and the obtained fractions were evaporated under reduced pressure to remove the solvent to obtain compound **152-8.** (White solid, 300 mg, yield: 68%). MS (ESI, m/z): 239.2 [M + H]⁺; ¹H NMR (400 MHz, CDCl₃) *δ* 4.08-3.95 (m, 2H), 3.28-3.21 (m, 2H), 2.15 (s, 1H), 2.00-1.88 (m, 4H), 1.75-1.72 (m, 2H), 1.65-1.62 (m, 2H), 1.46 (s, 9H).

### Step 9

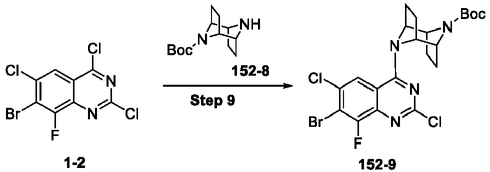

*N*,*N*-diisopropylethylamine (258 mg, 1.90 mmol, 3.0 eq) and compound **152-8** (159 mg, 0.63 mmol, 1.0 eq) were added to a solution of compound **1-2** (220 mg, 1.74 mmol, 1.0 eq) in dichloromethane (3 mL) with stirring under the protection of nitrogen at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→40% ethyl acetate/dichloromethane mobile phase, and the obtained fractions were evaporated under reduced pressure to remove the solvent to obtain compound **152-9** (yellow oil, 376 mg, yield: 99%). MS (ESI, *m*/*z):* 531.0/533.0/535.0 [M + H]⁺; ¹H NMR (300 MHz, CDCl₃) *δ* 7.89 (d, *J* = 2.0 Hz, 1H), 4.98-4.95 (m, 2H), 4.46-4.35 (m, 2H), 2.02 (m, 8H), 1.52 (s, 9H).

### Step 10

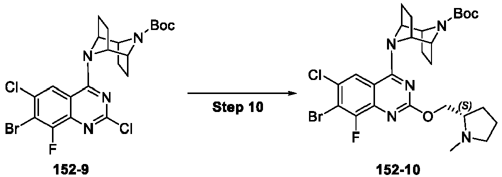

Potassium carbonate (327 mg, 2.25 mmol, 4.0 eq) and *N*-methyl-*L*-proline (204 mg, 1.69 mmol, 3.0 eq) were added to a solution of compound **152-9** (334 mg, 0.56 mmol, 1.0 eq) in acetonitrile (6 mL) with stirring at 25 °C. The obtained mixture was stirred for 16 hours at 80 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%--+ 12% methanol/dichloromethane mobile phase, and the obtained fractions were evaporated under reduced pressure to remove the solvent to obtain compound **152-10** (light yellow solid, 335 mg, yield: 88%). MS (ESI, m/z): 610.1/612.1/614.1 [M + H]⁺.

### Step 11

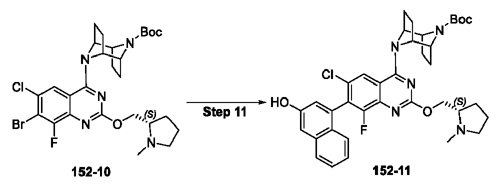

4-(4,4,5,5-Tetramethyl-1,3,2-dioxaborolan-2-yl)naphthalen-2-ol (176 mg, 0.62 mmol, 1.5 eq), potassium carbonate (120 mg, 0.82 mmol, 2.0 eq) and 1,1'-bis(diphenylphosphino)ferrocene-palladium(II)dichloride dichloromethane complex (35 mg, 0.04 mmol, 0.1 eq) were added to a solution of compound **152-10** (280 mg, 0.41 mmol, 1.0 eq) in 1,4-dioxane/water (5/1, 3 mL) with stirring under the protection of nitrogen at 25 °C. The reaction was carried out with stirring for 1 hour at 80 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→12% methanol/dichloromethane mobile phase, and the obtained fractions were evaporated under reduced pressure to remove the solvent to obtain compound **152-11** (a mixture of two stereoisomers, yellow solid, 287 mg, yield: 96%). MS (ESI, *m*/*z*): 674.3/676.3 [M + H]⁺.

### Step 12

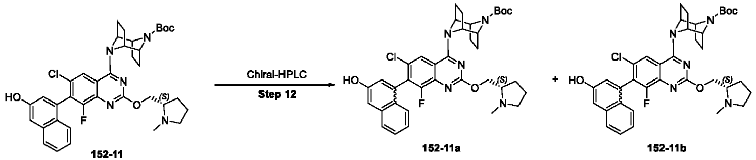

The compound **152-11** (287 mg) obtained in step 11 was subjected to chiral resolution by preparative chiral high pressure liquid chromatography under the following conditions: chiral column CHIRAL ART Cellulose-SC, 2.12 × 25 cm, 5 µm ; mobile phase A: *n-*hexane/dichloromethane = 5/1 (0.5% 2 mol/L ammonia methanol solution), mobile phase B: ethanol; flow rate: 20 mL/min; eluted with 10% phase B in 15 min; detector UV 210/207 nm; two products were obtained. The product with shorter retention time (4.23 min) was compound **152-11a,** *tert*-butyl(1*R*,2*R*,5*S*,6*S*)-10-((*S* or *R*)-6-chloro-8-fluoro-7-(3-hydroxynaphthalen-1-yl)-2-((*S*)-1-methylpyrrolidin-2-yl)methoxy)quinazolin-4-yl)-9,10-diazotricyclo[4.2.1.1^{2,5}]decane-9-carboxylate (white solid, 64 mg, recovery rate: 22%); the product with longer retention time (9.70 min) was compound **152-11b,** *tert*-butyl (1*R,*2*R,*5*S,*6*S*)-10-((*S* or *R*)-6-chloro-8-fluoro-7-(3-hydroxynaphthalen-1-yl)-2-((*S*)-1-methylpyrrolidin-2-yl)methoxy)quinazolin-4-yl)-9,10-diazotricyclo[4.2.1.1^{2,5}]decane-9-carboxylate (white solid, 72 mg, recovery rate: 26%).

### Step 13

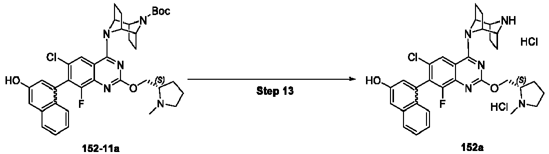

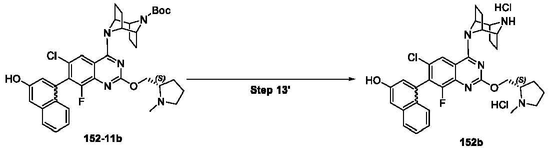

A solution of hydrochloric acid (4 mol/L, 2 mL) in 1,4-dioxane was added dropwise to a solution of the compound **152-11a** (64 mg, 0.09 mmol, 1.0 eq) in methanol (2 mL) with stirring at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 40% acetonitrile/water mobile phase (0.1% hydrochloric acid) in 20 min; detector, UV 254/220 nm; to obtain compound **152a** (yellow solid, 21 mg, yield: 34%). Compound **152b** (white solid, 50 mg, yield: 72%) can be obtained by the same method as above.
compound **152a:** MS (ESI, m/z): 574.2/576.2 [M + H]⁺; ¹H NMR (400 MHz, DMSO-*d₆*) *δ* 10.75 (s, 1H), 10.13 (s, 1H), 9.97-9.93 (m, 1H), 8.76-8.72 (m, 1H), 8.13 (d, *J* = 1.5 Hz, 1H), 7.82 (d, *J* = 8.3 Hz, 1H), 7.47-7.43 (m, 1H), 7.31 (d, *J* = 2.4 Hz, 1H), 7.25-7.18 (m, 2H), 7.10 (d, *J* = 2.4 Hz, 1H), 5.06-5.03 (m, 2H), 4.77-4.64 (m, 2H), 4.20-4.16 (m, 2H), 3.90-3.86 (m, 1H), 3.62-3.54 (m, 1H), 3.15-3.06 (m, 1H), 2.93 (d, *J* = 4.8 Hz, 3H), 2.33-2.19 (m, 5H), 2.08-1.82 (m, 7H); ¹⁹F NMR (377 MHz, DMSO-*d₆*) *δ*-122.37. The chiral analysis conditions of compound **152a** were: CHIRALPAK IC-3, 4.6 × 50 mm, 3 µm; mobile phase A: *n-*hexane/dichloromethane=5/1 (0.1% diethylamine), mobile phase B: ethanol; flow rate: 1 mL/min; isocratic elution with 10% phase B in 6 min; detector UV 220 nm; retention time: 3.123 min;. ee > 99%.
compound **152b:** MS (ESI, m/z): 574.2/576.2 [M + H]⁺; ¹H NMR (400 MHz, DMSO-*d₆*) *δ* 10.96-10.81 (m, 1H), 10.13-9.92 (m,2H), 8.82-8.75 (m, 1H), 8.13 (d, *J* = 1.6 Hz, 1H), 7.82 (d, *J* = 8.3 Hz, 1H), 7.47-7.43 (m, 1H), 7.32 (d, *J* = 2.4 Hz, 1H), 7.26-7.18 (m, 2H), 7.11 (q, *J* = 2.4 Hz, 1H), 5.07-5.03 (m, 2H), 4.79-4.64 (m, 2H), 4.19-4.17 (m, 2H), 3.87-3.78 (m, 1H), 3.61-3.54 (m, 1H), 3.14-3.05 (m, 1H), 2.92 (d, *J* = 4.8 Hz, 3H), 2.34-2.13 (m, 5H), 2.08-1.80 (m, 7H); ¹⁹F NMR (377 MHz, DMSO-*d₆*) *δ*-122.37. The chiral analysis conditions of compound **152b** were: CHIRALPAK IC-3, 4.6 × 50 mm, 3 µm; mobile phase A: *n-*hexane/dichloromethane=5/1 (0.1% diethylamine), mobile phase B: ethanol; flow rate: 1 mL/min; isocratic elution with 10% phase B in 6 min; detector UV 220 nm; retention time: 5.171 min. ee > 99%.

### Embodiment 36

### 2-((S)-4-((S or R)-6-chloro-8-fluoro-7-(3-hydroxynaphthalen-1-yl)-2-((S)-1-methylpyrrolidin-2-yl)methoxy)quinazolin-4-yl)piperazin-2-yl)acetonitrile 153a; 2-((S)-4-((R or S)-6-chloro-8-fluoro-7-(3-hydroxynaphthalen-1-yl)-2-((S)-1-methylpyrrolidin-2-yl)methoxy)quinazolin-4-yl)piperazin-2-yl) acetonitrile 153b

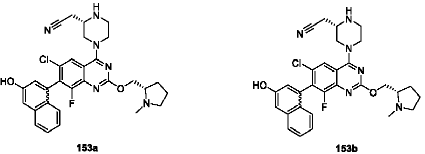

The synthetic route was as follows:

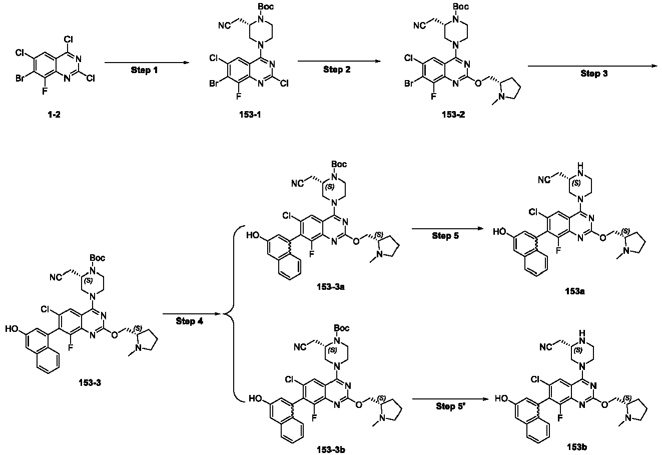

### Step 1

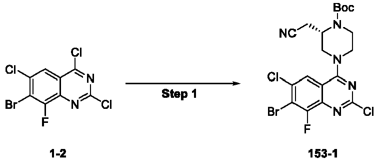

*N*,*N*-diisopropylethylamine (1.2 g, 9.08 mmol, 3 eq) and *tert*-butyl (2*S*)-2-(cyanomethyl)-1-piperazinecarboxylate (0.68 mg, 3.02 mmol, 1.1 eq) were added to a solution of the compound **1-2** (1 g, 2.87 mmol, 1.0 eq) in dichloromethane (10 mL) with stirring under the protection of nitrogen at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by silica gel column chromatography, eluted with a gradient of 0%→100% ethyl acetate/dichloromethane mobile phase, and the obtained fractions were evaporated under reduced pressure to remove the solvent to obtain compound **153-1** (yellow oil, 1.5 g, yield: 95%). MS (ESI, *m*/*z*): 518.1/520.1/522.1 [M + H]⁺; ¹H NMR (400 MHz, CDCl₃) *δ* 7.81 (d, *J* = 2.0 Hz, 1H), 4.68-4.63 (m, 1H), 4.38-4.33 (m, 1H), 4.29-4.24 (m, 1H), 4.17-4.09 (m, 1H), 3.82-3.77 (m, 1H), 3.65-3.58 (m, 1H), 3.47-3.41 (m, 1H), 2.89-2.82 (m, 1H), 2.74-2.68 (m, 1H), 1.51 (s, 9H).

### Step 2

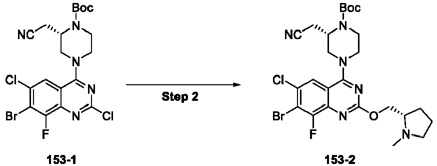

Potassium carbonate (798 mg, 5.49 mmol, 2.0 eq) and *N*-methyl-*L*-proline (499 mg, 4.12 mmol, 1.5 eq) were added to a solution of compound **153-1** (1.5 g, 2.74 mmol, 1.0 eq) in acetonitrile (13 mL) with stirring at 25 °C. The obtained mixture was stirred for 16 hours at 85 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→15% methanol/dichloromethane mobile phase, and the obtained fractions were evaporated under reduced pressure to remove the solvent to obtain compound **153-2** (light yellow solid, 1.43 g, yield: 82%). MS (ESI, m/z): 597.1/599.1/601.1 [M + H]⁺; ¹H NMR (400 MHz, CDCl₃) *δ* 7.72 (d, *J* = 1.9 Hz, 1H), 4.67-4.61 (m, 2H), 4.42-4.36 (m, 1H), 4.28-4.22 (m, 1H), 4.19-4.09 (m, 2H), 3.65-3.60 (m, 1H), 3.43-3.38 (m, 2H), 3.23-3.18 (m, 1H), 2.88-2.71 (m, 3H), 2.58 (s, 3H), 2.40-2.34 (m, 1H), 2.13-2.06 (m, 1H), 1.93-1.79 (m, 3H), 1.51 (s, 9H).

### Step 3

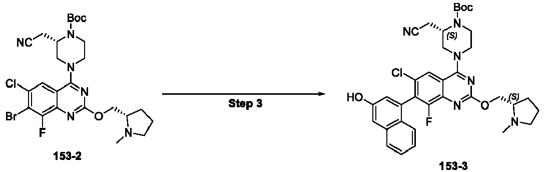

4-(4,4,5,5-Tetramethyl-1,3,2-dioxaborolan-2-yl)naphthalen-2-ol (881 mg, 3.26 mmol, 1.5 eq), potassium carbonate (600 mg, 4.35 mmol, 2.0 eq) and 1,1'-bis(diphenylphosphino)ferrocene-palladium(II)dichloride dichloromethane complex (177 mg, 0.21 mmol, 0.1 eq) were added to a solution of compound **153-2** (1.3 g, 2.17 mmol, 1 eq) in 1,4-dioxane/water (5/1, 12 mL) with stirring under the protection of nitrogen at 25 °C. The reaction was carried out with stirring for 10 hours at 80 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→15% methanol/dichloromethane mobile phase, and the obtained fractions were evaporated under reduced pressure to remove the solvent to obtain compound **153-3** (a mixture of two stereoisomers, yellow solid, 653 mg, yield: 45%). MS (ESI, *m*/*z*): 661.4/663.4 [M + H]⁺.

### Step 4

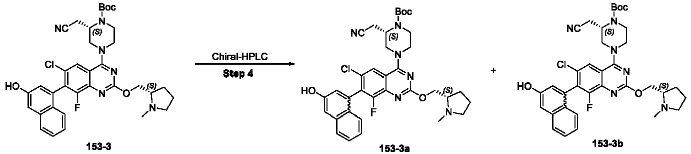

The compound **153-3** (500 mg) obtained in step 3 was subjected to chiral resolution by preparative chiral high pressure liquid chromatography under the following conditions: chiral column CHIRAL ART Cellulose-SC, 2.12 × 25 cm, 5 µm ; mobile phase A: *n*-hexane (10 mol/L ammonia methanol solution), mobile phase B: ethanol; flow rate: 17 mL/min; eluted with 50% phase B in 16 min; detector UV 220/254 nm. Two products were obtained. The product with shorter retention time (5.03 min) was compound **153-3a,** *tert*-butyl (*S*)-4-((*S* or *R*)-6-chloro-8-fluoro-7-(3-hydroxynaphthalen-1-yl)-2-((*S*)-1-methylpyrrolidin-2-yl)methoxy)quinazolin-4-yl)-2-(cyanomethyl)piperazin-1-carboxylate (light yellow solid, 196 mg, recovery rate: 38%); the product with longer retention time (14.08 min) was compound **153-3b,** *tert*-butyl (*S*)-4-((*R* or *S*)-6-chloro-8- fluoro-7 -(3 -hydroxynaphthalen-1-yl)-2-((*S*)-1-methylpyrrolidin-2-yl)methoxy)quinazolin-4-yl)-2-(cyanomethyl)piperazin-1-carboxylate (light yellow solid, 269 mg, recovery rate: 52%).

Compound **153-3a:** MS (ESI, m/z): 661.4/663.4 [M+H]⁺.

Compound **153-3b:** MS (ESI, m/z): 661.4/663.3 [M+H]⁺.

### Step 5

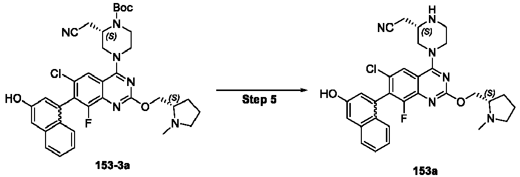

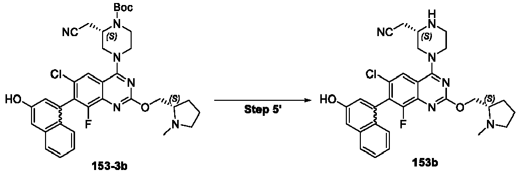

Trifluoroacetic acid (1 mL) was added dropwise to a solution of **153-3a** (196 mg, 0.29 mmol, 1.0 eq) in dichloromethane (3 mL) at 25 °C, after the dropwise addition, the reaction mixture was stirred at this temperature for 1 hour, and the reaction process was monitored by liquid chromatography-mass spectrometry. After the reaction was completed, the reaction mixture was concentrated under reduced pressure to obtain a crude product. The crude product was purified by reversed-phase flash chromatography (C18 column), eluted with 5%→95% methanol/water mobile phase (0.1% ammonium bicarbonate) in 20 min; detector, UV 254/220 nm; to obtain compound **153a** (white solid, 90.0 mg, yield: 44%). Compound **153b** (white solid, 90.0 mg, yield: 35%) can be obtained by the same method as above.
compound **153a:** MS (ESI, m/z): 561.2/563.2 [M + H]⁺; ¹H NMR (400 MHz, DMSO-*d₆*) *δ* 10.04 (s, 1H), 7.96 (s, 1H), 7.81 (d, *J* = 8.3 Hz, 1H), 7.46-7.42 (m, 1H), 7.29 (d, *J* = 2.5 Hz, 1H), 7.24-7.20 (m, 2H), 7.07 (d, *J* = 2.5 Hz, 1H), 4.43-4.38 (m, 1H), 4.31-4.28 (m, 1H), 4.20-4.16 (m, 2H), 3.36-3.29 (m, 1H), 3.14-3.03 (m, 3H), 2.98-2.86 (m, 2H), 2.77-2.74 (m, 2H), 2.63-2.56 (m, 2H), 2.36 (s, 3H), 2.22-2.15 (m, 1H), 2.00-1.90 (m, 1H), 1.71-1.61 (m, 3H); ¹⁹F NMR (377 MHz, DMSO-*d₆*) *δ*-122.34. The chiral analysis conditions of compound **153a** were: CHIRALPAK ID-3, 4.6 × 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 1.508 min; ee > 99%.
compound **153b:** MS (ESI, m/z): 561.2/563.2 [M + H]⁺; ¹H NMR (400 MHz, DMSO-*d₆*) *δ* 10.02 (s, 1H), 7.96 (d, *J* = 1.4 Hz, 1H), 7.81 (d, *J* = 8.3 Hz, 1H), 7.47-7.42 (m, 1H), 7.29 (d, *J* = 2.4 Hz, 1H), 7.26-7.19 (m, 2H), 7.07 (d, *J* = 2.4 Hz, 1H), 4.41-4.37 (m, 1H), 4.29-4.26 (m, 1H), 4.21-4.16 (m, 2H), 3.33-3.27 (m, 1H), 3.16-3.03 (m, 3H), 2.97-2.87 (m, 2H), 2.77-2.74 (m, 3H), 2.62-2.55 (m, 1H), 2.35 (s, 3H), 2.21-2.13 (m, 1H), 1.99-1.90 (m, 1H), 1.72-1.61 (m, 3H); ¹⁹F NMR (377 MHz, DMSO-*d₆*) *δ*-122.31. The chiral analysis conditions of compound **153b** were: CHIRALPAK ID-3, 4.6 × 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 2.593 min; ee > 98%.

### Embodiment 37 (Synthesis method XXIX)

### 4-((S or R)-4-((1R,5S)-3,8-diazabicyclo[3.2.1]octan-3-yl)-6-chloro-2-(4-(dimethylamino)butyl)-8-fluoroquinazolin-7-yl)naphthalen-2-ol dihydrochloride 154a; 4-((R or S)-4-((1R,5S)-3, 8-diazabicyclo[3.2.1]octan-3-yl)-6-chloro-2-(4-(dimethylamino)butyl)-8-fluoroquinazolin-7-yl)naphthalen-2-ol dihydrochloride 154b

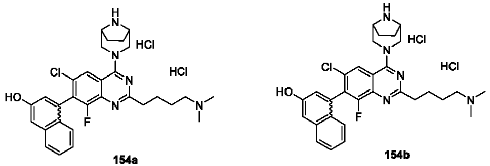

The synthetic route was as follows:

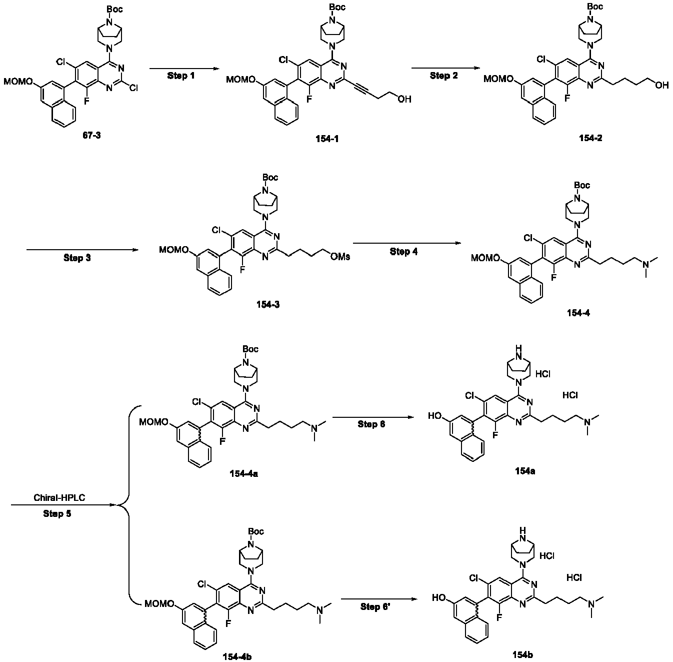

### Step 1

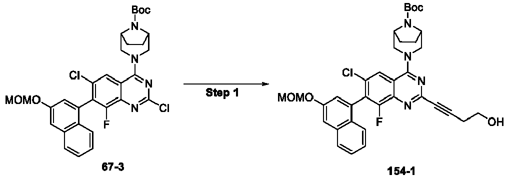

Compound **67-3** (850 mg, 1.31 mmol, 1.0 eq), triethylamine (10 mL), 3-butyn-1-ol (120 mg, 1.71 mmol, 1.3 eq), bis-triphenylpalladium phosphate dichloride (74 mg, 0.10 mmol, 0.08 eq) and cuprous iodide (15 mg, 0.08 mmol, 0.06 eq) were successively added to a 25 mL three-neck flask under the protection of nitrogen at 25 °C. The reaction was carried out for 16 hours at 80 °C under nitrogen atmosphere. The reaction process was monitored by liquid chromatography-mass spectrometry. After the reaction was completed, the reaction mixture was cooled to 25 °C, and concentrated to obtain a crude product. The crude product was purified by silica gel column chromatography, eluted with a gradient of 0% → 70% ethyl acetate/petroleum ether mobile phase, and the obtained fractions were evaporated under reduced pressure to remove the solvent to obtain compound **154-1** (orange solid, 660 mg, yield: 73%). MS (ESI, m/z): 647.3/649.3 [M + H]⁺; ¹H NMR (300 MHz, CDCl₃) *δ* 7.88-7.83 (m, 2H), 7.57 (d, *J* = 2.4 Hz, 1H), 7.52-7.46 (m, 1H), 7.33-7.30 (m, 2H), 7.21 (d, *J* = 2.4 Hz, 1H), 5.36 (s, 2H), 4.53-4.40(m, 4H), 3.91 (t, *J* = 6.2 Hz, 2H), 3.70-3.64 (m, 2H), 3.58 (s, 3H), 2.77 (t, *J* = 6.2 Hz, 2H), 2.04-1.99 (m, 2H), 1.88-1.83 (m, 2H), 1.55 (s, 9H).

### Step 2

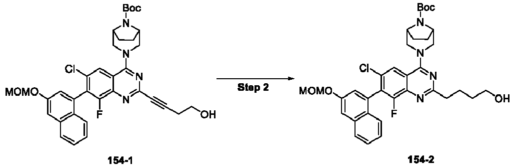

Anhydrous palladium carbon (10% palladium content, 50 mg) was added to a solution of compound **154-1** (250 mg, 0.37 mmol, 1.0 eq) in ethanol (3 mL) with stirring under the protection of nitrogen at 20 °C. The reaction was carried out for 2.5 hour at 20 °C under hydrogen atmosphere, and the reaction process was monitored by liquid chromatography-mass spectrometry. After the reaction was completed, the reaction mixture was cooled to 20 °C, filtered through diatomite, and the filter cake was washed with ethanol (30 mL × 3), and the filtrate was concentrated to obtain a crude product of compound **154-2** (orange solid, 300 mg). The crude product was used directly in the next synthesis without further purification. MS (ESI, m/z): 651.3/653.3 [M + H]⁺.

### Step 3

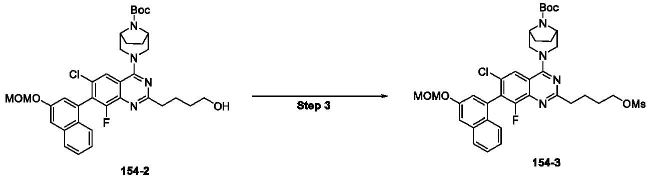

Triethylamine (88 mg, 0.83 mmol) and methylsulfonyl chloride (80 mg, 0.66 mmol) were successively added to a solution of compound **154-2** (300 mg) in dichloromethane (3 mL) with stirring under the protection of nitrogen at 20 °C. The reaction was carried out for 2 hours at 20 °C under nitrogen atmosphere, and the reaction process was monitored by liquid chromatography-mass spectrometry. After the reaction was completed, the reaction was quenched with 20 mL of water, the mixture was extracted with dichloromethane (20 mL × 3), and the organic phases were combined, washed with saturated brine, dried over anhydrous sodium sulfate, filtered, and the filtrate concentrated to obtain a crude product of compound **154-3** (orange solid, 334 mg). The crude product was used directly in the next step without further purification. MS (ESI, m/z): 729.4/731.4 [M + H]⁺.

### Step 4

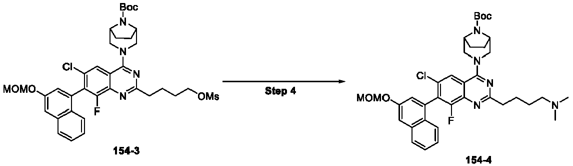

Compound **154-3** (334 mg) and a solution of dimethylamine (2 mol/L, 10 mL) in tetrahydrofuran was added to a 25 mL round bottom flask with stirring at 20 °C. The reaction was carried out at that temperature for 48 hours. The reaction process was monitored by liquid chromatography-mass spectrometry. After the reaction was completed, the reaction mixture was concentrated to obtain a crude product. The obtained crude product was purified by silica gel column chromatography, eluted with a gradient of 0%→15% methanol/dichloromethane mobile phase, and the obtained fractions were evaporated under reduced pressure to remove the solvent to obtain compound **154-4** (a mixture of two stereoisomers, light yellow solid, 252 mg). MS (ESI, m/z): 678.3/680.3 [M + H]⁺.

### Step 5

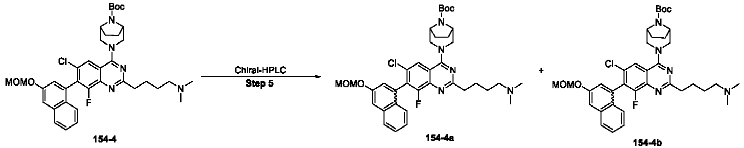

The compound **154-4** (252 mg) obtained in step 4 was subjected to chiral resolution by preparative chiral high pressure liquid chromatography under the following conditions: chiral column NB-Lux 5 µm i-Cellulose-5, 2.12 × 25 cm, 5 µm; mobile phase A: *n-*hexane/dichloromethane (0.5% 2 mmol/L ammonia methanol solution), mobile phase B: ethanol; flow rate: 20 mL/min; eluted with 3% mobile phase B in 44.4 min; detector UV 220/254 nm; two products were obtained. The product with shorter retention time (25.66 min) was **154-4a,** *tert*-butyl(1*R*,5*S*)-3-((*S* or *R*)-6-chloro-2-(4-(dimethylamino)butyl)-8-fluoro-7-(3-(methoxymethoxy)naphthalen-1-yl)quinazolin-4-yl)-3,8-diazabicyclo[3.2.1]octan-8-carboxylate (light yellow solid, 93 mg, recovery rate: 37%); the product with longer retention time (36.40 min) was **154-4b,** *tert*-butyl(1*R*,5*S*)-3-((*R* or *S*)-6-chloro-2-(4-(dimethylamino)butyl)-8-fluoro-7-(3-(methoxymethoxy)naphthalen-1-yl)quinazolin-4-yl)-3,8-diazabicyclo[3.2.1]octan-8-carboxylate (light yellow solid, 99 mg, recovery rate: 39%).
compound **154-4a:** MS (ESI, m/z): 678.3/680.3 [M + H]⁺; ¹H NMR (300 MHz, CDCl₃) *δ* 7.86 (d, *J* = 8.3 Hz, 1H), 7.83 (d, *J* = 1.7 Hz, 1H), 7.57 (d, *J* = 2.4 Hz, 1H), 7.52-7.46 (m, 1H), 7.38-7.29 (m, 2H), 7.22 (d, *J* = 2.4 Hz, 1H), 5.36 (s, 2H), 4.51-4.40 (m, 4H), 3.69-3.63 (m, 2H), 3.58 (s, 3H), 2.97 (t, *J* = 7.5 Hz, 2H), 2.59-2.53 (m, 2H), 2.40 (s, 6H), 2.03-1.84 (m, 6H), 1.76-1.70 (m, 2H), 1.56 (s, 9H).
compound **154-4b:** MS (ESI, m/z): 678.3/680.3 [M + H]⁺; ¹H NMR (300 MHz, CDCl₃) *δ* 7.86 (d, *J* = 8.3 Hz, 1H), 7.83 (d, *J* = 1.7 Hz, 1H), 7.57 (d, *J* = 2.5 Hz, 1H), 7.52-7.46 (m, 1H), 7.38-7.30 (m, 2H), 7.22 (d, *J* = 2.5 Hz, 1H), 5.36 (s, 2H), 4.51-4.41 (m, 4H), 3.66-3.62 (m, 2H), 3.58 (s, 3H), 2.97 (t, *J* = 7.5 Hz, 2H), 2.59-2.56 (m, 2H), 2.41 (s, 6H), 2.02-1.84 (m, 6H), 1.77-1.72 (m, 2H), 1.56 (s, 9H).

### Step 6

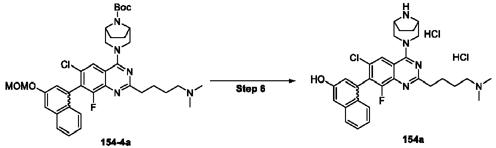

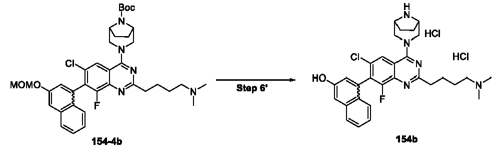

A solution of hydrochloric acid (4 mol/L, 2 mL) in 1,4-dioxane was added dropwise to a solution of compound **154-4a** (93 mg, 0.13 mmol, 1.0 eq) in methanol (2 mL) with stirring at 0 °C. The reaction was carried out for 1 hour at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 30% acetonitrile/water mobile phase (0.1% hydrochloric acid) in 20 min; detector, UV254/220 nm; to obtain compound **154a** (yellow solid, 24 mg, yield: 29%). Compound **154b** (yellow solid, 57 mg, yield: 65%) can be obtained by the same method as above.
compound **154a:** MS (ESI, m/z): 534.2/536.2 [M + H]⁺; ¹H NMR (300 MHz, DMSO-*d₆*) *δ* 10.69 (s, 1H), 10.30-10.16 (m, 2H), 9.90 (s, 1H), 8.16 (s, 1H), 7.84 (d, *J* = 8.3 Hz, 1H), 7.49-7.44 (m, 1H), 7.34 (d, *J* = 2.4 Hz, 1H), 7.28-7.20 (m, 2H), 7.15 (d, *J* = 2.4 Hz, 1H), 4.80-4.68 (m, 2H), 4.22-4.05 (m, 4H), 3.13-3.06 (m, 2H), 3.03-2.97 (m, 2H), 2.72 (d, *J* = 4.9 Hz, 6H), 2.01-1.76 (m, 8H); ¹⁹F NMR (282 MHz, DMSO-*d₆*) *δ*-120.76. The chiral analysis conditions of compound **154a** were: CHIRALPAK IC-3, 4.6 × 50 mm, 3 µm; mobile phase A: *n*-hexane/dichloromethane=5/1 (0.1% diethylamine), mobile phase B: isopropanol; flow rate: 1 mL/min; isocratic elution with 30% phase B in 6 min; detector UV 220 nm; retention time: 2.156 min; ee > 99%.
compound **154b:** MS (ESI, m/z): 534.2/536.2 [M + H]⁺; ¹H NMR (300 MHz, DMSO-*d₆*) *δ* 10.65 (s, 1H), 10.24-9.83 (m, 2H), 9.59 (s, 1H), 8.17 (d, *J* = 1.6 Hz, 1H), 7.83 (d, *J* = 8.3 Hz, 1H), 7.50-7.44 (m, 1H), 7.35 (d, *J* = 2.4 Hz, 1H), 7.29-7.20 (m, 2H), 7.12 (d, *J* = 2.4 Hz, 1H), 4.84-4.72 (m, 2H), 4.24-4.22 (m, 2H), 4.14-4.03 (m, 2H), 3.13-3.07 (m, 2H), 3.02-2.96 (m, 2H), 2.74 (s, 6H), 2.03-1.73 (m, 8H); ¹⁹F NMR (282 MHz, DMSO-*d₆*) *δ*-120.78. The chiral analysis conditions of compound **154b** were: CHIRALPAK IC-3, 4.6 × 50 mm, 3 µm; mobile phase A: *n*-hexane/dichloromethane=5/1 (0.1% diethylamine), mobile phase B: isopropanol; flow rate: 1 mL/min; isocratic elution with 30% phase B in 6 min; detector UV220 nm; retention time: 4.420 min; ee > 99%.

### Embodiment 38 (Synthesis method XXX)

### 2-((S)-4-((S or R)-6-chloro-8-fluoro-7-(3-hydroxynaphthalen-1-yl)-2-((S)-1-methylpyrrolidine-2-yl)methoxy)quinazolin-4-yl)-1-glycylpiperazin-2-yl)acetonitrile ditrifluoroacetate 155

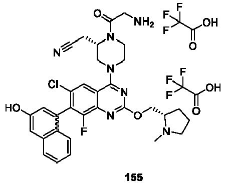

The synthetic route was as follows:

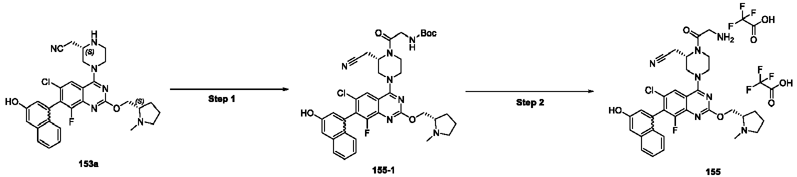

### Step 1

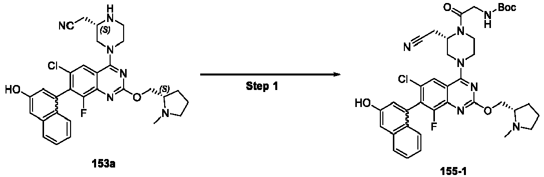

*N*-*tert*-butoxycarbonyl-glycine (16 mg, 0.09 mmol, 1.0 eq), *O*-(7-azabenzotriazol-1-yl)-*N*,*N*,*N',N*'-tetramethylurea hexafluorophosphate (34 mg, 0.09 mmol, 1.0 eq) and *N, N-*dimethylformamide (1 mL) were added to a 50 mL single-neck flask with stirring at 25 °C. The obtained reaction was carried out at this temperature for 0.5 hours, then **153a** (50 mg, 0.09 mmol, 1.0 eq) and *N*,*N*-diisopropylpropanamide (34 mg, 0.26 mmol, 3.0 eq) were added to the mixture. The reaction was carried out for 2 hours at 25 °C, and the reaction process was monitored by liquid chromatography-mass spectrometry. After the reaction was completed, the obtained crude product was purified by reversed-phase chromatography (C18 column), eluted with 5%→95% methanol/water (0.1% sodium bicarbonate) mobile phase in 30 min; detector: UV254/220 nm. Compound **155-1** (yellow solid, 37 mg, yield: 60%) was obtained. MS (ESI, m/z): 718.2/720.2 [M + H]⁺.

### Step 2

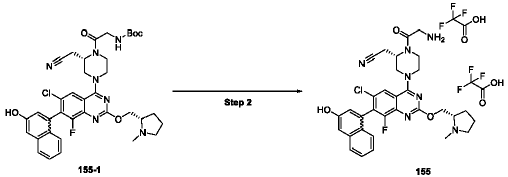

Trifluoroacetic acid (1 mL) was added dropwise to a solution of compound **155-1** (37 mg, 0.05 mmol, 1.0 eq) in dichloromethane (3 mL) with stirring at 20 °C. The reaction was carried out for 1 hour at this temperature, and the reaction process was monitored by liquid chromatography-mass spectrometry. After the reaction was completed, the reaction mixture was concentrated to obtain a crude product. The obtained crude product was purified by reversed-phase chromatography (C18 column), and eluted with 5% → 50% acetonitrile/water mobile phase (0.1% trifluoroacetic acid) in 20 min; detector, UV 254/220 nm; to obtain compound **155** (light yellow solid, 25 mg, yield: 55%). MS (ESI, m/z): 618.2/620.2 [M + H]⁺; ¹H NMR (400 MHz, DMSO-*d₆*) *δ* 10.36-9.97 (m, 2H), 8.24-8.15 (m, 4H), 7.83 (d, *J* = 8.3 Hz, 1H), 7.48-7.44 (m, 1H), 7.31 (d, *J* = 2.6 Hz, 1H), 7.26-7.19 (m, 2H), 7.08 (d, *J* = 2.6 Hz, 1H), 4.95-4.91 (m, 1H), 4.77-4.71 (m, 1H), 4.66-4.55 (m, 1H), 4.45-4.20 (m, 2H), 4.05-3.94 (m, 1H), 3.86-3.74 (m, 3H), 3.64-3.25 (m, 3H), 3.18-3.06 (m, 3H), 2.97 (d, *J* = 4.4 Hz, 3H), 2.68-2.58 (m, 1H), 2.31-2.24 (m, 1H), 2.10-2.04 (m, 1H), 1.97-1.87 (m, 2H); ¹⁹F NMR (377 MHz, DMSO-*d₆*) *δ*-74.12, -122.22.

### Embodiment 39

### 2-Amino-1-((1R,5S)-3-((S or R)-6-chloro-8-fluoro-7-(3-hydroxynaphthalen-1-yl)-2-((S)-1-methylpyrrolidin-2-yl)methoxy)quinazin-4-yl)-3,8-diazacyclo[3.2.1]octan-8-yl)ethan-1-one dihydrochloride 156

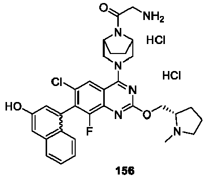

Compound **156** was synthesized according to Embodiment 38 (synthesis method XXX). Compound **156** (yellow solid, 44.9 mg, yield: 72%): MS (ESI, m/z): 605.2/607.2 [M+H]⁺. ¹H NMR (400 MHz, DMSO-*d₆*) *δ* 10.70 (s, 1H), 10.14 (s, 1H), 8.27-8.24 (m, 3H), 7.99 (s, 1H), 7.82 (d, *J* = 8.3 Hz, 1H), 7.47-7.43 (m, 1H), 7.31 (d, *J* = 2.3 Hz, 1H), 7.26-7.17 (m, 2H), 7.10-7.09 (m, 1H), 4.76-4.71 (m, 3H), 4.65-4.59 (m, 1H), 4.46-4.41 (m, 2H), 3.99-3.95 (m, 2H), 3.87-3.80 (m, 2H), 3.62-3.55 (m, 2H), 3.15-3.06 (m, 1H), 2.93 (d, *J* = 4.7 Hz, 3H), 2.30-2.22 (m, 1H), 2.07-1.78 (m, 7H); ¹⁹F NMR (377 MHz, DMSO-*d₆*) *δ*-122.09.

### Embodiment 40

### 2-((S)-1-(3-aminopropionyl)-4-((S or R)-6-chloro-8-fluoro-7-(3-hydroxynaphthalen-1-yl)-2-((S)-1-methylpyrrolidine-2-yl)methoxy)quinazolin-4-yl)-piperazin-2-yl)acetonitrile ditrifluoroacetate 157

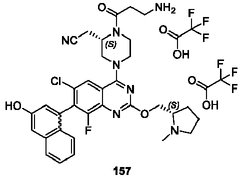

Compound **157** was synthesized according to Embodiment 38 (synthesis method XXX). compound **157** (yellow solid, 44.9 mg, yield: 72%): MS (ESI, m/z): 632.2/634.2 [M + H]⁺; ¹H NMR (300 MHz, DMSO-*d₆*) *δ* 10.14-9.88 (m, 2H), 8.17 (d, *J* = 1.6 Hz, 1H), 7.86-7.74 (m, 4H), 7.49-7.43 (m, 1H), 7.31 (d, *J* = 2.4 Hz, 1H), 7.27-7.19 (m, 2H), 7.08 (d, *J* = 2.4 Hz, 1H), 4.94-4.88 (m, 1H), 4.77-4.71 (m, 1H), 4.66-4.58 (m, 1H), 4.43-4.29 (m, 4H), 3.91-3.81 (m, 2H), 3.79-3.71 (m, 1H), 3.66-3.51 (m, 1H), 3.41-3.22 (m, 2H), 3.16-3.02 (m, 3H), 2.97 (d, *J* = 4.4 Hz, 3H), 2.84-2.72 (m, 2H), 2.33-2.22 (m, 1H), 2.11-2.04 (m, 1H), 2.00-1.83 (m, 2H); ¹⁹F NMR (282 MHz, DMSO-*d₆*) *δ*-74.03, -122.22.

### Embodiment 41 (Synthesis method XXXI)

### 4-(8-Fluoro-2-((S)-1-methylpyrrolidin-2-yl)methoxy)-4-((1R,2R,4S,5S)-7,9-diazatricyclo[3.3.1.0^{2,4}]nonan-7-yl)pyridinyl[4,3-d]pyrimidin-7-yl)naphthalen-2-ol diformate 158

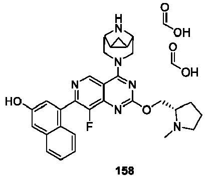

The synthetic route was as follows:

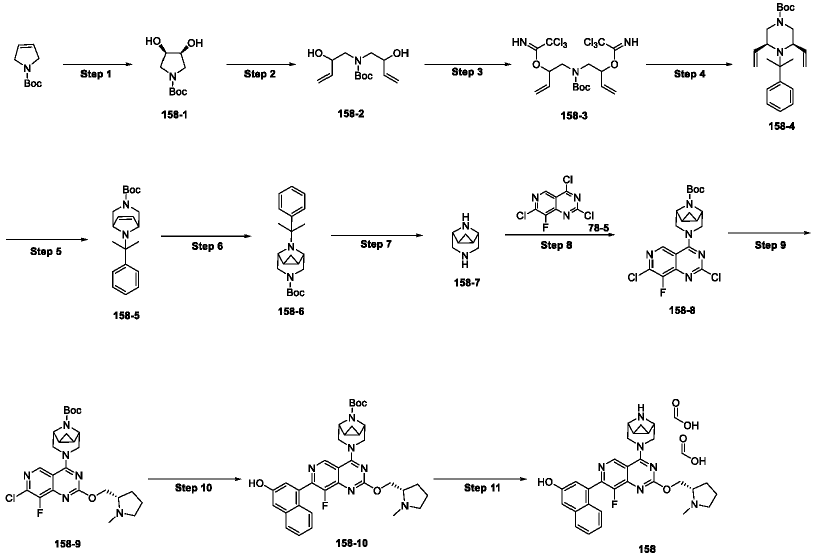

### Step 1

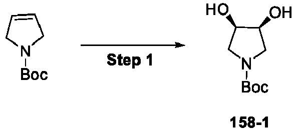

Water (2 mL), *N*-methylmorpholine oxide (15.8 g, 134.73 mmol, 1.2 eq) and osmium tetroxide (300 mg, 1.10 mmol, 1%) were added to a solution of *tert*-butyl 2,5-dihydro-1*H-*pyrrole-1-carboxylate (20 g, 112.27 mmol, 1.0 eq) in acetone (120 mL) with stirring at 25 °C. The reaction was carried out for 16 hours at 25 °C, and the reaction process was monitored by thin layer chromatography. After the reaction was completed, 300 mL of water was added to the reaction mixture for dilution, and the mixture was extracted with ethyl acetate (300 mL × 3), then the organic phases were combined, dried over anhydrous sodium sulfate, filtered, and the filtrate was concentrated to obtain a crude product. The crude product was purified by silica gel column chromatography, eluted with a gradient of 0% → 10% ethyl acetate/petroleum ether mobile phase, and the obtained fractions were evaporated under reduced pressure to obtain compound **158-1** (yellow oil, 20 g, yield: 83%). ¹H NMR (400 MHz, CDCl₃) *δ* 4.24-4.20 (m, 2H), 3.57-3.51 (m, 4H), 3.35-3.31 (m, 2H), 1.45 (s, 9H).

### Step 2

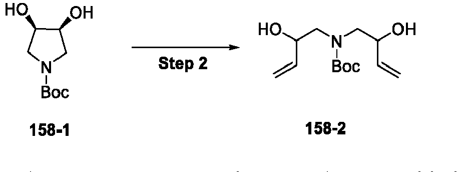

Diacetoxyiodobenzene (45 g, 140.23 mmol, 1.5 eq) was added to a solution of compound **158-1** (19 g, 88.81 mmol, 1.0 eq) in dichloromethane (300 mL) with stirring under the protection of nitrogen at 0 °C, and the reaction was carried out at this temperature for 1 hour. Subsequently, the reaction mixture was cooled to-78 °C, and 1 mol of vinyl magnesium bromide (560 mL) was added dropwise at this temperature for not less than 3 hours. After the dropwise addition, the temperature was slowly raised to 25 °C, and the reaction was continued for 16 hours, and the reaction process was monitored by thin layer chromatography. After the reaction was completed, the reaction mixture was quenched with 1 mol/L of hydrochloric acid aqueous solution at 0 °C. The mixture was extracted with dichloromethane (500 mL × 3), and the organic phases were combined, dried over anhydrous sodium sulfate, filtered and concentrated to obtain a crude product. The crude product was purified by silica gel column chromatography, eluted with a gradient of 0% → 50% ethyl acetate/petroleum ether mobile phase, and the obtained fractions were evaporated under reduced pressure to remove the solvent to obtain compound **158-2** (yellow oil, 19.6 g, yield: 81%). ¹H NMR (400 MHz, CDCl₃) *δ* 5.91-5.79 (m, 2H), 5.37-5.30 (m, 2H), 5.20-5.16 (m, 2H), 4.56-4.37 (m, 2H), 3.71-3.59 (m, 1H), 3.42-3.35 (m, 2H), 3.28-3.26 (m, 2H), 2.99-2.90 (m, 1H), 1.48-1.47 (m, 9H).

### Step 3

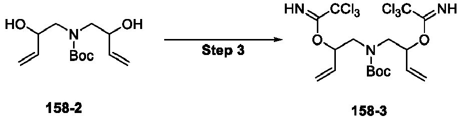

Trichloroacetonitrile (66.0 g, 457.00 mmol, 6.0 eq) and 1,8-diazabicyclo[5.4.0]undecan-7-ene (5.80 g, 38.08 mmol, 0.5 eq) were added to a solution of compound **158-2** (19.6 g, 76.16 mmol, 1.0 eq) in dichloromethane (250 mL) with stirring under the protection of nitrogen at 0 °C. The reaction was carried out for 16 hours at 25 °C, and the reaction process was monitored by thin layer chromatography. After the reaction was completed, the reaction mixture was concentrated to obtain a crude product. The crude product was purified by silica gel column chromatography, eluted with a gradient of 0% → 50% ethyl acetate/petroleum ether mobile phase, and the obtained fractions were evaporated under reduced pressure to remove the solvent to obtain compound **158-3** (yellow oil, 35 g, yield: 84%). ¹H NMR (400 MHz, CDCl₃) *δ* 8.37 (s, 2H), 5.90-5.76 (m, 2H), 5.70-5.59 (m, 2H), 5.46-5.38 (m, 2H), 5.30-5.25 (m, 2H), 3.85-3.77 (m, 1H), 3.72-3.58 (m, 2H), 3.50-3.39 (m, 1H), 1.48-1.46 (m, 9H).

### Step 4

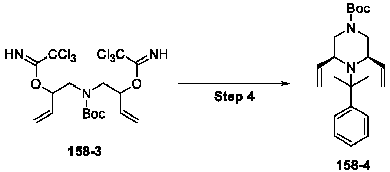

Compound isophenylpropylamine (3.0 g, 20.87 mmol, 1.2 eq), chloro(1,5-cyclooctadiene)iridium(I) dimer (615 mg, 0.87 mmol, 5%) and 1,2-dichloroethane (60 mL) were successively added to a 250 mL three-neck flask under the protection of nitrogen at 25 °C. The temperature of the system was lowered to 0 °C, and **158-3** (10.0 g, 17.39 mmol, 1.0 eq) was added dropwise to the mixture, and the reaction was carried out for 16 hours at 25 °C under nitrogen atmosphere, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 25% ethyl acetate/petroleum ether mobile phase, and the obtained fractions were evaporated under reduced pressure to remove the solvent to obtain compound **158-4** (yellow oil, 5.2 g, yield: 80%). MS (ESI, m/z): 357.2 [M + H]⁺. ¹H NMR (400 MHz, CDCl₃) *δ* 7.55-7.52 (m, 2H), 7.31-7.27 (m, 2H), 7.24-7.18 (m, 1H), 6.02-5.93 (m, 2H), 5.14-5.04 (m, 2H), 5.00-4.96 (m, 2H), 3.61-3.49 (m, 4H), 3.36-3.28 (m, 2H), 1.46-1.43 (m, 15H).

### Step 5

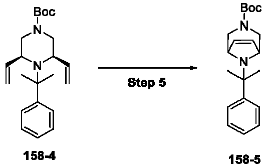

Compound **158-4** (5.2 g, 13.85 mmol, 1.0 eq), 1,3-bis-(2,4,6-trimethylphenyl)-2-(imidazolidinylidene)(dichlorophenylmethylene)(tricyclohexylphosphine)ruthenium (620 mg, 0.69 mmol, 5%) and toluene (160 mL) were successively added to a 500 mL three-neck flask under the protection of nitrogen at 25 ° C. The reaction was carried out for 20 hours at 120 °C under nitrogen atmosphere, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 25% ethyl acetate/petroleum ether mobile phase, and the obtained fractions were evaporated under reduced pressure to remove the solvent to obtain compound **158-5** (white solid, 3.5 g, yield: 75%). MS (ESI, m/z): 329.2 [M + H]⁺; ¹H NMR (400 MHz, CDCl₃) *δ* 7.59-7.57 (m, 2H), 7.33-7.29 (m, 2H), 7.23-7.19 (m, 1H), 6.00-5.94 (m, 2H), 3.64-3.58 (m, 2H), 3.53-3.45 (m, 2H), 3.13-3.05 (m, 2H), 1.41 (s, 9H), 1.25 (s, 3H), 1.24 (s, 3H).

### Step 6

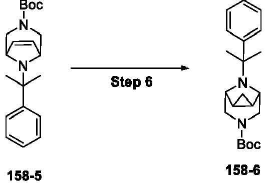

A solution of potassium hydroxide (15 g, 260 mmol, 90.0 eq) in water (60 mL) was added dropwise to a solution of *N*-methyl-*N*-nitrosourea (4 g, 37.01 mmol, 12.8 eq) in ether (100 mL) with stirring at -20 °C. The reaction was carried out for 0.5 hours at -20 °C, then left to stand for stratification, and the organic layer was separated.

The organic layer was added dropwise to a solution of compound **158-5** (1 g, 2.89 mmol, 1.0 eq) in ether (10 mL) with stirring under the protection of nitrogen at 0 °C, after the dropwise addition, palladium acetate (68 mg, 0.28 mmol, 0.1 eq) was added, and the reaction was continued at 0 °C for 1 hour, then the reaction process was monitored by liquid chromatography-mass spectrometry. After the reaction was completed, the reaction mixture was filtered, the filter cake was washed with ether (20 mL × 3), and the filtrate was concentrated to obtain a crude product. The crude product was purified by silica gel column chromatography, eluted with a gradient of 0% → 25% ethyl acetate/petroleum ether mobile phase, and the obtained fractions were evaporated under reduced pressure to remove the solvent to obtain compound **158-6** (white solid, 900 mg, yield: 86%). MS (ESI, m/z): 343.2 [M + H]⁺; ¹H NMR (400 MHz, CDCl₃) *δ* 7.41-7.38 (m, 2H), 7.32-7.28 (m, 2H), 7.23-7.18 (m, 1H), 3.39-3.35 (m, 1H), 3.28-3.22 (m, 2H), 3.18-3.11 (m, 3H), 1.54 (s, 6H), 1.45 (s, 9H), 1.07-1.03 (m, 1H), 0.99-0.95 (m, 1H), 0.70-0.66 (m, 1H), 0.25-0.21 (m, 1H).

### Step 7

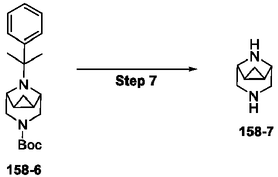

A solution of hydrochloric acid (4 mol/L, 5 mL) in ethyl acetate was added to a solution of compound **158-6** (400 mg, 1.11 mmol, 1.0 eq) in dichloromethane (5 mL) with stirring at 0 °C. The reaction was carried out for 1 hour at 0 °C, and the reaction process was monitored by liquid chromatography-mass spectrometry. After the reaction was completed, the reaction mixture was concentrated to obtain a crude product of **158-7** (white solid, 130 mg, yield: 75%), which was directly used in the next step without further purification. MS (ESI, m/z): 125.1 [M + H]⁺.

### Step 8

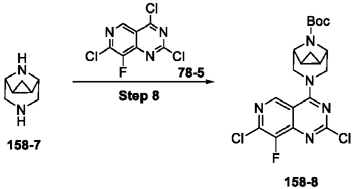

*N*,*N-*diisopropylethylamine (204 mg, 1.58 mmol, 4.0 eq) and compound **158-7** (100 mg, 0.37 mmol, 1.0 eq) were added to a solution of compound **78-5** (49 mg, 0.37 mmol, 1.0 eq) in dichloromethane (2 mL) with stirring at 0 °C. The reaction was carried out at 0 °C for 1 hour, di-*tert*-butyl dicarbonate (345 mg, 1.58 mmol, 4.0 eq) and *N*, *N*-diisopropylethylamine (102 mg, 0.79 mmol, 2.0 eq) were added to the reaction system. The reaction was carried out for 16 hours at 25 °C, and the reaction process was monitored by liquid chromatography-mass spectrometry. After the reaction was completed, the reaction mixture was concentrated to obtain a crude product. The crude product was purified by silica gel column chromatography, eluted with a gradient of 0% → 35% ethyl acetate/petroleum ether mobile phase, and the obtained fractions were evaporated under reduced pressure to remove the solvent to obtain compound **158-8** (white solid, 140 mg, yield: 80%). MS m/z (ESI) : 440.1/442.1 [M + H]⁺; ¹H NMR (400 MHz, CDCl₃) *δ* 8.88 (s, 1H), 4.67-4.59 (m, 2H), 4.33-4.32 (m, 1H), 4.21-4.20 (m, 1H), 3.81-3.66 (m, 2H), 1.50 (s, 9H), 1.27-1.24 (m, 2H), 0.52-0.46 (m, 1H), 0.22-0.18 (m, 1H).

### Step 9

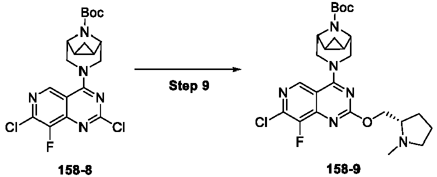

*N*-methyl-*L*-proline (34 mg, 0.28 mmol, 1.2 eq) and a solution of sodium *tert*-butoxide (2 mol/L, 0.15 mL, 0.28 mmol, 1.2 eq) in tetrahydrofuran were successively added to a solution of compound **158-8** (140 mg, 0.23 mmol, 1.0 eq) in anhydrous tetrahydrofuran under the protection of nitrogen at 0 °C. The reaction was carried out for 1 hour at 0 °C, and the reaction process was monitored by liquid chromatography-mass spectrometry. After the reaction was completed, the mixture was poured into water (10 mL) and extracted with ethyl acetate (10 mL × 3), the organic phases were combined, dried over anhydrous sodium sulfate, filtered, and the filtrate was concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **158-9** (white solid, 100 mg, yield: 77%). MS m/z (ESI): 519.1/521.2 [M+1]⁺; ¹H NMR (400 MHz, CDCl₃) *δ* 8.77 (s, 1H), 4.84-4.80 (m, 1H), 4.66-4.48 (m, 3H), 4.30-4.29 (m, 1H), 4.18-4.17 (m, 1H), 3.75-3.71 (m, 1H), 3.64-3.60 (m, 1H), 3.48-3.43 (m, 1H), 3.17-3.11 (m, 1H), 2.76 (s, 3H), 2.64-2.58 (m, 1H), 2.25-2.17 (m, 1H), 2.09-1.93 (m, 3H), 1.50 (s, 9H), 1.30-1.21 (m, 2H), 0.50-0.44 (m, 1H), 0.19-0.15 (m, 1H).

### Step 10

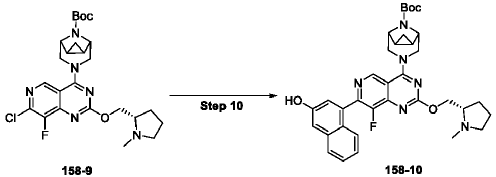

4-(4,4,5,5-Tetramethyl-1,3,2-dioxaborolan-2-yl)naphthalen-2-ol (58 mg, 0.20 mmol, 1.5 eq), cesium carbonate (94 mg, 0.27 mmol, 2.0 eq) and tetrakis(triphenylphosphine)palladium (17 mg, 0.01 mmol, 5%) were added to a solution of compound **158-9** (75 mg, 0.14 mmol, 1.0 eq) in 1,4-dioxane/water (5/1, 3 mL) with stirring under the protection of nitrogen at 25 °C. The reaction was carried out with stirring for 2 hours at 100 °C, and the reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→10% ammonia methanol solution (8 mol/L)/dichloromethane mobile phase, and the obtained fractions were evaporated under reduced pressure to remove the solvent to obtain compound **158-10** (white solid, 80 mg, yield: 88%). MS m/z (ESI): 627.1 [M+1]⁺.

### Step 11

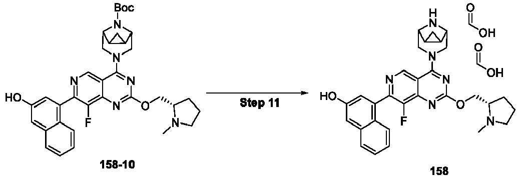

Trifluoroacetic acid (1 mL) was added dropwise to a solution of compound **158-10** (80 mg, 0.12 mmol, 1.0 eq) in dichloromethane (3 mL) with stirring at 25 °C. The reaction was carried out for 1 hour at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5% → 40% acetonitrile/water mobile phase (0.1% formic acid) in 20 min; detector, UV 254/220 nm; to obtain compound **158** (white solid, 25 mg, yield: 37%). MS m/z (ESI) : 527.2 [M+1]⁺; ¹H NMR (300 MHz, DMSO-*d₆*) *δ* 9.24 (s, 1H), 8.18 (s, 2H), 7.81 (d, *J* = 8.3 Hz, 1H), 7.60-7.55 (m, 1H), 7.47-7.42 (m, 1H), 7.30-7.22 (m, 3H), 4.57-4.22 (m, 2H), 4.44 (dd, *J* = 11.0, 5.0 Hz, 1H), 4.27 (dd, *J* = 11.0, 6.1 Hz, 1H), 3.68-3.64 (m, 2H), 3.26-3.25 (m, 2H), 3.06-3.00 (m, 1H), 2.78-2.70 (m, 1H), 2.44 (s, 3H), 2.34-2.24 (m, 1H), 2.03-1.94 (m, 1H), 1.77-1.62 (m, 3H), 1.22-1.19 (m, 2H), 0.43-0.38 (m, 1H), 0.27-0.20 (m, 1H); ¹⁹F NMR (282 MHz, DMSO-*d₆*) *δ*-139.75.

### Embodiment 42

### 4-(4-((1R,5S)-3,8-diazabicyclo[3.2.1]oct-6-en-3-yl)-8-fluoro-2-(((S)-1-methylpyrrolidin-2-yl)methoxy)pyrido[4,3-d]pyrimidin-7-yl)naphthalen-2-ol diformate 159

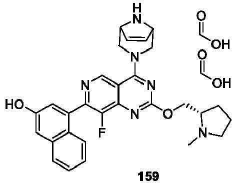

Compound **159** was synthesized according to Embodiment 41 (synthesis method XXXI). Compound **159** (white solid). MS m/z (ESI) : 513.1[M+1]⁺; ¹H NMR (400 MHz, DMSO-*d₆*) *δ* 9.23 (s, 1H), 8.18 (s, 2H), 7.80 (d, *J* = 8.2 Hz, 1H), 7.55 (d, *J* = 8.4 Hz, 1H), 7.47 - 7.40 (m, 1H), 7.29 - 7.21 (m, 3H), 6.28 - 6.26 (m, 2H), 4.45 - 4.38 (m, 3H), 4.24 (dd, *J* = 10.9, 6.1 Hz, 1H), 4.05 - 4.03 (m, 2H), 3.82 - 3.78 (m, 2H), 3.04 - 2.99 (m, 1H), 2.72 - 2.66 (m, 1H), 2.41 (s, 3H), 2.31 - 2.24 (m, 1H), 2.02 - 1.93 (m, 1H), 1.75 - 1.62 (m, 3H); ¹⁹F NMR (377 MHz, DMSO-*d₆*) *δ*-139.64.

### Embodiment 43

### (R or S) 4-(4-((1R,5S)-3,8-diazabicyclo[3.2.1]octan-3-yl)-6,8-difluoro-2-((S)-1-methylpyrrolidin-2-yl)methoxy)quinazolin-7-yl)naphthalen-2-ol dihydrochloride 160a; (S or R) 4-(4-((1R,5S)-3,8-diazabicyclo[3.2.1]octan-3-yl)-6,8-difluoro-2-((S)-1-methylpyrrolidin-2-yl)methoxy)quinazolin-7-yl)naphthalen-2-ol dihydrochloride 160b; 4-(4-((1R,5S)-3,8-diazabicyclo[3.2.1]octan-3-yl)-6,8-difluoro-2-((S)-1-methylpyrrolidin-2-yl)methoxy)quinazolin-7-yl)naphthalen-2-ol dihydrochloride 160

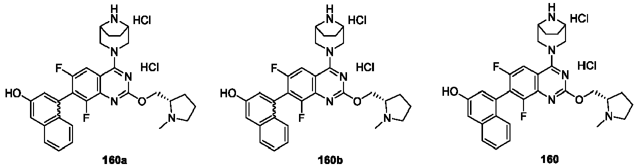

### Step 1

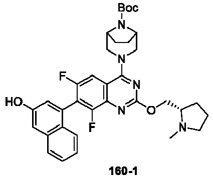

Compound **160-1** was synthesized according to Embodiment 15 (synthesis method XIII). Compound **160-1** (brown solid). MS (ESI, m/z): 632.3 [M + H]⁺.

### Step 2

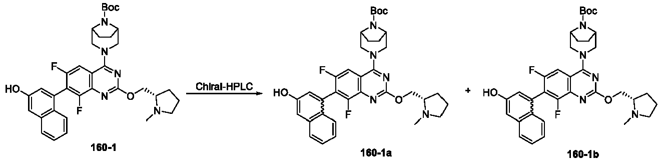

The compound **160-1** (280 mg) obtained in step 1 was subjected to chiral resolution by preparative chiral high pressure liquid chromatography under the following conditions: chiral column CHIRAL ART Cellulose-SC, 2 × 25 cm, 5 µm; mobile phase A: *n*-hexane (10 mmol/L ammonia methanol solution), mobile phase B: ethanol; flow rate: 25 mL/min; elution with 25% mobile phase B in 13 min; detector UV 220 nm; two products were obtained. The product with shorter retention time (5.08 min) was **160-1a,** *tert*-butyl(1*R*,5*S*)-3-(6,8-difluoro-7-((*R* or *S*)-3-hydroxynaphthalene-1-yl)-2-((*S*)-1-methylpyrrolidin-2-yl)methoxy)quinazolin-4-yl)-3,8-diazabicyclo[3.2.1]octane-8-carboxylate (yellow solid, 98 mg, recovery rate: 35%), the product with longer retention time (8.03 min) was compound **160-1b,** *tert*-butyl(1*R*,5*S*)-3-(6,8-difluoro-7-((*S* or *R*)-3-hydroxynaphthalen-1-yl)-2-((*S*)-1-methylpyrrolidin-2-yl)methoxy)quinazolin-4-yl)-3,8-diazabicyclo[3.2.1]octane-8-carboxylate (light yellow solid, 115 mg, recovery rate: 41%).

### Step 3

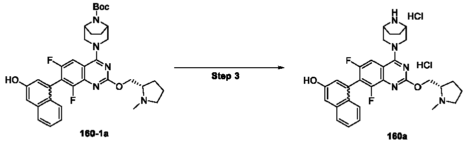

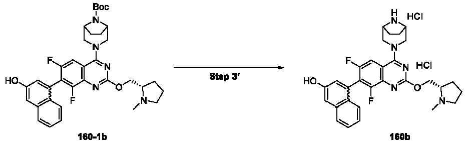

A solution of hydrochloric acid (4 mol/L, 2 mL) in 1,4-dioxane was added dropwise to a solution of compound **160-1a** (80 mg, 0.12 mmol, 1.0 eq) in methanol (2 mL) with stirring at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatography (C18 column), and eluted with 5%→ 40% acetonitrile/water mobile phase (0.1% hydrochloric acid) in 30 min; detector, UV 254/220 nm; to obtain compound **160a** (yellow solid, 48.5 mg, yield: 65%). Compound **160b** (yellow solid, 47.2 mg, yield: 63%) can be obtained by the same method as above.

Compound **160a:** MS m/z (ESI) : 532.2[M+1]⁺; ¹H NMR (300 MHz, DMSO-*d₆*) *δ* 10.90 (s, 1H), 10.18 - 9.74 (m, 3H), 7.85 - 7.80 (m, 2H), 7.50 - 7.44 (m, 1H), 7.35 - 7.32 (m, 2H), 7.29 - 7.26 (m, 1H), 7.24 - 7.20 (m, 1H), 4.77 - 4.73 (m, 2H), 4.56 - 4.44 (m, 2H), 4.18 - 4.15 (m, 2H), 3.97 - 3.82 (m, 2H), 3.68 - 3.56 (m, 2H), 3.18 - 3.05 (m, 1H), 2.94 (d, *J=* 4.8 Hz, 3H), 2.32 - 2.21 (m, 1H), 2.07 - 1.87 (m, 7H); ¹⁹F NMR (282 MHz, DMSO-*d₆*) *δ*-116.64, -123.39. The chiral analysis conditions of compound **160a** were: CHIRALPAK IC-3, 4.6 × 50 mm, 3 µm; mobile phase A: *n*-hexane (0.1% diethylamine), mobile phase B: isopropanol; flow rate: 1 mL/min; isocratic elution with 30% phase B in 6 min; detector UV254 nm; retention time: 2.198 min; ee= 74%.

Compound **160b:** MS m/z (ESI) : 532.2[M+1]⁺; ¹H NMR (400 MHz, DMSO-*d₆*) *δ* 10.80 (s, 1H), 10.19 - 9.67 (m, 3H), 7.86 - 7.80 (m, 2H), 7.50 - 7.44 (m, 1H), 7.35 - 7.24 (m, 3H), 7.21 - 7.19 (m, 1H), 4.76 - 4.72 (m, 2H), 4.55 - 4.45 (m, 2H), 4.18 - 4.16 (m, 2H), 3.94 - 3.81 (m, 2H), 3.62 - 3.50 (m, 2H), 3.17 - 3.07 (m, 1H), 2.94 (d, *J* = 4.6 Hz, 3H), 2.32 - 2.22 (m, 1H), 2.07 - 1.87 (m, 7H); ¹⁹F NMR (282 MHz, DMSO-*d₆*) *δ*-116.65, -123.38. The chiral analysis conditions of compound **160b** were: CHIRALPAK IC-3, 4.6 × 50 mm, 3 µm; mobile phase A: *n*-hexane (0.1% diethylamine), mobile phase B: isopropanol; flow rate: 1 mL/min; isocratic elution with 30% phase B in 6 min; detector UV254 nm; retention time: 3.411 min; ee = 74%.

### Step 4

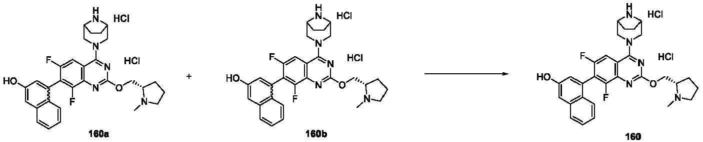

Compound **160b** (20 mg, 0.03 mmol, 1.0 eq) was added to a solution of compound **160a** (20 mg, 0.03 mmol, 1.0 eq) in acetonitrile (1 mL)/water (1 mL) with stirring at 25 °C. The obtained mixture was stirred at 25 °C for 5 min. The reaction mixture was concentrated under reduced pressure to obtain compound **160** (yellow solid, 35.6 mg, yield: 93%). MS (ESI, m/z): 532.3[M + H]⁺; ¹H NMR (300 MHz, DMSO-*d₆*) *δ* 10.85 - 10.68 (m, 1H), 10.34 - 9.58 (m, 3H), 7.90 - 7.77 (m, 2H), 7.52 - 7.43 (m, 1H), 7.37 - 7.23 (m, 3H), 7.20 (d, *J* = 2.3 Hz, 1H), 4.74 (d, *J* = 5.5 Hz, 2H), 4.62 - 4.46 (m, 2H), 4.17 (s, 2H), 3.97 - 3.85 (m, 3H), 3.62 - 3.54 (m, 1H), 3.20 - 3.05 (m, 1H), 2.94 (d, *J* = 4.8 Hz, 3H), 2.35 - 2.20 (m, 1H), 2.10 - 1.82 (m, 7H); ¹⁹F NMR (282 MHz, DMSO-*d₆*) *δ*-116.64, -116.65, -116.67, -123.38.

### Embodiment 44 (Synthesis method XXXII)

### 4-((1R,5S)-3,8-diazabicyclo[3.2.1]octan-3-yl)-2-(3-(dimethylamino)propoxy)-7-(3-hydroxynaphthalen-1-yl)pyrido[3,4-d]pyrimidin-8(7H)-one dihydrochloride 161

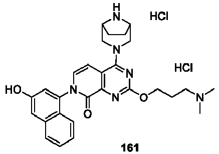

The synthetic route was as follows:

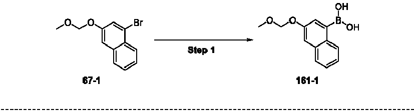

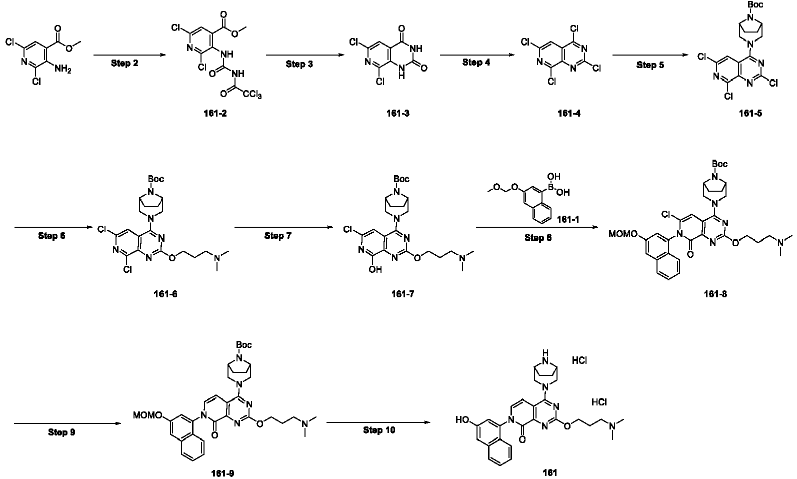

### Step 1

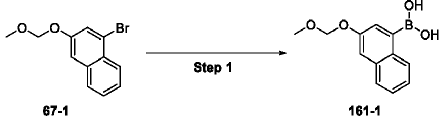

*n*-Butyl lithium (2.5 mol/L, 0.78 mL, 1.96 mmol, 1.1 eq) was added dropwise to a solution of the compound **67-1** (500 mg, 1.78 mmol, 1.0 eq) in anhydrous tetrahydrofuran (5 mL) under the protection of nitrogen at -78 °C. After the dropwise addition, the reaction was carried out at this temperature for 15 min, and then triisopropyl borate (422 mg, 2.25 mmol, 1.2 eq) was added, and the reaction was continued for 2 hours while maintaining the temperature. Then the reaction was slowly raised to room temperature and carried out overnight, and the reaction process was monitored by thin layer chromatography. After the reaction was completed, the reaction was quenched with 20 mL of water, the mixture was extracted with ethyl acetate (100 mL × 3), and the organic phases were combined, washed with 20 mL of saturated brine, dried over anhydrous sodium sulfate, filtered, and the filtrate was concentrated to obtain a crude product. The obtained crude product was purified by recrystallization (solvent system: ethyl acetate/n-hexane 1/1, 10 mL), and filtered to obtain compound **161-1** (white solid, 90 mg, yield: 20%). ¹H NMR (300 MHz, CDCl₃) *δ* 9.24 - 9.17 (m, 1H), 8.42 (d, *J* = 2.7 Hz, 1H), 7.90 - 7.85 (m, 1H), 7.69 (d, *J* = 2.7 Hz, 1H), 7.60 - 7.53 (m, 2H), 5.43 (s, 2H), 3.62 (s, 3H).

### Step 2

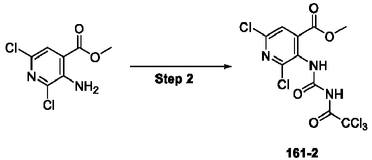

Trichloroacetyl isocyanate (11.5 g, 58.02 mmol, 1.5 eq) was added dropwise to a solution of compound methyl 2, 6-dichloro-3-aminoisonicotinate (9.0 g, 38.68 mmol, 1.0 eq) in tetrahydrofuran (80 mL) with stirring under the protection of nitrogen at 25 °C. The reaction was carried out for 1 hour at 25 °C, and the reaction process was monitored by liquid chromatography-mass spectrometry. After the reaction was completed, the reaction solution was concentrated to obtain compound **161-2** (yellow solid, 15 g, yield: 89%). The compound was used directly in the next step of synthesis without further purification. MS (ESI, m/z): 407.8/409.8/411.8[M + H]⁺.

### Step 3

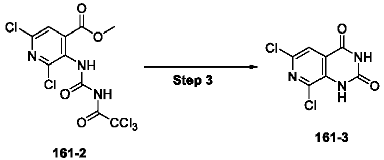

A solution of 7 mol/L ammonia methanol solution (40 mL) was added dropwise to a solution of compound **161-2** (15 g, 36.64 mmol, 1.0 eq) in methanol (40 mL) with stirring at 25°C. The reaction was carried out at 25 °C for 10 min, a large amount of solid was precipitated in the reaction solution, filtered, and the filter cake was washed with methyl *tert-*butyl ether (50 mL × 3), and the filter cake was dried to obtain compound **161-3** (yellow solid, 8 g, yield: 94%). MS (ESI, m/z): 231.9/233.9[M + H]⁺; ¹H NMR (300 MHz, DMSO-*d₆*) *δ* 11.09 (brs, 2H), 7.79 (s, 1H).

### Step 4

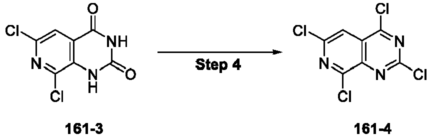

Compound **161-3** (8 g, 32.75 mmol, 1.0 eq), phosphorus oxychloride (100 mL) and 7V,7V-diisopropylethylamine (10 mL) were successively added to a dry 250 mL single-neck flask under the protection of nitrogen at 0 °C. The mixture was stirred at 0 °C for 10 min, and then transferred to an oil bath at 90 °C to react under reflux for 20 hours, and the reaction process was monitored by thin layer chromatography. After the reaction was completed, the reaction mixture was cooled to room temperature then concentrated under reduced pressure. Dichloromethane (100 mL) was added and the residual phosphorus oxychloride was removed by concentration, this operation was repeated three times to obtain a crude product. The crude product was purified by silica gel column chromatography, eluted with a gradient of 0%→ 30% ethyl acetate/petroleum ether mobile phase, and the obtained fractions were evaporated under reduced pressure to remove the solvent to obtain compound **161-4** (yellow solid, 4.9 g, yield: 52%). ¹H NMR (300 MHz, DMSO-*d₆*) *δ* 8.04 (s, 1H).

### Step 5

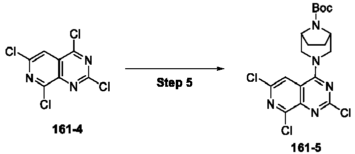

Compound **161-4** (4.9 g, 17.31 mmol, 1.0 eq) was dissolved in 5 mL of tetrahydrofuran with stirring under the protection of nitrogen at 25 °C. *N, N-*diisopropylethylamine (4.7 g, 34.62 mmol, 2.0 eq) and *tert*-butyl (1*R*, 5*S*)-3,8-diazabicyclo[3.2.1]octane-8-carboxylate (4.26 g, 19.04 mmol, 1.1 eq) were successively added to the solution. The reaction was carried out for 3 hours at 25 °C, and the reaction process was monitored by liquid chromatography-mass spectrometry. After the reaction was completed, the reaction mixture was concentrated to obtain a crude product. The obtained crude product was purified by silica gel column chromatography, eluted with dichloromethane mobile phase, and the obtained fractions were evaporated under reduced pressure to remove the solvent to obtain compound **161-5** (yellow solid, 5.2 g, yield: 64%). MS (ESI, m/z): 444.0/446.0[M + H]⁺; ¹H NMR (400 MHz, CDCl₃) *δ* 7.59 (s, 1H), 4.46 - 4.34 (m, 4H), 3.74 - 3.61 (m, 2H), 2.02 - 1.93 (m, 2H), 1.71 - 1.65 (m, 2H), 1.52 (s, 9H).

### Step 6

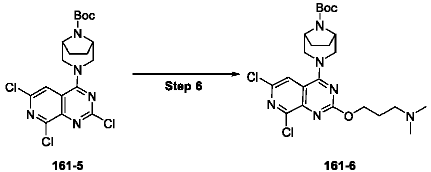

*N*-methyl-*L*-proline (232 mg, 2.14 mmol, 1.0 eq) and potassium carbonate (1.5 g, 4.28 mmol, 2.0 eq) were added to a solution of **161-5** (1.0 g, 2.14 mmol, 1.0 eq) in tetrahydrofuran (10 mL) at 25 °C. The reaction was carried out for 18 hours at 50 °C, and the reaction process was monitored by liquid chromatography-mass spectrometry. After the reaction was completed, the reaction mixture was cooled to 80 °C, and concentrated to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **161-6** (light yellow solid, 800 mg, yield: 69%). MS (ESI, m/z): 511.2/513.2[M + H]⁺; ¹H NMR (300 MHz, CDCl₃) *δ* 7.55 (s, 1H), 4.55 (t, *J* = 6.2 Hz, 2H), 4.40 - 4.34 (m, 4H), 3.68 - 3.57 (m, 2H), 2.92 - 2.87 (m, 2H), 2.57 (s, 6H), 2.30 - 2.20 (m, 2H), 2.01 - 1.94 (m, 2H), 1.77 - 1.70 (m, 2H), 1.52 (s, 9H).

### Step 7

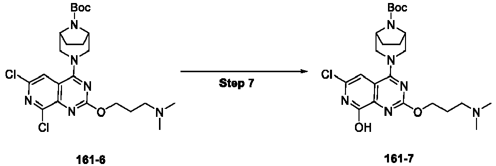

Potassium acetate (1.2 g, 12.07 mmol, 10.0 eq) was added to a solution of compound **161-6** (650 mg, 1.21 mmol, 1.0 eq) in *N*,*N-*dimethylformamide (10 mL) with stirring under the protection of nitrogen at 25 °C. The reaction was carried out for 10 hours at 100 °C, and the reaction process was monitored by liquid chromatography-mass spectrometry. After the reaction was completed, the reaction solution was cooled to room temperature, filtered, the filter cake was washed with methanol (10 mL × 3), and the filtrate was concentrated to obtain a crude product. The crude product was purified by reversed-phase chromatographic column (C18 column), and eluted with 5%→ 50% methanol/water mobile phase (0.1% trifluoroacetic acid) in 20 min; detector, UV 254/220 nm; to obtain compound **161-7** (purple solid, 300 mg, yield: 47%). MS (ESI, m/z): 493.1/495.1[M + H]⁺.

### Step 8

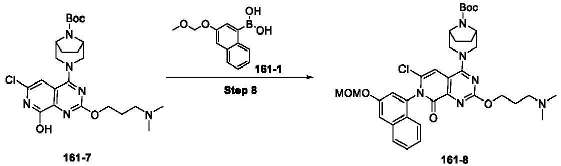

**161-7** (80 mg, 0.15 mmol, 1.0 eq), *N*,*N-*dimethylformamide (4 mL), **161-1** (56 mg, 0.23 mmol, 1.5 eq), pyridine (1 mL) and copper (II) trifluoromethane sulfonate (117 mg, 0.31 mmol, 2.0 eq) were successively added to a 25 mL Schlenk tube under the protection of nitrogen with stirring at 25 °C. The reaction was carried out for 16 hours at 40 °C under oxygen atmosphere, and the reaction process was monitored by liquid chromatography-mass spectrometry. After the reaction was completed, the reaction solution was directly purified by reversed-phase chromatography (C 18 column), eluted with 5%→ 100% methanol/water (0.1% trifluoroaceteic acid) mobile phase in 20 min; detector: UV 254/220 nm; to obtain compound **161-8** (yellow oil, 80 mg, yield: 72%). MS (ESI, m/z): 679.4/ 681.3[M + H]⁺; ¹H NMR (300 MHz, DMSO-*d₆*) *δ* 7.91 (d, *J* = 8.3 Hz, 1H), 7.76 (d, *J* = 8.4 Hz, 1H), 7.58 (s, 1H), 7.53 (t, *J* = 7.6 Hz, 1H), 7.43 (s, 1H), 7.36 (t, *J* = 7.6 Hz, 1H), 7.17 (s, 1H), 5.35 (s, 2H), 4.39 - 4.32 (m, 4H), 4.26 - 4.23 (m, 2H), 3.57 - 3.53 (m, 2H), 3.45 (s, 3H), 2.34 - 2.30 (m, 2H), 2.11 (s, 6H), 1.90 - 1.79 (m, 4H), 1.70 - 1.67 (m, 2H), 1.46 (s, 9H).

### Step 9

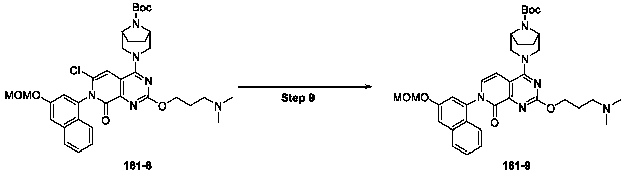

**161-8** (80 mg, 0.11 mmol, 1.0 eq), *N*,*N-*dimethylformamide (2 mL), triethylsilylhydrogen (137 mg, 1.12 mmol, 10.0 eq) and[1,1'-bis (diphenylphosphine)ferrocene]palladium dichloromethane complex (8.5 mg, 0.01 mmol, 0.1 eq) were added to a 25 mL Schlenk tube with stirring at 25 °C. The reaction mixture was carried out for 2 hours under nitrogen atmosphere at 100 °C. The reaction process was monitored by liquid chromatography-mass spectrometry. After the reaction was completed, the reaction mixture was cooled to 25 °C, directly purified by reversed-phase chromatographic column (C18 column), and eluted with 5%→ 95% methanol/water mobile phase (0.1% ammonium bicarbonate) in 30 min; detector, UV 254/220 nm; to obtain compound 161-9 (yellow solid, 60 mg, yield: 79%). MS (ESI, m/z): 645.3[M + H]⁺; ¹H NMR (400 MHz, CD₃OD) *δ* 7.83 - 7.80 (m, 2H), 7.68 (d, *J* = 5.9 Hz, 1H), 7.50 (d, *J* = 5.9 Hz, 1H), 7.47 - 7.44 (m,1H), 7.37 (d, *J* = 2.4 Hz, 1H), 7.30 - 7.26 (m, 1H), 7.01 (d, *J* = 2.4 Hz, 1H), 5.31 (s, 2H), 4.55 - 4.51 (m, 2H), 4.43 (t, *J* = 6.3 Hz, 2H), 4.39 - 4.36 (m, 2H), 3.63 - 3.58 (m, 2H), 3.50 (s, 3H), 2.59 (t, *J* = 7.7 Hz, 2H), 2.32 (s, 6H), 2.03 - 1.93 (m, 4H), 1.86 - 1.82 (m, 2H), 1.52 (s, 9H).

### Step 10

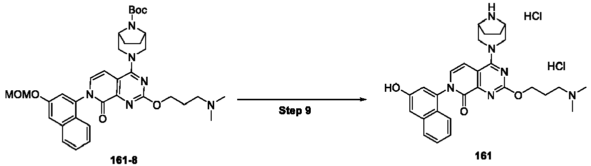

A solution of hydrochloric acid (4 mol/L, 2 mL) in 1,4-dioxane was added dropwise to a solution of compound **161-8** (60 mg, 0.09 mmol, 1.0 eq) in methanol (2 mL) with stirring at 0 °C. The reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatographic column (C18 column), and eluted with 5%→ 50% acetonitrile/water mobile phase (0.1% hydrochloric acid) in 20 min; detector, UV 254/220 nm; to obtain compound **161** (yellow solid, 35 mg, yield: 68%). MS (ESI, m/z): 501.2[M + H]⁺; ¹H NMR (400 MHz, DMSO-*d₆*) *δ* 10.54 (s, 1H), 9.97 - 9.93 (m, 1H), 9.67 (s, 2H), 7.79 - 7.75 (m, 2H), 7.66 (d, *J* = 8.4 Hz, 1H), 7.55 (d, *J* = 5.9 Hz, 1H), 7.42 (t, *J* = 7.6 Hz, 1H), 7.21 (t, *J* = 7.6 Hz, 1H), 7.09 (s, 1H), 6.95 (s, 1H), 4.48 - 4.45 (m, 4H), 4.16 - 4.14 (m, 2H), 3.89 - 3.86 (m, 2H), 3.25 - 3.18 (m, 2H), 2.76 (d, *J* = 4.8 Hz, 6H), 2.22 - 2.15 (m, 2H), 2.00 - 1.91 (m, 4H).

### Embodiment 45 (Synthesis method XXXIII)

### 4-((S or R)-4-((1R,5S)-3,8-diazabicyclo[3.2.1]octan-3-yl)-6-chloro-2-(3-(dimethylamino)bicyclo [1.1.1]pentan-1 -yl)-8-fluoroquinazolin-7-yl)naphthalen-2-ol dihydrochloride 162a; 4-((R or S)-4-((1R,5S)-3,8-diazabicyclo[3.2.1]octan-3-yl)-6-chloro-2-(3-(dimethylamino)bicyclo[1.1.1]pentan-1-yl)-8-fluoroquinazolin-7-yl)naphthalen-2-ol dihydrochloride 162b

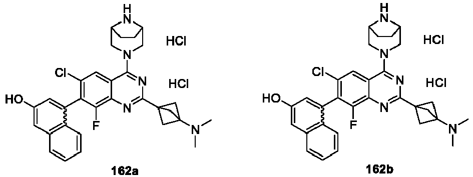

The syntetic route was as follows:

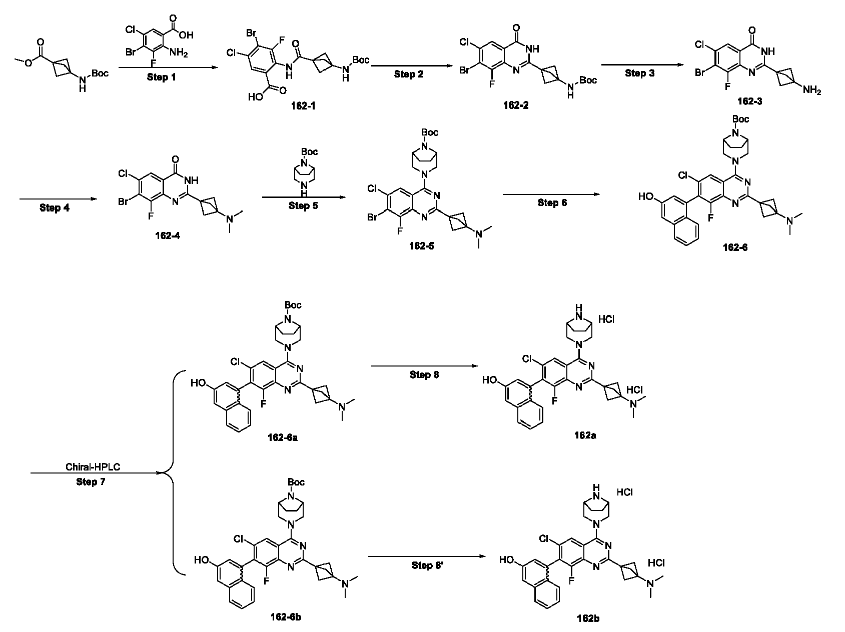

### Step 1

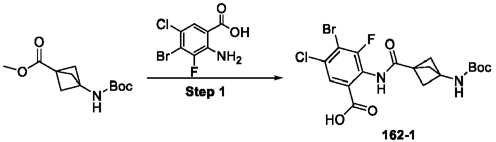

Methyl 3-[(*tert*-butoxycarbonyl)amino]bicyclo[1.1.1]pentane-1-carboxylate (1.6 g, 6.30 mmol, 1 eq) was added to a solution of 2-amino-4-bromo-5-chloro-3-fluorobenzoic acid (4.4 g, 15.75 mmol, 2.5 eq) in anhydrous toluene (50.00 mL) with stirring under the protection of nitrogen at 0 °C. Lithium bistrimethylsilylamide (1 mol/L tetrahydrofuran solution, 34 mL, 34 mmol, 5.5 eq) was slowly added dropwise to the above reaction solution while maintaining the temperature. After the dropwise addition, the reaction was carried out at 25 °C for 3 hours, and the reaction process was monitored by liquid chromatography-mass spectrometry. After the reaction was completed, the reaction solution was concentrated under reduced pressure, quenched by adding saturated ammonium chloride solution, extracted with ethyl acetate (150 mL × 3), and the organic phases were combined, dried over anhydrous sodium sulfate, filtered, and the filtrate was concentrated to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **162-1** (brownish yellow solid, 2 g, yield: 68%). MS (ESI, m/z): 474.9/476.9/478.9[M-H]⁻; ¹H NMR (300 MHz, DMSO-*d₆*) *δ* 12.50 (s, 1H), 7.87 (d, *J* = 1.8 Hz, 1H), 7.66 - 7.59 (m, 1H), 2.18 (s, 6H), 1.40 (s, 9H).

### Step 2

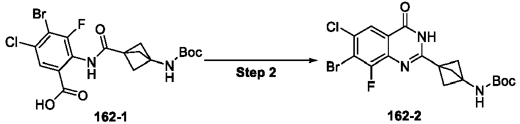

Compound **162-1** (700 mg, 1.35 mmol, 1.0 eq), *O*-(7-azabenzotriazol-1-yl)-*N,N,N',N*'-tetramethylurea hexafluorophosphate (812 mg, 2.03 mmol, 1.5 eq), *N*,*N-*dimethylformamide (28 mL) and *N,N-*diisopropylethylamine (644 mg, 4.73 mmol, 3.5 eq) were added to a 100 mL single-neck flask with stirring at 25 °C. The reaction was carried out at this temperature for 0.5 hours, and then to the reaction solution was added a solution of ammonia in tetrahydrofuran (1.30 mol/L, 2.6 mL, 2.5 eq). The reaction was carried out for 8 hours at 80 °C, and the reaction process was monitored by liquid chromatography-mass spectrometry. After the reaction was completed, the reaction mixture was cooled to 25 °C, then directly purified by reversed-phase chromatography (C18 column), eluted with 40%→80% actonitrile/water (0.1% formic acid) mobile phase in 20 min; detector: UV254/220 nm. Compound **162-2** (white solid, 430 mg, yield: 65%) was obtained. MS (ESI, m/z): 458.1/460.1/462.1[M + H]⁺; ¹H NMR (300 MHz, DMSO-*d₆*) *δ* 12.64 (s, 1H), 8.03 (d, *J* = 1.8 Hz, 1H), 7.66 (s, 1H), 2.33 (s, 6H), 1.40 (s, 9H).

### Step 3

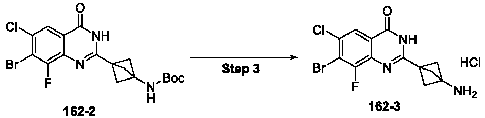

A solution of hydrochloric acid (4 mol/L, 10 mL) in 1,4-dioxane was added dropwise to a solution of compound **162-2** (430 mg, 0.89 mmol, 1.0 eq) in methanol (10 mL) with stirring at 0 °C. The reaction was carried out for 2 hours at 25 °C, and the reaction process was monitored by thin layer chromatography. After the reaction was completed, the reaction solution was concentrated to obtain a crude product of **162-3** (yellow solid, 300 mg). The crude product was used directly in the next synthesis without further purification.

### Step 4

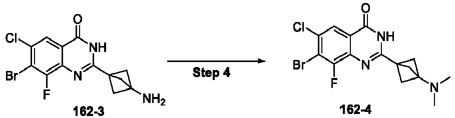

An aqueous formaldehyde solution (37%, 95 mg, 3.01 mmol), sodium acetate (260 mg, 3.01 mmol) and sodium cyanoborohydride (200 mg, 3.01 mmol) were successively added to a solution of compound **162-3** (300 mg) in methanol (48 mL) with stirring at 25 °C. The reaction was carried out for 2 hours at 25 °C, and the reaction process was monitored by liquid chromatography-mass spectrometry. After the reaction was completed, the reaction mixture was concentrated under reduced pressure to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **162-4** (white solid, 300 mg). (ESI, m/z): 386.0/388.0/390.0[M + H]⁺ ; ¹H NMR (300 MHz, DMSO-*d₆*) *δ* 12.63 (s, 1H), 8.04 (s, 1H), 2.16 (s, 6H), 2.13 (s, 6H).

### Step 5

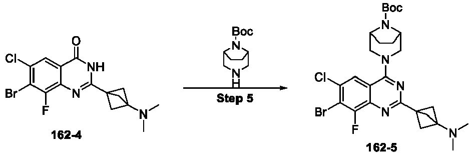

Bromo-tris-pyrrolidino-phosphonium hexafluorophosphate (506 mg, 1.03 mmol, 1.5 eq) and *N*,*N-*diisopropylethylamine (280 mg, 2.06 mmol, 3.0 eq) were added to a solution of compound **162-4** (280 mg, 0.68 mmol, 1.0 eq) in *N*-methylpyrrolidone (6 mL) under the protection of nitrogen at 25 °C. The reaction was carried out at 25 °C under nitrogen atmosphere for 30 min, and then *tert*-butyl 3,8-diazabicyclo[3.2.1]octane-8-carboxylate (230 mg, 1.03 mmol, 1.5 eq) was added to continue the reaction for 1 hour, and the reaction process was monitored by liquid chromatography-mass spectrometry. After the reaction was completed, the obtained crude product was directly purified by reversed-phase chromatography (C18 column), eluted with 60%→95% acetonitrile/water (0.1% sodium bicarbonate) mobile phase in 20 min; detector: UV254/220 nm. Compound **162-5** (yellow solid, 145 mg, yield: 34%) was obtained. (ESI, m/z): 580.1/582.1/584.1 [M + H]⁺; ¹H NMR (400 MHz, CDCl₃) *δ* 7.71 - 7.70 (m, 1H), 4.39 - 4.30 (m, 4H), 3.66 - 3.49 (m, 2H), 2.42 (s, 6H), 2.27 (s, 6H), 1.93 - 1.88 (m, 2H), 1.72 - 1.67 (m, 2H), 1.52 (s, 9H).

### Step 6

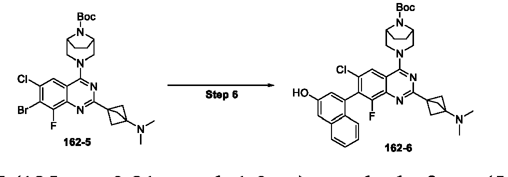

Compounds **162-5** (125 mg, 0.21 mmol, 1.0 eq), tetrahydrofuran (5 mL), water (0.5 mL), potassium phosphate (91 mg, 0.41 mmol, 2.0 eq), 4-(4,4,5,5-tetramethyl-1,3,2-dioxyboroboran-2-yl) naphthalen-2-ol (87 mg, 0.31 mmol, 1.5 eq) and chloro(2-dicyclohexylphosphino-2',4',6'-tri-i-propyl-1,1'-biphenyl)(2'-aMino-1,1 '-biphenyl-2-yl) palladium (II) (17 mg, 0.02 mmol, 0.1 eq) were successively added to a 25 mL Schlenk tube under the protection of nitrogen at 25 °C. The reaction was carried out for 1 hour at 60 °C under nitrogen atmosphere. The reaction process was monitored by liquid chromatography-mass spectrometry. After the reaction was completed, the reaction mixture was cooled to 25 °C, and concentrated to obtain a crude product. The obtained crude product was purified by silica gel column chromatography, eluted with a gradient of 0%→8% methanol/dichloromethane mobile phase, and the obtained fractions were evaporated under reduced pressure to remove the solvent to obtain compound **162-6** (yellow solid, 110 mg, yield: 79%). (ESI, m/z): 644.3/646.3[M + H]⁺.

### Step 7

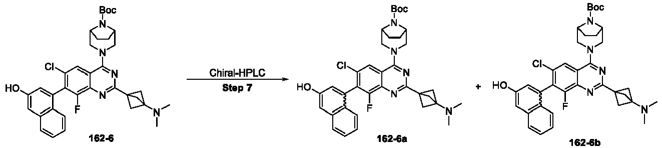

The compound **162-6** (100 mg) obtained in step 6 was subjected to chiral resolution by preparative chiral high pressure liquid chromatography under the following conditions: chiral column CHIRAL ART Cellulose-SC, 2 × 25 cm, 5 µm; mobile phase A: n-hexane (10 mmol/L ammonia methanol solution), mobile phase B: isopropanol; flow rate: 25 mL/min; elution with 40% mobile phase B in 15 min; detector: UV 252/318 nm; two products were obtained. The product with shorter retention time (4.15 min) was **162-6a,** *tert-butyl* (1*R*,5*S*)-3-((*S* or *R*)-6-chloro-2-(3-(dimethylamino)bicyclo[1.1]pentan-1-yl)-8-fluoro-7-(3-hydroxynaphthalen-1-yl)quinazolin-4-yl)-3,8-diazacyclo[3.2.1]octane-8-carboxylate (yellow solid, 36 mg, recovery rate: 36%); the product with longer retention time (6.42 min) was **162-6b,** *tert*-butyl (1*R*,5*S*)-3-((*R* or *S*)-6-chloro-2-(3-(dimethylamino)bicyclo[1.1]pentan-1-yl)-8-fluoro-7-(3-hydroxynaphthalen-1-yl)quinazolin-4-yl)-3,8-diazacyclo[3.2.1]octane-8-carboxylate (yellow solid, 33 mg, recovery rate: 33%).

### Step 8

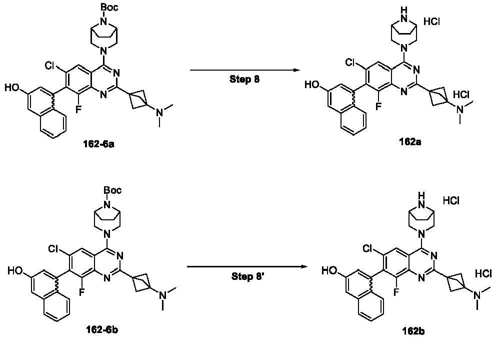

A solution of hydrochloric acid (2 mL, 4 mol/L) in 1,4-dioxane was added dropwise to a solution of compound **162-6a** (36 mg, 0.05 mmol, 1.0 eq) in methanol (2 mL) with stirring at 0 °C. The obtained reaction was carried out for 2 hours at 25 °C, and the reaction process was monitored by liquid chromatography-mass spectrometry. After the reaction was completed, the reaction mixture was concentrated to obtain a crude product. The obtained crude product was purified by reversed-phase chromatography (C18 column), and eluted with 10%→ 50% acetonitrile/water mobile phase (0.1% hydrochloric acid) in 20 min; detector, UV 254/220 nm; to obtain compound **162a** (yellow solid, 12 mg, yield: 41%). Compound **162b** (yellow solid, 20 mg, yield: 65%) can be obtained by the same method as above.

Compound **162a:** (ESI, m/z): 544.2/546.2[M + H]⁺; ¹H NMR (300 MHz, DMSO-*d₆*) *δ* 11.66 (s, 1H), 10.09 (s, 1H), 9.76 (s, 1H), 9.53 (s, 1H), 8.02 (s, 1H), 7.83 (d, *J* = 8.3 Hz, 1H), 7.49 - 7.42 (m, 1H), 7.31 (d, *J* = 2.4 Hz, 1H), 7.26 - 7.19 (m, 2H), 7.10 (d, *J* = 2.4 Hz, 1H), 4.65 - 4.52 (m, 2H), 4.20 - 4.16 (m, 2H), 3.97 - 3.86 (m, 2H), 2.77 - 2.74 (m, 6H), 2.43 (s, 6H), 1.99 - 1.90 (s, 4H); ¹⁹F NMR (282 MHz, DMSO-*d₆*) *δ*-120.21. The chiral analysis conditions of compound **162a** were: CHIRALPAK IC-3, 4.6 × 50 mm, 3 µm; mobile phase A: *n*-hexane (0.1% diethylamine), mobile phase B: isopropanol; flow rate: 1 mL/min; isocratic elution with 30% phase B in 6 min; detector: UV 220 nm; retention time: 2.239 min; ee > 99%.

Compound **162b:** (ESI, m/z): 544.2/546.2[M + H]⁺; ¹H NMR (300 MHz, DMSO-*d₆*) *δ* 11.79 (s, 1H), 10.10 (s, 1H), 9.85 - 9.80 (m, 1H), 9.61 (s, 1H), 8.02 (d, *J* = 1.6 Hz, 1H), 7.82 (d, *J* = 8.3 Hz, 1H), 7.48 - 7.43 (m, 1H), 7.32 (d, *J* = 2.4 Hz, 1H), 7.26 - 7.19 (m, 2H), 7.10 (d, *J* = 2.4 Hz, 1H), 4.65 - 4.51 (m, 2H), 4.20 - 4.16 (m, 2H), 3.98 - 3.87 (m, 2H), 2.75 (d, *J* = 4.0 Hz, 6H), 2.43 (s, 6H), 1.99 - 1.88 (m, 4H); ¹⁹F NMR (282 MHz, DMSO-*d₆*) *δ*-120.21. The chiral analysis conditions of compound **162b** were: CHIRALPAK IC-3, 4.6 × 50 mm, 3 µm; mobile phase A: *n*-hexane (0.1% diethylamine), mobile phase B: isopropanol; flow rate: 1 mL/min; isocratic elution with 30% phase B in 6 min; detector UV 220 nm; retention time: 3.881 min; ee > 99%.

### Embodiment 46

### 4-((7R or 7S)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)naphthalen-2-amine tritrifluoroacetate 192a; 4-((7S or 7R)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)naphthalen-2-amine tritrifluoroacetate 192b

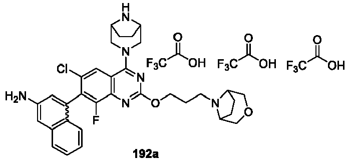

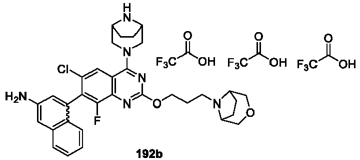

The synthetic route was as follows:

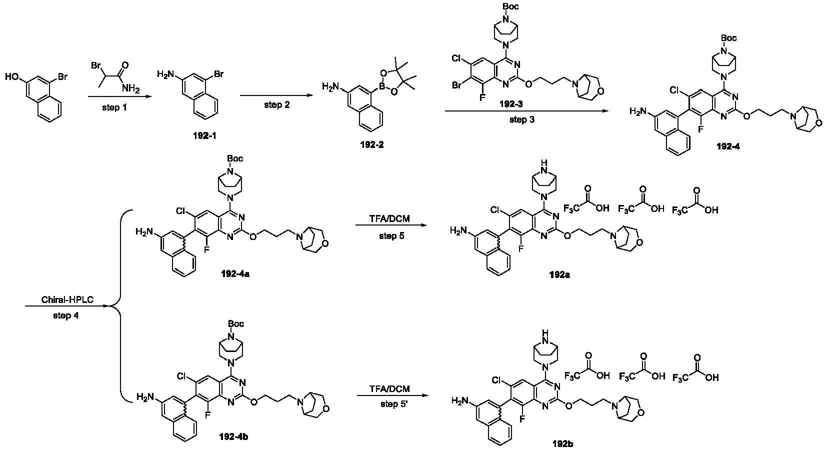

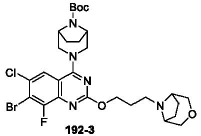

Compound **192-3** was synthesized according to Embodiment 6 (synthesis method V). MS (ESI, m/z): 640.2/642.2/644.2[M + H]⁺.

### Step 1

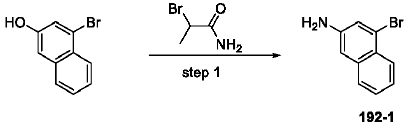

Potassium hydroxide (2 g, 35.82 mmol, 4 eq) was added in batches to a mixed solution of compound 1-bromo-3-hydroxynaphthalene (2 g, 8.96 mmol, 1.0 eq) and 2-bromopropionamide (2.7 g, 17.93 mmol, 2 eq) in dimethyl sulfoxide (30 mL) and *N,N-*dimethylpropenylurea (10 mL) with stirring under the protection of nitrogen at 25 °C. The reaction was carried out for 48 hours at 160 °C under nitrogen atmosphere, and the reaction process was monitored by liquid chromatography-mass spectrometry and thin layer chromatography. After the reaction was completed, the reaction mixture was returned to room temperature. 200 mL of water was added to the reaction solution for dilution, and the mixture was extracted with dichloromethane (200 mL x 3), then the organic phases were combined, dried over anhydrous sodium sulfate, filtered, and the filtrate was concentrated to obtain a crude product. The reaction mixture was directly purified by reversed-phase chromatographic column (C18 column), and eluted with 30%→ 50% acetonitrile/water (0.1% formic acid) in 10 min; detector, UV254/220 nm; to obtain compound **192-1** (white solid, 1.4 g, yield: 70%). MS (ESI, m/z): 222.0/224.0[M + H]⁺; ¹H NMR (400 MHz, CDCl₃) *δ* 8.05 (d, *J* = 8.4 Hz, 1H), 7.55 (d, *J* = 8.4 Hz, 1H), 7.43 - 7.35 (m, 1H), 7.33 - 7.28 (m, 1H), 7.27 (s, 1H), 6.93 (d, *J=* 2.1 Hz, 1H), 3.82 (s, 2H).

### Step 2

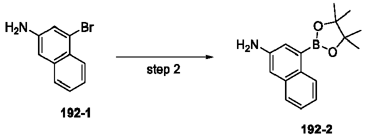

Potassium acetate (509 mg, 5.13 mmol, 3.0 eq) and [1,1'-bis(diphenylphosphino)ferrocene]palladium dichloride (141 mg, 0.17 mmol, 0.1 eq) were added to a solution of compound **192-1** (400 mg, 1.71 mmol, 1.0 eq) and bis(pinacolato)diboron (571 mg, 2.22 mmol, 1.3 eq) in 1,4-dioxane (4 mL) with stirring under the protection of nitrogen at 25 °C. The reaction was carried out for 1 hour at 80 °C under nitrogen atmosphere, and the reaction process was monitored by liquid chromatography-mass spectrometry and thin layer chromatography. After the reaction was completed, the reaction mixture was concentrated to obtain a crude product. The crude product was purified by silica gel column chromatography, eluted with a gradient of 0%→10% dichloromethane/petroleum ether mobile phase, and the obtained fractions were evaporated under reduced pressure to remove the solvent to obtain compound **192-2** (brown solid, 288 mg, yield: 59%). MS (ESI, m/z): 270.2[M + H]⁺; ¹H NMR (300 MHz, CDCl₃) *δ* 8.67 - 8.59 (m, 1H), 7.65 - 7.56 (m, 2H), 7.43 - 7.30 (m, 2H), 7.12 (d, *J* = 2.5 Hz, 1H), 1.44 (s, 12H).

### Step 3

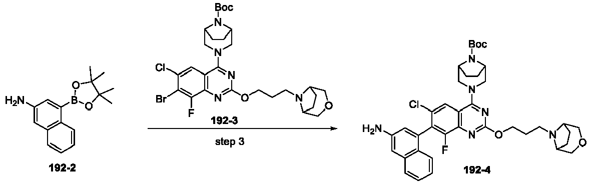

Compound **192-2** (302 mg, 1.06 mmol, 1.2 eq), potassium carbonate (248 mg, 1.78 mmol, 2.0 eq) and [1,1'-bis (diphenylphosphino)ferrocene]palladium dichloromethane complex (73 mg, 0.09 mmol, 0.1 eq) were added to a solution of compound **192-3** (600 mg, 0.88 mmol, 1.0 eq) in tetrahydrofuran/water (5/1, 6 mL) with stirring under the protection of nitrogen at 25 °C. The reaction was carried out for 2 hours at 80 °C, and the reaction process was monitored by liquid chromatography-mass spectrometry and thin layer chromatography. After the reaction was completed, the reaction mixture was concentrated to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **192-4** (yellow solid, 380 mg, yield: 57%). MS (ESI, m/z): 703.4/705.4[M + H]⁺.

### Step 4

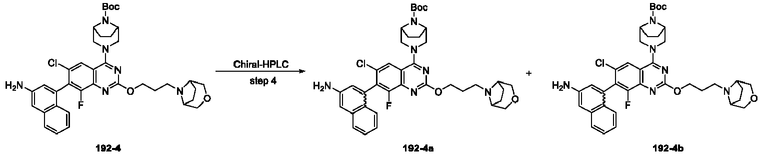

Compound **192-4** (300 mg) obtained in step 3 was subjected to chiral resolution by preparative chiral high performance liquid chromatography: chiral column CHIRAL ART Cellulose - SB, 2 × 25 cm, 5 µm; mobile phase A: *n*-hexane/methyl *tert*-butyl ether (1/1) (0.5% 2 mmol/L ammonia-methanol), mobile phase B: methanol; flow rate: 25 mL/min; elution with 10% phase B in 15 min, detector UV 240/214 nm. Two products were obtained, the compound with the shorter retention time (10.3575 min) was compound **192-4a,** *tert-*butyl(1*R*,5*S*)-3-((7*R* or 7*S*)-2-(3-(3-oxa-8-azabicyclo[3.2.1]octan-8-yl)propoxy)-7-(3-aminonaphthalen-1-yl)-6-chloro-8-fluoroquinazolin-4-yl)-3,8-diazabicyclo[3.2.1]octane-8-carboxylate (yellow solid, 120 mg, recovery rate: 40%), MS (ESI, m/z): 703.4/705.4[M + H]⁺; the product with longer retention time (12.8425 min) was compound **192-4b,** *tert-butyl*(1*R*,5*S*)-3-((7*S* or 7*R*)-2-(3-(3-oxa-8-azabicyclo[3.2.1]octan-8-yl)propoxy)-7-(3-aminonaphthalen-1-yl)-6-chloro-8-fluoroquinazolin-4-yl)-3,8-diazabicyclo[3.2.1]octane-8-carboxylate (yellow solid, 120 mg, recovery rate: 40%), MS (ESI, m/z): 703.4/705.4[M + H]⁺.

### Step 5

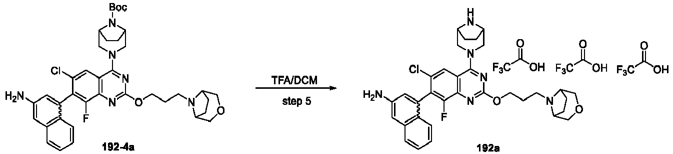

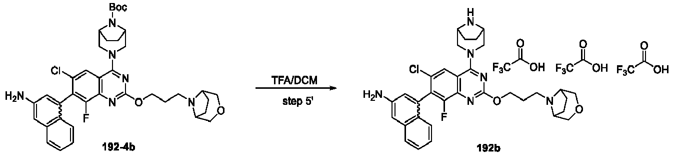

Trifluoroacetic acid (0.2 mL) was added dropwise to a solution of compound **192-4a** (30 mg, 0.04 mmol, 1.0 eq) in dichloromethane (0.8 mL) with stirring at 0 °C, and the reaction solution was stirred at 25 °C for 1 hour, the reaction process was monitored by liquid chromatography-mass spectrometry and thin layer chromatography. After the reaction was completed, the reaction solution was concentrated under reduced pressure to obtain a crude product. The obtained crude product was purified by reversed-phase rapid chromatographic column (C18 column), and eluted with 20%→ 50% acetonitrile/water mobile phase (10 mmol/L trifluoroacetic acid) in 20 min; detector, UV254/220 nm; to obtain **192a** (yellow solid, 10 mg, yield: 25%). Compound **192b** (yellow solid, 13 mg, yield: 33%) can be obtained by the same method as above.

Compound **192a:** MS (ESI, m/z): 603.3/605.3[M + H]⁺; ¹H NMR (300 MHz, DMSO-*d₆) δ* 7.96 (d, *J* = 1.5 Hz, 1H), 7.81 (d, *J* = 8.3 Hz, 1H), 7.51 - 7.43 (m, 1H), 7.41 (d, *J* = 2.2 Hz, 1H), 7.30 - 7.22 (m, 1H), 7.21 - 7.14 (m, 1H), 7.12 - 7.09 (m, 1H), 4.56 - 4.38 (m, 4H), 4.22 - 4.11 (m, 2H), 4.01 - 3.94 (m, 2H), 3.85 - 3.68 (m, 6H), 3.20 - 3.06 (m, 2H), 2.19 - 2.08 (m, 4H), 2.07 - 1.88 (m, 6H); ¹⁹F NMR (282 MHz, DMSO-*d*₆) *δ*-74.18, -122.16. The chiral analysis conditions of compound **192a** were: CHIRAL Cellulose-SB, 4.6 x 100 mm, 3 µm; mobile phase A: *n*-hexane/*tert*-butyl methyl ether=1/1 (0.1% diethylamine), mobile phase B: ethanol; flow rate: 1 mL/min; isocratic elution with 30% phase B in 8.5 min; detector UV 220/254 nm; retention time: 5.962 min;. ee > 99%.

Compound **192b:** MS (ESI, m/z): 603.3/605.3[M + H]⁺; ¹H NMR (300 MHz, DMSO-*d₆*) *δ* 7.97 (s, 1H), 7.81 (d, *J* = 8.3 Hz, 1H), 7.52 - 7.41 (m, 1H), 7.39 (d, *J* = 2.2 Hz, 1H), 7.30 - 7.12 (m, 2H), 7.10 (d, *J* = 2.2 Hz, 1H), 4.64 - 4.32 (m, 4H), 4.29 - 4.09 (m, 2H), 4.07 - 3.94 (m, 2H), 3.77 - 3.72 (m, 6H), 3.17 - 3.04 (m, 2H), 2.22-1.92 (m, 10H). ¹⁹F NMR (282 MHz, DMSO-*d*₆) *δ*-74.24, -122.16. The chiral analysis conditions of compound **192b** were: CHIRAL Cellulose-SB, 4.6 x 100 mm, 3 µm; mobile phase A: *n*-hexane/*tert*-butyl methyl ether=1/1 (0.1% diethylamine), mobile phase B: ethanol; flow rate: 1 mL/min; isocratic elution with 30% phase B in 8.5 min; detector UV 220/254 nm; retention time: 7.373 min. ee > 98%.

### Embodiment 47

### 4-((R or S)-6-chloro-4-((1R,5S)-1,5-dimethyl-3,8-diazabicyclo[3.2.1]octan-3-yl)-8-fluoro-2-(((S)-1-methylpyrrolidin-2-yl)methoxy)quinazolin-7-yl)naphthalen-2-ol diformate 194a; 4-((S or R)-6-chloro-4-((1R,5S)-1,5-dimethyl-3,8-diazabicyclo[3.2.1]octan-3-yl)-8-fluoro-2-(((S)-1-methylpyrrolidin-2-yl)methoxy)quinazolin-7-yl)naphthalen-2-ol diformate 194b.

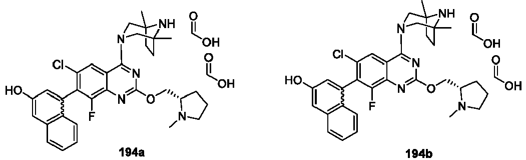

The synthetic route was as follows:

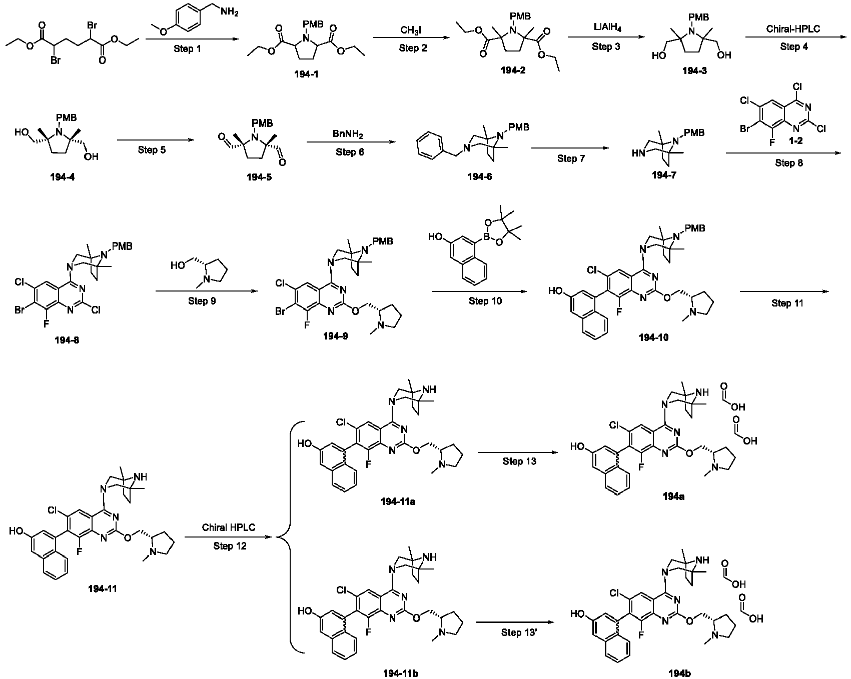

### Step 1

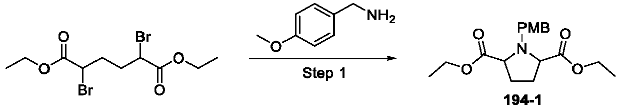

4-Methoxybenzylamine (228.61 g, 1583.148 mmol, 3 eq) was slowly added to a solution of diethyl 2,5-dibromohexanedioate (200 g, 527.716 mmol, 1 eq) in toluene (2 L) with stirring under the protection of nitrogen at 25 °C. The reaction was carried out for 16 hours at 110 °C, and the reaction process was monitored by liquid chromatography-mass spectrometry and thin layer chromatography. After the reaction was completed, the insolubles were removed by filtration, and the filter cake was washed with toluene (50 mL x 3), and the filtrate was concentrated under reduced pressure to obtain a crude product. The crude product was purified by silica gel column chromatography, eluted with a gradient of 0%→ 10% ethyl acetate/petroleum ether mobile phase, and the obtained fractions were evaporated under reduced pressure to obtain compound **194-1** (light yellow oil, 150 g, yield: 85%). MS (ESI, m/z): 336.2[M + H]⁺. ¹H NMR (300 MHz, CDCl₃) *δ* 7.34 - 7.19 (m, 2H), 6.90 - 6.77 (m, 2H), 4.07 (q, *J=* 7.1 Hz, 4H), 3.92 (s, 2H), 3.80 (s, 3H), 3.45 (s, 2H), 2.13 - 2.04 (m, 4H), 1.22 (t, *J=* 7.1 Hz, 6H).

### Step 2

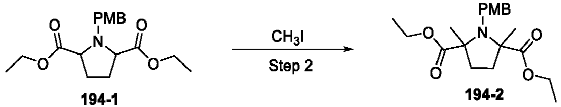

Lithium diisopropylamide (1 mol/L, 1062.2 mL, 1062.2 mmol, 2.5 eq) was added slowly to a solution of compound **194-1** (150 g, 424.866 mmol, 1 eq) in tetrahydrofuran (100 mL) with stirring under the protection of nitrogen at -40 °C. The obtained mixture was stirred at -40 °C for 0.5 hours, iodomethane (158.70 g, 1.12 mol, 2.5 eq) was slowly added to the reaction solution at the same temperature. The reaction was carried out for 2 hours at 25 °C, and the reaction process was monitored by liquid chromatography-mass spectrometry and thin layer chromatography. After the reaction was completed, the reaction was quenched by adding saturated ammonium chloride (500 mL) to the mixture at 0 °C. The reaction mixture was extracted with ethyl acetate (300 mL x 3), and the organic phases were combined, washed with saturated brine, dried over anhydrous sodium sulfate, filtered to remove the drying agent, and the filtrate was concentrated to obtain a crude product. The crude product was purified by silica gel column chromatography, eluted with a gradient of 0%→ 10% ethyl acetate/petroleum ether mobile phase, and the obtained fractions were evaporated under reduced pressure to remove the solvent to obtain compound **194-2** (colorless oil, 96 g, yield: 59%). MS (ESI, m/z): 364.2[M + H]⁺; ¹H NMR (300 MHz, CDCl₃) δ 7.33 - 7.16 (m, 2H), 6.86 - 6.74 (m, 2H), 4.20 - 3.93 (m, 5H), 3.79 (d, *J* = 4.6 Hz, 3H), 2.43 (m, 1H), 2.21 - 1.90 (m, 3H), 1.81 - 1.71 (m, 1H), 1.47 (s, 4H), 1.31 - 1.25 (m, 4H), 1.21 (m, 4H).

### Step 3

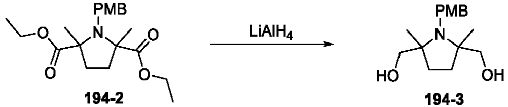

A solution of lithium aluminum hydride (2.5 mol/L, 194.47 mL, 486.168 mmol, 3 eq) in tetrahydrofuran was slowly added to a solution of compound **194-2** (62 g, 162.056 mmol, 1 eq) in tetrahydrofuran (650 mL) with stirring under the protection of nitrogen at 0 °C. The obtained mixture was stirred for 2 hours at 25 °C, and the reaction process was monitored by liquid chromatography-mass spectrometry and thin layer chromatography. After the reaction was completed, water (18.5 mL), 15% sodium hydroxide solution (18.5 mL) and water (55.5 mL) were successively added dropwise to the reaction solution with stirring at 0 °C. The insolubles were then removed by filtration and the filter cake was washed with tetrahydrofuran (50 mL x 50). The filtrate was concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound 194-3 (light yellow oil, 45 g, yield: 94%). MS (ESI, m/z): 280.2[M + H]⁺.

### Step 4

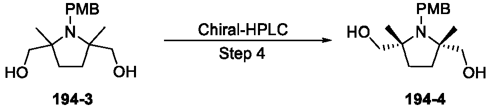

Compound **194-3** (45 g) obtained in step 3 was separated by preparative chiral high performance liquid chromatography under the following conditions: chiral column CHIRAL ART Cellulose-SC, 5 x 25 cm, 10 µm; mobile phase A : supercritical carbon dioxide fluid, mobile phase B: methanol/dichloromethane (1/1); flow rate: 150 mL/min; column temperature: 35 °C; elution with 30% mobile phase B in 8 min; detector UV220 nm; the compound with a retention time of 5.69 min was **194-4,** ((2*R*,5*S*)-1-(4-methoxybenzyl)-2,5-dimethylpyrrolidin-2,5-diyl)dimethanol (light yellow oil, 11 g, recovery rate: 24%), MS (ESI, m/z): 280.2[M + H]+.

### Step 5

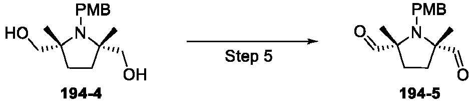

Oxalyl chloride (11.81 mL, 88.412 mmol, 4 eq) was added dropwise to a solution of dimethyl sulfoxide (7.44 mL, 99.464 mmol, 4.5 eq) in dichloromethane (80 mL) at -78 °C with stirring under the protection of nitrogen. The reaction was carried out with stirring at -78 °C for 0.5 hours, compound **194-4** (6.5 g, 22.103 mol, 1.0 eq) was added dropwise to the reaction solution at the same temperature. The obtained mixture was continued to react for 1 hour with stirring under the protection of nitrogen at -78 °C. *N*,*N*-diisopropylethylamine (32.42 mL, 176.82 mmol, 8 eq) was then added to the above reaction solution at -78°C. The obtained mixture was continued to react for 1 hour with stirring under the protection of nitrogen at room temperature. The reaction process was monitored by liquid chromatography-mass spectrometry and thin layer chromatography. After the reaction was completed, the reaction solution was concentrated to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **194-5** (light yellow oil, 6 g, yield: 69%). MS (ESI, m/z): 276.2[M + H]⁺.

### Step 6

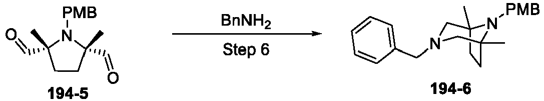

Sodium cyanoborohydride (3.03 g, 45.76 mmol, 3.0 eq)) and compound **194-5** (6 g, 15.253 mmol, 1.0 eq) were added to a solution of benzylamine (2.06 g, 18.30 mmol, 1.2 eq), acetic acid (1.16 g, 18.30 mmol, 1.2 eq) in methanol (60 mL) with stirring at 25°C. The reaction was carried out for 2 hours at 25 °C with stirring, and the reaction process was monitored by liquid chromatography-mass spectrometry and thin layer chromatography. After the reaction was completed, the reaction solution was concentrated to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **194-6** (light yellow oil, 2.2 g, yield: 39%). MS (ESI, m/z): 351.2[M + H]⁺; ¹H NMR (300 MHz, CDCl₃) *δ* 7.40 - 7.27 (m, 7H), 6.89 - 6.82 (m, 2H), 3.82 (s, 3H), 3.66 (s, 2H), 3.52 (s, 2H), 2.33 - 2.18 (m, 4H), 2.02 - 1.90 (m, 2H), 1.64 - 1.51 (m, 2H), 0.97 (s, 6H).

### Step 7

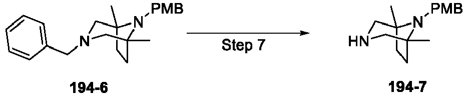

Palladium hydroxide/carbon (10%, 300 mg) and acetic acid (0.51 g, 8.13 mmol, 3.0 eq) were added to a solution of compound **194-6** (1 g, 2.71 mmol, 1.0 eq) in ethanol (7 mL) with stirring at 25 °C. The reaction was carried out for 8 hours at 60 °C under hydrogen atmosphere of 10 atmospheric pressures. After the reaction was completed, the reaction mixture was cooled to 25 °C. After depressurization, the insolubles were then removed by filtration and the filter cake was washed with ethanol (20 mL x 3). The filtrate was concentrated to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **194-7** (colorless oil, 370 mg, yield: 49%). MS (ESI, m/z): 261.2[M + H]⁺.

### Step 8

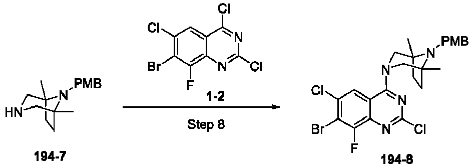

Triethylamine (422.7 g, 3.969 mmol, 3.0 eq) and compound **194-7** ((362.6 mg, 1.32 mmol, 1.0 eq) were added to a solution of compound **1-2** (460 mg, 1.32 mmol, 1.0 eq) in dichloromethane (7 mL) with stirring under the protection of nitrogen at 0 °C. The reaction was carried out for 1 hour at 25 °C with stirring, and the reaction process was monitored by liquid chromatography-mass spectrometry and thin layer chromatography. After the reaction was completed, the reaction solution was concentrated to obtain a crude product. The crude product was purified by silica gel column chromatography, eluted with a gradient of 0%→ 10% ethyl acetate/petroleum ether mobile phase, and the obtained fractions were evaporated under reduced pressure to remove the solvent to obtain compound **194-8** (yellow solid, 700 mg, yield: 90%). MS (ESI, m/z): 553.1/555.1/557.1[M + H]⁺; ¹H NMR (300 MHz, CDCl₃) *δ* 7.83 (s, 1H), 7.34 (d, *J* = 8.2 Hz, 2H), 6.86 (d, *J=* 8.2 Hz, 2H), 4.01 (d, *J* = 12.8 Hz, 2H), 3.85 - 3.79 (m, 5H), 3.51 (d, *J* = 12.8 Hz, 2H), 1.73 - 1.60 (m, 4H), 1.08 (s, 6H).

### Step 9

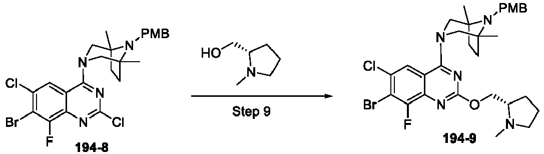

*N*-methyl-*L*-proline (208.0 g, 1.71 mmol, 2.0 eq) and potassium carbonate (376.7 mg, 2.57 mmol, 3.0 eq) were added to a solution of compound **194-8** (500 mg, 0.857 mmol, 1.0 eq) in acetonitrile (5 mL) with stirring under the protection of nitrogen at 25 °C. The reaction was carried out for 24 hours at 80 °C with stirring, and the reaction process was monitored by liquid chromatography-mass spectrometry and thin layer chromatography. After the reaction was completed, the reaction mixture was cooled to 25 °C. The insolubles were then removed by filtration and the filter cake was washed with dichloromethane (10 mL x 3). The filtrate was concentrated and purified by silica gel column chromatography, eluted with a gradient of 0%→10% methanol/dichloromethane mobile phase, and the obtained fractions were evaporated under reduced pressure to remove the solvent to obtain compound **194-9** (light yellow solid, 260 mg, yield: 45%). MS (ESI, m/z): 632.1/634.2/636.2[M + H]⁺; ¹H NMR (300 MHz, CDCl₃) *δ* 7.79 (d, *J* = 2.0 Hz, 1H), 7.34 (d, *J* = 8.5 Hz, 2H), 6.86 (d, *J* = 8.6 Hz, 2H), 3.94 (t, *J* = 12.5 Hz, 2H), 3.85 - 3.79 (m, 5H), 3.52 - 3.43 (m, 2H), 2.97 - 2.75 (m, 2H), 2.38 - 1.94 (m, 5H), 1.76 - 1.63 (m, 6H), 1.08 (s, 6H), 0.92 - 0.79 (m, 3H).

### Step 10

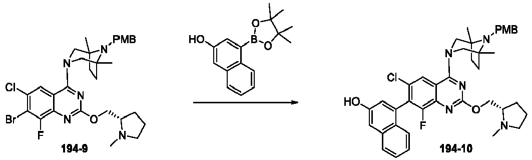

4-(4,4,5,5-Tetramethyl-1,3,2-dioxaborolan-2-yl)naphthalen-2-ol (160.0 mg, 0.56 mmol, 1.5 eq), potassium carbonate (164.9 mg, 1.1 mmol, 3.0 eq) and [1,1'-bis(diphenylphosphino)ferrocene]dichloropalladium(II) (28.9 mg, 0.04 mmol, 0.1 eq) were added to a solution of compound **194-9** (250 mg, 0.375 mmol, 1.0 eq) in 1,4-dioxane/water (5/1, 3.6 mL) with stirring under the protection of nitrogen at 25 °C. The reaction was carried out for 1 hour at 80 °C with stirring, and the reaction process was monitored by liquid chromatography-mass spectrometry and thin layer chromatography. After the reaction was completed, the reaction mixture was cooled to room temperature. The reaction mixture was concentrated and purified by silica gel column chromatography, eluted with a gradient of 0%→10% methanol/dichloromethane mobile phase, and the obtained fractions were evaporated under reduced pressure to remove the solvent to obtain compound **194-10** (a mixture of two stereoisomers, light yellow solid, 220 mg, yield: 80%). MS (ESI, m/z): 696.3/698.3 [M + H]⁺.

### Step 11

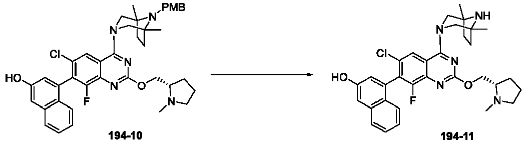

Compound **194-10** (220 mg, 0.3 mmol, 1.0 eq), trifluoroacetic acid (3 mL) and anisole (3 mL) were successively added to a 10 mL reaction flask with stirring at 25 °C. The obtained mixture was stirred at 100°C for 1 hour. The reaction process was monitored by liquid chromatography-mass spectrometry and thin layer chromatography. After the reaction was completed, the reaction mixture was cooled to room temperature, and concentrated under reduced pressure to remove the excess reagent to obtain a crude product. The pH value of the crude product was then adjusted to 8 with saturated sodium bicarbonate solution. The reaction mixture was extracted with dichloromethane (20 mL × 3), and the organic phases were combined, washed with saturated brine, dried over anhydrous sodium sulfate, filtered to remove the drying agent, and the filtrate was concentrated to obtain a crude product. The crude product was purified by preparative thin layer chromatography using dichloromethane/methanol (10/1) as elution agent to obtain compound **194-11** (a mixture of two stereoisomers, light yellow solid, 104 mg, yield: 57%). MS (ESI, *m*/z): 576.2/578.4[M + H]+.

### Step 12

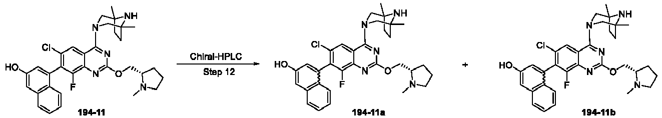

The compound **194-11** (104 mg) obtained in step 11 was subjected to chiral resolution by preparative chiral high performance liquid chromatography under the following conditions: chiral column CHIRAL ART Cellulose-SC, 2 x 25 cm, 5 µm; mobile phase A: *n*-hexane (10 mol/L ammonia methanol solution), mobile phase B: ethanol; flow rate: 20 mL/min; eluted with 20% phase B in 14 min; detector UV 225/210 nm. Two products were obtained. The product with shorter retention time (6.4 min) was compound **194-11a,** 4-((*R* or *S*)-6-chloro-4-((1*R*,5*S*)-1,5-dimethyl-3,8-diazabicyclo[3.2.1]octan-3-yl)-8-fluoro-2-(((*S*)-1-methylpyrrolidin-2-yl)methoxy)quinazolin-7-yl)naphthalen-2-ol (off-white solid, 42 mg, recovery rate: 40%), MS (ESI, m/z): 576.2/578.4[M + H]⁺; the product with longer retention time (10.2 min) was compound **194-11b,** 4-((*S* or *R*)-6-chloro-4-((1*R*,5*S*)-1,5-dimethyl-3,8-diazabicyclo[3.2.1]octan-3-yl)-8-fluoro-2-(((*S*)-1-methylpyrrolidin-2-yl)methoxy)quinazolin-7-yl)naphthalen-2-ol (off-white solid, 50 mg, recovery rate: 48%), MS (ESI, m/z): 576.2/578.4[M + H]⁺.

### Step 13

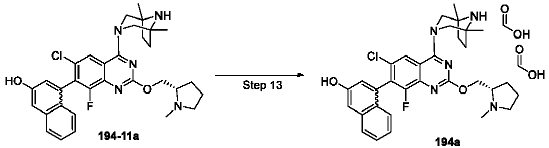

Compound **194-11a** (40 mg, 0.066 mmol, 1.00 eq) was purified by reversed-phase chromatography (C18 column), eluted with 10%→50% methanol/water (0.1% formic acid) mobile phase in 15 min; detector: UV254 nm; compound **194a** was obtained (off-white solid, 33 mg, yield: 74%). MS (ESI, *m*/z): 576.4/578.4[M + H]⁺; ¹H NMR (300 MHz, DMSO-*d*₆) *δ* 8.29 (s, 2H), 8.03 (d, *J* = 1.7 Hz, 1H), 7.91 - 7.85 (m, 1H), 7.54 - 7.49 (m, 1H), 7.36 (d, *J* = 2.4 Hz, 1H), 7.30 (d, *J* = 3.7 Hz, 2H), 7.13 (d, *J* = 2.4 Hz, 1H), 4.52 -4.47 (m, 1H), 4.41-4.33 (m, 2H), 4.26 - 4.22 (m, 1H), 3.42 - 3.36 (m, 2H), 3.08 - 3.02 (m, 1H), 2.76 - 2.67 (m, 1H), 2.45 (s, 3H), 2.34 - 2.25 (m, 1H), 2.06 - 1.96 (m, 1H), 1.92 - 1.67 (m, 5H), 1.60 - 1.55 (m, 2H), 1.33 (s, 6H); ¹⁹F NMR (282 MHz, DMSO-*d*₆) *δ*-122.36. The chiral analysis conditions of compound **194a** were: CHIRALPAK IC-3, 4.6 x 50 mm, 3 µm; mobile phase A: n-hexane (0.1% diethylamine), mobile phase B: ethanol; flow rate: 1 mL/min; isocratic elution with 20% phase B in 8 min; detector UV 220/254 nm; retention time: 1.999 min. ee > 99%.

### Step 13:

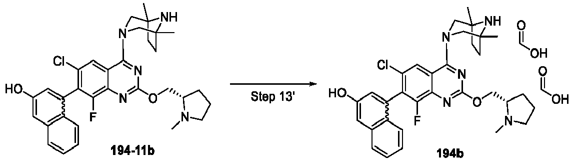

Compound **194-11b** (50 mg, 0.082 mmol, 1.00 eq) was purified by reversed-phase chromatography (C18 column), eluted with 10%→50% methanol/water (0.1% formic acid) mobile phase in 15 min; detector: UV254 nm; compound **194b** was obtained (off-white solid, 28.7 mg, yield: 52%). MS (ESI, *m*/z): 576.4/578.4[M + H]⁺; ¹H NMR (300 MHz, DMSO-*d*₆) *δ* 8.29 (s, 2H), 8.03 (d, *J* = 1.7 Hz, 1H), 7.91 - 7.85 (m, 1H), 7.54 - 7.49 (m, 1H), 7.36 (d, *J* = 2.4 Hz, 1H), 7.30 (d, *J* = 3.7 Hz, 2H), 7.13 (d, *J* = 2.4 Hz, 1H), 4.52 -4.47 (m, 1H), 4.41-4.33 (m, 2H), 4.26 - 4.22 (m, 1H), 3.42 - 3.36 (m, 2H), 3.08 - 3.02 (m, 1H), 2.76 - 2.67 (m, 1H), 2.45 (s, 3H), 2.34 - 2.25 (m, 1H), 2.06 - 1.96 (m, 1H), 1.92 - 1.67 (m, 5H), 1.60 - 1.55 (m, 2H), 1.33 (s, 6H); ¹⁹F NMR (282 MHz, DMSO-*d*₆) *δ*-122.37. The chiral analysis conditions of compound **194b** were: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 8 min; detector UV 220/254 nm; retention time: 3.292 min. ee > 99%.

### Embodiment 48

### (R or S)-4-(4-((1R,5S)-3,8-diazabicyclo[3.2.1]octan-3-yl)-6-chloro-8-fluoro-2-((S)-1-methylpyrrolidin-2-yl)methoxy)quinazolin-7-yl)-3-fluoronaphthalen-2-ol diformate 195a; (S or R)-4-(4-((1R,5S)-3,8-diazabicyclo[3.2.1]octan-3-yl)-6-chloro-8-fluoro-2-((S)-1-methylpyrrolidin-2-yl)methoxy)quinazolin-7-yl)-3-fluoronaphthalen-2-ol formate 195b.

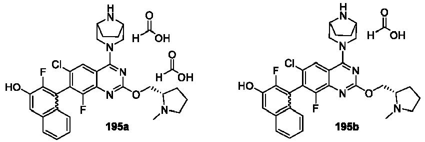

The synthetic route was as follows:

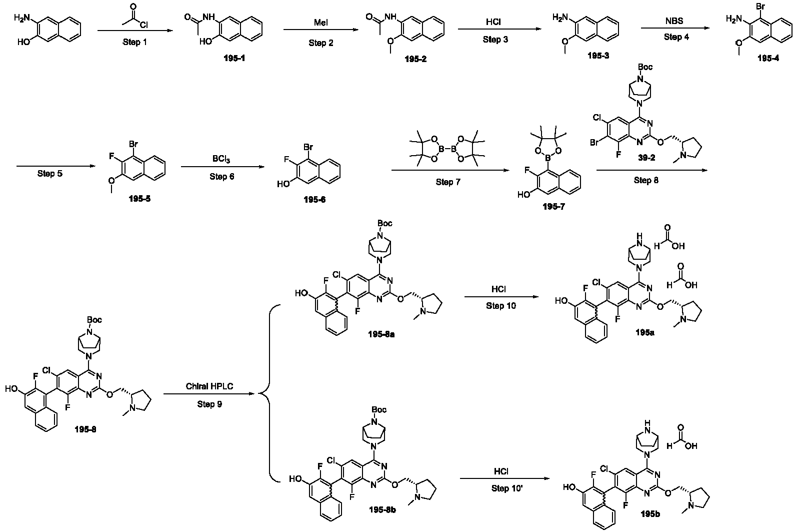

### Step 1

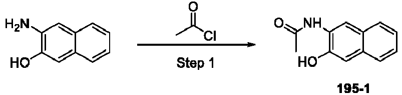

3-Amino-2-naphthalenol (10.0 g, 59.7 mmol, 1.0 eq), sodium bicarbonate (19.0 g, 214.8 mmol, 3.6 eq), water (100 mL) and ether (100 mL) were added to a 500 mL round bottom flask with stirring at 25°C. Acetyl chloride (12.82 g, 155.2 mmol, 2.6 eq) was then added dropwise to the reaction at 0 °C. The mixture was stirred at 0 °C for 4 hours. The reaction process was monitored by liquid chromatography-mass spectrometry and thin layer chromatography. After the reaction was completed, 800 mL of water was added to the reaction solution to quench the reaction, and the organic phase was extracted with ethyl acetate (800 mL x 3), and the organic phase was washed with 800 mL of saturated brine, and the washed organic phase was dried over anhydrous sodium sulfate and filtered to remove the drying agent; the filtrate was concentrated under reduced pressure to obtain a crude product. The crude product was purified by silica gel column chromatography, eluted with a gradient of 0%→ 80% ethyl acetate/petroleum ether mobile phase, and the obtained fractions were evaporated under reduced pressure to obtain compound **195-1** (brown solid, 8.4 g, yield: 66%). MS (ESI, m/z): 202.1[M + H]⁺. ¹H NMR (400 MHz, DMSO-*d*₆) *δ* 10.42 (s, 1H), 9.26 (s, 1H), 8.50 (s, 1H), 7.73 - 7.59 (m, 2H), 7.36 - 7.17 (m, 3H), 2.17 (s, 3H).

### Step 2

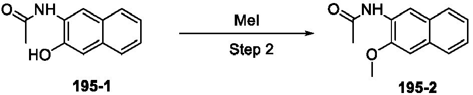

Compound **195-1** (8.3 g, 39.3 mmol, 1.0 eq), potassium carbonate (8.53 g, 58.9 mmol, 1.5 eq) and acetonitrile (166 mL) were successively added to a 500 mL reaction flask with stirring under the protection of nitrogen at 25 °C, then iodomethane (6.46 g, 43.21 mmol, 1.1 eq) was added dropwise at 25 °C. The obtained mixture was stirred at 80°C for 2 hours. The reaction process was monitored by liquid chromatography-mass spectrometry and thin layer chromatography. After the reaction was completed, the reaction mixture was cooled to room temperature, and concentrated under reduced pressure to remove the excess reagent to obtain a crude product. The crude product was purified by silica gel column chromatography, eluted with a gradient of 0%→ 60% ethyl acetate/petroleum ether mobile phase, and the obtained fractions were evaporated under reduced pressure to obtain compound **195-2** (brown solid, 7.3 g, yield: 82%). MS (ESI, *m*/*z):* 216.1[M + H]⁺; ¹H NMR (300 MHz, CDCl₃) *δ* 8.84 (s, 1H), 8.00 (s, 1H), 7.79 - 7.77 (m, 1H), 7.68 - 7.65 (m, 1H), 7.39 - 7.32 (m, 2H), 7.12 (s, 1H), 4.00 (s, 3H), 2.26 (s, 3H).

### Step 3

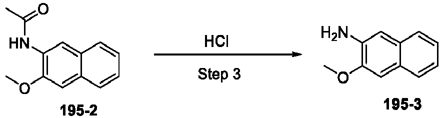

Compound **195-2** (7.3 g, 32.2 mmol, 1.0 eq), water (73 mL) and hydrochloric acid (73 mL) were added to a reaction flask at 25 °C with stirring under the protection of nitrogen. The reaction was carried out for 1 hour at 80 °C, and the reaction process was monitored by liquid chromatography-mass spectrometry and thin layer chromatography. After the reaction was completed, the reaction mixture was cooled to room temperature, and concentrated under reduced pressure to remove the excess reagent to obtain a crude product. The crude product was purified by silica gel column chromatography, eluted with a gradient of 0%→ 40% ethyl acetate/petroleum ether mobile phase, and the obtained fractions were evaporated under reduced pressure to remove the solvent to obtain compound **195-3** (white solid, 5.3 g, yield: 90%). MS (ESI, *m*/z): 174.1[M + H]⁺; ¹H NMR (300 MHz, CDCl₃) *δ* 7.70 - 7.64 (m, 1H), 7.60-7.58 (m, 1H), 7.33 - 7.22 (m, 2H), 7.08 (s, 1H), 7.05 (s, 1H), 4.01 (s, 3H).

### Step 4

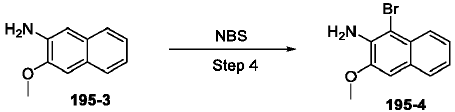

N-bromosuccinimide (6.0 g, 32.0 mmol, 1.1 eq) was added to a solution of compound **195-3** (5.3 g, 29.1 mmol, 1.0 eq) in *N, N*-dimethylformamide (53.0 mL) with stirring at 25 °C. The obtained mixture was stirred for 1 hour at 25 °C, and the reaction process was monitored by liquid chromatography-mass spectrometry and thin layer chromatography. After the reaction was completed, 500 mL of water was added to the reaction solution, the mixture was extracted with ethyl acetate (500 mL x 3). The organic phases were combined and washed with 500 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 20% ethyl acetate/petroleum ether mobile phase, and the obtained fractions were evaporated under reduced pressure to remove the solvent to obtain compound **195-4** (brown solid, 8.4 g, yield: 66%). MS (ESI, *m*/z): 252.0/254.0[M + H]⁺. ¹H NMR (400 MHz, CDCl₃) *δ* 7.97 - 7.95 (m, 1H), 7.623 - 7.61(m, 1H), 7.41 - 7.37 (m, 1H), 7.29 - 7.26 (m, 1H), 7.02 (s, 1H), 4.63 (s, 2H), 3.98 (s, 3H).

### Step 5

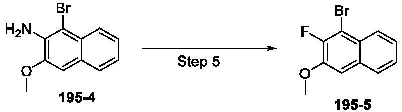

Compound **195-4** (2.0 g, 7.5 mmol, 1.0 eq), water (20 mL) and hydrochloric acid (20 mL) were added to a 100 mL round-bottom flask with stirring at 0 °C. Sodium nitrite (0.77 g, 10.55 mmol, 1.4 eq) was then added at 0 °C. The mixture was warmed to 25 °C and stirred at this temperature for 1 hour. The mixture was then cooled to 0 °C, sodium fluoroborate (0.96 g, 8.3 mmol, 1.1 eq) was added to the reaction solution, and the mixture was stirred at this temperature for 1 hour. After the reaction was completed, the reaction solution was returned to room temperature, filtered, and the filter cake was washed with saturated sodium fluoroborate aqueous solution (50 mL × 3), and the solid was collected and dried under reduced pressure to obtain an intermediate (orange-red solid, 2.3 g). The above solids were divided into 23 parts and dissolved in pyridine hydrofluorate (4 mL, 75%), and each solution was stirred for 2 hours at 20 °C with a 500 W high-pressure mercury lamp. All the reactions were completed and the reaction mixtures were combined, water (1000 mL) was added thereto, and the obtained mixture was extracted with ethyl acetate (1000 mL x 3). The organic phases were combined and washed with 1000 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 20% ethyl acetate/petroleum ether mobile phase, and the obtained fractions were evaporated under reduced pressure to obtain compound **195-5** (brown solid, 800 mg, yield: 40%). ¹H NMR (300 MHz, CDCl₃) *δ* 8.16 - 8.07 (m, 1H), 7.76 - 7.67 (m, 1H), 7.54 - 7.43 (m, 2H), 7.19 (d, *J* = 8.1 Hz, 1H), 4.01 (s, 3H); ¹⁹F NMR (282 MHz, CDCl₃) *δ*-122.12.

### Step 6

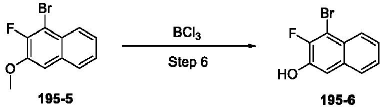

Compound **195-5** (500 mg, 1.96 mmol, 1.0 eq), tetrabutylammonium iodide (1.81 g, 4.66 mmol, 2.4 eq) and dichloromethane (10 mL) were successively added to a reaction flask with stirring under the protection of nitrogen at 25 °C, then boron trichloride (1 mol/L *n*-hexane solution, 4.7 mL, 2.4 eq) was added dropwise to the reaction solution at -78 °C. The reaction was carried out for 2 hours at 25 °C, and the reaction process was monitored by liquid chromatography-mass spectrometry and thin layer chromatography. After the reaction was completed, the reaction mixture was concentrated under reduced pressure to remove the excess reagent to obtain a crude product. The crude product was purified by silica gel column chromatography, elution with a gradient of 0%→ 50% ethyl acetate/petroleum ether mobile phase, the obtained fraction was evaporated under reduced pressure to remove the solvent to obtain compound **195-6** (white solid, 460 mg, yield: 97%). MS (ESI, *m*/z): 239.0/241.0[M-H]⁻; ¹H NMR (400 MHz, CDCl₃) *δ* 8.14 - 8.05 (m, 1H), 7.74 - 7.66 (m, 1H), 7.53 - 7.42 (m, 2H), 7.35 (d, *J=* 8.5 Hz, 1H); ¹⁹F NMR (377 MHz, CDCl₃) *δ*-127.0.

### Step 7

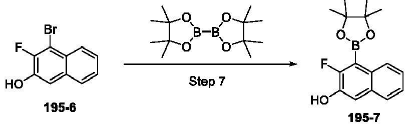

Potassium acetate (531 mg, 5.14 mmol, 4.0 eq) was added to a solution of compound **195-6** (326 mg, 1.29 mmol, 1.0 eq), bis(pinacolato)diboron (326 mg, 1.29 mmol, 1.0 eq) and [1,1'-bis(diphenylphosphine)ferrocene]palladium dichloromethane complex (98.95 mg, 0.13 mmol, 0.1 eq) in 1,4-dioxane (5 mL) with stirring under the protection of nitrogen at 25 °C. The reaction was carried out for 1.5 hours at 100 °C, and the reaction process was monitored by liquid chromatography-mass spectrometry and thin layer chromatography. After the reaction was completed, the reaction mixture was concentrated under reduced pressure to remove the excess reagent to obtain a crude product. The crude product was purified by silica gel column chromatography, elution with a gradient of 0%→ 25% ethyl acetate/petroleum ether mobile phase, the obtained fraction was evaporated under reduced pressure to remove the solvent to obtain compound **195-7** (white solid, 196 mg, yield: 50%). MS (ESI, m/z): 287.20[M + H]⁺.

### Step 8

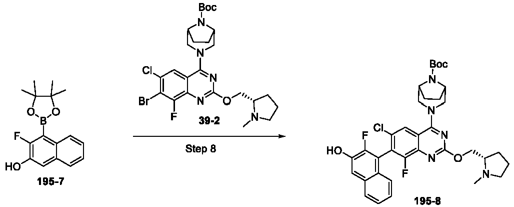

Potassium phosphate (88.79 mg, 1.29 mmol, 2.0 eq) was added to a mixed solution of compound **195-7** (196 mg, 0.65 mmol, 1.0 eq), **39-2** (397.9 mg, 0.65 mmol, 1.0 eq), methanesulfonato(2-dicyclohexylphosphino-2',6'-dimethoxybiphenyl)(2'-amino-1,1'-biphenyl-2-yl)palladium(II) (53.08 mg, 0.065 mmol, 0.1 eq) and 2-dicyclohexylphosphine-2',6'-dimethoxybiphenyl (27.93 mg, 0.065 mmol, 0.1 eq) in 1,4-dioxane (3 mL) and water (0.6 mL) with stirring under the protection of nitrogen at 25 °C. The reaction was carried out for 2 hours at 60 °C, and the reaction process was monitored by liquid chromatography-mass spectrometry and thin layer chromatography. After the reaction was completed, 50 mL of water was added to the reaction solution, the mixture was extracted with ethyl acetate (50 mL x 3). The organic phases were combined and washed with 50 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→12% methanol/dichloromethane mobile phase, and the obtained fractions were evaporated under reduced pressure to remove the solvent to obtain compound **195-8** (yellow solid, 193 mg, yield: 43%). MS (ESI, m/z): 666.3/668.3[M + H]⁺.

### Step 9

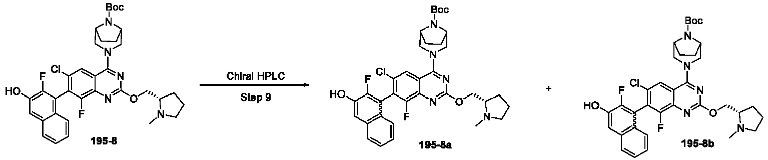

Compound **195-8** (193 mg) obtained in step 8 was subjected to chiral resolution by supercritical liquid chromatography: chiral column CHIRALPAK, 3 × 25 cm, 5 µm; mobile phase A: carbon dioxide, mobile phase B: isopropanol/dichloromethane (10/1, 0.5% ammonia methanol solution); flow rate: 60 mL/min; column temperature: 35 °C; eluted with 55% mobile phase B; detector UV 220 nm, two products were obtained. The product with shorter retention time (7.8 min) was compound **195-8a,** *tert*-butyl(1*R*,5*S*)-3-(6-chloro-8-fluoro-7-((*R* or *S*)-2-fluoro-3-hydroxynaphthalen-1-yl)-2-(((*S*)-1-methylpyrrolidin-2-yl)methoxy)quinazolin-4-yl)-3,8-diazabicyclo[3.2.1]octane-8-carboxylate (white solid, 67 mg, recovery rate: 35%), MS (ESI, m/z): 666.3/668.3[M + H]⁺; the product with longer retention time ( 15.52 min) was compound **195-8b,** *tert*-butyl(1*R*,5*S*)-3-(6-chloro-8-fluoro-7-((*S* or *R*)-2-fluoro-3-hydroxynaphthalen-1-yl)-2-(((*S*)-1-methylpyrrolidin-2-yl)methoxy)quinazolin-4-yl)-3,8-diazabicyclo[3.2.1]octane-8-carboxylate (white solid, 65 mg, recovery rate: 34%), MS (ESI, m/z): 666.3/668.3 [M + H]⁺.

### Step 10

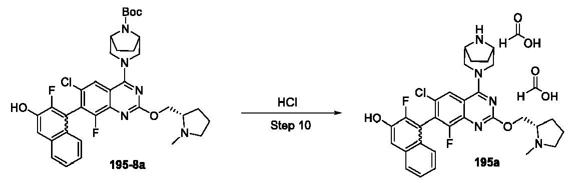

A solution of hydrochloric acid (4 mol/L, 2.0 mL) in 1,4-dioxane was added dropwise to a solution of the compound **195-8a** (67 mg, 0.096 mmol, 1.0 eq) in methanol (2.0 mL) with stirring at 25 °C. The reaction was carried out for 1 hour at 25 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 5%→ 95% acetonitrile/water mobile phase (0.1% formic acid) in 30 min; detector, UV254 nm; to obtain compound **195a** (white solid, 29.4 mg, yield: 46%). MS (ESI, m/z): 566.3/568.3[M + H]⁺; ¹H NMR (400 MHz, DMSO-*d*₆) *δ* 8.29 (s, 2H), 8.01 (s, 1H), 7.87 - 7.84 (m, 1H), 7.55-7.52 (m, 1H), 7.49-7.44 (m, 1H), 7.37-7.25 (m, 1H), 7.21-7.18 (m, 1H), 4.48-4.37 (m, 3H), 4.30-4.24 (m, 1H), 3.82 (s, 2H), 3.73-3.66 (m, 2H), 3.06-3.00(m, 1H), 2.82-2.73 (m, 1H), 2.43 (s, 3H), 2.35-2.27 (m, 1H), 2.03-1.94 (m, 1H), 1.81-1.60 m, 7H); ¹⁹F NMR (282 MHz, DMSO-*d*₆) *δ*-121.41, -131.44. The chiral analysis conditions of compound **195a** were: *N*--Lux 3µm Cellulose-4 (H18-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 3.734 min. dr > 40:1.

### Step 10:

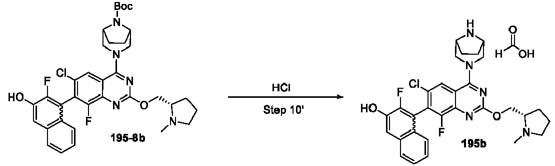

A solution of hydrochloric acid (4 mol/L, 2.0 mL) in 1,4-dioxane was added dropwise to a solution of the compound **195-8b** (65 mg, 0.093 mmol, 1.0 eq) in methanol (2.0 mL) with stirring at 25 °C. The reaction was carried out for 1 hour at 25 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 5%→ 95% acetonitrile/water mobile phase (0.1% formic acid) in 30 min; detector, UV254 nm; to obtain compound **195b** (white solid, 23.6 mg, yield: 40%). MS (ESI, *m*/z): 566.3/568.3[M + H]⁺. ¹H NMR (400 MHz, DMSO-*d*₆) *δ* 8.25 (s, 1H), 8.01 (s, 1H), 7.87 - 7.84 (m, 1H), 7.53 - 7.43 (m, 2H), 7.32 - 7.28 (m, 1H), 7.20 - 7.17 (m, 1H), 4.46 - 4.31 (m, 3H), 4.21 - 4.35 (m, 1H), 3.70 - 3.57 (m, 4H), 3.02 - 2.93 (m, 1H), 2.63 (s, 1H), 2.38 (s, 3H), 2.28 - 2.16 (m, 1H), 2.01 - 1.87 (m, 1H), 1.74 - 1.60 (m, 7H); ¹⁹F NMR (282 MHz, DMSO-d₆) *δ*-121.47, -131.29. The chiral analysis conditions of compound **195b** were: N -- Lux 3 Cellulose-4 (H18-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 2.913 min. dr > 40:1.

### Embodiment 49

### 3-((7R or 7S)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)-5-chloro-4-cyclopropylphenol formate 197a; 3-((7S or 7R)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)-5-chloro-4-cyclopropylphenol formate 197b.

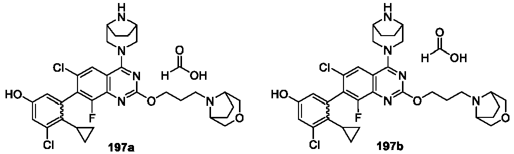

The synthetic route was as follows:

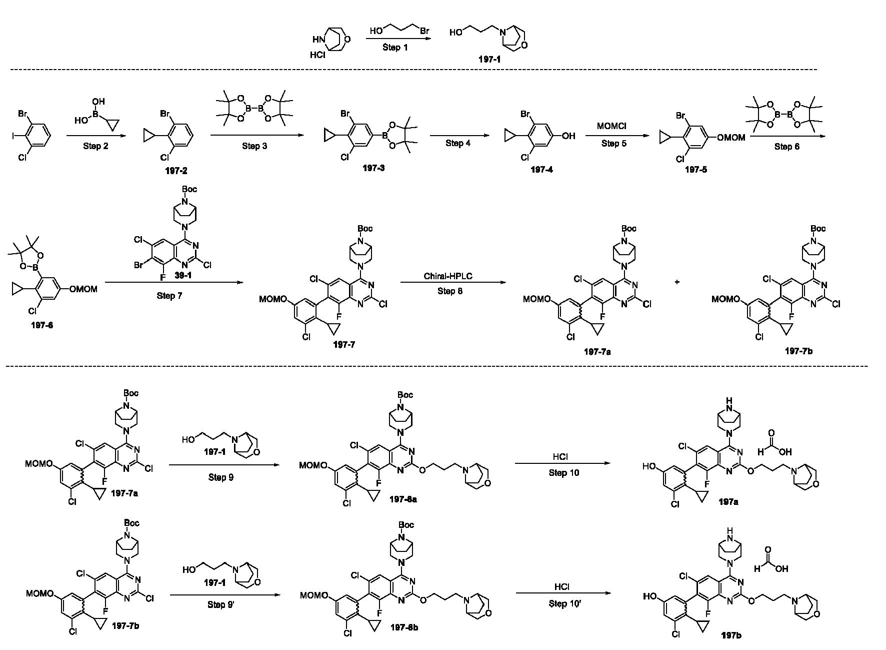

### Step 1

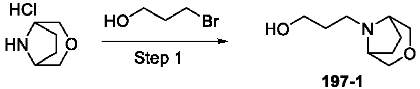

3-Bromo-1-propanol (2.95 g, 20.14 mmol, 0.8 eq) and potassium carbonate (14.7 g, 100.74 mmol, 4 eq) were added to a solution of compound 3-oxa-8-azabicyclo[3.2.1]octane hydrochloride (3 g, 25.18 mmol, 1.0 eq) in acetonitrile (50 mL) with stirring at 25 °C. The reaction was carried out for 16 hours at 50 °C, and the reaction process was monitored by thin layer chromatography. After the reaction was completed, the reaction solution was cooled to room temperature, the insolubles were removed by filtration, and the filter cake was washed with dichloromethane (50 mL x 5). The filtrate was concentrated under reduced pressure to obtain a crude product. The crude product was purified by silica gel column chromatography, eluted with a gradient of 0%→7% ammonia-methanol (7mol/L)/dichloromethane mobile phase, and the obtained fractions were evaporated under reduced pressure to remove the solvent to obtain compound **197-1** (light yellow oil, 1.4 g, yield: 31%). MS (ESI, *m*/z): 172.2[M + H]⁺; ¹H NMR (400 MHz, CDCl₃) *δ* 3.92 - 3.84 (m, 2H), 3.75 - 3.65 (m, 2H), 3.61 - 3.51 (m, 2H), 3.24 - 3.16 (m, 2H), 2.66 - 2.56 (m, 2H), 2.01 - 1.85 (m, 4H), 1.78 - 1.64 (m, 2H).

### Step 2

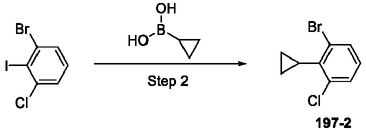

Cyclopropylboronic acid (3.5 g, 38.9 mmol, 1.3 eq), potassium phosphate (24 g, 107.7 mmol, 3.6 eq) and [1,1'-bis(diphenylphosphino)ferrocene]palladium dichloromethane complex (1.3 g, 15 mmol, 0.05 eq) were added to a solution of 1-bromo-2-iodo-3-chlorobenzene (10 g, 29.98 mmol, 1.0 eq) in 1,4-dioxane/water (3/1, 24 mL) with stirring under the protection of nitrogen at 25 °C. The reaction was carried out for 2 hours at 100 °C, and the reaction process was monitored by thin layer chromatography. After the reaction was completed, the reaction solution was cooled to room temperature, and the mixture was extracted with ethyl acetate (40 mL x 3). The organic phases were combined, washed with 20 mL of saturated brine, dried over anhydrous sodium sulfate, filtered, and concentrated under reduced pressure to obtain a crude product. The crude product was purified by silica gel column chromatography, eluted with a gradient of 0%→ 5% ethyl acetate/petroleum ether mobile phase, and the obtained fractions were evaporated under reduced pressure to remove the solvent to obtain compound **197-2** (colorless oil, 5.9 g, yield: 85%). ¹H NMR (400 MHz, CDCl₃) *δ* 7.49 - 7.42 (m, 1H), 7.34 - 7.27 (m, 1H), 7.02 - 6.95 (m, 1H), 1.81 - 1.72 (m, 1H), 1.22 -1.14 (m, 2H), 0.82 - 0.73 (m, 2H).

### Step 3

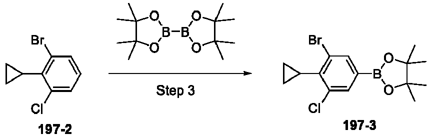

Methoxy(cyclooctadiene)iridium dimer (758 mg, 1.08 mmol, 0.05 eq) and 4,4'-di-tert-butyl-2,2'-dipyridine (368 mg, 1.30 mmol, 0.06 eq) was added to a solution of bis(pinacolato)diboron (17.4 g, 65.24 mmol, 3.0 eq) and compound **197-2** (5.3 g, 21.7 mmol, 1.0 eq) in *n*-hexane (40 mL) with stirring under the protection of nitrogen at 25°C. The reaction was carried out for 2 hours at 60 °C, and the reaction process was monitored by thin layer chromatography. After the reaction was completed, the reaction solution was concentrated under reduced pressure to remove the solvent to obtain compound **197-3** (21.3 g, crude product). The crude product was used directly in the next reaction without further purification.

### Step 4

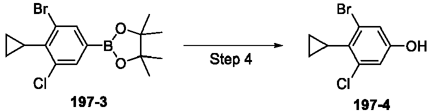

Acetic acid (86.3 mL, 1491.6 mmol, 71.5 eq) and hydrogen peroxide solution (30% aqueous solution, 43.2 mL, 556.0 mmol, 26.7 eq) were added to a mixed solution of compound **197-3** (21.3 g, 20.85 mmol, 1.0 eq) in tetrahydrofuran/water (2/1, 120 mL) with stirring at 0 °C. The reaction was carried out for 1 hour at 0 °C, and the reaction process was monitored by liquid chromatography-mass spectrometry and thin layer chromatography. After the reaction was completed, the reaction solution was extracted with ethyl acetate (120 mL x 3). The organic phases were combined, washed with 100 mL of saturated brine, dried over anhydrous sodium sulfate, filtered, and concentrated under reduced pressure to obtain a crude product. The obtained crude product was purified by reversed-phase rapid chromatographic column (C18 column), and eluted with 5%→ 95% acetonitrile/water mobile phase (0.1% formic acid) in 30 min; detector, UV254/220 nm; to obtain compound **197-4** (white solid, 4.6 g, two-step yield: 82%). MS (ESI, *m*/z): 245.1/247.1/249.1[M-H]⁻; ¹H NMR (400 MHz, CDCl₃) *δ* 7.03 - 6.96 (m, 1H), 6.87 - 6.81 (m, 1H), 1.70 - 1.58 (m, 1H), 1.15 -1.10 (m, 2H), 0.74 - 0.66 (m, 2H).

### Step 5

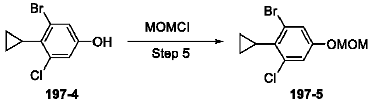

Chloromethyl methyl ether (1.1 g, 16.16 mmol, 2 eq) was added to a solution of compound **197-4** (2 g, 8.08 mmol, 1.0 eq) and A, A-diisopropylethylamine (4.2 mL, 24.24 mmol, 3.0 eq) in dichloromethane (20 mL) with stirring at 0 °C. The reaction was carried out for 1 hour at 20 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 0%→ 12% ethyl acetate/petroleum ether mobile phase, and the obtained fractions were evaporated under reduced pressure to remove the solvent to obtain compound **197-5** (colorless oil, 1.9 g, yield: 81%). ¹H NMR (400 MHz, CDCl₃) *δ* 7.19 (d, *J* = 2.6 Hz, 1H), 7.03 (d, *J* = 2.6 Hz, 1H), 5.11 (s, 2H), 3.46 (s, 3H), 1.76 - 1.66 (m, 1H), 1.19 - 1.11 (m, 2H), 0.78 - 0.67 (m, 2H).

### Step 6

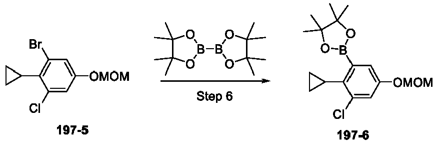

[1,1'-Bis (diphenylphosphino)ferrocene]palladium dichloromethane complex (279 mg, 0.32 mmol, 0.1 eq) and potassium acetate (1 g, 9.8 mmol, 3 eq) were added to a solution of bis(pinacolato)diboron (1.7 g, 6.51 mmol, 2.0 eq) and compound **197-5** (1 g, 3.25 mmol, 1.0 eq) in 1,4-dioxane (10 mL) with stirring under the protection of nitrogen at 25 °C. The reaction was carried out for 4 hours at 100 °C, and the reaction process was monitored by thin layer chromatography. After the reaction was completed, the mixture was filtered to remove insolubles, and the filter cake was washed with 1,4-dioxane (8 mL x 3). The filtrate was concentrated under reduced pressure to obtain a crude product. The crude product was purified by silica gel column chromatography, eluted with a gradient of 0%→ 13% tert-butyl methyl ether/petroleum ether mobile phase, and the obtained fractions were evaporated under reduced pressure to remove the solvent to obtain compound **197-6** (colorless oil, 846 mg, yield: 76%). ¹H NMR (300 MHz, CDCl₃) *δ* 7.13 -7.10 (m, 2H), 5.15 (s, 2H), 3.47 (s, 3H), 2.04 - 1.95 (m, 1H), 1.39 (s, 12H), 1.04 - 0.92 (m, 2H), 0.60 - 0.47 (m, 2H).

### Step 7

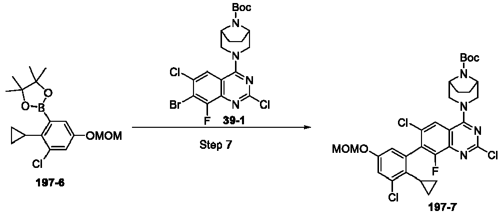

3-(*tert*-Butyl)-4- (2,6-dimethoxyphenyl) -2,3-dihydrobenzo [*d*][1,3]oxaphosphole (65 mg, 0.20 mmol, 0.1 eq), tris(dibenzylideneacetone)dipalladium (180 mg, 0.20 mmol, 0.1 eq) and potassium phosphate (838 mg, 3.95 mmol, 2.0 eq) were added to a mixed solution of compound **39-1** (1 g, 1.97 mmol, 1.0 eq) and **197-6** (735 mg, 2.17 mmol, 1.1 eq) in toluene/water (5/1, 12 mL) with stirring under the protection of nitrogen at 25 °C. The reaction was carried out for 5 hours at 80 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 0%→ 20% ethyl acetate/dichloromethane mobile phase, the obtained fraction was evaporated under reduced pressure to remove the solvent to obtain compound **197-7** (white solid, 667 mg, yield: 52%), MS (ESI, m/z): 637.2/639.2/641.2[M + H]⁺; ¹H NMR (400 MHz, CDCl₃) *δ* 8.16 - 8.12 (m, 1H), 7.60 - 7.56 (m, 1H), 7.16 - 7.13 (m, 1H), 5.53 (s, 2H), 5.02 - 4.88 (m, 1H), 4.84 - 4.67 (m, 3H), 4.20 - 3.93 (m, 2H), 3.85 (s, 3H), 2.42 - 2.31 (m, 2H), 2.23 - 2.08 (m, 3H), 1.90 (s, 9H), 1.07 - 0.86 (m, 2H), 0.75 - 0.53 (m, 2H).

### Step 8

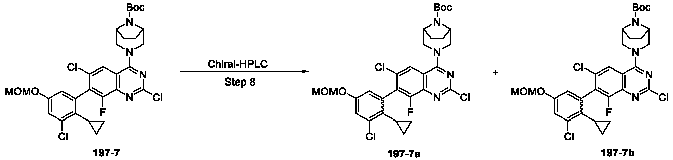

The compound **197-7** (667 mg) obtained in step 7 was subjected to chiral resolution by preparative chiral high performance liquid chromatography: chiral column CHIRAL ART Cellulose-SC, 2 x 25 cm, 5 µm; mobile phase A: *n*-hexane (10 mol/L ammonia methanol solution), mobile phase B: ethanol; flow rate: 20 mL/min; eluted with 10% phase B in 15 min; detector UV 225/278 nm. Two products were obtained, the product with shorter retention time (10.8 min) product was compound **197-7a,** *tert*-butyl (1*R*,5*S*)-3-((*R* or *S*)-2,6-dichloro-7-(3-chloro-2-cyclopropyl-5-(methoxymethoxy)phenyl)-8-fluoroquinazolin-4-yl)-3,8-diazabicyclo[3.2.1]octane-8-carboxylate (white solid, 100 mg, recovery rate: 15%), MS (ESI, m/z): 637.2/639.2/641.2[M + H]⁺; the product with longer retention time (12.8 min) was compound **197-7b,** *tert*-butyl (1*R*,5*S*)-3-((*S* or *R*)-2,6-dichloro-7-(3-chloro-2-cyclopropyl-5-(methoxymethoxy)phenyl)-8-fluoroquinazolin-4-yl)-3,8-diazabicyclo[3.2.1]octane-8-carboxylate (white solid, 120 mg, recovery rate: 18%), MS (ESI, m/z): 637.2/639.2/641.2[M + H]+.

### Step 9

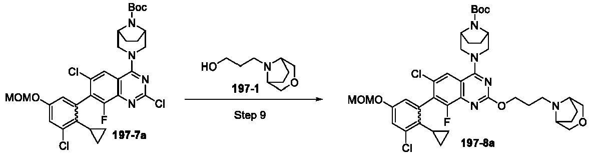

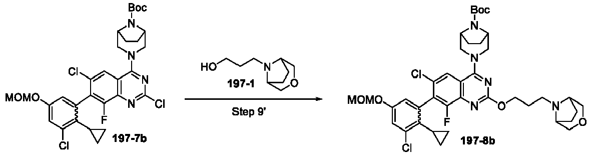

Potassium tert-butoxide (1 mol/L of tetrahydrofuran solution, 0.116 mL, 0.116 mmol, 1.3 eq) was added dropwise to a solution of **197-7a** (60 mg, 0.09 mmol, 1.0 eq) and **197-1** (19 mg, 0.11 mmol, 1.2 eq) in anhydrous tetrahydrofuran (2 mL) with stirring under the protection of nitrogen at 0 °C. After the dropwise addition, the reaction was carried out at 0 °C under the protection of nitrogen for 1 hour, and the reaction process was monitored by liquid chromatography-mass spectrometry and thin layer chromatography. After the reaction was completed, 10 mL of water was added to the reaction solution, the mixture was extracted with ethyl acetate (10 mL × 3), and the organic phases were combined, dried over anhydrous sodium sulfate, filtered, and the filtrate was concentrated to obtain a crude product. The crude product was purified by preparative thin layer chromatography using methanol/dichloromethane (1/12) as elution solvent to obtain compound **197-8a** (white solid, 38 mg, yield: 52%). MS (ESI, m/z): 772.2/774.2/776.2[M + H]⁺; compound **197-8b** (white solid, 45 mg, yield: 61%) can be obtained by the same method as above, MS (ESI, m/z): 772.2/774.2/776.2[M + H]⁺.

### Step 10

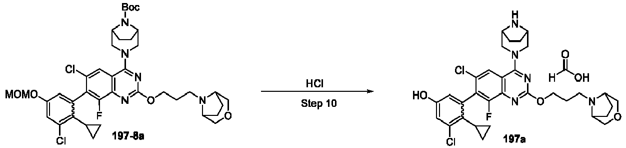

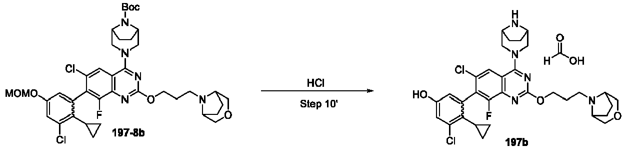

A solution of hydrogen chloride (4 mol/L, 2 mL) in methanol was added dropwise to a solution of compound **197-8a** (38 mg, 0.047 mmol, 1.0 eq) in methanol (2 mL) with stirring at 0 °C. The reaction was carried out for 2 hours at 25 °C with stirring, and the reaction process was monitored by liquid chromatography-mass spectrometry and thin layer chromatography. After the reaction was completed, the reaction solution was concentrated under reduced pressure to remove the solvent to obtain a crude product. The obtained crude product was purified by reversed-phase rapid chromatographic column (C18 column), and eluted with 5%→ 50% acetonitrile/methanol (1/1)/water mobile phase (0.1% formic acid) in 20 min; detector, UV254/220 nm; to obtain compound **197a** (white solid, 20 mg, yield: 63%). Compound **197b** (white solid, 23 mg, yield: 61%) can be obtained by the same method as above.

Compound **197a:** MS (ESI, m/z): 628.3/630.3 [M + H]⁺; ¹H NMR (400 MHz, DMSO-*d₆) δ* 8.27 (s, 1H), 7.92 - 7.85 (m, 1H), 6.96 (d, *J* = 2.5 Hz, 1H), 6.57 (d, *J* = 2.6 Hz, 1H), 4.44 - 4.34 (m, 3H), 4.29 - 4.23 (m, 1H), 3.73 - 3.59 (m, 4H), 3.58 - 3.53 (m, 2H), 3.43 - 3.38 (m, 2H), 3.10 - 3.05 (m, 2H), 2.42 - 2.35 (m, 2H), 1.91 - 1.81 (m, 4H), 1.76 - 1.62 (m, 7H), 0.64 - 0.49 (m, 2H), 0.22 - 0.09 (m, 2H); ¹⁹F NMR (377 MHz, DMSO-*d*₆) *δ*-122.64. The chiral analysis conditions of compound **197a** were: CHIRAL ART Cellulose-SB, 4.6 x 100 mm, 3 µm; mobile phase A: *n*-hexane (0.1% diethylamine), mobile phase B: ethanol; flow rate: 1 mL/min; isocratic elution with 10% phase B in 4.5 min; detector UV 220 nm; retention time: 1.346 min. ee > 99%.

Compound **197b:** MS (ESI, m/z): 628.3/630.3 [M + H]⁺; ¹H NMR (400 MHz, DMSO-*d₆*) *δ* 8.27 (s, 1H), 7.88 (s, 1H), 6.96 (d, *J* = 2.6 Hz, 1H), 6.57 (d, *J* = 2.6 Hz, 1H), 4.46 - 4.32 (m, 3H), 4.25 (d, *J* = 12.0 Hz, 1H), 3.74 - 3.58 (m, 4H), 3.56 - 3.52 (m, 2H), 3.43 - 3.37 (m, 2H), 3.14 - 3.02 (m, 2H), 2.44 - 2.33 (m, 2H), 1.90 - 1.79 (m, 4H), 1.79 - 1.61 (m, 7H), 0.64 - 0.49 (m, 2H), 0.14 (d, *J=* 5.4 Hz, 2H); ¹⁹F NMR (377 MHz, DMSO-*d*₆) *δ-*122.65. The chiral analysis conditions of compound **197b** were: CHIRALART Cellulose-SB, 4.6 x 100 mm, 3 µm; mobile phase A: n-hexane (0.1% diethylamine), mobile phase B: ethanol; flow rate: 1 mL/min; isocratic elution with 10% phase B in 4.5 min; detector UV 220 nm; retention time: 2.438 min. ee > 97%.

Other similar compound of the present disclosure can be prepared by the synthetic method shown in Embodiment **49** above. Some compounds prepared with reference to the above synthetic method and their characterization data are shown in table 9.

**Table 9**

| **Nu mbe r of the com pou nd** | Compound structure | Compound name | Chiral analysis conditions/retent ion time/dr value/specific rotation | Mas s spec trum [M+ H]+ | ¹H & 19F NMR |
|---|---|---|---|---|---|
| **198a** | 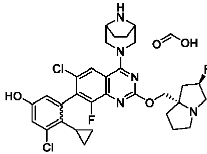 | 3-((*R* or *S*)-4-((1*R*,5*S*)-3,8-diazabicycl o[3.2.1]octa n-3-yl)-6-chloro-8-fluoro-2-((2*R*,7a*S*)-2-fluorotetrah ydro-1*H-*pyrrolin-7a(5*H*)-yl)methoxy) quinazolin-7-yl)-5-chloro-4-cyclopropyl phenol formate | CHIRALPAK IC-3, 4.6 x 50 mm, 3 µm; mobile phase A: n-hexane (0.1% diethylamine), mobile phase B: ethnaol; flow rate: 1 mL/min; isocratic elution with 20% phase B in 3.5 min; detector UV 220/254 nm; retention time: 1.198 min; *dr*> 40: 1. | 616. 2/61 8.2 | ¹H NMR (300 MHz, DMSO-*d*₆) *δ* 8.21 (s, 1H), 7.88 (d, *J* = 1.6 Hz, 1H), 6.95 (d, *J =* 2.6 Hz, 1H), 6.57 (d, *J* = 2.5 Hz, 1H), 5.42 - 5.17 (m, 1H), 4.39 - 4.32 (m, 1H), 4.29 - 4.20 (m, 1H), 4.13 - 4.05 (m, 1H), 4.03 - 3.95 (m, 1H), 3.68 - 3.57 (m, 3H), 3.54 - 3.46 (m, 2H), 3.13 - 3.05 (m, 2H), 3.04 - 2.99 (m, 1H), 2.90 - 2.80 (m, 1H), 2.20 - 2.11 (m, 1H), 2.09 - 1.97 (m, 2H), 1.94 - 1.52 (m, 8H), 0.64 - 0.48 (m, 2H), 0.21 - 0.11 (m, 2H). ¹⁹F NMR (282 MHz, DMSO-*d*₆) *δ*-122.57, - 172.13. |
| **198 b** | 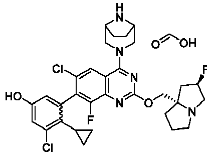 | 3-((*S* or R)-4-((1*R*,5*S*)-3,8-diazabicycl o[3.2.1]octa n-3-yl)-6-chloro-8-fluoro-2-((2*R*,7a*S*)-2-fluorotetrah ydro-1*H-*pyrrolin-7a(5*H*)-yl)methoxy) quinazolin-7-yl)-5-chloro-4-cyclopropyl phenol formate | CHIRALPAK IC-3, 4.6 x 50 mm, 3 µm; mobile phase A: *n*-hexane (0.1% diethylamine), mobile phase B: ethnaol; flow rate: 1 mL/min; isocratic elution with 20% phase Bin 4 min; detector UV 220/254 nm; retention time: 1.880 min;= 4: 1. | 616. 2/61 8.2 | ¹H NMR (300 MHz, DMSO-d₆) *δ* 8.21 (s, 1H), 7.88 (d, *J =* 1.6 Hz, 1H), 6.95 (d, *J =* 2.5 Hz, 1H), 6.57 (d, *J* = 2.5 Hz, 1H), 5.41 - 5.17 (m, 1H), 4.41 - 4.31 (m, 1H), 4.30 - 4.20 (m, 1H), 4.13 - 4.05 (m, 1H), 4.05 - 3.96 (m, 1H), 3.65 - 3.56 (m, 3H), 3.55 - 3.46 (m, 2H), 3.13 - 3.06 (m, 2H), 3.02 - 2.98 (m, 1H), 2.90 - 2.77 (m, 1H), 2.18 - 2.12 (m, 1H), 2.08 - 1.95 (m, 2H), 1.89 - 1.58 (m, 8H), 0.66 - 0.47 (m, 2H), 0.22 - 0.12 (m, 2H). ¹⁹F NMR (282 MHz, DMSO-d₆) *δ*-122.57, - 172.14. |
| **227** | 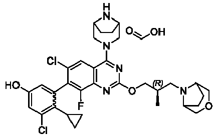 | 3-((7*S* or 7*R*)-2-((2*R*)-3-(3-oxa-8-azabicyclo[ 3.2.1]octan-8-yl)-2-methylprop oxy)-4-((1*R*,5*S*)-3,8-diazabicycl o[3.2.1]octa n-3-yl)-6-chloro-8-fluoroquina zolin-7-yl)-5-chloro-4-cyclopropyl phenol formate | | 642. 2/64 4.2 | ¹H NMR (300 MHz, DMSO-*d*₆) *δ* 8.25 (s, 1H), 7.87 (d, *J =* 1.7 Hz, 1H), 6.95 (d, *J =* 2.5 Hz, 1H), 6.56 (d, *J* = 2.5 Hz, 1H), 4.50 - 4.45 (m, 1H), 4.35 (d, *J* = 12.4 Hz, 1H), 4.25 - 4.13 (m, 2H), 3.61 - 3.58 (m, 3H), 3.52 - 3.46 (m, 3H), 3.40 - 3.35 (m, 2H), 3.05 - 2.95 (m, 2H), 2.29 - 2.14 (m, 2H), 2.11 - 2.02 (m, 1H), 1.84 - 1.58 (m, 9H), 1.00 (d, *J* = 6.6 Hz, 3H), 0.61 - 0.48 (m, 2H), 0.19 - 0.10 (m, 2H); ¹⁹F NMR (282 MHz, DMSO-*d*₆) *δ*-122.66. |

### Embodiment 50

### N-(4-((7S or 7R)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)naphthalen-2-yl)methanesulfonamide tristrifluoroacetate 211

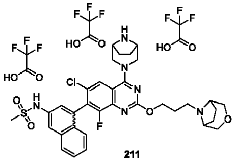

The synthetic route was as follows:

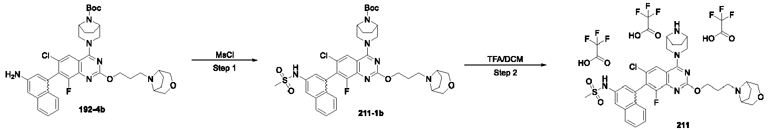

### Step 1

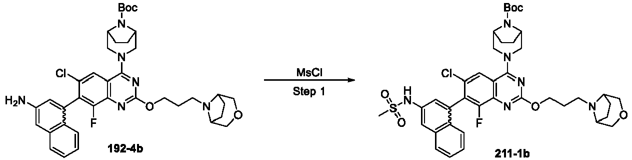

Methylsulfonyl chloride (8 mg, 0.07 mmol, 1.3 eq) was added to a solution of compound **192-4b** (40 mg, 0.05 mmol, 1.0 eq) and pyridine (6 mg, 0.08 mmol, 1.5 eq) in dichloromethane (2 mL) with stirring under the protection of nitrogen at 0 °C. The obtained reaction was carried out for 6 hours at 0 °C, and the reaction process was monitored by liquid chromatography-mass spectrometry and thin layer chromatography. After the reaction was completed, the reaction mixture was concentrated to obtain a crude product. The crude product was purified by silica gel column chromatography, eluted with a gradient of 0%→ 10% methanol/dichloromethane mobile phase, and the obtained fractions were evaporated under reduced pressure to remove the solvent to obtain compound **211-1b** (white solid, 40 mg, yield: 90%). MS (ESI, m/z): 781.4/783.3[M + H]⁺.

### Step 2

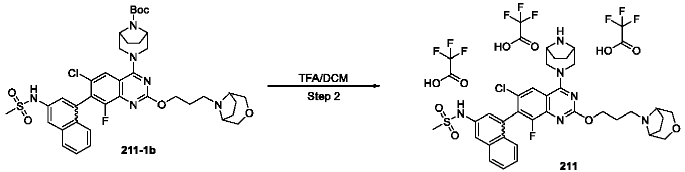

Trifluoroacetic acid (0.5 mL) was added dropwise to a solution of compound **211-1b** (40 mg, 0.05 mmol, 1.0 eq) in dichloromethane (2 mL) at 0 °C, and the reaction solution was stirred at 0 °C for 2 hours, the reaction process was monitored by liquid chromatography-mass spectrometry and thin layer chromatography. After the reaction was completed, the reaction solution was concentrated under reduced pressure to obtain a crude product. The obtained crude product was purified by reversed-phase rapid chromatographic column (C18 column), and eluted with 20%→ 50% acetonitrile/water mobile phase (5 mmol/L trifluoroacetic acid) in 20 min; detector, UV254/220 nm; to obtain compound **221** (yellow solid, 25 mg, yield: 49%). MS (ESI, m/z): 681.3/683.3[M + H]⁺; ¹H NMR (300 MHz, DMSO-*d*₆) *δ* 8.14 - 7.98 (m, 2H), 7.95 - 7.84 (m, 1H), 7.71 - 7.59 (m, 1H), 7.56 - 7.28 (m, 3H), 4.78 - 4.40 (m, 4H), 4.29 - 4.19 (m, 2H), 4.10 - 4.02 (m, 2H), 3.88 - 3.70 (m, 6H), 3.24 - 3.10 (m, 5H), 2.29 - 1.98 (m, 10H); ¹⁹F NMR (282 MHz, DMSO-*d*₆) *δ*-74.05, -121.95.

### Embodiment 51

### 8-(3-((S or R)-4-((1R,5S)-3,8-diazabicyclo[3.2.1]octan-3-yl)-6-chloro-8-fluoro-7-(3-hydroxynaphthalen-1-yl)quinazolin-2-yl)oxy)propyl)-3-thio-8-azabicyclo[3.2.1]octane-3,3-dioxide formate 212

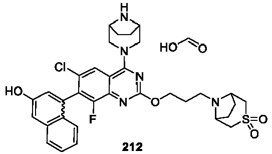

The synthetic route was as follows:

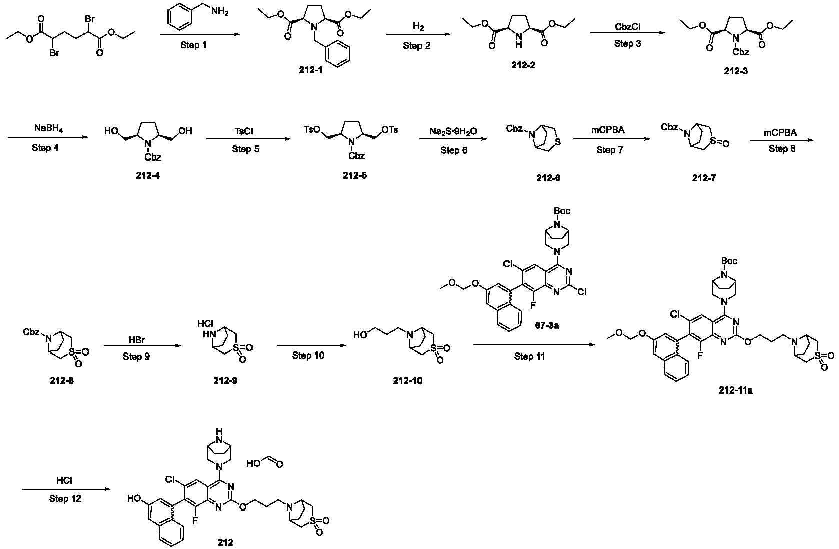

### Step 1

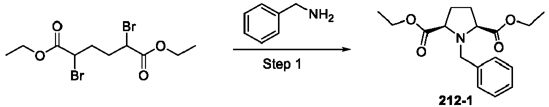

Benzylamine (178.57 g, 1583.148 mmol, 3 eq) was added to a solution of compound diethyl 2,5-dibromohexanedioate (200 g, 527.716 mmol, 1.0 eq) in toluene (2 L) with stirring under the protection of nitrogen at 25 °C. The reaction was carried out for 16 hours at 100 °C with stirring, and the reaction process was monitored by liquid chromatography-mass spectrometry and thin layer chromatography. After the reaction was completed, the mixture was cooled to room temperature, the insolubles were removed by filtration, and the filtrate was concentrated under reduced pressure to obtain a crude product. The crude product was purified by silica gel column chromatography, eluted with a gradient of 0%→ 20% ethyl acetate/petroleum ether mobile phase, and the obtained fractions were evaporated under reduced pressure to remove the solvent to obtain compound **212-1** (yellow oil, 101.1 g, yield: 59%). MS (ESI, m/z): 306.1[M + H]⁺.

### Step 2

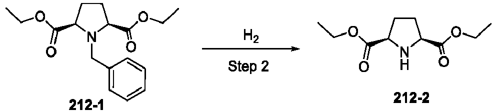

Palladium hydroxide /carbon (6 g, 10%) was added to a solution of compound **212-1** (60 g, 186.6 mmol, 1.0 eq) in methanol (500.0 mL) with stirring under the protection of nitrogen at 25 °C. The nitrogen gas was ventilated with hydrogen gas (1.5 atmospheric pressures) by a displacement gas operation. The mixture was stirred for 16 hours at 50 °C under hydrogen atmosphere, and the reaction process was monitored by liquid chromatography-mass spectrometry and thin layer chromatography. After the reaction was completed, the reaction solution was cooled to room temperature, filtered to remove the insolubles, and the filter cake was washed with methanol (100 mL x 3), and the filtrate was concentrated under reduced pressure to obtain a crude product. The crude product was purified by silica gel column chromatography, eluted with a gradient of 0%→5% methanol/dichloromethane mobile phase, and the obtained fractions were evaporated under reduced pressure to remove the solvent to obtain compound **212-2** (yellow oil, 36 g, yield: 89%). MS (ESI, m/z): 216.0[M + H]⁺; ¹H NMR (400 MHz, CDCl₃) *δ* 4.21 (q, *J* = 7.1 Hz, 4H), 3.88 - 3.80 (m, 2H), 2.22 - 2.12 (m, 2H), 1.99 - 1.89 (m, 2H), 1.28 (t, *J=* 7.1 Hz, 6H).

### Step 3

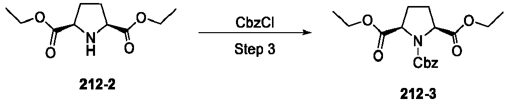

Benzyl chloroformate (57.06 g, 317.772 mmol, 2 eq) was slowly added dropwise to a solution of compound **212-2** (36 g, 158.8 mmol, 1.0 eq) and triethylamine (33.85 mg, 317.7 mmol, 2 eq) in dichloromethane (400.0 mL) with stirring under the protection of nitrogen at - 20 °C. The reaction was carried out with stirring for 3 hours at -20 °C, and the reaction process was monitored by liquid chromatography-mass spectrometry and thin layer chromatography. After the reaction was completed, 400 mL of water was added to the reaction solution to quench the reaction, and then the mixture was extracted with dichloromethane (400 mL x 3), the organic phases were combined, and washed with saturated brine (400 mL x 3), dried over sodium sulfate, filtered, and the filtrate was concentrated to obtain a crude product. The crude product was purified by silica gel column chromatography, eluted with a gradient of 0%→ 80% tert-butyl methyl ether/petroleum ether mobile phase, and the obtained fractions were evaporated under reduced pressure to remove the solvent to obtain compound **212-3** (white oil, 46.7 g, yield: 82%). MS (ESI, m/z): 350.0[M + H]⁺; ¹H NMR (400 MHz, CDCl₃) *δ* 7.37 - 7.29 (m, 5H), 5.21 - 5.09 (m, 2H), 4.50 - 4.45 (m, 1H), 4.44 - 4.37 (m, 1H), 4.26 - 4.19 (m, 2H), 4.15 - 4.03 (m, 2H), 2.29 - 2.10 (m, 4H), 1.33 - 1.23 (m, 3H), 1.23 - 1.12 (m, 3H).

### Step 4

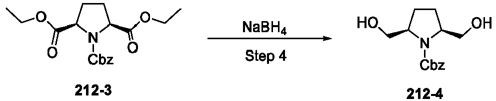

Calcium chloride (43.84 g, 375.234 mmol, 3.00 eq) and sodium borohydride (24.91 g, 625.39 mmol, 5.00 eq) were added in batches to a mixed solution of compound **212-3** (46 g, 125.078 mmol, 1.00 eq) in methanol (300 mL) and ethanol (300 mL) with stirring under the protection of nitrogen at 0 °C. The mixture was stirred at 20°C for 4 hours. The reaction process was monitored by liquid chromatography-mass spectrometry and thin layer chromatography. After the reaction was completed, the solvent was concentrated under reduced pressure to remove the solvent, the mixture was diluted with water (300 mL), and extracted with ethyl acetate (400 mL x 3), the organic phases were combined, dried over anhydrous sodium sulfate, filtered, and the filtrate was concentrated to obtain a crude product. The crude product was purified by silica gel column chromatography, eluted with a gradient of 0%→ 40% *tert*-butyl methyl ether/petroleum ether mobile phase, and the obtained fractions were evaporated under reduced pressure to remove the solvent to obtain compound **212-4** (colorless oil, 33.3 g, yield: 95%). MS (ESI, m/z): 266.0[M + H]⁺; ¹H NMR (300 MHz, CDCl₃) *δ* 7.41 - 7.32 (m, 5H), 5.17 (s, 2H), 4.11 - 3.78 (m, 4H), 3.63 - 3.53 (m, 2H), 3.12 - 3.06 (m, 2H), 2.05 - 1.87 (m, 4H).

### Step 5

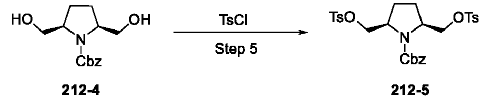

Methyl sulfonyl chloride (33.49 g, 166.862 mmol, 2 eq) was added to a solution of compound **212-4** (23.3 g, 83.431 mmol, 1.0 eq), triethylamine (19.55 g, 183.548 mmol, 2.2 eq) and 4-dimethylaminopyridine (1.01 g, 8.3 mmol, 0.1 eq) in dichloromethane (500 mL) with stirring under the protection of nitrogen at 0 °C. The reaction was carried out for 4 hours at 20 °C, and the reaction process was monitored by liquid chromatography-mass spectrometry and thin layer chromatography. After the reaction was completed, 400 mL of water was added to the reaction solution to quench the reaction, extracted with dichloromethane (400 mL x 3), and the organic phases were combined, washed with 300 mL of saturated brine, and the washed organic phase was dried over anhydrous sodium sulfate and filtered to remove the drying agent; the filtrate was concentrated under reduced pressure to obtain a crude product. The crude product was purified by silica gel column chromatography, eluted with a gradient of 0%→ 40% ethyl acetate/petroleum ether mobile phase, and the obtained fractions were evaporated under reduced pressure to remove the solvent to obtain compound **212-5** (colorless oil, 46.5 g, yield: 92%). MS (ESI, m/z): 574.1[M + H]⁺; ¹H NMR (400 MHz, CDCl₃) *δ* 7.79 - 7.70 (m, 4H), 7.38 - 7.26 (m, 9H), 5.10 - 4.88 (m, 2H), 4.21 -4.12 (m, 1H), 4.07 - 3.95 (m, 4H), 3.94 - 3.83 (m, 1H), 2.44 (s, 6H), 1.98 - 1.78 (m, 4H).

### Step 6

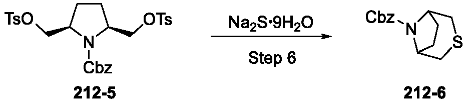

Sodium sulfide nonahydrate (42.77 g, 169.16 mmol, 3 eq) was added in batches to a mixed solution of compound **212-5** (34.1 g, 54.670 mmol, 1.0 eq) in ethanol (300 mL) and water (300 mL) with stirring under the protection of nitrogen at 20 °C. The reaction was carried out for 16 hours at 90 °C, and the reaction process was monitored by liquid chromatography-mass spectrometry and thin layer chromatography. After the reaction was completed, the reaction solution was cooled to room temperature, concentrated under reduced pressure to remove ethanol, the aqueous phase was extracted with ethyl acetate (200 mL x 3), the organic phases were combined and washed with 150 mL of saturated brine, the washed organic phase was dried over anhydrous sodium sulfate, filtered to remove the drying agent; the filtrate was concentrated under reduced pressure to obtain a crude product. The crude product was purified by silica gel column chromatography, eluted with a gradient of 0%→ 40% ethyl acetate/petroleum ether mobile phase, and the obtained fractions were evaporated under reduced pressure to remove the solvent to obtain compound **212-6** (colorless oil, 9.5 g, yield: 61%). MS (ESI, m/z): 264.0[M + H]⁺; ¹H NMR (400 MHz, CDCl₃) *δ* 7.38 - 7.30 (m, 5H), 5.16 (s, 2H), 4.58 - 4.43 (m, 2H), 3.29 - 3.07 (m, 2H), 2.19 - 2.10 (m, 2H), 2.08 - 2.03 (m, 4H).

### Step 7

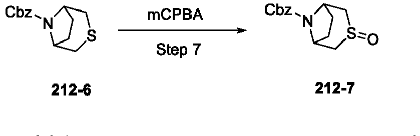

*m*-Chloroperoxybenzoic acid (80% content, 7.76 g, 35.982 mmol, 1.05 eq) was added in batches to a solution of compound **212-6** (9.5 g, 34.269 mmol, 1.0 eq) in dichloromethane (200 mL) with stirring under the protection of nitrogen at 0 °C. The reaction was carried out for 3 hours at 20 °C, and the reaction process was monitored by liquid chromatography-mass spectrometry and thin layer chromatography. After the reaction was completed, the reaction solution was diluted with 200 mL of water, the aqueous phase was extracted with dichloromethane (400 mL x 3), the organic phases were combined and washed with 150 mL of saturated brine, the washed organic phase was dried over anhydrous sodium sulfate, then filtered to remove the drying agent; the filtrate was concentrated under reduced pressure to obtain a crude product. The crude product was purified by silica gel column chromatography, eluted with a gradient of 0%→8% methanol/dichloromethane mobile phase, and the obtained fractions were evaporated under reduced pressure to remove the solvent to obtain compound 212-7 (yellow oil, 10 g, yield: 99%). MS (ESI, m/z): 280.0[M + H]⁺; ¹H NMR (300 MHz, CDCl₃) *δ* 7.43 - 7.35 (m, 5H), 5.26 - 5.13 (m, 2H), 4.73 - 4.52 (m, 2H), 3.65 - 3.53 (m, 2H), 3.09 - 2.66 (m, 2H), 2.30 - 2.08 (m, 2H), 1.91 - 1.75 (m, 2H).

### Step 8

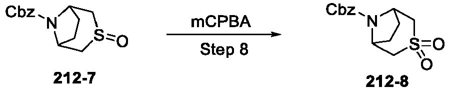

m-Chloroperoxybenzoic acid (80% content, 1.54 g, 7.142 mmol, 1.05 eq) was added in batches to a solution of compound **212-7** (2 g, 6.80 mmol, 1.0 eq) in dichloroethane (80 mL) with stirring under the protection of nitrogen at 0 °C. The reaction was carried out for 16 hours at 20 °C, and the reaction process was monitored by liquid chromatography-mass spectrometry and thin layer chromatography. After the reaction was completed, the reaction was quenched by adding 80 mL of 10% sulfite aqueous solution at 20 °C, the aqueous phase was extracted with dichloromethane (100 mL x 3), and the organic phases were combined and washed with 80 mL of saturated brine, and the washed organic phase was dried over anhydrous sodium sulfate and filtered to remove the drying agent; the filtrate was concentrated under reduced pressure to obtain a crude product. The crude product was purified by silica gel column chromatography, eluted with a gradient of 0%→ 50% ethyl acetate/petroleum ether mobile phase, and the obtained fractions were evaporated under reduced pressure to remove the solvent to obtain compound **212-8** (white oil, 2.04 g, yield: 98%). MS (ESI, m/z): 318.0[M + Na]⁺; ¹H NMR (300 MHz, CDCl₃) *δ* 7.43 - 7.35 (m, 5H), 5.25 - 5.17 (m, 2H), 4.79 - 4.64 (m, 2H), 3.67 - 3.34 (m, 2H), 3.25 - 3.12 (m, 2H), 2.57 - 2.42 (m, 2H), 2.27 - 2.11 (m, 2H).

### Step 9

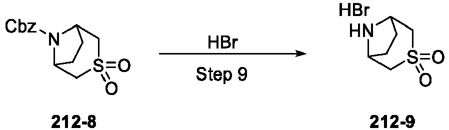

**212-8** (1.48 g, 4.760 mmol, 1.0 eq) and a solution of hydrobromic acid (33%, 19.00 mL) in acetic acid were added to a reaction flask with stirring at 20 °C. The reaction was carried out for 16 hours at 20 °C, and the reaction process was monitored by liquid chromatography-mass spectrometry and thin layer chromatography. After the reaction was completed, the reaction solution was concentrated under reduced pressure to obtain a crude product, and the crude product was washed with ethyl acetate (15 mL x 3) to obtain crude compound **212-9** (white solid, 1.05 g, yield: 86%). MS (ESI, m/z): 162.2[M + H]⁺; ¹H NMR (400 MHz, DMSO-*d*₆) *δ* 9.49 (s, 2H), 4.59 - 4.44 (m, 2H), 3.80 - 3.56 (m, 4H), 2.41 - 2.29 (m, 2H), 2.14 - 2.01 (m, 2H).

### Step 10

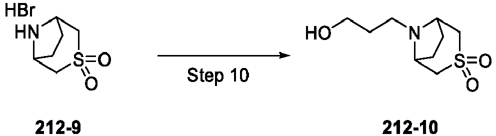

Potassium carbonate (913.26 g, 6.276 mmol, 4 eq) was added in batches to a solution of compound **212-9** (400 mg, 1.569 mmol, 1.0 eq) and 3-bromo-1-propanol (229.62 mg, 1.569 mmol, 1.0 eq) in acetonitrile (5 mL) with stirring at 20 °C. The reaction was carried out for 16 hours at 60 °C, and the reaction process was monitored by liquid chromatography-mass spectrometry and thin layer chromatography. After the reaction was completed, the reaction mixture was cooled to room temperature, and concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **212-10** (colorless oil, 128 mg, yield: 33%). MS (ESI, m/z): 220.1[M + H]⁺.

### Step 11

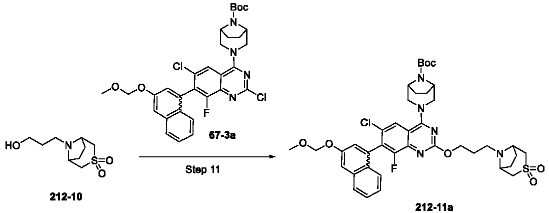

A solution of potassium tert-butoxide (1 mol/L, 0.27 mL, 0.27 mmol, 1.5 eq) in tetrahydrofuran was added dropwise to a solution of compound **67-3a** (120 mg, 0.185 mmol, 1.0 eq) and **212-10** (51.48 mg, 0.22 mmol, 1.2 eq) in tetrahydrofuran (2.5 mL) with stirring under the protection of nitrogen at 0 °C. The reaction was carried out for 1.5 hours at 0 °C, and the reaction process was monitored by liquid chromatography-mass spectrometry and thin layer chromatography. After the reaction was completed, 20 mL of water was added to the reaction solution at 0 °C to quench the reaction, the aqueous phase was extracted with ethyl acetate (20 mL x 3), and the organic phases were combined, the organic phase was dried over anhydrous sodium sulfate, and filtered to remove the drying agent; the filtrate was concentrated under reduced pressure to obtain a crude product. The crude product was purified by silica gel column chromatography, eluted with a gradient of 0%→4% methanol/dichloromethane mobile phase, and the obtained fractions were evaporated under reduced pressure to remove the solvent to obtain compound **212-11a** (white solid, 90 mg, yield: 55%). MS (ESI, m/z): 796.3/798.3[M + H]⁺.

### Step 12

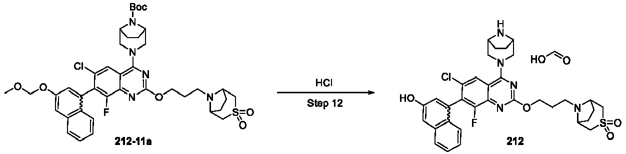

A solution of hydrochloric acid (4 mol/L, 2.0 mL) in dioxane was added to a solution of compound **212-11a** (85 mg, 0.101 mmol, 1 eq) in methanol (2.0 mL) with stirring under the protection of nitrogen at 0 °C. The reaction was carried out for 1 hour at 0 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 10%→ 50% (acetonitrile/methanol, 1/ 1)/water mobile phase (0.1% formic acid) in 20 min; detector, UV254 nm; to obtain compound 212 (white solid, 43.1 mg, yield: 58%). MS (ESI, m/z): 652.2/654.2[M + H]⁺; ¹H NMR (300 MHz, DMSO-*d*₆) *δ* 8.31 - 8.21 (m, 1H), 7.97 - 7.93 (m, 1H), 7.81 (d, *J* = 8.3 Hz, 1H), 7.48 - 7.40 (m, 1H), 7.29 (d, *J* = 2.4 Hz, 1H), 7.26 - 7.18 (m, 2H), 7.07 (d, *J* = 2.4 Hz, 1H), 4.47 - 4.31 (m, 4H), 3.75 - 3.58 (m, 7H), 3.36 - 3.26 (m, 2H), 3.09 - 2.98 (m, 2H), 2.72 - 2.63 (m, 2H), 2.14 - 2.01 (m, 2H), 2.02 - 1.89 (m, 4H), 1.80 - 1.68 (m, 4H); ¹⁹F NMR (282 MHz, DMSO) *δ*-122.44.

### Embodiment 52

### (1R,3S or 3R,5S)-8-(3-((4-((1R,5S)-3,8-diazabicyclo[3.2.1]octan-3-yl)-6-chloro-8-fluoro-7-(3-hydroxynaphthalen-1-yl)quinazolin-2-yl)oxy)propyl)-3-imino-3λ⁶-thia-8-azabicyclo[3.2.1]octane 3-oxide formate 213a; (1R,3R or 3S,5S)-8-(3-((4-((1R,5S)-3,8-diazabicyclo[3.2.1]octan-3-yl)-6-chloro-8-fluoro-7-(3-hydroxynaphthalen-1-yl)quinazolin-2-yl)oxy)propyl)-3-imino-3λ⁶-thia-8-azabicyclo[3.2.1]octane3-oxide formate

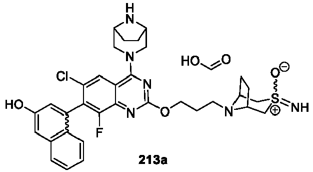

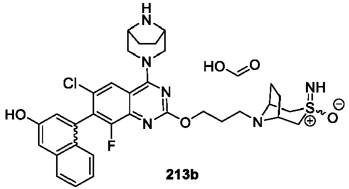

The synthetic route was as follows:

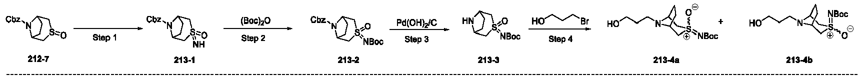

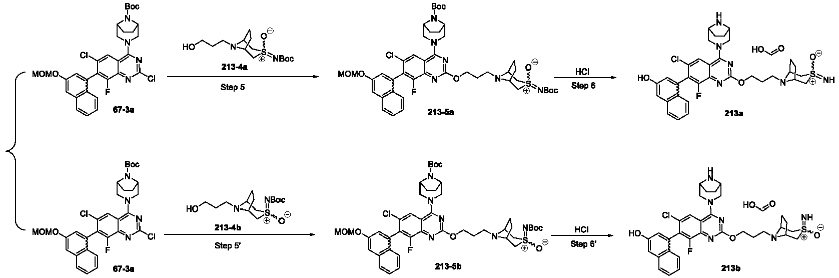

### Step 1

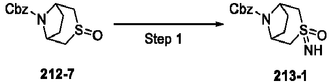

Compound **212-7** (2.94 g, 9.99 mmol, 1.00 eq), ammonium carbamate (2.05 g, 24.99 mmol, 2.50 eq), iodobenzene acetate (10.17 g, 29.99 mmol, 3.00 eq) and methanol (30 mL) were added to a 250 mL single-neck flask with stirring at 25 °C. The mixture was stirred at 20 °C for 2 hours. The reaction process was monitored by liquid chromatography-mass spectrometry and thin layer chromatography. After the reaction was completed, the reaction mixture was concentrated under reduced pressure to obtain a crude product. The obtained crude product was purified by silica gel column chromatography, eluted with a gradient of 0%→5% methanol/dichloromethane mobile phase, and the obtained fractions were evaporated under reduced pressure to remove the solvent to obtain compound **213-1** (oil, 2.4 g, yield: 82%). MS (ESI, m/z): 295.1[M + H]⁺; ¹H NMR (300 MHz, CDCl₃) *δ* 7.41 - 7.35 (m, 5H), 5.20 (s, 2H), 4.81 - 4.60 (m, 2H), 3.73 - 3.38 (m, 4H), 2.57 - 2.47 (m, 2H), 2.22 - 2.05 (m, 2H).

### Step 2

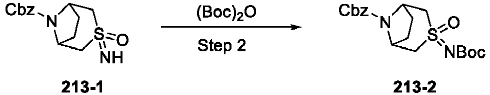

Sodium hydride (60% content, 929.3 mg, 23.23 mmol, 3.00 eq) was added in batches to a solution of compound **213-1** (2.4 g, 7.74 mmol, 1.00 eq) in tetrahydrofuran (20 mL) with stirring under the protection of nitrogen at 0 °C. The mixture was stirred and reacted at 0 °C for 5 min. Di-*tert*-butyl dicarbonate (5.34 g, 23.23 mmol, 3.00 eq) was then added dropwise to the reaction solution. After the dropwise addition, the mixture was stirred for 4 hours at 20 °C. The reaction process was monitored by liquid chromatography-mass spectrometry and thin layer chromatography. After the reaction was completed, ice water (40 mL) was added to the reaction solution at 0 °C to quench the reaction, then the mixture was extracted with dichloromethane (40 mL x 3), the organic phases were combined, dried over anhydrous sodium sulfate, and concentrated under reduced pressure to obtain a crude product. The obtained crude product was purified by reversed-phase chromatographic column (C18 column), and eluted with 5%→ 90% acetonitrile/water mobile phase (0.1% ammonia water) in 20 min; detector, UV210/200 nm; to obtain compound **213-2** (white solid, 6 g, yield: 55%). MS (ESI, m/z): 295.1[M + H]⁺.

### Step 3

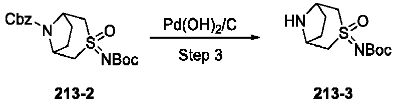

Palladium hydroxide /carbon catalyst (10%, 164.3 mg) was added to a solution of compound **213-2** (1.62 g, 3.90 mmol, 1.00 eq) in methanol (30 mL) with stirring under the protection of nitrogen at 25 °C. After the nitrogen was replaced with hydrogen by a gas replacement operation, the mixture was stirred and reacted at 50 °C for 3 hours. The reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 0%→ 90% (7% methanol/dichloromethane and 2% ammoniacal methanol)/dichloromethane mobile phase, and the obtained fractions were evaporated under reduced pressure to remove the solvent to obtain compound **213-3** (white solid, 578 mg, yield: 54%). MS (ESI, m/z): 261.1[M + H]⁺; ¹H NMR (400 MHz, CDCl₃) *δ* 4.19 - 4.09 (m, 2H), 3.98 - 3.91 (m, 2H), 3.37 - 3.29 (m, 2H), 2.39 - 2.30 (m, 2H), 1.95 - 1.87 (m, 2H), 1.49 (s, 9H).

### Step 4

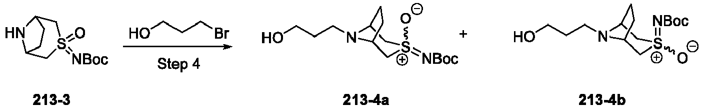

Compound **213-3** (577 mg, 2.10 mmol, 1.00 eq), 3-bromopropanol (308 mg, 2.10 mmol, 1.00 eq), potassium carbonate (612.6 mg, 4.21 mmol, 2.00 eq) and acetonitrile (8 mL) were successively added to a reaction flask under the protection of nitrogen at 25 °C. The reaction was carried out for 12 hours at 80 °C under nitrogen atmosphere. The reaction process was monitored by liquid chromatography-mass spectrometry and thin layer chromatography. After the reaction was completed, the reaction mixture was concentrated to obtain a crude product. The obtained crude product was purified by reversed-phase chromatographic column (C18 column), and eluted with 5%→ 30% acetonitrile/water mobile phase (0.05% ammonium bicarbonate) in 20 min; detector, UV200/220 nm; to obtain two compounds.

Compound **213-4a** (white solid, 41 mg, yield: 5%), MS (ESI, m/z): 319.4[M + H]⁺; ¹H NMR (300 MHz, CDCl₃) *δ* 3.94 - 3.73 (m, 6H), 3.58 - 3.46 (m, 2H), 2.95 - 2.83 (m, 2H), 2.54 - 2.39 (m, 2H), 2.26 - 2.16 (m, 2H), 1.89 - 1.76 (m, 2H), 1.51 (s, 9H).

Compound **213-4b** (white solid, 228 mg, yield: 30%), MS (ESI, m/z): 319.4[M + H]⁺; ¹H NMR (300 MHz, CDCl₃) *δ* 4.10 - 4.01 (m, 2H), 3.89 - 3.82 (m, 2H), 3.81 - 3.72 (m, 2H), 3.50 - 3.37 (m, 2H), 2.84 (t, *J=* 6.1 Hz, 2H), 2.36 - 2.26 (m, 2H), 2.17 - 2.08 (m, 2H), 1.85 - 1.72 (m, 2H), 1.51 (s, 9H).

### Step 5

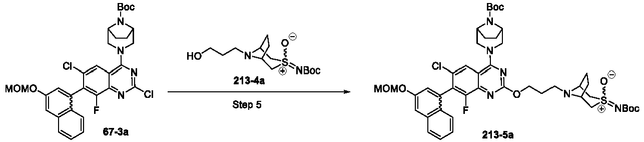

A solution of potassium tert-butoxide (1 mol/L, 0.12 mL, 0.12 mmol, 1.20 eq) in tetrahydrofuran was slowly dropwise added to a solution of compound **67-3a** (65 mg, 0.10 mmol, 1.00 eq) and **213-4a** (40 mg, 0.12 mmol, 1.20 eq) in tetrahydrofuran (2 mL) under the protection of nitrogen at 0 °C. The reaction was carried out at this temperature for 1 hour. The reaction process was monitored by liquid chromatography-mass spectrometry and thin layer chromatography. After the reaction was completed, ice water (5 mL) was added to the reaction solution at 0 °C to quench the reaction, then the mixture was extracted with ethyl acetate (10 mL × 3), the organic phases were combined, dried over anhydrous sodium sulfate, and concentrated under reduced pressure to obtain a crude product. The obtained crude product was purified by preparative silica gel thin layer chromatography (elution solvent system: dichloromethane/methanol = 16/1) to obtain compound **213-5a** (white solid, 56.2 mg, yield: 59%). MS (ESI, m/z): 895.2[M + H]⁺.

### Step 5:

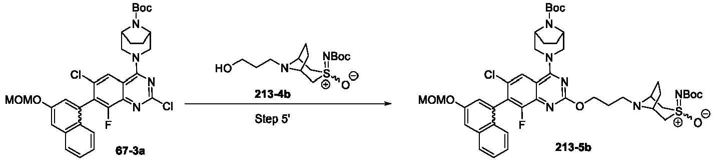

A solution of potassium *tert*-butoxide (1 mol/L, 0.19 mL, 0.18 mmol, 1.20 eq) in tetrahydrofuran was slowly added dropwise to a solution of compound **67-3a** (100 mg, 0.15 mmol, 1.00 eq) and **213-4b** (62 mg, 0.18 mmol, 1.20 eq) in tetrahydrofuran (2 mL) under the protection of nitrogen at 0 °C. The reaction was carried out at this temperature for 1 hour. The reaction process was monitored by liquid chromatography-mass spectrometry and thin layer chromatography. After the reaction was completed, ice water (5 mL) was added to the reaction solution at 0 °C to quench the reaction, then the mixture was extracted with ethyl acetate (10 mL × 3), the organic phases were combined, dried over anhydrous sodium sulfate, and concentrated under reduced pressure to obtain a crude product. The obtained crude product was purified by preparative silica gel thin layer chromatography (elution solvent system: dichloromethane/methanol = 16/1) to obtain compound **213-5b** (white solid, 110 mg, yield: 75%). MS (ESI, m/z): 895.4[M + H]⁺.

### Step 6

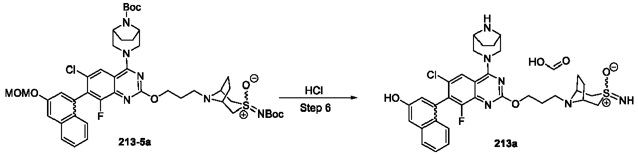

A solution of hydrochloric acid (4 mol/L, 1 mL) in 1,4-dioxane was added dropwise to a solution of compound **213-5a** (56 mg, 0.05 mmol, 1.00 eq) in methanol (1 mL) with stirring at room temperature, the reaction was carried out at room temperature for 1 hour, and the reaction process was monitored by liquid chromatography-mass spectrometry and thin layer chromatography. After the reaction was completed, the reaction mixture was concentrated to obtain a crude product. The obtained crude product was purified by reversed-phase chromatographic column (C18 column), and eluted with 5%→ 60% (acetonitrile/methanol = 1/1)/water mobile phase (0.1% formic acid) in 20 min; detector, UV254/220 nm; to obtain compound **213a** (white solid, 24 mg, yield: 57%). MS (ESI, m/z): 651.1/653.1[M + H]⁺; ¹H NMR (400 MHz, DMSO-*d*₆) *δ* 10.03 (s, 1H), 8.22 - 8.16 (m, 1H), 7.97 - 7.91 (m, 1H), 7.81 (d, *J=* 8.3 Hz, 1H), 7.49 - 7.40 (m, 1H), 7.28 (d, *J=* 2.4 Hz, 1H), 7.26 - 7.20 (m, 2H), 7.06 (d, *J=* 2.4 Hz, 1H), 4.46 - 4.26 (m, 4H), 3.92 - 3.75 (m, 1H), 3.65 - 3.51 (m, 6H), 3.24 - 3.19 (m, 2H), 2.94 (d, *J=* 13.5 Hz, 2H), 2.70 - 2.62 (m, 2H), 2.17 - 2.09 (m, 2H), 1.94 - 1.87 (m, 4H), 1.77 - 1.63 (m, 4H); ¹⁹F NMR (377 MHz, DMSO-*d*₆) *δ*-122.46.

### Step 6:

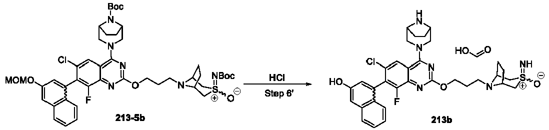

A solution of hydrochloric acid (4 mol/L, 2 mL) in 1,4-dioxane was added dropwise to a solution of compound **213-5b** (95 mg, 0.10 mmol, 1.00 eq) in methanol (2 mL) with stirring at room temperature. The reaction was carried out for 1 hour at room temperature, and the reaction process was monitored by liquid chromatography-mass spectrometry and thin layer chromatography. After the reaction was completed, the reaction mixture was concentrated to obtain a crude product. The obtained crude product was purified by reversed-phase chromatographic column (C18 column), and eluted with 5%→ 60% (acetonitrile/methanol = 1/1)/water mobile phase (0.1% formic acid) in 20 min; detector, UV254/220 nm; to obtain compound **213b** (white solid, 50 mg, yield: 71%). MS (ESI, m/z): 651.1/653.1[M + H]⁺; ¹H NMR (400 MHz, DMSO-*d*₆) *δ* 8.27 - 8.21 (m, 1H), 7.96 - 7.92 (m, 1H), 7.81 (d, *J* = 8.3 Hz, 1H), 7.47 - 7.40 (m, 1H), 7.28 (d, *J* = 2.4 Hz, 1H), 7.25 - 7.19 (m, 2H), 7.06 (d, *J* = 2.4 Hz, 1H), 4.45 - 4.31 (m, 4H), 3.69 - 3.55 (m, 6H), 3.29 - 3.21 (m, 2H), 3.11 - 3.03 (m, 2H), 2.66 (t, *J* = 7.0 Hz, 2H), 2.23 - 2.14 (m, 2H), 1.97 - 1.81 (m, 4H), 1.76 - 1.65 (m, 4H); ¹⁹F NMR (377 MHz, DMSO-*d*₆) *δ*-122.44.

### Embodiment 53

### 4-((S or R)-4-((1R, 5S)-3,8-diazabicyclo[3.2.1]octane-3-yl)-6-chloro-8-fluoro-2-((2R, 7aS)-2-fluorotetrahydro-1H-pyrrolidin-7a (5H)-yl) methoxy)quinazolin-7-yl)-5, 6-difluoronaphthalen-2-ol diformate 214a; 4-(R or S)-4-((1R, 5S)-3,8-diazabicyclo[3.2.1]octane-3-yl)-6-chloro-8-fluoro-2-((2R,7aS)-2-fluorotetrahydro-1H-pyrrolidin-7a(5H)-yl) methoxy) quinazolin-7-yl)-5,6-difluoronaphthalen-2-ol diformate 214b.

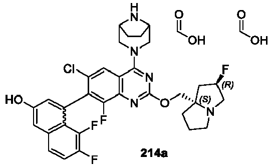

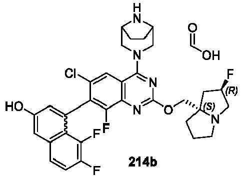

The synthetic route was as follows:

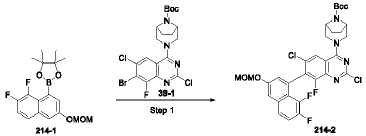

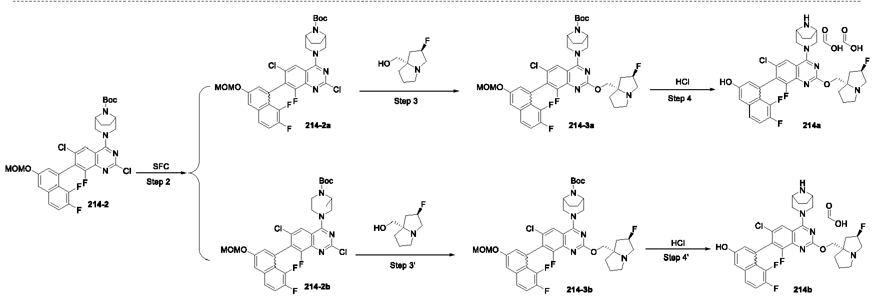

### Step 1

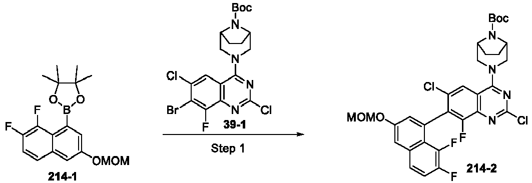

Compound **214-1** was synthesized with reference to patent (WO2021041671 (A1)).

Compound **214-1** (500 mg, 1.35 mmol, 1.00 eq), compound **39-1** (795.07 mg, 1.49 mmol, 1.10 eq), tris(dibenzylideneacetone)dipalladium (0) (130.75 mg, 0.13 mmol, 0.1 eq), 3-(tert-butyl)-4-(2,6-dimethoxyphenyl)-2,3-dihydrobenzo[d] [1,3]oxy,phosphopentyrojugate (47.17 mg, 0.13 mmol, 0.1 eq) and potassium phosphate (606.18 mg, 2.71 mmol, 2 eq) were dissolved in a toluene (10 mL) and water (2 mL) mixed solvent with stirring at 25 °C. The mixture was ventilated with nitrogen for 3 times, the reaction was carried out for 2 hours at 80 °C, and the reaction process was monitored by liquid chromatography-mass spectrometry and thin layer chromatography. After the reaction was completed, the reaction solution was concentrated to obtain a crude product, then purified by silica gel column chromatography, eluted with a gradient of 0% to 25% ethyl acetate/petroleum ether mobile phase, and the obtained fraction was evaporated under reduced pressure to remove the solvent to obtain compound **214-2** (white solid, 600 mg, yield: 65%). MS (ESI, *m*/z): 649.0/651.0[M + H]⁺; ¹H NMR (400 MHz, CDCl₃) *δ* 7.79 - 7.75 (m, 1H), 7.63 - 7.56 (m, 1H), 7.56 - 7.52 (m, 1H), 7.40 - 7.31 (m, 1H), 7.21 - 7.14 (m, 1H), 5.32 (s, 2H), 4.60 - 4.33 (m, 4H), 3.82 - 3.61 (m, 2H), 3.55 (s, 3H), 1.92 - 1.73 (m, 4H), 1.52 (s, 9H).

### Step 2

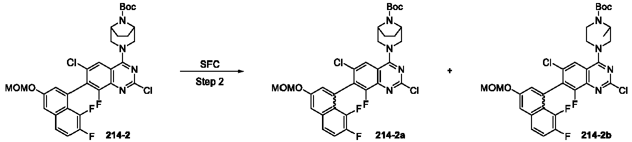

The compound **214-2** (600 mg) obtained in step 1 was subjected to chiral resolution by supercritical liquid chromatography: chiral column CHIRAL ART Amylose-SA, 3 x 25 cm, 5 µm; mobile phase A: supercritical carbon dioxide, mobile phase B: isopropanol; flow rate: 50 mL/min; eluted with 25% phase B in 12 min, detector: UV 220/235 nm, two products were obtained . The product with shorter retention time (2.442 min) was compound **214-2a,** *tert-*butyl (1*R*,5*S*)-3-((*S* or *R*)-2,6-dichloro-7-(7,8-difluoro-3-(methoxymethoxy)naphthalen-1-yl)-8-fluoroquinazolin-4-yl)-3,8-diazabicyclo[3.2.1]octane-8-carboxylate (white solid, 220.0 mg, recovery rate: 36%), MS (ESI, m/z): 649.0/651.0[M + H]⁺; the product with longer retention time (2.627 min) was compound **214-2b,** *tert*-butyl (1*R*,5*S*)-3-((*R* or S)-2,6-dichloro-7-(7,8-difluoro-3-(methoxymethoxy)naphthalen-1-yl)-8-fluoroquinazolin-4-yl)-3,8-diazabicyclo[3.2.1]octane-8-carboxylate (white solid, 240.0 mg, recovery rate: 40%), MS (ESI, m/z): 649.0/651.0[M + H]⁺.

### Step 3

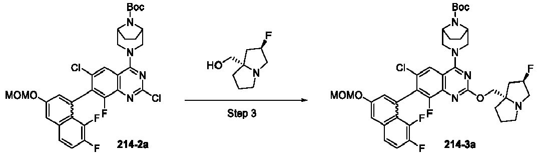

A solution of potassium tert-butoxide (1 mol/L, 0.18 mL, 0.18 mmol, 1.5 eq) in tetrahydrofuran was added dropwise to a solution of compound **214-2a** (80 mg, 0.117 mmol, 1.00 eq) and [(2*R*, 7a*S*)-2-fluoro-hexahydro-1*H*-pyrrolidinazin-7a-yl]methanol (23.53 mg, 0.14 mmol, 1.2 eq) in tetrahydrofuran (1 mL) with stirring under the protection of nitrogen at 0 °C. The reaction was carried out for 2 hours at 0 °C, and the reaction process was monitored by liquid chromatography-mass spectrometry and thin layer chromatography. After the reaction was completed, 15 mL of water was added to the reaction solution at 0 °C to quench the reaction, the aqueous phase was extracted with ethyl acetate (20 mL x 3), the organic phases were combined, the organic phase was dried over anhydrous sodium sulfate, and filtered to remove the drying agent; the filtrate was concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **214-3a** (white solid, 50 mg, yield: 52%). MS (ESI, *m*/*z*): 771.3/773.3[M + H]⁺.

### Step 4

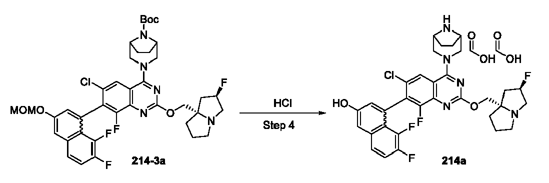

A solution of hydrochloric acid (4 mol/L, 1 mL) in 1,4-dioxane was added to a solution of compound **214-3a** (50 mg, 0.06 mmol, 1.00 eq) in methanol (1 mL) with stirring under the protection of nitrogen at 0 °C. The reaction was carried out for 2 hours at 0 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 0%→ 30% acetonitrile/water mobile phase (0.1% formic acid) in 20 min; detector, UV254 nm; to obtain compound **214a** (white solid, 13.5 mg, yield: 30%). MS (ESI, *m*/z): 628.1/630.1[M + H]⁺; ¹H NMR (400 MHz, DMSO-*d*₆) *δ* 8.24 (s, 2H), 7.92 - 7.87 (m, 1H), 7.78 - 7.70 (m, 1H), 7.64 - 7.52 (m, 1H), 7.42 - 7.37 (m, 1H), 7.11 (d, *J* = 2.3 Hz, 1H), 5.27 (d, *J* = 53.9 Hz, 1H), 4.37 (d, *J* = 12.4 Hz, 1H), 4.25 (d, *J* = 12.2 Hz, 1H), 4.09 (d, *J* = 10.4 Hz, 1H), 3.99 (d, *J* = 10.3 Hz, 1H), 3.66 - 3.57 (m, 3H), 3.54 - 3.48 (m, 1H), 3.13 - 3.00 (m, 3H), 2.87 - 2.79 (m, 1H), 2.16 - 1.99 (m, 3H), 1.91 - 1.63 (m, 7H); ¹⁹F NMR (377 MHz, DMSO-*d*₆) *δ*-123.63, -145.02, -145.07, -145.81, -145.86, -172.11. The chiral analysis conditions of compound **214a** were as follows: Column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 4.125 min; *dr* > 40: 1.

### Step 3:

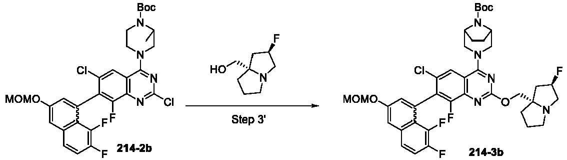

A solution of potassium *tert*-butoxide (1 mol/L, 0.18 mL, 0.18 mmol, 1.5 eq) in tetrahydrofuran was added dropwise to a solution of compound **214-2b** (80 mg, 0.117 mmol, 1.00 eq) and [(2*R*, 7a*S*)-2-fluoro-hexahydro-1*H*-pyrrolidinazin-7a-yl]methanol (23.53 mg, 0.14 mmol, 1.2 eq) in tetrahydrofuran (1 mL) with stirring under the protection of nitrogen at 0 °C. The reaction was carried out for 2 hours at 0 °C, and the reaction process was monitored by liquid chromatography-mass spectrometry and thin layer chromatography. After the reaction was completed, 15 mL of water was added to the reaction solution at 0 °C to quench the reaction, the aqueous phase was extracted with ethyl acetate (20 mL x 3), the organic phases were combined, the organic phase was dried over anhydrous sodium sulfate, and filtered to remove the drying agent; the filtrate was concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **214-3b** (white solid, 50 mg, yield: 52%). MS (ESI, *m*/*z*): 771.3/773.3[M + H]⁺.

### Step 4:

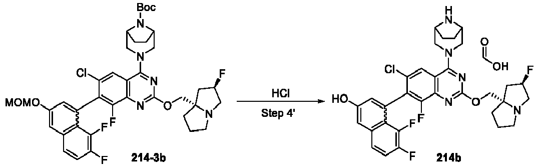

A solution of hydrochloric acid (4 mol/L, 1 mL) in 1,4-dioxane was added to a solution of compound **214-3b** (50 mg, 0.06 mmol, 1.00 eq) in methanol (1 mL) with stirring under the protection of nitrogen at 0 °C. The reaction was carried out for 2 hours at 0 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 0%→ 30% acetonitrile/water mobile phase (0.1% formic acid) in 20 min; detector, UV254 nm; to obtain compound **214b** (white solid, 26mg, yield: 61%). MS (ESI, *m*/z): 628.1/630.1[M + H]⁺; ¹H NMR (400 MHz, DMSO-*d*₆) *δ* 8.22 (s, 1H), 7.92 - 7.87 (m, 1H), 7.78 - 7.71 (m, 1H), 7.62 - 7.52 (m, 1H), 7.42 - 7.37 (m, 1H), 7.10 (d, *J* = 2.3 Hz, 1H), 5.37 - 5.19 (m, 1H), 4.37 (d, *J* = 12.3 Hz, 1H), 4.25 (d, *J* = 11.9 Hz, 1H), 4.09 (d, *J* = 10.4 Hz, 1H), 4.00 (d, *J* = 10.4 Hz, 1H), 3.66 - 3.57 (m, 3H), 3.54 - 3.48 (m, 1H), 3.16 - 3.05 (m, 2H), 3.04 - 2.99 (m, 1H), 2.87 - 2.78 (m, 1H), 2.16 - 2.10 (m, 1H), 2.07 - 1.97 (m, 2H), 1.89 - 1.62 (m, 7H). ¹⁹F NMR (377 MHz, DMSO-*d*₆) *δ*-123.62, - 144.99, -145.00, -145.05, -145.05, -145.77, -145.82, -172.13. The chiral analysis conditions of compound **214b** were as follows: Column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 2.126 min; *dr* > 40: 1.

Other similar compound of the present disclosure can be prepared by the synthetic method shown in Embodiment 53 above. Some compounds prepared with reference to the above synthetic method and their characterization data are shown in table 10.

**Table 10**

| Num ber of the com poun d | Compound structure | Compound name | Chiral analysis condition s/retentio n time/*dr* value/spe cific rotation | Mas s spec trum [M+ H]⁺ | ¹H & ¹⁹F NMR |
|---|---|---|---|---|---|
| **228** | 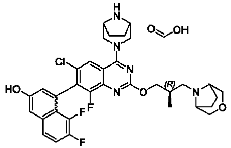 | ((4*S* or 4*R*) 4-(2-((2R)-3-(3-oxa-8-azabicyclo[3. 2.1]octan-8-yl)-2-methylpropo xy)-4-((1*R*,5*S*)-3,8-diazabicyclo[ 3.2.1]octan-3-yl)-6-chloro-8-fluoroquinaz olin-7-yl)-5,6-difluoronapht halen-2-ol formate | | 654. 2/65 6.2 | ¹H NMR (400 MHz, DMSO-*d*₆) *δ* 8.22 (s, 1H), 7.89 (s, 1H), 7.76 - 7.72 (m, 1H), 7.60-7.53 (m, 1H), 7.39-7.38 (m, 1H), 7.11 (d, *J* = 2.3 Hz, 1H), 4.50-4.46 (m, 1H), 4.38 (d, *J* = 12.3 Hz, 1H), 4.25 (d, *J* = 12.2 Hz, 1H), 4.19-4.14 (m, 1H), 3.63-3.60 (m, 2H), 3.52-3.47 (m, 4H), 3.39 - 3.35 (m, 2H), 3.04-3.03 (m, 1H), 2.97-2.96 (m, 1H), 2.28 - 2.15 (m, 2H), 2.10-2.02 (m, 1H), 1.83 - 1.80 (m, 2H), 1.74-1.65 (m, 6H), 1.00 (d, *J* = 6.6 Hz, 3H); ¹⁹F NMR (377 MHz, DMSO-*d*₆) *δ-*123.69, -144.99, -145.05, - 145.79, -145.84. |
| **248** | 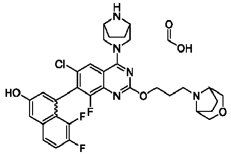 | (4*S* or 4*R*)-4-(2-(3-(3-oxa-8-azabicyclo[3. 2.1]octan-8-yl)propoxy)-4-(1*R*,5*S*)-3,8-diazabicyclo[ 3.2.1]octan-3-yl)-6-chloro-8-fluoroquinaz olin-7-yl)-5,6-difluoronapht halen-2-ol formate | | 640. 1/64 2.1 | ¹H NMR (300 MHz, DMSO-*d*₆) *δ* 8.41 - 8.14 (m, 1H), 7.90 (d, *J* = 1.6 Hz, 1H), 7.80 - 7.70 (m, 1H), 7.66 - 7.52 (m, 1H), 7.44 - 7.37 (m, 1H), 7.11 (d, *J* = 2.3 Hz, 1H), 4.53 - 4.37 (m, 3H), 4.27 (s, 1H), 3.70 - 3.59 (m, 4H), 3.53 - 3.47 (m, 2H), 3.43 - 3.35 (m, 2H), 3.11 - 3.03 (m, 2H), 2.44 - 2.33 (m, 2H), 1.96 -1.79 (m, 4H), 1.79 - 1.47 (m, 6H); ¹⁹F NMR (282 MHz, DMSO-*d*₆) *δ-*123.64, -144.99, -145.05, - 145.76, -145.83. |

### Embodiment 54

### 4-((7S or 7R)-2-(3-(3-oxa-8-azabicyclo[3.2.1]octan-8-yl)propoxy)-4-((1R,5S)-3,8-diazabicyclo[3.2.1]octan-3-yl)-6,8-difluoroquinazolin-7-yl)-5-ethyl-6-fluoronaphthalen-2-ol diformate 215a; 4-((7R or 7S)-2-(3-(3-oxa-8-azabicyclo[3.2.1]octan-8-yl)propoxy)-4-((1R,5S)-3,8-diazabicyclo[3.2.1]octan-3-yl)-6,8-difluoroquinazolin-7-yl)-5-ethyl-6-fluoronaphthalen-2-ol diformate 215b

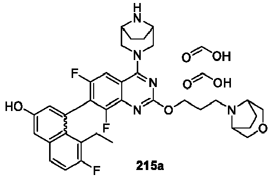

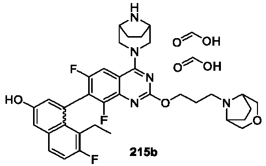

The synthetic route was as follows:

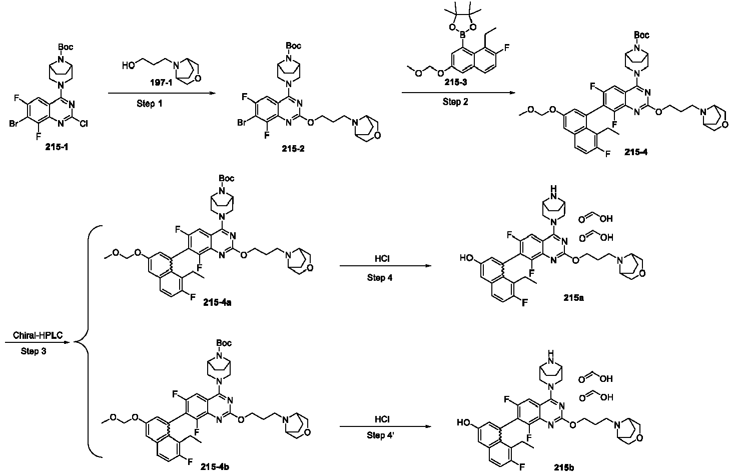

### Step 1

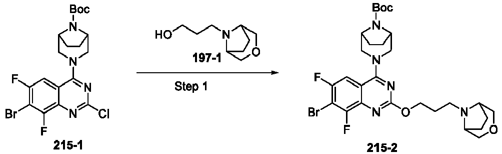

Compound **215-1** was synthesized according to Embodiment 15 (synthesis method XIII). Compound **215-1** (light yellow solid). MS (ESI, m/z): 488.9/490.9[M + H]⁺.

*N, N*-diisopropylethylamine (395.84 mg, 2.910 mmol, 3 eq) was added dropwise to a solution of compound **215-1** (500 mg, 0.970 mmol, 1.00 eq), **197-1** (349.65 mg, 1.94 mmol, 2.00 eq) and cesium fluoride (310.17 mg, 1.940 mmol, 2 eq) in *N*-methylpyrrolidone (5 mL) with stirring under the protection of nitrogen at 25 °C. The reaction was carried out for 8 hours at 120 °C, and the reaction process was monitored by liquid chromatography-mass spectrometry and thin layer chromatography. After the reaction was completed, the reaction mixture was cooled to room temperature. The reaction solution was then poured into 50 mL of water, then extracted with ethyl acetate (20 mL × 3), the organic phases were combined and washed with saturated brine (20 mL × 3), dried over anhydrous sodium sulfate, filtered and the filtrate was concentrated to obtain a crude product. The crude product was purified by silica gel column chromatography, eluted with a gradient of 0%→8% methanol/dichloromethane mobile phase, and the obtained fractions were evaporated under reduced pressure to remove the solvent to obtain compound **215-2** (white solid, 310 mg, 49%). MS (ESI, m/z): 624.2/626.2[M + H]⁺.

### Step 2

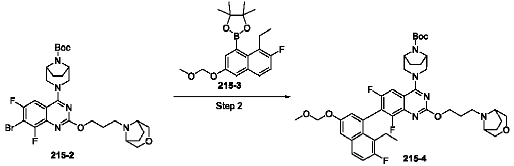

Compound **215-3** was synthesized with reference to patent (WO2021041671 (A1)).

Cesium carbonate (323.46 mg, 0.944 mmol, 2 eq)) was added in batches to a mixed solution of compound **215-2** (310 mg, 0.472 mmol, 1 eq), compound **215-3** (178.81 mg, 0.472 mmol, 1 eq), 2-dicyclohexylphosphine-2',4' 6 '-triisopropylbiphenyl (23.66 mg, 0.047 mmol, 0.1 eq) and methanesulfonic acid (2-dicyclohexylphosphine-2', 4 ', 6'-triisopropyl-1,1'-biphenyl)(2'-amino-1,1'-biphenyl-2-yl) palladium (II) (42.02 mg, 0.047 mmol, 0.1 eq) in 1,4-dioxane/water (5mL/1 mL). The reaction was carried out for 1 hour at 80 °C, and the reaction process was monitored by liquid chromatography-mass spectrometry and thin layer chromatography. After the reaction was completed, the reaction solution was cooled to room temperature, filtered to remove the insolubles, the filter cake was washed with 1,4-dixane (5 mL × 2), and the filtrate was concentrated pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **215-4** (colorless oil, 90 mg, 23%). MS (ESI, m/z): 778.1[M + H]⁺.

### Step 3

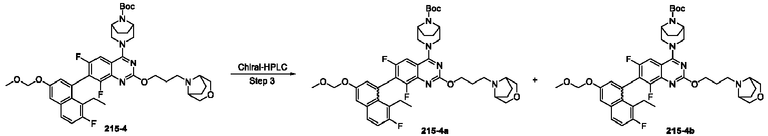

Compound **215-4** (90 mg) obtained in step 2 by supercritical liquid chromatography was subjected to chiral resolution: chiral column NBCHIRALPAK AD-H, 3 × 25 cm, 5 µm; mobile phase A: supercritical carbon dioxide, mobile phase B: isopropanol (0.5%, 2 mol/L ammonia methanol); flow rate: 50 mL/min; column temperature: 25 °C; eluted with 40% mobile phase B; detector UV223 nm, two products were obtained. The product with shorter retention time (5.62 min) was compound **215-4a**, *tert*-butyl (1*R*, 5*S*)-3-((7*S* or 7*R*)-2-(3-(3-oxa-8-azabicyclo[3.2. 1]octan-8-yl)propoxy)-7-(8-ethyl-7-fluoro-3-(methoxymethoxy)naphthalen-1-yl)-6,8-difluoroquinazolin-4-yl)-3,8-diazabicyclo[3.2.1]octane-8-carboxylate (colorless oil, 41 mg, recovery rate: 45%), MS (ESI, m/z): 778.1[M + H]⁺; the product with longer retention time (9.13 min) was compound **215-4b,** *tert*-butyl (1*R*, 5*S*)-3-((7*R* or 7*S*)-2-(3-(3-oxa-8-azabicyclo[3.2.1]octane-8-yl)propoxy)-7-(8-ethyl-7-fluoro-3-(methoxymethoxy)naphthalen-1-yl)-6,8-difluoroquinazolin-4-yl)-3,8-diazabicyclo[3.2.1]octane-8-carboxylate (colorless oil, 41 mg, recovery rate: 45%), MS (ESI, m/z): 778.1[M + H]⁺.

### Step 4

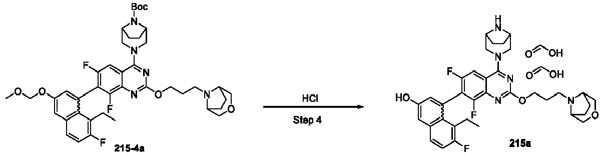

A solution of hydrochloric acid (4 mol/L, 2.0 mL) in 1,4-dioxane was added to a solution of compound **215-4a** (41 mg, 0.050 mmol, 1 eq) in methanol (2.0 mL) with stirring under the protection of nitrogen at 0 °C. The reaction was carried out for 1 hour at 0 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 10%→ 50% acetonitrile/water mobile phase (0.1% formic acid) in 20 min; detector, UV254 nm; to obtain compound **215a** (white solid, 20 mg, yield: 52%). MS (ESI, m/z): 634.2[M + H]⁺; ¹H NMR (400 MHz, DMSO-*d*₆) *δ* 8.20 (s, 2H), 7.83 - 7.75 (m, 1H), 7.72 - 7.66 (m, 1H), 7.41 - 7.33 (m, 2H), 7.01 (d, *J* = 2.6 Hz, 1H), 4.46 - 4.37 (m, 2H), 4.36 - 4.24 (m, 2H), 3.68 - 3.63 (m, 2H), 3.60 - 3.57 (m, 1H), 3.54 - 3.47 (m, 3H), 3.41 - 3.37 (m, 2H), 3.08 - 3.03 (m, 2H), 2.44 - 2.28 (m, 4H), 1.91 - 1.79 (m, 4H), 1.78 - 1.64 (m, 6H), 0.74 (t, *J* = 7.4 Hz, 3H); ¹⁹F NMR (377 MHz, DMSO-*d*₆) *δ*-118.39, -119.21, -123.82; the chiral resolution conditions of compound **215a** were: Column: XA-CHIRALPAK IG-3, 4.6 × 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min, detector UV 220 nm; retention time: 4.214 min; ee > 95%.

### Step 4:

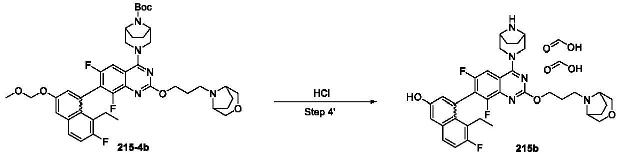

A solution of hydrochloric acid (4 mol/L, 2.0 mL) in 1,4-dioxane was added to a solution of compound **215-4b** (41 mg, 0.050 mmol, 1 eq) in methanol (2.0 mL) with stirring under the protection of nitrogen at 0 °C. The reaction was carried out for 1 hour at 0 °C with stirring, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 10%→ 50% acetonitrile/water mobile phase (0.1% formic acid) in 20 min; detector, UV254 nm; to obtain compound **215b** (white solid, 20 mg, yield: 52%). MS (ESI, m/z): 634.2[M + H]⁺; ¹H NMR (400 MHz, DMSO-*d*₆) *δ* 8.20 (s, 2H), 7.82 - 7.76 (m, 1H), 7.73 - 7.67 (m, 1H), 7.42 - 7.34 (m, 2H), 7.01 (d, *J* = 2.6 Hz, 1H), 4.46 - 4.38 (m, 2H), 4.36 - 4.24 (m, 2H), 3.68 - 3.63 (m, 2H), 3.61 - 3.56 (m, 1H), 3.54 - 3.46 (m, 3H), 3.41 - 3.36 (m, 2H), 3.08 - 3.04 (m, 2H), 2.45 - 2.29 (m, 4H), 1.93 - 1.78 (m, 4H), 1.77 - 1.64 (m, 6H), 0.74 (t, *J* = 7.4 Hz, 3H); ¹⁹F NMR (377 MHz, DMSO-*d*₆) *δ*-118.40, -119.21, -123.83. The chiral analysis conditions of compound **215b** were as follows: Column: XA-CHIRALPAK IG-3, 4.6 × 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min, detector UV 220 nm; retention time: 2.706 min; ee > 95%.

Other similar compound of the present disclosure can be prepared by the synthetic method shown in Embodiment **54** above. Some compounds prepared with reference to the above synthetic method and their characterization data are shown in table 11.

**Table 11**

| Num ber of the com poun d | Compound structure | Compound name | Chiral analysis conditions/rete ntion time/*dr value*/specific rotation | Mas s spec trum [M+ H]⁺ | ¹H & ¹⁹F NMR |
|---|---|---|---|---|---|
| **229a** | 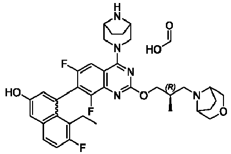 | 4-((7*S* or 7*R*)-2-((2*R*)-3-(3-oxa-8-azabicyclo[ 3.2.1]octan-8-yl)-2-methylprop oxy)-4-((1*R*,5*S*)-3,8-diazabicycl o[3.2.1]octa n-3-yl)-6,8-difluoroquin azolin-7-yl)-5-ethyl-6-fluoronapht halen-2-ol formate | CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 1.083 min; *dr* > 40: 1. | 648. 2/65 0.2 | ¹H NMR (400 MHz, DMSO-*d*₆) *δ* 8.23 (s, 1H), 7.81 - 7.77 (m, 1H), 7.70 - 7.68 (m, 1H), 7.40 - 7.35 (m, 2H), 7.02 (d, *J* = 2.7 Hz, 1H), 4.50 - 4.46 (m, 1H), 4.38 - 4.27 (m, 2H), 4.21 - 4.16 (m, 1H), 3.76 - 3.74 (m, 2H), 3.63 (d, *J* = 12.6 Hz, 1H), 3.56 - 3.53 (m, 2H), 3.46 (s, 1H), 3.39 - 3.35 (m, 2H), 3.06 - 3.05 (m, 1H), 2.96 - 2.95 (m, 1H), 2.48 - 2.42 (m, 1H), 2.38 - 2.31 (m, 1H), 2.28 - 2.16 (m, 2H), 2.11 - 2.05 (m, 1H), 1.89 - 1.72 (m, 6H), 1.70 - 1.65 (m, 2H), 1.00 (d, *J* = 6.6 Hz, 3H), 0.76 - 0.72 (m, 3H); ¹⁹F NMR (377 MHz, DMSO-*d*₆) *δ*-118.48, -119.19, -123.88. |
| **229b** | 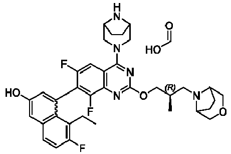 | 4-((7*R* or 7S)-2-((2*R*)-3-(3-oxa-8-azabicyclo[ 3.2.1]octan-8-yl)-2-methylprop oxy)-4-((1*R*,5*S*)-3,8-diazabicycl o[3.2.1]octa n-3-yl)-6,8-difluoroquin azolin-7-yl)-5-ethyl-6-fluoronapht halen-2-ol formate | CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 2.010 min; *dr* > 40: 1. | 648. 2/65 0.2 | ¹H NMR (400 MHz, DMSO-*d*₆) *δ* 10.01 (s, 1H), 8.19 - 8.15 (m, 1H), 7.81 - 7.77 (m, 1H), 7.69 (d, *J* = 10.2 Hz, 1H), 7.39 - 7.35 (m, 2H), 7.01 - 7.00 (m, 1H), 4.53 - 4.49 (m, 1H), 4.36 - 4.26 (m, 2H), 4.15 - 4.11 (m, 1H), 3.76 - 3.74 (m, 2H), 3.63 (d, *J* = 12.6 Hz, 1H), 3.56-3.53 (m, 2H), 3.46 (s, 1H), 3.39 - 3.35 (m, 2H), 3.06 - 3.05 (m, 1H), 2.96 - 2.95 (m, 1H),2.46 - 2.41 (m, 1H), 2.35 - 2.31 (m, 1H) 2.28 - 2.15 (m, 2H), 2.09 - 2.03 (m, 1H), 1.83 - 1.65 (m, 8H), 1.00 (d, *J* = 6.6 Hz, 3H), 0.76 - 0.72 (m, 3H); ¹⁹F NMR (377 MHz, DMSO-*d*₆) δ-118.60, -119.19, -123.90. |
| **249** | 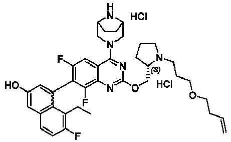 | 4-((*S* or *R*)-4-((1*R*,5*S*)-3,8-diazacyclo[ 3.2.1]octan-3-yl)-2-((*S*)-1-(3-(but-3-en-1-oxy)propyl) pyrrolidin-2-yl)methoxy) -6,8-difluoroquin azolin-7-yl)-5-ethyl-6-fluoronapht halen-2-ol dihydrochlo ride | | 676. 3 | ¹H NMR (300 MHz, DMSO-*d*₆) *δ* 10.77 - 10.41 (m, 1H), 10.03 - 9.81 (m, 1H), 9.70 (s, 1H), 7.88 - 7.73 (m, 2H), 7.46 - 7.33 (m, 2H), 7.11 - 7.02 (m, 1H), 5.86 - 5.65 (m, 1H), 5.08 - 4.90 (m, 2H), 4.86 - 4.74 (m, 1H), 4.74 - 4.63 (m, 1H), 4.55 - 4.39 (m, 2H), 4.24 - 4.12 (m, 2H), 4.01 - 3.88 (m, 6H), 3.47 - 3.33 (m, 4H), 3.23 - 3.06 (m, 2H), 2.40 - 2.13 (m, 4H), 2.11 - 1.85 (m, 9H), 0.83 - 0.69 (m, 3H); ¹⁹F NMR (282 MHz, DMSO-*d*₆) *δ*-116.43, -116.44, -119.18, -123.11, - 123.13. |
| **250a** | 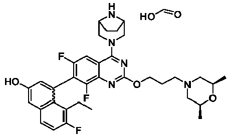 | (*S* or *R*)-4-(4-((1*R*,5*S*)-3,8-diazacyclo[ 3.2.1]octan-3-yl)-2-(3-((2*R*,6*S*)-2,6-dimethylmo rpholinyl)pr opoxy)-6,8-difluoroquin azolin-7-yl)-5-ethyl-6-fluoronapht halen-2-ol formate | NCHIRALPAK IC-3 (Lot No.IC3SCK-VK002), 3.0 x 100 mm, 3 µm; mobile phase A: supercritical carbon dioxide; mobile phase B: methanol (20 mmol/L ammonia); flow rate: 2 mL/min; isocratic elution with 10% phase B in 6 min; detector UV 220 nm; retention time: 4.514 min. *dr* > 40:1. | 636. 1 | ¹H NMR (300 MHz, DMSO-*d*₆) *δ* 8.28 - 8.20 (m, 1H), 7.83 - 7.76 (m, 1H), 7.74 - 7.67 (m, 1H), 7.42 - 7.34 (m, 2H), 7.01 (d, *J* = 2.6 Hz, 1H), 4.42 - 4.24 (m, 4H), 3.70 - 3.48 (m, 7H), 2.83 - 2.71 (m, 2H), 2.44 - 2.26 (m, 3H), 1.98 - 1.84 (m, 2H), 1.82 - 1.66 (m, 4H), 1.63 - 1.49 (m, 2H), 1.08 - 0.96 (m, 6H), 0.83 - 0.68 (m, 3H); ¹⁹F NMR (282 MHz, DMSO-*d*₆) *δ*-118.40, -119.22, -123.82. |
| **250b** | 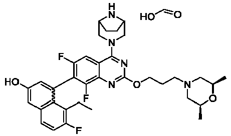 | (*R* or *S*)-4-(4-((1*R*,5*S*)-3,8-diazacyclo[ 3.2.1]octan-3-yl)-2-(3-((2*R*,6*S*)-2,6-dimethylmo rpholinyl)pr opoxy)-6,8-difluoroquin azolin-7-yl)-5-ethyl-6-fluoronapht halen-2-ol formate | NCHIRALPAK IC-3 (Lot No.IC3SCK-VK002), 3.0 x 100 mm, 3 µm; mobile phase A: supercritical carbon dioxide; mobile phase B: methanol (20 mmol/L ammonia); flow rate: 2 mL/min; isocratic elution with 10% phase B in 6 min; detector UV 220 nm; retention time: 4948 min. *dr* > 40:1. | 636. 1 | ¹H NMR (300 MHz, DMSO-*d*₆) *δ* 8.24 (s, 1H), 7.83 - 7.75 (m, 1H), 7.74 - 7.65 (m, 1H), 7.42 - 7.33 (m, 2H), 7.02 (d, *J* = 2.6 Hz, 1H), 4.42 - 4.26 (m, 4H), 3.75 - 3.61 (m, 3H), 3.61 - 3.44 (m, 4H), 2.81 - 2.71 (m, 2H), 2.44 - 2.27 (m, 3H), 1.98 - 1.83 (m, 2H), 1.82 - 1.70 (m, 4H), 1.65 - 1.50 (m, 2H), 1.08 - 0.95 (m, 6H), 0.83 - 0.69 (m, 3H); ¹⁹F NMR (282 MHz, DMSO-*d*₆) *δ*-118.30, -119.23, -123.78. |
| **271a** | 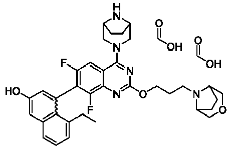 | 4-((7*S* or 7*R*)-2-(3-(3-oxa-8-azabicyclo[ 3.2.1]octan-8-yl)propoxy) -4-((1*R*,5*S*)-3,8-diazabicycl o[3.2.1]octa n-3-yl)-6,8-difluoroquin azolin-7-yl)-5-ethylnaphth alen-2-ol diformate | CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.822 min. *dr* > 40:1. | 616. 3 | ¹H NMR (300 MHz, DMSO-*d*₆) *δ* 8.35 - 8.31 (m, 2H), 7.81 - 7.70 (m, 2H), 7.47-7.42 (m, 1H), 7.38 (d, *J* = 2.7 Hz, 1H), 7.21 (d, *J* = 7.2 Hz, 1H), 7.03 (d, *J* = 2.7 Hz, 1H), 4.52 - 4.45 (m, 2H), 4.41 - 4.32 (m, 2H), 3.80 (s, 2H), 3.71 - 3.59 (m, 2H), 3.56 (d, *J* = 9.9 Hz, 2H), 3.47 - 3.43 (m, 2H), 3.14 (d, *J* = 4.5 Hz, 2H), 2.48 - 2.40 (m, 4H), 1.98 - 1.72 (m, 10H), 0.93 - 0.88 (m, 3H); ¹⁹F NMR (282 MHz, DMSO-*d*₆) *δ*-118.27, -124.13. |
| **271b** | 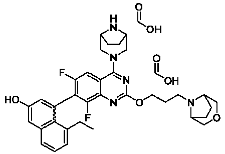 | 4-((7*R* or 7*S*)-2-(3-(3-oxa-8-azabicyclo[ 3.2.1]octan-8-yl)propoxy) -4-((1*R*,5*S*)-3,8-diazabicycl o[3.2.1]octa n-3-yl)-6,8-difluoroquin azolin-7-yl)-5-ethylnaphth alen-2-ol diformate | CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.296 min. *dr* > 40:1. | 616. 3 | ¹H NMR (400 MHz, DMSO-*d*₆) *δ* 8.25 - 8.18 (m, 2H), 7.72 - 7.63 (m, 2H), 7.42 - 7.33 (m, 1H), 7.30 (d, *J* = 2.8 Hz, 1H), 7.19 -7.10 (m, 1H), 6.95 (d, *J* = 2.8 Hz, 1H), 4.44 - 4.38 (m, 2H), 4.34 (d, *J* = 12.4 Hz, 1H), 4.26 (d, *J* = 12.4 Hz, 1H), 3.71 - 3.64 (m, 2H), 3.61 - 3.55 (m, 1H), 3.52 - 3.48 (m, 3H), 3.40 - 3.36 (m, 2H), 3.06 (d, *J* = 4.0 Hz, 2H), 2.40 - 2.34 (m, 4H), 1.89 - 1.81 (m, 4H), 1.77 - 1.65 (m, 6H), 0.85 - 0.82 (m, 3H); ¹⁹F NMR (282 MHz, DMSO-*d*₆) *δ*-118.47, -124.18. |
| **272a** | 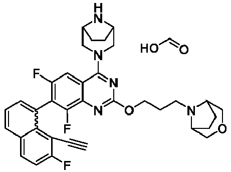 | 8-(3-((*S* or *R*)-4-((1*R*,5*S*)-3,8-diazabicycl o[3.2.1]octa n-3-yl)-7-(8-ethynyl-7-fluoronapht halen-1-yl)-6,8-difluoroquin azolin-2-yl)oxy)prop yl)-3-oxa-8-azabicyclo[ 3.2.1]octane formate | CHIRALART Cellulose-SB (Ser.No.105C A80166), 4.6 x 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 2.055 min. *dr* > 40:1. | 614. 3 | ¹H NMR (400 MHz, DMSO-*d*₆) *δ* 8.26 - 8.20 (m, 3H), 7.72 - 7.68 (m, 1H), 7.65 - 7.60 (m, 2H), 7.58 - 7.55 (m, 1H), 4.43 - 4.37 (m, 2H), 4.31 - 4.23 (m, 2H), 4.06 (d, *J* = 1.2 Hz, 1H), 3.66 (s, 2H), 3.59 (d, *J* = 12.4 Hz, 1H), 3.52 - 3.48 (m, 3H), 3.40 - 3.37 (m, 2H), 3.05 (s, 2H), 2.38 - 2.35 (m, 2H), 1.89 - 1.79 (m, 5H), 1.76 - 1.66 (m, 5H); ¹⁹F NMR (377 MHz, DMSO-*d*₆) *δ-*105.14, -118.76, - 118.79, -124.58, - 124.59. |
| **272 b** | 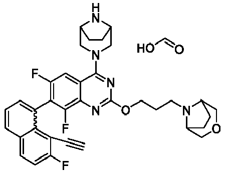 | 8-(3-((R or S)-4-((1*R*,5*S*)-3,8-diazabicycl o[3.2.1]octa n-3-yl)-7-(8-ethynyl-7-fluoronapht halen-1-yl)-6,8-difluoroquin azolin-2-yl)oxy)prop yl)-3-oxa-8-azabicyclo[ 3.2.1]octane formate | CHIRALART Cellulose-SB (Ser.No.105C A80166), 4.6 x 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 1.632 min. *dr >* 40:1. | 614. 3 | ¹H NMR (400 MHz, DMSO-*d*₆) *δ* 8.26 - 8.20 (m, 3H), 7.72 - 7.68 (m, 1H), 7.65 - 7.60 (m, 2H), 7.58 - 7.55 (m, 1H), 4.43 - 4.37 (m, 2H), 4.31 - 4.23 (m, 2H), 4.06 (d, *J* = 1.2 Hz, 1H), 3.66 (s, 2H), 3.59 (d, *J* = 12.4 Hz, 1H), 3.52 - 3.48 (m, 3H), 3.40 - 3.37 (m, 2H), 3.05 (s, 2H), 2.38 - 2.35 (m, 2H), 1.89 - 1.79 (m, 5H), 1.76 - 1.66 (m, 5H); ¹⁹F NMR (377 MHz, DMSO-*d*₆) *δ-*105.13, -118.75, - 118.76, -124.56, - 124.58. |
| **273a** | 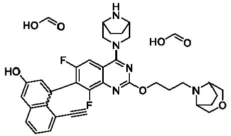 | 4-((7*S* or 7*R*)-2-(3-(3-oxa-8-azabicyclo[3.2.1]octan-8-yl)propoxy) -4-((1*R*,5*S*)-3,8-diazabicycl o[3.2.1]octa n-3-yl)-6,8-difluoroquin azolin-7-yl)-5-ethynylnaph thalen-2-ol diformate | CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.878 min. *dr* > 40:1. | 612. 3 | ¹H NMR (300 MHz, DMSO-*d*₆) *δ* 8.37 (s, 2H), 7.97 - 7.94 (m, 1H), 7.61 - 7.48 (m, 3H), 7.44 (d, *J* = 2.7 Hz, 1H), 7.16 (d, *J* = 2.7 Hz, 1H), 4.47 - 4.43 (m, 2H), 4.37 (d, *J* = 13.2 Hz, 2H), 3.97 (s, 2H), 3.69 - 3.59 (m, 5H), 3.48 (d, *J* = 10.5 Hz, 2H), 3.22 (s, 2H), 2.54 - 2.48 (m, 2H), 2.01 - 1.88 (m, 8H), 1.80-1.74 (m, 2H); ¹⁹F NMR (377 MHz, DMSO-*d*₆) *δ*-118.84, -124.78. |
| **273 b** | 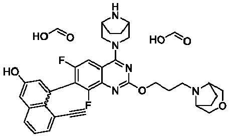 | 4-((7*R* or 7*S*)-2-(3-(3-oxa-8-azabicyclo[ 3.2.1]octan-8-yl)propoxy) -4-((1*R*,5*S*)-3,8-diazabicycl o[3.2.1]octa n-3-yl)-6,8-difluoroquin azolin-7-yl)-5-ethynylnaph thalen-2-ol diformate | CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.217 min. *dr* > 40:1. | 612. 3 | ¹H NMR (300 MHz, DMSO-*d*₆) *δ* 8.37 (s, 2H), 7.98 - 7.95 (m, 1H), 7.63 - 7.47 (m, 3H), 7.43 (d, *J* = 2.4 Hz, 1H), 7.16 (d, *J* = 2.4 Hz, 1H), 4.49 - 4.45 (m, 2H), 4.34 (d, J = 13.2 Hz, 2H), 3.75 (s, 2H), 3.66 - 3.56 (m, 5H), 3.45 (d, J = 10.5 Hz, 2H), 3.13 (s, 2H), 2.47 - 2.42 (m, 2H), 1.97 - 1.88 (m, 5H), 1.85-1.74 (m, 5H); ¹⁹F NMR (282 MHz, DMSO-*d*₆) *δ*-118.70, -124.73. |

### Embodiment 55

### 4-((S or R)-2-((R)-3-((1R,3R,5R,7R)-2-oxa-6-azadamantan-6-yl)-2-methylpropoxy)-4-((1R,5S)-3,8-diazabicyclo[3.2.1]octan-3-yl)-6-chloro-8-fluoroquinazolin-7-yl)naphthalen-2-ol diformate, 230

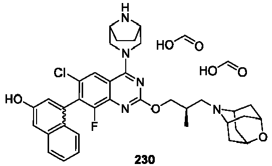

The synthetic route was as follows:

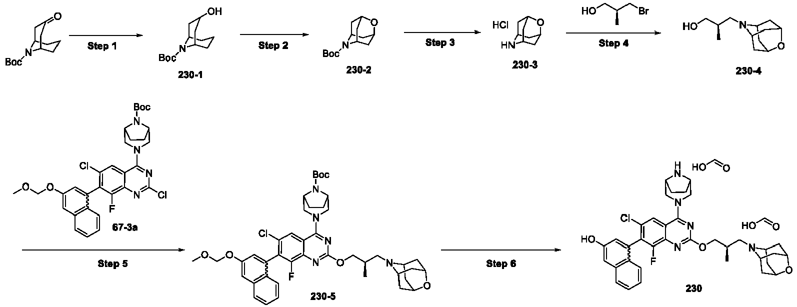

### Step 1

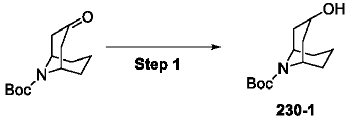

Sodium borohydride (1.52 g, 38.108 mmol, 1.2 eq) was added to a solution of *tert-*butyl 3-oxo-9-azabicyclo[3.3.1]nonane-9-carboxylate (8 g, 31.757 mmol, 1.00 eq) in methanol (100 mL) with stirring at 0 °C. The obtained mixture was stirred at 25°C for 3 hours. The reaction process was monitored by liquid chromatography-mass spectrometry and thin layer chromatography. After the reaction was completed, the reaction was quenched by adding saturated ammonium chloride solution (80 mL) to the system at 25 °C, then the methanol was removed by concentration under reduced pressure. The aqueous phase was extracted with dichloromethane (200 mL × 3), and the organic phases were combined, the organic phase was dried over anhydrous sodium sulfate, filtered, and the filtrate was concentrated to obtain a crude product. The crude product was purified by silica gel column chromatography, then eluted with a gradient of 0% to 50% ethyl acetate/petroleum ether mobile phase, and the obtained fraction was evaporated under reduced pressure to remove the solvent to obtain compound **230-1** (white solid, 7.8 g, yield: 96%); MS (ESI, M/Z): 242.2[M + H]⁺; 1HNMR (300 MHz, CDCl3) *δ* 4.57-4.30 (m, 2H), 3.78-3.60 (m, 1H), 2.41-2.26 (m, 2H), 2.19-1.96 (m, 1H), 1.69-1.54 (m, 4H), 1.49-1.24 (m, 12H).

### Step 2:

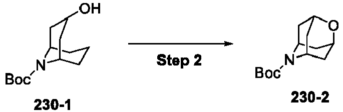

Calcium carbonate (9.62 g, 91.327 mmol, 5.8 eq) was added to a solution of lead tetraacetate (19.11 g, 40.940 mmol, 2.60 eq) in benzene (100 mL) with stirring under the protection of nitrogen at 25 °C. The reaction was carried out at 80 °C for 15 min, compound **230-1** (4 g, 15.746 mmol, 1.00 eq) and iodine (8.41 g, 31.492 mmol, 2 eq) were added to the reaction solution. The reaction was continued for 3 hours at 8 °C, and the reaction process was monitored by liquid chromatography-mass spectrometry and thin layer chromatography. After the reaction was completed, the mixture was filtered to remove the insolubles, the filter cake was washed with ethyl acetate (80 mL × 3) and the filtrate was washed with saturated sodium sulfite (200 mL × 2) solution. The organic phase was concentrated under reduced pressure to obtain a crude product. The crude product was purified by a reversed-phase rapid chromatographic column (C18 column) and eluted with 10%→ 50% acetonitrile/water mobile phase (0.05% trifluoroacetic acid) in 35 min; detector, UV200/210 nm; a crude product was obtained. The crude product was purified by silica gel column chromatography, eluted with a gradient of 0%→ 40% ethyl acetate/petroleum ether mobile phase, and the obtained fractions were evaporated under reduced pressure to remove the solvent to obtain compound **230-2** (white solid, 2.5 g, 63%). MS (ESI, m/z): 240.2[M + H]⁺; ¹H NMR (400 MHz, CDCl3) *δ* 4.54 - 4.38 (m, 2H), 4.22 - 4.14 (m, 2H), 2.09 - 2.01 (m, 4H), 1.89 - 1.69 (m, 4H), 1.47 (s, 9H).

### Step 3:

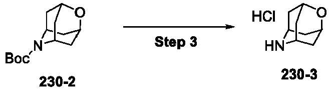

A solution of hydrochloric acid (4 mol/L, 2 mL) in 1,4-dioxane was slowly added to a solution of compound **230-2** (200 mg, 0.794 mmol, 1 eq) in methanol (2 mL) with stirring at 0 °C. The obtained mixture was stirred for 1 hour at 25 °C, and the reaction process was monitored by liquid chromatography-mass spectrometry and thin layer chromatography. After the reaction was completed, the reaction solution was concentrated under reduced pressure to obtain a crude product of compound **230-2** (white solid, 145 mg, yield: 98%). The crude product was used directly in the next reaction without further purification. MS (ESI, m/z): 140.1[M + H]⁺.

### Step 4

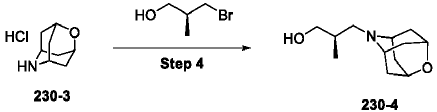

Potassium carbonate (456.33 mg, 3.136 mmol, 4 eq) was added in batches to a solution of compound **230-3** (145 mg, 0.784 mmol, 1 eq) and (2*S*)-3-bromo-2-methyl-1-propanol (138.94 mg, 0.862 mmol, 1.1 eq) in acetonitrile (3 mL) with stirring at 25 °C. The obtained reaction was carried out with stirring for 3 hours at -6 °C, and the reaction process was monitored by liquid chromatography-mass spectrometry and thin layer chromatography. After the reaction was completed, the mixture was filtered to remove the insolubles and the filter cake was washed with dichloromethane (10 mL × 3). The filtrate was concentrated under reduced pressure to obtain a crude product. The crude product was purified by silica gel column chromatography, eluted with a gradient of 0%→5% methanol (7mol/L of ammonia) /dichloromethane mobile phase, and the obtained fractions were evaporated under reduced pressure to remove the solvent to obtain compound **230-4** (colorless oil, 90 mg, 51%). MS (ESI, m/z): 212.1[M + H]⁺; ¹H NMR (400 MHz, CDCl3) *δ* 6.78 (s, 1H), 4.17 - 3.93 (m, 2H), 3.82 - 3.35 (m, 2H), 3.27 - 2.97 (m, 3H), 2.45 - 2.26 (m, 1H), 2.01 - 1.75 (m, 9H), 0.82 - 0.65 (m, 3H).

### Step 5

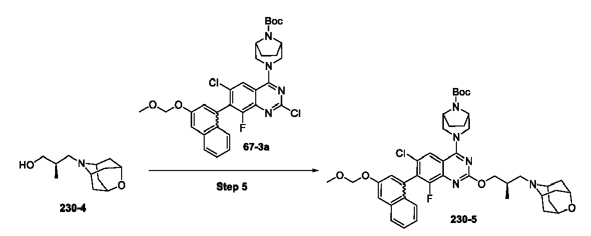

A solution of potassium *tert*-butoxide (1 mol/L, 0.27 mL, 0.27 mmol, 1.5 eq) in tetrahydrofuran was added dropwise to a solution of compound **67-3a** (120 mg, 0.185 mmol, 1 eq) and **230-4** (45.46 mg, 0.204 mmol, 1.1 eq) in tetrahydrofuran (2.5 mL) with stirring under the protection of nitrogen at 0 °C. The obtained reaction was carried out with stirring for 2 hours at 0 °C, and the reaction process was monitored by liquid chromatography-mass spectrometry and thin layer chromatography. After the reaction was completed, water (20 mL) was added to the system to quench the reaction. The aqueous phase was extracted with ethyl acetate (20 mL × 3), the organic phases were combined, the organic phase was dried over anhydrous sodium sulfate, filtered, and the filtrate was concentrated to obtain a crude product. The crude product was purified by column chromatography and eluted with methanol/dichloromethane (1/15) mobile phase to obtain compound **230-5** (white solid, 80 mg, yield: 51%). MS (ESI, m/z): 788.2/790.2[M + H]⁺.

### Step 6

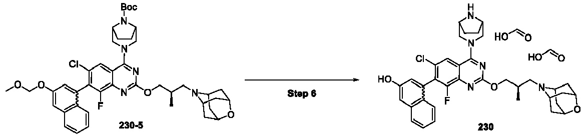

A solution of hydrogen chloride (4 mol/L, 2 mL) in 1,4-dioxane was slowly added to a solution of compound **230-5** (70 mg, 0.088 mmol, 1 eq) in methanol (2 mL) with stirring at 0 °C. The reaction was carried out for 2 hours at 25 °C, and the reaction process was monitored by liquid chromatography-mass spectrometry and thin layer chromatography. After the reaction was completed, the reaction solution was concentrated under reduced pressure to obtain a crude product, the crude product was purified by reversed-phase chromatography (C18 column) and eluted with 5%→ 95% (acetonitrile/methanol, 1/1)/water mobile phase (0.1% formic acid) in 30 min; detector, UV254/220 nm; compound **230** was obtained (white solid, 35.8 mg, yield: 53%). MS (ESI, m/z): 644.2/646.2[M + H]⁺; ¹H NMR (300 MHz, DMSO-*d*₆) *δ* 8.30 - 8.18 (m, 2H), 7.94 (d, *J* = 1.6 Hz, 1H), 7.81 (d, *J* = 8.3 Hz, 1H), 7.49 - 7.40 (m, 1H), 7.29 (d, *J* = 2.4 Hz, 1H), 7.22 (d, *J* = 4.0 Hz, 2H), 7.06 (d, *J* = 2.3 Hz, 1H), 4.47 - 4.32 (m, 3H), 4.21 -4.11 (m, 1H), 4.01 - 3.94 (m, 2H), 3.78 - 3.55 (m, 4H), 2.96 - 2.87 (m, 2H), 2.61 (d, *J* = 7.2 Hz, 2H), 2.13 - 1.99 (m, 1H), 1.87 - 1.66 (m, 12H), 0.98 (d, *J* = 6.7 Hz, 3H); ¹⁹F NMR (282 MHz, DMSO-*d*₆) *δ*-122.39.

### Embodiment 56

### 4-(2-(3-(3-oxa-8-azabicyclo[3.2.1]octan-8-yl)propoxy)-4-((1R,5S)-3,8-diazabicyclo[3.2.1]octan-3-yl)-5-methyl-5H-pyrrolo[3,2-d]pyrimidin-7-yl)naphthalen-2-ol formate, 231

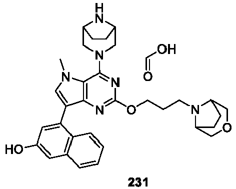

The synthetic route was as follows:

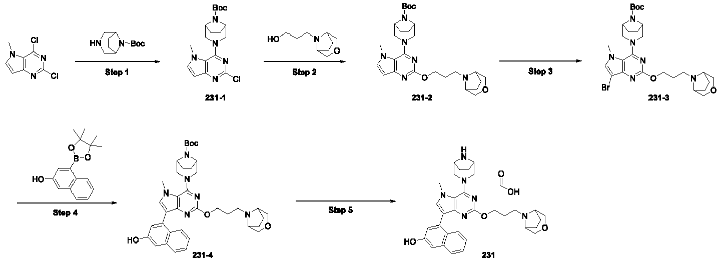

### Step 1

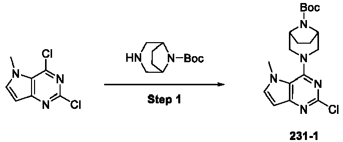

2,4-Dichloro-5-methyl-5*H*-pyrrolido[3.2-d]pyrimidine (1.0 g, 4.7 mmol, 1.0 eq), *N, N*-dimethylformamide (10.0 mL) and *tert*-butyl 3,8-diazabicyclo[3.2.1]octane-8-carboxylate (1.1 g, 4.7 mmol, 1.0 eq) were successively added to a 50 mL round-bottom flask with stirring at 25 °C. Then *N,N-*diisopropylethylamine (1.3 g, 9.4 mmol, 2.0 eq) was added dropwise to the reaction solution at 25 °C. The mixture was stirred at 25°C for 16 hours. The reaction process was monitored by liquid chromatography-mass spectrometry and thin layer chromatography. After the reaction was completed, the reaction solution was directly purified by a reversed-phase rapid chromatographic column (C18 column), and eluted with 0%→ 95% methanol/water mobile phase (0.1% ammonium bicarbonate) in 25 min; detector, UV254 nm; compound **231-1** (yellow oil, 1.5 g, yield: 85%) was obtained. MS (ESI, m/z): 378.1/380.1[M + H]⁺; ¹H NMR (400 MHz, CDCl3) *δ* 7.21 (d, *J* = 3.2 Hz, 1H), 6.52 (d, *J* = 3.2 Hz, 1H), 4.33 - 4.31 (m, 2H), 3.99 - 3.88 (m, 5H), 3.51 - 3.42 (m, 2H), 2.01 - 1.81 (m, 4H), 1.50 (s, 9H).

### Step 2

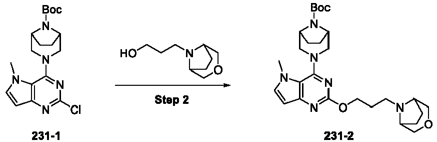

Potassium *tert*-butoxide (156.0 mg, 1.3 mmol, 1.5 eq) was slowly added to a solution of compound **231-1** (350.0 mg, 0.9 mmol, 1.0 eq) and 3-(3-oxo-8-azabicyclo[3.2.1]octan-8-yl) propyl-1-ol (191.0 mg, 1.1 mmol, 1.2 eq) in tetrahydrofuran (6.0 mL) with stirring at 25 °C under the protection of nitrogen. The obtained reaction was carried out for 16 hours at -6 °C, and the reaction process was monitored by liquid chromatography-mass spectrometry and thin layer chromatography. After the reaction was completed, the reaction solution was directly purified by a reversed-phase rapid chromatographic column (C18 column), and eluted with 0%→ 40% acetonitrile/water mobile phase (0.1% trifluoroacetic acid) in 25 min; detector, UV254 nm; compound **231-2** (light white solid, 400.0 mg, yield: 89%) was obtained. MS (ESI, m/z): 513.3[M + H]⁺.

### Step 3

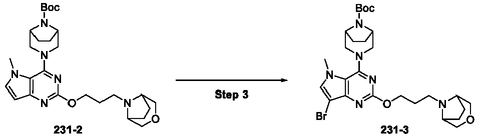

*N-*Bromosuccinimide (113.0 mg, 0.6 mmol, 0.9 eq) was added to a solution of compound **231-2** (360.0 mg, 0.7 mmol, 1.0 eq) in dichloromethane (10.0 mL) with stirring at 25 °C. The obtained mixture was stirred for 1 hour at 25 °C, and the reaction process was monitored by liquid chromatography-mass spectrometry and thin layer chromatography. After the reaction was completed, the mixture was concentrated under reduced pressure, the obtained crude product was purified by reversed-phase rapid chromatographic column (C18 column), and eluted with 0%→ 50% acetonitrile/water mobile phase (0.1% formic acid) in 25 min; detector, UV254 nm; compound **231-3** (red solid, 220.0 mg, yield: 56%) was obtained. MS (ESI, *m*/*z*): 591.2/593.2[M + H]⁺.

### Step 4

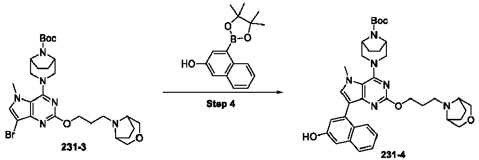

4-(4,4,5,5-Tetramethyl-1,3,2-dioxaborolan-2-yl)naphthalen-2-ol (37.7 mg, 0.13 mmol, 1.5 eq), potassium carbonate (19.7 mg, 0.18 mmol, 2.0 eq) and 1,1'-bis(diphenylphosphino)ferrocene-palladium(II)dichloride (7.6 mg, 0.009 mmol, 0.1 eq) were added to a solution of compound **231-3** (55.0 mg, 0.09 mmol, 1.0 eq) in 1,4-dioxane/water (5/1, 1.0 mL) with stirring under the protection of nitrogen at 25 °C. The reaction was carried out with stirring for 2 hours at 80 °C, and the reaction process was monitored by liquid chromatography-mass spectrometry and thin layer chromatography. After the reaction was completed, the reaction mixture was cooled to 25 °C. The reaction solution was concentrated and purified by silica gel column chromatography, eluted with a gradient of 0%→ 10% methanol/dichloromethane mobile phase, the obtained fraction was evaporated under reduced pressure to remove the solvent and to obtain compound **231-4** (brown solid, 30.0 mg, yield: 52%). MS (ESI, m/z): 655.3[M + H]⁺.

### Step 5

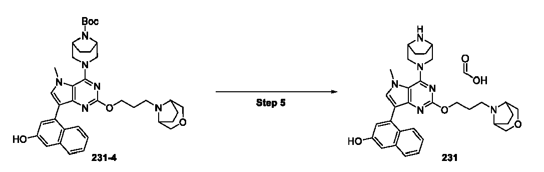

Trifluoroacetic acid (1.0 mL) was added to a solution of compound **231-4** (30.0 mg, 0.05 mmol, 1 eq) in dichloromethane (3.0 mL) with stirring at 25 °C. The obtained mixture was stirred for 1 hour at 25 °C, and the reaction process was monitored by liquid chromatography-mass spectrometry and thin layer chromatography. After the reaction was completed, the mixture was concentrated under reduced pressure to remove the solvent and trifluoroacetic acid, the obtained crude product was purified by reversed-phase rapid chromatographic column (C18 column), and eluted with 0%→ 40% acetonitrile/water mobile phase (0.1% formic acid) in 25 min; detector, UV254 nm; compound **231** (red solid, 5.5 mg, yield: 21%) was obtained. MS (ESI, *m*/*z*): 555.2[M + H]⁺; ¹H NMR (400 MHz, DMSO-*d*₆) *δ* 9.73 (s, 1H), 8.17 (s, 1H), 7.88 - 7.79 (m, 2H), 7.72 (d, *J* = 8.2 Hz, 1H), 7.42 - 7.34 (m, 1H), 7.26 - 7.15 (m, 2H), 7.11 (d, *J* = 2.5 Hz, 1H), 4.24 - 4.16 (m, 2H), 4.07 - 3.99 (m, 5H), 3.81 (d, *J* = 12.8 Hz, 2H), 3.48 - 3.45 (m, 2H), 3.45 - 3.42 (m, 2H), 3.36 - 3.33 (m, 2H), 3.03 - 2.97 (m, 2H), 2.34 - 2.27 (m, 2H), 2.14 - 2.06 (m, 2H), 1.98 - 1.90 (m, 2H), 1.82 - 1.72 (m, 4H), 1.70 - 1.61 (m, 2H).

### Embodiment 57

### ((3S or 3R, 7aS or 7aR)-7a-((4-((1R,5S)-3,8-diazabicyclo[3.2.1]octan-3-yl)-6-chloro-8-fluoro-7-(3-hydroxynaphthalen-1-yl)quinazolin-2-yl)oxy)methyl)hexahydro-1H-pyrrolin-3-yl)dimethylcarbamate tristrifluoroacetate, 232a; ((3R or 3S, 7aR or 7aS)-7a-((4-((1R,5S)-3,8-diazabicyclo[3.2.1]octan-3-yl)-6-chloro-8-fluoro-7-(3-hydroxynaphthalen-1-yl)quinazolin-2-yl)oxy)methyl)hexahydro-1H-pyrrolidin-3-yl)dimethylcarbamate tristrifluoroacetate, 232b

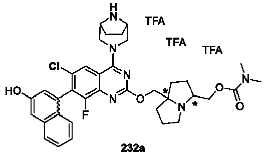

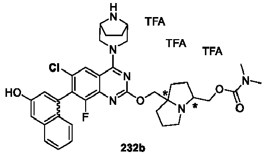

The synthetic route was as follows:

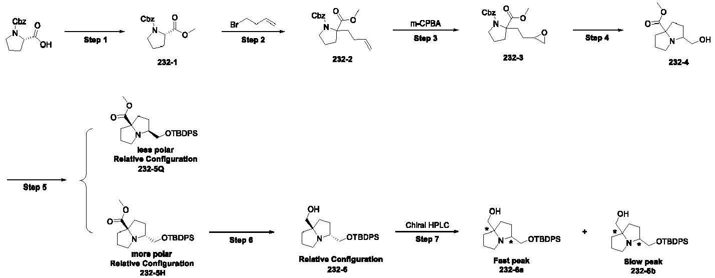

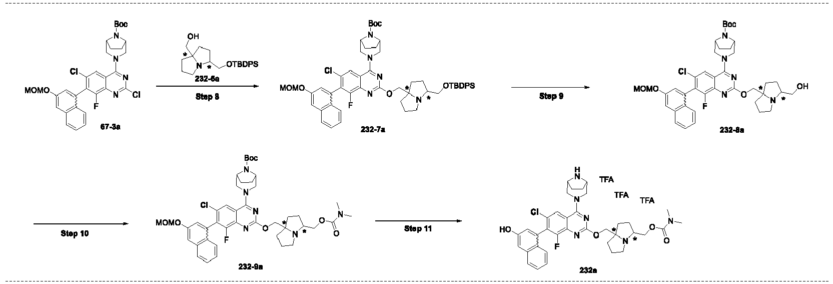

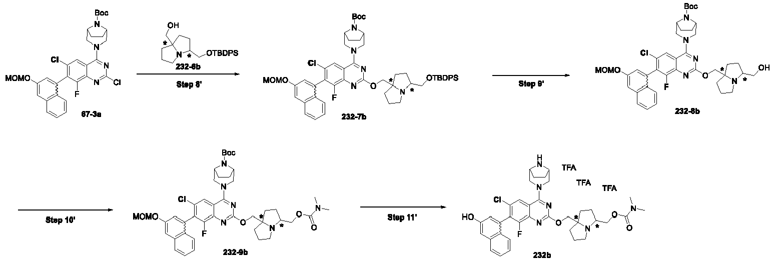

### Step 1

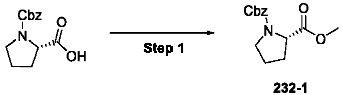

Thionyl chloride (27.48 g, 228.67 mmol, 3.0 eq) was added dropwise to a solution of *N*-benzyloxycarbonyl-*L*-proline (20 g, 76.22 mmol, 1.0 eq) in methanol (200.0 mL) with stirring under the protection of nitrogen at 0 °C. The reaction was carried out with stirring for 2 hours at 0 °C, and the reaction process was monitored by liquid chromatography-mass spectrometry and thin layer chromatography. After the reaction was completed, the filtrate was concentrated under reduced pressure to obtain a crude product. The crude product was purified by silica gel column chromatography, eluted with a gradient of 0%→ 30% ethyl acetate/petroleum ether mobile phase, and the obtained fractions were evaporated under reduced pressure to remove the solvent to obtain compound **232-1** (colorless oil, 20 g, yield: 94%). MS (ESI, m/z): 264.3[M + H]⁺; ¹H NMR (300 MHz, CDCl3) *δ* 7.40 - 7.28 (m, 5H), 5.22 - 5.00 (m, 2H), 4.41 - 4.33 (m, 1H), 3.80 - 3.41 (m, 5H), 2.31 - 2.13 (m, 1H), 2.09 -1.73 (m, 3H).

### Step 2

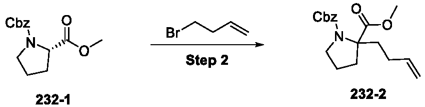

Bis-trimethylsilylamino lithium (1 mol/L, 144 mL, 2.0 eq) and 4-bromo-1-butene (12.3 g, 80.604 mmol, 1.2 eq) were successively added to a solution of compound **232-1** (20 g, 72.17 mmol, 1.0 eq) in anhydrous tetrahydrofuran (200.0 mL) with stirring under the protection of nitrogen at -78 °C. The reaction was carried out for 16 hours at 25 °C, and the reaction process was monitored by liquid chromatography-mass spectrometry and thin layer chromatography. After the reaction was completed, 300.0 mL of water was added to the reaction solution to quench the reaction, and then the mixture was extracted with ethyl acetate (300 mL × 3), the organic phases were combined, and washed with saturated brine (80 mL × 3), dried over sodium sulfate, filtered, the filtrate was concentrated to obtain a crude product. The crude product was purified by silica gel column chromatography, eluted with a gradient of 0%→ 30% ethyl acetate/petroleum ether mobile phase, and the obtained fractions were evaporated under reduced pressure to remove the solvent to obtain compound **232-2** (colorless oil, 17 g, yield: 69%). MS (ESI, m/z): 318.1[M + H]⁺; ¹H NMR (400 MHz, CDCl3) *δ* 7.42 - 7.29 (m, 5H), 5.90 - 5.67 (m, 1H), 5.21 - 4.90 (m, 4H), 3.88 - 3.67 (m, 3H), 3.57 - 3.44 (m, 2H), 2.53 - 2.19 (m, 1H), 2.18 - 1.79 (m, 7H).

### Step 3

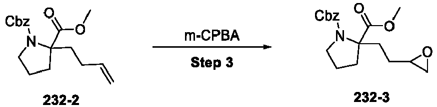

*m*-Chloroperoxybenzoic acid (80% content, 27.43 g, 127.21 mmol, 2.5 eq) was added to a solution of compound **232-2** (17 g, 50.884 mmol, 1.0 eq) in dichloromethane (200.0 mL) with stirring under the protection of nitrogen at 25 °C. The mixture was stirred for 5 hours at 25 °C and the reaction process was monitored by thin layer chromatography (ethyl acetate/petroleum ether = 1/4, R_{f} = 0.2). After the reaction was completed, 300 mL of saturated sodium thiosulfate aqueous solution was added to the reaction solution to quench the reaction, then the mixture was extracted with dichloromethane (300 mL × 3), the organic phases were combined and dried over anhydrous sodium sulfate, filtered, and the filtrate was concentrated to obtain a crude product. The crude product was purified by silica gel column chromatography, eluted with a gradient of 0%→ 50% ethyl acetate/petroleum ether mobile phase, and the obtained fractions were evaporated under reduced pressure to remove the solvent to obtain compound **232-3** (brownish yellow oil, 12 g, yield: 66%). MS (ESI, m/z): 334.1[M + H]⁺; ¹H NMR (300 MHz, CDCl3) *δ* 7.40 - 7.31 (m, 5H), 5.20 - 5.06 (m, 2H), 3.86 - 3.71 (m, 3H), 3.55 - 3.44 (m, 2H), 2.98 - 2.68 (m, 2H), 2.56 - 2.23 (m, 2H), 2.19 - 1.81 (m, 5H), 1.72 - 1.35 (m, 2H).

### Step 4

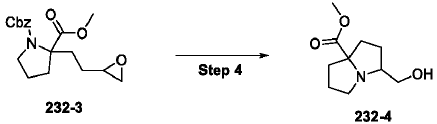

Palladium carbon (10% palladium content, 1.2 g) was added to a solution of compound **232-3** (12 g,34.195 mmol, 1.0 eq) in methanol (120.0 mL) with stirring under the protection of nitrogen at 25 °C. The nitrogen gas was ventilated with hydrogen gas (1.5 atmospheric pressures) by a displacement gas operation. The mixture was stirred for 5 hours at 25 °C under hydrogen atmosphere, and the reaction process was monitored by liquid chromatography-mass spectrometry and thin layer chromatography. After the reaction was completed, the mixture was filtered to remove the insolubles, the filter cake was washed with 100 mL of methanol and the filtrate was concentrated under reduced pressure to remove the solvent to obtain compound **212-4.** (Colorless oil, 7 g, yield: 97%). MS (ESI, m/z): 200.1[M + H]⁺.

### Step 5

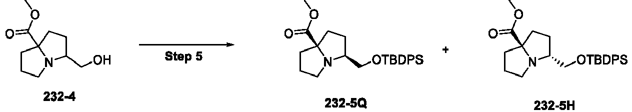

tert-Butyldiphenylchlorosilane (19.31 g, 66.75 mmol, 2.0 eq) was slowly added to a solution of compound **232-4** (7 g, 33.375 mmol, 1.0 eq) and imidazole (9.09 g, 126.845 mmol, 3.8 eq) in *N*, *N*-dimethylformamide (70.0 mL) with stirring under the protection of nitrogen at 0 °C. The mixture was stirred for 16 hours at 25 °C and the reaction process was monitored by thin layer chromatography (ethyl acetate/petroleum ether = 1/3, R_{f} = 0.5, 0.2). After the reaction was completed, 500 mL of water was added to the reaction solution to quench the reaction, and then the mixture was extracted with ethyl acetate (500 mL × 3), the organic phases were combined, and washed with saturated brine (200 mL × 3), dried over sodium sulfate, filtered, the filtrate was concentrated to obtain a crude product. The crude product was purified by silica gel column chromatography, elution with a gradient of 0% to 40% ethyl acetate/petroleum ether mobile phase, the fraction was evaporated under reduced pressure to remove the solvent to obtain compound **232-5Q** (colorless oil, 6 g, yield: 38%) and compound **232-5H** (colorless oil, 5.7 g, yield: 37%). **Compound 232-5H:** MS (ESI, m/z): 438.4[M + H]⁺; ¹H NMR (300 MHz, CDCl3) *δ* 7.74 - 7.63 (m, 4H), 7.51 - 7.35 (m, 6H), 4.03 - 3.96 (m, 1H), 3.88 - 3.81 (m, 1H), 3.74 (s, 3H), 3.46 - 3.35 (m, 1H), 3.01 - 2.79 (m, 2H), 2.55 - 2.45 (m, 1H), 2.30 - 2.15 (m, 1H), 2.04 - 1.75 (m, 5H), 1.70 - 1.53 (m, 1H), 1.07 (s, 9H).

### Step 6

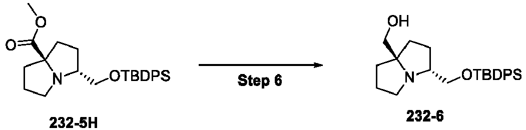

Compound **232-5H** (5.6 g, 12.156 mmol, 1.0 eq) was slowly added to a solution of lithium aluminum hydride (1.46 g, 36.468 mmol, 3.0 eq) in tetrahydrofuran (56 mL) with stirring under the protection of nitrogen at 0 °C. The reaction was carried out with stirring for 1 hour at -2 °C, and the reaction process was monitored by liquid chromatography-mass spectrometry and thin layer chromatography. After the reaction was completed, water (1.46 mL), 15% sodium hydroxide solution (1.46 mL) and water (5.38 mL) were successively added to the reaction solution with stirring at 0 °C. The mixture was filtered to remove the insolubles and the filter cake was washed with tetrahydrofuran (20 mL × 3), the filtrate was concentrated under reduced pressure to remove the solvent to obtain compound **232-6** (off-white solid, 5.0 g, yield: 95%). MS (ESI, m/z): 410.3[M + H]⁺; ¹H NMR (400 MHz, CDCl3) *δ* 7.74 - 7.64 (m, 4H), 7.49 - 7.33 (m, 6H), 3.97 - 3.89 (m, 1H), 3.79 - 3.72 (m, 1H), 3.32 - 3.22 (m, 2H), 3.22 - 3.13 (m, 1H), 2.86 - 2.79 (m, 1H), 2.74 - 2.65 (m, 1H), 1.99 - 1.91 (m, 1H), 1.82 - 1.57 (m, 6H), 1.56 - 1.47 (m, 1H), 1.06 (s, 9H).

### Step 7

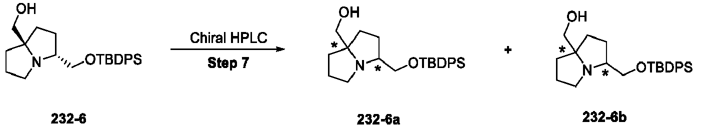

Compound **232-6** (5 g) obtained in step 6 was subjected to chiral resolution by a preparative supercritical chiral liquid chromatography: chiral column (*R*, *R*)-WHELK-O1-Kromasil, 3 × 25 cm, 5 µm; mobile phase A: supercritical carbon dioxide, mobile phase B: isopropanol/dichloromethane = 2/1 (0.1%, 2 mol/L ammonia methanol); flow rate: 70 mL/min; eluted with 55% phase B in 6.5 min, detector: UV 204 nm; two products were obtained. The product with shorter retention time (3.27 min) was compound **232-6a**, ((3*R* or 3*S*, 7a*R* or 7*aS*)-3-((*tert*-butyldiphenylsiloxy)methyl)tetrahydro-1*H-*pyrrolidin-7a (5*H*)-yl) methanol (colorless oil, 2.2 g, recovery rate: 44%), MS (ESI, m/z): 410.3 [M + H]⁺; the product with longer retention time (5.87 min) was compound **232-6b,** ((3*S* or 3*R*, 7*aS* or 7*aR*)-3-((*tert-*butyldiphenylsiloxy)methyl)tetrahydro-1*H-*pyrrolidin-7a (5*H*)-yl) methanol (colorless oil, 1.9 g, recovery rate: 38%), MS (ESI, m/z): 410.3[M + H]⁺.

### Step 8

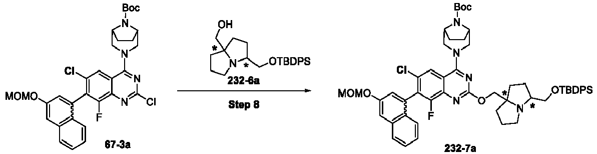

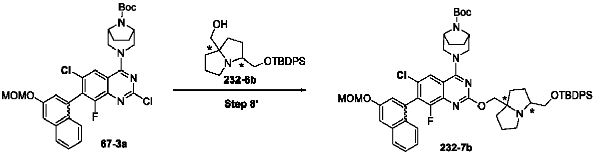

A solution of potassium *tert*-butoxide (1.0 mol/L, 2.5 mL, 2.527 mmol, 1.20 eq) in tetrahydrofuran was added dropwise to a solution of compound **67-3a** (1.36 g, 2.106 mmol, 1.00 eq) and **232-6a** (1.00 g, 2.317 mmol, 1.10 eq) in tetrahydrofuran (10 mL) with stirring under the protection of nitrogen at 0 °C. The reaction was carried out for 1 hour at 0 °C under the protection of nitrogen, and the reaction process was monitored by liquid chromatography-mass spectrometry and thin layer chromatography. After the reaction was completed, the reaction mixture was concentrated to obtain a crude product. The crude product was purified by a silica gel column, eluted with 0% to 10% methanol/dichloromethane mobile phase, and the fraction was evaporated to remove the solvent, compound **232-7a** (yellow solid, 1.09 g, yield: 49.83%) was obtained. MS (ESI, m/z): 986.3/988.3[M + H]⁺. Compound **232-7b** (light yellow solid, 1g, yield: 47%) can be prepared by the same method, MS (ESI, m/z): 986.3/988.3[M + H]⁺.

### Step 9

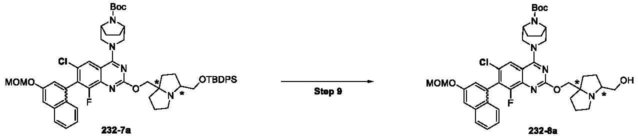

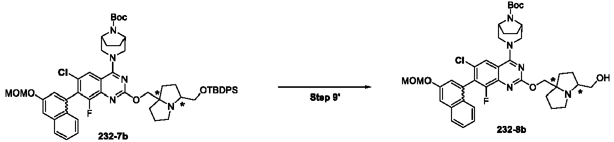

Tetrahydrofuran solution of tetrabutylammonium fluoride (1 mol/L, 5.8 mL, 6.00 eq) was added dropwise to a solution of compound **232-7a** (1.00 g, 0.963 mmol, 1.00 eq) in tetrahydrofuran (8 mL) with stirring at 25 °C. The reaction was carried out for 3 hours at 25 °C, and the reaction process was monitored by liquid chromatography-mass spectrometry and thin layer chromatography. After the reaction was completed, the reaction mixture was concentrated to obtain a crude product. The crude product was purified by a silica gel chromatographic column, eluted with 0% to 10% methanol/dichloromethane mobile phase, and the fraction was evaporated to remove the solvent, compound **232-8a** (white solid, 750 mg, yield: 98%) was obtained. MS (ESI, *m*/*z*): 748.2/750.2[M + H]⁺. Compound **232-8b** (white solid, 650mg, yield: 85%) can be prepared by the same method, MS (ESI, m/z): 748.2/750.2[M + H]⁺.

### Step 10

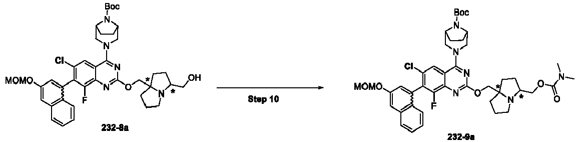

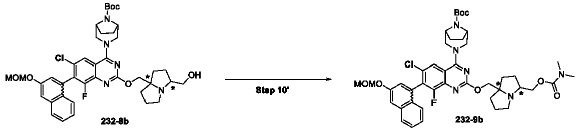

*p*-Nitrophenyl chloroformate (43.10 mg, 0.204 mmol, 2.0 eq) was slowly added to a solution of compound **232-8a** (80 mg, 0.102 mmol, 1.00 eq) and triethylamine (43.27 mg, 0.408 mmol, 4 eq) in tetrahydrofuran (1 mL) with stirring under the protection of nitrogen at 25 °C. The mixture was stirred at 25 °C for 2 hours, then dimethylamine (24.10 mg, 0.510 mmol, 5.0 eq) was added dropwise to the above mixture. The obtained mixture was stirred for 1 hour at 25 °C, and the reaction process was monitored by liquid chromatography-mass spectrometry and thin layer chromatography. After the reaction was completed, the reaction mixture was concentrated to obtain a crude product. The obtained crude product was purified by silica gel column chromatography, eluted with a gradient of 0%→5% methanol (7 mol/L ammonia)/dichloromethane mobile phase, and the obtained fractions were evaporated under reduced pressure to remove the solvent to obtain compound **232-9a** (white solid, 80 mg, yield: 91%). MS (ESI, *m*/*z*): 819.3/821.3[M + H]⁺. Compound **232-9b** (white solid, 50mg, yield: 57%) can be prepared by the same method, MS (ESI, m/z): 819.3/821.3 [M + H]⁺.

### Step 11

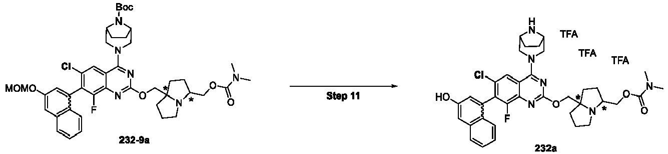

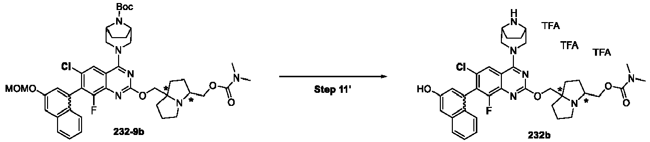

A solution of hydrochloric acid (4 mol/L, 2 mL) in 1,4-dioxane was added dropwise to a solution of the compound **232-9a** (75 mg, 0.087 mmol, 1.00 eq) in methanol (2 mL) with stirring at 25 °C. The reaction was carried out at 25 °C for 1 hour, and the reaction process was monitored by liquid chromatography-mass spectrometry and thin layer chromatography. After the reaction was completed, the reaction mixture was concentrated to obtain a crude product. The obtained crude product was purified by reversed-phase chromatographic column (C18 column), and eluted with 10%→ 50% (acetonitrile/ methanol, 1/1)/water mobile phase (0.1% trifluoroacetic acid) in 20 min; detector, UV254/220 nm; to obtain compound **232a** (yellow solid, 48.9 mg, yield: 54%). MS (ESI, *m*/*z*): 675.2/677.2[M + H]⁺; ¹H NMR (300 MHz, DMSO-*d*₆) *δ* 8.02 (d, *J* = 1.6 Hz, 1H), 7.82 (d, *J* = 8.3 Hz, 1H), 7.50 - 7.43 (m, 1H), 7.31 (d, *J* = 2.4 Hz, 1H), 7.29 - 7.13 (m, 2H), 7.06 (d, *J* = 2.4 Hz, 1H), 4.64 - 4.51 (m, 4H), 4.45 - 4.33 (m, 1H), 4.30 - 4.13 (m, 3H), 4.04 - 3.92 (m, 1H), 3.89 - 3.74 (m, 2H), 3.43 - 3.29 (m, 2H), 2.94 - 2.78 (m, 6H), 2.35 - 2.24 (m, 1H), 2.18 - 1.89 (m, 11H); ¹⁹F NMR (282 MHz, DMSO-*d*₆) *δ*-74.07, -121.87. The chiral analysis conditions of compound **232a** were as follows: chiral column CHIRALPAK IA-3, 4.6 × 50 mm, 3 µm; mobile phase A: n-hexane (0.1% diethylamine), mobile phase B: ethanol (10 mmol/L ammonia); flow rate: 1 mL/min; isocratic elution with 50% phase B in 4 min, detector UV 220 nm; retention time: 2.761 min. *dr >* 40:1.

Compound **232b** (yellow-green yellow solid, 34mg, yield: 55%) can be prepared by the same method, MS (ESI, m/z): 675.2/677.2[M + H]⁺. ¹H NMR (400 MHz, DMSO-*d*₆) *δ* 8.02 (d, *J* = 1.6 Hz, 1H), 7.83 (d, *J* = 8.3 Hz, 1H), 7.49 - 7.43 (m, 1H), 7.32 (d, *J* = 2.4 Hz, 1H), 7.29 - 7.21 (m, 1H), 7.21 - 7.16 (m, 1H), 7.06 (d, *J* = 2.4 Hz, 1H), 4.63 - 4.51 (m, 4H), 4.44 - 4.35 (m, 1H), 4.28 - 4.17 (m, 3H), 4.03 - 3.92 (m, 1H), 3.87 - 3.78 (m, 2H), 3.49 - 3.42 (m, 1H), 3.41 - 3.33 (m, 1H), 2.91 - 2.77 (m, 6H), 2.36 - 2.25 (m, 1H), 2.18 - 1.91 (m, 11H); ¹⁹F NMR (377 MHz, DMSO-*d*₆) *δ*-74.05, -121.91. The chiral analysis conditions of compound **232b** were as follows: chiral column CHIRALPAK IA-3, 4.6 × 50 mm, 3 µm; mobile phase A: n-hexane (0.1% diethylamine), mobile phase B: ethanol (10 mmol/L ammonia); flow rate: 1 mL/min; isocratic elution with 50% phase B in 4 min, detector UV 220 nm; retention time: 1.705 min. *dr* > 40:1.

### Embodiment 58

### 4-((7S or 7R)-2-((2R)-3-(3-oxa-8-azabicyclo[3.2.1]octan-8-yl)-2-methylpropoxy)-4-((1R,5S)-3,8-diazabicyclo[3.2.1]octan-3-yl)-6-chloro-8-fluoroquinazolin-7-yl)-5-ethyl-6-fluoronaphthalen-2-ol formate 233a; 4-((7R or 7S)-2-((2R)-3-(3-oxa-8-azabicyclo[3.2.1]octan-8-yl)-2-methylpropoxy)-4-((1R,5S)-3,8-diazabicyclo[3.2.1]octan-3-yl)-6-chloro-8-fluoroquinazolin-7-yl)-5-ethyl-6-fluoronaphthalen-2-ol formate 233b

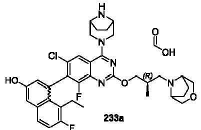

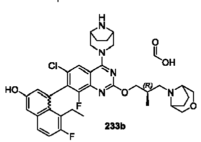

The synthetic route was as follows:

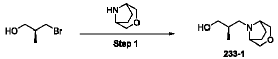

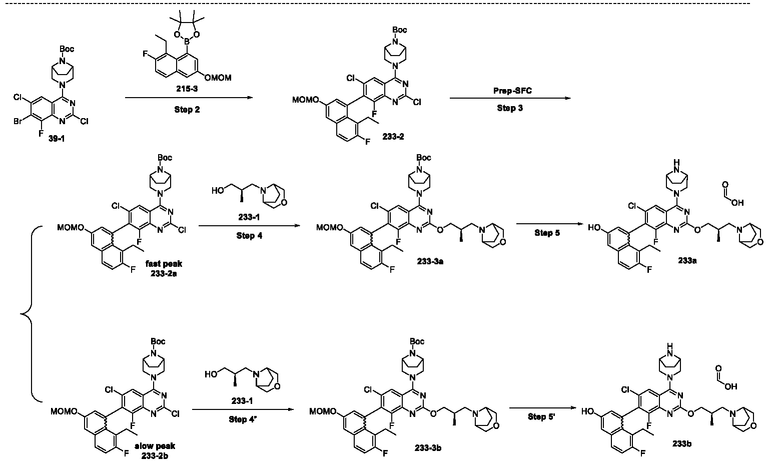

### Step 1

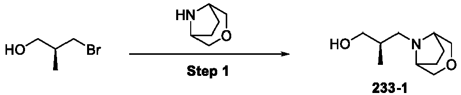

Potassium carbonate (9.03 g, 62 mmol, 2.0 eq) was added to a solution of compound 3-oxa-8-azabicyclo[3.2.1]octane (3.51 g, 31 mmol, 1.00 eq) and (*S*)-(+)-3-bromo-2-methyl-1-propanol (5 g, 31 mmol, 1.00 eq) in acetonitrile (50 mL) with stirring at 25 °C. The obtained reaction was carried out for 16 hours at 60 °C, and the reaction process was monitored by liquid chromatography-mass spectrometry and thin layer chromatography. After the reaction was completed, the reaction solution was concentrated under reduced pressure to remove acetonitrile, and then the mixture was diluted with 50 mL of water. The aqueous phase was extracted with ethyl acetate (50 mL × 3), the organic phases were combined, and the organic phase was dried over anhydrous sodium sulfate, filtered, and the filtrate was concentrated to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **233-1** (off-white solid, 4 g, yield: 66%). MS (ESI, m/z): 186.2[M + H]⁺; ¹H NMR (400 MHz, CDCl₃) *δ* 6.65 (br, 1H), 3.75 - 3.48 (m, 6H), 3.32 - 3.23 (m, 1H), 3.07 - 3.01 (m, 1H), 2.65 - 2.54 (m, 1H), 2.25 - 2.13 (m, 1H), 2.11 - 1.81 (m, 5H), 0.75 (d, *J* = 6.8 Hz, 3H).

### Step 2

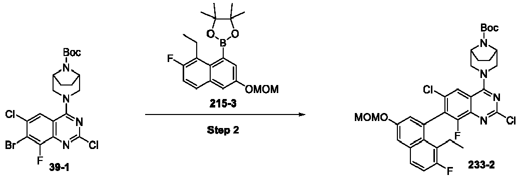

Compound **215-3** (853.96 mg, 2.25 mmol, 1.20 eq), compound **215-1** (1 g, 1.87 mmol, 1.00 eq), tris(dibenzylideneacetone)dipalladium(0) (180.90 mg, 0.188 mmol, 0.1 eq), 3-(*tert-*butyl)-4-(2,6-dimethoxyphenyl)-2,3-dihydrobenzo[*d*][1,3]oxaphosphole (146.36 mg, 0.375 mmol, 0.2 eq) and cesium carbonate (1287.32 mg, 3.75 mmol, 2 eq) were successively added to a reaction flask with stirring under the protection of nitrogen at 25 °C, then 30 mL of toluene and 6 mL of water were then added thereto, and the mixture was deoxygenated, and the reaction was carried out for 72 hours at 60 °C, and the reaction process was monitored by liquid chromatography-mass spectrometry and thin layer chromatography. After the reaction was completed, the reaction solution was concentrated under reduced pressure to obtain a crude product, then purified by silica gel column chromatography, eluted with a gradient of 0% to 25% ethyl acetate/petroleum ether mobile phase, and the obtained fraction was evaporated under reduced pressure to remove the solvent and to obtain compound **233-2** (white solid, 425 mg, yield: 33%). MS (ESI, *m*/*z*): 659.1/661.1[M + H]⁺; ¹H NMR (400 MHz, CDCl₃) *δ* 7.77 (d, *J* = 1.5 Hz, 1H), 7.73 - 7.66 (m, 1H), 7.53 (d, *J* = 2.4 Hz, 1H), 7.27 - 7.24 (m, 1H), 7.01 (d, *J* = 2.6 Hz, 1H), 5.32 - 5.26 (m, 2H), 4.58 - 4.35 (m, 4H), 3.79 - 3.58 (m, 2H), 3.53 (s, 3H), 2.71 - 2.58 (m, 1H), 2.29 - 2.18 (m, 1H), 2.06 - 1.93 (m, 2H), 1.92 - 1.76 (m, 2H), 1.53 (s, 9H), 0.82 (t, *J=* 7.4 Hz, 3H).

### Step 3

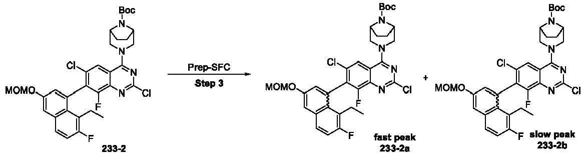

The compound **233-2** (420 mg) obtained in step 2 was subjected to chiral resolution by preparative chiral high-performance liquid chromatography: chiral column CHIRALPAK IH, 3 × 25 cm, 5 µm; mobile phase A: supercritical carbon dioxide, mobile phase B: methanol; flow rate: 60 mL/min; eluted with 30% phase B in 12 min, detector UV 224 nm, two products were obtained. The product with shorter retention time (4.60 min) was compound **233-2a**, *tert*-butyl (1*R*,5*S*)-3-((*S* or *R*)-2,6-dichloro-7-(8-ethyl-7fluoro-3-(methoxymethoxy)naphthalen-1 -yl)-8-fluoroquinazolin-4-yl)-3,8-diazabicyclo[3.2.1]octane-8-carboxylate (white solid, 160.0 mg, recovery rate: 38%), MS (ESI, m/z): 659.0/661.0[M + H]⁺; the product with longer retention time (7.85 min) was compound **233-2b**, *tert*-butyl (1*R*,5*S*)-3-((*R* or S)-2,6-dichloro-7-(8-ethy-7-fluoro-3-(methoxymethoxy)naphthalen-1 -yl)-8-fluoroquinazolin-4-yl)-3,8-diazabicyclo[3.2.1]octane-8-carboxylate (white solid, 161.0 mg, recovery rate: 38%), MS (ESI, m/z): 659.0/661.0[M + H]⁺.

### Step 4

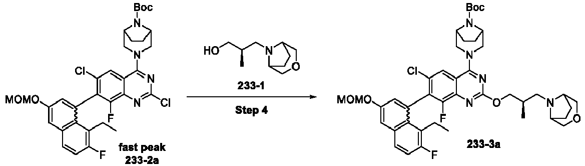

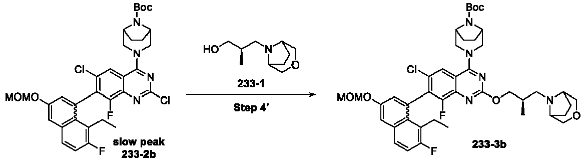

A solution of potassium *tert*-butoxide (1 mol/L, 0.27 mL, 0.27 mmol, 1.2 eq) in tetrahydrofuran was slowly added to a solution of compound **233-2a** (160 mg, 0.23 mmol, 1.00 eq) and **233-1** (49.44 mg, 0.25 mmol, 1.1 eq) in tetrahydrofuran (1 mL) with stirring under the protection of nitrogen at 0 °C. The reaction was carried out for 2 hours at 0 °C, and the reaction process was monitored by liquid chromatography-mass spectrometry and thin layer chromatography. After the reaction was completed, the reaction was quenched by adding 10 mL of water, and the mixture was extracted with ethyl acetate (10 mL × 3), then the organic phases were combined, dried over anhydrous sodium sulfate, filtered, and the filtrate was concentrated to obtain a crude product. The crude product was purified by silica gel column chromatography, eluted with a gradient of 0%--+ 10% methanol/dichloromethane mobile phase, and the obtained fractions were evaporated under reduced pressure to remove the solvent to obtain compound **233-3a** (white solid, 60 mg, yield: 31%). MS (ESI, m/z): 808.3/810.3[M + H]⁺. Compound **233-3b** (white solid, 100mg, yield: 52%) can be prepared by the same method, MS (ESI, m/z): 808.25/810.25[M + H]⁺.

### Step 5

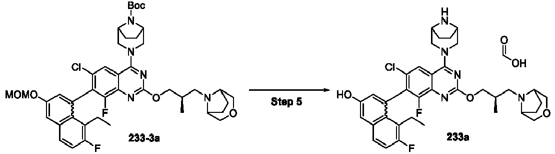

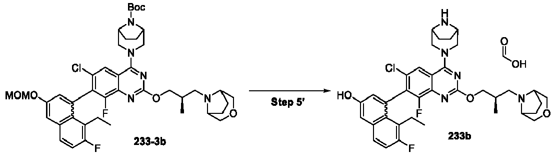

A solution of hydrogen chloride (4 mol/L, 1 mL) in 1,4-dioxane was slowly added to a solution of compound **233-3a** (60 mg, 0.07 mmol, 1.00 eq) in methanol (1 mL) with stirring at 0 °C. The reaction was carried out for 2 hours at 25 °C, and the reaction process was monitored by liquid chromatography-mass spectrometry and thin layer chromatography. After the reaction was completed, the reaction solution was concentrated under reduced pressure to obtain a crude product, the crude product was purified by reversed-phase chromatography (C18 column) and eluted with 0%→ 30% acetonitrile/water mobile phase (0.1% formic acid) in 20 min; detector, UV254/220 nm; compound **233a** was obtained (white solid, 25 mg, yield: 50%). MS (ESI, m/z): 664.0/666.0[M+H]⁺; ¹H NMR (400 MHz, DMSO-*d*₆) *δ* 8.26 (s, 1H), 7.91 (d, *J* = 1.5 Hz, 1H), 7.82 - 7.74 (m, 1H), 7.41 - 7.33 (m, 2H), 6.92 (d, *J* = 2.6 Hz, 1H), 4.57 - 4.51 (m, 1H), 4.45 - 4.36 (m, 1H), 4.36 - 4.28 (m, 1H), 4.18 - 4.09 (m, 1H), 3.79 - 3.72 (m, 2H), 3.71 - 3.65 (m, 1H), 3.61 - 3.56 (m, 1H), 3.50 - 3.45 (m, 2H), 3.39 - 3.33 (m, 2H), 3.08 - 3.00 (m, 1H), 2.99 - 2.91 (m, 1H), 2.57 - 2.52 (m, 1H), 2.29 - 2.13 (m, 3H), 2.11 - 2.00 (m, 1H), 1.87 - 1.62 (m, 8H), 1.00 (d, *J* = 6.6 Hz, 3H), 0.77 - 0.67 (m, 3H); ¹⁹F NMR (377 MHz, DMSO-*d*₆) *δ*-119.45, -121.45. The chiral conditions of compound **233a** were as follows: chiral column CHIRALPAK IC-3, 4.6 × 50 mm, 3 µm; mobile phase A: *n-*hexane (0.1% diethylamine), mobile phase B: ethanol; flow rate: 1 mL/min; isocratic elution with 40% phase B in 3.5 min, detector UV 254 nm; retention time: 2.301 min. *dr >* 40:1.

Compound **233b** (white solid, 38 mg, yield: 55%) can be obtained by the same method as above, MS (ESI, *m*/*z*): 664.0/666.0[M + H]⁺; ¹H NMR (300 MHz, DMSO-*d*₆) *δ* 8.28 (s, 1H), 7.91 (d, *J* = 1.6 Hz, 1H), 7.81 - 7.73 (m, 1H), 7.39 - 7.30 (m, 2H), 6.92 (d, *J* = 2.6 Hz, 1H), 4.53 - 4.44 (m, 1H), 4.42 - 4.25 (m, 2H), 4.24 - 4.14 (m, 1H), 3.83 - 3.59 (m, 5H), 3.47 - 3.42 (m, 2H), 3.39 - 3.30 (m, 2H), 3.08 - 2.91 (m, 2H), 2.30 - 2.00 (m, 4H), 1.84 - 1.65 (m, 8H), 0.99 (d, *J* = 6.5 Hz, 3H), 0.72 (t, *J* = 7.3 Hz, 3H); ¹⁹F NMR (282 MHz, DMSO-*d*₆) *δ*-119.34, - 121.42. The chiral conditions of compound **233b** were as follows: chiral column CHIRALPAK IC-3, 4.6 × 50 mm, 3 µm; mobile phase A: *n*-hexane (0.1% diethylamine), mobile phase B: ethanol; flow rate: 1 mL/min; isocratic elution with 40% phase B in 4.5 min, detector UV 254 nm; retention time: 1.465 min. *dr >* 40:1.

Other similar compound of the present disclosure can be prepared by the synthetic method shown in Embodiment **58** above. Some compounds prepared with reference to the above synthetic method and their characterization data are shown in table 12.

**Table 12**

| Number of the compound | Compound structure | Compound name | Chiral analysis conditions /retention time/*dr* value/specific rotation | Mass spectrum [M+ H]⁺ | ¹H & ¹⁹F NMR |
|---|---|---|---|---|---|
| **251** | 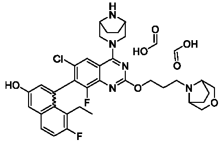 | 4-((7*S* or 7*R*)-2-(3-(3-oxa-8-azabicyclo[3. 2.1]octan-8-yl)propoxy)-4-((1*R*,5*S*)-3,8-diazabicyclo[ 3.2.1] octan-3-yl)-6-chloro-8-fluoroquinaz olin-7-yl)-5-ethyl-6-fluoronaphth alen-2-ol diformate | | 650. 5/65 2.5 | ¹H NMR (300 MHz, DMSO-*d*₆) *δ* 8.25 (s, 2H), 7.91 (d, *J* = 1.6 Hz, 1H), 7.82 - 7.72 (m, 1H), 7.41 - 7.31 (m, 2H), 6.92 (d, *J* = 2.6 Hz, 1H), 4.51 - 4.25 (m, 4H), 3.78 - 3.54 (m, 5H), 3.44 - 3.33 (m, 3H), 3.11 - 3.04 (m, 2H), 2.58 - 2.53 (m, 1H), 2.43 - 2.33 (m, 2H), 2.32 - 2.13 (m, 1H), 1.91 - 1.63 (m, 10H), 0.81- 0.64 (m, 3H); ¹⁹F NMR (282 MHz, DMSO-*d*₆) *δ*-119.45, -121.41. |

### Embodiment 59

### 4-((7S or 7R)-2-((2R)-3-(3-oxa-8-azabicyclo[3.2.1]octan-8-yl)-2-methylpropoxy)-4-((1R,5S)-3,8-diazabicyclo[3.2.1]octan-3-yl)-6-chloro-8-fluoroquinazolin-7-yl)-6-fluoronaphthalen-2-ol diformate 234a; 4-((7R or 7S)-2-((2R)-3-(3-oxa-8-azabicyclo[3.2.1]octan-8-yl)-2-methylpropoxy)-4-((1R,5S)-3,8-diazabicyclo[3.2.1]octan-3-yl)-6-chloro-8-fluoroquinazolin-7-yl)-6-fluoronaphthalen-2-ol formate 234b.

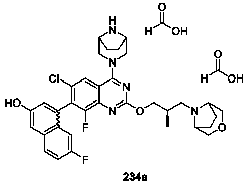

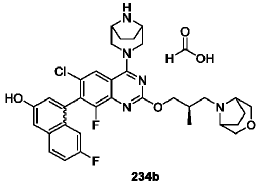

The synthetic route was as follows:

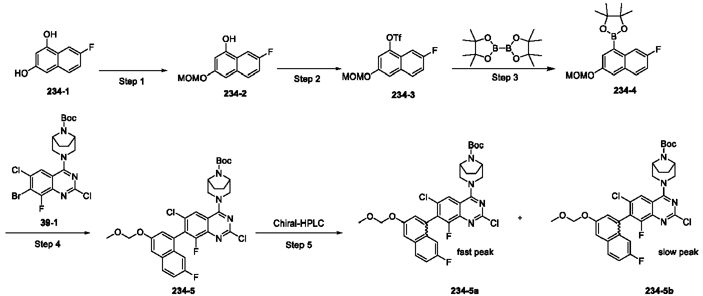

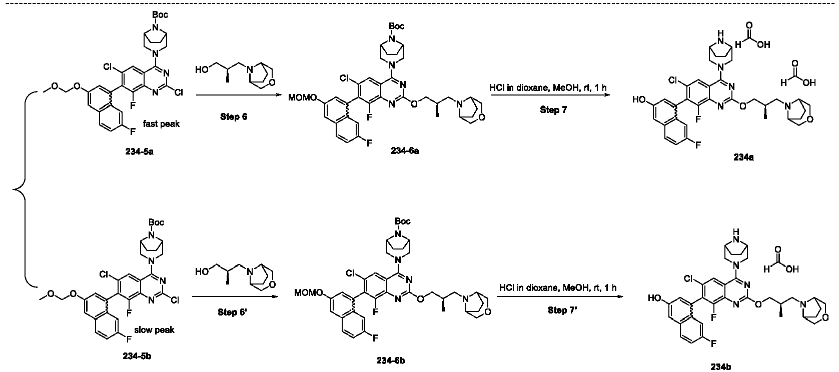

### Step 1

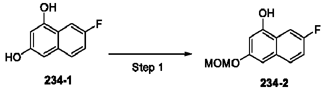

Compound **234-1** was synthesized with reference to patent (WO2021041671).

Chloromethyl methyl ether (11.20 g, 159.97 mol, 1.0 eq) was added to a solution of compound **234-1** (28.5 g, 159.97 mol, 1.0 eq) and *N,N*-diisopropylethylamine (97.93 g, 719.85 mol, 4.5 eq) in dichloromethane (300 mL) with stirring under the protection of nitrogen at 0 °C. The obtained mixture was stirred for 2 hours at 25 °C, and the reaction process was monitored by liquid chromatography-mass spectrometry and thin layer chromatography. After the reaction was completed, 500 mL of water was added to the reaction solution, the mixture was extracted with ethyl acetate (500 mL × 3). The organic phases were combined and washed with 500 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 10% ethyl acetate/petroleum ether mobile phase, and the obtained fractions were evaporated under reduced pressure to remove the solvent to obtain compound **234-2** (colorless oil, 5 g, yield: 14%). MS (ESI, m/z): 221.1[M-H]⁻. ¹H NMR (300 MHz, CDCl₃) *δ* 7.74 - 7.71 (m, 1H), 7.65 - 7.62 (m, 1H), 7.23 - 7.17 (m, 1H), 6.96 (d, *J* = 2.4 Hz, 1H), 6.64 - 6.63 (m, 1H), 5.23 (s, 2H), 3.50 (s, 3H). The structure of the compound was determined by NOESY.

### Step 2

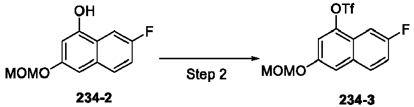

Trifluoromethylsulfonic anhydride (8.89 g, 29.93 mmol, 1.4 eq) was added to a solution of compound **234-2** (5 g, 21.38 mol, 1.0 eq) and triethylamine (4.55 g, 42.752 mol, 2.0 eq) in dichloromethane (300 mL) with stirring at 0 °C. The obtained mixture was stirred for 2 hours at 25 °C, and the reaction process was monitored by liquid chromatography-mass spectrometry and thin layer chromatography. After the reaction was completed, 200 mL of water was added to the reaction solution, and the mixture was extracted with ethyl acetate (200 mL × 3). The organic phases were combined and washed with 200 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 20% ethyl acetate/petroleum ether mobile phase, and the obtained fractions were evaporated under reduced pressure to remove the solvent to obtain compound **234-3** (colorless oil, 4.0 g, yield: 50%). MS (ESI, m/z): 353.0[M-H]⁻; ¹H NMR (300 MHz, CDCl₃) *δ* 7.80 - 7.76 (m, 1H), 7.60 - 7.57 (m, 1H), 7.46 (d, *J=* 2.3 Hz, 1H), 7.35 - 7.30 (m, 2H), 5.28 (s, 2H), 3.53 (s, 3H).

### Step 3

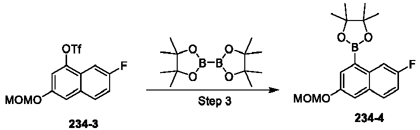

Potassium acetate (5.54 g, 53.630 mmol, 5.0 eq) was added to a solution of compound **234-3** (4.0 g, 10.73 mmol, 1.0 eq), bis(pinacolato)diboron (4.30 g, 16.10 mmol, 1.5 eq), [1,1'-bis(diphenylphosphino)ferrocene]palladium dichloride (0.83 g, 1.07 mmol, 0.1 eq) in 1,4-dioxane (40 mL) with stirring under the protection of nitrogen at 25 °C. The reaction was carried out for 8 hours at 85 °C, and the reaction process was monitored by liquid chromatography-mass spectrometry and thin layer chromatography. After the reaction was completed, 500 mL of water was added to the reaction solution, and the mixture was extracted with dichloromethane (500 mL × 3). The organic phases were combined and washed with 500 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 20% ethyl acetate/petroleum ether mobile phase, and the obtained fractions were evaporated under reduced pressure to remove the solvent to obtain compound **234-4** (colorless oil, 3.5 g, yield: 93%). MS (ESI, m/z): 331.2[M + H]⁺, ¹H NMR (300 MHz, CDCl₃) *δ* 8.37 - 8.36 (m, 1H), 7.83 (d, *J=* 2.6 Hz, 1H), 7.72 - 7.68 (m, 1H), 7.49 (d, *J=* 2.7 Hz, 1H), 7.24 - 7.19 (m, 1H), 5.29 (s, 2H), 3.51 (s, 3H), 1.41 (s, 12H).

### Step 4

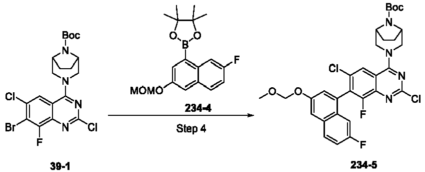

Compounds **39-1** (3.0 g, 5.63 mmol, 1.0 eq), **234-4** (2.95 g, 8.45 mmol, 1.5 eq), 3-(tert-butyl)-4-(2,6-dimethoxyphenyl)-2,3-dihydrobenzo[d][1,3]oxaphosphole (0.16 g, 0.45 mmol, 0.08 eq), tris(dibenzylidene indanone)dipalladium (0.43 g, 0.45 mmol, 0.08 eq) and potassium phosphate (2.52 mg, 11.260 mmol, 2.0 eq) were successively added to a reaction flask with stirring under the protection of nitrogen at 25 °C, then toluene (25 mL) and water (5 mL) were added thereto. After the addition, oxygen was removed, and the obtained reaction was carried out with stirring for 2 hours at 80 °C, and the reaction process was monitored by liquid chromatography-mass spectrometry and thin layer chromatography. After the reaction was completed, 200 mL of water was added to the reaction solution, and the mixture was extracted with ethyl acetate (200 mL × 3). The organic phases were combined and washed with 200 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→ 80% ethyl acetate/petroleum ether mobile phase, and the obtained fractions were evaporated under reduced pressure to remove the solvent to obtain compound **234-5** (white solid, 1.6 g, yield: 43%). MS (ESI, m/z): 631.2/633.2[M + H]⁺; ¹H NMR (400 MHz, CDCl₃) *δ* 7.84 - 7.80 (m, 2H), 7.56 (d, *J=* 2.4 Hz, 1H), 7.29 - 7.24 (m, 1H), 7.22 (d, *J=* 2.4 Hz, 1H), 6.93-6.89 (m, 1H), 5.34-5.31 (m, 2H), 4.58 - 4.42 (m, 4H), 3.73 (s, 2H), 3.55 (s, 3H), 2.03-1.98 (m, 2H), 1.83 - 1.80 (m, 2H), 1.54 (s, 9H).

### Step 5

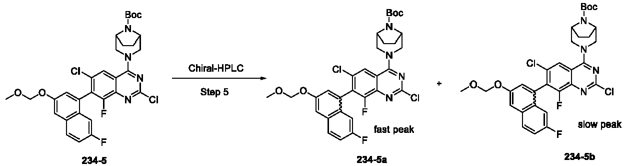

Compound **234-5** (1.6 g) was subjected to chiral resolution by preparative supercritical liquid chromatography: chiral column NB_ASA CHIRALPAK IG_2,5 × 30 cm, 10 µm; mobile phase A: supercritical carbon dioxide, mobile phase B: methanol/dichloromethane (1/1, 0.5% ammonia); flow rate: 180 mL/min; column temperature: 35 °C; eluted with 50% mobile phase B; detector UV 220 nm, two products were obtained. The product with shorter retention time (3.91 min) was compound **234-5a**, tert-butyl (1*R*,5*S*)-3-((*R* or 5)-2,6-dichloro-8-fluoro-7-(7-fluoro-3-(methoxymethoxy)naphthalen-1-yl)quinazolin-4-yl)-3,8-diazabicyclo[3.2.1]octane-8-carboxylate (white solid, 695 mg, 43%) ), MS (ESI, m/z): 631.2/633.2[M + H]⁺; the product with longer retention time (4.96 min) was compound **234-5b**, *tert*-butyl (1*R*,5*S*)-3-((*S* or *R*)-2,6-dichloro-8-fluoro-7-(7-fluoro-3-(methoxymethoxy)naphthalen-1-yl)quinazolin-4-yl)-3,8-diazabicyclo[3.2.1]octane-8-carboxylate (white solid, 698 mg, 44%), MS (ESI, m/z): 631.2/633.2[M + H]⁺.

### Step 6

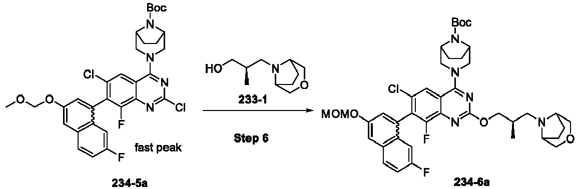

A solution of potassium *tert*-butoxide (1 mol/L, 0.331 mL, 0.331 mmol, 1.2 mL) in tetrahydrofuran was added to a solution of compound **234-5a** (200 mg, 0.301 mmol, 1.0 eq) and **233-1** (64.54 mg, 0.331 mmol, 1.2 eq) in tetrahydrofuran (4 mL) with stirring under the protection of nitrogen at 0 °C. The mixture was stirred at 0 °C for 2 hours. The reaction process was monitored by liquid chromatography-mass spectrometry and thin layer chromatography. After the reaction was completed, 50 mL of water was added to the reaction solution, and the mixture was extracted with ethyl acetate (50 mL × 3). The organic phases were combined and washed with 50 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **234-6a** (white solid, 75 mg, yield: 30%). MS (ESI, m/z): 780.5/782.5[M + H]⁺; ¹H NMR (300 MHz, CDCl₃) *δ* 7.87 - 7.81 (m, 2H), 7.57 (s, 1H), 7.32 (s, 1H), 7.25 (s, 1H), 7.00 - 6.93 (m, 1H), 5.35 (s, 1H), 4.84 - 4.61 (m, 2H), 4.50 - 4.27 (m, 5H), 4.13 - 4.05 (m, 1H), 3.78 - 3.64 (m, 4H), 3.58 (s, 3H), 3.52 - 3.47 (m, 2H), 3.07 - 2.98 (m, 2H), 2.87 (s, 1H), 2.34 - 2.15 (m, 3H), 2.02 (s, 2H), 1.92 - 1.82 (m, 4H), 1.55 (s, 9H), 1.28 - 1.10 (m, 3H).

### Step 7

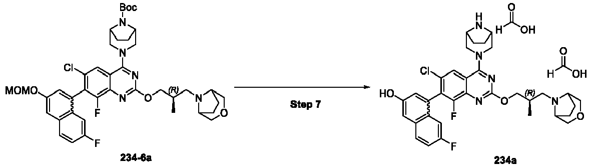

A solution of hydrochloride (4 mol/L, 1 mL) in 1,4-dioxane was added to a solution of compound **234-6a** (70 mg, 0.085 mmol, 1.0 eq) in methanol (2 mL) with stirring under the protection of nitrogen at 25 °C. The reaction was carried out at 25 °C for 1 hour, and the reaction process was monitored by liquid chromatography-mass spectrometry and thin layer chromatography. After the reaction was completed, the reaction mixture was concentrated under reduced pressure to remove the excess reagent to obtain a crude product. The obtained crude product was purified by reversed-phase rapid chromatographic column (C18 column), and eluted with 5%→ 95% acetonitrile/water mobile phase (0.1% formic acid) in 30 min; detector, UV254 nm; to obtain compound **234a** (white solid, 30.3 mg, yield: 49%). MS (ESI, m/z): 636.1/638.1[M + H]⁺; ¹H NMR (400 MHz, DMSO-*d*₆): *δ* 8.27 (s, 2H), 7.97 - 7.87 (m, 2H), 7.43 - 7.34 (m, 2H), 7.14 (d, *J=* 2.4 Hz, 1H), 6.94 - 6.91 (m, 1H), 4.52 - 4.48 (m, 1H), 4.44 - 4.38 (m, 2H), 4.20 - 4.16 (m, 1H), 3.81 (s, 2H), 3.69 - 3.63 (m, 2H), 3.52 - 3.47 (m, 2H), 3.40 - 3.36 (m, 2H), 3.0 - 2.97 (m, 2H), 2.29 - 2.17 (m, 2H), 2.11 - 2.03 (m, 1H), 1.84 - 1.79 (m, 6H), 1.70 - 1.66 (m, 2H), 1.00 (d, *J=* 6.7 Hz, 3H); ¹⁹F NMR (377 MHz, DMSO-*d*₆) *δ*-117.53, -122.49. The chiral analysis conditions of compound **234a** were: N-Lux 3 µm Cellulose-4 (H17-388767), 4.6 × 100 mm, 3 µm; mobile phase A: supercritical carbon dioxide fluid, mobile phase B: methanol (0.1% diethylamine); flow rate: 3.5 mL/min; isocratic elution with 48% phase B in 6 min; detector UV 220 nm; retention time: 2.41 min. *dr* > 40:1.

### Step 6:

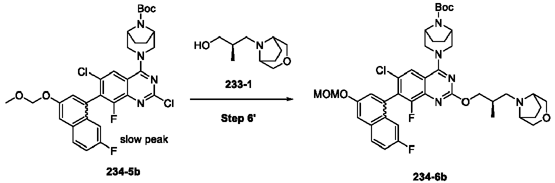

A solution of potassium tert-butoxide (1 mol/L, 0.339 mL, 0.339 mmol, 1.5 eq) in tetrahydrofuran was slowly added to a solution of compound **233-5b** (150 mg, 0.226 mmol, 1.0 eq) and **233-1** (48.41 mg, 0.25 mmol, 1.1 eq) in tetrahydrofuran (1 mL) with stirring under the protection of nitrogen at 0 °C. The mixture was stirred at 0 °C for 2 hours. The reaction process was monitored by liquid chromatography-mass spectrometry and thin layer chromatography. After the reaction was completed, 50 mL of water was added to the reaction solution, and the mixture was extracted with ethyl acetate (50 mL × 3). The organic phases were combined and washed with 50 mL of saturated brine, then dried over anhydrous sodium sulfate after washing, and the drying agent was removed by filtration; the filtrate was concentrated under reduced pressure to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **234-6b** (white solid, 63 mg, yield: 34%). MS (ESI, m/z): 780.5/782.5[M + H]⁺; ¹H NMR (300 MHz, CDCl₃) *δ* 7.86 - 7.78 (m, 2H), 7.57 (d, *J=* 2.4 Hz, 1H), 7.31 - 7.28 (m, 1H), 7.25 (d, *J=* 2.4 Hz, 1H), 7.00 - 6.96 (m, 1H), 5.37 - 5.33 (s, 2H), 4.80 - 4.62 (m, 2H), 4.50 - 4.26 (m, 5H), 3.72 - 3.46 (m, 9H), 3.07 - 2.98 (m, 2H), 2.38 - 2.16 (m, 3H), 2.07 -1.98 (m, 2H), 1.96 -1.82 (m, 5H), 1.55 (s, 9H), 1.12 - 1.09 (m, 3H).

### Step 7:

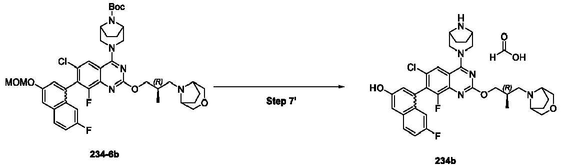

A solution of hydrochloride (4 mol/L, 1 mL) in 1,4-dioxane was added to a solution of compound **234-6b** (70 mg, 0.085 mmol, 1.0 eq) in methanol (2 mL) with stirring under the protection of nitrogen at 25 °C. The reaction was carried out at 25 °C for 1 hour, and the reaction process was monitored by liquid chromatography-mass spectrometry and thin layer chromatography. After the reaction was completed, the reaction mixture was concentrated under reduced pressure to remove the excess reagent to obtain a crude product. The obtained crude product was purified by reversed-phase rapid chromatographic column (C18 column), and eluted with 5%→ 95% acetonitrile/water mobile phase (0.1% formic acid) in 30 min; detector, UV254 nm; to obtain compound **234b** (white solid, 35.5 mg, yield: 60%). MS (ESI, m/z): 636.1 [M + H]⁺; ¹H NMR (400 MHz, DMSO-*d*₆): *δ* 8.27 (s, 1H), 7.94 - 7.89 (m, 2H), 7.41 - 7.35 (m, 2H), 7.14 (d, *J=* 2.4 Hz, 1H), 6.96 - 6.92 (m, 1H), 4.52 - 4.48 (m, 1H), 4.42 - 4.34 (m, 2H), 4.19 - 4.14 (m, 1H), 3.70 (s, 2H), 3.66 - 3.57 (m, 2H), 3.52 - 3.50 (m, 2H), 3.38 - 3.36 (m, 2H), 3.05 - 2.96 (m, 2H), 2.29 - 2.15 (m, 2H), 2.11 - 2.02 (m, 1H), 1.83 - 1.79 (m, 2H), 1.75 (s, 4H), 1.69 - 1.65 (m, 2H), 1.00 (d, *J=* 6.7 Hz, 3H); ¹⁹F-NMR (377 MHz, DMSO-*d*₆) *δ*-117.58, -122.55. The chiral analysis conditions of compound **234b** were: N-Lux 3 µm Cellulose-4 (H17-388767), 4.6 × 100 mm, 3 µm; mobile phase A: supercritical carbon dioxide fluid, mobile phase B: methanol (0.1% diethylamine); flow rate: 3.5 mL/min; isocratic elution with 48% phase B in 6 min; detector UV 220 nm; retention time: 3.66 min. *dr* > 40:1.

Other similar compound of the present disclosure can be prepared by the synthetic method shown in Embodiment **59** above. Some compounds prepared with reference to the above synthetic method and their characterization data are shown in table 13.

**Table 13**

| Num ber of the com poun d | Compound structure | Compound name | Chiral analysis conditions/r etention time/*dr* value/specif ic rotation | Mass spectr um[M +H]⁺ | ¹H & ¹⁹F NMR |
|---|---|---|---|---|---|
| **252** | 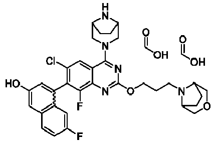 | 4-((7*S* or 7*R*)-2-(3-(3-oxa-8-azabicyclo[3.2. 1]octan-8-yl)propoxy)-4-((1*R*,5*S*)-3,8-diazabicyclo[3. 2.1]octan-3-yl)-6-chloro-8-fluoroquinazoli n-7-yl)-6-fluoronaphthal en-2-ol diformate | | 622.1/ 624.1 | ¹H NMR (400 MHz, DMSO-*d*₆) *δ* 8.24 (s, 2H), 7.97 - 7.88 (m, 2H), 7.42 - 7.33 (m, 2H), 7.13 (d, *J* = 2.3 Hz, 1H), 6.96 - 6.90 (m, 1H), 4.48 - 4.36 (m, 4H), 3.81 - 3.72 (m, 2H), 3.71 - 3.57 (m, 2H), 3.50 (d, *J* = 10.1 Hz, 2H), 3.44 - 3.35 (m, 2H), 3.13 - 3.04 (m, 2H), 2.42 - 2.34 (m, 2H), 1.93 - 1.81 (m, 4H), 1.80-1.72 (m, 4H), 1.72 - 1.67 (m, 2H); ¹⁹F NMR (377 MHz, DMSO-*d*₆) *δ*-117.67, - 122.49. |

### Embodiment 60

### 4-(2-(3-(3-Oxa-8-azabicyclo[3.2.1]octan-8-yl)propoxy)-4-((1R,5S)-3,8-diazabicyclo[3.2.1]octan-3-yl)-5,8-difluoroquinazolin-7-yl)naphthalen-2-ol formate 253

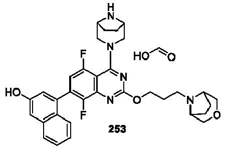

The synthetic route was as follows:

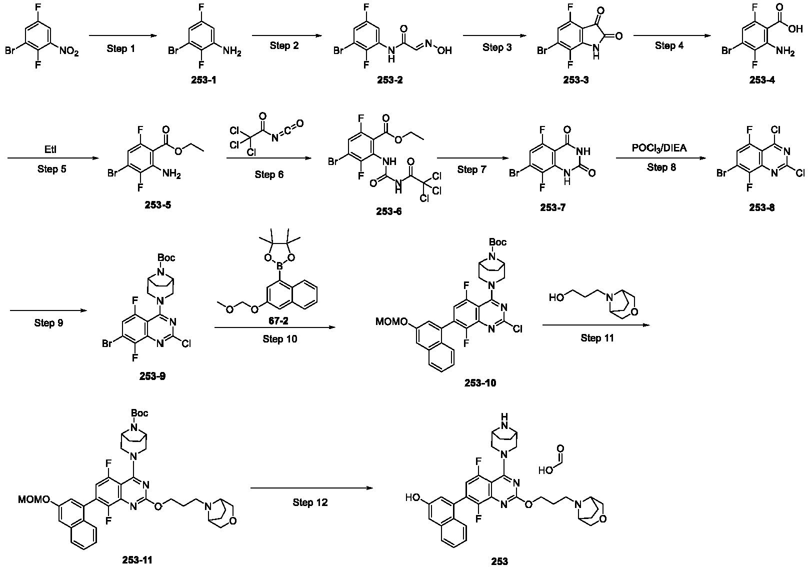

### Step 1:

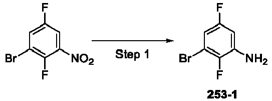

Ammonium chloride (100.02 g, 1776.35 mmol, 5 eq) was added to a mixed solution of 3-bromo-2,5-difluoronitrobenzene (89.0 g, 355.27 mmol, 1.0 eq) and iron powder (104.42 g, 1776.35 mmol, 5 eq) in ethanol (1200 mL) and water (240 mL) with stirring at 25 °C. The reaction was carried out for 16 hours at 25 °C, and the reaction process was monitored by liquid chromatography-mass spectrometry and thin layer chromatography. After the reaction was completed, the mixture was filtered to remove the insolubles, and the filter cake was washed with ethanol (500 × 3 mL) and concentrated under reduced pressure to obtain a crude product. The crude product was purified by silica gel column chromatography, and the mobile phase was eluted with a gradient of 0%→ 10% ethyl acetate/petroleum ether mobile phase, and the obtained fractions were evaporated under reduced pressure to remove the solvent to obtain compound **253-1** (orange oil, 44 g, yield: 56%). MS (ESI, m/z): 208.1/210.1[M + H]⁺; ¹H NMR (400 MHz, DMSO-*d*₆) *δ* 6.66 - 6.60 (m, 1H), 6.58 - 6.50 (m, 1H), 5.80 (s, 2H).

### Step 2:

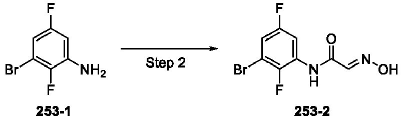

Sodium sulfate (390.6 g, 2612.42 mmol, 13 eq) and hydroxylamine hydrochloride (44.1 g, 602.86 mmol, 3 eq) were added to a mixed solution of compound **253-1** (44 g, 200.95 mmol, 1.0 eq) and chloral hydrate (38.48 g, 221.05 mmol, 1.1 eq) in sulfuric acid (220 mL) and water (924 mL) with stirring at 25 °C. The reaction was carried out for 16 hours at 70 °C, and the reaction process was monitored by liquid chromatography-mass spectrometry and thin layer chromatography. After the reaction was completed, the reaction mixture was cooled to room temperature. The mixture was filtered and the filter cake was washed with water (500 × 3 mL) to obtain a crude product of compound **253-2** (orange solid, 54 g). The crude product was directly used for the next reaction. MS (ESI, m/z): 277.1/279.1[M-H]⁻.

### Step 3:

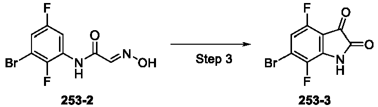

Compound **253-2** (54 g, 172.23 mmol, 1.0 eq) was dissolved in sulfuric acid (475 mL) at 25 °C. The mixture was stirred at 90 °C for 1 hour and the reaction process was monitored by thin layer chromatography (petroleum ether/ethyl acetate = 1/1, R_{f} = 0.5). After the reaction was completed, the reaction mixture was cooled to room temperature. The mixture was quenched by pouring into ice water. The mixture was filtered and the filter cake was washed with water (500 × 3 mL) to obtain the compound **253-3** (brown solid, 38 g). The crude product was used directly in the next reaction without further purification.

### Step 4:

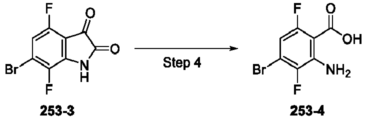

Hydrogen peroxide (30%, 646 mL) was slowly added to an aqueous sodium hydroxide solution (2 mol/L, 76 mL) of compound **253-3** (38 g, 137.78 mmol, 1.0 eq) with stirring at 25 °C. The reaction was carried out for 16 hours at 25 °C, and the reaction process was monitored by liquid chromatography-mass spectrometry and thin layer chromatography. After the reaction was completed, the pH value of the reaction solution was adjusted to 7 with dilute hydrochloric acid, and the mixture was filtered to remove the insolubles, and the filter cake was washed with water (500 × 3 mL); the pH value of the filtrate was adjusted to 1 with dilute hydrochloric acid, and the solid was precipitated, filtered, and the filter cake was washed with water (500 × 3 mL) and dried to obtain a crude product of compound **253-4** (gray solid, 20.4 g). The crude product was used directly in the next reaction without further purification. MS (ESI, m/z): 250.1/252.1[M-H]⁻.

### Step 5:

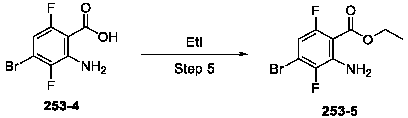

Iodoethane (3.34 g, 20.35 mmol, 1.2 eq) was slowly added to a solution of compound **253-4** (4.5 g, 16.96 mmol, 1.0 eq) and cesium carbonate (11.64 g, 33.92 mmol, 2 eq) in *N,N-*dimethylformamide (45 mL) under the protection of nitrogen at 0 °C. The reaction was carried out for 4 hours at 25 °C, and the reaction process was monitored by liquid chromatography-mass spectrometry and thin layer chromatography. After the reaction was completed, the reaction solution was diluted with 400 mL of water, extracted with ethyl acetate (500 mL × 3), and the organic phases were combined. The organic phase was washed with saturated brine (500 mL × 3), dried over anhydrous sodium sulfate, filtered to remove the drying agent, and the filtrate was concentrated under reduced pressure to obtain a crude product. The crude product was purified by silica gel column chromatography, and the mobile phase was eluted with a gradient of 0%→ 50% ethyl acetate/petroleum ether mobile phase, and the obtained fractions were evaporated under reduced pressure to remove the solvent to obtain compound **253-5** (orange oil, 3.92 g, yield: 78%). MS (ESI, m/z): 280.1/282.2[M + H]⁺; ¹H NMR (300 MHz, CDCl₃) *δ* 6.62 - 6.53 (m, 1H), 4.49 - 4.34 (m, 2H), 1.52 - 1.32 (m, 3H).

### Step 6:

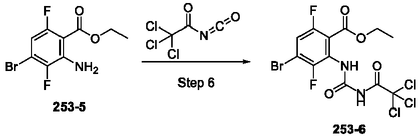

Trichloroacetyl isocyanate (3.96 g, 19.94 mmol, 1.5 eq) was slowly added to a solution of compound **253-5** (3.92 g, 13.29 mmol, 1.0 eq) in tetrahydrofuran (40 mL) under the protection of nitrogen at 25 °C. The reaction was carried out for 0.5 hours with stirring at 25 °C, and the reaction process was monitored by liquid chromatography-mass spectrometry and thin layer chromatography. After the reaction was completed, the reaction mixture was concentrated under reduced pressure to obtain a crude product. The crude product was slurried with 500 mL of methyl *tert*-butyl ether to obtain compound **253-6** (white solid, 6.5 g, yield: 99%). MS (ESI, m/z): 467.0/469.0[M + H]⁺; ¹H NMR (300 MHz, DMSO-*d*₆) *δ* 11.76 (s, 1H), 9.88 (s, 1H), 7.95 - 7.83 (m, 1H), 4.29 (q, *J* = 7.1 Hz, 2H), 1.26 (t, *J* = 7.1 Hz, 3H).

### Step 7:

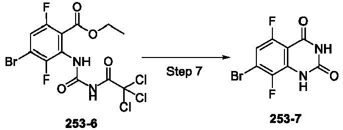

A solution of ammonia (7 mol/L, 7 mL) in methanol was slowly added to a solution of compound **253-6** (6.5 g, 13.18 mmol, 1.0 eq) in methanol (70 mL) with stirring at 25 °C. The reaction was carried out for 1 hour at 25 °C, and the reaction process was monitored by liquid chromatography-mass spectrometry and thin layer chromatography. After the reaction was completed, the reaction mixture was concentrated under reduced pressure to obtain a crude product. The crude product was slurried with 200 mL of methyl tert-butyl ether to obtain compound **253-7** (white solid, 3.92 g, yield: 99%). MS (ESI, m/z): 277.2/279.2[M + H]⁺; ¹H NMR (300 MHz, DMSO-*d*₆) *δ* 11.67 - 11.41 (m, 2H), 7.47 - 7.36 (m, 1H).

### Step 8:

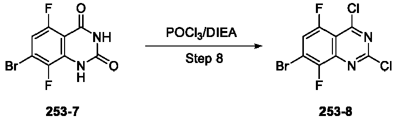

*N,N*-diisopropylethylamine (0.5 mL, 2.72 mmol, 3.18 eq) was slowly added to a solution of compound **253-7** (250 mg, 0.857 mmol, 1.0 eq) in phosphorus oxychloride (4.75 mL) with stirring under the protection of nitrogen at 0 °C. The mixture was stirred at 90 °C for 3 hours and the reaction process was monitored by thin layer chromatography (petroleum ether/ethyl acetate = 10/1, R_{f}= 0.5). After the reaction was completed, the reaction mixture was concentrated under reduced pressure to obtain a crude product. The crude product was purified by silica gel column chromatography, eluted with a gradient of 0%→ 10% ethyl acetate/petroleum ether mobile phase, and the obtained fractions were evaporated under reduced pressure to remove the solvent to obtain compound **253-8** (white solid, 100 mg, yield: 35%). ¹H NMR (400 MHz, CDCl₃) *δ* 7.62 - 7.53 (m, 1H).

### Step 9:

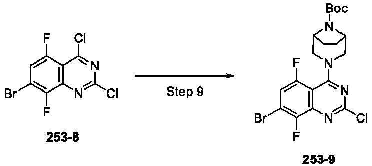

*tert*-Butyl 3,8-diazabicyclo[3.2.1]octane-8-carboxylate (65.6 mg, 0.294 mmol, 1 eq) was slowly added to a solution of compound **253-8** (97 mg, 0.294 mmol, 1.0 eq) and triethylamine (93.8 mg, 0.882 mmol, 3.0 eq) in dichloromethane (1.5 mL) with stirring under the protection of nitrogen at 0 °C. The reaction was carried out for 0.5 hours at 25 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, and the mobile phase was eluted with a gradient of 0%→ 20% ethyl acetate/petroleum ether mobile phase, and the obtained fractions were evaporated under reduced pressure to remove the solvent to obtain compound **253-9** (white solid, 130 mg, yield: 85%). MS (ESI, m/z): 489.1/491.1[M + H]⁺; ¹H NMR (300 MHz, CDCl₃) *δ* 7.28 - 7.21 (m, 1H), 4.42 - 4.06 (m, 4H), 3.73 - 3.49 (m, 2H), 2.02 - 1.84 (m, 2H), 1.71 - 1.59 (m, 2H), 1.53 (s, 9H).

### Step 10:

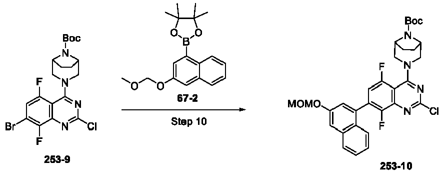

Tris(dibenzylideneacetone)dipalladium (24.3 mg, 0.025 mmol, 0.1 eq) and 3-(*tert-*butyl)-4-(2,6-dimethoxyphenyl)-2,3-dihydrobenzo[d][1,3]oxaphosphole (17.5 mg, 0.05 mmol, 0.2 eq) were slowly added to a mixed solution of compound **253-9** (130 mg, 0.25 mmol, 1.0 eq), compound **67-2** (75.0 mg, 0.227 mmol, 0.9 eq) and potassium phosphate (112.69 mg, 0.504 mmol, 2.0 eq) in toluene (1.0 mL) and water (0.2 mL) with stirring under the protection of nitrogen at 25 °C. The reaction was carried out for 3 hours at 75 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, and the mobile phase was eluted with a gradient of 0%→ 20% ethyl acetate/petroleum ether mobile phase, and the obtained fractions were evaporated under reduced pressure to remove the solvent to obtain compound **253-10** (white solid, 130 mg, yield: 82%). MS (ESI, m/z): 597.2/599.2[M + H]⁺; ¹H NMR (300 MHz, CDCl₃) δ 7.86 (d, *J*= 8.2 Hz, 1H), 7.63 - 7.48 (m, 3H), 7.42 - 7.33 (m, 1H), 7.28 - 7.26 (m, 1H), 7.18 - 7.08 (m, 1H), 5.36 (s, 2H), 4.51 - 4.15 (m, 4H), 3.72 - 3.53 (m, 5H), 2.01 - 1.90 (m, 2H), 1.84 - 1.72 (m, 2H), 1.54 (s, 9H).

### Step 11:

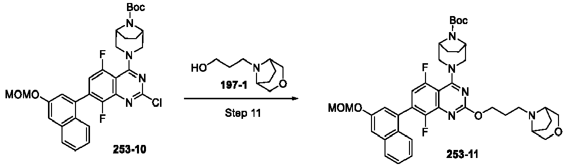

Triethylenediamine (4.5 mg, 0.038 mmol, 0.2 eq) and cesium carbonate (130.9 mg, 0.38 mmol, 2 eq) were added to a solution of compound **253-10** (120 mg, 0.191 mmol, 1.0 eq) and **197-1** (120 mg, 0.191 mmol, 1.0 eq) in *N,N*-dimethylformamide (1.5 mL) with stirring under the protection of nitrogen at 25 °C. The reaction was carried out for 2 hours at 95 °C, and the reaction process was monitored by liquid chromatography-mass spectrometry and thin layer chromatography. After the reaction was completed, the crude product was purified by reversed-phase chromatographic column (C18 column), eluted with 60%→95% methanol/water (0.1% ammonia water) mobile phase in 25 min; detector: UV254/220 nm; the compound **253-11** (white solid, 44 mg, yield: 29%) was obtained. MS (ESI, m/z): 732.3 [M + H]+.

### Step 12:

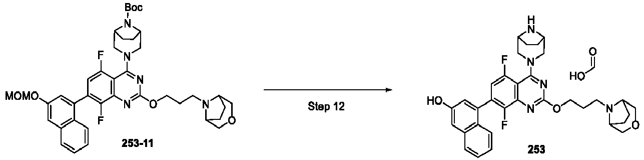

A solution of hydrochloric acid (4 mol/L, 2 mL) in 1,4-dioxane was added dropwise to a solution of compound **253-11** (44 mg, 0.06 mmol, 1.00 eq) in methanol (2 mL) with stirring at 0 °C. The reaction was carried out for 1 hour at room temperature, and the reaction process was monitored by liquid chromatography-mass spectrometry and thin layer chromatography. After the reaction was completed, the reaction mixture was concentrated to obtain a crude product. The obtained crude product was purified by reversed-phase rapid chromatographic column (C18 column), and eluted with 5%→ 95% acetonitrile/water mobile phase (0.1% formic acid) in 30 min; detector, UV254 nm; to obtain compound **253** (white solid, 20.5 mg, yield: 52%). MS (ESI, *m*/*z*): 588.1[M + H]⁺; ¹H NMR (300 MHz, CD₃OD) δ 8.54 (s, 1H), 7.77 (d, *J* = 8.3 Hz, 1H), 7.55 - 7.40 (m, 2H), 7.32 - 7.22 (m, 2H), 7.16 - 7.05 (m, 2H), 4.66 - 4.54 (m, 2H), 4.45 - 4.23 (m, 2H), 4.08 - 3.95 (m, 2H), 3.92 - 3.79 (m, 2H), 3.76 - 3.53 (m, 6H), 3.00 - 2.88 (m, 2H), 2.28 - 1.95 (m, 10H); ¹⁹F NMR (282 MHz, CD₃OD) *δ*-110.37, - 110.44, -132.84, -132.91.

### Embodiment 61

### 4-(2-(3-(3-Oxa-8-azabicyclo[3.2.1]octan-8-yl)propoxy)-4-((1R,5S)-3,8-diazabicyclo[3.2.1]octan-3-yl)thieno[3,2-d]pyrimidin-6-yl)naphthalen-2-ol diformate 254

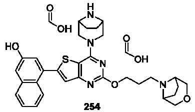

The synthetic route was as follows:

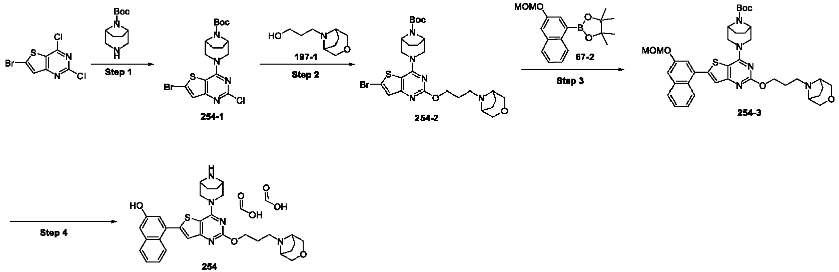

### Step 1:

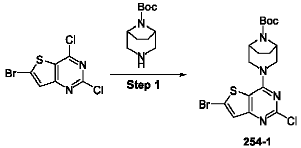

*tert*-Butyl 3,8-diazabicyclo[3.2.1]octane-8-carboxylate (0.75 g, 3.346 mmol, 1 eq) was slowly added to a solution of 6-bromo-2,4-dichlorothieno[3,2-*d*]pyrimidine (1 g, 3.346 mmol, 1.0 eq) and triethylamine (1.03 mg, 10.038 mmol, 3.0 eq) in dichloromethane (10 mL) with stirring under the protection of nitrogen at 0°C. The reaction was carried out for 1 hour at 25 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, and the mobile phase was eluted with a gradient of 0%→ 10% ethyl acetate/petroleum ether mobile phase, and the obtained fractions were evaporated under reduced pressure to remove the solvent to obtain compound **254-1** (white solid, 1.38 g, yield: 85%). MS (ESI, *m*/*z*): 459.1/461.1[M + H]⁺;¹H NMR (300 MHz, CDCl₃) *δ* 7.39 (s, 1H), 4.53 - 4.31 (m, 4H), 3.58 - 3.26 (m, 2H), 2.12 - 1.97 (m, 2H), 1.86 - 1.74 (m, 2H), 1.52 (s, 9H).

### Step 2:

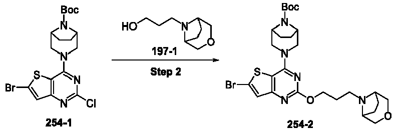

Triethylenediamine (24.4 mg, 0.207 mmol, 0.2 eq) and cesium carbonate (1.06 mg, 3.099 mmol, 3 eq) were added to a solution of compound **254-1** (500 mg, 1.033 mmol, 1.0 eq) and compound **197-1** (223.5 mg, 1.240 mmol, 1.2 eq) in *N*, *N*-dimethylformamide (7 mL) with stirring under the protection of nitrogen at 25 °C. The reaction was carried out for 2 hours at 100 °C, and the reaction process was monitored by liquid chromatography-mass spectrometry and thin layer chromatography. After the reaction was completed, the crude product was purified by reversed-phase chromatographic column (C18 column), eluted with 5%→95% methanol/water mobile phase in 25 min; detector: UV254/220 nm; the compound **254-2** (off-white solid, 300 mg, yield: 46%) was obtained. MS (ESI, *m*/*z*): 594.2/596.2[M + H]⁺. ¹H NMR (300 MHz, CDCl₃) *δ* 7.64 (s, 1H), 4.64 - 4.36 (m, 6H), 4.28 - 4.19 (m, 2H), 3.93 - 3.86 (m, 2H), 3.74 (d, *J* = 12.5 Hz, 2H), 3.66 - 3.42 (m, 2H), 3.35 - 3.23 (m, 2H), 2.46 - 2.23 (m, 6H), 2.17 - 1.96 (m, 2H), 1.85 - 1.71 (m, 2H), 1.53 (s, 9H).

### Step 3:

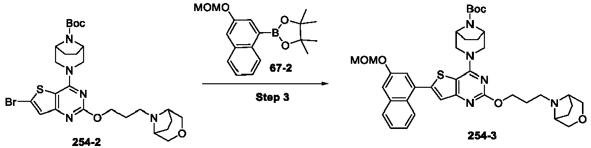

[1, 1'-Bis(diphenylphosphine)ferrocene]palladium dichloromethane complex (20.5 mg, 0.024 mmol, 0.1 eq) was slowly added to a mixed solution of compound **254-2** (150 mg, 0.24 mmol, 1.0 eq), compound **67-2** (118.9 mg, 0.36 mmol, 1.5 eq) and potassium phosphate (157.3 mg, 0.72 mmol, 3 eq) in 1,4-dioxane (2 mL) and water (0.4 mL) with stirring under the protection of nitrogen at 25 °C. The reaction was carried out for 1.5 hours at 95 °C, and the reaction process was monitored by liquid chromatography-mass spectrometry and thin layer chromatography. After the reaction was completed, the reaction mixture was concentrated under reduced pressure to obtain a crude product. The obtained crude product was purified by silica gel column chromatography, eluted with a gradient of 0%→6% methanol/dichloromethane mobile phase, and the obtained fractions were evaporated under reduced pressure to remove the solvent to obtain compound **254-3** (brown solid, 96.4 mg, yield: 56%). MS (ESI, m/z): 702.3 [M + H]⁺.

### Step 4:

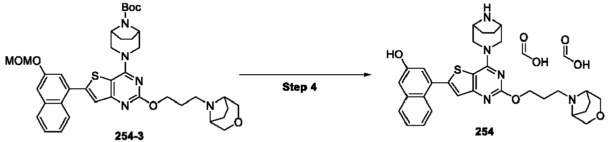

A solution of hydrochloric acid (4 mol/L, 2 mL) in 1,4-dioxane was added dropwise to a solution of the compound **254-3** (90 mg, 0.127 mmol, 1.00 eq) in methanol (2 mL) with stirring at 0 °C. The reaction was carried out for 1 hour at room temperature, and the reaction process was monitored by liquid chromatography-mass spectrometry and thin layer chromatography. After the reaction was completed, the reaction mixture was concentrated to obtain a crude product. The crude product was dissolved in 2 mL of methanol, and a solution of ammonia (7 mol/L, 2 mL) in methanol was then added thereto. The mixture was stirred at room temperature for 5 min and then concentrated under reduced pressure to obtain a crude product. The crude product was redissolved in 2 mL of methanol and then formic acid (0.2 mL) was added. The mixture was stirred at room temperature for 5 min and then concentrated under reduced pressure to obtain a crude product. The obtained crude product was purified by reversed-phase rapid chromatographic column (C18 column), and eluted with 5%→ 95% (acetonitrile/methanol= 1/ 1)/water mobile phase (0.1% formic acid) in 20 min; detector, UV254 nm; to obtain compound **254** (light yellow solid, 54.6 mg, yield: 65%). MS (ESI, *m*/*z*): 558.1[M + H]⁺; ¹H NMR (300 MHz, DMSO-d₆) δ 8.30 - 8.22 (m, 2H), 8.07 (d, *J* = 8.5 Hz, 1H), 7.89 (d, *J* = 8.2 Hz, 1H), 7.60 - 7.52 (m, 2H), 7.48 - 7.39 (m, 1H), 7.38 - 7.33 (m, 2H), 4.54 - 4.40 (m, 4H), 4.04 - 3.87 (m, 2H), 3.68 - 3.57 (m, 2H), 3.58 - 3.44 (m, 4H), 3.26 - 3.17 (m, 2H), 2.54 - 2.44 (m, 2H), 2.05 - 1.73 (m, 10H).

### Embodiment 62

### (4S or 4R)-4-(2-(3-(3-oxa-8-azabicyclo[3.2.1]octan-8-yl)propoxy)-4-(1R,5S)-3,8-diazabicyclo[3.2.1]octan-3-yl)-6-chloro-8-fluoroquinazolin-7-yl)-5-ethynyl-6-fluoronaphthalen-2-ol diformate 255a; (4R or 4S)-4-(2-(3-(3-oxa-8-azabicyclo[3.2.1]octan-8-yl)propoxy)-4-(1R,5S)-3,8-diazabicyclo[3.2.1]octan-3-yl)-6-chloro-8-fluoroquinazolin-7-yl)-5-ethynyl-6-fluoronaphthalen-2-ol diformate 255b.

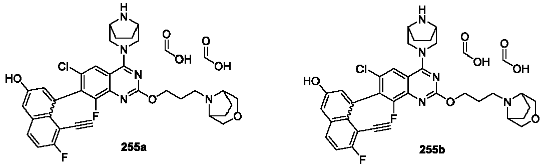

The synthetic route was as follows:

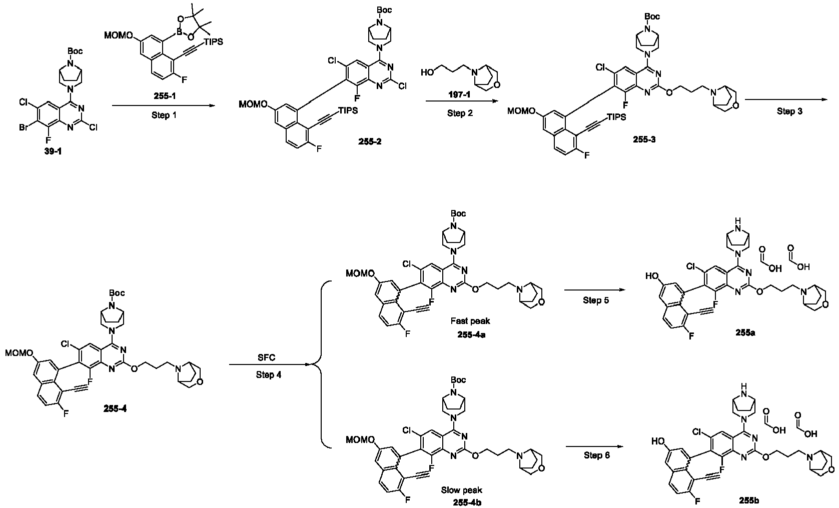

### Step 1:

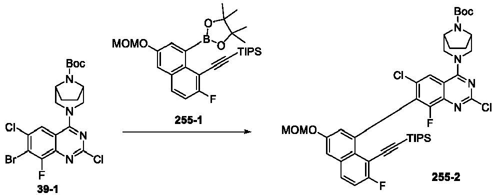

Compound **39-1** (2.0 g, 3.753 mmol, 1.0 eq), compound **255-1** (synthesized with reference to patent WO2021041671, 2.63 g, 4.879 mmol, 1.2 eq), cesium carbonate (2.47 g, 7.506 mmol, 2.0 eq), (*S*)-4-(9-anthryl)-3-(*tert*-butyl)-2,3-dihydrobenzo[*d*][1,3]oxaphosphole (0.28 g, 0.751 mmol, 0.1 eq), tris(dibenzylideneacetone)dipalladium (0.35 g, 0.375 mmol, 0.1 eq), toluene (20.0 mL) and water (4.0 mL) were successively added to the reaction flask under the protection of nitrogen at 25 °C. The reaction was carried out for 36 hours at 80 °C, and the reaction process was monitored by liquid chromatography-mass spectrometry and thin layer chromatography. After the reaction was completed, the temperature was lowered to 25 °C, and the reaction solution was concentrated under reduced pressure to remove excess reagents to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **255-2** (white solid, 800 mg, yield: 24%). MS (ESI, m/z): 811.1/813.1[M + H]+.

### Step 2:

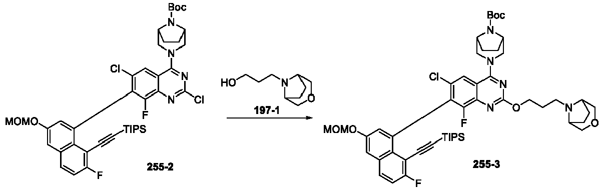

1 mol/L of tetrahydrofuran solution of potassium *tert*-butoxide (0.56 mL, 0.562 mmol, 1.2 eq) was added to a solution of compound **255-2** (400.0 mg, 0.468 mmol, 1.0 eq) and compound **197-1** (101.24 mg, 0.562 mmol, 1.2 eq) in anhydrous tetrahydrofuran (4.0 mL) with stirring under the protection of nitrogen at 25 °C. The reaction was carried out for 2 hours at 0 °C, and the reaction process was monitored by liquid chromatography-mass spectrometry and thin layer chromatography. After the reaction was completed, the reaction mixture was concentrated under reduced pressure to remove the excess reagent to obtain a crude product. The crude product was purified by silica gel column chromatography, eluted with a gradient of 0%→10% methanol/dichloromethane mobile phase, and the obtained fractions were evaporated under reduced pressure to remove the solvent to obtain compound **255-3** (white solid, 280 mg, yield: 61%). MS (ESI, *m*/*z*): 946.1/948.1[M + H]⁺.

### Step 3:

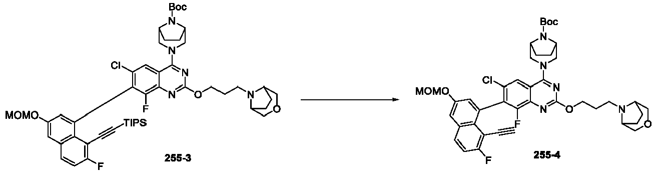

Compound **255-3** (250 mg, 0.259 mmol, 1.0 eq), cesium fluoride (198.55 mg, 1.295 mmol, 5.0 eq) and *N,N*-dimethylformamide (3.0 mL) were successively added to a reaction flask with stirring under the protection of nitrogen at 25 °C. The reaction was carried out for 1 hour at 25 °C, and the reaction process was monitored by liquid chromatography-mass spectrometry and thin layer chromatography. After the reaction was completed, the reaction solution was directly purified by reversed-phase rapid chromatographic column (C18 column), and eluted with 5%→ 95% acetonitrile/water mobile phase (0.1% ammonium bicarbonate) in 25 min; detector, UV254 nm; compound **255-4** (white solid, 200 mg, yield: 97%) was obtained. MS (ESI, *m*/*z):* 790.0/792.0[M + H]⁺.

### Step 4:

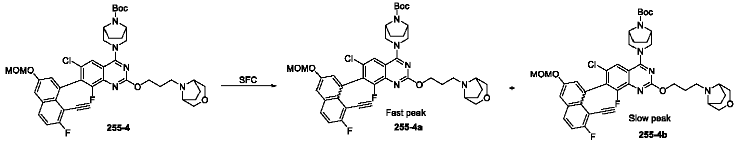

The compound **255-4** (200.0 mg) obtained in step 3 was subjected to chiral resolution by preparative supercritical liquid chromatography: CHIRAL ART Cellulose-SC, 3 × 25 cm, 5 µm; mobile phase A: supercritical carbon dioxide fluid, mobile phase B: ethanol (0.5% 2mol/L ammonium methanol); flow rate: 70 mL/min; eluted with 50% phase B in 14 min, detector: UV 225 nm, two products were obtained. The product with shorter retention time (9.08 min) was compound **255-4a** (white solid, 75.0 mg, recovery rate: 38%), compound **255-4a,** MS (ESI, m/z): 790.0/792.0[M + H]⁺; the product with longer retention time (11.95 min) was compound **255-4b** (white solid, 75.0 mg, recovery rate: 38%), compound **255-4b:** MS (ESI, m/z): 790.0/792.0[M + H]⁺.

### Step 5:

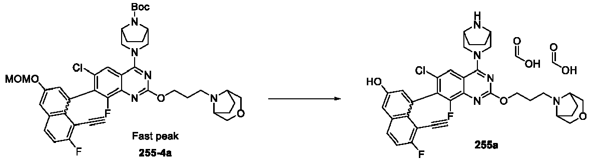

A solution of hydrochloric acid (4 mol/L, 1 mL) in 1,4-dioxane was added dropwise to a solution of the compound **255-4a** (70.0 mg, 0.088 mmol, 1.0 eq) in methanol (1 mL) with stirring at 0 °C. The reaction was carried out for 1 hour at room temperature, and the reaction process was monitored by liquid chromatography-mass spectrometry and thin layer chromatography. After the reaction was completed, the reaction mixture was concentrated to obtain a crude product. The obtained crude product was purified by reversed-phase rapid chromatographic column (C18 column), and eluted with 5%→ 95% methanol/water mobile phase (0.1% formic acid) in 25 min; detector, UV254 nm; to obtain compound **255a** (off-white solid, 40 mg, yield: 63%). MS (ESI, m/z): 646.1/648.1[M + H]⁺; ¹H NMR (300 MHz, DMSO-d₆) δ 8.27 - 8.17 (m, 2H), 8.02 - 7.95 (m, 1H), 7.79 (d, *J* = 1.6 Hz, 1H), 7.53 - 7.43 (m, 1H), 7.40 (d, *J* = 2.6 Hz, 1H), 7.06 (d, *J* = 2.5 Hz, 1H), 4.49 - 4.23 (m, 4H), 3.92 (d, *J* = 1.1 Hz, 1H), 3.64 - 3.57 (m, 2H), 3.55 - 3.47 (m, 3H), 3.43 - 3.35 (m, 3H), 3.12 - 3.04 (m, 2H), 2.44 - 2.33 (m, 2H), 1.93 - 1.63 (m, 10H); ¹⁹F NMR (282 MHz, DMSO-*d*₆) δ-110.35, - 122.69. The chiral analysis conditions of compound **255a** were: N-CHIRALPAK IG-3, 3.0 × 100 mm, 3 µm; mobile phase A: supercritical carbon dioxide fluid; mobile phase B: isopropanol (10 mmol/L ammonia); flow rate: 2 mL/min; isocratic elution with 50% phase B in 6 min, detector UV 220 nm; retention time: 0.845 min. *dr >* 40:1.

### Step 6:

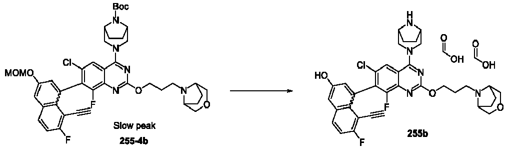

Compound **255b** (off-white solid) was obtained by using compound **255-4b** as raw material with reference to the method of **step 5.** MS (ESI, m/z): 646.1/648.1[M + H]⁺; ¹H NMR (300 MHz, DMSO-d₆) *δ* 8.23 (d, *J* = 1.6 Hz, 2H), 8.03 - 7.93 (m, 1H), 7.79 (d, *J* = 1.7 Hz, 1H), 7.53 - 7.42 (m, 1H), 7.39 (d, *J* = 2.6 Hz, 1H), 7.06 (d, *J* = 2.5 Hz, 1H), 4.48 - 4.23 (m, 4H), 3.92 (d, *J* = 1.1 Hz, 1H), 3.78 - 3.60 (m, 4H), 3.57 - 3.47 (m, 3H), 3.44 - 3.35 (m, 2H), 3.12 - 3.02 (m, 2H), 2.43 - 2.34 (m, 2H), 1.93 - 1.63 (m, 10H); ¹⁹F NMR (282 MHz, DMSO-d₆) δ-110.37, -122.66. The chiral analysis conditions of compound **255b** were: N-CHIRALPAK IG-3, 3.0 × 100 mm, 3 µm; mobile phase A: supercritical carbon dioxide fluid; mobile phase B: isopropanol (10 mmol/L ammonia); flow rate: 2 mL/min; isocratic elution with 50% phase B in 6 min, detector UV 220 nm; retention time: 1.905 min. *dr* > 40:1.

### Embodiment 63

### (4-((7S or 7R)-2-((2R)-3-(3-oxa-8-azabicyclo[3.2.1]octan-8-yl)-2-methylpropoxy)-4-((1R,5S)-3,8-diazabicyclo[3.2.1]octan-3-yl)-6-chloro-8-fluoroquinazolin-7-yl)naphthalen-2-yl)boronic acid ditrifluoroacetate 256

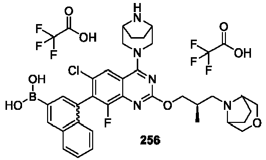

The synthetic route was as follows:

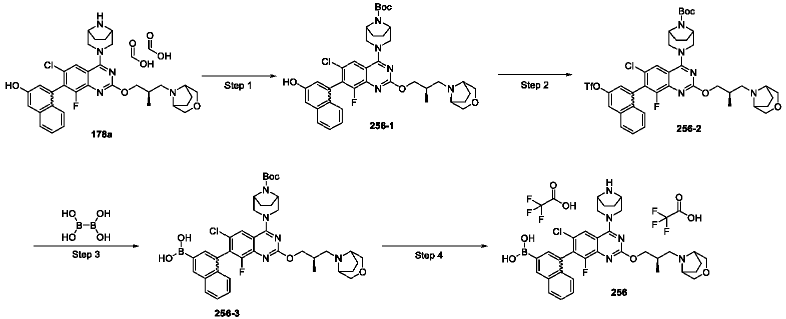

### Step 1:

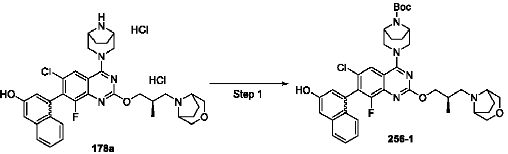

Di-*tert*-butyl dicarbonate (64.5 mg, 0.28 mmol, 1.1 eq) was added to a solution of compound **178a dihydrochloride** (172.8 mg, 0.25 mmol, 1.0 eq) and *N,N-*diisopropylethylamine (138.9 mg, 1.0 mmol, 4.0 eq) in dichloromethane (2 mL) with stirring at 0 °C. The reaction was carried out for 2 hours at 25 °C, and the reaction process was monitored by liquid chromatography-mass spectrometry and thin layer chromatography. After the reaction was completed, the reaction mixture was concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **256-1** (white solid, 180 mg, yield: 93%). MS (ESI, *m*/*z):* 718.1/720.1[M + H]⁺.

### Step 2:

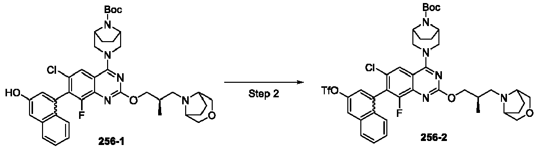

Trifluoromethanesulfonic anhydride (75.4 mg, 0.25 mmol, 1.2 eq) was added to a solution of compound **256-1** (160.0 mg, 0.2 mmol, 1.0 eq) and *N,N*-diisopropylethylamine (86.3 mg, 0.6 mmol, 3.0 eq) in anhydrous dichloromethane (2 mL) with stirring under the protection of nitrogen at 0 °C. The reaction was carried out for 2 hours at -78 °C, and the reaction process was monitored by liquid chromatography-mass spectrometry and thin layer chromatography. After the reaction was completed, the reaction mixture was concentrated under reduced pressure to obtain a crude product, the obtained crude product was purified by silica gel column chromatography, eluted with a gradient of 0%→10% methanol/dichloromethane mobile phase, and the obtained fractions were evaporated under reduced pressure to remove the solvent to obtain compound **256-2** (white solid, 160 mg, yield: 84%). MS (ESI, *m*/*z*): 850.0/852.0[M + H]⁺.

### Step 3:

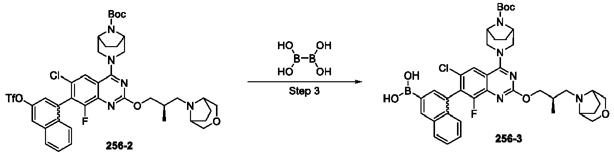

Compound **256-2** (60.0 mg, 0.06 mmol, 1.0 eq), tetrahydroxydiborane (10.0 mg, 0.1 mmol, 1.5 eq), 1,1'-bis(diphenylphosphino)ferrocene-palladium(II)dichloride dichloromethane complex (5.7 mg, 0.007 mmol, 0.1 eq) and potassium acetate (20.8 mg, 0.2 mmol, 3.0 eq) were dissolved in methanol (2 mL) with stirring under the protection of nitrogen at 25 °C. The reaction was carried out for 8 hours at 40 °C, and the reaction process was monitored by liquid chromatography-mass spectrometry and thin layer chromatography. After the reaction was completed, the crude product was purified by reversed-phase chromatographic column (C18 column), eluted with 5%→95% acetonitrile/water (0.1% trifluoroacetic acid) mobile phase in 25 min; detector: UV254/220 nm; compound **256-3** (white solid, 30 mg, yield: 57%) was obtained. MS (ESI, *m*/*z*): 746.1/748.1[M + H]⁺.

### Step 4:

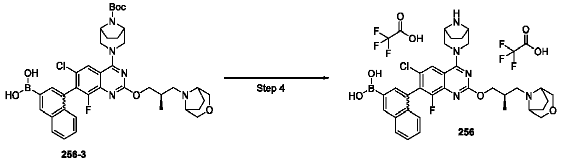

Trifluoroacetic acid (1 mL) was added to a solution of compound **256-3** (30.0 mg, 0.038 mmol, 1.0 eq) in dichloromethane (3 mL) with stirring at 0 °C. The reaction was carried out for 1 hour at 25 °C, and the reaction process was monitored by liquid chromatography-mass spectrometry and thin layer chromatography. After the reaction was completed, the reaction solution was concentrated under reduced pressure to obtain a crude product, and the crude product was purified by reversed-phase chromatography (C18 column) and eluted with 5% → 95% acetonitrile/water mobile phase (0.1% trifluoroacetic acid) in 25 min; detector, UV254/220 nm; compound **256** was obtained (white solid, 18 mg, yield: 52%). MS (ESI, m/z): 646.1/648.1[M + H]⁺; ¹H NMR (400 MHz, DMSO-*d*₆) *δ* 8.55 (s, 1H), 8.08 (d, *J* = 8.1 Hz, 1H), 8.02 (d, *J* = 1.6 Hz, 1H), 7.80 (d, *J* = 1.1 Hz, 1H), 7.64 - 7.58 (m, 1H), 7.57 - 7.51 (m, 1H), 7.35 (d, *J* = 8.3 Hz, 1H), 4.52 (d, *J* = 13.5 Hz, 2H), 4.42 - 4.31 (m, 2H), 4.24 - 4.17 (m, 2H), 4.14 - 4.09 (m, 1H), 4.07 - 4.01 (m, 1H), 3.99 - 3.94 (m, 2H), 3.85 - 3.81 (m, 1H), 3.80 - 3.76 (m, 1H), 3.76 - 3.68 (m, 2H), 3.15 - 2.98 (m, 2H), 2.25 - 2.15 (m, 2H), 2.15 - 2.09 (m, 1H), 2.09 - 2.03 (m, 2H), 2.02 - 1.92 (m, 4H), 1.17 - 1.05 (m, 3H); ¹⁹F NMR (377 MHz, DMSO-*d*₆) δ-73.97, -122.06.

### Embodiment 64

### 4-(2-(4-(3-Oxa-8-azabicyclo[3.2.1]octan-8-yl)butyl)-3-(3,8-diazabicyclo[3.2.1]octan-3-yl)-5-chloro-7-fluoro-2H-indazol-6-yl)naphthalen-2-ol dihydrochloride 276

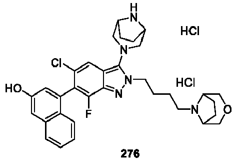

The synthetic route was as follows:

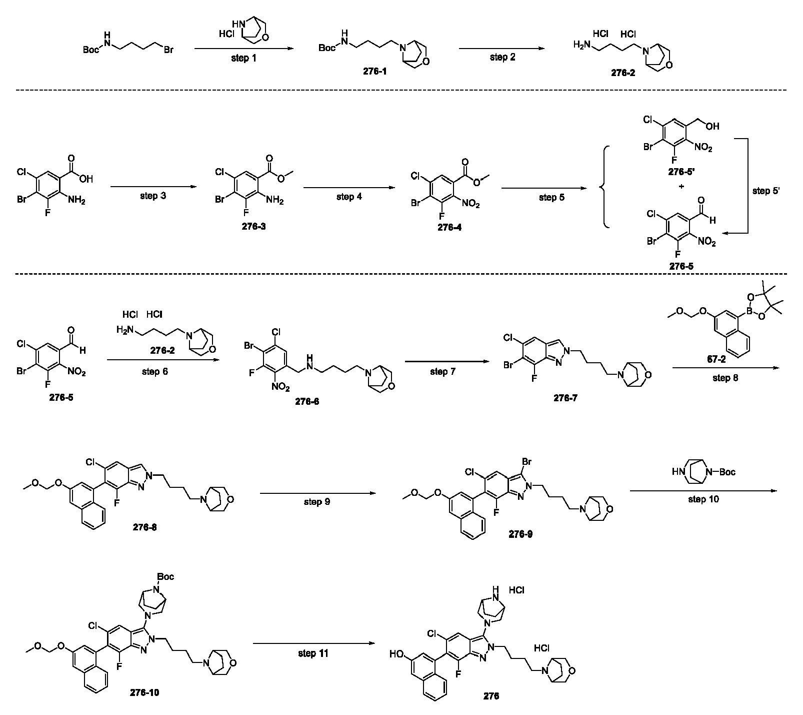

### Step 1:

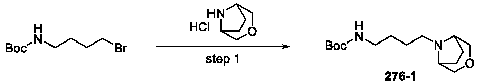

Potassium carbonate (9.87 g, 67.815 mmol, 3 eq) was slowly added to a solution of 3-oxa-8-azabicyclo[3.2.1]octane hydrochloride (3.56 g, 22.605 mmol, 1 eq) and *tert*-butyl *N-(4-*bromobutyl)carbamate (6 g, 22.605 mmol, 1.00 eq) in acetonitrile (60 mL) with stirring at 25 °C. The reaction was carried out for 16 hours at 60 °C, and the reaction process was monitored by liquid chromatography-mass spectrometry and thin layer chromatography. After the reaction was completed, the reaction mixture was cooled to room temperature. The reaction solution was filtered to remove the insolubles, and the filter cake was washed with acetonitrile (40 mL × 3), and the filtrate was concentrated under reduced pressure to obtain a crude product. The obtained crude product was purified by silica gel column chromatography, eluted with a gradient of 0% → 10% (methanol/7 mol/L of ammonia methanol = 10/1)/dichloromethane mobile phase, and the obtained fraction was purified by rotary evaporation under reduced pressure to remove the solvent to obtain compound **276-1** (yellow liquid, 4.2 g, yield: 62%). MS (ESI, m/z): 285.3[M + H]⁺; ¹H NMR (300 MHz, CDCl₃) δ 5.50 (s, 1H), 3.79 (d, *J* = 10.6 Hz, 2H), 3.54 - 3.46 (m, 2H), 3.17 - 3.02 (m, 4H), 2.38 - 2.28 (m, 2H), 1.95 - 1.83 (m, 4H), 1.60 - 1.49 (m, 4H), 1.43 (s, 9H).

### Step 2

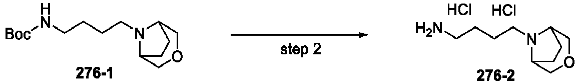

A solution of hydrochloric acid (4 mol/L, 42 mL) in 1,4-dioxane was added dropwise to a solution of compound **276-1** (4.2 g, 14.0308 mmol, 1 eq) in methanol (42 mL) with stirring at 25 °C. The reaction was carried out for 1 hour at room temperature, and the reaction process was monitored by liquid chromatography-mass spectrometry and thin layer chromatography. After the reaction was completed, the reaction solution was concentrated under reduced pressure to remove the solvent to obtain compound **276-2** (white solid, 3.47 g, crude product). MS (ESI, m/z): 185.2[M + H]⁺.

### Step 3:

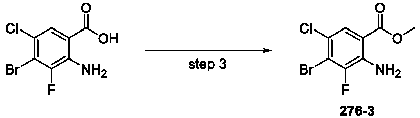

Thionyl chloride (30 mL, 392.872 mmol, 7.40 eq) was slowly added to a solution of compound 2-amino-4-bromo-3-fluoro-5-chlorobenzoic acid (15 g, 53.079 mmol, 1 eq) in methanol (200 mL) with stirring under the protection of nitrogen at 0 °C. The reaction was carried out at 65 °C for 24 hours, and the reaction process was monitored by liquid chromatography-mass spectrometry and thin layer chromatography. After the reaction was completed, the reaction mixture was cooled to room temperature. The reaction mixture was concentrated under reduced pressure to obtain a crude product. The crude product was purified by silica gel column chromatography, and the mobile phase was eluted with a gradient of 4% → 15% ethyl acetate/petroleum ether mobile phase, and the obtained fractions were evaporated under reduced pressure to remove the solvent to obtain compound **276-3** (white solid, 12.98 g, yield: 82%). MS (ESI, m/z): 281.9/283.9[M + H]⁺; ¹H NMR (300 MHz, CDCl₃) *δ* 7.80 (d, *J* = 2.1 Hz, 1H), 5.89 (s, 2H), 3.92 (s, 3H).

### Step 4:

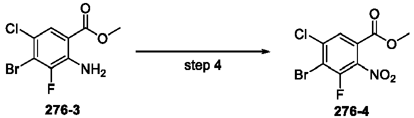

A solution of compound **276-3** (12.98 g, 43.651 mmol, 1 eq) in dichloromethane (85 mL) was slowly added to a solution of hydrogen peroxide (30%, 49.49 g, 436.510 mmol, 10 eq) and trifluoroacetic anhydride (112.91 g, 510.717 mmol, 11.7 eq) in dichloromethane (85 mL) with stirring at 25 °C. The reaction was carried out at 50 °C for 4 hours, and the reaction process was monitored by liquid chromatography-mass spectrometry and thin layer chromatography. After the reaction was completed, the reaction mixture was cooled to room temperature. The reaction mixture was concentrated under reduced pressure to obtain a crude product. The crude product was purified by silica gel column chromatography, and the mobile phase was eluted with a gradient of 0% → 8% ethyl acetate/petroleum ether mobile phase, and the obtained fractions were evaporated under reduced pressure to remove the solvent to obtain compound **276-4** (blue-green solid, 12.51 g, yield: 87%). ¹H NMR (300 MHz, CDCl₃) δ 7.96 (d, *J* = 2.0 Hz, 1H), 3.94 (s, 3H).

### Step 5

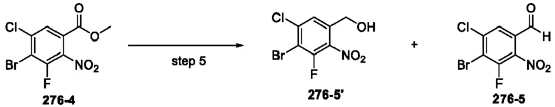

A solution of diisobutylaluminium hydride (1.5 mol/L, 37.5 mL, 56.3 mmol, 1.5 eq) in toluene was slowly added to a solution of compound **276-4** (12.34 g, 37.516 mmol, 1 eq) in dichloromethane (120 mL) with stirring under the protection of nitrogen at -78 °C. The reaction was carried out for 1.5 hours at -78 °C, and the reaction process was monitored by liquid chromatography-mass spectrometry and thin layer chromatography. After the reaction was completed, the reaction was quenched by adding saturated potassium sodium tartrate solution (120 mL) to the reaction flask at 0 °C. The aqueous phase was extracted with dichloromethane (150 mL × 3), and the organic phases were combined, then the organic phase was dried over anhydrous sodium sulfate, filtered to remove the drying agent, and the filtrate was concentrated under reduced pressure to obtain a crude product. The crude product was purified by silica gel column chromatography, and the mobile phase was eluted with a gradient of 0% → 23% ethyl acetate/petroleum ether mobile phase, and the obtained fractions were evaporated under reduced pressure to remove the solvent to obtain compound **276-5** (light yellow solid, 2.3 g, yield: 20%) and compound **276-5'** (light yellow solid, 8.2 g, yield: 72%).

Compound **276-5:** ¹H NMR (300 MHz, CDCl₃) δ 10.01 - 9.98 (m, 1H), 7.92 (d, *J* = 1.9 Hz, 1H).

Compound **276-5':** ¹H NMR (300 MHz, CDCl₃) *δ* 7.67 - 7.64 (m, 1H), 4.80 (s, 2H), 2.07 (s, 1H).

### Step 5:

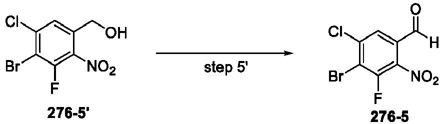

Silica gel (15 g) was added to a solution of compound **276-5'** (8.19 g, 27.351 mmol, 1.00 eq) in dichloromethane (80 mL) with stirring at 25 °C, then pyridinium chlorochromate (12.41 g, 54.702 mmol, 2 eq) was slowly added thereto. The reaction was carried out for 2 hours at 25 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 3% → 15% ethyl acetate/petroleum ether mobile phase, and the obtained fractions were evaporated under reduced pressure to remove the solvent to obtain compound **276-5** (light yellow solid, 5.254 g, yield: 64%). ¹H NMR (300 MHz, CDCl₃) *δ* 10.01 - 9.98 (m, 1H), 7.92 (d, *J* = 1.9 Hz, 1H).

### Step 6:

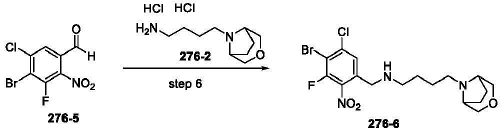

Compound **276-5** (4 g, 13.454 mmol, 1 eq) was slowly added to a solution of compound **276-2** (3.13 g, 16.145 mmol, 1.2 eq) and sodium acetate (2.79 g, 32.290 mmol, 2.4 eq) in methanol (40 mL) with stirring at 25 °C. The reaction was carried out at 60 °C for 2 hours. The reaction mixture was cooled to 0 °C, acetic acid (0.82 g, 13.454 mmol, 1 eq) and sodium cyanoborohydride (4.45 g, 67.270 mmol, 5 eq) were added to the reaction flask. The reaction was carried out for 2 hours at 60 °C, and the reaction process was monitored by liquid chromatography-mass spectrometry and thin layer chromatography. After the reaction was completed, the reaction mixture was cooled to room temperature. The reaction mixture was concentrated under reduced pressure to obtain a crude product. The crude product was purified by reversed-phase chromatographic column (C18 column), eluted with 40%→95% methanol/water mobile phase (0.1% ammonium bicarbonate) in 20 min; detector, UV 254/220 nm; to obtain compound **276-6** (brown oil, 3.5 g, yield: 62%). MS (ESI, m/z): 450.1/452.1[M + H]⁺.

### Step 7

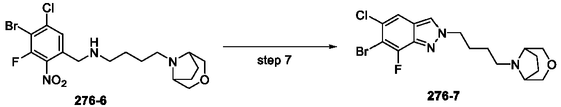

Zinc powder (2.58 g, 37.2 mmol, 5 eq) was added to a solution of compound **276-6** (3.34 g, 7.44 mmol, 1 eq) in methanol (35 mL) with stirring at 0 °C, and a solution of ammonium acetate (0.573 g, 7.44 mmol, 1 eq) in methanol (5 mL) was slowly added thereto. The reaction was carried out at 25 °C for 2 hours, and the reaction process was monitored by liquid chromatography-mass spectrometry and thin layer chromatography. After the reaction was completed, the reaction solution was filtered to remove the insolubles, and the filter cake was washed with methanol (50 mL × 3), then the filtrate was combined and concentrated under reduced pressure to obtain a crude product. The crude product was purified by reversed-phase chromatographic column (C18 column), eluted with 30%→55% methanol/water mobile phase (0.1% trifluoroacetic acid) in 30 min; detector, UV254/220 nm; to obtain compound **276-7** (yellow oil, 1 g, yield: 33%). MS (ESI, m/z): 415.9/417.9[M + H]⁺.

### Step 8

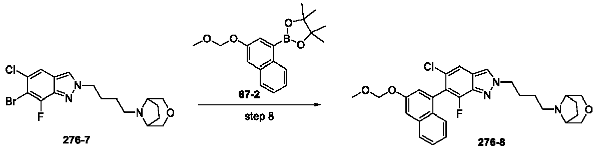

Tris(dibenzylideneacetone)dipalladium (0.22 g, 0.228 mmol, 0.1 eq) was added to a solution of compound **276-7** (1 g, 2.280 mmol, 1 eq), compound **67-2** (0.98 g, 2.964 mmol, 1.3 eq), 2-dicyclohexylphosphorus-2,4,6-triisopropylbiphenyl (0.23 g, 0.456 mmol, 0.2 eq) and cesium carbonate (2.35 g, 6.840 mmol) in 1,4-dioxane (12.5 mL)/water (2.5 mL) with stirring under the protection of nitrogen at 25 °C. The reaction was carried out for 2 hours at 80 °C, and the reaction process was monitored by liquid chromatography-mass spectrometry and thin layer chromatography. After the reaction was completed, the reaction mixture was cooled to room temperature, and concentrated under reduced pressure to obtain a crude product. The obtained crude product was purified by silica gel column chromatography, eluted with a gradient of 0%→6% methanol/dichloromethane mobile phase, and the obtained fractions were evaporated under reduced pressure to remove the solvent to obtain compound **276-8** (white solid, 500 mg, yield: 40%). MS (ESI, *m*/*z):* 524.1/526.1 [M + H]⁺.

### Step 9

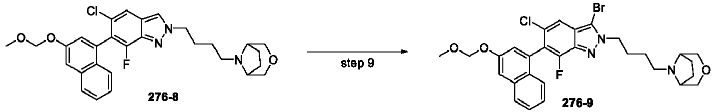

N-butyl lithium (2.5 mol/L, 0.62 mL, 1.546 mmol, 2.0 eq) was slowly added to a solution of compound **276-8** (450.0 mg, 0.773 mmol, 1.0 eq) in anhydrous tetrahydrofuran (4 mL) with stirring under the protection of nitrogen at -40 °C. The mixture was stirred at -40°C for 2 hours. The reaction solution was then reduced to -78 °C, and a solution of cyanogen bromide (172.34 mg, 1.546 mmol, 2.0 eq) in anhydrous tetrahydrofuran (2 mL) was slowly added to the reaction solution. The reaction was carried out for 1 hour at -78 °C, and the reaction process was monitored by liquid chromatography-mass spectrometry and thin layer chromatography. After the reaction was completed, the reaction solution was poured into 30 mL of ice water, and the aqueous phase was extracted with ethyl acetate (30 mL × 3), and the organic phases were combined; the organic phase was dried over anhydrous sodium sulfate, then filtered to remove the drying agent, and the filtrate was concentrated under reduced pressure to obtain a crude product. The crude product was purified by silica gel column chromatography, eluted with a gradient of 0% → 6% methanol/dichloromethane mobile phase, and the fraction was evaporated under reduced pressure to obtain a crude product. The crude product was purified by high performance liquid chromatography: chromatographic column Sunfire prep C18 column, 30 × 150 mm, 5 µm; mobile phase A: water (0.1% formic acid), mobile phase B: acetonitrile; flow rate: 60 mL/min; eluted with 28% → 45% phase B in 7 min; detector UV 220/254 nm; compound **276-9** (white solid, 392 mg, yield: 82%) was obtained. MS (ESI, *m*/*z):* 602.1/604.1[M + H]⁺; ¹H NMR (400 MHz, CDCl₃) δ 7.83 (d, *J* = 8.2 Hz, 1H), 7.57 - 7.50 (m, 2H), 7.48 - 7.43 (m, 1H), 7.40 - 7.36 (m, 1H), 7.31 - 7.27 (m, 1H), 7.21 (d, *J* = 2.4 Hz, 1H), 5.34 (d, *J* = 1.0 Hz, 2H), 4.59 (t, *J* = 7.2 Hz, 2H), 3.91 - 3.70 (m, 2H), 3.56 (s, 3H), 3.55 - 3.49 (m, 2H), 3.17 - 3.02 (m, 2H), 2.49 - 2.29 (m, 2H), 2.18 - 2.06 (m, 2H), 1.96 - 1.84 (m, 4H), 1.66 - 1.58 (m, 2H).

### Step 10

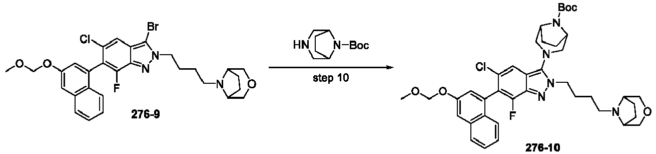

Compound **276-9** (300 mg, 0.473 mmol, 1 eq), *tert*-butyl 3,8-diazabicyclo[3.2.1]octane-8-carboxylate (211.26 mg, 0.946 mmol, 2 eq), 1,1'-binaphthyl-2.2'-diphenyl phosphine (61.97 mg, 0.095 mmol, 0.2 eq), tris(dibenzylideneacetone)dipalladium (45.56 mg, 0.047 mmol, 0.1 eq), sodium tert-butoxide (95.64 mg, 0.96 mmol, 2 eq) and anhydrous toluene (5 mL) were successively added to a reaction flask with stirring under the protection of nitrogen at 25 °C. The reaction was carried out for 5 hours at 80 °C, and the reaction process was monitored by liquid chromatography-mass spectrometry and thin layer chromatography. After the reaction was completed, the reaction mixture was cooled to room temperature. The reaction mixture was concentrated under reduced pressure to obtain a crude product. The obtained crude product was purified by silica gel column chromatography, eluted with a gradient of 0%→6% methanol/dichloromethane mobile phase, and the obtained fractions were evaporated under reduced pressure to remove the solvent to obtain compound **276-10** (yellow solid, 30 mg, yield: 8%). MS (ESI, *m*/*z):* 734.3/736.3 [M + H]⁺.

### Step 11

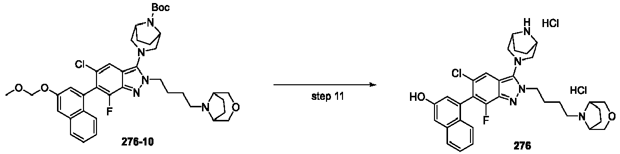

A solution of hydrochloric acid (4 mol/L, 1 mL) in 1,4-dioxane was added dropwise to a solution of compound **276-10** (28 mg, 0.036 mmol, 1 eq) in methanol (1 mL) with stirring at 0 °C. The reaction was carried out for 1.5 hours at room temperature, and the reaction process was monitored by liquid chromatography-mass spectrometry and thin layer chromatography. After the reaction was completed, the crude product was purified by reversed-phase chromatographic column (C18 column), eluted with 5%→95% acetonitrile/water (0.1% hydrochloric acid) mobile phase in 25 min; detector: UV254/220 nm; compound **276** (white solid, 14.9 mg, yield: 62%) was obtained. MS (ESI, *m*/*z):* 590.3/592.3[M + H]⁺; ¹H NMR (300 MHz, DMSO-d₆) δ 11.02 - 10.77 (m, 1H), 10.13 - 9.88 (m, 2H), 9.59 - 9.39 (m, 1H), 8.33 - 8.20 (m, 1H), 7.80 (d, *J* = 8.3 Hz, 1H), 7.51 - 7.36 (m, 1H), 7.27 (d, *J* = 2.4 Hz, 1H), 7.23 - 7.17 (m, 2H), 7.07 (d, *J* = 2.4 Hz, 1H), 4.55 - 4.40 (m, 2H), 4.27 - 4.07 (m, 4H), 4.06 - 3.88 (m, 4H), 3.71 - 3.59 (m, 2H), 3.18 - 3.04 (m, 2H), 3.04 - 2.91 (m, 2H), 2.34 - 1.91 (m, 10H), 1.88 - 1.72 (m, 2H); ¹⁹F NMR (282 MHz, DMSO-d₆) δ-126.00.

### Embodiment 65

### 4-((7R or 7S)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)-1-methylnaphthalen-2-ol formate 277a; 4-((7S or 7R)-2-(3-(3-oxa-8-azabicyclo[3.2.1]octan-8-yl)propoxy)-4-((1R,5S)-3,8-diazabicyclo[3.2.1]octan-3-yl)-6-chloro-8-fluoroquinazolin-7-yl)-1-methylnaphthalen-2-ol formate 277b.

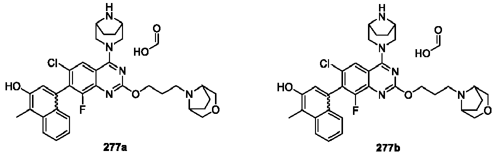

The synthetic route was as follows:

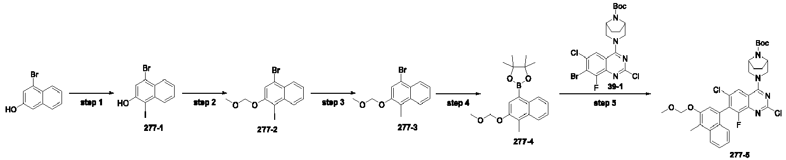

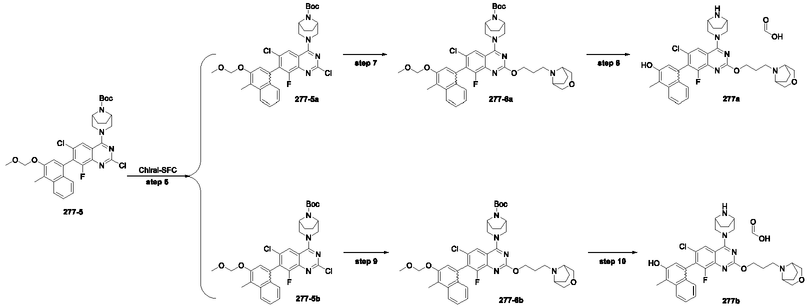

### Step 1

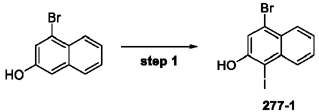

4-Bromo-2-naphthalenol (6 g, 26.360 mmol, 1 eq), *N*-iodosuccinimide (6.05 g, 26.360 mmol, 1 eq) and acetonitrile (60 mL) were successively added to a reaction flask with stirring at 0 °C. The obtained mixture was raised to 25 °C and stirred at 25 °C for 16 hours, and the reaction process was monitored by thin layer chromatography. After the reaction was completed, the reaction mixture was concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **277-1** (light yellow solid, 8 g, yield: 87%). ¹H NMR (400 MHz, CDCl₃) *δ* 8.146 - 8.13(m, 1H), 7.97 - 7.95 (m, 1H), 7.62 (s, 1H), 7.60 - 7.56 (m, 1H), 7.50 - 7.46 (m, 1H), 5.80 (s, 1H).

### Step 2

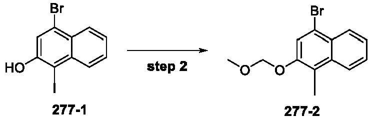

**277-1** (8 g, 21.779 mmol, 1 eq), dichloromethane (80 mL) and diisopropylethylamine (8.62 g, 65.337 mmol, 3 eq) were successively added to a reaction flask with stirring under the protection of nitrogen at 25 °C. The obtained mixture was then reduced to 0 °C. Chloromethyl methyl ether (2.33 g, 28.313 mmol, 1.3 eq) was added thereto with stirring at 0 °C. The obtained mixture was brought to 25 °C and stirred for 1 hour at 25 °C. The reaction process was monitored by thin layer chromatography. After the reaction was completed, the reaction solution was diluted with 80 mL of water, extracted with dichloromethane (80 mL × 2), and the organic phases were combined; the organic phase was washed with saturated brine (80 mL × 3), dried over anhydrous sodium sulfate, filtered to remove the drying agent, and concentrated under reduced pressure to obtain a crude product. The crude product was purified by silica gel column chromatography, eluted with a gradient of 0% → 5% ethyl acetate/petroleum ether mobile phase, and the obtained fractions were evaporated under reduced pressure to remove the solvent to obtain compound **277-2** (orange solid, 7.1 g, yield: 83%). ¹H NMR (400 MHz, CDCl₃) δ 8.22 - 8.20 (m, 1H), 8.17 - 8.13 (m, 1H), 7.73 (s, 1H), 7.60 - 7.56 (m, 1H), 7.53 - 7.49 (m, 1H), 5.35 (s, 2H), 3.58 (s, 3H).

### Step 3

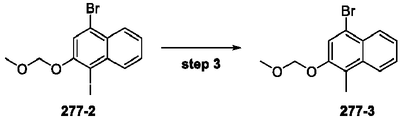

**277-2** (3 g, 7.252 mmol, 1 eq), 1,1'-bis(diphenylphosphino)ferrocene-palladium(II)dichloride dichloromethane complex (0.60 g, 0.725 mmol, 0.1 eq), cesium carbonate (7.23 g, 21.756 mmol, 3 eq), trimethylboroxine (50% tetrahydrofuran solution, 3.11 mL, 10.878 mmol, 1.5 eq) and 1,4-dioxane (30 mL) were successively added to a reaction flask with stirring under the protection of nitrogen. The mixture was raised to 60 °C, the reaction carried out for 4 hours at 60 °C, and the reaction process was monitored by liquid chromatography-mass spectrometry and thin layer chromatography. After the reaction was completed, the reaction solution was diluted with 20 mL of water, extracted with ethyl acetate (20 mL × 3), and the organic phases were combined; the organic phase was washed with saturated brine (20 mL × 3), dried over anhydrous sodium sulfate, filtered to remove the drying agent, and concentrated under reduced pressure to obtain a crude product. The crude product was purified by silica gel column chromatography, eluted with a gradient of 0% → 5% *tert-*butyl methyl ether/petroleum ether mobile phase, and the obtained fractions were evaporated under reduced pressure to remove the solvent to obtain compound **277-3** (light yellow oil, 2 g, yield: 80%). ¹H NMR (300 MHz, CDCl₃) δ 8.26 - 8.19 (m, 1H), 8.03 - 7.96 (m, 1H), 7.76 (s, 1H), 7.61 - 7.46 (m, 2H), 5.29 (s, 2H), 3.57 (s, 3H), 2.58 (s, 3H).

### Step 4

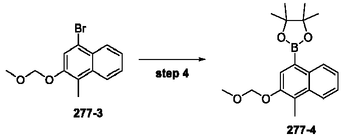

**277-3** (1.2 g, 3.500 mmol, 1 eq), bis(pinacolato)diboron (1.18 g, 4.550 mmol, 1.3 eq), potassium acetate (1.40 g, 14.000 mmol, 4 eq), 1,1'-bis(diphenylphosphino)ferrocene-palladium(II)dichloride dichloromethane complex (0.29 g, 0.350 mmol, 0.1 eq) and 1,4-dioxane (15 mL) were successively added to a reaction flask with stirring under the protection of nitrogen at 25 °C. The reaction was carried out for 2 hours at 100 °C, and the reaction process was monitored by thin layer chromatography. After the reaction was completed, the reaction mixture was filtered, and the filtrate was concentrated under reduced pressure to obtain a crude product. The crude product was purified by silica gel column chromatography, eluted with a gradient of 0% → 10% ethyl acetate/petroleum ether mobile phase, and the obtained fractions were evaporated under reduced pressure to remove the solvent to obtain compound **277-4** (light yellow solid, 600 mg, yield: 52.23%). ¹H NMR (400 MHz, CDCl₃) *δ* 8.79 - 8.73 (m, 1H), 8.02 - 7.95 (m, 1H), 7.90 (s, 1H), 7.52 - 7.41 (m, 2H), 5.33 (s, 2H), 3.56 (s, 3H), 2.63 (s, 3H), 1.42 (s, 12H).

### Step 5

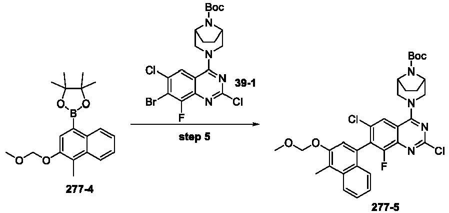

Compound **277-4** (518.72 mg, 1.5 mmol, 1 eq), compound **39-1** (800 mg, 1.5 mmol, 1 eq), 3-*tert*-butyl-4-(2,6-dimethoxyphenyl)-2*H*-1,3-benzoxaphos (50.61 mg, 0.150 mmol, 0.1 eq), potassium phosphate (650.39 mg, 3.002 mmol, 2 eq), tris(dibenzylideneacetone)dipalladium (0) (140.29 mg, 0.150 mmol, 0.1 eq), toluene (6 mL) and water (1.2 mL) were successively added to a reaction flask with stirring under the protection of nitrogen at 25 °C. The reaction was carried out for 2 hours at 75 °C, and the reaction process was monitored by liquid chromatography-mass spectrometry and thin layer chromatography. After the reaction was completed, the reaction mixture was concentrated under reduced pressure to obtain a crude product. The crude product was purified by silica gel column chromatography, eluted with a gradient of 0% → 20% ethyl acetate/petroleum ether mobile phase, and the obtained fractions were evaporated under reduced pressure to remove the solvent to obtain compound **277-5** (light yellow solid, 700 mg, yield: 73%). MS (ESI, m/z): 627.2/629.2[M + H]⁺; ¹H NMR (400 MHz, CDCl₃) *δ* 8.08 - 8.06 (m, 1H), 7.82 (d, *J* = 1.8 Hz, 1H), 7.56 - 7.51 (m, 1H), 7.37 (s, 1H), 7.36 - 7.28 (m, 2H), 5.28 (s, 2H), 4.52 (d, *J* = 12.5 Hz, 2H), 4.41 (s, 2H), 3.71 (s, 2H), 3.53 (s, 3H), 2.67 (s, 3H), 1.99 (d, *J* = 6.0 Hz, 2H), 1.80 (d, *J* = 8.3 Hz, 2H),1.53 (s, 9H).

### Step 6

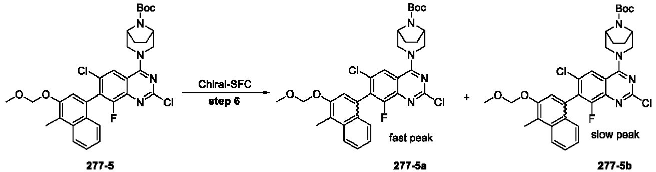

The compound **277-5** (700 mg) obtained in step 5 was subjected to chiral resolution by preparative supercritical liquid chromatography: chiral column: CHIRAL ART Cellulose-SB, 3 × 25 cm, 5 µm; mobile phase A: supercritical carbon dioxide fluid, mobile phase B: methanol (2mol/L ammonium methanol solution); flow rate: 60 mL/min; gradient: isocratic elution with 35% B phase in 12 min, detector: UV 226 nm, two products were obtained. The product with shorter retention time (8.13 min) was compound **277-5a** (light yellow solid, 300 mg, recovery rate: 43%), MS (ESI, m/z): 627.2/629.2[M + H]⁺; the product with longer retention time (9.03 min) was compound **277-5b** (light yellow solid, 280 mg, recovery rate: 40%), MS (ESI, m/z): 627.2/629.2[M + H]⁺.

### Step 7

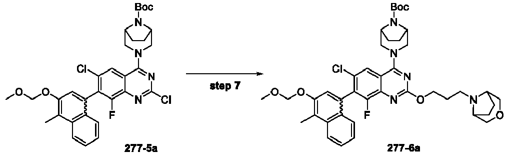

**277-5a** (120 mg, 0.182 mmol, 1 eq), 3-{3-oxa-8-azabicyclo[3.2.1]octan-8-yl}propan-1-ol (39.29 mg; 0.218 mmol; 1.2 eq) and anhydrous tetrahydrofuran (1.5 mL) were successively added to a reaction flask with stirring under the protection of nitrogen at 25 °C. The mixture was then cooled down to -15 °C. A solution of potassium *tert*-butoxide (1 mol/L, 0.22 mL, 0.218 mmol, 1.2 eq) in tetrahydrofuran was added dropwise to the reaction solution with stirring under the protection of nitrogen at -15 °C. After the addition, the reaction was carried out for 2 hours at -15 °C, and the reaction process was monitored by liquid chromatography-mass spectrometry and thin layer chromatography. After the reaction was completed, water (20 mL) was added to the reaction solution, and the mixture was extracted with ethyl acetate (20 mL × 3), and the organic phases were combined; the organic phase was washed with saturated brine (20 mL × 3), dried over anhydrous sodium sulfate, filtered to remove the drying agent, and concentrated under reduced pressure to obtain a crude product. The crude product was purified by high performance liquid chromatography: column Xselect CSH C18 OBD Column 30 × 150 mm, 5 µm; mobile phase A: water (0.05% trifluoroacetic acid), mobile phase B: acetonitrile; flow rate: 60 mL/min; gradient: elution with 30% → 60% phase B in 7 min, detector: UV254/220 nm; retention time: 6.78 min; compound **277-6a** (yellow solid, 80 mg, yield: 58%) was obtained. MS (ESI, m/z): 762.3/764.3[M+H]⁺.

### Step 8

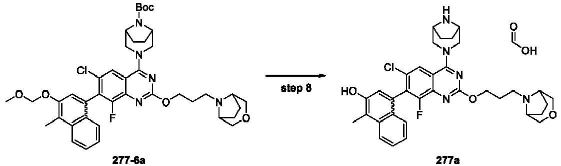

Compound **277-6a** (80 mg, 0.051 mmol, 1 eq), methanol (1.92 mL) and a solution of hydrochloric acid (4 mol/L, 1.94 mL, 7.76 mmol, 152.2 eq) in 1,4-dioxane were successively added to a reaction flask with stirring at 0 °C. The reaction was carried out for 2 hours at 0 °C, and the reaction process was monitored by liquid chromatography-mass spectrometry and thin layer chromatography. After the reaction was completed, the reaction mixture was concentrated under reduced pressure to obtain a crude product. The crude product was purified by reversed-phase chromatographic column (C18 column), mobile phase A: water (0.1% formic acid), mobile phase B: acetonitrile/methanol (1/1), eluted with 5% → 95% phase B in 25 min, detector: UV220/254 nm, and compound **277a** (white solid, 20.3 mg, yield: 31%) was obtained. MS (EIS, m/z): 618.4/620.4[M + H]⁺; ¹H NMR (300 MHz, DMSO-d₆) δ 8.31 (s, 1H), 8.01 - 7.95 (m, 1H), 7.93 (d, *J* = 1.7 Hz, 1H), 7.55 - 7.45 (m, 1H), 7.28 - 7.18 (m, 2H), 7.12 (s, 1H), 4.48 - 4.28 (m, 4H), 3.73 - 3.55 (m, 4H), 3.48 (s, 2H), 3.41 - 3.34 (m, 2H), 3.06 (d, *J* = 4.4 Hz, 2H), 2.50 (d, *J* = 1.7 Hz, 3H), 2.37 (t, *J* = 7.0 Hz, 2H), 1.93 - 1.78 (m, 4H), 1.77 - 1.61 (m, 6H); ¹⁹F NMR (282 MHz, DMSO-*d*₆) *δ* -122.36. The chiral analysis conditions of compound **277a** were: N-CHIRALPAK IG-3, 3.0 × 100 mm, 3 µm; mobile phase A: supercritical carbon dioxide fluid; mobile phase B: isopropanol (20 mmol/L ammonia); flow rate: 2 mL/min; isocratic elution with 50% phase B in 4 min, detector UV 230 nm; retention time: 2.601 min. *dr* > 40:1.

### Step 9

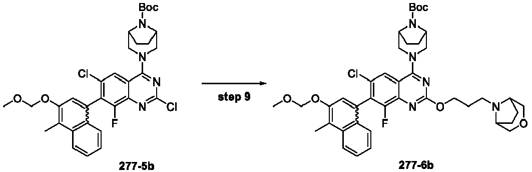

Compound **277-6b** (white solid, 90 mg) was obtained from compound **277-5b** with reference to step 7. MS (EIS, m/z): 762.1/764.1 [M + H]⁺.

### Step 10

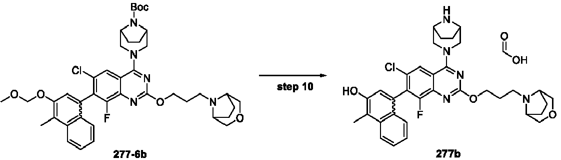

Compound **277b** (white solid, 40.8 mg) was obtained from compound **277-6b** with reference to step 8. MS (EIS, m/z): 618.4/620.4[M + H]⁺; ¹H NMR (300 MHz, DMSO-*d*₆) *δ* 8.29 (s, 1H), 7.98 (d, *J* = 8.6 Hz, 1H), 7.93 (d, *J* = 1.7 Hz, 1H), 7.56 - 7.47 (m, 1H), 7.29 - 7.19 (m, 2H), 7.13 (s, 1H), 4.45 - 4.32 (m, 4H), 3.73 (s, 2H), 3.67 - 3.58 (m, 2H), 3.49 (s, 2H), 3.43 - 3.34 (m, 2H), 3.12 - 3.02 (m, 2H), 2.50 (s, 3H), 2.38 (t, *J* = 7.1 Hz, 2H), 1.94 - 1.79 (m, 4H), 1.76 (s, 4H), 1.73 -1.61 (m, 2H); ¹⁹F NMR (282 MHz, DMSO-*d*₆) δ-122.35. The chiral analysis conditions of compound **277b** were: N-CHIRALPAK IG-3, 3.0 × 100 mm, 3 µm; mobile phase A: supercritical carbon dioxide fluid; mobile phase B: isopropanol (20 mmol/L ammonia); flow rate: 2 mL/min; isocratic elution with 50% phase B in 4 min, detector UV 230 nm; retention time: 0.815 min. *dr* > 40:1.

### Embodiment 66

### 8-((7S or 7R)-2-(3-(3-oxa-8-azabicyclo[3.2.1]octan-8-yl)propoxy)-4-((1R,5S)-3,8-diazabicyclo[3.2.1]octan-3-yl)-6,8-difluoroquinazolin-7-yl)-6-hydroxy-1-naphthonitrile diformate 278a; 8-((7R or 7S)-2-(3-(3-oxa-8-azabicyclo[3.2.1]octan-8-yl)propoxy)-4-((1R,5S)-3,8-diazabicyclo[3.2.1]octan-3-yl)-6,8-difluoroquinazolin-7-yl)-6-hydroxy-1-naphthonitrile diformate 278b

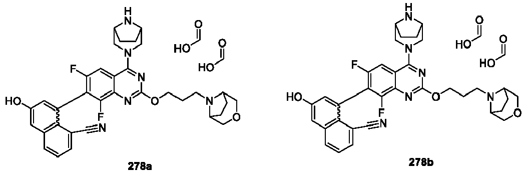

The synthetic route was as follows:

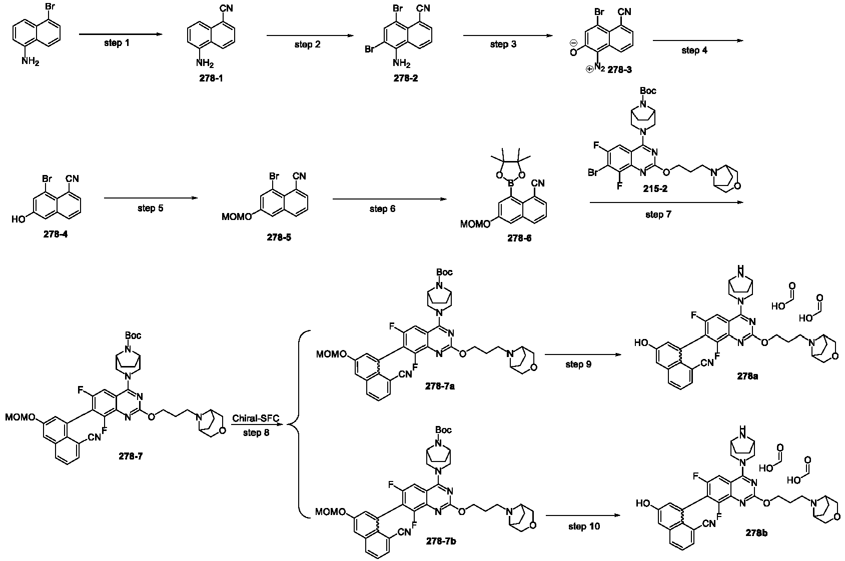

### Step 1

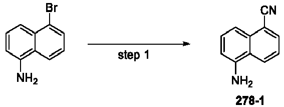

Tetrakis(triphenylphosphine)palladium (5.20 g, 4.278 mmol, 0.02 eq) was added to a mixed solution of 5-bromo-1-naphthylamine (50 g, 213.882 mmol, 1 eq), potassium ferrocyanide trihydrate (38.04 g, 85.553 mmol, 0.4 eq) and 1,8-diazabicyclo[5.4.0]undec-7-ene (8.57 g, 53.471 mmol, 0.25 eq) in tert-butanol (200 mL) and water (200 mL) with stirring under the protection of nitrogen at 25 °C. The reaction was carried out for 36 hours at 85 °C, and the reaction process was monitored by liquid chromatography-mass spectrometry and thin layer chromatography. After the reaction was completed, the reaction mixture was cooled to room temperature. The mixture was filtered to remove the insolubles, and the filter cake was washed with methanol (150 mL × 3) and dichloromethane (150 mL × 3) sequentially, and the combined filtrates were concentrated under reduced pressure to obtain a crude product. The crude product was purified by silica gel column chromatography, eluted with a gradient of 5% → 70% ethyl acetate/petroleum ether mobile phase, and the obtained fractions were evaporated under reduced pressure to remove the solvent to obtain compound **278-1** (yellow solid, 20.74 g, yield: 54%). MS (ESI, m/z):169.2[M + H]⁺; ¹H NMR (300 MHz, DMSO-d₆) *δ* 8.50 - 8.41 (m, 1H), 8.08 - 7.99 (m, 1H), 7.54 - 7.41 (m, 2H), 7.36 - 7.22 (m, 1H), 6.86 - 6.74 (m, 1H), 6.16 (s, 2H).

### Step 2

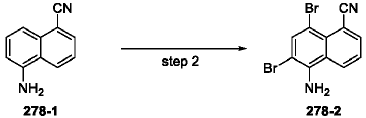

Bromine (41.26 g, 245.285 mmol, 2.2 eq) was slowly added to a solution of compound **278-1** (19.74 g, 111.493 mmol, 1 eq) in acetic acid (250 mL) with stirring at 0 °C. The reaction was carried out for 1.5 hours at 70 °C, and the reaction process was monitored by liquid chromatography-mass spectrometry and thin layer chromatography. After the reaction was completed, the reaction mixture was cooled to room temperature. The mixture was filtered to remove the solvent, and the filter cake was washed with acetic acid (60 mL × 3). The pH value of the filter cake was adjusted to 7 with 10% aqueous sodium hydroxide solution, and the precipitate was collected by filtration. The obtained filter cake was washed with water (150 mLx 3). The filter cake was dried to obtain compound **278-2** (yellow solid, 38 g, yield: 98%). MS (ESI, m/z): 324.9/326.9[M + H]⁺; ¹H NMR (300 MHz, DMSO-*d*₆) δ 8.71 - 8.60 (m, 1H), 8.27 - 8.18 (m, 1H), 7.99 (s, 1H), 7.71 - 7.59 (m, 1H), 6.49 (s, 2H).

### Step 3

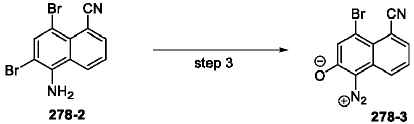

Sodium nitrite (3.05 g, 41.964 mmol, 1.2 eq) was slowly added to a mixed solution of compound **278-2** (12 g, 34.970 mmol, 1 eq) in acetic acid (180 mL)/propionic acid (30 mL) with stirring at 5 °C. The reaction was carried out for 0.5 hours at 5 °C, and the reaction process was monitored by liquid chromatography-mass spectrometry and thin layer chromatography. After the reaction was completed, the reaction solution was poured into ice water. The precipitate was then collected by filtration and the filter cake was washed with water (200 mL × 3) to obtain compound **278-3** (gray solid, 11 g, crude product). The crude product was used directly in the next reaction without further purification.

### Step 4

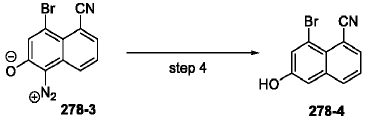

Sodium borohydride (3.04 g, 76.256 mmol, 2 eq) was slowly added to a solution of compound **278-3** (11 g, crude product) in ethanol (100 mL) with stirring at 0 °C. The reaction was carried out for 4 hours at 25 °C, and the reaction process was monitored by liquid chromatography-mass spectrometry and thin layer chromatography. After the reaction was completed, the filter was filtered, and the filter cake was washed with ethanol (50 mL×3), and the filtrate was concentrated under reduced pressure to obtain a crude product. The crude product was purified by silica gel column chromatography, and the mobile phase was eluted with a gradient of 0% → 30% ethyl acetate/petroleum ether mobile phase, and the obtained fractions were evaporated under reduced pressure to remove the solvent to obtain compound **278-4** (yellow solid, 5.24 g, yield: 52%). MS (ESI, m/z): 246.1/248.1[M-H]⁻; ¹H NMR (300 MHz, DMSO-*d*₆) *δ* 10.56 (s, 1H), 8.25 - 8.11 (m, 1H), 8.07 - 7.96 (m, 1H), 7.69 - 7.53 (m, 2H), 7.42 - 7.33 (m, 1H).

### Step 5

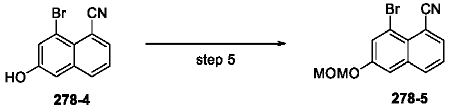

Chloromethyl methyl ether (1.53 g, 21.904 mmol, 1.3 eq) was slowly added to a solution of compound **278-4** (4.4 g, 16.849 mmol, 1 eq) and *N,N*-diisopropylethylamine (6.88 g, 50.547 mmol, 3 eq) in anhydrous dichloromethane (40 mL) with stirring under the protection of nitrogen at 0 °C. The reaction was carried out for 2 hours at 25 °C, and the reaction process was monitored by liquid chromatography-mass spectrometry and thin layer chromatography. After the reaction was completed, the reaction solution was quenched by pouring into ice water, and the aqueous phase was extracted with ethyl acetate (150 mL × 3) and the organic phase was combined; the organic phase was dried over anhydrous sodium sulfate, filtered to remove the drying agent and the filtrate was concentrated under reduced pressure to remove the solvent to obtain compound **278-5** (yellow solid, 5 g, yield: 96%). MS (ESI, m/z): 291.9/293.9[M + H]⁺; ¹H NMR (400 MHz, DMSO-*d*₆) *δ* 8.30 - 8.23 (m, 1H), 8.15 - 8.09 (m, 1H), 7.86 - 7.80 (m, 1H), 7.76 - 7.63 (m, 2H), 5.38 (d, *J* = 1.1 Hz, 2H), 3.44 (s, 3H).

### Step 6

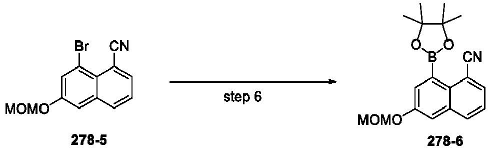

Compound **278-5** (3.8 g, 12.357 mmol, 1 eq), bis(pinacolato)diboron (4.95 g, 18.535 mmol, 1.5 eq), [1,1'-bis(diphenylphosphino)ferrocene]palladium dichloride (1.06 g, 1.236 mmol, 0.1 eq), potassium acetate (3.83 g, 37.071 mmol, 3 eq) and 1,4-dioxane (35 mL) were successively added to a reaction flask with stirring under the protection of nitrogen at 25 °C. The reaction was carried out for 1 hour at 100 °C, and the reaction process was monitored by liquid chromatography-mass spectrometry and thin layer chromatography. After the reaction was completed, the reaction mixture was cooled to room temperature. The mixture was filtered to remove the insolubles, and the filter cake was washed with 1,4-dioxane (20 mL × 3), and the filtrate was concentrated under reduced pressure to obtain a crude product. The crude product was purified by silica gel column chromatography, eluted with a gradient of 0% → 15% ethyl acetate/petroleum ether mobile phase, and the obtained fractions were evaporated under reduced pressure to remove the solvent to obtain compound **278-6** (light reddish brown solid, 2.6 g, yield: 58%). MS (ESI, m/z): 339.9[M + H]⁺; ¹H NMR (400 MHz, CDCl₃) δ 7.98 - 7.92 (m, 1H), 7.85 - 7.81 (m, 1H), 7.59 (d, *J* = 2.6 Hz, 1H), 7.49 - 7.42 (m, 2H), 5.31 (s, 2H), 3.51 (s, 3H), 1.49 (s, 12H).

### Step 7

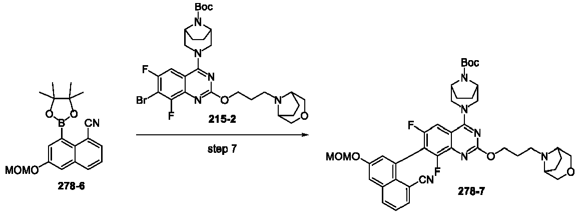

Compound **215-2** (350 mg, 0.532 mmol, 1 eq), compound **278-6** (228.11 mg, 0.638 mmol, 1.2 eq), 3-(tert-butyl)-4-(2,6-dimethoxyphenyl)-2,3-dihydrobenzo[d][1,3]oxaphosphole (37.03 mg, 0.106 mmol, 0.2 eq), tris(dibenzylideneacetone)dipalladium (51.32 mg, 0.053 mmol, 0.1 eq) and potassium phosphate (237.92 mg, 1.064 mmol, 2 eq), toluene (3 mL) and water (0.6 mL) were successively added to a reaction flask with stirring under the protection of nitrogen at 25 °C. The reaction was carried out for 4 hours at 80 °C, and the reaction process was monitored by liquid chromatography-mass spectrometry and thin layer chromatography. After the reaction was completed, the reaction mixture was cooled to room temperature. The reaction solution was concentrated under reduced pressure to obtain a crude product. The crude product was purified by high performance liquid chromatography: chromatographic column XBridge Prep C18 OBD Column, 30 × 50 mm, 5 µm; mobile phase A: water (10 mmol/L sodium bicarbonate), mobile phase B: acetonitrile; flow rate: 60 mL/min; eluted with 40% → 75% phase B in 9 min; detector UV 220 nm; compound **278-7** (white solid, 180 mg, yield: 42%) was obtained. MS (ESI, m/z): 757.1[M + H]⁺.

### Step 8

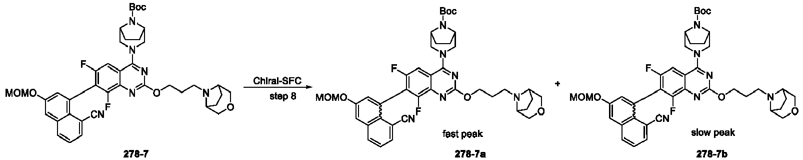

The compound **278-7** (180 mg) obtained in step 7 was subjected to chiral resolution by preparative supercritical liquid chromatography: chiral column: CHIRAL ART Amylose-SA, 3 × 25 cm, 5 µm; mobile phase A: supercritical carbon dioxide, mobile phase B: ethanol (0.5% 2mol/L ammonium methanol); flow rate: 60 mL/min; eluted with 35% phase B in 10 min; detector UV 234 nm, two products were obtained. The product with shorter retention time (6.32 min) was compound **278-7a** (white solid, 75.9 mg, recovery rate: 42%), MS (ESI, m/z): 757.1 [M + H]⁺; the product with longer retention time (7.78 min) was compound **278-7b** (white solid, 79.9 mg, recovery rate: 44%), MS (ESI, m/z): 757.1[M + H]⁺.

### Step 9

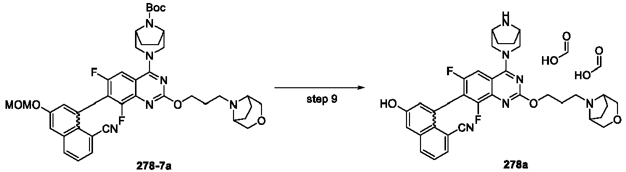

A solution of hydrochloric acid (4 mol/L, 1.5 mL) in 1,4-dioxane was slowly added dropwise to a solution of compound **278-7a** (75.9 mg, 0.095 mmol, 1 eq) in methanol (2 mL) with stirring at 0 °C. The reaction was carried out for 1 hour at room temperature, and the reaction process was monitored by liquid chromatography-mass spectrometry and thin layer chromatography. After the reaction was completed, the reaction mixture was concentrated to obtain a crude product. The crude product was dissolved in 2 mL of methanol and neutralized by adding methanol solution of ammonia (7 mol/L, 2 mL). The obtained mixture was stirred at room temperature for 5 min, and then concentrated under reduced pressure to remove solvent to obtain free base. The obtained free base was dissolved in 2 mL of methanol and then 0.2 mL of formic acid was added, and the mixture was stirred at room temperature for 5 min and then concentrated under reduced pressure to obtain a crude product. The obtained crude product was purified by reversed-phase rapid chromatographic column (C18 column), and eluted with 5% → 95% (acetonitrile/methanol= 1/ 1) /water mobile phase (0.1% formic acid) in 20 min; detector, UV254 nm; to obtain compound **278a** (light yellow solid, 49 mg, yield: 72%). MS (ESI, m/z): 613.1[M + H]⁺; ¹H NMR (400 MHz, DMSO-d₆) δ 8.30 - 8.18 (m, 3H), 7.90 - 7.86 (m, 1H), 7.72 - 7.64 (m, 1H), 7.63 - 7.57 (m, 1H), 7.49 (d, *J* = 2.6 Hz, 1H), 7.32 - 7.28 (m, 1H), 4.46 - 4.38 (m, 2H), 4.38 - 4.23 (m, 2H), 3.81 - 3.48 (m, 7H), 3.43 - 3.34 (m, 2H), 3.12 - 3.03 (m, 2H), 2.42 - 2.35 (m, 2H), 1.91 - 1.81 (m, 4H), 1.79 - 1.64 (m, 6H); ¹⁹F NMR (377 MHz, DMSO-*d*₆) *δ*-119.46, -124.36. The chiral analysis conditions of compound **278a** were: N-CHIRALPAK IG-3, 3.0 × 100 mm, 3 µm; mobile phase A: supercritical carbon dioxide; mobile phase B: isopropanol (20 mmol/L ammonia); flow rate: 2 mL/min; isocratic elution with 10% phase B in 6.5 min, detector UV 230 nm; retention time: 4.553 min. *ee* > 95%.

### Step 10

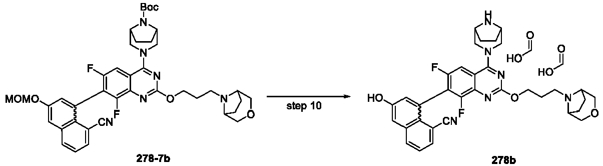

Compound **278b** (light yellow solid) can also be obtained with reference to the method of step 9. MS (ESI, m/z): 613.1 [M + H]⁺; ¹H NMR (400 MHz, DMSO-*d*₆) *δ* 8.29 - 8.16 (m, 3H), 7.91 - 7.84 (m, 1H), 7.72 - 7.63 (m, 1H), 7.64 - 7.56 (m, 1H), 7.49 (d, *J* = 2.5 Hz, 1H), 7.29 (d, *J* = 2.5 Hz, 1H), 4.47 - 4.38 (m, 2H), 4.36 - 4.24 (m, 2H), 3.72 - 3.46 (m, 6H), 3.42 - 3.36 (m, 3H), 3.10 - 3.02 (m, 2H), 2.42 - 2.34 (m, 2H), 1.93 - 1.79 (m, 4H), 1.79 - 1.64 (m, 6H); ¹⁹F NMR (377 MHz, DMSO-*d*₆) *δ* -119.73, -124.47. The chiral analysis conditions of compound **278b** were: N-CHIRALPAK IG-3, 3.0 × 100 mm, 3 µm; mobile phase A: supercritical carbon dioxide; mobile phase B: isopropanol (20 mmol/L ammonia); flow rate: 2 mL/min; isocratic elution with 10% phase B in 6.5 min, detector UV 230 nm; retention time: 4.074 min. *ee* > 95%.

### Embodiment 67

### (S or R)-4-((1R, 5S)-3,8-diazacyclo[3.2.1]octyl-3-yl)-7-(8-ethynyl-7-fluoronaphthalen-1-yl)-6,8-difluoro-2-((2R, 7aS)-2-fluorotetrahydro-1H-pyrrolidin-7a(5H)-yl)methoxy)quinazoline formate 279a; (R or S)-4-((1R,5S)-3,8-diazacyclo[3.2.1]octyl-3-yl)-7-(8-ethynyl-7-fluoronaphthalen-1-yl)-6,8-difluoro-2-((2R,7aS)-2-fluorotetrahydro-1H-pyrrolidin-7a(5H)-yl)methoxy)quinazoline formate 279b

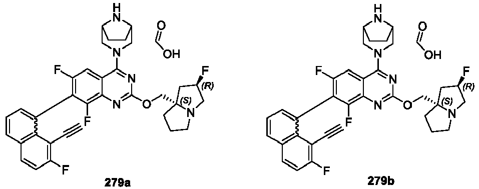

The synthetic route was as follows:

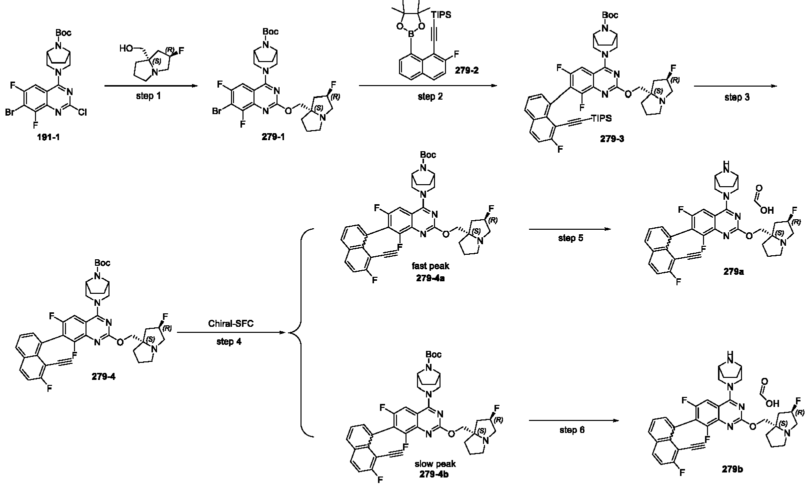

### Step 1:

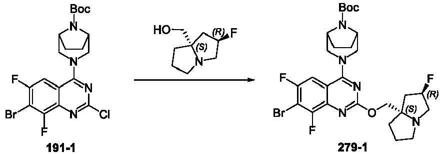

Compound **191-1** (2.0 g, 3.88 mmol, 1.0 eq), triethylenediamine (89.92 mg, 0.77 mmol, 0.2 eq), anhydrous cesium carbonate (2.55 g, 7.76 mmol, 2.0 eq), (2*R*, 7a*S*)-2-fluorotetrahydro-1*H*-pyrrolizine-7a(5*H*)-methanol (748.65 mg, 4.65 mmol, 1.2 eq) and *N,N-*dimethylformamide (20 mL) were successively added to a 25 mL reaction flask with stirring under the protection of nitrogen at 25 °C. The reaction was carried out for 3 hours at 100 °C, and the reaction process was monitored by liquid chromatography-mass spectrometry and thin layer chromatography. After the reaction was completed, the reaction solution was diluted with 200 mL of water, extracted with ethyl acetate (200 mL × 2), and the organic phases were combined; the organic phase was washed with saturated brine (200 mL × 3), dried over anhydrous sodium sulfate, filtered to remove the drying agent, and concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **279-1** (yellow solid, 1 g, yield: 39%). MS (ESI, *m*/*z):* 612.2/614.3 [M + H]⁺.

### Step 2

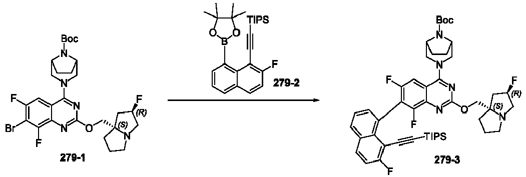

Compound **279-2** was synthesized with reference to patent (WO2021041671).

Compound **279-1** (600 mg, 0.93 mmol, 1.0 eq), compound **279-2** (467.8 mg, 1.02 mmol, 1.1 eq), potassium phosphate (399.0 mg, 1.86 mmol, 2.0 eq), 3-(*tert*-butyl)-4-(2,6-dimethoxyphenyl)-2,3-dihydrobenzo[*d*][1,3]oxaphosphole (31.0 mg, 0.09 mmol, 0.1 eq), tris(dibenzylideneacetone)dipalladium (86.08 mg, 0.09 mmol, 0.1 eq), toluene (5.0 mL) and water (1 mL) were successively added to a reaction flask with stirring under the protection of nitrogen at 25 °C. The reaction was carried out for 3 hours with stirring at 80 °C, and the reaction process was monitored by liquid chromatography-mass spectrometry and thin layer chromatography. After the reaction was completed, the reaction solution was cooled to room temperature, then diluted with 50 mL of water, extracted with ethyl acetate (50 mL × 3), and the organic phases were combined; the organic phase was washed with saturated brine (50 mL × 1), dried over anhydrous sodium sulfate, filtered to remove the drying agent, and concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **279-3** (yellow solid, 550 mg, yield: 65%). MS (ESI, m/z): 858.1[M + H]⁺.

### Step 3

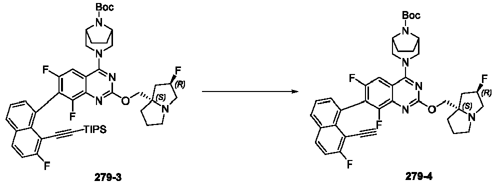

Compound **279-3** (500 mg, 0.55 mmol, 1.0 eq), cesium fluoride (442.5 mg, 2.77 mmol, 5.0 eq) and *N,N-*dimethylformamide (5.0 mL) were successively added to a reaction flask with stirring at 25 °C. The reaction was carried out for 1 hour at 25 °C, and the reaction process was monitored by liquid chromatography-mass spectrometry and thin layer chromatography. After the reaction was completed, the crude product was directly purified by reversed-phase chromatographic column (C18 column), eluted with 5%→95% methanol/water (0.1% sodium bicarbonate) mobile phase in 25 min; detector: UV254/220 nm; compound **279-4** (white solid, 400 mg, yield: 97%) was obtained. MS (ESI, *m*/*z*): 702.3[M + H]⁺; ¹H NMR (400 MHz, CDCl₃) *δ* 8.02 - 7.92 (m, 2H), 7.62 - 7.54 (m, 1H), 7.53 - 7.48 (m, 1H), 7.37 - 7.31 (m, 1H), 7.30 - 7.27 (m, 1H), 5.29 (d, *J* = 53.8 Hz, 1H), 4.48 - 4.14 (m, 6H), 3.67 - 3.45 (m, 2H), 3.40 - 3.16 (m, 3H), 3.05 - 2.95 (m, 1H), 2.84 - 2.80 (m, 1H), 2.35 - 2.14 (m, 3H), 2.05 - 1.79 (m, 7H), 1.52 (s, 9H).

### Step 4

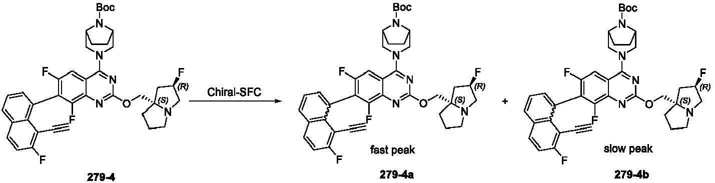

The compound **279-4** (150 mg) obtained in step 3 was subjected to chiral resolution by preparative supercritical liquid chromatography: chiral column NBCHIRALPAK AD-H, 3 × 25 cm, 5 µm; mobile phase A: supercritical carbon dioxide, mobile phase B: isopropanol (0.5% 2 mol/L ammonia methanol); flow rate: 50 mL/min; eluted with 35% phase B in 12 min; detector UV 220/230 nm; two products were obtained. The product with shorter retention time (4.67 min) was compound **279-4a** (light yellow solid, 60 mg, recovery rate: 40%), MS (ESI, m/z): 702.3 [M + H]⁺; the product with longer retention time (6.42 min) was compound **279-4b** (white solid, 60 mg, recovery rate: 40%), MS (ESI, m/z): 702.3 [M + H]⁺.

### Step 5

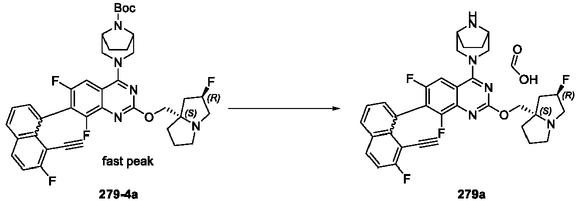

Compound **279-4a** (60 mg, 0.55 mmol, 1 eq), methanol (1.0 mL) and a solution of hydrochloric acid (4 mol/L, 1.0 mL) in 1,4-dioxane were successively added to a reaction flask with stirring at 0 °C. The reaction was carried out for 1 hour at 0 °C, and the reaction process was monitored by liquid chromatography-mass spectrometry and thin layer chromatography. After the reaction was completed, the filtrate was concentrated under reduced pressure to obtain a crude product. The obtained crude product was purified by reversed-phase chromatographic column (C18 column), and eluted with 5% → 95% acetonitrile/water mobile phase (0.1% formic acid) in 25 min; detector, UV254/220 nm; to obtain compound **279a** (white solid, 30 mg, yield: 54%). MS (ESI, *m*/*z*): 602.2[M + H]⁺; ¹H NMR (400 MHz, DMSO-*d*₆) *δ* 8.27 - 8.17 (m, 3H), 7.73 - 7.67 (m, 1H), 7.67 - 7.54 (m, 3H), 5.39 - 5.17 (m, 1H), 4.34 - 4.21 (m, 2H), 4.10 - 4.04 (m, 2H), 4.01 - 3.96 (m, 1H), 3.74 - 3.65 (m, 2H), 3.64 - 3.47 (m, 3H), 3.17 - 3.05 (m, 2H), 3.03 - 2.96 (m, 1H), 2.87 - 2.78 (m, 1H), 2.19 - 1.98 (m, 3H), 1.94 - 1.66 (m, 7H); ¹⁹F NMR (377 MHz, DMSO-*d*₆) *δ*-105.15, -118.63, -124.57, -172.08. The chiral conditions of compound **279a** were: chiral column CHIRALPAK IC-3 (Lot No.IC3SCK-VK002), 3 × 100 mm, 3 µm; mobile phase A: supercritical carbon dioxide, mobile phase B: methanol (20 mmol/L ammonia); flow rate: 2 mL/min; eluted with 50% phase B in 4 min, detector: UV 230 nm, and the retention time was 2.80 min. *dr* > 40:1.

### Step 6

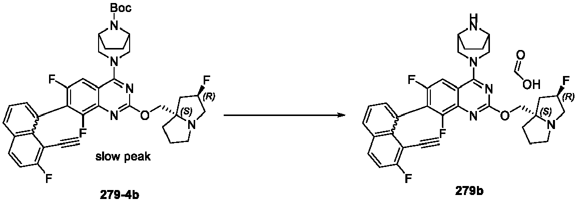

Compound **279b** (white solid) can also be obtained with reference to the method of step 5. MS (ESI, *m*/*z*): 602.2[M + H]⁺; ¹H NMR (400 MHz, DMSO-*d*₆) *δ* 8.32 - 8.19 (m, 3H), 7.77 - 7.68 (m, 1H), 7.67 - 7.55 (m, 3H), 5.39 - 5.18 (m, 1H), 4.38 - 4.23 (m, 2H), 4.13 - 3.96 (m, 3H), 3.81 - 3.71 (m, 2H), 3.68 - 3.60 (m, 2H), 3.16 - 2.97 (m, 3H), 2.89 - 2.77 (m, 1H), 2.19 - 1.99 (m, 3H), 1.91 - 1.69 (m, 7H); ¹⁹F NMR (377 MHz, DMSO-*d*₆) *δ*-105.13, -118.51, -124.50, -172.16. The chiral conditions of compound **279b** were: chiral column CHIRALPAK IC-3 (Lot No.IC3SCK-VK002), 3 × 100 mm, 3 µm; mobile phase A: supercritical carbon dioxide, mobile phase B: methanol (20 mmol/L ammonia); flow rate: 2 mL/min; eluted with 50% phase B in 4 min, detector: UV 230 nm, and the retention time was 2.318 min. *dr >* 40:1.

### Embodiment 68

### 4-((1R,5S)-3,8-diazacyclo[3.2.1]octan-3-yl)-7-((S or R)-8-ethyl-7-fluoronaphthalen-1-yl)-6,8-difluoro-2-((2R,7aS)-2-fluorotetrahydro-1H-pyrrolidin-7a(5H)-yl)methoxy)quinazoline dihydrochloride 280a; 4-((1R,5S)-3,8-diazacyclo[3.2.1]octan-3-yl)-7-((R or S)-8-ethyl-7-fluoronaphthalen-1-yl)-6,8-difluoro-2-((2R,7aS)-2-fluorotetrahydro-1H-pyrrolidin-7a(5H)-yl)methoxy)quinazoline dihydrochloride 280b

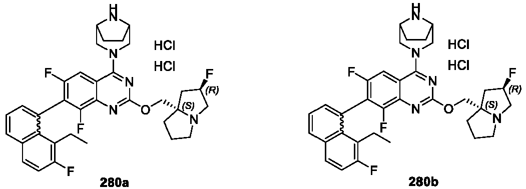

The synthetic route was as follows:

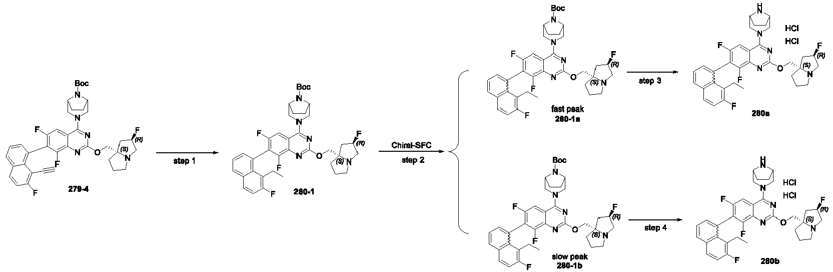

### Step 1

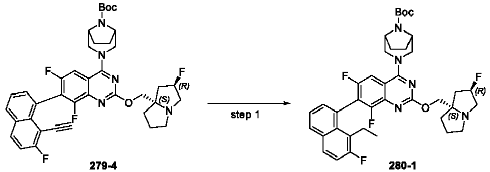

Compound **279-4** (270 mg, 0.36 mmol, 1.0 eq), ethanol (4.0 mL) and platinum dioxide (53.66 mg, 0.23 mmol, 0.6 eq) were successively added to a reaction flask with stirring under the protection of nitrogen at 25 °C. The nitrogen gas was ventilated with hydrogen gas by a displacement gas operation. The reaction was carried out for 20 hours at 25 °C under hydrogen atmosphere (1.5 atmospheric pressures), and the reaction process was monitored by liquid chromatography-mass spectrometry and thin layer chromatography. After the reaction was completed, the reaction solution was filtered through diatomite, and the filter cake was washed with ethanol (10 mL × 3), and the combined filtrates were concentrated under reduced pressure to obtain a crude product. The crude product was purified by preparative supercritical liquid chromatography: chiral column YMC-PACK CN, 3 × 25 cm, 5 µm; mobile phase A: supercritical carbon dioxide, mobile phase B: methanol (0.5% 2 mol/L ammonia methanol); flow rate: 60 mL/min; eluted with 15% phase B in 10 min; detector UV 220 nm; retention time 7.80 min; compound **280-1** (white solid, 170 mg, yield: 63%) was obtained. MS (ESI, m/z): 706.5[M + H]⁺.

### Step 2

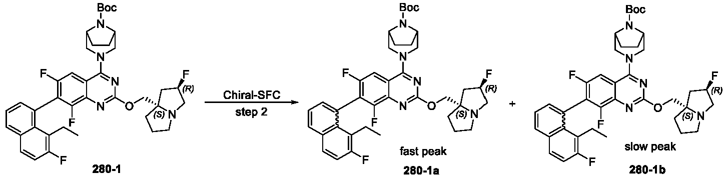

The compound **280-1** (170.0 mg) obtained in step 1 was subjected to chiral resolution by a preparative supercritical liquid chromatography: chiral column NBCHIRALPAK IA, 3 × 25 cm, 5 µm; mobile phase A: supercritical carbon dioxide, mobile phase B: isopropanol (0.5% 2 mol/L ammonia methanol); flow rate: 60 mL/min; eluted with 35% phase B in 10 min; detector UV 218 nm; two products were obtained. The product with shorter retention time (5.72 min) was compound **280-1a** (white solid, 60 mg, recovery rate: 36%), MS (ESI, m/z): 706.5 [M + H]⁺; the product with longer retention time (7.87 min) was compound **280-1b** (white solid, 60 mg, recovery rate: 36%), MS (ESI, m/z): 706.5[M + H]⁺.

### Step 3

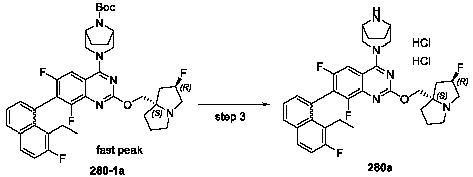

Compound **280-1a** (60 mg, 0.08 mmol, 1 eq), methanol (1.0 mL) and a solution of hydrochloric acid (4 mol/L, 1.0 mL) in 1,4-dioxane were successively added to a reaction flask with stirring at 0 °C. The reaction was carried out for 1 hour at 0 °C, and the reaction process was monitored by liquid chromatography-mass spectrometry and thin layer chromatography. After the reaction was completed, the crude product was purified by reversed-phase chromatographic column (C18 column), eluted with 5%→95% acetonitrile/water (0.1% hydrochloric acid) mobile phase in 25 min; detector: UV254/220 nm; compound **280a** (yellow solid, 50 mg, yield: 88%) was obtained. MS (ESI, *m*/*z):* 606.0[M + H]⁺; ¹H NMR (400 MHz, DMSO-*d*₆) *δ* 11.65 - 11.44 (m, 1H), 10.23 - 9.62 (m, 2H), 8.21 - 8.15 (m, 1H), 8.11 - 8.02 (m, 1H), 7.87 - 7.79 (m, 1H), 7.66 - 7.58 (m, 1H), 7.56 - 7.44 (m, 2H), 5.70 - 5.47 (m, 1H), 4.72 - 4.55 (m, 2H), 4.55 - 4.39 (m, 2H), 4.22 - 4.16 (m, 2H), 4.00 - 3.94 (m, 1H), 3.93 - 3.86 (m, 1H), 3.86 - 3.70 (m, 3H), 3.35 - 3.21 (m, 1H), 2.69 - 2.53 (m, 2H), 2.48 - 2.27 (m, 3H), 2.24 - 1.88 (m, 7H), 0.78 (t, *J* = 7.4 Hz, 3H); ¹⁹F NMR (377 MHz, DMSO-d₆) δ-114.04, -116.19, - 116.20, -122.99, -123.00, -172.62. The chiral analysis conditions of compound **280a** were: chiral column Cellulose-SB, 3 × 100 mm, 3 µm; mobile phase A: supercritical carbon dioxide, mobile phase B: methanol (20 mmol/L ammonia); flow rate: 2 mL/min; eluted with 10% phase Bin 3 min; detector UV 220 nm; retention time 1.911 min. *dr >* 40:1.

### Step 4

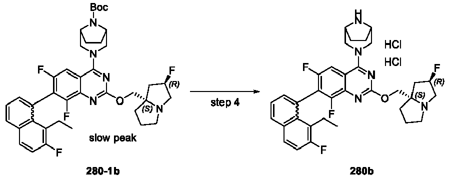

Compound 280b (yellow solid) can also be obtained with reference to the method of step 3. MS (ESI, *m*/*z):* 606.0[M + H]⁺; ¹H NMR (400 MHz, DMSO-*d*₆) *δ* 10.94 - 10.87 (m, 1H), 9.60 - 9.51 (m, 1H), 9.31 - 9.19 (m, 1H), 8.19 - 8.14 (m, 1H), 8.10 - 8.03 (m, 1H), 7.86 - 7.79 (m, 1H), 7.65 - 7.59 (m, 1H), 7.57 - 7.50 (m, 1H), 7.49 - 7.43 (m, 1H), 5.67 - 5.47 (m, 1H), 4.65 - 4.38 (m, 4H), 4.25 - 4.11 (m, 2H), 3.99 - 3.66 (m, 6H), 2.69 - 2.57 (m, 2H), 2.46 - 2.27 (m, 3H), 2.24 - 2.10 (m, 2H), 2.09 - 1.93 (m, 5H), 0.77 (t, *J* = 7.3 Hz, 3H); ¹⁹F NMR (377 MHz, DMSO-*d*₆) *δ*-114.01, -116.15, -123.03, -172.91. The chiral analysis conditions of compound **280b** were: chiral column Cellulose-SB, 3 × 100 mm, 3 µm; mobile phase A: supercritical carbon dioxide, mobile phase B: methanol (20 mmol/L ammonia); flow rate: 2 mL/min; eluted with 10% phase B in 3 min; detector UV 220 nm; retention time 2.171 min. *dr >* 40:1.

### Embodiment 69

### (S or R)-4-((1R,5S)-3,8-diazacyclo[3.2.1]octan-3-yl)-7-(8-ethylnaphthalen-1-yl)-6,8-difluoro-2-((2R,7aS)-2-fluorotetrahydro-1H-pyrrolin-7a(5H)-yl)methoxy)quinazoline dihydrochloride 281a; (R or S)-4-((1R,5S)-3,8-diazacyclo[3.2.1]octan-3-yl)-7-(8-ethylnaphthalen-1-yl)-6,8-difluoro-2-((2R,7aS)-2-fluorotetrahydro-1H-pyrrolin-7a(5H)-yl)methoxy)quinazoline dihydrochloride 281b

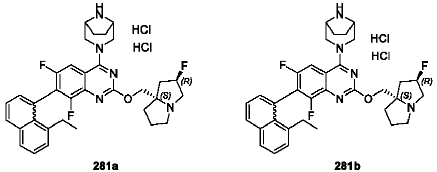

The synthetic route was as follows:

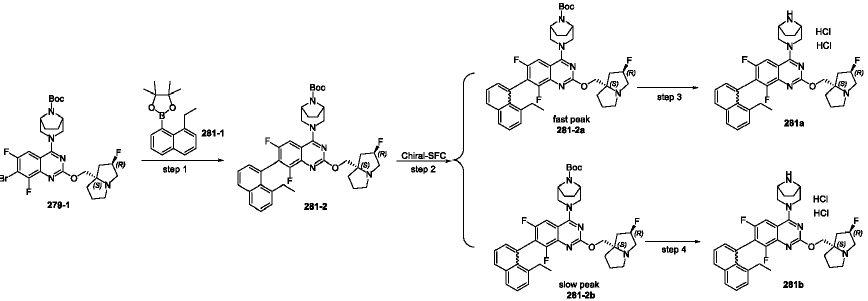

### Step 1

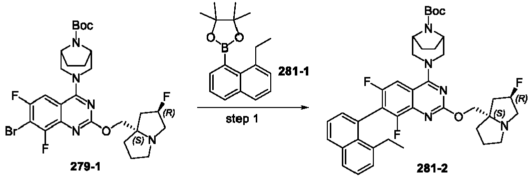

Compound **281-1** was synthesized with reference to patent (WO 2021041671).

Compound **279-1** (300 mg, 0.46 mmol, 1.0 eq), **281-1** (152.0 mg, 0.51 mmol, 1.1 eq), potassium phosphate (207.9 mg, 0.93 mmol, 2.0 eq), 3-(*tert*-butyl)-4-(2,6-dimethoxyphenyl)-2,3-dihydrobenzo[*d*][1,3]oxaphosphole (32.3 mg, 0.09 mmol, 0.2 eq), tris(dibenzylideneacetone)dipalladium (44.85 mg, 0.04 mmol, 0.1 eq), toluene (3.0 mL) and water (0.6 mL) were successively added to a reaction flask with stirring under the protection of nitrogen at 25 °C. The reaction was carried out for 4 hours at 80 °C, and the reaction process was monitored by liquid chromatography-mass spectrometry and thin layer chromatography. After the reaction was completed, the reaction solution was cooled to room temperature, then diluted with 50 mL of water, extracted with dichloromethane (50 mL × 3), and the organic phases were combined; the organic phase was washed with saturated brine (50 mL × 1), dried over anhydrous sodium sulfate, filtered to remove the drying agent, and concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **281-2** (yellow solid, 200 mg, yield: 57%). MS (ESI, m/z): 688.1[M + H]⁺.

### Step 2

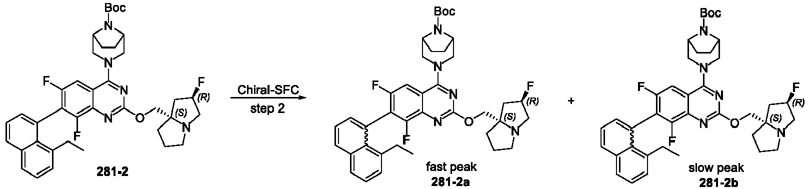

The compound **281-2** (200.0 mg) obtained in step 1 was subjected to chiral resolution by preparative supercritical liquid chromatography: chiral column: Cellulose-SB, 3 × 25 cm, 5 µm; mobile phase A: supercritical carbon dioxide, mobile phase B: methanol (0.5% 2mol/L ammonium methanol); flow rate: 60 mL/min; eluted with 50% phase B in 8 min, detector: UV 222 nm, two products were obtained. The product with shorter retention time (5.23 min) was compound **281-2a** (yellow solid, 70 mg, recovery rate: 35%), MS (ESI, m/z): 688.1[M + H]⁺; the product with longer retention time (6.45 min) was compound **281-2b** (yellow solid, 70 mg, recovery rate: 35%), MS (ESI, m/z): 688.1[M + H]⁺.

### Step 3

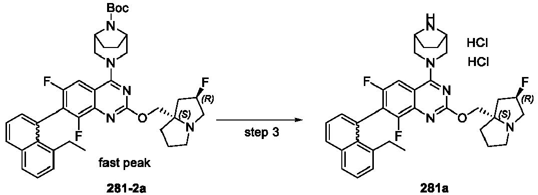

Compound **281-2a** (70 mg, 0.08 mmol, 1 eq), methanol (1.0 mL) and a solution of hydrochloric acid (4 mol/L, 1.0 mL) in 1,4-dioxane were successively added to a reaction flask with stirring at 0 °C. The reaction was carried out for 1 hour at 0 °C, and the reaction process was monitored by liquid chromatography-mass spectrometry and thin layer chromatography. After the reaction was completed, the reaction mixture was concentrated under reduced pressure to obtain a crude product. The obtained crude product was purified by reversed-phase chromatographic column (C18 column), and eluted with 5% → 95% acetonitrile/water mobile phase (0.1% hydrochloric acid) in 25 min; detector, UV254/220 nm; to obtain compound **281a** (white solid, 30 mg, yield: 53%). MS (ESI, *m*/*z*): 588.1[M + H]⁺; ¹H NMR (400 MHz, DMSO-*d*₆) *δ* 11.58 - 11.42 (m, 1H), 10.09 - 9.98 (m, 1H), 9.81 - 9.69 (m, 1H), 8.12 (d, *J* = 8.2 Hz, 1H), 7.95 (d, *J* = 8.1 Hz, 1H), 7.79 (d, *J* = 9.9 Hz, 1H), 7.66 - 7.59 (m, 1H), 7.57 - 7.50 (m, 1H), 7.46 - 7.38 (m, 2H), 5.71 - 5.46 (m, 1H), 4.67 - 4.55 (m, 2H), 4.54 - 4.39 (m, 2H), 4.21 - 4.12 (m, 2H), 3.99 - 3.91 (m, 1H), 3.90 - 3.82 (m, 2H), 3.77 (d, *J* = 13.2 Hz, 3H), 3.34 - 3.23 (m, 1H), 2.65 - 2.55 (m, 1H), 2.49 - 2.41 (m, 2H), 2.37 - 2.29 (m, 1H), 2.26 - 2.11 (m, 2H), 2.09 - 1.93 (m, 5H), 0.88 (t, *J* = 7.4 Hz, 3H); ¹⁹F NMR (377 MHz, DMSO-*d*₆) *δ*-116.32, -116.33, -123.37, -123.38, -172.65. The chiral analysis conditions of compound **281a** were: chiral column CHIRAL ART Cellulose-SB, 3 × 100 mm, 3 µm; mobile phase A: supercritical carbon dioxide, mobile phase B: methanol (20 mmol/L ammonia); flow rate: 2 mL/min; eluted with 10% phase B in 3 min; detector UV 220 nm; retention time 2.148 min. *dr >* 40:1.

### Step 4

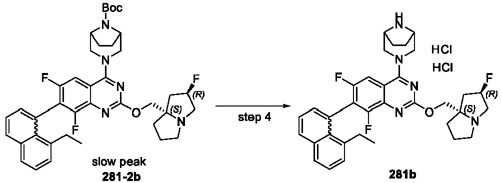

Compound 281b (white solid) can also be obtained with reference to the method of step 3. MS (ESI, *m*/*z*): 588.1[M + H]⁺; ¹H NMR (400 MHz, DMSO-*d*₆) *δ* 11.52 - 11.40 (m, 1H), 10.07 - 9.95 (m, 1H), 9.79 - 9.63 (m, 1H), 8.12 (d, *J* = 8.2 Hz, 1H), 7.95 (d, *J* = 8.1 Hz, 1H), 7.78 (d, *J* = 10.0 Hz, 1H), 7.66 - 7.59 (m, 1H), 7.58 - 7.51 (m, 1H), 7.42 (d, *J* = 7.2 Hz, 2H), 5.70 - 5.45 (m, 1H), 4.68 - 4.57 (m, 2H), 4.53 - 4.47 (m, 1H), 4.45 - 4.36 (m, 1H), 4.21 - 4.11 (m, 2H), 4.00 - 3.93 (m, 1H), 3.91 - 3.70 (m, 5H), 3.36 - 3.22 (m, 1H), 2.71 - 2.57 (m, 1H), 2.48 - 2.41 (m, 2H), 2.37 - 2.28 (m, 1H), 2.25 - 2.12 (m, 2H), 2.10 - 1.93 (m, 5H), 0.87 (t, *J* = 7.4 Hz, 3H); ¹⁹F NMR (377 MHz, DMSO) δ-116.33, -116.34, -123.34, -123.36, -172.69. The chiral analysis conditions of compound **281b** were: chiral column CHIRAL ART Cellulose-SB, 3 × 100 mm, 3 µm; mobile phase A: supercritical carbon dioxide, mobile phase B: methanol (20 mmol/L ammonia); flow rate: 2 mL/min; eluted with 10% phase B in 3 min; detector UV 220 nm; retention time 2.443 min. *dr* > 40:1.

### Embodiment 70

### (S or R)-4-(4-((1R,5S)-3,8-diazacyclo[3.2.1]octan-3-yl)-6,8-difluoro-2-(3-((R)-3-methylmorpholinyl)propoxy)quinazolin-7-yl)-5-ethylnaphthalen-2-ol dihydrochloride 282a; (R or S)-4-(4-((1R,5S)-3,8-diazacyclo[3.2.1]octan-3-yl)-6,8-difluoro-2-(3-((R)-3-methylmorpholinyl)propoxy)quinazolin-7-yl)-5-ethylnaphthalen-2-ol dihydrochloride 282b

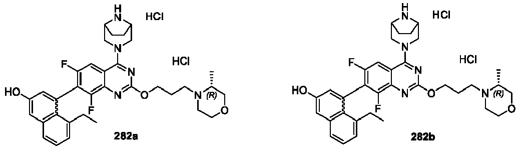

The synthetic route was as follows:

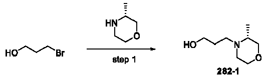

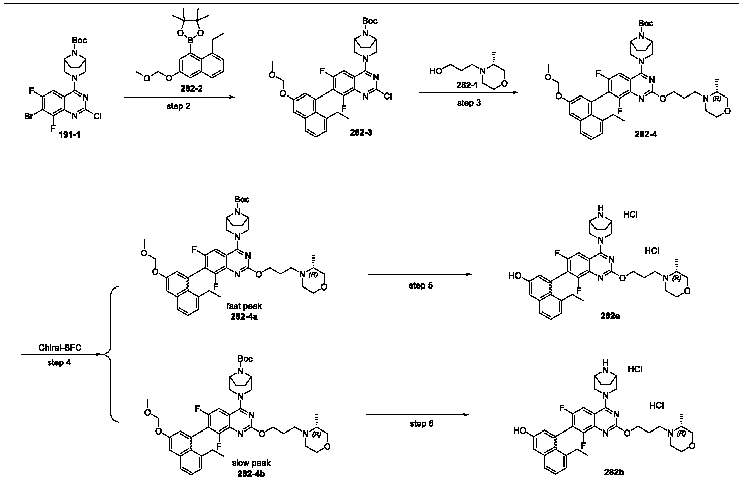

### Step 1

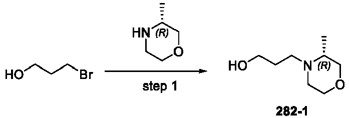

Potassium carbonate (24.86 g, 176 mmol, 2.0 eq) was added to a solution of 3-bromopropanol (12.5 g, 88 mmol, 1.0 eq) and (*R*)-3-methylmorpholine (10 g, 97 mmol, 1.1 eq) in acetonitrile (100 mL) with stirring at 25 °C. The reaction was carried out for 12 hours at 80 °C, and the reaction process was monitored by liquid chromatography-mass spectrometry and thin layer chromatography. After the reaction was completed, the mixture was filtered to remove the insolubles, and the filter cake was washed with dichloromethane (20 mL × 3), and the combined filtrates were concentrated under reduced pressure to obtain a crude product. The crude product was purified by silica gel column chromatography and eluted with a gradient of 0% → 10% methanol (0.8% of 7 mol/L ammoniacal methanol)/dichloromethane mobile phase, the obtained fraction was evaporated under reduced pressure to remove the solvent to obtain compound **282-1** (colorless oil, 12 g, yield: 86%). MS (ESI, *m*/*z):* 160.1[M + H]⁺; ¹H NMR (400 MHz, CDCl₃) *δ* 5.09 (s, 1H), 3.84 - 3.74 (m, 3H), 3.73 - 3.57 (m, 2H), 3.27-3.22 (m, 1H), 3.07 - 2.92 (m, 2H), 2.49 - 2.34 (m, 2H), 2.29-2.23 (m, 1H), 1.95 - 1.81 (m, 1H), 1.58-1.51 (m, 1H), 1.04 (d, *J* = 6.3 Hz, 3H).

### Step 2

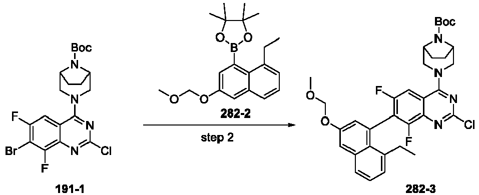

Compound **282-2** was synthesized with reference to patent (WO2021041671).

Compound **191-1** (639 mg, 1.24 mmol, 1.0 eq), compound **282-2** (401.88 g, 1.116 mmol, 0.9 eq), 3-(tert-butyl)-4-(2,6-dimethoxyphenyl)-2, 3-dihydrobenzo[d][1,3]oxaphosphole (63.32 mg, 0.186 mmol, 0.15 eq), tris(dibenzylideneacetone)dipalladium (83.64 mg, 0.087 mmol, 0.07 eq) and potassium phosphate (553.90 mg, 2.480 mmol, 2 eq), toluene (10 mL) and water (2 mL) were successively added to a reaction flask with stirring under the protection of nitrogen at 25 °C. The reaction was carried out for 3 hours at 80 °C, and the reaction process was monitored by liquid chromatography-mass spectrometry and thin layer chromatography. After the reaction was completed, the reaction mixture was cooled to room temperature, concentrated under reduced pressure, and diluted with 40 mL of water. The obtained mixture was extracted with ethyl acetate (50 mL × 2) and dichloromethane (50 mL × 1), then the organic phases were combined. The organic phase was dried over anhydrous sodium sulfate, filtered to remove the drying agent, and the filtrate was concentrated under reduced pressure to obtain a crude product. The crude product was purified by silica gel column chromatography, and the mobile phase was eluted with a gradient of 0% → 30% ethyl acetate/petroleum ether mobile phase, and the obtained fractions were evaporated under reduced pressure to remove the solvent to obtain compound **282-3** (white solid, 414 mg, yield: 52%). MS (ESI, *m*/*z*): 625.1/627.1[M + H]⁺; ¹H NMR (300 MHz, CDCl₃) δ 7.77 - 7.69 (m, 1H), 7.58 (d, *J* = 2.7 Hz, 1H), 7.47 - 7.38 (m, 2H), 7.27 - 7.22 (m, 1H), 7.10 (d, *J* = 2.7 Hz, 1H), 5.33 (s, 2H), 4.58 - 4.48 (m, 1H), 4.48 - 4.34 (m, 3H), 3.81 - 3.57 (m, 2H), 3.55 (s, 3H), 2.52 - 2.40 (m, 2H), 2.10 - 1.95 (m, 2H), 1.94 - 1.75 (m, 2H), 1.55 (s, 9H), 0.98 (t, *J* = 7.4 Hz, 3H).

### Step 3

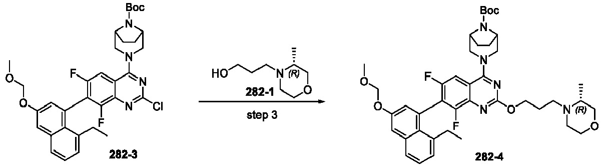

Anhydrous cesium carbonate (312.73 mg, 0.94 mmol, 2.0 eq) was added to a solution of compound **282-3** (300 mg, 0.470 mmol, 1.0 eq), compound **282-1** (102.48 mg, 0.611 mmol, 1.3 eq) and triethylenediamine (11.11 mg, 0.094 mmol, 0.2 eq) in *N,N*-dimethylformamide (5 mL) with stirring under the protection of nitrogen at 25 °C. The reaction was carried out for 2 hours at 100 °C, and the reaction process was monitored by liquid chromatography-mass spectrometry and thin layer chromatography. After the reaction was completed, the reaction mixture was cooled to room temperature, and concentrated under reduced pressure to obtain a crude product. The obtained crude product was purified by silica gel column chromatography, eluted with a gradient of 0%→10% methanol/dichloromethane mobile phase, and the obtained fractions were evaporated under reduced pressure to remove the solvent to obtain compound **282-4** (light yellow solid, 218 mg, yield: 59%). MS (ESI, *m*/*z*):748.1[M + H]⁺.

### Step 4

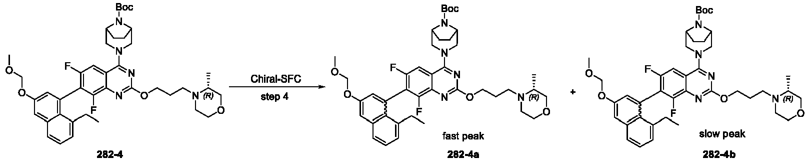

The compound **282-4** (218 mg) obtained in step 4 was subjected to chiral resolution by chiral supercritical liquid chromatography: chiral column: CHIRALPAK IA ID, 3 × 25 cm, 5 µm; mobile phase A: supercritical carbon dioxide, mobile phase B: isopropanol (0.5% 2 mol/L ammonia methanol); flow rate: 60 mL/min; eluted with 40% phase B in 10 min; detector UV 224 nm; two products were obtained. The product with shorter retention time (5.72 min) was compound **282-4a** (yellow solid, 100 mg, recovery rate: 46%), MS (ESI, m/z): 748.1[M + H]⁺; the product with longer retention time (7.52 min) was compound **282-4b** (yellow solid, 106 mg, recovery rate: 49%), MS (ESI, m/z): 748.1[M + H]⁺.

For the chiral resolution methods of some similar chiral compounds in the present disclosure, their retention time and ee values are shown in table 14 below.

**Table 14**

| Nu mbe r of the com pou nd | Compound structure | Compound name | Mass spectr um [M+H ]⁺ | Chiral resolution conditions/retention time/ee value |
|---|---|---|---|---|
| **283-2a** | 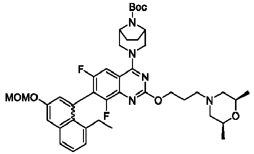 | *tert*-Butyl (1*R*,5*S*)-3-(2-((2*S*,6*R*)-2,6-dimethylmorpholin yl)propoxy)-7-((*S* or *R*)-8-ethyl-3-(methoxymethoxy) naphthalen-1-yl)-6,8-difluoroquinazolin-4-yl)-3,8-diazabicyclo[3.2.1] octane-8-carboxylate | 762.4 | Chiral column: CHIR ALPAK IA, 3 x 25 cm, 5 µm; mobile phase A: supercritical carbon dioxide, mobile phase B: isopropanol (0.5% 2 mol/L ammonia methanol); flow rate: 60 mL/min; gradient: elution with 50% phase B in 8 min; detector UV 226 nm; retention time: 3.72 min. *ee* > 95%. |
| **283-2b** | 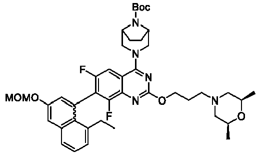 | *tert*-Butyl (1*R*,5*S*)-3-(2-((2*S*,6*R*)-2,6-dimethylmorpholin yl)propoxy)-7-((R or S)-8-ethyl-3-(methoxymethoxy) naphthalen-1-yl)-6,8-difluoroquinazolin-4-yl)-3,8-diazabicyclo[3.2.1] octane-8-carboxylate | 762.4 | Chiral column: CHIR ALPAK IA, 3 x 25 cm, 5 µm; mobile phase A: supercritical carbon dioxide, mobile phase B: isopropanol (0.5% 2 mol/L ammonia methanol); flow rate: 60 mL/min; gradient: elution with 50% phase B in 8 min; detector UV 226 nm; retention time: 5.27 min. ee > 95%. |
| **284-2a** | 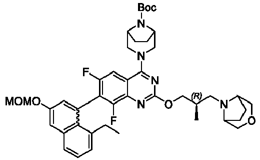 | *tert*-Butyl (1*R*,5*S*)-3-(2-((2*R*)-3-(3-oxa-8-azabicyclo[3.2.1]oc tan-8-yl)-2-methylpropoxy)-7-((*S* or *R*)-8-ethyl-3-(methoxymethoxy) naphthalen-1-yl)-6,8-difluoroquinazolin-4-yl)-3,8-diazabicyclo[3.2.1] octane-8-carboxylate | 774.4 | Chiral column: CHIR ALPAK IA, 3 x 25 cm, 5 µm; mobile phase A: supercritical carbon dioxide, mobile phase B: isopropanol (0.5% 2 mol/L ammonia methanol); flow rate: 60 mL/min; gradient: elution with 50% phase B in 6 min; detector UV 226 nm; retention time: 4.17 min. *dr* > 40:1. |
| **284-2b** | 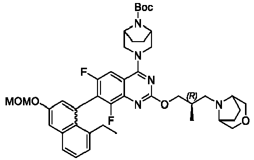 | *tert*-Butyl (1*R*,5*S*)-3-(2-((2*R*)-3-(3-oxa-8-azabicyclo[3.2.1]oc tan-8-yl)-2-methylpropoxy)-7-((*R* or *S*)-8-ethyl-3-(methoxymethoxy) naphthalen-1-yl)-6,8-difluoroquinazolin-4-yl)-3,8-diazabicyclo[3.2.1] octane-8-carboxylate | 774.4 | Chiral column: CHIR ALPAK IA, 3 x 25 cm, 5 µm; mobile phase A: supercritical carbon dioxide, mobile phase B: isopropanol (0.5% 2 mol/L ammonia methanol); flow rate: 60 mL/min; gradient: elution with 50% phase B in 6 min; detector UV 226 nm; retention time: 5.22 min. *dr* > 40:1. |
| **285-2a** | 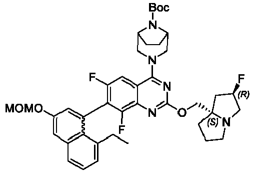 | *tert*-Butyl (1*R*,5*S*)-3-(7-((*S* or *R*)-8-ethyl-3-(methoxymethoxy) naphthalen-1-yl)-6,8-difluoro-2-((2*R*,7*aS*)-2-fluorotetrahydro-1*H*-pyrrolidin-7*a*(5*H*)-yl)methoxy)quinaz olin-4-yl)-3,8-diazabicyclo[3.2.1] octane-8-carboxylate | 748.4 | Chiral column: CHIR ALPAK IA, 3 x 25 cm, 5 µm; mobile phase A: supercritical carbon dioxide, mobile phase B:isopropanol/dichloro methane=2/1 (0.1% 2 mol/L ammonia methanol); flow rate: 50 mL/min; gradient: elution with 50% phase B in 6 min; detector UV 224 nm; retention time: 3.17 min. *dr* > 40:1*.* |
| **285-2b** | 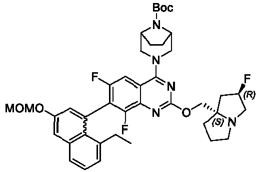 | *tert*-Butyl (1*R*,5*S*)-3-(7-((*R* or *S*)-8-ethyl-3-(methoxymethoxy) naphthalen-1-yl)-6,8-difluoro-2-((2*R*,7*aS*)-2-fluorotetrahydro-1*H*-pyrrolidin-7*a*(5*H*)-yl)methoxy)quinaz olin-4-yl)-3,8-diazabicyclo[3.2.1] octane-8-carboxylate | 748.4 | Chiral column: CHIR ALPAK IA, 3 x 25 cm, 5 µm; mobile phase A: supercritical carbon dioxide, mobile phase B: isopropanol/dichlorome thane=2/1 (0.1% 2 mol/L ammonia methanol); flow rate: 50 mL/min; gradient: elution with 50% phase B in 6 min; detector UV 224 nm; retention time: 4.17 min. *Jr* > 40:1. |
| **286-2a** | 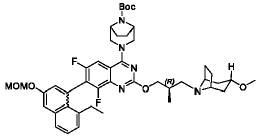 | *tert*-Butyl (1*R*,5*S*)-3-(7-((*S* or *R*)-8-ethyl-3-(methoxymethoxy) naphthalen-1-yl)-6,8-difluoro-2-((*R*)-3-((1*R*,3*S*,5*S*)-3-methoxy-8-azabicyclo[3.2.1]oc tan-8-yl)-2-methylpropoxy)qui nazolin-4-yl)-3,8-diazabicyclo[3.2.1] octane-8-carboxylate | 802.4 | Chiral column: CHIR ALPAK IC; 2 x 25 cm, 5 µm; mobile phase A: *n*-hexane (10 mmol/L ammonia methanol solution), mobile phase B: ethanol; flow rate: 20 mL/min; gradient: elution with 50% phase B in 21 min, detector UV 224/199 nm, retention time: 5.19 min. *dr* > 40:1. |
| **286-2b** | 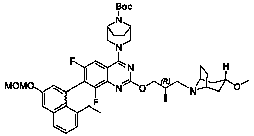 | *tert*-Butyl (1*R*,5*S*)-3-(7-((*R* or *S*)-8-ethyl-3-(methoxymethoxy) naphthalen-1-yl)-6, 8-difluoro-2-((*R*)-3-((1*R*,3*S*,5*S*)-3-methoxy-8-azabicyclo[3.2.1]oc tane-8-yl)-2-methylpropoxy)qui nazolin-4-yl)-3,8-diazabicyclo[3.2.1] octane-8-carboxylate | 802.4 | Chiral column: CHIR ALPAK IC; 2 x 25 cm, 5 µm; mobile phase A: *n*-hexane (10 mmol/L ammonia methanol), mobile phase B: ethanol; flow rate: 20 mL/min; gradient: elution with 50% phase B in 21 min, detector UV 224/199 nm, retention time: 13.56 min. *dr* > 40:1. |
| **287-2a** | 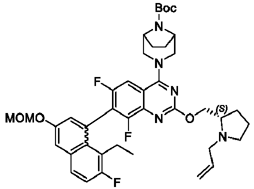 | *tert-*Butyl (1*R*,5*S*)-3-((*S* or *R*)*-2-*((*S*)-1-allylpyrrolidin-2-yl)methoxy)-7-(8-ethyl-7-fluoro-3-(methoxymethoxy) naphthalen-1-yl)-6,8-difluoroquinazolin-4-yl)-3,8-diazabicyclo[3.2.1] octane-8-carboxylate | 748.4 | Chiral column: CHIRAL ART Cellulose-SC, 3 x 25 cm, 5 µm; mobile phase A: supercritical carbon dioxide, mobile phase B: ethanol (0.5% 2 mol/L ammonia methanol); flow rate: 60 mL/min; gradient: elution with 35% phase B in 13 min; detector UV 202 nm; retention time: 8.27 min. *dr* > 40:1. |

### Step 5

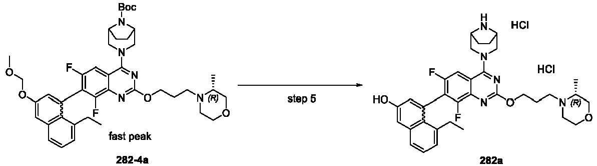

A solution of hydrochloric acid (4 mol/L, 2 mL) in 1,4-dioxane was added dropwise to a solution of compound **282-4a** (100 mg, 0.127 mmol, 1 eq) in methanol (2 mL) with stirring at 0 °C. The reaction was carried out for 1 hour at room temperature, and the reaction process was monitored by liquid chromatography-mass spectrometry and thin layer chromatography. After the reaction was completed, the reaction mixture was concentrated under reduced pressure. The obtained crude product was purified by reversed-phase chromatographic column (C18 column), and eluted with 5% → 95% (acetonitrile/methanol = 1/1)/water mobile phase (0.1% hydrochloric acid) in 20 min; detector, UV254/220 nm; to obtain compound **282a** (yellow solid, 52.2 mg, yield: 57%). MS (ESI, *m*/z): 604.2[M + H]⁺; ¹H NMR (300 MHz, DMSO-*d*₆) *δ* 11.32 (s, 1H), 10.21 - 9.51 (m, 3H), 7.80 - 7.67 (m, 2H), 7.44 - 7.31 (m, 2H), 7.20 - 7.12 (m, 1H), 6.99 (d, *J=* 2.6 Hz, 1H), 4.55 - 4.34 (m, 4H), 4.22 - 4.12 (m, 2H), 4.03 - 3.93 (m, 3H), 3.79 - 3.69 (m, 2H), 3.65 - 3.44 (m, 3H), 3.44 - 3.32 (m, 1H), 3.30 - 3.04 (m, 2H), 2.46 - 2.31 (m, 2H), 2.31 - 2.15 (m, 2H), 2.09 - 1.88 (m, 4H), 1.35 - 1.17 (m, 3H), 0.91 - 0.80 (m, 3H); ¹⁹F NMR (282 MHz, DMSO-*d*₆) *δ*-116.90, -116.91, -123.69, -123.70. The chiral analysis conditions of compound **282a** were: N-Lux 3 µm Cellulose-4 (H17-388767), 4.6 x 100 mm, 3 µm; mobile phase A: supercritical carbon dioxide, mobile phase B: ethanol (20mmol/L ammonia); flow rate: 3.5 mL/min; isocratic elution with 35% phase B in 6.5 min; detector UV 220 nm; retention time: 3.455 min. *dr >* 40:1.

### Step 6

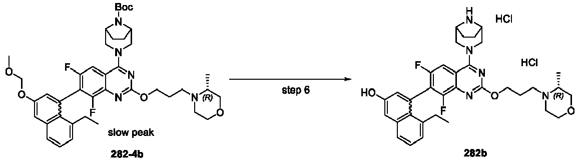

Compound **282b** (yellow solid) can also be obtained with reference to the method of step 5. MS (ESI, *m*/z): 604.2[M + H]⁺; ¹H NMR (300 MHz, DMSO-*d*₆) *δ* 11.69 - 11.04 (m, 1H), 10.53 - 9.45 (m, 3H), 7.81 - 7.64 (m, 2H), 7.45 - 7.28 (m, 2H), 7.20 - 7.09 (m, 1H), 7.04 - 6.96 (m, 1H), 4.52 - 4.38 (m, 4H), 4.21 - 4.10 (m, 2H), 4.04 - 3.70 (m, 6H), 3.65 - 3.56 (m, 1H), 3.55 - 3.31 (m, 2H), 3.28 - 3.05 (m, 2H), 2.44 - 2.33 (m, 2H), 2.31 - 2.17 (m, 2H), 2.10 - 1.89 (m, 4H), 1.35 - 1.22 (m, 3H), 0.93 - 0.79 (m, 3H); ¹⁹F NMR (282 MHz, DMSO-*d*₆) *δ-*116.90, -123.70. The chiral analysis conditions of compound **282b** were: N--Lux 3 µm Cellulose-4 (H17-388767), 4.6 x 100 mm, 3 µm; mobile phase A: supercritical carbon dioxide, mobile phase B: ethanol (20mmol/L ammonia); flow rate: 3.5 mL/min; isocratic elution with 35% phase B in 6.5 min; detector UV 220 nm; retention time: 4.723 min. *dr* > 40:1.

Other similar compounds of the present disclosure can be prepared by the synthetic method shown in Embodiment **70** above. Some compounds prepared with reference to the above synthetic method and their characterization data are shown in the table below.

Other similar compounds of the present disclosure can be prepared by the synthetic method shown in Embodiment **70** above. Some compounds prepared with reference to the above synthetic method and their characterization data are shown in table 15 below.

**Table 15**

| Num ber of the com poun d | Compound structure | Compoun d name | Chiral analysis conditions/retent ion time/ee value/specific rotation | Mas s spec trum [M+ H]⁺ | ¹H & ¹⁹F NMR |
|---|---|---|---|---|---|
| **283a** | 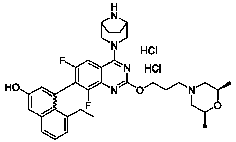 | (*S* or *R*)-4-(4-((1*R*,5*S*)-3,8-diazacyclo [3.2.1]octa n-3-yl)-2-(3-((2*S*,6*R*)-2,6-dimethylm orpholinyl )propoxy)-6,8-difluoroqu inazolin-7-yl)-5-ethylnapht halen-2-ol dihydrochl oride | Chiral column: N-Lux 3 µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient: elution with 50% phase B in 6 min; detector: UV 254 nm; retention time: 1.349 min. *ee* > 95%. | 618. 3 | ¹H NMR (300 MHz, DMSO-*d*₆) *δ* 11.50 (s, 1H), 9.98 (s, 1H), 9.78 (s, 1H), 7.80 - 7.67 (m, 2H), 7.45 - 7.31 (m, 2H), 7.19 - 7.12 (m, 1H), 7.00 (d, *J* = 2.6 Hz, 1H), 4.59 - 4.37 (m, 4H), 4.27 - 4.11 (m, 2H), 4.10 - 3.74 (m, 4H), 3.48 (d, *J* = 11.9 Hz, 2H), 3.35 - 3.16 (m, 2H), 2.76 - 2.56 (m, 2H), 2.43 - 2.19 (m, 4H), 2.10 - 1.93 (m, 4H), 1.21 - 1.07 (m, 6H), 0.86 (t, *J* = 7.4 Hz, 3H); ¹⁹F NMR (282 MHz, DMSO-*d*₆) *δ-*116.84, -123.66. |
| **283 b** | 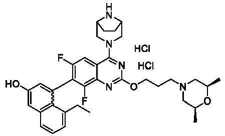 | (*R* or *S*)-4-(4-((1*R*,5*S*)-3,8-diazacyclo [3.2.1]octa n-3-yl)-2-(3-*((2S,6R)-*2,6-dimethylm orpholinyl )propoxy)-6,8-difluoroqu inazolin-7-yl)-5-ethylnapht halen-2-ol dihydrochl oride | Chiral column: N-Lux 3 µm Cellulose-2 (H1 8-089501), 4.6 x 100 mm, 3 µm; mobile phase A: supercritical carbon dioxide, mobile phase B: methanol (0. 1% diethylamine); flow rate: 4 mL/min; gradient: elution with 50% phase B in 6 min; detector: UV 254 nm; retention time: 1.903 min. *ee* > 95%. | 618. 3 | ¹H NMR (300 MHz, DMSO-*d*₆) *δ* 7.80 - 7.66 (m, 2H), 7.44 - 7.36 (m, 1H), 7.33 (d, *J* = 2.6 Hz, 1H), 7.18 - 7.13 (m, 1H), 6.97 (d, *J =* 2.7 Hz, 1H), 4.56 - 4.34 (m, 4H), 4.22 - 4.12 (m, 2H), 4.01 - 3.74 (m, 4H), 3.52 - 3.42 (m, 2H), 3.34 - 3.18 (m, 2H), 2.73 - 2.58 (m, 2H), 2.44 - 2.31 (m, 2H), 2.31 - 2.19 (m, 2H), 2.07 - 1.92 (m, 4H), 1.19 -1.04 (m, 6H), 0.85 (t, *J* = 7.4 Hz, 3H); ¹⁹F NMR (282 MHz, DMSO-*d*₆) *δ-*116.86, -123.65. |
| **284a** | 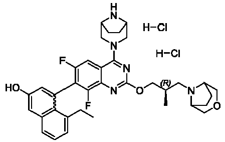 | (4*S* or 4*R*)-4-(2-((2*R*)-3-(3-oxa-8-azabicyclo [3.2.1]octa n-8-yl)-2-methylpro poxy)-4-((1*R*,5*S*)-3,8-diazabicyc lo[3.2.1]oc tan-3-yl)-6,8-difluoroqu inazolin-7-yl)-5-ethylnapht halen-2-ol dihydrochl oride | Chiral column: N-Lux 3 µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient: elution with 50% phase Bin 6 min; detector: UV 254 nm; retention time: 2.715 min. *dr* > 40:1. | 630. 3 | ¹H NMR (400 MHz, DMSO-*d*₆) *δ* 7.79 - 7.66 (m, 2H), 7.44 - 7.36 (m, 1H), 7.34 (d, *J =* 2.6 Hz, 1H), 7.20 - 7.14 (m, 1H), 6.97 (d, *J =* 2.6 Hz, 1H), 4.55 - 4.44 (m, 1H), 4.43 - 4.31 (m, 3H), 4.24 - 4.05 (m, 5H), 4.02 - 3.95 (m, 1H), 3.93 - 3.85 (m, 1H), 3.82 - 3.66 (m, 3H), 3.17 - 2.97 (m, 2H), 2.60 - 2.54 (m, 1H), 2.43 - 2.32 (m, 2H), 2.25 - 2.15 (m, 2H), 2.11 - 1.93 (m, 6H), 1.14 (d, *J* = 6.8 Hz, 3H), 0.85 (t, *J* = 7.4 Hz, 3H); ¹⁹F NMR (377 MHz, DMSO-*d*₆) *δ*-116.85, -116.86, -123.84, -123.85. |
| **284 b** | 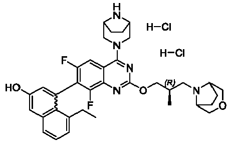 | (4*R* or 4*S*)-4-(2-((2*R*)-3-(3-oxa-8-azabicyclo [3.2.1]octa n-8-yl)-2-methylpro poxy)-4-((1*R*,5*S*)-3,8-diazabicyc lo[3.2.1]oc tan-3-yl)-6,8-difluoroqu inazolin-7-yl)-5-ethylnapht halen-2-ol dihydrochl oride | Chiral column: N-Lux 3 µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient: elution with 50% phase B in 6 min; detector: UV 254 nm; retention time: 3.767 min; dr > 40:1. | 630. 3 | ¹H NMR (400 MHz, DMSO-*d*₆) *δ* 7.79 - 7.68 (m, 2H), 7.44 - 7.36 (m, 1H), 7.35 (d, *J =* 2.7 Hz, 1H), 7.20 - 7.13 (m, 1H), 6.98 (d, *J =* 2.7 Hz, 1H), 4.52 - 4.32 (m, 4H), 4.25 - 4.03 (m, 5H), 4.03 - 3.95 (m, 1H), 3.92 - 3.66 (m, 4H), 3.15 - 2.96 (m, 2H), 2.61 - 2.55 (m, 1H), 2.42 - 2.31 (m, 2H), 2.24 - 2.13 (m, 2H), 2.11 - 1.95 (m, 6H), 1.13 (d, *J =* 6.7 Hz, 3H), 0.85 (t, *J =* 7.4 Hz, 3H); ¹⁹F NMR (377 MHz, DMSO-*d*₆) *δ*-116.84, -116.85, -123.85, -123.87. |
| **285a** | 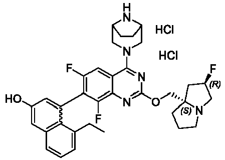 | (*S* or *R*)-4-(4-((1*R*,5*S*)-3,8-diazacyclo [3.2.1]octa n-3-yl)-6,8-difluoro-2-((2*R*,7*aS*)-2-fluorotetra hydro-1*H* pyrrolidin-7*a*(5*H-*yl)methox y)quinazol in-7-yl)-5-ethylnapht halen-2-ol dihydrochl oride | Chiral column: N-Lux 3 µm Cellulose-2 (H18-089501), 4.6 x 100 mm, 3 µm; mobile phase A: supercritical carbon dioxide, mobile phase B: methanol (0. 1% diethylamine); flow rate: 4 mL/min; gradient: elution with 40% phase B in 7 min; detector: UV 220 nm; retention time: 3.698 min. *dr* > 40:1. | 604. 3 | ¹H NMR (300 MHz, DMSO-*d*₆) *δ* 7.80 - 7.65 (m, 2H), 7.44 - 7.29 (m, 2H), 7.20 - 7.13 (m, 1H), 6.97 (d, *J* = 2.6 Hz, 1H), 5.57 (d, *J* = 53.1 Hz, 1H), 4.65 - 4.38 (m, 4H), 4.25 - 4.12 (m, 2H), 3.97 - 3.71 (m, 5H), 3.39 - 3.19 (m, 1H), 2.68 - 2.54 (m, 1H), 2.48 - 2.45 (m, 1H), 2.41 - 2.26 (m, 3H), 2.24-2.10 (m, 2H), 2.09 - 1.91 (m, 5H), 0.83 (t, *J* = 7.3 Hz, 3H); ¹⁹F NMR (282 MHz, DMSO-*d*₆) *δ-*116.41, -123.49, - 172.75. |
| **285 b** | 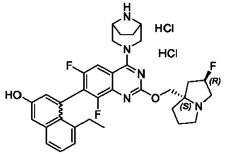 | (*R* or *S*)-4-(4-((1*R*,5*S*)-3,8-diazacyclo [3.2.1]octa n-3-yl)-6,8-difluoro-2-((2*R*,7*aS*)-2-fluorotetra hydro-1*H-*pyrrolidin-7*a*(5*H-*yl)methox y)quinazolin-7-yl)-5-ethylnapht halen-2-ol dihydrochl oride | Chiral column: N-Lux 3 µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient: elution with 40% phase B in 7 min; detector: UV 220 nm; retention time: 5.109 min. *dr* > 40:1. | 604. 3 | ¹H NMR (300 MHz, DMSO-*d*₆) *δ* 11.60 - 11.19 (m, 1H), 10.51 - 9.52 (m, 3H), 7.84 - 7.74 (m, 1H), 7.70 (d, *J =* 8.2 Hz, 1H), 7.45 - 7.30 (m, 2H), 7.20 - 7.10 (m, 1H), 7.04 - 6.94 (m, 1H), 5.58 (d, *J =* 52.4 Hz, 1H), 4.66 - 4.57 (m, 2H), 4.58 - 4.35 (m, 2H), 4.25 - 4.10 (m, 2H), 3.88 - 3.79 (m, 5H), 3.38 - 3.22 (m, 1H), 2.74 - 2.59 (m, 1H), 2.49 - 2.45 (m, 1H), 2.43 - 2.27 (m, 3H), 2.25 - 2.09 (m, 2H), 2.08 - 1.92 (m, 5H), 0.85 (t, *J* = 7.4 Hz, 3H); ¹⁹F NMR (282 MHz, DMSO-*d*₆) *δ*-116.42, -116.43, -123.47, -123.48, - 172.79. |
| **286a** | 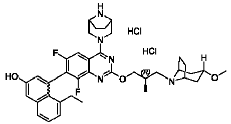 | (*S* or *R*)-4-(4-((1*R*,5*S*)-3,8-diazabicyc lo[3.2.1]oc tan-3-yl)-6,8-difluoro-2-((*R*)-3-((1*R*,3*S*,5*S* )-3-methoxy-8-azabicyclo [3.2.1]octa n-8-yl)-2-methylpro poxy) quinazolin-7-yl)-5-ethylnapht halen-2-ol dihydrochl oride | Chiral column: CHIRALPAK IC-3, 4.6 x 50 mm, 3 µm; mobile phase A: n-hexane (0.1% diethylamine), mobile phase B: ethanol; flow rate: 1 mL/min; gradient: elution with 50% phase B in 4 min; detector UV 254 nm; retention time: 0.617 min. *dr* > 40:1. | 658. 3 | ¹H NMR (400 MHz, DMSO-*d*₆) *δ* 7.78 - 7.72 (m, 1H), 7.72 - 7.67 (m, 1H), 7.42 - 7.37 (m, 1H), 7.34 (d, *J = 2.6 Hz,* 1H), 7.20 - 7.13 (m, 1H), 6.98 (d, *J =* 2.7 Hz, 1H), 4.55 -4.46 (m, 1H), 4.44 - 4.31 (m, 3H), 4.19 (d, *J =* 15.6 Hz, 2H), 4.11 - 4.04 (m, 1H), 3.97 - 3.85 (m, 2H), 3.83 - 3.74 (m, 1H), 3.55 - 3.50 (m, 2H), 3.49 - 3.41 (m, 1H), 3.24 - 3.21 (m, 3H), 3.10 - 3.02 (m, 1H), 3.01 - 2.93 (m, 1H), 2.49 - 2.30 (m, 4H), 2.20 - 1.88 (m, 10H), 1.18 - 1.09 (m, 3H), 0.85 (t, *J* = 7.4 Hz, 3H); ¹⁹F NMR (377 MHz, DMSO-*d*₆) *δ*-116.86, -116.87, -123.83, -123.84. |
| **286 b** | 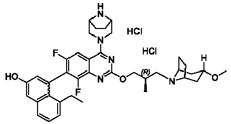 | (*R* or *S*)-4-(4-((1*R*,5*S*)-3,8-diazabicyc lo[3.2.1]oc tan-3-yl)-6,8-difluoro-2-((*R*)-3-((1*R*,3*S*,5*S* )-3-methoxy-8-azabicyclo [3.2.1]octa n-8-yl)-2-methylpro poxy)quin azolin-7-yl)-5-ethylnaphthalen-2-ol dihydrochl oride | Chiral column: CHIRALPAK IC-3, 4.6 x 50 mm, 3 µm; mobile phase A: *n*-hexane (0.1% diethylamine), mobile phase B: ethanol; flow rate: 1 mL/min; gradient: elution with 50% phase B in 4 min; detector UV 254 nm; retention time: 1.334 min. *dr* > 40:1. | 658. 3 | ¹H NMR (400 MHz, DMSO-*d*₆) *δ* 7.78 - 7.68 (m, 2H), 7.43 - 7.37 (m, 1H), 7.34 (d, *J* = 2.7 Hz, 1H), 7.16 (d, *J =* 7.0 Hz, 1H), 7.01 - 6.96 (m, 1H), 4.52 - 4.28 (m, 4H), 4.23 - 4.15 (m, 2H), 4.11 - 4.04 (m, 1H), 3.97 - 3.74 (m, 3H), 3.62 - 3.54 (m, 3H), 3.24 - 3.20 (m, 3H), 3.11 - 2.93 (m, 2H), 2.47 - 2.31 (m, 4H), 2.18 -1.94 (m, 10H), 1.18 - 1.09 (m, 3H), 0.85 (t, *J =* 7.4 Hz, 3H); ¹⁹F NMR (377 MHz, DMSO-*d*₆) *δ-*116.84, -116.85, - 123.81, -123.82. |
| **287a** | 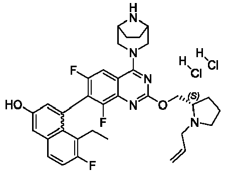 | 4-((*S* or *R*)-2-((*S*)-1-allylpyrrol idin-2-yl)methox y)-4-((1*R*,5*S*)-3,8-diazacyclo [3.2.1]octa n-3-yl)-6,8-difluoroqu inazolin-7-yl)-5-ethyl-6-fluoronaph thalen-2-ol dihydrochl oride | | 604. 3 | ¹H NMR (400 MHz, CD₃OD) *δ* 7.79 - 7.74 (m, 1H), 7.73 - 7.67 (m, 1H), 7.34 - 7.30 (m, 1H), 7.30 - 7.22 (m, 1H), 6.98 (d, *J* = 2.6 Hz, 1H), 6.13 - 6.00 (m, 1H), 5.68 - 5.59 (m, 1H), 5.58 - 5.51 (m, 1H), 4.92 -4.88 (m, 1H), 4.78 - 4.67 (m, 3H), 4.34 - 4.18 (m, 3H), 4.12 - 4.03 (m, 1H), 4.01 - 3.82 (m, 3H), 3.72 - 3.59 (m, 1H), 2.64 - 2.51 (m, 1H), 2.50 - 2.36 (m, 2H), 2.26 - 2.06 (m, 7H), 1.32 - 1.28 (m, 1H), 0.82 (t, *J =* 7.4 Hz, 3H); ¹⁹F NMR (377 MHz, CD₃OD) *δ*-115.46, - 120.91, -124.47, - 124.49. |

### Comparative embodiment 1 preparation of BI-2852 (positive control)

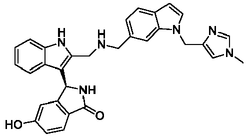

References (doi: 10.1073/pnas.1904529116) was obtained from the Supplementary Information preparation.

### Comparative embodiment 2 Comparative example a and Comparative embodiment b

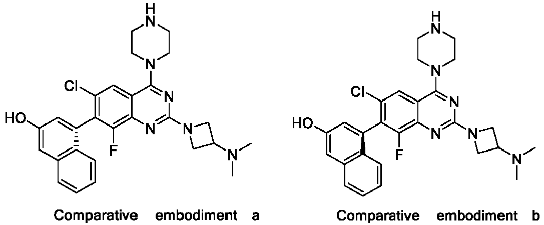

Comparative embodiment a and Comparative embodiment b were prepared with reference to Embodiment 4 in WO 2017172979.

### Effect embodiment A

### 1.Experimental objectives:

The inhibitory ability of small molecule compounds on the binding activity of KRAS-G12D and SOS1 was examined by a drug screening system based on the combination of KRAS_G12D and SOS1.

### 2. Experimental materials and instruments and equipment

| Reagents: | Brand | Item number |
|---|---|---|
| KRAS-G12D/SOS1 binding kits | Cisbio | 63ADK000CB21PEH |
| GTP | Sigma | V900868 |
| Consumables | Brand | Item number |
| Topseal A | PerkinElmer | E5341 |
| 384-Well Polypropylene microplate | labcyte | PP-0200 |
| 96 Well Plates | Nunc | 249944 |
| 384-well plates | Corning | CLS4514 |
| Instruments | Brand | Item number |
| Envision | Perkin Elmer | 2104 |
| Centrifuge | Eppendorf | 5810R |
| Multi-channel pipettes | Eppendorf/Sartorius | / |
| Echo | Labcyte | / |

### 3. Experimental methods:

### 3.1 Experimental steps:

a) BI-2852 was used as a positive control, and its stock solution was the first point of dilution, then the solution was 3-fold diluted, with 10+0 points of dilution. Similarly, the first dilution point of the compound to be tested was its storage solution, and the solution was 3-fold diluted, with 11 +0 points of dilution. Echo was used to transfer 0.2 µL gradient diluted solution of the compounds into a 384-well plate, with 2 duplicated wells for each compound, and the final concentration of DMSO was 1%. The solution was centrifuged for 1 min at 1000 rpm/min. The final concentrations of Reference were 100, 33.33, 11.11, 3.70, 1.23, 0.412, 0.137, 0.046, 0.015, 0.005 and 0 µm. The final concentrations of the compounds to be tested were 200, 66.67, 22.22, 7.41, 2.47, 0.27, 0.091, 0.03, 0.0152, 0.01, 0 µm.
b) The KRAS_G12D in the kit and GTP with a final concentration of 10 µm were co-prepared in diluent, 5 µL of the mixture was transferred to a 384 reaction plate, centrifuged at 1000 rpm/min for 1 min,
c) 5 µL of theSOS1 mixture was transferred to a 384 reaction plate, centrifuged at 1000 rpm/min for 1 min, incubated at 25 °C for 15min.
d) 10 µL of the mixture to be tested was transferred to a 384 reaction plate, centrifuged at 1000 rpm/min for 1 min, incubated at 4 °C overnight.
e) Excitation wavelength 665 nm and emission wavelength 615 nm were read using the Envision multi-function plate reader. 665/615 Ratio signal intensity was used to characterize the activity of enzyme.
(f) Analyzing raw data.

### 3.2 Experimental data processing method:

IC₅₀ of the compounds was fitted by Graphpad Prism 8 nonlinear regression equation:
Negative control: DMSO
Positive control: 100 µM BI-2852

The IC₅₀ (half inhibitory concentration) of the compound was obtained by using the following nonlinear fitting formula:
$Y = Bottom + \left(Top-Bottom\right) / \left(1+{10}^{∧}\left(\left({LogIC}_{50}-X\right)*HillSlope\right)\right)$
X: Compound concentration log value
Y: 665/615 Ratio

### Effect embodiment B

### 1. Experimental objectives:

The inhibitory ability of small molecule compounds on the binding activity of KRAS_G12D and cRAF was examined by a drug screening system based on the combination of KRAS_G12D and cRAF.

### 2. Experimental materials and instruments and equipment

| Reagents: | Brand | Item number |
|---|---|---|
| KRAS-G12D/cRAF binding kits | Cisbio | 63ADK000CB21PEG |
| GTP | Sigma | V900868 |
| Consumables | Brand | Item number |
| Topseal A | PerkinElmer | E5341 |
| 384-Well Polypropylene microplate | labcyte | PP-0200 |
| 96 Well Plates | Nunc | 249944 |
| 384-well plates | Corning | CLS4514 |
| Instrument | Brand | Item number |
| Envision | Perkin Elmer | 2104 |
| Centrifuge | Eppendorf | 5810R |
| Multi-channel pipettes | Eppendorf/Sartorius | / |
| Echo | Labcyte | / |

### 3. Experimental methods:

### 3.1 Experimental steps:

a) BI-2852 was used as a positive control, and its stock solution was the first point of dilution, then the solution was 3-fold diluted, with 10+0 points of dilution. Similarly, the first dilution point of the compound to be tested was its storage solution, and the solution was 3-fold diluted, with 11 +0 points of dilution. Echo was used to transfer 0.2 µL gradient diluted solution of the compounds into a 384-well plate, with 2 duplicated wells for each compound, and the final concentration of DMSO was 1%. The solution was centrifuged for 1 min at 1000 rpm/min. The final concentrations of positive controls were 100, 33.33, 11.11, 3.70, 1.23, 0.412, 0.137, 0.046, 0.015, 0.005, 0 µm. The final concentrations of the compounds to be tested were 200, 66.67, 22.22, 7.41, 2.47, 0.27, 0.091, 0.03, 0.0152, 0.01, 0 µm.
b) The KRAS_G12D in the kit and GTP with a final concentration of 10 µm were co-prepared in diluent, 5 µL of the mixture was transferred to a 384 reaction plate, centrifuged at 1000 rpm/min for 1 min,
c) 5 µL of the cRAF mixture was transferred to a 384 reaction plate, centrifuged at 1000 rpm/min for 1 min, incubated at 25 °C for 15min.
d) 10 µL of the mixture to be tested was transferred to a 384 reaction plate, centrifuged at 1000 rpm/min for 1 min, incubated at 4 °C overnight.
e) Excitation wavelength 665 nm and emission wavelength 615 nm were read using the Envision multi-function plate reader. 665/615 Ratio signal intensity was used to characterize the activity of enzyme.
f) Analyzing raw data.

### 3.2 Experimental data processing method:

IC₅₀ of the compounds was fitted by Graphpad Prism 8 nonlinear regression equation:
Negative control: DMSO
Positive control: 100 µM BI-2852

The IC₅₀ (half inhibitory concentration) of the compound was obtained by using the following nonlinear fitting formula:
$Y = Bottom + \left(Top-Bottom\right) / \left(1+{10}^{∧}\left(\left(LogIC50-X\right)*HillSlope\right)\right)$
X: Compound concentration log value
Y: 665/615 Ratio

### Effect embodiment C

### 1 Experimental objectives

The inhibitory ability of small molecule compounds on the binding activity of KRAS_WT and SOS1 was examined by a drug screening system based on the combination of KRAS_WT and SOS1.

### 2. Experimental materials and instruments and equipment

| Reagents: | Brand | Item number |
|---|---|---|
| KRAS-WT/SOS1 binding kits | Cisbio | 63ADK000CB15PEH |
| GTP | Sigma | V900868 |
| Consumables | Brand | Item number |
| Topseal A | PerkinElmer | E5341 |
| 384-Well Polypropylene microplate | labcyte | PP-0200 |
| 96 Well Plates | Nunc | 249944 |
| 384-well plates | Corning | CLS4514 |
| Instrument | Brand | Item number |
| Envision | Perkin Elmer | 2104 |
| Centrifuge | Eppendorf | 5810R |
| Multi-channel pipettes | Eppendorf/Sartorius | / |
| Echo | Labcyte | / |

### Experimental methods:

### 3.1 Experimental steps:

a) BI-2852 was used as a positive control, and its stock solution was the first point of dilution, then the solution was 3-fold diluted, with 10+0 points of dilution. Similarly, the first dilution point of the compound to be tested was its storage solution, and the solution was 3-fold diluted, with 11 +0 points of dilution. Echo was used to transfer 0.2 µL gradient diluted solution of the compounds into a 384-well plate, with 2 duplicated wells for each compound, and the final concentration of DMSO was 1%. The solution was centrifuged for 1 min at 1000 rpm/min. The final concentrations of positive controls were 100, 33.33, 11.11, 3.70, 1.23, 0.412, 0.137, 0.046, 0.015, 0.005, 0 µm. The final concentrations of the compounds to be tested were 200, 66.67, 22.22, 7.41, 2.47, 0.27, 0.091, 0.03, 0.0152, 0.01, 0 µm.
b) The KRAS_WT in the kit and GTP with a final concentration of 10 µm were co-prepared in diluent, 5 µL of the mixture was transferred to a 384 reaction plate, centrifuged at 1000 rpm/min for 1 min,
c) 5 µL of the SOS1 mixture was transferred to a 384 reaction plate, centrifuged at 1000 rpm/min for 1 min, incubated at 25 °C for 15min.
d) 10 µL of the mixture to be tested was transferred to a 384 reaction plate, centrifuged at 1000 rpm/min for 1 min, incubated at 4 °C overnight.
e) Excitation wavelength 665 nm and emission wavelength 615 nm were read using the Envision multi-function plate reader. 665/615 Ratio signal intensity was used to characterize the activity of enzyme.
f) Analyzing raw data.

### 3.2 Experimental data processing method:

IC₅₀ of the compounds was fitted by Graphpad Prism 8 nonlinear regression equation:
Negative control: DMSO
Positive control: 100 µM BI-2852

The IC₅₀ (half inhibitory concentration) of the compound was obtained by using the following nonlinear fitting formula:
$Y = Bottom + \left(Top-Bottom\right) / \left(1+{10}^{∧}\left(\left({LogIC}_{50}-X\right)*HillSlope\right)\right)$
X: Compound concentration log value
Y: 665/615 Ratio

**Experimental results:** The experimental results of the above effects embodiments A, B and C are shown in table 16:

**Table 16 The test results of the effect embodiments A, B, C**

| Number of the compound | GTP-KRAS-G12D:SOS1 | GTP-KRAS-G12D:CRAF | GTP-KRAS-WT:SOS1 |
|---|---|---|---|
| | IC₅₀ (nM) | IC₅₀ (nM) | IC₅₀ (nM) |
| BI-2852 (positive control) | 238.4 | 2696 | 135.5 |
| Comparative embodiment a | 1348 | 7306 | - |
| Comparative embodiment b | 83372 | - | - |
| **9a** | 81579 | - | - |
| **9b** | > 200000 | - | - |
| **1a** | 6445 | - | - |
| **1b** | 31930 | - | - |
| **2a** | 247.6 | 589.1 | 9749 |
| **3a** | 1365 | - | - |
| **4a** | 36.94 | 213.2 | 401.5 |
| **4b** | 4585 | - | - |
| **5a** | 41.41 | 250.7 | 1813 |
| **5b** | 63263 | - | - |
| **6a** | 66.41 | 66 | 292.6 |
| **7a** | 277.8 | 3331 | 15886 |
| **8a** | 460.8 | 1169 | 5338 |
| **8b** | 70976 | - | - |
| **10a** | 780.3 | 3231 | 10308 |
| **10b** | 65088 | - | - |
| **11a** | 1527 | - | - |
| **12a** | 7116 | - | - |
| **13a** | 2237 | - | - |
| **14c** | 9490 | - | - |
| **14d** | 56683 | - | - |
| **15a** | 1346 | - | - |
| **15b** | 52672 | - | - |
| **16a** | 3491 | - | - |
| **16b** | 83353 | - | - |
| **17a** | 5811 | - | - |
| **18b** | 2363 | - | - |
| **20a** | 4796 | - | - |
| **19a** | 897.6 | 1632 | 9415 |
| **21a** | 1285 | - | - |
| **22b** | 2417 | - | - |
| **23** | 946.5 | 22599 | 12439 |
| **25** | 293.8 | 4380 | 2551 |
| **28** | 312 | 4699 | 22528 |
| **30** | 1234 | - | - |
| **31** | ~ 69672 | - | - |
| **32** | 186837 | - | - |
| **34** | 188.2 | - | 279.1 |
| **36** | 276.9 | - | >100000 |
| **37** | 629.4 | 2141 | 1014 |
| **39a** | 27.73 | 26.21 | 341.6 |
| **39b** | 17666 | - | - |
| **40a** | 15.21 | 103.7 | 1042 |
| **40b** | 4800 | - | - |
| **42b** | 6473 | - | - |
| **43b** | 2574 | - | - |
| **45a** | 1259 | - | - |
| **47a** | 207.6 | 823.9 | 5700 |
| **48a** | 23.83 | 130 | 6017 |
| **49a** | 36.24 | 159.4 | 1713 |
| **49b** | 13790 | - | - |
| **50a** | 44.34 | 155.1 | 1695 |
| **51a** | 30.76 | 144 | 1423 |
| **51b** | 50831 | - | - |
| **52a** | 1135 | 9210 | 28860 |
| **52b** | 73904 | - | - |
| **53a** | 59.67 | 146.8 | 2170 |
| **53b** | 2294 | - | - |
| **54a** | 26.22 | 102.7 | 1193 |
| **54b** | 22090 | - | - |
| **55a** | 1122 | 9952 | >100000 |
| **56a** | 64.14 | 461.5 | 13957 |
| **56b** | 15729 | - | - |
| **57b** | 419.4 | 7908 | 44507 |
| **58a** | 20.42 | 12.57 | 47.15 |
| **58b** | 15103 | - | - |
| **59a** | 45.35 | 182.1 | 2077 |
| **59b** | 5980 | - | - |
| **60a** | 62.96 | 357.5 | 5904 |
| **60b** | 72389 | - | - |
| **61a** | 229.6 | 1597 | 68296 |
| **63a** | 17.75 | 133.8 | 1579 |
| **63b** | 12947 | - | - |
| **64a** | 31661 | - | - |
| **64b** | 44.61 | 282.6 | 1251 |
| **65a** | 44571 | - | - |
| **65b** | 84.26 | 434.2 | 270.3 |
| **66a** | 38.33 | 138.8 | 1022 |
| **66b** | 18003 | - | - |
| **67** | 275.2 | 1224 | 33087 |
| **68** | 214.2 | 645.9 | 16736 |
| **69a** | 225.3 | 1847 | >100000 |
| **69b** | 157.5 | 1957 | >100000 |
| **70a** | 93.88 | 591.9 | >100000 |
| **70b** | 66.05 | 514.9 | >100000 |
| **72a** | >200000 | - | - |
| **72b** | 154569 | - | - |
| **72c** | >200000 | - | - |
| **72d** | 175620 | - | - |
| **73** | 15732 | - | - |
| **74a** | 100324 | - | - |
| **74b** | >200000 | - | - |
| **76** | 8964 | - | - |
| **77** | 1035 | - | - |
| **78** | 44.18 | - | 846.7 |
| **82** | 21.6 | 102.1 | 1561 |
| **84** | 3049 | - | - |
| **85** | 4791 | - | - |
| **86** | 3571 | - | - |
| **87** | 23259 | - | - |
| **70c** | 74.09 | 416.7 | 38283 |
| **70d** | 93.88 | 356.1 | 60453 |
| **89a** | 47.68 | 109.8 | 2189 |
| **89b** | 5339 | - | - |
| **90a** | 263 | - | - |
| **91** | 15.09 | 78.25 | 1098 |
| **92** | 37.39 | 138.9 | 4714 |
| **93** | 12.33 | 82.11 | 2027 |
| **94** | 53.25 | 164.4 | 5224 |
| **95** | 48.18 | 283.8 | 6665 |
| **96a** | 22.03 | 88.51 | 1538 |
| **96b** | 34.28 | 156.3 | 2970 |
| **97** | 18.92 | 15.6 | 290.8 |
| **98** | 95.27 | 476.9 | 7700 |
| **99** | 52.25 | 167.8 | 2604 |
| **100** | 19.09 | 53.71 | 504.4 |
| **101** | 31.72 | 182 | 2472 |
| **102** | 19.8 | 39.01 | 1101 |
| **103** | 19.37 | 119.5 | 1261 |
| **104** | 40.87 | 247.5 | >10000 |
| **105** | 22.44 | 184.9 | 3040 |
| **106** | 39.15 | 157.7 | 1766 |
| **107** | 47 | 159.7 | 3485 |
| **108a** | 56.26 | 212.1 | 2300 |
| **108b** | 108.1 | - | - |
| **109a** | 26.96 | 89.34 | 2573 |
| **109b** | 58.21 | 208.5 | 5350 |
| **110** | 39.61 | 142.9 | 1532 |
| **111** | 22.42 | 106.9 | 1110 |
| **112a** | 57.38 | 119.3 | 2517 |
| **112b** | 120.5 | - | - |
| **113** | 51.84 | 130.2 | 4001 |
| **114** | 37.16 | 100.3 | 4530 |
| **115** | 67.76 | 129.8 | 4127 |
| **116** | 65.67 | 188.5 | 3377 |
| **117** | 59.33 | 133.5 | 2126 |
| **118** | 19.47 | 53.71 | 2411 |
| **119** | 14.48 | 14.37 | 159.6 |
| **120** | 36.45 | 72.7 | 2666 |
| **121** | 147.3 | - | - |
| **122** | 218.7 | - | - |
| **123** | 105.7 | - | - |
| **124** | 75.84 | 149 | 2891 |
| **125** | 52.68 | 169.7 | 1681 |
| **126** | 134.2 | - | - |
| **127** | 87.62 | 251.1 | 6390 |
| **128** | 9.412 | 23.55 | 242.7 |
| **129** | 42 | 179.7 | 2271 |
| **130** | 842.5 | - | - |
| **131** | 20.87 | 37.03 | 222.1 |
| **132** | 762.5 | - | - |
| **133** | 19.34 | 18.69 | 85.42 |
| **134** | 17.93 | 26.24 | 498.3 |
| **135** | 133 | - | - |
| **136** | 30.67 | 67.52 | 1165 |
| **137** | 13.33 | 10.69 | 123.2 |
| **138** | 108.3 | - | - |
| **139** | 37.19 | 128.6 | 1915 |
| **140** | 32.8 | 95.71 | 1358 |
| **141** | 87.64 | - | - |
| **142** | 15.98 | 30.48 | 393.7 |
| **143** | 16.65/21.24 | 47.68 | 764.9 |
| **144** | 14.4 | 17.86 | 180.2 |
| **145** | 5937 | - | - |
| **146** | 6935 | - | - |
| **147** | 605.3 | - | - |
| **148** | 28.55 | 121.8 | 2968 |
| **149a** | 39.63 | 96.89 | 854.2 |
| **150** | 39.19 | 113.6 | 2038 |
| **152a** | 1055 | - | - |
| **153a** | 965.2 | - | - |
| **154a** | 203.7 | - | - |
| **155** | 78.84 | 311.8 | 243.3 |
| **156** | 620.9 | - | - |
| **157** | 315.6 | - | - |
| **158** | 199.2 | - | - |
| **159** | 30.76 | 55.23 | 195.8 |
| **160a** | 19.39 | 15.13 | 151.6 |
| **160b** | 116.7 | - | - |
| **161** | >10000 | - | - |
| **162a** | 678 | - | - |
| **160** | 27.43 | 32.38 | 390.3 |
| **163** | 18.61 | 49.83 | 786.9 |
| **164** | 43.88 | 115.1 | 2531 |
| **165** | 27.81 | 85.42 | 1884 |
| **166** | 25 | 31.92 | 553.2 |
| **167** | 17.88 | 19.46 | 247.5 |
| **168** | 18.73 | 22.37 | 457.9 |
| **169** | 39.2 | 71.26 | 1874 |
| **170** | 12.78 | 24.35 | 269.7 |
| **171** | 24.83 | 60.44 | 395.4 |
| **172** | 19.34 | 62.69 | 658 |
| **173** | 14.83 | 37.92 | 251.1 |
| **174** | 57.8 | 170.5 | 1819 |
| **175** | 104.2 | 303.2 | 4297 |
| **176** | 13.56 | 6.978 | 28.05 |
| **177** | 40.46 | 121.4 | 1189 |
| **178a** | 21.58 | 18.09 | 836.5 |
| **178b** | 39.73 | 150.2 | 1046 |
| **179a** | 276.4 | - | - |
| **179b** | 59.98 | 133.5 | 5403 |
| **180** | 87.28 | 255.5 | 3925 |
| **181** | 38.09 | 136.4 | 3090 |
| **182** | 53.35 | 219 | 3400 |
| **183** | 29.25 | 88.92 | 1505 |
| **184** | 113.3 | 460.6 | - |
| **185** | 386.8 | - | - |
| **186** | 16.89 | 18.07 | 232.8 |
| **187a** | 27.37 | 66.19 | 1351 |
| **187b** | 27.72 | 115.1 | 1675 |
| **188** | 35.64 | 133.1 | 1017 |
| **189** | 8319 | - | - |
| **190** | 15.34 | 28.88 | 292.1 |
| **192b** | 186.4 | 431.3 | - |
| **194a** | 27.89 | 154.7 | 204.8 |
| **195a** | 8768 | - | - |
| **195b** | 57.66 | 333.5 | 6489 |
| **197a** | 17.77 | 59.81 | 832.7 |
| **197b** | 2708 | - | - |
| **198a** | 11.4 | 6.115 | 23.53 |
| **198b** | 54.14 | 38.46 | 105.5 |
| **199** | 13.21 | 9.002 | 58.42 |
| **200** | 61.96 | 73.74 | 2487 |
| **201a** | 29 | 23.18 | 1044 |
| **201b** | 54.56 | 45.81 | 2184 |
| **202** | 51.82 | 168.1 | 2152 |
| **203** | 19.79 | 33.92 | 893.2 |
| **204** | 77.37 | 120.6 | 4248 |
| **205** | 11.58 | 12.98 | 505.3 |
| **206** | 46.07 | 134.4 | 6834 |
| **207** | 11.59 | 7.233 | 195.8 |
| **208a** | 42.53 | 109 | - |
| **208b** | 97.48 | 272.2 | - |
| **209a** | 36.12 | 59.42 | 5648 |
| **209b** | 90.94 | 292.9 | - |
| **210** | 17.84 | 17.83 | 2067 |
| **212** | 555.8 | - | - |
| **213a** | 514.2 | - | - |
| **213b** | 673.1 | - | - |
| **214a** | 13.16 | 8.684 | 8.008 |
| **214b** | 2661 | - | - |
| **215a** | 11.91 | 5.441 | 52.37 |
| **215b** | 1583 | - | - |
| **216** | 51.79 | 49.4 | 2023.0 |
| **217** | 190.8 | - | - |
| **218a** | 91.86 | 578.7 | - |
| **218b** | 249.8 | - | - |
| **219** | 17.35 | 21.62 | - |
| **220** | 19.33 | 29.65 | - |
| **221** | 63.58 | 175.2 | 5028 |
| **222** | 580.9 | - | - |
| **223** | 17.46 | 15.58 | 1701 |
| **224** | 13.24 | 18.65 | 1348 |
| **225** | 17.16 | 17.78 | 969.2 |
| **226** | 67.94 | 153.5 | - |
| **227** | 16.72 | 23.4 | 593.1 |
| **228** | 14.16 | 18.3 | 92.3 |
| **229a** | 16.44 | 11.82 | 160.1 |
| **230** | 15.71 | 28.1 | 626.4 |
| **232a** | 13.75 | 7.788 | 10.28 |
| **232b** | 13.5 | 14.82 | 66.21 |
| **233a** | 3275 | - | - |
| **233b** | 9.101 | 7.184 | 226.7 |
| **234a** | 11.82 | 5.793 | 199.7 |
| **235** | 52.36 | 148.7 | 1767 |
| **236** | 19.09 | 15.04 | 309.1 |
| **237** | 19.51 | 18.63 | 568.6 |
| **238** | 43.3 | 155.5 | 3077 |
| **239** | 15.32 | 18.27 | 495.7 |
| **240** | 39.15 | 164.3 | 2207 |
| **241** | 49.7 | 202.5 | 3602 |
| **242** | 13.66 | 23.27 | 731.2 |
| **243** | 19.16 | 37.5 | 1247 |
| **244** | 48.5 | 260.2 | 3336 |
| **245** | 27.42 | 110.4 | 1476 |
| **246** | 37.42 | 218.5 | 3699 |
| **247** | 13.33 | 28.51 | 943.9 |
| **248** | 31.08 | 67.19 | 98.12 |
| **249** | 23.72 | 16.39 | 84.59 |
| **250a** | 8.423 | 14.61 | 75.18 |
| **250b** | 1626 | - | - |
| **251** | 27.55 | 67.63 | 257 |
| **252** | 21.67 | 48.05 | 413.9 |
| **253** | 63 | 489.3 | 1978 |
| **255a** | 5124 | - | - |
| **255b** | 4.321 | 13.19 | 75.6 |
| **256** | 92.12 | 358.4 | - |
| **257** | 64.83 | 295.7 | >10000 |
| **258** | 26.9 | 218.9 | 6128 |
| **259** | 25.31 | 146.4 | 1469 |
| **260** | 25.86 | 157.4 | 4423 |
| **261** | 18.7 | 102.2 | 1078 |
| **262** | 24.27 | 141.3 | 1656 |
| **263** | 23.15 | 118.00 | 863.10 |
| **266** | 173.6 | 746.4 | >10000 |
| **268** | 52.37 | 346 | 3609 |
| **269** | 67.88 | 339.1 | 1757 |
| **270** | 43.12 | 202.1 | 1210 |
| **264a** | 11.74 | 32.07 | 341.70 |
| **264b** | 19.35 | 101.10 | 196.00 |
| **265a** | 91.24 | 285.8 | 5827 |
| **265b** | 59.6 | 199 | 7854 |
| **265c** | 49.81 | 134.8 | 1282 |
| **265d** | 50.04 | 184.3 | 4896 |
| **267a** | 76.67 | 258.9 | 6343 |
| **267b** | 117.6 | 277.5 | 4595 |
| **271a** | 9.855 | 11.36 | 179 |
| **271b** | 9411 | \ | \ |
| **272b** | 18.01 | 76.8 | 4257 |
| **273a** | 11.3 | 7.905 | 69.8 |
| **273b** | 1846 | \ | \ |
| **278a** | 17.56 | 38.62 | 122 |
| **278b** | 2482 | \ | \ |
| **279a** | 8.769 | 10.94 | 27.29 |
| **279b** | 1386 | \ | \ |
| **280a** | 17.09 | 14.07 | 233.9 |
| **280b** | 310.2 | \ | \ |
| **281a** | 17.84 | 20.32 | 564 |
| **281b** | 448.9 | \ | \ |
| **282a** | 16.46 | 13.65 | 175 |
| **282b** | 5069 | \ | \ |
| **283a** | 15.78 | 19.15 | 761.3 |
| **283b** | 9183 | \ | \ |
| **284a** | 17.27 | 11 | 251.5 |
| **284b** | 2012 | \ | \ |
| **285a** | 16.62 | 9.121 | 18.42 |
| **285b** | 1456 | \ | \ |
| **286a** | 13.5 | 9.768 | 261.8 |
| **287a** | 10.11 | 11.35 | 29.13 |

| | | | |
|---|---|---|---|
| + "-" means not tested. | | | |

## Claims

1. A quinazoline compound represented by formula I, a pharmaceutically acceptable salt thereof, a solvate thereof, a prodrug thereof, a metabolite thereof or an isotopic compound thereof, 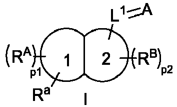
wherein, ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 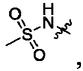 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being "halogen, C₁-C₆ alkyl, cyano or C₂-C₆ alkynyl", C₄-C₈ cycloalkyl-fused C₆-C₁₀ aryl substituted by one or more R^{a-2}, or C₆-C₁₀ aryl substituted by one or more R^{a-4}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 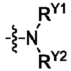 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 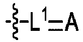
is 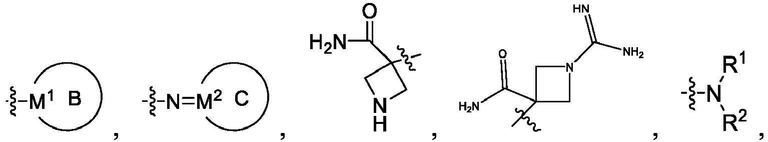 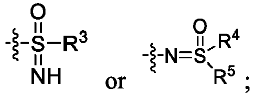
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 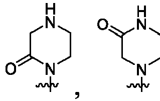 , or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 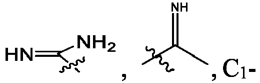 C₆ alkyl, C₁-C₃ alkyl substituted by CN, or C₄-C₆ alkyl substituted by CN; m is 0, 1 or 2; n is 1, 2 or 3;
R¹⁰ is amino or 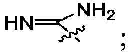
R¹¹ is independently halogen, 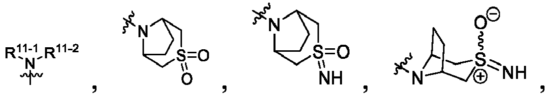 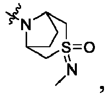 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4-to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 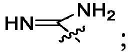
R¹¹⁻³ is H, 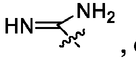 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, C₁-C₆ alkyl substituted by R¹¹⁻³⁻¹, C₁-C₆ alkoxy substituted by R¹¹⁻³⁻², or C₂-C₆ alkenyl;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 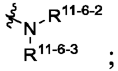
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 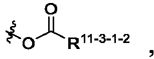 unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R¹¹⁻³⁻¹⁻² is independently 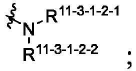
R¹¹⁻³⁻¹⁻²⁻¹ and R¹¹⁻³⁻¹⁻²⁻² are independently H or C₁-C₆ alkyl;
R¹¹⁻³⁻² is C₁-C₁₂ alkenyl;
R^{L} is H or 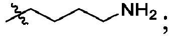
R^{a-1} is independently hydroxyl, halogen, cyano, C₁-C₆ alkyl, C₃-C₁₂ cycloalkyl, unsubstituted C₂-C₆ alkynyl or C₂-C₆ alkynyl substituted by one or more R^{a-1-1}, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R^{a-4} is 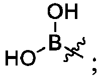
R¹² is C₁-C₆ alkyl;
R¹³ is H, C₁-C₆ alkyl, 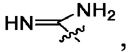 or C₃-C₆ cycloalkyl substituted by R¹³⁻¹;
R¹³⁻¹ is 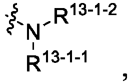 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 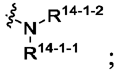
R¹⁴⁻¹⁻¹ and R¹⁴⁻¹⁻² are independently H or C₁-C₆ alkyl;
R¹⁵ is 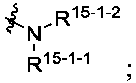
R¹⁵⁻¹⁻¹ and R¹⁵⁻¹⁻² are independently H or C₁-C₆ alkyl;
R¹⁶ is 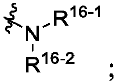
R¹⁶⁻¹ and R¹⁶⁻² are independently H or C₁-C₆ alkyl;
R¹⁷ is 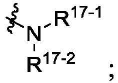
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 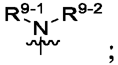
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), 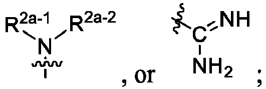
R^{2a-1} and R^{2a-2} are independently H, -C(=O)CH₃, C₁-C₃ alkyl or C₄-C₆ alkyl;
when ring 1 is independently pyridine, R^{B} is

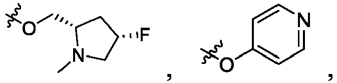

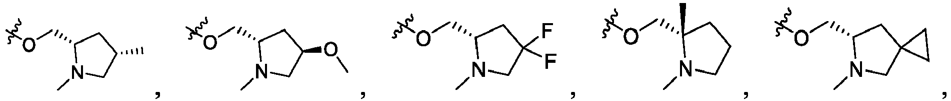

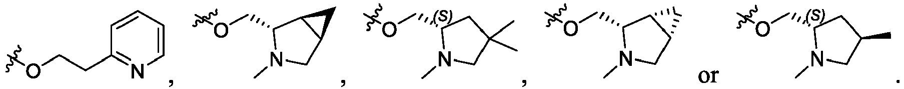

2. The quinazoline compound represented by formula I, the pharmaceutically acceptable salt thereof, the solvate thereof, the prodrug thereof, the metabolite thereof or the isotopic compound thereof according to claim 1, wherein, the quinazoline compound represented by formula I is a compound represented by formula I-AI, 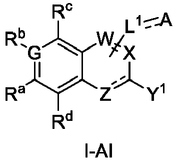
wherein, R^{b}, R^{c} and R^{d} are independently H or halogen;
W is C;
X and Z are N;
G is C or N;
" 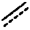 " in 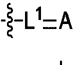 is a single bond or a double bond;
" 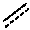 " in 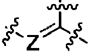 is a single bond or a double bond.

3. The quinazoline compound represented by formula I, the pharmaceutically acceptable salt thereof, the solvate thereof, the prodrug thereof, the metabolite thereof or the isotopic compound thereof according to claim 1, wherein, the quinazoline compound represented by formula I, the pharmaceutically acceptable salt thereof, the solvate thereof, the prodrug thereof, the metabolite thereof or the isotopic compound thereof according to claim 1 is any of the following schemes:
scheme 1:
wherein, 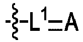 is 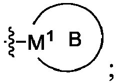 ring B is 7- to 12-membered heterocycloalkyl with 2 to 3 heteroatoms selected from N, S and O;
scheme 2:
wherein, 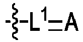 is 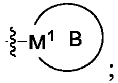 ring B is 7- to 12-membered bridged heterocycloalkyl with 2 heteroatoms being N;
scheme 3:
ring B is 7- to 12-membered spiro heterocycloalkyl with 2 heteroatoms being N;
scheme 4:
wherein, 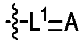 is 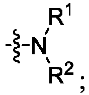
scheme 5:
wherein, 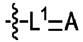 is 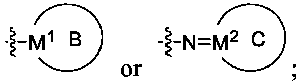
ring B is unsubstituted 4- to 10-membered heterocycloalkyl with 1 to 3 heteroatoms or heteroatom groups selected from N and P(=O) and containing only one N;
ring C is unsubstituted 4- to 10-membered heterocycloalkyl or 4- to 10-membered heterocycloalkyl substituted by R⁹ with 1 to 3 heteroatoms or heteroatom groups selected from N and S(=O) and containing only one N;
scheme 6:
wherein, 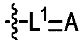 is 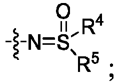
scheme 7:
the quinazoline compound represented by formula I is a compound represented by formula I-B, 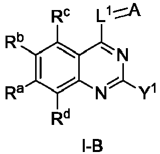
wherein, R^{a} is 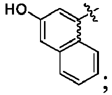
" 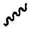 " indicates " 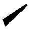 ", " 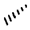 " or a mixture thereof;
R^{b} is halogen;
R^{c} is H;
R^{d} is halogen;
Y¹ is 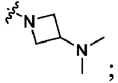
" 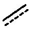 " in 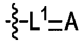 is a single bond; 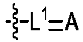
is 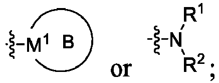
M¹ is N or CH;
ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring;
R¹ is H, or C₁-C₃ alkyl;
R² is -(CH₂)ₙ-R^{2a};
n is 1, 2, 3 or 4;
R^{2a} is H, -OH or 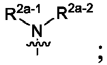
R^{2a-1} and R^{2a-2} are independently H or C₁-C₃ alkyl;
scheme 8:
the quinazoline compound represented by formula I is a compound represented by formula I-B, 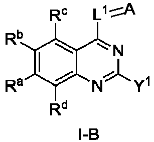
wherein, R^{a} is 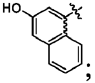
" 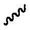 " indicates " 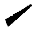 ", " 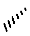 " or a mixture thereof;
R^{b} is halogen;
R^{c} is H;
R^{d} is halogen;
Y¹ is 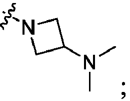
" 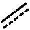 " is a single bond; 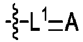
is 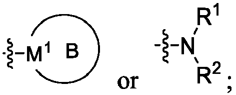
M¹ is N or CH;
ring B is unsubstituted 7- to 12-membered bridged heterocycloalkyl with 2 heteroatoms being N, unsubstituted 7- to 12-membered spiro heterocycloalkyl with 2 heteroatoms being N, or unsubstituted 7- to 12-membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring;
R¹ is H or C₁-C₃ alkyl;
R² is -(CH₂)ₙ-R^{2a};
n is 1, 2 or 3;
R^{2a} is H, -OH or 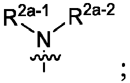
R^{2a-1} and R^{2a-2} are independently H or C₁-C₃ alkyl, and R^{2a-1} and R^{2a-2} are not C₁-C₃ alkyl at the same time;
scheme 9:
the quinazoline compound represented by formula I is a compound represented by formula I-B, 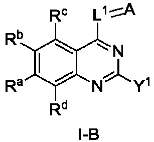
wherein, R^{a} is 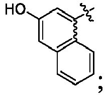
" 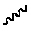 " indicates " 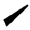 ", " 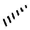 " or a mixture thereof;
R^{b} is halogen;
R^{c} is H;
R^{d} is halogen;
Y¹ is 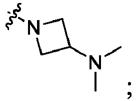
" 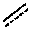 " in 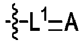 is a single bond; 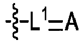
is 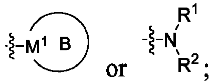
M¹ is N;
ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
R¹ is H or C₁-C₃ alkyl;
R² is -(CH₂)ₙ-R^{2a};
n is 1, 2 or 3;
R^{2a} is 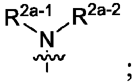
R^{2a-1} and R^{2a-2} are H;
scheme 10:
the quinazoline compound represented by formula I is a compound represented by formula I-B, 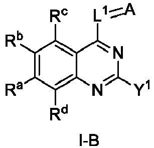
wherein, R^{a} is 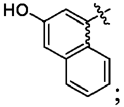
" 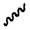 " indicates " 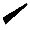 ", " 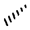 " or a mixture thereof;
R^{b} is halogen;
R^{c} is H;
R^{d} is halogen;
Y¹ is 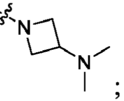
" 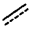 " is a single bond; 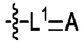
is 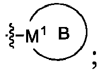
M¹ is N;
ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
the 7- to 12-membered bridged heterocycloalkyl with 2 heteroatoms being N is 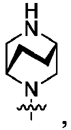 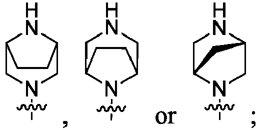
the 7- to 12-membered spiro heterocycloalkyl with 2 heteroatoms being N, wherein one ring is a 4-membered heterocycloalkyl with 1 atom being N, and the N atom in the 4-membered heterocycloalkyl is attached to the quinazoline ring;
scheme 11:
in the quinazoline compound represented by formula I, ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P;
p1 and p2 are independently 1, 2, 3, 4 or 5;
R^{A} is H, halogen, hydroxyl, cyano, amino, C₁-C₃ alkyl, or C₁-C₃ alkoxy;
R^{a} is C₆-C₁₀ aryl substituted by one or more hydroxyl;
R^{B} is H, halogen, hydroxyl, cyano, amino, C₁-C₃ alkyl, C₁-C₃ alkoxy or Y¹;
Y¹ is 3- to 10-membered heterocycloalkyl substituted by one or more 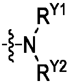 with 1 to 4 heteroatoms selected from one or more of N, O and S;
R^{Y1} and R^{Y2} are independently H or C₁-C₃ alkyl; 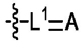
is 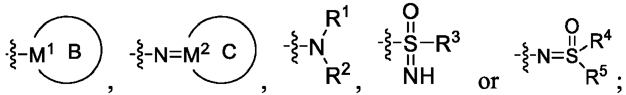
M¹ is N, CH or P(=O);
ring B is unsubstituted 4- to 10-membered heterocycloalkyl with 1 to 3 heteroatoms or heteroatom groups selected from N and P(=O) and containing only one N, unsubstituted 7- to 12-membered heterocycloalkyl or 7- to 12-membered substituted by R⁷ with 2 to 3 heteroatoms selected from one or more of N, O and S, 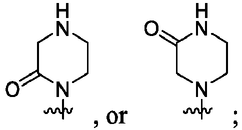 one N atom in the 7- to 12-membered heterocycloalkyl is attached to the quinazoline ring;
R⁷ is -C(=O)O-(C₁-C₄ alkyl), or C₁-C₃ alkyl substituted by CN;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 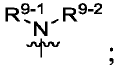
R⁹⁻¹ and R⁹⁻² are independently H, C₁-C₃ alkyl, or C₁-C₃ alkyl substituted by CN;
R¹ and R⁴ are independently H or C₁-C₃ alkyl;
R², R³ and R⁵ are independently -(CH₂)ₙ-R^{2a};
n is 1, 2, 3 or 4;
R^{2a} is H, -OH, -O-(C₁-C₃ alkyl) or 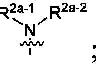
R^{2a-1} and R^{2a-2} are independently H or C₁-C₃ alkyl;
scheme 12:
the quinazoline compound represented by formula I is a compound represented by formula I-BI, 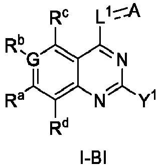
wherein, 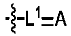 is 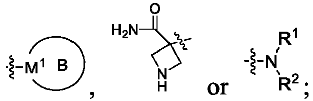
ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N is 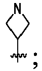
the 4- to 10-membered monocyclic heterocycloalkyl with 1 to 3 heteroatoms or heteroatom groups selected from N and P(=O) and containing only one N is 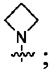
the 4- to 10-membered bridged heterocycloalkyl substituted by R¹⁰ with 1 to 3 heteroatoms or heteroatom groups selected from N and P(=O) and containing only one N is 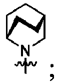
the 7- to 12-membered bridged heterocycloalkyl substituted by R⁷ with 2 to 3 heteroatoms selected from one or more of N, O and S is 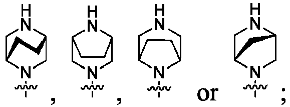
R^{2a-1} and R^{2a-2} are independently H, -C(=O)CH₃, C₁-C₃ alkyl, or C₄-C₆ alkyl, and R^{2a-1} and R^{2a-2} are not C₁-C₃ alkyl or C₄-C₆ alkyl at the same time;
R⁷ is 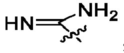 , C₁-C₃ alkyl, or C₄-C₆ alkyl;
scheme 13:
the quinazoline compound represented by formula I is a compound represented by formula I-BI, 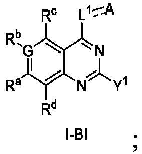
wherein, G is C or N;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl;
R^{b} is halogen;
R^{c} is H;
R^{d} is halogen;
Y¹ is 3- to 10-membered heterocycloalkyl substituted by one or more 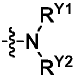 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
" 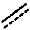 " in 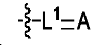 is a single bond; 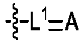
is 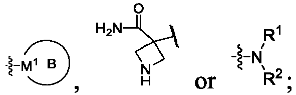
M¹ is N or CH;
ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalky with 2 heteroatoms being N, unsubstituted 7- to 12-membered fused heterocycloalky with 2 heteroatoms being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S; one N atom in the 7- to 12-membered heterocycloalkyl is attached to ring 2;
wherein, the 4- to 10-membered heterocycloalkyl with 1 heteroatom or heteroatom group being N can be 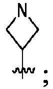
the 4- to 10-membered monocyclic heterocycloalkyl with 1 to 3 heteroatoms or heteroatom groups selected from N and P(=O) and containing only one N can be 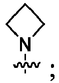
the 4- to 10-membered bridged heterocycloalkyl substituted by R¹⁰ with 1 to 3 heteroatoms or heteroatom groups selected from N and P(=O) and containing only one N can be 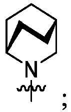
the 7- to 12-membered bridged heterocycloalkyl substituted by R⁷ with 2 to 3 heteroatoms selected from one or more of N, O and S can be 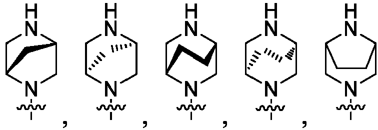 or 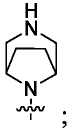
R¹ is H, C₁-C₃ alkyl, or C₄-C₆ alkyl;
R² is -(CH₂)ₙ-R^{2a};
n is 1 or 2;
R^{2a} is 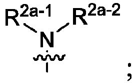
R^{2a-1} and R^{2a-2} are independently H, -C(=O)CH₃, C₁-C₃ alkyl or C₄-C₆ alkyl;
R¹⁰ is amino or 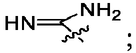
R⁷ is -C(=O)O-(C₁-C₄ alkyl), 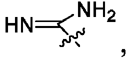 C₁-C₆ alkyl, C₁-C₃ alkyl substituted by CN, or C₄-C₆ alkyl substituted by CN;
R¹¹ is optionally halogen, 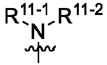 , unsubstituted 4- to 10-membered heterocycloalkyl or 4- to 10-membered heterocycloalkyl substituted by one or more R¹¹⁻³ with 1 to 3 heteroatoms selected from N and O, or unsubstituted 5- to 6-membered heteroaryl or 5- to 6-membered heteroaryl substituted by one or more R¹¹⁻⁴ with 1 to 3 heteroatoms selected from N and O;
R¹¹⁻¹ and R¹¹⁻² are independently H, unsubstituted C₁-C₆ alkyl or C₁-C₆ alkyl substituted by R¹¹⁻¹⁻¹, or 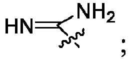
R¹¹⁻³ is H, 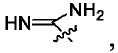 halogen, unsubstituted 4- to 10-membered heterocycloalkyl or 4-to 10-membered heterocycloalkyl substituted by C₁-C₆, or C₁-C₆ alkyl; the heteroatom is selected from N and O, and the number of heteroatoms is 1 to 3;
R¹¹⁻⁴ is C₁-C₆ alkyl;
R¹¹⁻¹⁻¹ is 4- to 10-membered heterocycloalkyl substituted by R^{L} with 1 to 3 heteroatoms selected from N and O;
R^{L} is H or 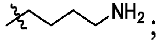
R^{a-1} is optionally hydroxyl, halogen or C₁-C₆ alkyl;
R¹² is C₁-C₆ alkyl;
R¹³ is H, C₁-C₆ alkyl, 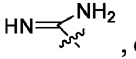 or C₃-C₆ cycloalkyl substituted by R¹³⁻¹;
R¹³⁻¹ is 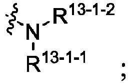
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 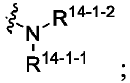
R¹⁴⁻¹⁻¹ and R¹⁴⁻¹⁻² are independently H or C₁-C₆ alkyl;
R¹⁵ is 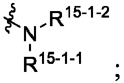
R¹⁵⁻¹⁻¹ and R¹⁵⁻¹⁻² are independently H or C₁-C₆ alkyl;
R¹⁶ is 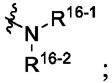
R¹⁶⁻¹ and R¹⁶⁻² are independently H or C₁-C₆ alkyl;
R¹⁷ is 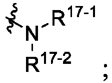
R¹⁷⁻¹ and R¹⁷⁻² are independently H or C₁-C₆ alkyl;
scheme 14:
the quinazoline compound represented by formula I is a compound represented by formula I-BI, 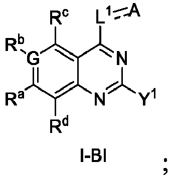
wherein, G is C or N;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl;
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 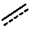 " is a single bond; 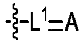
is 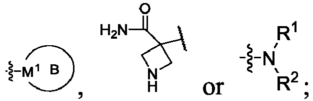
M¹ is N or CH;
ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalky with 2 heteroatoms being N, unsubstituted 7- to 12-membered fused heterocycloalky with 2 heteroatoms being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 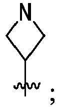
the 4- to 10-membered monocyclic heterocycloalkyl with 1 to 3 heteroatoms or heteroatom groups selected from N and P(=O) and containing only one N can be 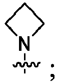
the 4- to 10-membered bridged heterocycloalkyl substituted by R¹⁰ with 1 to 3 heteroatoms or heteroatom groups selected from N and P(=O) and containing only one N can be 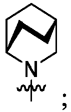
the 7- to 12-membered bridged heterocycloalkyl substituted by R⁷ with 2 to 3 heteroatoms selected from one or more of N, O and S can be 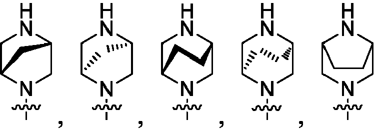 or 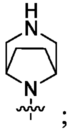
R¹ is H, C₁-C₃ alkyl, or C₄-C₆ alkyl;
R² is -(CH₂)ₙ-R^{2a};
n is 1 or 2;
R^{2a} is 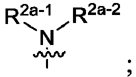
R^{2a-1} and R^{2a-2} are independently H, -C(=O)CH₃ or C₁-C₆ alkyl;
R¹⁰ is amino or 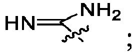
R¹⁰⁻¹ and R¹⁰⁻² are independently H or C₁-C₆ alkyl;
R⁷ is 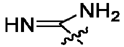 or C₁-C₆ alkyl;
R¹¹ is 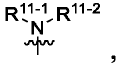 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 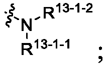
R¹³⁻¹⁻¹ and R¹³⁻¹⁻² are independently H or C₁-C₆ alkyl;
R¹⁴ is C₁-C₆ alkyl substituted by 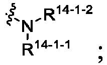
R¹⁴⁻¹⁻¹ and R¹⁴⁻¹⁻² are independently H or C₁-C₆ alkyl;
scheme 15:
the quinazoline compound represented by formula I is a compound represented by formula I-BI, 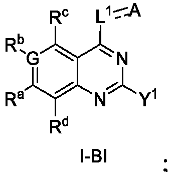
wherein, G is C or N;
R^{a} is 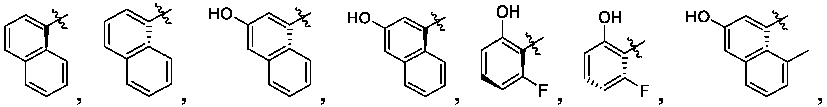 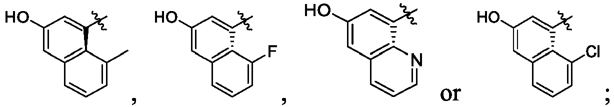
R^{b} is halogen;
R^{c} is H;
R^{d} is halogen;
Y¹ is 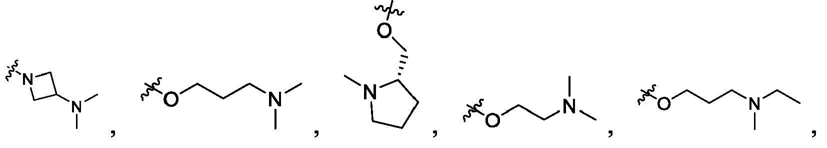 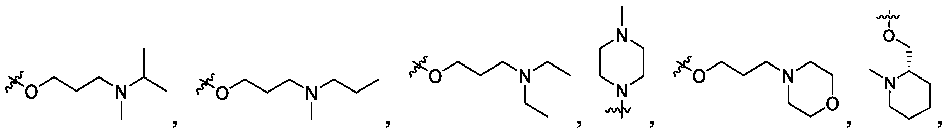 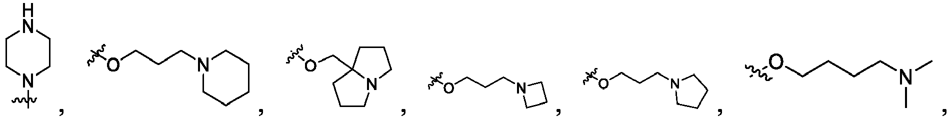 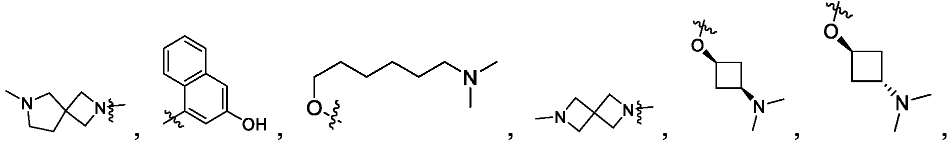 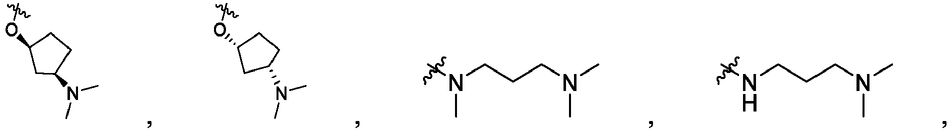 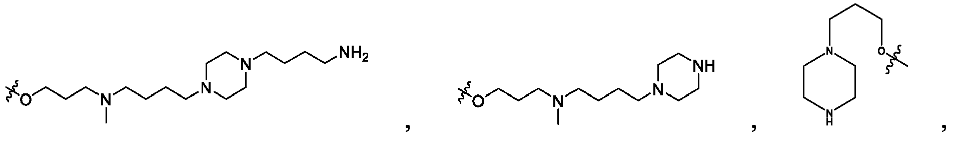 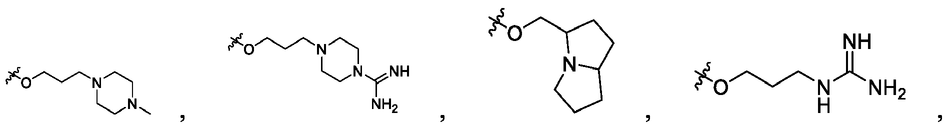 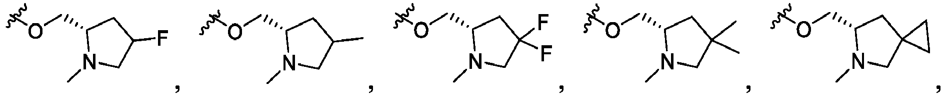 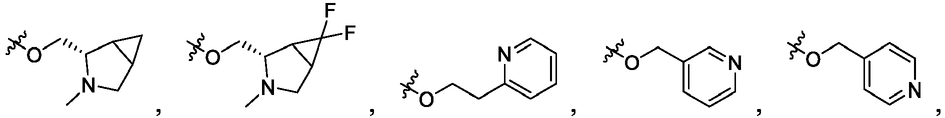 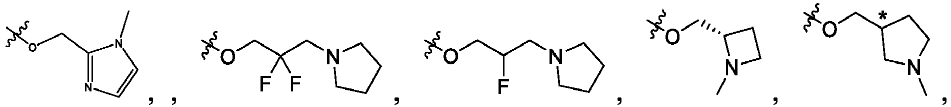 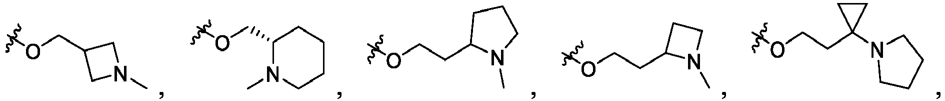 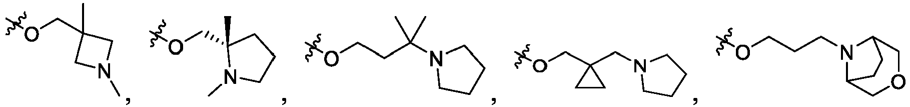 or 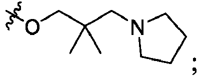
" 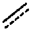 " in 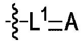 is a single bond; 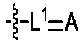
is 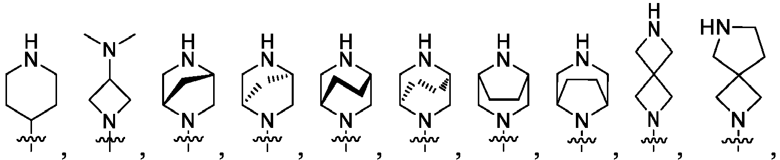 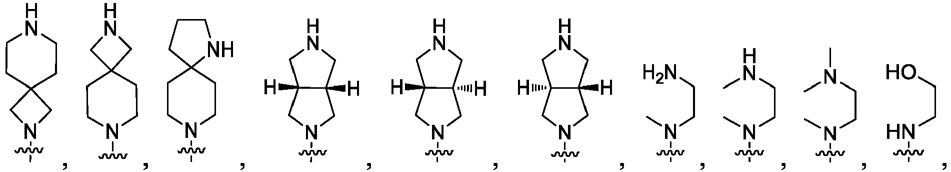 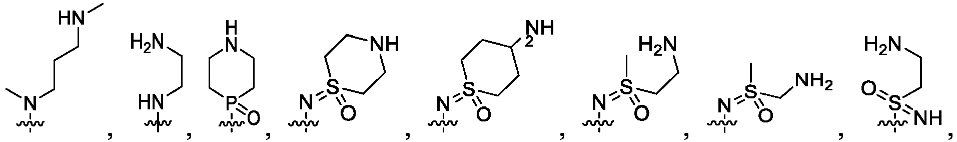 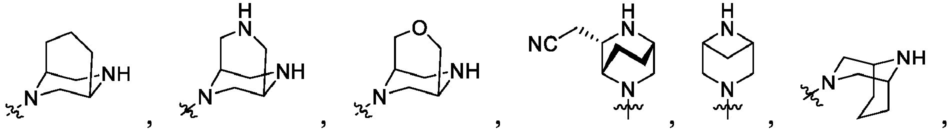 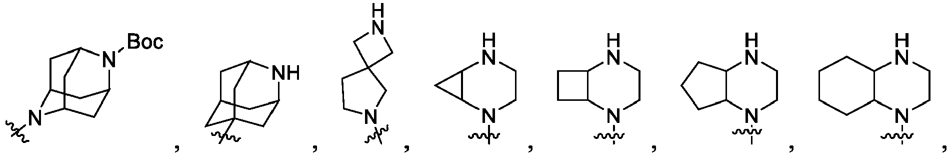 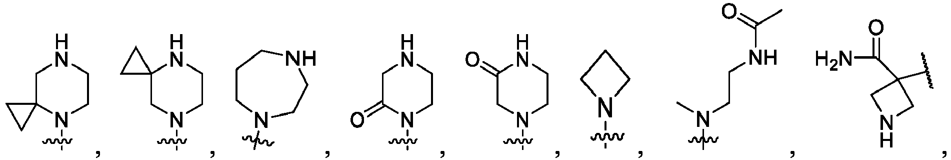 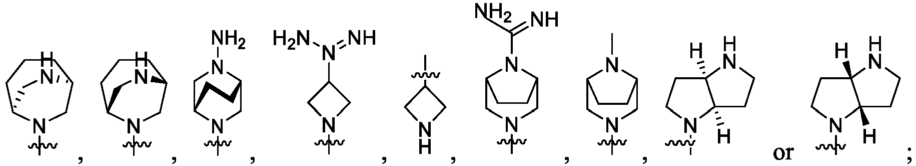
scheme 16:
the quinazoline compound represented by formula I is a compound represented by formula I-BI, 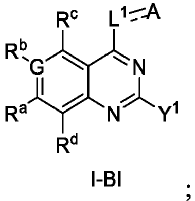
wherein, G is C or N;
R^{a} is 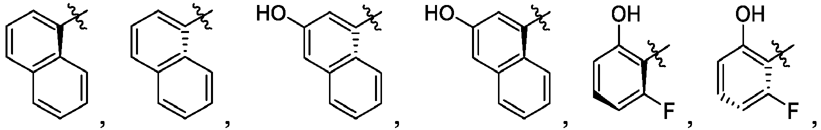 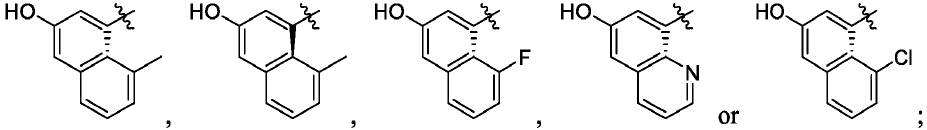
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 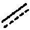 " is a single bond; 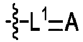
is 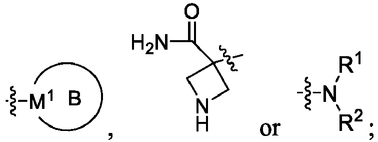
M¹ is N or CH;
ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 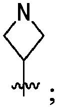
the 4- to 10-membered monocyclic heterocycloalkyl with 1 to 3 heteroatoms or heteroatom groups selected from N and P(=O) and containing only one N can be 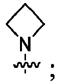
the 4- to 10-membered bridged heterocycloalkyl substituted by R¹⁰ with 1 to 3 heteroatoms or heteroatom groups selected from N and P(=O) and containing only one N can be 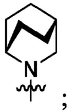
the 7- to 12-membered bridged heterocycloalkyl substituted by R⁷ with 2 to 3 heteroatoms selected from one or more of N, O and S can be 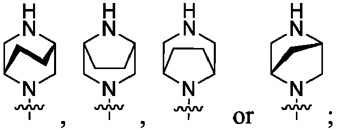
R¹ is H, C₁-C₃ alkyl, or C₄-C₆ alkyl;
R² is -(CH₂)ₙ-R^{2a};
n is 1 or 2;
R^{2a} is 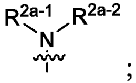
R^{2a-1} and R^{2a-2} are independently H, -NH-C(=O)CH₃ or C₁-C₆ alkyl;
R¹⁰ is amino or 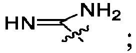
R⁷ is 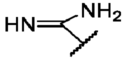 or C₁-C₆ alkyl;
R¹¹ is 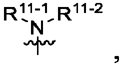 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 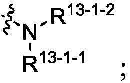
R¹³⁻¹⁻¹ and R¹³⁻¹⁻² are independently H or C₁-C₆ alkyl;
R¹⁴ is C₁-C₆ alkyl substituted by 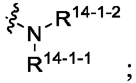
R¹⁴⁻¹⁻¹ and R¹⁴⁻¹⁻² are independently H or C₁-C₆ alkyl;
scheme 17:
the quinazoline compound represented by formula I is a compound represented by formula I-BI, 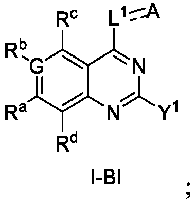
wherein, G is C or N;
R^{a} is 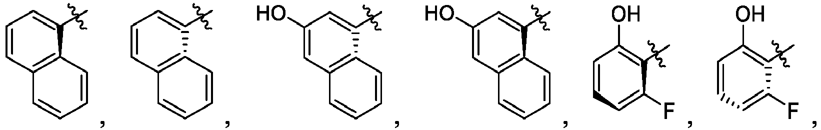 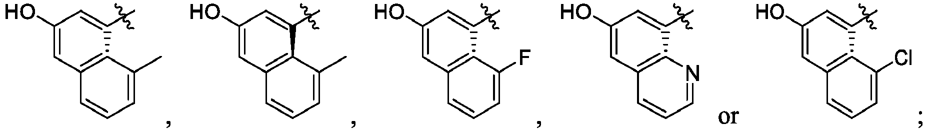
R^{b} is halogen;
R^{c} is H;
R^{d} is halogen;
Y¹ is 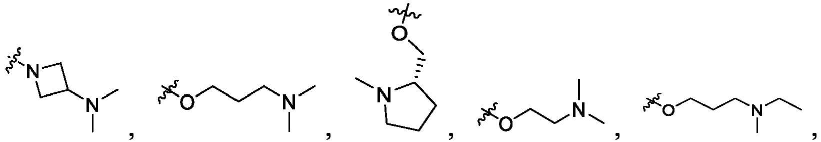 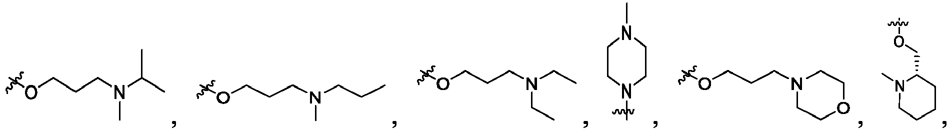 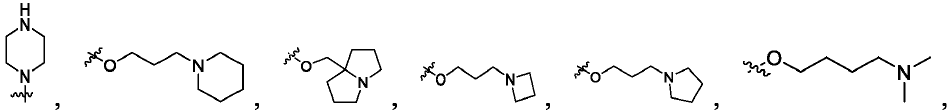 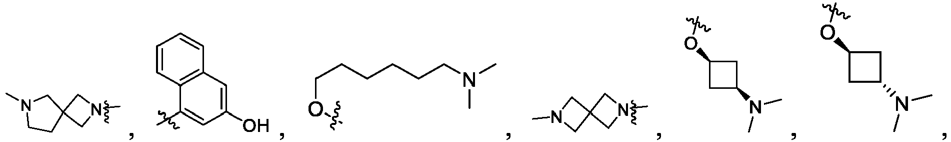 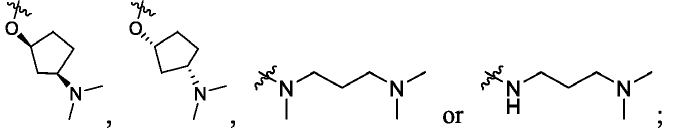
" 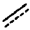 " in 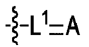 is a single bond; 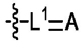
is 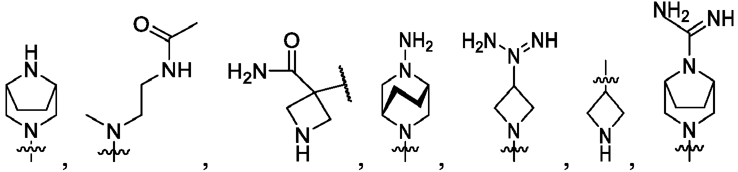 or 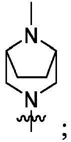
scheme 18:
the quinazoline compound represented by formula I, the pharmaceutically acceptable salt thereof, the solvate thereof, the prodrug thereof, the metabolite thereof or the isotopic compound thereof, 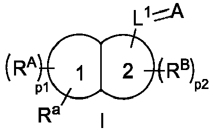
wherein, ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 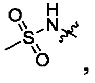 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, C₄-C₈ cycloalkyl-fused C₆-C₁₀ aryl substituted by one or more R^{a-2}, or C₆-C₁₀ aryl substituted by one or more R^{a-4}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 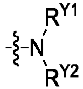 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 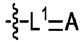
is 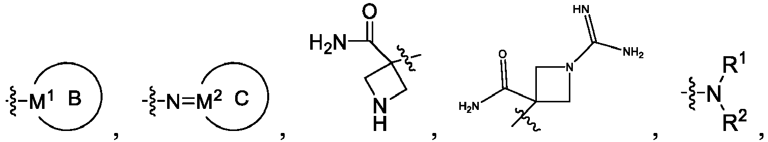 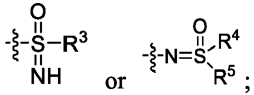
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 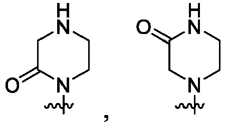 , or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 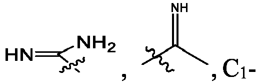 C₆ alkyl, C₁-C₃ alkyl substituted by CN, or C₄-C₆ alkyl substituted by CN; m is 0, 1 or 2; n is 1, 2 or 3;
R¹⁰ is amino or 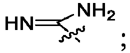
R¹¹ is independently halogen, 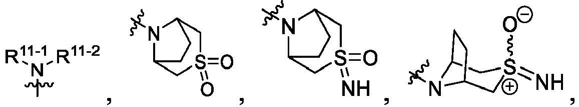 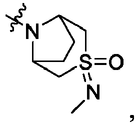 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4-to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 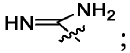
R¹¹⁻³ is H, 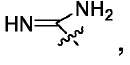 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, C₁-C₆ alkyl substituted by R¹¹⁻³⁻¹, or C₁-C₆ alkoxy substituted by R¹¹⁻³⁻²;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 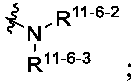
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 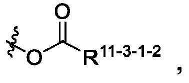 unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R¹¹⁻³⁻¹⁻² is independently 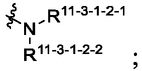
R¹¹⁻³⁻¹⁻²⁻¹ and R¹¹⁻³⁻¹⁻²⁻² are independently H or C₁-C₆ alkyl;
R¹¹⁻³⁻² is C₁-C₁₂ alkenyl;
R^{L} is H or 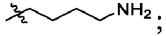
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R^{a-4} is 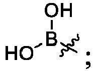
R¹² is C₁-C₆ alkyl;
R¹³ is H, C₁-C₆ alkyl, 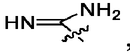 or C₃-C₆ cycloalkyl substituted by R¹³¹;
R¹³⁻¹ is 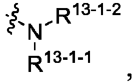 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 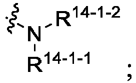
R¹⁴⁻¹⁻¹ and R¹⁴⁻¹⁻² are independently H or C₁-C₆ alkyl;
R¹⁵ is 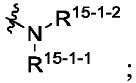
R¹⁵⁻¹⁻¹ and R¹⁵⁻¹⁻² are independently H or C₁-C₆ alkyl;
R¹⁶ is 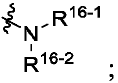
R¹⁶⁻¹ and R¹⁶⁻² are independently H or C₁-C₆ alkyl;
R¹⁷ is 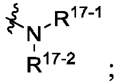
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 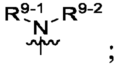
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 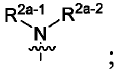
R^{2a-1} and R^{2a-2} are independently H, -C(=O)CH₃, C₁-C₃ alkyl or C₄-C₆ alkyl;
when ring 1 is independently pyridine, R^{B} is 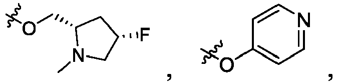 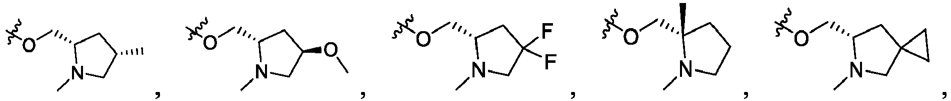 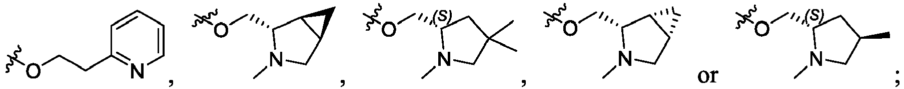
scheme 19:
the quinazoline compound represented by formula I, the pharmaceutically acceptable salt thereof, the solvate thereof, the prodrug thereof, the metabolite thereof or the isotopic compound thereof, 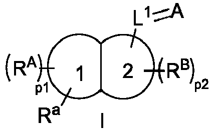
wherein, ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 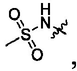 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6-to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 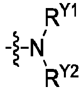 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 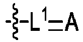
is 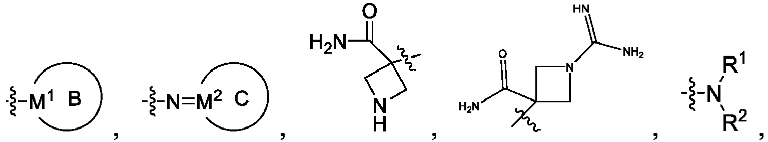 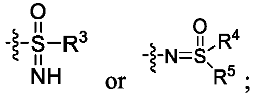
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R ¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 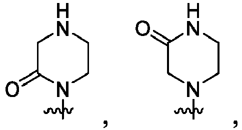 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 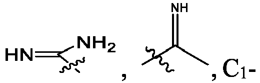 C₆ alkyl, C₁-C₃ alkyl substituted by CN, or C₄-C₆ alkyl substituted by CN; m is 0, 1 or 2; n is 1, 2 or 3;
R¹⁰ is amino or 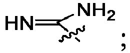
R¹¹ is independently halogen, 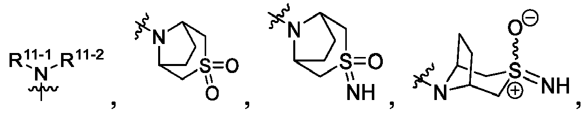 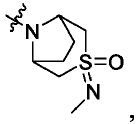 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4-to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 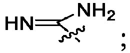
R¹¹⁻³ is H, 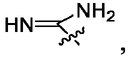 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 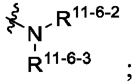
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, 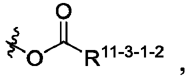 unsubstituted 4- to 10-membered heterocycloalkyl or 4- to 10-membered heterocycloalkyl substituted by R¹¹⁻³¹⁻¹ with 1 to 3 heteroatoms selected from N and O;
R^{ll-3-1-1} is independently H or C₁-C₆ alkyl;
R¹¹⁻³⁻¹⁻² is independently 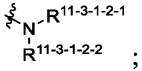
R¹¹⁻³⁻¹⁻²⁻¹ and R¹¹⁻³⁻¹⁻²⁻² are independently H or C₁-C₆ alkyl;
R^{L} is H or 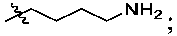
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 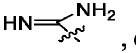 or C₃-C₆ cycloalkyl substituted by R¹³¹;
R¹³⁻¹ is 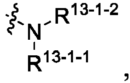 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 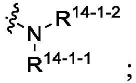
R¹⁴⁻¹⁻¹ and R¹⁴⁻¹⁻² are independently H or C₁-C₆ alkyl;
R¹⁵ is 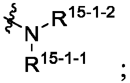
R¹⁵⁻¹⁻¹ and R¹⁵⁻¹⁻² are independently H or C₁-C₆ alkyl;
R¹⁶ is 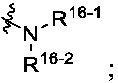
R¹⁶⁻¹ and R¹⁶⁻² are independently H or C₁-C₆ alkyl;
R¹⁷ is 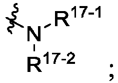
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 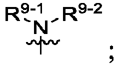
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 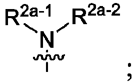
R^{2a-1} and R^{2a-2} are independently H, -C(=O)CH₃, C₁-C₃ alkyl or C₄-C₆ alkyl;
when ring 1 is independently pyridine, R^{B} is 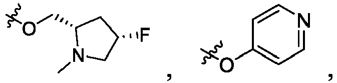 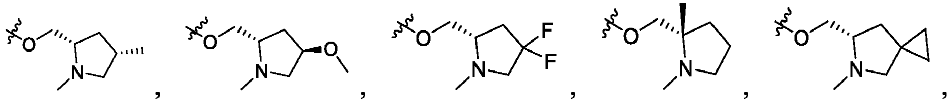 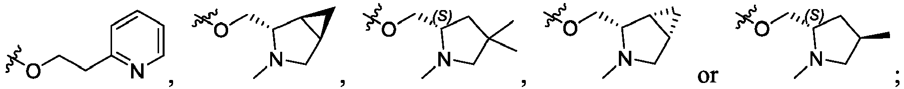
scheme 20:
the quinazoline compound represented by formula I, the pharmaceutically acceptable salt thereof, the solvate thereof, the prodrug thereof, the metabolite thereof or the isotopic compound thereof, 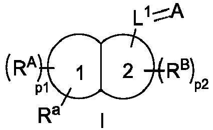
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, C₆-C₁₀ aryl substituted by 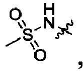 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6-to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 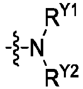 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 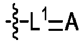
is 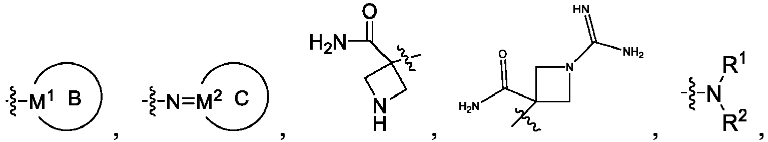 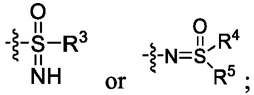
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 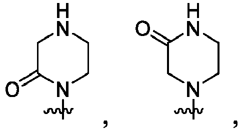 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 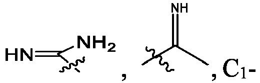 C₆ alkyl, C₁-C₃ alkyl substituted by CN, or C₄-C₆ alkyl substituted by CN; m is 0, 1 or 2; n is 1, 2 or 3;
R¹⁰ is amino or 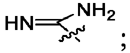
R¹¹ is independently halogen, 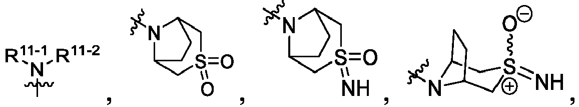 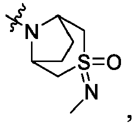 cyano, unsubstituted C₃-C₁₂ cycloalkyl or C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4-to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 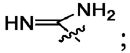
R¹¹⁻³ is H, 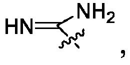 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is unsubstituted 4- to 10-membered heterocycloalkyl or 4- to 10-membered heterocycloalkyl substituted by R¹¹⁻⁶⁻¹⁻¹ with 1 to 3 heteroatoms selected from N and O, or 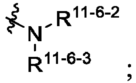
R¹¹⁻⁶⁻² and R¹¹⁻⁶⁻³ are independently H or C₁-C₆ alkyl;
R¹¹⁻⁶⁻¹⁻¹ is independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl, unsubstituted 4- to 10-membered heterocycloalkyl or 4- to 10-membered heterocycloalkyl substituted by R¹¹⁻³⁻¹⁻¹ with 1 to 3 heteroatoms selected from N and O;
R¹¹⁻³⁻¹⁻¹ is independently H or C₁-C₆ alkyl;
R^{L} is H or 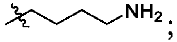
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 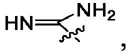 or C₃-C₆ cycloalkyl substituted by R¹³¹;
R¹³⁻¹ is 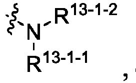 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 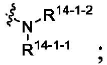
R¹⁴⁻¹⁻¹ and R¹⁴⁻¹⁻² are independently H or C₁-C₆ alkyl;
R¹⁵ is 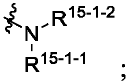
R¹⁵⁻¹⁻¹ and R¹⁵⁻¹⁻² are independently H or C₁-C₆ alkyl;
R¹⁶ is 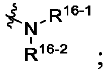
R¹⁶⁻¹ and R¹⁶⁻² are independently H or C₁-C₆ alkyl;
R¹⁷ is 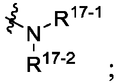
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 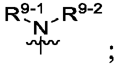
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 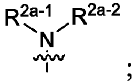
R^{2a-1} and R^{2a-2} are independently H, -C(=O)CH₃, C₁-C₃ alkyl or C₄-C₆ alkyl;
when ring 1 is independently pyridine, R^{B} is 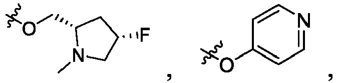 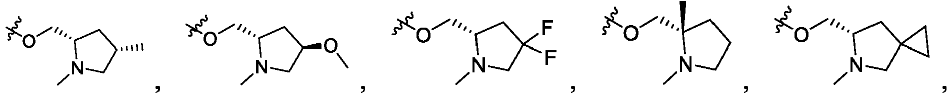 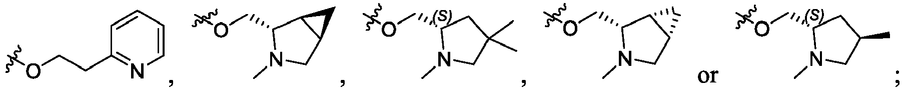
scheme 21:
the quinazoline compound represented by formula I, the pharmaceutically acceptable salt thereof, the solvate thereof, the prodrug thereof, the metabolite thereof or the isotopic compound thereof, 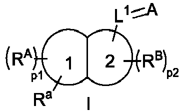
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, C₆-C₁₀ aryl substituted by amino, 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 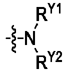 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 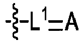
is 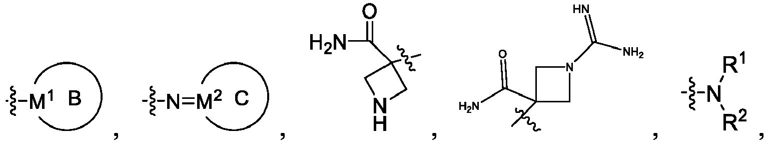 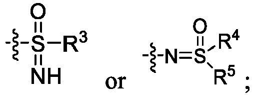
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 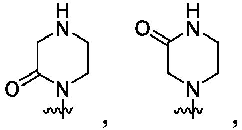 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 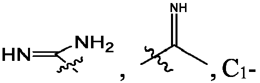 C₆ alkyl, C₁-C₃ alkyl substituted by CN, or C₄-C₆ alkyl substituted by CN; m is 0, 1 or 2; n is 1, 2 or 3;
R¹⁰ is amino or 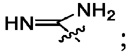
R¹¹ is independently halogen, 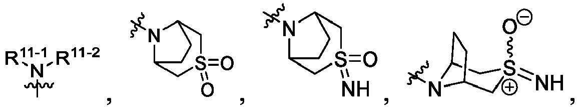 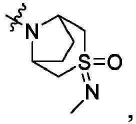 cyano, C₃-C₁₂ cycloalkyl substituted by R¹¹⁻⁶, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 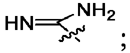
R¹¹⁻³ is H, 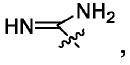 cyano, C₂-C₆ alkynyl, C₃-C₁₂ cycloalkyl, C₁-C₆ alkoxy, halogen, unsubstituted 4- to 10-membered heterocycloalkyl or 4- to 10-membered heterocycloalkyl substituted by C₁-C₆, C₁-C₆ alkyl, or C₁-C₆ alkyl substituted by R¹¹⁻³⁻¹;
R¹¹⁻⁴ is C₁-C₆ alkyl;
R¹¹⁻⁶ is unsubstituted C₁-C₆ alkyl or C₁-C₆ alkyl substituted by R¹¹⁻⁶⁻¹;
R¹¹⁻⁶⁻¹ is 4- to 10-membered heterocycloalkyl with 1 to 3 heteroatoms selected from N and O, or 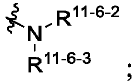
R¹¹⁻⁶⁻² and R¹¹⁻⁶⁻³ are independently H or C₁-C₆ alkyl;
R¹¹⁻¹⁻¹ is -OH, 4- to 10-membered heterocycloalkyl substituted by R^{L} with 1 to 3 heteroatoms selected from N and O;
R¹¹⁻³⁻¹ is C₃-C₁₂ cycloalkyl;
R^{L} is H or 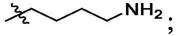
R^{a-1} is independently hydroxyl, halogen, C₁-C₆ alkyl, C₃-C₁₂ cycloalkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 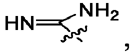 or C₃-C₆ cycloalkyl substituted by R¹³¹;
R¹³⁻¹ is 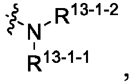 or 4- to 10-membered heterocycloalkyl substituted by R¹³⁻¹⁻³ with 1 to 3 heteroatoms selected from N and O;
R¹³⁻¹⁻¹ and R¹³⁻¹⁻² are independently H or C₁-C₆ alkyl;
R¹³⁻¹⁻³ is independently H or C₁-C₆ alkyl;
R¹⁴ is unsubstituted C₁-C₆ alkyl or C₁-C₆ alkyl substituted by 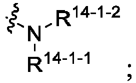
R¹⁴⁻¹⁻¹ and R¹⁴⁻¹⁻² are independently H or C₁-C₆ alkyl;
R¹⁵ is 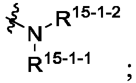
R¹⁵⁻¹⁻¹ and R¹⁵⁻¹⁻² are independently H or C₁-C₆ alkyl;
R¹⁶ is 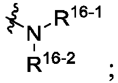
R¹⁶⁻¹ and R¹⁶⁻² are independently H or C₁-C₆ alkyl;
R¹⁷ is 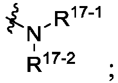
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 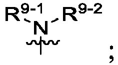
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 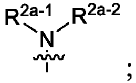
R^{2a-1} and R^{2a-2} are independently H, -C(=O)CH₃, C₁-C₃ alkyl or C₄-C₆ alkyl;
when ring 1 is independently pyridine, R^{B} is 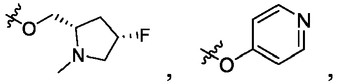 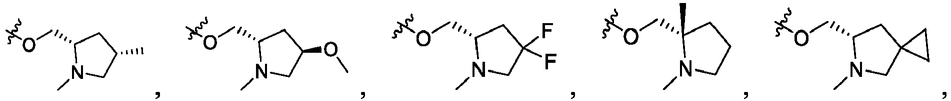 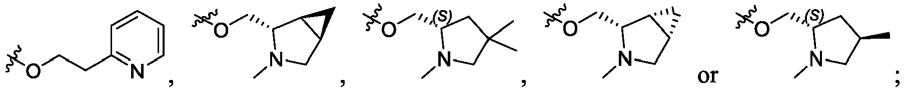
scheme 22:
the quinazoline compound represented by formula I, the pharmaceutically acceptable salt thereof, the solvate thereof, the prodrug thereof, the metabolite thereof or the isotopic compound thereof, 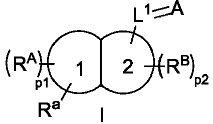
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, 6- to 10-membered heteroaryl substituted by one or more hydroxyl, 6- to 10-membered heteroaryl substituted by one or more R^{a-3}, C₆-C₁₀ aryl, C₆-C₁₀ aryl substituted by one or more R^{a-1} with at least one substituent being halogen or C₁-C₆ alkyl, or C₄-C₈ cycloalkyl-fused C₆-C₁₀ aryl substituted by one or more R^{a-2}; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 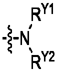 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 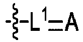
is 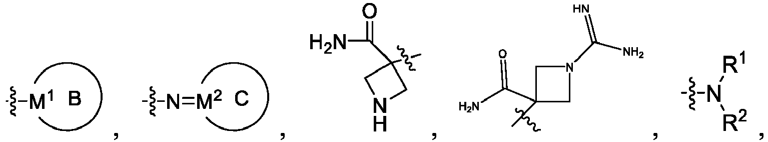 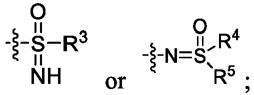
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 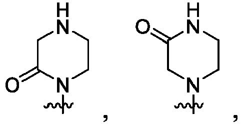 or unsubstituted 4- to 10-membered heterocycloalkenyl with 1 to 3 heteroatoms or heteroatom groups selected from N and P(=O); one N atom of the 7- to 12-membered heterocycloalkyl is attached to ring 2;
R⁷ is -(CH₂)ₘ-CN, -C(=O)(CH₂)ₙNH₂, -C(=O)O-(C₁-C₄ alkyl), 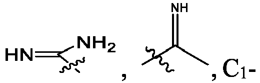 C₆ alkyl, C₁-C₃ alkyl substituted by CN, or C₄-C₆ alkyl substituted by CN; m is 0, 1 or 2; n is 1, 2 or 3;
R¹⁰ is amino or 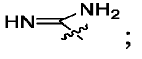
R¹¹ is independently halogen, 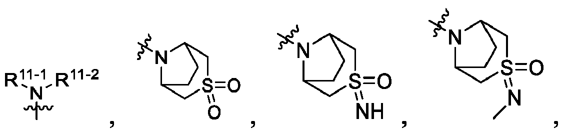 cyano, unsubstituted C₁-C₆ alkoxy or C₁-C₆ alkoxy substituted by one or more R¹¹⁻⁵, unsubstituted 4- to 10-membered heterocycloalkyl or 4- to 10-membered heterocycloalkyl substituted by one or more R¹¹⁻³ with 1 to 3 heteroatoms selected from N and O, unsubstituted 5- to 6-membered heteroaryl or 5- to 6-membered heteroaryl substituted by one or more R¹¹⁻⁴ with 1 to 3 heteroatoms selected from N and O, or 4- to 10-membered heterocycloalkyl with 1 to 3 heteroatoms selected from N and S;
R¹¹⁻⁵ is independently 4- to 10-membered heterocycloalkyl; the heteroatoms in the 4- to 10-membered heterocycloalkyl are selected from one or more of N, O and S, and the number of heteroatoms is 1 to 3;
R¹¹⁻¹ and R¹¹⁻² are independently H, unsubstituted C₁-C₆ alkyl or C₁-C₆ alkyl substituted by one or more R¹¹⁻¹⁻¹, or 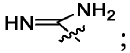
R¹¹⁻³ is H, 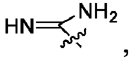 cyano, C₂-C₆ alkynyl, C₁-C₆ alkoxy, halogen, unsubstituted 4- to 10-membered heterocycloalkyl or 4- to 10-membered heterocycloalkyl substituted by C₁-C₆, or C₁-C₆ alkyl;
R¹¹⁻⁴ is C₁-C₆ alkyl;
R¹¹⁻¹⁻¹ is -OH, 4- to 10-membered heterocycloalkyl substituted by R^{L} with 1 to 3 heteroatoms selected from N and O;
R^{L} is H or 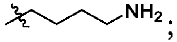
R^{a-1} is independently hydroxyl, halogen, C₁-C₆ alkyl, unsubstituted C₂-C₆ alkynyl or C₂-C₆ alkynyl substituted by one or more R^{a-1-1}, C₃-C₁₂ cycloalkyl, or C₁-C₆ alkyl substituted by one or more R^{a-1-2};
R^{a-2} is independently hydroxyl or C₁-C₆ alkyl;
R^{a-1-1} is independently halogen;
R^{a-1-2} is independently halogen;
R^{a-3} is C₁-C₆ alkyl;
R¹² is C₁-C₆ alkyl;
R¹³ is H, C₁-C₆ alkyl, 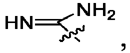 or C₃-C₆ cycloalkyl substituted by R¹³¹;
R¹³⁻¹ is 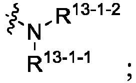
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 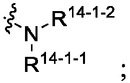
R¹⁴⁻¹⁻¹ and R¹⁴⁻¹⁻² are independently H or C₁-C₆ alkyl;
R¹⁵ is 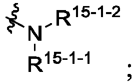
R¹⁵⁻¹⁻¹ and R¹⁵⁻¹⁻² are independently H or C₁-C₆ alkyl;
R¹⁶ is 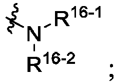
R¹⁶⁻¹ and R¹⁶⁻² are independently H or C₁-C₆ alkyl;
R¹⁷ is 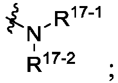
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 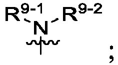
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 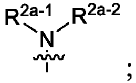
R^{2a-1} and R^{2a-2} are independently H, -C(=O)CH₃, C₁-C₃ alkyl or C₄-C₆ alkyl;
when ring 1 is independently pyridine, R^{B} is 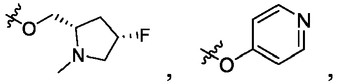 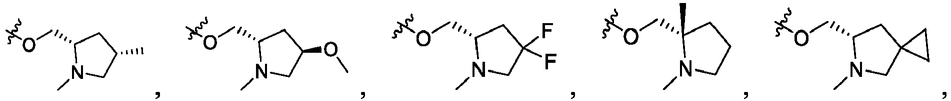 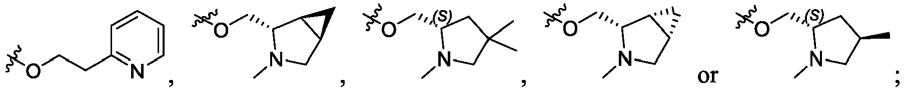
scheme 23:
the quinazoline compound represented by formula I is, 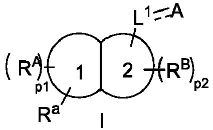
wherein ring 1 and ring 2 are independently C₅-C₆ aryl, 5- to 6-membered heteroaryl with 1 to 3 heteroatoms selected from one or more of N, O and S, C₄-C₈ cycloalkyl, or 4- to 8-membered heterocycloalkyl with 1 to 4 heteroatoms selected from one or more of N, O, S, B and P; (ring 1 and ring 2 are connected by fusion)
p1 and p2 are independently 1, 2, 3, 4 or 5;
R^{A} is H, halogen, hydroxyl, cyano, amino, C₁-C₃ alkyl, C₁-C₃ alkoxy, C₄-C₆ alkyl, or C₄-C₆ alkoxy;
R^{a} is C₆-C₁₀ aryl substituted by one or more hydroxyl, 6- to 10-membered heteroaryl substituted by one or more hydroxyl, C₆-C₁₀ aryl, or C₆-C₁₀ aryl substituted by one or more R^{a-1} with at least one substituent being halogen or C₁-C₆ alkyl; the heteroatom in the 6- to 10-membered heteroaryl is selected from one or more of N, O and S, and the number of heteroatoms is 1 to 3;
R^{B} is H, halogen, hydroxyl, cyano, amino, C₁-C₃ alkyl, C₁-C₃ alkoxy, C₄-C₆ alkyl, C₄-C₆ alkoxy or Y¹;
Y¹ is 3- to 10-membered heterocycloalkyl substituted by one or more 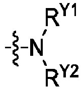 with 1 to 4 heteroatoms selected from one or more of N, O and S, C₁-C₆ alkoxy substituted by one or more R¹¹, unsubstituted 4- to 10-membered heterocycloalkyl or 4- to 10-membered heterocycloalkyl substituted by R¹² with 1 to 3 heteroatoms selected from N and O, amino substituted by one or more R¹⁴, -OR¹³, C₆-C₁₀ aryl substituted by one hydroxyl, C₁-C₆ alkyl substituted by R¹⁵, C₁-C₆ alkylthio substituted by R¹⁶, or C₃-C₁₀ cycloalkyl substituted by R¹⁷;
R^{Y1} and R^{Y2} are independently H, C₁-C₃ alkyl or C₄-C₆ alkyl; 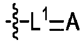
is 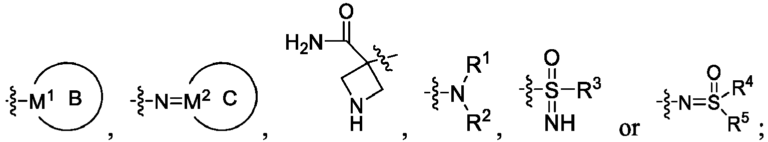
M¹ is N, CH or P(=O);
ring B is unsubstituted 4- to 10-membered heterocycloalkyl with 1 to 3 heteroatoms or heteroatom groups selected from N and P(=O) and containing only one N, 4- to 10-membered heterocycloalkyl substituted by R¹⁰ with 1 to 3 heteroatoms or heteroatom groups selected from N and P(=O) and containing only one N, unsubstituted 5- to 6-membered heterocycloalkyl or 5- to 6-membered heterocycloalkyl substituted by R⁷ with 2 to 3 heteroatoms selected from one or more of N, O and S, unsubstituted 7- to 12-membered heterocycloalkyl or 7- to 12-membered heterocycloalkyl substituted by R⁷ with 2 to 3 heteroatoms selected from one or more of N, O and S, 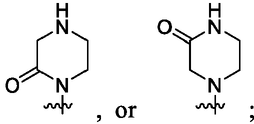 one N atom of the 7- to 12-membered heterocycloalkyl is attached to ring 2;
R⁷ is -C(=O)O-(C₁-C₄ alkyl), 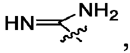 C₁-C₆ alkyl, C₁-C₃ alkyl substituted by CN, or C₄-C₆ alkyl substituted by CN;
R¹⁰ is amino or 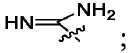
R¹¹ is independently halogen, 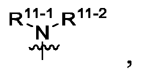 unsubstituted 4- to 10-membered heterocycloalkyl or 4- to 10-membered heterocycloalkyl substituted by one or more R¹¹⁻³ with 1 to 3 heteroatoms selected from N and O, or unsubstituted 5- to 6-membered heteroaryl or 5-to 6-membered heteroaryl substituted by one or more R¹¹⁻⁴ with 1 to 3 heteroatoms selected from N and O;
R¹¹⁻¹ and R¹¹⁻² are independently H, unsubstituted C₁-C₆ alkyl or C₁-C₆ alkyl substituted by R¹¹⁻¹⁻¹, or 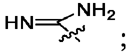
R¹¹⁻³ is H, 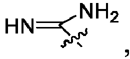 halogen, unsubstituted 4- to 10-membered heterocycloalkyl or 4-to 10-membered heterocycloalkyl substituted by C₁-C₆, or C₁-C₆ alkyl;
R¹¹⁻⁴ is C₁-C₆ alkyl;
R¹¹⁻¹⁻¹ is 4- to 10-membered heterocycloalkyl substituted by R^{L} with 1 to 3 heteroatoms selected from N and O;
R^{L} is H or 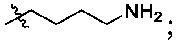
R^{a-1} is independently hydroxyl, halogen, or C₁-C₆ alkyl;
R¹² is C₁-C₆ alkyl;
R¹³ is H, C₁-C₆ alkyl, 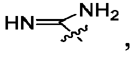 or C₃-C₆ cycloalkyl substituted by R¹³¹;
R¹³⁻¹ is 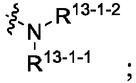
R¹³⁻¹⁻¹ and R¹³⁻¹⁻² are independently H or C₁-C₆ alkyl;
R¹⁴ is unsubstituted C₁-C₆ alkyl or C₁-C₆ alkyl substituted by 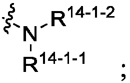
R¹⁴⁻¹⁻¹ and R¹⁴⁻¹⁻² are independently H or C₁-C₆ alkyl;
R¹⁵ is 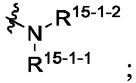
R¹⁵⁻¹⁻¹ and R¹⁵⁻¹⁻² are independently H or C₁-C₆ alkyl;
R¹⁶ is 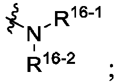
R¹⁶⁻¹ and R¹⁶⁻² are independently H or C₁-C₆ alkyl;
R¹⁷ is 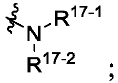
R¹⁷⁻¹ and R¹⁷⁻² are independently H or C₁-C₆ alkyl;
ring C is unsubstituted 4- to 10-membered heterocycloalkyl or 4- to 10-membered heterocycloalkyl substituted by R⁹ with 1 to 3 heteroatoms or heteroatom groups selected from N and S(=O); and M² is S(=O);
R⁹ is 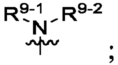
R⁹⁻¹ and R⁹⁻² are independently H, C₁-C₃ alkyl, C₄-C₆ alkyl, C₁-C₃ alkyl substituted by CN, or C₄-C₆ alkyl substituted by CN;
R¹ and R⁴ are independently H, C₁-C₃ alkyl or C₄-C₆ alkyl;
R², R³ and R⁵ are independently -(CH₂)ₙ-R^{2a} or 6- to 10-membered heterocycloalkyl;
n is 1, 2, 3 or 4;
R^{2a} is H, -OH, -O-(C₁-C₃ alkyl), -O-(C₄-C₆ alkyl), or 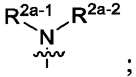
R^{2a-1} and R^{2a-2} are independently H, -C(=O)CH₃, C₁-C₃ alkyl or C₄-C₆ alkyl;
scheme 24:
the quinazoline compound represented by formula I is a compound represented by formula I-CI, 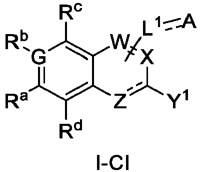
wherein R^{b}, R^{c} and R^{d} are independently H or halogen;
W is C;
G is C or N;
X and Z are N;
"" 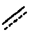 in 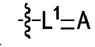 is a double bond;
" 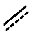 " in 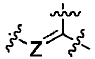 is a double bond;
Y¹ is C₁-C₆ alkoxy substituted by one or more R¹¹;
R¹¹ is independently 4- to 10-membered heterocycloalkyl substituted by one or more R¹¹⁻³ with 1 to 3 heteroatoms selected from N and O;
R¹¹⁻³ is C₁-C₆ alkyl substituted by R¹¹⁻³⁻¹;
R¹¹⁻³⁻¹ is 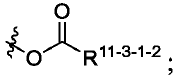
R¹¹⁻³⁻¹⁻² is independently 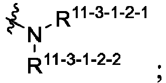
R¹¹⁻³⁻¹⁻²⁻¹ and R¹¹⁻³⁻¹⁻²⁻² are independently H or C₁-C₆ alkyl;
scheme 25:
the quinazoline compound represented by formula I is a compound represented by formula I-BI, 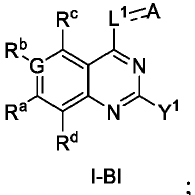
wherein, G is C or N;
R^{a} is 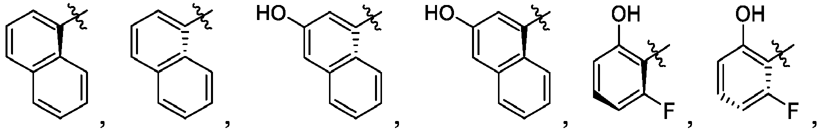 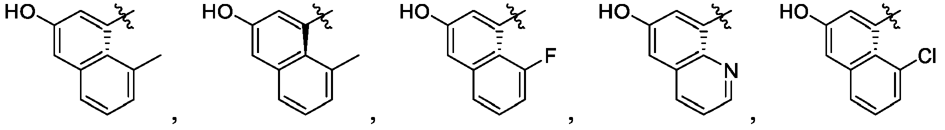 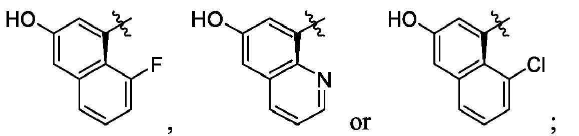
R^{b} is halogen;
R^{c} is H;
R^{d} is halogen;
Y¹ is 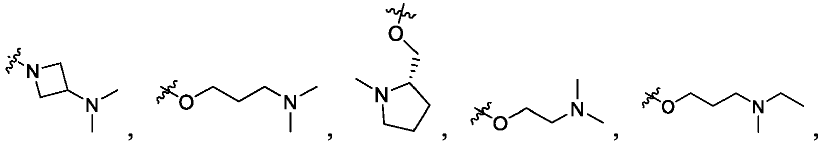 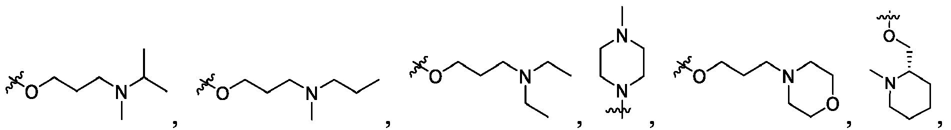 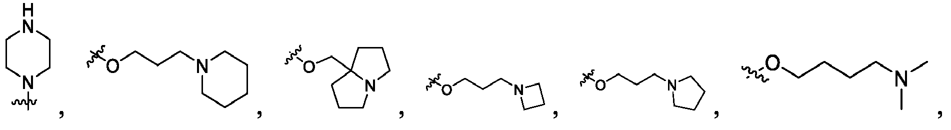 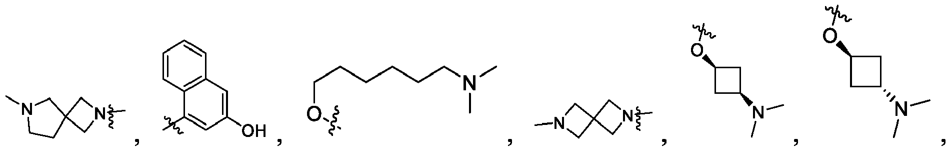 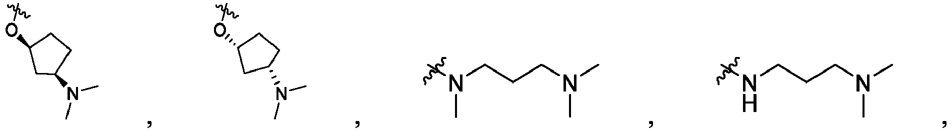 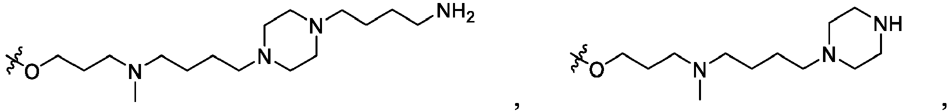 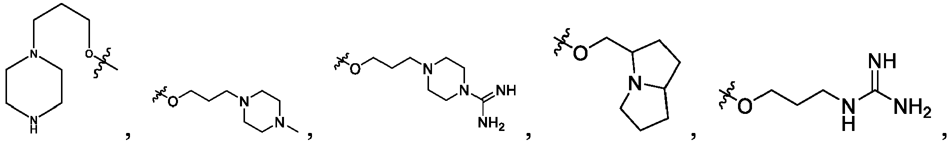 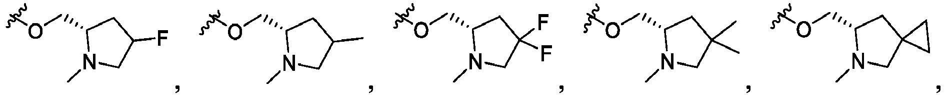 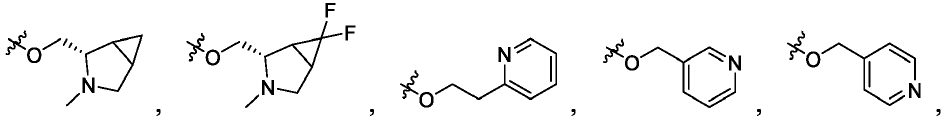 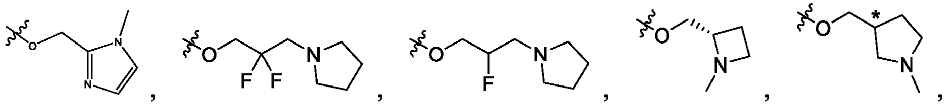 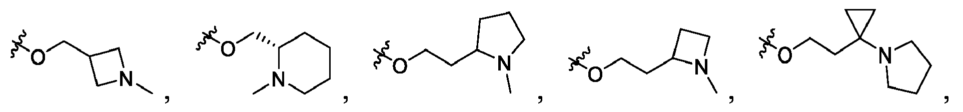 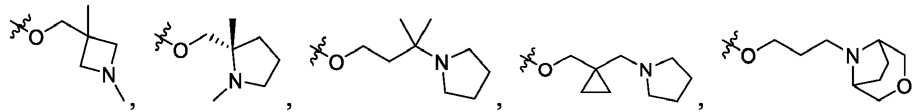 or 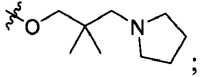
" 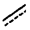 " in 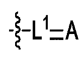 is a single bond; 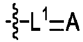
is 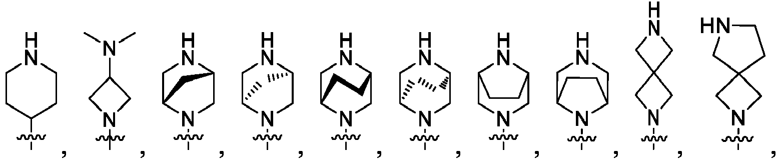 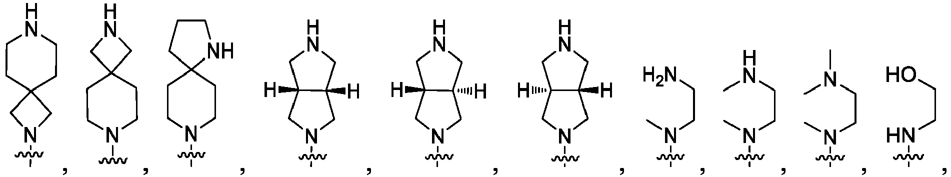 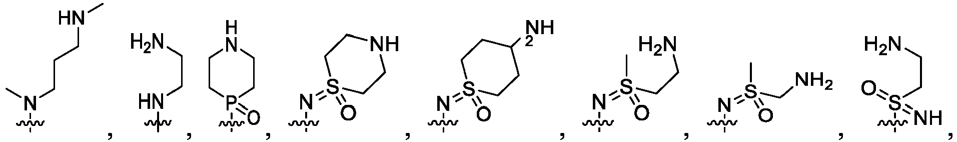 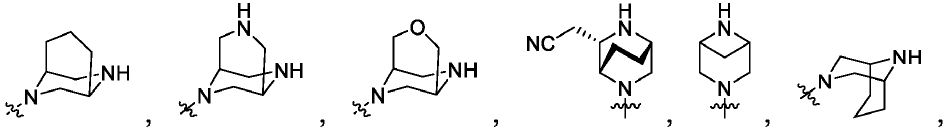 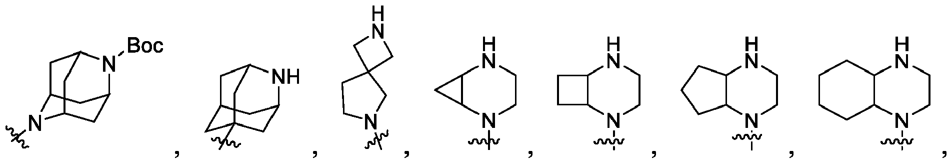 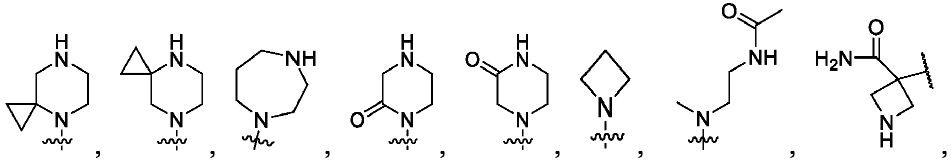 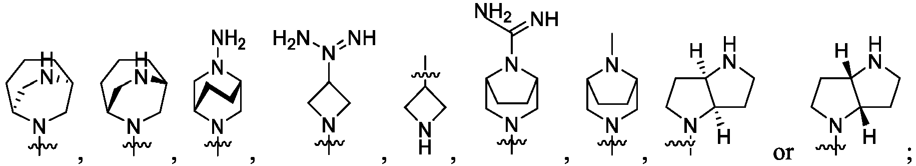
scheme 26:
the quinazoline compound represented by formula I is a compound represented by formula I-BI, 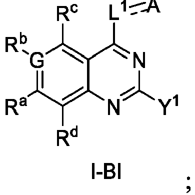
wherein, G is C or N;
R^{a} is 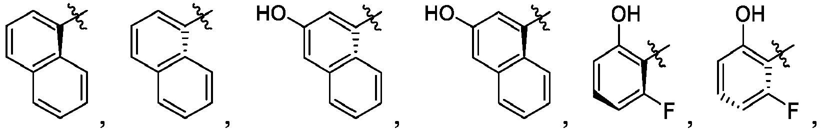 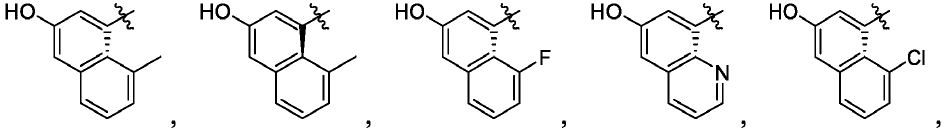 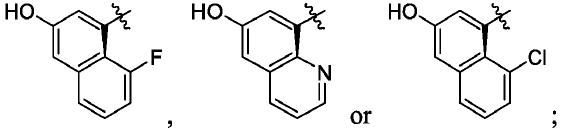
R^{b} is halogen;
R^{c} is H;
R^{d} is halogen;
Y¹ is 3- to 10-membered heterocycloalkyl with 1 heteroatom being N, C₁-C₆ alkoxy substituted by R¹¹, unsubstituted 4- to 10-membered heterocycloalkyl or 4- to 10-membered heterocycloalkyl substituted by R¹² with 2 heteroatoms or heteroatom groups selected from N and O, amino substituted by one or more R¹⁴, -OR¹³, or C₆-C₁₀ aryl substituted by one hydroxyl;
" 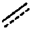 " in 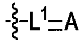 is a single bond; 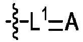
is 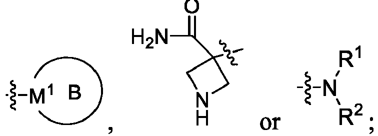
M¹ is N or CH;
ring B is unsubstituted 4- to 10-membered heterocycloalkyl with 1 heteroatom or heteroatom group being N, 4- to 10-membered monocyclic heterocycloalkyl substituted by R¹⁰ with 1 to 3 heteroatoms or heteroatom groups selected from N and P(=O) and containing only one N, 4- to 10-membered bridged heterocycloalkyl substituted by R¹⁰ with 1 to 3 heteroatoms or heteroatom groups selected from N and P(=O) and containing only one N, or 7- to 12-membered bridged heterocycloalkyl substituted by R⁷ with 2 to 3 heteroatoms selected from one or more of N, O and S;
wherein, the 4- to 10-membered heterocycloalkyl with 1 heteroatom or heteroatom group being N can be 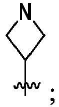
the 4- to 10-membered monocyclic heterocycloalkyl with 1 to 3 heteroatoms or heteroatom groups selected from N and P(=O) and containing only one N can be 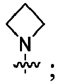
the 4- to 10-membered bridged heterocycloalkyl substituted by R¹⁰ with 1 to 3 heteroatoms or heteroatom groups selected from N and P(=O) and containing only one N can be 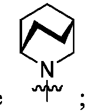
the 7- to 12-membered bridged heterocycloalkyl substituted by R⁷ with 2 to 3 heteroatoms selected from one or more of N, O and S can be 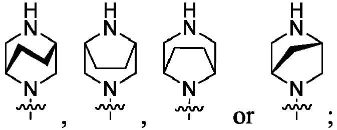
R¹ is H, C₁-C₃ alkyl, or C₄-C₆ alkyl;
R² is -(CH₂)ₙ-R^{2a};
n is 1 or 2;
R^{2a} is 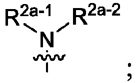
R^{2a-1} and R^{2a-2} are independently H, -NH-C(=O)CH₃ or C₁-C₆ alkyl;
R¹⁰ is amino or 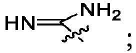
R⁷ is 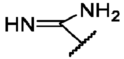 or C₁-C₆ alkyl;
R¹¹ is 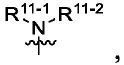 unsubstituted 4- to 10-membered heterocycloalkyl or 4- to 10-membered heterocycloalkyl substituted by R¹¹⁻³ with 1 to 3 heteroatoms or heteroatom groups selected from N and O;
R¹¹⁻¹ and R¹¹⁻² are independently H or C₁-C₆ alkyl;
R¹¹⁻³ is C₁-C₆ alkyl;
R¹² is C₁-C₆ alkyl;
R¹³ is C₃-C₆ cycloalkyl substituted by R¹³⁻¹;
R¹³⁻¹ is 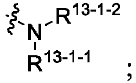
R¹³⁻¹⁻¹ and R¹³⁻¹⁻² are independently H or C₁-C₆ alkyl;
R¹⁴ is C₁-C₆ alkyl substituted by 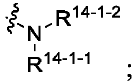
R¹⁴⁻¹⁻¹ and R¹⁴⁻¹⁻² are independently H or C₁-C₆ alkyl.

4. The quinazoline compound represented by formula I-AI, the pharmaceutically acceptable salt thereof, the solvate thereof, the prodrug thereof, the metabolite thereof or the isotopic compound thereof according to claim 2, wherein, when R^{a} is C₆-C₁₀ aryl substituted by one or more hydroxyl, the R^{a} is 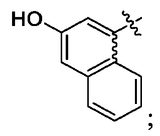 " 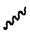 " indicates " 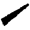 ", " 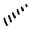 " or a mixture thereof;
and/or, when R^{a} is 6- to 10-membered heteroaryl substituted by one or more hydroxyl, the 6- to 10-membered heteroaryl is 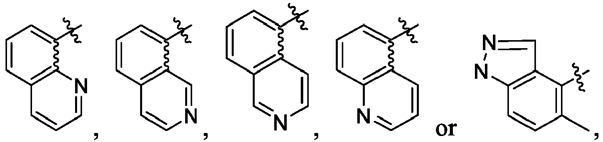 for example, R^{a} is 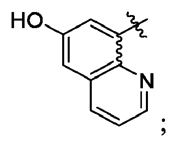 " 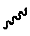 " indicates " 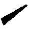 ", " 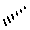 " or a mixture thereof;
and/or, when R^{a} is C₆-C₁₀ heteroaryl, the C₆-C₁₀ heteroaryl is 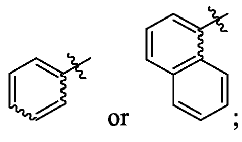 " 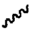 "
indicates " 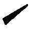 ", " 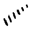 " or a mixture thereof;
and/or, when R^{a} is C₆-C₁₀ aryl substituted by one or more R^{a-1} with at least one substituent being halogen or C₁-C₆ alkyl, the C₆-C₁₀ aryl is 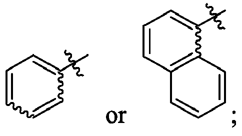 " 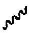 " indicates " 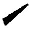 ", " 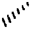 " or a mixture thereof;
and/or, in R^{a-1}, the C₁-C₆ alkyl is methyl, ethyl, n-propyl or isopropyl, preferably methyl or ethyl;
and/or, in R^{a-1}, the halogen is F, Cl, Br or I, preferably F or Cl;
and/or, in R^{a-1}, the C₂-C₆ alkynyl can be ethynyl, propynyl, 1-butynyl or 2-butynyl, preferably ethynyl or butynyl;
and/or, in R^{a-1}, the C₃-C₁₂ cycloalkyl can be C₃-C₁₂ monocyclic cycloalkyl, C₃-C₁₂ bridged cycloalkyl, or C₃-C₁₂ spiro cycloalkyl; preferably C₃-C₁₂ monocyclic cycloalkyl, or C₃-C₁₂ bridged cycloalkyl; preferably cyclopropyl or bicyclo [1.1.1]pentyl;
the C₁-C₆ alkyl in the C₁-C₆ alkyl substituted by R^{a-1-2} can be methyl, ethyl, n-propyl or isopropyl, preferably methyl;
and/or, in R^{a-1-1}, the halogen is F, Cl, Br, or I, preferably F;
and/or, in R^{a-1-2}, the halogen is F, Cl, Br, or I, preferably F;
and/or, R^{a-1} is hydroxyl, methyl, fluorine, chlorine, ethyl, ethynyl, butynyl, cyclopropyl, bicyclo[1.1.1]pentyl, -CF₃, or fluoroethynyl;
and/or, in R^{b}, R^{c} and R^{d}, the halogen is fluorine, chlorine, bromine or iodine;
and/or, in R^{b}, R^{c} and R^{d}, the C₁-C₃ alkyl is methyl, ethyl, propyl or isopropyl;
and/or, in R^{b}, R^{c} and R^{d}, the C₁-C₃ alkyl is methoxy, ethoxy, propoxy or isopropoxy;
and/or, in R^{A}, the halogen is F or Cl;
and/or, in R^{A}, the C₁-C₃ alkyl is methyl;
and/or, R¹¹⁻³⁻¹⁻²⁻¹ and R¹¹⁻³⁻¹⁻²⁻² are independently H, methyl, ethyl, propyl, *n*-butyl or *tert-*butyl;
and/or, in the C₁-C₆ alkoxy substituted by R¹¹⁻³⁻², the C₁-C₆ alkoxy is methoxy, ethoxy or propoxy, preferably propoxy;
and/or, the C₁-C₆ alkyl in R¹¹⁻³⁻¹⁻¹ is methyl, ethyl, n-propyl, isopropyl, n-butyl, isobutyl or tert-butyl;
and/or, the C₃-C₆ alkyl in R¹¹⁻³⁻¹ can be cyclopropyl, cyclobutyl, cyclopentyl or cyclohexyl, for example, cyclopropyl;
and/or, in R¹¹⁻³⁻², the C₂-C₆ alkenyl is vinyl, propylenyl, n-butylenyl or isobutylenyl, preferably vinyl;
and/or, in R^{Y1} and R^{Y2}, the C₁-C₃ alkyl is methyl, ethyl, n-propyl or isopropyl;
and/or, the 3- to 10-membered heterocycloalkyl in the 3- to 10-membered heterocycloalkyl substituted by one or more 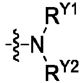 with 1 to 4 heteroatoms selected from one or more of N, O and S is 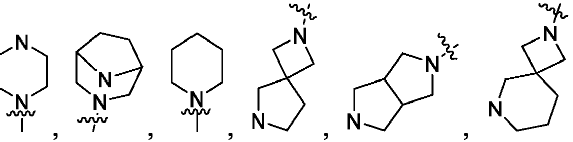 or 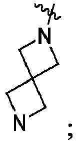
and/or, when Y¹ is C₆-C₁₀ aryl substituted by one hydroxyl, the C₆-C₁₀ aryl is 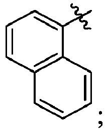
and/or, when Y¹ is C₁-C₆ alkoxy substituted by R¹¹, the C₁-C₆ alkoxy can be methoxy, ethoxy, n-propoxy, isopropoxy, n-butoxy, isobutoxy, sec-butoxy, n-pentyloxy, isopentyloxy, neopentyloxy, n-hexyloxy, isohexyloxy, neohexyloxy, 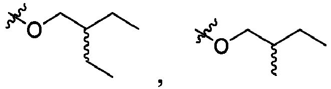 (preferably 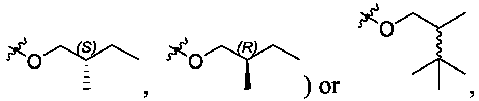 for example, methoxy, n-propoxy, ethoxy, n-butoxy, isobutoxy, isopentyloxy, neopentyloxy, n-hexyloxy, 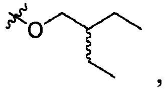 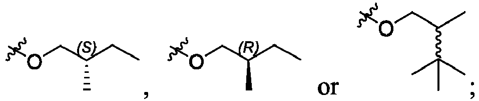
and/or, in the 4- to 10-membered heterocycloalkyl substituted by R^{L} with 1 to 3 heteroatoms selected from N and O, the 4- to 10-membered heterocycloalkyl with 1 to 3 heteroatoms selected from N and O is 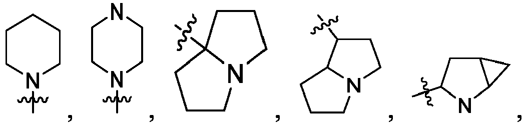 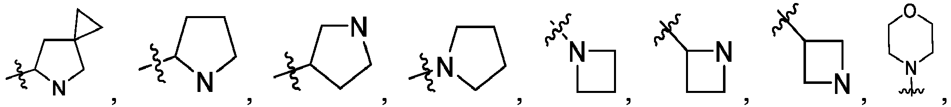 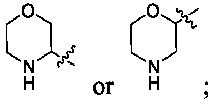
and/or, in R¹³, the C₃-C₆ cycloalkyl can be 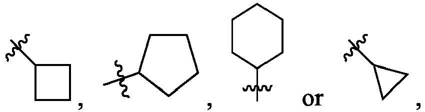 preferably 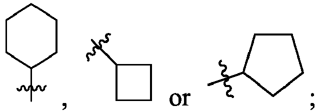
and/or, in R¹⁴, the C₁-C₆ alkyl is methyl, ethyl, n-propyl or isopropyl, preferably n-propyl;
and/or, in the C₁-C₆ alkyl substituted by R¹⁵, the C₁-C₆ alkyl is methyl, ethyl, n-propyl or isopropyl;
and/or, in the C₁-C₆ alkylthio substituted by R¹⁶, the C₁-C₆ alkylthio is methylthio, ethylthio, n-propylthio or isopropylthio;
and/or, in the C₃-C₁₀ cycloalkyl substituted by R¹⁷, the C₃-C₁₀ cycloalkyl is cyclopropyl, cyclobutyl, cyclopentyl or cyclohexyl;
and/or, in R¹¹⁻³, the C₁-C₆ alkyl is methyl, ethyl, n-propyl or isopropyl, preferably methyl;
and/or, in R¹¹⁻³, the C₃-C₁₂ cycloalkyl can be cyclopropyl, cyclobutyl, cyclopentyl or cyclohexyl, for example, cyclopropyl;
and/or, in R¹¹⁻⁴, the C₁-C₆ alkyl is methyl, ethyl, n-propyl or isopropyl;
and/or, in R¹², the C₁-C₆ alkyl is methyl, ethyl, n-propyl or isopropyl, preferably methyl;
and/or, the unsubstituted 4- to 10-membered heterocycloalkyl or 4- to 10-membered heterocycloalkyl substituted by R¹² with 1 to 3 heteroatoms selected from N and O is unsubstituted 6- to 9-membered monocyclic, fused, bridged or spiro heterocycloalkyl with 1 or 2 heteroatoms being N, or 6- to 9-membered monocyclic, fused, bridged or spiro heterocycloalkyl substituted by R¹² with 1 or 2 heteroatoms being N; the 4- to 10-membered heterocycloalkyl is preferably 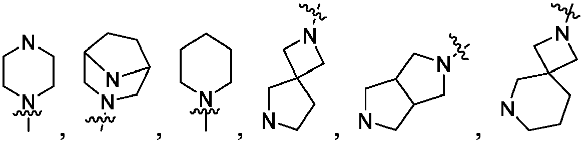 or 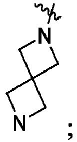
and/or, in R¹³⁻¹⁻¹ and R¹³⁻¹⁻², the C₁-C₆ alkyl is methyl, ethyl, n-propyl or isopropyl, for example, methyl;
and/or, in R¹⁴⁻¹⁻¹ and R¹⁴⁻¹⁻², the C₁-C₆ alkyl is methyl, ethyl, n-propyl or isopropyl, preferably methyl;
and/or, in R¹⁵⁻¹⁻¹ and R¹⁵⁻¹⁻², the C₁-C₆ alkyl is methyl, ethyl, n-propyl or isopropyl;
and/or, in R¹⁶⁻¹⁻¹ and R¹⁶⁻¹⁻², the C₁-C₆ alkyl is methyl, ethyl, n-propyl or isopropyl;
and/or, in R¹⁷⁻¹⁻¹ and R¹⁷⁻¹⁻², the C₁-C₆ alkyl is methyl, ethyl, n-propyl or isopropyl;
and/or, in R⁷, the C₁-C₆ alkyl is methyl, ethyl, n-propyl or isopropyl;
and/or, in R¹¹⁻¹ and R¹¹⁻², and in unsubstituted C₁-C₆ alkyl or C₁-C₆ alkyl substituted by R¹¹⁻¹⁻¹, the C₁-C₆ alkyl is methyl, ethyl, n-propyl, isopropyl, n-butyl, isobutyl or tert-butyl, preferably methyl or *n*-butyl;
and/or, in R¹¹, the unsubstituted 4- to 10-membered heterocycloalkyl or 4- to 10-membered heterocycloalkyl substituted by R¹¹⁻³ with 1 to 3 heteroatoms or heteroatom groups selected from N and O is preferably 4- to 8-membered monocyclic heterocycloalkyl with 1 to 2 heteroatoms or heteroatom groups selected from N and O (for example, 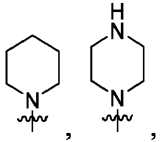 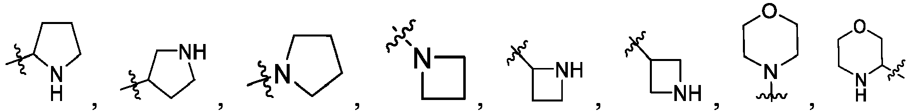 or 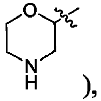 4- to 8-membered bridged heterocycloalkyl with 1 to 2 heteroatoms or heteroatom groups selected from N and O (for example, 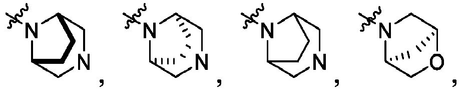 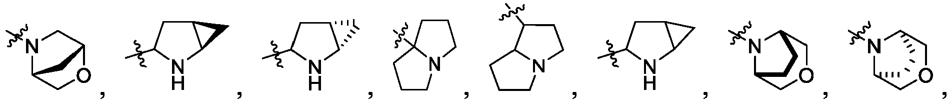 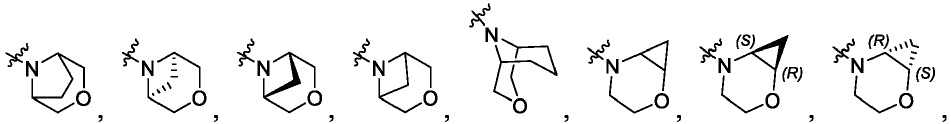 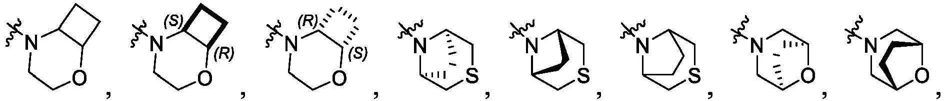 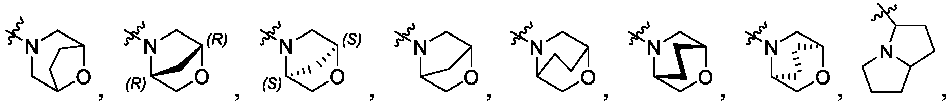 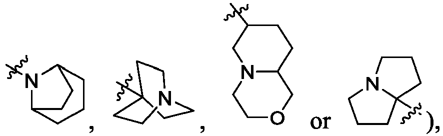 or 4- to 8-membered spiro heterocycloalkyl with 1 to 2 heteroatoms or heteroatom groups selected from N and O (for example, 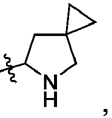 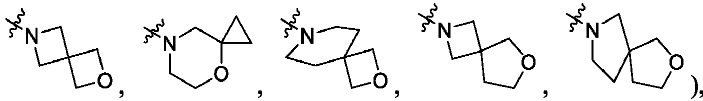 or 9- to 10-membered heterocycloalkyl with 1 to 2 heteroatoms or heteroatom groups selected from N and O (for example, 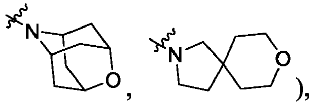 more preferably

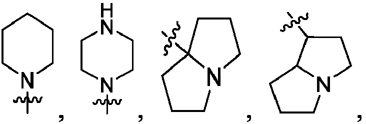

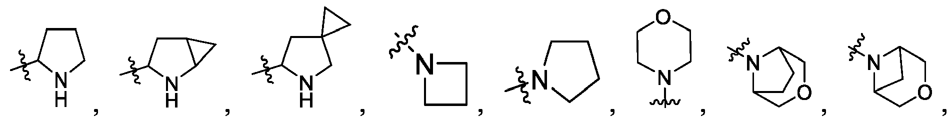

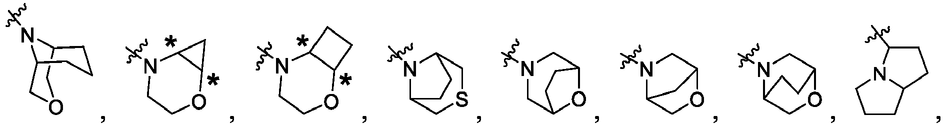

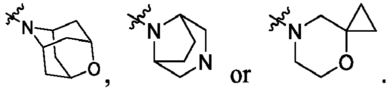

5. The quinazoline compound represented by formula I, the pharmaceutically acceptable salt thereof, the solvate thereof, the prodrug thereof, the metabolite thereof or the isotopic compound thereof according to claim 2, wherein, R^{a} is 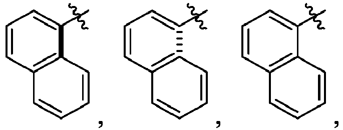 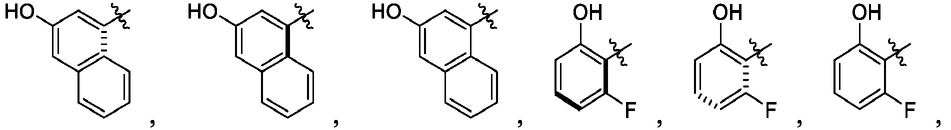 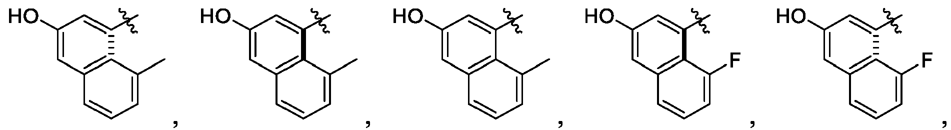 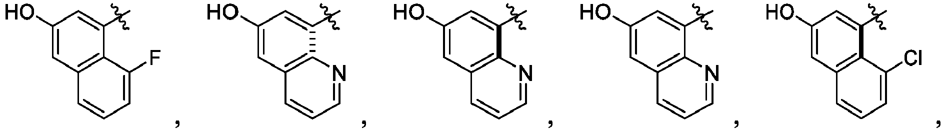 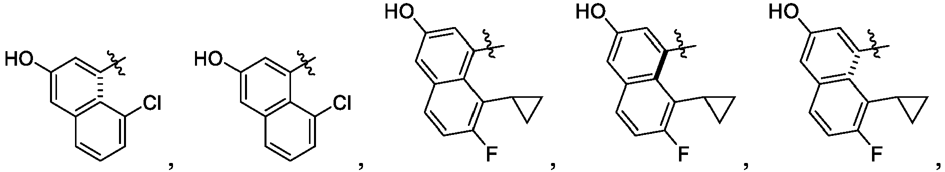 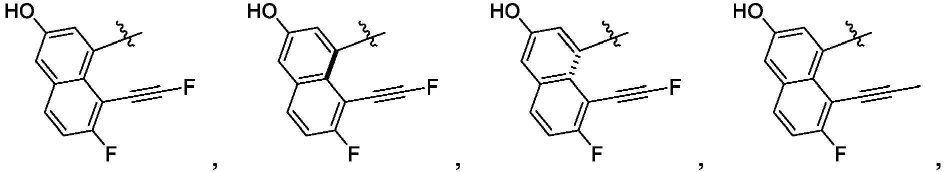 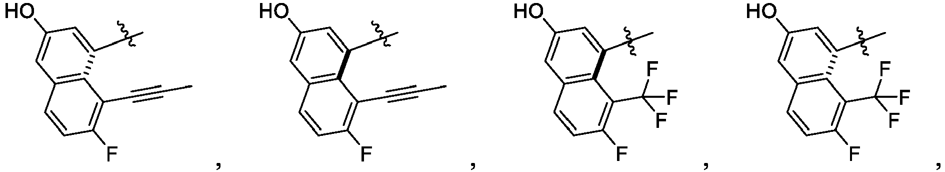 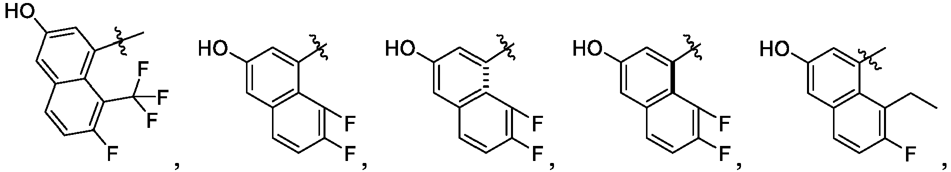 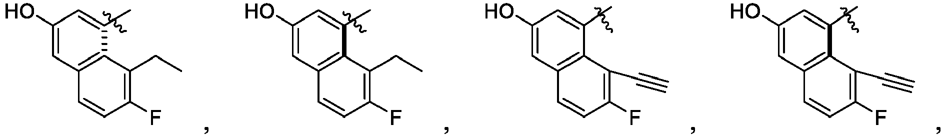 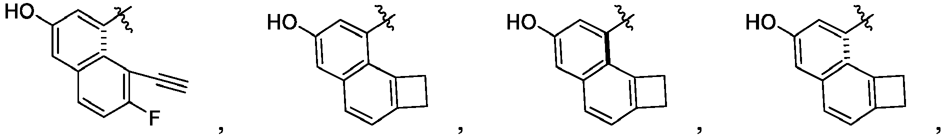 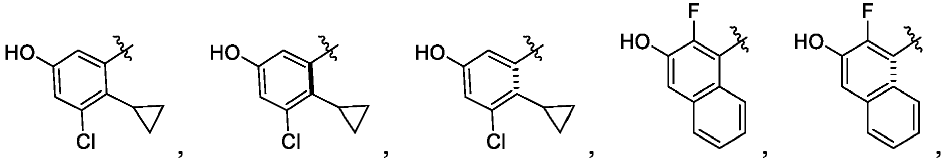 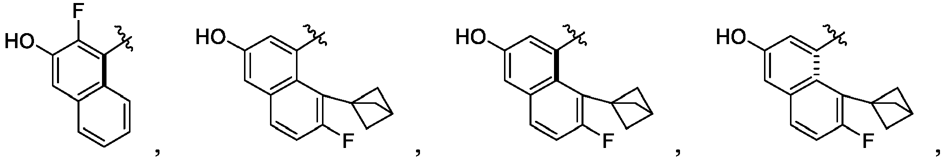 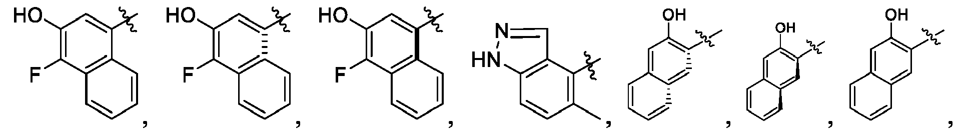 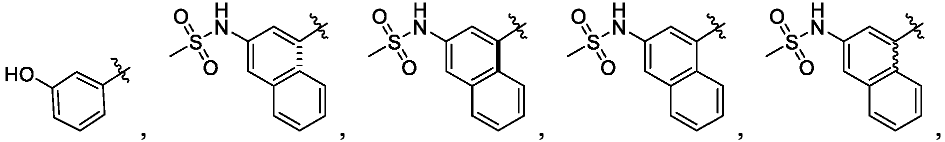 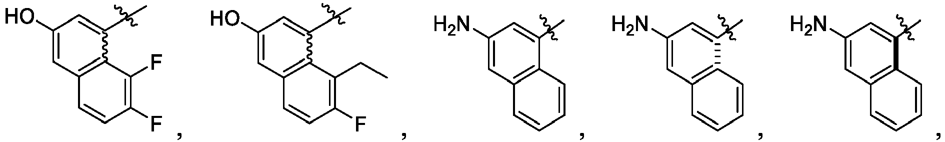 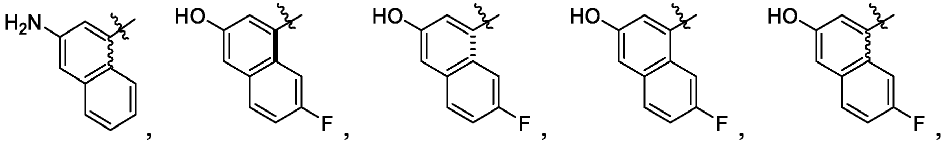 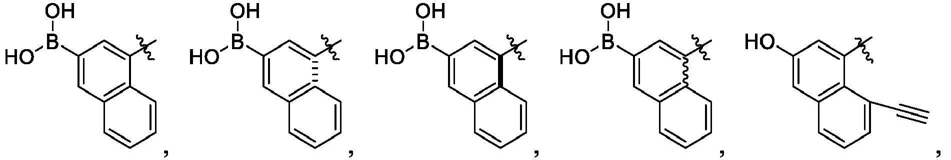 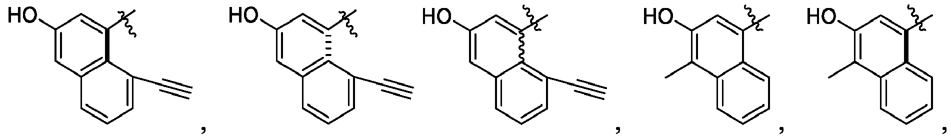 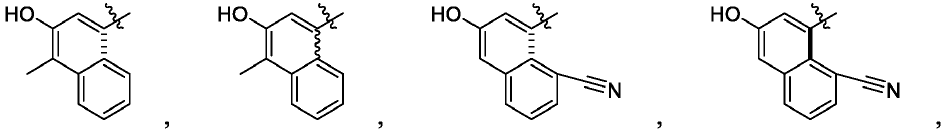 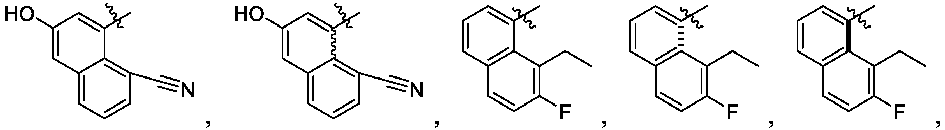 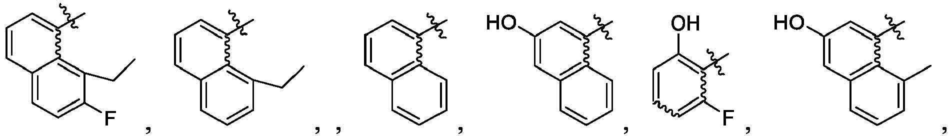 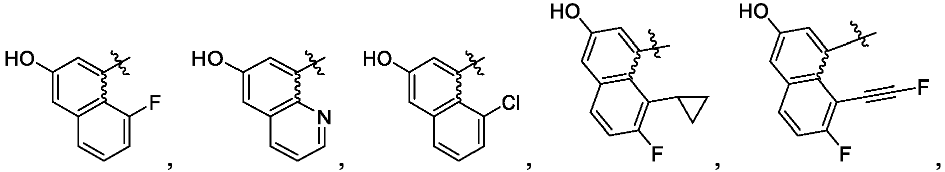 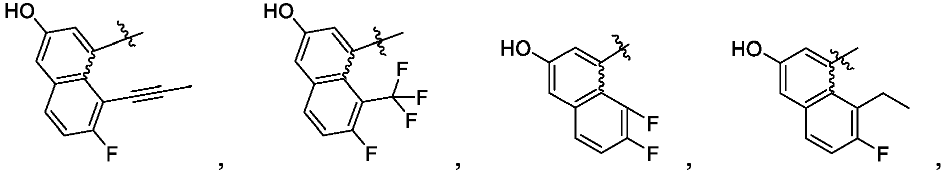 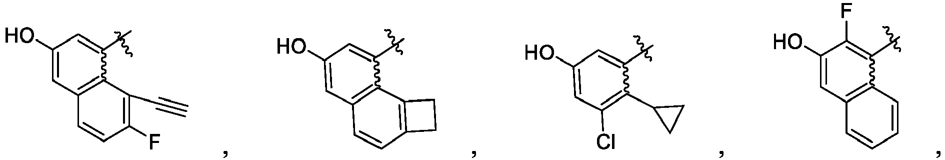 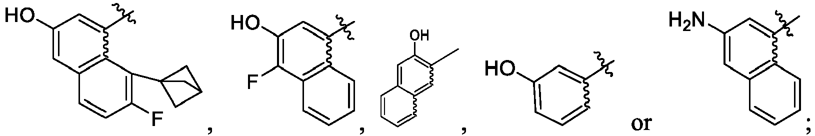
and/or, 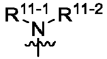 is 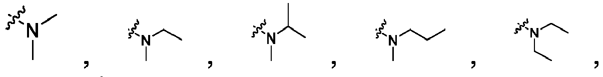 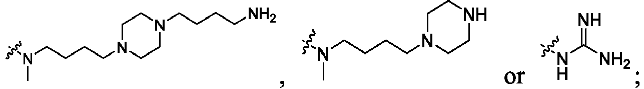
and/or, the 3- to 10-membered heterocycloalkyl substituted by one or more 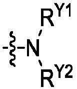 with 1 to 4 heteroatoms selected from one or more of N, O and S is 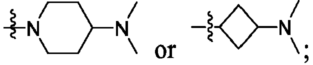
and/or, when Y¹ is C₆-C₁₀ aryl substituted by one hydroxyl, Y¹ is 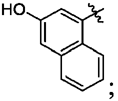
and/or, the unsubstituted 4- to 10-membered heterocycloalkyl or 4- to 10-membered heterocycloalkyl substituted by R¹² with 1 to 3 heteroatoms or heteroatom groups selected from N and O is 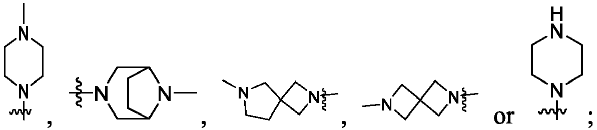
and/or, in R¹¹, the unsubstituted 5- to 6-membered heteroaryl or 5- to 6-membered heteroaryl substituted by one or more R¹¹⁻⁴ with 1 to 3 heteroatoms selected from N and O is 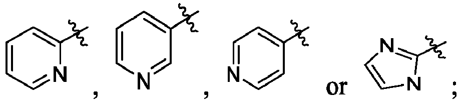
and/or, when R¹¹ is 4- to 10-membered heterocycloalkyl, the C₁-C₆ alkoxy substituted by R¹¹ is *cis, trans,* or a mixture thereof;
and/or, R¹³ is 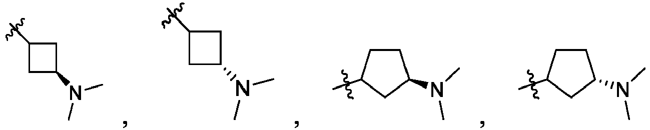 or 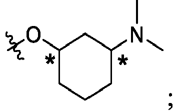
and/or, the amino substituted by one or more R¹⁴ is 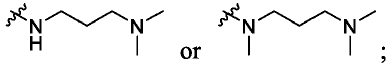
and/or, the 7- to 12-membered heterocycloalkyl substituted by R⁷ with 2 to 3 heteroatoms selected from one or more of N, O and S is 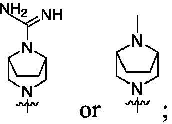
and/or, in ring B, the 4- to 10-membered heterocycloalkyl with 1 to 3 heteroatoms or heteroatom groups selected from N and P(=O) and containing only 1 N is 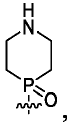 azetidinyl, tetrahydropyrrolyl or piperidinyl;
and/or, in ring B, the 4- to 10-membered heterocycloalkyl substituted by R¹⁰ with 1 to 3 heteroatoms or heteroatom groups selected from N and P(=O) and containing only 1 N is 4- to 10-membered monocyclic heterocycloalkyl with 1 to 3 heteroatoms or heteroatom groups selected from N and P(=O) and containing only 1 N, 4- to 10-membered bridged heterocycloalkyl with 1 to 3 heteroatoms or heteroatom groups selected from N and P(=O) and containing only 1 N, 4- to 10-membered spiro heterocycloalkyl with 1 to 3 heteroatoms or heteroatom groups selected from N and P(=O) and containing only 1 N, or 4- to 10-membered fused heterocycloalkyl with 1 to 3 heteroatoms or heteroatom groups selected from N and P(=O) and containing only 1 N, preferably 4- to 10-membered monocyclic heterocycloalkyl with 1 to 3 heteroatoms or heteroatom groups selected from N and P(=O) and containing only 1 N, or 4-to 10-membered bridged heterocycloalkyl with 1 to 3 heteroatoms or heteroatom groups selected from N and P(=O) and containing only 1 N;
wherein, the 4- to 10-membered monocyclic heterocycloalkyl with 1 to 3 heteroatoms or heteroatom groups selected from N and P(=O) and containing only 1 N is 4- to 10-membered monocyclic heterocycloalkyl with 1 heteroatom being N, preferably 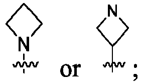
the 4- to 10-membered bridged heterocycloalkyl with 1 to 3 heteroatoms or heteroatom groups selected from N and P(=O) and containing only 1 N is 7- to 10-membered bridged heterocycloalkyl with 1 heteroatom being N, preferably 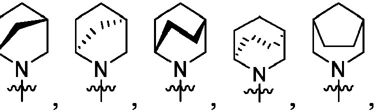 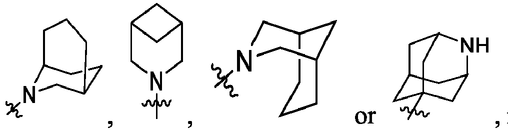 more preferably 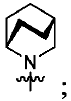
the 4- to 10-membered spiro heterocycloalkyl with 1 to 3 heteroatoms or heteroatom groups selected from N and P(=O) and containing only 1 N is preferably 7- to 10-membered spiro heterocycloalkyl with 1 heteroatom being N, preferably 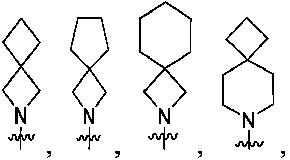 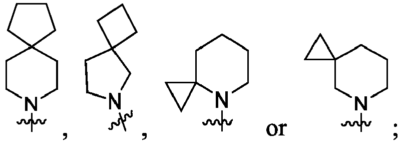
the 4- to 10-membered fused heterocycloalkyl with 1 to 3 heteroatoms or heteroatom groups selected from N and P(=O) and containing only 1 N is preferably 4- to 10-membered fused heterocycloalkyl with 1 heteroatom being N;
and/or, the 4- to 10-membered heterocycloalkyl substituted by R¹⁰ with 1 to 3 heteroatoms or heteroatom groups selected from N and P(=O) and containing only one N is 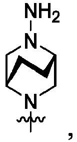 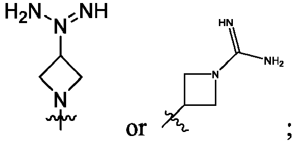
and/or, in ring B, the 7- to 12-membered heterocycloalkyl with 2 to 3 heteroatoms selected from one or more of N, O and S is C₇ monocyclic heterocycloalkyl with 2 heteroatoms being N, 7- to 12-membered bridged heterocycloalkyl with 2 to 3 heteroatoms selected from N and O and containing at least 2 N, 7- to 12-membered spiro heterocycloalkyl with 2 heteroatoms being N, or 7- to 12-membered fused heterocycloalkyl with 2 heteroatoms being N, preferably 7- to 12-membered bridged heterocycloalkyl with 2 heteroatoms being N, or 7- to 12-membered spiro heterocycloalkyl with 2 heteroatoms being N; wherein,
the 7-membered monocyclic heterocycloalkyl with 2 heteroatoms being N can be 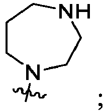
the 7- to 12-membered bridged heterocycloalkyl with 2 to 3 heteroatoms selected from N and O and containing at least 2 N is preferably 7- to 9-membered bridged heterocycloalkyl (for example, 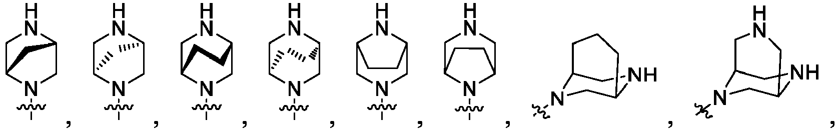 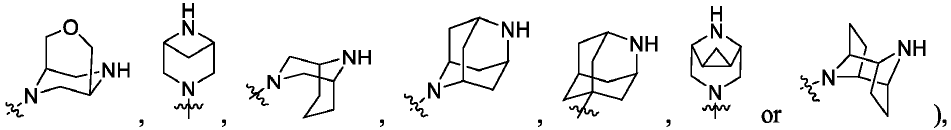 more preferably 7- to 8-membered bridged heterocycloalkyl with 2 heteroatoms being N (for example, 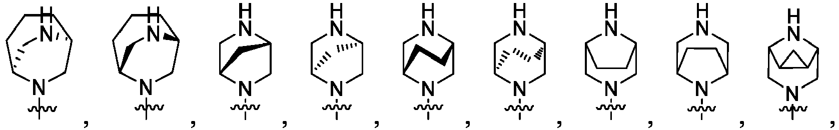 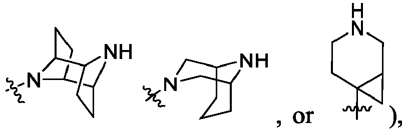 further preferably 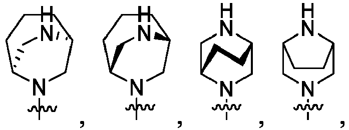 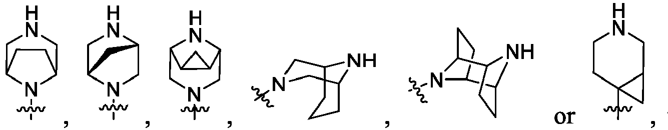 further preferably 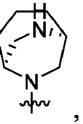 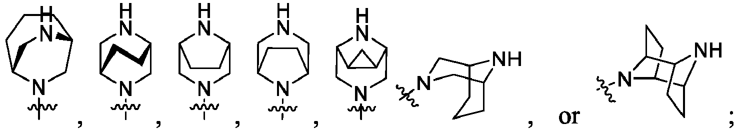
the 7- to 17-membered spiro heterocycloalkyl with 2 heteroatoms being N is preferably 7- to 10-membered spiro heterocycloalkyl with 2 heteroatoms being N (for example, 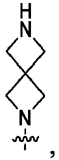 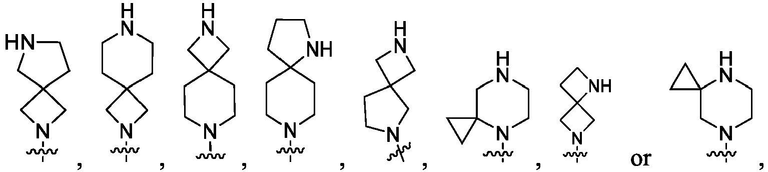 preferably 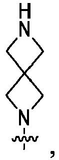 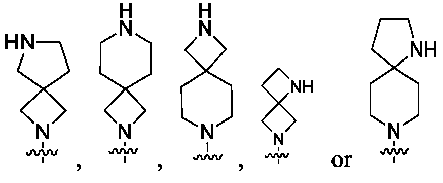 ); and more preferably 7- to 10-membered spiro heterocycloalkyl with 2 heteroatoms being N, wherein one ring is a 4-membered heterocycloalkyl containing one N atom, for example, 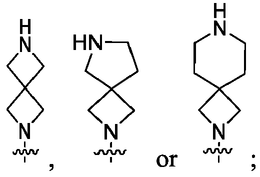
the 7- to 12-membered fused heterocycloalkyl with 2 heteroatoms being N is preferably 7- to 10-membered fused heterocycloalkyl with 2 heteroatoms being N, more preferably 3-membered-fused 6-membered heterocycloalkyl with 2 heteroatoms being N (for example, 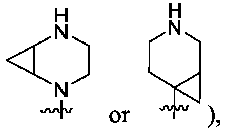 4-membered-fused 6-membered heterocycloalkyl with 2 heteroatoms being N (for example, 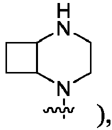 5-membered-fused 6-membered heterocycloalkyl with 2 heteroatoms being N (for example, 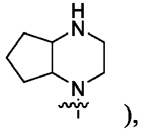 6-membered-fused 6-membered heterocycloalkyl with 2 heteroatoms being N (for example, 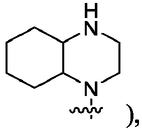 or 5-membered-fused 5-membered heterocycloalkyl with 2 heteroatoms being N (for example, 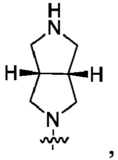 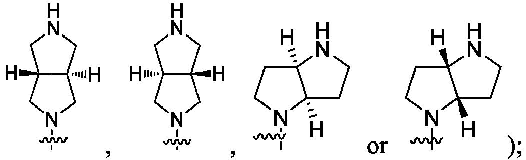
the 7- to 12-membered spiro heterocycloalkyl with 2 heteroatoms being N is preferably 7- to 10-membered spiro heterocycloalkyl with 2 heteroatoms being N, and more preferably 7-to 10-membered spiro heterocycloalkyl with 2 heteroatoms being N wherein one ring is a 4-membered heterocycloalkyl containing one N atom, for example, 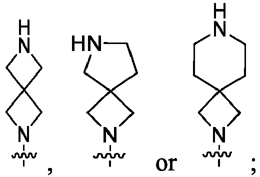
the 7- to 17-membered fused heterocycloalkyl with 2 heteroatoms being N is preferably 7- to 10-membered fused heterocycloalkyl with 2 heteroatoms being N, more preferably 3-membered-fused 6-membered heterocycloalkyl with 2 heteroatoms being N, 4-membered-fused 6-membered heterocycloalkyl with 2 heteroatoms being N, 5-membered-fused 6-membered heterocycloalkyl with 2 heteroatoms being N, 6-membered-fused 6-membered heterocycloalkyl with 2 heteroatoms being N, or 5-membered-fused 5-membered heterocycloalkyl with 2 heteroatoms being N, further preferably 5-membered-fused 5-membered heterocycloalkyl with 2 heteroatoms being N;
and/or, in R⁷, the C₁-C₄ alkyl in -C(=O)O-(C₁-C₄ alkyl) is methyl, ethyl, n-propyl, isopropyl, n-butyl, sec-butyl, isobutyl or tert-butyl;
and/or, in R⁷, the C₁-C₃ alkyl in the C₁-C₃ alkyl substituted by CN is methyl, ethyl, n-propyl or isopropyl;
and/or, in ring C, the 4- to 10-membered heterocycloalkyl with 1 to 3 heteroatoms or heteroatom groups selected from N and S(=O) is 6- to 7-membered heterocycloalkyl with 1 or 2 heteroatoms or heteroatom groups selected from N and S(=O), for example, 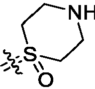 or 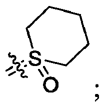
and/or, in R⁹⁻¹ and R⁹⁻², the C₁-C₃ alkyl is methyl, ethyl, n-propyl or isopropyl;
and/or, in R⁹⁻¹ and R⁹⁻², the C₁-C₃ alkyl in the C₁-C₃ alkyl substituted by CN is methyl, ethyl, n-propyl or isopropyl, for example, methyl;
and/or, in R¹ and R⁴, the C₁-C₃ alkyl is methyl, ethyl, n-propyl or isopropyl, for example, methyl;
and/or, in R^{2a}, the C₁-C₃ alkyl in the -O-(C₁-C₃ alkyl) is methyl, ethyl, n-propyl or isopropyl, for example, tert-butyl;
and/or, in R^{2a-1} and R^{2a-2}, the C₁-C₃ alkyl is methyl, ethyl, n-propyl or isopropyl, for example, methyl.

6. The quinazoline compound represented by formula I, the pharmaceutically acceptable salt thereof, the solvate thereof, the prodrug thereof, the metabolite thereof or the isotopic compound thereof according to claim 2, wherein, the C₁-C₆ alkoxy substituted by R¹¹ is 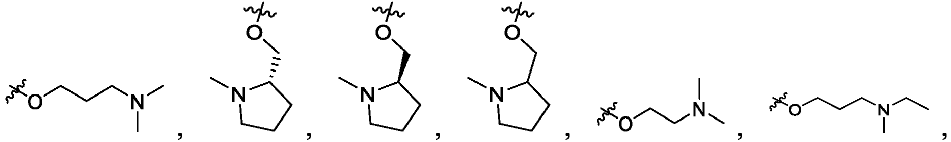 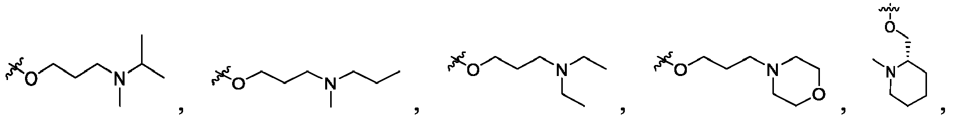 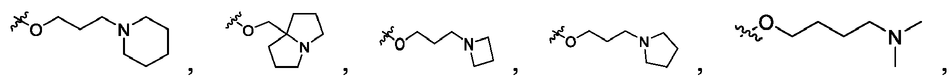 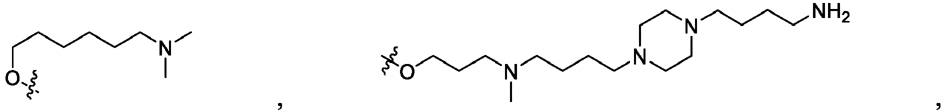 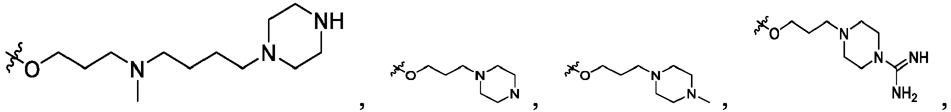 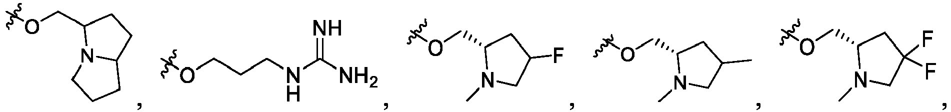 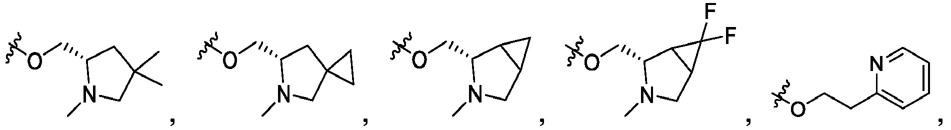 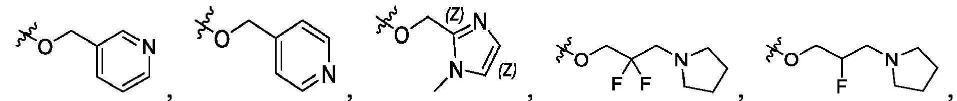 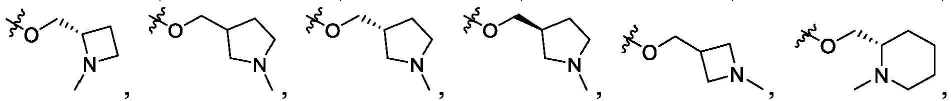 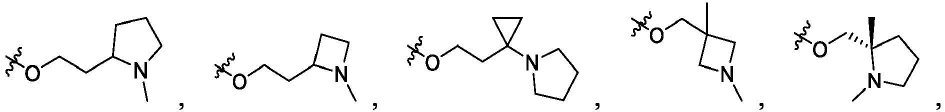 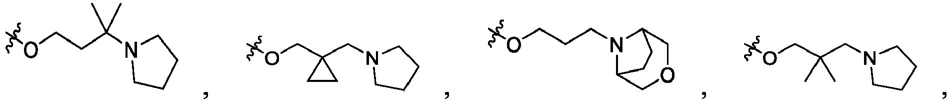 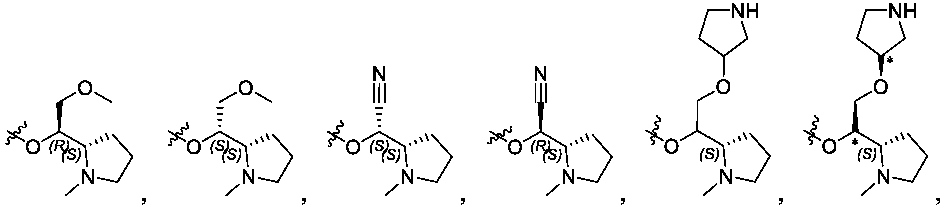 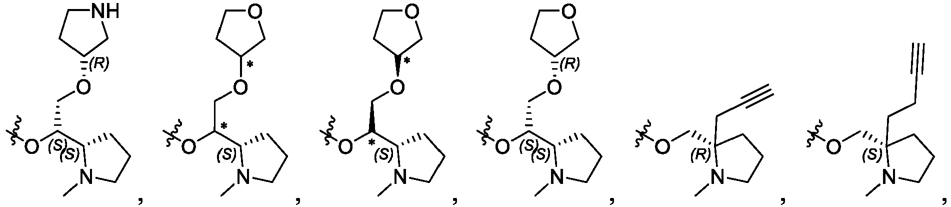 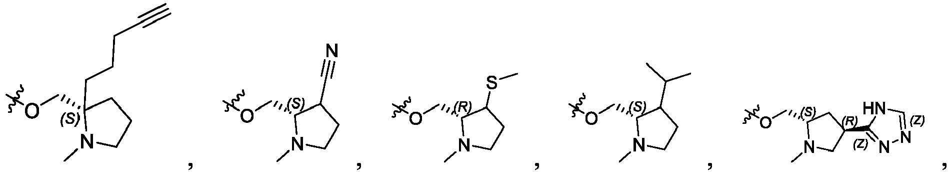 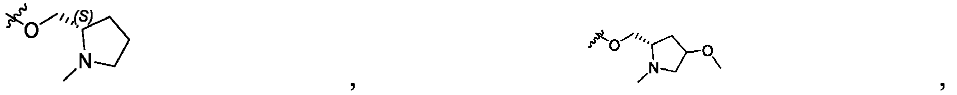 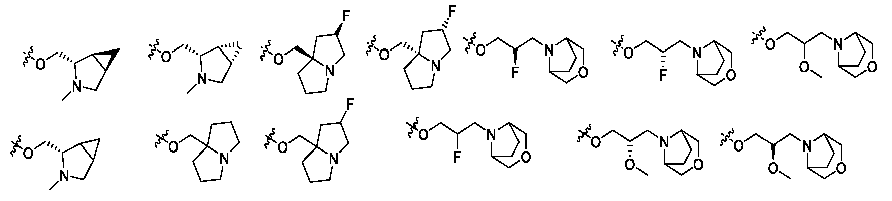 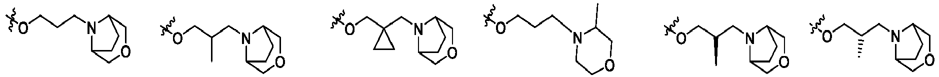 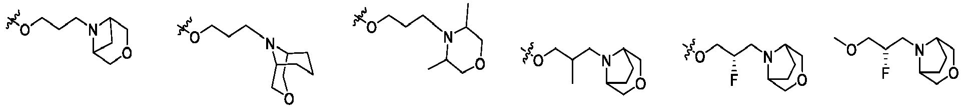 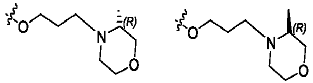 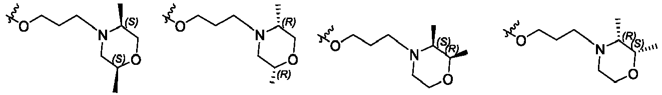 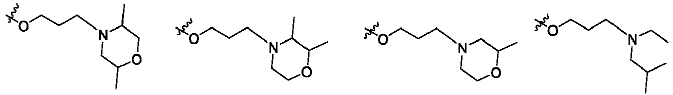 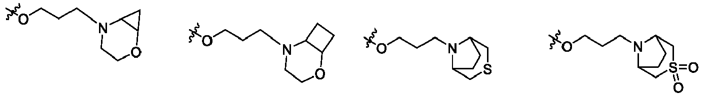 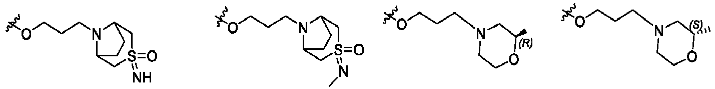 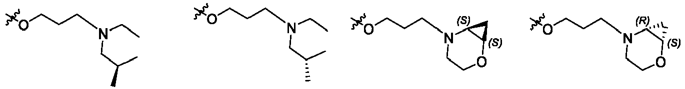 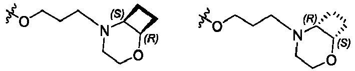 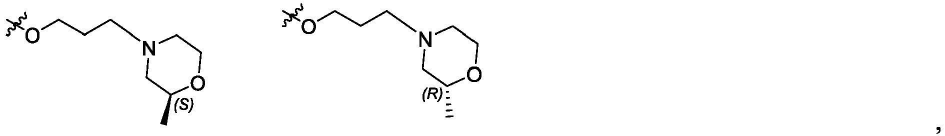 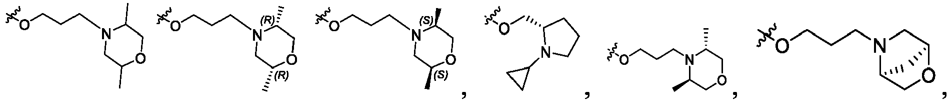 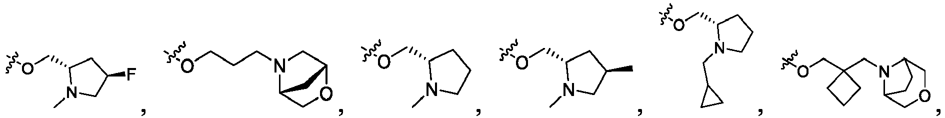 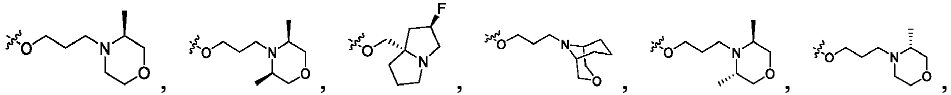 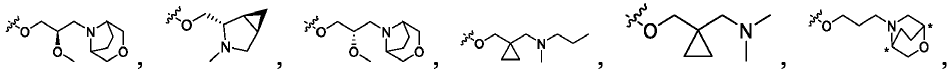 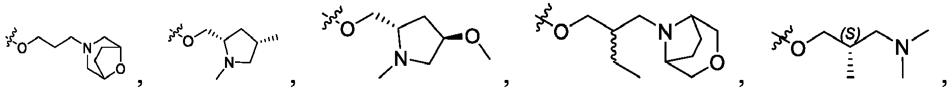 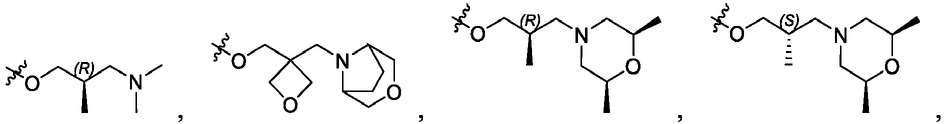 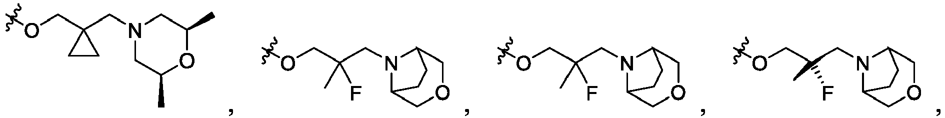 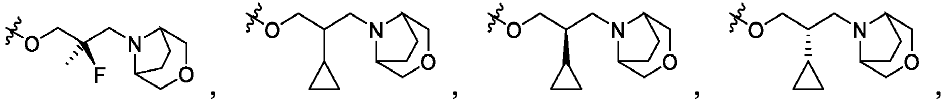 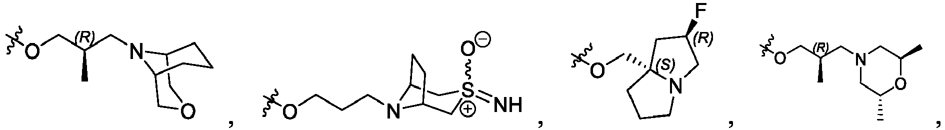 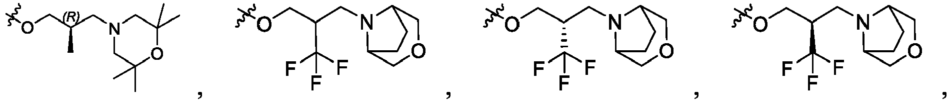 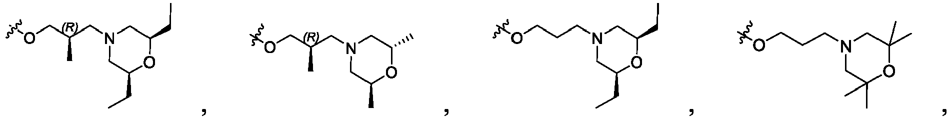 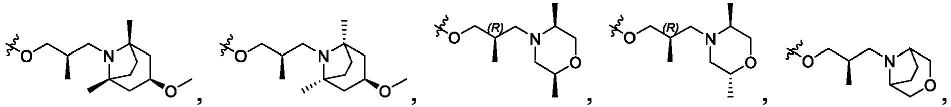 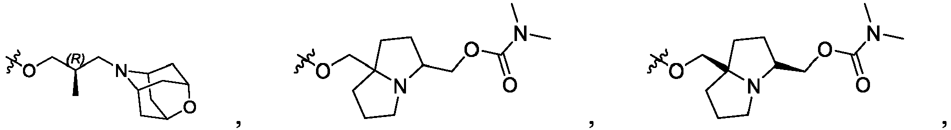 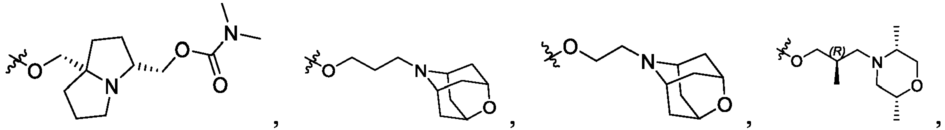 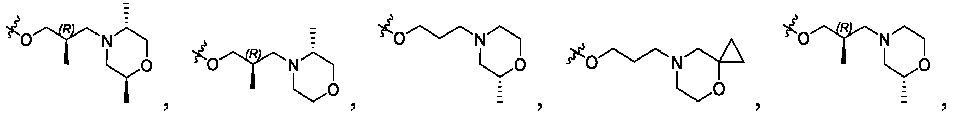 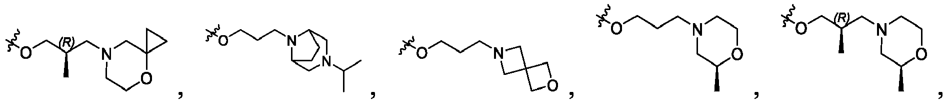 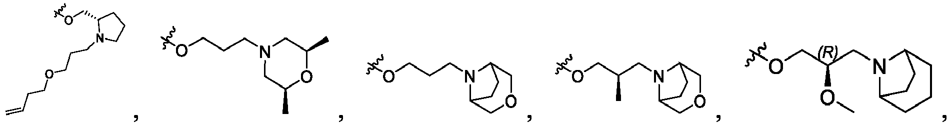 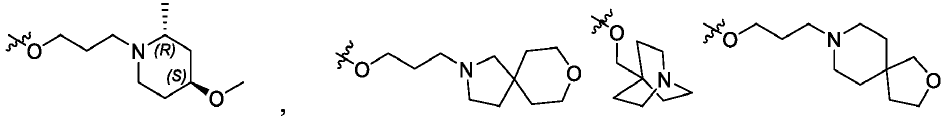 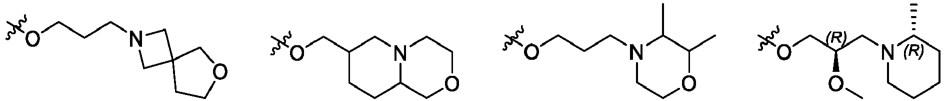 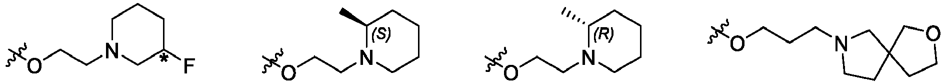 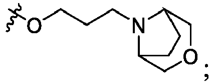
and/or, R¹³ is 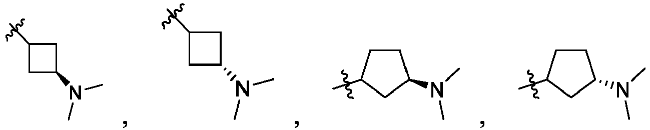 or 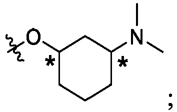
and/or, the amino substituted by one or more R¹⁴ is 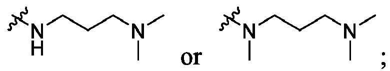
and/or, in ring B, the 4- to 10-membered heterocycloalkyl with 1 to 3 heteroatoms or heteroatom groups selected from N and P(=O) and containing only 1 N is 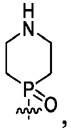 azetidinyl, tetrahydropyrrolyl or piperidinyl.

7. The quinazoline compound represented by formula I, the pharmaceutically acceptable salt thereof, the solvate thereof, the prodrug thereof, the metabolite thereof or the isotopic compound thereof according to claim 2, wherein, Y¹ is 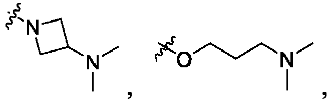 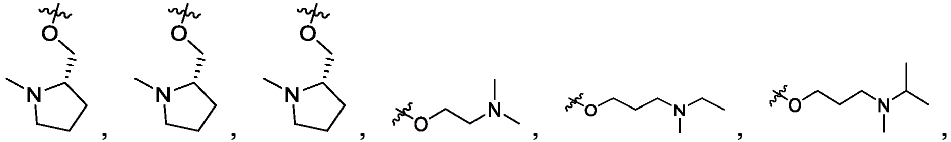 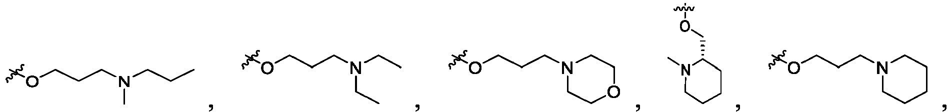 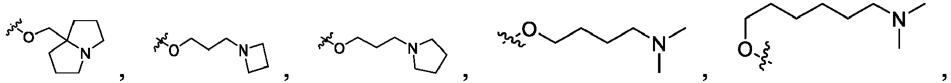 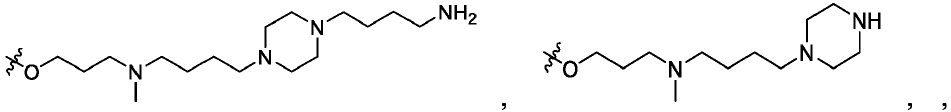 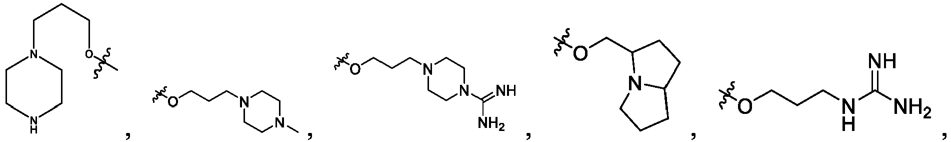 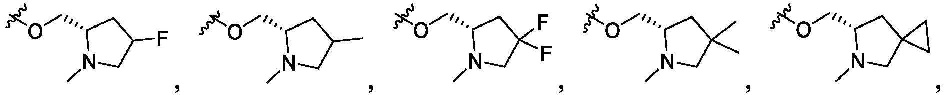 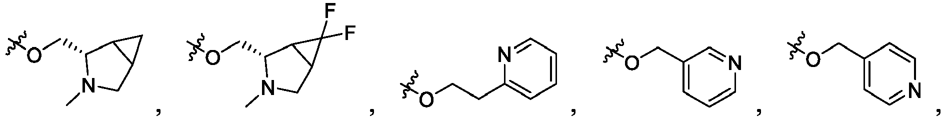 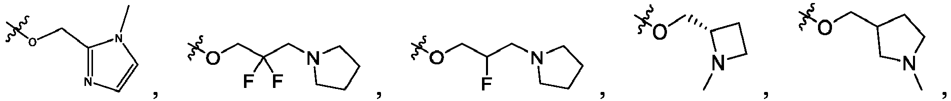 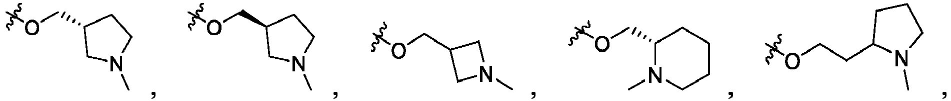 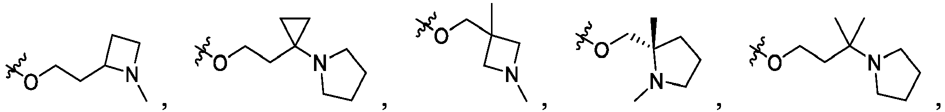 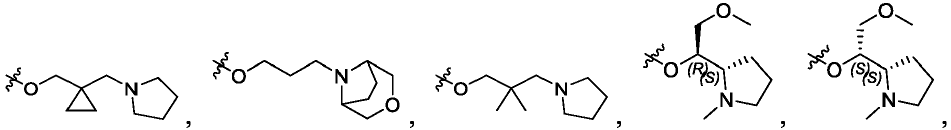 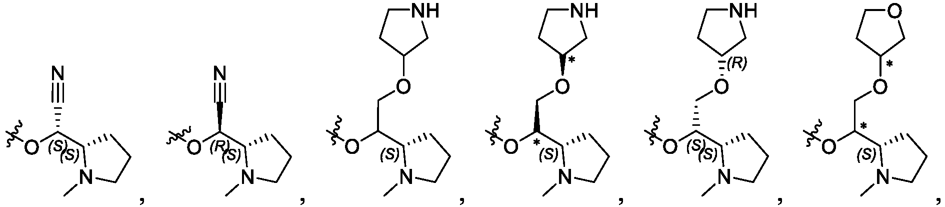 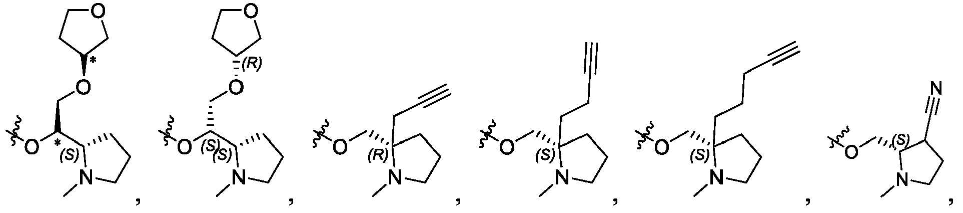 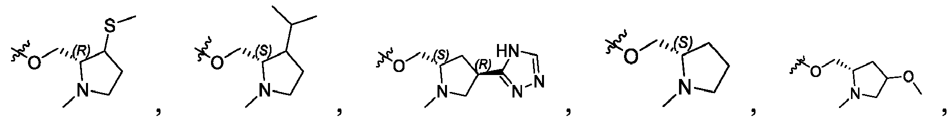 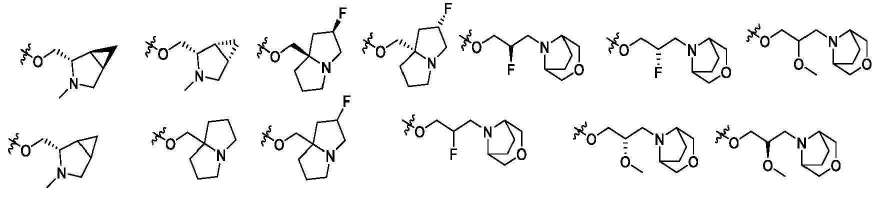 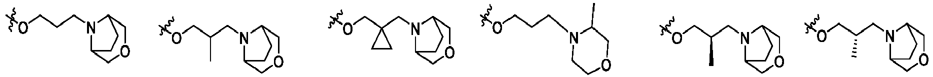 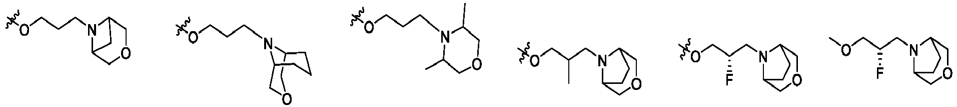 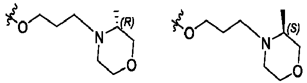 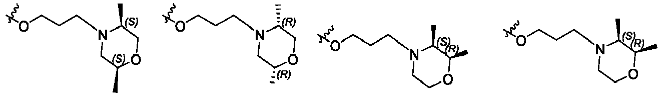 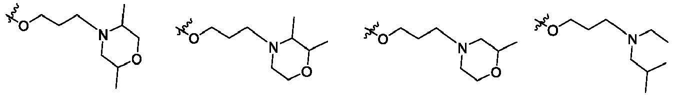 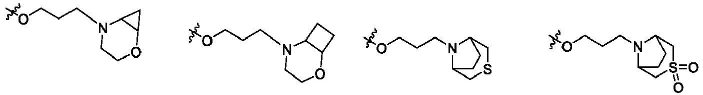 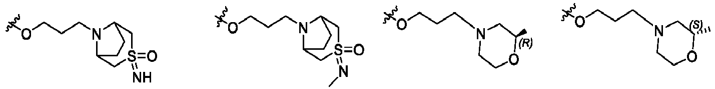 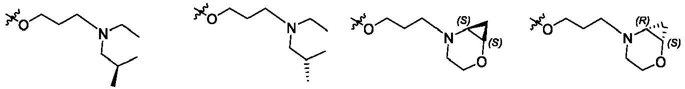 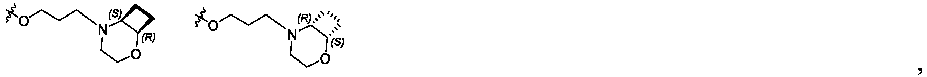 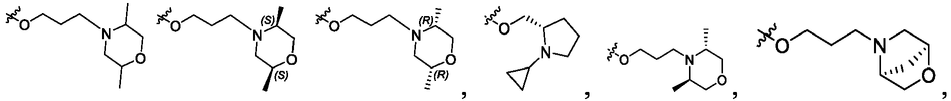 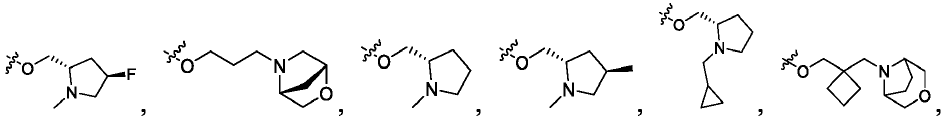 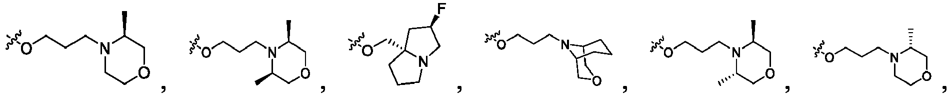 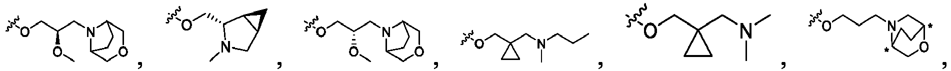 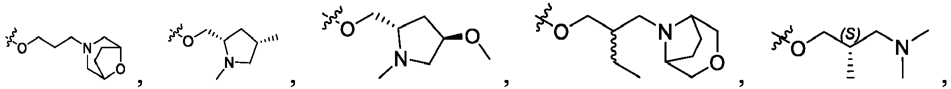 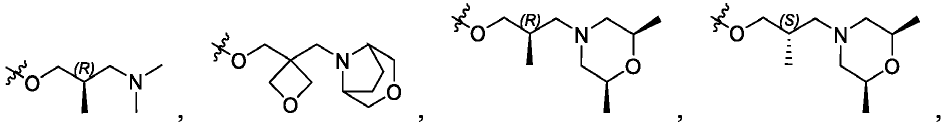 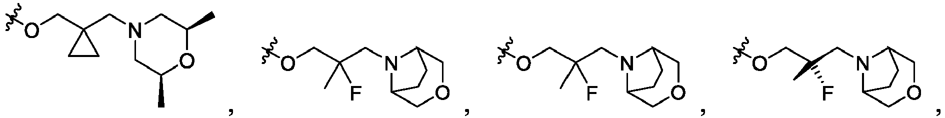 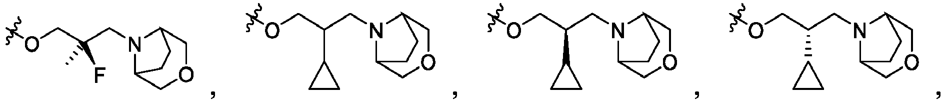 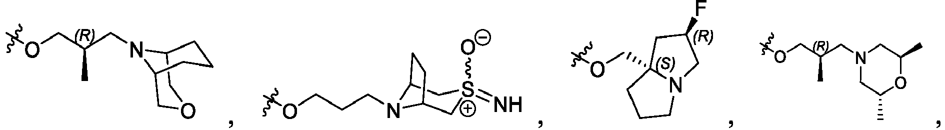 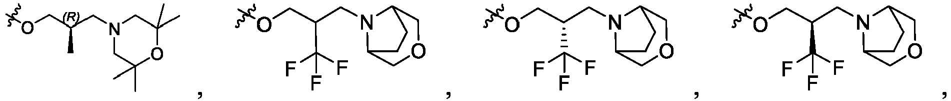 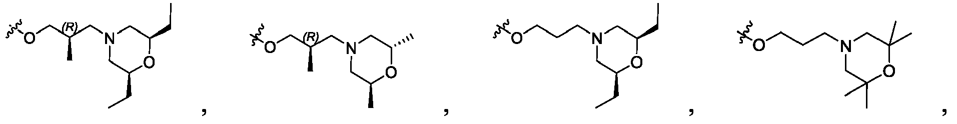 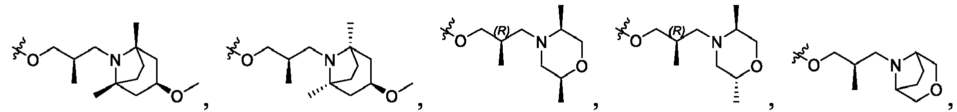 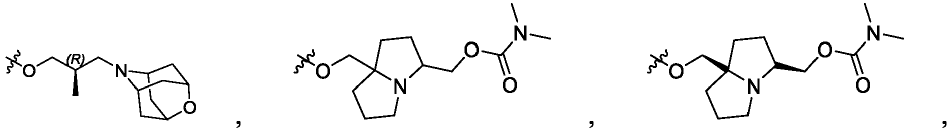 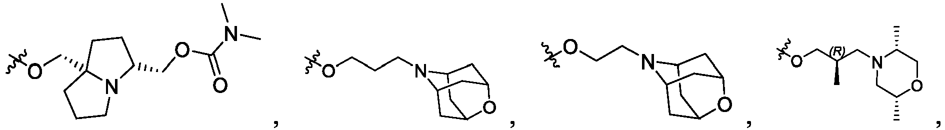 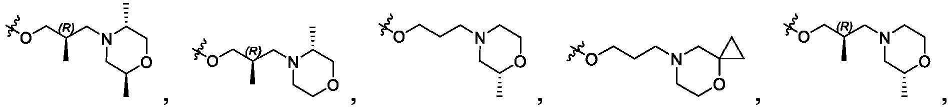 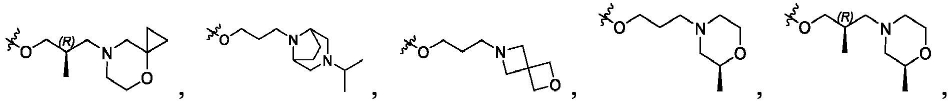 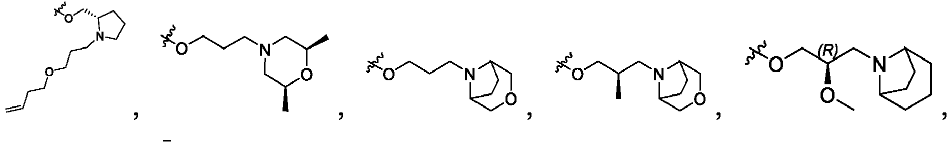 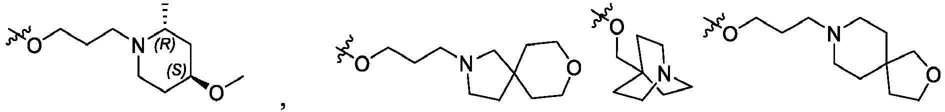 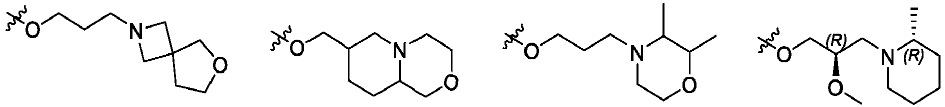 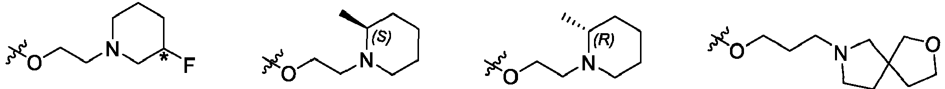 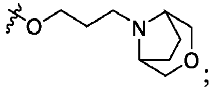
and/or, in 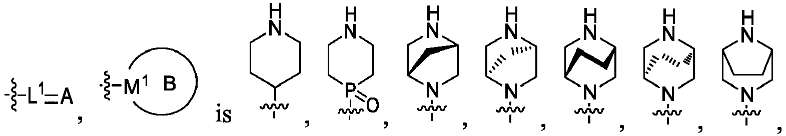 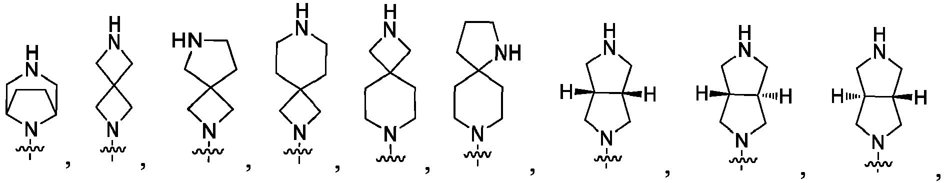 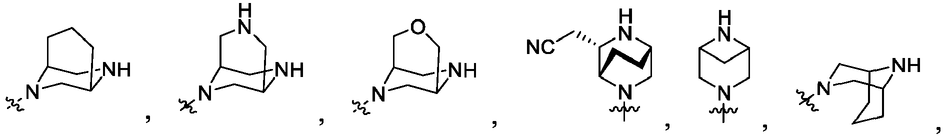 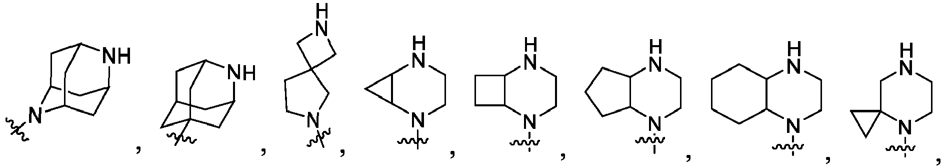 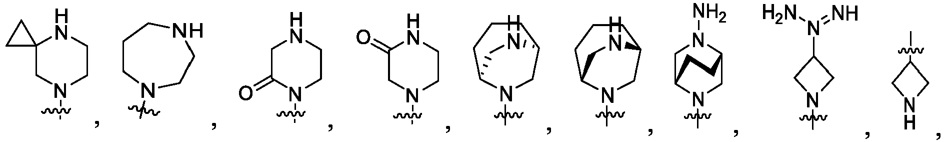 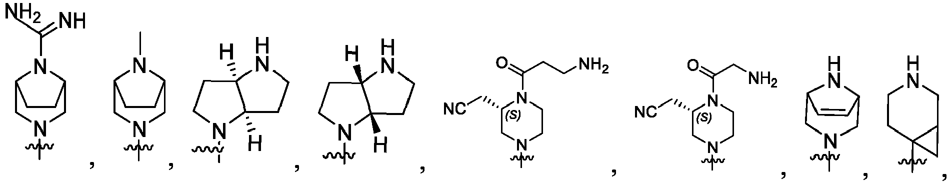 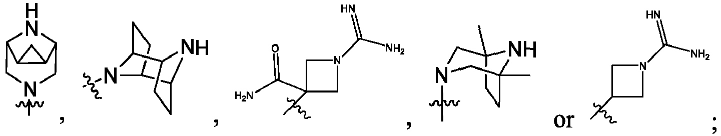
and/or, in 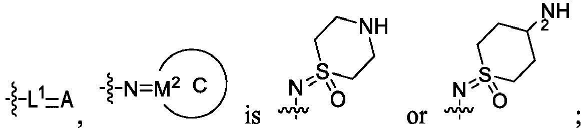
and/or, in 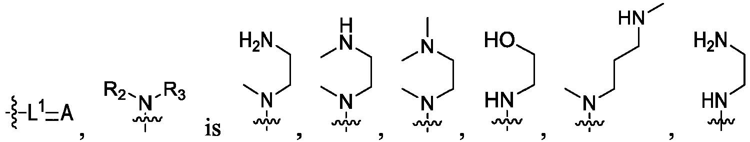 or 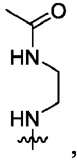 preferably

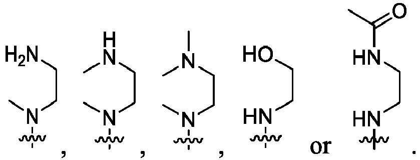

8. The quinazoline compound represented by formula I, the pharmaceutically acceptable salt thereof, the solvate thereof, the prodrug thereof, the metabolite thereof or the isotopic compound thereof according to claim 1, wherein, the quinazoline compound represented by formula I is a compound represented by formula I-A, 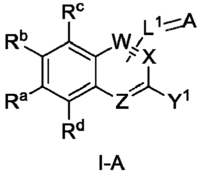
wherein, R^{a} is 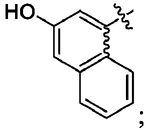
" 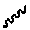 " indicates " 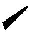 ", " 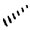 " or a mixture thereof;
R^{b}, R^{c} and R^{d} are independently H, halogen, C₁-C₃ alkyl or C₁-C₃ alkoxy;
W is C;
X and Z are N;
Y¹is 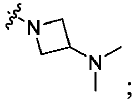
" 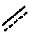 " in 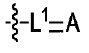 is a single bond or a double bond;
" 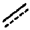 " in 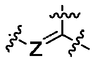 is a single bond or a double bond.

9. The quinazoline compound represented by formula I, the pharmaceutically acceptable salt thereof, the solvate thereof, the prodrug thereof, the metabolite thereof or the isotopic compound thereof according to claim 8, wherein,
in R^{b}, R^{c} and R^{d}, the halogen is fluorine, chlorine, bromine or iodine;
and/or, in R^{b}, R^{c} and R^{d}, the C₁-C₃ alkyl is methyl, ethyl, propyl or isopropyl;
and/or, in R^{b}, R^{c} and R^{d}, the C₁-C₃ alkoxy is methoxy, ethoxy, propoxy or isopropoxy;
and/or, in ring B, the 4- to 10-membered heterocycloalkyl with 1 to 3 heteroatoms or heteroatom groups selected from N and P(=O) and containing only 1 N is 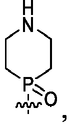 azetidinyl, tetrahydropyrrolyl or piperidinyl;
and/or, in ring B, the 7- to 12-membered heterocycloalkyl with 2 to 3 heteroatoms selected from one or more of N, O and S is 7-membered monocyclic heterocycloalkyl with 2 heteroatoms being N, 7- to 12-membered bridged heterocycloalkyl with 2 to 3 heteroatoms selected from N and O and containing at least 2 N, 7- to 12-membered spiro heterocycloalkyl with 2 heteroatoms being N, or 7- to 12-membered fused heterocycloalkyl with 2 heteroatoms being N;
and/or, in R⁷, the C₁-C₄ alkyl in -C(=O)O-(C₁-C₄ alkyl) is methyl, ethyl, n-propyl, isopropyl, n-butyl, sec-butyl, isobutyl or tert-butyl;
and/or, in R⁷, the C₁-C₃ alkyl in the C₁-C₃ alkyl substituted by CN is methyl, ethyl, n-propyl or isopropyl;
and/or, in ring C, the 4- to 10-membered heterocycloalkyl with 1 to 3 heteroatoms or heteroatom groups selected from N and S(=O) is 6- to 7-membered heterocycloalkyl with 1 or 2 heteroatoms or heteroatom groups selected from N and S(=O);
and/or, in R⁹⁻¹ and R⁹⁻², the C₁-C₃ alkyl is methyl, ethyl, n-propyl or isopropyl;
and/or, in R⁹⁻¹ and R⁹⁻², the C₁-C₃ alkyl in the C₁-C₃ alkyl substituted by CN is methyl, ethyl, n-propyl or isopropyl;
and/or, in R¹ and R⁴, the C₁-C₃ alkyl is methyl, ethyl, n-propyl or isopropyl;
and/or, in R^{2a}, the C₁-C₃ alkyl in the -O-(C₁-C₃ alkyl) is methyl, ethyl, n-propyl or isopropyl;
and/or, in R^{2a-1} and R^{2a-2}, the C₁-C₃ alkyl is methyl, ethyl, n-propyl or isopropyl.

10. The quinazoline compound represented by formula I, the pharmaceutically acceptable salt thereof, the solvate thereof, the prodrug thereof, the metabolite thereof or the isotopic compound thereof according to claim 9, wherein,
the 4- to 10-membered heterocycloalkyl with 1 to 3 heteroatoms or heteroatom groups selected from N and P(=O) and containing only one N is 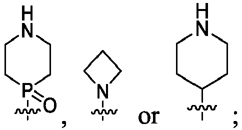
and/or, in ring B, when the 7- to 12-membered heterocycloalkyl with 2 to 3 heteroatoms selected from one or more of N, O and S is 7-membered monocyclic heterocycloalkyl with 2 heteroatoms being N, the 7-membered monocyclic heterocycloalkyl with 2 heteroatoms being N is 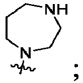
and/or, in ring B, when the 7- to 12-membered heterocycloalkyl with 2 to 3 heteroatoms selected from one or more of N, O and S is 7- to 12-membered bridged heterocycloalkyl with 2 to 3 heteroatoms selected from N and O and containing at least 2 N, the 7- to 12-membered bridged heterocycloalkyl with 2 to 3 heteroatoms selected from N and O and containing at least 2 N is 7- to 9-membered bridged heterocycloalkyl;
and/or, in ring B, when the 7- to 12-membered heterocycloalkyl with 2 to 3 heteroatoms selected from one or more of N, O and S is 7- to 12-membered spiro heterocycloalkyl with 2 heteroatoms being N, the 7- to 12-membered spiro heterocycloalkyl with 2 heteroatoms being N is 7- to 10-membered spiro heterocycloalkyl with 2 heteroatoms being N;
and/or, in ring B, when the 7- to 12-membered heterocycloalkyl with 2 to 3 heteroatoms selected from one or more of N, O and S is 7- to 12-membered fused heterocycloalkyl with 2 heteroatoms being N, the 7- to 12-membered fused heterocycloalkyl with 2 heteroatoms being N is 7- to 10-membered fused heterocycloalkyl with 2 heteroatoms being N;
and/or, in R⁷, the C₁-C₄ alkyl in -C(=O)O-(C₁-C₄ alkyl) is tert-butyl;
and/or, in R⁷, the C₁-C₃ alkyl in the C₁-C₃ alkyl substituted by CN is methyl;
and/or, in ring C, the 4- to 10-membered heterocycloalkyl with 1 to 3 heteroatoms or heteroatom groups selected from N and S(=O) is 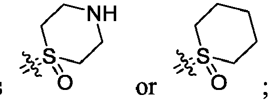
and/or, in R¹ and R⁴, the C₁-C₃ alkyl is methyl;
and/or, in R^{2a-1} and R^{2a-2}, the C₁-C₃ alkyl is methyl.

11. The quinazoline compound represented by formula I, the pharmaceutically acceptable salt thereof, the solvate thereof, the prodrug thereof, the metabolite thereof or the isotopic compound thereof according to claim 10, wherein,
in ring B, when the 7- to 12-membered heterocycloalkyl with 2 to 3 heteroatoms selected from one or more of N, O and S is 7- to 12-membered bridged heterocycloalkyl with 2 to 3 heteroatoms selected from N and O and containing at least 2 N, the 7- to 12-membered bridged heterocycloalkyl with 2 to 3 heteroatoms selected from N and O and containing at least 2 N is 7- to 8-membered bridged heterocycloalkyl with 2 heteroatoms being N;
and/or, in ring B, when the 7- to 12-membered heterocycloalkyl with 2 to 3 heteroatoms selected from one or more of N, O and S is 7- to 12-membered spiro heterocycloalkyl with 2 heteroatoms being N, the 7- to 12-membered spiro heterocycloalkyl with 2 heteroatoms being N is 7- to 10-membered spiro heterocycloalkyl with 2 heteroatoms being N wherein one ring is a 4-membered heterocycloalkyl, the N atom in the 4-membered heterocycloalkyl is attached to the quinazoline ring;
and/or, in ring B, when the 7- to 12-membered heterocycloalkyl with 2 to 3 heteroatoms selected from one or more of N, O and S is 7- to 12-membered fused heterocycloalkyl with 2 heteroatoms being N, the 7- to 12-membered fused heterocycloalkyl with 2 heteroatoms being N is 3-membered-fused 6-membered heterocycloalkyl with 2 heteroatoms being N, 4-membered-fused 6-membered heterocycloalkyl with 2 heteroatoms being N, 5-membered-fused 6-membered heterocycloalkyl with 2 heteroatoms being N, 6-membered-fused 6-membered heterocycloalkyl with 2 heteroatoms being N, or 5-membered-fused 5-membered heterocycloalkyl with 2 heteroatoms being N.

12. The quinazoline compound represented by formula I, the pharmaceutically acceptable salt thereof, the solvate thereof, the prodrug thereof, the metabolite thereof or the isotopic compound thereof according to claim 9, wherein,
in ring B, the 7- to 12-membered heterocycloalkyl with 2 to 3 heteroatoms selected from one or more of N, O and S is 7- to 12-membered bridged heterocycloalkyl with 2 to 3 heteroatoms selected from N and O and containing at least 2 N, the 7- to 12-membered bridged heterocycloalkyl with 2 to 3 heteroatoms selected from N and O and containing at least 2 N is 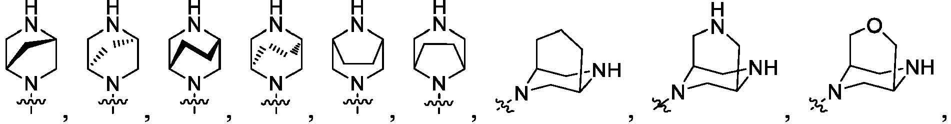 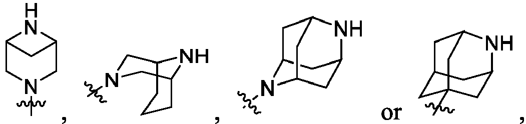 preferably 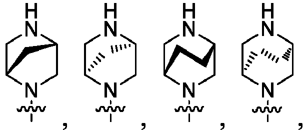 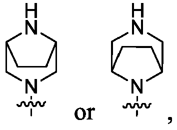 more preferably 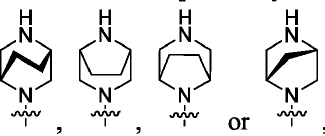 , further preferably 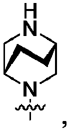 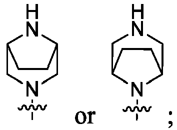
and/or, in ring B, when the 7- to 12-membered heterocycloalkyl with 2 to 3 heteroatoms selected from one or more of N, O and S is 7- to 12-membered spiro heterocycloalkyl with 2 heteroatoms being N, the 7- to 12-membered spiro heterocycloalkyl with 2 heteroatoms being N is 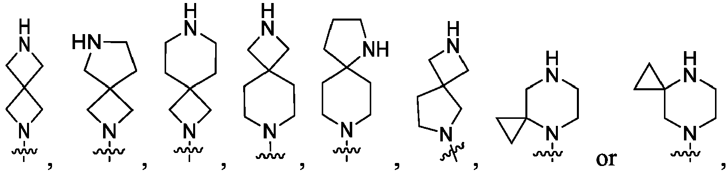 preferably 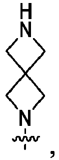 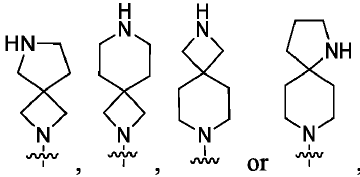 more preferably 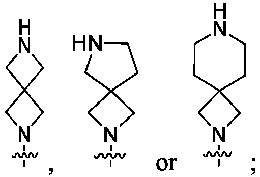
and/or, in ring B, when the 7- to 12-membered heterocycloalkyl with 2 to 3 heteroatoms selected from one or more of N, O and S is 7- to 12-membered fused heterocycloalkyl with 2 heteroatoms being N, the 7- to 12-membered fused heterocycloalkyl with 2 heteroatoms being N is 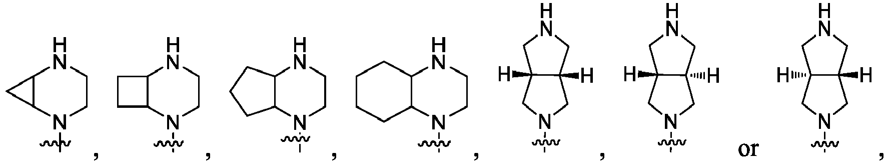 preferably

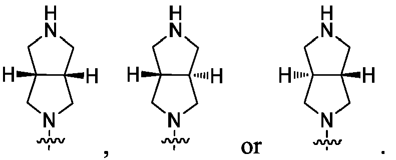

13. The quinazoline compound represented by formula I, the pharmaceutically acceptable salt thereof, the solvate thereof, the prodrug thereof, the metabolite thereof or the isotopic compound thereof according to claim 12, wherein,
in 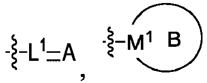 is 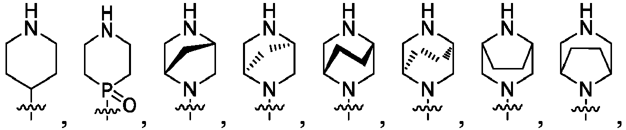 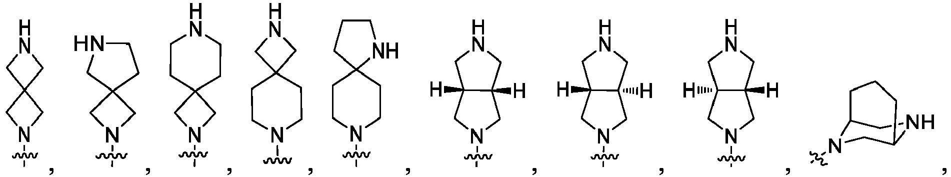 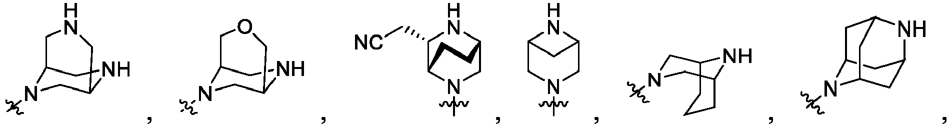 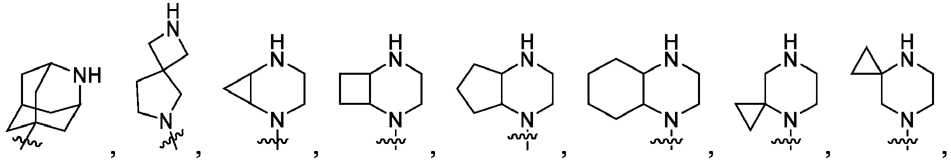 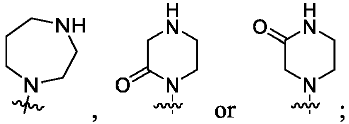
and/or, in 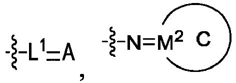 is 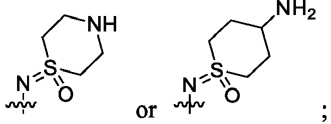
and/or, in 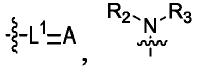 is 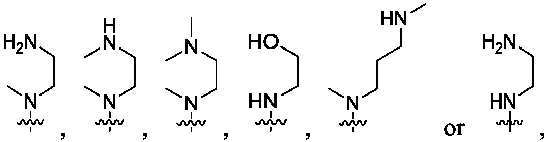 preferably 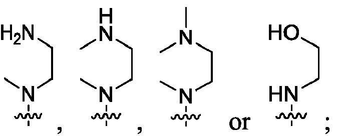
and/or, in 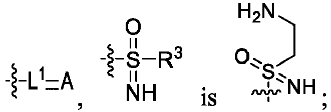
and/or, in

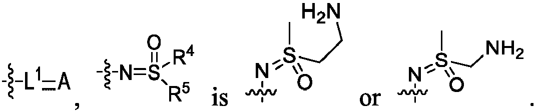

14. The quinazoline compound represented by formula I, the pharmaceutically acceptable salt thereof, the solvate thereof, the prodrug thereof, the metabolite thereof or the isotopic compound thereof according to claim 1, wherein,
the quinazoline compound represented by formula I is a compound represented by formula I-B, 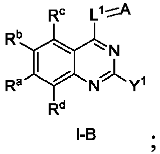
and/or, R^{b} is halogen;
and/or, R^{c} is H;
and/or, R^{d} is halogen;
and/or, " 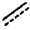 " in 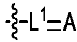 is a single bond;
and/or, 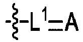 is 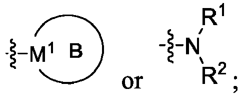
and/or, M¹ is N or CH;
and/or, ring B is unsubstituted 4- to 10-membered heterocycloalkyl with 1 heteroatom being N, unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring; preferably, ring B is unsubstituted 7- to 12-membered bridged heterocycloalkyl with 2 heteroatoms being N, unsubstituted 7- to 12-membered spiro heterocycloalkyl with 2 heteroatoms being N, or unsubstituted 7-12 membered fused heterocycloalkyl with 2 heteroatoms being N; one N atom in the 7- to 12-membered bridged heterocycloalkyl, 7- to 12-membered spiro heterocycloalkyl or 7- to 12-membered fused heterocycloalkyl is attached to the quinazoline ring;
and/or, R¹ is H or C₁-C₃ alkyl;
and/or, R² is -(CH₂)ₙ-R₂ₐ;
and/or, n is 1 or 2;
and/or, R^{2a} is 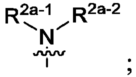
and/or, R^{2a-1} and R^{2a-2} are independently H or C₁-C₃ alkyl, and R^{2a-1} and R^{2a-2} are not C₁-C₃ alkyl at the same time.

15. The quinazoline compound represented by formula I, the pharmaceutically acceptable salt thereof, the solvate thereof, the prodrug thereof, the metabolite thereof or the isotopic compound thereof according to claim 13, wherein, 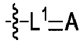
is 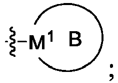
and/or, ring B is unsubstituted 7- to 12-membered bridged heterocycloalkyl with 2 heteroatoms being N, or unsubstituted 7- to 12-membered spiro heterocycloalkyl with 2 heteroatoms being N; one N atom in the 7- to 12-membered bridged heterocycloalkyl or 7- to 12-membered membered spiro heterocycloalkyl is attached to the quinazoline ring;
preferably, the 7- to 12-membered bridged heterocycloalkyl with 2 heteroatoms being N is 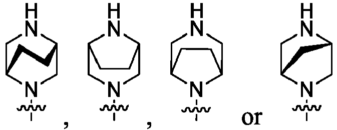 ; the 7- to 12-membered spiro heterocycloalkyl with 2 heteroatoms being N wherein one ring is a 4-membered heterocycloalkyl with 1 atom being N, and the N atom in the 4-membered heterocycloalkyl is attached to the quinazoline ring;
and/or, R^{2a-1} and R^{2a-2} are H.

16. The quinazoline compound represented by formula I, the pharmaceutically acceptable salt thereof, the solvate thereof, the prodrug thereof, the metabolite thereof or the isotopic compound thereof according to any one of claims 1 to 15, wherein, the quinazoline compound represented by formula I or the pharmaceutically acceptable salt thereof is any of the following structures: 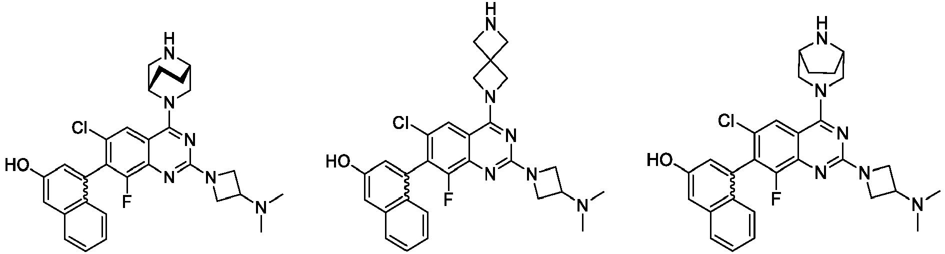 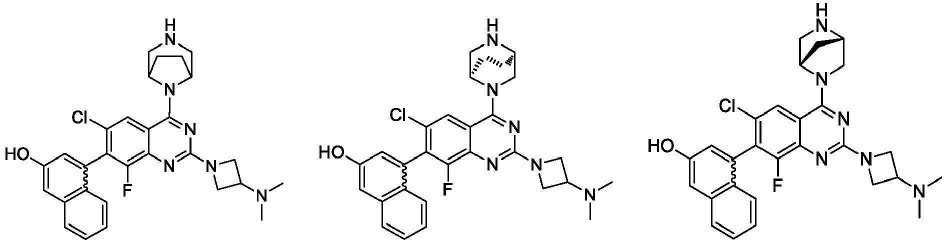 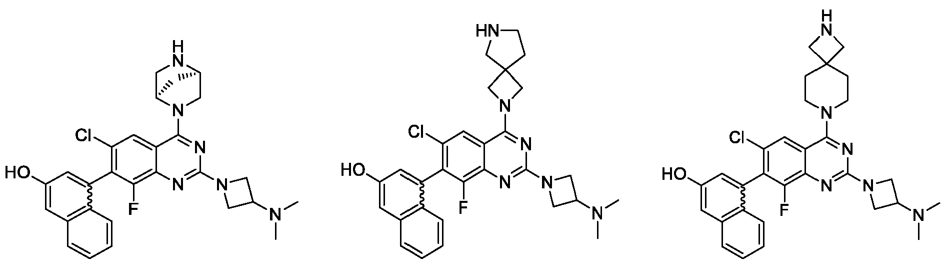 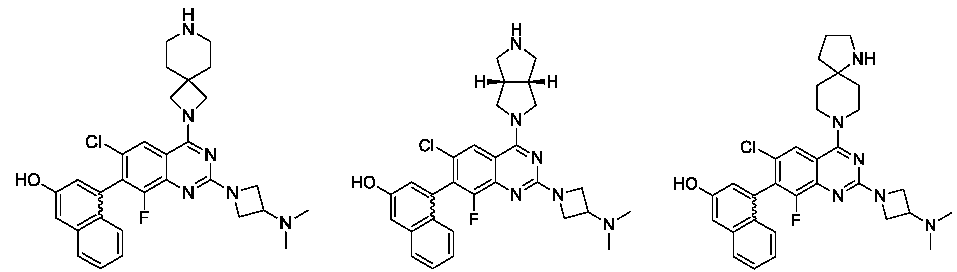 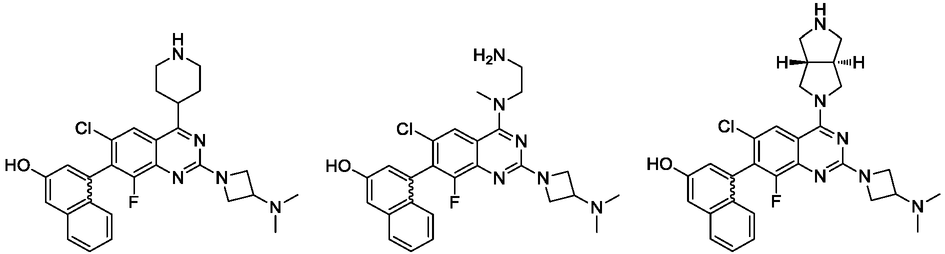 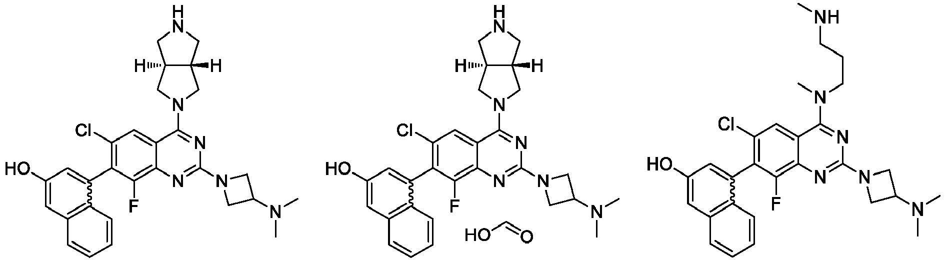 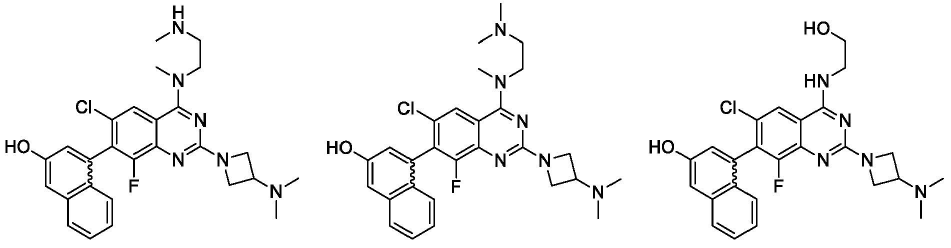 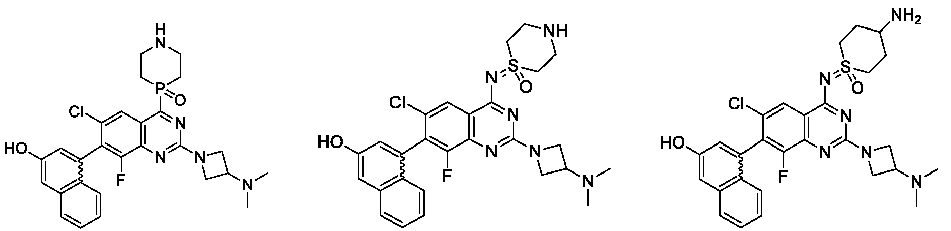 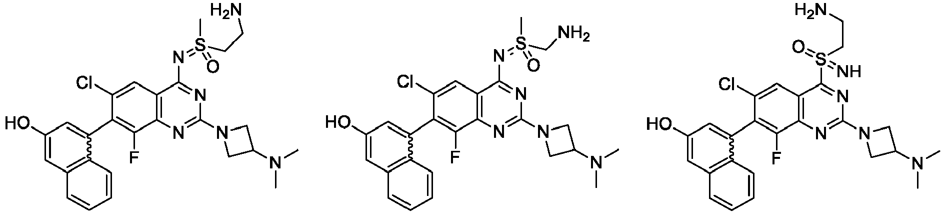 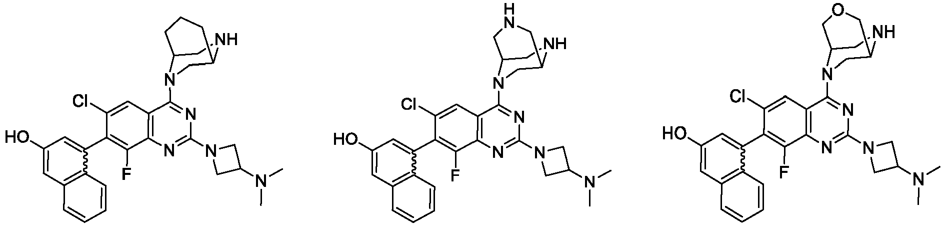 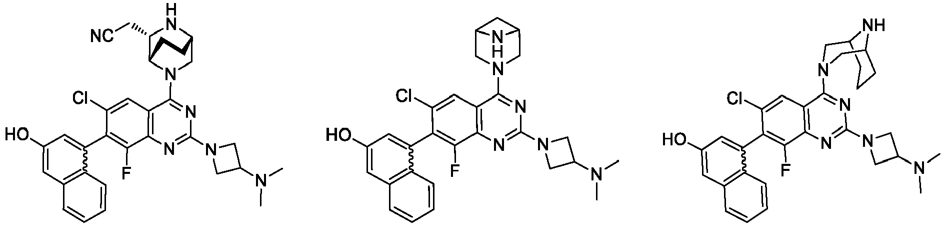 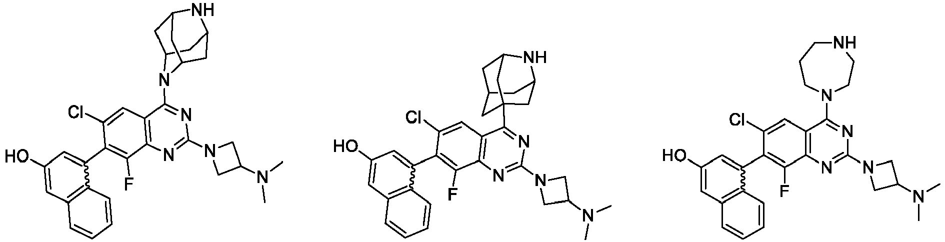 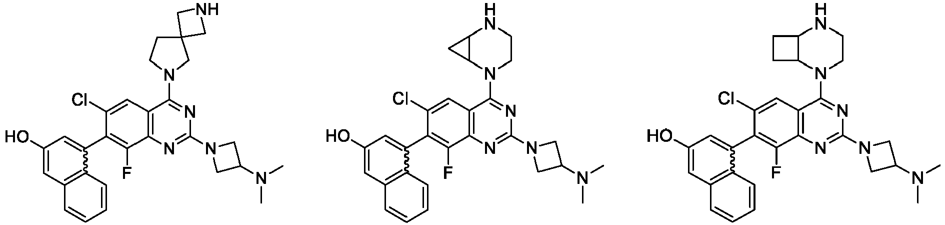 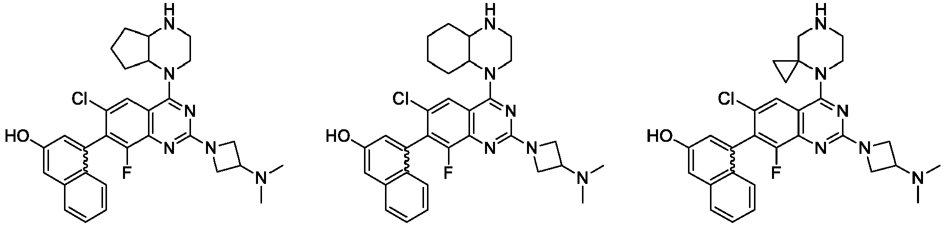 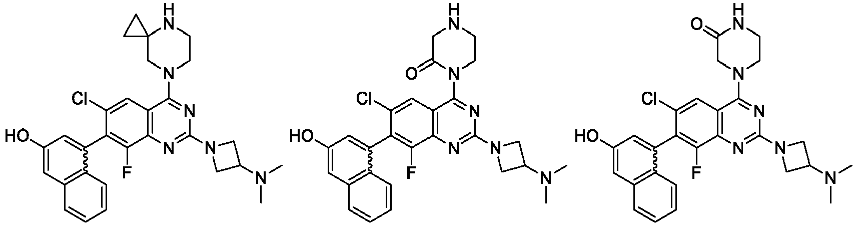 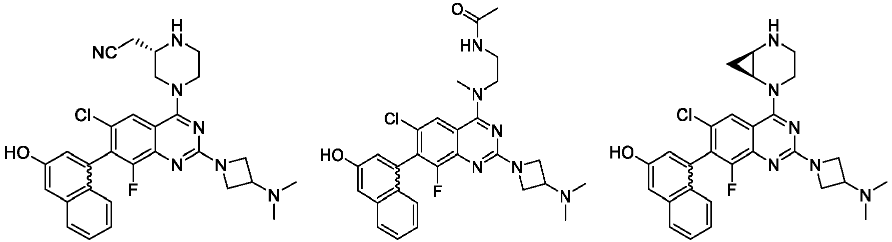 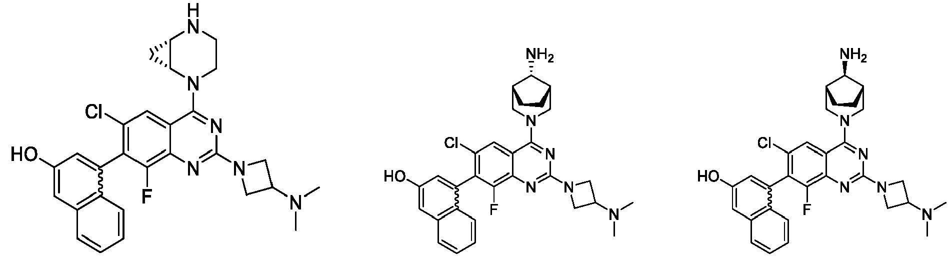 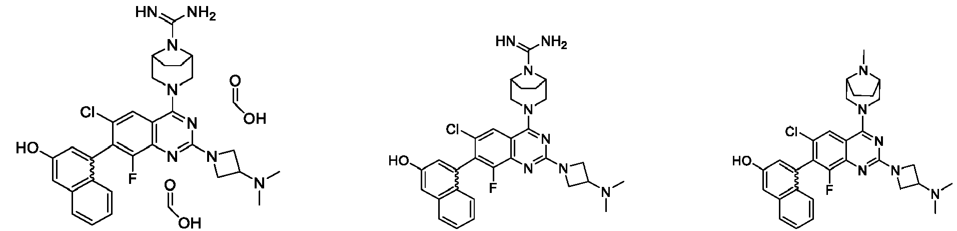 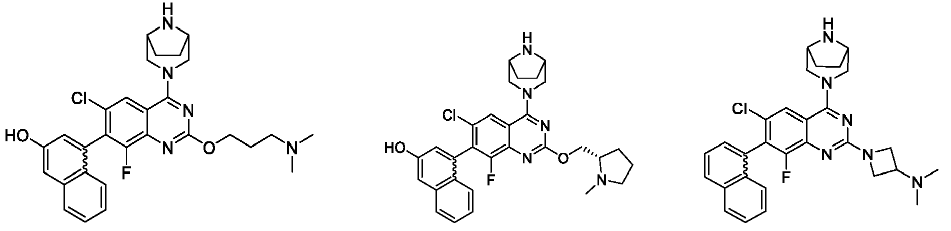 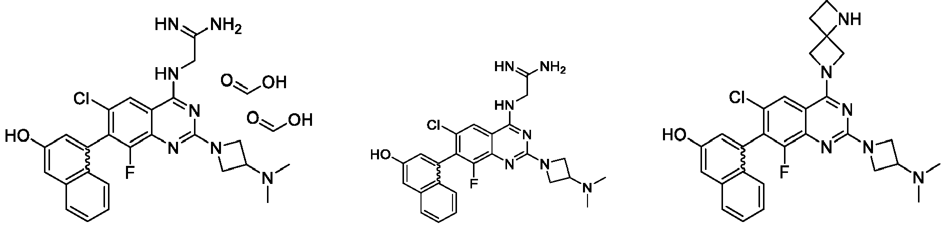 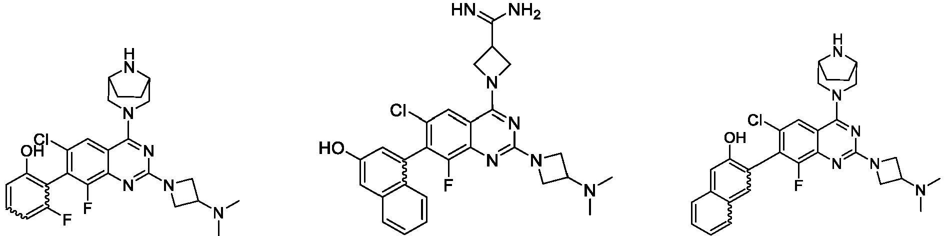 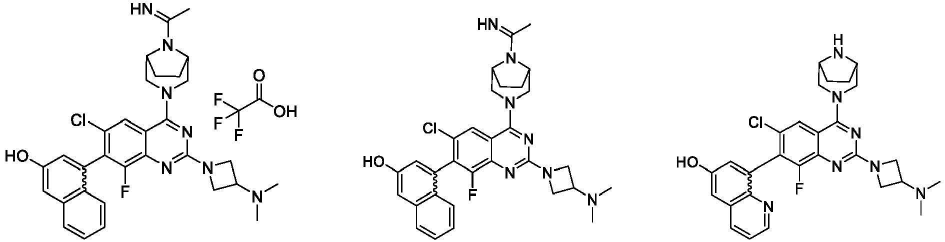 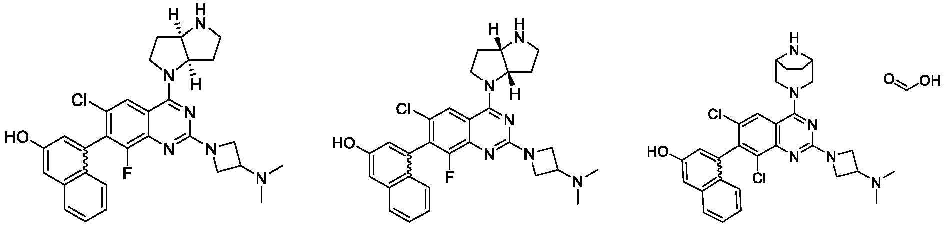 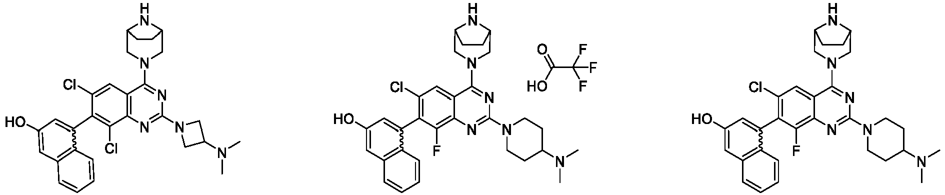 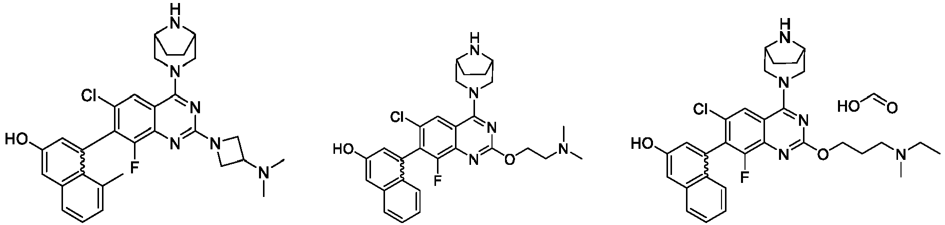 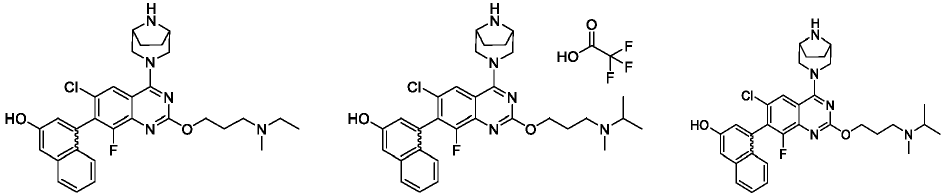 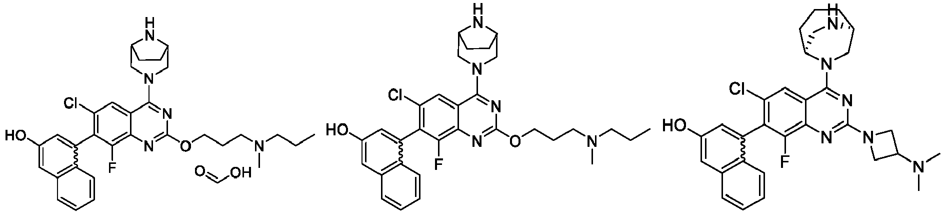 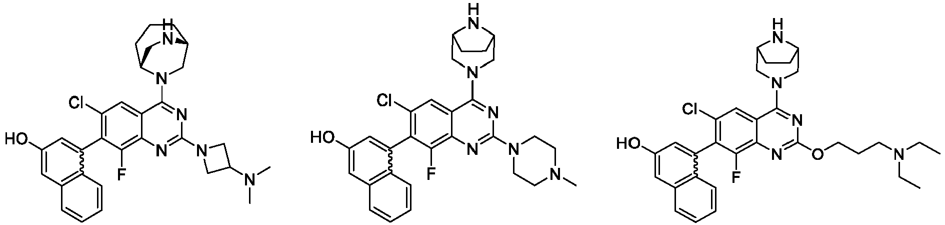 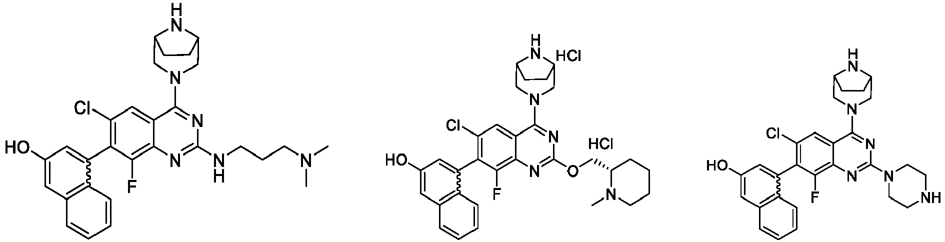 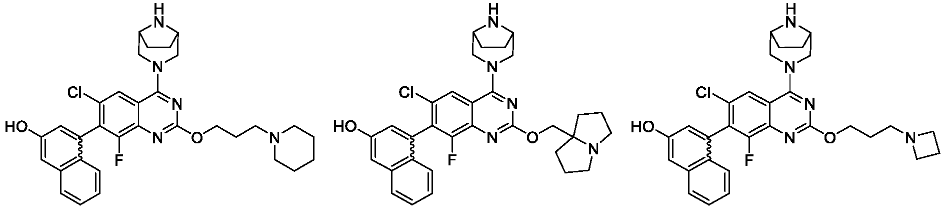 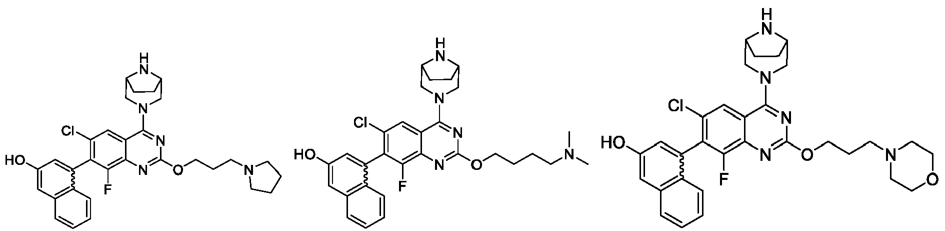 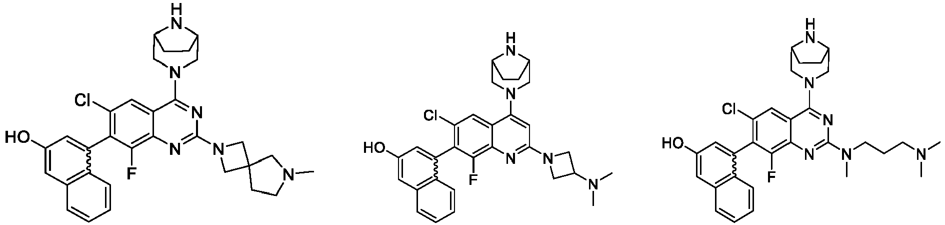 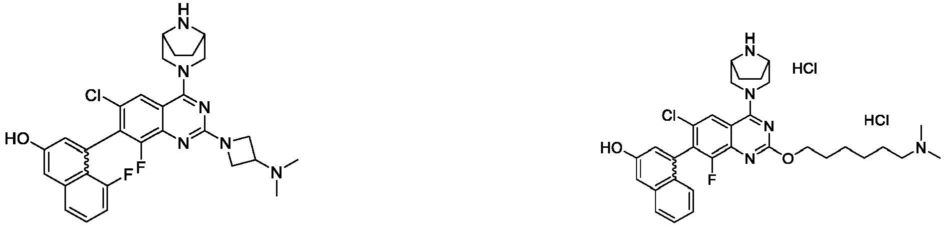 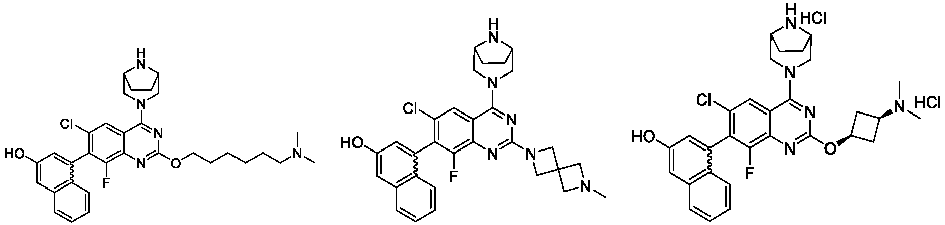 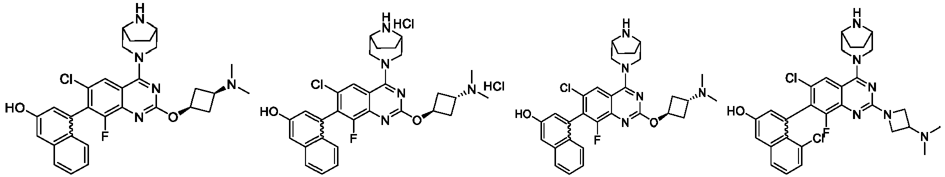 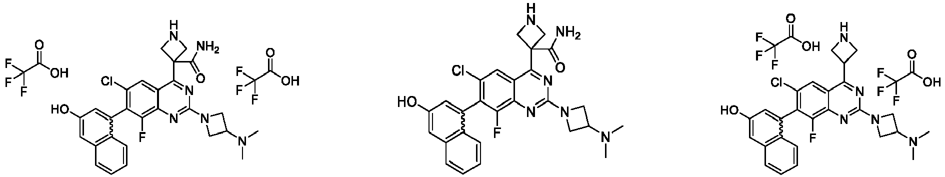 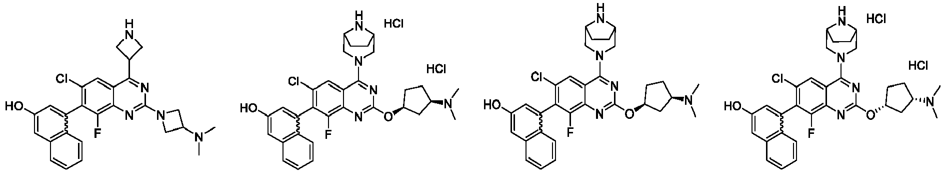 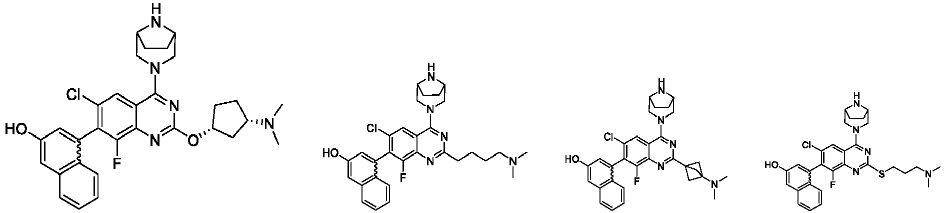 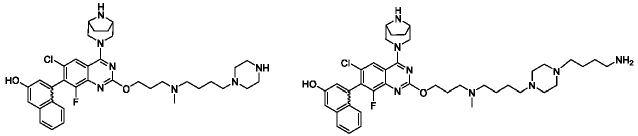 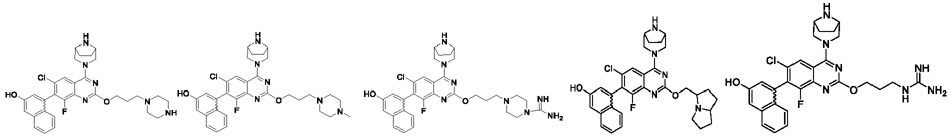 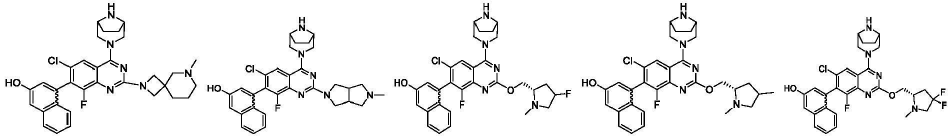 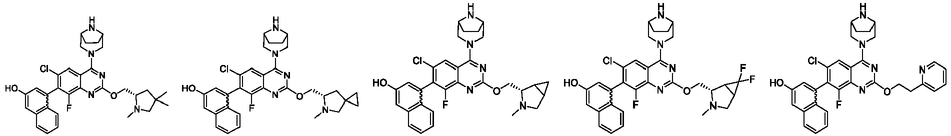 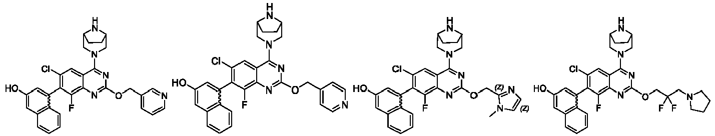 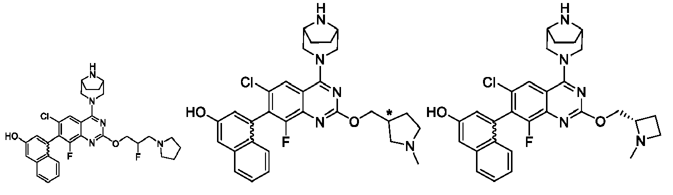 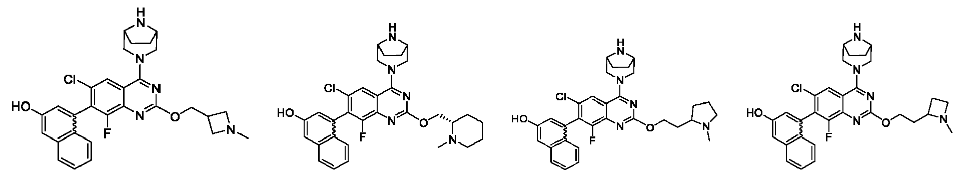 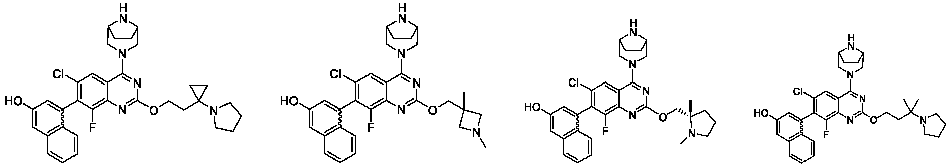 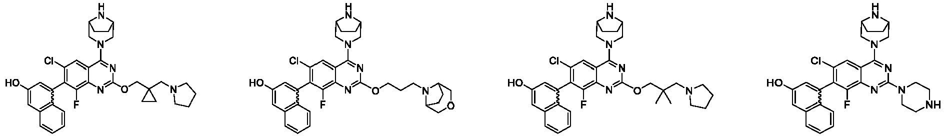 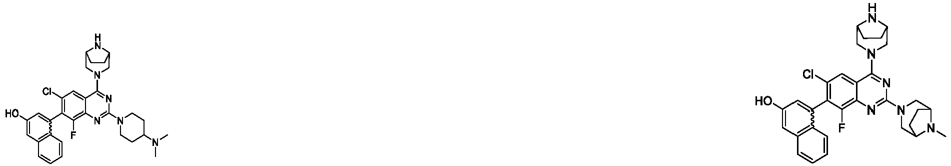 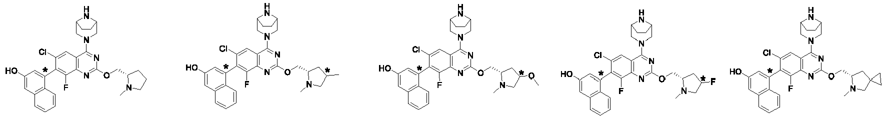 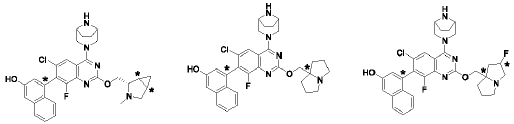 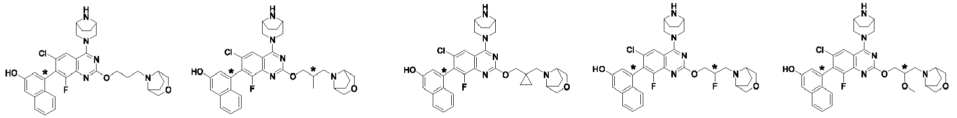 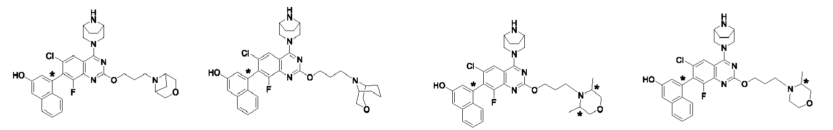 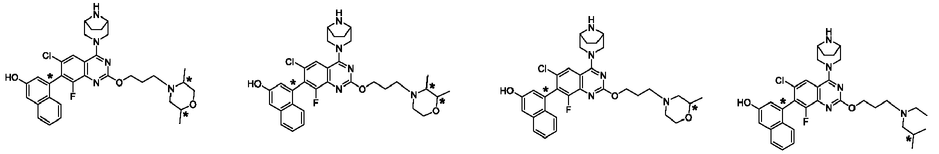 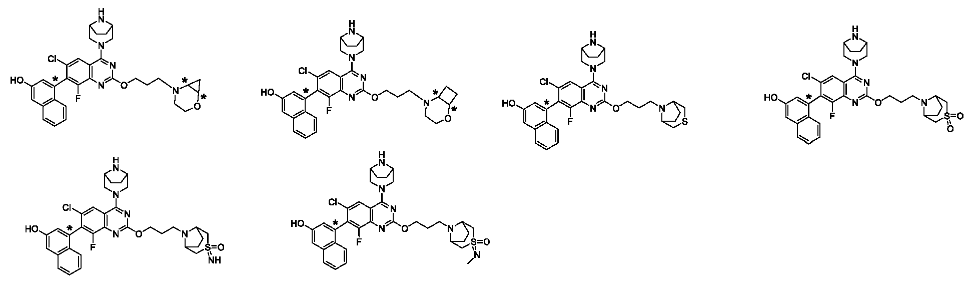 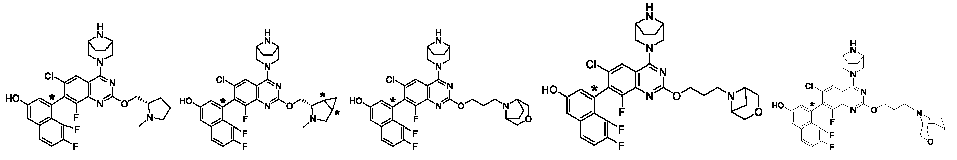 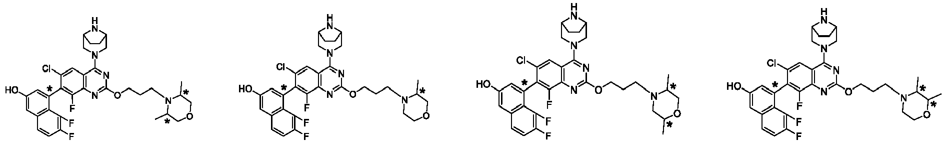 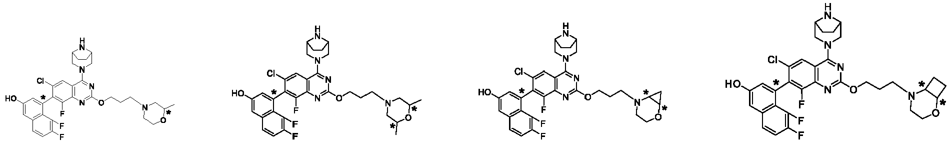 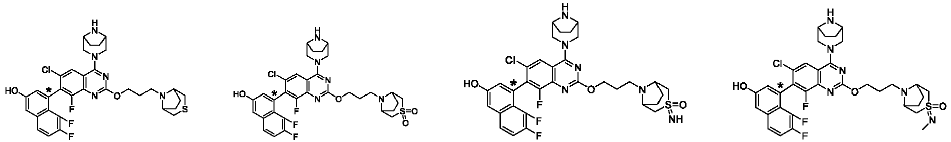 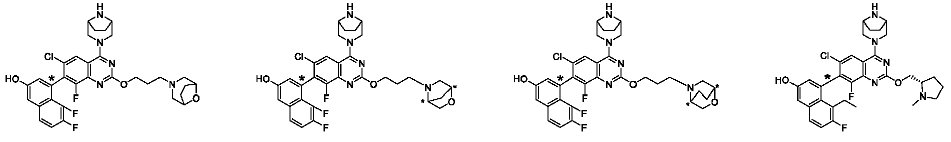 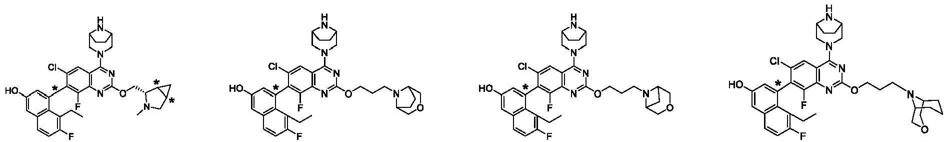 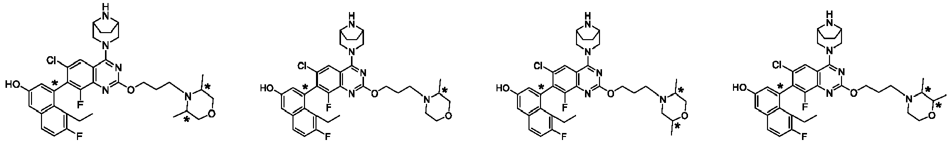 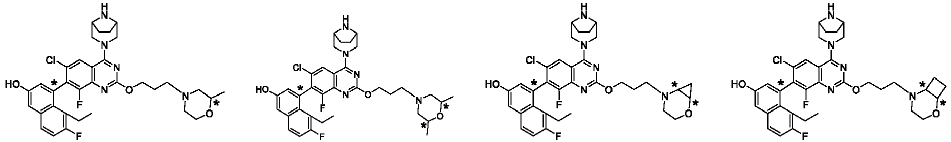 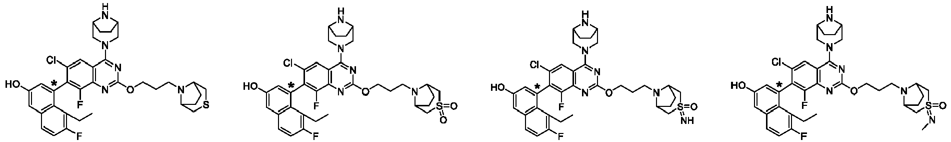 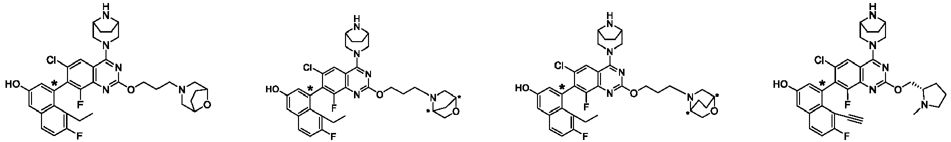 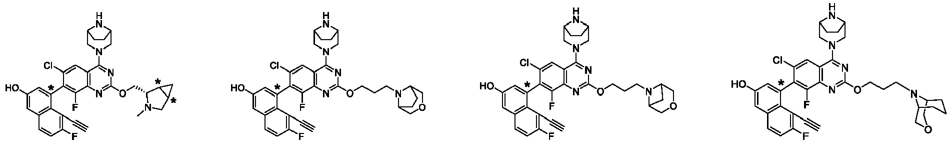 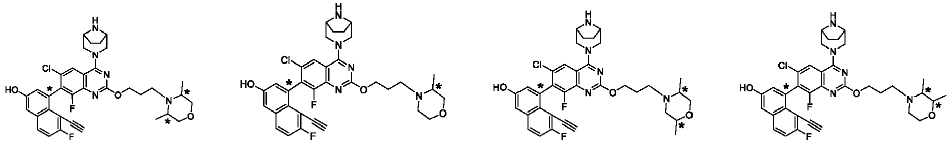 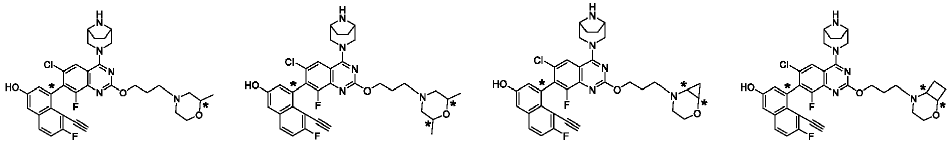 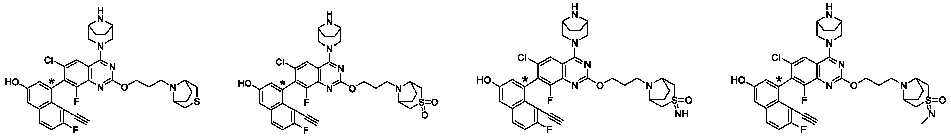 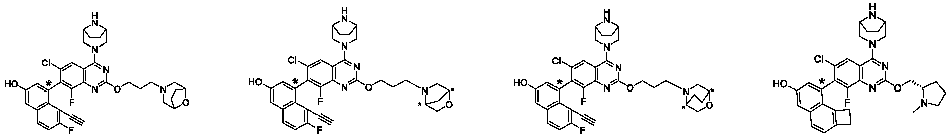 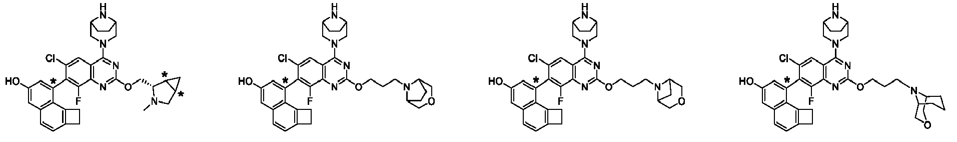 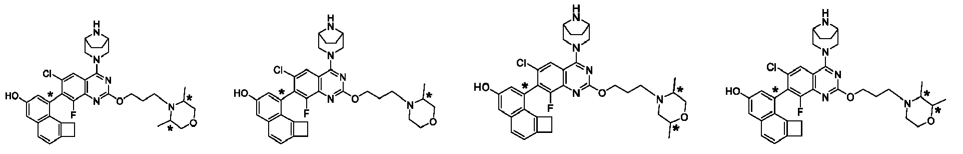 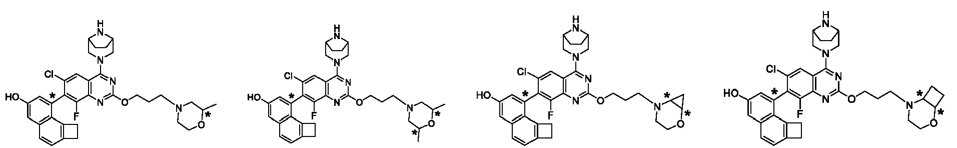 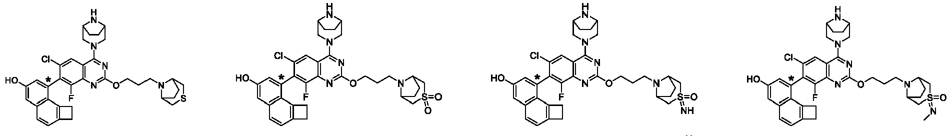 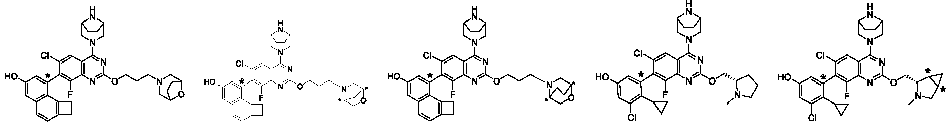 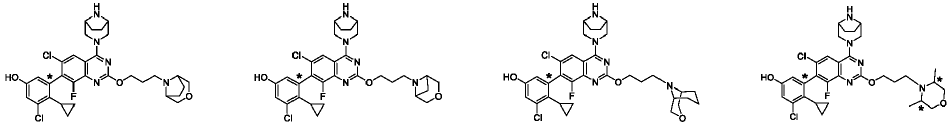 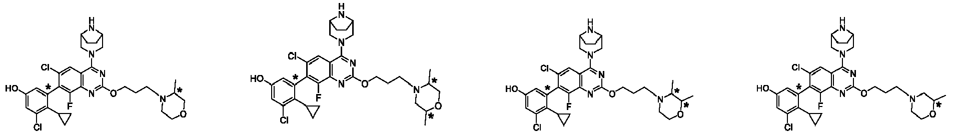 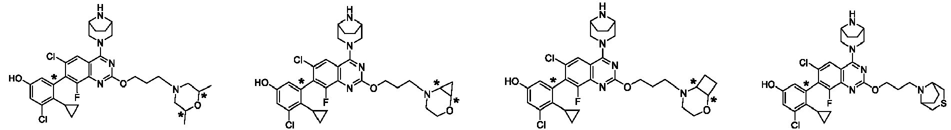 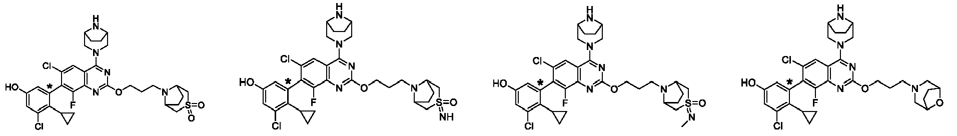 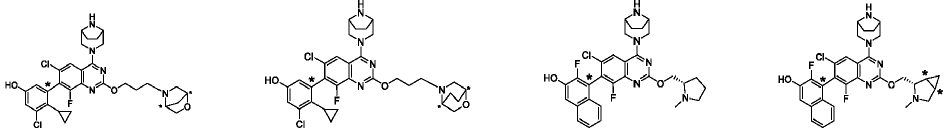 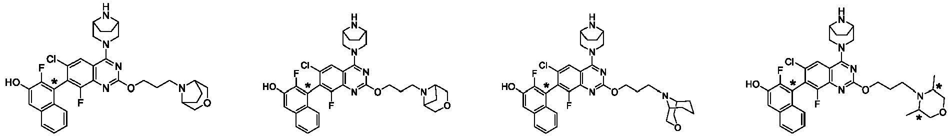 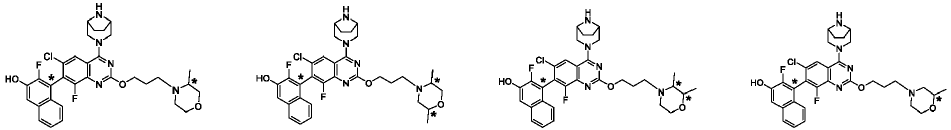 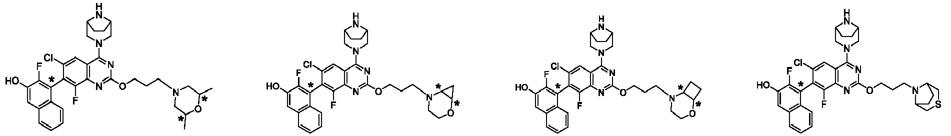 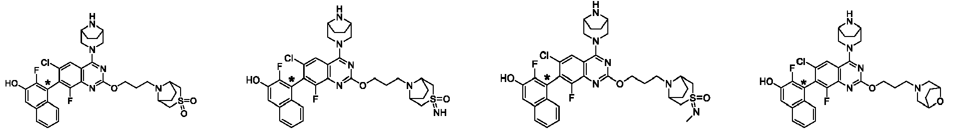 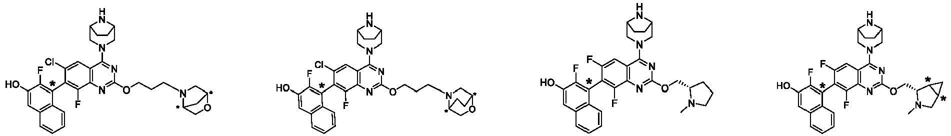 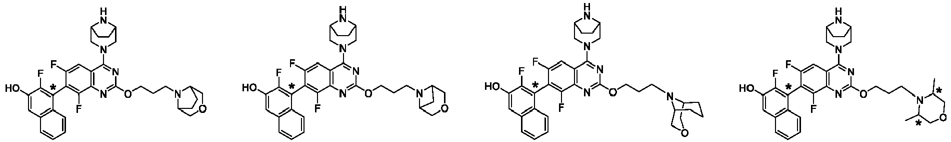 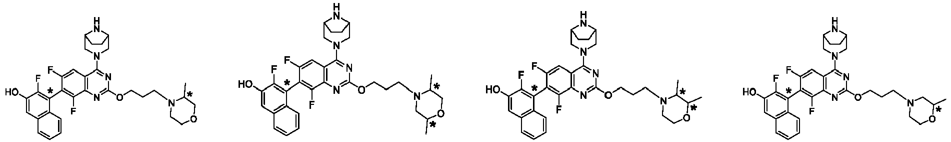 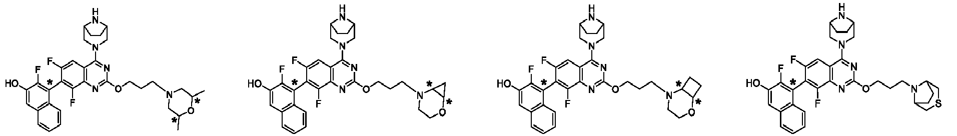 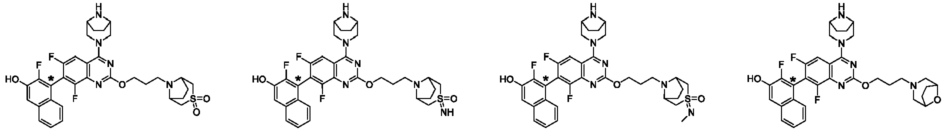 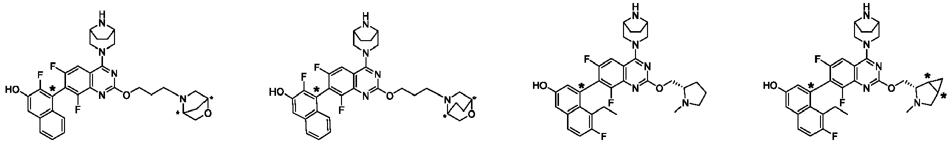 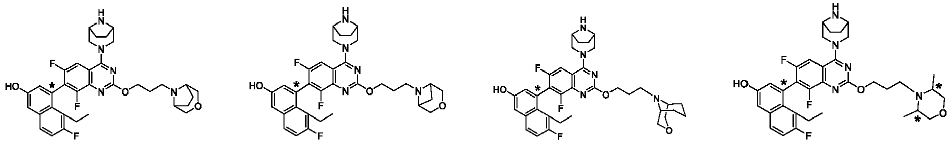 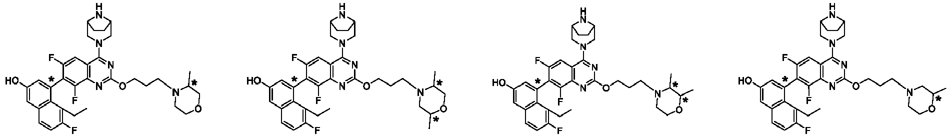 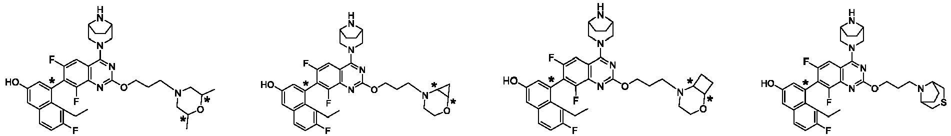 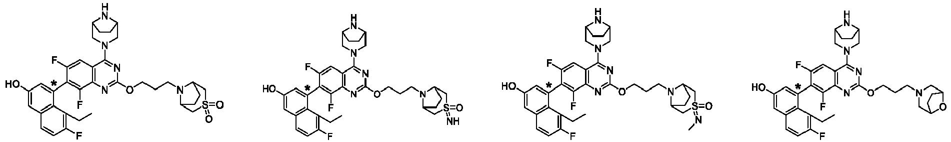 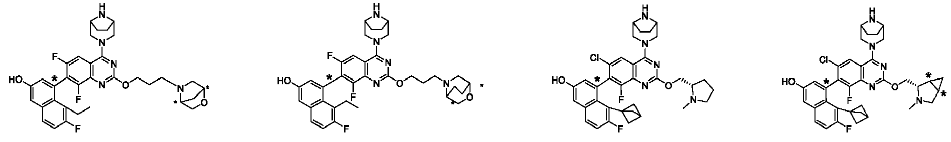 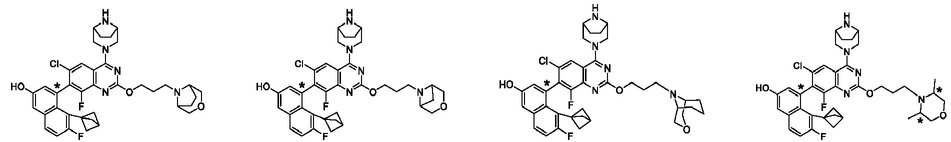 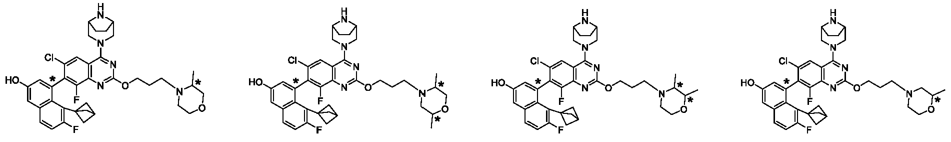 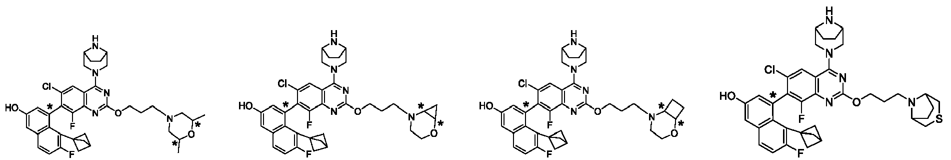 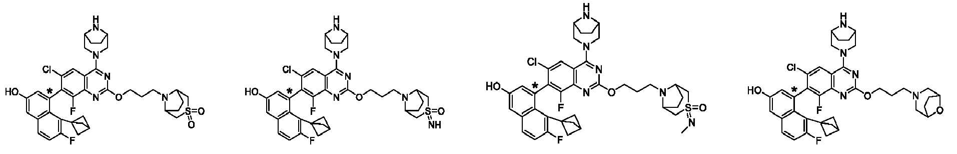 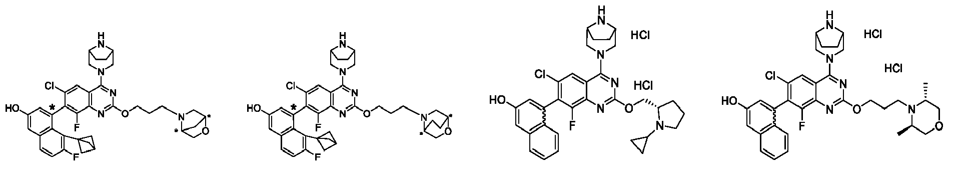 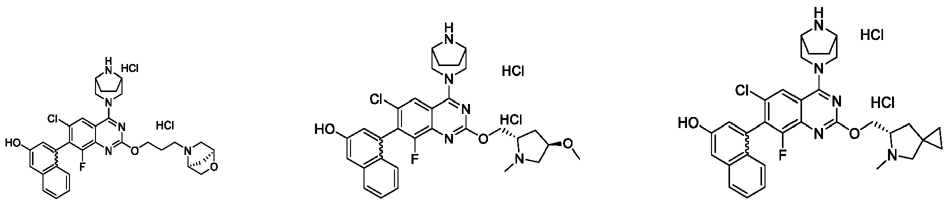 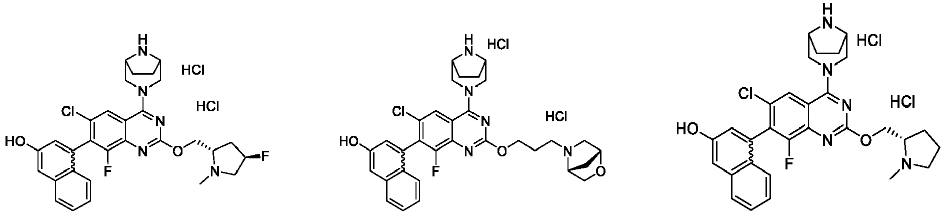 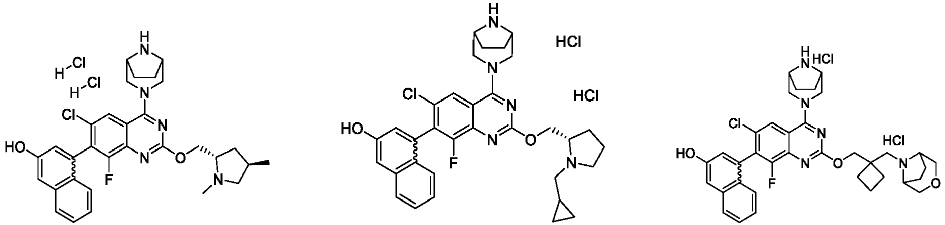 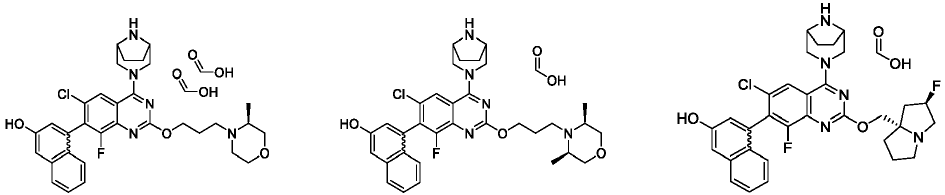 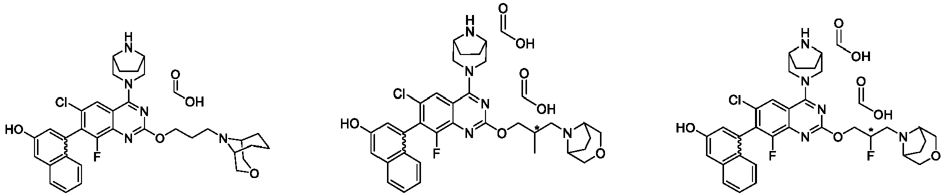 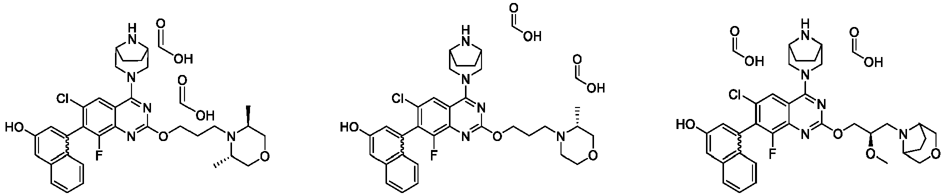 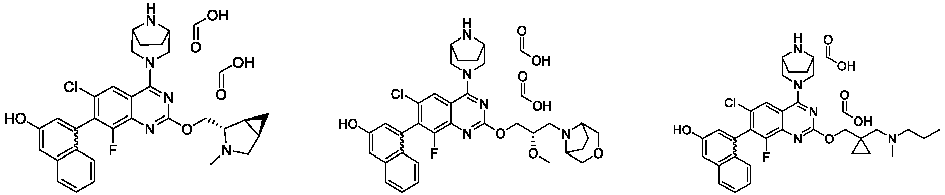 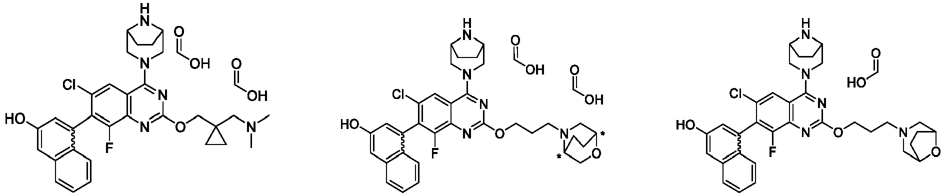 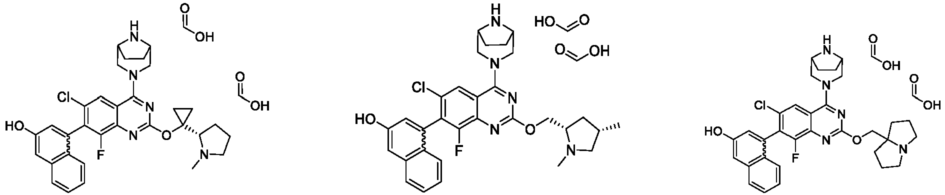 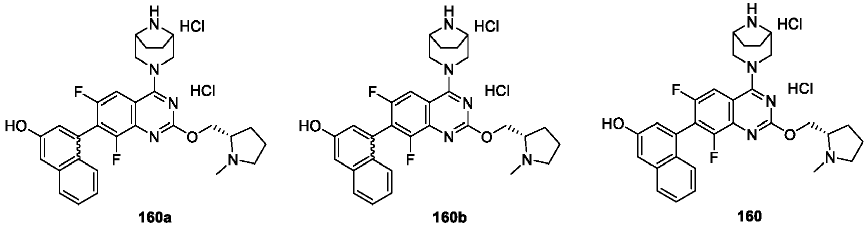 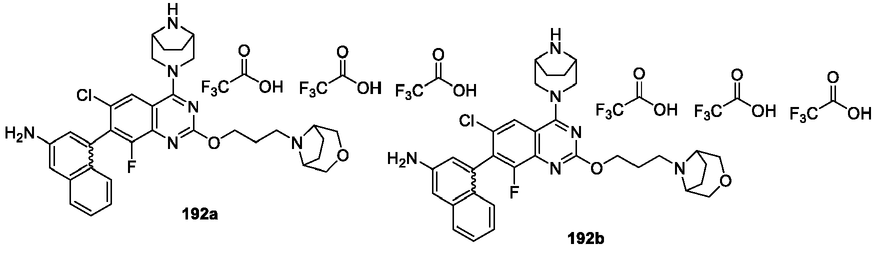 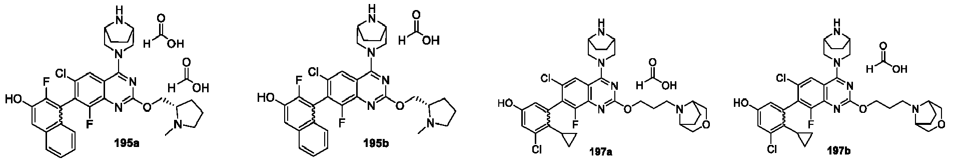 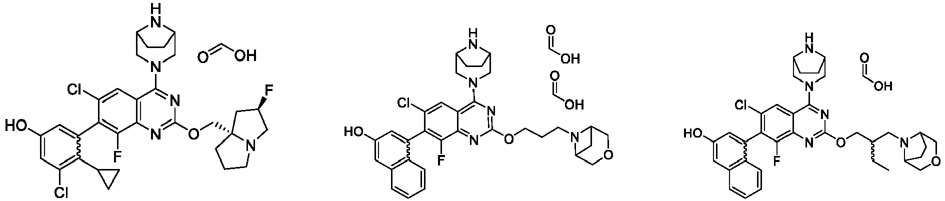 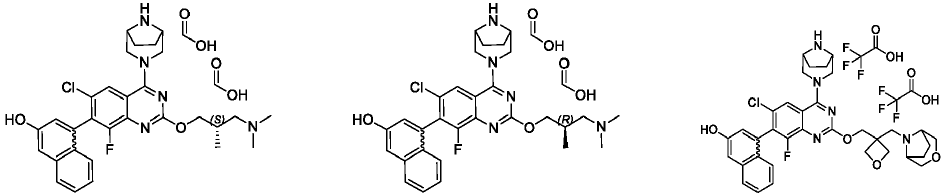 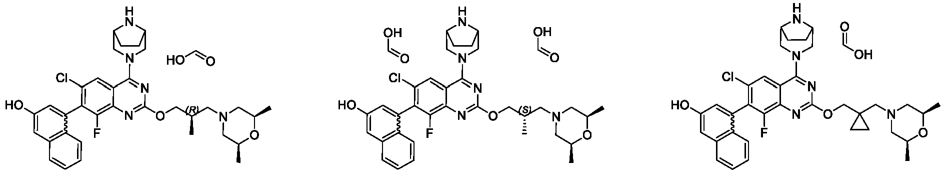 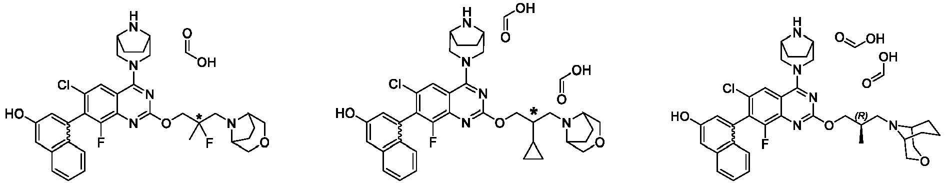 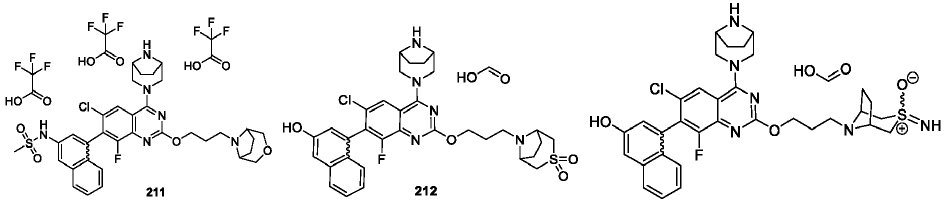 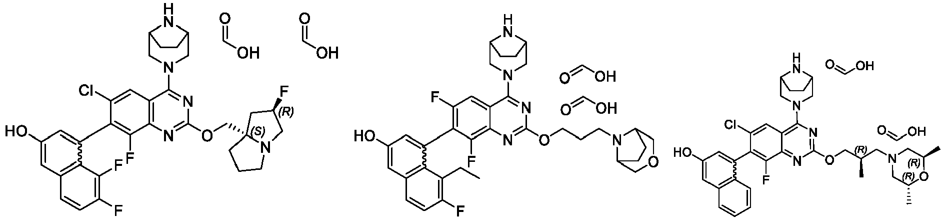 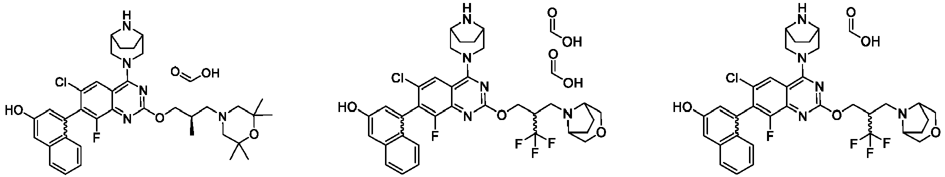 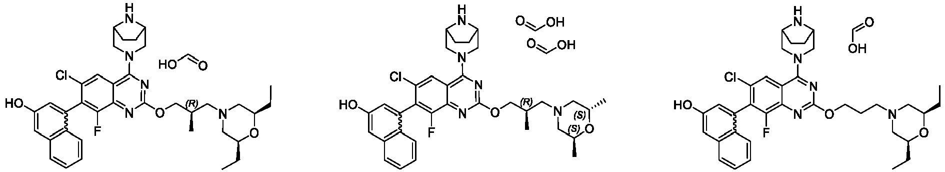 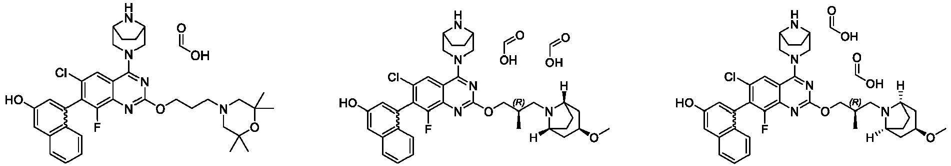 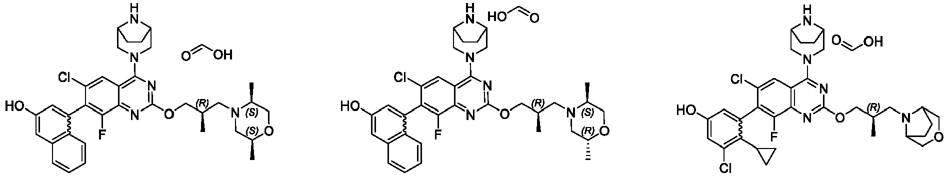 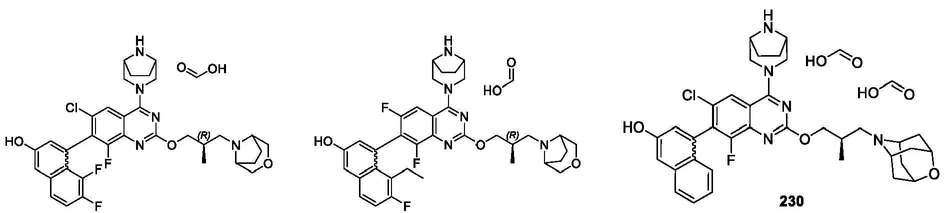 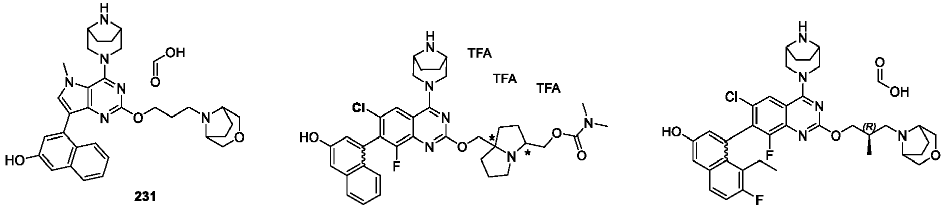 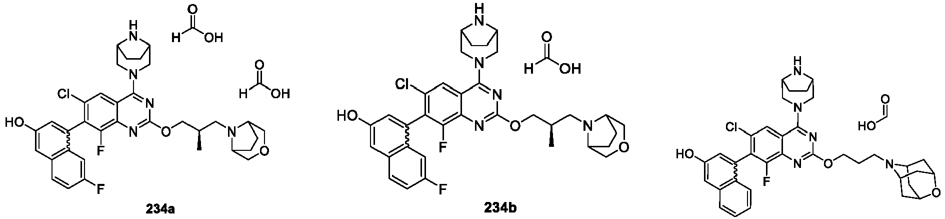 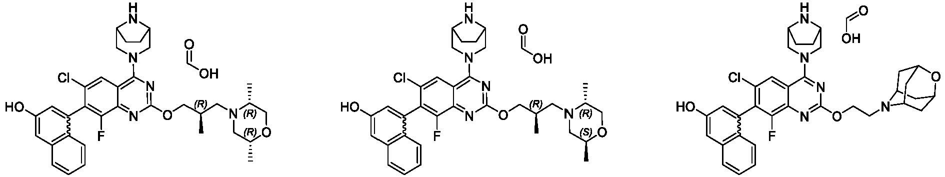 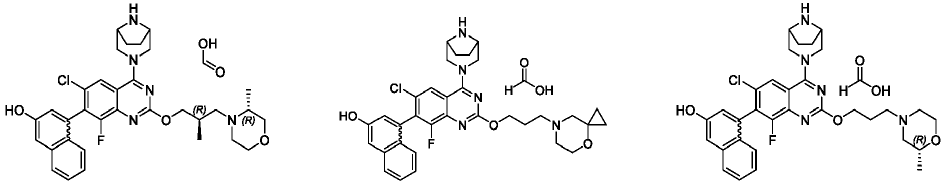 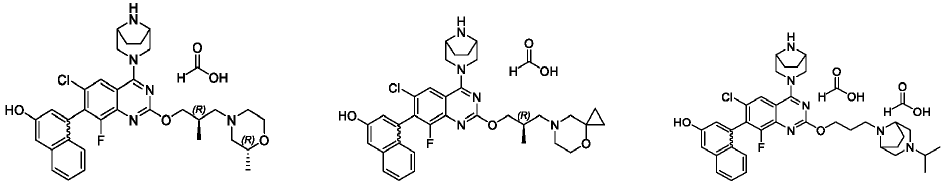 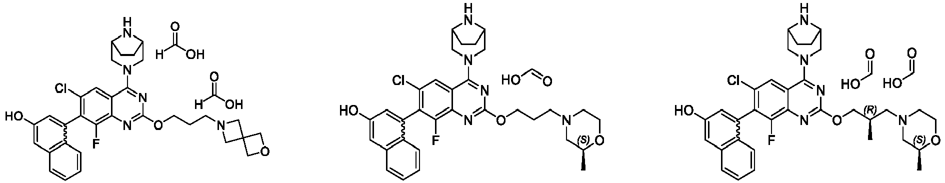 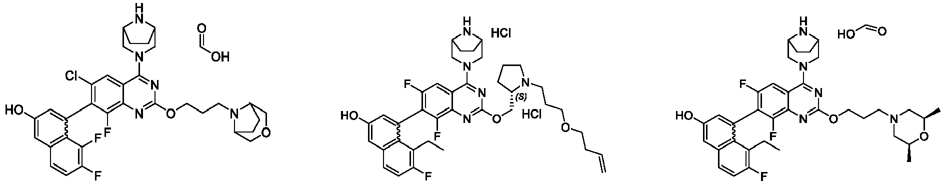 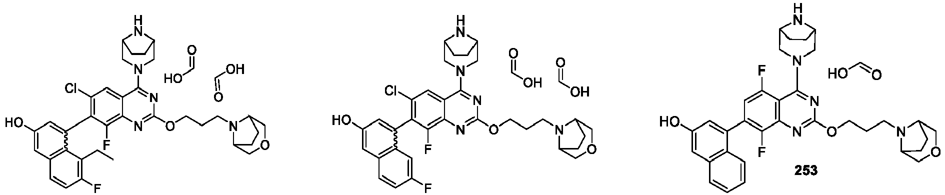 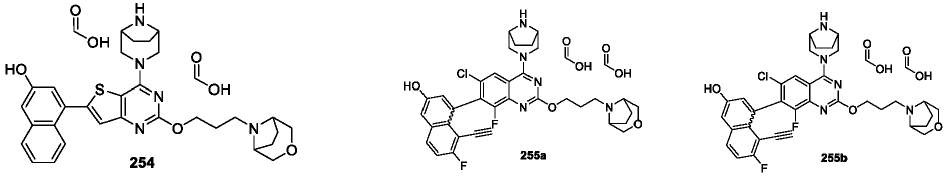 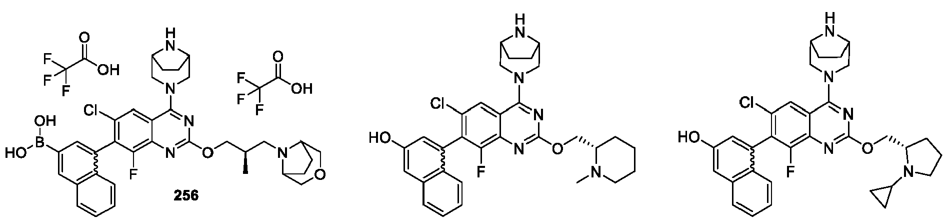 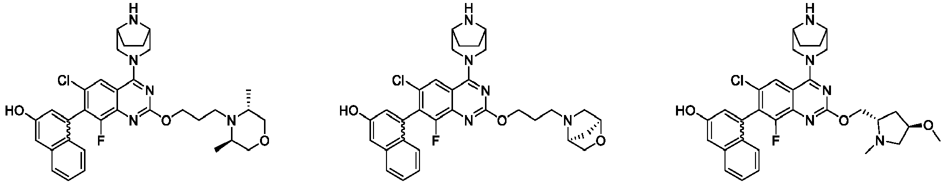 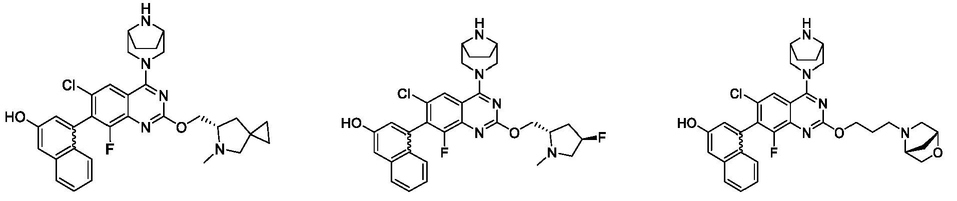 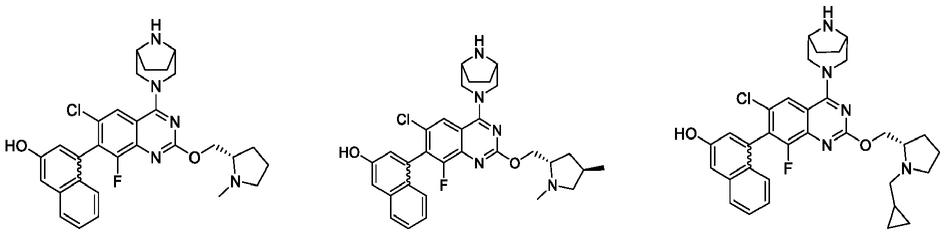 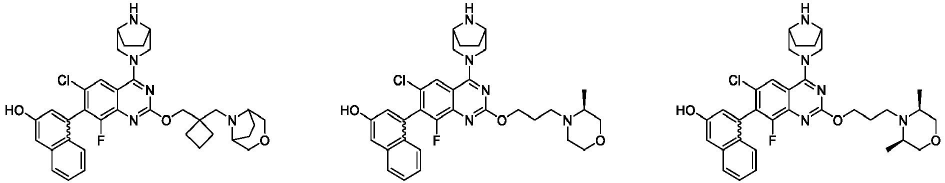 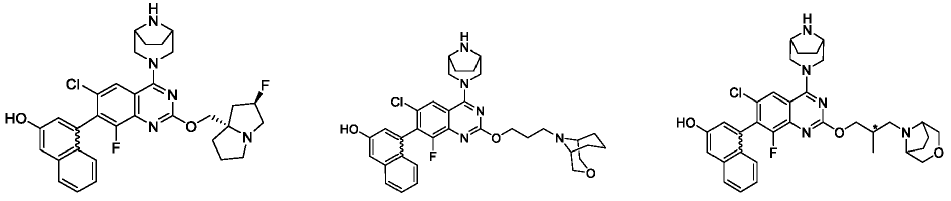 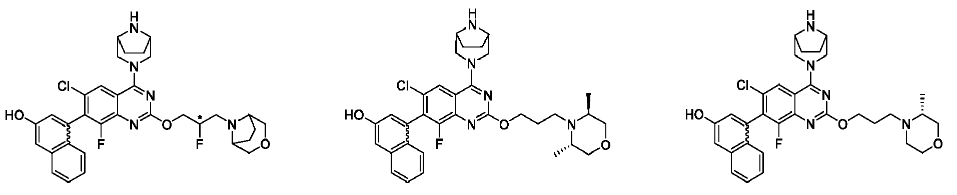 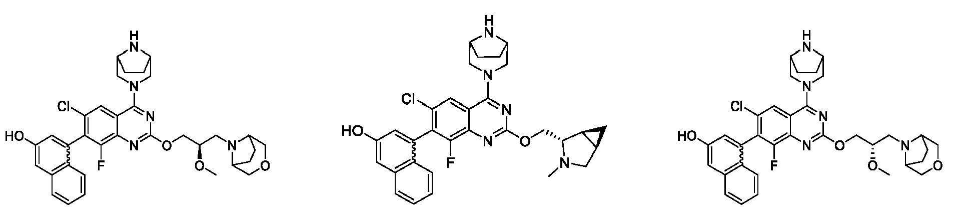 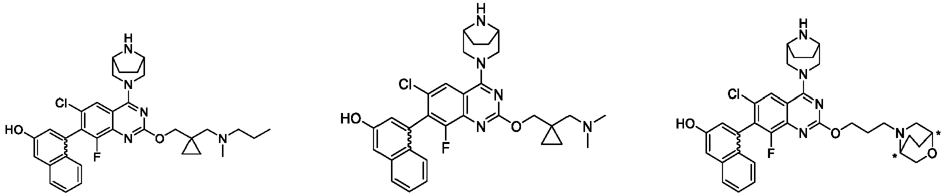 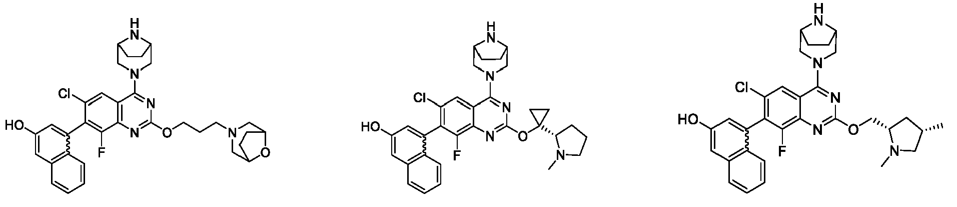 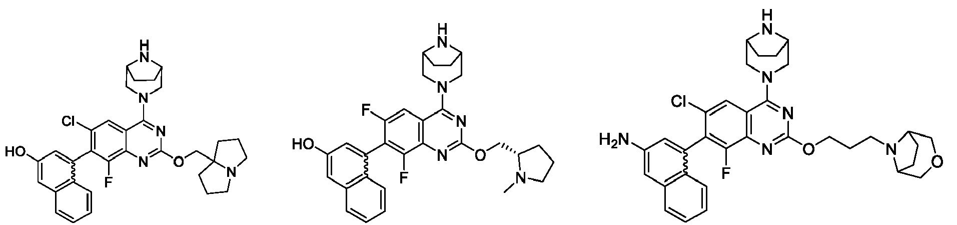 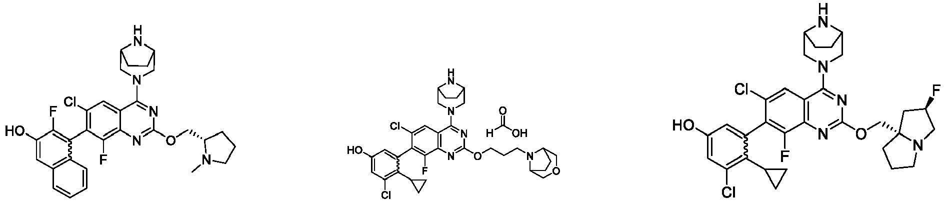 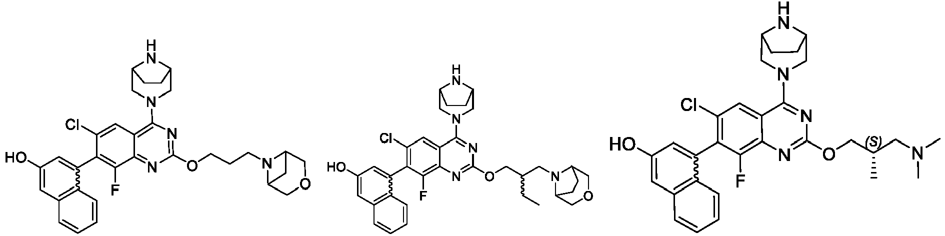 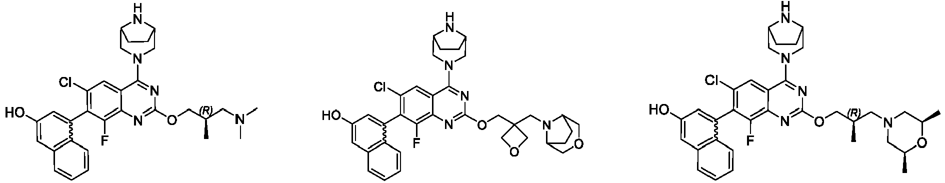 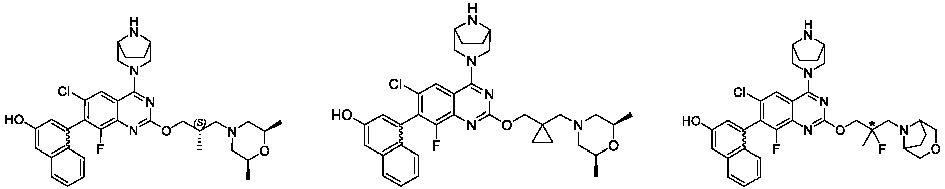 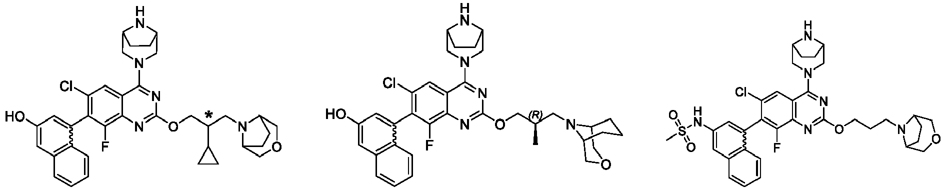 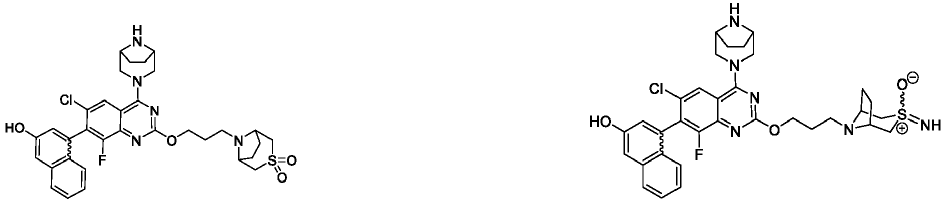 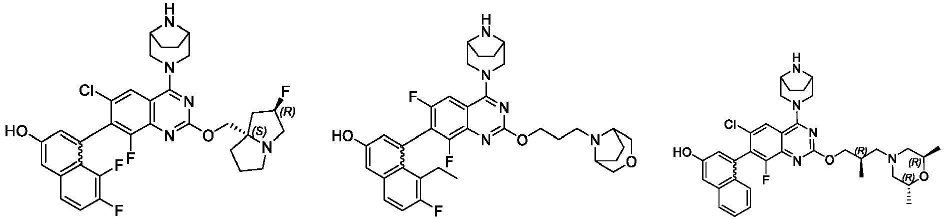 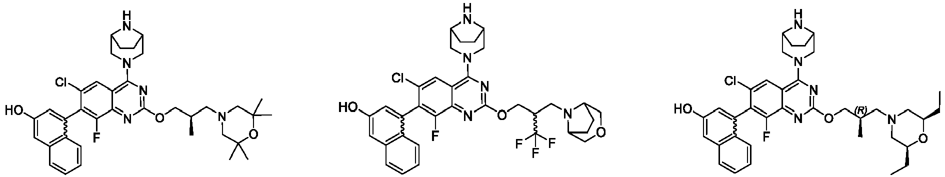 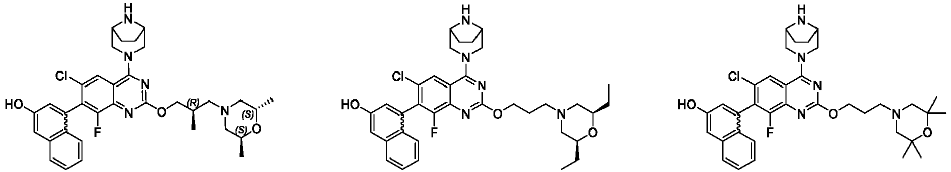 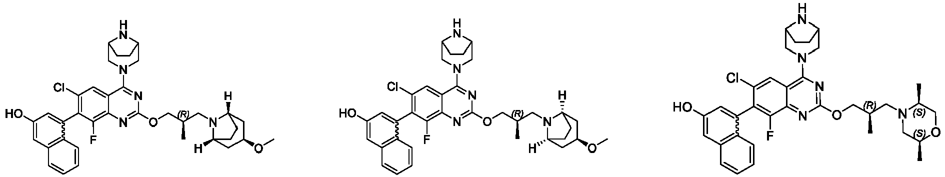 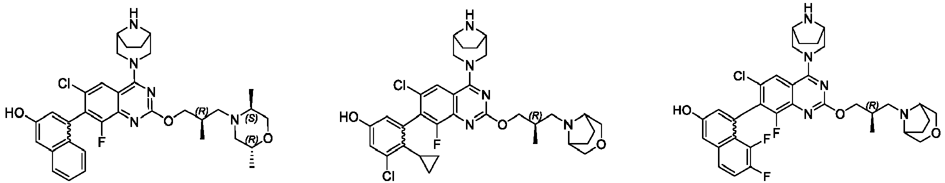 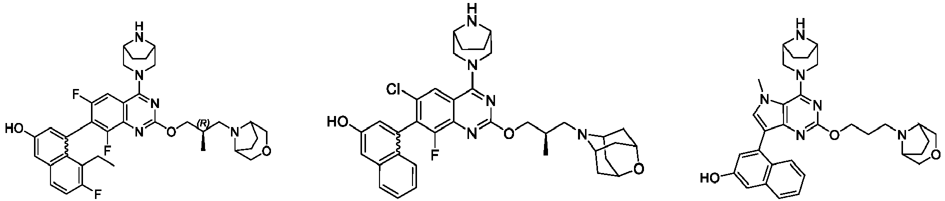 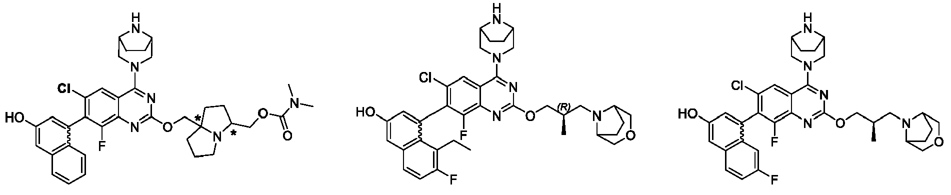 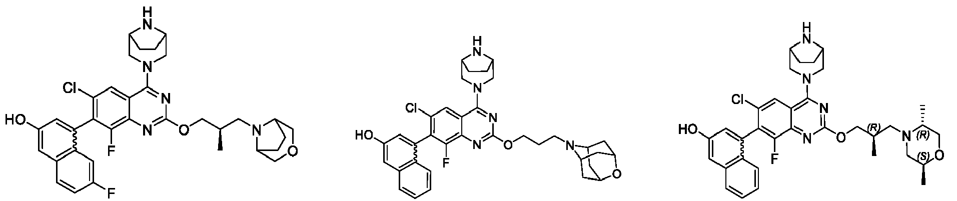 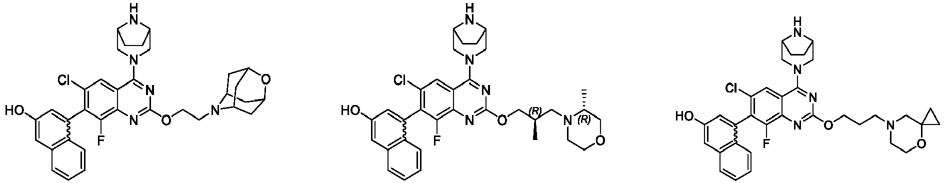 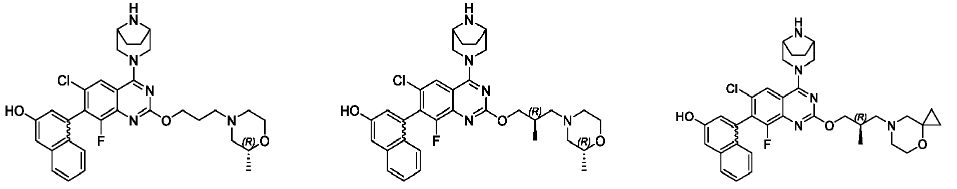 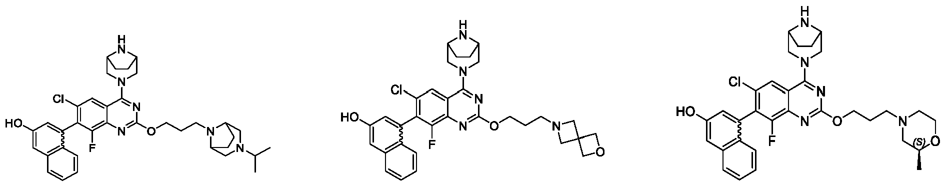 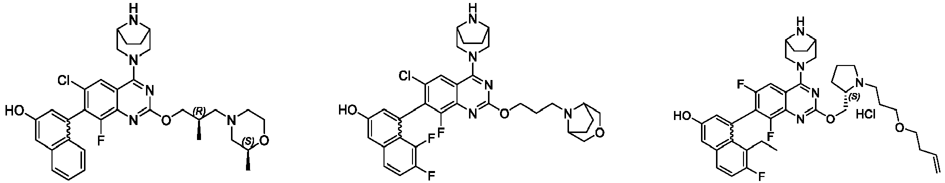 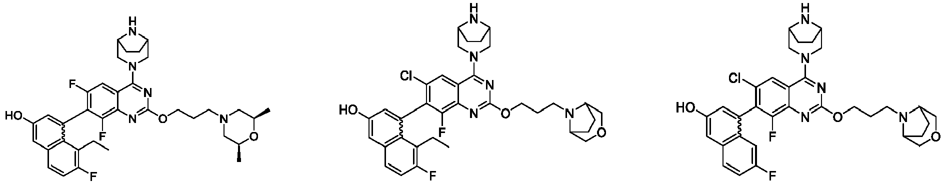 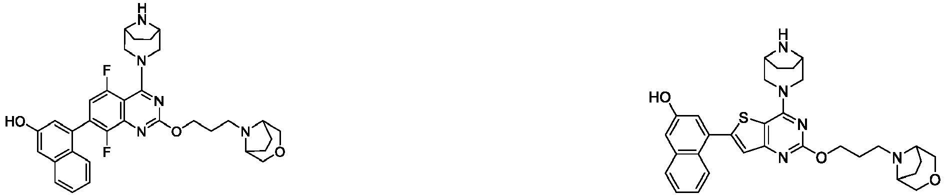 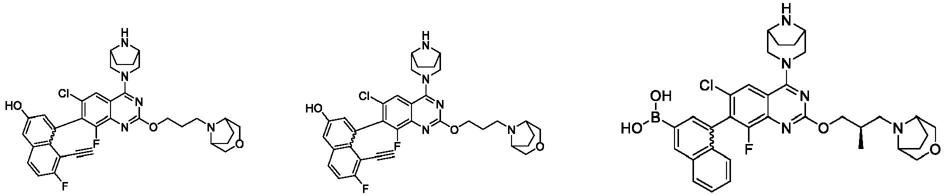 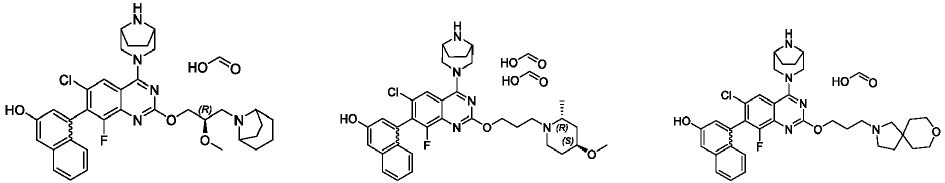 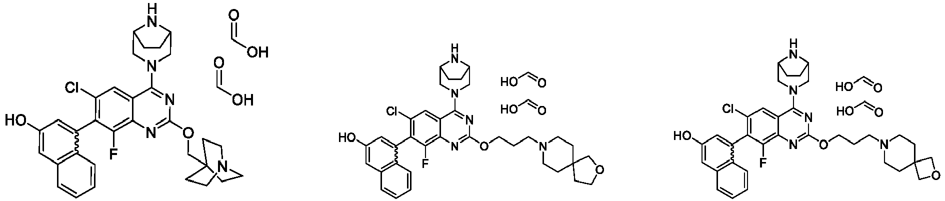 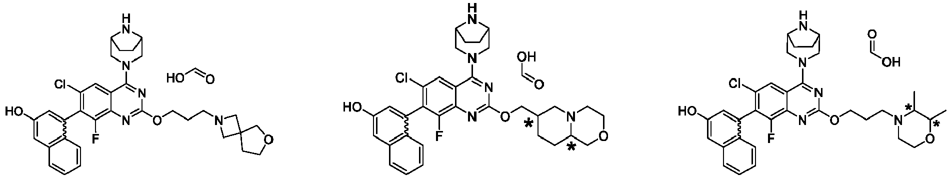 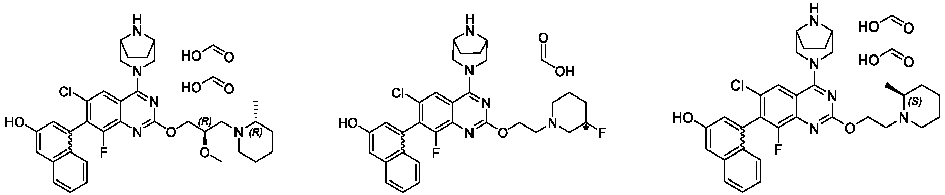 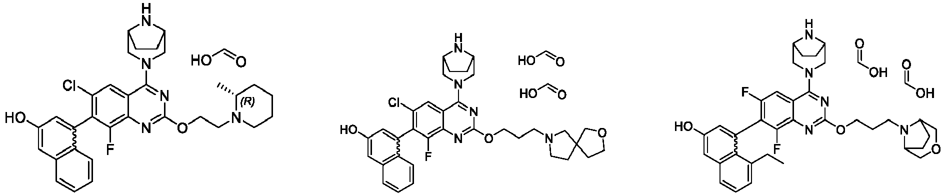 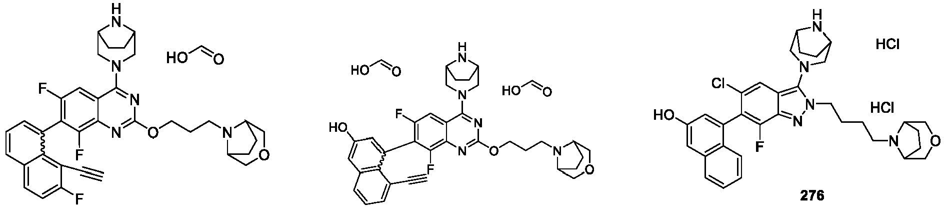 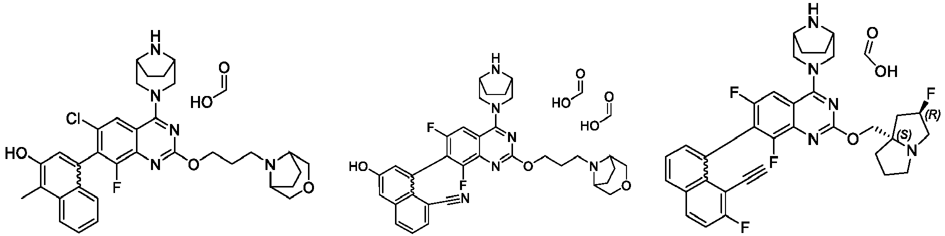 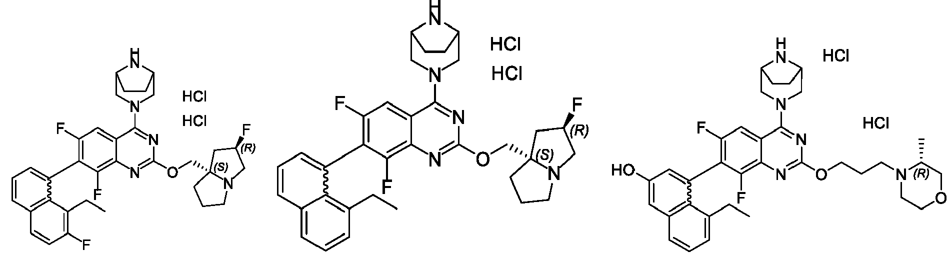 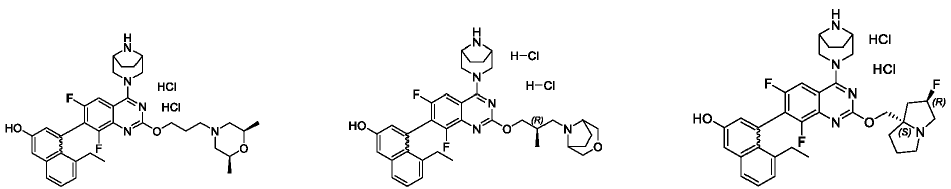 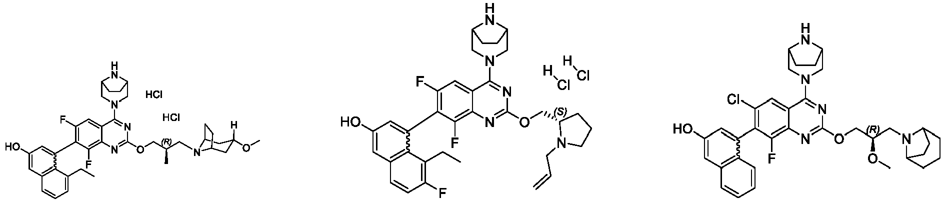 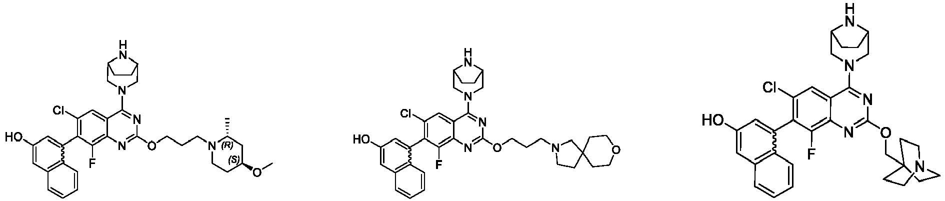 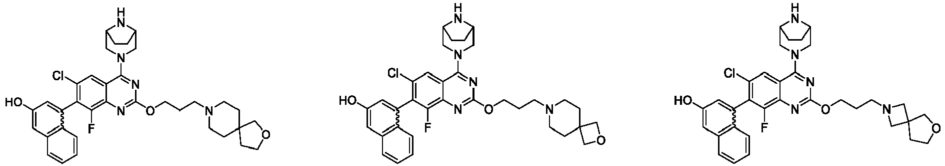 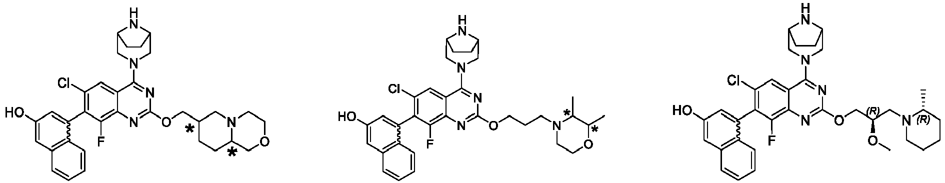 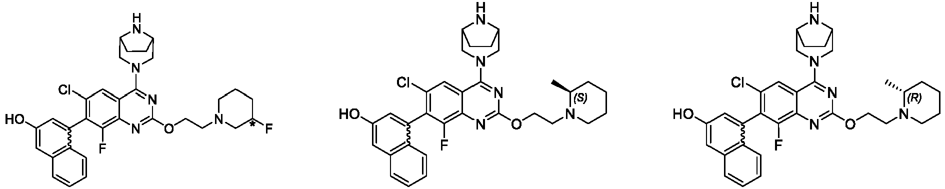 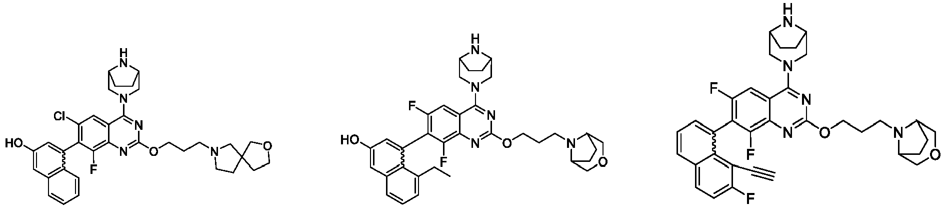 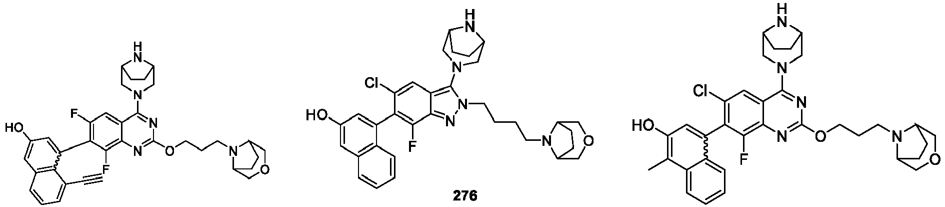 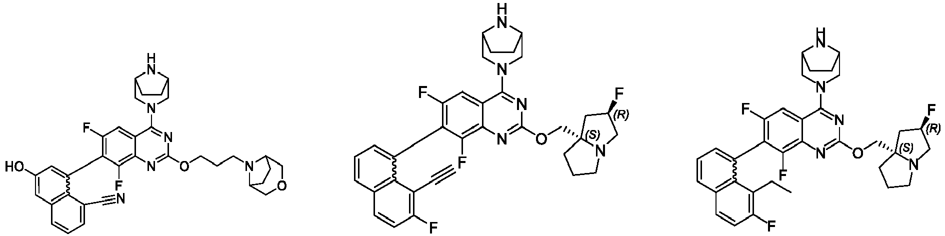 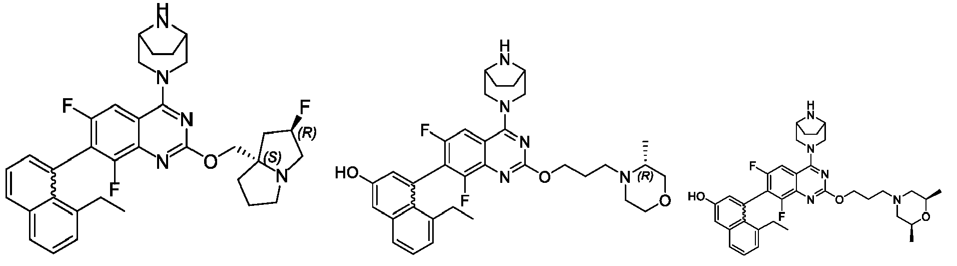 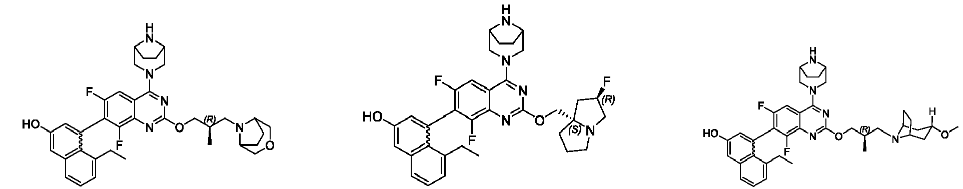 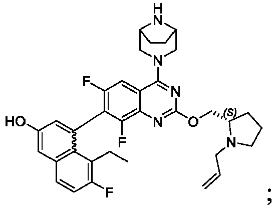 the carbon atom with "*" indicates a carbon atom with a chiral center; " 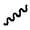 " means " 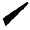 ", " 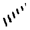 " or a mixture thereof.

17. The quinazoline compound represented by formula I, the pharmaceutically acceptable salt thereof, the solvate thereof, the prodrug thereof, the metabolite thereof or the isotopic compound thereof according to any one of claims 1 to 15, wherein, the quinazoline compound represented by formula I or the pharmaceutically acceptable salt thereof is any of the following structures:

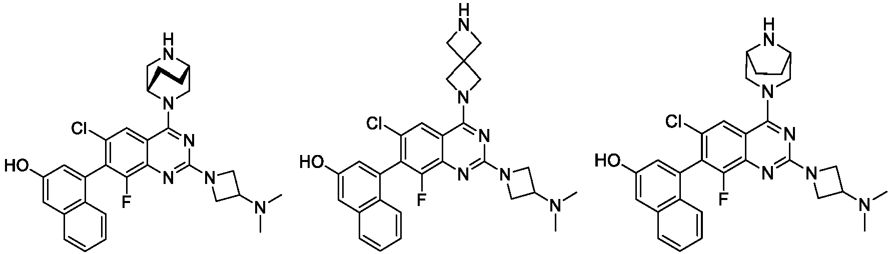

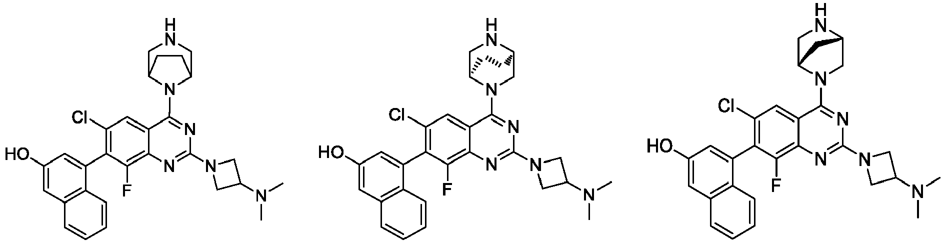

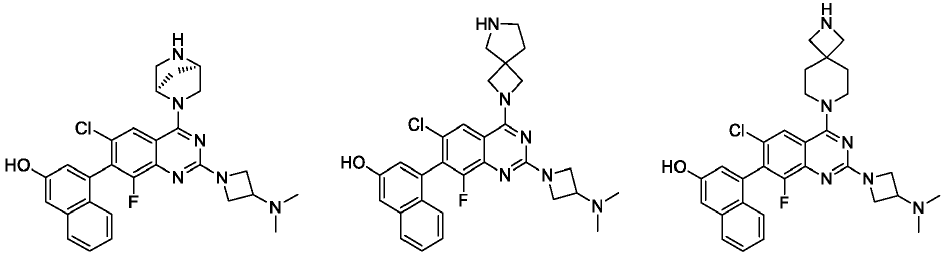

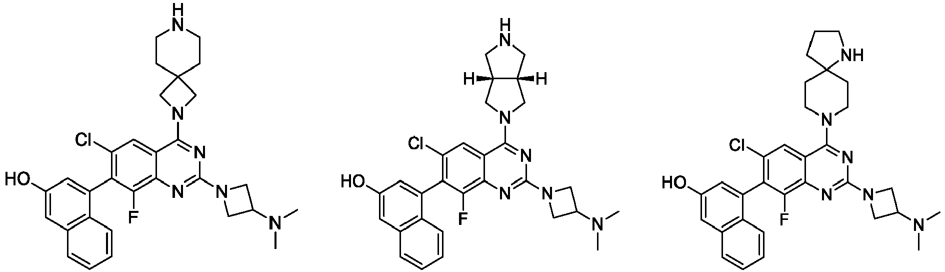

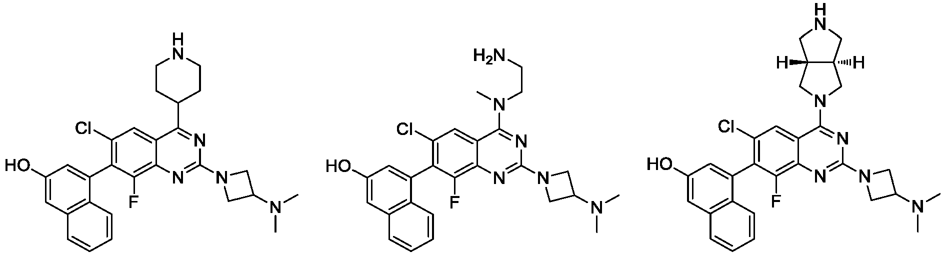

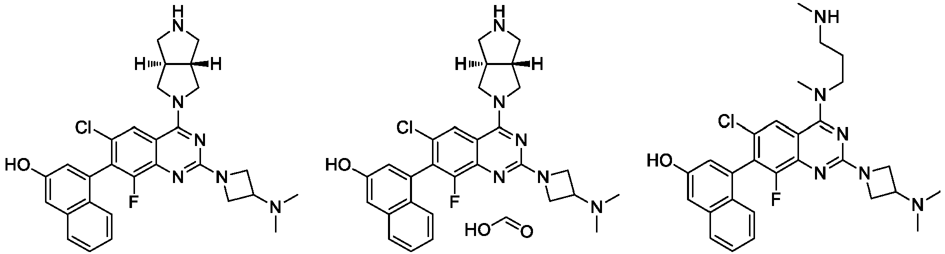

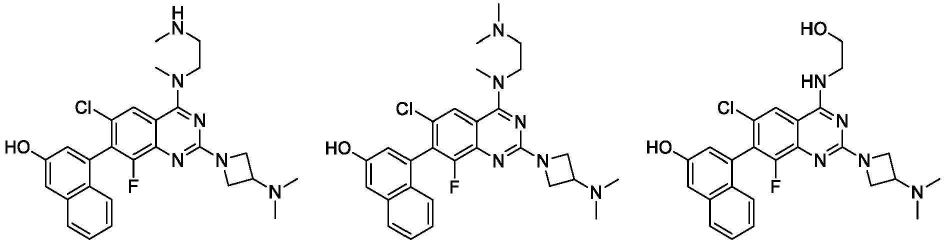

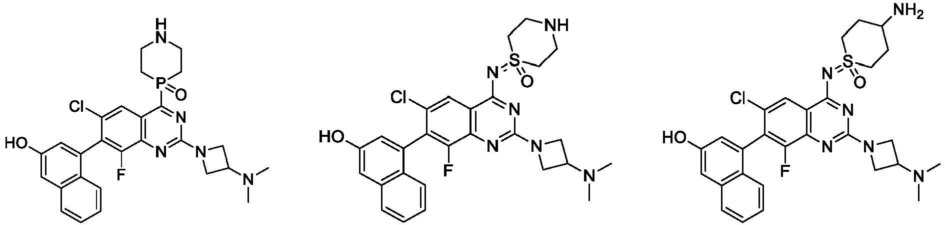

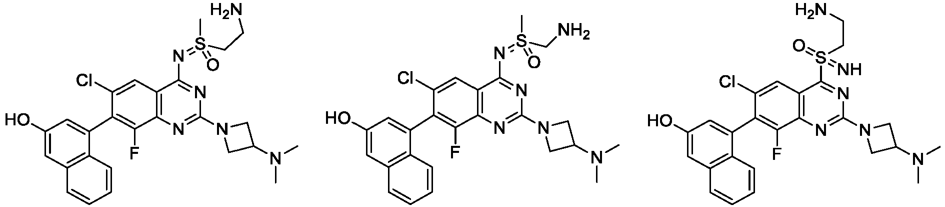

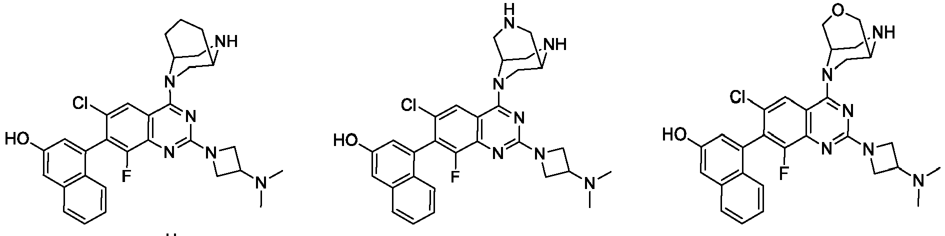

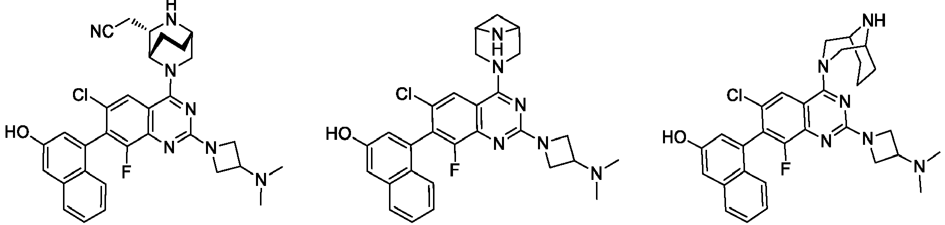

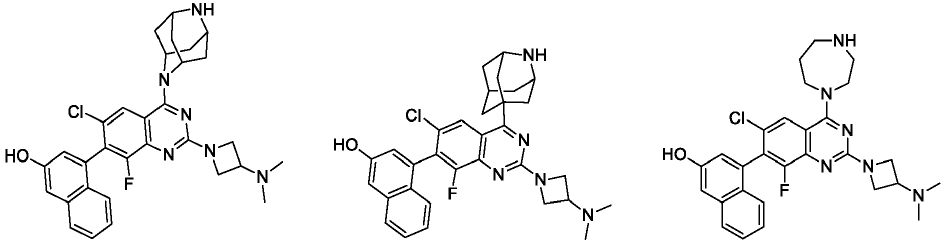

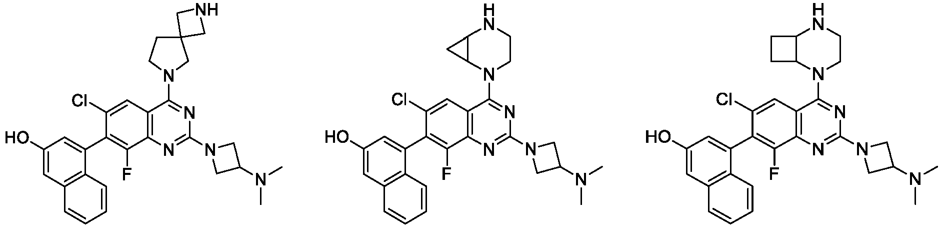

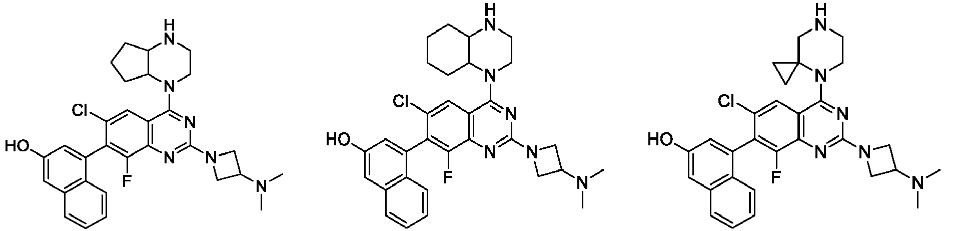

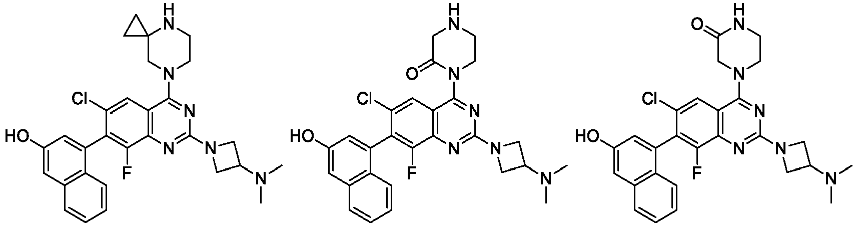

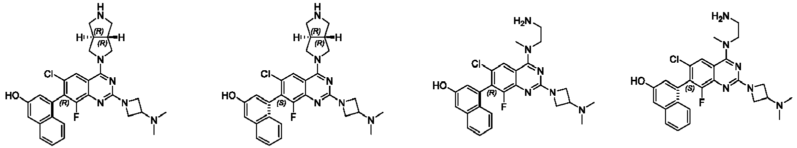

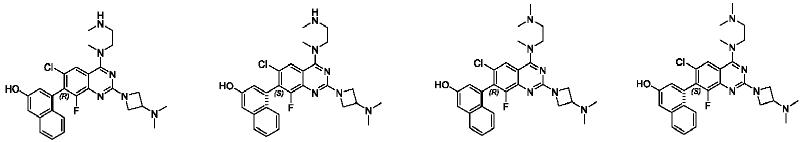

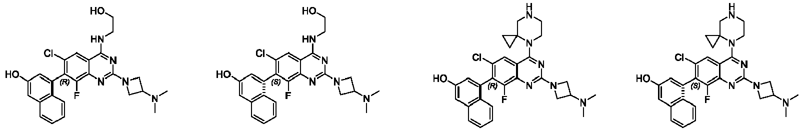

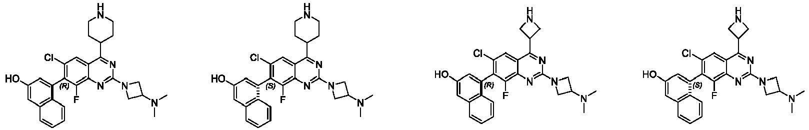

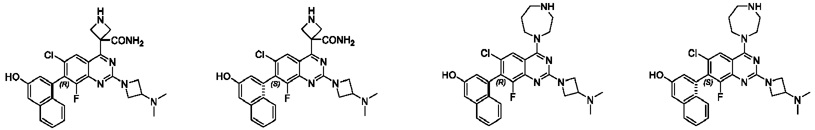

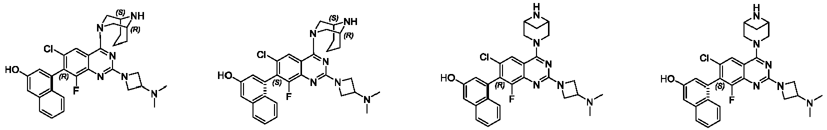

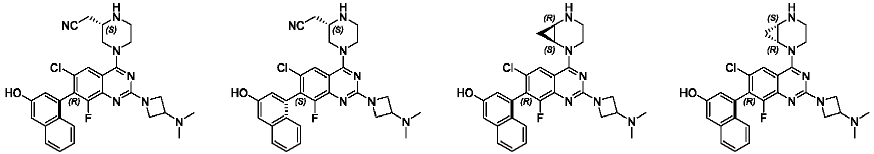

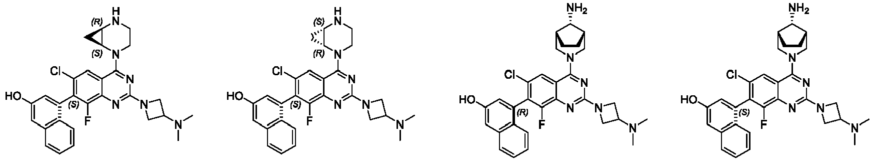

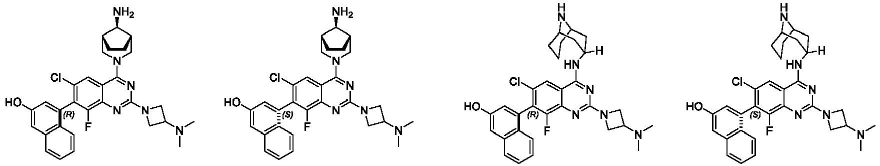

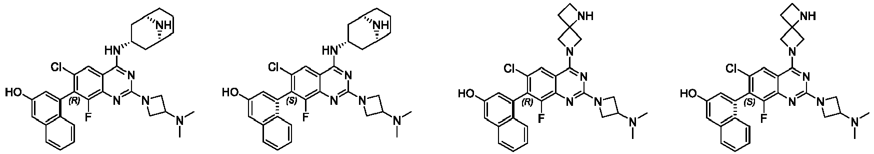

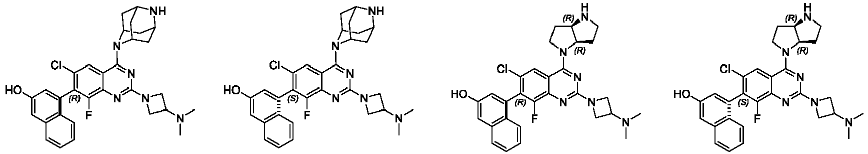

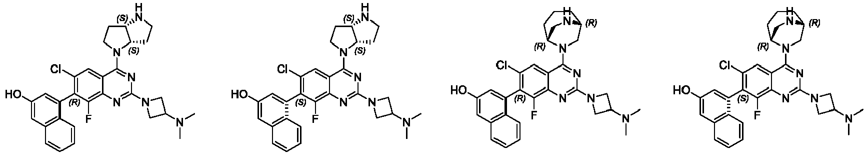

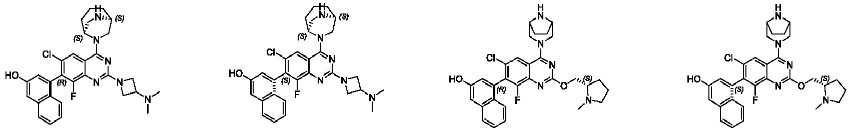

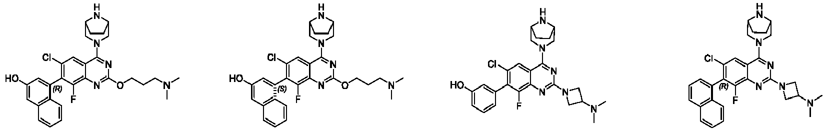

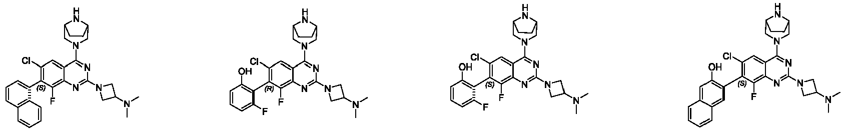

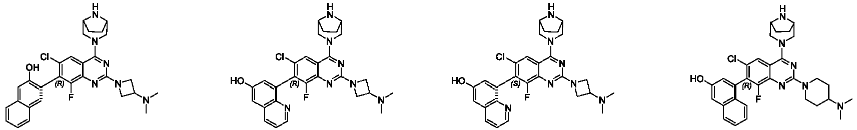

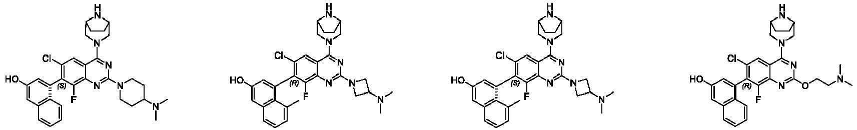

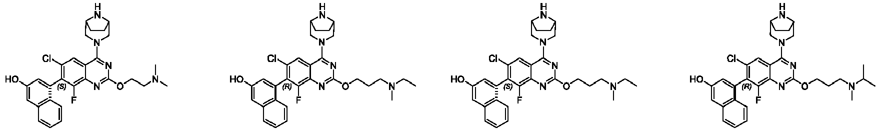

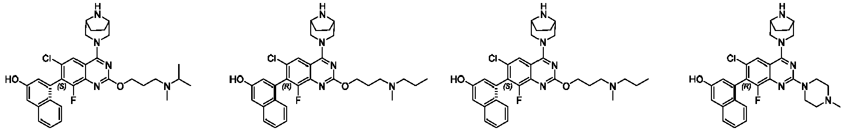

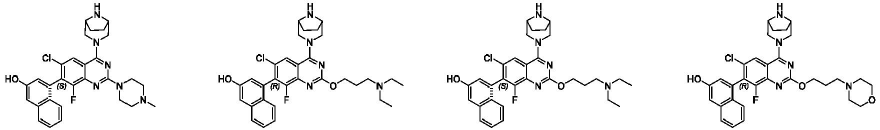

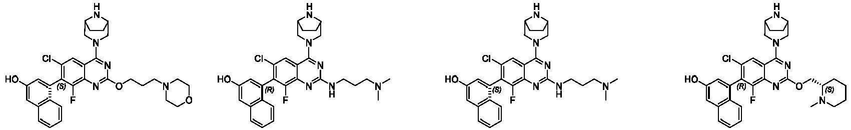

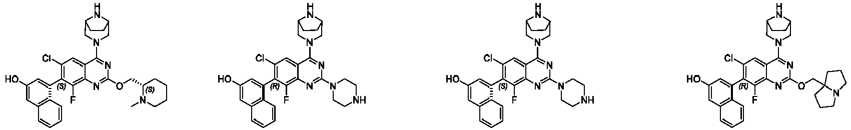

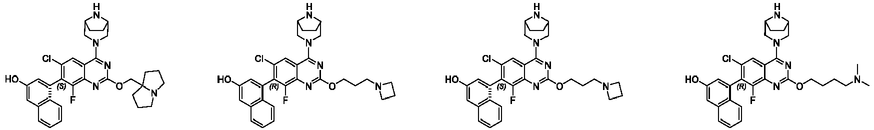

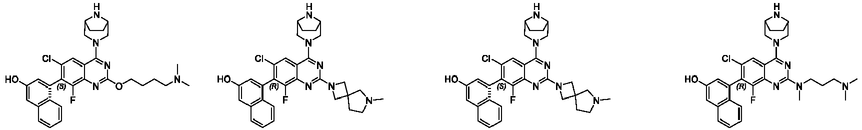

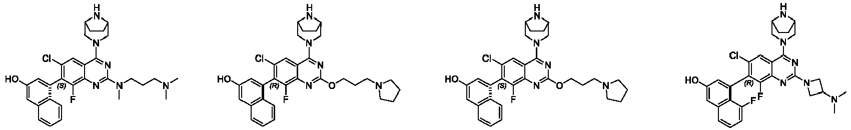

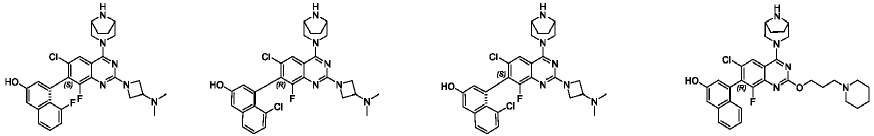

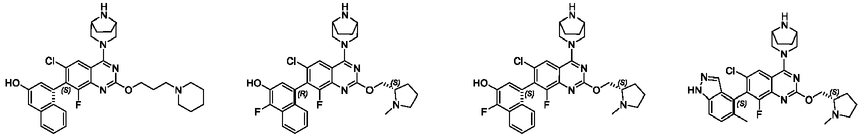

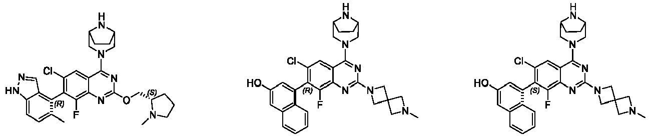

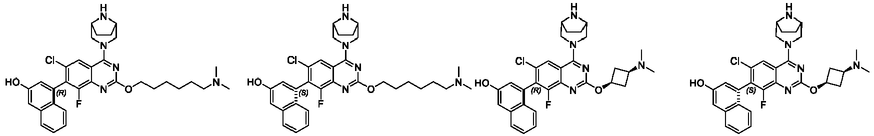

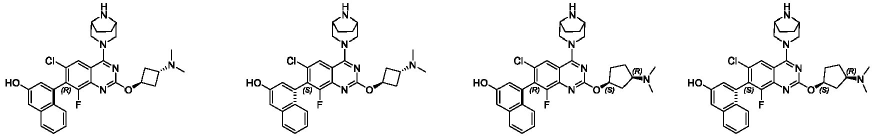

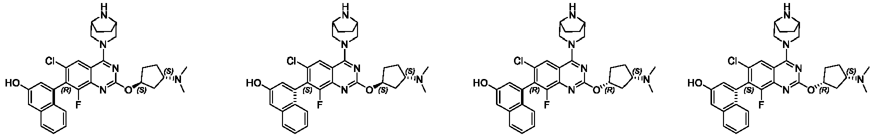

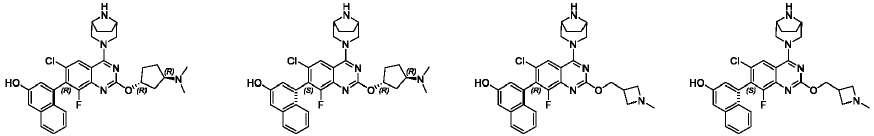

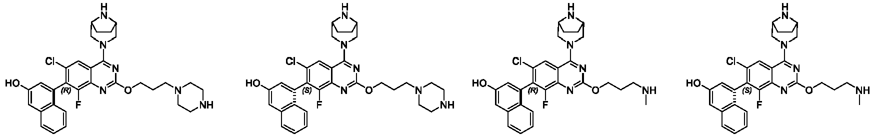

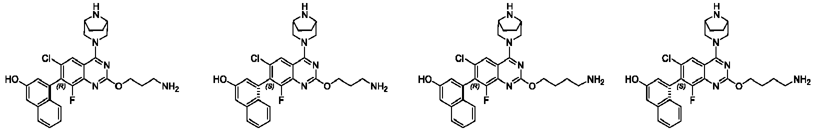

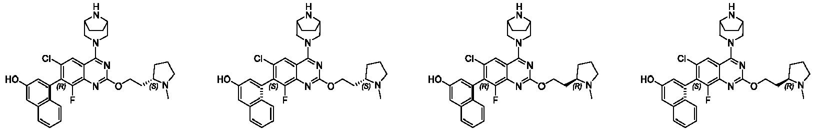

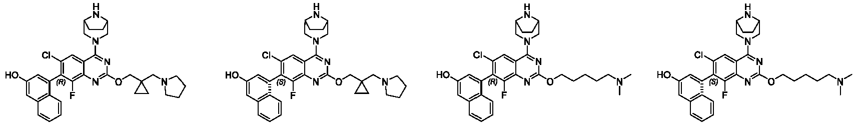

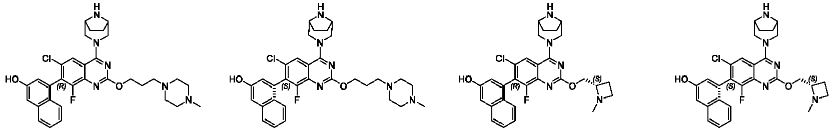

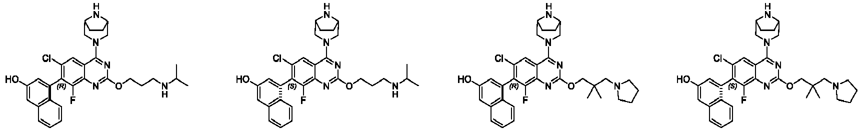

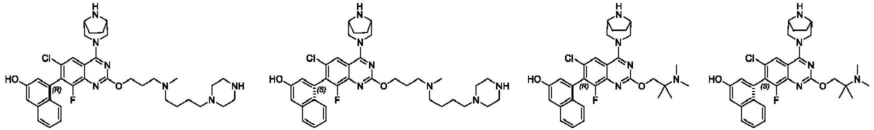

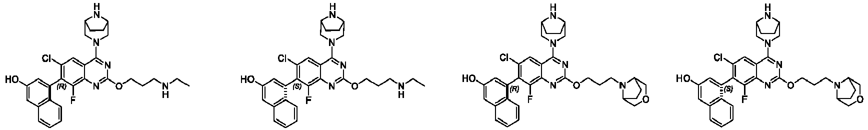

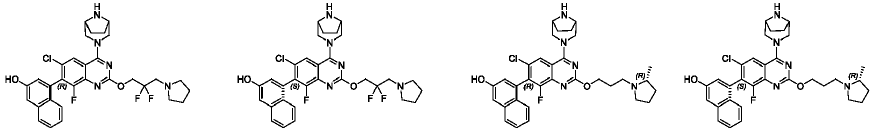

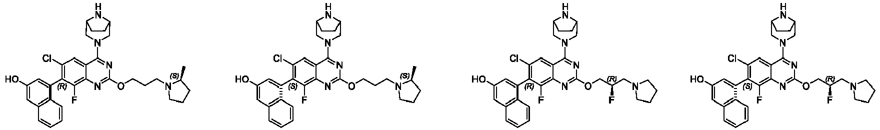

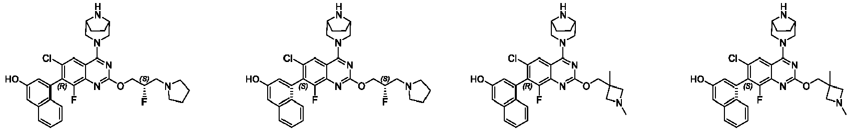

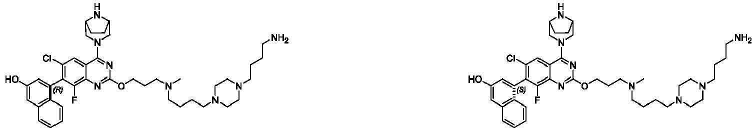

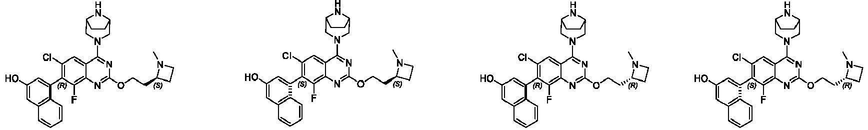

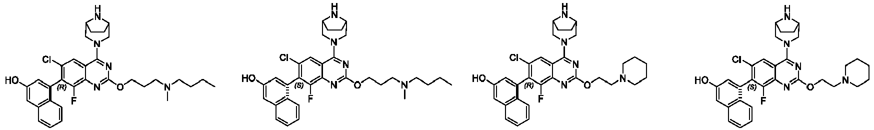

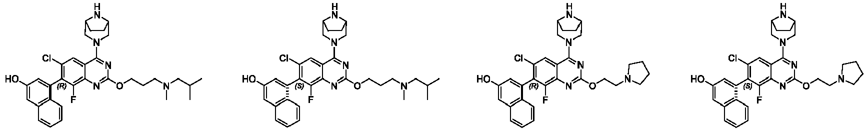

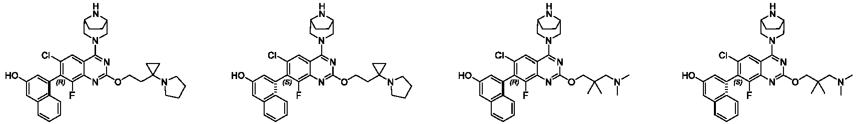

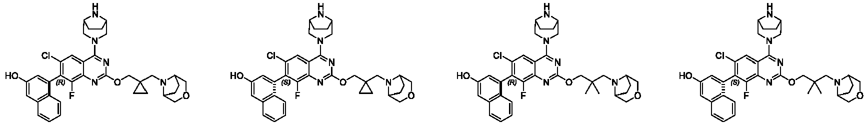

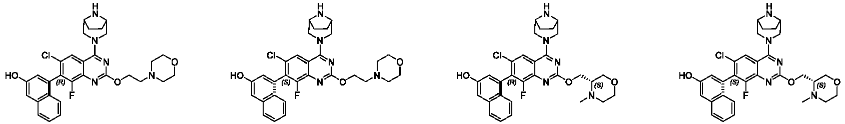

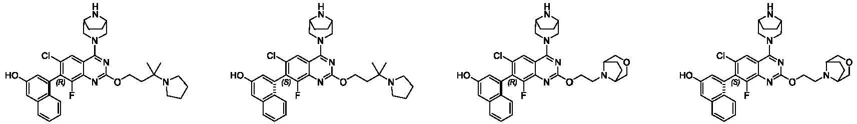

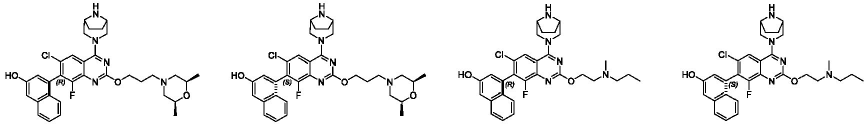

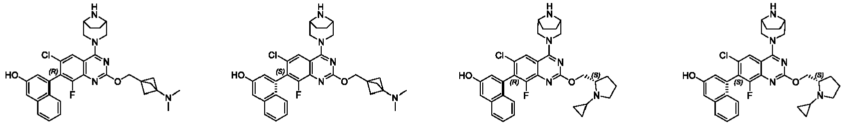

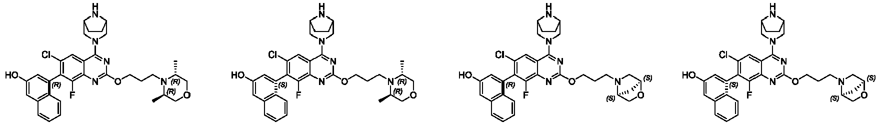

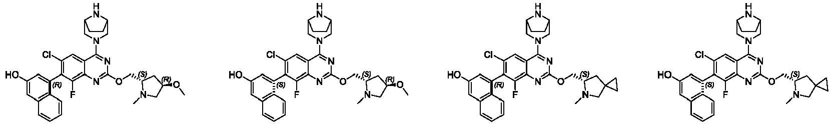

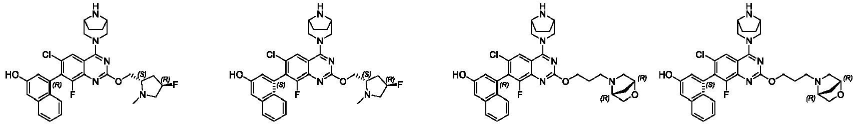

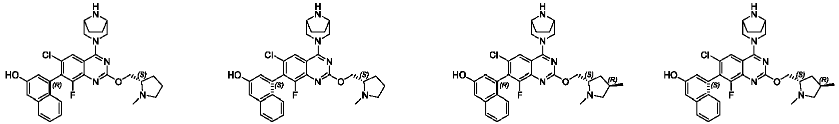

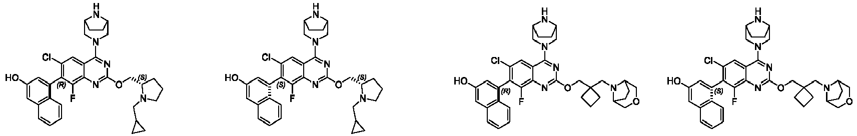

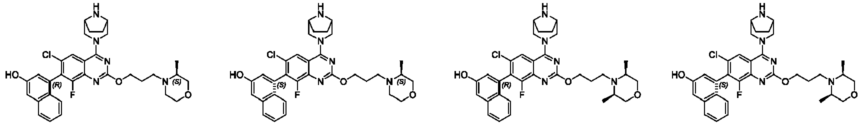

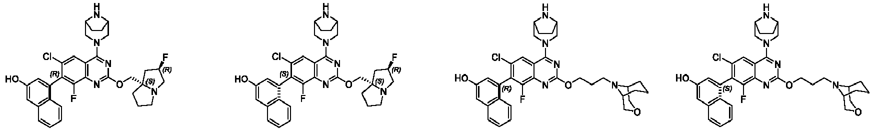

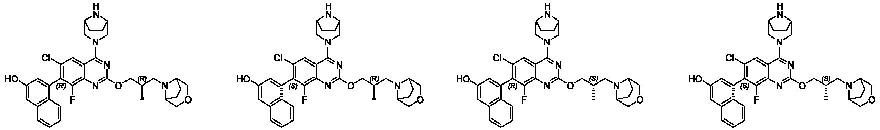

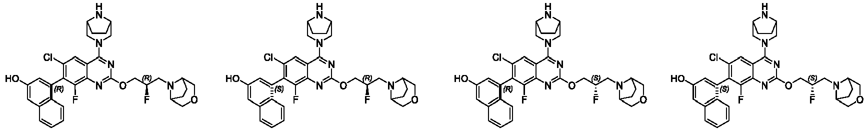

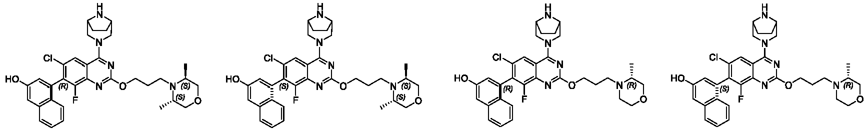

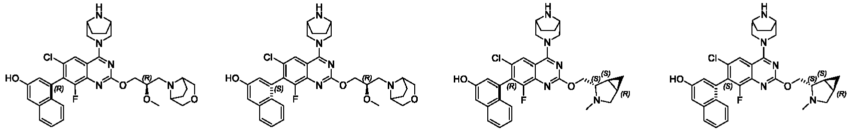

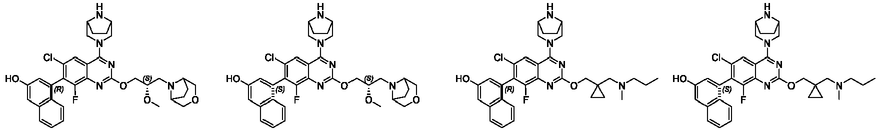

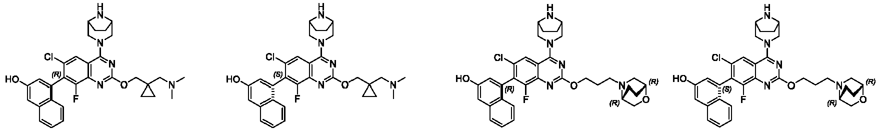

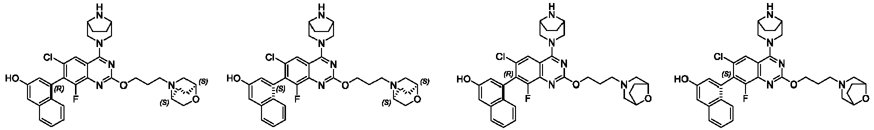

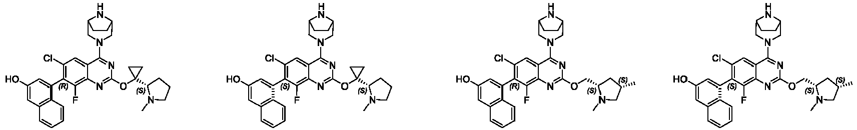

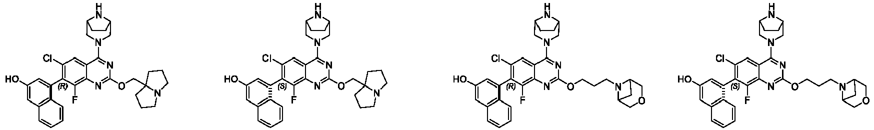

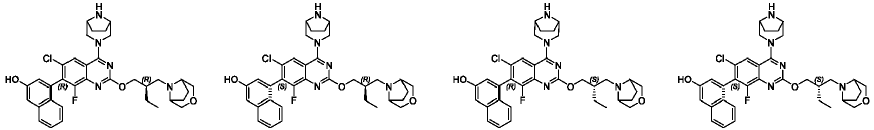

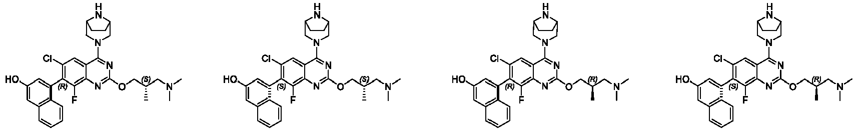

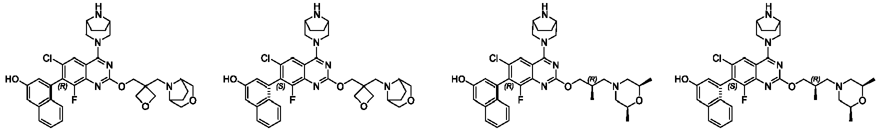

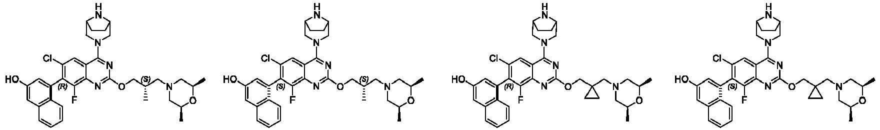

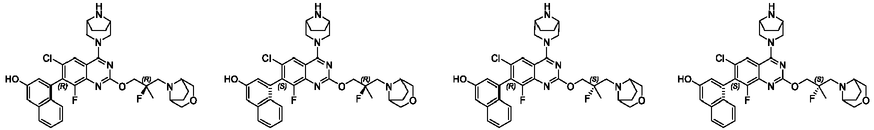

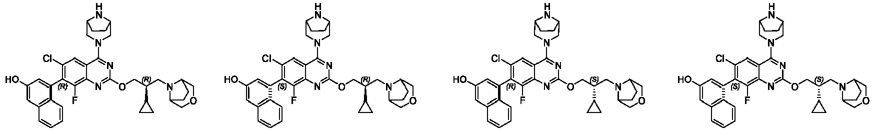

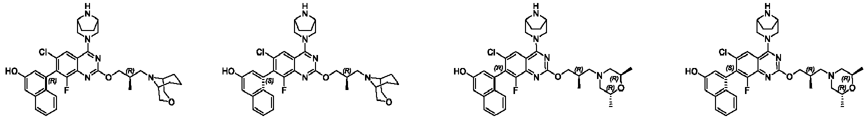

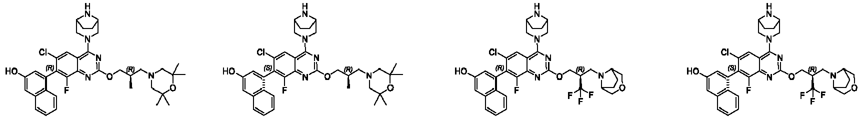

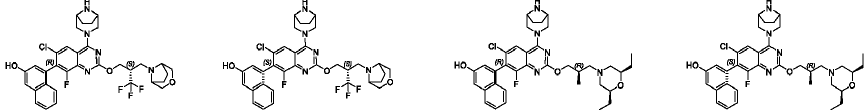

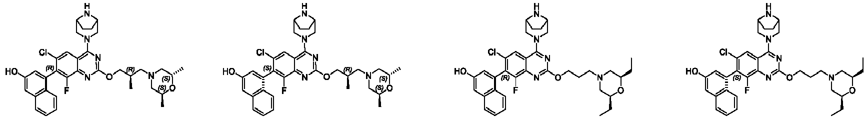

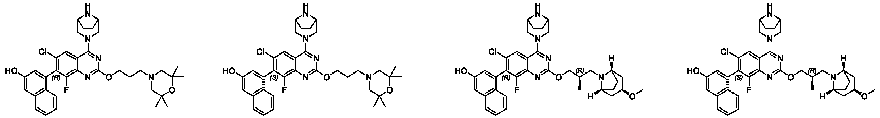

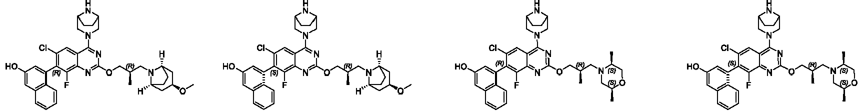

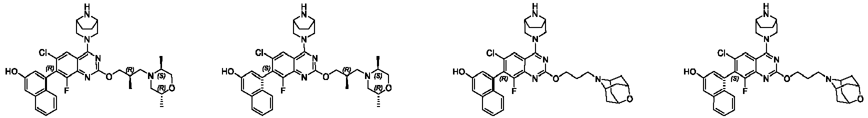

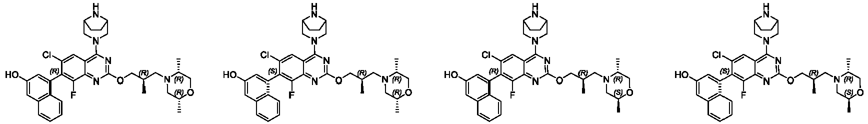

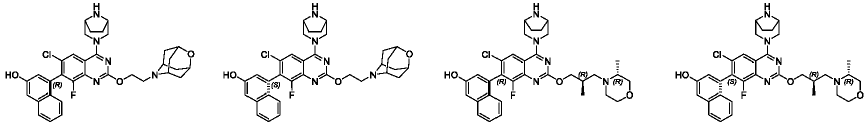

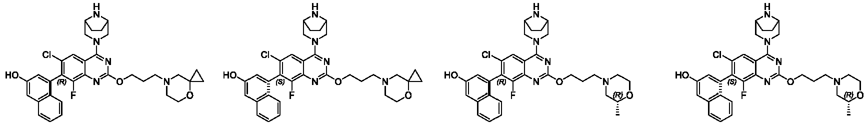

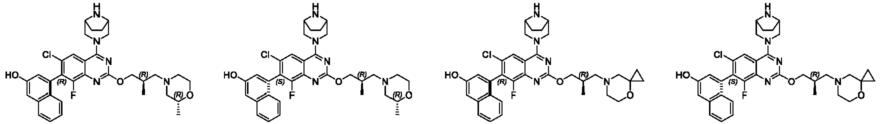

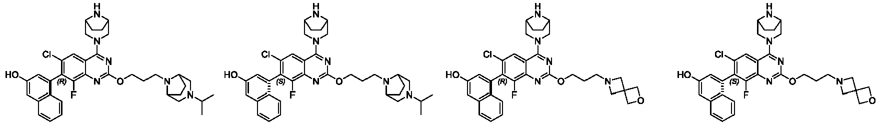

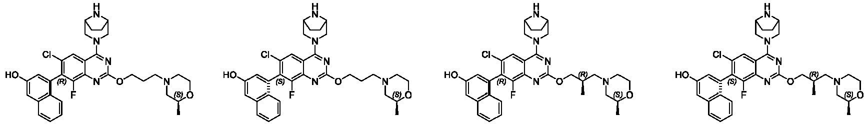

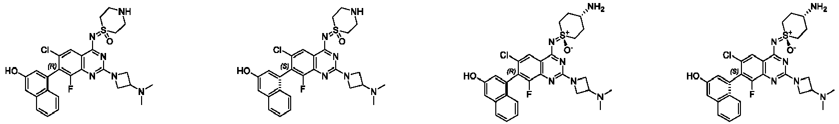

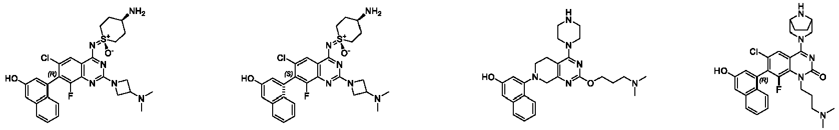

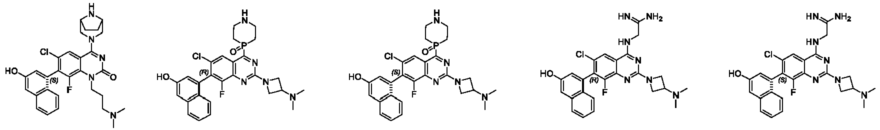

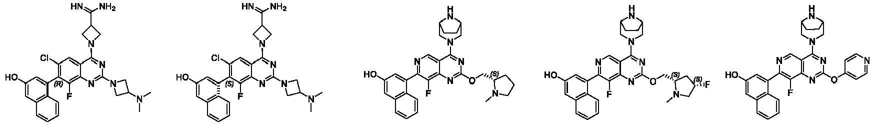

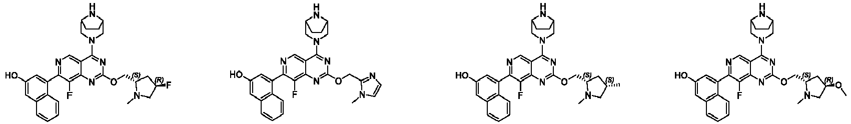

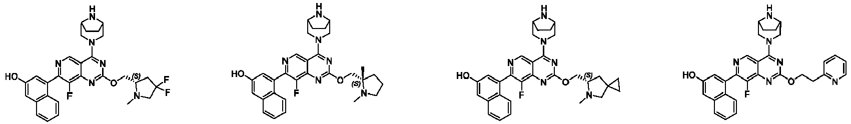

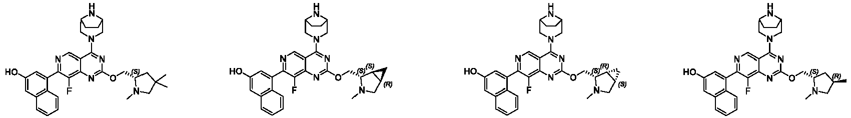

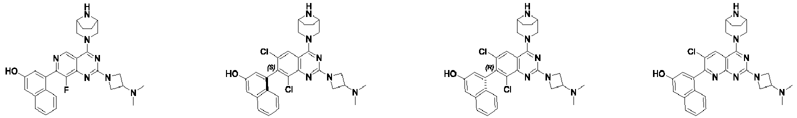

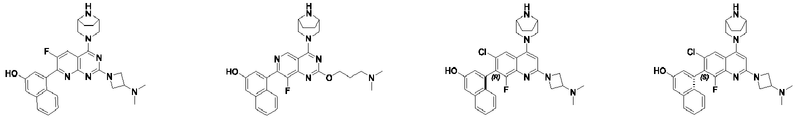

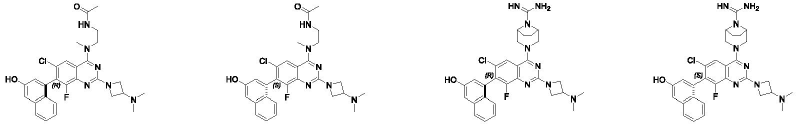

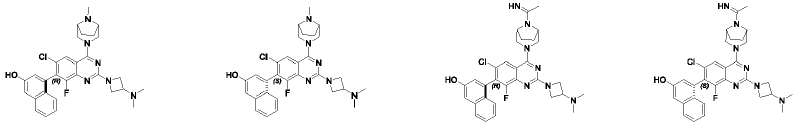

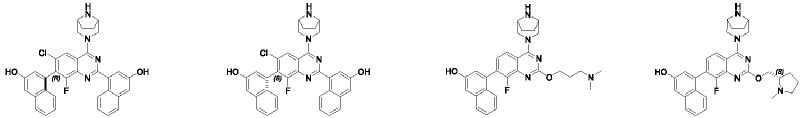

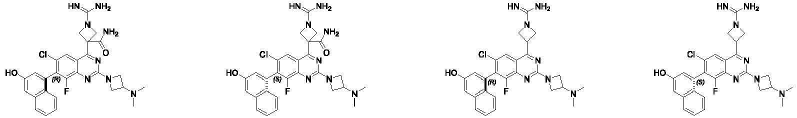

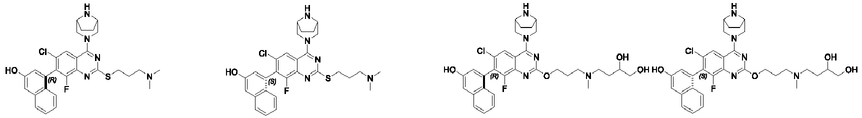

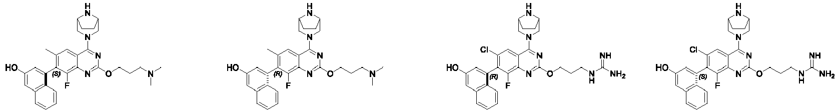

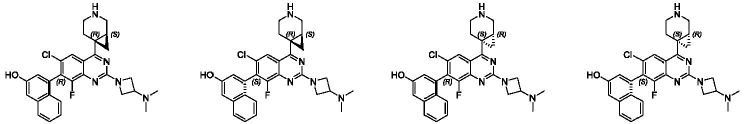

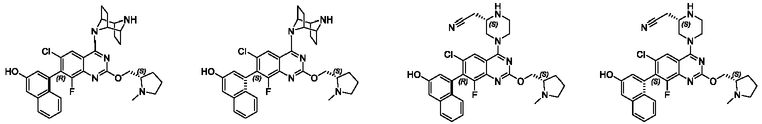

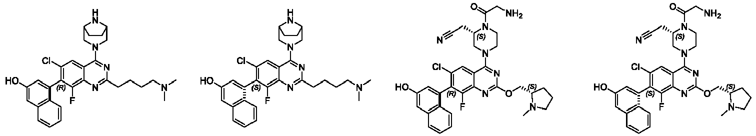

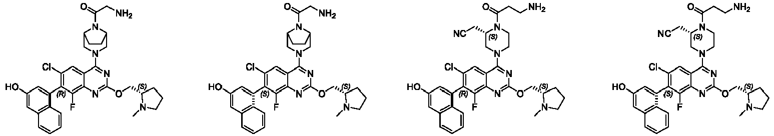

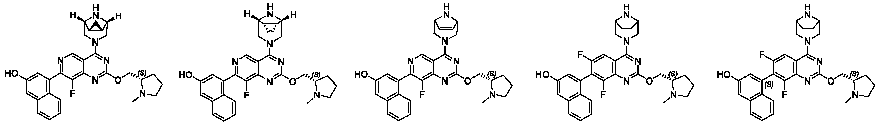

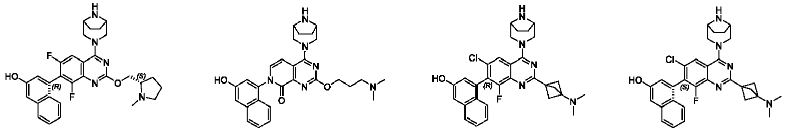

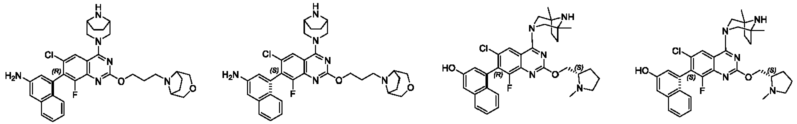

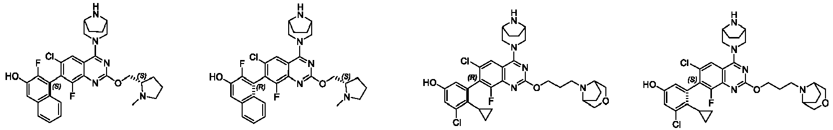

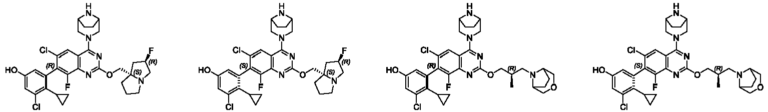

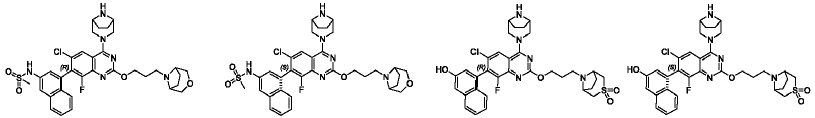

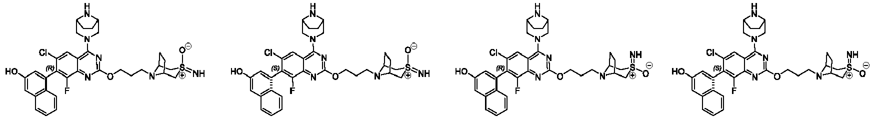

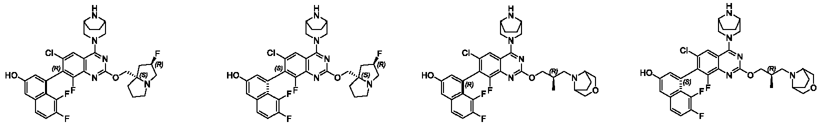

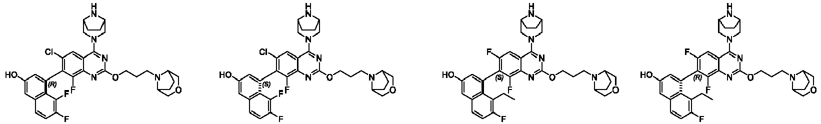

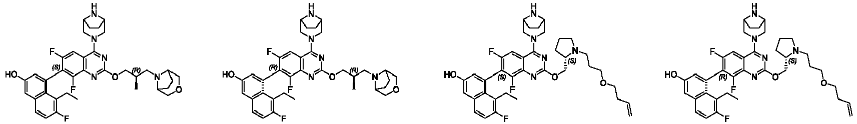

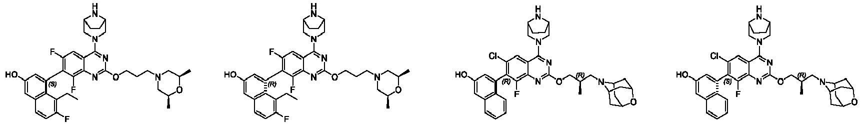

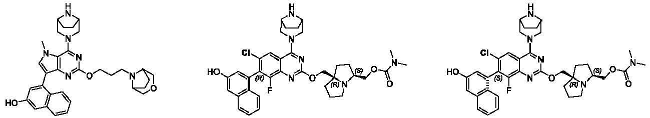

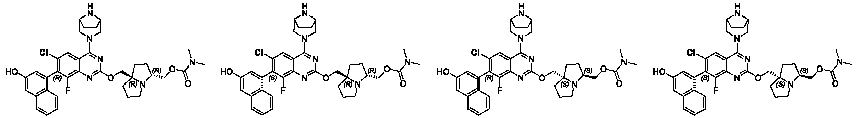

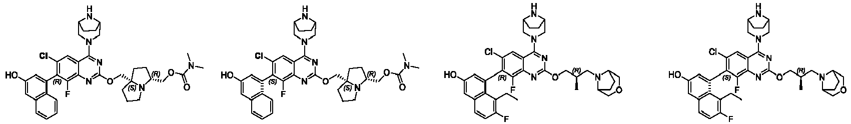

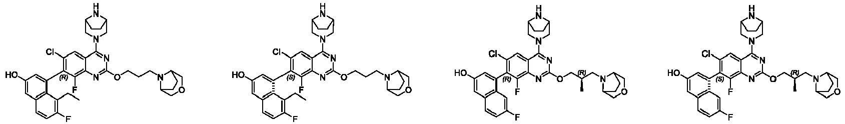

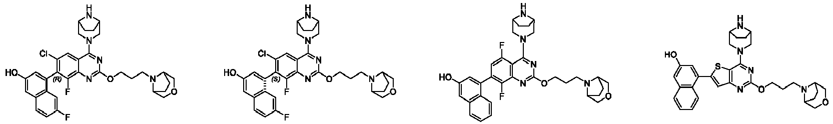

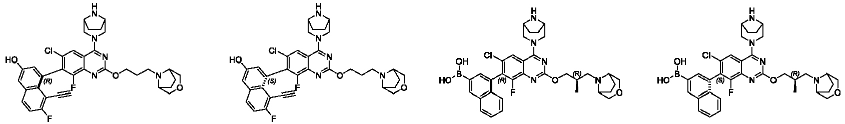

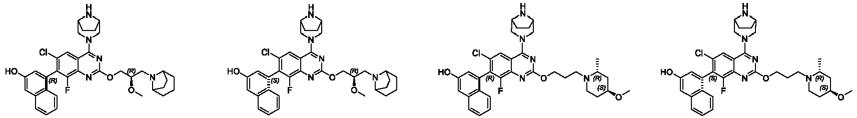

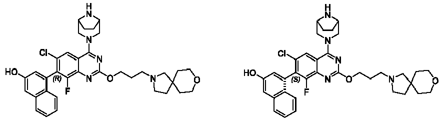

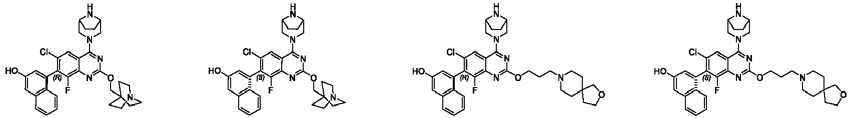

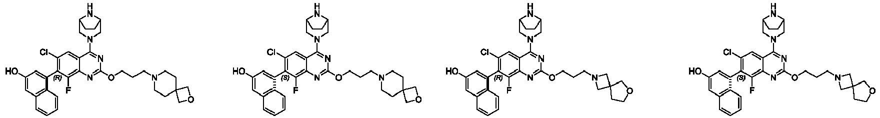

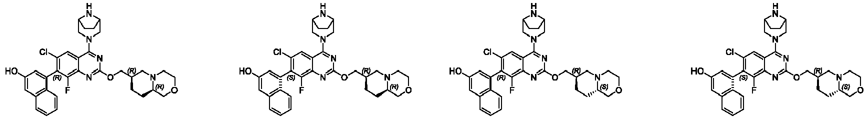

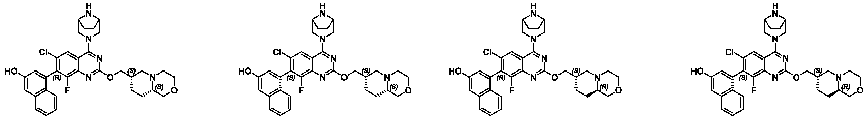

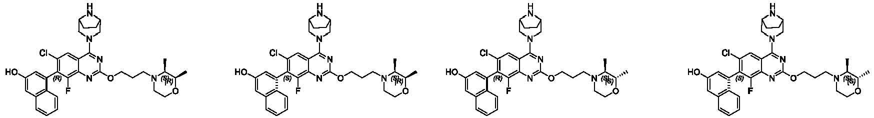

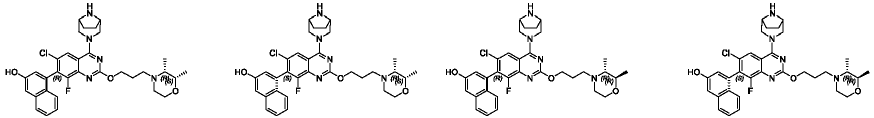

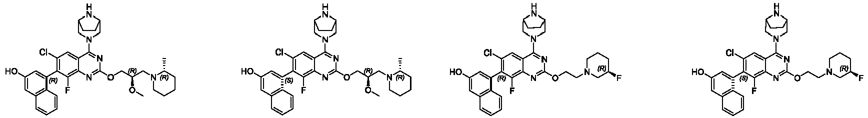

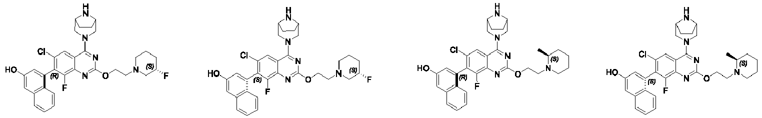

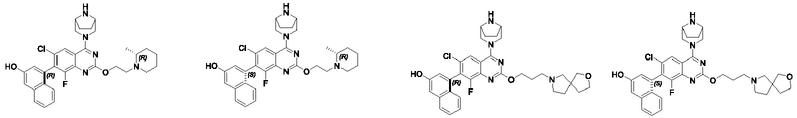

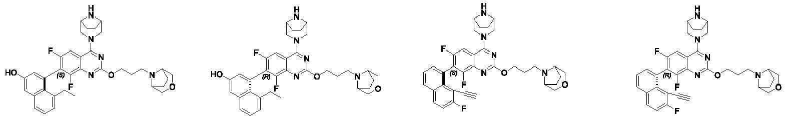

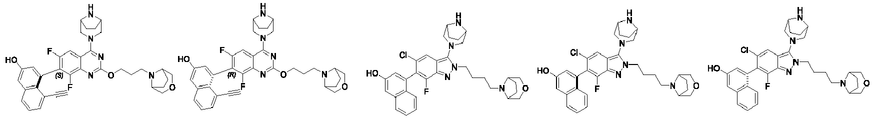

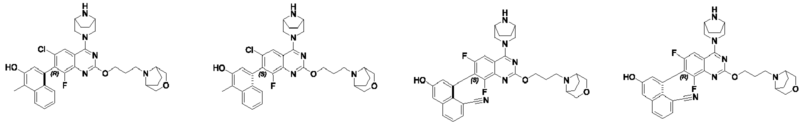

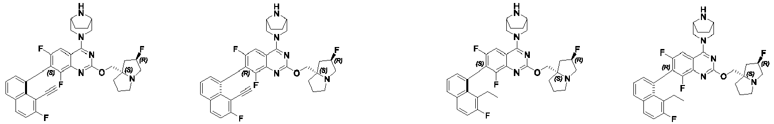

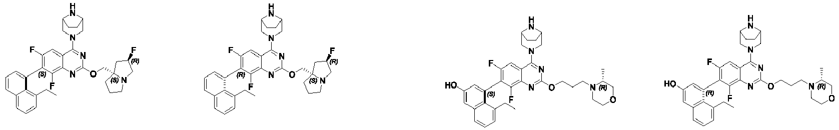

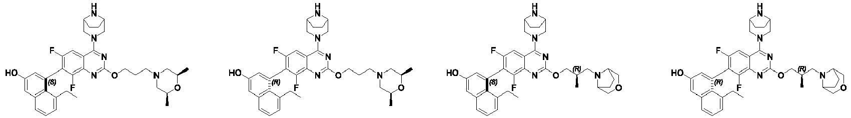

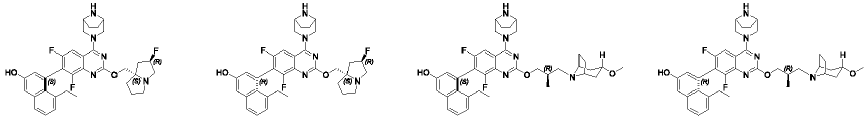

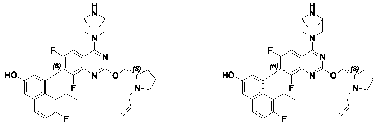

18. The quinazoline compound represented by formula I, the pharmaceutically acceptable salt thereof, the solvate thereof, the prodrug thereof, the metabolite thereof or the isotopic compound thereof according to any one of claims 1 to 15, wherein,
the quinazoline compound represented by formula I, the pharmaceutically acceptable salt thereof, the solvate thereof, the prodrug thereof, the metabolite thereof or the isotopic compound thereof is any of the following structures, wherein, the carbon atom with "*" indicates a carbon atom with *S* configuration or R configuration, 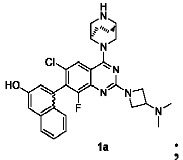
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.946 min; 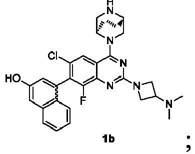
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 5.115 min; 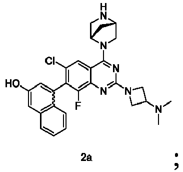
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.104 min; 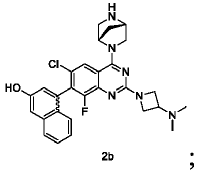
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 3.656 min; 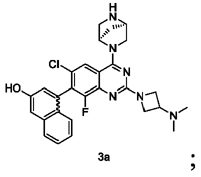
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 10 min; detector UV 220/254 nm; retention time: 2.705 min; 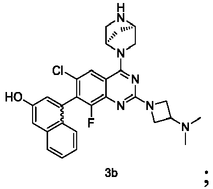
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 10 min; detector UV 220/254 nm; retention time: 6.915 min; 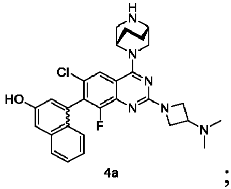
its HPLC conditions are: chiral column: CHIRALPAK IC-3, 4.6 x 50 mm, 3 µm; mobile phase A: n-hexane (0.1% diethylamine), mobile phase B: isopropanol; flow rate: 1 mL/min; isocratic elution with 30% phase B in 15 min; detector UV 220/254 nm; retention time: 5.828 min; 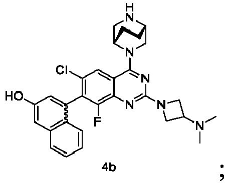
its HPLC conditions are: chiral column: CHIRALPAK IC-3, 4.6 x 50 mm, 3 µm; mobile phase A: n-hexane (0.1% diethylamine), mobile phase B: isopropanol; flow rate: 1 mL/min; isocratic elution with 30% phase B in 15 min; detector UV 220/254 nm; retention time: 9.588 min; 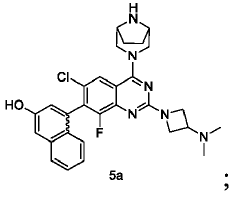
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 2.549 min; 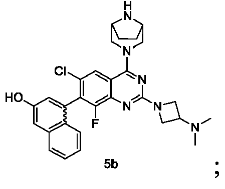
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 6 min; detector UV 220/254 nm; retention time: 3.363 min; 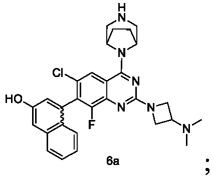
its HPLC conditions are: chiral column: CHIRALPAK IA-3, 4.6 x 50 mm, 3 µm; mobile phase A: n-hexane (0.1% diethylamine), mobile phase B: isopropanol; flow rate: 1 mL/min; isocratic elution with 20% phase B in 9 min; detector UV 220/254 nm; retention time: 6.871 min; 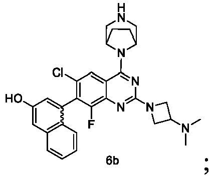
its HPLC conditions are: chiral column: CHIRALPAK IA-3, 4.6 x 50 mm, 3 µm; mobile phase A: n-hexane (0.1% diethylamine), mobile phase B: isopropanol; flow rate: 1 mL/min; isocratic elution with 20% phase B in 9 min; detector UV 220/254 nm; retention time: 4.373 min; 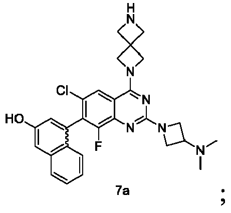
its HPLC conditions are: chiral column: Lux 3 µm Cellulose-2, 4.6 x 50 mm, 3 µm; mobile phase A: n-hexane (0.1% diethylamine), mobile phase B: ethanol; flow rate: 1 mL/min; isocratic elution with 30% phase B in 12 min; detector UV 220/254 nm; retention time: 4.914 min; 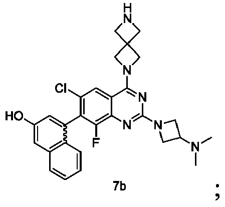
its HPLC conditions are: chiral column: Lux 3 µm Cellulose-2, 4.6 x 50 mm, 3 µm; mobile phase A: n-hexane (0.1% diethylamine), mobile phase B: ethanol; flow rate: 1 mL/min; isocratic elution with 30% phase B in 12 min; detector UV 220/254 nm; retention time: 7.935 min; 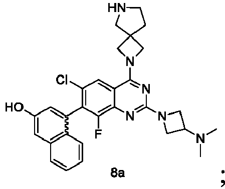
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.844 min; 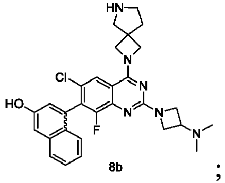
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 4.215 min; 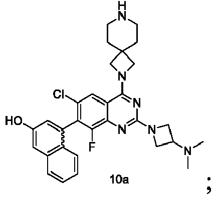
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 4.5 min; detector UV 220/254 nm; retention time: 2.562 min; 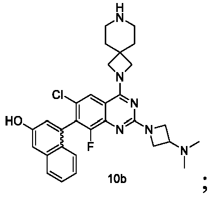
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 4.5 min; detector UV 220/254 nm; retention time: 3.662 min; 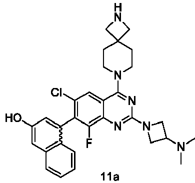
; its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.856 min; 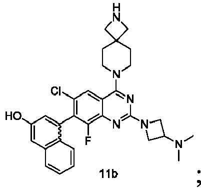
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 4.120 min; 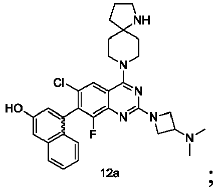
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 4.5 min; detector UV 220/254 nm; retention time: 2.317 min; 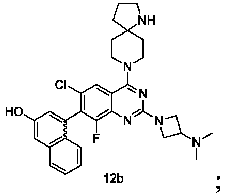
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 4.5 min; detector UV 220/254 nm; retention time: 3.182 min; 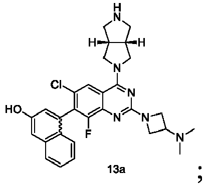
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 10 min; detector UV 220/254 nm; retention time: 4.576 min; 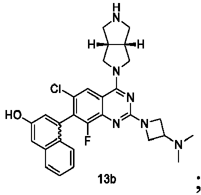
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 10 min; detector UV 220/254 nm; retention time: 8.266 min; 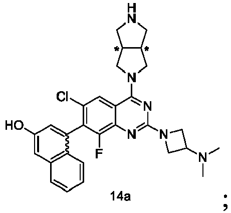
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 3.616 min; 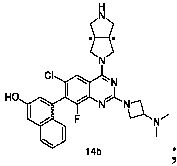
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 5.340 min; 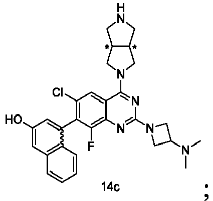
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 10 min; detector UV 220/254 nm; retention time: 5.189 min; 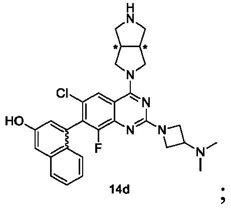
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 30% phase B in 10 min; detector UV 220/254 nm; retention time: 7.314 min; 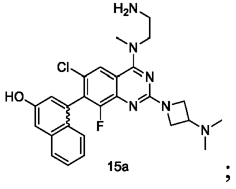
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 3.5 min; detector UV 220/254 nm; retention time: 1.347 min; 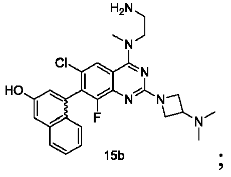
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 3.5 min; detector UV 220/254 nm; retention time: 2.180 min; 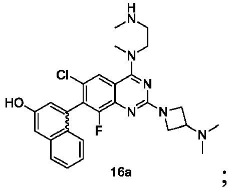
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.690 min; 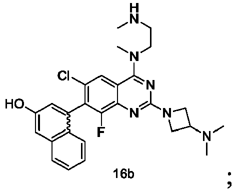
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 2.820 min; 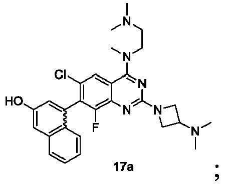
its HPLC conditions are: chiral column: CHIRALPAK IG-3, 4.6 x 50 mm, 3 µm; mobile phase A: n-hexane/dichloromethane = 3/1 (0.1% diethylamine), mobile phase B: ethanol; flow rate: 1 mL/min; isocratic elution with 50% phase B in 6 min; detector UV 220/254 nm; retention time: 4.257 min; 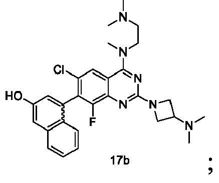
its HPLC conditions are: chiral column: CHIRALPAK IG-3, 4.6 x 50 mm, 3 µm; mobile phase A: n-hexane/dichloromethane = 3/1 (0.1% diethylamine), mobile phase B: ethanol; flow rate: 1 mL/min; isocratic elution with 50% phase B in 6 min; detector UV 220/254 nm; retention time: 2.734 min; 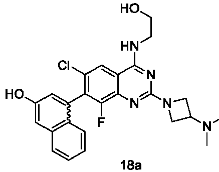
; its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 5.5 min; detector UV 220/254 nm; retention time: 3.619 min; 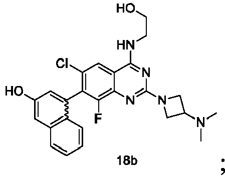
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 5.5 min; detector UV 220/254 nm; retention time: 2.324 min; 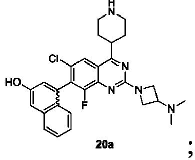
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.433 min; 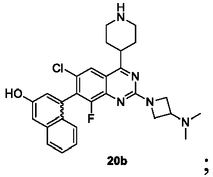
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220/254 nm; retention time: 1.905 min; 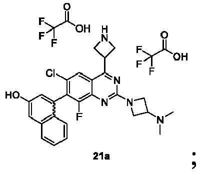
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 10 min; detector UV 220/254 nm; retention time: 3.80 min; 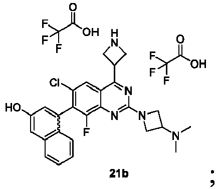
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 6 min; detector UV 220/254 nm; retention time: 5.950 min; 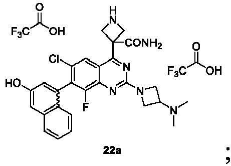
its HPLC conditions are: chiral column: CHIRALPAK ID-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 8 min; detector UV 220/254 nm; retention time: 3.40 min; 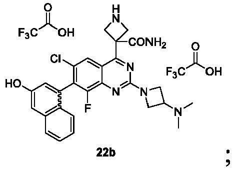
its HPLC conditions are: chiral column: CHIRALPAK ID-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 30% phase B in 8 min; detector UV220/254 nm; retention time: 5.31 min; 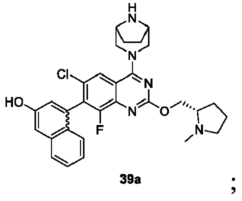
its HPLC conditions are: chiral column: CHIRALPAK IG-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 1.203 min; 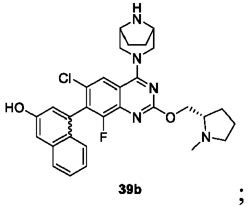
its HPLC conditions are: chiral column: CHIRALPAK IG-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 50% phase B in 6 min; detector UV 220/254 nm; retention time: 2.391 min; 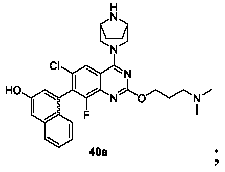
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.960 min; 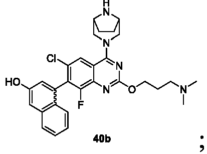
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.715 min; 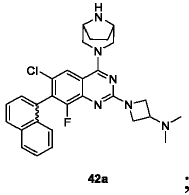
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 10% phase B in 8 min; detector UV 220/254 nm; retention time: 3.759 min; 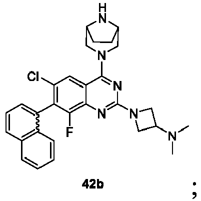
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 10% phase B in 8 min; detector UV 220/254 nm; retention time: 5.749 min; 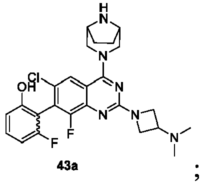
its HPLC conditions are: chiral column: CHIRALPAK IE-3, 3.0 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 3.306 min; 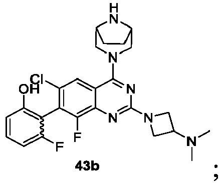
its HPLC conditions are: chiral column: CHIRALPAK IE-3, 3.0 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.803 min; 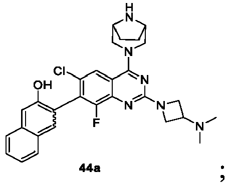
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 2.737 min; 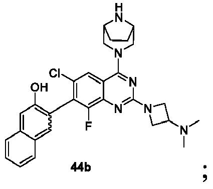
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 30% phase B in 6 min; detector UV 220/254 nm; retention time: 4.713 min; 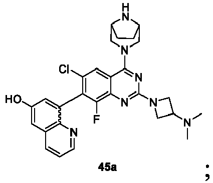
its HPLC conditions are: chiral column: CHIRALPAK IE-3, 4.6 x 50 mm, 3 µm; mobile phase A: methyl *tert*-butyl ether (0.1% diethylamine), mobile phase B: methanol; flow rate: 1 mL/min; isocratic elution with 50% phase B in 6 min; detector UV 220/254 nm; retention time: 1.200 min; 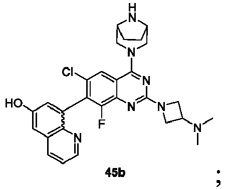
its HPLC conditions are: chiral column: CHIRALPAK IE-3, 4.6 x 50 mm, 3 µm; mobile phase A: methyl tert-butyl ether (0.1% diethylamine), mobile phase B: methanol; flow rate: 1 mL/min; isocratic elution with 50% phase B in 6 min; detector UV 220/254 nm; retention time: 3.550 min; 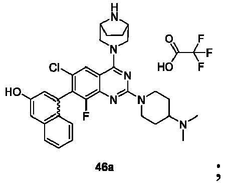
its HPLC conditions are: chiral column: CHIRALPAK IC-3, 4.6 x 50 mm, 3 µm; mobile phase A: *n*-hexane: methyl *tert*-butyl ether = 1:1 (0.1% diethylamine), mobile phase B: ethanol; flow rate: 1 mL/min; isocratic elution with 5% phase B in 10 min; detector UV 220/254 nm; retention time: 5.305 min; 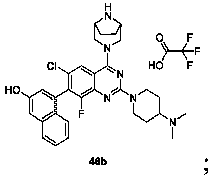
its HPLC conditions are: chiral column: CHIRALPAK IC-3, 4.6 x 50 mm, 3 µm; mobile phase A: *n*-hexane: methyl *tert*-butyl ether = 1:1 (0.1% diethylamine), mobile phase B: ethanol; flow rate: 1 mL/min; isocratic elution with 5% phase B in 10 min; detector UV 220/254 nm; retention time: 7.357 min; 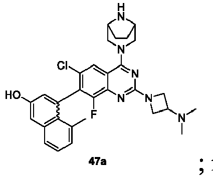
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 220/254 nm; retention time: 3.197 min; 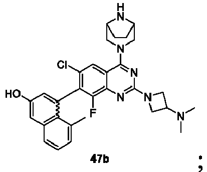
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 220/254 nm; retention time: 4.394 min; 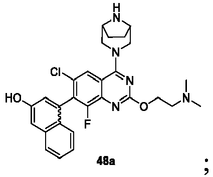
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 7 min; detector UV 220/254 nm; retention time: 4.100 min; 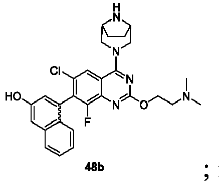
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 7 min; detector UV 220/254 nm; retention time: 5.751 min; 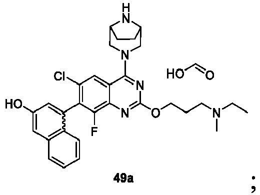
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 4 min; detector UV 220/254 nm; retention time: 1.910 min; 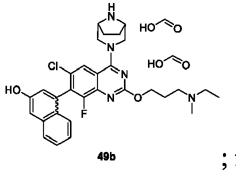
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 4 min; detector UV 220/254 nm; retention time: 2.941 min; 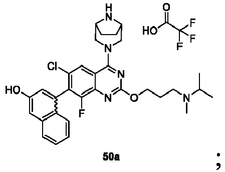
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 3.684 min; 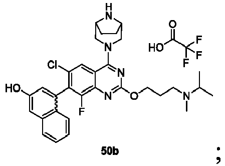
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 6.409 min; 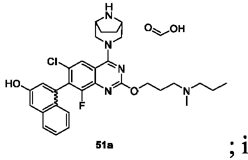
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 3.667 min; 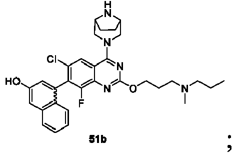
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 220/254 nm; retention time: 6.387 min; 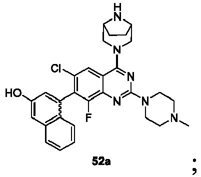
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 5 min; detector UV 220/254 nm; retention time: 1.686 min; 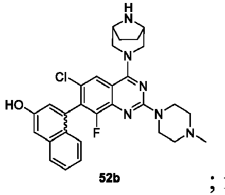
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 5 min; detector UV 220/254 nm; retention time: 2.959 min; 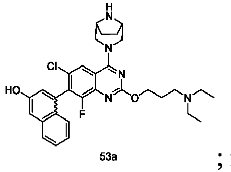
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 20% phase B in 6 min; detector UV 220/254 nm; retention time: 2.759 min; 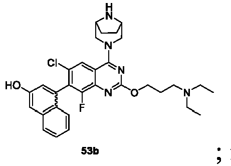
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 20% phase B in 6 min; detector UV 220/254 nm; retention time: 4.652 min; 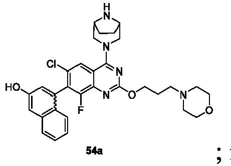
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: ethanol; flow rate: 1 mL/min; isocratic elution with 30% phase B in 5 min; detector UV 220/254 nm; retention time: 1.963 min; 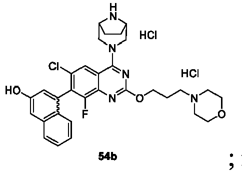
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 30% phase B in 5 min; detector UV 220/254 nm; retention time: 3.148 min; 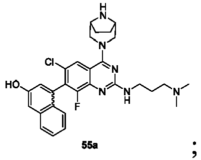
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8 min; detector UV 220 nm; retention time: 4.211 min; 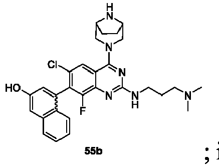
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8 min; detector UV 220 nm; retention time: 6.385 min; 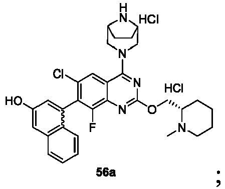
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 3.762 min; 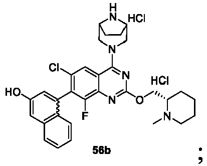
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8 min; detector UV 254 nm; retention time: 5.467 min; 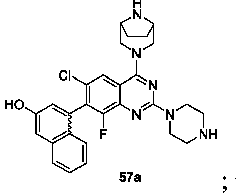
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 5.5 min; detector UV 254 nm; retention time: 2.713 min; 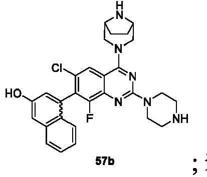
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 5.5 min; detector UV 254 nm; retention time: 3.537 min; 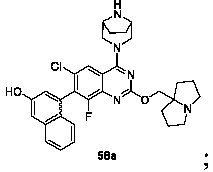
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 2.045 min; 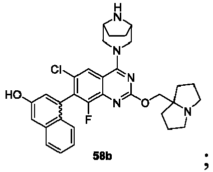
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 3.463 min; 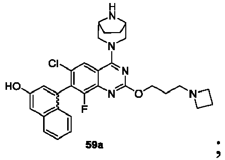
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 5.080 min; 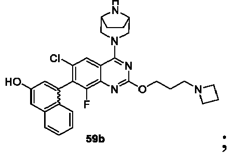
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 6 min; detector UV 254 nm; retention time: 3.566 min; 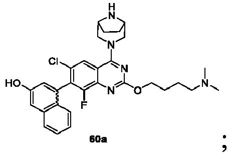
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 15 min; detector UV 254 nm; retention time: 5.980 min; 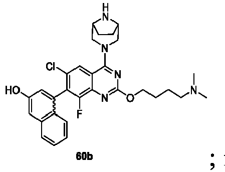
its HPLC conditions are: chiral column: CHIRALPAK IC-3, 4.6 x 50 mm, 3 µm; mobile phase A: n-hexane/dichloromethane = 5/1 (0.1% diethylamine), mobile phase B: ethanol; flow rate: 1 mL/min; isocratic elution with 10% phase B in 15 min; detector UV 254 nm; retention time: 10.313 min; 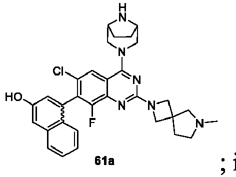
its HPLC conditions are: chiral column: CHIRALPAK ID-3, 4.6 x 50 mm, 3 µm; mobile phase A: *n*-hexane: methyl *tert*-butyl ether = 1/1 (0.1% diethylamine), mobile phase B: ethanol; flow rate: 1 mL/min; isocratic elution with 20% phase B in 6 min; detector UV 254 nm; retention time: 2.202 min; 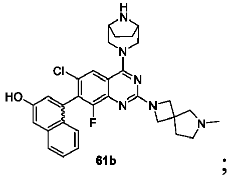
its HPLC conditions are: chiral column: CHIRALPAK ID-3, 4.6 x 50 mm, 3 µm; mobile phase A: *n*-hexane: methyl *tert*-butyl ether = 1/1 (0.1% diethylamine), mobile phase B: ethanol; flow rate: 1 mL/min; isocratic elution with 20% phase B in 6 min; detector UV 254 nm; retention time: 1.694 min; 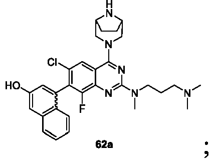
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 254 nm; retention time: 3.894 min; 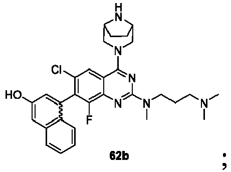
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 254 nm; retention time: 1.814 min; 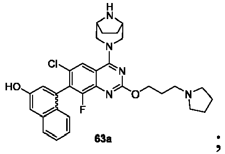
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 2.813 min; 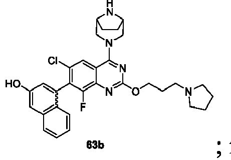
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: isopropanol; flow rate: 1 mL/min; isocratic elution with 30% phase B in 6 min; detector UV 220 nm; retention time: 1.965 min; 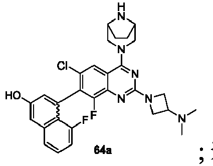
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 10% phase B in 8 min; detector UV 254 nm; retention time: 3.932 min; 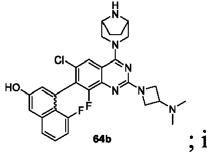
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 10% phase B in 10 min; detector UV 254 nm; retention time: 7.304 min; 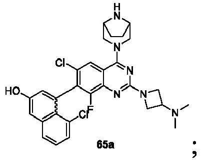
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 10% phase B in 10 min; detector UV 254 nm; retention time: 4.668 min; 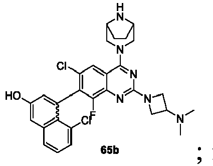
its HPLC conditions are: chiral column: CHIRALPAK IC-3, 4.6 x 100 mm, 3 µm; mobile phase A: *n*-hexane/dichloromethane = 5/1 (0.1% diethylamine), mobile phase B: ethanol; flow rate: 1 mL/min; isocratic elution with 10% phase B in 10 min; detector UV 254 nm; retention time: 6.605 min; 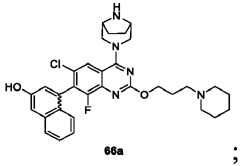
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 30% phase B in 4 min; detector UV 254 nm; retention time: 1.465 min; 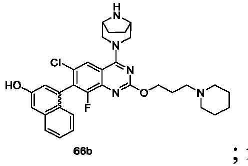
its HPLC conditions are: chiral column: CHIRALPAK IC-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 30% phase B in 4 min; detector UV 254 nm; retention time: 2.173 min; 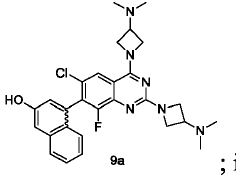
its HPLC conditions are: chiral column: CHIRALPAK IC-3, 4.6 x 50 mm, 3 µm; mobile phase A: *n*-hexane/dichloromethane = (5/1) (0.1% diethylamine), mobile phase B: isopropanol; flow rate: 1 mL/min; isocratic elution with 30% phase B in 6 min; detector UV 220/254 nm; retention time: 2.097 min; 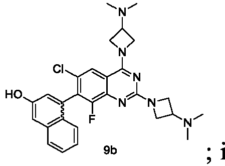
its HPLC conditions are: chiral column: CHIRALPAK IC-3, 4.6 x 50 mm, 3 µm; mobile phase A: *n*-hexane/dichloromethane = (5/1) (0.1% diethylamine), mobile phase B: isopropanol; flow rate: 1 mL/min; isocratic elution with 30% phase B in 6 min; detector UV 220/254 nm; retention time: 3.541 min; 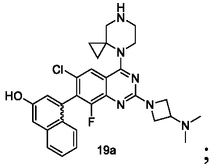
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 50% phase B in 6 min; detector UV 220/254 nm; retention time: 1.741 min; 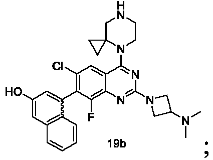
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 50% phase B in 6 min; detector UV 220/254 nm; retention time: 3.291 min; 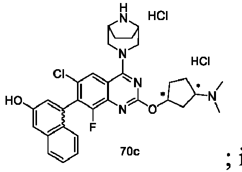
its HPLC conditions are: chiral column: XA-CHIRALPAK AD-3, 4.6 x 250 mm, 3 µm; mobile phase A: *n*-hexane (0.5% diethylamine), mobile phase B: ethanol; flow rate: 1 mL/min; isocratic elution with 50% phase B in 19 min; detector UV 254 nm; retention time: 8.374 min; 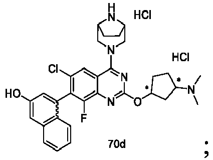
its HPLC conditions are: chiral column: XA-CHIRALPAK AD-3, 4.6 x 250 mm, 3 µm; mobile phase A: *n*-hexane (0.5% diethylamine), mobile phase B: ethanol; flow rate: 1 mL/min; isocratic elution with 50% phase B in 19 min; detector UV 254 nm; retention time: 13.763 min; 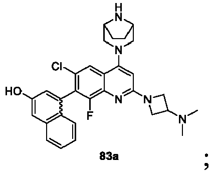
it is prepared from 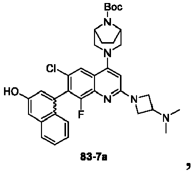 the HPLC conditions of 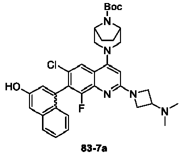 are: chiral column NB-Lux 5 µM i-Cellulose-5, 2.12 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 8% phase B in 40 min; detector: 220 nm; retention time: 16.81 min; 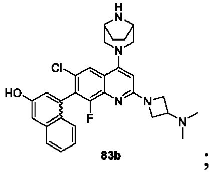
it is prepared from 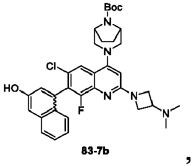 the HPLC conditions of 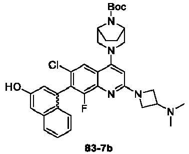 are: chiral column NB-Lux 5 µM i-Cellulose-5, 2.12 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 8% phase B in 40 min; detector: 220 nm; retention time: 25.09 min; 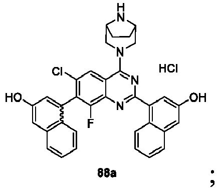
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 8.5 min; detector UV 254 nm; retention time: 4.033 min; 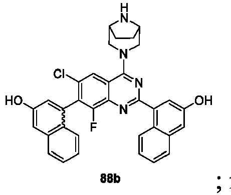
its HPLC conditions are: chiral column: CHIRALPAK ID-3, 4.6 x 50 mm, 3 µm; mobile phase A: *n*-hexane/dichloromethane = 3/1 (0.1% diethylamine), mobile phase B: ethanol; flow rate: 1 mL/min; isocratic elution with 10% phase B in 8.5 min; detector UV 254 nm; retention time: 6.515 min; 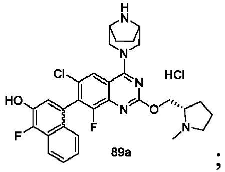
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 3.401 min; 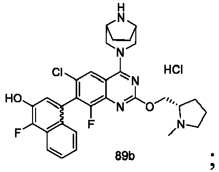
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 6 min; detector UV 220 nm; retention time: 4.503 min; 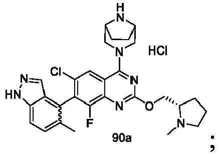
its HPLC conditions are: chiral column: CHIRALPAK IC-3, 3.0 x 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 1.442 min; 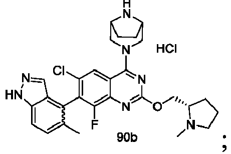
its HPLC conditions are: chiral column: CHIRALPAK IC-3, 3.0 x 50 mm, 3 µm; mobile phase A: *n*-hexane (0.1% diethylamine), mobile phase B: ethanol; flow rate: 1 mL/min; isocratic elution with 50% phase B in 4 min; detector UV 220 nm; retention time: 2.226 min; 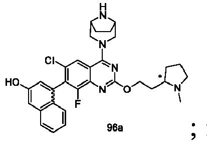
its HPLC conditions are: chiral column: CHIRAL ART Amylose-C Neo, 50 x 4.6 mm, 3 µm; mobile phase A: *n*-hexane (0.1% diethylamine), mobile phase B: isopropanol/acetonitrile = 2/1; flow rate: 1 mL/min; isocratic elution with 50% phase B in 6.5 min; detector UV 230 nm; retention time: 2.045 min; 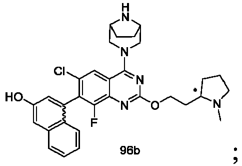
its HPLC conditions are: chiral column: CHIRAL ART Amylose-C Neo, 50 x 4.6 mm, 3 µm; mobile phase A: *n*-hexane (0.1% diethylamine), mobile phase B: isopropanol/acetonitrile = 2/1; flow rate: 1 mL/min; isocratic elution with 50% phase B in 6.5 min; detector UV 230 nm; retention time: 4.319 min; 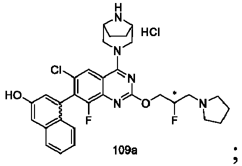
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 12.5 min; detector UV 220 nm; retention time: 7.498 min; 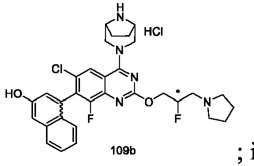
its HPLC conditions are: chiral column: CHIRALPAK ID-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 12.5 min; detector UV 220 nm; retention time: 9.454 min; 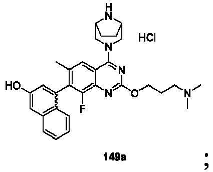
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 6 min; detector UV 220 nm; retention time: 2.457 min; 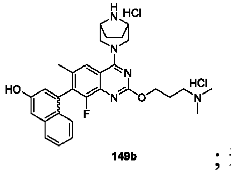
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 6 min; detector UV 220 nm; retention time: 3.982 min; 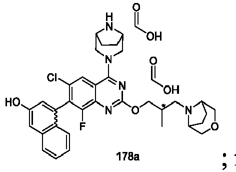
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 6 min; detector UV 220 nm; retention time: 4.024 min; 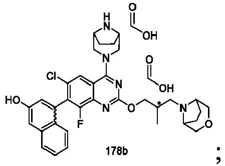
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 6 min; detector UV 220 nm; retention time: 5.203 min; 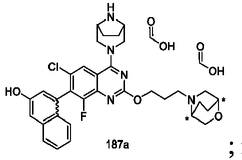
its HPLC conditions are: chiral column: XA-CHIRALPAK AS-3, 4.6 x 50 mm, 3 µm; mobile phase A: *n*-hexane (0.1% diethylamine), mobile phase B: acetonitrile; flow rate: 1.67 mL/min; isocratic elution with 20% phase B in 2 min; detector UV 220 nm; retention time: 0.821 min; 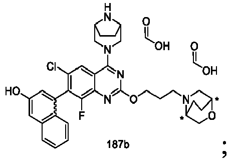
its HPLC conditions are: chiral column: XA-CHIRALPAK AS-3, 4.6 x 50 mm, 3 µm; mobile phase A: *n*-hexane (0.1% diethylamine), mobile phase B: acetonitrile; flow rate: 1.67 mL/min; isocratic elution with 20% phase B in 2 min; detector UV 220 nm; retention time: 1.215 min; 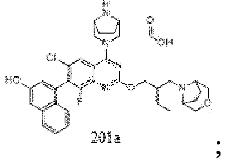
its HPLC conditions are: chiral column: CHIRALPAK IA-3, 4.6 x 100 mm, 3 µm; mobile phase A: *n*-hexane (0.2% n-butylamine), mobile phase B: isopropanol: acetonitrile (2: 1); flow rate: 1 mL/min; isocratic elution with 20% phase B in 8 min; detector UV 254 nm; retention time: 4.688 min; 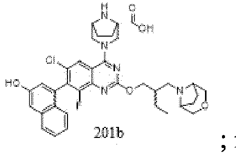
its HPLC conditions are: chiral column: CHIRALPAK IA-3, 4.6 x 100 mm, 3 µm; mobile phase A: *n*-hexane (0.2% *n*-butylamine), mobile phase B: isopropanol: acetonitrile (2: 1); flow rate: 1 mL/min; isocratic elution with 20% phase B in 8 min; detector UV 254 nm; retention time: 6.033 min; 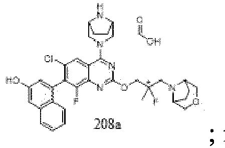
its HPLC conditions are: chiral column: CHIRALPAK IA-3, 4.6 x 100 mm, 3 µm; mobile phase A: *n*-hexane/dichloromethane (5: 1) (0. 1% diethylamine), mobile phase B: ethanol; flow rate: 1 mL/min; isocratic elution with 10% phase B in 12.5 min; detector UV 220 nm; retention time: 8.927 min; 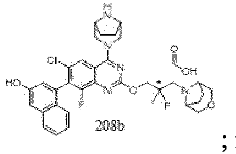
its HPLC conditions are: chiral column: CHIRALPAK IA-3, 4.6 x 100 mm, 3 µm; mobile phase A: *n*-hexane/dichloromethane (5: 1) (0.1% diethylamine), mobile phase B: ethanol; flow rate: 1 mL/min; isocratic elution with 10% phase B in 12.5 min; detector UV 220 nm; retention time: 9.894 min; 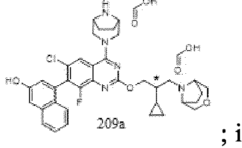
its HPLC conditions are: chiral column: CHIRALPAK ID-3, 3.0 x 100 mm, 3 µm; mobile phase A: supercritical carbon dioxide, mobile phase B: isopropanol (10 mmol ammonia); flow rate: 2 mL/min; gradient elution with 40% to 50% phase B in 8 min; detector UV 220 nm; retention time: 5.095 min; 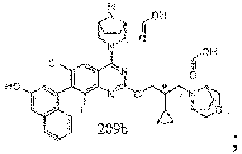
its HPLC conditions are: chiral column: CHIRALPAK ID-3, 3.0 x 100 mm, 3 µm; mobile phase A: supercritical carbon dioxide, mobile phase B: isopropanol (10 mmol ammonia); flow rate: 2 mL/min; gradient elution with 40% to 50% phase B in 8 min; detector UV 220 nm; retention time: 6.135 min; 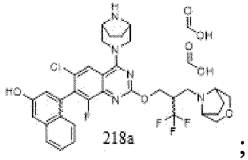
its HPLC conditions are: chiral column: CHIRALPAK IA-3, 3.0 x 100 mm, 3 µm; mobile phase A: supercritical carbon dioxide, mobile phase B: isopropanol (10 mmol ammonia); flow rate: 2 mL/min; isocratic elution with 50% phase B in 4 min; detector UV 220 nm; retention time: 1.742 min; 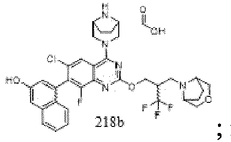
its HPLC conditions are: chiral column: CHIRALPAK IA-3, 3.0 x 100 mm, 3 µm; mobile phase A: supercritical carbon dioxide, mobile phase B: isopropanol (10 mmol ammonia); flow rate: 2 mL/min; isocratic elution with 50% phase B in 4 min; detector UV 220 nm; retention time: 2.904 min; 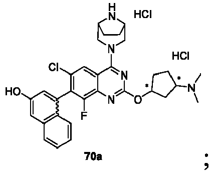
its HPLC conditions are: chiral column: CHIRALPAK IA-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 254 nm; retention time: 4.874 min; 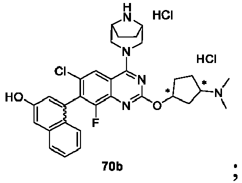
its HPLC conditions are: chiral column: CHIRALPAK IA-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 254 nm; retention time: 2.414 min; 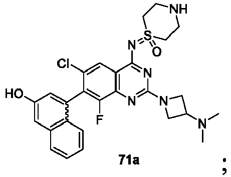
its HPLC conditions are: chiral column: CHIRALPAK IF-3, 4.6 x 50 mm, 3 µm; mobile phase A: *n*-hexane (0.1% diethylamine), mobile phase B: ethanol; flow rate: 1 mL/min; isocratic elution, elution with 20% phase B in 12 min; detector UV 220/254 nm; retention time: 6.743 min; 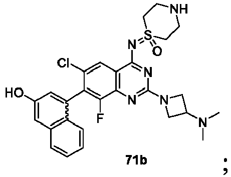
its HPLC conditions are: chiral column: CHIRALPAK IF-3, 4.6 x 50 mm, 3 µm; mobile phase A: *n*-hexane (0.1% diethylamine), mobile phase B: ethanol; flow rate: 1 mL/min; gradient, elution with 20% phase B in 12 min; detector UV 220/254 nm; retention time: 9.968 min; 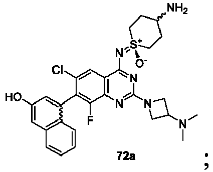
it is prepared from 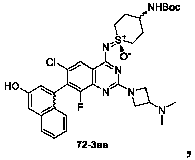 the HPLC conditions of 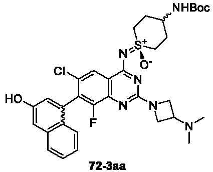 are: chiral column CHIRALART Cellulose-SB, 2 x 25 cm, 5 µm; mobile phase A: n-hexane/dichloromethane = 5/1 (0.5% 2 mol/L ammonia methanol solution), mobile phase B: ethanol; flow rate: 20 mL/min; gradient: gradient elution with 30% phase B in 10 min; detector UV 250/220 nm; retention time: 3.2 min; 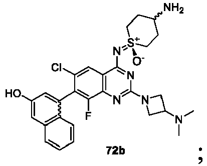
it is prepared from 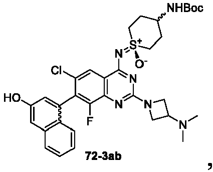 the HPLC conditions of 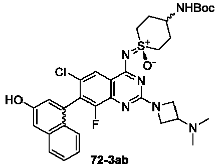 are: chiral column CHIRALART Cellulose-SB, 2 x 25 cm, 5 µm; mobile phase A: n-hexane/dichloromethane = 5/1 (0.5% 2 mol/L ammonia methanol solution), mobile phase B: ethanol; flow rate: 20 mL/min; gradient: gradient elution with 30% phase B in 10 min; detector UV 250/220 nm; retention time: 5.7 min; 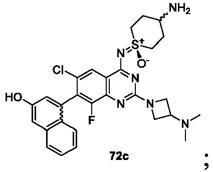
it is prepared from 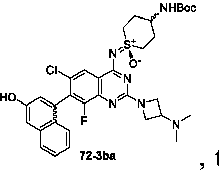 the HPLC conditions of 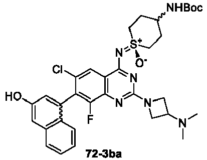 are: chiral column CHIRAL ART Cellulose-SB, 2 x 25 cm, 5 µm; mobile phase A: *n*-hexane (10 mol/L ammonia methanol solution), mobile phase B: ethanol; flow rate: 20 mL/min; gradient: gradient elution with 30% phase B in 23 min; detector UV 250/220 nm; retention time: 5.8 min; 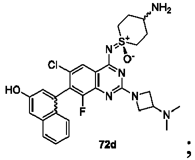
it is prepared from 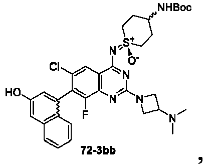 the HPLC conditions of 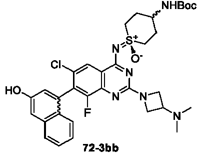 are: chiral column CHIRAL ART Cellulose-SB, 2 x 25 cm, 5 µm; mobile phase A: *n*-hexane (10 mol/L ammonia methanol solution), mobile phase B: ethanol; flow rate: 20 mL/min; gradient: gradient elution with 30% phase B in 23 min; detector UV 250/220 nm; shorter retention time: 15.8 min; 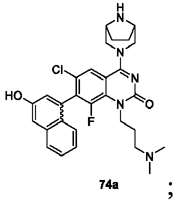
its HPLC conditions are: chiral column: CHIRALPAK ID-3, 4.6 x 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV220/254 nm; retention time: 3.994 min; 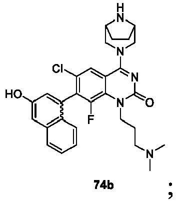
its HPLC conditions are: chiral column: CHIRALPAK ID-3, 4.6 x 50 mm, 3 µm; mobile phase A: methyl *tert*-butyl ether (0.1% diethylamine), mobile phase B: ethanol; flow rate: 1 mL/min; isocratic elution with 20% phase B in 6 min; detector UV 220/254 nm; retention time: 4.737 min; 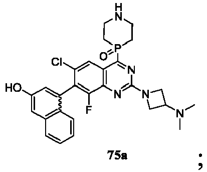
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 17 min; detector UV 254 nm; retention time: 11.543 min; 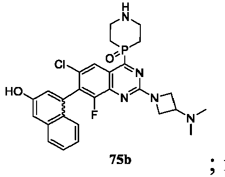
its HPLC conditions are: chiral column: CHIRALPAK IA-3, 4.6 x 50 mm, 3 µm; mobile phase A: *n*-hexane (0.1% diethylamine), mobile phase B: isopropanol; flow rate: 1 mL/min; isocratic elution with 20% phase B in 17 min; detector UV 254 nm; retention time: 6.706 min; 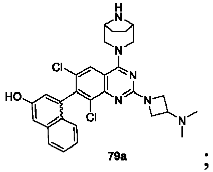
it is prepared from 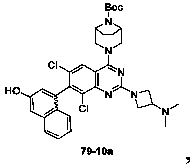 the HPLC conditions of 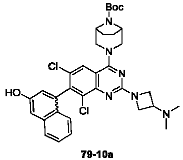 are: chiral column CHIRAL IA, 2 x 25 cm, 5 µm; mobile phase A: n-hexane (10 mol/L ammonia methanol solution), mobile phase B: isopropanol; flow rate: 20 mL/min; gradient: elution with 30% mobile phase in 11.5 min; detector UV 220/210 nm; retention time: 4.342 min; 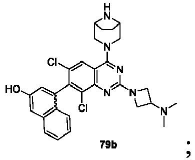
it is prepared from 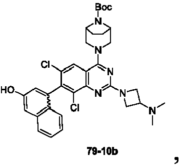 the HPLC conditions of 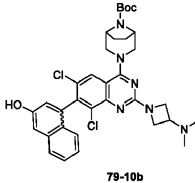 are: chiral column CHIRAL IA, 2 x 25 cm, 5 µm; mobile phase A: *n-*hexane (10 mol/L ammonia methanol solution), mobile phase B: isopropanol; flow rate: 20 mL/min; gradient: elution with 30% mobile phase in 11.5 min; detector UV 220/210 nm; retention time: 7.54 min; 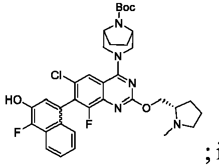
its HPLC conditions are: chiral column CHIRALART Cellulose-SC, 2 x 25 cm, 5 µm; mobile phase A: *n*-hexane (0.5% 2 mol/L ammonia methanol solution), mobile phase B: ethanol; flow rate: 20 mL/min; gradient: elution with 20% phase B in 12 min; detector UV 220/254 nm; retention time: 5.875 min; 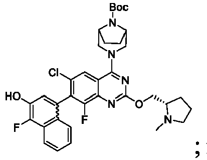
its HPLC conditions are: chiral column: CHIRALART Cellulose-SC, 2 x 25 cm, 5 µm; mobile phase A: *n*-hexane/dichloromethane = 5/1 (0.5% 2 mol/L ammonia methanol solution), mobile phase B: ethanol; flow rate: 20 mL/min; gradient: elution with 20% phase B in 12 min; detector UV 220/254 nm; retention time: 8.193 min; 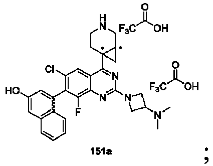
it is prepared from 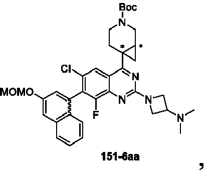 the HPLC conditions of 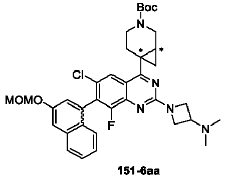 are: chiral column: CHIRALCELAY-H, 2 x 25 cm, 5 µm; mobile phase A: *n*-hexane (0.5% 2 mol/L ammonia methanol solution), mobile phase B: isopropanol/acetonitrile= 2/1; flow rate: 20 mL/min; gradient: gradient elution with 30% phase B in 13 min; detector UV 226/254 nm; retention time: 3.7 min; 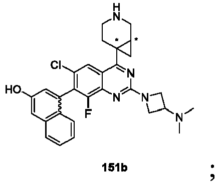
it is prepared from 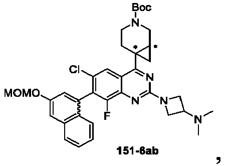 the HPLC conditions of 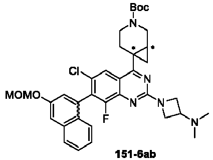 are: chiral column: CHIRALCELAY-H, 2 x 25 cm, 5 µm; mobile phase A: *n*-hexane (0.5% 2 mol/L ammonia methanol solution), mobile phase B: isopropanol/acetonitrile= 2/1; flow rate: 20 mL/min; gradient: gradient elution with 30% phase B in 13 min; detector UV 226/254 nm; retention time: 6.8 min; 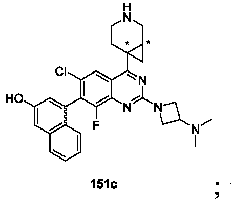
it is prepared from 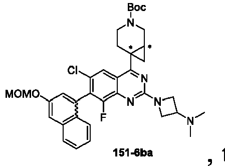 the HPLC conditions of 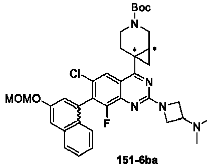 are: chiral column: CHIRALPAK IE, 2 x 25 cm, 5 µm; mobile phase A: *n*-hexane (10 mol/L ammonia methanol solution), mobile phase B: isopropanol/acetonitrile= 2/1; flow rate: 20 mL/min; gradient elution with 10% phase B in 18 min; detector UV 226/254 nm; retention time: 6 min; 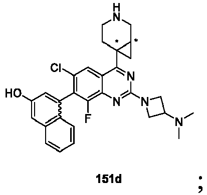
it is prepared from 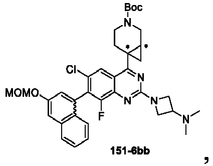 the HPLC conditions of 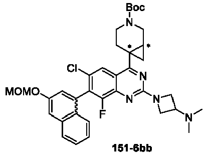 are: chiral column: CHIRALPAK IE, 2 x 25 cm, 5 µm; mobile phase A: n-hexane (10 mol/L ammonia methanol solution), mobile phase B: isopropanol/acetonitrile= 2/1; flow rate: 20 mL/min; gradient elution with 10% phase B in 18 min; detector UV 226/254 nm; retention time: 8.5 min; 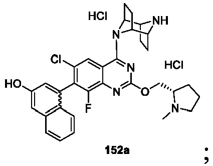
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 6 min; detector UV 220 nm; retention time: 3.123 min; 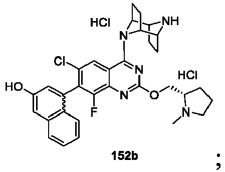
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: ethanol; flow rate: 1 mL/min; isocratic elution with 10% phase B in 6 min; detector UV 220 nm; retention time: 5.171 min; 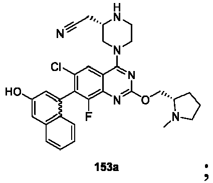
its HPLC conditions are: chiral column: CHIRALPAK ID-3, 4.6 x 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 1.508 min; 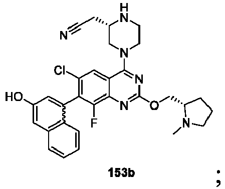
its HPLC conditions are: chiral column: CHIRALPAK ID-3, 4.6 x 50 mm, 3 µm; mobile phase A: *n*-hexane (0.1% diethylamine), mobile phase B: ethanol; flow rate: 1 mL/min; isocratic elution with 50% phase B in 6 min; detector UV 254 nm; retention time: 2.593 min; 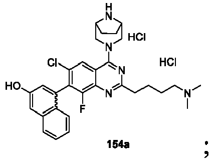
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220 nm; retention time: 2.156 min; 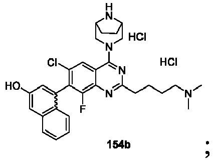
its HPLC conditions are: chiral column: CHIRALPAK IC-3, 4.6 x 50 mm, 3 µm; mobile phase A: *n*-hexane/dichloromethane = 5/1 (0.1% diethylamine), mobile phase B: isopropanol; flow rate: 1 mL/min; isocratic elution with 30% phase B in 6 min; detector UV 220 nm; retention time: 4.420 min; 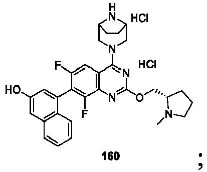
it is prepared from 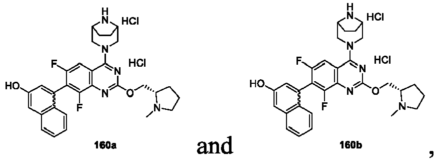 the HPLC conditions of 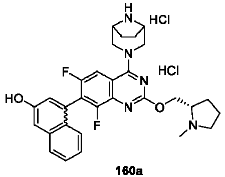 are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector UV 254 nm; retention time: 2.198 min; the HPLC conditions of 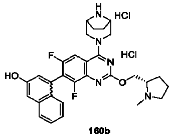 are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector UV 254 nm; retention time: 3.411 min; 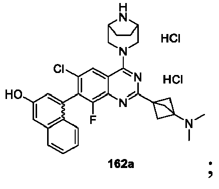
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector: UV 220 nm; retention time: 2.239 min; 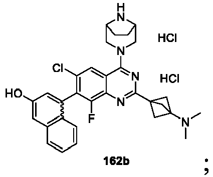
its HPLC conditions are: chiral column: CHIRALPAK IC-3, 4.6 x 50 mm, 3 µm; mobile phase A: *n*-hexane (0.1% diethylamine), mobile phase B: isopropanol; flow rate: 1 mL/min; isocratic elution with 30% phase B in 6 min; detector UV 220 nm; retention time: 3.881 min; 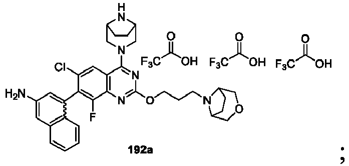
its HPLC conditions are: chiral column: CHIRAL Cellulose-SB, 4.6 x 100 mm, 3 µm; mobile phase A: *n*-hexane: methyl *tert*-butyl ether = 1:1 (0.1% diethylamine), mobile phase B: ethanol; flow rate: 1 mL/min; isocratic elution with 30% phase B in 8.5 min; detector UV 220/254 nm; retention time: 5.962 min; 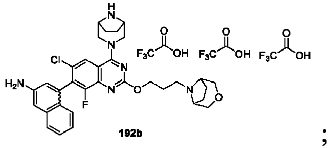
its HPLC conditions are: chiral column: CHIRAL Cellulose-SB, 4.6 x 100 mm, 3 µm; mobile phase A: *n*-hexane: methyl *tert*-butyl ether = 1:1 (0.1% diethylamine), mobile phase B: ethanol; flow rate: 1 mL/min; isocratic elution with 30% phase B in 8.5 min; detector UV 220/254 nm; retention time: 7.373 min; 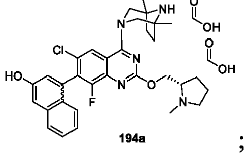
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 8 min; detector UV 220/254 nm; retention time: 1.999 min; 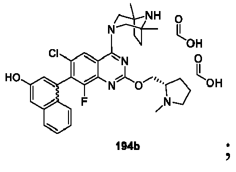
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; isocratic elution with 20% phase B in 8 min; detector UV 220/254 nm; retention time: 3.292 min; 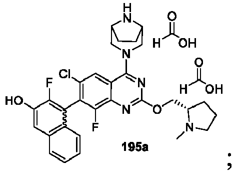
its HPLC conditions are: chiral column: N--Lux 3µm Cellulose-4 (H1 8-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 3.734 min; 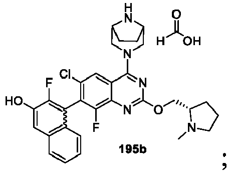
its HPLC conditions are: chiral column: N--Lux 3µm Cellulose-4 (H18-063498), 4.6 x 100 mm, 3 µm; mobile phase A: supercritical carbon dioxide fluid, mobile phase B: methanol (0.1% diethylamine); flow rate: 3 mL/min; isocratic elution with 40% phase B in 10 min; detector UV 220 nm; retention time: 2.913 min; 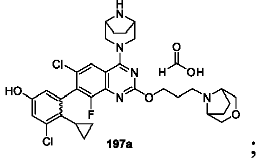
its HPLC conditions are: chiral column: CHIRAL ART Cellulose-SB, 4.6 x 100 mm, 3 µm; mobile phase A: *n*-hexane (0.1% diethylamine), mobile phase B: ethanol; flow rate: 1 mL/min; isocratic elution with 10% phase B in 4.5 min; detector UV 220 nm; retention time: 1.346 min; 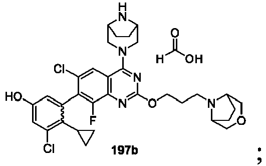
its HPLC conditions are: chiral column: CHIRAL ART Cellulose-SB, 4.6 x 100 mm, 3 µm; mobile phase A: *n*-hexane (0.1% diethylamine), mobile phase B: ethanol; flow rate: 1 mL/min; isocratic elution with 10% phase B in 4.5 min; detector UV 220 nm; retention time: 2.438 min; 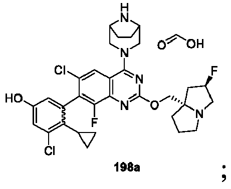
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 3.5 min; detector UV 220/254 nm; retention time: 1.198 min; 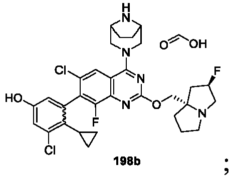
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 20% phase B in 4 min; detector UV 220/254 nm; retention time: 1.880 min; 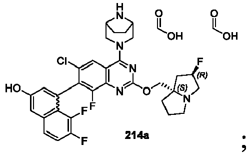
its HPLC conditions are: chiral column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 4.125 min; 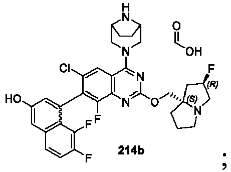
its HPLC conditions are: chiral column: CHIRALPAK IA-3, 3.0 x 50 mm, 3 µm; mobile phase A: *n*-hexane (0.1% diethylamine), mobile phase B: isopropanol; flow rate: 1 mL/min; isocratic elution with 30% phase B in 6 min; detector UV 210 nm; retention time: 2.126 min; 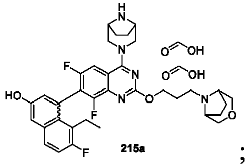
its HPLC conditions are: chiral column: XA-CHIRALPAK IG-3, 4.6 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 4.214 min; 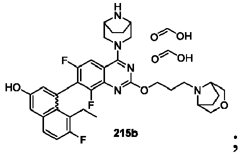
its HPLC conditions are: chiral column: XA-CHIRALPAK IG-3, 4.6 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 2.706 min; 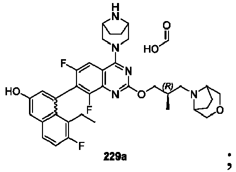
its HPLC conditions are: chiral column: CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 1.083 min; 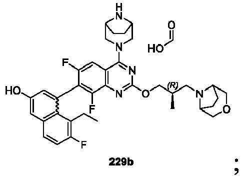
its HPLC conditions are: chiral column: CHIRALPAK IA-3, 3.0 x 100 mm, 3 µm; mobile phase A: *n*-hexane (0.1% diethylamine), mobile phase B: ethanol; flow rate: 2 mL/min; isocratic elution with 30% phase B in 4 min; detector UV 254 nm; retention time: 2.010 min; 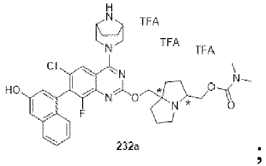
its HPLC conditions are: chiral column: CHIRALPAK IA-3, 4.6 x 50 mm, 3 µm; mobile phase A: *n*-hexane (0.1% diethylamine), mobile phase B: ethanol (10mmol/L ammonia); flow rate: 1 mL/min; isocratic elution with 50% phase B in 4 min; detector UV 220 nm; retention time: 2.761 min; 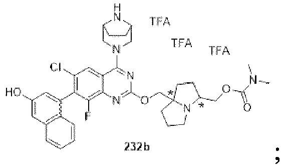
its HPLC conditions are: chiral column: CHIRALPAK IA-3, 4.6 x 50 mm, 3 µm; mobile phase A: *n*-hexane (0.1% diethylamine), mobile phase B: ethanol (10mmol/L ammonia); flow rate: 1 mL/min; isocratic elution with 50% phase B in 4 min; detector UV 220 nm; retention time: 1.705 min; 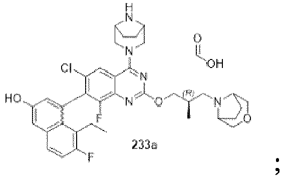
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 3.5 min; detector UV 254 nm; retention time: 2.301 min; 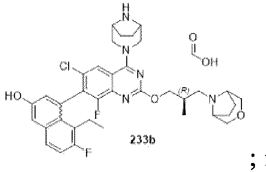
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; isocratic elution with 40% phase B in 4.5 min; detector UV 254 nm; retention time: 1.465 min; 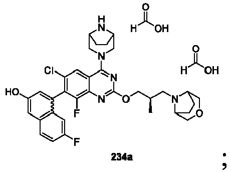
its HPLC conditions are: chiral column: N-Lux 3µm Cellulose-4 (H17-388767); 4.6 x 100 mm, 3 µm; mobile phase A: supercritical carbon dioxide, mobile phase B: methanol (0.1% diethylamine); flow rate: 3.5 mL/min; isocratic elution with 48% phase B in 6 min; detector UV 220 nm; retention time: 2.41 min; 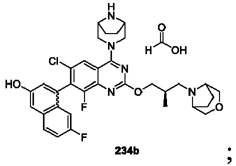
its HPLC conditions are: chiral column: N-Lux 3µm Cellulose-4 (H17-388767); 4.6 x 100 mm, 3 µm; mobile phase A: supercritical carbon dioxide, mobile phase B: methanol (0.1% diethylamine); flow rate: 3.5 mL/min; isocratic elution with 48% phase B in 6 min; detector UV 220 nm; retention time: 3.66 min; 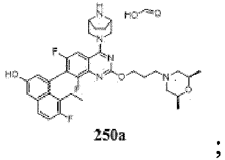
its HPLC conditions are: chiral column: N--CHIRALPAK IC-3 (Lot No.IC3SCK-VK002), 3.0 x 100 mm, 3 µm; mobile phase A: supercritical carbon dioxide, mobile phase B: methanol (20 mmol/L ammonia); flow rate: 2 mL/min; isocratic elution with 10% phase B in 6 min; detector UV 220 nm; retention time: 4.514 min; 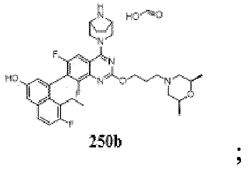
its HPLC conditions are: chiral column: N--CHIRALPAK IC-3 (LotNo.IC3SCK-VK002), 3.0 x 100 mm, 3 µm; mobile phase A: supercritical carbon dioxide, mobile phase B: methanol (20 mmol/L ammonia); flow rate: 2 mL/min; isocratic elution with 10% phase B in 6 min; detector UV 220 nm; retention time: 4.948 min; 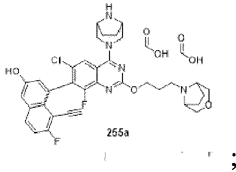
its HPLC conditions are: chiral column: N-CHIRALPAK IG-3, 3.0 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 0.845 min; 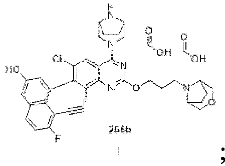
its HPLC conditions are: chiral column: N-CHIRALPAK IG-3, 3.0 x 100 mm, 3 µm; mobile phase A: supercritical carbon dioxide fluid, mobile phase B: isopropanol (10 mmol/L ammonia); flow rate: 2 mL/min; isocratic elution with 50% phase B in 6 min; detector UV 220 nm; retention time: 1.905 min; 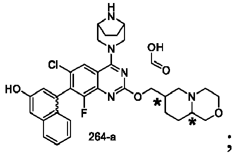
its HPLC conditions are: chiral column: N-CHIRALPAK IE-3 (Lot No.IF3SCK-SD016), 3 x 100 mm, 3 µm; mobile phase A: supercritical carbon dioxide, mobile phase B: methanol (20 mmol/L ammonia); flow rate: 2 mL/min; gradient: isocratic elution with 50% phase B in 8 min; detector: UV 220 nm; retention time: 4.857 min; 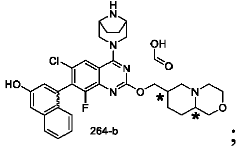
its HPLC conditions are: chiral column: N-CHIRALPAK IE-3 (Lot No.IF3SCK-SD016), 3 x 100 mm, 3 µm; mobile phase A: supercritical carbon dioxide fluid, mobile phase B: methanol (20 mmol/L ammonia); flow rate: 2 mL/min; gradient: isocratic elution with 50% phase B in 8 min; detector: UV 220 nm; retention time: 5.877 min; 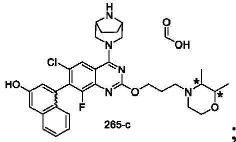
its HPLC conditions are: chiral column: N-Lux 3u i-Cellulose-5; 0.46 x 10 cm, 3 µm; mobile phase A: supercritical carbon dioxide, mobile phase B: methanol: dichloromethane =1:1 (20 mmol/L ammonia); flow rate: 2 mL/min; gradient: isocratic elution with 50% phase B; detector UV 230 nm; retention time: 4.976 min; 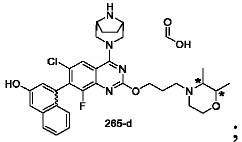
its HPLC conditions are: chiral column: N-Lux 3u i-Cellulose-5, 0.46 x 10 cm, µm; mobile phase A: supercritical carbon dioxide, mobile phase B: methanol: dichloromethane =1:1 (20 mmol/L ammonia); flow rate: 2 mL/min; gradient: isocratic elution with 50% phase B; detector UV 230 nm; retention time: 5.657 min; 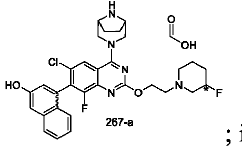
its HPLC conditions are: chiral column: N-Lux 3 µm Cellulose-2 (H18-089501), 4.6 x 100 mm, 3.0 µm; mobile phase A: supercritical carbon dioxide, mobile phase B: methanol (0.1% diethylamine); flow rate: 4 mL/min; gradient: isocratic elution with 35% phase B; detector: UV 220 nm; retention time: 9.221 min; 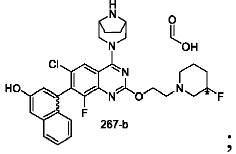
its HPLC conditions are: chiral column: N-Lux 3 µm Cellulose-2 (H18-089501), 4.6 x 100 mm, 3.0 µm; mobile phase A: supercritical carbon dioxide, mobile phase B: methanol (0.1% diethylamine); flow rate: 4 mL/min; gradient: isocratic elution with 35% phase B; detector: UV 220 nm; retention time: 7.830 min; 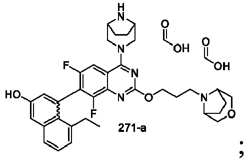
its HPLC conditions are: chiral column: CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.822 min; 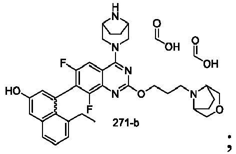
its HPLC conditions are: chiral column: CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 220 nm; retention time: 1.296 min; 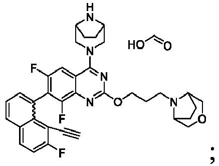
its HPLC conditions are: chiral column: CHIRALART Cellulose-SB (Ser.No. 105CA80166), 4.6 x 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 2.055 min; 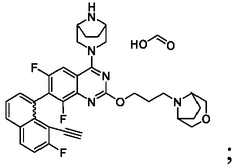
its HPLC conditions are: chiral column: CHIRALART Cellulose-SB (Ser.No. 105CA80166), 4.6 x 100 mm, 3 µm; mobile phase A: supercritical carbon dioxide fluid, mobile phase B: methanol (20 mmol/L ammonia); flow rate: 2 mL/min; isocratic elution with 50% phase B in 4 min; detector UV 230 nm; retention time: 1.632 min; 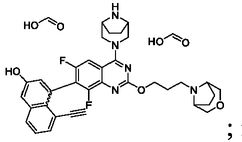
its HPLC conditions are: chiral column: CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.878 min; 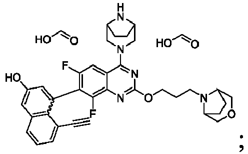
its HPLC conditions are: chiral column: CHIRALPAK ID-3 (Lot No.ID3SCK-TB004), 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 1.217 min; 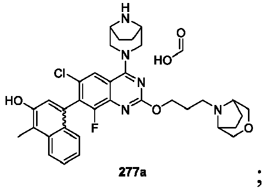
its HPLC conditions are: chiral column: N-CHIRALPAK IG-3, 3.0 x 100 mm, 3 µm; mobile phase A: supercritical carbon dioxide fluid, mobile phase B: isopropanol (20 mmol/L ammonia); flow rate: 2 mL/min; isocratic elution with 50% phase B in 4 min; detector UV 230 nm; retention time: 2.601 min; 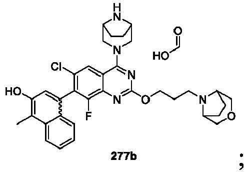
its HPLC conditions are: chiral column: N-CHIRALPAK IG-3, 3.0 x 100 mm, 3 µm; mobile phase A: supercritical carbon dioxide, mobile phase B: isopropanol (20 mmol/L ammonia); flow rate: 2 mL/min; isocratic elution with 50% phase B in 4 min; detector UV 230 nm; retention time: 0.815 min; 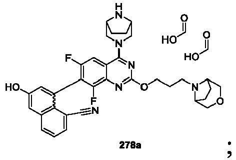
its HPLC conditions are: chiral column: N-CHIRALPAK IG-3, 3.0 x 100 mm, 3 µm; mobile phase A: supercritical carbon dioxide, mobile phase B: isopropanol (20 mmol/L ammonia); flow rate: 2 mL/min; isocratic elution with 10% phase B in 6.5 min; detector UV 230 nm; retention time: 4.553 min; 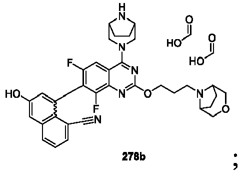
its HPLC conditions are: chiral column: N-CHIRALPAK IG-3, 3.0 x 100 mm, 3 µm; mobile phase A: supercritical carbon dioxide, mobile phase B: isopropanol (20 mmol/L ammonia); flow rate: 2 mL/min; isocratic elution with 10% phase B in 6.5 min; detector UV 230 nm; retention time: 4.074 min; 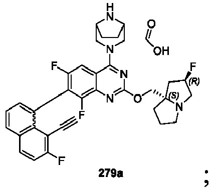
its HPLC conditions are: chiral column: CHIRALPAK IC-3 (Lot No.IC3SCK-VK002), 3 x 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 2.80 min; 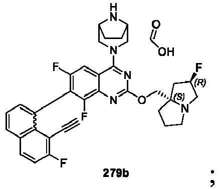
its HPLC conditions are: chiral column: CHIRALPAK IC-3 (Lot No.IC3SCK-VK002), 3 x 100 mm, 3 µm; mobile phase A: supercritical carbon dioxide, mobile phase B: methanol (20 mmol/L ammonia); flow rate: 2 mL/min; isocratic elution with 50% phase B in 4 min; detector UV 230 nm; retention time: 2.318 min; 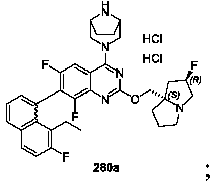
its HPLC conditions are: chiral column: Cellulose-SB, 3 x 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 1.911 min; 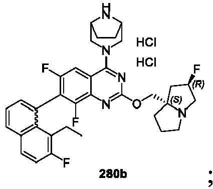
its HPLC conditions are: chiral column: Cellulose-SB, 3 x 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.171 min; 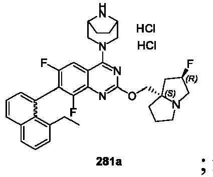
its HPLC conditions are: chiral column: CHIRALART Cellulose-SB, 3 x 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.148 min; 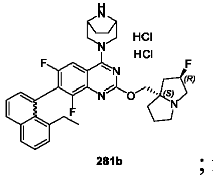
its HPLC conditions are: chiral column CHIRAL ART Cellulose-SB, 3 x 100 mm, 3 µm; mobile phase A: supercritical carbon dioxide, mobile phase B: methanol (20 mmol/L ammonia); flow rate: 2 mL/min; elution with 10% phase B in 3 min; detector UV 220 nm; retention time: 2.443 min; 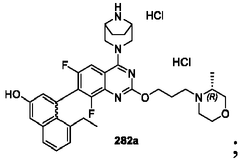
its HPLC conditions are: chiral column: N-Lux 3um Cellulose-4 (H17-388767); 4.6 x 100 mm, 3 µm; mobile phase A: supercritical carbon dioxide, mobile phase B: ethanol (20 mmol/L ammonia); flow rate: 3.5 mL/min; isocratic elution with 35% phase B in 6.5 min; detector UV 220 nm; retention time: 3.455 min; 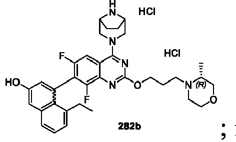
its HPLC conditions are: chiral column: N--Lux 3µm Cellulose-4 (H17-388767); 4.6 x 100 mm, 3 µm; mobile phase A: supercritical carbon dioxide, mobile phase B: ethanol (20 mmol/L ammonia); flow rate: 3.5 mL/min; isocratic elution with 35% phase B in 6.5 min; detector UV 220 nm; retention time: 4.723 min; 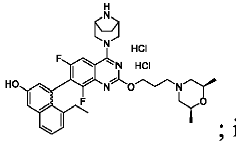
its HPLC conditions are: chiral column: N--Lux 3µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 1.349 min; 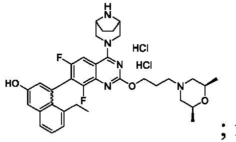
its HPLC conditions are: chiral column: N--Lux 3µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 1.903 min; 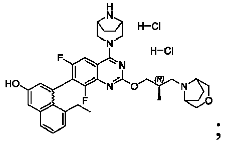
its HPLC conditions are: chiral column: N-Lux 3µm Cellulose-2 (H18-089501), 4.6 x 100 mm, 3 µm; supercritical mobile phase A: carbon dioxide, mobile phase B: methanol (0.1% diethylamine); flow rate: 4 mL/min; gradient, elution with 50% phase B in 6 min; detector UV 254 nm; retention time: 2.715 min; 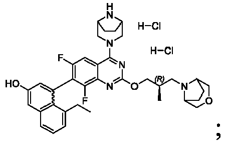
its HPLC conditions are: chiral column: N-Lux 3µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient, elution with 50% phase B in 6 min; detector UV 254 nm; retention time: 3.767 min; 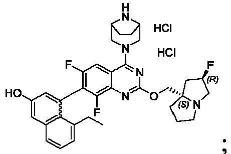
its HPLC conditions are: chiral column: N-Lux 3µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient, elution with 40% phase B in 7 min; detector UV 220 nm; retention time: 3.698 min; 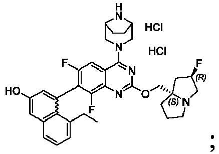
its HPLC conditions are: chiral column: N-Lux 3µm Cellulose-2 (H18-089501), 4.6 x 100 mm, 3 µm; mobile phase A: supercritical carbon dioxide, mobile phase B: methanol (0.1% diethylamine); flow rate: 4 mL/min; gradient, elution with 40% phase B in 7 min; detector UV 220 nm; retention time: 5.109 min; 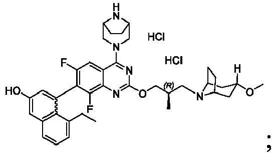
its HPLC conditions are: chiral column: CHIRALPAK IC-3, 4.6 x 50 mm, 3 µm; mobile phase A: n-hexane (0.1% diethylamine), mobile phase B: ethanol; flow rate: 1 mL/min; gradient, elution with 50% phase B in 4 min; detector UV 254 nm; retention time: 0.617 min; 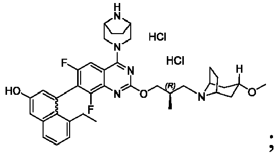
its HPLC conditions are: chiral column: CHIRALPAK IC-3, 4.6 x 50 mm, 3 µm; mobile phase A: *n*-hexane (0.1% diethylamine), mobile phase B: ethanol; flow rate: 1 mL/min; gradient, elution with 50% phase B in 4 min; detector UV 254 nm; retention time: 1.334 min.

19. A pharmaceutical composition comprising the quinazoline compound represented by formula I, the pharmaceutically acceptable salt thereof, the solvate thereof, the prodrug thereof, the metabolite thereof or the isotopic compound thereof according to any one of claims 1 to 18, and one or more pharmaceutical excipients.

20. A use of the quinazoline compound represented by formula I, the pharmaceutically acceptable salt thereof, the solvate thereof, the prodrug thereof, the metabolite thereof or the isotopic compound thereof according to any one of claims 1 to 18 or the pharmaceutical composition according to claim 19 in the manufacture of a KRAS mutant protein inhibitor; the KRAS mutant protein can be a KRAS G12D mutant protein.

21. A use of the quinazoline compound represented by formula I, the pharmaceutically acceptable salt thereof, the solvate thereof, the prodrug thereof the metabolite thereof or the isotopic compound thereof according to any one of claims 1 to 18 or the pharmaceutical composition according to claim 19 in the manufacture of a medicament, the medicament is preferably used for the prevention and/or treatment of cancer mediated by KRAS mutation, and the KRAS mutation protein can be KRAS G12D mutant protein; the cancer can be hematological cancer, pancreatic cancer, MYH-associated polyposis, colorectal cancer or lung cancer.

22. A use of the quinazoline compound represented by formula I, the pharmaceutically acceptable salt thereof, the solvate thereof, the prodrug thereof, the metabolite thereof or the isotopic compound thereof according to any one of claims 1 to 18 or the pharmaceutical composition according to claim 19 in the manufacture of a medicament, the medicament is preferably used for the prevention and/or treatment of cancer, the cancer is for example hematological cancer, pancreatic cancer, MYH-associated polyposis, colorectal cancer or lung cancer.
